(19) Europäisches Patentamt
European Patent Office
Office européen des brevets

(11) **EP 4 745 242 A1**

(12) **EUROPEAN PATENT APPLICATION**
published in accordance with Art. 153(4) EPC

(43) Date of publication:
**20.05.2026 Bulletin 2026/21**

(51) International Patent Classification (IPC):
**C12N 15/87** (2006.01)

(21) Application number: **24842464.0**

(86) International application number:
**PCT/CN2024/112170**

(22) Date of filing: **14.08.2024**

(87) International publication number:
**WO 2025/016479 (23.01.2025 Gazette 2025/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.07.2023 CN 202310871667**

(71) Applicant: **Bai Yao Zhi Da (Beijing) Nanobio Technology Co., Ltd.**
**Beijing 102206 (CN)**

(72) Inventors:
• **WANG, Liyuan**
**Beijing 102206 (CN)**
• **WEI, Hong**
**Beijing 102206 (CN)**
• **WANG, Meng**
**Beijing 102206 (CN)**

(74) Representative: **Winter, Brandl - Partnerschaft mbB**
**Alois-Steinecker-Straße 22**
**85354 Freising (DE)**

Remarks:
•A request for restoration of the right of priority by the EPO as designated Office has been granted (R. 49ter.2 PCT, Art.122 EPC)
•The complete document including Reference Table(s) and the Sequence Listing(s) can be downloaded from the EPO website

(54) **NUCLEIC ACID VECTOR, NUCLEIC ACID DRUG, PREPARATION METHOD THEREFOR, AND USE THEREOF**

(57) The present invention provides a nucleic acid carrier, a nucleic acid drug, and methods for their preparation and use. The nucleic acid carrier includes sequences a, b, and c, and is selected from a DNA carrier, an RNA carrier, or a DNA-RNA hybrid carrier; the sequences a, b, and c are capable of self-assembling to form the nucleic acid carrier; each of the sequences a, b, and c include scaffold sequences that include the following core sequences: an sequence a1 as shown in SEQ ID No. 1 or 4, a sequence b1 as shown in SEQ ID No. 2 or 5, and a sequence c1 as shown in SEQ ID No. 3 or 6, or any variant thereof comprising an insertion, deletion, or substitution of at least one nucleotide in at least one of the sequences a1, b1, and c1; the DNA-RNA hybrid carrier is a carrier self-assembled from a combination of the sequence of the DNA carrier and the sequence of the RNA carrier. The carrier exhibits high delivery efficiency, provides a more stable delivery system, and enables a simplified and efficient manufacturing process.

**Chromatogram of Sample C2**

| Peak No. | Retention Time | Peak Area | Peak Height | Peak Width | Symmetry Factor | Peak Area (%) |
|---|---|---|---|---|---|---|
| 1 | 19.135 | 852.1 | 15.9 | 1.092 | 0 | 3.501 |
| 2 | 20.667 | 19222.4 | 336.1 | 0.9531 | 0.654 | 78.986 |
| 3 | 22.284 | 3237.1 | 51 | 1.0585 | 0.275 | 13.301 |
| 4 | 24.068 | 436.2 | 7.4 | 0.9843 | 0 | 1.792 |
| 5 | 25.835 | 588.7 | 6.5 | 1.5181 | 0 | 2.419 |

**FIG. 1-2**

EP 4 745 242 A1

**Description**

Cross-Reference to Related Applications

This application claims priority to Chinese Patent Application No. 202310871667.8, filed on July 14, 2023. The entire disclosure of the above Chinese patent application is incorporated herein by reference in its entirety.

Technical Field

The present invention relates to the field of nucleic acid therapeutics, and more particularly to a nucleic acid carrier, a nucleic acid drug, and methods for their preparation and use.

Background Art

Single- or double-stranded RNA drugs having relatively small molecular weight (the number of nucleotides is typically no more than 100) are collectively referred to as "oligonucleotide drugs." Oligonucleotide drugs constitute a novel class of therapeutics fundamentally different from small-molecule drugs and antibody drugs. Comprising nucleotide sequences, they act on mRNA to suppress the expression of target proteins via gene silencing mechanisms, that is, after gene transcription and before protein translation, thereby achieving therapeutic effects. This field represents a strategic frontier of biopharmaceutical innovation.

Compared with antibody drugs, the R&D of oligonucleotide drugs does not require complex protein modification and Chemistry, Manufacturing and Controls (CMC) development, and the manufacturing processes at the production stage are relatively simple, without the need for large-scale mammalian cell fermentation and protein purification. They offer advantages such as an abundant pool of candidate targets, shorter R&D cycles, durable efficacy, and higher clinical development success rates. By intervening at the post-transcriptional level, oligonucleotide drugs can achieve breakthroughs against special protein targets that are difficult to drug, and are expected to address diseases for which no drug therapy currently exists, including genetic diseases and other refractory conditions; they thus have great potential to deliver therapeutics for "non-targetable" and "undruggable" diseases, and are poised to constitute the third wave of modern new drugs after small-molecule and antibody drugs.

However, the following drawbacks of oligonucleotide drugs are key factors limiting their development: on the one hand, oligonucleotide drugs are excessively fragile and are readily degraded by nucleases in vivo, and chemical modifications alone are insufficient to overcome this; on the other hand, oligonucleotide drugs generally exhibit immunotoxicity and are prone to cause serious adverse effects in humans. Accordingly, the delivery of oligonucleotide drugs and their combined use remain major bottlenecks in this field that are urgently in need of breakthroughs.

Aside from the two clinically validated delivery technologies-lipid nanoparticles (LNPs) and N-acetylgalactosamine (GalNAc)-other delivery approaches have also been reported. For example, Shengnuo Pharmaceutical has developed a PNP (polypeptide nanoparticle) delivery technology, and there are the GalAhead and GalNAc-PDoV technologies. PNP, like LNP, belongs to the class of organic nanoparticle delivery systems, but allows a single PNP to load multiple siRNA molecules and thus can be used to develop multi-target nucleic acid drugs; the latter two achieve a similar effect, namely enabling delivery of more than one siRNA on a GalNAc basis. Such delivery systems are mainly directed to siRNA drugs, whereas delivery systems for other types of oligonucleotide drugs still face challenges. Therefore, the efficiency, breadth of drug types, and reliability of nucleic acid drug delivery remain to be further improved.

Summary of the Invention

The principal object of the present invention is to provide a nucleic acid carrier, a nucleic acid drug, and methods for their preparation and use, so as to solve the problem in the prior art of low delivery efficiency of various oligonucleotide drugs, particularly the difficulty of simultaneously delivering multiple oligonucleotide drugs.

To achieve the foregoing object, according to one aspect of the present invention, there is provided a nucleic acid carrier, the nucleic acid carrier comprising a sequence a, a sequence b, and a sequence c, the nucleic acid carrier being selected from a DNA carrier, an RNA carrier, or a DNA-RNA hybrid carrier; the sequence a, the sequence b, and the sequence c self-assemble to form the nucleic acid carrier, wherein the sequence a, the sequence b, and the sequence c include carrier backbone sequences that include the following core sequences: an sequence a1, a sequence b1, and a sequence c1, or any variant sequence of at least one of the sequence a1, the sequence b1, and the sequence c1, the variant sequence comprising insertion, deletion, or substitution of at least one base. In the DNA carrier, the sequence a1 is SEQ ID No. 1, the sequence b1 is SEQ ID No. 2, and the sequence c1 is SEQ ID No. 3; in the RNA carrier, the sequence a1 is SEQ ID No. 4, the sequence b1 is SEQ ID No. 5, and the sequence c1 is SEQ ID No. 6. The DNA-RNA hybrid carrier is a carrier formed by self-assembly of a combination of the sequences of the DNA carrier and the sequences of the RNA carrier.; SEQ ID No. 1: ACGAGCGTTCCG; SEQ ID No. 2: CGGTTCGCCG; SEQ ID No. 3: CGGCCATAGCCGT; SEQ ID No. 4: ACGAGCGUUCCG; SEQ ID No. 5: CGGUUCGCCG; SEQ ID No. 6: CGGCCAUAGCCGU.

Further, the carrier backbone sequence further includes a first extension segment and an optional second extension segment, the first extension segment and the second extension segment being disposed at the 5' end and/or the 3' end of any of the a1, b1, and c1 sequences; preferably, the first extension segment and the second extension segment are each, independently, 1-14 nucleotides (nt) in length; preferably, the first extension segment is selected from any one or more of the following DNA extension segments or RNA extension segments corresponding thereto: (1) at the 5' end of the sequence a1: GACGCCC; at the 3' end of the sequence c1: GGGCGTC; (2) at the 3' end of the sequence a1: GGAGAGG; at the 5' end of the sequence b1: CCTCTCC; (3) at the 3' end of the sequence b1: CGAGCC; at the 5' end of the sequence c1: GGCTCG or GGCACG; (4) at the 5' end of the sequence a1: GGCGCCC or GACGCCC; at the 3' end of the sequence c1: GGGCGCC; (5) at the 3' end of the sequence a1: GGAG; at the 5' end of the sequence b1: CTCC; (6) at the 3' end of the sequence b1: CCAGCC; at the 5' end of the sequence c1: GGCACG or GGCTCG. preferably, the second extension segment is selected from any one or more of the following DNA extension segments or RNA extension segments corresponding thereto: (1) at the 5' end of the sequence a1: C or GC; at the 3' end of the sequence c1: GC or GCT; (2) at the 3' end of the sequence a1: AG, AGC, or AGCT; at the 5' end of the sequence b1: GCT or CT; (3) at the 3' end of the sequence b1: GC, GCG, or GCGT; at the 5' end of the sequence c1: GC or CGC; (4) at the 3' end of the sequence a1: C, AGGCC, AGGCCT, or AGGAG; at the 5' end of the sequence b1: G, GGCCT, or CTCCT; (5) at the 3' end of the sequence b1: GCC or GCCT; at the 5' end of the sequence c1: GGC.

Further, the nucleic acid carrier further includes a single-stranded connecting bridge sequence and/or a transition sequence. The single-stranded connecting bridge sequence is selected from any one or more of the following: (1) located at the 3' end of the backbone sequence of the sequence a: SEQ ID No. 7: TGTAGCACGGTGGC, or the complementary sequence thereof, SEQ ID No. 8: GCCACCGTGCTACA; (2) located at the 3' end of the backbone sequence of the sequence b: SEQ ID No. 9: TCGGCGCGGCCGTG, or the complementary sequence thereof, SEQ ID No. 10: CACGGCCGCGCCGA; (3) located at the 3' end of the backbone sequence of the sequence c: SEQ ID No. 11: TGCTGCTGCTGCTG, or the complementary sequence thereof, SEQ ID No. 12: CAGCAGCAGCAGCA; The transition sequence is sequence a formed of n consecutive U, T, or A, where n is 2-8, preferably an integer of 3-6, or sequence a comprising a random combination of A, U, T, C and/or G; the transition sequence is disposed at the 5' end or the 3' end of the core sequence; preferably, the sequence a, the sequence b, and the sequence c in the nucleic acid carrier are, respectively, as follows:

The sequence a is SEQ ID No. 15: GACGCCCACGAGCGTTCCGGGAGAGG<u>TGTAGCACGGTGGC</u>, wherein the underlined portion denotes the single-stranded connecting bridge sequence;

The sequence b is SEQ ID No. 16: CCTCTCCCGGTTCGCCGCGAGCC<u>TCGGCGCGGCCGTG</u>, wherein the underlined portion denotes the single-stranded connecting bridge sequence;

The sequence c is SEQ ID No. 17: GGCTCGCGGCCATAGCCGTGGGCGTC<u>TGCTGCTGCTGCTG</u>, wherein the underlined portion denotes the single-stranded connecting bridge sequence;Or,

The sequence a, the sequence b, and the sequence c in the nucleic acid carrier are, respectively, as follows::

The sequence a is SEQ ID No. 18: GCGACGCCCACGAGCGTTCCGGGAGAGGAGC;

The sequence b is SEQ ID No. 19: GCTCCTCTCCCGGTTCGCCGCGAGCCGCG;

The sequence c SEQ ID No. 20: <u>CAGCAGCAGCAGCA</u>CGCGGCTCGCGGCCATAGCCGTGGGCGTCGC, wherein the underlined portion denotes the single-stranded connecting bridge sequence.

Further, the backbone sequences that self-assemble to form the nucleic acid carrier are selected, respectively, from any one of the following sets:

1) sequence a: SEQ ID No. 21: GCGACGCCCACGAGCGTTCCGGGAGAGGAG,

sequence b: SEQ ID No. 22: CTCCTCTCCCGGTTCGCCGCGAGCCGCG,

sequence c: SEQ ID No. 23: CGCGGCACGCGGCCATAGCCGTGGGCGTCGC;

2) sequence a: SEQ ID No. 24: GCGACGCCCACGAGCGTTCCGGGAGAGGAGCT,

sequence b: SEQ ID No. 19: GCTCCTCTCCCGGTTCGCCGCGAGCCGCG,
sequence c: SEQ ID No. 23: CGCGGCACGCGGCCATAGCCGTGGGCGTCGC;

3) sequence a: SEQ ID No. 24: GCGACGCCCACGAGCGTTCCGGGAGAGGAGCT,

sequence b: SEQ ID No. 25: GCTCCTCTCCCGGTTCGCCGCGAGCCGCGT,
sequence c: SEQ ID No. 26: CGCGGCTCGCGGCCATAGCCGTGGGCGTCGCT;

4) sequence a: SEQ ID No. 24: GCGACGCCCACGAGCGTTCCGGGAGAGGAGCT,

sequence b: SEQ ID No. 25: GCTCCTCTCCCGGTTCGCCGCGAGCCGCGT,
sequence c: SEQ ID No. 27: CGCGGCACGCGGCCATAGCCGTGGGCGTCGCT;

5) sequence a: SEQ ID No. 28: GACGCCCACGAGCGTTCCGGGAGAGG,

sequence b: SEQ ID No. 29: CCTCTCCCGGTTCGCCGCGAGCC,
sequence c: SEQ ID No. 30: GGCTCGCGGCCATAGCCGTGGGCGTC;

6) sequence a: SEQ ID No. 18: GCGACGCCCACGAGCGTTCCGGGAGAGGAGC,

sequence b: SEQ ID No. 19: GCTCCTCTCCCGGTTCGCCGCGAGCCGCG,
sequence c: SEQ ID No. 31: CGCGGCTCGCGGCCATAGCCGTGGGCGTCGC;

7) sequence a: SEQ ID No. 24: GCGACGCCCACGAGCGTTCCGGGAGAGGAGCT,

sequence b: SEQ ID No. 19: GCTCCTCTCCCGGTTCGCCGCGAGCCGCG,
sequence c: SEQ ID No. 26: CGCGGCTCGCGGCCATAGCCGTGGGCGTCGCT;

8) sequence a: SEQ ID No. 24: GCGACGCCCACGAGCGTTCCGGGAGAGGAGCT,

sequence b: SEQ ID No. 19: GCTCCTCTCCCGGTTCGCCGCGAGCCGCG,
sequence c: SEQ ID No. 31: CGCGGCTCGCGGCCATAGCCGTGGGCGTCGC;

9) sequence a: SEQ ID No. 24: GCGACGCCCACGAGCGTTCCGGGAGAGGAGCT,

sequence b: SEQ ID No. 25: GCTCCTCTCCCGGTTCGCCGCGAGCCGCGT,
sequence c: SEQ ID No. 26: CGCGGCTCGCGGCCATAGCCGTGGGCGTCGCT;

10) sequence a: SEQ ID No. 24: GCGACGCCCACGAGCGTTCCGGGAGAGGAGCT,

sequence b: SEQ ID No. 25: GCTCCTCTCCCGGTTCGCCGCGAGCCGCGT,
sequence c: SEQ ID No. 31: CGCGGCTCGCGGCCATAGCCGTGGGCGTCGC;

11) sequence a: SEQ ID No. 18: GCGACGCCCACGAGCGTTCCGGGAGAGGAGC;

sequence b: SEQ ID No. 25: GCTCCTCTCCCGGTTCGCCGCGAGCCGCGT;
sequence c: SEQ ID No. 26: CGCGGCTCGCGGCCATAGCCGTGGGCGTCGCT;

12) sequence a: SEQ ID No. 32: CGACGCCCACGAGCGTTCCGGGAGAGGAGC;

sequence b: SEQ ID No. 19: GCTCCTCTCCCGGTTCGCCGCGAGCCGCG;
sequence c: SEQ ID No. 31: CGCGGCTCGCGGCCATAGCCGTGGGCGTCGC;

13) sequence a: SEQ ID No. 33: GCGGCGCCCACGAGCGTTCCGGGAGC;

sequence b: SEQ ID No. 34: GCTCCCGGTTCGCCGCCAGCCGCC;
sequence c: SEQ ID No. 35: GGCGGCAGGCGGCCATAGCCGTGGGCGCCGC;

14) sequence a: SEQ ID No. 36: GCGGCGCCCACGAGCGTTCCGGGAGAGGCC;

sequence b: SEQ ID No. 37: GGCCTCTCCCGGTTCGCCGCCAGCCGCC;
sequence c: SEQ ID No. 38: GGCGGCTGGCGGCCATAGCCGTGGGCGCCGC;

15) sequence a: SEQ ID No. 39: GCGGCGCCCACGAGCGTTCCGGGAGAGGCCT;

sequence b: SEQ ID No. 40: GGCCTCTCCCGGTTCGCCGCCAGCCGCCT;
sequence c: SEQ ID No. 41: GGCGGCTGGCGGCCATAGCCGTGGGCGCCGCT;

16) sequence a: SEQ ID No. 42: GCGGCGCCCACGAGCGUUCCGGGAGAGGCC;

sequence b: SEQ ID No. 43: GGCCUCUCCCGGUUCGCCGCCAGCCGCC;
sequence c: SEQ ID No. 44: GGCGGCUGGCGGCCAUAGCCGUGGGCGCCGC;

17) sequence a: SEQ ID No. 45: CGACGCCCACGAGCGTTCCGGGAGAGGAG;

sequence b: SEQ ID No. 46: CTCCTCTCCCGGTTCGCCGCCAGCCGCC;
sequence c: SEQ ID No. 35: GGCGGCAGGCGGCCATAGCCGTGGGCGCCGC;

18) sequence a: SEQ ID No. 36: GCGGCGCCCACGAGCGTTCCGGGAGAGGCC;

sequence b: SEQ ID No. 43: GGCCUCUCCCGGUUCGCCGCCAGCCGCC;
sequence c: SEQ ID No. 44: GGCGGCUGGCGGCCAUAGCCGUGGGCGCCGC;

19) sequence a: SEQ ID No. 36: GCGGCGCCCACGAGCGTTCCGGGAGAGGCC;

sequence b: SEQ ID No. 40: GGCCTCTCCCGGTTCGCCGCCAGCCGCCT;
sequence c: SEQ ID No. 38: GGCGGCTGGCGGCCATAGCCGTGGGCGCCGC;

20) sequence a: SEQ ID No. 36: GCGGCGCCCACGAGCGTTCCGGGAGAGGCC;

sequence b: SEQ ID No. 40: GGCCTCTCCCGGTTCGCCGCCAGCCGCCT;
sequence c: SEQ ID No. 41: GGCGGCTGGCGGCCATAGCCGTGGGCGCCGCT;

21) sequence a: SEQ ID No. 36: GCGGCGCCCACGAGCGTTCCGGGAGAGGCC;

sequence b: SEQ ID No. 37: GGCCTCTCCCGGTTCGCCGCCAGCCGCC;
sequence c: SEQ ID No. 44: GGCGGCUGGCGGCCAUAGCCGUGGGCGCCGC;

22) sequence a: SEQ ID No. 33: GCGGCGCCCACGAGCGTTCCGGGAGC;

sequence b: SEQ ID No. 46: CTCCTCTCCCGGTTCGCCGCCAGCCGCC;
sequence c: SEQ ID No. 35: GGCGGCAGGCGGCCATAGCCGTGGGCGCCGC;

23) sequence a: SEQ ID No. 47: GCGGCGCCCACGAGCGTTCCGGGAGAGGAG;

sequence b: SEQ ID No. 46: CTCCTCTCCCGGTTCGCCGCCAGCCGCC;
sequence c: SEQ ID No. 35: GGCGGCAGGCGGCCATAGCCGTGGGCGCCGC;

24) sequence a: SEQ ID No. 48: CGCCCACGAGCGTTCCGGGAGA;

sequence b: SEQ ID No. 49: TCTCCCGGTTCGCCGCGAG;
sequence c: SEQ ID No. 50: CTCGCGGCCATAGCCGTGGGCG;

25) sequence a: SEQ ID No. 51: UCGCCCACGAGCGUUCCGGGAGA;

sequence b: SEQ ID No. 52: UCUCCCGGUUCGCCGCGAG;
sequence c: SEQ ID No. 53: UCUCGCGGCCAUAGCCGUGGGCG;

26) sequence a: SEQ ID No. 54: GCGCCCACGAGCGTTCCGGGAGAGC;

sequence b: SEQ ID No. 55: CCCUGCTCTCCCGGTTCGCCGCCAGCCGCC;
sequence c: SEQ ID No. 56: GGCGGCAGGCGGCCATAGCCGTGGGCGC.

Further, in each of the sequences a, b, and c, at least one of the base, the ribose, and the phosphate ester includes at least one modifiable site, and any modifiable site with at least one of the following modifications: fluoro (-F), methyl, amino, disulfide, carbonyl, carboxyl, thiol (mercapto), aldehyde, and thio. preferably, a portion of the phosphate backbone of the sequences a, b, and c is phosphorothioate-modified. preferably, when the nucleic acid carrier is an RNA carrier or a DNA-RNA hybrid carrier, in the sequences of the carrier that are in RNA form, C and U are substituted with 2'-F, and A and G are modified with 2'-OMe.

In a second aspect of the present application, there is provided a nucleic acid drug, the nucleic acid drug comprising any one of the foregoing nucleic acid carriers and a bioactive substance loaded on the nucleic acid carrier, the bioactive substance comprising an oligonucleotide effector molecule and a targeting molecule for targeted delivery of the oligonucleotide effector molecule. The oligonucleotide effector molecule is selected from at least one of: a nucleic acid immunostimulant, a nucleic acid aptamer, siRNA, miRNA, and an antisense oligonucleotide (ASO). When the oligonucleotide effector molecule includes a nucleic acid aptamer, the targeting molecule includes at least a nucleic acid aptamer; when the oligonucleotide effector molecule does not include a nucleic acid aptamer, the targeting molecule includes at least a small-molecule compound having a targeting function.

Further, the nucleic acid immunostimulant includes one or more of CpG2006, variants of CpG2006, CpG1826, CpG2216, CpG2395, CpG-ODNT7, and CpG-BYZD.
preferably, the sequence of CpG2006 is:

SEQ ID No. 57: TCGTCGTTTTGTCGTTTTGTCGTT,or

SEQ ID No. 58: UCGUCGUUUUGUCGUUUUGUCGUU;

preferably, the sequence of the CpG2006 variant is:GTCGTT;

preferably, the sequence of CpG1826 is: SEQ ID No. 59: TCCATGACGTTCCTGACG;

preferably, the sequence of CpG2216 is: SEQ ID No. 60: GGGGGACGATCGTCGGGGGG;

preferably, the sequence of CpG2395 is: SEQ ID No. 61: TCGTCGTTTTCGGCGCGCGCCG;

preferably, the sequence of CpG-ODNT7 is:SEQ ID No. 62: TCGTCGTCGTCGTCGTCGTCG or SEQ ID No. 63: TCGTCGTCGTCGTCGTCGTCG;

preferably, the sequence of CpG-BYZD is: AGCGAA.

Further, the siRNA includes siRNA targeting one or more of the following molecules: ASAP1, ATAD2, CD24, CD47, EGFR, HBV, HSP, HS70, PD-L1, PAPP-1, Survivin, TAP, TIM-3, TGF-β1 and VEGF-C.

preferably, the siRNA targeting ASAP1 is selected from any of the following: i) sense strand: SEQ ID No. 64: AUUUGCUUCCAUAAUAUCAUU , antisense strand: SEQ ID No. 65: UGAUAUUAUGGAAGCAAAUUU; ii) sense strand: SEQ ID No. 66: UUAGGUUUGGGGUUGGAUCUU, antisense strand: SEQ ID No. 67: GAUCCAACCCCAAACCUAAUU.

preferably, the siRNA targeting ATAD2 includes an antisense strand of SEQ ID No. 68: GCGUCGAAGUUGUAGGAUUUU and a sense strand of SEQ ID No. 69: AAUCCUACAACUUCGACGCUU.

preferably, the siRNA targeting CD24 includes a sense strand of SEQ ID No. 70: UGUUUACAUUGUUGAGGUAUU and an antisense strand of SEQ ID No. 71: UACCUCAACAAUGUAAACUUU.

preferably, the siRNA targeting CD47 is selected from any of the following: i) sense strand: SEQ ID No. 72: GGUGAUUACCCAGAGAUAUTT; antisense strand: SEQ ID No. 73: AUAUCUCUGGGUAAUCACCTT; ii) sense strand: SEQ ID No. 74: UGGUGAAAGAGGUCAUUCCUU; antisense strand: SEQ ID No. 75: GGAAUGACCUCUUUCACCAUU; iii) sense strand: SEQ ID No. 76: GGAAUGACCUCUUUCACCATT; antisense strand: SEQ ID No. 77: UGGUGAAAGAGGUCAUUCCTT; iv) sense strand: SEQ ID No. 78: GGUGAUUACCCAGAGAUAUUU; antisense strand: SEQ ID No. 79: AUAUCUCUGGGUAAUCACCUU.

preferably, the siRNA targeting EGFR is selected from any of the following: i) sense strand: SEQ ID No. 80: GGCUGGUUAUGUCCUCAUUUU; antisense strand: SEQ ID No. 81: AAUGAGGACAUAACCAGCCUU; ii) sense strand: SEQ ID No. 82: CCUUAGCAGUCUUAUCUAAUU; antisense strand: SEQ ID No. 83: UUAGAUAAGACUGCUAAGGUU; iii) sense strand: SEQ ID No. 84: UGCCUUAGCAGUCUUAUCUAAUU; antisense strand: SEQ ID No. 85: UUAGAUAAGACUGCUAAGGCAUU; iv) sense strand: SEQ ID No. 86: UGCCUUAGCAGUCUUAUCUAAUUUU; antisense strand: SEQ ID No. 87: AAUUAGAUAAGACUGCUAAGGCAUU.

preferably, the siRNA targeting HBV includes a sense strand having the sequence of SEQ ID No. 88: GGACUUCUCUCAAUUUUCUUU, and an antisense strand having the sequence of SEQ ID No. 89: AGAAAAUUGAGAGAAGUCCUU.

preferably, the siRNA targeting HSP includes a sense strand having the sequence of SEQ ID No. 90: CGCAGAACACCGUGUUCGAUU, and an antisense strand having the sequence of SEQ ID No. 91: UCGAACACGGUGUUCUGCGUU.

preferably, the siRNA targeting HS70 includes a sense strand having the sequence of SEQ ID No. 92: GGCCAACAAGAUCACCAUCUU, and an antisense strand having the sequence of SEQ ID No. 93: GAUGGUGAUCUUGUUGGCCUU.

preferably, the siRNA targeting PD-L1 is selected from any of the following: i) sense strand: SEQ ID No. 94: CCAGCACACUGAGAAUCAAUU, antisense strand: SEQ ID No. 95: UUGAUUCUCAGUGUGCUGGUU; ii sense strand: SEQ ID No. 96: AGACGUAAGCAGUGUUGAATT, antisense strand: SEQ ID No. 97: UUCAACACUGCUUACGUCUTT.

preferably, the siRNA targeting PAPP-1 includes a sense strand of SEQ ID No. 98: GAGGAAGGUAUCAACAAAUTT and an antisense strand of SEQ ID No. 99: AUUUGUUGAUACCUUCCUCTT.

preferably, the siRNA targeting Survivin is selected from any of the following: i) sense strand: SEQ ID No. 100: GCAGGUUCCUUAUCUGUCACAUU; antisense strand: SEQ ID No. 101: UGUGACAGAUAAGGAACCUGCUU; ii) sense strand: SEQ ID No. 102: GGAAUUGGAAGGCUGGGAACCUU; antisense strand: SEQ ID No. 103: GGUUCCCAGCCUUCCAAUUCCUU; iii) sense strand: SEQ ID No. 104: UGCAGGUUCCUUAUCUGUCATT; antisense strand: SEQ ID No. 105: UGACAGAUAAGGAACCUGCTT; iv) sense strand: SEQ ID No. 106: CUGCAGGUUCCUUAUCUGUCACAUU; antisense strand: SEQ ID No. 107: UGUGACAGAUAAGGAACCUGCAGUU.

preferably, the siRNA targeting TAP is selected from any of the following: i) sense strand: SEQ ID No. 108: GCUGCACACGGUUCAGAAUUU; antisense strand: SEQ ID No. 109: AUUCUGAACCGUGUGCAGCUU; ii) sense strand: SEQ ID No. 110: CAGGAUGAGUUACUUGAAAUU; antisense strand: SEQ ID No. 111: UUUCAAGUAACUCAUCCUGUU.

preferably, the siRNA targeting TIM-3 includes a sense strand having the sequence of SEQ ID No. 112: GUGCUCAGGACUGAUGAAATT, and an antisense strand having the sequence of SEQ ID No. 113: UUUCAUCAGUCCUGAGCACTT.

preferably, the siRNA targeting TGF-β1 is selected from any of the following: i) sense strand: SEQ ID No. 114: GUCAACUGUGGAGCAACACUU; antisense strand: SEQ ID No. 115: GUGUUGCUCCACAGUUGACUU; ii) sense strand: SEQ ID No. 116: GCAACAACGCCAUCUAUGATT; antisense strand: SEQ ID No. 117: UCAUAGAUGGCGUUGUUGCTT.

preferably, the siRNA targeting VEGF-C is selected from any of the following: i) sense strand: SEQ ID No. 118: GCAAGACGUUGUUUGAAAUUAUU; antisense strand: SEQ ID No. 119: UAAUUUCAAACAACGUCUUGCUU; ii) sense strand: SEQ ID No. 120: CAGCAAGACGUUGUUUGAAAUUAUU; antisense strand: SEQ ID No. 121:

UAAUUUCAAACAACGUCUUGCUGUU; iii) sense strand: SEQ ID No. 122: CAGGAUGGUAAAGACUACAUU; antisense strand: SEQ ID No. 123: UGUAGUCUUUACCAUCCUGUU.

preferably, the antisense oligonucleotide (ASO) includes one or more selected from A-miR21, A-miR-10a, A-miR-30c, and AmiR1306, and the miRNA includes one or more selected from miR-34, miR-542, miR-126, and miR-122.

preferably, A-miR21 is selected from any of the following in DNA or RNA form:

    i) GATAAGCT;

    ii) SEQ ID No. 124: GTCAACATCAGTCTGATAAGCTA;

    iii) SEQ ID No. 125: TCAACATCAGTCTGATAAGCTA;

    iv) the sequences of item (i) or item (ii) with T substituted by U;

preferably, A-miR-10a is ACAGGGTA;

preferably, A-miR-30c is SEQ ID No. 126: GCTGAGAGTGTAGGATGTTTACA;

preferably, the sequence of miR-34 is TGTGACAG;

preferably, the sequence of miR-542 is TGGCAGTGT;

preferably, the sequence of miR-126 is selected from UCGUACC, UCGUACCG, CGTACCG, or GTCGTT;

preferably, the sequence of miR-122 is GGAAGTGT;

preferably, the sequence of AmiR1306 is SEQ ID No. 127: CATCACCACCAGAGCCAACGTC.

preferably, the nucleic acid aptamer is in DNA form or RNA form; preferably, the DNA-form nucleic acid aptamer includes at least one aptamer selected from the group consisting of:A1, A15, AS 1411, AFP, ATP, Act-12c, A18, BAF7-1, C-MetSL-1, CH6, CA2, CRAC Orail target, CEA, CEA-18, CEA-T84, CSC1, CSC13, CD40, CD16a, CD19, CD3-4, CD44, CD12, CD20, CD24, CD24A-2, CD33, CD38, CD105, CD117, CD63, CD123, EGFR, EpCAM, EcR, FAP, GPC1, GPC3, GSK836, HBsAg, Her2, Her3, HMGA2, H2, IFN-γ, IL-4Ra, IL-17, LZH8, MUC1, M5, M7, M1, N5, N-G-Dua, NKG2D_#-20-N-15, NSE, SARS-CoV-2-N15, SARS-CoV-2-N48, SARS-CoV-2-N58, SARS-CoV-2-N61, OX40, PSMA, PDGFRβ, PDGF, PD-L1, PD1, PTK-7, ProGRP-48, SF, TBA15, TBA29, TIMC-d, TRRA4, TFRA3, TTA1, TLS9a, TGF-βII, TNF-α, TNF, T1, Vap7, VEGF121, VEGF-V7t1, VCAM-1, VCAM-12d, AX02, AX104, PL-45, EP166, AGC, Karpas299, SW620, MDA-MB-231, MCF-7 and PC-3; and the RNA-form nucleic acid aptamer includes at least one aptamer selected from the group consisting of: A15, AS1411, BCMA, CTLA-4, CCL1, CEA, CD4-3, CD28, CD44, IL-4RA, EGFR, EpCAM, FGF2, FGF5, Her2, Her3, LAG-3, MUC1, MRP1, OX40, PSMA, PDGFRβ, TFRA4, TFRA3, TTA1, TIM3, TIMC-11, VEGF, VEGF165 and 4-1BB.

preferably, the nucleic acid aptamer is selected from at least one aptamer having any of the following sequences:

    A1 aptamer: SEQ ID No. 128: GGTTGCATGCCGTGGGGAGGGGGGTGGGTTTTATAGCGTACTCAG;

    A15 aptamer: SEQ ID No. 129: CCCTCCTACATAGGG; or SEQ ID No. 130: CCCUCCUACAUAGGG;

    AS1411 aptamer:

      1)SEQ ID No. 131: GGTGGTGGTGGTTGTGGTGGTGGTGG; or

      2)SEQ ID No. 132: GGUGGUGGUGGUUGUGGUGGUGGUGG; or

      3)SEQ ID No. 133: AUUCUGAACCGUGUGCAGCACCACGCUGCACACGGUUCAGAAUACACA;

AFP aptamer: SEQ ID No. 134:

GGCAGGAAGACAAACAAGCTTGGCGGCGGGAAGGTGTTTAAATTCCCGGGTCTGCGTG
GTCTGTGGTGCTGT;

ATP aptamer: SEQ ID No. 135: ACCTGGGGAGTATTGGGGAGGAAGG; or SEQ ID No. 136: GGGAGGACGATGCGGAGGAAGGGTAGG;

Act-12c aptamer: SEQ ID No. 137:
CGGGGAAAGTCACGGGGGGTTTCAGATGTTCTGATCGGTGTGGAG;

A18 aptamer: SEQ ID No. 138: CCAGATAGTCCCTGG;

BAF aptamer: SEQ ID No. 139: GATAACGGGCACGAATTCGGAGTG;

BCMA aptamer: SEQ ID No. 140:
AGUGCAAGACGUUCGCAGAUUAGCGAAAAGAGGGUCUCAUUGACUAGUAC;

CTLA-4 aptamer: 1) SEQ ID No. 141: GGUGGAAGAGGGAUGGGCCGACGUGCCGCAU; or 2) SEQ ID No. 142:

TCCCTACGGCGCTAACGATGGTGAAAATGGGCCTAGGGTGGACGGTGCCACCGTGCTAC
AAC;

C-Met-SL1 aptamer:
SEQ ID No. 143: ATCAGGCTGGATGGTAGCTCGGTCGGGGTGGGTGGGTTGGCAAGTCTGAT;

CH6 aptamer:
SEQ ID No. 144: AGTCTGTTGGACCGAATCCCGTGGACGCACCCTTTGGACG;

CA2 aptamer: SEQ ID No. 145: CCCACGTCTGCGCTTAGCTCCTGGGCCTGGATGGGC;

CCL1 aptamer:
SEQ ID No. 146: UGACUCCUCUGACAGCCUAAUUUCUCCCGAUUACCCUG;

CRAC Orail target aptamer:
SEQ ID No. 147: CCAGTAGCCATACCGGTTTGTGGATGGGGTGTATGCGAGT;

CEA aptamer:

1) SEQ ID No. 148: CTAGGATCCCCACTCACCATCTCTCAGCTTGCTTCCTAGC; or
2) SEQ ID No. 149: CUAGGAUCCCCACUCACCAUCUCUCAGCUUGCUUCCUAGC; or
3) SEQ ID No. 150: TTAACTTATTCGACCATA; or
4) SEQ ID No. 151:

TCGCGCGAGTCGTCTGGGGAACCATCGAGTTACACCGACCTTCTATGTGCGGCCCCCCGC
ATCGTCCTCCC;

CSC1 aptamer:
SEQ ID No. 152:

ACCTTGGCTGTCGTGTTGTAGGTGGTTTGCTGCGGTGGGCTCAAGAAGAAAGCGCAAAG
AGGTCAGTGGTCAGAGCGT;

CSC13 aptamer:
SEQ ID No. 153:

ACCTTGGCTGTCGTGTTGTGGGGTGTCGTATCTTTCGTGTCTTATTATTTTCTAGGTGGAGGTCAGTGGTCAGAGCGT;

CD40 aptamer:

1)SEQ ID No. 154: CCAACGAGTAGGCGATAGCGCGTGG;
2)SEQ ID No. 155: AGAGACGATGCGGCCAACGAGTAGGCGATAGCGCGTGGCAGAGCGTCGCT;
3)SEQ ID No. 156: GCCAACGAGTAGGCGATAGCGCGTGGC;

CD16a aptamer:
SEQ ID No. 157: CCACTGCGGGGGTCTATACGTGAGGAAGAAGTGG;

CD19 aptamer:

1)SEQ ID No. 158: TGCGTGTGTAGTGTGTGTCGTTTCTCCTTTTTTTGGTTGCTGCTCTTAGGGATTTGGGCGG; or

2)SEQ ID No. 159: TGCGTGTGTAGTGTGTCTGTTCTCCTTTTTTTGGTTGCTGCTCTTAGGGATTTGGGCGG,

CD4-3 aptamer:
SEQ ID No. 161: GGGAGGACGAUGCGGUUUGGGGUUUUCCCGUGCCCCAGACGACUCGCCCGA;

CD3-4 aptamer:
SEQ ID No. 162:

TCTCGGACGCGTGTGGTCGGCCGAGTGGCCCACGGTAGAAGGGTTAGAACTGCTGGTTGGTGAATCTCGCTGCCTGGCCCTAGAGTG;

CD44 aptamer:
SEQ ID No. 163: CCAAGGCCTGCAAGGGAACCAAGGACACAG;

CD12 aptamer:
SEQ ID No. 164: GTGGATTGTTGTGTTCTGTTGGTTTTTGTGTTGTC;

CD20 aptamer:
SEQ ID No. 165: TGCGTGTGTAGTGTGTCTGTTTTTTATCTTCTTTTATCTACTCTTAGGGATTTGGGCGG;

CD24 aptamer:
SEQ ID No. 166: TATGTGGGTGGGTGGGCGGTTATGCTGAGTCAGCCTTGCT;

CD24A-2 aptamer:
SEQ ID No. 167:

ATCCAGAGTGACGCAGCATATGTGGGTGGGTGGGCGGTTATGCTGAGTCAGCCTTGCTTGGACACGGTGGCTTAGT;

CD28 aptamer:
SEQ ID No. 168:

GGGAGAGAGGAAGAGGGAUGGGGAUUAGACCAUAGGCUCCCAACCCCCCCCGGGAGAGAGGAAGAGGGAUGGGGAUUAGACCAUAGGCUCCCAACCCCCGGG;

CD33 aptamer:
SEQ ID No. 169:
TACCAGTGCGATGCTCAGCACGCTTATAGGGGCTGGACAAAATTCTACCCAGCCTTT;

CD38 aptamer: SEQ ID No. 170: TACGTGAATCTCGTACGATACTCTGTAAGCGT;

CD44 aptamer:
SEQ ID No. 171:

GGGAUGGAUCCAAGCUUACUGGCAUCUGGAUUUGCGCGUGCCAGAAUAAAGAGUAU
AACGUGUGAAUGGGAAGCUUCGAUAGGAAUUCGG;

CD105 aptamer:
SEQ ID No. 172: GATCAGTTTTCCATGCCAGTTGGTATTCCGCGACAGTTTGATCTC;

CD117 aptamer:: SEQ ID No. 173:
GGGGCCGGGGCAAGGGGGGGGTACCGTGGTAGGAC;

CD63 aptamer: SEQ ID No. 174: CACCCCACCTCGCTCCCGTGACACTAATGCTA;

CD123 aptamer:
SEQ ID No. 175:

TGCGTGTGTAGTGTGTCTGGGCTACATCGATGAGCTGCCTAGGGTCCCTCTTAGGGATTT
GGGCGG;

EGFR aptamer:

1)SEQ ID No. 176: GCCTTAGTAACGTGCTTTGATGTCGATTCGACAGGAGGC; or
2)SEQ ID No. 177: GCCUUAGUAACGUGCUUUGAUGUCGAUUCGACAGGAGGC; or
3)SEQ ID No. 178: UGCCGCUAUAAUGCACGGAUUUAAUCGCCGUAGAAAAGCAUGUCAAAGCCGU
U;

EpCAM aptamer:

1)SEQ ID No. 179: GCGACUGGUUACCCGGUCG; or
2)SEQ ID No. 180:
CACTACAGAGGTTGCGTCTGTCCCACGTTGTCATGGGGGGTTGGCCTG; or
3)SEQ ID No. 181: GACAAACGGGGGAAGATTTGACGTCGACGAC;

EcR aptamer:
SEQ ID No. 182:
GCAGGTCCACTGCGGGGGTCTATACGTGAGGAAGAAGTGGGCAGGTC;

FGF2 aptamer:

1)SEQ ID No. 183: GGGAUACUAGGGCAUUAAUGUUACCAGUGUAGUCCC;
2)SEQ ID No. 184: GGGAAACUAGGGCGUUAACGUGACCAGUGUUUCUCGA;
3)SEQ ID No. 185: GGGAAACUAGGGCGUUAACGUGACCAGUGUUUCCC;

FGF5 aptamer:
SEQ ID No. 186:
GGGCGACCUCUCCGUACUGACCUACAGAGCGACAUACUAGUGUAUCCAGAUCGCCC;

FAP aptamer: 1)SEQ ID No. 187: TGGGGGTTGAGGCTAAGCCGA; or

2)SEQ ID No. 188: CCGCTCGAGCTAGTCTGACAAAGAGAAACAC;

GPC1 aptamer: SEQ ID No. 189: AACGGAGTGTGGCTAACTCGA;

GPC3 aptamer: SEQ ID No. 190: TAACGCTGACCTTAGCTGCATGGCTTTACATGTTCCA;

GSK836 aptamer: SEQ ID No. 191: GCAGAGGTGAAGCGAAGTCG;

HBsAg aptamer:
SEQ ID No. 192: CACAGCGAACAGCGGCGGACATAATAGTGCTTACTACGAC;

Her2 aptamer:

　　1)SEQ ID No. 193: AGCCGCGAGGGGAGGGATAGGGTAGGGCGCGGCT; or
　　2)SEQ ID No. 194: AGCCGCGAGGGGAGGGAUAGGGUAGGGCGCGGCU;

Her3 aptamer:

　　1)SEQ ID No. 195:

　　GGGAGCTCAGAATAAACGCTCAAAGGGTCAAGCTGATTACACTTTGTCCACTATTGGGTC
　　CTTCGACATGAGGCCCGGATC;

　　or

　　2)SEQ ID No. 196:

　　GGGAGCTCAGAATAAACGCTCAAGGCTAACAGCACGCAACGGGGGGGAGTAATCGTGT
　　CTGTTCGACATGAGGCCCGGATC;

　　or

　　3)SEQ ID No. 197:
　　CAGCGAAAGUUGCGUAUGGGUCACAUCGCAGGCACAUGUCAUCUGGGCG; or

　　4)SEQ ID No. 198:

　　GAAUUCCGCGUGUGCCAGCGAAAGUUGCGUAUGGGCCACAUCGCAGGCACAUGUCAU
　　CUGGGCGGUCCGUUCGGGAUCC;

HMGA2 aptamer: SEQ ID No. 199: GGAAAAAATTTTTTAAAAAACCC;

H2 aptamer:
SEQ ID No. 200:
GGGCCGTCGAACACGAGCATGGTGCGTGGACCTAGGATGACCTGAGTACTGTCC;

IFN-$\gamma$ aptamer:
SEQ ID No. 201:
CCGCCCAAATCCCTAAGAGAAGACTGTAATGACATCAAACCAGACACACACTACACA CGCA;

IL-4R$\alpha$ (i.e., CD124) aptamer:

　　1)SEQ ID No. 202:

　　GGAGGACGAUGCGGAAAAAGCAACAGGGUGCUCCAUGCGCAUGGAACCUGCGCGCAG
　　ACGACUCGCUGAGGAUCCGAGA;

　　or

2)SEQ ID No. 203: AAAAAGCAACAGGGUGCUCCAUGCGCAUGGAACCUGCGCG;

IL-17 aptamer:

1)SEQ ID No. 204: CTTGGATCACCATAGTCGCTAGTCGAGGCT; or
2)SEQ ID No. 205: GCGGCATCCTATCACGCATTGACC

LAG-3 aptamer:
SEQ ID No. 206:

GGGAGAGAGAUAUAAGGGCCUCCUGAUACCCGCUGCUAUCUGGACCGAUCCCAUUAC CAAAUUCUCUCCC;

LZH8 aptamer:
SEQ ID No. 207:
ATCCAGAGTGACGCAGCATATTAGTACGGCTTAACCCCATGGTGGACACGGTGGCTTAGT;

MUC1 aptamer:

1)SEQ ID No. 208: GCAGTTGATCCTTTGGATACCCTGG; or
2)SEQ ID No. 209: GAAGTGAAAATGACAGAACACAACA; or
3)SEQ ID No. 210:

AACCGCCCAAATCTCTAAGAGTCGGACTGCAACCTATGCTATCGTTGATGTCTGTCCAAG CAACACAGACACACTACACACACGCACA; or

4)SEQ ID No. 211: AATGACAGAACACAACATT; or 5)SEQ ID No. 212: GCAGUUGAUCCUUUGGAUACCCUGG;

M5 aptamer:
SEQ ID No. 213:

AGCAGCACAGAGGTCAGATGCTTGGTTCCACCGTACTGACTGTAGTAAAATCTGATCACT CCTATGCGTGCTACCGTGAA;

M7 aptamer:
SEQ ID No. 214:

AGCAGCACAGAGGTCAGATGTAGTCGGTCTTCTTGTTTGAAACTGCTAATTTTGAAAAAA CCTATGCGTGCTACCGTGAA;

M1 aptamer:
SEQ ID No. 215:

AGCAGCACAGAGGTCAGATGATATAACCTTAATAAATAAAATATAAATTATTTAATCTTACC TATGCGTGCTACCGTGAA;

MRP1 aptamer:
SEQ ID No. 216:
GGGAGAAUAGUCAACAAAUCGUUUGGGGCGACUUCUCCUUCCUUUCUCCC;

N5 aptamer:
SEQ ID No. 217: GATTGAGTAGATAGTGGTTCTGTACGTAGTGAAAGAGTGG;

N-G-Dua aptamer:

SEQ ID No. 218:

CAAGTTGCTCGTCGCGATACGTTTGGTTGGTGTGGTTGGCAGTATCGCAGGTCCAAGTTG
CTCGTCGCGATACAACGGAGTGTGGCTAACTCGA;

NKG2D_#-20-N-15 aptamer:
SEQ ID No. 219: CAAGTTGCTCGTCGCGATACGTTTGGTTGGTGTGGTTGGCAGTATC;

NSE aptamer:
SEQ ID No. 220: TCACACGGACCTCTCTCTACATTAATTGCGCATTTCGTT;

SARS-CoV-2-N15 aptamer:
SEQ ID No. 221:
GCTGGATGTTCATGCTGGCAAAATTCCTTAGGGGCACCGTTACTTTGACACATCCAGC;

SARS-CoV-2-N48 aptamer:
SEQ ID No. 222:
GCTGGATGTCGCTTACGACAATATTCCTTAGGGGCACCGCTACATTGACACATCCAGC;

SARS-CoV-2-N58 aptamer:
SEQ ID No. 223:
GCTGGATGTCACCGGATTGTCGGACATCGGATTGTCTGAGTCATATGACACATCCAGC;

SARS-CoV-2-N61 aptamer:
SEQ ID No. 224:
GCTGGATGTTGACCTTTACAGATCGGATTCTGTGGGGCGTTAAACTGACACATCCAGC;

OX40 aptamer:

1)SEQ ID No. 225:

GGGAGGACGATGCGGCAGTCTGCATCGTAGGAATCGCCACCGTATACTTTCCCACCAGAC
GACTCGCTGAGGATCCGAGA;

2)SEQ ID No. 226:

GGGAGGACGAUGCGGCAGUCUGCAUCGUAGGAAUCGCCACCGUAUACUUUCCCACCA
GACGACUCGCUGAGGAUCCGAGA;

3)SEQ ID No. 227: CAGTCTGCATCGTAGGATTAGCCACCGUATCTTTCCCAC;

4)SEQ ID No. 228:

GGGAGGACGAUGCGGCAGUCUGCAUCGUAGGAAUCGCCACCGUAUACUUUCCCACCA
GACGACUCGCUG;

5)SEQ ID No. 229: CAGUCUGCAUCGUAGGAAUCGCCACCGUAUACUUUCCCAC;

6)SEQ ID No. 230:
GGGAUGCGGAAAAAAGAACACUUCCGAUUAGGGCCCACCCUAACGGCCGCAGAC;

PSMA aptamer:

1)SEQ ID No. 231: GGGAGGACGATGCGGATCAGCCATGTTTACGTCACTCCT; or

2)SEQ ID No. 232:
GCGTTTTCGCTTTTGCGTTTTGGGTCATCTGCTTACGATAGCAATGCT; or

3)SEQ ID No. 233: GGGAGGACGAUGCGGAUCAGCCAUGUUUACGUCACUCCU; or

4)SEQ ID No. 234:
GGGACCGAAAAAGACCUGACUUCUAUACUAAGUCUACGUUCCC;

PDGFRβ aptamer:

1)SEQ ID No. 235: TGTCGTGGGGCATCGAGTAAATGCAATTCGACA; or

2)SEQ ID No. 236: UGUCGUGGGGCAUCGAGUAAAUGCAAUUCGACA;

PDGF aptamer:
SEQ ID No. 237: CAGGCTACGGCACGTAGAGCATCACCATGATCCTG;

The PD-L1 aptamer is selected from any one or more of the following sequences:

1)SEQ ID No. 238: AACAACGTATAACAATGCCCACGTCACCAGAGTACTATGG;

2)SEQ ID No. 239: GCCCCAGTTATGCTTTCCCCCTCTGTCTCTTTG;

3)SEQ ID No. 240: ATCGCCCGCAGCACCCATTTGTTTTTTTTTG;

4)SEQ ID No. 241: ACGGGCCACATCAACTCATTGATAGACAATGCGTCCACTGCCCGT;

5)SEQ ID No. 242: TGCCCGCACATCAACTCATTGATAGACAATGCGTCCACTCGGGCA;

6)SEQ ID No. 243: TGCCCGCACATCAACTCATTGATAGACAATGCGTCCACTACGGGC;

7)SEQ ID No. 244: CGGGCACACATCAACTCATTGATAGACAATGCGTCCACTGCCCGT;

8)SEQ ID No. 245: GTTGGTCACATCAACTCATTGATAGACAATGCGTCCACTACCAAC;

9)SEQ ID No. 246: GGGCCACATCAACTCATTGATAGACAATGCGTCCACTGCCC;

10)SEQ ID No. 247:
TGGTTGCACATCAACTCATTGATAGACAATGCGTCCACTCAACCA;

11)SEQ ID No. 248: TACAGGTTCTGGGGGGTGGGTGGGGAACCTGTT;

12)SEQ ID No. 249: CTACGAGACGAACTTATGCGTAATAATGACTGTCGTAG;

The PD-1 aptamer is selected from any one or more of the following sequences:

1)SEQ ID No. 250:

GACGATAGCGGTGACGGCACAGACGGTACAGTTCCCGTCCCTGCACTACACGTATGCCG
CTTCCGTCCGTCGCTC;

2)SEQ ID No. 251:

GAGCGACGGACGGAAGCGGCATACGTGTAGTGCAGGGACGGGAACTGTACCGTCTGTG
CCGTCACCGCTATCGTC;

3)SEQ ID No. 252:

GGATCCTATGACGCATTGACCCGCTGCCTCTACTGAGGCTGTGTCAGTGTGCGGCTCGGA
CTGTTGAATTC;

4)SEQ ID No. 253:
AGCGGTGACGGCACAGACGGTACAGTTCCCGTCCCTGCACTACACGTATGCCGCT;

5)SEQ ID No. 254: ACCGACAGTGAAGGACTCAGCGAACTCTCAGACTCGGTTC;

PTK-7 aptamer:
SEQ ID No. 255: ATCTAACTGCTGCGCCGCCGGGAAAATACTGTACGGTTAGA;

ProGRP-48 aptamer:
SEQ ID No. 256:
CATGCGGAGTAGAGCGAGCCCAGATAGTCCCTGGTTATTTCCTTAGG;

SF aptamer:
SEQ ID No. 257: GATCTCTCTCTGCCCTAAGTCCGCACCCGTGCTTCCCTGT;

TBA15 aptamer: SEQ ID No. 258: GGTTGGTGTGGTTGG;

TBA29 aptamer: SEQ ID No. 259: AGTCCGTGGTAGGGCAGGTTGGGGTGACT;

TFRA4 aptamer: SEQ ID No. 260: GCGTGGTACCACG;or SEQ ID No. 261:
GCGUGGUACCACGC;

TFRA3 aptamer:

1)SEQ ID No. 262: GCGTGGTCACACGC; or

2)SEQ ID No. 263: GCGUGGUCACACGC; or

3)SEQ ID No. 264:

GCGGCGCCCACGAGCGTTCGCGTGGTCACACGCGTTCCGCCCTCCTACATAGGGCGCAT
AGCCGTGGGCGCCGC;

TTA1 aptamer:

SEQ ID No. 265: CCTGCACTTGGCTTGGATTTCAGAAGGGAGACCC; or
SEQ ID No. 267: CCUGCACUUGGCTTGGAUUUCAGAAGGGAGACCC;

TLS9a aptamer:
SEQ ID No. 268: AGTCCATTTTATTCCTGAATATTTGTTAACCTCATGGAC;

TGF-β aptamer:
SEQ ID No. 269:
ACATTGCTGCGTGATCGCCTCACATGGGTTTGTCTGGTCGATTTGGAGGTGGTGGGTGGC;

TIM3 aptamer:

SEQ ID No. 270:

GGGAGAGGACCAGUA-GCCACUAUGGUGUUGGAGCUAGCGG-CAGAGCGUCGCGGUCC
CUCCC;

or

SEQ ID No. 271:

GGGAGAGGACCAGUA-CUGGUAGUUCUCUGUGCGACUCCUA-CAGAGCGUCGCGGUCC CUCCC;

TIMC-d aptamer:
SEQ ID No. 272: AAGCAACACUUAGUCGCGAUUGAUACGUGCGCAGUCAU;

TIMC-11 aptamer:
SEQ ID No. 273:

GGAGGACGAUGCGGGGAAGCAACACUUAGUCGCGAUUGAUACGUGCGCAGUCAUCA GACGACUCGCUGAGGAUCCGAGA;

TNF-α aptamer: SEQ ID No. 274: GCGGCCGATAAGGTCTTTCCAAGCGAACGAAAA;

TNF aptamer:
SEQ ID No. 275: GCGCCACTACAGGGGAGCTGCCATTCGAATAGGTGGGCCGC;

T1 aptamer:
SEQ ID No. 276:

CGCTCGATAGATCGAGCTTCGCTCGATGTGGTGTTGTGGGGGCTTGTATTGGTCGATCAC GCTCTAGAGCACTG;

Vap7 aptamer: SEQ ID No. 277: TGGTGGGGGTGGACGGGCCGGGTAGA;

VEGF aptamer:
SEQ ID No. 278: AUGCAGUUUGAGAAGUCGCGCAU; or SEQ ID No. 279: GGTGGGGGTGGACGGGCCGGGTAGA;

VEGF165 aptamer: SEQ ID No. 280: CGGAAUCAGUGAAUGCUUAUACAUCCG;

VEGF121 aptamer: SEQ ID No. 281: TGTGGGGGTGGACTGGGTGGGTACC;

VEGF-V7t1 aptamer: SEQ ID No. 282: TGTGGGGGTGGACGGGCCGGGTAGA;

VCAM-1 aptamer:

SEQ ID No. 283:

ATACCAGCTTATTCAATTGGACACGGCAAAGGGGTATAGCCTACCGGACCGTGAACATGG AATGGTGTGCTGCGTGGAGATAGTAAGTGCAATCT;

or

SEQ ID No. 284: GGACACGGCAAAGGGGTATAGCCTACCGGACCGTGAACATGGAATGGTGTGCTG CGTGG;

VCAM-12d aptamer:
SEQ ID No. 285: AGGGAATCTTGCCTAGGGAGGGAGTAGCGAAAGGGCTCA;

4-1BB aptamer:
SEQ ID No. 286:

GGGAGAGAGGAAGAGGGAUGGGCGACCGAACGUGCCCUUCAAAGCCGUUCACUAACC
AGUGGCAUAACCCAGAGGUCGAUAGUACUGGAUCCCCCC;

PL-45 aptamer:
SEQ ID No. 287:
ACTCATAGGGTTAGGGGCTGCTGGCCAGATACTCAGATGGTAGGGTTACTATGAGC;

EP166 aptamer:
SEQ ID No. 288: AACAGAGGGACAAACGGGGGAAGATTTGACGTCGACGACA;

AGC aptamer:
SEQ ID No. 289:
CGACCCGGCACAAACCCAGAACCATATACACGATCATTAGTCTCCTGGGCCG;

Karpas299 aptamer:
SEQ ID No. 290:
ATCCAGAGTGACGCAGCACCACCACCGTACAATTTTTTCATTACCTACTCGGC;

SW620 aptamer:
SEQ ID No. 291: CCCATCAATGTTACGACCCGCTAGGGCTGCTGTGCCATCGGGTAA;

MDA-MB-231 aptamer:
SEQ ID No. 292:

AGAATTCAGTCGGACAGCGAAGTAGTTTTCCTTCTAACCTAAGAACCCGCGGCAGTTTAA
TGTAGATGGACGAA;

MCF-7 aptamer:
SEQ ID No. 293: GCATGGGGTTTCGGCGTTTCGTCTATCTTGTTTCTGTTAGCGTCT;

PC-3 aptamer: SEQ ID No. 294:
TGCCACTACAGCTGGTTCGGTTTGGTGACTTCGTTCTTCGTTGTGGTGCTTAGTGGC.

Further, the targeting small-molecule compound is selected from one or more of folic acid, biotin, vitamin B12, and mannose, and is disposed at the 5' end or the 3' end of at least one of the sequences a, b, and c.

Further, the respective different oligonucleotide effector molecules are, independently, conjugated/loaded onto the nucleic acid carrier by any one or more of the following: (1) a single-stranded connecting bridge sequence; (2) a complementary sequence of the single-stranded connecting bridge sequence; and (3) a transition sequence, wherein the single-stranded connecting bridge sequence and the transition sequence are the single-stranded connecting bridge sequence and the transition sequence of the nucleic acid carrier above.

Further, one or more nucleotides of at least one oligonucleotide effector molecule with a modification selected from at least one of: fluoro (F) substitution, methyl, amino, disulfide, carbonyl, carboxyl, thiol (mercapto), aldehyde, thio (sulfur substitution), inverted dT, and locked nucleic acid (LNA).

Further, the siRNA has a phosphorothioate modification at the phosphate backbone of the 5'-end internucleotide linkage of each of the antisense and sense strands, and has a 3' end bearing a dTdT modification or a UU overhang; alternatively, the 3' end of the antisense strand of the siRNA is linked to AA, UU, or any combination of two nucleotides.

Further, the miRNA with a phosphorothioate modification or a locked nucleic acid (LNA) modification.

Further, the nucleic acid aptamer with any one or more of the following modifications: phosphorothioate (PS), 2'-fluoro (2'-F), or 2'-O-methoxyethyl (2'-O-MOE).

Further, the nucleic acid immunostimulant is phosphorothioate-modified.

Further, the nucleic acid drug is selected from any one of the following ninety-nine (99) drugs.

Drug 1): 3*CpG2006-DNA carrier, wherein the DNA carrier corresponds to group 13) among the foregoing twenty-six (26) sets of backbone sequences of the nucleic acid carrier, and three CpG2006 units are respectively linked via transition sequences to the 5' ends of the sequences a, b, and c;

preferably, the sequence of CpG2006 is SEQ ID No. 57: TCGTCGTTTTGTCGTTTTGTCGTT, and the internucleotide phosphate linkages between adjacent nucleotides are phosphorothioate-modified;
preferably, the transition sequence is TTTTT.

Drug 2): 2*CpG1826-2*mannose-DNA carrier, wherein the DNA carrier corresponds to group 1) among the foregoing twenty-six (26) sets of backbone sequences of the nucleic acid carrier; two CpG1826 moieties are respectively linked via a transition sequence to the 5' ends of the sequence a and the sequence c, and two mannose moieties are disposed at the 5' end and the 3' end of the sequence b;

preferably, the transition sequence is TTTTT;

preferably, the sequence of CpG1826 is SEQ ID No. 59: TCCATGACGTTCCTGACG, and the internucleotide phosphate linkages between adjacent nucleotides are phosphorothioate-modified.

Drug 3): 2*CpG1826-CD40 aptamer-DNA carrier, wherein the DNA carrier corresponds to group 1) among the foregoing twenty-six (26) sets of backbone sequences of the nucleic acid carrier; two CpG1826 moieties are respectively linked via a transition sequence to the 5' ends of the sequence a and the sequence c, and the CD40 aptamer is linked via a transition sequence to the 5' end of the sequence b;

preferably, the sequence of CpG1826 is SEQ ID No. 59: TCCATGACGTTCCTGACG, with the internucleotide phosphate linkages between adjacent nucleotides phosphorothioate-modified;

preferably, the transition sequence is TTTTT;

preferably, the sequence of the CD40 aptamer is SEQ ID No. 154: CCAACGAGTAGGCGATAGCGCGTGG.

Drug 4): 2*CpG2006-CD40 aptamer-DNA carrier, wherein the DNA carrier corresponds to group 1) among the foregoing twenty-six (26) sets of backbone sequences of the nucleic acid carrier; two CpG2006 moieties are respectively linked via a transition sequence to the 5' ends of the sequence a and the sequence c, and the CD40 aptamer is linked via a transition sequence to the 5' end of the sequence b;

preferably, the sequence of CpG2006 is SEQ ID No. 57: TCGTCGTTTTGTCGTTTTGTCGTT, with the internucleotide phosphate linkages between adjacent nucleotides phosphorothioate-modified;

preferably, the transition sequence is TTTTT;

preferably, the sequence of the CD40 aptamer is SEQ ID No. 154: CCAACGAGTAGGCGATAGCGCGTGG.

Drug 5): 4*CpG2006-CD40 aptamer-DNA carrier, wherein the DNA carrier corresponds to group 1) among the foregoing twenty-six (26) sets of backbone sequences of the nucleic acid carrier; two CpG2006 units are tandemly linked to form a CpG2006 dimer, two CpG2006 dimers are respectively linked to the 5' ends of the sequence a and the sequence c, and the CD40 aptamer is linked via a transition sequence to the 5' end of the sequence b;

preferably, the sequence of CpG2006 is SEQ ID No. 57: TCGTCGTTTTGTCGTTTTGTCGTT, with the internucleotide phosphate linkages between adjacent nucleotides phosphorothioate-modified;

preferably, the transition sequence is TTTTT;

preferably, the sequence of the CD40 aptamer is SEQ ID No. 154: CCAACGAGTAGGCGATAGCGCGTGG.

Drug 6): 2*CpG1826-CD40 aptamer-2*mannose-DNA carrier, wherein the DNA carrier corresponds to group 1) among the foregoing twenty-six (26) sets of backbone sequences of the nucleic acid carrier; two CpG1826 moieties are respectively linked via a transition sequence to the 5' ends of the sequence a and the sequence c; the CD40 aptamer is linked via a transition sequence to the 5' end of the sequence b; and two mannose moieties are respectively disposed at the 3' ends of the sequence b and the sequence c;

preferably, the sequence of CpG1826 is SEQ ID No. 59: TCCATGACGTTCCTGACG, with the internucleotide phosphate linkages between adjacent nucleotides phosphorothioate-modified;

preferably, the transition sequence is TTTTT;

preferably, the sequence of the CD40 aptamer is SEQ ID No. 154: CCAACGAGTAGGCGATAGCGCGTGG, with the internucleotide phosphate linkages between the first three nucleotides from the 5' end being phosphorothioate-modified.

Drug 7): CpG1826-CD40 aptamer-mannose-MUC1 aptamer-DNA carrier, wherein the DNA carrier corresponds to group 1) among the foregoing twenty-six (26) sets of backbone sequences of the nucleic acid carrier; CpG1826 is linked via a transition sequence to the 5' end of the sequence a; the CD40 aptamer is linked via a transition sequence to the 5' end of the sequence b; the MUC1 aptamer is linked via a transition sequence to the 5' end of the sequence c; and mannose is disposed at the 3' end of the sequence c;

preferably, the sequence of CpG1826 is SEQ ID No. 59: TCCATGACGTTCCTGACG, with the internucleotide phosphate linkages between adjacent nucleotides phosphorothioate-modified;

preferably, the transition sequence is TTTTT;

preferably, the sequence of the CD40 aptamer is SEQ ID No. 154: CCAACGAGTAGGCGATAGCGCGTGG;

preferably, the sequence of the MUC1 aptamer is SEQ ID No. 208: GCAGTTGATCCTTTGGATACCCTGG.

Drug 8): 2*CpG2006-CD40 aptamer-AmiR-21-3*mannose-DNA carrier, wherein the DNA carrier corresponds to group 1) among the foregoing twenty-six (26) sets of backbone sequences of the nucleic acid carrier; two CpG2006 moieties are respectively linked via a transition sequence to the 5' ends of the sequence a and the sequence c; the CD40 aptamer is linked via a transition sequence to the 5' end of the sequence b; AmiR-21 is directly linked to the 3' end of the sequence b; and three mannose moieties are respectively linked to the 3' ends of the sequence a, AmiR-21, and the sequence c;

preferably, the sequence of CpG2006 is SEQ ID No. 57: TCGTCGTTTTGTCGTTTTGTCGTT, with the internucleotide phosphate linkages between adjacent nucleotides being phosphorothioate-modified;

preferably, the transition sequence is TTTTT;

preferably, the sequence of the CD40 aptamer is SEQ ID No. 154: CCAACGAGTAGGCGATAGCGCGTGG;

preferably, the sequence of AmiR-21 is GATAAGCT, in which each nucleotide is locked nucleic acid (LNA)-modified.

Drug 9): 2*CpG2006-CD40 aptamer-CD16a aptamer-CTLA-4 aptamer-DNA carrier, wherein the DNA carrier corresponds to group 2) among the foregoing twenty-six (26) sets of backbone sequences of the nucleic acid carrier; two CpG2006 moieties are respectively linked via a transition sequence to the 5' ends of the sequence a and the sequence c; the CD40 aptamer is linked via a transition sequence to the 3' end of the sequence b; the CD16a aptamer is linked via a transition sequence to the 5' end of the sequence b; and the CTLA-4 aptamer is linked via a transition sequence to the 3' end of the sequence c;

preferably, the sequence of CpG2006 is SEQ ID No. 57: TCGTCGTTTTGTCGTTTTGTCGTT, with the internucleotide phosphate linkages between adjacent nucleotides being phosphorothioate-modified;

preferably, the transition sequence is TTTTT;

preferably, the sequence of the CD16a aptamer:
SEQ ID No. 157: CCACTGCGGGGGTCTATACGTGAGGAAGAAGTGG, with the internucleotide phosphate linkage between the first two nucleotides from the 5' end being phosphorothioate-modified;

preferably, the sequence of the CD40 aptamer:
SEQ ID No. 154: CCAACGAGTAGGCGATAGCGCGTGG, with the internucleotide phosphate linkage between the last two nucleotides at the 3' end being phosphorothioate-modified;

preferably, the sequence of the CTLA-4 aptamer:

SEQ ID No. 141: GGUGGAAGAGGGAUGGGCCGACGUGCCGCAU, with the internucleotide phosphate linkage between the second and third nucleotides counted from the 3' end being phosphorothioate-modified.

Drug 10): 2*CpG2006-CTLA-4 aptamer-PD-L1 aptamer-DNA carrier, wherein the DNA carrier corresponds to group 3) among the foregoing twenty-six (26) sets of backbone sequences of the nucleic acid carrier; two CpG2006 units are tandemly linked to form a CpG2006 dimer, the CpG2006 dimer is linked via a transition sequence to the 5' end of the sequence a, the PD-L1 aptamer is linked via a transition sequence to the 5' end of the sequence c, and the CTLA-4 aptamer is linked via a transition sequence to the 5' end of the sequence b;

preferably, the sequence of CpG2006 is SEQ ID No. 57: TCGTCGTTTTGTCGTTTTGTCGTT, with the internucleotide phosphate linkages between adjacent nucleotides phosphorothioate-modified;

preferably, the sequence of the PD-L1 aptamer:
SEQ ID No. 241: ACGGGCCACATCAACTCATTGATAGACAATGCGTCCACTGCCCGT, with the internucleotide phosphate linkage between the first two nucleotides from the 5' end phosphorothioate-modified;

preferably, the sequence of the CTLA-4 aptamer:
SEQ ID No. 141: GGUGGAAGAGGGAUGGGCCGACGUGCCGCAU, with the internucleotide phosphate linkage between the first two nucleotides from the 5' end phosphorothioate-modified;

preferably, the sequence of CpG2006 is SEQ ID No. 57: TCGTCGTTTTGTCGTTTTGTCGTT, with the internucleotide phosphate linkages between adjacent nucleotides phosphorothioate-modified;

preferably, the transition sequence is TTTTT.

Drug 11): CpG2006-CD40 aptamer-PD-L1 aptamer-DNA carrier, selected from any one of the following structures:

(i) CCPD3: the DNA carrier is the nucleic-acid carrier of the twelfth set among the foregoing twenty-six backbone sequence sets; CpG2006 is linked to the 5' end of the sequence a via a linker sequence; the PD-L1 aptamer is linked to the 5' end of the sequence c via a linker sequence; and the CD40 aptamer is linked to the 5' end of the sequence b via a linker sequence; wherein, in the DNA carrier, the three 3'-end internucleotide phosphate-backbone linkages of each of the sequences a, b and c are phosphorothioate;

preferably, the sequence of CpG2006 is SEQ ID No. 57: TCGTCGTTTTGTCGTTTTGTCGTT, in which the internucleotide phosphate-backbone linkages between adjacent nucleotides are phosphorothioate;

preferably, the PD-L1 aptamer has the sequence:
SEQ ID No. 241: ACGGGCCACATCAACTCATTGATAGACAATGCGTCCACTGCCCGT, with the first three 5'-end internucleotide linkages being phosphorothioate;

preferably, the CD40 aptamer has the sequence:
SEQ ID No. 154: CCAACGAGTAGGCGATAGCGCGTGG, with the first three 5'-end internucleotide linkages being phosphorothioate;

preferably, the linker sequence is TTTTT;
Or,

(ii) CCPD3 Variant 1: the DNA carrier is the nucleic-acid carrier of the twelfth set among the foregoing twenty-six backbone sequence sets; CpG2006 is linked to the 5' end of the sequence a via the linker sequence TTTTT; the CD40 aptamer is linked to the 5' end of the sequence b via the linker sequence TTTTT; and the PD-L1 aptamer is linked to the 5' end of the sequence c via the linker sequence ATTT; wherein, in the DNA carrier, the two 3'-end internucleotide phosphate-backbone linkages of each of the sequences a, b and c are phosphorothioate;

preferably, the sequence of CpG2006 is SEQ ID No. 57: TCGTCGTTTTGTCGTTTTGTCGTT, in which the internucleotide phosphate-backbone linkages between adjacent nucleotides are phosphorothioate;

preferably, the CD40 aptamer has the sequence:

SEQ ID No. 154: CCAACGAGTAGGCGATAGCGCGTGG, with the first three 5'-end internucleotide linkages being phosphorothioate;

preferably, the PD-L1 aptamer has the sequence:
SEQ ID No. 238: AACAACGTATAACAATGCCCACGTCACCAGAGTACTATGG, with the first four 5'-end internucleotide linkages being phosphorothioate;
Or,

(iii) CCPD3 Variant 2: The DNA carrier has the following sequences:

The sequence a is SEQ ID No. 295: GCCCACGAGCGTTCCGGGAGA;

The sequence b is SEQ ID No. 49: TCTCCCGGTTCGCCGCGAG;

The sequence c is: SEQ ID No. 296: CTCGCGGCCATAGCCGTGGGC;

CpG2006 is linked to the 5' end of the sequence a via the linker sequence TTTT; the CD40 aptamer is linked to the 5' end of the sequence b via the linker sequence TTTT; and the PD-L1 aptamer is linked to the 5' end of the sequence c via the linker sequence TTTT;

preferably, the sequence of CpG2006 is SEQ ID No. 57: TCGTCGTTTTGTCGTTTTGTCGTT, in which the internucleotide phosphate-backbone linkages between adjacent nucleotides are phosphorothioate;

preferably, the CD40 aptamer has the sequence:
SEQ ID No. 156: GCCAACGAGTAGGCGATAGCGCGTGGC, with the first four 5'-end internucleotide phosphate-backbone linkages being phosphorothioate;

preferably, the PD-L1 aptamer has the sequence:
SEQ ID No. 241: ACGGGCCACATCAACTCATTGATAGACAATGCGTCCACTGCCCGT, with the first five 5'-end internucleotide phosphate-backbone linkages being phosphorothioate;
Or,

(iv) CCPD3 Variant 3: The DNA carrier has the following sequences:

The sequence a is SEQ ID No. 295: GCCCACGAGCGTTCCGGGAGA;

The sequence b is SEQ ID No. 49: TCTCCCGGTTCGCCGCGAG;

The sequence c is SEQ ID No. 296: CTCGCGGCCATAGCCGTGGGC;

CpG2006 is linked to the 5' end of the sequence a via the linker sequence TTTT; the CD40 aptamer is linked to the 5' end of the sequence b via the linker sequence TTTT; and the PD-L1 aptamer is linked to the 5' end of the sequence c via the linker sequence TTTT;

preferably, the sequence of CpG2006 is SEQ ID No. 57: TCGTCGTTTTGTCGTTTTGTCGTT, in which the internucleotide phosphate-backbone linkages between adjacent nucleotides are phosphorothioate;

preferably, the CD40 aptamer has the sequence:
SEQ ID No. 156: GCCAACGAGTAGGCGATAGCGCGTGGC, with the first four 5'-end internucleotide phosphate-backbone linkages being phosphorothioate;

preferably, the PD-L1 aptamer has the sequence:
SEQ ID No. 246: GGGCCACATCAACTCATTGATAGACAATGCGTCCACTGCCC, with the first four 5'-end internucleotide phosphate-backbone linkages being phosphorothioate;

(v) CCPD3 Variant 4:
The DNA carrier has the following sequences:

The sequence a is SEQ ID No. 295: GCCCACGAGCGTTCCGGGAGA;

The sequence b is SEQ ID No. 49: TCTCCCGGTTCGCCGCGAG;

The sequence c is SEQ ID No. 296: CTCGCGGCCATAGCCGTGGGC ;

CpG2006 is linked to the 5' end of the sequence a via the linker sequence TTTT; the CD40 aptamer is linked to the 5' end of the sequence b via the linker sequence TTTT; and the PD-L1 aptamer is linked to the 5' end of the sequence c via the linker sequence TTTT;

preferably, the sequence of CpG2006 is SEQ ID No. 57: TCGTCGTTTTGTCGTTTTGTCGTT, in which the internucleotide phosphate-backbone linkages between adjacent nucleotides are phosphorothioate;

preferably, the CD40 aptamer has the sequence:
SEQ ID No. 156: GCCAACGAGTAGGCGATAGCGCGTGGC, with the first four 5'-end internucleotide phosphate-backbone linkages being phosphorothioate;

preferably, the PD-L1 aptamer has the sequence:
SEQ ID No. 248: TACAGGTTCTGGGGGGTGGGTGGGGAACCTGTT, with the first four 5'-end internucleotide phosphate-backbone linkages being phosphorothioate;

(vi) CCPD3 Variant 5:
The DNA carrier has the following sequences:

The sequence a is SEQ ID No. 18: GCGACGCCCACGAGCGTTCCGGGAGAGGAGC;

The sequence b is SEQ ID No. 19: GCTCCTCTCCCGGTTCGCCGCGAGCCGCG;

The sequence c is SEQ ID No. 20: <u>CAGCAGCAGCAGCA</u>CGCGGCTCGCGGCCATAGCCGTGGGCGTCGC, wherein SEQ ID No. 12: CAGCAGCAGCAGCA is a single-stranded connecting bridge sequence;

CpG2006 is linked to the 5' end of the sequence a via the linker sequence TTTT; the CD40 aptamer is linked to the 5' end of the sequence b via the linker sequence TTTTT; and the PD-L1 aptamer is linked to the 3' end of the complementary sequence of the single-stranded connecting bridge sequence via the linker sequence TTTTT, and-through complementary base pairing between the single-stranded connecting bridge sequence and its complementary sequence-is connected to the 5' end of the sequence c;

preferably, the sequence of CpG2006 is SEQ ID No. 57: TCGTCGTTTTGTCGTTTTGTCGTT, in which the internucleotide phosphate-backbone linkages between adjacent nucleotides are phosphorothioate;

preferably, the CD40 aptamer has the sequence:
SEQ ID No. 154: CCAACGAGTAGGCGATAGCGCGTGG, with the first five 5'-end internucleotide phosphate-backbone linkages being phosphorothioate;

preferably, the sequence of the complementary-sequence-PD-L1 aptamer conjugate is SEQ ID No. 297: <u>TG CTGCTGCTGCTG</u>TTTTT ACGGGCCACATCAACTCATTGATAGACAATGCGTCCACTGCCCGT, with the first four 3'-end internucleotide phosphate-backbone linkages being phosphorothioate, wherein SEQ ID No. 11: <u>TGCTGCTGCTGCTG</u> is the complementary sequence of the single-stranded connecting bridge sequence;

(vii) CCPD3 Variant 6:
The DNA carrier has the following sequences:

The sequence a is SEQ ID No. 18: GCGACGCCCACGAGCGTTCCGGGAGAGGAGC;

The sequence b is SEQ ID No. 19: GCTCCTCTCCCGGTTCGCCGCGAGCCGCG;

The sequence c is SEQ ID No. 20: <u>CAGCAGCAGCAGCA</u>CGCGGCTCGCGGCCATAGCCGTGGGCGTCGC, wherein SEQ ID No. 12: CAGCAGCAGCAGCA is a single-stranded connecting bridge sequence;

CpG2006 is linked to the 5' end of the sequence a via the linker sequence TTTT; the CD40 aptamer is linked to the 5' end of the sequence b via the linker sequence TTTTT; and the PD-L1 aptamer is linked to the 3' end of the complementary sequence of the single-stranded connecting bridge sequence via the linker sequence TTTTT, and-through complementary base pairing between the single-stranded connecting bridge sequence and its complementary sequence-is connected to the 5' end of the sequence c;

preferably, the sequence of CpG2006 is SEQ ID No. 57: TCGTCGTTTTGTCGTTTTGTCGTT, in which the internucleotide phosphate-backbone linkages between adjacent nucleotides are phosphorothioate;

preferably, the CD40 aptamer has the sequence:
SEQ ID No. 154: CCAACGAGTAGGCGATAGCGCGTGG, with the first five 5'-end internucleotide phosphate-backbone linkages being phosphorothioate;

referably, the sequence of the complementary-sequence-TTTTT-PD-L1 aptamer conjugate is SEQ ID No. 298: TGCTGCTGCTGCTGTTTTTTACAGGTTCTGGGGGGTGGGTGGGGAACCTGTT, with the first four 3'-end internucleotide phosphate-backbone linkages being phosphorothioate, wherein SEQ ID No. 11: TGCTGCTGCTGCTG is the complementary sequence of the single-stranded connecting bridge sequence;

(viii) CCPD3 Variant 7:
The DNA carrier has the following sequences:

The sequence a is SEQ ID No. 15: GACGCCCACGAGCGTTCCGGGAGAGGTGTAGCACGGTGGC, wherein SEQ ID No. 7: TGTAGCACGGTGGC is a single-stranded connecting bridge sequence A;

The sequence b is SEQ ID No. 16: CCTCTCCCGGTTCGCCGCGAGCCTCGGCGCGGCCGTG, wherein SEQ ID No. 9: TCGGCGCGGCCGTG is a single-stranded connecting bridge sequence B;

The sequence c is SEQ ID No. 17: GGCTCGCGGCCATAGCCGTGGGCGTCTGCTGCTGCTGCTG, wherein SEQ ID No. 11: TGCTGCTGCTGCTG is a single-stranded connecting bridge sequence C;

CpG2006 is linked to the 5' end of the complementary sequence C' of the single-stranded connecting bridge sequence C via a linker sequence, and-through complementary base pairing between the complementary sequence C' and the single-stranded connecting bridge sequence C-is connected to the 3' end of the sequence c;

The PD-L1 aptamer is sequentially linked to a linker sequence and the complementary sequence B' of the single-stranded connecting bridge sequence B, and-through complementary base pairing between the complementary sequence B' and the single-stranded connecting bridge sequence B-is connected to the 3' end of the sequence b;

The CD40 aptamer is sequentially linked to a linker sequence and the complementary sequence A' of the single-stranded connecting bridge sequence A, and-through complementary base pairing between the complementary sequence A' and the single-stranded connecting bridge sequence A-is connected to the 3' end of the sequence a;

preferably, the sequence of CpG2006 is SEQ ID No. 57: TCGTCGTTTTGTCGTTTTGTCGTT, in which the internucleotide phosphate-backbone linkages between adjacent nucleotides are phosphorothioate;

preferably, the CD40 aptamer has the sequence SEQ ID No. 156: GCCAACGAGTAGGCGATAGCGCGTGGC, with the first four 5'-end internucleotide phosphate-backbone linkages being phosphorothioate;

preferably, the PD-L1 aptamer has the sequence SEQ ID No. 241: ACGGGCCACATCAACTCATTGATAGAC AATGCGTCCACTGCCCGT, with the first four 5'-end internucleotide phosphate-backbone linkages being phosphorothioate;

preferably, the linker sequence is TTTTT.

(iv) CCPD3-Variant 8:

The sequence a is SEQ ID No. 18: GCGACGCCCACGAGCGTTCCGGGAGAGGAGC;

The sequence b is SEQ ID No. 19: GCTCCTCTCCCGGTTCGCCGCGAGCCGCG;

The sequence c is SEQ ID No. 31: CGCGGCTCGCGGCCATAGCCGTGGGCGTCGC;

CpG2006 is linked to the 5' end of the sequence a via a linker sequence; the CD40 aptamer is linked to the 5' end of the sequence b via a linker sequence; and the PD-L1 aptamer is linked to the 5' end of the sequence c via the indicated linker sequence;

preferably, CpG2006 has the sequence SEQ ID No. 57: TCGTCGTTTTGTCGTTTTGTCGTT, in which the internucleotide phosphate-backbone linkages between adjacent nucleotides are phosphorothioate;

preferably, the CD40 aptamer has the sequence SEQ ID No. 156: GCCAACGAGTAGGCGATAGCGCGTGGC, with 2'-O-MOE modifications on the ribose of the first four 5'-end nucleotides, and the base(s) of the cytidine nucleotide(s) denoted Cm with 5-methyl substitutions;

preferably, the PD-L1 aptamer has the sequence SEQ ID No. 241: ACGGGCCACATCAACTCATTGATAGAC AATGCGTCCACTGCCCGT, with 2'-O-MOE modifications on the ribose of the first four 5'-end nucleotides, and the base(s) of the cytidine nucleotide(s) denoted Cm with 5-methyl substitutions;

preferably, the linker sequence is TTTTT.

(v) CCPD3-Variant 9,

The sequence a is SEQ ID No. 18: GCGACGCCCACGAGCGTTCCGGGAGAGGAGC;

The sequence b is SEQ ID No. 19: GCTCCTCTCCCGGTTCGCCGCGAGCCGCG;

The sequence c is SEQ ID No. 31: CGCGGCTCGCGGCCATAGCCGTGGGCGTCGC;

CpG2006 is linked to the 5' end of the sequence a via a linker sequence; the CD40 aptamer is linked to the 5' end of the sequence b via a linker sequence; and the PD-L1 aptamer is linked to the 5' end of the sequence c via the indicated linker sequence;

preferably, CpG2006 has the sequence SEQ ID No. 57: TCGTCGTTTTGTCGTTTTGTCGTT, in which the internucleotide phosphate-backbone linkages between adjacent nucleotides are phosphorothioate;

preferably, the CD40 aptamer has the sequence SEQ ID No. 154: CCAACGAGTAGGCGATAGCGCGTGG, with 2'-O-MOE modifications on the ribose of the first five 5'-end nucleotides, and three cytidine nucleotide(s) denoted Cm have 5-methyl substitutions on the base;

preferably, the PD-L1 aptamer has the sequence SEQ ID No. 241: ACGGGCCACATCAACTCATTGATAGAC AATGCGTCCACTGCCCGT, with 2'-O-MOE modifications on the ribose of the first five 5'-end nucleotides, and one cytidine nucleotide denoted Cm has a 5-methyl substitution on the base;

preferably, the linker sequence is TTTTT.

(vi) CCPD3-Variant 10,

The sequence a is SEQ ID No. 18: GCGACGCCCACGAGCGTTCCGGGAGAGGAGC;

The sequence b is SEQ ID No. 19: GCTCCTCTCCCGGTTCGCCGCGAGCCGCG;

The sequence c is SEQ ID No. 31: CGCGGCTCGCGGCCATAGCCGTGGGCGTCGC;

CpG2006 is linked to the 5' end of the sequence a via a linker sequence; the CD40 aptamer is linked to the 5' end of the sequence b via a linker sequence; and the PD-L1 aptamer is linked to the 5' end of the sequence c via the indicated linker sequence;

preferably, CpG2006 has the sequence SEQ ID No. 57: TCGTCGTTTTGTCGTTTTGTCGTT, in which the internucleotide phosphate-backbone linkages between adjacent nucleotides are phosphorothioate;

preferably, the CD40 aptamer has the sequence SEQ ID No. 154: CCAACGAGTAGGCGATAGCGCGTGG, with 2'-O-MOE modifications on the ribose of the first five 5'-end nucleotides, and the bases of three cytidine nucleotides denoted Cm with 5-methyl substitutions;

preferably, the PD-L1 aptamer has the sequence SEQ ID No. 249: CTACGAGACGAACTTATGCGTAATAAT GACTGTCGTAG, with 2'-O-MOE modifications on the ribose of the first five 5'-end nucleotides, and the base of one cytidine nucleotide denoted Cm with a 5-methyl substitution;

preferably, the linker sequence is TTTTT.

(vii) CCPD3-Variant 11,

The sequence a is SEQ ID No. 18: GCGACGCCCACGAGCGTTCCGGGAGAGGAGC;

The sequence b is SEQ ID No. 19: GCTCCTCTCCCGGTTCGCCGCGAGCCGCG;

The sequence c is SEQ ID No. 299: <u>CGCGGCTCGCGGCT</u>CGCGGCTCGCGGCCATAGCCGTGGGCGTCGC, wherein the underlined portion constitutes a connecting bridge sequence;

CpG2006 is linked to the 5' end of the sequence a via a linker sequence; the CD40 aptamer is linked to the 5' end of the sequence b via a linker sequence; and the PD-L1 aptamer is linked to the 3' end of the complementary sequence of the connecting bridge sequence (SEQ ID No. 14: AGCCGCGAGCCGCG), and-through complementary base pairing between the complementary sequence of the connecting bridge sequence and the connecting bridge sequence-is connected to the 5' end of the sequence c;

preferably, CpG2006 has the sequence SEQ ID No. 57: TCGTCGTTTTGTCGTTTTGTCGTT, in which the internucleotide phosphate-backbone linkages between adjacent nucleotides are phosphorothioate;

preferably, the CD40 aptamer has the sequence SEQ ID No. 154: CCAACGAGTAGGCGATAGCGCGTGG, with 2'-O-MOE modifications on the ribose of the first five 5'-end nucleotides;

preferably, the PD-L1 aptamer has the sequence SEQ ID No. 241: ACGGGCCACATCAACTCATTGATAGAC AATGCGTCCACTGCCCGT, with 2'-O-MOE modifications on the ribose of the first 1-5 3'-end nucleotides;

preferably, the linker sequence is TTTTT.

(viii) CCPD3-Variant 12,

The sequence a is SEQ ID No. 18: GCGACGCCCACGAGCGTTCCGGGAGAGGAGC;

The sequence b is SEQ ID No. 19: GCTCCTCTCCCGGTTCGCCGCGAGCCGCG;

The sequence c is SEQ ID No. 299: <u>CGCGGCTCGCGGCT</u>CGCGGCTCGCGGCCATAGCCGTGGGCGTCGC, wherein the underlined portion constitutes a connecting bridge sequence;

CpG2006 is linked to the 5' end of the sequence a via a linker sequence; the CD40 aptamer is linked to the 5' end of the sequence b via a linker sequence; and the PD-L1 aptamer is linked to the 3' end of the complementary sequence of the connecting bridge sequence (SEQ ID No. 14: AGCCGCGAGCCGCG) via a linker sequence, and-through complementary base pairing between the complementary sequence of the connecting bridge sequence and the connecting bridge sequence-is connected to the 5' end of the sequence c;

preferably, CpG2006 has the sequence SEQ ID No. 57: TCGTCGTTTTGTCGTTTTGTCGTT, in which the internucleotide phosphate-backbone linkages between adjacent nucleotides are phosphorothioate;

preferably, the CD40 aptamer has the sequence SEQ ID No. 154: CCAACGAGTAGGCGATAGCGCGTGG, with 2'-O-MOE modifications on the ribose of the first five 5'-end nucleotides;

preferably, the PD-L1 aptamer has the sequence SEQ ID No. 249: CTACGAGACGAACTTATGCGTAATAAT GACTGTCGTAG, with 2'-O-MOE modifications on the ribose of the first 5 nucleotides on 3'-end;

preferably, the linker sequence is TTTTT.

Drug 12): CD16a aptamer-CTLA-4 aptamer-CpG2006-DNA carrier, wherein the DNA carrier is the nucleic-acid carrier of the fourth set among the foregoing twenty-six backbone sequence sets; the CD16a aptamer is linked to the 5' end of the sequence a via a linker sequence; the CTLA-4 aptamer is linked to the 5' end of the sequence b via a linker sequence; and CpG2006 is linked to the 5' end of the sequence c via a linker sequence; wherein, in the DNA carrier, the two 3'-end internucleotide phosphate-backbone linkages of each of the sequences a, b and c are phosphorothioate;

preferably, the CD16a aptamer has the sequence:
SEQ ID No. 157: CCACTGCGGGGGTCTATACGTGAGGAAGAAGTGG, in which the 5'-end internucleotide phosphate-backbone linkage between the first two nucleotides is phosphorothioate;

preferably, the CTLA-4 aptamer has the sequence:
SEQ ID No. 141: GGUGGAAGAGGGAUGGGCCGACGUGCCGCAU, in which the 5'-end internucleotide phosphate-backbone linkage between the first two nucleotides is phosphorothioate, the cytidine (C) and uridine (U) residues are 2'-fluoro (2'-F) modified, and the adenosine (A) and guanosine (G) residues are 2'-O-methyl (2'-OMe) modified;

preferably, CpG2006 has the sequence SEQ ID No. 57: TCGTCGTTTTGTCGTTTTGTCGTT, in which the internucleotide phosphate-backbone linkages between adjacent nucleotides are phosphorothioate;

preferably, the linker sequence is TTTTT.

Drug 13): 2*CpG2006-CD40 aptamer-PD-L1 aptamer-C12 aptamer-DNA carrier, wherein the DNA carrier is the nucleic-acid carrier of the fifth set among the foregoing twenty-six backbone sequence sets; two CpG2006 sequences are respectively linked to the 5' ends of the sequence a and the sequence c via linker sequences, and the CD40 aptamer is disposed at the 5' end of the sequence b; in the DNA carrier, for each of the sequences a, b and c, the two 3'-end internucleotide phosphate-backbone linkages are phosphorothioate;

The PD-L1 aptamer is sequentially linked to a linker sequence and to the complementary sequence B' of a single-stranded connecting bridge sequence B, the single-stranded connecting bridge sequence B being located at the 3' end of the sequence b, and-through complementary base pairing between the complementary sequence B' and the single-stranded connecting bridge sequence B-is connected to the 3' end of the sequence b;

The C12 aptamer is sequentially linked to a linker sequence and to the complementary sequence C' of a single-stranded connecting bridge sequence C, the single-stranded connecting bridge sequence C being located at the 3' end of the sequence c, and-through complementary base pairing between the complementary sequence C' and the single-stranded connecting bridge sequence C-is connected to the 3' end of the sequence c;

preferably, CpG2006 has the sequence SEQ ID No. 57: TCGTCGTTTTGTCGTTTTGTCGTT, in which the internucleotide phosphate-backbone linkages between adjacent nucleotides are phosphorothioate;

preferably, the linker sequence is TTTTT;

preferably, the CD40 aptamer has the sequence:
SEQ ID No. 154: CCAACGAGTAGGCGATAGCGCGTGG, with the first three 5'-end internucleotide phosphate-backbone linkages being phosphorothioate;

preferably, the PD-L1 aptamer has the sequence:
SEQ ID No. 241: ACGGGCCACATCAACTCATTGATAGACAATGCGTCCACTGCCCGT, with the first three 5'-end internucleotide phosphate-backbone linkages being phosphorothioate;

preferably, the C12 aptamer has the sequence:

SEQ ID No. 164: GTGGATTGTTGTGTTCTGTTGGTTTTTGTGTTGTC, with the first three 5'-end internucleotide phosphate-backbone linkages being phosphorothioate;

preferably, the single-stranded connecting bridge sequence B is SEQ ID No. 7: TGTAGCACGGTGGC, and its complementary sequence B' is SEQ ID No. 8: GCCACCGTGCTACA;

preferably, the single-stranded connecting bridge sequence C is SEQ ID No. 11: TGCTGCTGCTGCTG, and its complementary sequence C' is SEQ ID No. 12: CAGCAGCAGCAGCA.

Drug 14): CpG2006-C12 aptamer-CD47 siRNA-PD-L1 aptamer-PD-L1 siRNA-DNA carrier, wherein the DNA carrier is the nucleic-acid carrier of the sixth set among the foregoing twenty-six backbone sequence sets; CpG2006 is linked to the 5' end of the sequence a via a linker sequence; the C12 aptamer is linked to the antisense strand of CD47 siRNA via a linker sequence; the sense strand of CD47 siRNA is disposed at the 5' end of the sequence b; the antisense strand of CD47 siRNA, through complementarity to the sense strand of CD47 siRNA, connects the C12 aptamer and the CD47 siRNA at the 5' end of the sequence b; the sense strand of PD-L1 siRNA is connected to the 5' end of the sequence c; the PD-L1 aptamer is linked to the antisense strand of PD-L1 siRNA via a linker sequence; and, in the DNA carrier, for each of the sequences a, b and c, the three 3'-end internucleotide phosphate-backbone linkages are phosphorothioate;

preferably, the C12 aptamer has the sequence:
SEQ ID No. 164: GTGGATTGTTGTGTTCTGTTGGTTTTTGTGTTGTC, in which the first three 5'-end internucleotide phosphate-backbone linkages are phosphorothioate;

preferably, the antisense strand of CD47 siRNA is SEQ ID No. 79: AUAUCUCUGGGUAAUCACCUU and the sense strand is SEQ ID No. 78: GGUGAUUACCCAGAGAUAUUU, wherein the first three 5'-end internucleotide phosphate-backbone linkages of the antisense strand are phosphorothioate, the first three 5'-end internucleotide phosphate-backbone linkages of the sense strand are phosphorothioate, and the first three 3'-end internucleotide phosphate-backbone linkages of the sense strand are phosphorothioate;

preferably, the PD-L1 aptamer has the sequence:
SEQ ID No. 239: GCCCCAGTTATGCTTTCCCCCTCTGTCTCTTTG, with the first three 5'-end internucleotide phosphate-backbone linkages being phosphorothioate;

preferably, the sense strand of PD-L1 siRNA is SEQ ID No. 94: CCAGCACACUGAGAAUCAAUU and the antisense strand is SEQ ID No. 95: UUGAUUCUCAGUGUGCUGGUU, wherein the first three 5'-end and the first three 3'-end internucleotide phosphate-backbone linkages of the sense strand are phosphorothioate, and the first three 5'-end internucleotide phosphate-backbone linkages of the antisense strand are phosphorothioate;

preferably, CpG2006 has the sequence SEQ ID No. 57: TCGTCGTTTTGTCGTTTTGTCGTT, in which the internucleotide phosphate-backbone linkages between adjacent nucleotides are phosphorothioate;

preferably, the linker sequence is TTTTT.

Drug 15): 2*CpG2006-CD40 aptamer-CD16a aptamer-DNA carrier, wherein the DNA carrier is the nucleic-acid carrier of the seventh set among the foregoing twenty-six backbone sequence sets; two CpG2006 sequences are respectively linked to the 5' ends of the sequence a and the sequence c via linker sequences; the CD40 aptamer is linked to the 5' end of the sequence b via a linker sequence; the CD16a aptamer is linked to the 3' end of the sequence b via a linker sequence; and, in the DNA carrier, for each of the sequences a and c, the two 3'-end internucleotide phosphate-backbone linkages are phosphorothioate;

preferably, CpG2006 has the sequence SEQ ID No. 57: TCGTCGTTTTGTCGTTTTGTCGTT, in which the internucleotide phosphate-backbone linkages between adjacent nucleotides are phosphorothioate;

preferably, the linker sequence is TTTTT;

preferably, the CD16a aptamer has the sequence:
SEQ ID No. 157: CCACTGCGGGGGGTCTATACGTGAGGAAGAAGTGG, in which the phosphate-backbone linkage between the two nucleotides immediately upstream of the 3' end is phosphorothioate;

preferably, the CD40 aptamer has the sequence:
SEQ ID No. 154: CCAACGAGTAGGCGATAGCGCGTGG, in which the phosphate-backbone linkage between the terminal two nucleotides at the 5' end is phosphorothioate.

Drug 16): 2*CpG2006-CD40 aptamer-CD16a aptamer-FAP aptamer-DNA carrier, wherein the DNA carrier is the nucleic-acid carrier of the eighth set among the foregoing twenty-six backbone sequence sets; two CpG2006 sequences are respectively linked to the 5' ends of the sequence a and the sequence c via linker sequences; the CD40 aptamer is linked to the 5' end of the sequence b via a linker sequence; the CD16a aptamer is linked to the 3' end of the sequence b via a linker sequence; the FAP aptamer is linked to the 3' end of the sequence c via a linker sequence; and, in the DNA carrier, the phosphate-backbone linkage between the two nucleotides immediately upstream of the 3' end of the sequence a is phosphorothioate;

preferably, CpG2006 has the sequence SEQ ID No. 57: TCGTCGTTTTGTCGTTTTGTCGTT, in which the internucleotide phosphate-backbone linkages between adjacent nucleotides are phosphorothioate;

preferably, the linker sequence is TTTTT;

preferably, the CD16a aptamer has the sequence:
SEQ ID No. 157: CCACTGCGGGGGGTCTATACGTGAGGAAGAAGTGG, in which the phosphate-backbone linkage between the two nucleotides immediately upstream of the 3' end is phosphorothioate;

preferably, the CD40 aptamer has the sequence:
SEQ ID No. 154: CCAACGAGTAGGCGATAGCGCGTGG, in which the phosphate-backbone linkage between the terminal two nucleotides at the 5' end is phosphorothioate;

preferably, the FAP aptamer has the sequence:
SEQ ID No. 188: CCGCTCGAGCTAGTCTGACAAAGAGAAACAC, in which the phosphate-backbone linkage between the terminal two nucleotides at the 3' end is phosphorothioate.

Drug 17): 2*CpG2006-VEGF aptamer-CD40 aptamer-PD-L1 aptamer-CD16a aptamer-DNA carrier, wherein the DNA carrier is the nucleic acid carrier of group (6) among the foregoing 26 backbone-sequence carriers; two CpG2006 moieties are respectively linked via linker sequences to the 5' ends of the sequence a and the sequence c; the CD40 aptamer is linked via a linker sequence to the 5' end of the sequence b; the PD-L1 aptamer is linked via a linker sequence to the 3' end of the sequence b; the CD16a aptamer is linked via a linker sequence to the 3' end of the sequence c; and the VEGF aptamer is linked via a linker sequence to the 3' end of the sequence a;

preferably, the sequence of CpG2006 is SEQ ID No. 57: TCGTCGTTTTGTCGTTTTGTCGTT, in which the phosphate backbone between adjacent nucleotides is phosphorothioate-modified;

preferably, the VEGF aptamer:
SEQ ID No. 279: GGTGGGGGTGGACGGGCCGGGTAGA, with a phosphorothioate modification in the phosphate backbone between the last two nucleotides at the 3' end;

preferably, the sequence of the CD40 aptamer:
SEQ ID No. 154: CCAACGAGTAGGCGATAGCGCGTGG, with a phosphorothioate modification in the phosphate backbone between the first two nucleotides at the 5' end;

preferably, the sequence of the PD-L1 aptamer:
SEQ ID No. 241: ACGGGCCACATCAACTCATTGATAGACAATGCGTCCACTGCCCGT, with a phosphorothioate modification in the phosphate backbone between the last two nucleotides at the 3' end;

preferably, the sequence of the CD16a aptamer:
SEQ ID No. 157: CCACTGCGGGGGGTCTATACGTGAGGAAGAAGTGG, with a phosphorothioate modification in the phosphate backbone between the last two nucleotides at the 3' end.

Drug 18): 2*AmiR21-TTA1 aptamer-DNA carrier, wherein the DNA carrier is the nucleic acid carrier of group (13) among the foregoing 26 backbone-sequence carriers; two AmiR21 moieties are respectively directly linked to the 5' ends of the sequence a and the sequence c; the TTA1 aptamer is linked via a linker sequence to the 5' end of the sequence b;

preferably, the linker sequence is TTTTT;

preferably, the sequence of AmiR21 is GATAAGCT, with each nucleotide bearing a locked nucleic acid (LNA) modification;

preferably, the sequence of the TTA1 aptamer:
SEQ ID No. 266: CTGCACTTGGCTTGGATTTCAGAAGGGAGACCC.

Drug 19): 2*AmiR-21-3*biotin-DNA carrier, wherein the DNA carrier is the nucleic acid carrier of group (13) among the foregoing 26 backbone-sequence carriers; two AmiR-21 moieties are respectively directly linked to the 5' ends of the sequence a and the sequence c; and three biotin groups are respectively linked to the 5' and 3' ends of the sequence b and to the 3' end of the sequence c;
preferably, the sequence of AmiR-21 is GATAAGCT, in which each position is a locked nucleic acid (LNA) modification.
Drug 20): 2*AmiR-21-4*biotin-DNA carrier, wherein the DNA carrier is the nucleic acid carrier of group (13) among the foregoing 26 backbone-sequence carriers; two AmiR-21 moieties are respectively directly linked to the 5' ends of the sequence a and the sequence c; four biotin groups are respectively linked to the 3' end of the sequence a, the 5' and 3' ends of the sequence b, and the 3' end of the sequence c;
preferably, the sequence of AmiR-21 is GATAAGCT, in which each position is a locked nucleic acid (LNA) modification.
Drug 21): 2* AmiR-21-AS1411 aptamer-DNA carrier, wherein the DNA carrier is the nucleic acid carrier of group (13) among the foregoing 26 backbone-sequence carriers; two AmiR-21 moieties are respectively directly linked to the 5' ends of the sequence a and the sequence c; the AS1411 aptamer is linked via a linker sequence to the 5' end of the sequence b;

preferably, the linker sequence is TTTTT;

preferably, the sequence of AmiR-21 is GATAAGCT, in which each position is a locked nucleic acid (LNA) modification;

preferably, the sequence of the AS1411 aptamer:
SEQ ID No. 131: GGTGGTGGTGGTTGTGGTGGTGGTGG.

Drug 22): 2*AmiR-21-AS1411 aptamer-DNA carrier, wherein the DNA carrier is the nucleic acid carrier of group (23) among the foregoing 26 backbone-sequence carriers; two AmiR-21 moieties are respectively directly linked to the 5' ends of the sequence a and the sequence c; the AS1411 aptamer is linked via a linker sequence to the 5' end of the sequence b;

preferably, the linker sequence is TTTTT;

preferably, the sequence of AmiR-21 is GATAAGCT, wherein positions 1 and 3-5 with 2'-O-methoxyethyl (2'-OME) modifications, and positions 2 and 6-8 with 2'-fluoro (2'-F) modifications;

preferably, the sequence of the AS1411 aptamer:
SEQ ID No. 131: GGTGGTGGTGGTTGTGGTGGTGGTGG.

Drug 23): 2* AmiR-21-AS1411 aptamer-biotin-DNA carrier, wherein the DNA carrier is the nucleic acid carrier of group (13) among the foregoing 26 backbone-sequence carriers; two AmiR-21 moieties are respectively directly linked to the 5' ends of the sequence a and the sequence c; the AS1411 aptamer is linked via a linker sequence to the 5' end of the sequence b; and a biotin is linked to the 3' end of the sequence c;

preferably, the linker sequence is TTTTT;

preferably, the sequence of AmiR-21 is GATAAGCT, in which each position is a locked nucleic acid (LNA) modification;

preferably, the sequence of the AS1411 aptamer:
SEQ ID No. 131: GGTGGTGGTGGTTGTGGTGGTGGTGG.

Drug 24): 2* AmiR-21-CD40 aptamer-DNA carrier, wherein the DNA carrier is the nucleic acid carrier of group (13) among the foregoing 26 backbone-sequence carriers; two AmiR-21 moieties are respectively directly linked to the 5' ends of the sequence a and the sequence c; the CD40 aptamer is linked via a linker sequence to the 5' end of the sequence b;

preferably, the linker sequence is TTTTT;

preferably, the sequence of AmiR-21 is GATAAGCT, in which each position is a locked nucleic acid (LNA) modification;

preferably, the sequence of the CD40 aptamer is SEQ ID No. 154: CCAACGAGTAGGCGATAGCGCGTGG.

Drug 25): 2*AmiR-21-MUC1 aptamer-3*biotin-DNA carrier, wherein the DNA carrier is the nucleic acid carrier of group (13) among the foregoing 26 backbone-sequence carriers; two AmiR-21 moieties are respectively directly linked to the 5' ends of the sequence a and the sequence c; the MUC1 aptamer is linked via a linker sequence to the 5' end of the sequence b;

preferably, the linker sequence is TTTTT;

preferably, the sequence of AmiR-21 is GATAAGCT, in which each position is locked nucleic acid (LNA) modified;

preferably, the sequence of the MUC1 aptamer is SEQ ID No. 208: GCAGTTGATCCTTTGGATACCCTGG.

Drug 26): AmiR-21-AS1411 aptamer-A15 aptamer-2*biotin-DNA carrier, wherein the DNA carrier is the nucleic acid carrier of group (13) among the foregoing 26 backbone-sequence carriers; AmiR-21 is directly linked to the 5' end of the sequence a; the AS1411 aptamer and the A15 aptamer are respectively linked via linker sequences to the 5' ends of the sequence b and the sequence c; two biotin groups are respectively linked to the 3' end of the sequence b and the 3' end of the sequence c;

preferably, the linker sequence is TTTTT;

preferably, the sequence of AmiR-21 is GATAAGCT, in which each position is locked nucleic acid (LNA) modified;

preferably, the sequence of the AS1411 aptamer is SEQ ID No. 131: GGTGGTGGTGGTTGTGGTGGTGGTGG;

preferably, the sequence of the A15 aptamer is SEQ ID No. 129: CCCTCCTACATAGGG.

Drug 27): 2*mannose-2*AmiR-21-DNA carrier, wherein the DNA carrier is the nucleic-acid carrier of group (13) among the foregoing 26 backbone-sequence carriers; two mannose groups are respectively linked to the 5' and 3' ends of the sequence b; two AmiR-21 moieties are respectively directly linked to the 5' ends of the sequence a and the sequence c; preferably, the sequence of AmiR-21 is GATAAGCT, in which each position is locked nucleic acid (LNA) modified.

Drug 28): 2*mannose-A15 aptamer-2*AmiR-21-DNA carrier, wherein the DNA carrier is the nucleic-acid carrier of group (13) among the foregoing 26 backbone-sequence carriers; the A15 aptamer is linked via a linker sequence to the 5' end of the sequence b; two mannose groups are respectively linked to the 5' end of the A15 aptamer and the 3' end of the sequence b; two AmiR-21 moieties are respectively directly linked to the 5' ends of the sequence a and the sequence c;

preferably, the sequence of AmiR-21 is GATAAGCT, in which each position is locked nucleic acid (LNA) modified;

preferably, the sequence of the A15 aptamer is SEQ ID No. 129: CCCTCCTACATAGGG.

Drug 29): 2*mannose-survivin siRNA-DNA carrier, wherein the DNA carrier is the nucleic-acid carrier of group (13) among the foregoing 26 backbone-sequence carriers; two mannose groups are respectively linked to the 5' and 3' ends of the sequence b; the sense strand of survivin siRNA is linked to the 3' end of the sequence c; the antisense strand of survivin siRNA is connected to the 3' end of the c-strand by complementary pairing with the sense strand of survivin siRNA;

preferably, the sense strand of survivin siRNA is SEQ ID No. 104: UGCAGGUUCCUUAUCUGUCATT,

The antisense strand of survivin siRNA is SEQ ID No. 105: UGACAGAUAAGGAACCUGCTT; The TT at the 3' end of the sense and antisense strands of survivin siRNA are dTdT modifications.

Drug 30): 2*mannose-A15 aptamer-survivin siRNA-DNA carrier, wherein the DNA carrier is the nucleic acid carrier of group (22) among the foregoing 26 backbone-sequence carriers; the A15 aptamer is linked via the linker sequence TTTTT to the 5' end of the sequence b; two mannose groups are respectively linked to the 5' end of the A15 aptamer and the 3' end of the sequence b; the sense strand of survivin siRNA is linked to the 3' end of the sequence c; the antisense strand of survivin siRNA is connected to the 3' end of the sequence c by complementary pairing with the sense strand of survivin siRNA;

preferably, the sense strand of survivin siRNA is SEQ ID No. 104: UGCAGGUUCCUUAUCUGUCATT;

The antisense strand of survivin siRNA is SEQ ID No. 105: UGACAGAUAAGGAACCUGCdTT; The TT at the 3' end of the antisense and sense strands of survivin siRNA are dTdT modifications;

preferably, the sequence of the A15 aptamer is SEQ ID No. 129: CCCTCCTACATAGGG.

Drug 31): AS1411 aptamer-survivin siRNA-PSMA aptamer-3*AmiR-21-RNA carrier, wherein the DNA carrier is the nucleic acid carrier of group (16) among the foregoing 26 backbone-sequence carriers; the PSMA aptamer is linked via the linker sequence AA to the 3' end of the sequence a; three AmiR-21 moieties are tandemly linked via linker sequences AA at the 3' end of the sequence b; the sense strand of survivin siRNA is linked via the linker sequence AAA to the 3' end of the sequence c; the AS1411 aptamer is connected with the antisense strand of survivin siRNA via the linker sequence TT, and is connected to the 3' end of the sequence c through complementary pairing of the antisense strand with the sense strand of survivin siRNA; in which the phosphate backbone between the first two nucleotides at the 5' end of the sequences a, b and c of the DNA carrier is independently phosphorothioate-modified;

preferably, the sequence of the AS1411 aptamer is SEQ ID No. 131: GGTGGTGGTGGTTGTGGTGGTGGTGG;

preferably, the sense strand of survivin siRNA is SEQ ID No. 100: GCAGGUUCCUUAUCUGUCACAUU, and the antisense strand of survivin siRNA is SEQ ID No. 300: UGUGACAGAUAAGGAACCUGCAG; the phosphate backbone between adjacent nucleotides within the first three positions at the 5' end and within the last three positions at the 3' end of both the sense and antisense strands of survivin siRNA is phosphorothioate-modified;

preferably, the sequence of the PSMA aptamer:
SEQ ID No. 234: GGGACCGAAAAAGACCUGACUUCUAUACUAAGUCUACGUUCCC, with a phosphorothioate modification in the phosphate backbone between the last two nucleotides at the 3' end;

preferably, the sequence of AmiR-21 is GAUAAGCU, with the phosphate backbone between adjacent nucleotides being phosphorothioate-modified.

Drug 32): 3*AmiR-21-ATP aptamer-FGF2 aptamer-DNA carrier, wherein the DNA carrier is the nucleic-acid carrier of group (15) among the foregoing 26 backbone-sequence carriers; three AmiR-21 moieties are directly linked in tandem to the 5' end of the sequence a; the ATP aptamer is linked via the linker sequence TTTTT to the 5' end of the sequence b; the FGF2 aptamer is linked via the linker sequence U to the 5' end of the sequence c; wherein the phosphate backbone between the first two nucleotides at the 3' end of the sequences a, b and c of the DNA carrier is independently phosphorothioate-modified;

preferably, the sequence of the ATP aptamer is SEQ ID No. 135: ACCTGGGGAGTATTGGGGAGGAAGG, with a phosphorothioate modification in the phosphate backbone between the first two nucleotides at the 5' end;

preferably, the sequence of the FGF2 aptamer:
SEQ ID No. 183: GGGAUACUAGGGCAUUAAUGUUACCAGUGUAGUCCC, with a phosphorothioate modification in the phosphate backbone between the first two nucleotides at the 5' end;

preferably, the sequence of AmiR-21 is GATAAGCT, with a phosphorothioate modification in the phosphate backbone between adjacent nucleotides.

Drug 33): AS1411 aptamer-survivin siRNA-PSMA aptamer-3*AmiR-21-DNA/RNA hybrid carrier, wherein the DNA/RNA hybrid carrier is the nucleic-acid carrier of group (18) among the foregoing 26 backbone-sequence carriers; the PSMA aptamer is linked via the linker sequence AA to the 3' end of the sequence a; three AmiR-21 moieties are linked in tandem via the linker sequence AA to the 3' end of the sequence b; the sense strand of survivin siRNA is linked via the linker sequence AAAA to the 3' end of the sequence c; the AS1411 aptamer is linked via the linker sequence AA to the 5' end of the antisense strand of survivin siRNA, and the antisense strand is connected to the sense strand of survivin siRNA by complementary pairing; wherein the phosphate backbone between the first two nucleotides at the 5' end of the sequences b and c of the DNA/RNA hybrid carrier is independently phosphorothioate-modified;

preferably, the sequence of the AS1411 aptamer is SEQ ID No. 131: GGTGGTGGTGGTTGTGGTGGTGGTGG, with a phosphorothioate modification in the phosphate backbone between the first two nucleotides at the 5' end;

preferably, the antisense strand of survivin siRNA is SEQ ID No. 300: UGUGACAGAUAAGGAACCUGCAG; and the sense strand is SEQ ID No. 100: GCAGGUUCCUUAUCUGUCACAUU; the phosphate backbone between adjacent nucleotides within the first three positions at the 5' end and within the last three positions at the 3' end of both the sense and antisense strands of survivin siRNA is phosphorothioate-modified;

preferably, the sequence of the PSMA aptamer:
SEQ ID No. 234: GGGACCGAAAAAGACCUGACUUCUAUACUAAGUCUACGUUCCC, in which C and U are 2'-fluoro (2'-F) modified and A and G are 2'-O-methoxyethyl (2'-OME) modified, and the phosphate backbone between the last two nucleotides at the 3' end are phosphorothioate-modified;

preferably, the sequence of AmiR-21 is GAUAAGCU, with a phosphorothioate modification in the phosphate backbone between adjacent nucleotides.

Drug 34): 3*AmiR-21-AS1411 aptamer-FAP aptamer-DNA carrier, wherein the RNA carrier is the nucleic-acid carrier of group (15) among the foregoing 26 backbone-sequence carriers; three AmiR-21 moieties are linked in tandem via the linker sequence TTTTTT to the 5' end of the sequence a; the AS1411 aptamer is linked via the linker sequence TTTTT to the 5' end of the sequence b; and the FAP aptamer is linked via the linker sequence TTTTT to the 5' end of the sequence c;

preferably, the sequence of AmiR-21 is GATAAGCT, with a phosphorothioate modification in the phosphate backbone between adjacent nucleotides;

preferably, the sequence of the AS1411 aptamer:
SEQ ID No. 131: GGTGGTGGTGGTTGTGGTGGTGGTGG, with a phosphorothioate modification in the phosphate backbone between the first two nucleotides at the 5' end;

preferably, the sequence of the FAP aptamer:
SEQ ID No. 188: CCGCTCGAGCTAGTCTGACAAAGAGAAACAC, with a phosphorothioate modification in the phosphate backbone between the first two nucleotides at the 5' end.

Drug 35): AS1411 aptamer-survivin siRNA-EpCAM aptamer-3*AmiR-21-DNA carrier, wherein the DNA carrier is the nucleic-acid carrier of group (14) among the foregoing 26 backbone-sequence carriers; the AS1411 aptamer is linked via the linker sequence AA to the 5' end of the antisense strand of survivin siRNA; the sense strand of survivin siRNA is linked via the linker sequence AAAA to the 3' end of the sequence c; the antisense strand of survivin siRNA is connected to the sense strand by complementary pairing; three AmiR-21 moieties are linked in tandem via the linker sequence AA to the 3' end of the sequence b; and the EpCAM aptamer is linked via the linker sequence AA to the 3' end of the sequence a; wherein the phosphate backbone between the first two nucleotides at the 5' end of the sequence b of the DNA carrier is independently phosphorothioate-modified;

preferably, the sequence of the AS1411 aptamer is SEQ ID No. 131: GGTGGTGGTGGTTGTGGTGGTGGTGG, with a phosphorothioate modification in the phosphate backbone between the first two nucleotides at the 5' end;

preferably, the antisense strand of survivin siRNA is SEQ ID No. 300: UGUGACAGAUAAGGAACCUGCAG, and the sense strand is SEQ ID No. 100: GCAGGUUCCUUAUCUGUCACAUU; the phosphate backbone between adjacent nucleotides within the first three positions at the 5' end and within the last three positions at the 3' end of both the sense and antisense strands of survivin siRNA is phosphorothioate-modified; the antisense strand of survivin siRNA is 2'-F modified at positions 2, 6, 8, 9, 12, 14 and 16, and 2'-OMe modified at the remaining positions; the sense strand of survivin siRNA is 2'-F modified at positions 7, 9, 10 and 11, and 2'-OMe modified at the remaining positions;

preferably, the sequence of AmiR-21 is GATAAGCT, with a phosphorothioate modification in the phosphate backbone between adjacent nucleotides;

preferably, the sequence of the EpCAM aptamer is SEQ ID No. 179: GCGACUGGUUACCCGGUCG, in which C and U are 2'-fluoro (2'-F) modified and A and G are 2'-O-methoxyethyl (2'-OME) modified; the phosphate backbone between adjacent nucleotides within the first three positions at the 5' end and within the last three positions at the 3' end is phosphorothioate-modified.

Drug 36): IL-4RA aptamer-TGF-β aptamer-PD-L1 aptamer-2*AmiR-21-DNA carrier, wherein the DNA carrier is the nucleic-acid carrier of group (15) among the foregoing 26 backbone-sequence carriers; the IL-4RA aptamer is linked via one AmiR-21 to the 5' end of the sequence a; the TGF-β aptamer is linked via the linker sequence TTTTT to the 5' end of the sequence b; the PD-L1 aptamer is linked via one AmiR-21 to the 5' end of the sequence c; wherein the phosphate backbone between the first two nucleotides at the 3' end of the sequences a, b and c of the DNA carrier is independently phosphorothioate-modified;

preferably, the sequence of the IL-4RA aptamer:
SEQ ID No. 203: AAAAAGCAACAGGGUGCUCCAUGCGCAUGGAACCUGCGCG, with a phosphorothioate modification in the phosphate backbone between adjacent nucleotides within the first four positions at the 5' end;

preferably, the sequence of the TGF-β aptamer:
SEQ ID No. 269: ACATTGCTGCGTGATCGCCTCACATGGGTTTGTCTGGTCGATTTGGAGGTGGTGGGTGGC, with a phosphorothioate modification in the phosphate backbone between adjacent nucleotides within the first four positions at the 5' end;

preferably, the sequence of the PD-L1 aptamer:
SEQ ID No. 241: ACGGGCCACATCAACTCATTGATAGACAATGCGTCCACTGCCCGT, with a phosphorothioate modification in the phosphate backbone between adjacent nucleotides within the first four positions at the 5' end;

preferably, the sequence of AmiR-21 is GATAAGCT, with a phosphorothioate modification in the phosphate backbone between adjacent nucleotides.

Drug 37): AS1411 aptamer-ATAD2 siRNA-GPC3 aptamer-5*AmiR-21-DNA carrier, wherein the DNA carrier is the nucleic-acid carrier of group (19) among the foregoing 26 backbone-sequence carriers; the AS1411 aptamer is linked via one AmiR-21 to the 5' end of the antisense strand of ATAD2 siRNA; the sense strand of ATAD2 siRNA is linked via one AmiR-21 to the 3' end of the sequence c; the antisense strand of ATAD2 siRNA is connected to the sense strand by complementary pairing; one AmiR-21 is linked to the 3' end of the sequence a; the GPC3 aptamer is linked via one AmiR-21 to the 5' end of the sequence b; and one AmiR-21 is linked to the 5' end of the sequence c; wherein the phosphate backbone between the first two nucleotides at the 5' end of the sequence b of the DNA carrier is independently phosphorothioate-modified;

preferably, the sequence of the AS1411 aptamer is SEQ ID No. 131:
GGTGGTGGTGGTTGTGGTGGTGGTGG, with a phosphorothioate modification in the phosphate backbone between the first two nucleotides at the 5' end;

preferably, the antisense strand of ATAD2 siRNA is SEQ ID No. 68: GCGUCGAAGUUGUAGGAUUUU, and the sense strand is SEQ ID No. 69: AAUCCUACAACUUCGACGCUU; both the sense and antisense strands of ATAD2 siRNA have phosphorothioate modifications in the phosphate backbone between adjacent nucleotides within the first three positions at the 5' end and within the last three positions at the 3' end, and are 2'-fluoro (2'-F) modified at positions 2, 6, 8, 9, 12, 14 and 16 from the 5' end, with the remaining positions modified with 2'-O-methoxyethyl (2'-OME);

The sequence of the GPC3 aptamer is SEQ ID No. 190: TAACGCTGACCTTAGCTGCATGGCTTTACATGTTCCA, with a phosphorothioate modification in the phosphate backbone between adjacent nucleotides within the first three positions at the 5' end;

preferably, the sequence of AmiR-21 is GATAAGCT, with a phosphorothioate modification in the phosphate backbone between adjacent nucleotides.

Drug 38): Vap7 aptamer-TGF-β aptamer-Act-12c aptamer-3*AmiR-21-DNA carrier, wherein the DNA carrier is the nucleic-acid carrier of group (15) among the foregoing 26 backbone-sequence carriers; the Vap7 aptamer is linked to the 5' end of the sequence a via two AmiR-21 moieties in tandem; the TGF-β aptamer is linked via the linker sequence TTTTT to the 5' end of the sequence b; the Act-12c aptamer is linked via one AmiR-21 to the 5' end of the sequence c;

wherein the phosphate backbone between the first two nucleotides at the 3' end of the sequences a, b and c of the DNA carrier is independently phosphorothioate-modified;

preferably, the sequence of the Vap7 aptamer is SEQ ID No. 277: TGGTGGGGGTGGACGGGCCGGGTAGA, with a phosphorothioate modification in the phosphate backbone between adjacent nucleotides within the first four positions at the 5' end;

preferably, the sequence of AmiR-21 is GATAAGCT, with a phosphorothioate modification in the phosphate backbone between adjacent nucleotides;

preferably, the sequence of the TGF-β aptamer:
SEQ ID No. 269: ACATTGCTGCGTGATCGCCTCACATGGGTTTGTCTGGTCGATTTGGAGGTGGTGGGTGGC, with a phosphorothioate modification in the phosphate backbone between adjacent nucleotides within the first four positions at the 5' end;

preferably, the sequence of the Act-12c aptamer:
SEQ ID No. 137: CGGGGAAAGTCACGGGGGGGTTTCAGATGTTCTGATCGGTGTGGAG, with a phosphorothioate modification in the phosphate backbone between adjacent nucleotides within the first four positions at the 5' end.

Drug 39): AS1411 aptamer-3*AmiR-21-IL-4RA aptamer-VCAM-1 aptamer-DNA carrier, wherein the DNA carrier is the nucleic-acid carrier of group (15) among the foregoing 26 backbone-sequence carriers; the AS1411 aptamer is linked sequentially via the linker sequence TTTTT and three tandem AmiR-21 moieties to the 5' end of the sequence a; the IL-4RA aptamer and the VCAM-1 aptamer are respectively linked via the linker sequence TTTTT to the 5' ends of the sequence b and the sequence c; wherein the phosphate backbone between the first two nucleotides at the 3' ends of the sequences a, b and c of the DNA carrier is independently phosphorothioate-modified;

preferably, the sequence of the AS1411 aptamer is SEQ ID No. 131: G*GTGGTGGTGGTTGTGGTGGTGGTGG, with a phosphorothioate modification in the phosphate backbone between the first two nucleotides at the 5' end;

preferably, the sequence of AmiR-21 is GATAAGCT, with a phosphorothioate modification in the phosphate backbone between adjacent nucleotides;

preferably, the sequence of the IL-4RA aptamer:
SEQ ID No. 203: AAAAAGCAACAGGGUGCUCCAUGCGCAUGGAACCUGCGCG, with a phosphorothioate modification in the phosphate backbone between the first two nucleotides at the 5' end, and in which C or U are 2'-fluoro (2'-F) modified and A or G are 2'-O-methoxyethyl (2'-OME) modified;

preferably, the sequence of the VCAM-1 aptamer:
SEQ ID No. 284: GGACACGGCAAAGGGGTATAGCCTACCGGACCGTGAACATGGAATGGTGTGCTGCGTGG, with a phosphorothioate modification in the phosphate backbone between the first two nucleotides at the 5' end.

Drug 40): GPC1 aptamer-AS1411 aptamer-EpCAM aptamer-2*AmiR-21-DNA carrier, wherein the DNA carrier is the nucleic-acid carrier of group (14) among the foregoing 26 backbone-sequence carriers; the GPC1 aptamer is linked via one AmiR-21 to the 5' end of the sequence a; the AS1411 aptamer is linked via the linker sequence TTTTT to the 5' end of the sequence b; the EpCAM aptamer is linked via one AmiR-21 to the 5' end of the sequence c; wherein the phosphate backbone between adjacent nucleotides within the first three positions at the 3' ends of the sequences a, b and c of the DNA carrier is independently phosphorothioate-modified, and the nucleotides at the first two positions at the 3' end are independently 2'-O-methoxyethyl (2'-OME) modified;

preferably, the sequence of the GPC1 aptamer is SEQ ID No. 189: AACGGAGTGTGGCTAACTCGA, with a phosphorothioate modification in the phosphate backbone between adjacent nucleotides within the first four positions at the 5' end;

preferably, the sequence of AmiR-21 is GATAAGCT, with a phosphorothioate modification in the phosphate backbone between adjacent nucleotides;

preferably, the sequence of the AS1411 aptamer:
SEQ ID No. 131: GGTGGTGGTGGTTGTGGTGGTGGTGG, in which the phosphate backbone between the nucleotides at positions 1-2, 3-4, 13-14, 16-17 and 24-25 from the 5' end is phosphorothioate-modified;

preferably, the sequence of the EpCAM aptamer is SEQ ID No. 179: GCGACUGGUUACCCGGUCG, with a phosphorothioate modification in the phosphate backbone between adjacent nucleotides within the first four positions at the 5' end, and in which C or U are 2'-fluoro (2'-F) modified and A or G are 2'-O-methoxyethyl (2'-OME) modified.

Drug 41): 3*AmiR-21-AS1411 aptamer-CD40 aptamer-2*biotin-DNA carrier, wherein the DNA carrier is the nucleic-acid carrier of group (1) among the foregoing 26 backbone-sequence carriers; three AmiR-21 moieties are respectively linked to the 5' end of the sequence a, the 3' end of the sequence a, and the 3' end of the sequence b; the AS1411 aptamer is linked via the linker sequence TTTTT to the 5' end of the sequence b; the CD40 aptamer is linked via the linker sequence TTTTT to the 5' end of the sequence c; one biotin is attached to the 3' end of the AmiR-21 that is at the 3' end of the sequence a, and another biotin is attached to the 3' end of the sequence c;

preferably, the sequence of AmiR-21 is GATAAGCT, in which each nucleotide is locked nucleic acid (LNA) modified;

preferably, the sequence of the AS1411 aptamer is SEQ ID No. 131: GGTGGTGGTGGTTGTGGTGGTGGTGG;

preferably, the sequence of the CD40 aptamer is SEQ ID No. 154: CCAACGAGTAGGCGATAGCGCGTGG.

Drug 42): IL-4RA aptamer-VCAM-12d aptamer-PD-L1 aptamer-3*AmiR-21-DNA carrier, wherein the DNA carrier is the nucleic-acid carrier of group (3) among the foregoing 26 backbone-sequence carriers; the IL-4RA aptamer is linked via the first AmiR-21 to the 5' end of the sequence a; the VCAM-12d aptamer is linked via the second AmiR-21 to the 5' end of the sequence b; the PD-L1 aptamer is linked via the third AmiR-21 to the 5' end of the sequence c; wherein the phosphate backbone between the first two nucleotides at the 3' ends of the sequences a, b and c of the DNA carrier is independently phosphorothioate-modified;

preferably, the sequence of the IL-4RA aptamer:
SEQ ID No. 203: AAAAAGCAACAGGGUGCUCCAUGCGCAUGGAACCUGCGCG, with a phosphorothioate modification in the phosphate backbone between adjacent nucleotides within the first four positions at the 5' end, and in which C or U are 2'-fluoro (2'-F) modified and A or G are 2'-O-methoxyethyl (2'-OME) modified;

preferably, the sequence of the VCAM-12d aptamer:
SEQ ID No. 285: AGGGAATCTTGCCTAGGGAGGGAGTAGCGAAAGGGCTCA, with a phosphorothioate modification in the phosphate backbone between adjacent nucleotides within the first four positions at the 5' end;

preferably, the sequence of the PD-L1 aptamer:
SEQ ID No. 241: ACGGGCCACATCAACTCATTGATAGACAATGCGTCCACTGCCCGT, with a phosphorothioate modification in the phosphate backbone between adjacent nucleotides within the first four positions at the 5' end;

preferably, the sequence of AmiR-21 is GATAAGCT, with a phosphorothioate modification in the phosphate backbone between adjacent nucleotides.

Drug 43): TAPsiRNA-3*AmiR-21-2*AS1411 aptamer-DNA carrier, wherein the DNA carrier is the nucleic-acid carrier of group (6) among the foregoing 26 backbone-sequence carriers; the sense strand of TAPsiRNA is linked via the linker sequence AAAA to the 3' end of the sequence c; one AS1411 aptamer is linked via the linker sequence AAAA to the antisense strand of TAPsiRNA and, through complementary pairing between the antisense and sense strands, thereby connects the AS1411 aptamer to the 3' end of the sequence c; another AS1411 aptamer is linked via the linker sequence AAAA to the 3' end of the sequence a; three AmiR-21 moieties are linked in tandem via the linker sequence AA to the 3' end of the sequence b; the phosphate backbone between the first two nucleotides at the 5' ends of the sequences a, b and c of the DNA carrier is independently phosphorothioate-modified;

preferably, the sense strand of TAPsiRNA is SEQ ID No. 301: GCUGCACACGGUUCAGAAU, and the antisense strand is SEQ ID No. 109: AUUCUGAACCGUGUGCAGCUU; the phosphate backbone between adjacent nucleotides within the first three positions at the 5' end and within the last three positions at the 3' end of both the sense and antisense strands of TAPsiRNA is phosphorothioate-modified; the antisense strand of TAPsiRNA is 2'-F modified at positions 2, 6, 8, 9, 12, 14 and 16 from the 5' end, with the remaining positions bearing 2'-OME modifications, and the sense strand is 2'-F modified at positions 7, 9, 10 and 11 from the 5' end, with the remaining positions modified with 2'-OME;

preferably, the sequence of AmiR-21 is GATAAGCT, with a phosphorothioate modification in the phosphate backbone between adjacent nucleotides;

preferably, the sequence of AS 1411-1 :
SEQ ID No. 131: GGTGGTGGTGGTTGTGGTGGTGGTGG, with a phosphorothioate modification in the phosphate backbone between the first two nucleotides at the 5' end.

Drug 44): PD-1 aptamer-AS1411 aptamer-PD-L1 aptamer-3*AmiR-21-DNA carrier, wherein the DNA carrier is the nucleic-acid carrier of group (3) among the foregoing 26 backbone-sequence carriers; the PD-1 aptamer is linked via one AmiR-21 to the 5' end of the sequence a; the AS1411 aptamer is linked via one AmiR-21 to the 5' end of the sequence b; the PD-L1 aptamer is linked via one AmiR-21 to the 5' end of the sequence c; wherein the phosphate backbone between the first two nucleotides at the 3' ends of the sequences a, b and c of the DNA carrier is independently phosphorothioate-modified;

preferably, the sequence of the PD-1 aptamer is SEQ ID No. 253: AGCGGTGACGGCACAGACGGTACAGTTCC CGTCCCTGCACTACACGTATGCCGCT, with a phosphorothioate modification in the phosphate backbone between adjacent nucleotides within the first four positions at the 5' end;

preferably, the sequence of the AS1411 aptamer is SEQ ID No. 131: GGTGGTGGTGGTTGTGGTGGTGGTGG, with a phosphorothioate modification in the phosphate backbone between the first two nucleotides at the 5' end;

preferably, the sequence of the PD-L1 aptamer is SEQ ID No. 241: ACGGGCCACATCAACTCATTGATAGACAA TGCGTCCACTGCCCGT, with a phosphorothioate modification in the phosphate backbone between adjacent nucleotides within the first four positions at the 5' end;

preferably, the sequence of AmiR-21 is GATAAGCT, with a phosphorothioate modification in the phosphate backbone between adjacent nucleotides.

Drug 45): CPG1826-CD40 aptamer-AmiR-21-3*biotin-DNA carrier, wherein the DNA carrier is the nucleic-acid carrier of group (13) among the foregoing 26 backbone-sequence carriers; CPG1826 is linked via a linker sequence to the 5' end of the sequence a; the CD40 aptamer is linked via a linker sequence to the 5' end of the sequence b; AmiR-21 is linked via a linker sequence to the 5' end of the sequence c; and three biotin groups are respectively linked to the 3' ends of the sequences a, b and c;

preferably, the sequence of CPG1826 is SEQ ID No. 59: TCCATGACGTTCCTGACG, with a phosphorothioate modification in the phosphate backbone between adjacent nucleotides;

preferably, the linker sequence is TTTTT;

preferably, the sequence of the CD40 aptamer is SEQ ID No. 154: CCAACGAGTAGGCGATAGCGCGTGG;

preferably, the sequence of AmiR-21 is GATAAGCT, in which each nucleotide is locked nucleic acid (LNA) modified.

Drug 46): ATP aptamer-AmiR-21-FGF2 aptamer-AmiR-1306-DNA carrier, wherein, in the DNA carrier, the sequence a is SEQ ID No. 18: GCGACGCCCACGAGCGTTCCGGGAGAGGAGC; the sequence b is SEQ ID No. 19: GCTCCTCTCCCGGTTCGCCGCGAGCCGCG; and the sequence c is SEQ ID No. 31: CGCGGCTCGCGGCCATAGCCGTGGGCGTCGC; the ATP aptamer is linked via the linker sequence TTTTT to the 5' end of the sequence a; the FGF2 aptamer is linked via the linker sequence U to the 5' end of the sequence b; and AmiR-1306 and AmiR-21 are sequentially linked, in that order, to the 5' end of the sequence c;

preferably, the sequence of the ATP aptamer is SEQ ID No. 136: GGGAGGACGATGCGGAGGAAGGGTAGG, with a phosphorothioate modification in the phosphate backbone between adjacent nucleotides within the first four positions at the 5' end;

The sequence of the FGF2 aptamer is SEQ ID No. 183: GGGAUACUAGGGCAUUAAUGUUACCAGUGUAGUCCC, in which C and U are 2'-fluoro (2'-F) modified;

preferably, the sequence of AmiR-1306 is SEQ ID No. 127: CATCACCACCAGAGCCAACGTC, with a phosphorothioate modification in the phosphate backbone between adjacent nucleotides within the first five positions at the 5' end;

The sequence of AmiR-21 is GATAAGCT, in which each nucleotide is locked nucleic acid (LNA) modified.

Drug 47): CD16a aptamer-CTLA-4 aptamer-PD-L1 aptamer-DNA carrier, wherein the DNA carrier is the nucleic-acid carrier of group (9) among the foregoing 26 backbone-sequence carriers; the CD16a aptamer is linked via a linker sequence to the 5' end of the sequence a; the CTLA-4 aptamer is linked via a linker sequence to the 5' end of the sequence b; and the PD-L1 aptamer is linked to the 5' end of the sequence c; wherein the phosphate backbone between the first two nucleotides at the 3' end of the sequences a, b and c of the DNA carrier is independently phosphorothioate-modified;

preferably, the sequence of the CD16a aptamer:
SEQ ID No. 157: CCACTGCGGGGGGTCTATACGTGAGGAAGAAGTGG, with a phosphorothioate modification in the phosphate backbone between the first two nucleotides at the 5' end;

preferably, the sequence of the CTLA-4 aptamer:
SEQ ID No. 141: GGUGGAAGAGGGAUGGGCCGACGUGCCGCAU, with a phosphorothioate modification in the phosphate backbone between the first two nucleotides at the 5' end, and in which C or U are 2'-fluoro (2'-F) modified and A or G are 2'-O-methoxyethyl (2'-OME) modified;

preferably, the sequence of the PD-L1 aptamer:
SEQ ID No. 241: ACGGGCCACATCAACTCATTGATAGACAATGCGTCCACTGCCCGT, with a phosphorothioate modification in the phosphate backbone between the first two nucleotides at the 5' end.

Drug 48): CD16a aptamer-Act-12c aptamer-PD-L1 aptamer-DNA carrier, wherein the DNA carrier is the nucleic-acid carrier of group 9 among the foregoing 26 backbone-sequence carriers; the CD16a aptamer is linked via a linker sequence to the 5' end of the sequence a; the Act-12c aptamer is linked via a linker sequence to the 5' end of the sequence b; the PD-L1 aptamer is linked to the 5' end of the sequence c; wherein the phosphate backbone between the first two nucleotides at the 3' ends of the sequences a, b and c of the DNA carrier is independently phosphorothioate-modified;

preferably, the sequence of the CD16a aptamer:
SEQ ID No. 157: CCACTGCGGGGGGTCTATACGTGAGGAAGAAGTGG, with a phosphorothioate modification in the phosphate backbone between the first two nucleotides at the 5' end;

preferably, the sequence of the Act-12c aptamer:
SEQ ID No. 137: CGGGGAAAGTCACGGGGGGGTTTCAGATGTTCTGATCGGTGTGGAG, with a phosphorothioate modification in the phosphate backbone between the first two nucleotides at the 5' end;

preferably, the sequence of the PD-L1 aptamer:
SEQ ID No. 241: ACGGGCCACATCAACTCATTGATAGACAATGCGTCCACTGCCCGT, with a phosphorothioate modification in the phosphate backbone between the first two nucleotides at the 5' end.

Drug 49): CTLA-4 aptamer-PD-L1 aptamer-PD-1 aptamer-DNA carrier, wherein the DNA carrier is the nucleic-acid carrier of group (5) among the foregoing 26 backbone-sequence carriers;

The PD-L1 aptamer is connected in sequence with a linker sequence and a complementary sequence A' of single-stranded bridging sequence A, the single-stranded bridging sequence A being located at the 3' end of the sequence a, and the PD-L1 aptamer is connected to the 3' end of the sequence a through complementary pairing between the complementary sequence A' and the single-stranded bridging sequence A;

The PD-1 aptamer is connected in sequence with a linker sequence and a complementary sequence B' of single-stranded bridging sequence B, the single-stranded bridging sequence B being located at the 3' end of the sequence b, and the PD-1 aptamer is connected to the 3' end of the sequence b through complementary pairing between the complementary sequence B' and the single-stranded bridging sequence B;

The CTLA-4 aptamer is connected with a complementary sequence C' of single-stranded bridging sequence C, the single-stranded bridging sequence C being located at the 3' end of the sequence c, and the CTLA-4 aptamer is connected to the 3' end of the sequence c through complementary pairing between the complementary sequence C' and the single-stranded bridging sequence C;

preferably, the sequence of the PD-L1 aptamer:
SEQ ID No. 241: ACGGGCCACATCAACTCATTGATAGACAATGCGTCCACTGCCCGT, with a phosphorothioate modification in the phosphate backbone between adjacent nucleotides within the first three positions at the 5' end;

preferably, the sequence of the PD-1 aptamer:
SEQ ID No. 254: ACCGACAGTGAAGGACTCAGCGAACTCTCAGACTCGGTTC, with a phosphorothioate modification in the phosphate backbone between adjacent nucleotides within the first three positions at the 5' end;

preferably, the sequence of the CTLA-4 aptamer:
SEQ ID No. 141: GGUGGAAGAGGGAUGGGCCGACGUGCCGCAU, with a phosphorothioate modification in the phosphate backbone between adjacent nucleotides within the first three positions at the 5' end, and the ribose of the first two nucleotides at the 5' end are 2'-O-methoxyethyl (2'-OME) modified, while the remaining C or U nucleotides are 2'-fluoro (2'-F) modified;

preferably, the single-stranded bridging sequence A is SEQ ID No. 7: TGTAGCACGGTGGC, and the complementary sequence A' is SEQ ID No. 8: GCCACCGTGCTACA;

preferably, the single-stranded bridging sequence B is SEQ ID No. 7: TGTAGCACGGTGGC, and the complementary sequence B' is SEQ ID No. 8: GCCACCGTGCTACA;

preferably, the single-stranded bridging sequence C is SEQ ID No. 11: TGCTGCTGCTGCTG, and the complementary sequence C' is SEQ ID No. 12: CAGCAGCAGCAGCA.

Drug 50): PD-1 aptamer-IL-4Ra aptamer-OX40 aptamer-DNA carrier, wherein the DNA carrier is the nucleic-acid carrier of group (6) among the foregoing 26 backbone-sequence carriers; the PD-1 aptamer is linked via the linker sequence AAA to the 5' end of the sequence a; the IL-4Ra aptamer is linked via the linker sequence AAA to the 5' end of the sequence b; the OX40 aptamer is linked via the linker sequence AAA to the 5' end of the sequence c; wherein the phosphate backbone between adjacent nucleotides within the first three positions at the 3' ends of the sequences a, b and c of the DNA carrier is independently phosphorothioate-modified;

preferably, the sequence of the PD-1 aptamer:
SEQ ID No. 253: AGCGGTGACGGCACAGACGGTACAGTTCCCGTCCCTGCACTACACGTATGCCGCT, with a phosphorothioate modification in the phosphate backbone between adjacent nucleotides within the first four positions at the 5' end;

preferably, the sequence of the IL-4Ra aptamer:
SEQ ID No. 203: AAAAAGCAACAGGGUGCUCCAUGCGCAUGGAACCUGCGCG, with a phosphorothioate modification in the phosphate backbone between adjacent nucleotides within the first four positions at the 5' end;

preferably, the sequence of the OX40 aptamer:
SEQ ID No. 229: CAGUCUGCAUCGUAGGAAUCGCCACCGUAUACUUUCCCAC, with a phosphorothioate modification in the phosphate backbone between adjacent nucleotides within the first four positions at the 5' end, and with 2'-O-methoxyethyl (2'-OME) modifications on the ribose of the first three nucleotides at the 5' end, the remaining C or U are 2'-fluoro (2'-F) modified and A or G are 2'-OME modified.

Drug 51): CD16a aptamer-OX40 aptamer-PD-L1 aptamer-DNA carrier, wherein the DNA carrier is the nucleic-acid carrier of group 3 among the foregoing 26 backbone-sequence carriers; the CD16a aptamer is linked via a linker sequence to the 5' end of the sequence a; the OX40 aptamer is linked via a linker sequence to the 5' end of the sequence b; the PD-L1 aptamer is linked via a linker sequence to the 5' end of the sequence c; wherein the phosphate backbone between the first two nucleotides at the 3' ends of the sequences a, b and c of the DNA carrier is independently phosphorothioate-modified;

preferably, the sequence of the CD16a aptamer:
SEQ ID No. 157: CCACTGCGGGGGGTCTATACGTGAGGAAGAAGTGG, with a phosphorothioate modification in the phosphate backbone between the first two nucleotides at the 5' end;

preferably, the sequence of the OX40 aptamer:
SEQ ID No. 230: GGGAUGCGGAAAAAAGAACACUUCCGAUUAGGGCCCACCCUAACGGCCGCAGAC, in which C or U are 2'-fluoro (2'-F) modified and A or G are 2'-O-methoxyethyl (2'-OME) modified, and with a phosphorothioate modification in the phosphate backbone between the first two nucleotides at the 5' end;

preferably, the sequence of the PD-L1 aptamer:
SEQ ID No. 241: ACGGGCCACATCAACTCATTGATAGACAATGCGTCCACTGCCCGT, with a phosphorothioate modification in the phosphate backbone between the first two nucleotides at the 5' end.

Drug 52): CD16a aptamer-VEGF165 aptamer-PD-L1 aptamer-DNA carrier, wherein the DNA carrier is the nucleic-acid carrier of group (3) among the foregoing 26 backbone-sequence carriers; the CD16a aptamer is linked via a linker sequence to the 5' end of the sequence a; the VEGF165 aptamer is linked via a linker sequence to the 5' end of the sequence b; the PD-L1 aptamer is linked via a linker sequence to the 5' end of the sequence c; wherein the phosphate backbone between the first two nucleotides at the 3' ends of the sequences a, b and c of the DNA carrier is independently phosphorothioate-modified;

preferably, the sequence of the CD16a aptamer:
SEQ ID No. 157: CCACTGCGGGGGGTCTATACGTGAGGAAGAAGTGG, with a phosphorothioate modification in the phosphate backbone between the first two nucleotides at the 5' end;

preferably, the sequence of the VEGF165 aptamer:
SEQ ID No. 280: CGGAAUCAGUGAAUGCUUA UACAUCCG, in which C or U are 2'-fluoro (2'-F) modified and A or G are 2'-O-methoxyethyl (2'-OME) modified, and with a phosphorothioate modification in the phosphate backbone between the first two nucleotides at the 5' end;

preferably, the sequence of the PD-L1 aptamer:
SEQ ID No. 241: ACGGGCCACATCAACTCATTGATAGACAATGCGTCCACTGCCCGT, with a phosphorothioate modification in the phosphate backbone between the first two nucleotides at the 5' end.

Drug 53): CD16a aptamer-CTLA-4 aptamer-TIM3 aptamer-DNA carrier, wherein the DNA carrier is the nucleic-acid carrier of group (3) among the foregoing 26 backbone-sequence carriers; the CD16a aptamer is linked via a linker sequence to the 5' end of the sequence a; the CTLA-4 aptamer is linked via a linker sequence to the 5' end of the sequence b; the TIM3 aptamer is linked via a linker sequence to the 5' end of the sequence c; wherein the phosphate backbone between the first two nucleotides at the 3' ends of the sequences a, b and c of the DNA carrier is independently phosphorothioate-modified;

preferably, the sequence of the CD16a aptamer:
SEQ ID No. 157: CCACTGCGGGGGGTCTATACGTGAGGAAGAAGTGG, with a phosphorothioate modification in the phosphate backbone between the first two nucleotides at the 5' end;

preferably, the sequence of the CTLA-4 aptamer:
SEQ ID No. 141: GGUGGAAGAGGGAUGGGCCGACGUGCCGCAU, with a phosphorothioate modification in the phosphate backbone between the first two nucleotides at the 5' end, in which C or U are 2'-fluoro (2'-F) modified and A or G are 2'-O-methoxyethyl (2'-OME) modified;

preferably, the sequence of the TIM3 aptamer:
SEQ ID No. 270: GGGAGAGGACCAGUAGCCACUAUGGUGUUGGAGCUAGCGGCAGAGCGUCGCGGUCCC UCCC, with a phosphorothioate modification in the phosphate backbone between the first two nucleotides at the 5' end, in which C or U are 2'-fluoro (2'-F) modified and A or G are 2'-O-methoxyethyl (2'-OME) modified.

Drug 54): PD-1 aptamer-CTLA-4 aptamer-LAG-3 aptamer-DNA carrier, wherein the DNA carrier is the nucleic-acid carrier of group (3) among the foregoing 26 backbone-sequence carriers; the PD-1 aptamer is linked via a linker sequence to the 5' end of the sequence a; the CTLA-4 aptamer is linked via a linker sequence to the 5' end of the sequence b; the LAG-3 aptamer is linked via a linker sequence to the 5' end of the sequence c; wherein the phosphate backbone between the first two nucleotides at the 3' ends of the sequences a, b and c of the DNA carrier is independently phosphorothioate-modified;

preferably, the sequence of the PD-1 aptamer:

SEQ ID No. 253: AGCGGTGACGGCACAGACGGTACAGTTCCCGTCCCTGCACTACACGTATGCCGCT, with a phosphorothioate modification in the phosphate backbone between the first two nucleotides at the 5' end;

preferably, the sequence of the CTLA-4 aptamer:
SEQ ID No. 141: GGUGGAAGAGGGAUGGGCCGACGUGCCGCAU, with a phosphorothioate modification in the phosphate backbone between the first two nucleotides at the 5' end, in which C or U are 2'-fluoro (2'-F) modified and A or G are 2'-O-methoxyethyl (2'-OME) modified;

preferably, the sequence of the LAG-3 aptamer:
SEQ ID No. 206:

GGGAGAGAGAUAUAAGGGCCUCCUGAUACCCGCUGCUAUCUGGACCGAUCCCAUUAC   CAAAUUCUCUCCC, with a phosphorothioate modification in the phosphate backbone between the first two nucleotides at the 5' end, in which C or U are 2'-fluoro (2'-F) modified and A or G are 2'-O-methoxyethyl (2'-OME) modified.

Drug 55): CD16a aptamer-Act-12c aptamer-MUC1 aptamer-DNA carrier, wherein the DNA carrier is the nucleic-acid carrier of group (3) among the foregoing 26 backbone-sequence carriers; the CD16a aptamer is linked via a linker sequence to the 5' end of the sequence a; the Act-12c aptamer is linked via a linker sequence to the 5' end of the sequence b; the MUC1 aptamer is linked via a linker sequence to the 5' end of the sequence c; wherein the phosphate backbone between the first two nucleotides at the 3' ends of the sequences a, b and c of the DNA carrier is independently phosphorothioate-modified;

preferably, the sequence of the CD16a aptamer:
SEQ ID No. 157: CCACTGCGGGGGGTCTATACGTGAGGAAGAAGTGG, with a phosphorothioate modification in the phosphate backbone between the first two nucleotides at the 5' end;

preferably, the sequence of the Act-12c aptamer:
SEQ ID No. 137: CGGGGAAAGTCACGGGGGGGTTTCAGATGTTCTGATCGGTGTGGAG, with a phosphorothioate modification in the phosphate backbone between the first two nucleotides at the 5' end;

preferably, the sequence of the MUC1 aptamer is SEQ ID No. 209: GAAGTGAAAATGACAGAACACAACA.

Drug 56): CD16a aptamer-TGF-β aptamer-PD-L1 aptamer-DNA carrier, wherein the DNA carrier is the nucleic-acid carrier of group (3) among the foregoing 26 backbone-sequence carriers; the CD16a aptamer is linked via a linker sequence to the 5' end of the sequence a; the TGF-β aptamer is linked via a linker sequence to the 5' end of the sequence b; the PD-L1 aptamer is linked via a linker sequence to the 5' end of the sequence a; wherein the phosphate backbone between the first two nucleotides at the 3' ends of the sequences a, b and c of the DNA carrier is independently phosphorothioate-modified;

preferably, the sequence of the CD16a aptamer:
SEQ ID No. 157: CCACTGCGGGGGGTCTATACGTGAGGAAGAAGTGG, with a phosphorothioate modification in the phosphate backbone between the first two nucleotides at the 5' end;

preferably, the sequence of the TGF-β aptamer:
SEQ ID No. 269: ACATTGCTGCGTGATCGCCTCACATGGGTTTGTCTGGTCGATTTGGAGGTGGTGGGTGGC, with a phosphorothioate modification in the phosphate backbone between the first two nucleotides at the 5' end;

preferably, the sequence of the PD-L1 aptamer:
SEQ ID No. 241: ACGGGCCACATCAACTCATTGATAGACAATGCGTCCACTGCCCGT, with a phosphorothioate modification in the phosphate backbone between the first two nucleotides at the 5' end.

Drug 57): CD16a aptamer-CD40 aptamer-CTLA-4 aptamer-DNA carrier, wherein the DNA carrier is the nucleic-acid carrier of group (3) among the foregoing 26 backbone-sequence carriers; the CD16a aptamer is linked via a linker sequence to the 5' end of the sequence a; the CD40 aptamer is linked via a linker sequence to the 5' end of the sequence b; the CTLA-4 aptamer is linked via a linker sequence to the 5' end of the sequence c; wherein the phosphate backbone between the first two nucleotides at the 3' ends of the sequences a, b and c of the DNA carrier is independently phosphorothioate-modified;

preferably, the sequence of the CD16a aptamer:

SEQ ID No. 157: CCACTGCGGGGGTCTATACGTGAGGAAGAAGTGG, with a phosphorothioate modification in the phosphate backbone between the first two nucleotides at the 5' end;

preferably, the sequence of the CD40 aptamer:
SEQ ID No. 154: CCAACGAGTAGGCGATAGCGCGTGG, with a phosphorothioate modification in the phosphate backbone between the first two nucleotides at the 5' end;

preferably, the sequence of the CTLA-4 aptamer:
SEQ ID No. 141: GGUGGAAGAGGGAUGGGCCGACGUGCCGCAU, with a phosphorothioate modification in the phosphate backbone between the first two nucleotides at the 5' end, in which C or U are 2'-fluoro (2'-F) modified and A or G are 2'-O-methoxyethyl (2'-OME) modified.

Drug 58): HER2 aptamer-HER3 aptamer-CD40 aptamer-CD16a aptamer-DNA carrier, wherein the DNA carrier is the nucleic-acid carrier of group (7) among the foregoing 26 backbone-sequence carriers; the HER3 aptamer is linked via the linker sequence AA to the 5' end of the sequence a; the HER2 aptamer is linked via the linker sequence AA to the 5' end of the sequence c; the CD40 aptamer and the CD16a aptamer are respectively linked via the linker sequence AA to the 5' end and the 3' end of the sequence b; wherein the phosphate backbone between the first two nucleotides at the 3' ends of the sequences a and c of the DNA carrier is independently phosphorothioate-modified;

preferably, the sequence of the HER2 aptamer:
SEQ ID No. 194: AGCCGCGAGGGGAGGGAUAGGGUAGGGCGCGGCU, with a phosphorothioate modification in the phosphate backbone between the first two nucleotides at the 5' end, and in which C or U are 2'-fluoro (2'-F) modified and A or G are 2'-O-methoxyethyl (2'-OME) modified;

preferably, the sequence of the HER3 aptamer:
SEQ ID No. 197: CAGCGAAAGUUGCGUAUGGGUCACAUCGCAGGCACAUGUCAUCUGGGCG, with a phosphorothioate modification in the phosphate backbone between the first two nucleotides at the 5' end, and in which C or U are 2'-fluoro (2'-F) modified and A or G are 2'-O-methoxyethyl (2'-OME) modified;

preferably, the sequence of the CD40 aptamer:
SEQ ID No. 154: CCAACGAGTAGGCGATAGCGCGTGG, with a phosphorothioate modification in the phosphate backbone between the first two nucleotides at the 5' end;

preferably, the sequence of the CD16a aptamer:
SEQ ID No. 157: CCACTGCGGGGGTCTATACGTGAGGAAGAAGTGG, with a phosphorothioate modification in the phosphate backbone between the first two nucleotides at the 5' end.

Drug 59): CD16a aptamer-Act-12c aptamer-CTLA-4 aptamer-GPC1 aptamer-DNA carrier, wherein the DNA carrier is the nucleic-acid carrier of group (10) among the foregoing 26 backbone-sequence carriers; the CD16a aptamer is linked via the linker sequence TTTTT to the 5' end of the sequence a; the Act-12c aptamer is linked via the linker sequence TTTTT to the 5' end of the sequence b; the CTLA-4 aptamer and the GPC1 aptamer are respectively linked via the linker sequence TTT to the 5' end and the 3' end of the sequence c; wherein the phosphate backbone between the first two nucleotides at the 3' ends of the sequences a and b of the DNA carrier is independently phosphorothioate-modified;

preferably, the sequence of the CD16a aptamer:
SEQ ID No. 157: CCACTGCGGGGGTCTATACGTGAGGAAGAAGTGG, with a phosphorothioate modification in the phosphate backbone between the first two nucleotides at the 5' end;

preferably, the sequence of the Act-12c aptamer:
SEQ ID No. 137: CGGGGAAAGTCACGGGGGGTTTCAGATGTTCTGATCGGTGTGGAG, with a phosphorothioate modification in the phosphate backbone between the first two nucleotides at the 5' end;

preferably, the sequence of the CTLA-4 aptamer:
SEQ ID No. 141: GGUGGAAGAGGGAUGGGCCGACGUGCCGCAU, with a phosphorothioate modification in the phosphate backbone between the first two nucleotides at the 5' end, and in which C or U are 2'-fluoro (2'-F) modified and A or G are 2'-O-methoxyethyl (2'-OME) modified;

preferably, the sequence of the GPC1 aptamer:

SEQ ID No. 189: AACGGAGTGTGGCTAACTCGA, with a phosphorothioate modification in the phosphate backbone between the last two nucleotides at the 3' end.

Drug 60): CD16a aptamer-HER3 aptamer-VEGF165 aptamer-DNA carrier, wherein the DNA carrier is the nucleic acid carrier of the aforesaid twenty-six backbone sequences in group 3; the CD16a aptamer is connected to the 5' end of the sequence a via the linker sequence TTTTT; the HER3 aptamer is connected to the 5' end of the sequence b via the linker sequence TTTTT; the VEGF165 aptamer is connected to the 5' end of the sequence c via the linker sequence TTTTT; and the phosphate backbone between the first two nucleotides at the 3' ends of the sequences a, b and c of the DNA carrier independently are phosphorothioate modified;

preferably, the sequence of the CD16a aptamer:
SEQ ID No. 157: CCACTGCGGGGGGTCTATACGTGAGGAAGAAGTGG, with a phosphorothioate modification in the phosphate backbone between the first two nucleotides at the 5' end;

preferably, the sequence of the HER3 aptamer:
SEQ ID No. 197: CAGCGAAAGUUGCGUAUGGGUCACAUCGCAGGCACAUGUCAUCUGGGCG, with a phosphorothioate modification in the phosphate backbone between the first two nucleotides at the 5' end; C or U are a 2'-F modified, and A or G are a 2'-OME modified;

preferably, the sequence of the VEGF165 aptamer:
SEQ ID No. 280: CGGAAUCAGUGAAUGCUUAUACAUCCG, with a phosphorothioate modification in the phosphate backbone between the first two nucleotides at the 5' end; C or U are a 2'-F modified, and A or G are a 2'-OME modified.

Drug 61): VEGF aptamer-GPC1 aptamer-PD-L1 aptamer-CD16a aptamer-DNA carrier, wherein the DNA carrier is the nucleic acid carrier of the aforesaid twenty-six backbone sequence sets of group 11; the VEGF aptamer and the GPC1 aptamer are respectively connected to the 5' end and the 3' end of the sequence a via the linker sequence TTTTT; the PD-L1 aptamer and the CD16a aptamer are respectively connected to the 5' ends of the sequence b and the sequence c via the linker sequence TTTTT; and the phosphate backbone between the first two nucleotides at the 3' ends of the sequence b and the sequence c of the DNA carrier each independently with a phosphorothioate modification;

preferably, the sequence of the VEGF aptamer:
SEQ ID No. 279: GGTGGGGGTGGACGGGCCGGGTAGA, with a phosphorothioate modification in the phosphate backbone between the first two nucleotides at the 5' end;

preferably, the sequence of the GPC1 aptamer:
SEQ ID No. 189: AACGGAGTGTGGCTAACTCG*A, with a phosphorothioate modification in the phosphate backbone between the last two nucleotides at the 3' end;

preferably, the sequence of the PD-L1 aptamer:
SEQ ID No. 241: ACGGGCCACATCAACTCATTGATAGACAATGCGTCCACTGCCCGT, with a phosphorothioate modification in the phosphate backbone between the first two nucleotides at the 5' end;

preferably, the sequence of the CD16a aptamer:
SEQ ID No. 157: CCACTGCGGGGGGTCTATACGTGAGGAAGAAGTGG, with a phosphorothioate modification in the phosphate backbone between the first two nucleotides at the 5' end.

Drug 62): PD-1 aptamer-PD-L1 aptamer-2*AmiR21Act-12c aptamer-DNA carrier, wherein the DNA carrier is the nucleic acid carrier of the aforesaid twenty-six backbone sequence sets of group 3; the PD-1 aptamer is connected to the 5' end of the sequence a via one AmiR21; the PD-L1 aptamer is connected to the 5' end of the sequence b via the other AmiR21; the Act-12c aptamer is connected to the 5' end of the sequence c via the linker sequence TTTTT; and the phosphate backbone between the first two nucleotides at the 3' ends of the sequences a, b and c of the DNA carrier each independently with a phosphorothioate modification;

preferably, the sequence of the PD-1 aptamer:
SEQ ID No. 253: AGCGGTGACGGCACAGACGGTACAGTTCCCGTCCCTGCACTACACGTATGCCGCT, with phosphorothioate modifications in the phosphate backbone between each pair of adjacent nucleotides among the first four nucleotides at the 5' end;

preferably, the sequence of the PD-L1 aptamer:

SEQ ID No. 241: ACGGGCCACATCAACTCATTGATAGACAATGCGTCCACTGCCCGT, with phosphorothioate modifications in the phosphate backbone between each pair of adjacent nucleotides among the first four nucleotides at the 5' end;

preferably, the sequence of the Act-12c aptamer:

SEQ ID No. 137: CGGGGAAAGTCACGGGGGGTTTCAGATGTTCTGATCGGTGTGGAG, with phosphorothioate modifications in the phosphate backbone between each pair of adjacent nucleotides among the first four nucleotides at the 5' end;

preferably, the sequence of AmiR21 is GATAAGCT, with phosphorothioate modifications in the phosphate backbone between adjacent nucleotides.

Drug 63): HER3 aptamer-EGFR siRNA-AS1411 aptamer-Survivin siRNA-HER2 aptamer-DNA carrier, wherein the DNA carrier is the nucleic acid carrier of the aforesaid twenty-six backbone sequence sets of group 14; the AS 1411 aptamer is connected to the 3' end of the sequence a via the linker sequence AAAA; the HER2 aptamer is connected to the 3' end of the sequence b via the linker sequence AAAA; the sense strand of EGFR siRNA is connected to the 3' end of the sequence c via the linker sequence AAAA; the sense strand of Survivin siRNA is connected to the 3' end of the sequence b via the linker sequence AAAA; and the phosphate backbone between the first two nucleotides at the 5' ends of the sequences a, b and c of the DNA carrier each independently with a phosphorothioate modification;

The HER3 aptamer is connected to the antisense strand of EGFR siRNA via the linker sequence AA, and is connected to the 3' end of the sequence c through complementary pairing between the antisense strand of EGFR siRNA and the sense strand of EGFR siRNA;

The HER2 aptamer is connected to the antisense strand of Survivin siRNA via the linker sequence AA, and is connected to the 3' end of the sequence b through complementary pairing between the antisense strand of Survivin siRNA and the sense strand of Survivin siRNA;

preferably, the sequence of the HER3 aptamer:

SEQ ID No. 197: CAGCGAAAGUUGCGUAUGGGUCACAUCGCAGGCACAUGUCAUCUGGGCG, with phosphorothioate modifications in the phosphate backbone between each pair of adjacent nucleotides among the first three nucleotides at the 5' end;

preferably, the antisense strand of EGFR siRNA is SEQ ID No. 302: AAUUAGAUAAGACUGCUAAGGCA; the sense strand is SEQ ID No. 82: CCUUAGCAGUCUUAUCUAAUU; the EGFR siRNA antisense and sense strands each have phosphorothioate modifications in the phosphate backbone between each pair of adjacent nucleotides among the first three nucleotides at the 5' end and among the last three nucleotides at the 3' end;

preferably, the sequence of the AS1411 aptamer is SEQ ID No. 131: GGTGGTGGTGGTTGTGGTGGTGGTGG, with a phosphorothioate modification in the phosphate backbone between the last two nucleotides at the 3' end;

preferably, the sense strand of Survivin siRNA is SEQ ID No. 303: GCAGGUUCCUUAUCUGUCACA,

The antisense strand of Survivin siRNA is SEQ ID No. 300: UGUGACAGAUAAGGAACCUGCAG, wherein there are phosphorothioate modifications in the phosphate backbone between each pair of adjacent nucleotides among the first three nucleotides at the 5' end and between each pair of adjacent nucleotides among the last three nucleotides at the 3' end;

The sequence of the HER2 aptamer:

SEQ ID No. 194: AGCCGCGAGGGGAGGGAUAGGGUAGGGCGCGGCU, with a phosphorothioate modification in the phosphate backbone between the first two nucleotides at the 5' end; C or U are a 2'-F modified, and A or G are a 2'-OME modified.

Drug 64): Survivin siRNA-TFRA3 aptamer-AS1411 aptamer-3 *AmiR-21-DNA carrier, wherein the DNA carrier is the nucleic acid carrier of the aforesaid twenty-six backbone sequence sets of group 17; the TFRA3 aptamer is connected to the 5' end of the sequence a via one AmiR-21; FA is connected to the 3' end of the sequence a; the AS1411 aptamer is connected to the 5' end of the sequence b via one AmiR-21; the sense strand of Survivin siRNA is connected to the 5' end of the sequence c via one AmiR-21; and the antisense strand of Survivin siRNA is connected to the sense strand of Survivin siRNA through complementary pairing;

preferably, the sequence of the TFRA3 aptamer is SEQ ID No. 262: GCGTGGTCACACGC;

preferably, the sequence of AmiR-21 is GATAAGCT;

preferably, the sequence of the AS1411 aptamer is SEQ ID No. 131: GGTGGTGGTGGTTGTGGTGGTGGTGG;

preferably, the sense strand of Survivin siRNA is SEQ ID No. 304: GCAGGUUCCUUAUCUGUCA, wherein the phosphate backbone has phosphorothioate modifications between each pair of adjacent nucleotides among the first three nucleotides at the 5' end and among the last three nucleotides at the 3' end, and positions 7 and 9-11 from the 5' end are 2'-F modified, the remaining positions are 2'-OMe modified;

The antisense strand of Survivin siRNA is SEQ ID No. 305: UGACAGAUAAGGAACCUGCAGUU, with phosphorothioate modifications in the phosphate backbone between each pair of adjacent nucleotides among the first three nucleotides at the 5' end and among the last three nucleotides at the 3' end, and with 2'-F modifications at positions 2, 6, 8, 9, 12, 14 and 16 from the 5' end, the remaining positions are 2'-OMe-modified.

Drug 65): AS1411 aptamer-EGFR siRNA-VEGF 165 aptamer-PD-L1 aptamer-RNA carrier, wherein the RNA carrier is the nucleic acid carrier of the aforesaid twenty-six backbone sequence sets of group 16; the VEGF165 aptamer is connected to the 3' end of the sequence a via the linker sequence AA; the PD-L1 aptamer is connected to the 3' end of the sequence b via the linker sequence AA; the sense strand of EGFR siRNA is connected to the 3' end of the sequence c via the linker sequence AA; the AS1411 aptamer is connected to the 5' end of the antisense strand of EGFR siRNA via the linker sequence AA; the antisense strand of EGFR siRNA is connected to the sense strand of EGFR siRNA through complementary pairing; and the phosphate backbone between the first two nucleotides at the 5' end of the sequence a of the DNA carrier with a phosphorothioate modification;

preferably, the sequence of the AS1411 aptamer is SEQ ID No. 131: GGTGGTGGTGGTTGTGGTGGTGGTGG, with a phosphorothioate modification in the phosphate backbone between the first two nucleotides at the 5' end;

preferably, the sense strand of EGFR siRNA is SEQ ID No. 82: CCUUAGCAGUCUUAUCUAAUU, with phosphorothioate modifications in the phosphate backbone between each pair of adjacent nucleotides among the last three nucleotides at the 3' end;

The antisense strand of EGFR siRNA:
SEQ ID No. 302: AAUUAGAUAAGACUGCUAAGGCA, with phosphorothioate modifications between each pair of adjacent nucleotides among the first three nucleotides at the 5' end and among the last three nucleotides at the 3' end;

preferably, the sequence of the VEGF165 aptamer:
SEQ ID No. 280: CGGAAUCAGUGAAUGCUUAUACAUCCG, wherein C and U are 2'-F modified, A and G are 2'-OME modified, and there is a phosphorothioate modification in the phosphate backbone between the last two nucleotides at the 3' end;

preferably, the sequence of the PD-L1 aptamer:
SEQ ID No. 241: ACGGGCCACATCAACTCATTGATAGACAATGCGTCCACTGCCCGT, wherein C and U are 2'-F modified, A and G are 2'-OME modified, and there is a phosphorothioate modification in the phosphate backbone between the last two nucleotides at the 3' end.

Drug 66): CEA aptamer-CD16a aptamer-Act-12c aptamer-PD-L1 aptamer-DNA carrier, wherein the DNA carrier is the nucleic acid carrier of the aforesaid twenty-six backbone sequence sets of group 20; the CEA aptamer is connected to the 5' end of the sequence a via the linker sequence AAA; the CD16a aptamer is connected to the 3' end of the sequence a via the linker sequence AA; the Act-12c aptamer and the PD-L1 aptamer are respectively connected to the 5' ends of the sequence b and the sequence c via the linker sequence AA; and the phosphate backbone between the first two nucleotides at the 3' ends of the sequence b and the sequence c of the DNA carrier each independently with a phosphorothioate modification;

preferably, the sequence of the CEA aptamer is SEQ ID No. 150: TTAACTTATTCGACCATA, with a phosphorothioate modification in the phosphate backbone between the first two nucleotides at the 5' end;

preferably, the sequence of the CD16a aptamer:
SEQ ID No. 157: CCACTGCGGGGGGTCTATACGTGAGGAAGAAGTGG, with a phosphorothioate modification in the phosphate backbone between the last two nucleotides at the 3' end;

preferably, the sequence of the Act-12c aptamer:
SEQ ID No. 137: CGGGGAAAGTCACGGGGGGGTTTCAGATGTTCTGATCGGTGTGGAG, with a phosphorothioate modification in the phosphate backbone between the first two nucleotides at the 5' end;

preferably, the sequence of the PD-L1 aptamer:
SEQ ID No. 241: ACGGGCCACATCAACTCATTGATAGACAATGCGTCCACTGCCCGT, with a phosphorothioate modification in the phosphate backbone between the first two nucleotides at the 5' end.

Drug 67): AS1411 aptamer-TAP siRNA-CD16a aptamer-CTLA-4 aptamer-DNA carrier, wherein the DNA carrier is the nucleic acid carrier of the aforesaid twenty-six backbone sequence sets of group 14; the CD16a aptamer is connected to the 3' end of the sequence a via the linker sequence AAAA; the CTLA-4 aptamer is connected to the 3' end of the sequence b via the linker sequence AAAA; the sense strand of TAP siRNA is connected to the 3' end of the sequence c via the linker sequence AAAA; the AS 1411 aptamer is connected to the 5' end of the antisense strand of TAP siRNA via the linker sequence AAA; the antisense strand of TAP siRNA is connected to the sense strand of TAP siRNA through complementary pairing; and the phosphate backbone between the first two nucleotides at the 5' ends of the sequences a, b and c of the DNA carrier each independently with a phosphorothioate modification;

preferably, the sequence of the AS 1411 aptamer:
SEQ ID No. 131: GGTGGTGGTGGTTGTGGTGGTGGTGG, with a phosphorothioate modification in the phosphate backbone between the first two nucleotides at the 5' end;

preferably, the sense strand of TAP siRNA is SEQ ID No. 301: GCUGCACACGGUUCAGAAU, with phosphorothioate modifications in the phosphate backbone between each pair of adjacent nucleotides among the first three nucleotides at the 5' end and among the last three nucleotides at the 3' end, and are 2'-F modified at positions 7 and 9-11 from the 5' end, the remaining nucleotides are 2'-OME modified;

The antisense strand of TAP siRNA is SEQ ID No. 109: AUUCUGAACCGUGUGCAGCUU, with phosphorothioate modifications in the phosphate backbone between each pair of adjacent nucleotides among the first three nucleotides at the 5' end and among the last three nucleotides at the 3' end, and are 2'-F modified at positions 2, 6, 8, 9, 12, 14 and 16 from the 5' end, the remaining nucleotides are 2'-OME modified;

preferably, the sequence of the CD16a aptamer:
SEQ ID No. 157: CCACTGCGGGGGTCTATACGTGAGGAAGAAGTGG, with phosphorothioate modifications in the phosphate backbone between the first two nucleotides at the 5' end and between the last two nucleotides at the 3' end;

preferably, the sequence of the CTLA-4 aptamer:
SEQ ID No. 141: GGUGGAAGAGGGAUGGGCCGACGUGCCGCAU, with a phosphorothioate modification in the phosphate backbone between the last two nucleotides at the 3' end, wherein C and U are 2'-F modified and A and G are 2'-OME modified.

Drug 68): AS1411 aptamer-TAP siRNA-CD16a aptamer-Act-12c aptamer-DNA carrier, wherein the DNA carrier is the nucleic acid carrier of the aforesaid twenty-six backbone sequence sets of group 14; the CD16a aptamer is connected to the 3' end of the sequence a via the linker sequence AAAA; the Act-12c aptamer is connected to the 3' end of the sequence b via the linker sequence AAAA; the sense strand of TAP siRNA is connected to the 3' end of the sequence c via the linker sequence AAAA; the AS 1411 aptamer is connected to the 5' end of the antisense strand of TAP siRNA via the linker sequence AAA; the antisense strand of TAP siRNA is connected to the sense strand of TAP siRNA through complementary pairing; and the phosphate backbone between the first two nucleotides at the 5' ends of the sequences a, b and c of the DNA carrier each independently with a phosphorothioate modification;

preferably, the sequence of the AS 1411 aptamer:
SEQ ID No. 131: GGTGGTGGTGGTTGTGGTGGTGGTGG, with a phosphorothioate modification in the phosphate backbone between the first two nucleotides at the 5' end;

preferably, the sense strand of TAP siRNA is SEQ ID No. 301: GCUGCACACGGUUCAGAAU, with phosphorothioate modifications in the phosphate backbone between each pair of adjacent nucleotides among the first three nucleotides at the 5' end and among the last three nucleotides at the 3' end, and with 2'-F modified at positions 7 and 9-11 from the 5' end, the remaining nucleotides are 2'-OME modified;

The antisense strand of TAP siRNA is SEQ ID No. 109: AUUCUGAACCGUGUGCAGCUU, with phosphorothioate modifications in the phosphate backbone between each pair of adjacent nucleotides among the first three nucleotides at the 5' end and among the last three nucleotides at the 3' end, and with 2'-F modifications at positions 2, 6, 8, 9, 12, 14 and 16 from the 5' end, the remaining nucleotides are 2'-OME modified;

preferably, the sequence of the CD16a aptamer:
SEQ ID No. 157: CCACTGCGGGGGTCTATACGTGAGGAAGAAGTG*G, with a phosphorothioate modification in the phosphate backbone between the last two nucleotides at the 3' end;

preferably, the sequence of the Act-12c aptamer:
SEQ ID No. 137: CGGGGAAAGTCACGGGGGGGTTTCAGATGTTCTGATCGGTGTGGAG, with a phosphorothioate modification in the phosphate backbone between the last two nucleotides at the 3' end.

Drug 69): CD16a aptamer-AS 1411 aptamer-TAP siRNA-OX40 aptamer-Act-12c aptamer-DNA carrier, wherein the DNA carrier is the nucleic acid carrier of the aforesaid twenty-six backbone sequence sets of group 14; the CD16a aptamer is connected to the 5' end of the sequence a via the linker sequence AA; the sense strand of TAP siRNA is connected to the 3' end of the sequence a via the linker sequence AA; the AS 1411 aptamer is connected to the 5' end of the antisense strand of TAP siRNA via the linker sequence AAA; the antisense strand of TAP siRNA is connected to the sense strand of TAP siRNA through complementary pairing; the OX40 aptamer is connected to the 3' end of the sequence b via the linker sequence AA; the Act-12c aptamer is connected to the 5' end of the sequence c via the linker sequence AAAA; and the phosphate backbone between the first two nucleotides at the 5' ends of the sequence b and the sequence c of the DNA carrier each independently with a phosphorothioate modification;

preferably, the sequence of the CD16a aptamer:
SEQ ID No. 157: CCACTGCGGGGGTCTATACGTGAGGAAGAAGTGG, with a phosphorothioate modification in the phosphate backbone between the first two nucleotides at the 5' end;

preferably, the sequence of the AS1411 aptamer is SEQ ID No. 131: GGTGGTGGTGGTTGTGGTGGTGGTGG, with a phosphorothioate modification in the phosphate backbone between the first two nucleotides at the 5' end;

preferably, the sense strand of TAP siRNA is SEQ ID No. 301: GCUGCACACGGUUCAGAAU, with phosphorothioate modifications in the phosphate backbone between each pair of adjacent nucleotides among the first three nucleotides at the 5' end and among the last three nucleotides at the 3' end, and are 2'-F modified at positions 7, 9, 10 and 11 from the 5' end, the remaining nucleotides are 2'-OME modified;

The antisense strand of TAP siRNA is SEQ ID No. 109: AUUCUGAACCGUGUGCAGCUU, with phosphorothioate modifications in the phosphate backbone between each pair of adjacent nucleotides among the first three nucleotides at the 5' end and among the last three nucleotides at the 3' end, and with 2'-F modifications at positions 2, 6, 8, 9, 12, 14 and 16 from the 5' end, the remaining nucleotides are 2'-OME modified;

preferably, the sequence of the OX40 aptamer:
SEQ ID No. 228: GGGAGGACGAUGCGGCAGUCUGCAUCGUAGGAAUCGCCACCGUAUACUUUCCCACCAGACGACUCGCUG, with a phosphorothioate modification in the phosphate backbone between the last two nucleotides at the 3' end, wherein C and U are 2'-F modified and A and G are 2'-OMe modified;

preferably, the sequence of the Act-12c aptamer:
SEQ ID No. 137: CGGGGAAAGTCACGGGGGGGTTTCAGATGTTCTGATCGGTGTGGAG, with a phosphorothioate modification in the phosphate backbone between the first two nucleotides at the 5' end.

Drug 70): CTLA-4 aptamer-CD40 aptamer-FAP aptamer-DNA carrier, wherein the DNA carrier is the nucleic acid carrier of the aforesaid twenty-six backbone sequence sets of group 15; the CTLA-4 aptamer is connected to the 5' end of the sequence a via the linker sequence TTTTT; the CD40 aptamer is connected to the 5' end of the sequence b via the linker sequence TTTTT; the FAP aptamer is connected to the 5' end of the sequence c via the linker sequence TTTTT; and the phosphate backbone between the first two nucleotides at the 3' end of the sequences a, b and c of the DNA carrier each independently with a phosphorothioate modification;

preferably, the sequence of the CTLA-4 aptamer:
SEQ ID No. 141: GGUGGAAGAGGGAUGGGCCGACGUGCCGCAU, with a phosphorothioate modification in the phosphate backbone between the first two nucleotides at the 5' end, and wherein C and U are 2'-F modified, and A and G are 2'-OMe modified;

preferably, the sequence of the CD40 aptamer:
SEQ ID No. 154: CCAACGAGTAGGCGATAGCGCGTGG, with a phosphorothioate modification in the phosphate backbone between the first two nucleotides at the 5' end;

preferably, the sequence of the FAP aptamer:
SEQ ID No. 188: CCGCTCGAGCTAGTCTGACAAAGAGAAACAC, with a phosphorothioate modification in the phosphate backbone between the first two nucleotides at the 5' end.

Drug 71): VEGF165 aptamer-ASAP1 siRNA-CD24A-2 aptamer-SARS-CoV-2-N48 aptamer-DNA carrier, wherein the DNA carrier is the nucleic acid carrier of the aforesaid twenty-six backbone sequence sets of group 14; the sense strand of ASAP1 siRNA is connected to the 3' end of the sequence c via the linker sequence AA; the VEGF165 aptamer is connected to the 5' end of the antisense strand of ASAP1 siRNA via the linker sequence AA; the antisense strand of ASAP1 siRNA is connected to the sense strand of ASAP1 siRNA through complementary pairing; the CD24A-2 aptamer is connected to the 3' end of the sequence b via the linker sequence AA; the SARS-CoV-2-N48 aptamer is connected to the 3' end of the sequence a via the linker sequence AA; and the phosphate backbone between the first two nucleotides at the 5' ends of the sequence b and the sequence c of the DNA carrier each independently with a phosphorothioate modification;

preferably, the sequence of the VEGF165 aptamer:
SEQ ID No. 280: CGGAAUCAGUGAAUGCUUAUACAUCCG, with a phosphorothioate modification in the phosphate backbone between the first two nucleotides at the 5' end, and wherein C and U are 2'-F modified, and A and G are 2'-OME modified;

preferably, the antisense strand of ASAP1 siRNA is SEQ ID No. 65: UGAUAUUAUGGAAGCAAAUUU, with phosphorothioate modifications in the phosphate backbone between each pair of adjacent nucleotides among the first three nucleotides at the 5' end and among the last three nucleotides at the 3' end, and with 2'-F modified at positions 2, 6, 8, 9, 12, 14 and 16 from the 5' end, the remaining nucleotides are 2'-OME-modified;

The sense strand of ASAP1 siRNA is SEQ ID No. 64: AUUUGCUUCCAUAAUAUCAUU, with phosphorothioate modifications in the phosphate backbone between each pair of adjacent nucleotides among the first three nucleotides at the 5' end and among the last three nucleotides at the 3' end, and with 2'-F modified at positions 7 and 9-11 from the 5' end, the remaining nucleotides are 2'-OME-modified;

preferably, the sequence of the SARS-CoV-2-N48 aptamer:
SEQ ID No. 222: GCTGGATGTCGCTTACGACAATATTCCTTAGGGGCACCGCTACATTGACACATCCAGC, with a phosphorothioate modification in the phosphate backbone between the last two nucleotides at the 3' end;

preferably, the sequence of the CD24A-2 aptamer:
SEQ ID No. 167: ATCCAGAGTGACGCAGCATATGTGGGTGGGTGGGCGGTTATGCTGAGTCAGCCTTGCTT G GACACGGTGGCTTAGT, with a phosphorothioate modification in the phosphate backbone between the first two nucleotides at the 5' end.

Drug 72): 3*FGF5 aptamer-DNA carrier, wherein the DNA carrier is the nucleic acid carrier of the aforesaid twenty-six backbone sequence sets of group 15; the three FGF5 aptamers are respectively connected to the 5' ends of the sequence a, the sequence b, and the sequence c via the linker sequence U; and the phosphate backbone between the first two nucleotides at the 3' ends of the sequences a, b and c of the DNA carrier each independently with a phosphorothioate modification;

preferably, the sequence of the FGF5 aptamer:
SEQ ID No. 186: GGGCGACCUCUCCGUACUGACCUACAGAGCGACAUACUAGUGUAUCCAGAUCGCCC, with a phosphorothioate modification in the phosphate backbone between the first two nucleotides at the 5' end, and wherein C and U are 2'-F modified and A and G are 2'-OMe modified.

Drug 73): AS1411 aptamer-ASAP1 siRNA-VEGF aptamer-SARS-CoV-2-N48 aptamer-RNA carrier, wherein the RNA carrier is the nucleic acid carrier of the aforesaid twenty-six backbone sequence sets of group 21; the VEGF aptamer is connected to the 3' end of the sequence a via the linker sequence AA; the SARS-CoV-2-N48 aptamer is connected to the 3' end of the sequence b via the linker sequence AA; the sense strand of ASAP1 siRNA is connected to the 3' end of the sequence c; the AS1411 aptamer is connected to the 5' end of the antisense strand of ASAP1 siRNA via the linker sequence AA; the antisense strand of ASAP1 siRNA is connected to the sense strand of ASAP1 siRNA through complementary pairing; and the phosphate backbone between the first two nucleotides at the 5' end of the sequences a, b and c of the RNA carrier each independently with a phosphorothioate modification; moreover, within the sequence c, A and G are 2'-OMe modified while C and U are 2'-F modified;

preferably, the sequence of the AS1411 aptamer:
SEQ ID No. 131: GGTGGTGGTGGTTGTGGTGGTGGTGG, with a phosphorothioate modification in the phosphate backbone between the first two nucleotides at the 5' end;

preferably, the antisense strand of ASAP1 siRNA is SEQ ID No. 65: UGAUAUUAUGGAAGCAAAUUU, with phosphorothioate modifications in the phosphate backbone between each pair of adjacent nucleotides among the first three nucleotides at the 5' end and among the last three nucleotides at the 3' end, and are 2'-F modified at positions 2, 6, 8, 9, 12, 14 and 16 from the 5' end, the remaining nucleotides are 2'-OMe-modified;

The sense strand of ASAP1 siRNA is SEQ ID No. 64: AUUUGCUUCCAUAAUAUCAUU, with phosphorothioate modifications in the phosphate backbone between each pair of adjacent nucleotides among the first three nucleotides at the 5' end and among the last three nucleotides at the 3' end;

preferably, the sequence of the VEGF aptamer is SEQ ID No. 280: CGGAAUCAGUGAAUGCUUAUACAUCCG, wherein C and U are 2'-F modified and A and G are 2'-OMe modified;

preferably, the sequence of the SARS-CoV-2-N48 aptamer:
SEQ ID No. 222: GCTGGATGTCGCTTACGACAATATTCCTTAGGGGCACCGCTACATTGACACATCCAGC, with a phosphorothioate modification in the phosphate backbone between the last two nucleotides at the 3' end.

Drug 74): CpG2006-CD40 aptamer-PD-1 aptamer-DNA carrier, wherein the DNA carrier is the nucleic acid carrier of the aforesaid twenty-six backbone sequence sets of group 6; CpG2006 is connected to the 5' end of the sequence a via a linker sequence; the CD40 aptamer is connected to the 5' end of the sequence b via a linker sequence; the PD-1 aptamer is connected to the 5' end of the sequence c via a linker sequence; and the phosphate backbone between the last two nucleotides at the 3' ends of the sequences a, b and c of the DNA carrier each independently with a phosphorothioate modification;

preferably, the sequence of CpG2006:
SEQ ID No. 57: TCGTCGTTTTGTCGTTTTGTCGTT, with phosphorothioate modifications in the phosphate backbone between adjacent nucleotides;

preferably, the sequence of the CD40 aptamer:
SEQ ID No. 154: CCAACGAGTAGGCGATAGCGCGTGG, with 2'-O-methoxyethyl (MOE) modified at the 2-position of the ribose of each of the first four nucleotides at the 5' end, and with phosphorothioate modifications in the phosphate backbone between each pair of adjacent nucleotides among the first five nucleotides at the 5' end;

preferably, the sequence of the PD-1 aptamer:
SEQ ID No. 254: ACCGACAGTGAAGGACTCAGCGAACTCTCAGACTCGGTTC, with 2'-O-methoxyethyl (MOE) modified at the 2-position of the ribose of each of the first four nucleotides at the 5' end, and with phosphorothioate modifications in the phosphate backbone between each pair of adjacent nucleotides among the first five nucleotides at the 5' end.

Drug 75): CD24 aptamer-CpG2006 variant-CD40 aptamer-PD-L1 aptamer-DNA carrier, wherein the DNA carrier is the nucleic acid carrier of the aforesaid twenty-six backbone sequence sets of group 6; the CD24 aptamer is connected to the 5' end of the sequence a via the CpG2006-variant sequence GTCGTT; the CD40 aptamer is connected to the 5' end of the sequence b via the linker sequence TTTTT; and the PD-L1 aptamer is connected to the 5' end of the sequence c via the linker sequence TTTTT;

preferably, the sequence of the CD24 aptamer:
SEQ ID No. 166: TATGTGGGTGGGTGGGCGGTTATGCTGAGTCAGCCTTGCT, with phosphorothioate modifications in the phosphate backbone between each pair of adjacent nucleotides among the first four nucleotides at the 5' end;

preferably, the sequence of the CD40 aptamer:
SEQ ID No. 154: CCAACGAGTAGGCGATAGCGCGTGG, with phosphorothioate modifications in the phosphate backbone between each pair of adjacent nucleotides among the first five nucleotides at the 5' end;

preferably, the sequence of the PD-L1 aptamer:
SEQ ID No. 238: AACAACGTATAACAATGCCCACGTCACCAGAGTACTATGG, with 2'-O-methoxyethyl (MOE) modifications at the 2-position of the ribose of each of the first six nucleotides at the 5' end;

The linker sequence TTTTT is a PEG-modified TTTTT.

Drug 76): HBsAg aptamer-HBV siRNA-miR-34-PD-L1 aptamer-miR-542-AS1411 aptamer-DNA carrier, wherein the DNA carrier is the nucleic acid carrier of the aforesaid twenty-six backbone sequence sets of group 24; the sense strand of HBV siRNA is connected to the 3' end of the sequence a; the HBsAg aptamer is connected to the 5' end of the antisense strand of HBV siRNA; the PD-1 aptamer is connected to the 3' end of the sequence b via miR-34; and the AS1411 aptamer is connected to the 3' end of the sequence c via miR-542;

preferably, the sense strand of HBV siRNA is SEQ ID No. 88: GGACUUCUCUCAAUUUUCUUU; the antisense strand is SEQ ID No. 89: AGAAAAUUGAGAGAAGUCCUU; wherein, in both the sense and antisense strands, C and U are 2'-F modified, and the phosphate backbone between each pair of adjacent nucleotides among the last three nucleotides at the 3' end has phosphorothioate modifications;

preferably, the sequence of the HBsAg aptamer:
SEQ ID No. 192: CACAGCGAACAGCGGCGGACATAATAGTGCTTACTACGAC, with phosphorothioate modifications in the phosphate backbone between each pair of adjacent nucleotides among the first four nucleotides at the 5' end;

preferably, the sequence of miR-34 is TGTGACAG;

preferably, the sequence of the PD-L1 aptamer:
SEQ ID No. 238: AACAACGTATAACAATGCCCACGTCACCAGAGTACTATGG, with 2'-O-methoxyethyl (MOE) modified on the ribose of each of the three nucleotides at the 3' end;

preferably, the sequence of miR-542 is TGGCAGTGT;

preferably, the sequence of the AS1411 aptamer:
SEQ ID No. 131: GGTGGTGGTGGTTGTGGTGGTGGTGG, with 2'-O-MOE-modified phosphorothioate linkages in the phosphate backbone between each pair of adjacent nucleotides among the last four nucleotides at the 3' end.

Drug 77): IL-4Ra aptamer-TIMC-d aptamer-4*miR-126-RNA carrier, wherein the RNA carrier is the nucleic acid carrier of the aforesaid twenty-six backbone sequence sets of group 25; the IL-4Ra aptamer is directly connected to the 5' end of the sequence a; the TIMC-d aptamer is directly connected to the 5' end of the sequence b; and four miR-126 units are connected in tandem to the 5' end of the sequence c;

The sequence of the IL-4Ra aptamer:
SEQ ID No. 203: AAAAAGCAACAGGGUGCUCCAUGCGCAUGGAACCUGCGCG, with phosphorothioate modifications in the phosphate backbone between each pair of adjacent nucleotides among the first four nucleotides at the 5' end;

The sequence of the TIMC-d aptamer:
SEQ ID No. 272: AAGCAACACUUAGUCGCGAUUGAUACGUGCGCAGUCAU, with phosphorothioate modifications in the phosphate backbone between each pair of adjacent nucleotides among the first four nucleotides at the 5' end;

The sequence of miR-126:
UCGUACC, with phosphorothioate modifications in the phosphate backbone between adjacent nucleotides.

Drug 78): CD3-4 aptamer-PD-1 aptamer-BCMA aptamer-DNA carrier, wherein the DNA carrier is the nucleic acid carrier of the aforesaid twenty-six backbone sequence sets of group 24; the CD3-4 aptamer is connected via the linker sequence TTTTTT to the complementary sequence A' of the single-stranded linker sequence A in the sequence a, and is attached to the 3' end of the sequence a through complementary pairing between the complementary sequence A' and the single-stranded linker sequence A;

PD-1 aptamer is connected via the linker sequence TTTTT to the complementary sequence B' of the single-stranded linker sequence B in the sequence b, and is attached to the 3' end of the sequence b through complementary pairing between the complementary sequence B' and the single-stranded linker sequence B;

BCMA aptamer is connected to the complementary sequence C' of the single-stranded linker sequence C in the sequence c, and is attached to the 3' end of the sequence c through complementary pairing between the complementary sequence C' and the single-stranded linker sequence C;

The sequence a:
SEQ ID No. 15: GACGCCCACGAGCGTTCCGGGAGAGGTGTAGCACGGTGGC, wherein the underlined portion is the single-stranded linker sequence A;

preferably, the sequence of the CD3-4 aptamer-linker sequence-complementary sequence A' of the single-stranded linker sequence A:
SEQ ID No. 306:

TCTCGGACGCGTGTGGTCGGCCGAGTGGCCCACGGTAGAAGGGTTAGAACTGCTGGTTG GTGAATCTCGCTGCCTGGCCCTAGAGTG**TTTTTTGCCACCGTGCTACA;**

Within the DNA carrier, the sequence b:
SEQ ID No. 16: CCTCTCCCGGTTCGCCGCGAGCC<u>TCGGCGCGGCCGTG,</u> wherein the underlined portion is the single-stranded linker sequence B;

preferably, the sequence of the PD-1 aptamer-linker sequence TTTTT-complementary sequence B' of the single-stranded linker sequence B:
SEQ ID No. 307: ACCGACAGTGAAGGACTCAGCGAACTCTCAGACTCGGTTCTTTTT<u>CACGGCCGCGCCGA,</u> with phosphorothioate modifications in the phosphate backbone between each pair of adjacent nucleotides among the first five nucleotides at the 5' end;

The sequence c is SEQ ID No. 17: GGCTCGCGGCCATAGCCGTGGGCG<u>TCTGCTGCTGCTGCTG,</u> wherein the underlined portion is the single-stranded linker sequence C;

preferably, the sequence of the BCMA aptamer-linker sequence U-complementary sequence C' of the single-stranded linker sequence C is SEQ ID No. 308: AGUGCAAGACGUUCGCAGAUUAGCGAAAAGAGGGUCUCAU UGACUAGUACU<u>CAGCA GCAGCAGCA,</u> wherein the underlined portion is the complementary sequence C' of the single-stranded linker sequence C; the phosphate backbone between each pair of adjacent nucleotides among the last three nucleotides at the 3' end has phosphorothioate modifications; the ribose of each of the first four nucleotides at the 5' end are 2'-O-MOE modified; and, among the remaining nucleotides, C and U are 2'-F modified.

Drug 79): CpG2006-CD38 aptamer-PD-L1 aptamer-DNA carrier, wherein the DNA carrier is the nucleic acid carrier of the aforesaid twenty-six backbone sequence sets of group 6; CpG2006 is connected to the 5' end of the sequence a via the linker sequence TTTTT; the CD38 aptamer is connected to the 5' end of the sequence b via the linker sequence TTTTT; and the PD-L1 aptamer is connected to the 5' end of the sequence c via the linker sequence TTTTT;

preferably, the CpG2006-linker sequence TTTTT-sequence a:
SEQ ID No. 309: TCGTCGTTTTGTCGTTTTGTCGTTTTTTTGCGACGCCCACGAGCGTTCCGGGAGAGGAGC, wherein the phosphate backbone between adjacent nucleotides of CpG2006 are phosphorothioate modified;

preferably, the CD38 aptamer-linker sequence TTTTT-sequence b:
SEQ ID No. 310: TACGTGAATCTCGTACGATACTCTGTAAGCGTTTTTTGCTCCTCTCCCGGTTCGCCGCGA GCCGCG,wherein the ribose of the first three nucleotides at the 5' end are 2'-O-MOE modified;

preferably, the PD-L1 aptamer-linker sequence TTTTT-sequence c:
SEQ ID No. 311: AACAACGTATAACAATGCCCACGTCACCAGAGTACTATGGTTTTTCGCGGCTCGCGGCC AT AGCCGTGGGCGTCGC, wherein the ribose of the first six nucleotides at the 5' end are 2'-O-MOE modified.

Drug 80): CD20 aptamer-CpG-BYZD-CD38 aptamer-miR-34-CD3 aptamer-DNA carrier; wherein the DNA carrier is the nucleic acid carrier of the aforesaid twenty-six backbone sequence sets of group 6; the CD20 aptamer is connected to the 5' end of the sequence a via the CpG-BYZD sequence AGCGAA; the CD38 aptamer is connected to the 5' end of the sequence b via miR-34; and the CD3 aptamer is connected to the 5' end of the sequence c via the linker sequence TTTTT;

preferably, the CD20 aptamer-CpG-BYZD-sequence a:
SEQ ID No. 312: TGCGTGTGTAGTGTGTCTGTTTTTTATCTTCTTTTATCTACTCTTAGGGATTTGGGCGGAGC GAAGCGACGCCCACGAGCGTTCCGGGAGAGGAGC, SEQ ID No. 165: TGCGTGTGTAGTGTGTCTGTTTTTT ATCTTCTTTTATCTACTCTTAGGGATTTGGGCGG is the CD20 aptamer, AGCGAA is CpG-BYZD, and the phosphate backbone between each pair of adjacent nucleotides among the first four nucleotides at the 5' end are phosphorothioate modified;

preferably, the CD38 aptamer-miR-34-sequence b:
SEQ ID No. 313: TACGTGAATCTCGTACGATACTCTGTAAGCGTTGTGACAGGCTCCTCTCCCGGTTCGCCG CGAGCCGCG, wherein the ribose of each of the first three nucleotides at the 5' end are 2'-O-MOE modified; TGTGACAG is miR-34, the portion preceding TGTGACAG is the CD38 aptamer; and the portion following TGTGACAG is the sequence b;

preferably, the CD3 aptamer-linker sequence TTTTT-sequence c:
SEQ ID No. 314: GCCGCGGGGTGGGTCTAGTGTGGATGTTTAGGGGGCGGC**TTTTTCGCGGCTCGCGGCC A TAGCCGTGGGCGTCGC,** wherein the ribose of each of the first four nucleotides at the 5' end are 2'-O-MOE modified.

Drug 81): CpG2006-CD38 aptamer-miR-126-PD-L1 aptamer-DNA carrier; wherein the DNA carrier is the nucleic acid carrier of the aforesaid twenty-six backbone sequence sets of group 6; CpG2006 is connected to the 5' end of the sequence a via the linker sequence TTTTT; the CD38 aptamer is connected to the 5' end of the sequence b via miR-126; and the PD-L1 aptamer is connected to the 5' end of the sequence c via the linker sequence TTTTT;

preferably, the CpG2006-linker sequence TTTTT-sequence a:
SEQ ID No. 309: TCGTCGTTTTGTCGTTTTGTCGTT**TTTTTGCGACGCCCACGAGCGTTCCGGGAGAGGA GC,** wherein the phosphate backbone between adjacent nucleotides of CpG2006 are phosphorothioate modified, and the phosphate backbone between the last two nucleotides at the 3' end of the sequence a is phosphorothioate modified;

preferably, the CD38 aptamer-miR-126-sequence b:
SEQ ID No. 315: TACGTGAATCTCGTACGATACTCTGTAAGCGT**UCGUACCG**GCTCCTCTCCCGGTTCGCCG CGAGCCGCG,wherein the sequence of the CD38 aptamer is SEQ ID No. 170: TACGTGAATCTCGTACGATACT CTGTAAGCGT, with 2'-O-MOE modifications on the ribose of each of the first three nucleotides at the 5' end; and UCGUACCG is the sequence of miR-126, with 2'-O-MOE modifications on the ribose of each nucleotide;

preferably, the PD-L1 aptamer-linker sequence TTTTT-sequence c:
SEQ ID No. 311: AACAACGTATAACAATGCCCACGTCACCAGAGTACTATGG**TTTTT**CGCGGCTCGCGGCCA T AGCCGTGGGCGTCGC,wherein the ribose of each of the first six nucleotides at the 5' end of the PD-L1 aptamer is 2'-O-MOE modified.

Drug 82): BCMA aptamer-miR-126-CD38 aptamer-miR-122-CD3 aptamer-miR-34-DNA carrier; wherein the DNA carrier is the nucleic acid carrier of the aforesaid twenty-six backbone sequence sets of group 6; the BCMA aptamer is connected to the 5' end of the sequence a via miR-126; the CD38 aptamer is connected to the 5' end of the sequence b via miR-122; and the CD3 aptamer is connected to the 5' end of the sequence c via miR-34;

preferably, the BCMA aptamer-miR-126-sequence a :
SEQ ID No. 316: AGUGCAAGACGUUCGCAGAUUAGCGAAAAGAGGGUCUCAUUGACUAGUACU**CGTAC CG GCGACGCCCACGAGCGTTCCGGGGAGAGGAGC**, wherein CGTACCG is the sequence of miR-126, the portion preceding CGTACCG is the BCMA aptamer sequence, and the portion following CGTACCG is the sequence a; within the BCMA aptamer sequence, the ribose of each of the first four nucleotides at the 5' end is 2'-O-MOE modified, and at the remaining positions C and U are 2'-F modified;

preferably, the CD38 aptamer-miR-122-sequence b:
SEQ ID No. 317: TmAmCmGTGAATCTCGTACGATACTCTGTAAGCGT**GGAAGTGT**GCTCCTCTCCCGGTTC GCCGCGAGCCGCG, wherein the ribose of each of the first three nucleotides at the 5' end is 2'-O-MOE modified; GGAAGTGT is the sequence of miR-122; the portion preceding GGAAGTGT is the CD38 aptamer sequence; and the portion following GGAAGTGT is the sequence b;

preferably, the CD3 aptamer-miR-34-sequence c:
SEQ ID No. 318: AmGmCmCmGCGGGGTGGGTCTAGTGTGGATGTTTAGGGGGCGGCT**TGTGACA**GCGCGG CTCGCGGCCATAGCCGTGGGCGTCGC, wherein the ribose of each of the first four nucleotides at the 5' end is 2'-O-MOE modified; TGTGACA is the sequence of miR-34; the portion preceding TGTGACA is the CD3 aptamer sequence; and the portion following TGTGACA is the sequence c.

Drug 83): GPC3 aptamer-AmiR-21-CD38 aptamer-miR-122-PD-L1 aptamer-miR-34-DNA carrier; wherein the DNA carrier is the nucleic acid carrier of the aforesaid twenty-six backbone sequence sets of group 6; the GPC3 aptamer is connected to the 5' end of the sequence a via AmiR-21; the CD38 aptamer is connected to the 5' end of the sequence b via miR-122; and the PD-L1 aptamer is connected to the 5' end of the sequence c via miR-34;

preferably, the GPC3 aptamer-AmiR-21-sequence a is: SEQ ID No. 319: TAACGCTGACCTTAGCTGCATGGCTT TACATGTTCCA**GATAAGCT**GCGACGCCCACGAG CGTTCCGGGGAGAGGAGC, with phosphorothioate modifications in the phosphate backbone between each pair of adjacent nucleotides among the first four nucleotides at the 5' end; GATAAGCT is the sequence of AmiR-21, the portion preceding GATAAGCT is the GPC3 aptamer sequence, and the portion following GATAAGCT is the sequence a;

preferably, the CD38 aptamer-miR-122-sequence b is: SEQ ID No. 317: TACGTGAATCTCGTACGATACTCTGTA AGCGT**GGAAGTGTGCT**CCTCTCCCGGTTCGCC GCGAGCCGCG, with phosphorothioate modifications in the phosphate backbone between each pair of adjacent nucleotides among the first four nucleotides at the 5' end; GGAAGTGT is the sequence of miR-122, the portion preceding GGAAGTGT is the CD38 aptamer sequence, and the portion following GGAAGTGT is the sequence b;

preferably, the PD-L1 aptamer-miR-34-sequence c is SEQ ID No. 320: AACAACGTATAACAATGCCCACGTCAC CAGAGTACTATGG**TGTGACAG**CGCGGCTCGC GGCCATAGCCGTGGGCGTCGC, wherein the ribose of each of the first six nucleotides at the 5' end is 2'-O-MOE modified; TGTGACAG is the sequence of miR-34, the portion preceding TGTGACAG is the PD-L1 aptamer sequence, and the portion following TGTGACAG is the sequence c.

Drug 84): CpG2006-VEGF165 aptamer-miR-122-PD-L1 aptamer-DNA carrier; wherein the DNA carrier is the nucleic acid carrier of the aforesaid twenty-six backbone sequence sets of group 6; CpG2006 is connected to the 5' end of the sequence a via the linker sequence TTTTT; the VEGF 165 aptamer is connected to the 5' end of the sequence b via miR-122; and the PD-L1 aptamer is connected to the 5' end of the sequence c via the linker sequence TTTTT;

preferably, the CpG2006-linker sequence TTTTT-sequence a:
SEQ ID No. 309: TCGTCGTTTTGTCGTTTTGTCGTT**TTTTT**GCGACGCCCACGAGCGTTCCGGGAGAGGAG C,wherein the phosphate backbone between adjacent nucleotides of CpG2006 are phosphorothioate modified;

preferably, the VEGF165 aptamer-miR-122-sequence b:
SEQ ID No. 321: CGGAAUCAGUGAAUGCUUAUACAUCCG**GGAAGTGT**GCTCCTCTCCCGGTTCGCCGCG AGCCGCG,wherein the ribose of each of the first four nucleotides at the 5' end is 2'-O-MOE modified, and in the remaining RNA portion C and U are 2'-F modified; GGAAGTGT is the sequence of miR-122, the portion preceding GGAAGTGT is the VEGF 165 aptamer sequence, and the portion following GGAAGTGT is the sequence b;

preferably, the PD-L1 aptamer-linker sequence TTTTT-sequence c:
SEQ ID No. 311: AACAACGTATAACAATGCCCACGTCACCAGAGTACTATGG**TTTTTCGCGGCTCGCGGC CAT AGCCGTGGGCGTCGC**,wherein the ribose of each of the first six nucleotides at the 5' end is 2'-O-MOE modified.

Drug 85): CpG2006-HBsAg aptamer-miR-122-PD-L1 aptamer-DNA carrier; wherein the DNA carrier is the nucleic acid carrier of the aforesaid twenty-six backbone sequence sets of group 6; CpG2006 is connected to the 5' end of the sequence a via the linker sequence TTTTT; the HBsAg aptamer is connected to the 5' end of the sequence b via miR-122; and the PD-L1 aptamer is connected to the 5' end of the sequence c via the linker sequence TTTTT;

preferably, the CpG2006-linker sequence TTTTT-sequence a:
SEQ ID No. 309: TCGTCGTTTTGTCGTTTTGTCGTT**TTTTT**GCGACGCCCACGAGCGTTCCGGGAGAGGAG C; wherein the phosphate backbone between adjacent nucleotides of CpG2006 is phosphorothioate modified;

preferably, the HBsAg aptamer-miR-122-sequence b:
SEQ ID No. 322: CACAGCGAACAGCGGCGGACATAATAGTGCTTACTACGAC**GGAAGTGT**GCTCCTCTCCC G GTTCGCCGCGAGCCGCG, with phosphorothioate modifications in the phosphate backbone between each pair of adjacent nucleotides among the first four nucleotides at the 5' end; GGAAGTGT is the sequence of miR-122; the portion preceding GGAAGTGT is the HBsAg aptamer sequence; and the portion following GGAAGTGT is the sequence b;

preferably, the PD-L1 aptamer-linker sequence TTTTT-sequence c:
SEQ ID No. 311: AACAACGTATAACAATGCCCACGTCACCAGAGTACTATGG**TTTTT**CGCGGCTCGCGGCCA T AGCCGTGGGCGTCGC, wherein the ribose of each of the first six nucleotides at the 5' end is 2'-O-MOE modified.

Drug 86): ASO-GSK836-HBsAg aptamer-miR-122-PD-L1 aptamer-DNA carrier; wherein the DNA carrier is the nucleic acid carrier of the aforesaid twenty-six backbone sequence sets of group 6; ASO-GSK836 is connected to the 5' end of the sequence a via the linker sequence TTTTT; the HBsAg aptamer is connected to the 5' end of the sequence b via miR-122; and the PD-L1 aptamer is connected to the 5' end of the sequence c via the linker sequence TTTTT;

preferably, the ASO-GSK836-linker sequence TTTTT-sequence a:
SEQ ID No. 323: GCAGAGGTGAAGCGAAGTCG**TTTTT**GCGACGCCCACGAGCGTTCCGGGAGAGGAGC, with phosphorothioate modifications in the phosphate backbone between each pair of adjacent nucleotides among the first six nucleotides at the 5' end;

preferably, the HBsAg aptamer-miR-122-sequence b:
SEQ ID No. 322: CACAGCGAACAGCGGCGGACATAATAGTGCTTACTACGAC**GGAAGTGT**GCTCCTCTCCC G GTTCGCCGCGAGCCGCG, with phosphorothioate modifications in the phosphate backbone between each pair of adjacent nucleotides among the first four nucleotides at the 5' end; GGAAGTGT is the sequence of miR-122, the portion preceding GGAAGTGT is the HBsAg aptamer sequence, and the portion following GGAAGTGT is the sequence b;

preferably, the PD-L1 aptamer-linker sequence TTTTT-sequence c:
SEQ ID No. 311: AACAACGTATAACAATGCCCACGTCACCAGAGTACTATGG**TTTTT**CGCGGCTCGCGGCCA T AGCCGTGGGCGTCGC, wherein the ribose of each of the first six nucleotides at the 5' end is 2'-O-MOE modified.

Drug 87): BCMA aptamer-CD38 aptamer-miR-126-PD-L1 aptamer-miR-34-DNA carrier; wherein the DNA carrier is the nucleic acid carrier of the aforesaid twenty-six backbone sequence sets of group 6; the BCMA aptamer is directly connected to the 5' end of the sequence a; the CD38 aptamer is connected to the 5' end of the sequence b via miR-126; and the PD-L1 aptamer is connected to the 5' end of the sequence c via miR-34;

preferably, the BCMA aptamer-sequence a:
SEQ ID No. 324: AGUGCAAGACGUUCGCAGAUUAGCGAAAAGAGGGUCUCAUUGACUAGUACUUGCGA CG CCCACGAGCGTTCCGGGAGAGGAGC, wherein SEQ ID No. 140: AGUGCAAGACGUUCGCAGAUUAGCGAA AAGAGGGUCUCAUUGACUAGUAC is the BCMA aptamer in RNA form, in which all nucleotides are 2'-F modifications; the remainder is the sequence a;

preferably, the CD38 aptamer-miR-126-sequence b:
SEQ ID No. 325: TACGTGAATCTCGTACGATACTCTGTAAGCGT**GTCGTT**GCTCCTCTCCCGGTTCGCCGCG AGCCGCG, with phosphorothioate modifications in the phosphate backbone between each pair of adjacent nucleotides among the first four nucleotides at the 5' end; GTCGTT is the sequence of miR-126, the portion preceding GTCGTT is the CD38 aptamer sequence, and the portion following GTCGTT is the sequence b;

preferably, the PD-L1 aptamer-miR-34-sequence c:
SEQ ID No. 320: AACAACGTATAACAATGCCCACGTCACCAGAGTACTATGG**TGTGACAG**CGCGGCTCGCG G CCATAGCCGTGGGCGTCGC, wherein the ribose of each of the first six nucleotides at the 5' end is 2'-O-MOE modified; TGTGACAG is the sequence of miR-34; the portion preceding TGTGACAG is the PD-L1 aptamer sequence, and the portion following TGTGACAG is the sequence c.

Drug 88): AS1411 aptamer-FGF2 aptamer-3*AmiR-21-DNA carrier; the AS1411 aptamer is connected to the 5' end of the sequence a of the DNA carrier via the linker sequence TTTTT; the FGF2 aptamer is connected to the 5' end of the sequence b of the DNA carrier via the linker sequence U; and three AmiR-21 units are directly connected in tandem to the 5' end of the sequence c of the DNA carrier;

preferably, the AS1411 aptamer-linker sequence TTTTT-sequence a:
SEQ ID No. 326: GGTGGTGGTGGTTGTGGTGGTGGTGG**TTTTT**GCGCCCACGAGCGTTCCGGGAGAGC;

preferably, the FGF2 aptamer-linker sequence U-sequence b is SEQ ID No. 327: GGGAUACUAGGGCAUUAAU GUUACCAGUGUAGUCCCUCCCUGCTCTCCCGGTTCGCCG CCAGCCGCC, wherein SEQ ID No. 183: GGGAUACUAGGGCAUUAAUGUUACCAGUGUAGUCCC is the FGF2 aptamer in RNA form, in which C and U are 2'-F modified; the remainder is the sequence b;

preferably, the 3*AmiR-21-sequence c:
SEQ ID No. 328: GATAAGCTGATAAGCTGATAAGCTGGCGGCAGGCGGCCATAGCCGTGGGCGC, GATAAGCT, wherein GATAAGCT is the sequence of AmiR-21, and the phosphate backbone between adjacent nucleotides is phosphorothioate modified.

Drug 89): HBsAg aptamer-CD40 aptamer-PD-L1 aptamer-DNA carrier; the HBsAg aptamer is connected to the 5' end of the sequence a of the DNA carrier via the linker sequence TTTTT; the CD40 aptamer is connected to the 5' end of the sequence b of the DNA carrier via the linker sequence TTTTT; and the PD-L1 aptamer is connected to the 5' end of the sequence c of the DNA carrier via the linker sequence TTTTT;

preferably, the HBsAg aptamer-linker sequence TTTTT-sequence a:
SEQ ID No. 329: CACAGCGAACAGCGGCGGACATAATAGTGCTTACTACGAC**TTTTT**GCGACGCCCACGAG C GTTCCGGGAGAGGAGC, with phosphorothioate modifications in the phosphate backbone between each pair of adjacent nucleotides among the first four nucleotides at the 5' end; the portion preceding the linker sequence TTTTT is the HBsAg aptamer sequence, and the portion following the linker sequence TTTTT is the sequence a;

preferably, the CD40 aptamer-linker sequence TTTTT-sequence b:
SEQ ID No. 330: CCAACGAGTAGGCGATAGCGCGTGG**TTTTT**GCTCCTCTCCCGGTTCGCCGCGAGCCGCG, with phosphorothioate modifications in the phosphate backbone between each pair of adjacent nucleotides among the first three nucleotides at the 5' end and among the last three nucleotides at the 3' end; the portion preceding the linker sequence TTTTT is the CD40 aptamer sequence, and the portion following the linker sequence TTTTT is the sequence b;

preferably, the PD-L1 aptamer-linker sequence TTTTT-sequence c:
SEQ ID No. 331: ACGGGCCACATCAACTCATTGATAGACAATGCGTCCACTGCCCGT**TTTTTCGCGGCTCG C GGCCATAGCCGTGGGCGTCGC,** with phosphorothioate modifications in the phosphate backbone between each pair of adjacent nucleotides among the first three nucleotides at the 5' end and among the last three nucleotides at the 3' end; the portion preceding the linker sequence TTTTT is the PD-L1 aptamer sequence, and the portion following the linker sequence TTTTT is the sequence c.

Drug 90): HBsAg aptamer-CD40 aptamer-PD-L1 aptamer-DNA carrier; compared with Drug 89), except for the sequence of the PD-L1 aptamer, all other aspects are identical; wherein the PD-L1 aptamer-linker sequence TTTTT-sequence c:

SEQ ID No. 311: AACAACGTATAACAATGCCCACGTCACCAGAGTACTATGGTTTTTCGCGGCTCGCGGCC ATAGC CGTGGGCGTCGC, wherein the ribose of each of the first six nucleotides at the 5' end is 2'-O-MOE modified; the portion preceding the linker sequence TTTTT is the PD-L1 aptamer sequence, and the portion following the linker sequence TTTTT is the sequence c.

Drug 91): BCMA aptamer-CD38 aptamer-CpG-BYZD-CD19 aptamer-AmiR-21-DNA carrier, wherein the sequences a, b and c of the DNA carrier are as follows:

The sequence a is SEQ ID No. 15: GACGCCCACGAGCGTTCCGGGAGAGG<u>TGTAGCACGGTGGC</u>, wherein the underlined portion is the single-stranded linker sequence A;

The sequence b is SEQ ID No. 16: CCTCTCCCGGTTCGCCGCGAGCC<u>TCGGCGCGGCCGTG</u>, wherein the underlined portion is the single-stranded linker sequence B;

The sequence c is SEQ ID No. 17: GGCTCGCGGCCATAGCCGTGGGCGTC<u>TGCTGCTGCTGCTG</u>, wherein the underlined portion is the single-stranded linker sequence C;

The CD38 aptamer is connected, via the linker sequence TT and CpG-BYZD, to the complementary sequence A' of the single-stranded linker sequence A, and the complementary sequence A' is complementarily paired with the single-stranded linker sequence A; wherein the CD38 aptamer-CpG-BYZD-complementary sequence A' is:
SEQ ID No. 332: TACGTGAATCTCGTACGATACTCTGTAAGCGT**TT**<u>AGCGAA</u>GCCACCGTGCTACA, wherein SEQ ID No. 170: TACGTGAATCTCGTACGATACTCTGTAAGCGT is the CD38 aptamer, with phosphorothioate modifications in the phosphate backbone between each pair of adjacent nucleotides among the first four nucleotides at the 5' end; SEQ ID No. 8: GCCACCGTGCTACA is the complementary sequence A'; TT is the linker sequence; and

AGCGAA 为 CpG-BYZD;

The CD19 aptamer is connected to the 5' end of the complementary sequence B' of the single-stranded linker sequence B via AmiR-21, and the complementary sequence B' is complementarily paired with the single-stranded linker sequence B; wherein the CD19 aptamer-AmiR-21-complementary sequence B' is:
SEQ ID No. 333: UGAGCCCUGUUCGACAGGAGGCUCA**GAUAAGCU**<u>CACGGCCGCGCCGA</u>, wherein SEQ ID No. 160: UGAGCCCUGUUCGACAGGAGGCUCA is the CD19 aptamer sequence, with 2'-F modifications on C and U bases; GAUAAGCU is the sequence of AmiR-21, with 2'-F modifications on C and U; and SEQ ID No. 10: CACGGCCGCGCCGA is the complementary sequence B';

The BCMA aptamer is connected to the 5' end of the complementary sequence C' of the single-stranded linker sequence C via the linker sequence AA, and the complementary sequence C' is complementarily paired with the single-stranded linker sequence C; wherein the BCMA aptamer-linker sequence AA-complementary sequence C' is:
SEQ ID No. 334: AGUGCAAGACGUUCGCAGAUUAGCGAAAAGAGGGUCUCAUUGACUAGUAC**AA**<u>CAGC AG-CAGCAGCA</u>, wherein SEQ ID No. 12: CAGCAGCAGCAGCA is the complementary sequence C'; and SEQ ID No. 140: AGUGCAAGACGUUCGCAGAUUAGCGAAAAGAGGGUCUCAUUGACUAGUAC is the BCMA aptamer sequence, in which the C and U bases in the RNA are 2'-F modified.

Drug 92): BCMA aptamer-CD38 aptamer-CD19 aptamer-AmiR-21-DNA carrier, wherein the sequences a, b and c of the DNA carrier are as follows:

The sequence a is SEQ ID No. 15: GACGCCCACGAGCGTTCCGGGAGAGG<u>TGTAGCACGGTGGC</u>, wherein the underlined portion is the single-stranded linker sequence A;

The sequence b is SEQ ID No. 16: CCTCTCCCGGTTCGCCGCGAGCC<u>TCGGCGCGGCCGTG</u>, wherein the underlined portion is the single-stranded linker sequence B;

The sequence c is:: SEQ ID No. 17: GGCTCGCGGCCATAGCCGTGGGCGTC<u>TGCTGCTGCTGCTG</u>, wherein the underlined portion is the single-stranded linker sequence C;

The CD38 aptamer is connected, via the linker sequence TTTTT, to the complementary sequence A' of the single-stranded linker sequence A, and the complementary sequence A' is complementarily paired with the single-stranded linker sequence A; wherein the sequence of the CD38 aptamer-linker sequence TTTTT-complementary sequence A' is:

SEQ ID No. 335: TACGTGAATCTCGTACGATACTCTGTAAGCGT**TTTTT**GCCACCGTGCTACA, SEQ ID No. 170: TACGTGAATCTCGTACGATACTCTGTAAGCGT is the CD38 aptamer sequence, with phosphorothioate modifications in the phosphate backbone between each pair of adjacent nucleotides among the first four nucleotides at the 5' end; and SEQ ID No. 8: GCCACCGTGCTACA is the complementary sequence A';

The CD19 aptamer is connected to the complementary sequence B' of the single-stranded linker sequence B via AmiR-21, and the complementary sequence B' is complementarily paired with the single-stranded linker sequence B; wherein the sequence of the CD19 aptamer-AmiR-21-complementary sequence B' is:
SEQ ID No. 333: UGAGCCCUGUUCGACAGGAGGCUCA**GAUAAGCU**CACGGCCGCGCCGA, wherein SEQ ID No. 160: UGAGCCCUGUUCGACAGGAGGCUCA is the CD19 aptamer sequence, with 2'-F modifications on C and U bases; GAUAAGCU is the sequence of AmiR-21, with 2'-F modifications on C and U; and SEQ ID No. 10: CACGGCCGCGCCGA is the complementary sequence B';

The BCMA aptamer is connected to the 5' end of the complementary sequence C' of the single-stranded linker sequence C via the linker sequence AA, and the complementary sequence C' is complementarily paired with the single-stranded linker sequence C; wherein the sequence of the BCMA aptamer-linker sequence AA-complementary sequence C' is:
SEQ ID No. 334: AGUGCAAGACGUUCGCAGAUUAGCGAAAAGAGGGUCUCAUUGACUAGUAC**AA**CAGC AGCAGCAGCA, wherein SEQ ID No. 12: CAGCAGCAGCAGCA is the complementary sequence C'; and SEQ ID No. 140: AGUGCAAGACGUUCGCAGAUUAGCGAAAAGAGGGUCUCAUUGACUAGUAC is the BCMA aptamer sequence, in which the C and U bases in the RNA are 2'-F modified.

Drug 93): CpG2006-CD38 aptamer-PD-L1 aptamer-DNA carrier, wherein the sequences a, b and c of the DNA carrier are as follows:

The sequence a is SEQ ID No. 15: GACGCCCACGAGCGTTCCGGGAGAGG<u>TGTAGCACGGTGGC,</u> wherein the underlined portion is the single-stranded linker sequence A;

The sequence b is SEQ ID No. 16: CCTCTCCCGGTTCGCCGCGAGCC<u>TCGGCGCGGCCGTG,</u> wherein the underlined portion is the single-stranded linker sequence B;

The sequence c is:: SEQ ID No. 17: GGCTCGCGGCCATAGCCGTGGGCGTCT<u>GCTGCTGCTGCTG,</u> wherein the underlined portion is the single-stranded linker sequence C;

The CD38 aptamer is connected, via the linker sequence TTTTT, to the complementary sequence A' of the single-stranded linker sequence A, and the complementary sequence A' is complementarily paired with the single-stranded linker sequence A to attach at the 3' end of the sequence a; wherein the sequence of the CD38 aptamer-linker sequence TTTTT-complementary sequence A' is:
SEQ ID No. 335: TACGTGAATCTCGTACGATACTCTGTAAGCGT**TTTTT**GCCACCGTGCTACA, SEQ ID No. 170: TACGTGAATCTCGTACGATACTCTGTAAGCGT is the CD38 aptamer sequence, with phosphorothioate modifications in the phosphate backbone between each pair of adjacent nucleotides among the first four nucleotides at the 5' end; and SEQ ID No. 8: GCCACCGTGCTACA is the complementary sequence A';

the PD-L1 aptamer is connected to the 5' end of the complementary sequence B' of the single-stranded linker sequence B via the linker sequence TTTTT, and is attached to the 3' end of the sequence b through complementary pairing between the complementary sequence B' and the single-stranded linker sequence B;

CpG2006 is connected to the 5' end of the complementary sequence C' of the single-stranded linker sequence C via the linker sequence TTTTT, and is attached to the 3' end of the sequence c through complementary pairing between the complementary sequence C' and the single-stranded linker sequence C;

The sequence of the PD-L1 aptamer-linker sequence TTTTT-complementary sequence B' is: SEQ ID No. 336: ACGGGCCACATCAACTCATTGATAGACAATGCGTCCACTGCCCGT**TTTTT**CACGGCCGCG CCGA, with phosphorothioate modifications in the phosphate backbone between each pair of adjacent nucleotides among the first four nucleotides at the 5' end; wherein SEQ ID No. 241: ACGGGCCACATCAACTCATTGATAGACAATGCGTCC ACTGCCCGT is the PD-L1 aptamer sequence, and SEQ ID No. 10: CACGGCCGCGCCGA is the complementary sequence B';

The sequence of the CpG2006-linker sequence TTTTT-complementary sequence C' is:

SEQ ID No. 337: TCGTCGTTTTGTCGTTTTGTCGTT**TTTTT**CAGCAGCAGCAGCA, wherein SEQ ID No. 57: TC GTCGTTTTGTCGTTTTGTCGTT is the CpG2006 sequence, with phosphorothioate modifications in the phosphate backbone between adjacent nucleotides of CpG2006; and SEQ ID No. 12: CAGCAGCAGCAGCA is the complementary sequence C'.

Drug 94): BCMA aptamer-CD38 aptamer-CpG-BYZD-PD-L1 aptamer-DNA carrier, wherein the sequences a, b and c of the DNA carrier are as follows:

The sequence a is SEQ ID No. 15: GACGCCCACGAGCGTTCCGGGAGAGG<u>TGTAGCACGGTGGC,</u> wherein the underlined portion is the single-stranded linker sequence A;

The sequence b is SEQ ID No. 16: CCTCTCCCGGTTCGCCGCGAGCC<u>TCGGCGCGGCCGTG,</u> wherein the underlined portion is the single-stranded linker sequence B;

The sequence c is SEQ ID No. 17: GGCTCGCGGCCATAGCCGTGGGCGTC<u>TGCTGCTGCTGCTG,</u> wherein the underlined portion is the single-stranded linker sequence C;

The CD38 aptamer is connected, via CpG-BYZD, to the complementary sequence A' of the single-stranded linker sequence A, and the complementary sequence A' is complementarily paired with the single-stranded linker sequence A to attach at the 3' end of the sequence a; wherein the CD38 aptamer-CpG-BYZD-complementary sequence A' is: SEQ ID No. 332: TACGTGAATCTCGTACGATACTCTGTAAGCGT**TT**AGCGAAGCCACCGTGCTACA, SEQ ID No. 170: TACGTGAATCTCGTACGATACTCTGTAAGCGT is the CD38 aptamer sequence, with phosphorothioate modifications in the phosphate backbone between each pair of adjacent nucleotides among the first four nucleotides at the 5' end; SEQ ID No. 8: GCCACCGTGCTACA is the complementary sequence A'; TT is the linker sequence; and AGCGAA is CpG-BYZD;

The PD-L1 aptamer is connected to the 5' end of the complementary sequence B' of the single-stranded linker sequence B via the linker sequence TTTTT, and is attached to the 3' end of the sequence b through complementary pairing between the complementary sequence B' and the single-stranded linker sequence B;

The BCMA aptamer is directly connected to the 5' end of the complementary sequence C' of the single-stranded linker sequence C, and is attached to the 3' end of the sequence c through complementary pairing between the complementary sequence C' and the single-stranded linker sequence C;

The PD-L1 aptamer-linker sequence TTTTT-complementary sequence B' is SEQ ID No. 336: ACGGGCCACATC AACTCATTGATAGACAATGCGTCCACTGCCCGTTTTTTCACGGCCGCG CCGA,with phosphorothioate modifi- cations in the phosphate backbone between each pair of adjacent nucleotides among the first four nucleotides at the 5' end; wherein SEQ ID No. 241: ACGGGCCACATCAACTCATTGATAGACAATGCGTCCACTGCCCGT is the PD-L1 aptamer sequence, and SEQ ID No. 10: CACGGCCGCGCCGA is the complementary sequence B';

The BCMA aptamer-linker sequence AA-complementary sequence C' :

SEQ ID No. 334: AGUGCAAGACGUUCGCAGAUUAGCGAAAAGAGGGUCUCAUUGACUAGUACAACAGC AGCAGCAGCA,wherein SEQ ID No. 140: AGUGCAAGACGUUCGCAGAUUAGCGAAAAGAGGGUCUCAUUGACU AGUAC is the BCMA aptamer sequence, in which C and U bases in the RNA are 2'-F modified; and SEQ ID No. 12: CAGCAGCAGCAGCA is the complementary sequence C'.

Drug 95): BCMA aptamer-CD40 aptamer-PD-L1 aptamer-DNA carrier, wherein the sequences a, b and c of the DNA carrier are as follows:

The sequence a is SEQ ID No. 15: GACGCCCACGAGCGTTCCGGGAGAGG<u>TGTAGCACGGTGGC,</u> wherein the underlined portion is the single- stranded linker sequence A;

The sequence b is SEQ ID No. 16: CCTCTCCCGGTTCGCCGCGAGCC<u>TCGGCGCGGCCGTG,</u> wherein the underlined portion is the single-stranded linker sequence B;

The sequence c is SEQ ID No. 17: GGCTCGCGGCCATAGCCGTGGGCGTCTGCT<u>GCTGCTGCTG,</u> wherein the

underlined portion is the single-stranded linker sequence C;

The CD40 aptamer is connected, via the linker sequence TTTTT, to the complementary sequence A' of the single-stranded linker sequence A, and the complementary sequence A' is complementarily paired with the single-stranded linker sequence A to attach at the 3' end of the sequence a; wherein the sequence of the CD40 aptamer-linker sequence TTTTT-complementary sequence A' is:
SEQ ID No. 338: GCCAACGAGTAGGCGATAGCGCGTGGCT**TTTT**GCCACCGTGCTACA,wherein SEQ ID No. 156: GCCAACGAGTAGGCGATAGCGCGTGGC is the CD40 aptamer sequence, with phosphorothioate modifications in the phosphate backbone between each pair of adjacent nucleotides among the first four nucleotides at the 5' end; and SEQ ID No. 8: GCCACCGTGCTACA is the complementary sequence A';

The PD-L1 aptamer is connected to the 5' end of the complementary sequence B' of the single-stranded linker sequence B via the linker sequence TTTTT, and is attached to the 3' end of the sequence b through complementary pairing between the complementary sequence B' and the single-stranded linker sequence B;

The BCMA aptamer is directly connected to the 5' end of the complementary sequence C' of the single-stranded linker sequence C, and is attached to the 3' end of the sequence c through complementary pairing between the complementary sequence C' and the single-stranded linker sequence C;

The sequence of the PD-L1 aptamer-linker sequence TTTTT-complementary sequence B' is: SEQ ID No. 336: ACGGGCCACATCAACTCATTGATAGACAATGCGTCCACTGCCCGT**TTTTT**CACGGCCGCG CCGA,with phosphorothioate modifications in the phosphate backbone between each pair of adjacent nucleotides among the first four nucleotides at the 5' end; wherein SEQ ID No. 241: ACGGGCCACATCAACTCATTGATAGACAATGCGTCC ACTGCCCGT is the PD-L1 aptamer sequence, and SEQ ID No. 10: CACGGCCGCGCCGA is the complementary sequence B';

The sequence of the BCMA aptamer-linker sequence AA-complementary sequence C' is:
SEQ ID No. 334: AGUGCAAGACGUUCGCAGAUUAGCGAAAAGAGGGUCUCAUUGACUAGUACAACAGC AGCAGCAGCA,wherein SEQ ID No. 140: AGUGCAAGACGUUCGCAGAUUAGCGAAAAGAGGGUCUCAUUGACU AGUAC is the BCMA aptamer sequence, in which the C and U bases in the RNA are 2'-F modifications; and SEQ ID No. 12: CAGCAGCAGCAGCA is the complementary sequence C'.

Drug 96): AmiR-21-AS1411 aptamer-CD40 aptamer-2*biotin-DNA carrier, wherein the DNA carrier is the nucleic acid carrier of the aforesaid twenty-six backbone sequence sets of group 1; AmiR-21 is connected to the 5' end of the sequence a; the AS1411 aptamer is connected to the 5' end of the sequence b via the linker sequence TTTTT; the CD40 aptamer is connected to the 5' end of the sequence c via the linker sequence TTTTT; one biotin modification is located at the 3' end of the sequence a and the other biotin modification is located at the 3' end of the sequence c;

The sequence ofAmiR-21 is GATAAGCT, wherein each nucleotide is locked nucleic acid (LNA) modified;

The sequence of the AS1411 aptamer is SEQ ID No. 131: GGTGGTGGTGGTTGTGGTGGTGGTGG;

The sequence of the CD40 aptamer is SEQ ID No. 154: CCAACGAGTAGGCGATAGCGCGTGG.

Drug 97): 2*AmiR-21-4*biotin-DNA carrier;

Wherein the DNA carrier is the nucleic acid carrier of the aforesaid twenty-six backbone sequence sets of group 11; two AmiR-21 units are respectively and directly connected to the 5' ends of the sequence a and the sequence c; and four biotin modifications are respectively attached at the 3' end of the sequence a, the 5' end and the 3' end of the sequence b, and the 3' end of the sequence c;

preferably, the sequence of AmiR-21 is GATAAGCT, wherein each nucleotide is locked nucleic acid (LNA) modified.

Drug 98): TTA1 aptamer-doxorubicin-4*biotin-DNA carrier;

Wherein the DNA carrier is the nucleic acid carrier of the aforesaid twenty-six backbone sequence sets of group 13; four AmiR-21 units are respectively and directly connected to the 5' and 3' ends of the sequence a and the sequence c; the TTA1 aptamer is connected to the 5' end of the sequence b via a linker sequence; and doxorubicin is inserted, in a specifically intercalative manner, between adjacent GC base pairs of the nucleic acid carrier;

preferably, the sequence of the TTA1 aptamer:
SEQ ID No. 265: CCTGCACTTGGCTTGGATTTCAGAAGGGAGACCC.

Drug 99): TTA1 aptamer-2*Survivin siRNA-DNA carrier;

Wherein the DNA carrier is the nucleic acid carrier of the aforesaid twenty-six backbone sequence sets of group 13; the TTA1 aptamer is connected to the 5' end of the sequence b via a linker sequence; the sense strands of two Survivin siRNAs are connected to the 3' ends of the sequence b and the sequence c, respectively; and the antisense strands of the two Survivin siRNAs are connected to the 3' ends of the sequence b and the sequence c through complementary pairing with the respective sense strands;

preferably, the TTA1 aptamer:
SEQ ID No. 265: CCTGCACTTGGCTTGGATTTCAGAAGGGAGACCC;

preferably, the antisense strand of Survivin siRNA is SEQ ID No. 300: UGUGACAGAUAAGGAACCUGCAG;

The sense strand of Survivin siRNA is SEQ ID No. 100: GCAGGUUCCUUAUCUGUCACAUU and the phosphate backbone of both the sense and antisense strands are phosphorothioate modified between each pair of adjacent nucleotides among the first three nucleotides at the 5' end and among the last three nucleotides at the 3' end.

Drug 100): CD16a aptamer-CD40 aptamer-CpG2006-DNA carrier, wherein the DNA carrier is the nucleic acid carrier of the aforesaid nucleic acid carriers of group 6; the CD 16a aptamer is connected to the 5' end of the sequence a via a linker sequence; the CD40 aptamer is connected to the 3' end of the sequence b via a linker sequence; and CpG2006 is connected to the 5' end of the sequence c via a linker sequence;

preferably, the sequence of the CD16a aptamer: CCACTGCGGGGGTCTATACGTGAGGAAGAAGTGG, with phosphorothioate modifications in the phosphate backbone between each pair of adjacent nucleotides among the first three nucleotides at the 5' end;

preferably, the sequence of the CD40 aptamer: CCAACGAGTAGGCGATAGCGCGTGG, with phosphorothioate modifications in the phosphate backbone between each pair of adjacent nucleotides among the first three nucleotides at the 5' end;

preferably, the sequence of CpG2006 is SEQ ID No. 57: TCGTCGTTTTGTCGTTTTGTCGTT, with phosphorothioate modifications in the phosphate backbone between adjacent nucleotides throughout the sequence;

preferably, the linker sequence is TTTTT.

In a third exemplary embodiment of the present application, there is provided a pharmaceutical composition comprising any one or more of the above nucleic acid drugs and, optionally, a pharmaceutically acceptable carrier and/or excipient.

Further, in the pharmaceutical composition, at least one of the drugs is the nucleic acid drug above, and the pharmaceutical composition is selected from any one of the following combinations:

Combination 1): Drug 2) + Drug 21);

Combination 2): Drug 6) + Drug 26);

Combination 3): Drug 6) + an OX40 aptamer in RNA form;

Combination 4): Drug 3) + Drug 26);

Combination 5): Drug 3) + Drug 21) + an OX40 aptamer in DNA form;

Combination 6): Drug 4) + Drug 21) + an OX40 aptamer in DNA form;

Combination 7): Drug 4) + Drug 21);

Combination 8): Drug 3) + Drug 23);

Combination 9): Drug 4) + Drug 23);

Combination 10): Drug 3) + Drug 24) + an OX40 aptamer in DNA form;

Combination 11): Drug 3) + Drug 41);

Combination 12): Drug 5) + Drug 41);

Combination 13): Drug 5) + Drug 96);

Combination 14): Drug 96) + Drug 41);

Combination 15): Drug 3) + Drug 97) + an OX40 aptamer in DNA form;

Combination 16): Drug 4) + Drug 20) + an OX40 aptamer in DNA form;

Combination 17): Drug 4) + Drug 20);

Combination 18): Drug 7) + Drug 25) + an OX40 aptamer in DNA form;

Combination 19): Drug 1) + Drug 18) + Drug 98);

Combination 20): Drug 1) + Drug 18) + an OX40 aptamer in DNA form + Drug 99);

Combination 21): Drug 3) + Drug 18) + an OX40 aptamer in DNA form;

Combination 22): Drug 3) + Drug 18);

Combination 23): Drug 3) + an OX40 aptamer in DNA form;

Combination 24): Drug 3) + Drug 18) + an OX40 aptamer in locked nucleic acid (LNA) form;

Combination 25): Drug 3) + Drug 18) + an OX40 aptamer in RNA form;

Combination 26): Drug 3) + Drug 18) + a CD40 aptamer in DNA form;

Combination 27): Drug 3) + Drug 20) + a CD40 aptamer in DNA form;

Combination 28): Drug 4) + Drug 19) + a CD40 aptamer in DNA form.

Wherein, the sequence of the OX40 aptamer in DNA form:
SEQ ID No. 227: CAGTCTGCATCGTAGGATTAGCCACCGUATCTTTCCCAC;
The sequence of the OX40 aptamer in RNA form:

SEQ ID No. 226:
GGGAGGACGAUGCGGCAGUCUGCAUCGUAGGAAUCGCCACCGUAUACUUUCCCACCA
GACGACUCGCUGAGGAUCCGAGA;

The OX40 aptamer in locked nucleic acid form is defined on the basis of the sequence of the OX40 aptamer in DNA form, wherein the first five nucleotides from the 5' end and the first four nucleotides from the 3' end are locked nucleic acid (LNA) modifications.
In a fourth exemplary embodiment of the present application, there is provided the use of any one of the foregoing nucleic acid carriers, any one of the foregoing nucleic acid drugs, or any one of the foregoing pharmaceutical compositions in the preparation of a medicament for treating tumors, liver diseases other than liver cancer, or diseases caused by coronavirus infection.

Further, the liver disease other than liver cancer is hepatitis B or hepatitis C; the diseases caused by coronavirus infection include coronavirus disease 2019 (COVID-19); the tumor is selected from andrological tumors, gynecologic tumors, respiratory system tumors, digestive system tumors, hematologic tumors, urinary system tumors, bone tumors, neurologic tumors, dermatologic tumors, general surgery tumors, or otorhinolaryngologic tumors. preferably, the andrological tumor is selected from prostate cancer, penile cancer, testicular tumor, or male urethral carcinoma; the gynecologic tumor is selected from ovarian cancer, cervical cancer, endometrial cancer, uterine fibroid (leiomyoma), vulvar cancer, or malignant hydatidiform mole; the respiratory system tumor is selected from lung cancer, non-small cell lung cancer (NSCLC), small cell lung cancer (SCLC), nasopharyngeal carcinoma, tracheal tumor, lung cancer metastasis, pulmonary inflammatory pseudotumor, or radiation-induced lung cancer; the digestive system tumor is selected from liver cancer, gastric cancer, colorectal cancer, gallbladder cancer, esophageal cancer, rectal cancer, pancreatic cancer, or colon cancer; the hematologic tumor is selected from leukemia, lymphoma, lymphosarcoma, or multiple myeloma; the urinary system tumor is selected from renal cancer, bladder cancer, or urinary tract cancer; the bone tumor is selected from giant cell tumor of bone, osteochondroma, or osteosarcoma; the neurologic tumor is selected from brain tumor, meningioma, intracranial tuberculoma, pituitary tumor, neuroblastoma, glioblastoma, or glioma; the dermatologic tumor is selected from skin cancer or melanoma; the general surgery tumor is selected from breast cancer, lipoma, thyroid cancer, or thyroid tumor; and the otorhinolaryngologic tumor is selected from oral cancer, tongue cancer, laryngeal cancer, middle ear cancer, gingival cancer, or intraorbital tumor.

Further, in use, the route of administration of the drug is intratumoral administration, intravenous administration, or intraperitoneal administration.

Further, in use, the daily administration dose of the drug is 0.1 $\mu$g/kg to 100 mg/kg.

In a fifth exemplary embodiment of the present application, there is provided a method for preparing any one of the foregoing nucleic acid drugs, the method comprising obtaining the nucleic acid drug by a self-assembly process involving at least one strand, wherein a nucleic acid carrier comprising a sequence a, a sequence b, and a sequence c and at least one oligonucleotide effector molecule loaded onto the nucleic acid carrier are subjected to self-assembly; the oligonucleotide effector molecule is selected from at least one of: a nucleic acid immunostimulant, a nucleic acid aptamer, siRNA, miRNA, and an antisense oligonucleotide (ASO).

Further, the nucleic acid drug is obtained by self-assembly of 2-8 strands, preferably 3-6 strands.

Further, when self-assembly with more than three strands is employed, the method includes: providing the following three sequences, in modified or unmodified form, for self-assembly to form the nucleic acid carrier: the sequence a, the sequence b, and the sequence c, wherein "modified" means modified with a targeting small chemical molecule; and, by sequence complementarity, loading the sequence of at least one oligonucleotide effector molecule onto the 5' end and/or the 3' end of at least one of the sequence a, the sequence b, and the sequence c; wherein the oligonucleotide effector molecule is selected from at least one of: a nucleic acid immunostimulant, a nucleic acid aptamer, siRNA, miRNA, and an antisense oligonucleotide (ASO).

Further, where the oligonucleotide effector molecule includes an siRNA, the sense strand of the siRNA is synthesized at the 5' end and/or the 3' end of at least one of the sequence a, the sequence b, and the sequence c, in modified or unmodified form, and the antisense strand of the siRNA is co-assembled together with the sequence a, the sequence b, and the sequence c, in modified or unmodified form, such that the siRNA is linked to the nucleic acid carrier through complementary pairing between the antisense and sense strands of the siRNA; preferably, the sense strand of the siRNA is directly synthesized at the 5' end and/or the 3' end of at least one of the sequence a, the sequence b, and the sequence c via a linker sequence; preferably, the linker sequence consists of 2-8, more preferably 3-6, consecutive T, A, or U nucleotides.

Further, where the oligonucleotide effector molecule includes an miRNA and/or a nucleic acid immunostimulant, the miRNA is directly synthesized and/or the nucleic acid immunostimulant is synthesized via a linker sequence at the 5' end and/or the 3' end of at least one of the sequences a, b, and c, in modified or unmodified form, and the miRNA and/or the nucleic acid immunostimulant is/are loaded onto the nucleic acid carrier by self-assembly of the modified or unmodified sequences a, b, and c; preferably, the linker sequence consists of 2-8, more preferably 3-6, consecutive T or A nucleotides.

Further, where the oligonucleotide effector molecule includes a nucleic acid aptamer, the nucleic acid aptamer is synthesized at the 5' end and/or the 3' end of at least one of the sequences a, b, and c, in modified or unmodified form, and is loaded onto the nucleic acid carrier by self-assembly of the modified or unmodified sequences a, b, and c; preferably, the nucleic acid aptamer is directly synthesized via a linker sequence at the 5' end and/or the 3' end of at least one of the sequences a, b, and c; preferably, the linker sequence consists of 2-8, more preferably 3-6, consecutive T or A nucleotides.

Further, where the oligonucleotide effector molecule, in addition to comprising an siRNA, further includes a nucleic acid immunostimulant and/or a nucleic acid aptamer, the nucleic acid immunostimulant is synthesized via a linker sequence at the 5' end and/or the 3' end of at least one of the sequences a, b, and c, in modified or unmodified form, at an end different from the end at which the siRNA sense strand is located, and/or the nucleic acid aptamer is synthesized via a linker sequence at the 5' end of the siRNA antisense strand to obtain an aptamer-siRNA fragment; the aptamer-siRNA fragment is co-incubated with the modified or unmodified sequences a, b, and c on which the nucleic acid immunostimulant has been synthesized to undergo self-assembly, thereby loading the nucleic acid aptamer onto the nucleic acid carrier through complementary pairing between the siRNA antisense strand and the siRNA sense strand.

Further, where the oligonucleotide effector molecule includes an miRNA and a nucleic acid aptamer, the nucleic acid aptamer is synthesized at the 5' or 3' end of the miRNA to obtain an aptamer-miRNA fusion single-stranded sequence; the aptamer-miRNA fusion single-stranded sequence is allowed to participate in self-assembly, thereby loading the nucleic acid aptamer and the miRNA onto the nucleic acid carrier.

Further, where the oligonucleotide effector molecule includes a nucleic acid immunostimulant and a nucleic acid aptamer, the nucleic acid immunostimulant and the nucleic acid aptamer are each independently synthesized at either end of at least one of the sequences a, b, and c, in modified or unmodified form, to obtain nucleic acid immunostimulant and/or nucleic acid aptamer fusion sequences; the nucleic acid immunostimulant and/or nucleic acid aptamer fusion sequences are allowed to participate in self-assembly, thereby loading the nucleic acid aptamer and the nucleic acid immunostimulant onto the nucleic acid carrier.

Further, the sequences a, b, and c respectively include a carrier backbone sequence and a single-stranded bridging sequence, and one or more oligonucleotide effector molecules self-assemble with the sequences a, b, and c through sequence a complementary to the single-stranded bridging sequence to form the nucleic acid drug; preferably, the sequences a, b, and c are respectively as follows:

The sequence a is SEQ ID No. 15: GACGCCCACGAGCGTTCCGGGAGAGG<u>TGTAGCACGGTGGC</u>, wherein the underlined portion is the single-stranded connecting-bridge sequence A;

The sequence b is SEQ ID No. 16: CCTCTCCCGGTTCGCCGCGAGCC<u>TCGGCGCGGCCGTG</u>, wherein the underlined portion is the single-stranded connecting-bridge sequence B;

The sequence c is SEQ ID No. 17: GGCTCGCGGCCATAGCCGTGGGCGTC<u>TGCTGCTGCTGCTG</u>, wherein the underlined portion is the single-stranded connecting-bridge sequence C.

Further, the nucleic acid drug is a CpG2006-CD40 aptamer-PD-L1 aptamer-DNA carrier, and the method of preparation includes any one of the following self-assembly approaches;

1) Three-strand self-assembly, wherein the three strands are as follows:

CpG2006-sequence a:
SEQ ID No. 309: TCGTCGTTTTGTCGTTTTGTCGTT**TTTTT**GCGACGCCCACGAGCGTTCCGGGAGAGGAG C,wherein the phosphate backbone between adjacent nucleotides at positions 1-24 from the 5' end are phosphorothioate modified; "TTTTT" is a linker sequence, the portion preceding "TTTTT" is the CpG2006 sequence, and the portion following "TTTTT" is the sequence a;

CD40 aptamer-sequence b:
SEQ ID No. 330: CCAACGAGTAGGCGATAGCGCGTGG**TTTTT**GCTCCTCTCCCGGTTCGCCGCGAGCCG CG, wherein the phosphate backbone between adjacent nucleotides at positions 1-5 from the 5' end is phosphorothioate modified; "TTTTT" is a linker sequence, the portion preceding "TTTTT" is the CD40 aptamer sequence, and the portion following "TTTTT" is the sequence b; and

PD-L1 aptamer-sequence c:
SEQ ID No. 331: ACGGGCCACATCAACTCATTGATAGACAATGCGTCCACTGCCCG**TTTTTT**CGCGGCTC GC GGCCATAGCCGTGGGCGTCGC,wherein the phosphate backbone between adjacent nucleotides at positions 1-5 from the 5' end is phosphorothioate modified; "TTTTT" is a linker sequence, the portion preceding "TTTTT" is the PD-L1 aptamer sequence, and the portion following "TTTTT" is the sequence c;

2) Four-strand self-assembly, wherein the four strands:

CpG2006-sequence a:
SEQ ID No. 309: TCGTCGTTTTGTCGTTTTGTCGTT**TTTTT**GCGACGCCCACGAGCGTTCCGGGAGAGGAG C,wherein the phosphate backbone between adjacent nucleotides at positions 1-24 from the 5' end is phosphorothioate modified; "TTTTT" is a linker sequence; the portion preceding "TTTTT" is the CpG2006 sequence, and the portion following "TTTTT" is the sequence a;

CD40 aptamer-sequence b:

SEQ ID No. 339: CCAACGAGTAGGCGATAGCGCGTGG**TTT**GCTCCTCTCCCGGTTCGCCGCGAGCCTCG GC GCGGCCGTG,wherein the phosphate backbone between adjacent nucleotides at positions 1-4 from the 5' end is phosphorothioate modified; "TTT" is a linker sequence; the portion preceding "TTT" is the CD40 aptamer sequence, and the portion following "TTT" is the sequence b;

PD-L1 aptamer-linker sequence-complementary sequence B' of the single-stranded bridging sequence B:

SEQ ID No. 336: ACGGGCCACATCAACTCATTGATAGACAATGCGTCCACTGCCCGT**TTTTT**CACGGCCG CG CCGA,wherein the phosphate backbone between adjacent nucleotides at positions 1-4 from the 5' end is phosphorothioate modified; "TTTTT" is a linker sequence; the portion preceding "TTTTT" is the PD-L1 aptamer sequence, and the portion following "TTTTT" is the complementary sequence B'; and

sequence c:

SEQ ID No. 340: GGCTCGCGGCCATAGCCGTGGGCGTCGC;

or, the four strands:

CpG2006-linker sequence-complementary sequence C' of the single-stranded bridging sequence C:

SEQ ID No. 341: TCGTCGTTTTGTCGTTTTGTCGTTT**TTTTT**CAGCAGCAGCAGCA, wherein the phosphate backbone between adjacent nucleotides at positions 1-24 from the 5' end are phosphorothioate modified; "TTTTT" is a linker sequence; the portion preceding "TTTTT" is the CpG2006 sequence, and the portion following "TTTTT" is the complementary sequence C';

sequence a: SEQ ID No. 18: GCGACGCCCACGAGCGTTCCGGGAGAGGAGC;

CD40 aptamer-sequence b:

SEQ ID No. 339: CCAACGAGTAGGCGATAGCGCGTGGTTTGCTCCTCTCCCGGTTCGCCGCGAGCC TCGGC GCGGCCGTG,wherein the phosphate backbone between adjacent nucleotides at positions 1-4 from the 5' end is phosphorothioate modified; "TTT" is a linker sequence; the portion preceding "TTT" is the CD40 aptamer sequence, and the portion following "TTT" is the sequence b;

PD-L1 aptamer-sequence c:

SEQ ID No. 342: ACGGGCCACATCAACTCATTGATAGACAATGCGTCCACTGCCCGT**TTTTT**GGCTCG CGGC CATAGCCGTGGGCGTCGC,wherein the phosphate backbone between adjacent nucleotides at positions 1-4 from the 5' end is phosphorothioate modified; "TTTTT" is a linker sequence; the portion preceding "TTTTT" is the PD-L1 aptamer sequence, and the portion following "TTTTT" is the sequence c;

3) Five-strand self-assembly, wherein the five strands:

sequence a: SEQ ID No. 15: GACGCCCACGAGCGTTCCGGGAGAGGTGTAGCACGGTGGC;

CD40 aptamer-linker sequence-sequence b:

SEQ ID No. 339: CCAACGAGTAGGCGATAGCGCGTGG**TTT**GCTCCTCTCCCGGTTCGCCGCGAGCCTCG GC GCGGCCGTG,wherein the phosphate backbone between adjacent nucleotides at positions 1-4 from the 5' end is phosphorothioate modified; "TTT" is a linker sequence; the portion preceding "TTT" is the CD40 aptamer sequence, and the portion following "TTT" is the sequence b;

PD-L1 aptamer-linker sequence-complementary sequence B' of the single-stranded bridging sequence B:

SEQ ID No. 336: ACGGGCCACATCAACTCATTGATAGACAATGCGTCCACTGCCCGT**TTTTT**CACGGCCG CG CCGA,wherein the phosphate backbone between adjacent nucleotides at positions 1-4 from the 5' end is phosphorothioate modified; "TTTTT" is a linker sequence; the portion preceding "TTTTT" is the PD-L1 aptamer sequence, and the portion following "TTTTT" is the complementary sequence B';

CpG2006 sequence-linker sequence-complementary sequence C' of the single-stranded bridging sequence C:

SEQ ID No. 337: TCGTCGTTTTGTCGTTTTGTCGTT**TTTTT**CAGCAGCAGCAGCA, wherein the phosphate backbone between adjacent nucleotides at positions 1-24 from the 5' end is phosphorothioate modified; "TTTTT" is a linker sequence; the portion preceding "TTTTT" is the CpG2006 sequence, and the portion following "TTTTT" is the complementary sequence C';

sequence c: SEQ ID No. 17: GGCTCGCGGCCATAGCCGTGGGCGTCTGCTGCTGCTGCTG;

or, the five strands:

CpG2006-linker sequence-sequence a:
SEQ ID No. 343: TCGTCGTTTGTCGTTTTGTCGTT**TTTTT**GACGCCCACGAGCGTTCCGGGAGAGG TGTAG CACGGTGGC,wherein the phosphate backbone between adjacent nucleotides at positions 1-24 from the 5' end is phosphorothioate modified; "TTTTT" is a linker sequence; the portion preceding "TTTTT" is the CpG2006 sequence, and the portion following "TTTTT" is the sequence a;

CD40 aptamer-linker sequence-complementary sequence A' of the single-stranded bridging sequence A:
SEQ ID No. 338: GCCAACGAGTAGGCGATAGCGCGTGGCT**TTTT**GCCACCGTGCTACA,wherein the phosphate backbone between adjacent nucleotides at positions 1-4 from the 5' end are phosphorothioate modified; "TTTTT" is a linker sequence; the portion preceding "TTTTT" is the CD40 aptamer sequence, and the portion following "TTTTT" is the complementary sequence A';

sequence b: SEQ ID No. 16: CCTCTCCCGGTTCGCCGCGAGCCTCGGCGCGGCCGTG;

PD-L1 aptamer - linker sequence - complementary sequence B' of the single-stranded bridging sequence B:
SEQ ID No. 336: ACGGGCCACATCAACTCATTGATAGACAATGCGTCCACTGCCCGT**TTTTT**CACGGC CGCG CCGA,wherein the phosphate backbone between adjacent nucleotides at positions 1-4 from the 5' end are phosphorothioate modified; "TTTTT" is a linker sequence; the portion preceding "TTTTT" is the PD-L1 aptamer sequence, and the portion following "TTTTT" is the complementary sequence B';

sequence c: SEQ ID No. 340: GGCTCGCGGCCATAGCCGTGGGCGTCGC;

4) Six-strand self-assembly, wherein the six strands:

sequence a: SEQ ID No. 15: GACGCCCACGAGCGTTCCGGGAGAGGTGTAGCACGGTGGC;

sequence b: SEQ ID No. 16: CCTCTCCCGGTTCGCCGCGAGCCTCGGCGCGGCCGTG;

sequence c: SEQ ID No. 17: GGCTCGCGGCCATAGCCGTGGGCGTCTGCTGCTGCTGCTG;

CpG2006-linker sequence-complementary sequence C' of the single-stranded bridging sequence C:
SEQ ID No. 337: TCGTCGTTTGTCGTTTTGTCGTT**TTTTT**CAGCAGCAGCAGCA; wherein the phosphate backbone between adjacent nucleotides at positions 1-24 from the 5' end are phosphorothioate modified; "TTTTT" is a linker sequence; the portion preceding "TTTTT" is the CpG2006 sequence, and the portion following "TTTTT" is the complementary sequence C';

CD40 aptamer-linker sequence-complementary sequence A' of the single-stranded bridging sequence A: SEQ ID No. 338: GCCAACGAGTAGGCGATAGCGCGTGGCT**TTTT**GCCACCGTGCTACA,wherein the phosphate backbone between adjacent nucleotides at positions 1-4 from the 5' end are phosphorothioate modified; "TTTTT" is a linker sequence; the portion preceding "TTTTT" is the CD40 aptamer sequence, and the portion following "TTTTT" is the complementary sequence A';

PD-L1 aptamer-linker sequence-complementary sequence B' of the single-stranded bridging sequence B:
SEQ ID No. 336: ACGGGCCACATCAACTCATTGATAGACAATGCGTCCACTGCCCGT**TTTTT**CACGGCCG CG CCGA,wherein the phosphate backbone between adjacent nucleotides at positions 1-4 from the 5' end are phosphorothioate modified; "TTTTT" is a linker sequence; the portion preceding "TTTTT" is the PD-L1 aptamer sequence, and the portion following "TTTTT" is the complementary sequence B'.

Further, the self-assembly step includes: dissolving the modified or unmodified sequences a, b, and c bearing at least one oligonucleotide effector molecule, and optionally the separately synthesized sequence(s) of the oligonucleotide effector molecule(s), in an assembly solution, and sequentially subjecting the solution to a denaturation reaction and a renaturation reaction to form a self-assembled crude product; subjecting the self-assembled crude product to purification, elution, and evaporation to dryness to obtain a targeted nucleic acid carrier bearing at least one oligonucleotide effector molecule. preferably, the molar ratio among the sequences a, b, and c is 0.90-1.10 : 0.90-1.10 : 0.90-1.10, more preferably 1:1:1. preferably, the assembly solution is a TMS aqueous solution, a sodium chloride aqueous solution, a magnesium chloride aqueous solution, or purified water. preferably, the temperature of the denaturation reaction is 80-99 °C, more preferably 85-99 °C, and further preferably 90-99 °C. preferably, upon completion of the denaturation reaction, the reaction system is cooled to the renaturation temperature to carry out a renaturation reaction, and finally cooled to obtain the self-assembled crude product; wherein the holding temperature is 70-50 °C, more preferably 65-55 °C, and further preferably 63-57 °C; the duration of the renaturation reaction is 3-15 min, more preferably 3-10 min, and further preferably 3-5 min. preferably, during cooling of the reaction system to the holding temperature, the cooling rate is 2-10 °C/min, more preferably 2-6 °C/min, and further preferably 2-3 °C/min. preferably, the final temperature after cooling is 0-25 °C, more preferably 0-15 °C, and further preferably 0-4 °C. preferably, the pH of the denaturation reaction is 5.4-8.8.

By implementing the technical solution of the present application, nucleic acid nanoparticles self-assembled from the core sequences set forth herein are employed as delivery carriers for oligonucleotide drugs. Compared with LNPs and PNPs in the prior art, since both the carrier and the delivered drug are nucleotide sequences, greater physicochemical compatibility with oligonucleotide drugs is achieved, facilitating the loading of different types of small nucleic acid molecules according to differences in the carrier sequence structure or length. Utilizing the advantage of base complementary pairing not only improves delivery efficiency but also renders the delivery system more stable. In addition, the preparation process of the nucleic acid carrier is simpler and more efficient.

Brief Description of the Drawings

The drawings forming a part of the present specification are provided to further facilitate an understanding of the invention. The exemplary embodiments and their descriptions are intended to explain the invention and are not to be construed as unduly limiting the invention. In the drawings:

FIGs. 1-1 to 1-4 illustrate HPLC chromatograms of the assembled sequence products in Example 1, wherein FIG. 1-1 is the HPLC chromatogram of C1, FIG. 1-2 is the HPLC chromatogram of C2, FIG. 1-3 is the HPLC chromatogram of D1, and FIG. 1-4 is the HPLC chromatogram of D2;

FIG. 2 illustrates an agarose gel electrophoresis image of the self-assembled product of 3*CpG2006-DNA carrier in Example 2;

FIG. 3 illustrates an agarose gel electrophoresis image of the self-assembled product of 2*CpG1826-2*mannose-DNA carrier in Example 3;

FIG. 4 illustrates an agarose gel electrophoresis image of the self-assembled product of 2*CpG1826-CD40 aptamer-DNA carrier in Example 4;

FIG. 5-1 illustrates an agarose gel electrophoresis image of the self-assembled product of 2*CpG2006-CD40 aptamer-DNA carrier in Example 5;

FIG. 5-2 illustrates an HPLC chromatogram of the self-assembled product of 2*CpG2006-CD40 aptamer-DNA carrier in Example 5;

FIG. 5-3 illustrates the melting peak (Tm) and melting curve plots of the 2*CpG2006-CD40 aptamer-DNA carrier in Example 5;

FIG. 5-4 illustrates an agarose gel electrophoresis image for the serum stability assay of the 2*CpG2006-CD40 aptamer-DNA carrier in Example 5;

FIG. 6 illustrates an agarose gel electrophoresis image of the self-assembled product of 4*CpG2006-CD40 aptamer-DNA carrier in Example 6;

FIG. 7 illustrates an agarose gel electrophoresis image of the self-assembled product of 2*CpG1826-CD40 aptamer-2*mannose-DNA carrier in Example 7;

FIG. 8 illustrates an agarose gel electrophoresis image of the self-assembled product of CpG1826-CD40 aptamer-mannose-MUC1 aptamer-DNA carrier in Example 8;

FIG. 9 illustrates an agarose gel electrophoresis image of the self-assembled product of 2*CpG2006-CD40 aptamer-

AmiR-21-3*mannose-DNA carrier in Example 9;

FIG. 10 illustrates an agarose gel electrophoresis image of the self-assembled product of 2*CpG2006-CD40 aptamer-CD16a aptamer-CTLA-4 aptamer-DNA carrier in Example 10;

FIG. 11 illustrates an agarose gel electrophoresis image of the self-assembled DNA product of 2*CpG2006-CTLA-4 aptamer-PD-L1 aptamer in Example 11;

FIG. 12-1 illustrates an agarose gel electrophoresis image of the self-assembled product of CCPD3 in Example 12;

FIG. 12-3 illustrates the melting peak (Tm) and melting curve plots of the self-assembled product of CCPD3 in Example 12.

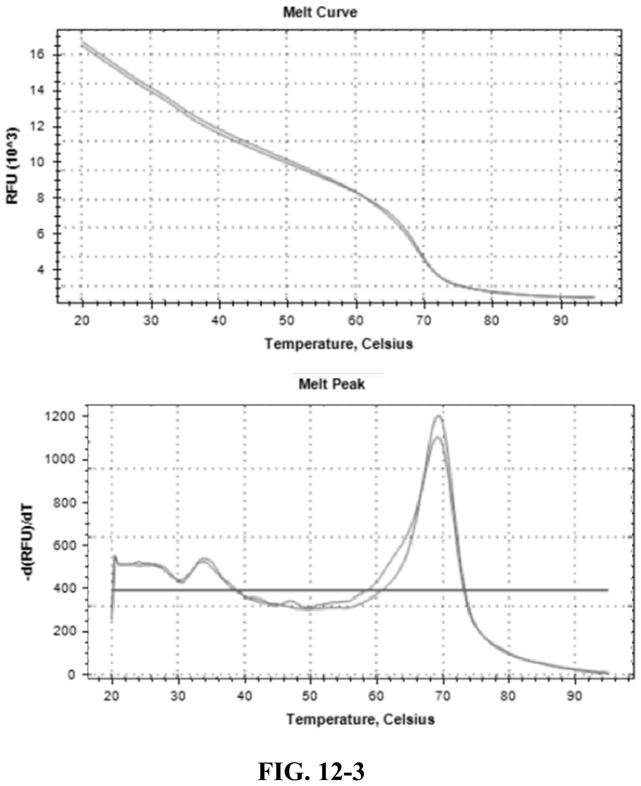

FIG. 12-3 illustrates the melting peak (Tm) and melting curve plots of the self-assembled product of CCPD3 in Example 12;

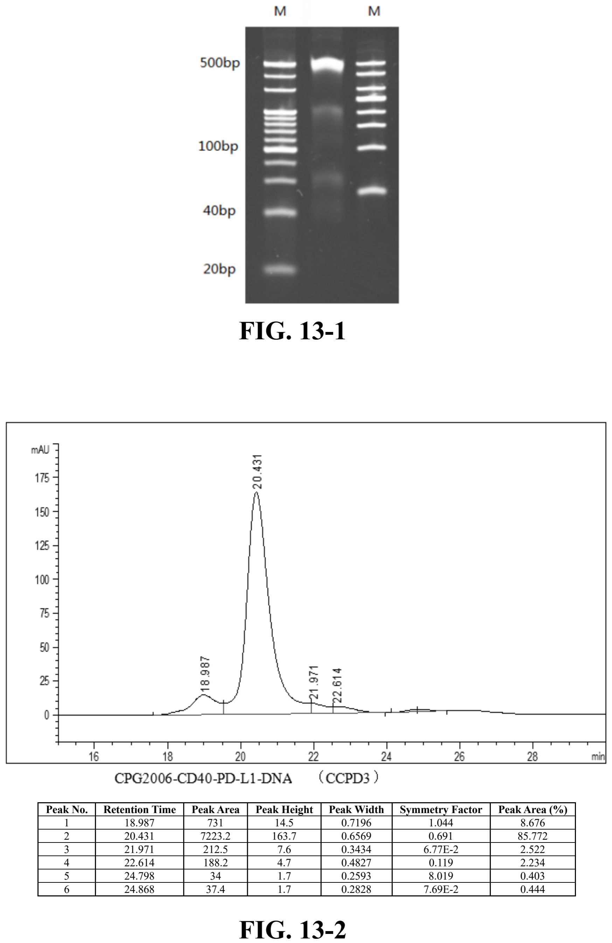

FIG. 13-1 illustrates an agarose gel electrophoresis image of the self-assembled product of CCPD3 Variant 1 in Example 13;

FIG. 13-2 illustrates an HPLC chromatogram of the self-assembled product of CCPD3 Variant 1 in Example 13;

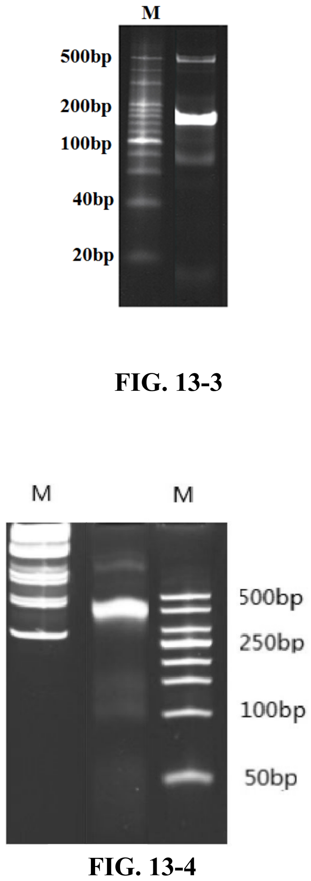

FIG. 13-3 illustrates an agarose gel electrophoresis image of the self-assembled product of CCPD3 Variant 2 in Example 13;

FIG. 13-4 illustrates an agarose gel electrophoresis image of the self-assembled product of CCPD3 Variant 3 in Example 13;

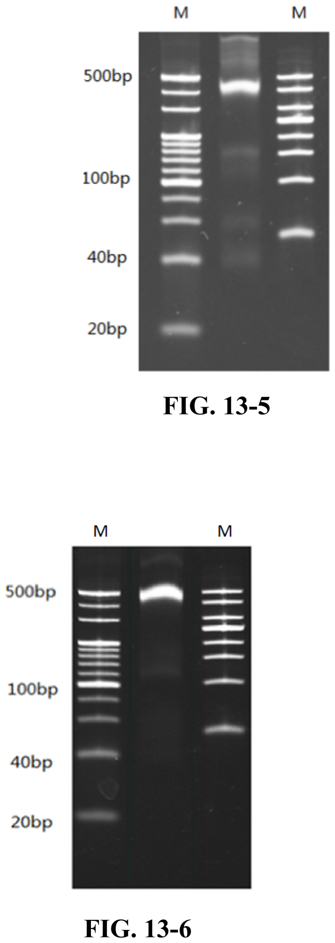

FIG. 13-5 illustrates an agarose gel electrophoresis image of the self-assembled product of CCPD3 Variant 4 in Example 13;

FIG. 13-6 illustrates an agarose gel electrophoresis image of the self-assembled product of CCPD3 Variant 5 in Example 13;

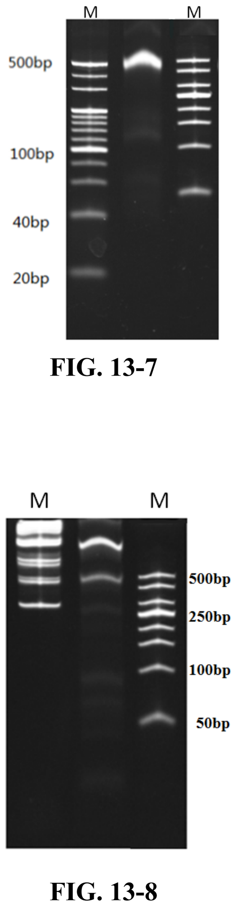

FIG. 13-7 illustrates an agarose gel electrophoresis image of the self-assembled product of CCPD3 Variant 6 in Example 13;

FIG. 13-8 illustrates an agarose gel electrophoresis image of the self-assembled product of CCPD3 Variant 7 in Example 13;

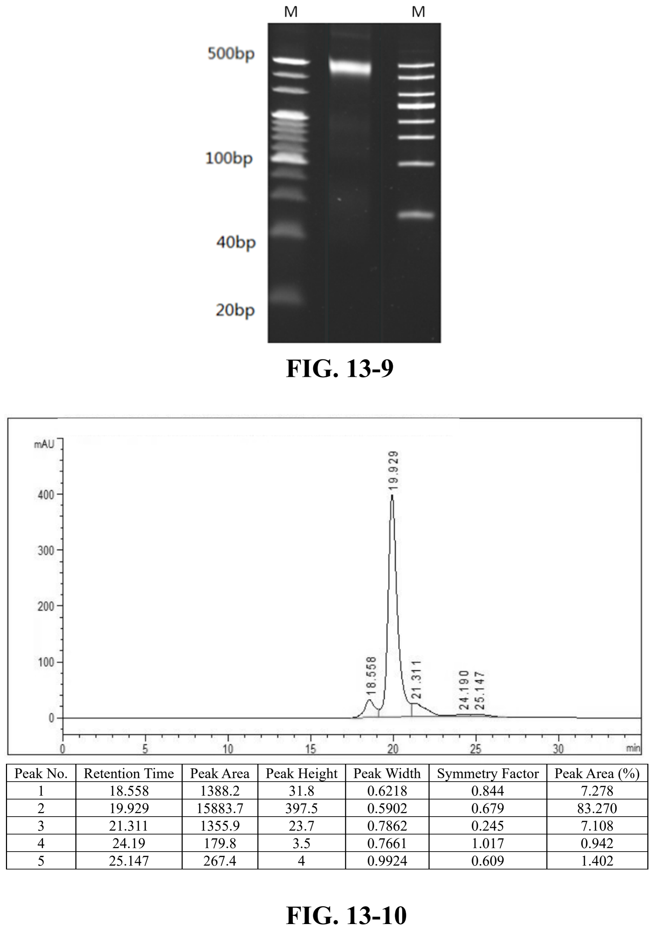

FIG. 13-9 illustrates an agarose gel electrophoresis image of the self-assembled product of CCPD3 Variant 8 in Example 13;

FIG. 13-10 illustrates an agarose gel electrophoresis image of the self-assembled product of CCPD3 Variant 9 in Example 13;

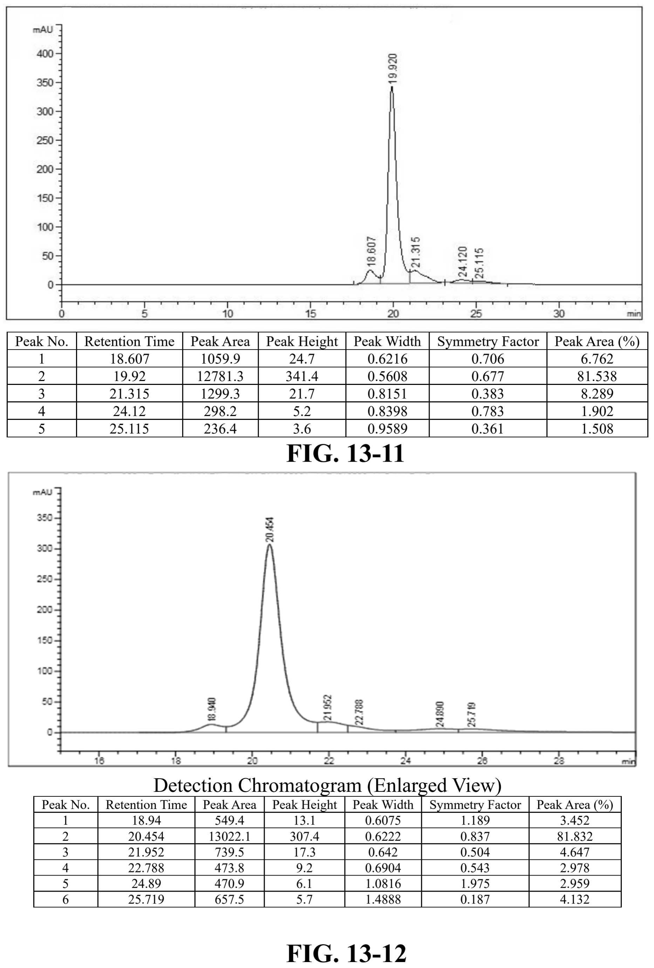

FIG. 13-11 illustrates an agarose gel electrophoresis image of the self-assembled product of CCPD3 Variant 10 in Example 13;

FIG. 13-12 illustrates an HPLC chromatogram of the self-assembled product of CCPD3 Variant 11 in Example 13;

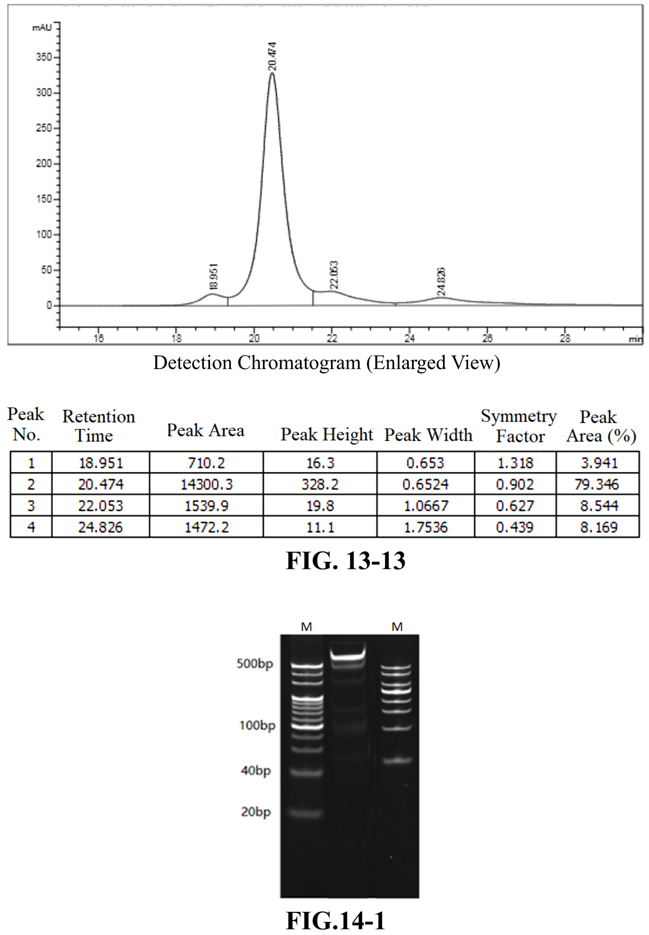

FIG. 13-13 illustrates an HPLC chromatogram of the self-assembled product of CCPD3 Variant 12 in Example 13;

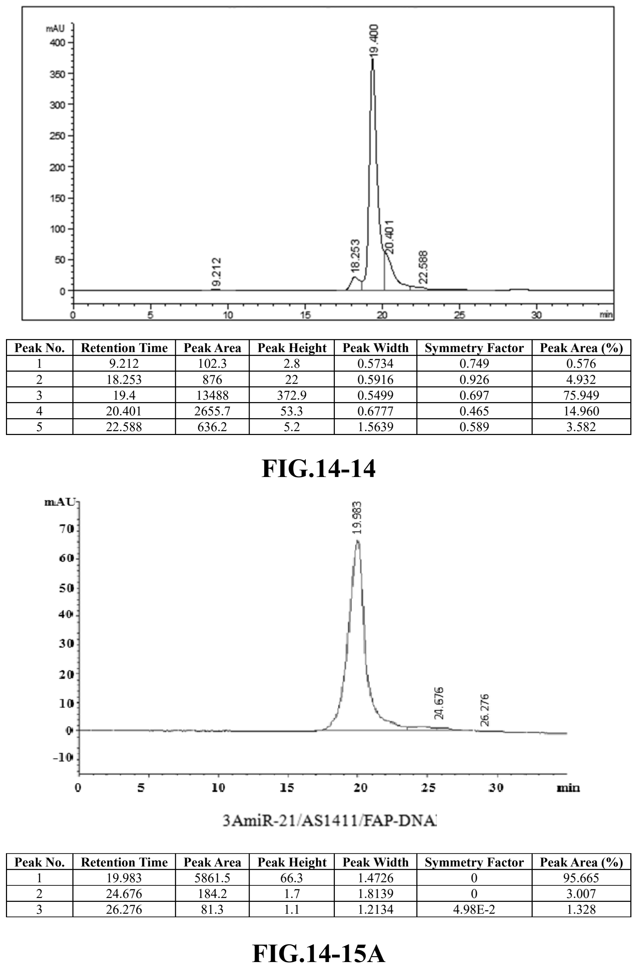

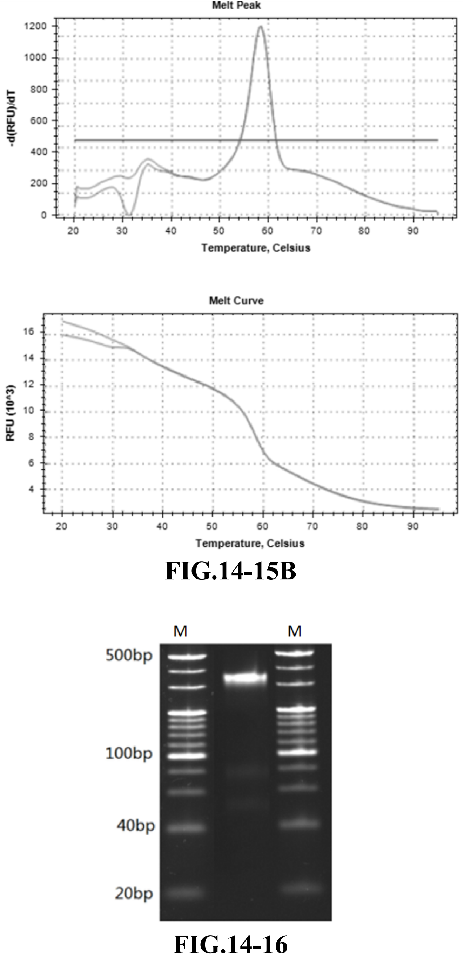

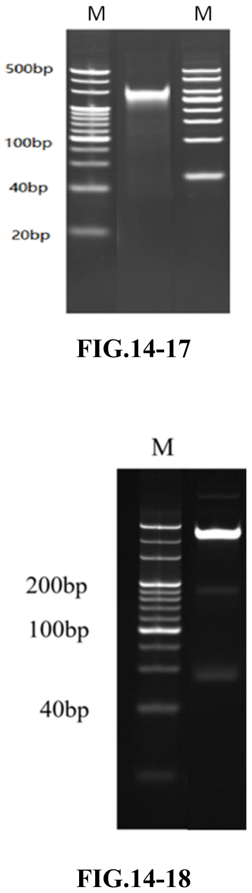

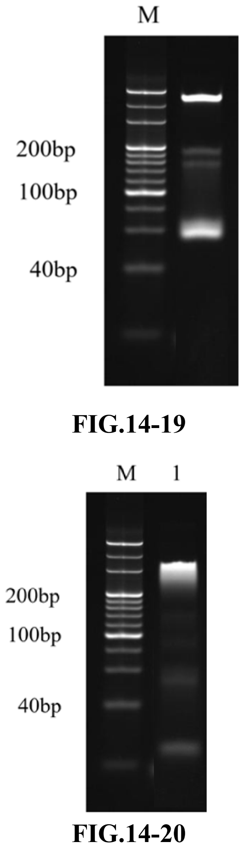

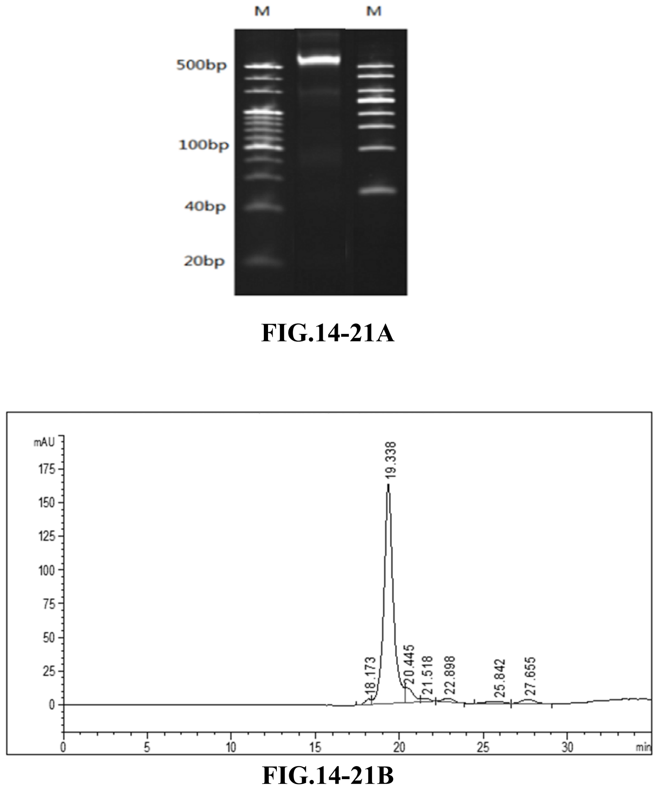

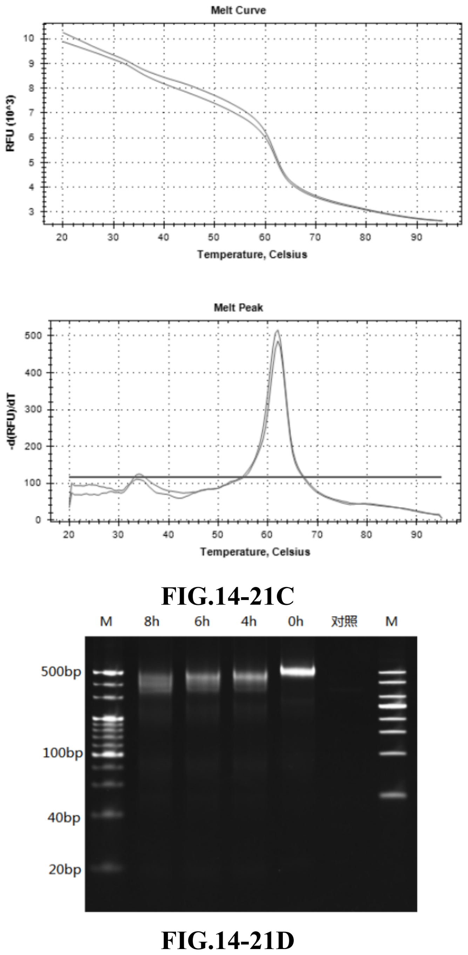

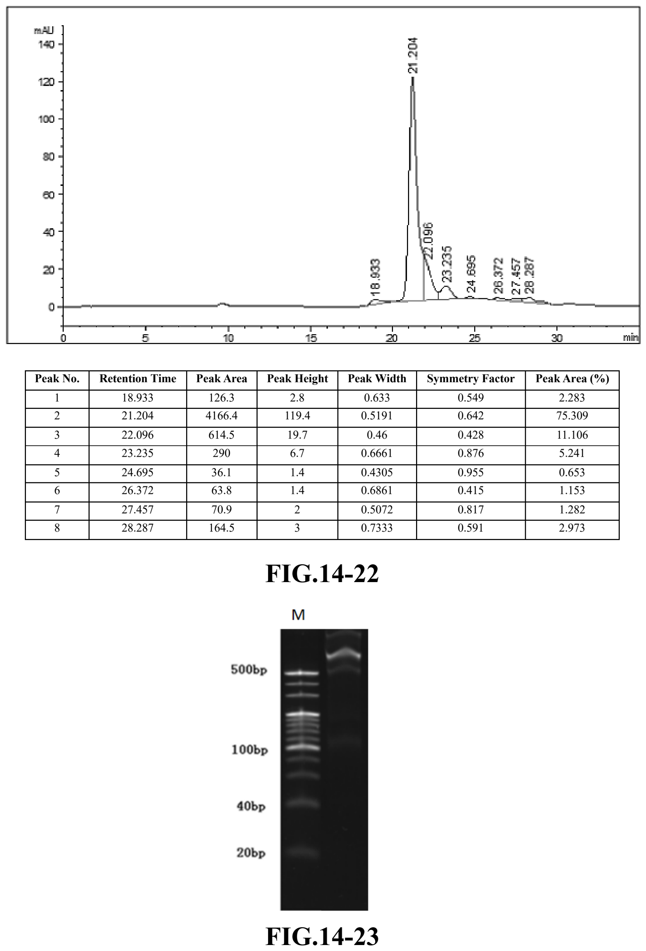

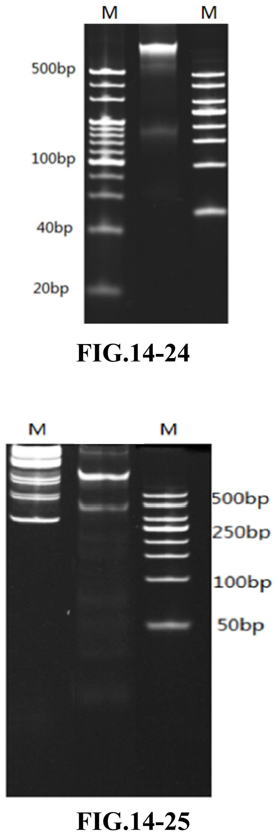

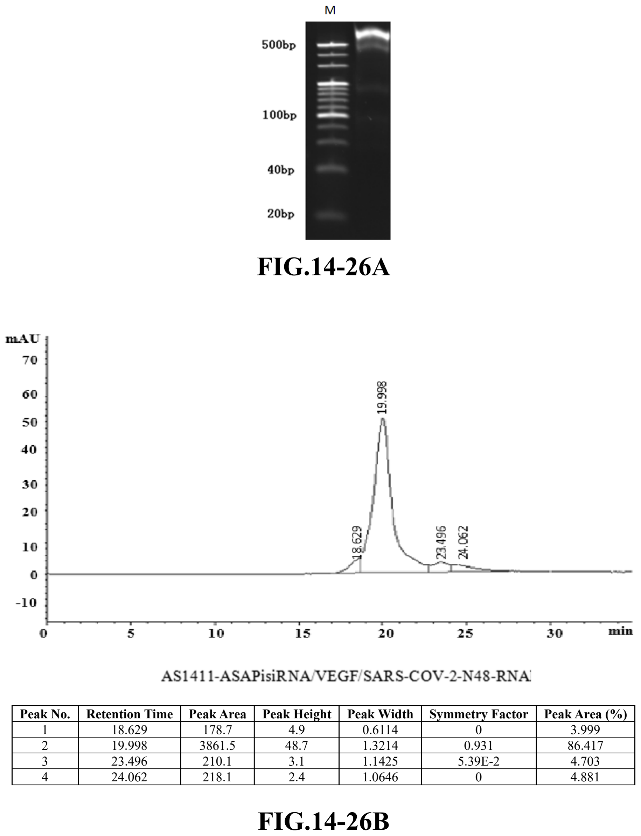

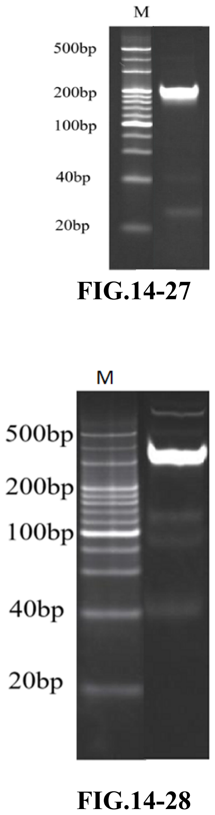

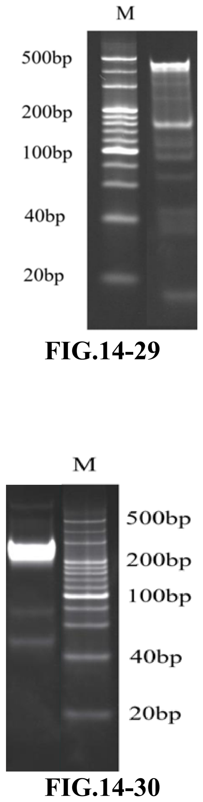

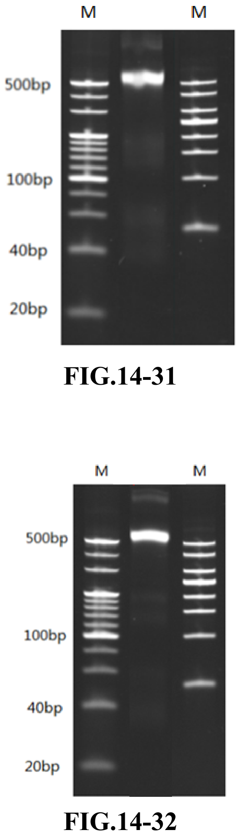

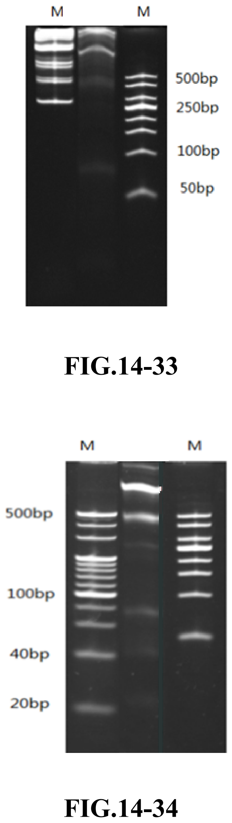

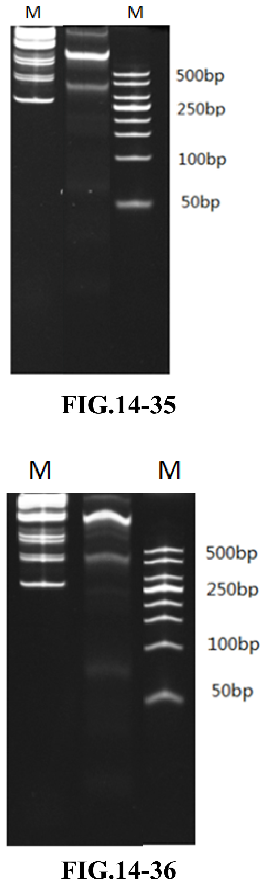

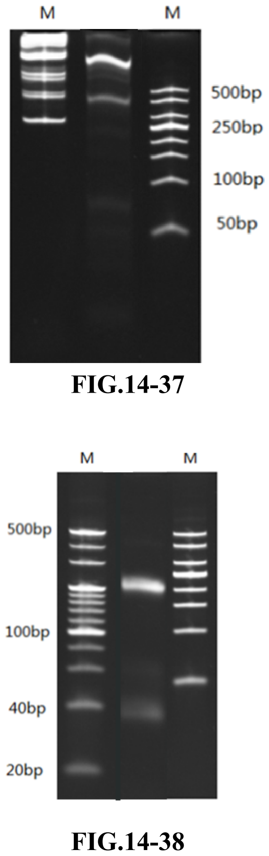

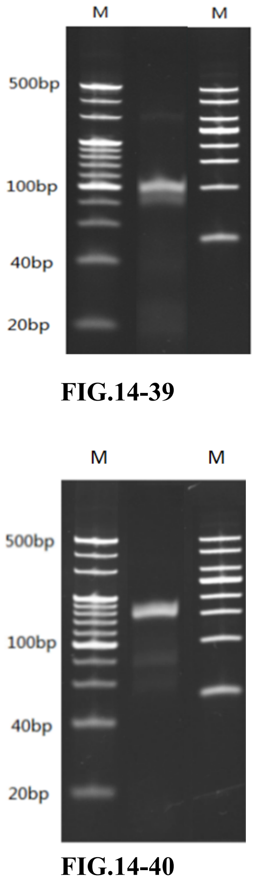

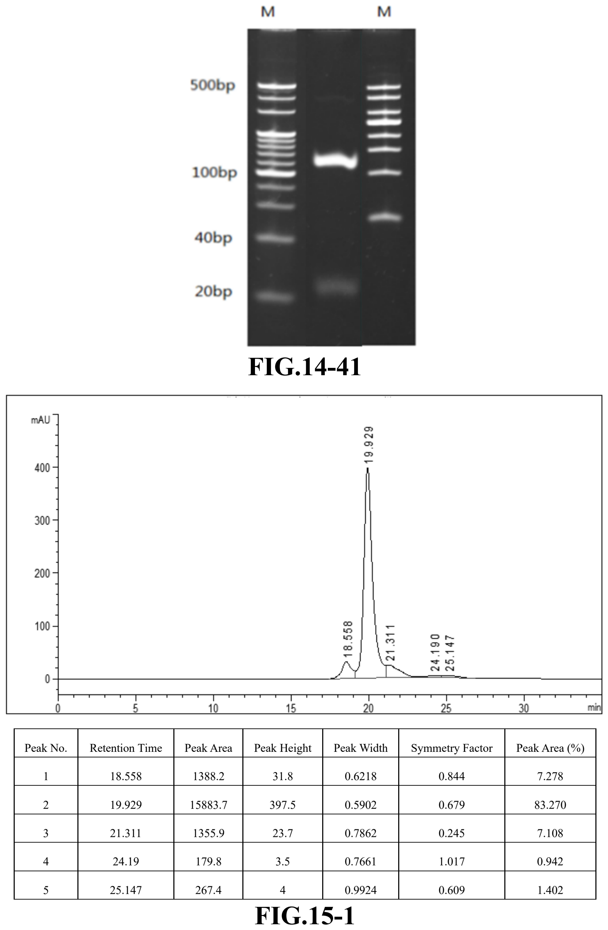

FIG. 14-1 illustrates an agarose gel electrophoresis image of the self-assembled product of 2*CpG2006-CD40 aptamer-PD-L1 aptamer-C12 aptamer-DNA carrier in Example 14;

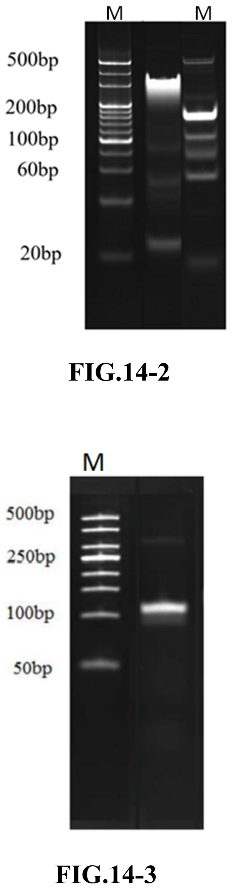

FIG. 14-2 illustrates an agarose gel electrophoresis image of the self-assembled product of 2*AmiR-21-TTA1 aptamer-DNA carrier in Example 14;

FIG. 14-3 illustrates an agarose gel electrophoresis image of the self-assembled product of 2*AmiR-21-3*Bio-DNA carrier in Example 14;

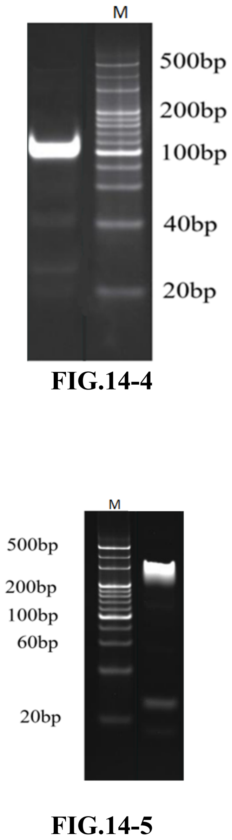

FIG. 14-4 illustrates an agarose gel electrophoresis image of the self-assembled product of 2* AmiR-21-4*Bio-DNA carrier in Example 14;

FIG. 14-5 illustrates an agarose gel electrophoresis image of the self-assembled product of 2*AmiR-21-AS1411 aptamer-DNA carrier in Example 14;

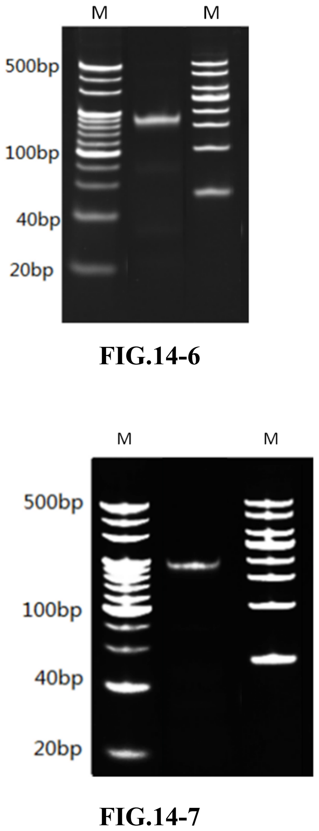

FIG. 14-6 illustrates an agarose gel electrophoresis image of the self-assembled product of 2*AmiR-21-AS1411 aptamer-DNA carrier (enhanced version) in Example 14;

FIG. 14-7 illustrates an agarose gel electrophoresis image of the self-assembled product of 2*AmiR-21-AS1411 aptamer-Bio-DNA carrier in Example 14;

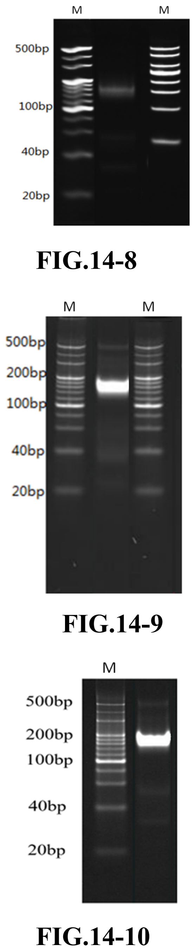

FIG. 14-8 illustrates an agarose gel electrophoresis image of the self-assembled product of 2*AmiR-21-CD40 aptamer-DNA carrier in Example 14;

FIG. 14-9 illustrates an agarose gel electrophoresis image of the self-assembled product of 2*AmiR-21-MUC1 aptamer-3*Bio-DNA carrier in Example 14;

FIG. 14-10 illustrates an agarose gel electrophoresis image of the self-assembled product of 2*mannose-2*AmiR-21-DNA carrier in Example 14;

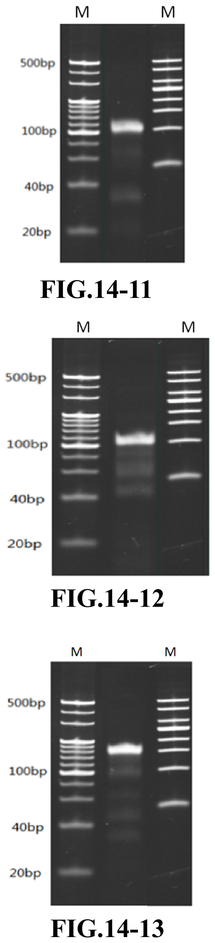

FIG. 14-11 illustrates an agarose gel electrophoresis image of the self-assembled product of 2*mannose-A15 aptamer-2*AmiR-21-DNA carrier in Example 14;

FIG. 14-12 illustrates an agarose gel electrophoresis image of the self-assembled product of 2*mannose-survivin siRNA-DNA carrier in Example 14;

FIG. 14-13 illustrates an agarose gel electrophoresis image of the self-assembled product of 2*mannose-A15 aptamer-survivin siRNA-AmiR-21-DNA carrier in Example 14;

IG. 14-14 illustrates an agarose gel electrophoresis image of the self-assembled product of AS1411 aptamer-survivin siRNA-PSMA aptamer-3*AmiR-21-RNA carrier in Example 14;

FIG. 14-15A illustrates an agarose gel electrophoresis image of the self-assembled product of 3*AmiR-21-AS1411 aptamer-FAP aptamer-DNA carrier in Example 14, and FIG. 14-15B illustrates an HPLC chromatogram;

FIG. 14-16 illustrates an agarose gel electrophoresis image of the self-assembled product of 3*AmiR-21-AS1411 aptamer-CD40 aptamer-2*Bio-DNA carrier in Example 14;

FIG. 14-17 illustrates an agarose gel electrophoresis image of the self-assembled product of TAP siRNA-3*A-miR-21-2*AS1411-1 aptamer-DNA carrier in Example 14;

FIG. 14-18 illustrates an agarose gel electrophoresis image of the self-assembled product of CpG1826-CD40 aptamer-AmiR-21-3*Bio-DNA carrier in Example 14;

FIG. 14-19 illustrates an agarose gel electrophoresis image of the self-assembled product of CD16a aptamer-CTLA-4 aptamer-PD-L1 aptamer-DNA carrier in Example 14;

FIG. 14-20 illustrates an agarose gel electrophoresis image of the self-assembled product of CD16a aptamer-Act-12c aptamer-PD-L1 aptamer-DNA carrier in Example 14;

FIG. 14-21A illustrates an agarose gel electrophoresis image of the self-assembled product of CTLA-4 aptamer-PD-L1 aptamer-PD-1 aptamer-DNA carrier in Example 14, FIG. 14-21B illustrates an HPLC chromatogram, FIG. 14-21C illustrates the melting peak (Tm) and melting curve plots, and FIG. 14-21D illustrates an agarose gel electrophoresis image for the serum stability assay;

FIG. 14-22 illustrates an agarose gel electrophoresis image of the self-assembled product of CD16a aptamer-Act-12c aptamer-MUC1 aptamer (5TR1)-DNA carrier in Example 14;

FIG. 14-23 illustrates an agarose gel electrophoresis image of the self-assembled product of HER3 aptamer-EGFR siRNA-AS1411 aptamer-survivin siRNA-HER2 aptamer-DNA carrier in Example 14;

FIG. 14-24 illustrates an agarose gel electrophoresis image of the self-assembled product of AS1411 aptamer-TAP siRNA-CD16a aptamer-Act-12c aptamer-DNA carrier in Example 14;

FIG. 14-25 illustrates an agarose gel electrophoresis image of the self-assembled product of CD16a aptamer-AS1411 aptamer-TAP siRNA-OX40 aptamer-Act-12c aptamer-DNA carrier in Example 14;

FIG. 14-26A illustrates an agarose gel electrophoresis image of the self-assembled product of AS1411 aptamer-ASAP1 siRNA-VEGF aptamer-SARS-CoV-2 N48 aptamer-RNA carrier in Example 14, and FIG. 14-26B illustrates an HPLC chromatogram;

FIG. 14-27 illustrates an agarose gel electrophoresis image of the self-assembled product of CD3-4 aptamer-PD-1 aptamer-BCMA aptamer-DNA carrier in Example 14;

FIG. 14-28 illustrates an agarose gel electrophoresis image of the self-assembled product of CpG2006-CD38 aptamer-miR-126-PD-L1 aptamer-DNA carrier in Example 14;

FIG. 14-29 illustrates an agarose gel electrophoresis image of the self-assembled product of BCMA aptamer-CD38 aptamer-miR-126-PD-L1 aptamer-miR-34-DNA carrier (MM) in Example 14;

FIG. 14-30 illustrates an agarose gel electrophoresis image of the self-assembled product of AS1411 aptamer-FGF2 aptamer-3 *AmiR-21-DNA carrier in Example 14;

FIG. 14-31 illustrates an agarose gel electrophoresis image of the self-assembled product of HBsAg aptamer-CD40 aptamer-PD-L1 aptamer (CCPD3 Strand c)-DNA carrier (HBV) in Example 14;

FIG. 14-32 illustrates an agarose gel electrophoresis image of the self-assembled product of HBsAg aptamer-CD40 aptamer-PD-L1 aptamer (HD)-DNA carrier (HBV) in Example 14;

FIG. 14-33 illustrates an agarose gel electrophoresis image of the self-assembled product of BCMA aptamer-CD38 aptamer-CpG aptamer-CD19 aptamer-AmiR-21-DNA3-TY (MM) in Example 14;

FIG. 14-34 illustrates an agarose gel electrophoresis image of the self-assembled product of BCMA aptamer-CD38 aptamer-CD19 aptamer-AmiR-21-DNA3-TY (MM) in Example 14;

FIG. 14-35 illustrates an agarose gel electrophoresis image of the self-assembled product of CpG2006-CD38 aptamer-PD-L1 aptamer-DNA3-TY (tumor) in Example 14;

FIG. 14-36 illustrates an agarose gel electrophoresis image of the self-assembled product of BCMA aptamer-CD38 aptamer-CpG aptamer-PD-L1 aptamer-DNA3-TY (MM) carrier in Example 14;

FIG. 14-37 illustrates an agarose gel electrophoresis image of the self-assembled product of BCMA aptamer-CD40 aptamer-PD-L1 aptamer-DNA3-TY (MM) carrier in Example 14;

FIG. 14-38 illustrates an agarose gel electrophoresis image of the self-assembled product of AmiR-21-AS1411 aptamer-CD40 aptamer-2*biotin-DNA carrier in Example 14;

FIG. 14-39 illustrates an agarose gel electrophoresis image of the self-assembled product of 2*AmiR-21-4*biotin-DNA carrier in Example 14;

FIG. 14-40 illustrates an agarose gel electrophoresis image of the self-assembled product of TTA1 aptamer-epirubicin-4*biotin-DNA carrier in Example 14;

FIG. 14-41 illustrates an agarose gel electrophoresis image of the self-assembled product of the universal carrier DNA3-TY in Example 14;

FIG. 15-1 illustrates an HPLC chromatogram of CCPD3 self-assembled product 1 in Example 15;

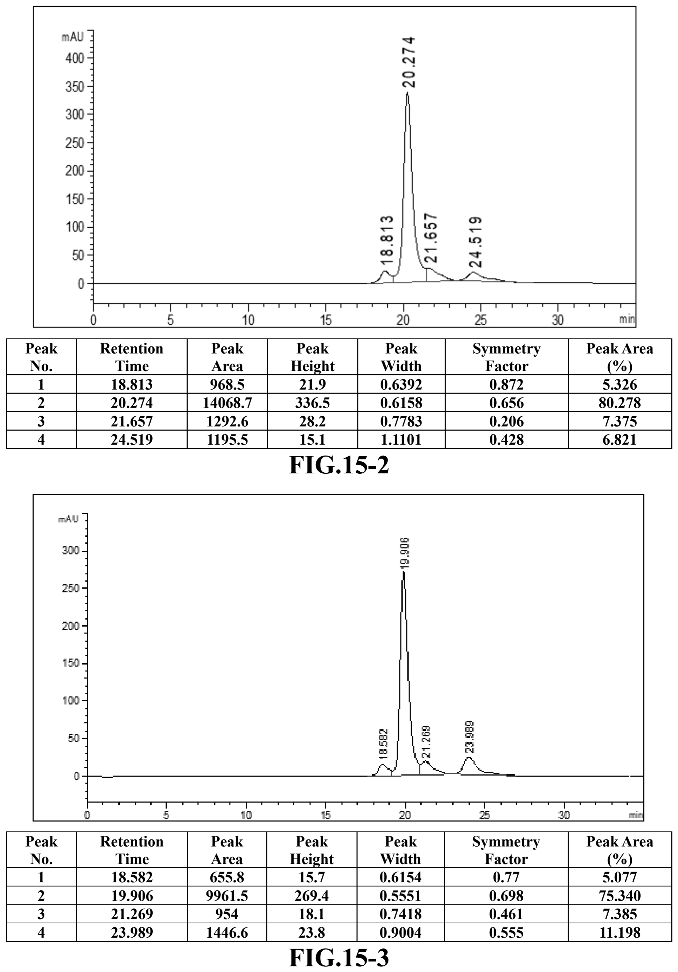

FIG. 15-2 illustrates an HPLC chromatogram of CCPD3 self-assembled product 2 in Example 15;

FIG. 15-3 illustrates an HPLC chromatogram of CCPD3 self-assembled product 3 in Example 15;

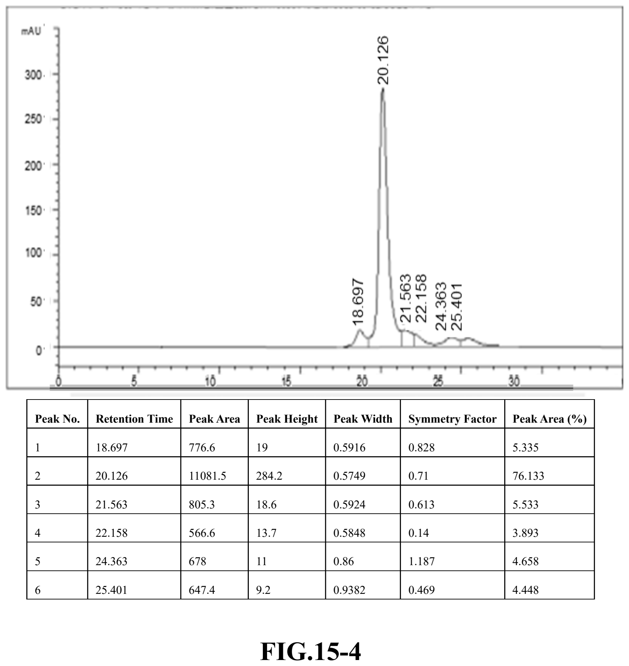

FIG. 15-4 illustrates an HPLC chromatogram of CCPD3 self-assembled product 4 in Example 15;

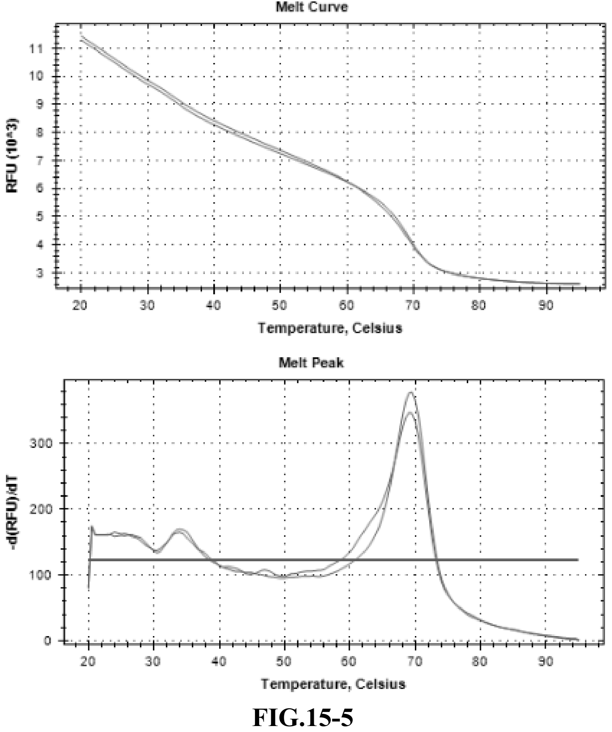

FIG. 15-5 illustrates the melting peak (Tm) and melting curve plots of CCPD3 self-assembled product 1 in Example 15;

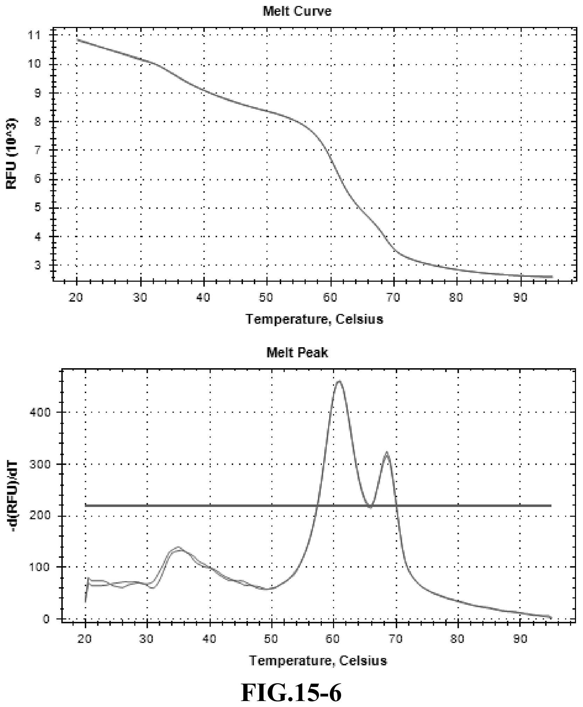

FIG. 15-6 illustrates the melting peak (Tm) and melting curve plots of CCPD3 self-assembled product 5 in Example 15;

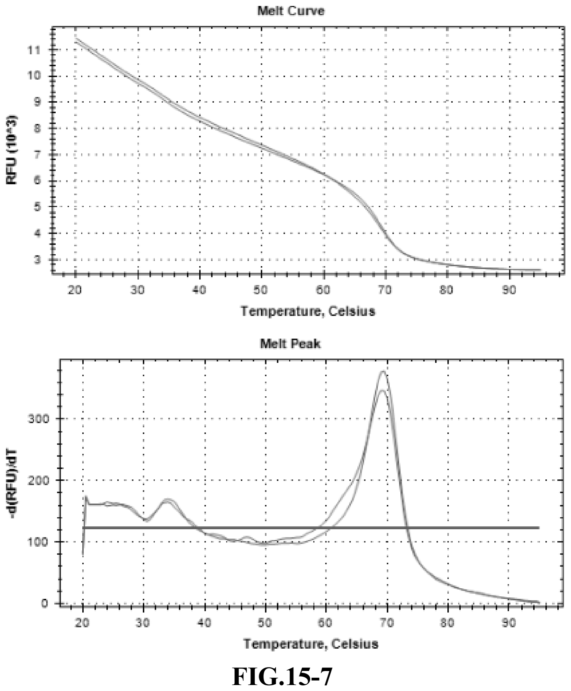

FIG. 15-7 illustrates the melting peak (Tm) and melting curve plots of CCPD3 self-assembled product 6 in Example 15;

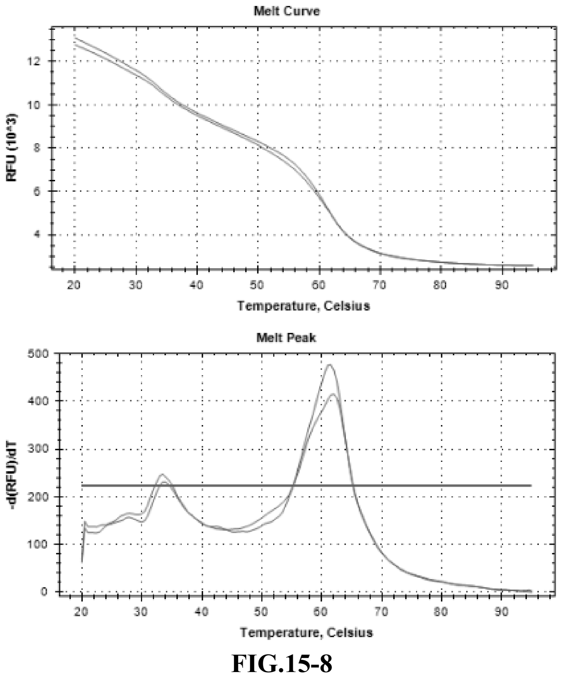

FIG. 15-8 illustrates the melting peak (Tm) and melting curve plots of CCPD3 self-assembled product 7 in Example 15;

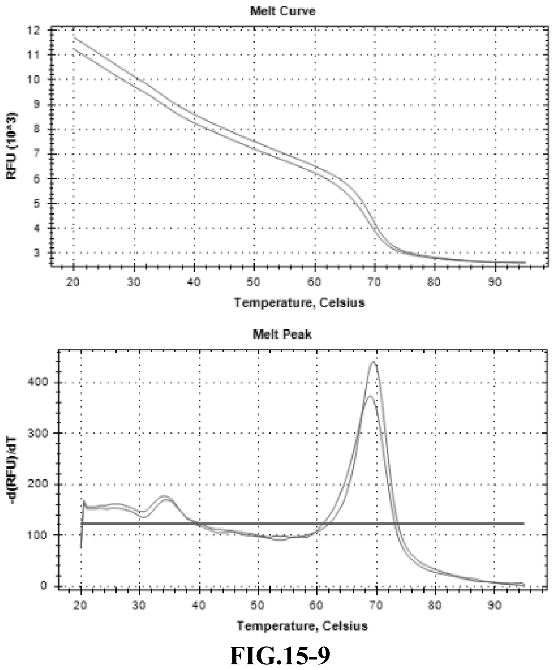

FIG. 15-9 illustrates the melting peak (Tm) and melting curve plots of CCPD3 self-assembled product 8 in Example 15;

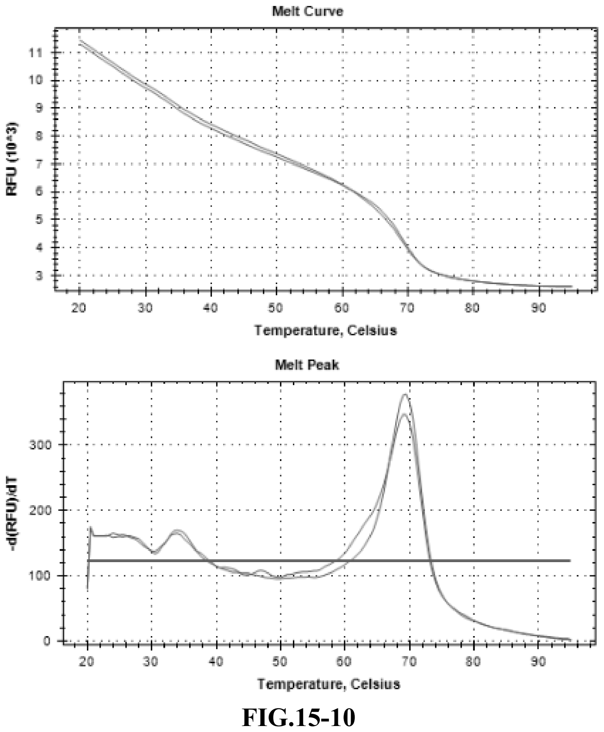

FIG. 15-10 illustrates the melting peak (Tm) and melting curve plots of CCPD3 self-assembled product 9 in Example 15;

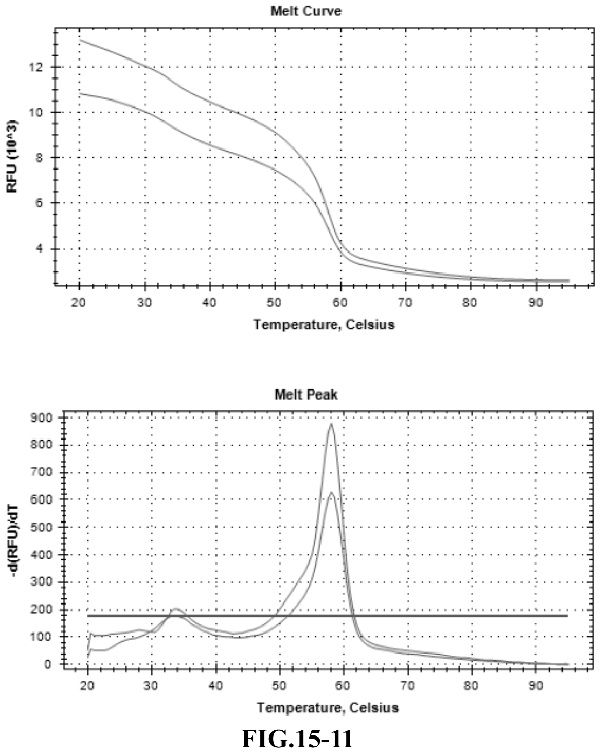

FIG. 15-11 illustrates the melting peak (Tm) and melting curve plots of CCPD3 self-assembled product 10 in Example 15;

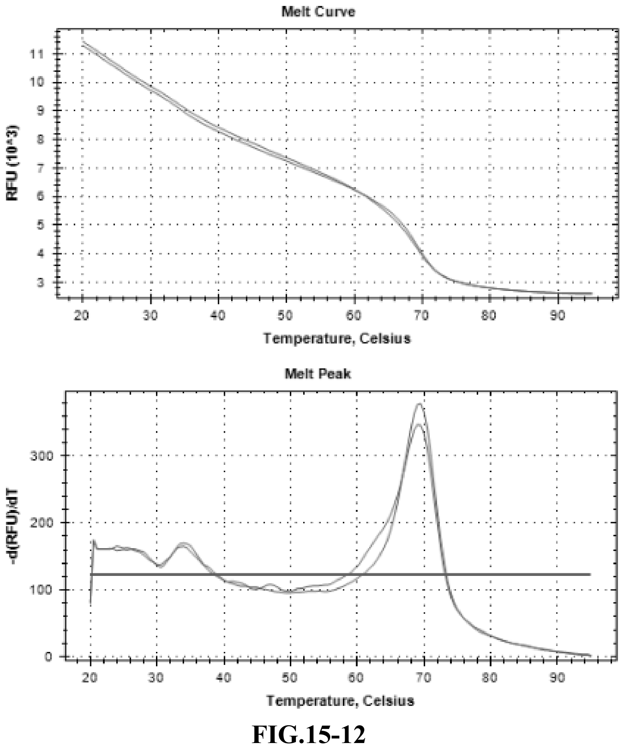

FIG. 15-12 illustrates the melting peak (Tm) and melting curve plots of CCPD3 self-assembled product 11 in Example 15;

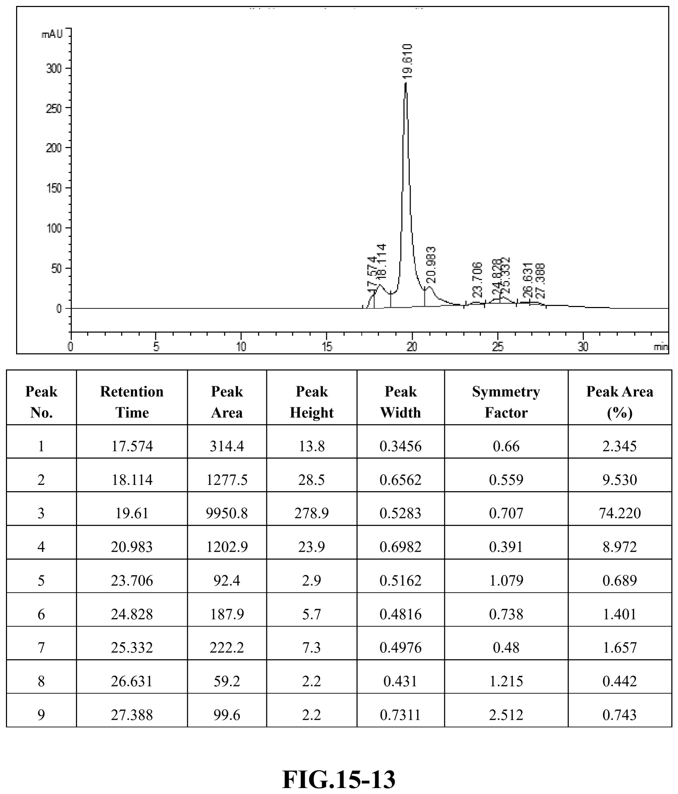

FIG. 15-13 illustrates the melting peak (Tm) and melting curve plots of CCPD3 self-assembled product 12 in Example 15;

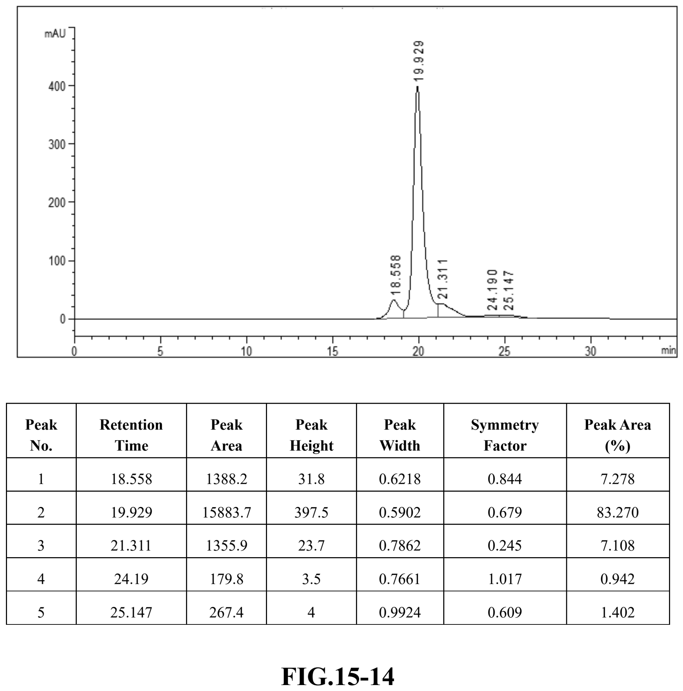

FIG. 15-14 illustrates the melting peak (Tm) and melting curve plots of CCPD3 self-assembled product 13 in Example 15;

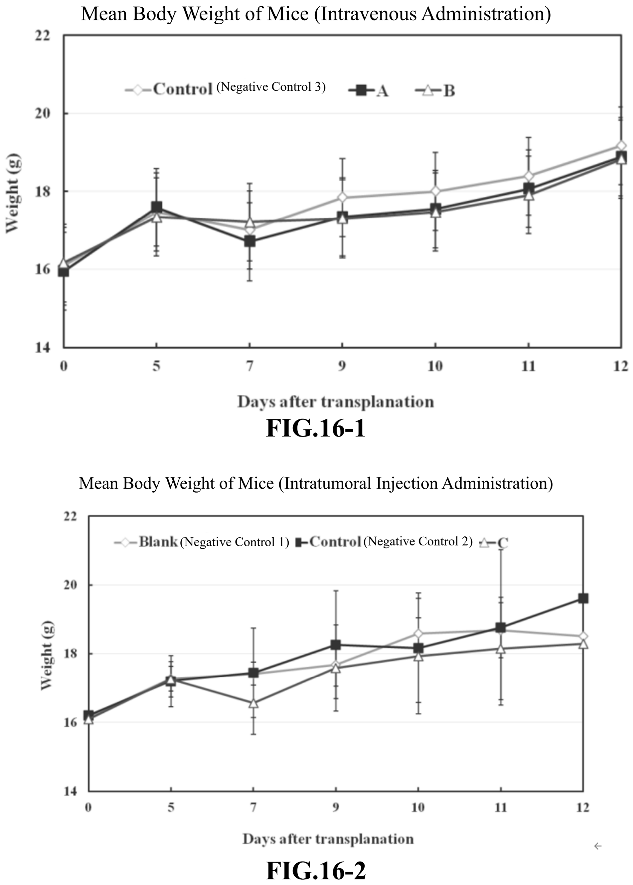

FIG. 16-1 illustrates a graph of animal body-weight changes (intravenous administration) in Example 16;

FIG. 16-2 illustrates a graph of animal body-weight changes (intratumoral injection) in Example 16;

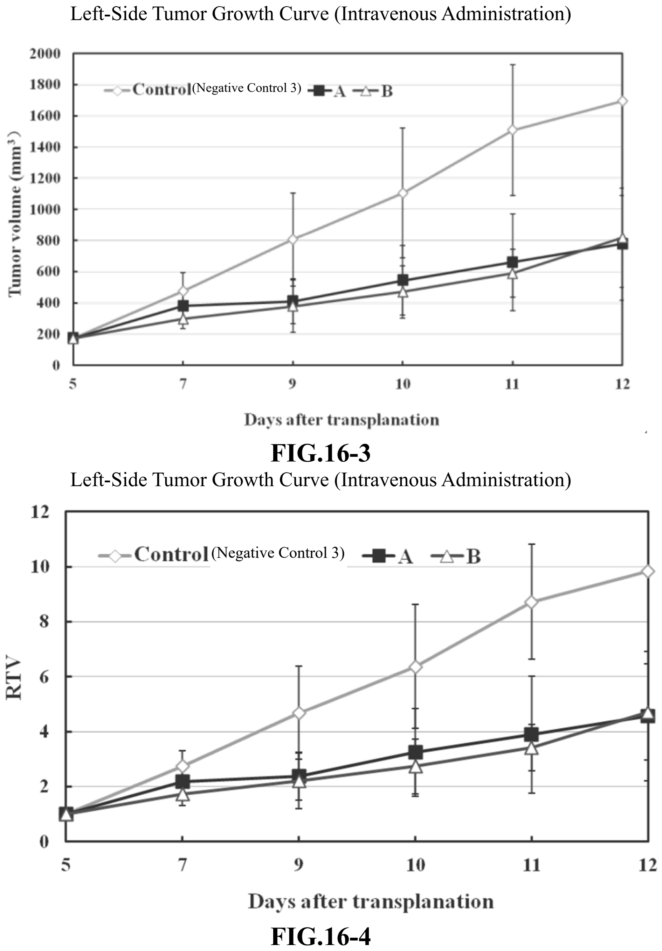

FIG. 16-3 illustrates a graph of left-side tumor volume changes (intravenous administration) in Example 16;

FIG. 16-4 illustrates a graph of left-side relative tumor volume changes (intravenous administration) in Example 16;

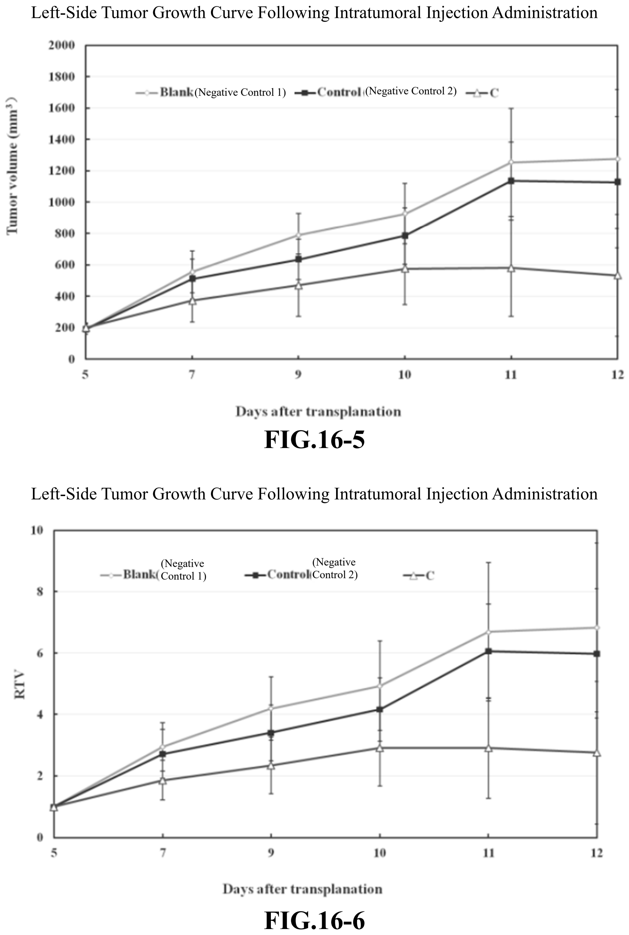

FIG. 16-5 illustrates a graph of left-side tumor volume changes (intratumoral injection) in Example 16;

FIG. 16-6 illustrates a graph of left-side relative tumor volume changes (intratumoral injection) in Example 16;

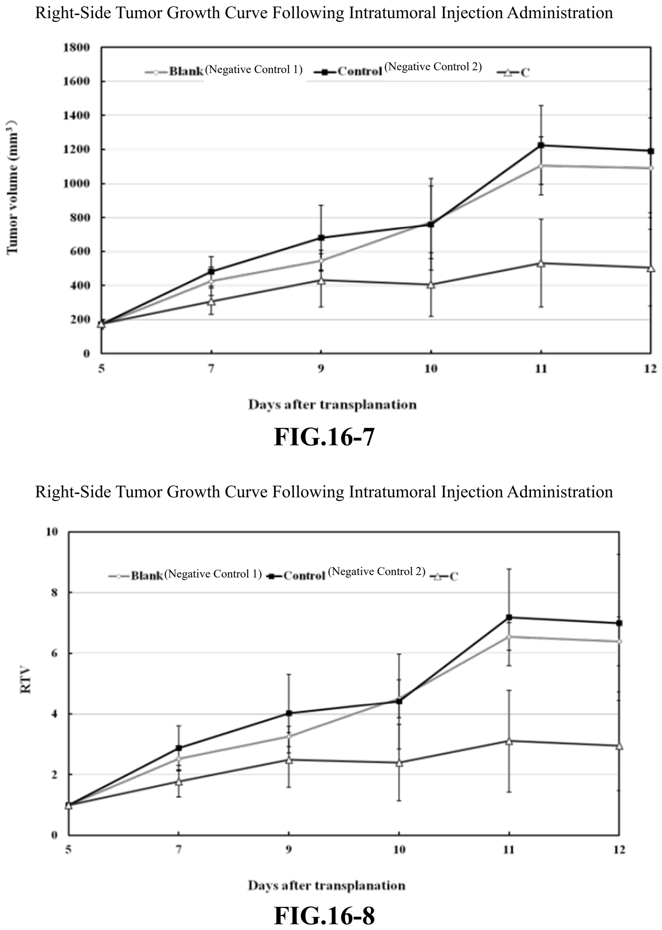

FIG. 16-7 illustrates a graph of right-side tumor volume changes (intratumoral injection) in Example 16;

FIG. 16-8 illustrates a graph of right-side relative tumor volume changes (intratumoral injection) in Example 16;

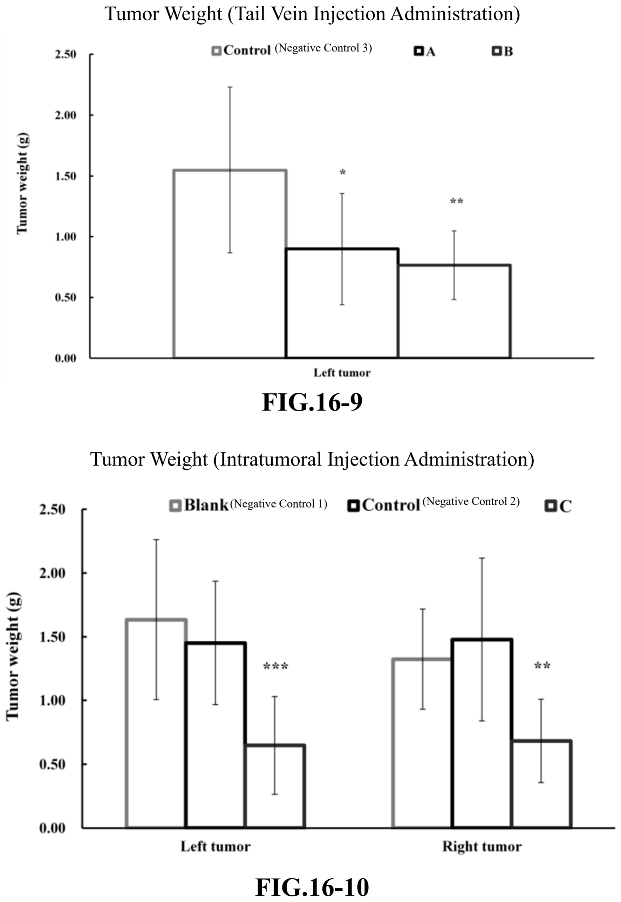

FIG. 16-9 illustrates a graph of right-side tumor weight changes (tail-vein injection) in Example 16;

FIG. 16-10 illustrates a graph of right-side tumor weight changes (intratumoral injection) in Example 16;

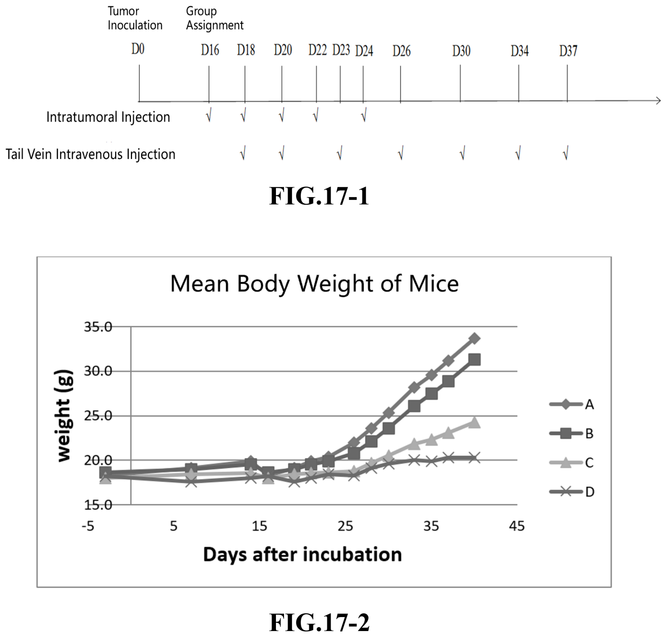

FIG. 17-1 illustrates a schematic diagram of the animal inoculation and dosing regimen in Example 17;

FIG. 17-2 illustrates a graph of animal body-weight changes in Example 17;

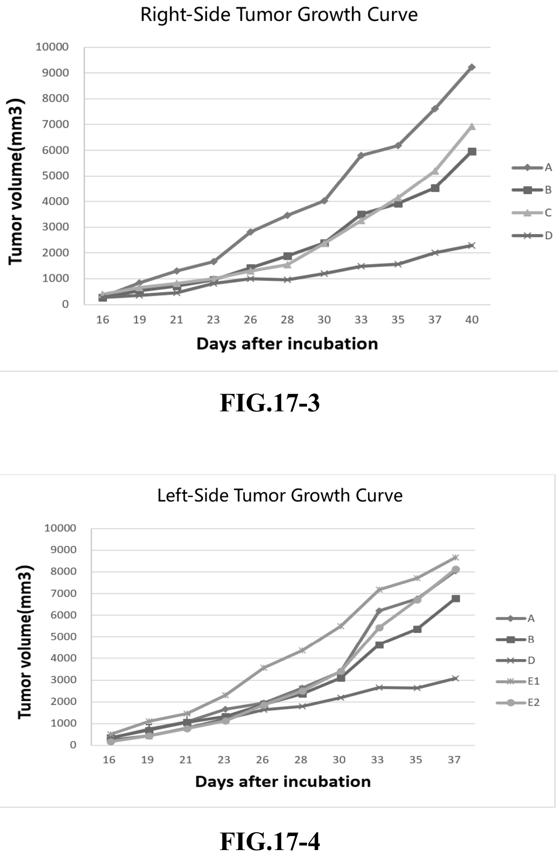

FIG. 17-3 illustrates a graph of right-side tumor growth in Example 17;

FIG. 17-4 illustrates a graph of left-side tumor growth in Example 17;

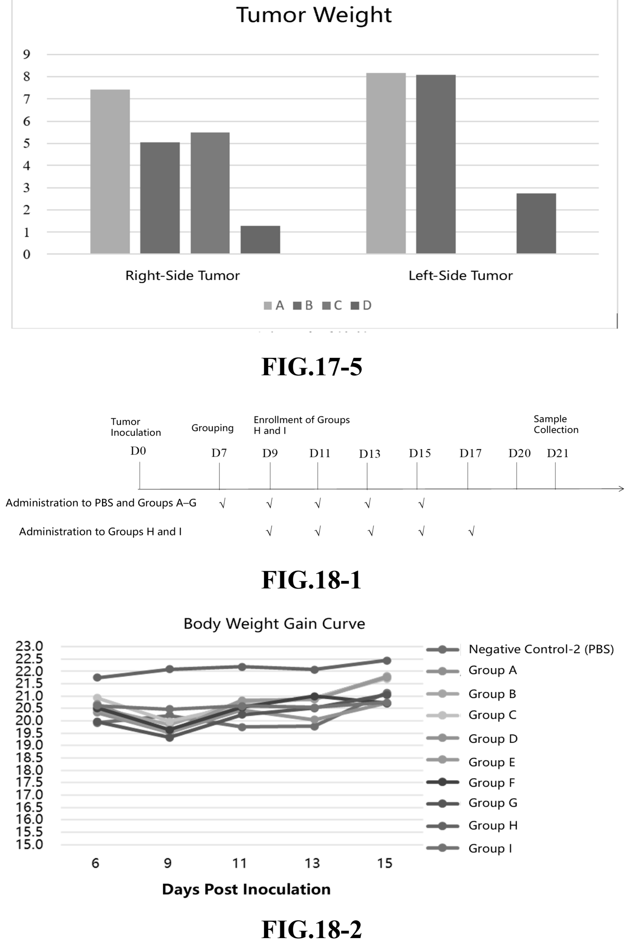

FIG. 17-5 illustrates a tumor weight comparison chart in Example 17;

FIG. 18-1 illustrates a schematic diagram of the animal inoculation and dosing regimen in Example 18;

FIG. 18-2 illustrates a graph of animal body-weight changes in Example 18;

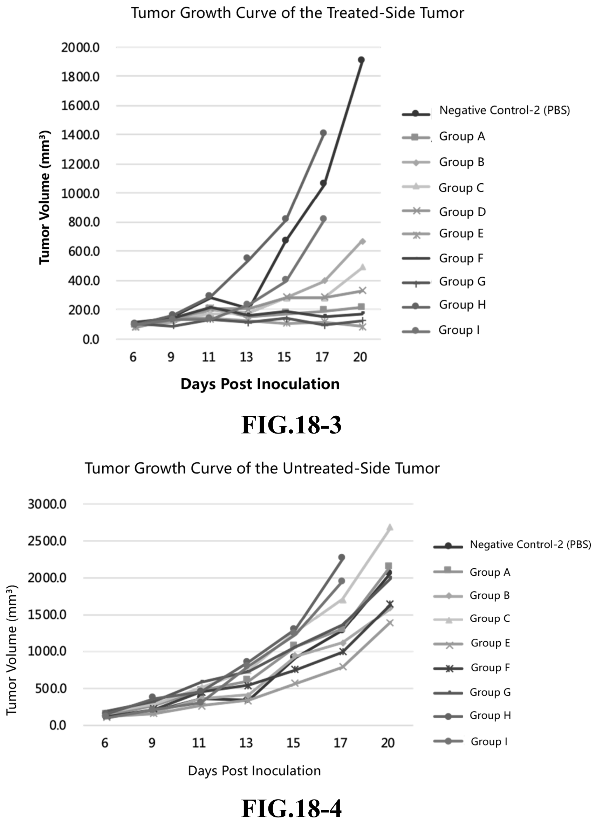

FIG. 18-3 illustrates a graph of tumor growth on the dosed side in Example 18;

FIG. 18-4 illustrates a graph of tumor growth on the undosed side in Example 18;

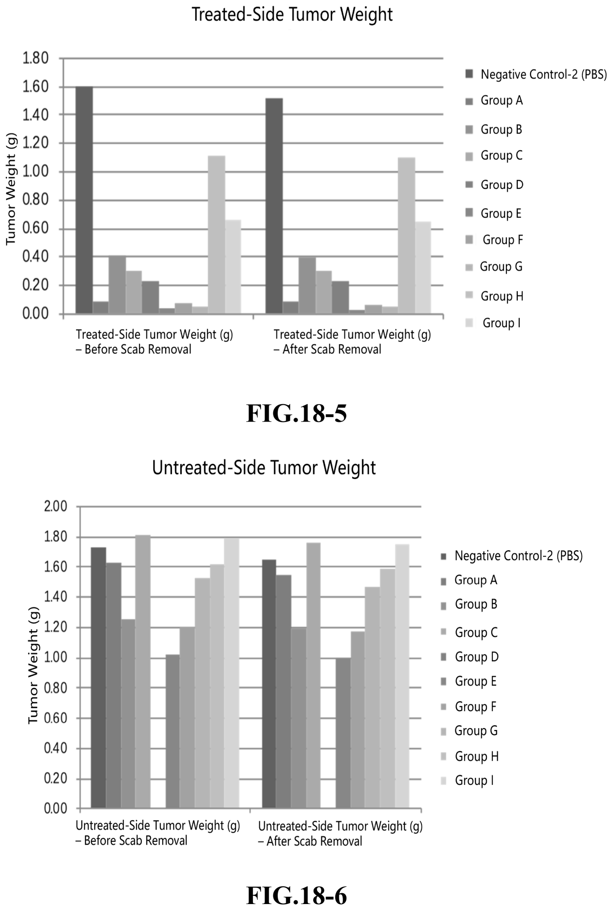

FIG. 18-5 illustrates a tumor weight comparison chart for the dosed side in Example 18;

FIG. 18-6 illustrates a tumor weight comparison chart for the undosed side in Example 18;

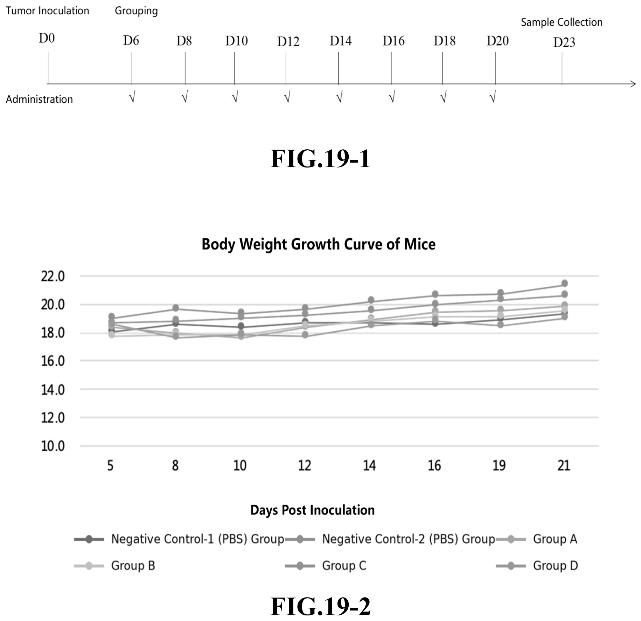

FIG. 19-1 illustrates a schematic diagram of the animal inoculation and dosing regimen in Example 19;

FIG. 19-2 illustrates a graph of animal body-weight changes in Example 19;

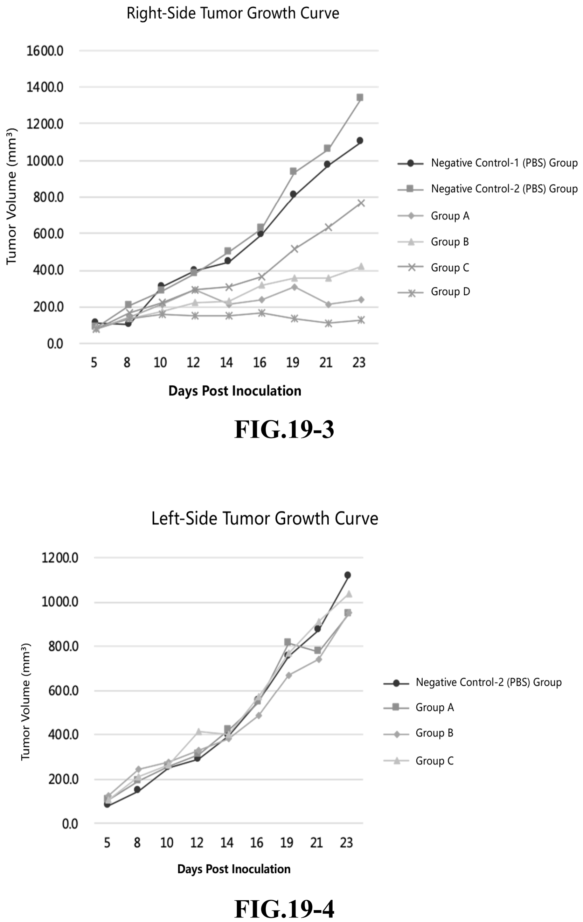

FIG. 19-3 illustrates a graph of right-side tumor growth in Example 19;

FIG. 19-4 illustrates a graph of left-side tumor growth in Example 19;

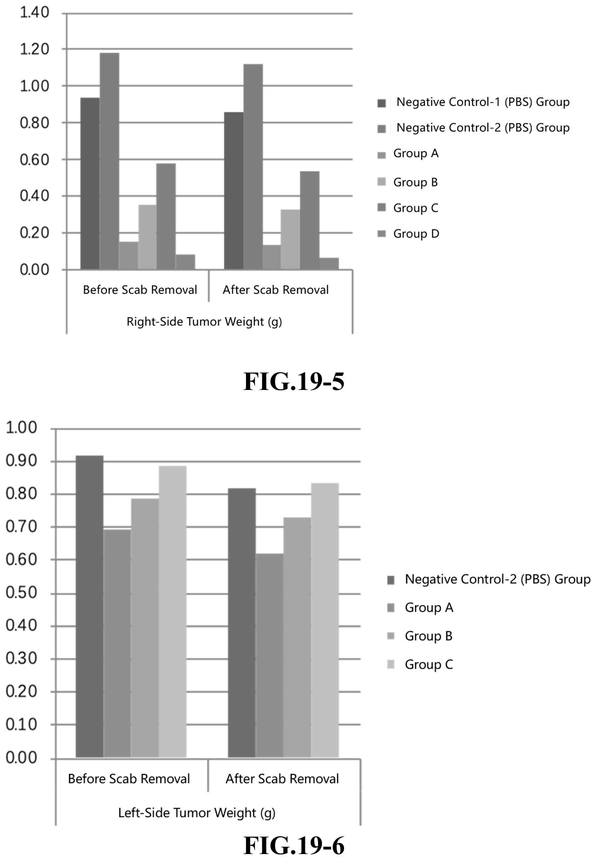

FIG. 19-5 illustrates a tumor weight comparison chart for the right side in Example 19;

FIG. 19-6 illustrates a tumor weight comparison chart for the left side in Example 19;

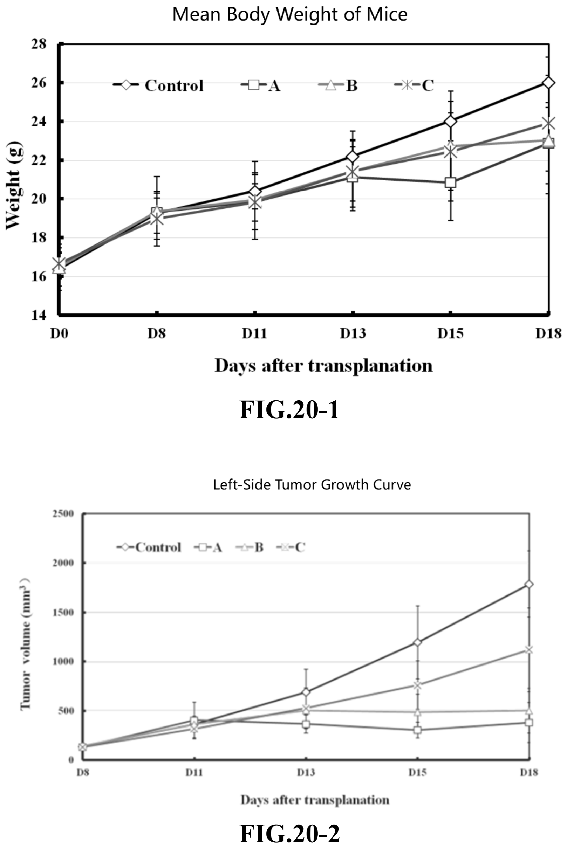

FIG. 20-1 illustrates a graph of animal body-weight changes in Example 20;

FIG. 20-2 illustrates a graph of left-side tumor growth in Example 20;

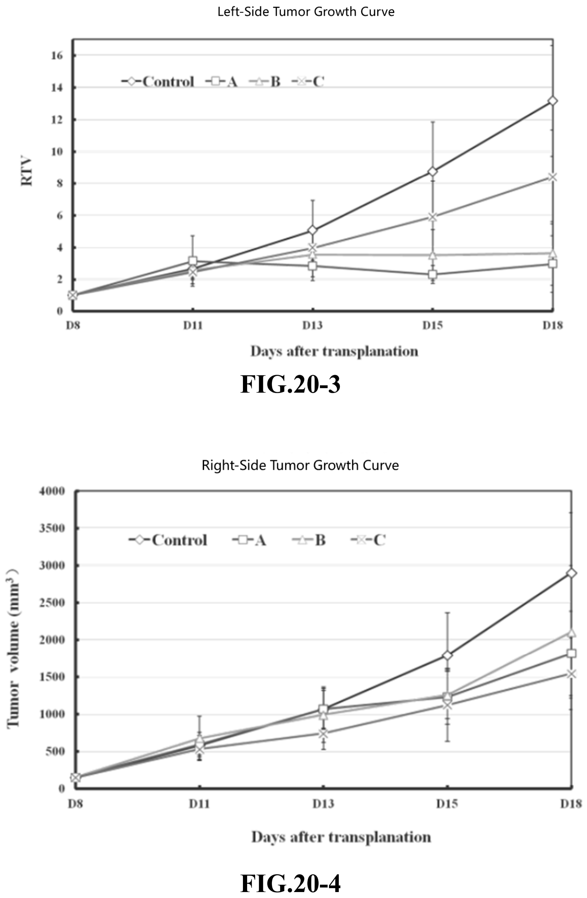

FIG. 20-3 illustrates a graph of left-side relative tumor growth in Example 20;

FIG. 20-4 illustrates a graph of right-side tumor growth in Example 20;

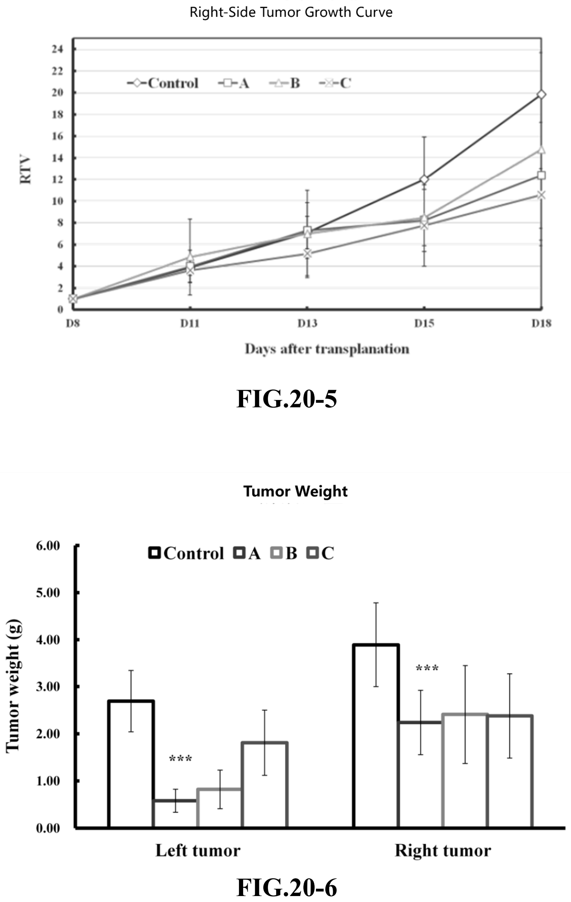

FIG. 20-5 illustrates a graph of left-side relative tumor growth in Example 20;

FIG. 20-6 illustrates a tumor weight comparison chart for the right side in Example 20;

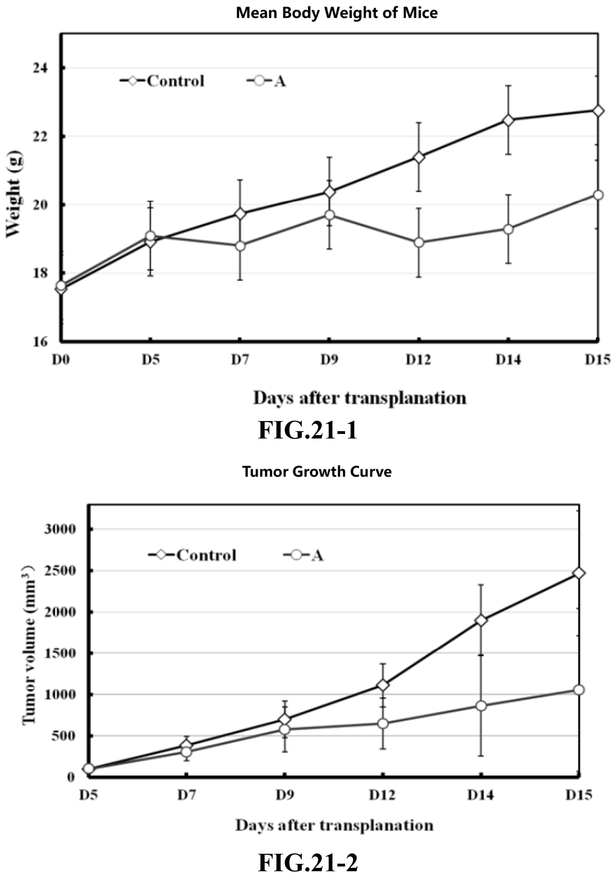

FIG. 21-1 illustrates a graph of animal body-weight changes in Example 21;

FIG. 21-2 illustrates a graph of tumor growth in Example 21;

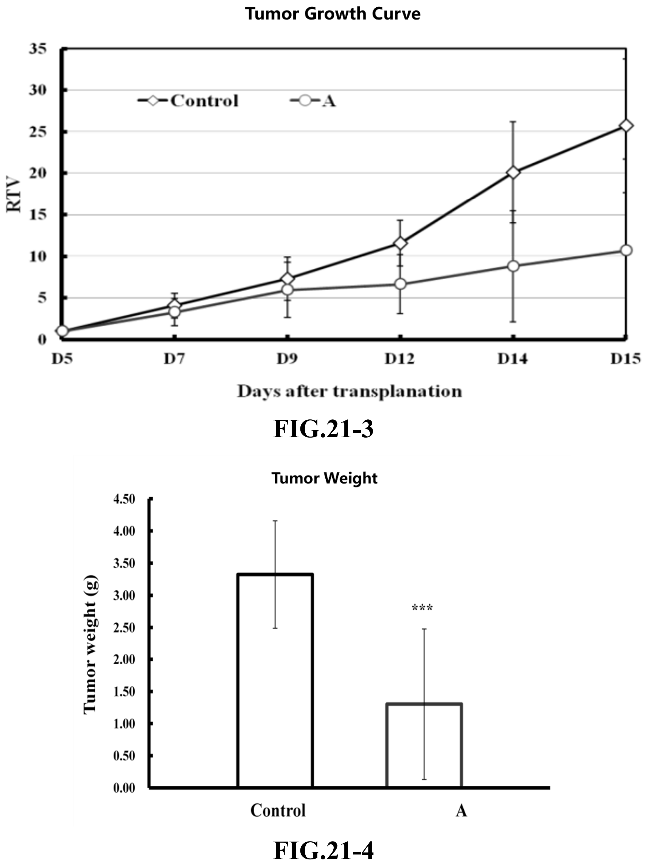

FIG. 21-3 illustrates a graph of relative tumor growth in Example 21;

FIG. 21-4 illustrates a tumor weight comparison chart in Example 21;

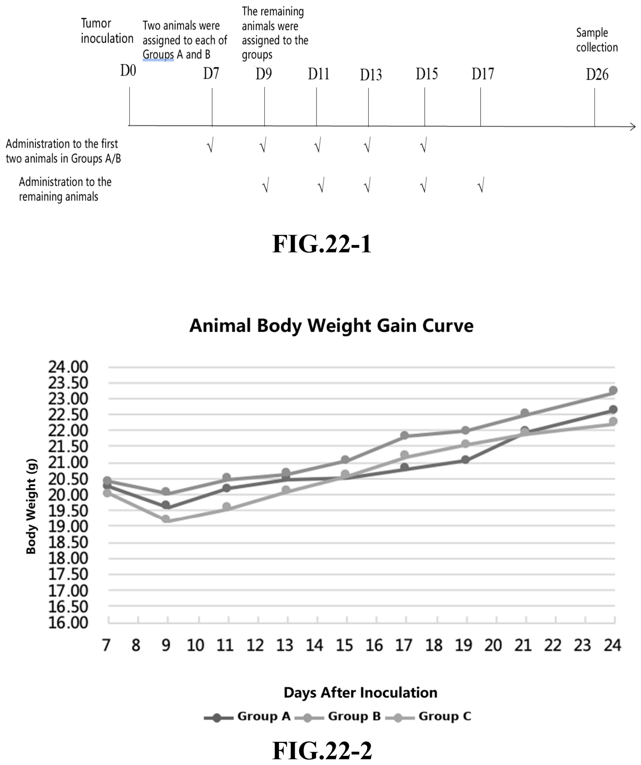

FIG. 22-1 illustrates a schematic diagram of the animal inoculation and dosing regimen in Example 22;

FIG. 22-2 illustrates a graph of animal body-weight changes in Example 22;

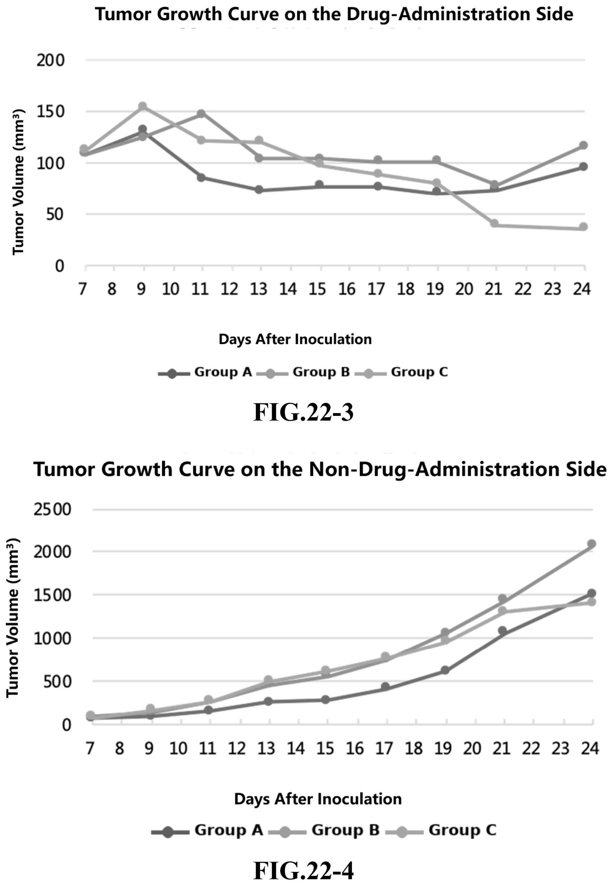

FIG. 22-3 illustrates a graph of tumor growth on the dosed side in Example 22;

FIG. 22-4 illustrates a graph of tumor growth on the undosed side in Example 22;

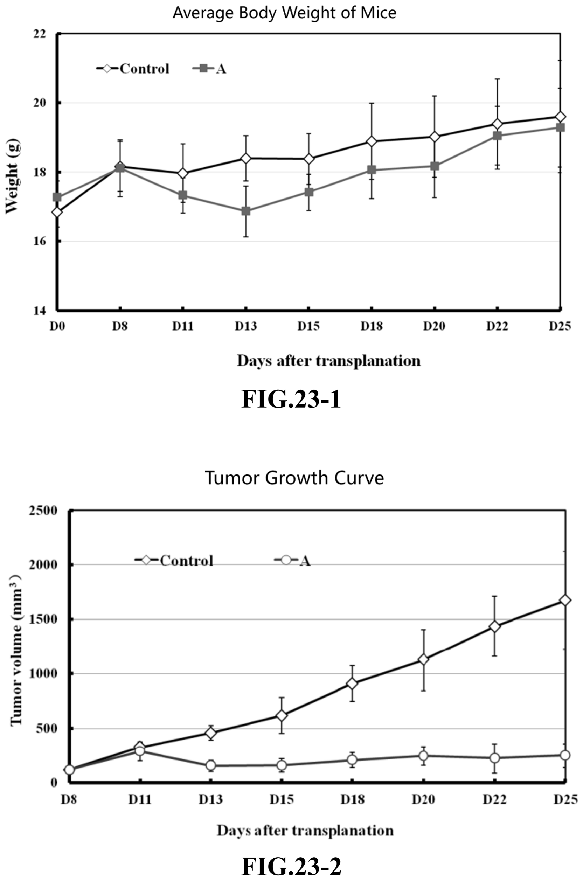

FIG. 23-1 illustrates a graph of animal body-weight changes in Example 23;

FIG. 23-2 illustrates a graph of tumor growth in Example 23;

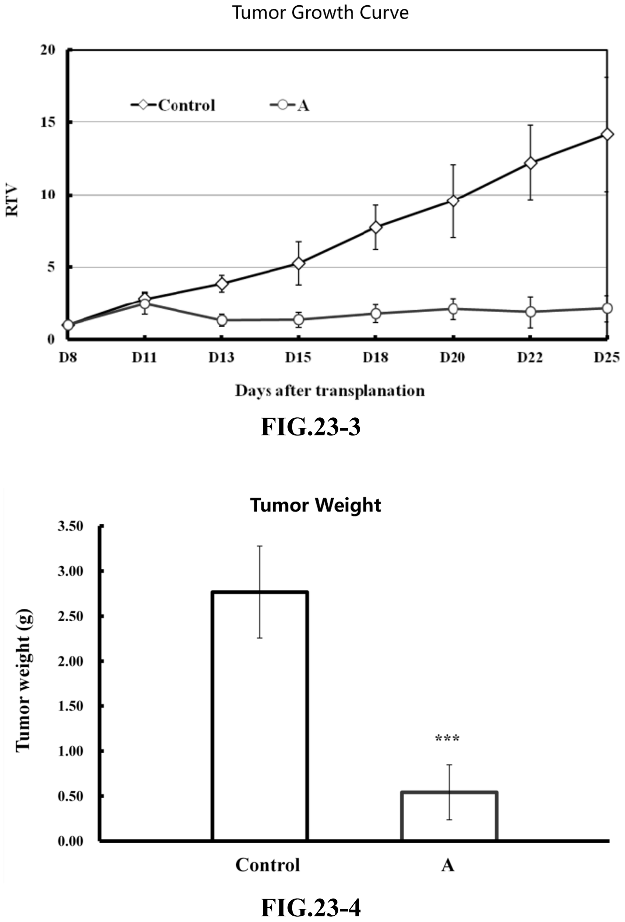

FIG. 23-3 illustrates a graph of relative tumor growth in Example 23;

FIG. 23-4 illustrates a tumor weight comparison chart in Example 23;

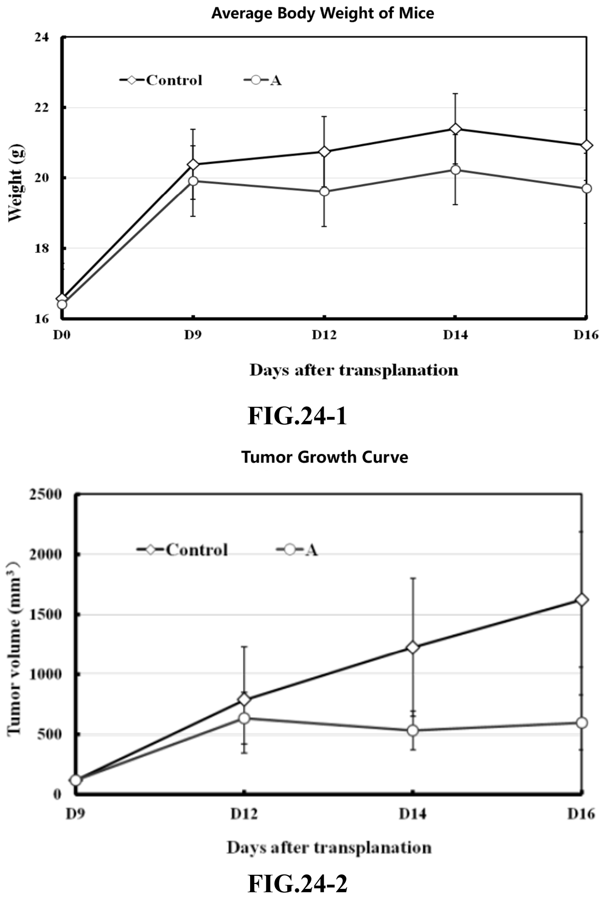

FIG. 24-1 illustrates a graph of animal body-weight changes in Example 24;

FIG. 24-2 illustrates a graph of tumor growth in Example 24;

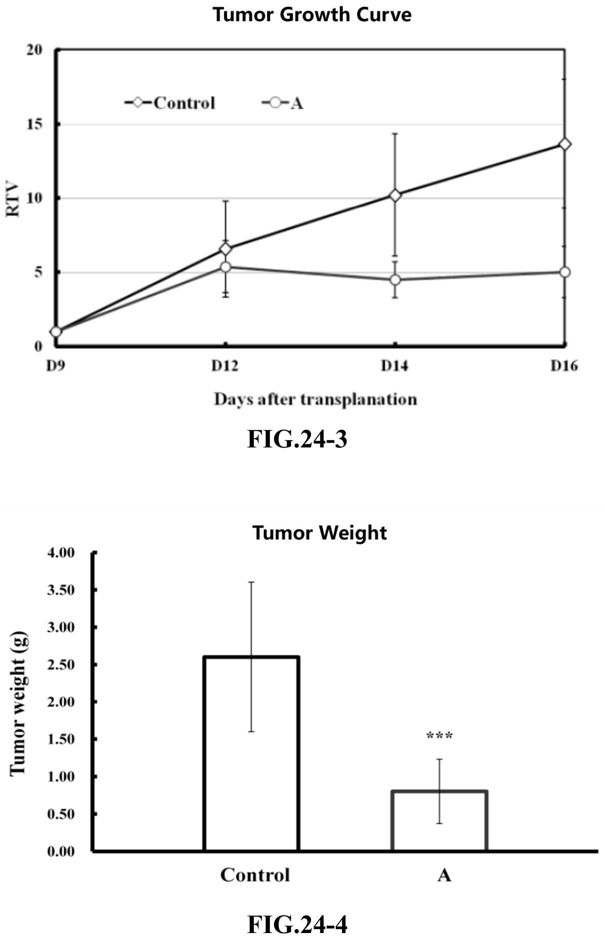

FIG. 24-3 illustrates a graph of relative tumor growth in Example 24;

FIG. 24-4 illustrates a tumor weight comparison chart in Example 24;

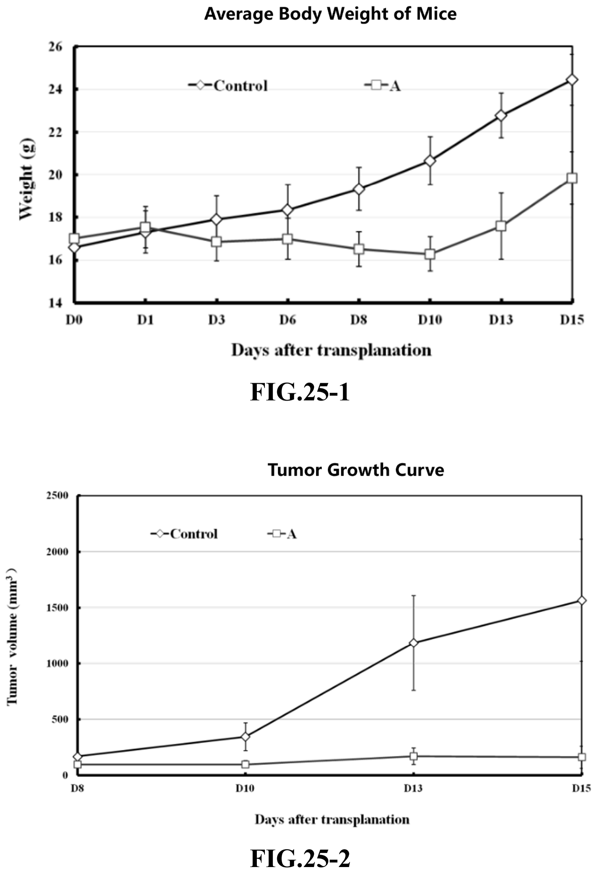

FIG. 25-1 illustrates a graph of animal body-weight changes in Example 25;

FIG. 25-2 illustrates a graph of tumor growth in Example 25;

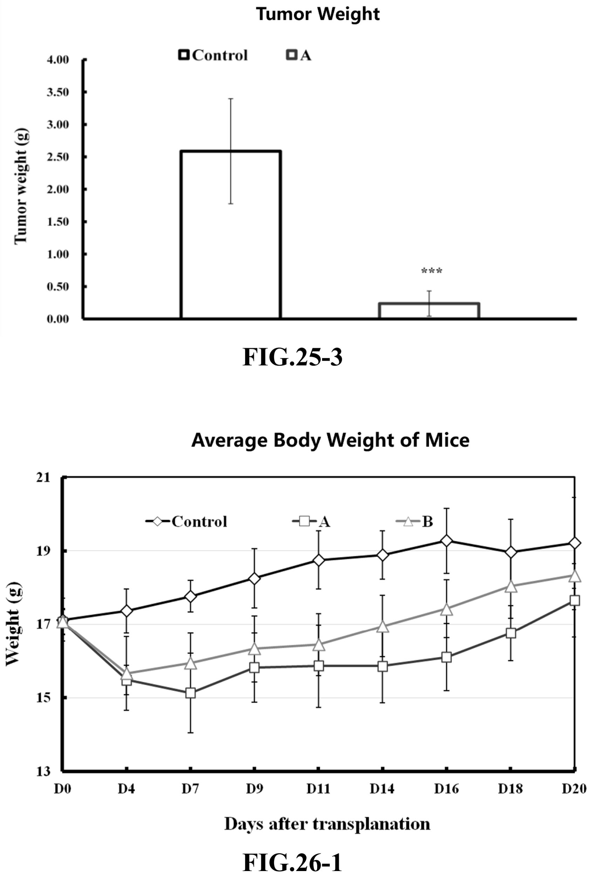

FIG. 25-3 illustrates a tumor weight comparison chart in Example 25;

FIG. 26-1 illustrates a graph of animal body-weight changes in Example 26;

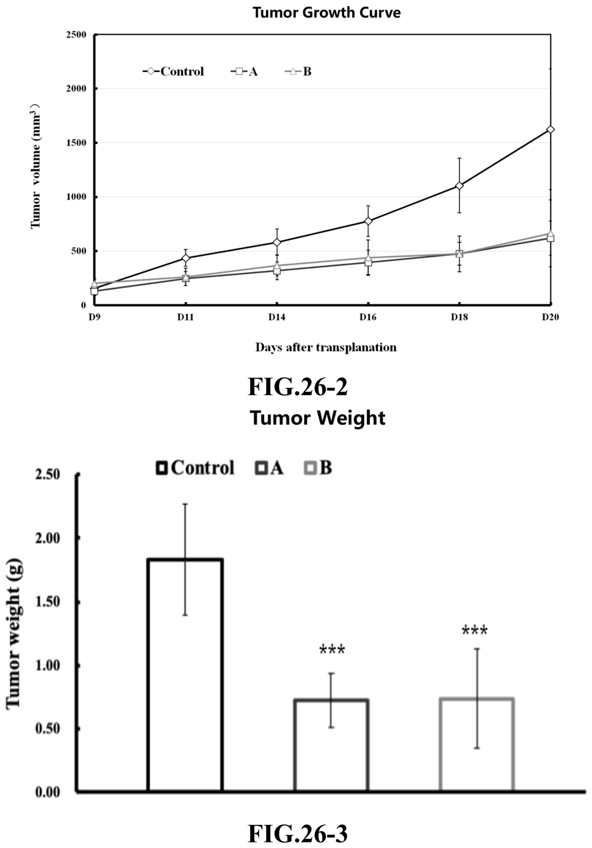

FIG. 26-2 illustrates a graph of tumor growth in Example 26;

FIG. 26-3 illustrates a tumor weight comparison chart in Example 26;

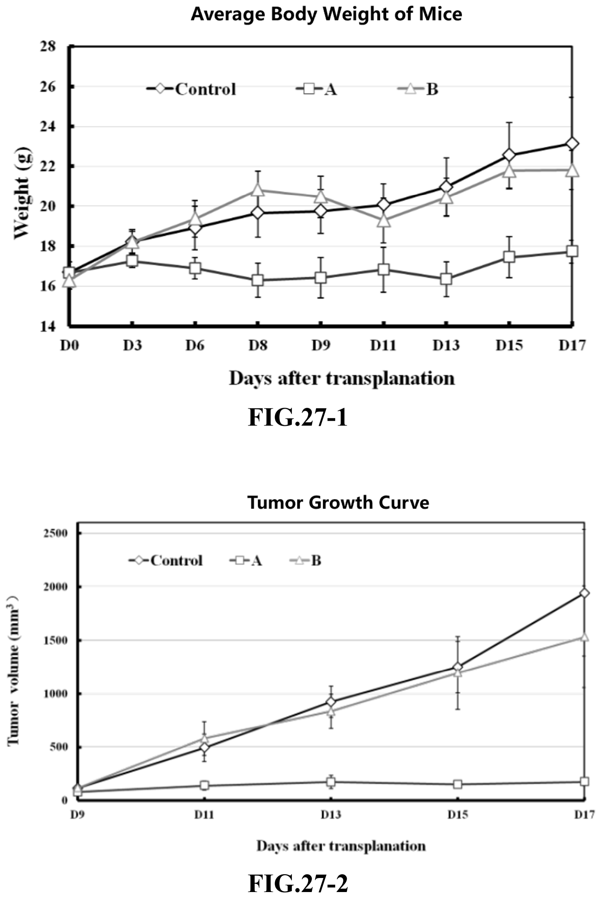

FIG. 27-1 illustrates a graph of animal body-weight changes in Example 27;

FIG. 27-2 illustrates a graph of tumor growth in Example 27;

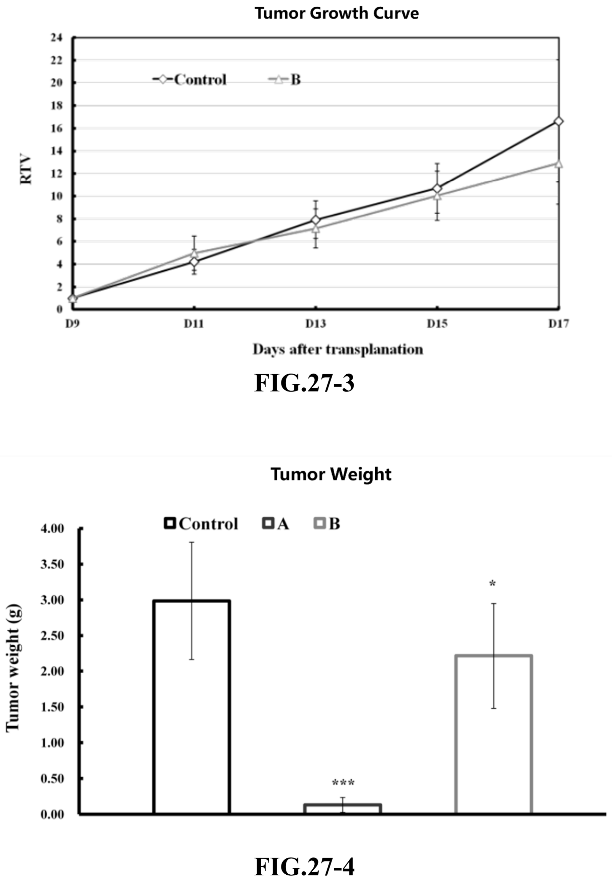

FIG. 27-3 illustrates a graph of relative tumor growth in Example 27;

FIG. 27-4 illustrates a tumor weight comparison chart in Example 27;

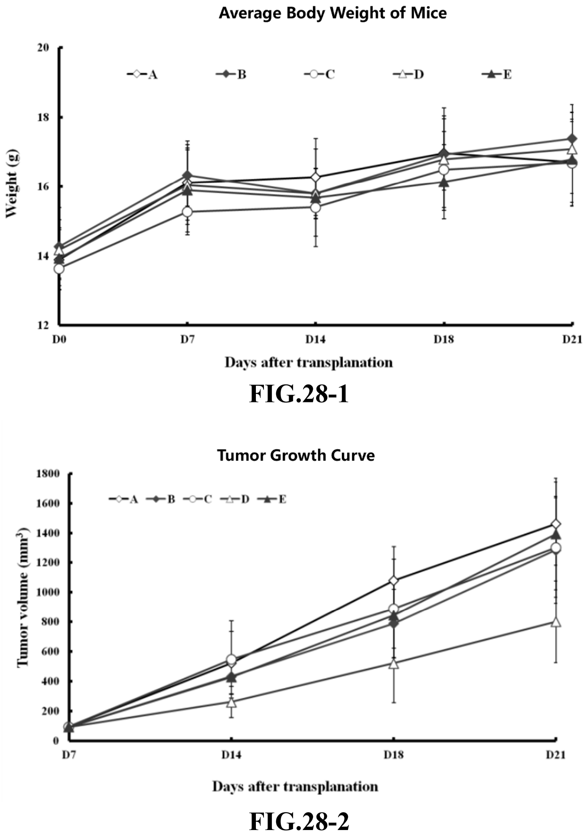

FIG. 28-1 illustrates a graph of animal body-weight changes in Example 28;

FIG. 28-2 illustrates a graph of tumor growth in Example 28;

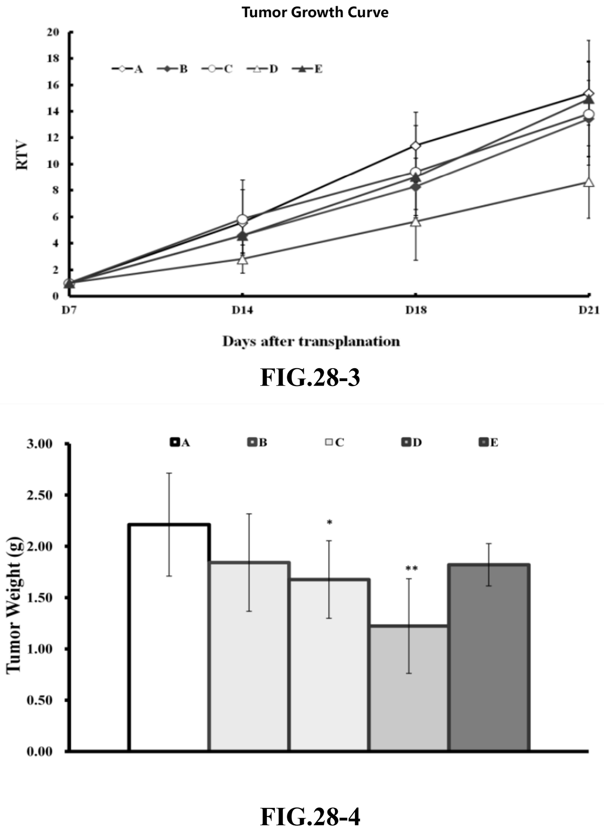

FIG. 28-3 illustrates a graph of relative tumor growth in Example 28;

FIG. 28-4 illustrates a tumor weight comparison chart in Example 28;

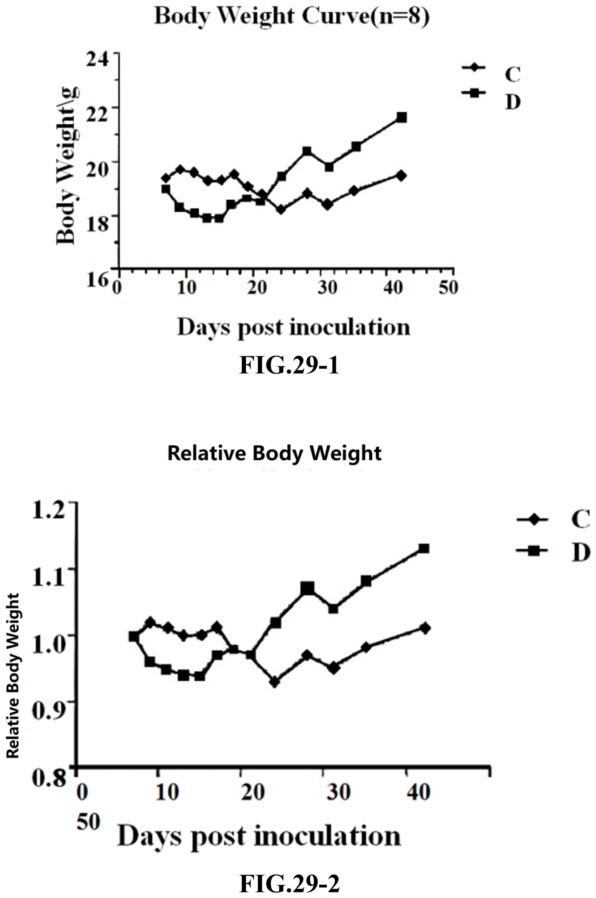

FIG. 29-1 illustrates a graph of tumor growth in Example 29;

FIG. 29-2 illustrates a graph of relative tumor growth in Example 29;

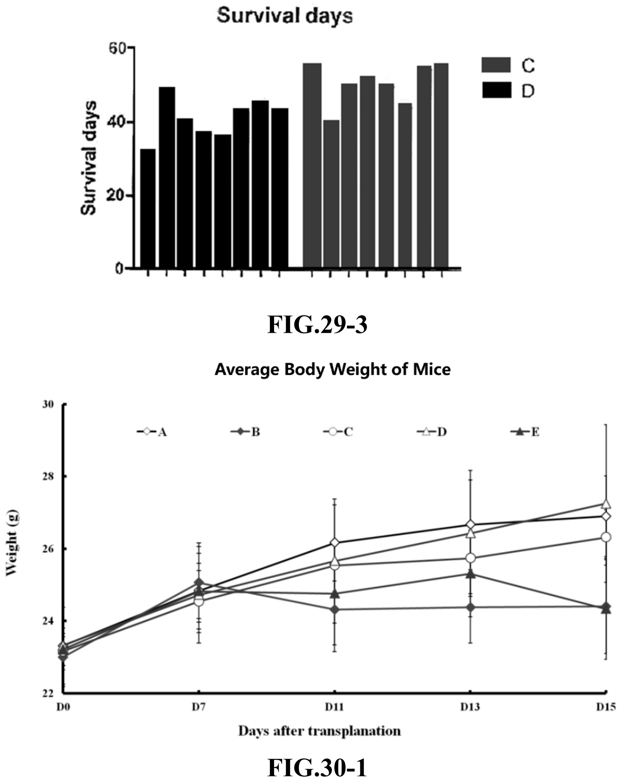

FIG. 29-3 illustrates a plot of survival days in Example 29;

FIG. 30-1 illustrates a graph of animal body-weight changes in Example 30;

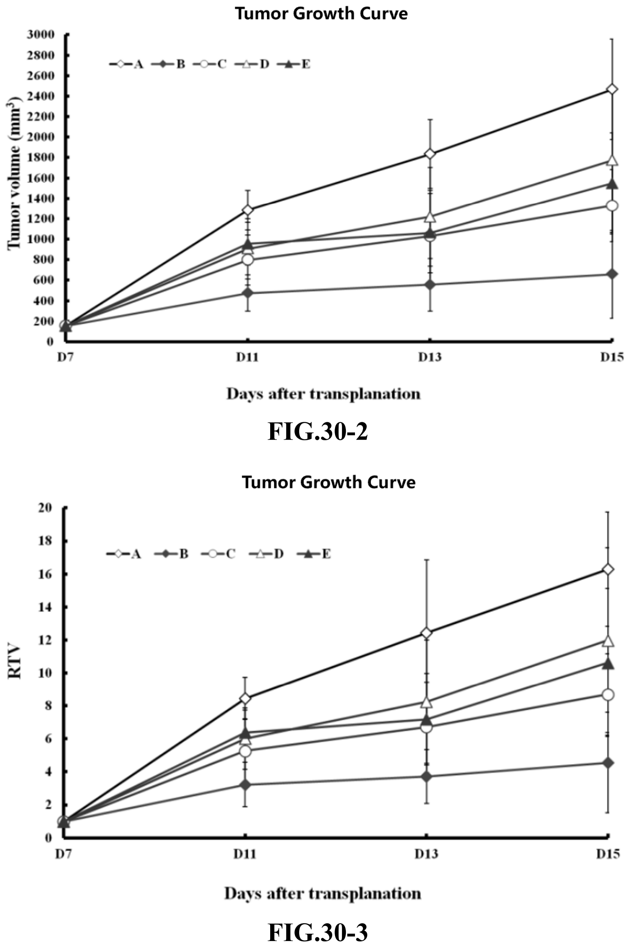

FIG. 30-2 illustrates a graph of tumor growth in Example 30;

FIG. 30-3 illustrates a graph of relative tumor growth in Example 30;

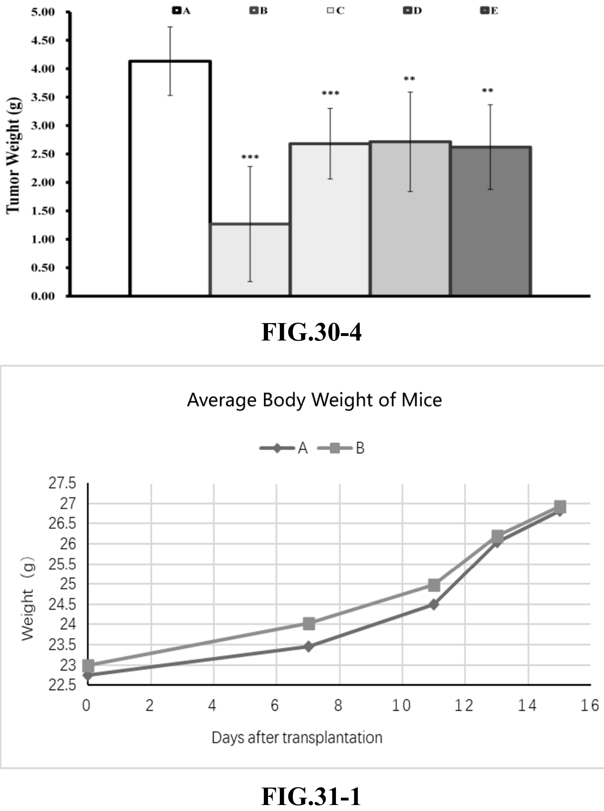

FIG. 30-4 illustrates a tumor weight comparison chart in Example 30;

FIG. 31-1 illustrates a graph of animal body-weight changes in Example 31;

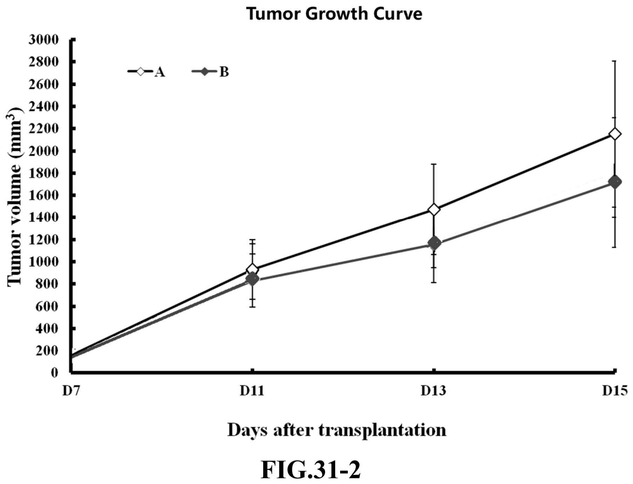

FIG. 31-2 illustrates a graph of tumor growth in Example 31;

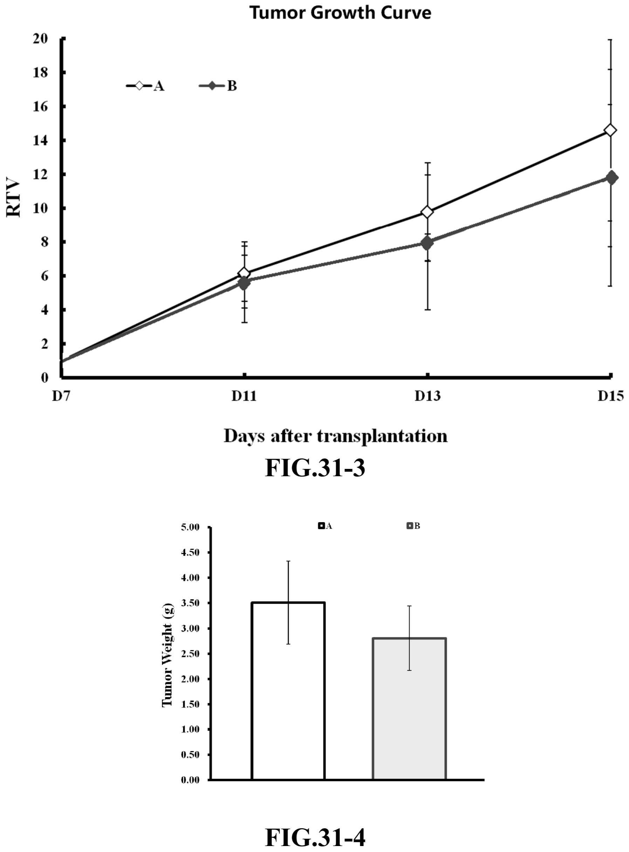

FIG. 31-3 illustrates a graph of relative tumor growth in Example 31;

FIG. 31-4 illustrates a tumor weight comparison chart in Example 31;

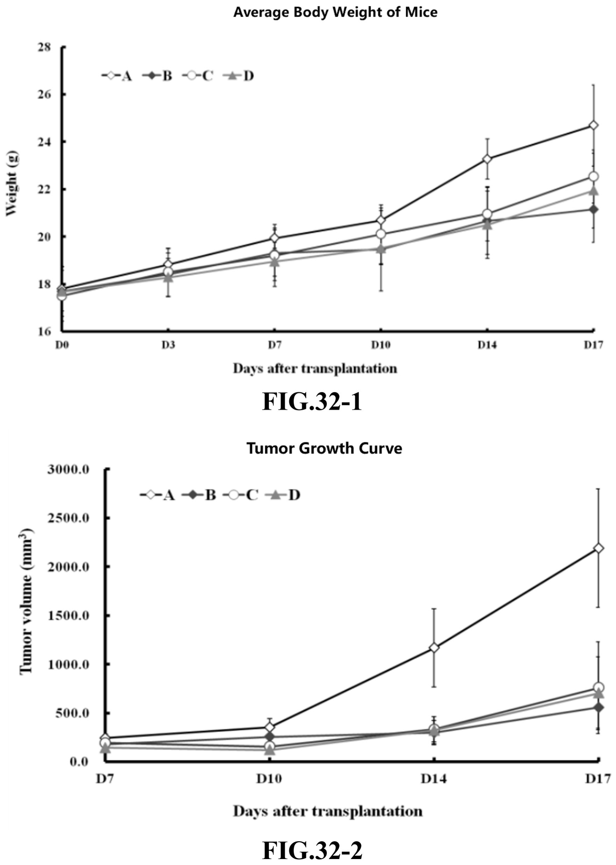

FIG. 32-1 illustrates a graph of animal body-weight changes in Example 32;

FIG. 32-2 illustrates a graph of tumor growth in Example 32;

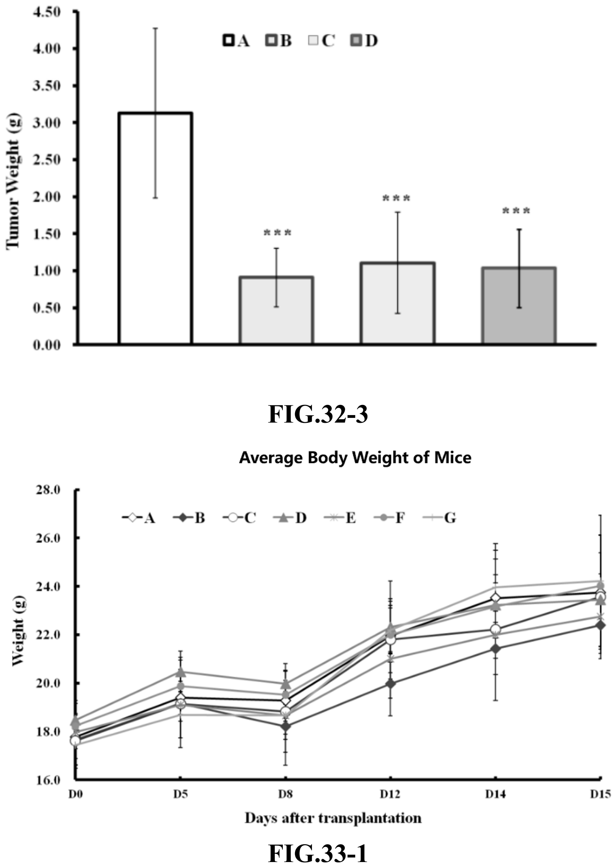

FIG. 32-3 illustrates a tumor weight comparison chart in Example 32;

FIG. 33-1 illustrates a graph of animal body-weight changes in Example 33;

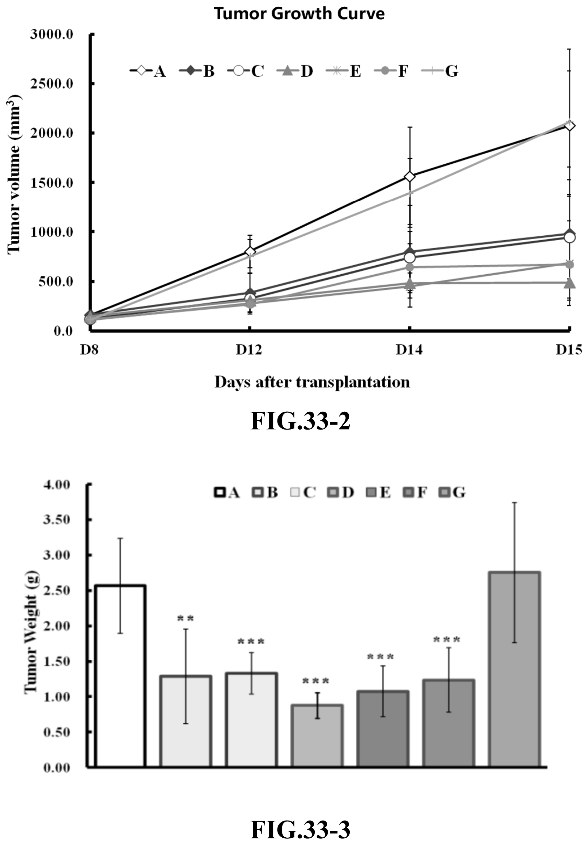

FIG. 33-2 illustrates a graph of tumor growth in Example 33;

FIG. 33-3 illustrates a tumor weight comparison chart in Example 33;

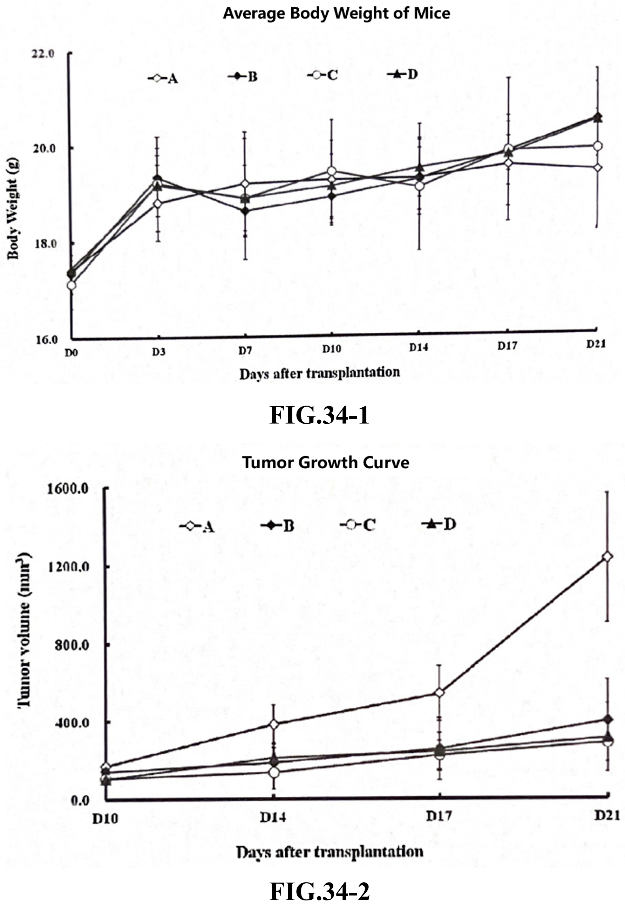

FIG. 34-1 illustrates a graph of animal body-weight changes in Example 34;

FIG. 34-2 illustrates a graph of tumor growth in Example 34;

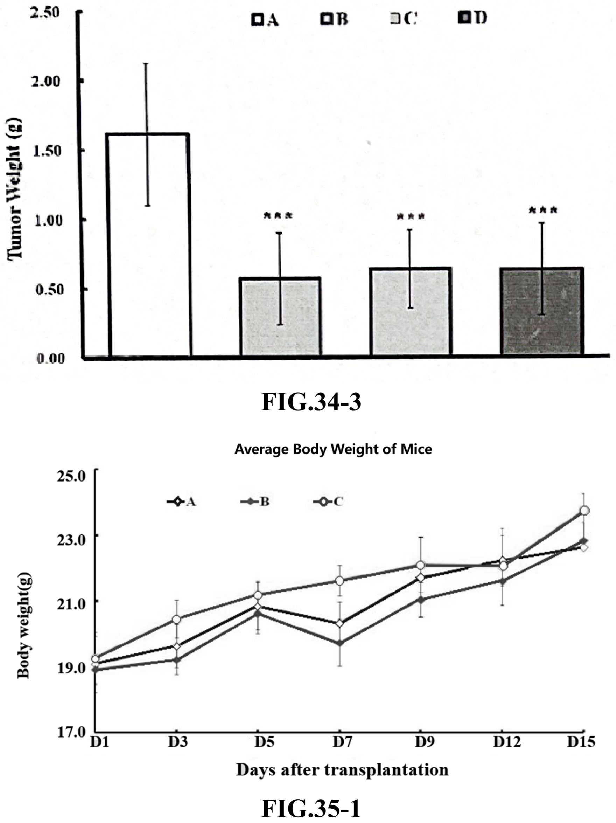

FIG. 34-3 illustrates a tumor weight comparison chart in Example 34;

FIG. 35-1 illustrates a graph of animal body-weight changes in Example 35;

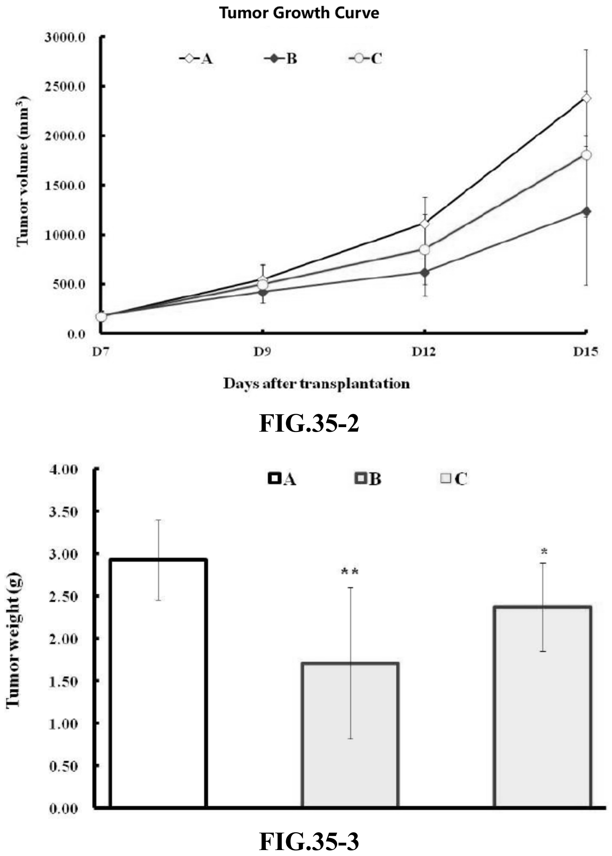

FIG. 35-2 illustrates a graph of tumor growth in Example 35;

FIG. 35-3 illustrates a tumor weight comparison chart in Example 35;

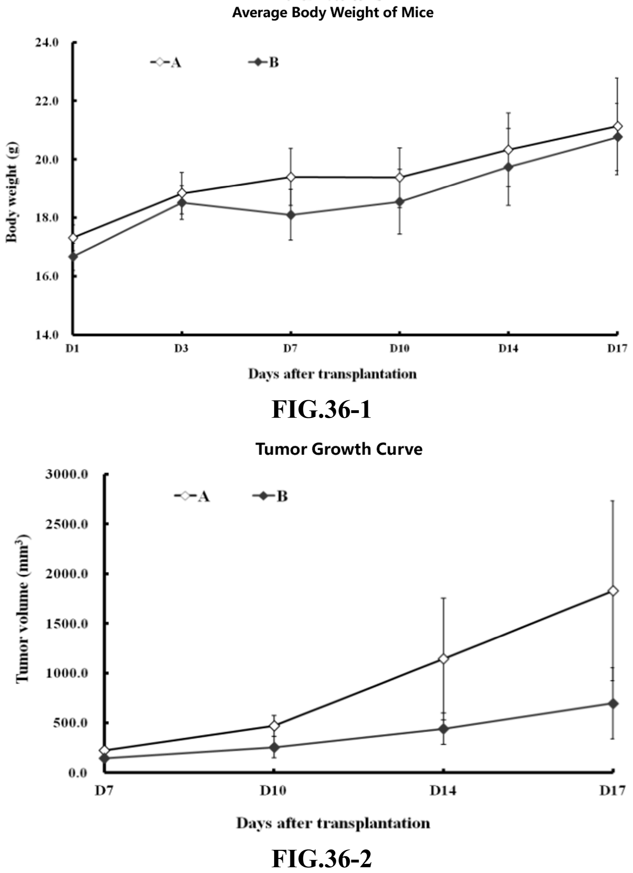

FIG. 36-1 illustrates a graph of animal body-weight changes in Example 36;

FIG. 36-2 illustrates a graph of tumor growth in Example 36;

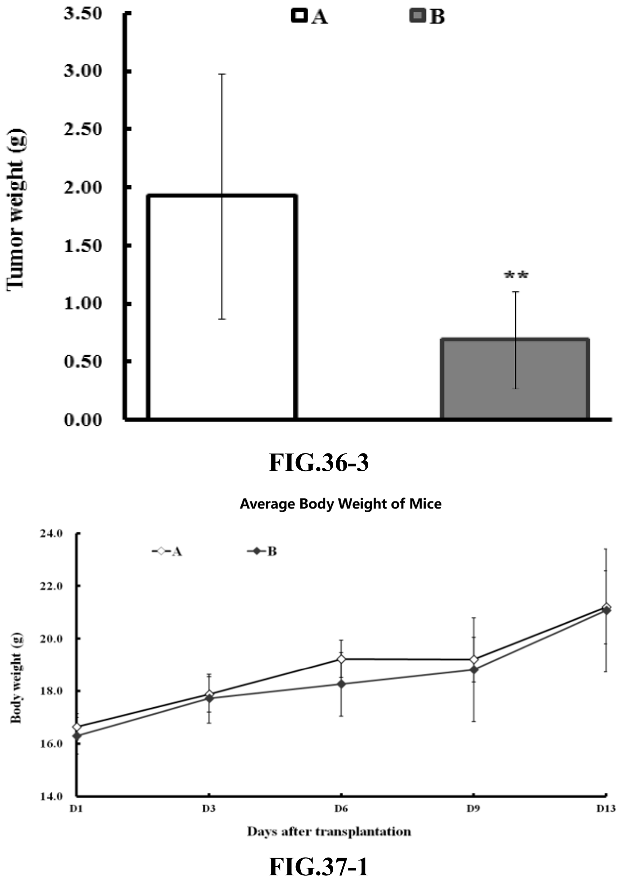

FIG. 36-3 illustrates a tumor weight comparison chart in Example 36;

FIG. 37-1 illustrates a graph of animal body-weight changes in Example 37;

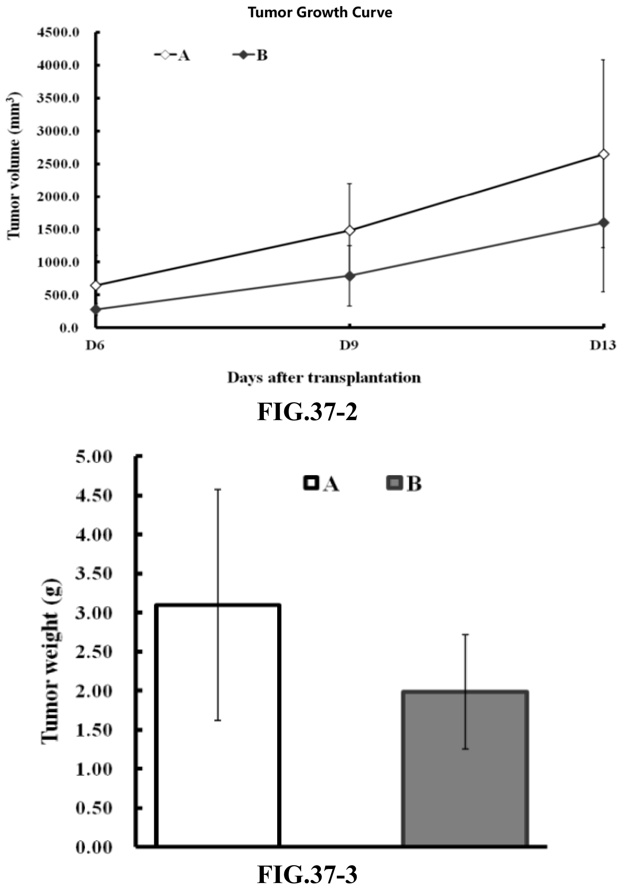

FIG. 37-2 illustrates a graph of tumor growth in Example 37;

FIG. 37-3 illustrates a tumor weight comparison chart in Example 37;

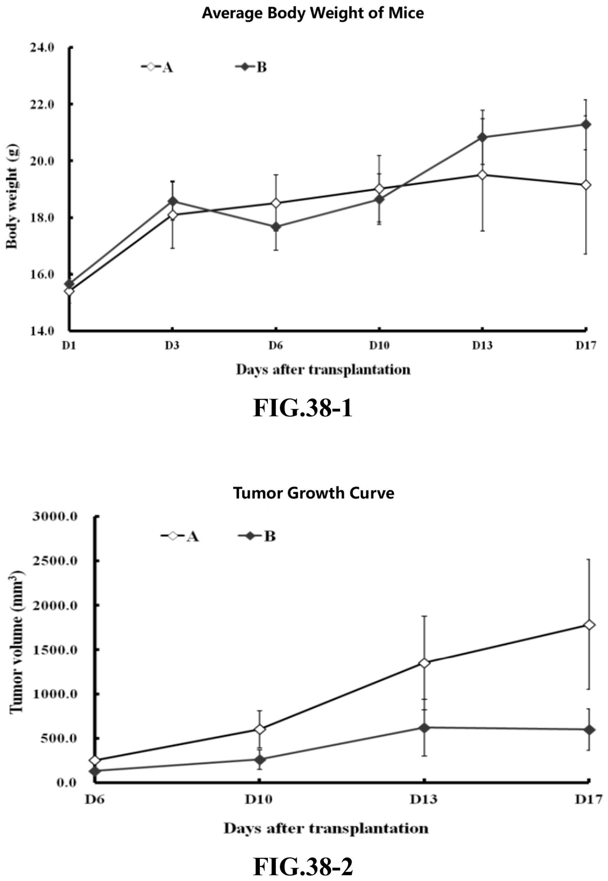

FIG. 38-1 illustrates a graph of animal body-weight changes in Example 38;

FIG. 38-2 illustrates a graph of tumor growth in Example 38;

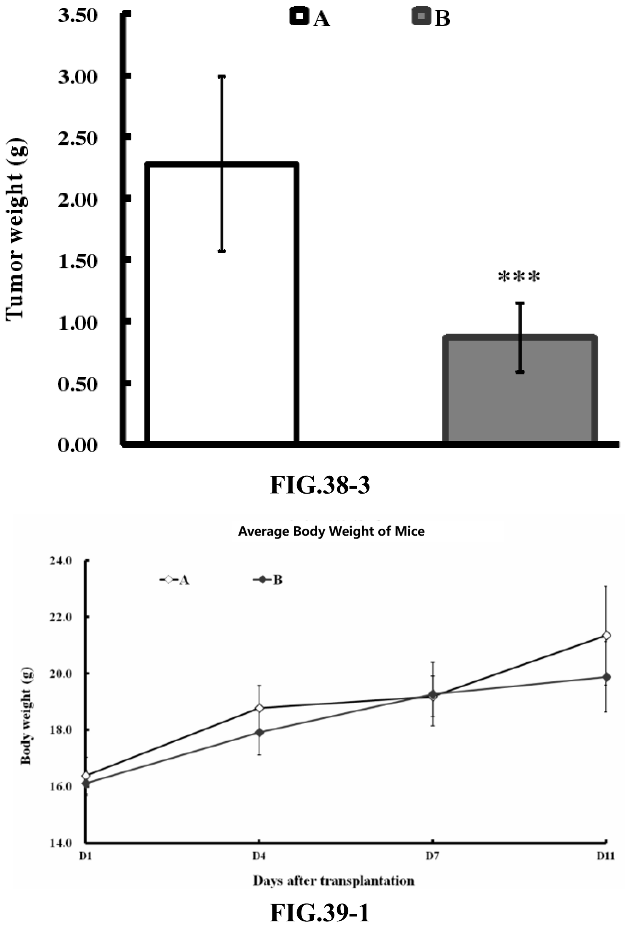

FIG. 38-3 illustrates a tumor weight comparison chart in Example 38;

FIG. 39-1 illustrates a graph of animal body-weight changes in Example 39;

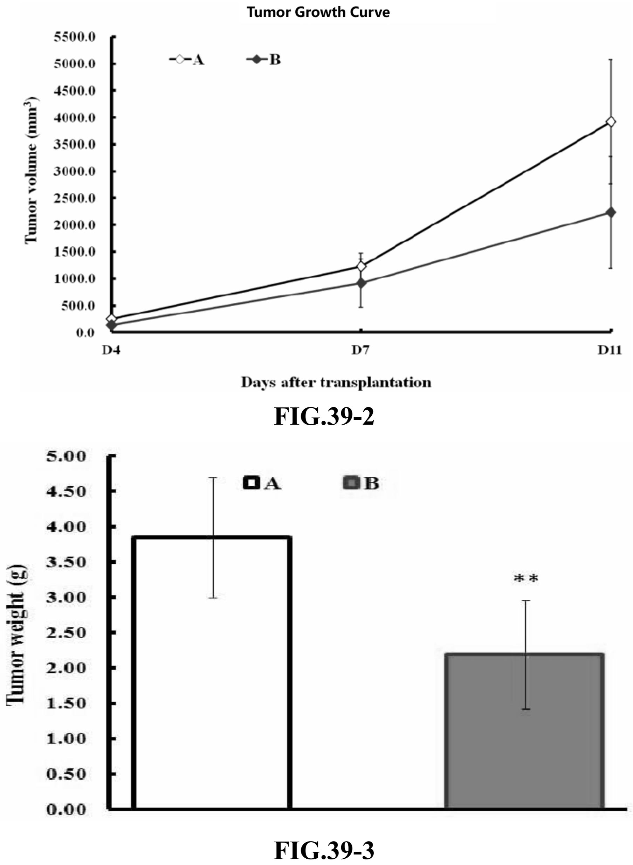

FIG. 39-2 illustrates a graph of tumor growth in Example 39;

FIG. 39-3 illustrates a tumor weight comparison chart in Example 39;

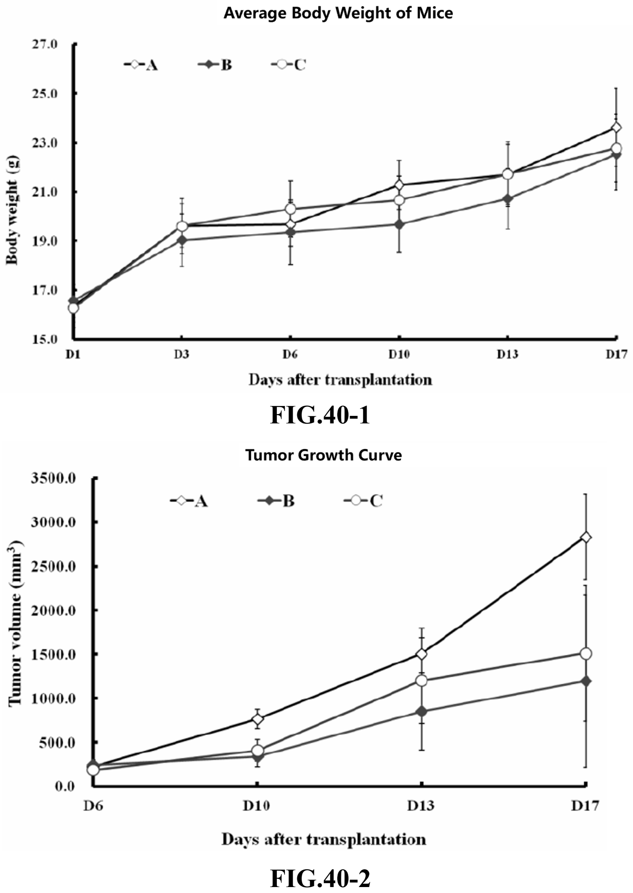

FIG. 40-1 illustrates a graph of animal body-weight changes in Example 40;

FIG. 40-2 illustrates a graph of tumor growth in Example 40;

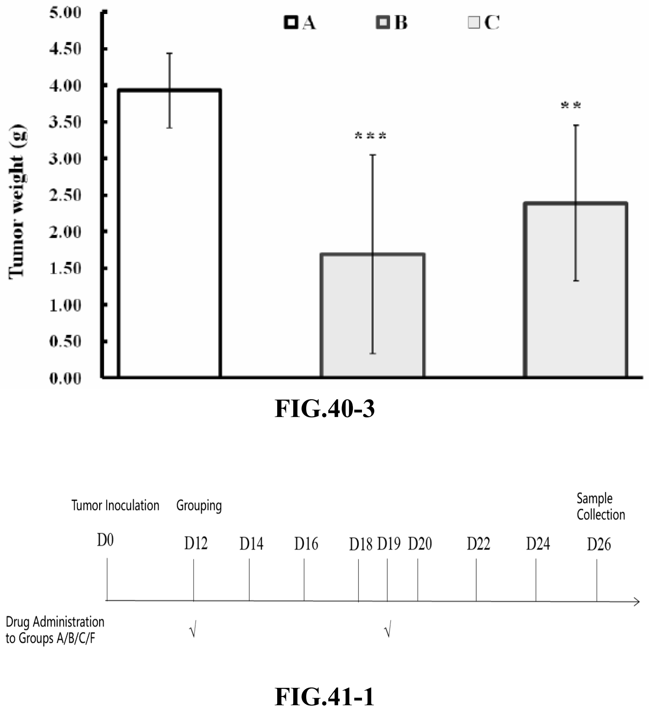

FIG. 40-3 illustrates a tumor weight comparison chart in Example 40;

FIG. 41-1 illustrates a schematic diagram of the animal inoculation and dosing regimen in Example 41;

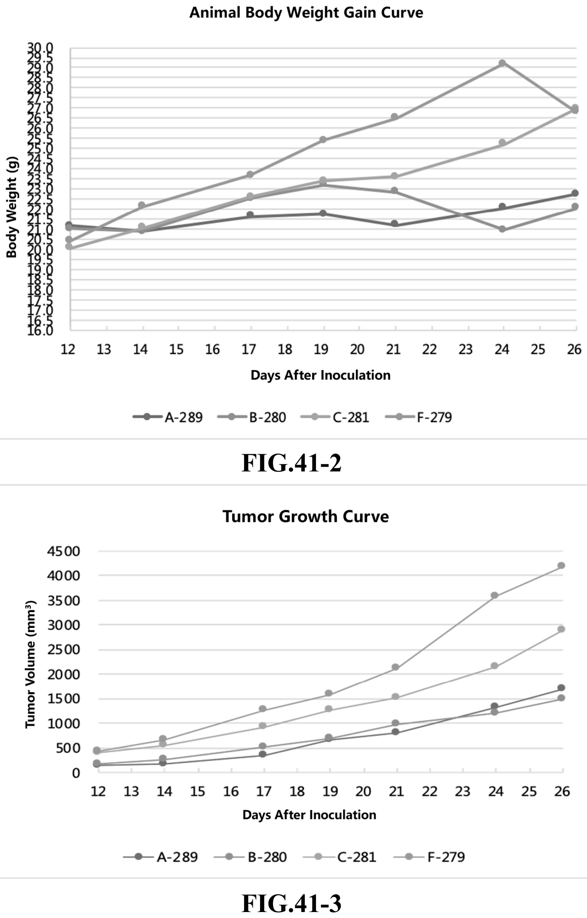

FIG. 41-2 illustrates a graph of animal body-weight changes in Example 41;

FIG. 41-3 illustrates a graph of tumor growth in Example 41;

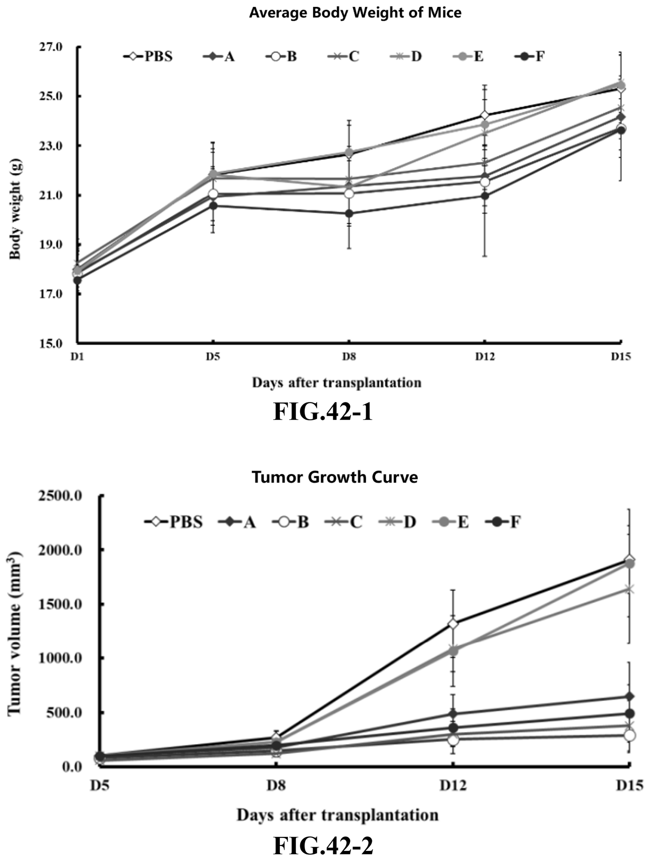

FIG. 42-1 illustrates a graph of animal body-weight changes in Example 42;

FIG. 42-2 illustrates a graph of tumor growth in Example 42;

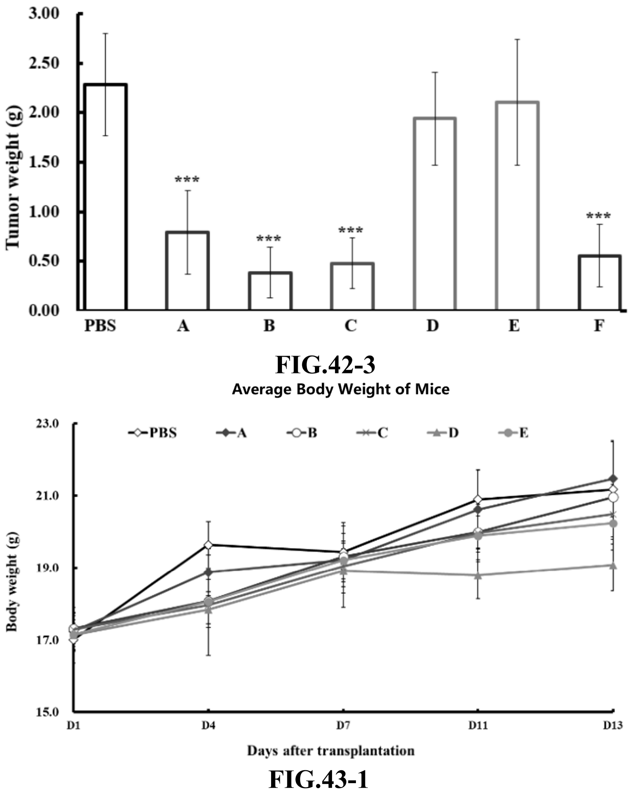

FIG. 42-3 illustrates a tumor weight comparison chart in Example 42;

FIG. 43-1 illustrates a graph of animal body-weight changes in Example 43;

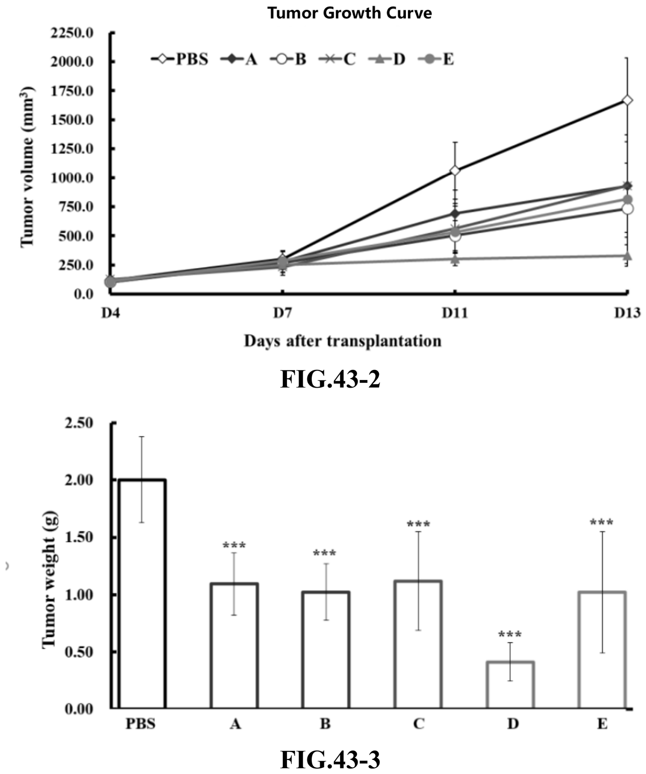

FIG. 43-2 illustrates a graph of tumor growth in Example 43;

FIG. 43-3 illustrates a tumor weight comparison chart in Example 43;

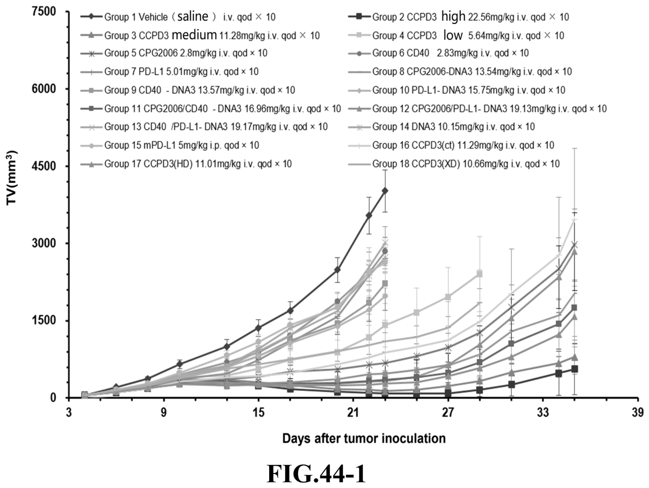

FIG. 44-1 illustrates the tumor growth curve of the MC38 subcutaneous xenograft tumor model after initiation of treatment in Example 44;

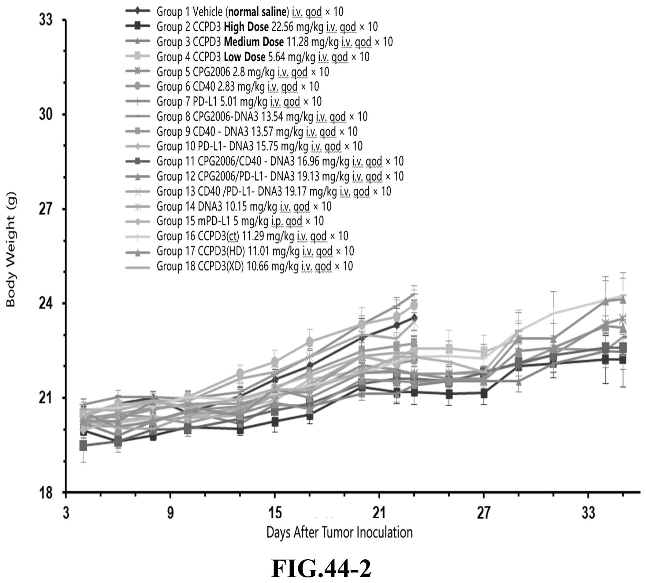

FIG. 44-2 illustrates the animal body-weight change curve of the MC38 subcutaneous xenograft tumor model after initiation of treatment in Example 44;

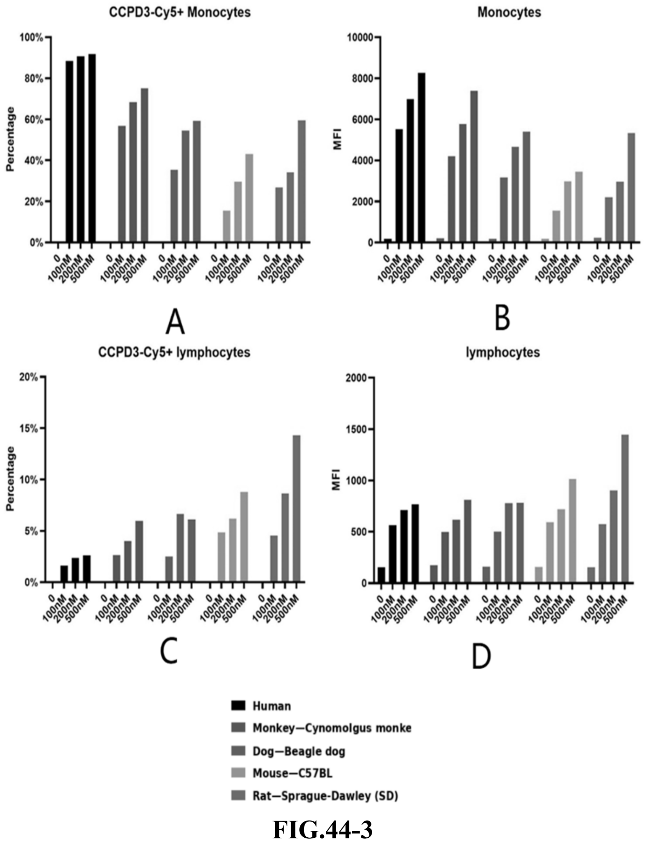

FIG. 44-3 illustrates: (A) the binding percentage of CCPD3-Cy5 at different concentrations (0, 100 nM, 200 nM, 500 nM) to monocytes from different species; (B) the mean fluorescence intensity (MFI) of CCPD3-Cy5 at different concentrations (0, 100 nM, 200 nM, 500 nM) bound to monocytes from different species; (C) the binding percentage of CCPD3-Cy5 at different concentrations (0, 100 nM, 200 nM, 500 nM) to lymphocytes from different species; and (D) the MFI of CCPD3-Cy5 at different concentrations (0, 100 nM, 200 nM, 500 nM) bound to lymphocytes from different species, in Example 44;

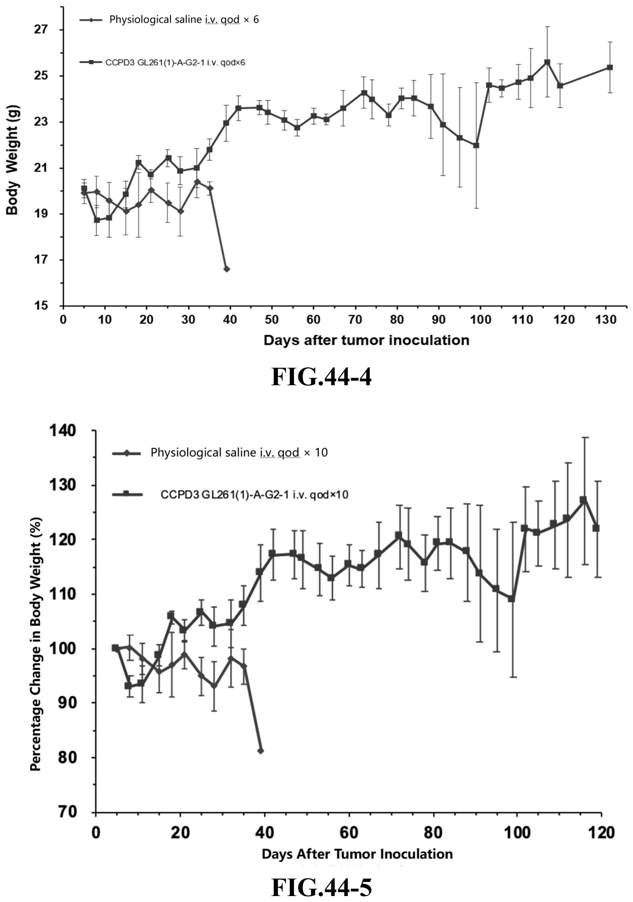

FIG. 44-4 illustrates the body-weight change curve of mice in the in vivo efficacy study of CCPD3 in the GL261-Luc orthotopic glioma mouse model in Example 44;

FIG. 44-5 illustrates the percent body-weight change curve of mice in the in vivo efficacy study of CCPD3 in the GL261-Luc orthotopic glioma mouse model in Example 44;

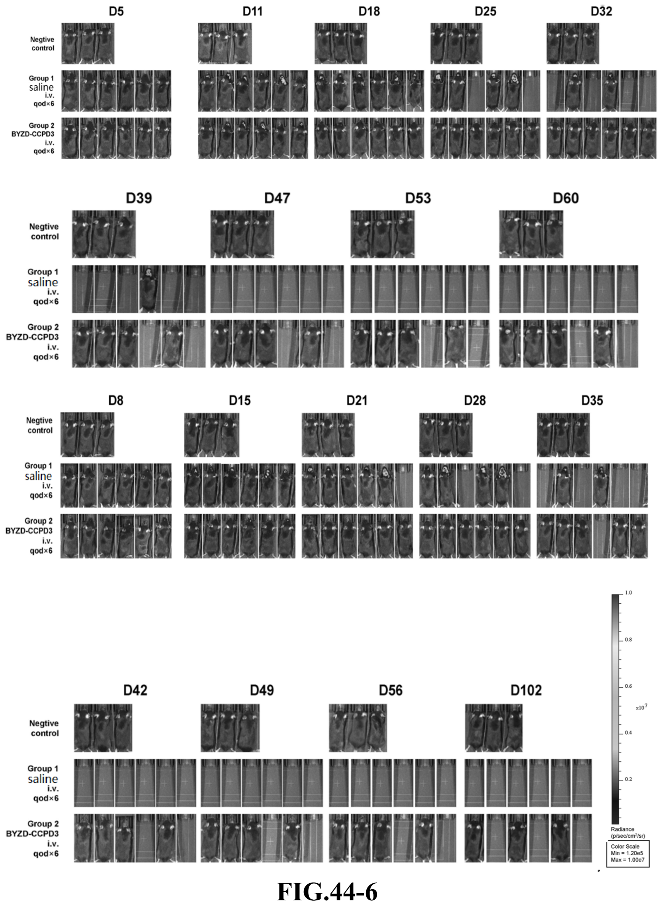

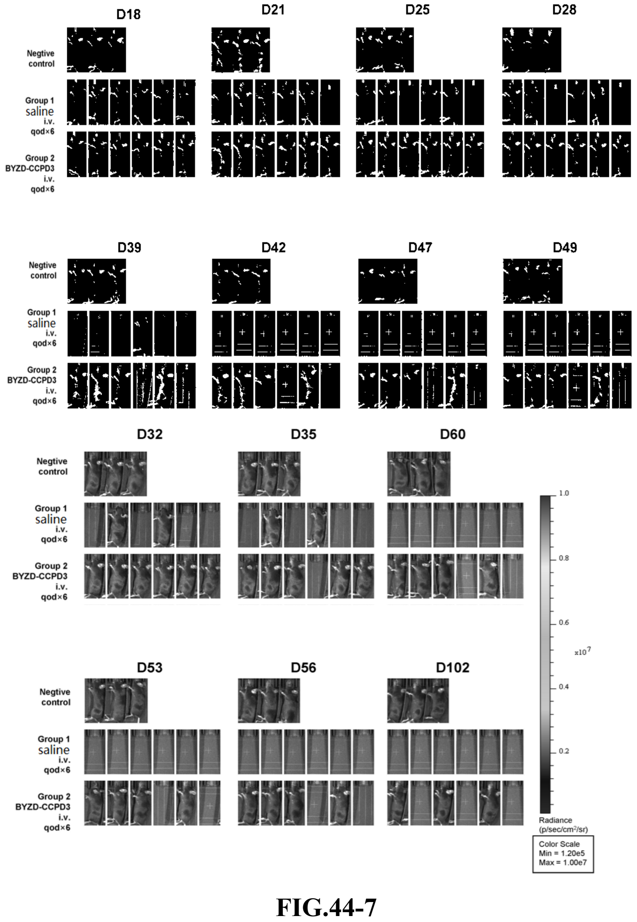

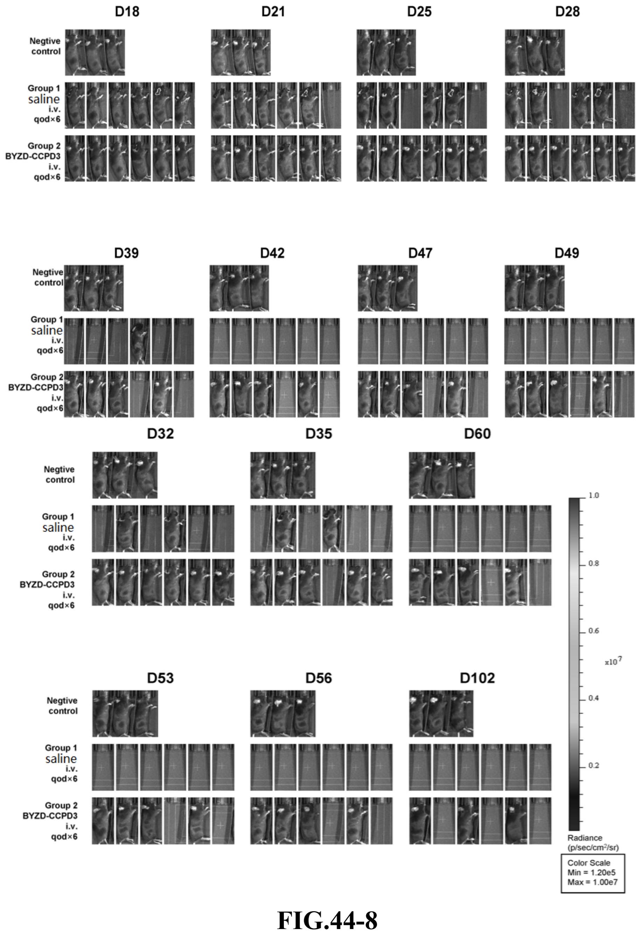

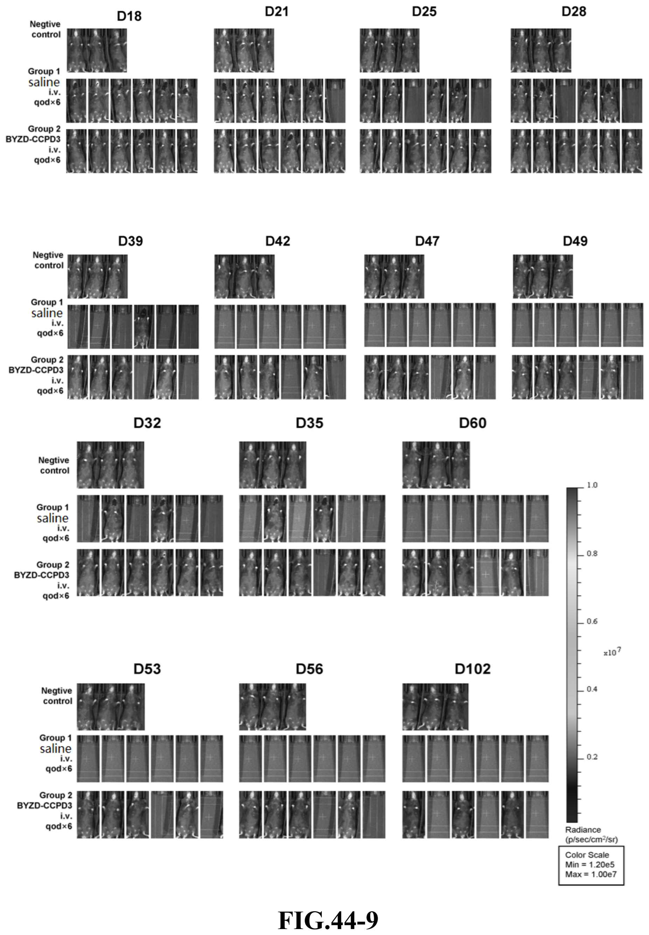

FIGS. 44-6 to 44-9 illustrate in vivo bioluminescence imaging (BLI) images of mice in each group after tumor inoculation in Example 44, wherein FIG. 44-6 is the dorsal view, FIG. 44-7 is the left lateral view, FIG. 44-8 is the right lateral view, and FIG. 44-9 is the ventral view;

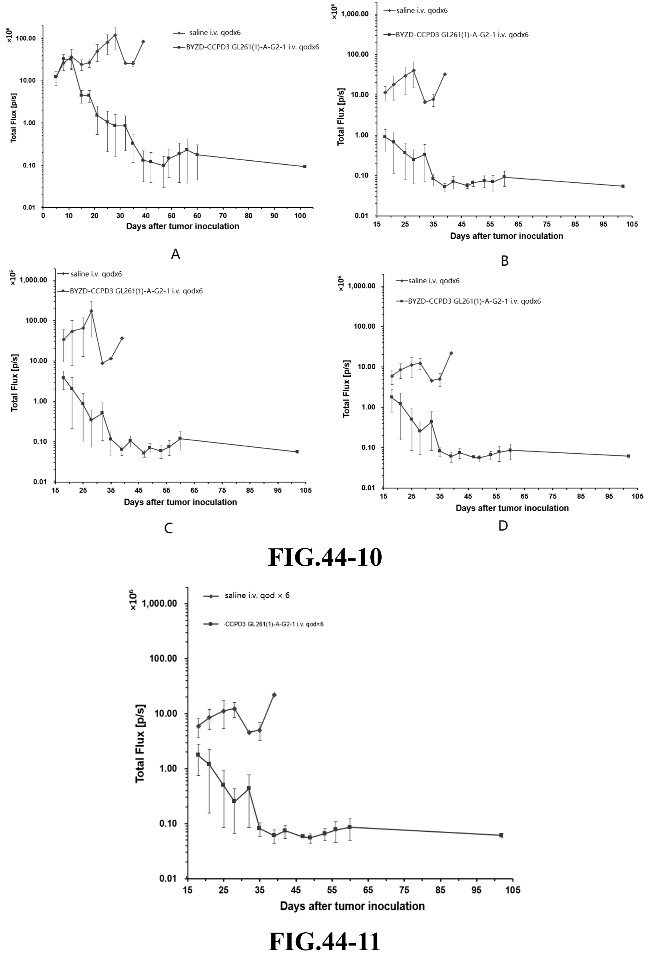

FIG. 44-10 illustrates curves of BLI value changes for each group in Example 44, wherein (A) is the dorsal-view BLI value change curve, (B) is the left lateral-view BLI value change curve, (C) is the right lateral-view BLI value change curve, and (D) is the ventral-view BLI value change curve;

FIG. 44-11 illustrates the mouse survival curve of the GL261-Luc orthotopic xenograft tumor model in Example 44;

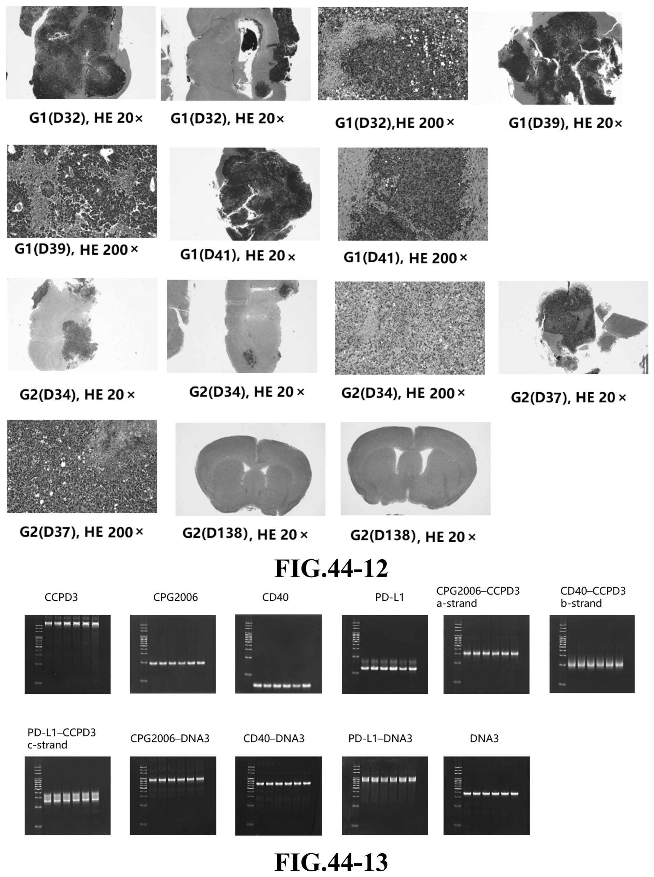

FIG. 44-12 illustrates hematoxylin-eosin (H&E)-stained histopathological sections of brain tissues from groups in the GL261-Luc orthotopic xenograft tumor model in Example 44;

FIG. 44-13 illustrates an electrophoresis image of the serum stability assay after 100-fold dilution in Example 44;

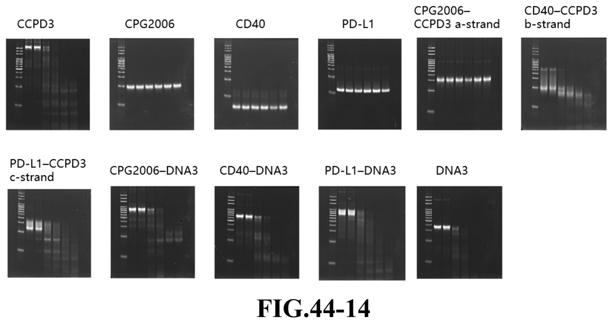

FIG. 44-14 illustrates an electrophoresis image of the serum stability assay in Example 44.

The "CD40" shown in the appendix figures is an abbreviation of the "CD40 aptamer" described in the embodiments; which have the same meaning, it is also equivalent in meaning to "Apt CD40" in Table 5. Likewise, the same applies toMUC1, CD16a, CTLA-4, PD-L1, C12, TTA1, AS1411, A15, PSMA, FAP, CD16a, Act-12c, PD1, Her2, Her3, VEGF, N48, CD3-4, PD-1, BCMA, CD38, FGF2, HBsAg, CPG 和 CD-19. "Bio" is equivalent to "biotin" , "survivin" is equivalent to " survivin iRNA" .

**Detailed Description**

It should be noted that, insofar as there is no conflict, the embodiments of the present application and the features of the embodiments may be combined with one another. The invention will be described in detail below with reference to the accompanying drawings and the embodiments.

Definitions:

**Oligonucleotide:** As used herein, "oligonucleotide" refers to oligonucleotide molecules including small interfering RNA (siRNA), antisense nucleic acid (ASO), microRNA (miRNA), and nucleic acid aptamers (aptamers). Oligonucleotide drugs are composed of nucleotides and constitute a novel class of therapeutics distinct from small-molecule drugs and antibody drugs. Compared with traditional chemical drug molecules, oligonucleotide drugs possess high specificity, strong efficacy, long-lasting activity, simple design and numerous potential targets. The major oligonucleotide drugs are siRNA drugs and antisense nucleic acid drugs; both primarily act on cytoplasmic mRNA and regulate protein expression to achieve therapeutic effect by recognizing and inhibiting target mRNA through complementary base pairing. As used in the present application, unless the context clearly indicates otherwise, the terms "nucleic acid" and "small nucleic acid" are used interchangeably and refer to small nucleic acids.

**Antisense nucleic acid (ASO):** ASO refers to an antisense oligonucleotide, typically a short (about 16-53 nucleotides), synthetic oligomer used to block the function of RNA (including mRNA or miRNA). ASOs generally bind with a high degree of complementarity to the target RNA and typically include chemically modified nucleotides and/or specific terminal sequences to increase affinity for the target sequence and to resist intracellular nuclease degradation.

ASOs are usually designed to hybridize to specific regions of the target mRNA to block access by the translation machinery, induce degradation of the mRNA, or modulate mRNA splicing.

ASOs (also referred to by function as anti-miRNAs) are typically designed to hybridize to a specific miRNA, thereby preventing the miRNA from binding to its target mRNAs and inhibiting the miRNA's function (miRNAs are short non-coding RNAs that can form complementary base pairs with the 3' untranslated region (3' UTR) of target mRNAs to affect mRNA stability and translation, thereby regulating protein expression). ASOs that act as antimiRs in this application include, without limitation, A-miR21, A-miR-10a, A-miR-30c, and AmiR1306.

**MicroRNA (miRNA):** A short non-coding RNA of approximately 22 nucleotides in length. Its mechanism of action involves complementary base-pairing with the 3' untranslated region (3' UTR) of a target mRNA, thereby regulating the stability and/or translation of the target mRNA and, in turn, affecting protein expression. miRNAs have been shown to play key roles in numerous biological processes, including development, differentiation, proliferation, and apoptosis.

miRNA exists in multiple forms. The primary transcript (pri-miRNA) is processed to generate the precursor microRNA (pre-miRNA), and the pre-miRNA is further cleaved by the endonuclease Dicer to yield mature miRNA. In practice, pre-miRNA was adopted earliest and remains widely used; many commercial microRNA libraries are provided in pre-miRNA form. Recently, studies have shown that the two arms (5p and 3p) of the microRNA hairpin make important contributions to the formation of mature miRNAs; accordingly, the native pri-miRNA form is being increasingly utilized by researchers.

In addition, as part of a therapeutic strategy to increase the level of a specific miRNA in vivo (e.g., miR-34a or miR-122), the concept of an miRNA "supplement" has been proposed. Such supplements are typically miRNA analogs or mimics and may be in RNA form or in DNA form. When in DNA form, provided that they can be transcribed into an RNA having the same sequence, they can perform the same function in cells as the native miRNA.

Using DNA-sequence miRNA supplements offers certain advantages. First, DNA is more stable and more resistant to degradation by endogenous nucleases. Second, DNA can be read by the cellular transcription machinery and transcribed into the corresponding RNA, thereby increasing intracellular miRNA levels. Finally, DNA is relatively easier and less costly to synthesize, facilitating design and manufacturing, particularly for large-scale production and application.

By way of example, miR-34a has been demonstrated to exert important antitumor effects in many cancer types;

thus, increasing miR-34a levels in vivo may be an effective cancer therapy. Providing a DNA-sequence miR-34a supplement (i.e., an miRNA analog) can raise miR-34a levels and thereby inhibit cancer cell growth and survival.

Accordingly, as used in this application, the term "miRNA" is employed in a broad sense and encompasses various forms, including the aforementioned pri-miRNA, pre-miRNA, mature miRNA, and miRNA analogs (in DNA form) that can be transcribed to generate an oligonucleotide having the same sequence and function as the mature miRNA. In the present application, the miRNA analogs include, without limitation, one or more of miR-34, miR-542, miR-126, and miR-122.

Chemical modification of oligonucleotide drugs at specific positions can enhance the stability of these small nucleic acid molecules and avoid eliciting immune responses. The chemical modifications involved in the oligonucleotide drugs of the present application include, but are not limited to, the following:

1) Backbone modification: Phosphorothioate (PS) linkage modification is achieved by substituting a non-bridging oxygen atom in the phosphodiester linkage with a sulfur atom. PS linkages confer resistance to nucleases and, as compared with unmodified oligonucleotides, increase binding to plasma proteins and prolong the half-life.

2) Ribose modification: Ribose modification refers to replacement of the 2'-OH on the nucleoside ribose with 2'-F, 2'-OMe, or 2'-O-MOE. Such modification can affect the affinity between the small nucleic acid and the target RNA, stability against nucleases (e.g., ribonucleases), and the properties of the complex formed upon binding to RNA. A commonly used modification is the 2'-O-methoxyethyl modification (2'-O-MOE).

3) Base modification: Base modification can strengthen interactions between the oligonucleotide drug and the bases of the mRNA. The only base modification in widespread use at present is 5-methyl substitution at the C5 position of the pyrimidine ring (5-methylcytosine).

Although various carriers that improve drug-delivery efficiency, including nucleic acid carriers, are known in the art, it remains difficult to overcome limitations on clinical application. To address the foregoing, the present invention provides a nucleic acid carrier comprising a sequence a, a sequence b, and a sequence c, the nucleic acid carrier being selected from any of: a DNA carrier, an RNA carrier, or a DNA-RNA hybrid carrier; wherein the sequences a, b, and c self-assemble to form the nucleic acid carrier. The sequences a, b, and c include carrier backbone sequences, which include the following core sequences: a sequence a1, a sequence b1, and a sequence c1, or any variant sequence of at least one of the sequences a1, b1, and c1, the variant sequence comprising insertion, deletion, or substitution of at least one base. In the DNA carrier, the sequence a1 is SEQ ID No. 1, the sequence b1 is SEQ ID No. 2, and the sequence c1 is SEQ ID No. 3; in the RNA carrier, the sequence a1 is SEQ ID No. 4, the sequence b1 is SEQ ID No. 5, and the sequence c1 is SEQ ID No. 6. The DNA-RNA hybrid carrier is a carrier formed by self-assembly from a combination of the sequences of the DNA carrier and the sequences of the RNA carrier,

SEQ ID No. 1: ACGAGCGTTCCG;

SEQ ID No. 2: CGGTTCGCCG;

SEQ ID No. 3: CGGCCATAGCCGT;

SEQ ID No. 4: ACGAGCGUUCCG;

SEQ ID No. 5: CGGUUCGCCG;

SEQ ID No. 6: CGGCCAUAGCCGU.

In the preferred embodiments described above, the nucleic acid carrier is formed by self-assembly of sequences comprising the foregoing core sequences. Self-assembly yields a three-dimensional nanostructure that effectively reduces or prevents nuclease degradation and increases carrier stability. When nucleic acid nanoparticles self-assembled from three single strands containing the core sequences set forth herein are used as delivery carriers for oligonucleotide drugs, they exhibit greater physicochemical compatibility with oligonucleotide drugs than lipid nanoparticles (LNPs) and polymeric nanoparticles (PNPs) in the prior art, because both the carrier and the delivered drug are nucleotide sequences. This facilitates loading of different types of small nucleic acid molecules according to differences in the carrier's sequence architecture or length and, by leveraging the advantage of complementary base pairing, not only improves delivery efficiency but also renders the delivery system more stable. Moreover, the nucleic acid carrier comprising the above core sequences has inherent immunostimulatory activity; thus, when used as a delivery carrier for nucleic acid drugs, it not only serves as a carrier but also, to some extent, enhances the pharmacological efficacy of the nucleic acid drug. In addition, the preparation process of the nucleic acid carrier is simpler and more efficient.

In the above nucleic acid carrier, the lengths of the three sequences may be appropriately extended, depending on the length of the oligonucleotide drug sequence to be delivered subsequently, so as to further improve stability after loading the oligonucleotide drug. In a preferred embodiment of the present application, the carrier backbone sequence further includes a first extension segment and an optional second extension segment, the first extension segment and the second extension segment being located at the 5' end and/or the 3' end of any one of the sequences a1, b1, and c1. preferably, the lengths of the first extension segment and the second extension segment are each independently 1-14 nt, preferably 1-10 nt, more preferably 2-7 nt, and further preferably 3-7 nt. Specifically, they may be 1 nt, 2 nt, 3 nt, 4 nt, 5 nt, 6 nt, 7 nt, 8 nt, 9 nt, 10 nt, 11 nt, 12 nt, 13 nt, or 14 nt.

In some preferred embodiments, the first extension segment is selected from any one or more of the following DNA extension segments or RNA extension segments corresponding thereto:

> 1) 5' end of sequence a1: GACGCCC; 3' end of sequence c1: GGGCGTC; or the RNA extension segments corresponding to the DNA extension segments in (1);

> 2) 3' end of sequence a1: GGAGAGG; 5' end of sequence b1: CCTCTCC; or the RNA extension segments corresponding to the DNA extension segments in (2);

> 3) 3' end of sequence b1: CGAGCC; 5' end of sequence c1: GGCTCG or GGCACG; or the RNA extension segments corresponding to the DNA extension segments in (3);

> 4) 5' end of sequence a1: GGCGCCC or GACGCCC; 3' end of sequence c1: GGGCGCC; or the RNA extension segments corresponding to the DNA extension segments in (4);

> 5) 3' end of sequence a1: GGAG; 5' end of sequence b1: CTCC; or the RNA extension segments corresponding to the DNA extension segments in (5);

> 6) 3' end of sequence b1: CCAGCC; 5' end of sequence c1: GGCACG or GGCTCG; or the RNA extension segments corresponding to the DNA extension segments in (6).

preferably, the second extension segment is selected from any one or more of the following DNA extension segments or RNA extension segments corresponding thereto:

> 1) 5' end of sequence a1: C or GC; 3' end of sequence c1: GC or GCT; or the RNA extension segments corresponding to the DNA extension segments in (1);

> 2) 3' end of sequence a1: AG, AGC, or AGCT; 5' end of sequence b1: GCT or CT; or the RNA extension segments corresponding to the DNA extension segments in (2);

> 3) 3' end of sequence b1: GC, GCG, or GCGT; 5' end of sequence c1: GC or CGC; or the RNA extension segments corresponding to the DNA extension segments in (3);

> 4) 3' end of sequence a1: C, AGGCC, AGGCCT, or AGGAG; 5' end of sequence b1: G, GGCCT, or CTCCT; or the RNA extension segments corresponding to the DNA extension segments in (4);

> 5) 3' end of sequence b1: GCC or GCCT; 5' end of sequence c1: GGC; or the RNA extension segments corresponding to the DNA extension segments in (5).

Incorporating extension segments of the foregoing lengths helps to further enhance the stability of different nucleic acid carriers when carrying effector molecules of various molecular weights, and also facilitates selection of a suitable nucleic acid carrier according to the particular oligonucleotide effector molecule to be loaded.

It should be noted that the carrier backbone sequence in the present application includes the aforesaid sequences a1, b1, and c1 (or variant sequences thereof) and the optional first and second extension segments described above. On the basis of such a carrier backbone sequence, in certain embodiments, to further improve the stability of small oligonucleotide effector molecules of different sequence lengths after loading, the nucleic acid carrier may additionally include a single-stranded bridging sequence and/or a linker sequence, wherein the single-stranded bridging sequence is selected from any one or more of: at the 3' end of the core sequence of the sequence a: SEQ ID No. 7: TGTAGCACGGTGGC or its complementary sequence SEQ ID No. 8: GCCACCGTGCTACA; at the 3' end of the core sequence of the sequence b: SEQ ID No. 9: TCGGCGCGGCCGTG or its complementary sequence SEQ ID No. 10: CACGGCCGCGCCGA; and at the 3' end of the core sequence of the sequence c: SEQ ID No. 11: TGCTGCTGCTGCTG or its compleentary sequence SEQ ID No. 12: CAGCAGCAGCAGCA.The linker sequence is a sequence composed of n consecutive U, T, or A nucleotides, where n is an integer from 2 to 8, preferably 3 to 6; or a sequence formed by a random combination of A, U, T, C, and/or G. The linker sequence is located at the 5' end or the 3' end of the core sequence.

The single-stranded bridging sequences and their complementary sequences are designed to connect the oligonucleotide effector molecule to the nucleic acid carrier sequence through complementary base pairing. Accordingly, appropriate numbers and specific sequences of single-stranded bridging sequences are selected in view of the particular carrier core sequence and the sequence of the oligonucleotide effector molecule.

In certain embodiments, to facilitate synthesis of the nucleic acid carrier and/or the oligonucleotide drug and to reduce nucleic acid synthesis costs, the nucleic acid carrier sequence is configured in the structural form of "carrier backbone sequence + single-stranded bridging sequence." In this way, multiple types of oligonucleotide effector molecules (excluding siRNA) can, at the time of synthesis, be provided with a terminal sequence complementary to the single-stranded bridging sequence and, in subsequent self-assembly, simultaneously achieve formation of the carrier and loading of the oligonucleotide drug. This also avoids the issues of long fragments, high synthesis difficulty, and elevated cost that arise when directly synthesizing a continuous nucleic acid sequence combining the carrier nucleic acid sequence and the oligonucleotide drug sequence.

In certain preferred embodiments, the sequences a, b, and c of the nucleic acid carrier are as follows:

sequence a: SEQ ID No. 15: GACGCCCACGAGCGTTCCGGGAGAGG<u>TGTAGCACGGTGGC</u>, wherein the underlined portion represents the single-stranded bridging sequence A;

sequence b: SEQ ID No. 16: CCTCTCCCGGTTCGCCGCGAGCC<u>TCGGCGCGGCCGTG,</u> wherein the underlined portion represents the single-stranded bridging sequence B;

sequence c: SEQ ID No. 17: GGCTCGCGGCCATAGCCGTGGGCGT<u>CTGCTGCTGCTGCTG,</u> wherein the underlined portion represents the single-stranded bridging sequence C.

It should be noted that the aforesaid single-stranded bridging sequences are not necessarily present simultaneously on the sequences a, b, and c of the nucleic acid carrier, and may be selected as needed. For example, they may be present only on the sequence a, only on the sequence b, only on the sequence c, or only on the sequences a and b, only on the sequences a and c, or only on the sequences b and c.

In other embodiments, the sequences a, b, and c of the nucleic acid carrier are as follows:

sequence a: SEQ ID No. 18: GCGACGCCCACGAGCGTTCCGGGAGAGGAGC;

sequence b: SEQ ID No. 19: GCTCCTCTCCCGGTTCGCCGCGAGCCGCG;

sequence c: SEQ ID No. 20: <u>CAGCAGCAGCAGC</u>ACGCGGCTCGCGGCCATAGCCGTGGGCGTCGC,wherein the underlined portion represents the single-stranded bridging sequence.

It should be noted that, depending on whether the small nucleic acid drug to be loaded on the nucleic acid carrier is an siRNA, an miRNA, or a nucleic acid aptamer, the specific mode of loading onto the nucleic acid carrier differs; for example, when the cargo is a nucleic acid aptamer, it may be connected via a linker sequence or a single-stranded bridging sequence to any end of any one or more of the sequences a, b, or c, or an miRNA may serve as a linker sequence to connect the aptamer sequence to any end of any one of the sequences a, b, or c (for example, when the miRNA sequence is disposed at the 5' end of the sequence a, the aptamer sequence may be disposed at the 5' end of the miRNA sequence; similarly, when the miRNA sequence is disposed at the 3' end of the sequence b, the aptamer sequence may be disposed at the 3' end of the miRNA sequence); it should be understood that where the aptamer sequence is connected via a linker sequence or an miRNA sequence, the connection is covalent, whereas when the connection is made via a single-stranded bridging sequence, the linkage is realized by complementary pairing with a complementary sequence of the single-stranded bridge that is provided (i.e., covalently linked) on the sequences a, b, or c and is therefore effected by intermolecular interactions (hybridization) rather than chemical covalent bonding; similarly, when the cargo is an siRNA, the antisense strand of the siRNA is connected by complementary pairing to the sense strand that is provided (i.e.,

covalently linked) on the sequences a, b, or c, and the sense strand is typically connected via a linker sequence; in some cases, when the nucleic acid aptamer is in RNA form, analogous to the miRNA, it may be directly covalently linked to the sequences a, b, or c without a linker sequence, or it may be covalently linked to the sequences a, b, or c via only TT, AA, or UU dinucleotides, or a single U or A.

In some preferred embodiments, the core sequences for self-assembly to form the nucleic acid carrier are selected from any one of the following sets:

1) sequence a: SEQ ID No. 21: GCGACGCCCACGAGCGTTCCGGGAGAGGAG,

sequence b: SEQ ID No. 22: CTCCTCTCCCGGTTCGCCGCGAGCCGCG,
sequence c: SEQ ID No. 23: CGCGGCACGCGGCCATAGCCGTGGGCGTCGC;

2) sequence a: SEQ ID No. 24: GCGACGCCCACGAGCGTTCCGGGAGAGGAGCT,

sequence b: SEQ ID No. 19: GCTCCTCTCCCGGTTCGCCGCGAGCCGCG,
sequence c: SEQ ID No. 23: CGCGGCACGCGGCCATAGCCGTGGGCGTCGC;

3) sequence a: SEQ ID No. 24: GCGACGCCCACGAGCGTTCCGGGAGAGGAGCT,

sequence b: SEQ ID No. 25: GCTCCTCTCCCGGTTCGCCGCGAGCCGCGT,
sequence c: SEQ ID No. 26: CGCGGCTCGCGGCCATAGCCGTGGGCGTCGCT;

4) sequence a: SEQ ID No. 24: GCGACGCCCACGAGCGTTCCGGGAGAGGAGCT,

sequence b: SEQ ID No. 25: GCTCCTCTCCCGGTTCGCCGCGAGCCGCGT,
sequence c: SEQ ID No. 27: CGCGGCACGCGGCCATAGCCGTGGGCGTCGCT;

5) sequence a: SEQ ID No. 28: GACGCCCACGAGCGTTCCGGGAGAGG,

sequence b: SEQ ID No. 29: CCTCTCCCGGTTCGCCGCGAGCC,
sequence c: SEQ ID No. 30: GGCTCGCGGCCATAGCCGTGGGCGTC;

6) sequence a: SEQ ID No. 18: GCGACGCCCACGAGCGTTCCGGGAGAGGAGC,

sequence b: SEQ ID No. 19: GCTCCTCTCCCGGTTCGCCGCGAGCCGCG,
sequence c: SEQ ID No. 31: CGCGGCTCGCGGCCATAGCCGTGGGCGTCGC;

7) sequence a: SEQ ID No. 24: GCGACGCCCACGAGCGTTCCGGGAGAGGAGCT,

sequence b: SEQ ID No. 19: GCTCCTCTCCCGGTTCGCCGCGAGCCGCG,
sequence c: SEQ ID No. 26: CGCGGCTCGCGGCCATAGCCGTGGGCGTCGCT;

8) sequence a: SEQ ID No. 24: GCGACGCCCACGAGCGTTCCGGGAGAGGAGCT,

sequence b: SEQ ID No. 19: GCTCCTCTCCCGGTTCGCCGCGAGCCGCG,
sequence c: SEQ ID No. 31: CGCGGCTCGCGGCCATAGCCGTGGGCGTCGC;

9) sequence a: SEQ ID No. 24: GCGACGCCCACGAGCGTTCCGGGAGAGGAGCT,

sequence b: SEQ ID No. 25: GCTCCTCTCCCGGTTCGCCGCGAGCCGCGT,
sequence c: SEQ ID No. 26: CGCGGCTCGCGGCCATAGCCGTGGGCGTCGCT;

10) sequence a: SEQ ID No. 24: GCGACGCCCACGAGCGTTCCGGGAGAGGAGCT,

sequence b: SEQ ID No. 25: GCTCCTCTCCCGGTTCGCCGCGAGCCGCGT,
sequence c: SEQ ID No. 31: CGCGGCTCGCGGCCATAGCCGTGGGCGTCGC;

11) sequence a: SEQ ID No. 18: GCGACGCCCACGAGCGTTCCGGGAGAGGAGC,

sequence b: SEQ ID No. 25: GCTCCTCTCCCGGTTCGCCGCGAGCCGCGT,
sequence c: SEQ ID No. 26: CGCGGCTCGCGGCCATAGCCGTGGGCGTCGCT;

12) sequence a: SEQ ID No. 32: CGACGCCCACGAGCGTTCCGGGAGAGGAGC;

sequence b: SEQ ID No. 19: GCTCCTCTCCCGGTTCGCCGCGAGCCGCG;
sequence c: SEQ ID No. 31: CGCGGCTCGCGGCCATAGCCGTGGGCGTCGC;

13) sequence a: SEQ ID No. 33: GCGGCGCCCACGAGCGTTCCGGGAGC;

sequence b: SEQ ID No. 34: GCTCCCGGTTCGCCGCCAGCCGCC;
sequence c: SEQ ID No. 35: GGCGGCAGGCGGCCATAGCCGTGGGCGCCGC;

14) sequence a: SEQ ID No. 36: GCGGCGCCCACGAGCGTTCCGGGAGAGGCC;

sequence b: SEQ ID No. 37: GGCCTCTCCCGGTTCGCCGCCAGCCGCC;
sequence c: SEQ ID No. 38: GGCGGCTGGCGGCCATAGCCGTGGGCGCCGC;

15) sequence a: SEQ ID No. 39: GCGGCGCCCACGAGCGTTCCGGGAGAGGCCT;

sequence b: SEQ ID No. 40: GGCCTCTCCCGGTTCGCCGCCAGCCGCCT;
sequence c: SEQ ID No. 41: GGCGGCTGGCGGCCATAGCCGTGGGCGCCGCT;

16) sequence a: SEQ ID No. 42: GCGGCGCCCACGAGCGUUCCGGGAGAGGCC;

sequence b: SEQ ID No. 43: GGCCUCUCCCGGUUCGCCGCCAGCCGCC;
sequence c: SEQ ID No. 44: GGCGGCUGGCGGCCAUAGCCGUGGGCGCCGC;

17) sequence a: SEQ ID No. 45: CGACGCCCACGAGCGTTCCGGGAGAGGAG;

sequence b: SEQ ID No. 46: CTCCTCTCCCGGTTCGCCGCCAGCCGCC;
sequence c: SEQ ID No. 35: GGCGGCAGGCGGCCATAGCCGTGGGCGCCGC;

18) sequence a: SEQ ID No. 36: GCGGCGCCCACGAGCGTTCCGGGAGAGGCC;

sequence b: SEQ ID No. 43: GGCCUCUCCCGGUUCGCCGCCAGCCGCC;
sequence c: SEQ ID No. 44: GGCGGCUGGCGGCCAUAGCCGUGGGCGCCGC;

19) sequence a: SEQ ID No. 36: GCGGCGCCCACGAGCGTTCCGGGAGAGGCC;

sequence b: SEQ ID No. 40: GGCCTCTCCCGGTTCGCCGCCAGCCGCCT;
sequence c: SEQ ID No. 38: GGCGGCTGGCGGCCATAGCCGTGGGCGCCGC;

20) sequence a: SEQ ID No. 36: GCGGCGCCCACGAGCGTTCCGGGAGAGGCC;

sequence b: SEQ ID No. 40: GGCCTCTCCCGGTTCGCCGCCAGCCGCCT;
sequence c: SEQ ID No. 41: GGCGGCTGGCGGCCATAGCCGTGGGCGCCGCT;

21) sequence a: SEQ ID No. 36: GCGGCGCCCACGAGCGTTCCGGGAGAGGCC;

sequence b: SEQ ID No. 37: GGCCTCTCCCGGTTCGCCGCCAGCCGCC;
sequence c: SEQ ID No. 44: GGCGGCUGGCGGCCAUAGCCGUGGGCGCCGC;

22) sequence a: SEQ ID No. 33: GCGGCGCCCACGAGCGTTCCGGGAGC;

sequence b: SEQ ID No. 46: CTCCTCTCCCGGTTCGCCGCCAGCCGCC;
sequence c: SEQ ID No. 35: GGCGGCAGGCGGCCATAGCCGTGGGCGCCGC;

23) sequence a: SEQ ID No. 47: GCGGCGCCCACGAGCGTTCCGGGAGAGGAG;

sequence b: SEQ ID No. 46: CTCCTCTCCCGGTTCGCCGCCAGCCGCC;
sequence c: SEQ ID No. 35: GGCGGCAGGCGGCCATAGCCGTGGGCGCCGC;

24) sequence a: SEQ ID No. 48: CGCCCACGAGCGTTCCGGGAGA;

sequence b: SEQ ID No. 49: TCTCCCGGTTCGCCGCGAG;
sequence c: SEQ ID No. 50: CTCGCGGCCATAGCCGTGGGCG;

25) sequence a: SEQ ID No. 51: UCGCCCACGAGCGUUCCGGGAGA;

sequence b: SEQ ID No. 52: UCUCCCGGUUCGCCGCGAG;
sequence c: SEQ ID No. 53: UCUCGCGGCCAUAGCCGUGGGCG;

26) sequence a: SEQ ID No. 54: GCGCCCACGAGCGTTCCGGGAGAGC;

sequence b: SEQ ID No. 55: CCCUGCTCTCCCGGTTCGCCGCCAGCCGCC;
sequence c: SEQ ID No. 56: GGCGGCAGGCGGCCATAGCCGTGGGCGC.

The nucleic acid carriers formed by self-assembly of core sequences of differing composition can accommodate loading of different types of oligonucleotide drugs, and different types of oligonucleotide drugs may also be loaded on the same nucleic acid carrier, thereby expanding the range of loadable oligonucleotide modalities and improving loading efficiency.

It should be noted that, in order to further reduce the sensitivity of the above nucleic acid nanoparticles to degradation by ribonucleases (RNases) and to enhance stability during delivery, certain sites on the nucleic acid carrier may be modified in some cases. In some preferred embodiments, at least one modifiable site in at least one of the bases, the ribose, and the phosphodiester of the sequences a, b, and c carries at least one modification selected from: -F, methyl, amino, disulfide, carbonyl, carboxyl, thiol (mercapto), aldehyde, and thio (sulfur substitution); preferably, a portion of the bases in the sequences a, b, and c are thio modified. When the modification site is a thiol group, it is considered a thio modification; such modification has a relatively modest effect, is low in cost, and can improve the stability of RNA sequences and of RNA carriers or DNA-RNA hybrid carriers assembled therefrom. To further increase stability, in nucleic acid carriers that include RNA, the RNA preferably contains 2'-F and 2'-OMe modifications, more preferably 2'-F at U and C and 2'-OMe at A and G.

In a second exemplary embodiment of the present application, there is provided a nucleic acid drug comprising the above nucleic acid carrier and a bioactive substance loaded on the nucleic acid carrier, the bioactive substance comprising oligonucleotide effector molecule(s) and a targeting molecule for targeted delivery of the oligonucleotide effector molecule(s), wherein the oligonucleotide effector molecule is selected from at least one of: a nucleic acid immunostimulant, a nucleic acid aptamer, siRNA, miRNA, and an antisense oligonucleotide (ASO); and where the oligonucleotide effector molecule includes a nucleic acid aptamer, the targeting molecule includes at least a nucleic acid aptamer; where the oligonucleotide effector molecule does not include a nucleic acid aptamer, the targeting molecule includes at least a small-molecule compound having targeting activity.

The foregoing nucleic acid drug addresses (1) the challenge of co-formulating multiple oligonucleotide drugs into a single therapeutic and (2) the challenge of systematic preparation of nano-nucleic-acid drugs having complex architectures. Building on the improved nucleic acid nanocarrier structure described above, and by employing different loading strategies to load one or more oligonucleotide effector molecules, the present disclosure not only provides an integrated, all-in-one formulation of multiple oligonucleotide therapeutics but also furnishes a novel concept and implementation pathway for constructing such oligonucleotide therapeutics.

In the present application, the term "nucleic acid immunostimulant" (immunostimulatory factor) is used as follows: tumor cells often exhibit low expression of major histocompatibility complex (MHC) molecules, lack co-stimulatory molecules, present defects in antigen processing, and display altered expression of T-cell signaling molecules, resulting in diminished antitumor immunity, which is a principal cause of tumor initiation and progression.

CpG ODN (CpG oligodeoxynucleotide) refers to a synthetic oligodeoxynucleotide containing unmethylated cytosine-phosphate-guanine (CpG) dinucleotide motifs, which can mimic bacterial DNA and stimulate immune cells of various mammals, including humans.

Different types of CpG ODNs have distinct structural characteristics and immunologic effects and are generally categorized as A-class, B-class, and C-class.

A-class CpG ODNs are characterized by a palindromic sequence containing CpG dinucleotides at the core, flanked by poly(G) tails, and a phosphodiester backbone that is partially phosphorothioate-modified. Through the palindrome and the poly(G) tails they form higher-order structures, activate plasmacytoid dendritic cells (pDCs) to induce large amounts of type I interferon, and exhibit weak activity on B cells.

B-class CpG ODNs are fully phosphorothioate-modified linear CpG ODNs that possess strong immunostimulatory activity on B cells but do not activate plasmacytoid dendritic cells.

C-class CpG ODNs are fully phosphorothioate-modified CpG ODNs capable of forming dimers via palindromic sequences; they combine the activities of A- and B-class CpG ODNs, activating both plasmacytoid dendritic cells and B cells.

As used in the present application, the term "nucleic acid immunostimulant" (immunostimulatory factor) refers to DNA or synthetic oligonucleotides containing CpG motifs that can activate multiple immune-cell types, including dendritic cells, natural killer cells, B cells, and T cells; induce secretion of Th1-type cytokines predominantly interleukin-12; up-regulate the expression of co-stimulatory molecules; and elicit a Th1-type immune response, thereby having important utility in antitumor applications. Accordingly, existing CpG immunostimulants are applicable to the present application. In a preferred embodiment, the nucleic acid immunostimulant includes one or more selected from CpG2006, a CpG2006 variant, CpG1826, CpG2216, CpG2395, and CpG-ODNT7; more preferably, the sequence of CpG2006 is SEQ ID No. 57: TCGTCGTTTTGTCGTTTTGTCGTT or SEQ ID No. 58: UCGUCGUUUUGUCGUUUUGUCGUU; preferably, the sequence of the CpG2006 variant is GTCGTT; preferably, the sequence of CpG1826 is SEQ ID No. 59: TCCATGACGTTCCTGACG; preferably, the sequence of CpG2216 is SEQ ID No. 60: GGGGGACGATCGTCGGGGGG; preferably, the sequence of CpG2395 is SEQ ID No. 61: TCGTCGTTTTCGGCGCGCGCCG; preferably, the sequence(s) of CpG-ODNT7 is/are SEQ ID No. 62: TCGTCGTCGTCGTCGTCGTCG or SEQ ID No. 63: TCGTCGTCGTCGTCGTCGTCGTCG ; and, preferably, the nucleic acid immunostimulant further includes CpG-BYZD: AGCGAA.

The siRNAs herein may be any known siRNA therapeutics. In a preferred embodiment of the present application, they include, without limitation, siRNAs targeting any one or more of the following molecules: ASAP1, ATAD2, CD24, CD47, EGFR, HBV, HSP, HS70, PD-L1, PAPP-1, survivin, TAP, TIM-3, TGF-β1, and VEGF-C. Existing siRNAs for the foregoing targets are applicable to the present application.

More preferably, the ASAP1 siRNA is selected from any one of the following: i) sense strand: SEQ ID No. 64: AUUUGCUUCCAUAAUAUCAUU; antisense strand: SEQ ID No. 65: UGAUAUUAUGGAAGCAAAUUU; ii) sense strand: SEQ ID No. 66: UUAGGUUUGGGGUUGGAUCUU; antisense strand: SEQ ID No. 67: GAUCCAACCCCAAACCUAAUU.

More preferably, the ATAD2 siRNA includes an antisense strand SEQ ID No. 68: GCGUCGAAGUUGUAGGAUUUU and a sense strand SEQ ID No. 69: AAUCCUACAACUUCGACGCUU.

More preferably, the CD24 siRNA includes a sense strand SEQ ID No. 70: UGUUUACAUUGUUGAGGUAUU and an antisense strand SEQ ID No. 71: UACCUCAACAAUGUAAACUUU.

More preferably, the CD47 siRNA is selected from any one of the following: i) sense strand:SEQ ID No. 72: GGUGAUUACCCAGAGAUAUTT; antisense strand: SEQ ID No. 73: AUAUCUCUGGGUAAUCACCTT; ii) sense strand: SEQ ID No. 74: UGGUGAAAGAGGUCAUUCCUU; antisense strand: SEQ ID No. 75: GGAAUGACCUCUUUCACCAUU; iii) sense strand: SEQ ID No. 76: GGAAUGACCUCUUUCACCATT; antisense strand: SEQ ID No. 77: UGGUGAAAGAGGUCAUUCCTT; iv) sense strand: SEQ ID No. 78: GGUGAUUACCCAGAGAUAUUU; antisense strand: SEQ ID No. 79: AUAUCUCUGGGUAAUCACCUU;

preferably, the EGFR siRNA is selected from any one of the following: i) sense strand: SEQ ID No. 80: GGCUGGUUAUGUCCUCAUUUU; antisense strand: SEQ ID No. 81: AAUGAGGACAUAACCAGCCUU; ii) sense strand: SEQ ID No. 82: CCUUAGCAGUCUUAUCUAAUU; antisense strand: SEQ ID No. 83: UUAGAUAAGACUGCUAAGGUU; iii) sense strand: SEQ ID No. 84: UGCCUUAGCAGUCUUAUCUAAUU; antisense strand: SEQ ID No. 85: UUAGAUAAGACUGCUAAGGCAUU; iv) sense strand: SEQ ID No. 86: UGCCUUAGCAGUCUUAUCUAAUUUU; antisense strand: SEQ ID No. 87: AAUUAGAUAAGACUGCUAAGGCAUU;

preferably, the HBV siRNA includes a sense strand SEQ ID No. 88: GGACUUCUCUCAAUUUUCUUU, and an antisense strand SEQ ID No. 89: AGAAAAUUGAGAGAAGUCCUU;

preferably, the HSP siRNA includes a sense strand:SEQ ID No. 90: CGCAGAACACCGUGUUCGAUU, and an antisense strand SEQ ID No. 91: UCGAACACGGUGUUCUGCGUU;

preferably, the HS70 siRNA includes a sense strand SEQ ID No. 92: GGCCAACAAGAUCACCAUCUU, and an antisense strand SEQ ID No. 93: GAUGGUGAUCUUGUUGGCCUU;

preferably, the PD-L1 siRNA is selected from any one of the following: i) sense strand: SEQ ID No. 94: CCAGCACACUGAGAAUCAAUU; antisense strand: SEQ ID No. 95: UUGAUUCUCAGUGUGCUGGUU; ii) sense strand: SEQ ID No. 96: AGACGUAAGCAGUGUUGAATT; antisense strand: SEQ ID No. 97: UUCAACACUGCUUACGUCUTT;

preferably, the PARP-1 siRNA includes a sense strand SEQ ID No. 98: GAGGAAGGUAUCAACAAAUTT and an antisense strand SEQ ID No. 99: AUUUGUUGAUACCUUCCUCTT;

preferably, the survivin siRNA is selected from any one of the following: i) sense strand: SEQ ID No. 100: GCAGGUUCCUUAUCUGUCACAUU; antisense strand: SEQ ID No. 101: UGUGACAGAUAAGGAACCUGCUU; ii) sense strand: SEQ ID No. 102: GGAAUUGGAAGGCUGGGAACCUU; antisense strand: SEQ ID No. 103: GGUUCCCAGCCUUCCAAUUCCUU; iii) sense strand: SEQ ID No. 104: UGCAGGUUCCUUAUCUGUCATT; antisense strand: SEQ ID No. 105: UGACAGAUAAGGAACCUGCTT; iv) sense strand: SEQ ID No. 106: CUGCAGGUUCCUUAUCUGUCACAUU; antisense strand: SEQ ID No. 107: UGUGACAGAUAAGGAACCUGCAGUU;

preferably, the TAP siRNA is selected from any one of the following: i) sense strand: SEQ ID No. 108: GCUGCACACGGUUCAGAAUUU; antisense strand: SEQ ID No. 109: AUUCUGAACCGUGUGCAGCUU; ii) sense strand: SEQ ID No. 110: CAGGAUGAGUUACUUGAAAUU; antisense strand: SEQ ID No. 111: UUUCAAGUAACUCAUCCUGUU;

preferably, the TIM-3 siRNA includes a sense strand SEQ ID No. 112: GUGCUCAGGACUGAUGAAATT and an antisense strand SEQ ID No. 113: UUUCAUCAGUCCUGAGCACTT;

preferably, the TGF-β1 siRNA is selected from any one of the following: i) sense strand: SEQ ID No. 114: GUCAACUGUGGAGCAACACUU; antisense strand: SEQ ID No. 115: GUGUUGCUCCACAGUUGACUU; ii) sense strand: SEQ ID No. 116: GCAACAACGCCAUCUAUGATT; antisense strand: SEQ ID No. 117: UCAUAGAUGGCGUUGUUGCTT;

preferably, the VEGF-C siRNA is selected from any one of the following: i) sense strand: SEQ ID No. 118: GCAAGACGUUGUUUGAAAUUAUU; antisense strand: SEQ ID No. 119: UAAUUUCAAACAACGUCUUGCUU; ii) sense strand: SEQ ID No. 120: CAGCAAGACGUUGUUUGAAAUUAUU; antisense strand: SEQ ID No. 121: UAAUUUCAAACAACGUCUUGCUGUU; iii) sense strand: SEQ ID No. 122: CAGGAUGGUAAAGACUACAUU; antisense strand: SEQ ID No. 123: UGUAGUCUUUACCAUCCUGUU.

The sequences of the foregoing siRNAs are shown in the table below. It should be noted that, unless otherwise specified, all sequences presented herein are shown in the 5'→3' direction. In addition, the meanings of modification symbols in this application are as follows: "*" denotes a phosphorothioate modification of the phosphate backbone (also referred to as a thio modification); "+" denotes a locked nucleic acid (LNA) modification; and "m" denotes a 2'-O-methoxyethyl (2'-O-MOE) modification.

Table 1:

| siRNA name | Sense strand (5'→3') | Antisense strand (5'→3') |
|---|---|---|
| ASAP1 siRNA | SEQ ID No. 64: AUUUGCUUCCAUAAUAUCAUU | SEQ ID No. 65: UGAUAUUAUGGAAGCAAAUUU |
| ASAP1 siRNA | SEQ ID No. 66: UUAGGUUUGGGGUUGGAUCUU | SEQ ID No. 67: GAUCCAACCCCAAACCUAAUU |
| ATAD2 siRNA-1 | SEQ ID No. 69: AAUCCUACAACUUCGACGCUU | SEQ ID No. 68: GCGUCGAAGUUGUAGGAUUUU |
| CD24 siRNA (human) | SEQ ID No. 70: UGUUUACAUUGUUGAGGUAUU | SEQ ID No. 71: UACCUCAACAAUGUAAACUUU |
| CD47 siRNA (human) | SEO ID No. 72: GGUGAUUACCCAGAGAUAUTT(TT denotes dTdT) | SEQ ID No. 73: AUAUCUCUGGGUAAUCACCTT (TT denotes dTdT) |
| CD47 siRNA (human) | SEQ ID No. 78: GGUGAUUACCCAGAGAUAUUU | SEQ ID No. 79: AUAUCUCUGGGUAAUCACCUU |
| CD47 siRNA (mouse) | SEQ ID No. 74: UGGUGAAAGAGGUCAUUCCUU | SEQ ID No. 75: GGAAUGACCUCUUUCAC-CAUU |

(continued)

| siRNA name | Sense strand (5'→3') | Antisense strand (5'→3') |
|---|---|---|
| CD47 siRNA (mouse) | SEQ ID No. 76: GGAAUGACCUCUUUCACCATT | SEQ ID No. 77: UGGUGAAAGAGGUCAUUCCTT |
| EGFR siRNA-1 | SEQ ID No. 80: GGCUGGUUAUGUCCUCAUUUU | SEQ ID No. 81: AAUGAGGACAUAACCAGCCUU |
| EGFR siRNA-2 | SEQ ID No. 82: CCUUAGCAGUCUUAUCUAAUU | SEQ ID No. 83: UUAGAUAAGACUGCUAAGGUU |
| EGFR siRNA-3 | SEQ ID No. 84: UGCCUUAGCAGUCUUAUCUAAUU | SEQ ID No. 85: UUAGAUAAGACUGCUAAGGCAUU |
| EGFR siRNA-4 | SEQ ID No. 86: UGCCUUAGCAGUCUUAUCUAAUUU U | SEO ID No. 87: AAUUAGAUAAGACUGCUAAGGCAU U |
| HBV siRNA | SEQ ID No. 88: GGACUUCUCUCAAUUUUCUUU, C/U: 2'-F | SEO ID No. 89: AGAAAAUUGAGAGAAGUCCUU , C/U: 2'-F |
| HSP siRNA (mouse) | SEQ ID No. 90: CGCAGAACACCGUGUUCGAUU | SEQ ID No. 91: UCGAACACGGUGUUCUGCGUU |
| HS70 siRNA (human) | SEQ ID No. 92: GGCCAACAAGAUCACCAUCUU | SEQ ID No. 93: GAUGGUGAUCUUGUUGGCCUU |
| PD- L1 siRNA (human) | SEQ ID No. 94: CCAGCACACUGAGAAUCAAUU | SEQ ID No. 95: UUGAUUCUCAGUGUGCUGGUU |
| PD- L1 siRNA (mouse) | SEQ ID No. 96: AGACGUAAGCAGUGUUGAATT (TT denotes dTsdT) | SEQ ID No. 97: UUCAACACUGCUUACGUCUTT (TT denotes dTsdT) |
| PARP-1 siRNA | SEQ ID No. 98: GAGGAAGGUAUCAACAAAUTT | SEQ ID No. 99: AUUUGUUGAUACCUUCCUCTT |
| Survivin | SEQ ID No. 100: GCAGGUUCCUUAUCUGUCACAUU | SEQ ID No. 101: UGUGACAGAUAAGGAACCUGCUU |
| Survivin | SEQ ID No. 104: UGCAGGUUCCUUAUCUGUCATT | SEQ ID No. 105: UGACAGAUAAGGAACCUGCTT |
| Survivin | SEQ ID No. 106: CUGCAGGUUCCUUAUCUGUCACAU U J | SEO ID No. 107: UGUGACAGAUAAGGAACCUGCAGU U |
| Survivin (mouse) | SEQ ID No. 102: GGAAUUGGAAGGCUGGGAACCUU | SEQ ID No. 103: GGUUCCCAGCCUUCCAAUUCCUU |
| TAPsiRNA (mouse) | SEQ ID No. 108: GCUGCACACGGUUCAGAAUUU | SEQ ID No. 109: AUUCUGAACCGUGUGCAGCUU |
| TAPsiRNA (human) | SEQ ID No. 110: CAGGAUGAGUUACUUGAAAUU | SEQ ID No. 111: UUUCAAGUAACUCAUCCUGUU |
| TIM-3 siRNA(451) | SEQ ID No. 112: GUGCUCAGGACUGAUGAAATT | SEQ ID No. 113: UUUCAUCAGUCCUGAGCACTT |
| TGF-β1(mouse) | SEQ ID No. 114: GUCAACUGUGGAGCAACACUU | SEQ ID No. 115: GUGUUGCUCCACAGUUGACUU |
| TGF-β1(human) | SEQ ID No. 116: GCAACAACGCCAUCUAUGATT | SEQ ID No. 117: UCAUAGAUGGCGUU-GUUGCTT |

(continued)

| siRNA name | Sense strand (5'→3') | Antisense strand (5'→3') |
|---|---|---|
| VEGF-C siR-NA(mouse) | SEQ ID No. 118: GCAAGACGUUGUUUGAAAUUAUU | SEQ ID No. 119: UAAUUUCAAACAACGUCUUGCUU |
| VEGF-C siRNA (mouse) | SEQ ID No. 120: CAGCAAGACGUUGUUUGAAAUUAUUU | SEO ID No. 121: UAAUUUCAAACAACGUCUUGCUGUU |
| VEGF-C siRNA (human) | SEQ ID No. 122: CAGGAUGGUAAAGACUACAUU | SEQ ID No. 123: UGUAGUCUUUACCAUCCUGUU |
| (Note: In the table above, when "TT" appears at the end of sequence a, "TT" denotes a dTdT modification; it is abbreviated as "TT" for convenience in preparing the sequence list). | | |

More preferably, the ASO includes one or more of A-miR21, A-miR-10a, A-miR-30c, and AmiR1306, and the miRNA includes one or more of miR-34, miR-542, miR-126, and miR-122.

More preferably, A-miR21 is selected from any of the following: i) GATAAGCT, entirely phosphorothioate-modified or entirely locked-nucleic-acid (LNA)-modified; ii) SEQ ID No. 124: GTCAACATCAGTCTGATAAGCTA; iii) SEQ ID No. 125: TCAACATCAGTCTGATAAGCTA; iv) variant of (i) or (ii) in which T is replaced by U(i.e., GAUAAGCU, SEQ ID No. 344: GUCAACAUCAGUCUGAUAAGCUA,or SEQ ID No. 345: UCAACAUCAGUCUGAUAAGCUA).

More preferably, A-miR-10a is ACAGGGTA, all LNA-modified.

More preferably, A-miR-30c is SEQ ID No. 126: GCTGAGAGTGTAGGATGTTTACA.

More preferably, the sequence of miR-34 is TGTGACAG.

More preferably, the sequence of miR-542 is TGGCAGTGT.

More preferably, the sequence of miR-126 is UCGUACC, or UCGUACCG, each nucleotide ribose is 2'-O-methoxyethyl (2'-O-MOE) modified; alternatively, the sequence is CGTACCG or GTCGTT.

More preferably, the sequence of miR-122 is GGAAGTGT.

More preferably, the sequence of AmiR1306: SEQ ID No. 127: CATCACCACCAGAGCCAACGTC.

The specific sequences and modifications of the foregoing miRNAs are set forth in the table below.

Table 2:

| Name | DNA-form sequence (5'→3') | RNA-form sequence (5'→3') |
|---|---|---|
| AmiR21 | GATAAGCT (all LNA-modified or PS-modified) | GAUAAGCU (all LNA-modified or PS-modified) |
| AmiR21 | SEQ ID No. 124: GTCAACATCAGTCTGATAAGCTA | SEQ ID No. 344: GUCAACAUCAGUCUGAUAAGCUA |
| AmiR21 | SEQ ID No. 125: TCAACATCAGTCTGATAAGCTA | |
| AmiR-10a | ACAGGGTA*(all LNA-modified)* | |
| AmiR-30c | SEQ ID No. 126: GCTGAGAGTGTAGGATGTTTACA | |
| miR-34 | TGTGACAG | |
| miR-122 | GGAAGTGT | |
| miR-126 | CGTACCG or GTCGTT | UCGUACCG or UCGUACCG(all 2'-O-MOE-modified) |
| miR-542 | TGGCAGTGT | |
| AmiR1306 | SEQ ID No. 127: CATCACCACCAGAGCCAACGTC | |

In the present application, the nucleic acid aptamer includes a DNA-form aptamer or an RNA-form aptamer. The length of any aptamer listed herein is not particularly limited and may be set at 8-150 nt, for example 10-100 nt, 15-95 nt, 18-90 nt, 20-88 nt, 25-85 nt, 25-80 nt, 25-75 nt, 25-70 nt, 25-65 nt, 25-60 nt, 25-55 nt, 25-50 nt, or 25-45 nt, 25-40 nt, etc.

In some embodiments, the DNA-form aptamer includes an aptamer having the sequence shown in the table below, or sequence a having at least 85% identity (e.g., at least 90%, at least 95%, or at least 99%) to the sequence shown.

As used herein, a "nucleic acid aptamer" means a nucleic acid molecule (DNA or RNA) that has binding activity to a specific target molecule (e.g., CD40 or PD-L1). An aptamer can bind to the target molecule and inhibit its activity by, for example, blocking the target's binding to its cognate ligand, causing a conformational change of the target, and/or blocking the active site of the target. The nucleic acid aptamers of the present application may be linear or circular, may be RNA or DNA (e.g., single-stranded DNA), and may be modified nucleic acids or mixtures thereof.

SEQ ID No. 134:

GGCAGGAAGACAAACAAGCTTGGCGGCGGGAAGGTGTTTAAATTCCCGGGTCTGCGTGGTCTGT GGTGCTGT

Table 3:

| Nucleic acid aptamer | DNA-form sequence (5'→3') |
|---|---|
| A1 aptamer | SEQ ID No. 128:<br>GGTTGCATGCCGTGGGGAGGGGGGTGGGTTTTATAGCGTACTCAG |
| A15 aptamer | SEQ ID No. 129: CCCTCCTACATAGGG |
| AS1411 aptamer (human, mouse) | SEQ ID No. 131: GGTGGTGGTGGTTGTGGTGGTGGTGG |
| AFP aptamer | SEQ ID No. 134:<br>GGCAGGAAGACAAACAAGCTTGGCGGCGGGAAGGTGTTTAAATTCCCG GGTCTGCGTGGTCTGTGGTGCTGT |
| ATP aptamer 1 | SEQ ID No. 135: ACCTGGGGAGTATTGGGGAGGAAGG |
| ATP aptamer 2 | SEQ ID No. 136: GGGAGGACGATGCGGAGGAAGGGTAGG |
| Act-12c aptamer | SEQ ID No. 137:<br>CGGGGAAAGTCACGGGGGGTTTCAGATGTTCTGATCGGTGTGGAG |
| A18 aptamer | SEQ ID No. 138: CCAGATAGTCCCTGG |
| BAF aptamer 7-1 | SEQ ID No. 139: GATAACGGGCACGAATTCGGAGTG |
| C-Met-SL1 aptamer | SEQ ID No. 143:<br>ATCAGGCTGGATGGTAGCTCGGTCGGGGTGGGTGGGTTGGCAAGTCTG AT |
| CA2 aptamer | SEQ ID No. 145: CCCACGTCTGCGCTTAGCTCCTGGGCCTGGATGGGC |
| CRAC Orai1 targeting-site aptamer | SEQ ID No. 147:<br>CCAGTAGCCATACCGGTTTGTGGATGGGGTGTATGCGAGT |
| CEA aptamer | SEQ ID No. 148:<br>CTAGGATCCCCACTCACCATCTCTCAGCTTGCTTCCTAGC |
| CEA-18 aptamer | SEQ ID No. 150: TTAACTTATTCGACCATA |
| CEA-T84 aptamer | SEQ ID No. 151:<br>TCGCGCGAGTCGTCTGGGGAACCATCGAGTTACACCGACCTTCTATGT GCGGCCCCCGCATCGTCCTCCC |
| CSC1 aptamer | SEQ ID No. 152:<br>ACCTTGGCTGTCGTGTTGTAGGTGGTTTGCTGCGGTGGGCTCAAGAAG AAAGCGCAAAGAGGTCAGTGGTCAGAGCGT |
| CSC13 | SEQ ID No. 153:<br>ACCTTGGCTGTCGTGTTGTGGGGTGTCGTATCTTTCGTGTCTTATTATTT TCTAGGTGGAGGTCAGTGGTCAGAGCGT |

(continued)

| Nucleic acid aptamer | DNA-form sequence (5'→3') |
|---|---|
| CD40 aptamer (human/-mouse) | SEQ ID No. 154: CCAACGAGTAGGCGATAGCGCGTGG |
| CD40 aptamer | SEQ ID No. 155: AGAGACGATGCGGCCAACGAGTAGGCGATAGCGCGTGGCAGAGCGTCGCT |
| | SEQ ID No. 156: GCCAACGAGTAGGCGATAGCGCGTGGC |
| CD19 aptamer (L7) | SEQ ID No. 158: TGCGTGTGTAGTGTGTGTCGTTTCTCCTTTTTTTGGTTGCTGCTCTTAGGGATTTGGGCGG |
| CD19 aptamer (L7) | SEQ ID No. 159: TGCGTGTGTAGTGTGTCTGTTCTCCTTTTTTTGGTTGCTGCTCTTAGGGATTTGGGCGG |
| CD3-4 aptamer | SEQ ID No. 162: TCTCGGACGCGTGTGGTCGGCCGAGTGGCCCACGGTAGAAGGGTTAGAACTGCTGGTTGGTGAATCTCGCTGCCTGGCCCTAGAGTG |
| CD44 aptamer | SEQ ID No. 163: CCAAGGCCTGCAAGGGAACCAAGGACACAG |
| | SEQ ID No. 163: CCAAGGCCTGCAAGGGAACCAAGGACACAG |
| CD12 aptamer | SEQ ID No. 164: GTGGATTGTTGTGTTCTGTTGGTTTTTGTGTTGTC |
| CD20 aptamer | SEO ID No. 165: TGCGTGTGTAGTGTGTCTGTTTTTTATCTTCTTTTATCTACTCTTAGGGATTTGGGCGG |
| CD24 aptamer | SEQ ID No. 166: TATGTGGGTGGGTGGGCGGTTATGCTGAGTCAGCCTTGCT |
| CD24A-2 aptamer | SEO ID No. 167: ATCCAGAGTGACGCAGCATATGTGGGTGGGTGGGCGGTTATGCTGAGTCAGCCTTGCTTGGACACGGTGGCTTAGT |
| CD33 aptamer | SEQ ID No. 169: TACCAGTGCGATGCTCAGCACGCTTATAGGGGCTGGACAAAATTCTACCCAGCCTTT |
| CD38 aptamer | SEQ ID No. 170: TACGTGAATCTCGTACGATACTCTGTAAGCGT |
| CD105 aptamer | SEQ ID No. 172: GATCAGTTTTCCATGCCAGTTGGTATTCCGCGACAGTTTGATCTC |
| CD117 aptamer | SEQ ID No. 173: GGGGCCGGGGCAAGGGGGGGGTACCGTGGTAGGAC |
| CD63 aptamer | SEQ ID No. 174: CACCCCACCTCGCTCCCGTGACACTAATGCTA |
| CD123 aptamer | SEQ ID No. 175: TGCGTGTGTAGTGTGTCTGGGCTACATCGATGAGCTGCCTAGGGTCCCTCTTAGGGATTTGGGCGG |
| EGFR aptamer | SEQ ID No. 176: GCCTTAGTAACGTGCTTTGATGTCGATTCGACAGGAGGC |
| EpCAM aptamer | SEQ ID No. 180: CACTACAGAGGTTGCGTCTGTCCCACGTTGTCATGGGGGGTTGGCCTG |
| EpCAM aptamer | SEQ ID No. 181: GACAAACGGGGGAAGATTTGACGTCGACGAC |

(continued)

| Nucleic acid aptamer | DNA-form sequence (5'→3') |
|---|---|
| EcR aptamer (mouse) | SEQ ID No. 182: GCAGGTCCACTGCGGGGGTCTATACGTGAGGAAGAAGTGGGCAGGTC |
| FAP aptamer: | SEQ ID No. 187: TGGGGGTTGAGGCTAAGCCGA |
| FAP aptamer (human) | SEQ ID No. 188: CCGCTCGAGCTAGTCTGACAAAGAGAAACAC |
| GPC1 aptamer-#30-G-30 | SEQ ID No. 189: AACGGAGTGTGGCTAACTCGA |
| GPC3 (APS613-1) aptamer | SEQ ID No. 190: TAACGCTGACCTTAGCTGCATGGCTTTACATGTTCCA |
| GSK836 aptamer | SEQ ID No. 191: GCAGAGGTGAAGCGAAGTCG |
| HBsAg aptamer | SEQ ID No. 192: CACAGCGAACAGCGGCGGACATAATAGTGCTTACTACGAC |
| Her2 aptamer | SEQ ID No. 193: AGCCGCGAGGGGAGGGATAGGGTAGGGCGCGGCT |
| Her3 aptamer | SEO ID No. 195: GGGAGCTCAGAATAAACGCTCAAAGGGTCAAGCTGATTACACTTTGTC CACTATTGGGTCCTTCGACATGAGGCCCGGATC |
| Her3 aptamer | SEQ ID No. 196: GGGAGCTCAGAATAAACGCTCAAGGCTAACAGCACGCAACGGGGGGG AGTAATCGTGTCTGTTCGACATGAGGCCCGGATC |
| HMGA2 aptamer | SEQ ID No. 199: GGAAAAAATTTTTTAAAAAACCC (fully phosphorothioate-modified) |
| H2 aptamer | SEQ ID No. 200: GGGCCGTCGAACACGAGCATGGTGCGTGGACCTAGGATGACCTGAGT ACTGTCC |
| IFN-γ aptamer B-4: | SEQ ID No. 201: CCGCCCAAATCCCTAAGAGAAGACTGTAATGACATCAAACCAGACAC ACACACTACACACGCA |
| IL-4Ra (i.e., CD124) aptamer | SEQ ID No. 202: GGAGGACGAUGCGGAAAAAGCAACAGGGUGCUCCAUGCGCAUGGAA CCUGCGCGCAGACGACUCGCUGAGGAUCCGAGA |
| IL-4Ra (i.e., CD124) aptamer (truncated) | SEQ ID No. 203: AAAAAGCAACAGGGUGCUCCAUGCGCAUGGAACCUGCGCG |
| IL-17 aptamer (mouse) | SEQ ID No. 204: CTTGGATCACCATAGTCGCTAGTCGAGGCT |
| IL-17 aptamer: | SEQ ID No. 205: GCGGCATCCTATCACGCATTGACC |
| LZH8 aptamer | SEQ ID No. 207: ATCCAGAGTGACGCAGCATATTAGTACGGCTTAACCCCATGGTGGACA CGGTGGCTTAGT |
| MUC1 aptamer | SEQ ID No. 208: GCAGTTGATCCTTTGGATACCCTGG |
| MUC1 aptamer 5TR1 | SEQ ID No. 209: GAAGTGAAAATGACAGAACACAACA |
| MUC1 aptamer | SEQ ID No. 210: AACCGCCCAAATCTCTAAGAGTCGGACTGCAACCTATGCTATCGTTGA TGTCTGTCCAAGCAACACAGACACACTACACACACGCACA |
| MUC1 aptamer | SEQ ID No. 211: AATGACAGAACACAACATT |

(continued)

| Nucleic acid aptamer | DNA-form sequence (5'→3') |
|---|---|
| M5 aptamer | SEQ ID No. 213:<br>AGCAGCACAGAGGTCAGATGCTTGGTTCCACCGTACTGACTGTAGTAA AATCTGATCACTCCTATGCGTGCTACCGTGAA |
| M7 aptamer | SEQ ID No. 214:<br>AGCAGCACAGAGGTCAGATGTAGTCGGTCTTCTTGTTTGAAACTGCTA ATTTTGAAAAAACCTATGCGTGCTACCGTGAA |
| M1 aptamer | SEQ ID No. 215:<br>AGCAGCACAGAGGTCAGATGATATAACCTTAATAAATAAAATATAAAT TATTTAATCTTACCTATGCGTGCTACCGTGAA |
| N5 aptamer | SEQ ID No. 217:<br>GATTGAGTAGATAGTGGTTCTGTACGTAGTGAAAGAGTGG |
| N-G-Dua aptamer: | SEO ID No. 218:<br>CAAGTTGCTCGTCGCGATACGTTTGGTTGGTGTGGTTGGCAGTATCGCA GGTCCAAGTTGCTCGTCGCGATACAACGGAGTGTGGCTAACTCGA |
| NKG2D_#-20-N-15 aptamer | SEQ ID No. 219:<br>CAAGTTGCTCGTCGCGATACGTTTGGTTGGTGTGGTTGGCAGTATC |
| NSE aptamer | SEQ ID No. 220: TCACACGGACCTCTCTCTACATTAATTGCGCATTTCGTT |
| SARS-CoV-2-N15 aptamer | SEQ ID No. 221:<br>GCTGGATGTTCATGCTGGCAAAATTCCTTAGGGGCACCGTTACTTTGAC ACATCCAGC |
| SARS-CoV-2-N48 aptamer | SEQ ID No. 222:<br>GCTGGATGTCGCTTACGACAATATTCCTTAGGGGCACCGCTACATTGA CACATCCAGC |
| SARS-CoV-2-N58 aptamer | SEQ ID No. 223:<br>GCTGGATGTCACCGGATTGTCGGACATCGGATTGTCTGAGTCATATGA CACATCCAGC |
| SARS-CoV-2-N61 aptamer | SEQ ID No. 224:<br>GCTGGATGTTGACCTTTACAGATCGGATTCTGTGGGGCGTTAAACTGA CACATCCAGC |
| OX40 aptamer (mouse) | SEQ ID No. 225:<br>GGGAGGACGATGCGGCAGTCTGCATCGTAGGAATCGCCACCGTATACT TTCCCACCAGACGACTCGCTGAGGATCCGAGA |
| OX40 aptamer | SEQ ID No. 227:<br>CAGTCTGCATCGTAGGATTAGCCACCGUATCTTTCCCAC |
| OX40 aptamer (human/-mouse) | SEQ ID No. 154: CCAACGAGTAGGCGATAGCGCGTGG |
| PSMA aptamer (A10-) | SEQ ID No. 231:<br>GGGAGGACGATGCGGATCAGCCATGTTTACGTCACTCCT |
| PSMA aptamer | SEQ ID No. 232:<br>GCGTTTTCGCTTTTGCGTTTTGGGTCATCTGCTTACGATAGCAATGCT |
| PDGFRβ aptamer | SEQ ID No. 235: TGTCGTGGGGCATCGAGTAAATGCAATTCGACA |
| PDGF aptamer | SEQ ID No. 237: CAGGCTACGGCACGTAGAGCATCACCATGATCCTG |

(continued)

| Nucleic acid aptamer | DNA-form sequence (5'→3') |
|---|---|
| PDL-1 aptamer B10 | SEQ ID No. 238: AACAACGTATAACAATGCCCACGTCACCAGAGTACTATGG |
| PDL-1 aptamer 1-33s | SEQ ID No. 239: GCCCCAGTTATGCTTTCCCCCTCTGTCTCTTTG |
| PDL-1 aptamer 1-30s | SEQ ID No. 240: ATCGCCCGCAGCACCCATTTGTTTTTTTTTG |
| PD-L1 aptamer | SEQ ID No. 241: ACGGGCCACATCAACTCATTGATAGACAATGCGTCCACTGCCCGT |
| PD-L1 aptamer - BYZD | SEQ ID No. 242: TGCCCGCACATCAACTCATTGATAGACAATGCGTCCACTCGGGCA |
| | SEQ ID No. 243: TGCCCGCACATCAACTCATTGATAGACAATGCGTCCACTACGGGC |
| | SEQ ID No. 244: CGGGCACACATCAACTCATTGATAGACAATGCGTCCACTGCCCGT |
| | SEQ ID No. 245: GTTGGTCACATCAACTCATTGATAGACAATGCGTCCACTACCAAC |
| | SEQ ID No. 246: GGGCCACATCAACTCATTGATAGACAATGCGTCCACTGCCC |
| | SEQ ID No. 247: TGGTTGCACATCAACTCATTGATAGACAATGCGTCCACTCAACCA |
| | SEQ ID No. 248: TACAGGTTCTGGGGGGTGGGTGGGGAACCTGTT |
| | SEQ ID No. 249: CTACGAGACGAACTTATGCGTAATAATGACTGTCGTAG |
| PD1 aptamer (mouse) MP7 | SEQ ID No. 250: GACGATAGCGGTGACGGCACAGACGGTACAGTTCCCGTCCCTGCACTA CACGTATGCCGCTTCCGTCCGTCGCTC |
| PD1 aptamer | SEQ ID No. 251: GAGCGACGGACGGAAGCGGCATACGTGTAGTGCAGGGACGGGAACTG TACCGTCTGTGCCGTCACCGCTATCGTC |
| PD1 aptamer (human) | SEO ID No. 252: GGATCCTATGACGCATTGACCCGCTGCCTCTACTGAGGCTGTGTCAGT GTGCGGCTCGGACTGTTGAATTC |
| PD1 aptamer-T (mouse) | SEQ ID No. 253: AGCGGTGACGGCACAGACGGTACAGTTCCCGTCCCTGCACTACACGTA TGCCGCT |
| PD1 aptamer-A6 | SEQ ID No. 254: ACCGACAGTGAAGGACTCAGCGAACTCTCAGACTCGGTTC |
| PTK-7 aptamer | SEQ ID No. 255: ATCTAACTGCTGCGCCGCCGGGAAAATACTGTACGGTTAGA |
| ProGRP-48 aptamer | SEQ ID No. 256: CATGCGGAGTAGAGCGAGCCCAGATAGTCCCTGGTTATTTCCTTAGG |
| SF aptamer | SEQ ID No. 257: GATCTCTCTCTGCCCTAAGTCCGCACCCGTGCTTCCCTGT |
| TBA15 aptamer | SEQ ID No. 258: GGTTGGTGTGGTTGG |
| TBA29 aptamer | SEQ ID No. 259: AGTCCGTGGTAGGGCAGGTTGGGGTGACT |
| TFRA4 aptamer | SEQ ID No. 260: GCGTGGTACCACGC |

(continued)

| Nucleic acid aptamer | DNA-form sequence (5'→3') |
|---|---|
| TFRA3 aptamer | SEQ ID No. 262: GCGTGGTCACACGC |
| TFRA3 aptamer | SEQ ID No. 264:<br>GCGGCGCCCACGAGCGTTCGCGTGGTCACACGCGTTCCGCCCTCCTAC<br>ATAGGGCGCATAGCCGTGGGCGCCGC |
| TTA1 aptamer | SEQ ID No. 265: CCTGCACTTGGCTTGGATTTCAGAAGGGAGACCC |
| | SEQ ID No. 266: CTGCACTTGGCTTGGATTTCAGAAGGGAGACCC |
| TLS9a aptamer | SEQ ID No. 268: AGTCCATTTTATTCCTGAATATTTGTTAACCTCATGGAC |
| TGF-βII aptamer S58 | SEQ ID No. 269:<br>ACATTGCTGCGTGATCGCCTCACATGGGTTTGTCTGGTCGATTTGGAGG<br>TGGTGGGTGGC |
| TNF-a aptamer | SEQ ID No. 274: GCGGCCGATAAGGTCTTTCCAAGCGAACGAAAA |
| TNF aptamer | SEQ ID No. 275:<br>GCGCCACTACAGGGGAGCTGCCATTCGAATAGGTGGGCCGC |
| T1 aptamer | SEQ ID No. 276:<br>CGCTCGATAGATCGAGCTTCGCTCGATGTGGTGTTGTGGGGGCTTGTAT<br>TGGTCGATCACGCTCTAGAGCACTG |
| Vap7 aptamer | SEQ ID No. 277: TGGTGGGGGTGGACGGGCCGGGTAGA; |
| VEGF121 | SEQ ID No. 281: TGTGGGGGTGGACTGGGTGGGTACC |
| VEGF-V7t1 | SEQ ID No. 282: TGTGGGGGTGGACGGGCCGGGTAGA |
| VCAM-1 aptamer | SEO ID No. 283:<br>ATACCAGCTTATTCAATTGGACACGGCAAAGGGGTATAGCCTACCGGA<br>CCGTGAACATGGAATGGTGTGCTGCGTGGAGATAGTAAGTGCAATCT |
| VCAM-1 aptamer | SEQ ID No. 284:<br>GGACACGGCAAAGGGGTATAGCCTACCGGACCGTGAACATGGAATGG<br>TGTGCTGCGTGG |
| VCAM-12d aptamer | SEQ ID No. 285:<br>AGGGAATCTTGCCTAGGGAGGGAGTAGCGAAAGGGCTCA |
| CH6 aptamer | SEQ ID No. 144:<br>AGTCTGTTGGACCGAATCCCGTGGACGCACCCTTTGGACG |
| PL 45 aptamer | SEQ ID No. 287:<br>ACTCATAGGGTTAGGGGCTGCTGGCCAGATACTCAGATGGTAGGGTTA<br>CTATGAGC |
| EP166 aptamer | SEQ ID No. 288:<br>AACAGAGGGACAAACGGGGGAAGATTTGACGTCGACGACA |
| AGC aptamer | SEQ ID No. 289:<br>CGACCCGGCACAAACCCAGAACCATATACACGATCATTAGTCTCCTGG<br>GCCG |
| Karpas299 aptamer | SEQ ID No. 290:<br>ATCCAGAGTGACGCAGCACCACCACCGTACAATTTTTTCATTACCTACT<br>CGGC |
| SW620 aptamer | SEQ ID No. 291:<br>CCCATCAATGTTACGACCCGCTAGGGCTGCTGTGCCATCGGGTAA |

(continued)

| Nucleic acid aptamer | DNA-form sequence (5'→3') |
|---|---|
| MDA-MB-231 aptamer | SEQ ID No. 292:<br>AGAATTCAGTCGGACAGCGAAGTAGTTTTCCTTCTAACCTAAGAACCC GCGGCAGTTTAATGTAGATGGACGAA |
| MCF-7 aptamer | SEQ ID No. 293:<br>GCATGGGGTTTCGGCGTTTCGTCTATCTTGTTTCTGTTAGCGTCT |
| PC-3 aptamer | SEQ ID No. 294:<br>TGCCACTACAGCTGGTTCGGTTTGGTGACTTCGTTCTTCGTTGTGGTGC TTAGTGGC |

RNA-form nucleic acid aptamers include the aptamers having the sequences listed below, or sequences having at least 85% identity (e.g., at least 90%, at least 95%, or at least 99% identity) to the sequences shown.

Table 4:

| Aptamer | RNA version (3' direction) |
|---|---|
| A15 aptamer | SEQ ID No. 130: CCCUCCUACAUAGGG |
| AS1411 aptamer | SEQ ID No. 132: GGUGGUGGUGGUUGUGGUGGUGGUGG |
| AS1411 aptamer (human,mouse) | SEQ ID No. 133:<br>AUUCUGAACCGUGUGCAGCACCACGCUGCACACGGUUCAGAAUACACA |
| BCMA | SEQ ID No. 140:<br>AGUGCAAGACGUUCGCAGAUUAGCGAAAAGAGGGUCUCAUUGACUAGUAC |
| CTLA-4 aptamer (human,mouse) | SEQ ID No. 141: GGUGGAAGAGGGAUGGGCCGACGUGCCGCAU |
| CTLA-4 aptamer (human,mouse) | SEQ ID No. 142:<br>TCCCTACGGCGCTAACGATGGTGAAAATGGGCCTAGGGTGGACGGTGCCACCGT GCTACAAC |
| CCL1 aptamer | SEQ ID No. 146: UGACUCCUCUGACAGCCUAAUUUCUCCCGAUUACCCUG |
| CEA aptamer | SEQ ID No. 149: CUAGGAUCCCCACUCACCAUCUCUCAGCUUGCUUCCUAGC |
| CD4-3 aptamer | SEQ ID No. 161:<br>GGGAGGACGAUGCGGUUUGGGGUUUUCCCGUGCCCCAGACGACUCGCCCGA |
| CD28 aptamer | SEQ ID No. 168:<br>GGGAGAGAGGAAGAGGGAUGGGGAUUAGACCAUAGGCUCCCAACCCCCCCCGG GAGAGAGGAAGAGGGAUGGGGAUUAGACCAUAGGCUCCCAACCCCCGGG |
| CD44 aptamer | SEQ ID No. 171:<br>GGGAUGGAUCCAAGCUUACUGGCAUCUGGAUUUGCGCGUGCCAGAAUAAAGA GUAUAACGUGUGAAUGGGAAGCUUCGAUAGGAAUUCGG |
| IL-4RA aptamer | SEQ ID No. 203: AAAAAGCAACAGGGUGCUCCAUGCGCAUGGAACCUGCGCG |
| EGFR aptamer | SEQ ID No. 177: GCCUUAGUAACGUGCUUUGAUGUCGAUUCGACAGGAGGC |
| EGFR aptamer | SEQ ID No. 178:<br>UGCCGCUAUAAUGCACGGAUUUAAUCGCCGUAGAAAAGCAUGUCAAAGCCGUU |
| EpCAM aptamer | SEQ ID No. 179: GCGACUGGUUACCCGGUCG |
| FGF2 | SEQ ID No. 183: GGGAUACUAGGGCAUUAAUGUUACCAGUGUAGUCCC |

(continued)

| Aptamer | RNA version (3' direction) |
|---|---|
| aptamer -F2 | |
| FGF2 aptamer | SEQ ID No. 184: GGGAAACUAGGGCGUUAACGUGACCAGUGUUUCUCGA |
| FGF2 aptamer | SEQ ID No. 185: GGGAAACUAGGGCGUUAACGUGACCAGUGUUUCCC |
| FGF5 aptamer | SEQ ID No. 186: GGGCGACCUCUCCGUACUGACCUACAGAGCGACAUACUAGUGUAUCCAGAUCGCCC |
| Her2 aptamer | SEQ ID No. 194: AGCCGCGAGGGGAGGGAUAGGGUAGGGCGCGGCU |
| HER3-A30 aptamer | SEQ ID No. 197: CAGCGAAAGUUGCGUAUGGGUCACAUCGCAGGCACAUGUCAUCUGGGCG |
| Her3 | SEQ ID No. 198: GAAUUCCGCGUGUGCCAGCGAAAGUUGCGUAUGGGCCACAUCGCAGGCACAUGUCAUCUGGGCGGUCCGUUCGGGAUCC |
| LAG-3 aptamer | SEQ ID No. 206: GGGAGAGAGAUAUAAGGGCCUCCUGAUACCCGCUGCUAUCUGGACCGAUCCCAUUACCAAAUUCUCUCCC |
| MUC1 aptamer | SEQ ID No. 212: GCAGUUGAUCCUUUGGAUACCCUGG |
| MRP1 aptamer | SEQ ID No. 216: GGGAGAAUAGUCAACAAAUCGUUUGGGGCGACUUCUCCUUCCUUUCUCCC |
| OX40 aptamer (mouse) | SEQ ID No. 226: GGGAGGACGAUGCGGCAGUCUGCAUCGUAGGAAUCGCCACCGUAUACUUUCCCACCAGACGACUCGCUGAGGAUCCGAGA |
| OX40 aptamer | SEQ ID No. 228: GGGAGGACGAUGCGGCAGUCUGCAUCGUAGGAAUCGCCACCGUAUACUUUCCCACCAGACGACUCGCUG |
| OX40 aptamer 9.8(mouse) | SEQ ID No. 229: CAGUCUGCAUCGUAGGAAUCGCCACCGUAUACUUUCCCAC |
| OX40 aptamer -9C7T | SEQ ID No. 230: GGGAUGCGGAAAAAAGAACACUUCCGAUUAGGGCCCACCCUAACGGCCGCAGAC |
| PSMA aptamer (A10-) | SEQ ID No. 233: GGGAGGACGAUGCGGAUCAGCCAUGUUUACGUCACUCCU |
| PSMA(A9g) aptamer | SEQ ID No. 234: GGGACCGAAAAAGACCUGACUUCUAUACUAAGUCUACGUUCCC |
| PDGFRβ aptamer | SEQ ID No. 236: UGUCGUGGGGCAUCGAGUAAAUGCAAUUCGACA |
| TFRA4 aptamer | SEQ ID No. 261: GCGUGGUACCACGC |
| TFRA3 aptamer | SEQ ID No. 263: GCGUGGUCACACGC |
| TTA1 aptamer | SEQ ID No. 267: CCUGCACUUGGCTTGGAUUUCAGAAGGGAGACCC |
| TIM3 aptamer 1(mouse) | SEQ ID No. 270: GGGAGAGGACCAGUAGCCACUAUGGUGUUGGAGCUAGCGGCAGAGCGUCGCGGUCCCUCCC |

(continued)

| Aptamer | RNA version (3' direction) |
|---|---|
| TIM3-aptamer 2 | SEQ ID No. 271: <br><br> GGGAGAGGACCAGUACUGGUAGUUCUCUGUGCGACUCCUACAGAGCGUCGCGG UCCCUCCC |
| TIMC-d aptamer | SEQ ID No. 272: AAGCAACACUUAGUCGCGAUUGAUACGUGCGCAGUCAU |
| TIMC-11 aptamer | SEQ ID No. 273: <br><br> GGAGGACGAUGCGGGGAAGCAACACUUAGUCGCGAUUGAUACGUGCGCAGUCA UCAGACGACUCGCUGAGGAUCCGAGA |
| VEGF aptamer | SEQ ID No. 278: AUGCAGUUUGAGAAGUCGCGCAU |
| VEGF165 aptamer | SEQ ID No. 280: CGGAAUCAGUGAAUGCUUAUACAUCCG |
| 4-1BB aptamer (mouse) | SEQ ID No. 286: <br><br> GGGAGAGAGGAAGAGGGAUGGGCGACCGAACGUGCCCUUCAAAGCCGUUCACU AACCAGUGGCAUAACCCAGAGGUCGAUAGUACUGGAUCCCCCC |

In certain preferred embodiments, the nucleic acid drug contains only a small-molecule compound having targeting activity; in other preferred embodiments, it contains only a nucleic acid aptamer having targeting activity; and in still other embodiments, it simultaneously includes a nucleic acid aptamer and a small-molecule compound, each having targeting activity. Specific small-molecule targeting compounds include, without limitation, one or more of folic acid, biotin, vitamin B12, and mannose, and the small-molecule targeting compound is disposed at the 5' end or the 3' end of at least one of the sequence a, sequence b, and sequence c. Where the small-molecule compound is biotin or folic acid, its role is to impart targeting to the nucleic acid nanoparticles, for example to specifically target cancer cells. Malignant tumor tissues require larger amounts of vitamin B12, and the corresponding receptor (transcobalamin II receptor, CD320) is overexpressed in many cancer cells; therefore, vitamin B12 is a highly suitable vehicle for in vivo tumor diagnosis and therapy. Conjugating vitamin B 12 to the surface of nucleic acid nanoparticles is a practical method to achieve safety and improve efficiency, including for imaging and therapeutic applications. Mannose per se exhibits an inhibitory effect on the proliferation of cancer cells.

It should be understood that, for different oligonucleotide effector molecules, depending on whether they are single- or double-stranded, sequence length, secondary structure, and other factors, when being loaded onto the nucleic acid carrier, in order to improve the stability of the carrier after drug loading or to maintain the inherent three-dimensional conformation, each is independently attached to the nucleic acid carrier by any one or more of the following modes: 1) a single-stranded bridging sequence; 2) sequence a complementary to the single-stranded bridging sequence; 3) a transition sequence, wherein the single-stranded bridging sequence and the transition sequence are the single-stranded bridging sequence and the transition sequence of the foregoing nucleic acid carrier.

The foregoing targeting molecules and loading modes help to further improve the reliability of targeted delivery of the oligonucleotide effector molecules, thereby conferring superior specificity. They also further promote loading stability and loading amount. These loading modes render the drug particle size more appropriate, enabling entry into cells via cell-surface receptor-mediated endocytosis while avoiding non-specific cellular permeation and renal filtration/clearance; accordingly, a favorable particle size contributes to improved pharmacokinetics, pharmacodynamics, biodistribution, and toxicological distribution.

It should be understood that the foregoing (1) single-stranded bridging sequence; (2) sequence complementary to the single-stranded bridging sequence; and (3) transition sequence are not essential and may be omitted depending on the particular oligonucleotide effector molecule. For example, a miRNA can itself function as a transition sequence; alternatively, certain RNA-type aptamers possess inherent flexibility and can be directly linked to the nucleic acid carrier sequence without the interposition of a transition sequence or a single-stranded bridging sequence.

It should be understood that, as required for actual pharmacodynamic needs, at least one of the oligonucleotide effector molecules disclosed herein is modified at one or more nucleotides, the modification being selected from at least one of: fluoro (F) substitution, methyl, amino, disulfide, carbonyl, carboxyl, thiol, aldehyde, thioate (phosphorothioate), inverted dT, and locked nucleic acid (LNA) (e.g., AmiR-21 in a phosphorothioate-modified form or in an LNA-modified form). Similarly, for siRNA, the internucleotidic phosphate linkages at the 5' end of the antisense and sense strands are phosphorothioate-modified, and the 3' end carry dTdT modifications; alternatively, the 3' end of the siRNA antisense strand is linked to AA, UU, or any dinucleotide combination (including but not limited to CC, GG, CG, UG, or TT). Preferably, the miRNA includes a phosphorothioate modification or an LNA modification; preferably, the nucleic acid aptamer includes a phosphorothioate modification; preferably, the nucleic acid immunostimulant includes a phosphorothioate modification. Such modifications enhance the intrinsic stability of the oligonucleotide effector molecules and their stability after loading onto the nucleic acid carrier.

Among these, phosphate-backbone modification is the most fundamental chemical modification. The most commonly used backbone modification is the thioate (phosphorothioate) modification, i.e., replacement of a non-bridging oxygen atom of the phosphodiester linkage with sulfur (P-S in place of P-O), which reduces oligonucleotide hydrophilicity, increases resistance to nuclease degradation, improves stability and prolong the half-life.

In addition to phosphate-backbone modification, to enhance the resistance of RNA oligonucleotides to RNase-mediated degradation, the ribose moiety may, in some instances, be modified-primarily by chemically modifying hydroxyl groups on the ribose. Ribose modification can affect the affinity of a small nucleic acid for its target RNA, its stability toward nucleases, and its properties after binding RNA. Because RNA and DNA differ structurally only at the 2'-hydroxyl group, even minor alterations at this position can produce significant effects and determine the conformation of the RNA ribose, ultimately affecting affinity for the target RNA. Hydroxyl modification can also alter the nuclease sensitivity of the phosphate portion and influence the in vivo stability of oligonucleotide drugs. Common modifications include 2'-O-methyl, 2'-O-methoxyethyl (2'-O-MOE), and fluoro (F) substitution.

Additionally, certain chemical modifications are made to the parent ring of the ribose moiety, including LNA (locked nucleic acid), PNA (peptide nucleic acid), and PMO (phosphorodiamidate morpholino oligomer). LNA is a classical nucleotide-bridging modification that locks the ribose conformation by introducing a bridge between the 2' and 4' positions, thereby enhancing the resistance of oligonucleotide drugs to nucleases and increasing their affinity for the target mRNA. PNA- and PMO-based modifications can likewise enhance resistance to nucleases and improve affinity and specificity.

In various embodiments, the nucleic acid drug of the present application is configured such that different oligonucleotide effector molecules are loaded onto nucleic acid carriers having different sequence architectures, or, where necessary, it simultaneously includes small-molecule compounds having targeting activity. Representative sequence structures of certain nucleic acid drugs are set forth in the table below.

Table 5:

| | Molecule | Single strand | Sequence | Modification or remarks |
|---|---|---|---|---|
| 1 | 3*CpG2006-DNA carrier | strand a | SEQ ID No. 346:<br>TCGTCGTTTTGTCGTTTTGTCG TTTTTTTGCGGCGCCCACGAG CGTTCCGGGAGC | In the 5'-end CpG2006, all phosphodiester bonds between the 1st to 23rd nucleotides counted from the 5'-end are phosphorothioate-modified. |
| | | strand b | SEQ ID No. 347:<br>TCGTCGTTTTGTCGTTTTGTCG TTTTTTTGCTCCCGGTTCGCCG CCAGCCGCC | In the 5'-end CpG2006, all phosphodiester bonds between the 1st to 23rd nucleotides counted from the 5'-end are phosphorothioate-modified. |
| | | strand c | SEQ ID No. 348:<br>TCGTCGTTTTGTCGTTTTGTCG TTTTTTTGGCGGCAGGCGGCC ATAGCCGTGGGCGCCGC | In the 5'-end CpG2006, all phosphodiester bonds between the 1st to 23rd nucleotides counted from the 5'-end are phosphorothioate-modified. |
| 2 | 2*CpG1826-2*mannose-D NA carrier | strand a | SEQ ID No. 349:<br>TCCATGACGTTCCTGACGTTT TTGCGACGCCCACGAGCGTTC CGGGAGAGGAG | In the 5'-end CpG1826, all phosphodiester bonds between the 1st to 17th nucleotides counted from the 5'-end are phosphorothioate-modified. |
| | | strand b | SEO ID No. 22:<br>CTCCTCTCCCGGTTCGCCGCG AGCCGCG | One mannose modification is respectively introduced at each of the 5'- and 3'-termini. |
| | | strand c | SEQ ID No. 350:<br>TCCATGACGTTCCTGACGTTT TTCGCGGCACGCGGCCATAGC CGTGGGCGTCGC | In the 5'-end CpG1826, all phosphodiester bonds between the 1st to 17th nucleotides counted from the 5'-end are phosphorothioate-modified. |

| | Molecule | Single strand | Sequence | Modification or remarks |
|---|---|---|---|---|
| 3 | 2*CpG1826-Apt CD40-DNA carrier | strand a | SEQ ID No. 349:<br><br>TCCATGACGTTCCTGACGTTT TTGCGACGCCCACGAGCGTTC CGGGAGAGGAG | In the 5'-end CpG1826, all phosphodiester bonds between the 1st to 17th nucleotides counted from the 5'-end are phosphorothioate-modified. |
| | | strand b | SEO ID No. 351:<br><br>CCAACGAGTAGGCGATAGCG CGTGGTTTTTCTCCTCTCCCGG TTCGCCGCGAGCCGCG | |
| | | strand c | SEQ ID No. 350:<br><br>TCCATGACGTTCCTGACGTTT TTCGCGGCACGCGGCCATAGC CGTGGGCGTCGC | In the 5'-end CpG1826, all phosphodiester bonds between the 1st to 17th nucleotides counted from the 5'-end are phosphorothioate-modified. |
| 4 | 2*CpG2006-Apt CD40-DNA carrier | strand a | SEO ID No. 352:<br><br>TCGTCGTTTTGTCGTTTTGTCG TTTTTTTGCGACGCCCACGAG CGTTCCGGGAGAGGAG | In the 5'-end CpG2006, all phosphodiester bonds between the 1st to 23rd nucleotides counted from the 5'-end are phosphorothioate-modified. |
| | | strand b | SEQ ID No. 351:<br><br>CCAACGAGTAGGCGATAGCG CGTGGTTTTTCTCCTCTCCCGG TTCGCCGCGAGCCGCG | 5'-end CD40 aptamer. |
| | | strand c | SEQ ID No. 353:<br><br>TCGTCGTTTTGTCGTTTTGTCG TTTTTTTCGCGGCACGCGGCC ATAGCCGTGGGCGTCGC | In the 5'-end CpG2006, all phosphodiester bonds between the 1st to 23rd nucleotides counted from the 5'-end are phosphorothioate-modified. |

(continued)

| | Molecule | Single strand | Sequence | Modification or remarks |
|---|---|---|---|---|
| 5 | 4*CpG2006-Apt CD40-DNA carrier | strand a | SEQ ID No. 354:<br>TCGTCGTTTTGTCGTTTTGTCG TTTCGTCGTTTTGTCGTTTTGT CGTTTTTTTGCGACGCCCACG AGCGTTCCGGGAGAGGAG | In the 5'-end CpG2006-2006, all phosphodiester bonds between the 1st to 17th nucleotides and between the 19th to 35th nucleotides counted from the 5'-end are phosphorothioate-modified. |
| | | strand b | SEQ ID No. 351:<br>CCAACGAGTAGGCGGATAGCG CGTGGTTTTCTCCCTCTCCCGG TTCGCCGCGAGCCGCG | 5'-end CD40 aptamer. |
| | | strand c | SEQ ID No. 355:<br>TCGTCGTTTTGTCGTTTTGTCG TTTCGTCGTTTTGTCGTTTTGT CGTTTTTTTCGCGGCACGCGG CCATAGCCGTGGGCGTCGC | In the 5'-end CpG2006-2006, all phosphodiester bonds between the 1st to 23rd nucleotides and between the 25th to 47th nucleotides counted from the 5'-end are phosphorothioate-modified. |
| 6 | 2*CpG1826-Apt CD40-2*man nose-DNA carrier | strand a | SEQ ID No. 349:<br>TCCATGACGTTCCTGACGTTT TTGCGACCCCACGAGCCGTTC CGGGAGAGGAG | In the 5'-end CpG1826, all phosphodiester bonds between the 1st to 17th nucleotides counted from the 5'-end are phosphorothioate-modified, and the phosphodiester bonds between the 1st to 2nd nucleotides counted from the 3'-end are phosphorothioate-modified. |
| | | strand b | SEQ ID No. 351:<br>CCAACGAGTAGGCGGATAGCG CGTGGTTTTCTCCCTCTCCCGG TTCGCCGCGAGCCGCG | The phosphodiester bonds between the 1st to 2nd nucleotides counted from the 5'-end are phosphorothioate-modified, and a mannose is attached to the 3'-end. |
| | | strand c | SEQ ID No. 350:<br>TCCATGACGTTCCTGACGTTT TTCGCGGCACGCGGCCATAGC CGTGGGCGTCGC | In the 5'-end CpG1826, all phosphodiester bonds between the 1st to 17th nucleotides counted from the 5'-end are phosphorothioate-modified, and a mannose is attached to the 3'-end. |

| | Molecule | Single strand | Sequence | Modification or remarks |
|---|---|---|---|---|
| 7 | CpG1826-Apt CD40-Apt MUC1-manno se-DNA carrier | strand a | SEQ ID No. 349: TCCATGACGTTCCTGACGTTT TTGCGACGCCCACGAGCGTTC CGGGAGAGGAG | In the 5'-end CpG1826, all phosphodiester bonds between the 1st to 17th nucleotides counted from the 5'-end are phosphorothioate-modified. |
| | | strand b | SEO ID No. 351: CCAACGAGTAGGCGATAGCG CGTGGTTTTTCTCCTCTCCCGG TTCGCCGCGAGCCGCG | 5'-end CD40 aptamer. |
| | | strand c | SEQ ID No. 356: GCAGTTGATCCTTTGGATACC CTGGTTTTTCGCGGCACGCGG CCATAGCCGTGGGCGTCGC | 5'-end MUC1 aptamer, and a mannose is attached to the 3'-end. |
| 8 | 2*CpG2006-Apt CD40-AmiR-21-3*mannos e-DNA carrier | strand a | SEQ ID No. 352: TCGTCGTTTTGTCGTTTTGTCG TTTTTTTGCGACGCCCACGAG CGTTCCGGGAGAGGAG | In the 5'-end CpG2006, all phosphodiester bonds between the 1st to 23rd nucleotides counted from the 5'-end are phosphorothioate-modified, and a mannose is attached to the 3'-end. |
| | | strand b | SEQ ID No. 357: CCAACGAGTAGGCGATAGCG CGTGGTTTTTCTCCTCTCCCGG TTCGCCGCGAGCCGCGGATAA GCT | 5'-end CD40 aptamer; at the 3'-end, an AmiR-21 locked nucleic acid (LNA) is introduced, and the 3'-end is capped with biotin (H). |
| | | strand c | SEQ ID No. 353: TCGTCGTTTTGTCGTTTTGTCG TTTTTTTCGCGGCACGCGGCC ATAGCCGTGGGCGTCGC-mannose | In the 5'-end CpG2006, all phosphodiester bonds between the 1st to 23rd nucleotides counted from the 5'-end are phosphorothioate-modified, and a mannose is attached to the 3'-end. |

| | Molecule | Single strand | Sequence | Modification or remarks |
|---|---|---|---|---|
| 9 | 2*CpG2006-Apt CD40-Apt CD16a-Apt CTLA-4-DN A carrier | strand a | SEQ ID No. 358:<br>TCGTCGTTTTGTCGTTTTGTCG TTTTTTTGCGACGCCCACGAG CGTTCCGGGAGAGGAGCT | In the 5'-end CpG2006, all phosphodiester bonds between the 1st to 23rd nucleotides counted from the 5'-end are phosphorothioate-modified, and the first phosphodiester bond from the 3'-end is phosphorothioate-modified. |
| | | strand b | SEQ ID No. 359:<br>C*CAACGAGTAGGCGATAGCG CGTGGTTTTTGCTCCTCTCCCG GTTCGCCGCGAGCCGCGTTTT TCCACTGCGGGGGTCTATACG TGAGGAAGAAGTG*G | The first phosphodiester bond from each of the 5'- and 3'-termini is phosphorothioate-modified. |
| | | strand c | SEQ ID No. 360:<br>TCGTCGTTTTGTCGTTTTGTCC TTTTTTTCGCGGCACGCGGCC ATAGCCGTGGGCGTCGCTTTT TGGTGGAAGAGGGAUGGGCC GACGUGCCGCAU | All phosphodiester bonds between the 1st to 23rd nucleotides counted from the 5'-end are phosphorothioate-modified, and the first phosphodiester bond from the 3'-end is phosphorothioate-modified. |
| 10 | 2*CpG2006-Apt CTLA-4-Apt | strand a | SEQ ID No. 361:<br>T*C*G*T*C*G*T*T*T*T*G*T*C *G*T*T*T*T*G*T*C*G*T*TT*C | In the 5'-end CpG2006-2006, all phosphodiester bonds between the 1st to 23rd nucleotides and between the 25th to |
| | PD-L1-DNA | | *G*T*C*G*T*T*T*T*T*G*T*C*G* T*T*T*T*G*T*C*G*T*TTTTTT GCGACGCCCACGAGCGTTCCG GGAGAGGAGC*T | 47th nucleotides counted from the 5'-end are phosphorothioate-modified, and the first phosphodiester bond from the 3'-end is phosphorothioate-modified. |
| | | strand b | SEQ ID No. 362:<br>GGUGGAAGAGGGAUGGGCCG ACGUGCCGCAUTTTTTGCTCC TCTCCCGGTTCGCCGCGAGCC GCGT | RNA C/U 2'-F, A/G 2'-OMe; the first phosphodiester bond from each of the 5'- and 3'-termini is phosphorothioate-modified. |
| | | strand c | SEQ ID No. 363:<br>ACGGGCCACATCAACTCATTG ATAGACAATGCGTCCACTGCC CGTTTTTTCGCGGCTCGCGGC TCATAGCCGTGGGCGTCGCT | The first phosphodiester bond from each of the 5'- and 3'-termini is phosphorothioate-modified. |

EP 4 745 242 A1

100

| | Molecule | Single strand | Sequence | Modification or remarks |
|---|---|---|---|---|
| 11 | CpG2006-Apt CD40-Apt PD-L1-DNA( CCPD3) | strand a | SEQ ID No. 309:<br>TCGTCGTTTTGTCGTTTTGTCG TTTTTTTGCGACGCCCACGAG CGTTCCGGGAGAGGAGC | The phosphodiester bonds between the 1st to 23rd nucleotides counted from the 5'-end and the first and second phosphodiester bonds from the 3'-end are phosphorothioate-modified. |
| | | strand b | SEQ ID No. 330:<br>CCAACGAGTAGGCGATAGCG CGTGGTTTTTGCTCCTCTCCCG GTTCGCCGCGAGCCGCG | The first and second phosphodiester bonds from each of the 5'- and 3'-termini are phosphorothioate-modified. |
| | | strand c | SEQ ID No. 331:<br>ACGGGCCACATCAACTCATTG ATAGACAATGCGTCCACTGCC CGTTTTTTCGCGGCTCGCGGC CATAGCCGTGGGCGTCGC | The first and second phosphodiester bonds from each of the 5'- and 3'-termini are phosphorothioate-modified. |
| 11 variant (1) | CpG2006-Apt CD40-Apt PD-L1-DNA (CCPD3) strand c replaced by HQ PD-L1 | strand a | SEQ ID No. 309:<br>TCGTCGTTTTGTCGTTTTGTCG TTTTTTTGCGACGCCCACGAG CGTTCCGGGAGAGGAGC | The phosphodiester bonds between the 1st to 23rd nucleotides counted from the 5'-end and the first and second phosphodiester bonds from the 3'-end are phosphorothioate-modified. |
| | | strand b | SEQ ID No. 330:<br>CCAACGAGTAGGCGATAGCG CGTGGTTTTTGCTCCTCTCCCG GTTCGCCGCGAGCCGCG | The first and second phosphodiester bonds from each of the 5'- and 3'-ends are phosphorothioate-modified. |
| | | strand c | SEQ ID No. 364:<br>AACAACGTATAACAATGCCCA CGTCACCAGAGTACTATGGAT TTCGCGGCTCGCGGCCATAGC CGTGGGCGTCGC | The phosphodiester bonds between the 1st to 3rd nucleotides counted from the 5'-end and the first and second phosphodiester bonds from the 3'-end are phosphorothioate-modified. |

|  | Molecule | Single strand | Sequence | Modification or remarks |
|---|---|---|---|---|
| 11 variant (2) | CpG2006-Apt CD40-Apt PD-L1-DNA (CCPD3) with shortened a, b anstrand d cs (1) | strand a | SEQ ID No. 365:<br><br>TCGTCGTTTTGTCGTTTTGTCG TTTTTTGCCCACGAGCGTTCC GGGAGA | The phosphodiester bonds between the 1st to 24th nucleotides counted from the 5'-end are phosphorothioate-modified. |
|  |  | strand b | SEO ID No. 366:<br><br>GCCAACGAGTAGGCGATAGC GCGTGGCTTTTTCTCCCGGTTC GCCGCGAG | The phosphodiester bonds between the 1st to 3rd nucleotides counted from the 5'-end are phosphorothioate-modified. |
|  |  | strand c | SEQ ID No. 367:<br><br>ACGGGCCACATCAACTCATTG ATAGACAATGCGTCCACTGCC CGTTTTTCTCGCGGCCATAGC CGTGGGC | The phosphodiester bonds between the 1st to 4th nucleotides counted from the 5'-end are phosphorothioate-modified. |
| 11 variant (3) | CpG2006-Apt CD40-Apt PD-L1-DNA (CCPD3) with shortened a, b anstrand d cs (2) | strand a | SEQ ID No. 365:<br><br>TCGTCGTTTTGTCGTTTTGTCG TTTTTTGCCCACGAGCGTTCC GGGAGA | The phosphodiester bonds between the 1st to 24th nucleotides counted from the 5'-end are phosphorothioate-modified. |
|  |  | strand b | SEQ ID No. 366:<br><br>GCCAACGAGTAGGCGATAGC GCGTGGCTTTTTCTCCCGGTTC GCCGCGAG | The phosphodiester bonds between the 1st to 3rd nucleotides counted from the 5'-end are phosphorothioate-modified. |
|  |  | strand c | SEQ ID No. 368:<br><br>GGGCCACATCAACTCATTGAT AGACAATGCGTCCACTGCCCT TTTCTCGCGGCCATAGCCGTG GGC | The phosphodiester bonds between the 1st to 3rd nucleotides counted from the 5'-end are phosphorothioate-modified. |

|  | Molecule | Single strand | Sequence | Modification or remarks |
|---|---|---|---|---|
| 11 variant (4) | CpG2006-Apt CD40-Apt PD-L1-DNA (CCPD3) a, b, c-XDPD-L1 shortened (3) | strand a | SEQ ID No. 365:<br>TCGTCGTTTTGTCGTTTTGTCG TTTTTTGCCCACGAGCGTTCC GGGAGA | The phosphodiester bonds between the 1st to 24th nucleotides counted from the 5'-end are phosphorothioate-modified. |
|  |  | strand b | SEQ ID No. 366:<br>GCCAACGAGTAGGCGATAGC GCGTGGCTTTTTCTCCCGGTTC GCCGCGAG | The phosphodiester bonds between the 1st to 3rd nucleotides counted from the 5'-end are phosphorothioate-modified. |
|  |  | strand c | SEQ ID No. 369:<br>TACAGGTTCTGGGGGGTGGGT GGGGAACCTGTTTTTTTCTCGC GGCCATAGCCGTGGGC |  |
| 11 variant (5) | CpG2006-Apt CD40-Apt PD-L1-DNA (CCPD3) with a, b, c and d four strands | strand a | SEQ ID No. 309:<br>TCGTCGTTTTGTCGTTTTGTCG TTTTTTTGCGACGCCCACGAG CGTTCCGGGAGAGGAGC | The phosphodiester bonds between the 1st to 25th nucleotides counted from the 5'-end are phosphorothioate-modified. |
|  |  | strand b | SEQ ID No. 330:<br>CCAACGAGTAGGCGATAGCG CGTGGTTTTTGCTCCTCTCCCG GTTCGCCGCGAGCCGCG | The phosphodiester bonds between the 1st to 4th nucleotides counted from the 5'-end are phosphorothioate-modified. |
|  |  | strand c | SEQ ID No. 20:<br>CAGCAGCAGCAGCACGCGGC TCGCGGCCATAGCCGTGGGCG TCGC |  |
|  |  | strand d | SEQ ID No. 297:<br>TGCTGCTGCTGCTGTTTTTACG GGCCACATCAACTCATTGATA GACAATGCGTCCACTGCCCGT | The phosphodiester bonds between the 1st to 3rd nucleotides counted from the 3'-end are phosphorothioate-modified. |

EP 4 745 242 A1

| | Molecule | Single strand | Sequence | Modification or remarks |
|---|---|---|---|---|
| 11 variant (6) | CpG2006-Apt CD40-Apt PD-L1-DNA (CCPD3) with a, b, c and d four strands | strand a | SEQ ID No. 309:<br>TCGTCGTTTTGTCGTTTTGTCG TTTTTTTGCGACGCCCACGAG CGTTCCGGGAGAGGAGC | The phosphodiester bonds between the 1st to 25th nucleotides counted from the 5'-end are phosphorothioate-modified. |
| | | strand b | SEQ ID No. 298:<br>TGCTGCTGCTGCTGTTTTTTAC AGGTTCTGGGGGGTGGGTGG GGAACCTGTT | The phosphodiester bonds between the 1st to 3rd nucleotides counted from the 3'-end are phosphorothioate-modified. |
| | | strand c | SEO ID No. 20:<br>CAGCAGCAGCAGCACGCGGC TCGCGGCCATAGCCGTGGGCG TCGC | |
| | | strand d | SEQ ID No. 330:<br>CCAACGAGTAGGCGATAGCG CGTGGTTTTTGCTCCTCTCCCG GTTCGCCGCGAGCCGCG | The phosphodiester bonds between the 1st to 4th nucleotides counted from the 5'-end are phosphorothioate-modified. |
| 11 variant (7) | CpG2006-Apt CD40-Apt PD-L1-DNA3 -TY (CCPD3 short structure, split into six strands) | strand a | SEQ ID No. 15:<br>GACGCCCACGAGCGTTCCGGG AGAGGTGTAGCACGGTGGC | |
| | | strand b | SEQ ID No. 16:<br>CCTCTCCCGGTTCGCCGCGAG CCTCGGCGCGGCCGTG | |
| | | strand c | SEQ ID No. 17:<br>GGCTCGCGGCCATAGCCGTGG GCGTCTGCTGCTGCTGCTG | |
| | | strand d | SEQ ID No. 338:<br>GCCAACGAGTAGGCGATAGC GCGTGGCTTTTTGCCACCGTG CTACA | The phosphodiester bonds between the 1st to 3rd nucleotides counted from the 5'-end are phosphorothioate-modified. |

| | Molecule | Single strand | Sequence | Modification or remarks |
|---|---|---|---|---|
| | | strand e | SEQ ID No. 336:<br>ACGGGCCACATCAACTCATTG | The phosphodiester bonds between the 1st to 3rd nucleotides counted from the |
| | | | ATAGACAATGCGTCCACTGCC CGTTTTTTCACGGCCGCGCCG A | 5'-end are phosphorothioate-modified. |
| | | strand f | SEQ ID No. 337:<br>TCGTCGTTTTGTCGTTTTGTCG TTTTTTTCAGCAGCAGCAGCA | In the 5'-end CpG2006, all phosphodiester bonds between the 1st to 23rd nucleotides counted from the 5'-end are phosphorothioate-modified. |
| 11 variant (8) | CpG2006-Apt CD40-Apt PD-L1-DNA carrier (modification pattern changed to increase structural stability) | strand a | SEQ ID No. 309:<br>TCGTCGTTTTGTCGTTTTGTCG TTTTTTTGCGACGCCCACGAG CGTTCCGGGAGAGGAGC | In the 5'-end CpG2006, all phosphodiester bonds between the 1st to 23rd nucleotides counted from the 5'-end are phosphorothioate-modified. |
| | | strand b | SEQ ID No. 370:<br>GCCAACGAGTAGGCGATAGC GCGTGGCTTTTTGCTCCTCTCC CGGTTCGCCGCGAGCCGCG | The 1st to 4th nucleotides counted from the 5'-end are 2'-O-methoxyethyl (2'-O-MOE) modified, and the base corresponding to the Cm nucleotide is 5-methyl-modified. |
| | | strand c | SEQ ID No. 331:<br>ACGGGCCACATCAACTCATTG ATAGACAATGCGTCCACTGCC CGTTTTTTCGCGGCTCGCGGC CATAGCCGTGGGCGTCGC | The 1st to 4th nucleotides counted from the 5'-end are 2'-O-methoxyethyl (2'-O-MOE) modified, and the base corresponding to the Cm nucleotide is 5-methyl-modified. |

EP 4 745 242 A1

| | Molecule | Single strand | Sequence | Modification or remarks |
|---|---|---|---|---|
| 11 variant (9) | CpG2006-Apt CD40-Apt PD-L1-DNA carrier (number of modification sites changed to increase structural stability) | strand a | SEQ ID No. 309:<br>TCGTCGTTTTGTCGTTTTGTCG TTTTTTTGCGACGCCCACGAG CGTTCCGGGAGAGGAGC | In the 5'-end CpG2006, all phosphodiester bonds between the 1st to 23rd nucleotides counted from the 5'-end are phosphorothioate-modified |
| | | strand b | SEQ ID No. 330:<br>CCAACGAGTAGGCGATAGCG CGTGGTTTTTGCTCCTCTCCCG GTTCGCCGCGAGCCGCG | The 1st to 5th nucleotides counted from the 5'-end are 2'-O-methoxyethyl (2'-O-MOE) modified, and the base corresponding to the Cm nucleotide is 5-methyl-modified. |
| | | strand c | SEQ ID No. 331:<br>ACGGGCCACATCAACTCATTG ATAGACAATGCGTCCACTGCC CGTTTTTTCGCGGCTCGCGGC CATAGCCGTGGGCGTCGC | The 1st to 5th nucleotides counted from the 5'-end are 2'-O-methoxyethyl (2'-O-MOE) modified, and the base corresponding to the Cm nucleotide is 5-methyl-modified. |
| 11 variant (10) | CpG2006-Apt CD40-Apt PD-L1-DNA carrier (PD-L1 sequence changed, number of modifications increased to enhance structural stability) | strand a | SEQ ID No. 309:<br>TCGTCGTTTTGTCGTTTTGTCG TTTTTTTGCGACGCCCACGAG CGTTCCGGGAGAGGAGC | In the 5'-end CpG2006, all phosphodiester bonds between the 1st to 23rd nucleotides counted from the 5'-end are phosphorothioate-modified. |
| | | strand b | SEQ ID No. 330:<br>CCAACGAGTAGGCGATAGCG CGTGGTTTTTGCTCCTCTCCCG GTTCGCCGCGAGCCGCG | The 1st to 5th nucleotides counted from the 5'-end are 2'-O-methoxyethyl (2'-O-MOE) modified, and the base corresponding to the Cm nucleotide is 5-methyl-modified. |
| | | strand c | SEQ ID No. 371:<br>CTACGAGACGAACTTATGCGT AATAATGACTGTCGTAGTTTT TCGCGGCTCGCGGCCATAGCC GTGGGCGTCGC | The 1st to 5th nucleotides counted from the 5'-end are 2'-O-methoxyethyl (2'-O-MOE) modified, and the base corresponding to the Cm nucleotide is 5-methyl-modified. |

| | Molecule | Single strand | Sequence | Modification or remarks |
|---|---|---|---|---|
| 11 variant (11) | CpG2006-Apt CD40-Apt PD-L1-DNA carrier (strand c truncated, modification pattern changed) | strand a | SEQ ID No. 309:<br>TCGTCGTTTTGTCGTTTTGTCG TTTTTTTGCGACGCCCACGAG CGTTCCGGGAGAGGAGC | In the 5'-end CpG2006, all phosphodiester bonds between the 1st to 23rd nucleotides counted from the 5'-end are phosphorothioate-modified. |
| | | strand b | SEQ ID No. 330:<br>CCAACGAGTAGGCGATAGCG CGTGGTTTTTGCTCCTCTCCCG GTTCGCCGCGAGCCGCG | The 1st to 5th nucleotides counted from the 5'-end are 2'-O-methoxyethyl (2'-O-MOE) modified, and the base corresponding to the Cm nucleotide is 5-methyl-modified. |
| | | strand c | SEQ ID No. 299:<br>CGCGGCTCGCGGCTCGCGGCT CGCGGCCATAGCCGTGGGCGT CGC | |
| | | strand d | SEQ ID No. 372:<br>AGCCGCGAGCCGCGTTTTTAC | The 1st to 5th nucleotides counted from the 3'-end are 2'-O-methoxyethyl |
| | | | GGGCCACATCAACTCATTGAT AGACAATGCGTCCACTGCCCG T | (2'-O-MOE) modified. |

| | Molecule | Single strand | Sequence | Modification or remarks |
|---|---|---|---|---|
| 11 var-ian t (12) | CpG2006-Apt CD40-Apt PD-L1-DNA carrier (strand c truncated, PD-L1 aptamer sequence and modification changed) | strand a | SEQ ID No. 309:<br>TCGTCGTTTTGTCGTTTTGTCG TTTTTTTGCGACGCCCACGAG CGTTCCGGGAGAGGAGC | In the 5'-end CpG2006, all phosphodiester bonds between the 1st to 23rd nucleotides counted from the 5'-end are phosphorothioate-modified. |
| | | strand b | SEQ ID No. 330:<br>CCAACGAGTAGGCGATAGCG CGTGGTTTTTGCTCCTCTCCCG GTTCGCCGCGAGCCGCG | The 1st to 5th nucleotides counted from the 5'-end are 2'-O-methoxyethyl (2'-O-MOE) modified, and the base corresponding to the Cm nucleotide is 5-methyl-modified. |
| | | strand c | SEQ ID No. 299:<br><u>CGCGGCTCGCGGCTCGCGGCT CGCGGCCATAGCCGTGGGCGT CGC</u> | |
| | | strand d | ID No. 373:<br>AGCCGCGAGCCGCGTTTTTCT ACGAGACGAACTTATGCGTAA TAATGACTGTCGTAG | The 1st to 5th nucleotides counted from the 3'-end are 2'-O-methoxyethyl (2'-O-MOE) modified. |
| 12 | Apt CD16a-Apt CTLA-4-CpG 2006-DNA carrier | strand a | SEQ ID No. 374:<br>CCACTGCGGGGGTCTATACGT GAGGAAGAAGTGGTTTTTGCG ACGCCCACGAGCGTTCCGGGA GAGGAGCT | The first phosphodiester bond from each of the 5'- and 3'-termini is phosphorothioate-modified. |
| | | strand b | SEQ ID No. 362:<br>GGUGGAAGAGGGAUGGGCCG ACGUGCCGCAUTTTTTGCTCC TCTCCCGGTTCGCCGCGAGCC GCGT | RNA C/U 2'-F and A/G 2'-OMe; the first phosphodiester bond from each of the 5'- and 3'-termini is phosphorothioate-modified. |
| | | strand c | SEQ ID No. 375:<br>TCGTCGTTTTGTCGTTTTGTCG TTTTTTTCGCGGCACGCGGCC ATAGCCGTGGGCGTCGCT | In the 5'-end CpG2006, all phosphodiester bonds between the 1st to 23rd nucleotides counted from the 5'-end are phosphorothioate-modified, and the first phosphodiester bond from the 3'-end is phosphorothioate-modified. |

| | Molecule | Single strand | Sequence | Modification or remarks |
|---|---|---|---|---|
| 13 | 2CpG2006-Apt CD40-Apt PD-L1-Apt C12-DNA carrier | strand a | SEQ ID No. 376:<br>TCGTCGTTTTGTCGTTTTGTCG TTTTTTTGACGCCCACGAGCG TTCCGGGAGAGG | In the 5'-end CpG2006, all phosphodiester bonds between the 1st to 23rd nucleotides counted from the 5'-end are phosphorothioate-modified. |
| | | strand b | SEQ ID No. 377:<br>CCAACGAGTAGGCGATAGCG CGTGGTTTTTCCTCTCCCGGTT CGCCGCGAGCCTGTAGCACGG TGGC | The phosphodiester bonds between the 1st to 2nd nucleotides counted from the 5'-end are phosphorothioate-modified. |
| | | strand d | SEQ ID No. 378:<br>ACGGGCCACATCAACTCATTG ATAGACAATGCGTCCACTGCC CGTTTTTTGCCACCGTGCTAC A | The phosphodiester bonds between the 1st to 2nd nucleotides counted from the 5'-end are phosphorothioate-modified. |
| | | strand c | SEQ ID No. 379:<br>TCGTCGTTTTGTCGTTTTGTCG TTTTTTGGCTCGCGGCCATAG CCGTGGGCGTCTGCTGCTGCT GCTG | In the 5'-end CpG2006, all phosphodiester bonds between the 1st to 23rd nucleotides counted from the 5'-end are phosphorothioate-modified. |
| | | strand e | SEQ ID No. 380:<br>GTGGATTGTTGTGTTCTGTTG GTTTTTGTGTTGTCTTTTTCAG CAGCAGCAGCA | The phosphodiester bonds between the 1st to 2nd nucleotides counted from the 5'-end are phosphorothioate-modified. |
| 14 | CpG2006-Apt C12-CD47 siRNA-Apt PD-L1-PD-L1 siRNA-DNA | strand a | SEQ ID No. 309:<br>TCGTCGTTTTGTCGTTTTGTCG TTTTTTTGCGACGCCCACGAG CGTTCCGGGAGAGGAGC | The phosphodiester bonds between the 1st to 23rd nucleotides counted from the 5'-end and the first and second phosphodiester bonds from the 3'-end are phosphorothioate-modified. |

(continued)

| Molecule | Single strand | Sequence | Modification or remarks |
|---|---|---|---|
| carrier | strand b | SEQ ID No. 381:<br>GTGGATTGTTGTTGTTCTGTTG GTTTTTGTTGTTGTCTTTTTAUA UCUCUGGGUAAUCACCUU | The phosphodiester bonds between the 1st to 2nd nucleotides counted from the 5'-end are phosphorothioate-modified; in the RNA region downstream of 11111, the first and second phosphodiester bonds from the 5'-end are phosphorothioate-modified; the first and second nucleotides from each of the 5'- and 3'-termini are 2'-O-methyl (2'-OMe) modified, and the remaining C/U nucleotides are 2'-fluoro (2'-F) modified. |
| | strand c | SEQ ID No. 382:<br>GGUGAUUACCCAGAGAUAUU UGCTCCTCTCCCGGTTCGCCG CGAGCCGCG | For the RNA, the first two nucleotides from each of the 5'- and 3'-termini are 2'-O-methyl (2'-OMe) modified, and the remaining C/U nucleotides are 2'-fluoro (2'-F) modified; the phosphodiester bonds between the 1st to 2nd and 19th to 20th nucleotides counted from the 5'-end and the first two phosphodiester bonds from the 3'-end are all phosphorothioate-modified. |
| | strand d | SEQ ID No. 383:<br>GCCCCAGTTATGCTTTCCCCC TCTGTCTCTTTGTTTTTUUGAU UCUCAGUGUGCUGGGUU | The phosphodiester bonds between the 1st to 2nd nucleotides counted from the 5'-end are phosphorothioate-modified; in the RNA sequence downstream of 11111, the first two nucleotides from the 5'-end are 2'-O-methyl (2'-OMe) modified and the remaining C/U nucleotides are 2'-fluoro (2'-F) modified, and the first and second phosphodiester bonds from the 5'-end are phosphorothioate-modified. |
| | strand e | SEQ ID No. 384:<br>CCAGCACACUGAGAAUCAAU UCGCGGCTCGCGGCCATAGCC GTGGGCGTCGC | In the RNA, the first two nucleotides from each of the 5'- and 3'-termini are 2'-O-methyl (2'-OMe) modified and the remaining C/U nucleotides are 2'-fluoro (2'-F) modified; over the entire sequence, the first and second phosphodiester bonds from each of the 5'- and 3'-termini are phosphorothioate-modified. |
| 2*CpG2006-Apt CD40-Apt CD16a-DNA carrier | strand a | SEQ ID No. 358:<br>TCGTCGTTTTGTCGTTTTGTCG TTTTTTGCGACGCCCACGAG CGTTCCGGGAGAGGAGCT | The phosphodiester bonds between the 1st to 23rd nucleotides counted from the 5'-end and the first phosphodiester bond from the 3'-end are phosphorothioate-modified. |
| | strand b | SEQ ID No. 359:<br>CCAACGAGTAGGCGATAGCG CGTGGTTTTGCTCCTCTCCCG GTTCGCCGCGAGCCGCGTTT TCCACTGCGGGGGTCTATACG TGAGGAAGAAGTGG | The first phosphodiester bond from each of the 5'- and 3'-termini is phosphorothioate-modified. |
| | strand c | SEQ ID No. 385:<br>TCGTCGTTTTGTCGTTTTGTCG TTTTTTCGCGGCTCGCGGCC ATAGCCGTGGGCGTCGCT | The phosphodiester bonds between the 1st to 23rd nucleotides counted from the 5'-end and the first phosphodiester bond from the 3'-end are phosphorothioate-modified. |

| | Molecule | Single strand | Sequence | Modification or remarks |
|---|---|---|---|---|
| 16 | 2*CpG2006-Apt CD40-Apt CD16a-Apt FAP-DNA carrier | strand a | SEQ ID No. 358:<br><br>TCGTCGTTTTGTCGTTTTGTCG TTTTTTTGCGACGCCCACGAG CGTTCCGGGAGAGGAGCT | The phosphodiester bonds between the 1st to 23rd nucleotides counted from the 5'-end and the first phosphodiester bond from the 3'-end are phosphorothioate-modified. |
| | | strand b | SEO ID No. 359:<br><br>CCAACGAGTAGGCGATAGCG CGTGGTTTTTGCTCCTCTCCCG | The first phosphodiester bond from each of the 5'- and 3'-termini is phosphorothioate-modified. |
| | | | GTTCGCCGCGAGCCGCGTTTT TCCACTGCGGGGGTCTATACG TGAGGAAGAAGTGG | |
| | | strand c | SEQ ID No. 386:<br><br>TCGTCGTTTTGTCGTTTTGTCG TTTTTTTCGCGGCTCGCGGCC ATAGCCGTGGGCGTCGCTTTT TCCGCTCGAGCTAGTCTGACA AAGAGAAACAC | The phosphodiester bonds between the 1st to 23rd nucleotides counted from the 5'-end and the first phosphodiester bond from the 3'-end are phosphorothioate-modified. |

(continued)

| | Molecule | Single strand | Sequence | Modification or remarks |
|---|---|---|---|---|
| 17 | 2*CpG2006-Apt VEGF-Apt CD40-Apt PD-L1-Apt CD16a-DNA carrier | strand a | SEQ ID No. 387:<br>TCGTCGTTTTGTCGTTTTGTCG TTTTTTTGCGACGCCCACGAG CGTTCCGGGAGAGGAGCTTTT TTGGTGGGGGTGGACGGGCC GGGTAGA | The phosphodiester bonds between the 1st to 23rd nucleotides counted from the 5'-end and the first phosphodiester bond from the 3'-end are phosphorothioate-modified. |
| | | strand b | SEQ ID No. 388:<br>CCAACGAGTAGGCGATAGCG CGTGGTTTTTGCTCCTCTCCCG GTTCGCCGCGAGCCGCGTTTT TACGGGCCACATCAACTCATT GATAGACAATGCGTCCACTGC CCGT | The first phosphodiester bond from each of the 5'- and 3'-termini is phosphorothioate-modified. |
| | | strand c | SEQ ID No. 389:<br>TCGTCGTTTTGTCGTTTTGTCG TTTTTTTCGCGGCTCGCGGCC ATAGCCGTGGGCGTCGCTTTT TCCACTGCGGGGGTCTATACG TGAGGAAGAAGTGG | The phosphodiester bonds between the 1st to 23rd nucleotides counted from the 5'-end and the first phosphodiester bond from the 3'-end are phosphorothioate-modified. |
| 18 | 2*AmiR-21-Apt TTA1-DNA | strand a | SEQ ID No. 390:<br>GATAAGCTGCGGCGCCCACG AGCGTTCCGGGAGC | The 5'-end GATAAGCT sequence is fully locked nucleic acid (LNA) modified. |
| | | strand b | SEQ ID No. 391:<br>CTGCACTTGGCTTGGATTTCA GAAGGGAGACCCTTTTTGCTC CCGGTTCGCCGCCAGCCGCC | 5'-end TTA1 aptamer. |
| | | strand c | SEQ ID No. 392:<br>GATAAGCTGGCGGCAGGCGG CCATAGCCGTGGGCGCCGC | The 5'-end GATAAGCT sequence is fully locked nucleic acid (LNA) modified. |

| | Molecule | Single strand | Sequence | Modification or remarks |
|---|---|---|---|---|
| 19 | 2*AmiR-21-3 *Bio-DNA carrier | strand a | SEQ ID No. 390: GATAAGCTGCGGCGCCCACGAGCGTTCCGGGAGC | The 5'-end GATAAGCT sequence is fully locked nucleic acid (LNA) modified. |
| | | strand b | SEQ ID No. 34: GCTCCCGGTTCGCCGCCAGCCGCC | Biotin (H) is attached to each of the 5'- and 3'-termini. |
| | | strand c | SEQ ID No. 392: GATAAGCTGGCGGCAGGCGGCCATAGCCGTGGGCGCCGC | The 5'-end GATAAGCT sequence is fully locked nucleic acid (LNA) modified, and the 3'-end is biotin (H) modified. |
| 20 | 2*AmiR-21-4 *Bio-DNA carrier | strand a | SEQ ID No. 390: GATAAGCTGCGGCGCCCACGAGCGTTCCGGGAGC | The 5'-end GATAAGCT sequence is fully locked nucleic acid (LNA) modified, and the 3'-end is biotin (H) modified. |
| | | strand b | SEQ ID No. 34: GCTCCCGGTTCGCCGCCAGCCGCC | Biotin (H) is attached to each of the 5'- and 3'-termini. |
| | | strand c | SEQ ID No. 392: GATAAGCTGGCGGCAGGCGGCCATAGCCGTGGGCGCCGC | The 5'-end GATAAGCT sequence is fully locked nucleic acid (LNA) modified, and the 3'-end is biotin (H) modified. |
| 21 | 2*AmiR-21-Apt AS1411-DNA carrier | strand a | SEO ID No. 393: GATAAGCTGCGACGCCCACGAGCGTTCCGGGAGAGGAG | The 5'-end GATAAGCT sequence is fully locked nucleic acid (LNA) modified. |
| | | strand b | SEQ ID No. 394: GGTGGTGGTGGTTGTGGTGGT | 5'-end AS1411 aptamer. |
| | | | GGTGGTTTTTCTCCTCTCCCGGTTCGCCGCGAGCCGCG | |
| | | strand c | SEO ID No. 395: GATAAGCTCGCGGCACGCGGCCATAGCCGTGGGCGTCGC | The 5'-end GATAAGCT sequence is fully locked nucleic acid (LNA) modified. |

(continued)

| | Molecule | Single strand | Sequence | Modification or remarks |
|---|---|---|---|---|
| 22 | 2*AmiR-21-Apt AS1411-DNA carrier | strand a | SEQ ID No. 396: GATAAGCTGCGGCGCCCACGAGCGTTCCGGGAGAGGAG | In the 5'-end "GATAAGCT" sequence, the bases at positions 1, 3, 4 and 5 are 2'-O-methyl (2'-OMe) modified, and the bases at positions 2, 6, 7 and 8 are 2'-fluoro (2'-F) modified. |
| | | strand b | SEQ ID No. 397: GGTGGTGGTGGTTGTGGTGGTGGTGGTTTTCTCCTCTCCCGGTTCGGCCGCCAGCCGCC | 5'-end AS1411 aptamer. |
| | | strand c | SEQ ID No. 392: GATAAGCTGCGGCAGGCGGCCATAGCCGTGGGCGCCGC | In the 5'-end "GATAAGCT" sequence, the bases at positions 1, 3, 4 and 5 are 2'-O-methyl (2'-OMe) modified, and the bases at positions 2, 6, 7 and 8 are 2'-fluoro (2'-F) modified. |
| 23 | 2*AmiR-21-Apt AS1411-Bio-D NA carrier | strand a | SEQ ID No. 390: GATAAGCTGCGGCGCCCACGAGCGTTCCGGGAGC | The 5'-end GATAAGCT sequence is fully locked nucleic acid (LNA) modified. |
| | | strand b | SEQ ID No. 398: GGTGGTGGTGGTTGTGTGGTGGTGGTGGTTTTTGCTCCCGGTTCGCCGCCAGCCGCC | 5'-end AS1411 aptamer. |
| | | strand c | SEQ ID No. 392: GATAAGCTGGCGGTAGGCGGCCATAGCCGTGGGCGCCGC; | 5'-end AS1411 aptamer, and the 3'-end is biotin (H) modified. |
| 24 | 2*AmiR-21-Apt CD40-DNA carrier | strand a | SEQ ID No. 390: GATAAGCTGCGGCGCCCACGAGCGTTCCGGGAGC | The 5'-end GATAAGCT sequence is fully locked nucleic acid (LNA) modified. |
| | | strand b | SEQ ID No. 399: CCAACGAGTAGGCGGATAGCGCGTGGTTTTTGCTCCCGGTTCGCCGCCAGCCGCC | 5'-end CD40 aptamer. |
| | | strand c | SEQ ID No. 392: GATAAGCTGCGGCAGGCGGCCATAGCCGTGGGCGCCGC | The 5'-end GATAAGCT sequence is fully locked nucleic acid (LNA) modified. |

| | Molecule | Single strand | Sequence | Modification or remarks |
|---|---|---|---|---|
| 25 | 2*AmiR-21-Apt MUC1-3*Bio-D NA carrier | strand a | SEQ ID No. 390: GATAAGCTGCGGCGCCCACG AGCGTTCCGGGAGC | The 5'-end GATAAGCT sequence is fully locked nucleic acid (LNA) modified. |
| | | strand b | SEO ID No. 400: GCAGTTGATCCTTTGGATACC CTGGTTTTTGCTCCCGGTTCGC CGCCAGCCGCC | 5'-end MUC1 aptamer. |
| | | strand c | SEQ ID No. 392: GATAAGCTGGCGGCAGGCGG CCATAGCCGTGGGCGCCGC | The 5'-end GATAAGCT sequence is fully locked nucleic acid (LNA) modified. |
| 26 | AmiR-21-Apt AS1411-Apt A15-2*Bio-D NA carrier | strand a | SEQ ID No. 390: GATAAGCTGCGGCGCCCACG AGCGTTCCGGGAGC | The 5'-end GATAAGCT sequence is fully locked nucleic acid (LNA) modified. |
| | | strand b | SEQ ID No. 398: GGTGGTGGTGGTTGTGGTGGT GGTGGTTTTTGCTCCCGGTTC GCCGCCAGCCGCC; | 5'-end AS1411 aptamer, and the 3'-end is biotin-modified. |
| | | strand c | SEQ ID No. 401: CCCTCCTACATAGGGTTTTTG GCGGCAGGCGGCCATAGCCG TGGGCGCCGC; | 5'-end A15 aptamer, and the 3'-end is biotin (H) modified. |
| 27 | 2*mannose-2*A miR-21-DNA carrier | strand a | SEQ ID No. 390: GATAAGCTGCGGCGCCCACG AGCGTTCCGGGAGC | The 5'-end GATAAGCT sequence is fully locked nucleic acid (LNA) modified. |

| | Molecule | Single strand | Sequence | Modification or remarks |
|---|---|---|---|---|
| | | strand b | SEQ ID No. 34:<br>GCTCCCGGTTCGCCGCCAGCC GCC | Mannose is attached to each of the 5'- and 3'-termini, and the first and second phosphodiester bonds from the 3'-end are phosphorothioate-modified. |
| | | strand c | SEQ ID No. 392:<br>GATAAGCTGGCGGCAGGCGG CCATAGCCGTGGGCGCCGC | The 5'-end is AmiR-21 with locked nucleic acid (LNA) modification. |
| 28 | 2*mannose-Apt A15-2*AmiR-2 1-DNA carrier | strand a | SEQ ID No. 390:<br>GATAAGCTGCGGCGCCCACG AGCGTTCCGGGAGC | The 5'-end is AmiR-21 with locked nucleic acid (LNA) modification. |
| | | strand b | SEQ ID No. 402:<br>CCCTCCTACATAGGGTTTTTG CTCCCGGTTCGCCGCCAGCCG CC | Mannose is attached to each of the 5'- and 3'-termini, and the first and second phosphodiester bonds from the 3'-end are phosphorothioate-modified. |
| | | strand c | SEQ ID No. 392:<br>GATAAGCTGGCGGCAGGCGG CCATAGCCGTGGGCGCCGC | The 5'-end is AmiR-21 with locked nucleic acid (LNA) modification. |
| 29 | 2*mannose-su rvivin siRNA-DNA carrier | strand a | SEQ ID No. 105:<br>UGACAGAUAAGGAACCUGCT T | At the 3'-end, "TT" represents a dT-dT (dTdT) modification. |
| | | strand b | SEQ ID No. 33:<br>GCGGCGCCCACGAGCGTTCCG GGAGC | |
| | | strand c | SEQ ID No. 34:<br>GCTCCCGGTTCGCCGCCAGCC GCC | Mannose is attached to each of the 5'- and 3'-termini, and the first and second phosphodiester bonds from the 3'-end are phosphorothioate-modified. |
| | | strand d | SEQ ID No. 403:<br>GGCGGCAGGCGGCCATAGCC GTGGGCGCCGCUGCAGGUUC CUUAUCUGUCATT | At the 3'-end, "TT" represents a dT-dT (dTdT) modification. |

| | Molecule | Single strand | Sequence | Modification or remarks |
|---|---|---|---|---|
| 30 | 2*mannose-A pt A15-survivin-AmiR-21-DN A carrier | strand a | SEQ ID No. 105:<br>UGACAGAUAAGGAACCUGCT T | At the 3'-end, "TT" represents a dT-dT (dTdT) modification. |
| | | strand b | SEQ ID No. 33:<br>GCGGCGCCCACGAGCGTTCCG GGAGC | |
| | | strand c | SEQ ID No. 404:<br>CCCTCCTACATAGGGTTTTTCT CCTCTCCCGGTTCGCCGCCAG CCGCC | Mannose is attached to each of the 5'- and 3'-termini, and the first and second phosphodiester bonds from the 3'-end are phosphorothioate-modified. |
| | | strand d | SEQ ID No. 403:<br>GGCGGCAGGCGGCCATAGCC GTGGGCGCCGCUGCAGGUUC CUUAUCUGUCATT | At the 3'-end, the first and second positions "TT" represent a dT-dT (dTdT) modification. |
| 31 | Apt AS1411-survivi n siRNA-Apt PSMA-3*A-miR -21-RNA carrier | strand a | SEQ ID No. 405:<br>GGTGGTGGTGGTTGTGGTGGT GGTGGTTUGUGACAGAUAAG GAACCUGCAG | The 29th and 30th phosphodiester bonds counted from the 5'-end and the first and second phosphodiester bonds from the 3'-end are phosphorothioate-modified. |
| | | strand b | SEQ ID No. 406:<br>GCGGCGCCCACGAGCGUUCC GGGAGAGGCCAAGGGACCGA AAAAGACCUGACUUCUAUAC UAAGUCUACGUUCCC | The first phosphodiester bond from each of the 5'- and 3'-termini is phosphorothioate-modified. |
| | | strand c | SEQ ID No. 407:<br>GGCCUCUCCCGGUUCGCCGC CAGCCGCCAAGAUAAGCUGA UAAGCUGAUAAGCU | The first phosphodiester bond from the 5'-end, and from the 3'-end the first to seventh, ninth to fifteenth, and sixteenth to twenty-third phosphodiester bonds are phosphorothioate-modified. |
| | | strand d | SEQ ID No. 408:<br>GGCGGCUGGCGGCCAUAGCC GUGGGCGCCGCAAAAGCAGG UUCCUUAUCUGUCACAUU | The first phosphodiester bond from the 5'-end, and in the RNA region downstream of "AAAA", the first and second phosphodiester bonds from each of the 5'- and 3'-termini are |

(continued)

| | Molecule | Single strand | Sequence | Modification or remarks |
|---|---|---|---|---|
| | | | | phosphorothioate-modified. |
| 32 | 3*AmiR-21-A pt ATP-Apt FGF2-DNA carrier | strand a | SEQ ID No. 409: GATAAGCTGATAAGCTGATAA GCTGCGGCGCCCACGAGCGTT CCGGGAGAGGCCT | The phosphodiester bonds between the 1st to 7th, 9th to 15th and 16th to 23rd nucleotides counted from the 5'-end, and the first phosphodiester bond from the 3'-end, are phosphorothioate-modified. |
| | | strand b | SEQ ID No. 410: ACCTGGGGAGTATTGGGGAG GAAGGTTTTTGGCCTCTCCCG GTTCGCCGCCAGCCGCCT | The first phosphodiester bond from each of the 5'- and 3'-termini is phosphorothioate-modified. |
| | | strand c | SEQ ID No. 411: GGGAUACUAGGGCAUUAAUG UUACCAGUGUAGUCCCUGGC GGCTGGCGGCCATAGCCGTGG GCGCCGCT | The first phosphodiester bond from each of the 5'- and 3'-termini is phosphorothioate-modified; in the RNA sequence, C/U are 2'-fluoro (2'-F) modified and A/G are 2'-O-methyl (2'-OMe) modified. |

(continued)

| | Molecule | Single strand | Sequence | Modification or remarks |
|---|---|---|---|---|
| 33 | Apt AS1411-survi vin siRNA-Apt PSMA-3*Ami R-21-DNA-R NA mixed carrier | strand a | SEQ ID No. 412:<br><br>GGTGGTGGTGGTTGTGGTGGT GGTGGAAUGUGACAGAUAAG GAACCUGCAG | The first phosphodiester bond from the 5'-end is phosphorothioate-modified; in the RNA sequence downstream of "AA", the first and second phosphodiester bonds from each of the 5'- and 3'-termini are phosphorothioate-modified; in the RNA sequence, the 2nd, 6th, 8th, 9th, 12th, 14th and 16th nucleotides counted from the 5'-end are 2'-fluoro (2'-F) modified, and the remaining nucleotides are 2'-O-methyl (2'-OMe) modified. |
| | | strand b | SEQ ID No. 413:<br>GCGGCGCCCACGAGCGTTCCG GGAGAGGCCAAGGGACCGAA AAAGACCUGACUUCUAUACU AAGUCUACGUUCCC | RNA C/U 2'-F, A/G 2'-OMe; the first and second phosphodiester bonds from each of the 5'- and 3'-termini are phosphorothioate-modified. |
| | | strand c | SEQ ID No. 407:<br>GGCCUCUCCCGGUUCGCCGC CAGCCGCCAAGAUAAGCUGA UAAGCUGAUAAGCU | C/U 2'-F, A/G 2'-OMe; the first phosphodiester bond from the 5'-end, and, in each of the three AmiR-21 units at the 3'-end, the phosphodiester bonds between the 1st to 7th nucleotides of each AmiR-21 are phosphorothioate-modified. |
| | | strand d | SEQ ID No. 408:<br><br>GGCGGCUGGCGGCCAUAGCC GUGGGCGCCGCAAAAGCAGG UUCCUUAUCUGUCACAUU | For the first 35 bases, A/G are 2'-O-methyl (2'-OMe) modified and C/U are 2'-fluoro (2'-F) modified; from the 36th base onwards, in the RNA sequence, the first and second phosphodiester bonds from each of the 5'- and 3'-termini are phosphorothioate-modified, and the 7th, 9th, 10th and 11th nucleotides counted from the 5'-end are 2'-fluoro (2'-F) modified while the remaining RNA nucleotides are 2'-O-methyl (2'-OMe) modified. |

| | Molecule | Single strand | Sequence | Modification or remarks |
|---|---|---|---|---|
| 34 | 3*AmiR-21-Apt AS1411-Apt FAP-DNA carrier | strand a | SEQ ID No. 414:<br>GATAAGCTGATAAGCTGATAA GCTTTTTTGCGGCGCCCACGA GCGTTCCGGGAGAGGCCT | The phosphodiester bonds between the 1st to 7th, 9th to 15th and 16th to 23rd nucleotides counted from the 5'-end, and the first phosphodiester bond from the 3'-end, are phosphorothioate-modified. |
| | | strand b | SEQ ID No. 415:<br>GGTGGTGGTGGTTGTGGTGGT GGTGGTTTTTGGCCTCTCCCG GTTCGCCGCCAGCCGCCT | The first phosphodiester bond from each of the 5'- and 3'-termini is phosphorothioate-modified. |
| | | strand c | SEQ ID No. 416:<br>CCGCTCGAGCTAGTCTGACAA AGAGAAACACTTTTTGGCGGC TGGCGGCCATAGCCGTGGGCG CCGCT | The first phosphodiester bond from each of the 5'- and 3'-termini is phosphorothioate-modified. |
| 35 | Apt AS1411-survivi n siRNA-Apt EpCAM-3*Ami R-21-DNA carrier | strand a | SEQ ID No. 412:<br>GGTGGTGGTGGTTGTGGTGGT GGTGGAAUGUGACAGAUAAG GAACCUGCAG | The first and second phosphodiester bonds from each of the 5'- and 3'-termini are phosphorothioate-modified; in the RNA region downstream of the 29th nucleotide, the first and second phosphodiester bonds from each of the 5'- and 3'-termini are phosphorothioate-modified; in this RNA region, the 2nd, 6th, 8th, 9th, 12th, 14th and 16th nucleotides counted from the 5'-end are 2'-fluoro (2'-F) modified and the remaining nucleotides are 2'-O-methyl (2'-OMe) modified. |
| | | strand b | SEQ ID No. 417:<br>GCGGCGCCCACGAGCGTTCCG GGAGAGGCCAAGCGACUGGU UACCCGGUCG | RNA C/U are 2'-fluoro (2'-F) modified and A/G are 2'-O-methyl (2'-OMe) modified. |
| | | strand c | SEQ ID No. 418:<br>GGCCTCTCCCGGTTCGCCGCC AGCCGCCAAGATAAGCTGAT AAGCTGATAAGCT | The first phosphodiester bond from the 5'-end is phosphorothioate-modified; in each AmiR-21 unit of the three AmiR-21 units at the 3'-end, all phosphodiester bonds are phosphorothioate-modified. |
| | | strand d | SEQ ID No. 419:<br>GGCGGCTGGCGGCCATAGCC GTGGGCGCCGCAAAAGCAGG UUCCUUAUCUGUCACAUU | In the RNA sequence downstream of the 36th base, the first and second phosphodiester bonds from each of the 5'- and 3'-termini are phosphorothioate-modified; in this RNA region, the 7th, 9th, 10th and 11th nucleotides counted from the 5'-end are 2'-fluoro (2'-F) modified and the remaining RNA nucleotides are 2'-O-methyl (2'-OMe) modified. |

(continued)

| | Molecule | Single strand | Sequence | Modification or remarks |
|---|---|---|---|---|
| 36 | Apt IL-4RA-Apt TGF-β-Apt PD-L1-2*Ami R-21-DNA carrier | strand a | SEQ ID No. 420:<br>AAAAAGCAACAGGGUGCUCC AUGCGCAUGGAACCUGCGCG GATAAGCTGCGGCGCCCACG AGCGTTCCGGGAGAGGCCT | The first to third phosphodiester bonds from the 5'-end and the first phosphodiester bond from the 3'-end are phosphorothioate-modified; the internal GATAAGCT sequence is an AmiR-21 motif with phosphorothioate modification; in the RNA region, C/U are 2'-fluoro (2'-F) modified and A/G are 2'-O-methyl (2'-OMe) modified. |
| | | strand b | SEQ ID No. 421:<br>ACATTGCTGCGTGATCGCCTC ACATGGGTTTGTCTGGTCGAT TTGGAGGTGGTGGGTGGCTTT TTGGCCTCTCCCGGTTCGCCG CCAGCCGCCT | The first to third phosphodiester bonds from the 5'-end and the first phosphodiester bond from the 3'-end are phosphorothioate-modified. |
| | | strand c | SEQ ID No. 422:<br>ACGGGCCACATCAACTCATTG ATAGACAATGCGTCCACTGCC CGTGATAAGCTGGCGGCTGGC GGCCATAGCCGTGGGCGCCGC T | The first to third phosphodiester bonds from the 5'-end and the first phosphodiester bond from the 3'-end are phosphorothioate-modified; the internal GATAAGCT sequence is an AmiR-21 motif with phosphorothioate modification. |
| 37 | Apt AS1411-ATAD 2 siRNA-Apt GPC3-5*AimR-21-DNA carrier | strand a | SEQ ID No. 423:<br>GGTGGTGGTGGTTGTGGTGGT GGTGGGATAAGCT*GCGUCGAA GUUGUAGGAUUUU* | The first phosphodiester bond from the 5'-end, the phosphodiester bonds within the internal GATAAGCT sequence, and, in the RNA sequence downstream of the GATAAGCT sequence, the first and second phosphodiester bonds from each of the 5'- and 3'-termini are phosphorothioate-modified; in the RNA sequence downstream of the GATAAGCT sequence, the 2nd, 6th, 8th, |

121

EP 4 745 242 A1

(continued)

| | Molecule | Single strand | Sequence | Modification or remarks |
|---|---|---|---|---|
| | | | | 9th, 12th, 14th and 16th nucleotides counted from the 5'-end are 2'-fluoro (2'-F) modified and the remaining nucleotides are 2'-O-methyl (2'-OMe) modified. |
| | | strand b | SEQ ID No. 424:<br><br>GCGGCGCCCACGAGCGTTCCG GGAGAGGCCGATAAGCT | The first phosphodiester bond from the 5'-end and the phosphodiester bonds within the 3'-end GATAAGCT sequence are phosphorothioate-modified. |
| | | strand c | SEQ ID No. 425:<br><br>TAACGCTGACCTTAGCTGCAT GGCTTTACATGTTCCAGATAA GCTGGCCTCTCCCGGTTCGCC GCCAGCCGCCT | The first and second phosphodiester bonds from each of the 5'- and 3'-termini are phosphorothioate-modified; the phosphodiester bonds within the internal GATAAGCT sequence are phosphorothioate-modified. |
| | | strand d | SEQ ID No. 426:<br><br>GATAAGCTGGCGGCTGGCGG CCATAGCCGTGGGCGCCGCGA TAAGCTAAUCCUACAACUUC GACGCUU | The phosphodiester bonds within the 5'-end GATAAGCT and within the internal GATAAGCT sequence are phosphorothioate-modified; in the RNA sequence downstream of the internal GATAAGCT sequence, the first and second phosphodiester bonds from each of the 5'- and 3'-termini are phosphorothioate-modified. |

| | Molecule | Single strand | Sequence | Modification or remarks |
|---|---|---|---|---|
| 38 | Apt Vap7-Apt TGF-β-Apt Act-12c-3*A miR-21-DNA carrier | strand a | SEQ ID No. 427:<br>TGGTGGGGGTGGACGGGCCG GGTAGAGATAAGCTGATAAG CTGCGGCGCCCACGAGCGTTC CGGGAGAGGCCT | The phosphodiester bonds between the 1st to 3rd nucleotides counted from the 5'-end, the first phosphodiester bond from the 3'-end, and the phosphodiester bonds within the internal AmiR-21 sequence GATAAGCT are phosphorothioate-modified. |
| | | strand b | SEQ ID No. 421:<br>ACATTGCTGCGTGATCGCCTC ACATGGGTTTGTCTGGTCGAT TTGGAGGTGGTGGGTGGCTTT TTGGCCTCTCCCGGTTCGCCG CCAGCCGCCT | The first to third phosphodiester bonds from the 5'-end and the first phosphodiester bond from the 3'-end are phosphorothioate-modified. |
| | | strand c | SEQ ID No. 428:<br>CGGGGAAAGTCACGGGGGGT TTCAGATGTTCTGATCGGTGT GGAGGATAAGCTGGCGGCTG GCGGCCATAGCCGTGGGCGCC GCT | The phosphodiester bonds between the 1st to 3rd nucleotides counted from the 5'-end, the first phosphodiester bond from the 3'-end, and the phosphodiester bonds within the internal AmiR-21 sequence GATAAGCT are phosphorothioate-modified. |
| 39 | Apt AS1411-3*A miR-21-Apt IL-4Ra-Apt VCAM-1-DN A carrier | strand a | SEQ ID No. 429:<br>GGTGGTGGTGGTTGTGGTGGT GGTGGTTTTTGATAAGCTGAT AAGCTGATAAGCTGCGGCGCC CACGAGCGTTCCGGGAGAGG CCT | The first phosphodiester bond from each of the 5'- and 3'-termini and the phosphodiester bonds within the internal AmiR-21 sequences GATAAGCT are phosphorothioate-modified. |
| | | strand b | SEQ ID No. 430:<br>AAAAAGCAACAGGGUGCUCC AUGCGCAUGGAACCUGCGCG TTTTTGGCCTCTCCCGGTTCGC CGCCAGCCGCCT | The first phosphodiester bond from each of the 5'- and 3'-termini is phosphorothioate-modified; in the RNA portion upstream of "TTTTT", C/U are 2'-fluoro (2'-F) modified and A/G are 2'-O-methyl (2'-OMe) modified. |
| | | strand c | SEO ID No. 431:<br>GGACACGGCAAAGGGGTATA GCCTACCGGACCGTGAACATG GAATGGTGTGCTGCGTGGTTT TTGGCGGCTGGCGGCCATAGC CGTGGGCGCCGCT | The first phosphodiester bond from each of the 5'- and 3'-termini is phosphorothioate-modified. |

(continued)

| | Molecule | Single strand | Sequence | Modification or remarks |
|---|---|---|---|---|
| 40 | Apt GPC1-Apt | strand a | SEQ ID No. 432:<br>AACGGAGTGTGGCTAACTCGA | The phosphodiester bonds between the 1st to 3rd nucleotides counted from the |
| | AS1411-Apt EpCAM-2*A miR-21-DNA carrier | | GATAAGCTGCGGCGCCCACG AGCGTTCCGGGAGAGGCC | 5'-end, the first and second phosphodiester bonds from the 3'-end, and the phosphodiester bonds within the internal AmiR-21 sequence GATAAGCT are phosphorothioate-modified; the first and second nucleotides from the 3'-end are 2'-O-methoxyethyl (2'-O-MOE) modified. |
| | | strand b | SEQ ID No. 433:<br>GGTGGTGGTGGTTGTGGTGGT GGTGGTTTTTGGCCTCTCCCG GTTCGCCGCCAGCCGCC | The phosphodiester bonds at the 1st, 3rd, 13th, 16th and 24th positions counted from the 5'-end and the first and second phosphodiester bonds from the 3'-end are phosphorothioate-modified, and the first and second nucleotides from the 3'-end are 2'-O-methyl (2'-O-MOE) modified. |
| | | strand c | SEQ ID No. 434:<br>GCGACUGGUUACCCGGUCGG ATAAGCTGGCGGCTGGCGGCC ATAGCCGTGGGCGCCGC | In the RNA portion, C/U are 2'-fluoro (2'-F) modified and A/G are 2'-O-methyl (2'-OMe) modified; the phosphodiester bonds between the 1st to 3rd nucleotides counted from the 5'-end, the first and second phosphodiester bonds from the 3'-end, and the phosphodiester bonds within the internal AmiR-21 sequence GATAAGCT are phosphorothioate-modified, and the first and second nucleotides from the 3'-end are 2'-O-methoxyethyl (2'-O-MOE) modified. |
| 41 | 3*AmiR-21-Apt AS1411-Apt CD40-2*Bio-D NA carrier | strand a | SEQ ID No. 435:<br>GATAAGCTGCGACGCCCACG AGCGTTCCGGGAGAGGAGGA TAAGCT | The 5'-end and 3'-end AmiR-21 GATAAGCT sequences are fully locked nucleic acid (LNA) modified, and the 3'-end is biotin (H) modified. |
| | | strand b | SEQ ID No. 436:<br>GGTGGTGGTGGTTGTGGTGGT GGTGGTTTTTCTCCTCTCCCGG TTCGCCGCGAGCCGCGGATAA GCT | The 3'-end AmiR-21 GATAAGCT sequence is fully locked nucleic acid (LNA) modified. |
| | | strand c | SEQ ID No. 437:<br>CCAACGAGTAGGCGATAGCG CGTGGTTTTTCGCGGCACGCG GCCATAGCCGTGGGCGTCGC | 5'-end CD40 aptamer, and the 3'-end is biotin (H) modified. |

124

EP 4 745 242 A1

(continued)

| | Molecule | Single strand | Sequence | Modification or remarks |
|---|---|---|---|---|
| | | strand a | SEQ ID No. 438:<br>AAAAAGCAACAGGGGUGCUCC AUGCGCAUGGAACCUGCGCG GATAAGCTGCGACGCCCACG AGCGTTCCGGGGAGGAGCT | The phosphodiester bonds between the 1st to 3rd nucleotides counted from the 5'-end, the first phosphodiester bond from the 3'-end, and the phosphodiester bonds within the internal AmiR-21 GATAAGCT sequences are phosphorothioate-modified; in the RNA region, C/U are 2'-fluoro (2'-F) modified and A/G are 2'-O-methyl (2'-OMe) modified. |
| 42 | Apt IL-4RA-Apt VCAM-12d-Apt PD-L1-3*Ami R-21-DNA car-rier | strand b | SEQ ID No. 439:<br>AGGGAATCTTGCCTAGGGAG GGAGTAGCGAAAGGGCTCAG ATAAGCTGCTCCTCTCCCGGT TCGCCGCGAGCCGCGT | The phosphodiester bonds between the 1st to 3rd nucleotides counted from the 5'-end, the first phosphodiester bond from the 3'-end, and the phosphodiester bonds within the internal AmiR-21 GATAAGCT sequences are phosphorothioate-modified. |
| | | strand c | SEQ ID No. 440:<br>ACGGGCCACATCAACTCATTG ATAGACAATGCGTCCACTGCC CGTGATAAGCTCGCGGCTCGC GGCCATAGCCGTGGGCGTCGC | The phosphodiester bonds between the 1st to 3rd nucleotides counted from the 5'-end, the first phosphodiester bond from the 3'-end, and the phosphodiester bonds within the internal AmiR-21 |
| | | | T | GATAAGCT sequences are phosphorothioate-modified. |

| | Molecule | Single strand | Sequence | Modification or remarks |
|---|---|---|---|---|
| 43 | TAP siRNA-3*Am iR-21-Apt 2*AS1411-1-DNA carrier | strand a | SEQ ID No. 441:<br><br>GGTGGTGGTGGTTGTGGTGGT GGTGGAAAAAUUCUGAACCG UGUGCAGCUU | The first phosphodiester bond from the 5'-end is phosphorothioate-modified; in the RNA sequence downstream of "AAAA", the first and second phosphodiester bonds from each of the 5'- and 3'-termini are phosphorothioate-modified; in this RNA sequence, the 2nd, 6th, 8th, 9th, 12th, 14th and 16th nucleotides counted from the 5'-end are 2'-fluoro (2'-F) modified and the remaining nucleotides are 2'-O-methyl (2'-OMe) modified. |
| | | strand b | SEQ ID No. 442:<br><br>GCGACGCCCACGAGCGTTCCG GGAGAGGAGCAAAAGGTGGT GGTGGTTGTGGTGGTGGTGG | The first phosphodiester bond from each of the 5'- and 3'-termini is phosphorothioate-modified. |
| | | strand c | SEQ ID No. 443:<br><br>GCTCCTCTCCCGGTTCGCCGC GAGCCGCGAAGATAAGCTGA TAAGCTGATAAGCT | The first phosphodiester bond from the 5'-end and the phosphodiester bonds within each GATAAGCT sequence of the three AmiR-21 motifs at the 3'-end are phosphorothioate-modified. |
| | | strand d | SEQ ID No. 444:<br><br>CGCGGCTCGCGGCCATAGCCG TGGGCGTCGCAAAAGCUGCA CACGGUUCAGAAU | The first phosphodiester bond from the 5'-end and, in the RNA sequence downstream of "AAAA", the first and second phosphodiester bonds from each of the 5'- and 3'-termini are phosphorothioate-modified; in this RNA sequence, the 7th, 9th, 10th and 11th nucleotides counted from the 5'-end are 2'-fluoro (2'-F) modified and the remaining RNA nucleotides are 2'-O-methyl (2'-OMe) modified. |

126

EP 4 745 242 A1

| | Molecule | Single strand | Sequence | Modification or remarks |
|---|---|---|---|---|
| 44 | Apt PD-1-Apt AS1411-Apt PD-L1-3*Ami R-21-DNA carrier | strand a | SEQ ID No. 445:<br><br>AGCGGTGACGGCACAGACGG TACAGTTCCCGTCCCTGCACT ACACGTATGCCGCTGATAAGC TGCGACGCCCACGAGCGTTCC GGGAGAGGAGCT | The phosphodiester bonds between the 1st to 3rd nucleotides counted from the 5'-end, the first phosphodiester bond from the 3'-end, and the phosphodiester bonds within the internal AmiR-21 GATAAGCT sequence are phosphorothioate-modified. |
| | | strand b | SEQ ID No. 446:<br>GGTGGTGGTGGTTGTGGTGGT GGTGGGATAAGCTGCTCCTCT CCCGGTTCGCCGCGAGCCGCG T | The first phosphodiester bond from each of the 5'- and 3'-termini and the phosphodiester bonds within the internal AmiR-21 GATAAGCT sequence are phosphorothioate-modified. |
| | | strand c | SEQ ID No. 440:<br>ACGGGCCACATCAACTCATTG ATAGACAATGCGTCCACTGCC CGTGATAAGCTCGCGGCTCGC GGCCATAGCCGTGGGCGTCGC T | The phosphodiester bonds between the 1st to 3rd nucleotides counted from the 5'-end, the first phosphodiester bond from the 3'-end, and the phosphodiester bonds within the internal AmiR-21 GATAAGCT sequence are phosphorothioate-modified; in the RNA region, C/U are 2'-fluoro (2'-F) modified and A/G are 2'-O-methyl (2'-OMe) modified. |
| 45 | CPG1826-Apt CD40-AmiR-21-3*Bio-DN A | strand a | SEQ ID No. 447:<br>TCCATGACGTTCCTGACGTTT TTGCGGCGCCCACGAGCGTTC CGGGAGC | In the 5'-end CpG1826, all phosphodiester bonds between the 1st to 17th nucleotides counted from the 5'-end are phosphorothioate-modified, and the 3'-end is biotin (H) modified. |
| | | strand b | SEQ ID No. 399:<br>CCAACGAGTAGGCGATAGCG CGTGGTTTTTGCTCCCGGTTC | 5'-end CD40 aptamer, and the 3'-end is biotin (H) modified. |
| | | | GCCGCCAGCCGCC | |
| | | strand c | SEO ID No. 392:<br>GATAAGCTGGCGGCAGGCGG CCATAGCCGTGGGCGCCGC | The 5'-end GATAAGCT sequence is locked nucleic acid (LNA) modified, and the 3'-end is biotin (H) modified. |

127

EP 4 745 242 A1

| | Molecule | Single strand | Sequence | Modification or remarks |
|---|---|---|---|---|
| 46 | Apt ATP-AmiR-2 1-Apt FGF2-AmiR-1306-DNA carrier | strand a | SEO ID No. 448:<br><br>GGGAGGACGATGCGGAGGAA GGGTAGGTTTTTGCGACGCCC ACGAGCGTTCCGGGAGAGGA GC | The phosphodiester bonds between the 1st to 3rd nucleotides counted from the 5'-end are phosphorothioate-modified. |
| | | strand b | SEO ID No. 449:<br><br>GGGAUACUAGGGCAUUAAUG UUACCAGUGUAGUCCCUGCT CCTCTCCCGGTTCGCCGCGAG CCGCG | In the RNA sequence, C/U are 2'-fluoro (2'-F) modified. |
| | | strand c | SEQ ID No. 450:<br><br>CATCACCACCAGAGCCAACGT CGATAAGCTCGCGGCTCGCGG CCATAGCCGTGGGCGTCGC | The phosphodiester bonds between the 1st to 4th nucleotides counted from the 5'-end are phosphorothioate-modified, and the internal GATAAGCT sequence is locked nucleic acid (LNA) modified. |
| 47 | Apt CD16a-Apt CTLA-4-Apt PD-L1-DNA carrier | strand a | SEQ ID No. 374:<br><br>CCACTGCGGGGGTCTATACGT GAGGAAGAAGTGGTTTTTGCG ACGCCCACGAGCGTTCCGGGA GAGGAGCT | The first phosphodiester bond from each of the 5'- and 3'-termini is phosphorothioate-modified. |
| | | strand b | SEQ ID No. 362:<br><br>GGUGGAAGAGGGAUGGGCCG ACGUGCCGCAUTTTTTGCTCC TCTCCCGGTTCGCCGCGAGCC GCGT | RNA C/U 2'-F, A/G 2'-OMe; the first phosphodiester bond from each of the 5'- and 3'-termini is phosphorothioate-modified. |
| | | strand c | SEO ID No. 363:<br><br>ACGGGCCACATCAACTCATTC ATAGACAATGCGTCCACTGCC CGTTTTTTCGCGGCTCGCGGC TCATAGCCGTGGGCGTCGCT | The first phosphodiester bond from each of the 5'- and 3'-termini is phosphorothioate-modified. |

| | Molecule | Single strand | Sequence | Modification or remarks |
|---|---|---|---|---|
| 48 | Apt CD16a-Apt Act-12c-Apt PD-L1-DNA carrier | strand a | SEQ ID No. 374:<br>CCACTGCGGGGGTCTATACGT GAGGAAGAAGTGGTTTTTGCG ACGCCCACGAGCGTTCCGGGA GAGGAGCT | The first phosphodiester bond from each of the 5'- and 3'-termini is phosphorothioate-modified. |
| | | strand b | SEQ ID No. 451:<br>CGGGGAAAGTCACGGGGGGT TTCAGATGTTCTGATCGGTGT GGAGTTTTTGCTCCTCTCCCG GTTCGCCGCGAGCCGCGT | The first phosphodiester bond from each of the 5'- and 3'-termini is phosphorothioate-modified. |
| | | strand c | SEQ ID No. 363:<br>ACGGGCCACATCAACTCATTG ATAGACAATGCGTCCACTGCC CGTTTTTTCGCGGCTCGCGGC TCATAGCCGTGGGCGTCGCT | The first phosphodiester bond from each of the 5'- and 3'-termini is phosphorothioate-modified. |
| 49 | Apt CTLA-4-Apt PD-L1-Apt PD-1-DNA carrier. | strand a | SEQ ID No. 15:<br>GACGCCCACGAGCGTTCCGGG AGAGGTGTAGCACGGTGGC | |
| | | strand b | (PD-L1) SEQ ID No. 378:<br>ACGGGCCACATCAACTCATTG ATAGACAATGCGTCCACTGCC CGTTTTTTGCCACCGTGCTAC A | The phosphodiester bonds between the 1st to 2nd nucleotides counted from the 5'-end are phosphorothioate-modified. |
| | | strand c | SEQ ID No. 16:<br>CCTCTCCCGGTTCGCCGCGAG CCTCGGCGCGGCCGTG | |
| | | strand d | (PD-1) SEQ ID No. 307:<br>ACCGACAGTGAAGGACTCAG CGAACTCTCAGACTCGGTTCT | The phosphodiester bonds between the 1st to 2nd nucleotides counted from the 5'-end are phosphorothioate-modified. |

(continued)

| | Molecule | Single strand | Sequence | Modification or remarks |
|---|---|---|---|---|
| | | | TTTTCACGGCCGCGCCGA | |
| | | strand e | SEQ ID No. 17:<br>GGCTCGCGGCCATAGCCGTGG GCGTCTGCTGCTGCTGCTG | |
| | | strand f | (CTLA4) SEQ ID No. 452:<br>GGUGGAAGAGGGAUGGGCCG ACGUGCCGCAUCAGCAGCAG CAGCA | The phosphodiester bonds between the 1st to 2nd nucleotides counted from each of the 5'- and 3'-termini are phosphorothioate-modified; in the RNA sequence, the first and second nucleotides from each of the 5'- and 3'-termini are 2'-O-methoxyethyl (2'-O-MOE) modified, and the remaining C/U nucleotides are 2'-fluoro (2'-F) modified. |
| 50 | Apt PD-1-Apt IL-4Ra-Apt OX40-DNA carrier | strand a | SEQ ID No. 453:<br>AGCGGTGACGGCACAGACGG TACAGTTCCCGTCCCTGCACT ACACGTATGCCGCTAAAGCGA CGCCCACGAGCGTTCCGGGAG AGGAGC | The phosphodiester bonds between the 1st to 3rd nucleotides counted from the 5'-end and the first and second phosphodiester bonds from the 3'-end are phosphorothioate-modified; the nucleotides at positions 1 to 4 from the 5'-end and at positions 1 and 2 from the 3'-end are 2'-O-methoxyethyl (2'-O-MOE) modified. |
| | | strand b | SEQ ID No. 454:<br>AAAAAGCAACAGGGUGCUCC AUGCGCAUGGAACCUGCGCG AAAGCTCCTCTCCCGGTTCGC CGCGAGCCGCG | The phosphodiester bonds between the 1st to 3rd nucleotides counted from the 5'-end and the first and second phosphodiester bonds from the 3'-end are phosphorothioate-modified; the nucleotides at positions 1, 3, 13, 16 and 24 from the 5'-end and the first two nucleotides from the 3'-end are 2'-O-MOE modified. |
| | | strand c | SEQ ID No. 455:<br>CAGUCUGCAUCGUAGGAAUC GCCACCGUAUACUUUCCCAC AAACGCGGCTCGCGGCCATA GCCGTGGGCGTCGC | RNA with C/U 2'-fluoro (2'-F) and A/G 2'-O-methyl (2'-OMe); the phosphodiester bonds between the 1st to 3rd nucleotides counted from the 5'-end and the first and second phosphodiester bonds from the 3'-end are phosphorothioate-modified; the nucleotides at positions 1 to 3 from the 5'-end and at positions 1 and 2 from the 3'-end are 2'-O-MOE modified. |

| | Molecule | Single strand | Sequence | Modification or remarks |
|---|---|---|---|---|
| 51 | Apt CD16a-Apt OX40-Apt PD-L1-DNA carrier | strand a | SEO ID No. 374:<br>CCACTGCGGGGGTCTATACGT GAGGAAGAAGTGGTTTTTGCG ACGCCCACGAGCGTTCCGGGA GAGGAGCT | The first and second phosphodiester bonds from each of the 5'- and 3'-termini are phosphorothioate-modified. |
| | | strand b | SEQ ID No. 456:<br>GGGAUGCGGAAAAAAGAACA CUUCCGAUUAGGGCCCACCC UAACGGCCGCAGACTTTTTGC TCCTCTCCCGGTTCGCCGCGA GCCGCG*T | RNA with C/U 2'-F and A/G 2'-O-methyl (2'-OMe); the first phosphodiester bond from the 3'-end is phosphorothioate-modified. |
| | | strand c | SEQ ID No. 457:<br>ACGGGCCACATCAACTCATTG ATAGACAATGCGTCCACTGCC CGTTTTTTCGCGGCTCGCGGC CATAGCCGTGGGCGTCGCT | The first phosphodiester bond from each of the 5'- and 3'-termini is phosphorothioate-modified. |
| 52 | Apt CD16a-Apt VEGF165-Ap t PD-L1-DNA carrier | strand a | SEQ ID No. 374:<br>CCACTGCGGGGGTCTATACGT GAGGAAGAAGTGGTTTTTGCG ACGCCCACGAGCGTTCCGGGA GAGGAGCT | The first phosphodiester bond from each of the 5'- and 3'-termini is phosphorothioate-modified. |
| | | strand b | SEQ ID No. 458:<br>CGGAAUCAGUGAAUGCUUAU ACAUCCGTTTTTGCTCCTCTCC | RNA with C/U 2'-fluoro (2'-F) and A/G 2'-O-methyl (2'-OMe); the first phosphodiester bond from the 3'-end is |
| | | | CGGTTCGCCGCGAGCCGCGT | phosphorothioate-modified. |
| | | strand c | SEQ ID No. 457:<br>ACGGGCCACATCAACTCATTG ATAGACAATGCGTCCACTGCC CGTTTTTTCGCGGCTCGCGGC CATAGCCGTGGGCGTCGCT | The first phosphodiester bond from each of the 5'- and 3'-termini is phosphorothioate-modified. |

EP 4 745 242 A1

| | Molecule | Single strand | Sequence | Modification or remarks |
|---|---|---|---|---|
| 53 | Apt CD16a-Apt CTLA4-Apt TIM3-DNA carrier | strand a | SEQ ID No. 374:<br><br>CCACTGCGGGGGTCTATACGT GAGGAAGAAGTGGTTTTTGCG ACGCCCACGAGCGTTCCGGGA GAGGAGCT | The first phosphodiester bond from each of the 5'- and 3'-termini is phosphorothioate-modified. |
| | | strand b | SEQ ID No. 362:<br><br>GGUGGAAGAGGGAUGGGCCG ACGUGCCGCAUTTTTTGCTCC TCTCCCGGTTCGCCGCGAGCC GCGT | The first phosphodiester bond from each of the 5'- and 3'-termini is phosphorothioate-modified; RNA with C/U 2'-fluoro (2'-F) and A/G 2'-O-methyl (2'-OMe). |
| | | strand c | SEQ ID No. 459:<br><br>GGGAGAGGACCAGUAGCCAC UAUGGUGUUGGAGCUAGCGG CAGAGCGUCGCGGUCCCUCC CTTTTTCGCGGCTCGCGGCCA TAGCCGTGGGCGTCGCT | RNA with C/U 2'-fluoro (2'-F) and A/G 2'-O-methyl (2'-OMe); the first phosphodiester bond from each of the 5'- and 3'-termini is phosphorothioate-modified. |
| 54 | Apt PD-1-Apt CTLA-4-Apt LAG-3-DNA carrier | strand a | SEQ ID No. 460:<br><br>AGCGGTGACGGCACAGACGG TACAGTTCCCGTCCCTGCACT ACACGTATGCCGCTTTTTTGC GACGCCCACGAGCGTTCCGGG AGAGGAGCT | The first phosphodiester bond from each of the 5'- and 3'-termini is phosphorothioate-modified. |
| | | strand b | SEO ID No. 362:<br><br>GGUGGAAGAGGGAUGGGCCG ACGUGCCGCAUTTTTTGCTCC TCTCCCGGTTCGCCGCGAGCC GCGT | RNA with C/U 2'-fluoro (2'-F) and A/G 2'-O-methyl (2'-OMe); the first phosphodiester bond from each of the 5'- and 3'-termini is phosphorothioate-modified. |
| | | strand c | SEQ ID No. 461:<br><br>GGGAGAGAGAUAUAAGGGCC UCCUGAUACCCGCUGCUAUC UGGACCGAUCCCAUUACCAA AUUCUCUCCCTTTTTCGCGGC TCGCGGCCATAGCCGTGGGCG TCGCT | RNA with C/U 2'-fluoro (2'-F) and A/G 2'-O-methyl (2'-OMe); the first phosphodiester bond from each of the 5'- and 3'-termini is phosphorothioate-modified. |

| | Molecule | Single strand | Sequence | Modification or remarks |
|---|---|---|---|---|
| 55 | Apt CD16a-Apt Act-12c-Apt MUC1 (5TR1)-DNA carrier | strand a | SEQ ID No. 374:<br>CCACTGCGGGGGTCTATACGT GAGGAAGAAGTGGTTTTTGCG ACGCCCACGAGCGTTCCGGGA GAGGAGCT | The first phosphodiester bond from each of the 5'- and 3'-termini is phosphorothioate-modified. |
| | | strand b | SEQ ID No. 451:<br>CGGGGAAAGTCACGGGGGGT TTCAGATGTTCTGATCGGTGT GGAGTTTTTGCTCCTCTCCCG GTTCGCCGCGAGCCGCGT | The first phosphodiester bond from each of the 5'- and 3'-termini is phosphorothioate-modified. |
| | | strand c | SEQ ID No. 462:<br>GAAGTGAAAATGACAGAACA CAACATTTTTCGCGGCTCGCG GCCATAGCCGTGGGCGTCGCT | The first phosphodiester bond from the 3'-end is phosphorothioate-modified. |
| 56 | Apt CD16a-Apt TGF-β-Apt PD-L1-DNA carrier | strand a | SEQ ID No. 374:<br>CCACTGCGGGGGTCTATACGT GAGGAAGAAGTGGTTTTTGCG ACGCCCACGAGCGTTCCGGGA GAGGAGCT | The first phosphodiester bond from each of the 5'- and 3'-termini is phosphorothioate-modified. |
| | | strand b | SEQ ID No. 463:<br>ACATTGCTGCGTGATCGCCTC ACATGGGTTTGTCTGGTCGAT TTGGAGGTGGTGGGTGGCTTT TTGCTCCTCTCCCGGTTCGCC | The first phosphodiester bond from each of the 5'- and 3'-termini is phosphorothioate-modified. |
| | | | GCGAGCCGCGT | |
| | | strand c | SEQ ID No. 457:<br>ACGGGCCACATCAACTCATTG ATAGACAATGCGTCCACTGCC CGTTTTTTCGCGGCTCGCGGC CATAGCCGTGGGCGTCGCT | The first phosphodiester bond from each of the 5'- and 3'-termini is phosphorothioate-modified. |

| | Molecule | Single strand | Sequence | Modification or remarks |
|---|---|---|---|---|
| 57 | Apt CD16a-Apt CD40-Apt CTLA-4-DN A carrier | strand a | SEO ID No. 374:<br><br>CCACTGCGGGGGGTCTATACGT GAGGAAGAAGTGGTTTTTGCG ACGCCCACGAGCGTTCCGGGA GAGGAGCT | The first phosphodiester bond from each of the 5'- and 3'-termini is phosphorothioate-modified. |
| | | strand b | SEO ID No. 464:<br><br>CCAACGAGTAGGCGATAGCG CGTGGTTTTTGCTCCTCTCCC( GTTCGCCGCGAGCCGCGT | The first phosphodiester bond from each of the 5'- and 3'-termini is phosphorothioate-modified. |
| | | strand c | SEO ID No. 465:<br><br>GGUGGAAGAGGGAUGGGCCG ACGUGCCGCAUTTTTTCGCGG CTCGCGGCCATAGCCGTGGGC GTCGCT | RNA with C/U 2'-fluoro (2'-F) and A/G 2'-O-methyl (2'-OMe); the first phosphodiester bond from each of the 5'- and 3'-termini is phosphorothioate-modified. |
| 58 | Apt HER2-Apt HER3-Apt CD40-Apt CD16a-4-DN A carrier | strand a | SEQ ID No. 466:<br><br>CAGCGAAAGUUGCGUAUGGG UCACAUCGCAGGCACAUGUC AUCUGGGCGAAGCGACGCCC ACGAGCGTTCCGGGAGAGGA GCT | RNA with C/U 2'-fluoro (2'-F) and A/G 2'-O-methyl (2'-OMe); the first phosphodiester bond from each of the 5'- and 3'-termini is phosphorothioate-modified. |
| | | strand b | SEQ ID No. 467:<br><br>CCAACGAGTAGGCGATAGCG CGTGGAAGCTCCTCTCCCGGT TCGCCGCGAGCCGCGAACCAC TGCGGGGGTCTATACGTGAGG AAGAAGTGG | The first phosphodiester bond from each of the 5'- and 3'-termini is phosphorothioate-modified. |
| | | strand c | SEQ ID No. 468:<br><br>AGCCGCGAGGGGAGGGAUAG GGUAGGGCGCGGCUAACGCG GCTCGCGGCCATAGCCGTGGG CGTCGCT | RNA with C/U 2'-fluoro (2'-F) and A/G 2'-O-methyl (2'-OMe); the first phosphodiester bond from each of the 5'- and 3'-termini is phosphorothioate-modified. |

134

EP 4 745 242 A1

| | Molecule | Single strand | Sequence | Modification or remarks |
|---|---|---|---|---|
| 59 | Apt CD16a-Apt Act-12c-Apt CTLA-4-Apt GPC1-DNA carrier | strand a | SEQ ID No. 374:<br>CCACTGCGGGGGTCTATACGT GAGGAAGAAGTGGTTTTTGCG ACGCCCACGAGCGTTCCGGGA GAGGAGCT | The first phosphodiester bond from each of the 5'- and 3'-termini is phosphorothioate-modified. |
| | | strand b | SEO ID No. 451:<br>CGGGGAAAGTCACGGGGGGT TTCAGATGTTCTGATCGGTGT GGAGTTTTTGCTCCTCTCCCG GTTCGCCGCGAGCCGCGT | The first phosphodiester bond from each of the 5'- and 3'-termini is phosphorothioate-modified. |
| | | strand c | SEQ ID No. 469:<br>GGUGGAAGAGGGAUGGGCCG ACGUGCCGCAUTTTCGCGGCT CGCGGCCATAGCCGTGGGCGT CGCTTTAACGGAGTGTGGCTA ACTCGA | RNA with C/U 2'-fluoro (2'-F) and A/G 2'-O-methyl (2'-OMe); the first phosphodiester bond from each of the 5'- and 3'-termini is phosphorothioate-modified. |
| 60 | Apt CD16a-Apt Her3-Apt VEGF165-D NA carrier | strand a | SEQ ID No. 374:<br>CCACTGCGGGGGTCTATACGT GAGGAAGAAGTGGTTTTTGCG ACGCCCACGAGCGTTCCGGGA GAGGAGCT | The first phosphodiester bond from each of the 5'- and 3'-termini is phosphorothioate-modified. |
| | | strand b | SEQ ID No. 470:<br>CAGCGAAAGUUGCGUAUGGG UCACAUCGCAGGCACAUGUC AUCUGGGCGTTTTTGCTCCTC TCCCGGTTCGCCGCGAGCCGC GT | The first phosphodiester bond from each of the 5'- and 3'-termini is phosphorothioate-modified; in the RNA region, C/U are 2'-fluoro (2'-F) modified and A/G are 2'-O-methyl (2'-OMe) modified. |
| | | strand c | SEQ ID No. 471:<br>CGGAAUCAGUGAAUGCUUAU ACAUCCGTTTTTCGCGGCTCG CGGCCATAGCCGTGGGCGTCG CT | The first phosphodiester bond from each of the 5'- and 3'-termini is phosphorothioate-modified; in the RNA region, C/U are 2'-fluoro (2'-F) modified and A/G are 2'-O-methyl (2'-OMe) modified. |

(continued)

| | Molecule | Single strand | Sequence | Modification or remarks |
|---|---|---|---|---|
| 61 | Apt VEGF-Apt GPC1-Apt PD-L1-Apt CD16a-DNA carrier | strand a | SEQ ID No. 472:<br>GGTGGGGGTGGACGGGGCCGG GTAGATTTTTGCGACGCCCA CGAGCGTTCCGGGAGAGGAG CTTTTTAACGGAGTGTGGCT AACTCGA | The first phosphodiester bond from each of the 5'- and 3'-termini is phosphorothioate-modified. |
| | | strand b | SEQ ID No. 473:<br>ACGGGCCACATCAACTCATTG ATAGACAATGCGTCCACTGCC CGTTTTTTGCCTCCTCTCCGGT TCGCCGCGAGCCGCGT | The first phosphodiester bond from each of the 5'- and 3'-termini is phosphorothioate-modified. |
| | | strand c | SEQ ID No. 474:<br>CCACTGCTGCGGGGTCTATACGT GAGGAAGAAGTGGTTTTTCGC GGCTCGCGGCCATAGCCGGTGG GCGTCGCT | The first phosphodiester bond from each of the 5'- and 3'-termini is phosphorothioate-modified, and the phosphodiester bond between the GG immediately upstream of "TTTTT" is phosphorothioate-modified. |
| | | strand a | SEQ ID No. 445:<br>AGCGGTGACGGCACAGACGG TACAGTTCCGTCCCTGCACT ACACGTATGCCGCTGATAAGC TGCGACGCCCACGAGCGTTCC GGGAGAGGAGCT | The phosphodiester bonds between the 1st to 3rd nucleotides counted from the 5'-end, the first phosphodiester bond from the 3'-end, and the phosphodiester bonds within the internal GATAAGCT sequence are phosphorothioate-modified. |
| 62 | Apt PD-1-Apt PD-L1-2*Ami R-21-Apt Act-12c-DNA carrier | strand b | SEQ ID No. 475:<br>ACGGGCCACATCAACTCATTG ATAGACAATGCGTCCACTGCC CGTGATAAGCTGCTCCTCTCC CGGTTCGCCGCGAGCCGCGT | The phosphodiester bonds between the 1st to 3rd nucleotides counted from the 5'-end, the first phosphodiester bond from the 3'-end, and the phosphodiester bonds within the internal GATAAGCT sequence are phosphorothioate-modified. |
| | | strand c | SEQ ID No. 476:<br>CGGGGAAAGTCACGGGGGGT TTCAGATGTTCTGATCGGTGT GGAGTTTTCGCGGCTCGCGG CCATAGCCGGTGGGCGTCGCT | The phosphodiester bonds between the 1st to 3rd nucleotides counted from the 5'-end and the first phosphodiester bond from the 3'-end are phosphorothioate-modified. |

| | Molecule | Single strand | Sequence | Modification or remarks |
|---|---|---|---|---|
| 63 | Apt Her3-EGFR siRNA-Apt AS1411-Survi vin siRNA-Apt Her2-DNA carrier | strand a | SEQ ID No. 477: CAGCGAAAGUUGCGUAUGGG UCACAUCGCAGGCACAUGUC AUCUGGGCGAAAAUUAGAUA AGACUGCUAAGGCA | The phosphodiester bonds between the 1st to 2nd nucleotides counted from the 5'-end are phosphorothioate-modified; in the 3'-end RNA sequence "AAUUAGAUAAGACUGCUAAGGCA ", the first and second phosphodiester bonds from each of the 5'- and 3'-termini are phosphorothioate-modified. |
| | | strand b | SEQ ID No. 478: GCGGCGCCCACGAGCGTTCCG GGAGAGGCCAAAAGGTGGTG GTGGTTGTGGTGGTGGTGG | The first phosphodiester bond from each of the 5'- and 3'-termini is phosphorothioate-modified. |
| | | strand c | SEQ ID No. 479: GGCCTCTCCCGGTTCGCCGCC AGCCGCCAAAAGCAGGUUCC UUAUCUGUCACA | The first phosphodiester bond from the 5'-end is phosphorothioate-modified. |
| | | strand d | SEQ ID No. 480: AGCCGCGAGGGGAGGGAUAG GGUAGGGCGCGGCUAAUGUG ACAGAUAAGGAACCUGCAG | The first phosphodiester bond from the 5'-end is phosphorothioate-modified; in the 3'-end RNA sequence "UGUGACAGAUAAGGAACCUGCAG ", the first and second phosphodiester bonds from each of the 5'- and 3'-termini are phosphorothioate-modified. |
| | | strand e | SEQ ID No. 481: GGCGGCTGGCGGCCATAGCC GTGGGCGCCGCAAAACCUUA GCAGUCUUAUCUAAUU | The first phosphodiester bond from the 5'-end is phosphorothioate-modified; in the RNA sequence downstream of "AAAA", the first and second phosphodiester bonds from each of the 5'- and 3'-termini are phosphorothioate-modified. |

| | Molecule | Single strand | Sequence | Modification or remarks |
|---|---|---|---|---|
| 64 | Survivin siRNA-Apt TFRA3-Apt AS1411-3*A miR-21-DNA carrier | strand a | SEQ ID No. 482:<br>GCGTGGTCACACGCGGATAA GCTCGACGCCCACGAGCGTTC CGGGAGAGGAG | 5'-end TFRA3 aptamer, and the 3'-end is folic acid (FA) modified. |
| | | strand b | SEQ ID No. 483:<br>GGTGGTGGTGGTTGTGGTGGT GGTGGGATAAGCTCTCCTCTC CCGGTTCGCCGCCAGCCGCC | 5'-end AS1411 aptamer. |
| | | strand c | SEQ ID No. 305:<br>UGACAGAUAAGGAACCUGCA GUU | The first and second phosphodiester bonds from each of the 5'- and 3'-termini are phosphorothioate-modified; the nucleotides at positions 2, 6, 8, 9, 12, 14 and 16 counted from the 5'-end are 2'-fluoro (2'-F) modified, and the remaining nucleotides are 2'-O-methyl (2'-OMe) modified. |
| | | strand d | SEQ ID No. 484:<br>GCAGGUUCCUUAUCUGUCAG ATAAGCTGGCGGCAGGCGGC CATAGCCGTGGGCGCCGC | The first and second phosphodiester bonds from each of the 5'- and 3'-termini are phosphorothioate-modified; the nucleotides at positions 7, 9, 10 and 11 counted from the 5'-end are 2'-fluoro (2'-F) modified, and the remaining RNA nucleotides are 2'-O-methyl (2'-OMe) modified. |

(continued)

| | Molecule | Single strand | Sequence | Modification or remarks |
|---|---|---|---|---|
| 65 | Apt AS1411-EGF R siRNA-Apt VEGF165-Ap t PD-L1-RNA carrier | strand a | SEO ID No. 485:<br><br>GGTGGTGGTGGTTGTGGTGGT GGTGGAAAAUUAGAUAAGAC UGCUAAGGCA | The first phosphodiester bond from the 5'-end is phosphorothioate-modified; in the RNA sequence "AAUUAGAUAAGACUGCUAAGGCA ", the first and second phosphodiester bonds from each of the 5'- and 3'-termini are phosphorothioate-modified; in this RNA sequence, the 2nd, 6th, 8th, 9th, 12th, 14th and 16th nucleotides counted from the 5'-end are 2'-fluoro (2'-F) modified and the remaining nucleotides are 2'-O-methyl (2'-OMe) modified. |
| | | strand b | SEQ ID No. 486:<br><br>GCGGCGCCCACGAGCGUUCC GGGAGAGGCCAACGGAAUCA GUGAAUGCUUAUACAUCCG | RNA with C/U 2'-fluoro (2'-F) and A/G 2'-O-methyl (2'-OMe); the first phosphodiester bond from the 5'-end is phosphorothioate-modified. |
| | | strand c | SEQ ID No. 487:<br>GGCCUCUCCCGGUUCGCCGC CAGCCGCCAAACGGGCCACA TCAACTCATTGATAGACAATG CGTCCACTGCCCGT | RNA with C/U 2'-fluoro (2'-F) and A/G 2'-O-methyl (2'-OMe); the first and second phosphodiester bonds from each of the 5'- and 3'-termini are phosphorothioate-modified. |
| | | strand d | SEQ ID No. 488:<br>GGCGGCUGGCGGCCAUAGCC GUGGGCGCCGCAACCUUAGC AGUCUUAUCUAAUU | The first and second phosphodiester bonds from the 3'-end are phosphorothioate-modified; in the RNA sequence, the 7th, 9th, 10th and 11th nucleotides counted from the 5'-end are 2'-fluoro (2'-F) modified and the remaining RNA nucleotides are 2'-O-methyl (2'-OMe) modified. |
| 66 | Apt CEA-Apt CD16a-Apt Act-12c-Apt PD-L1-DNA carrier | strand a | SEQ ID No. 489:<br>TTAACTTATTCGACCATAAAG CGGCGCCCACGAGCGTTCCGG GAGAGGCCAACCACTGCGGG GGTCTATACGTGAGGAAGAA | The first phosphodiester bond from each of the 5'- and 3'-termini is phosphorothioate-modified. |

(continued)

| | Molecule | Single strand | Sequence | Modification or remarks |
|---|---|---|---|---|
| | | | GTGG | |
| | | strand b | SEQ ID No. 490:<br><br>CGGGGAAAGTCACGGGGGGT TTCAGATGTTCTGATCGGTGT GGAGAAGGCCTCTCCCGGTTC GCCGCCAGCCGCCT | The first phosphodiester bond from each of the 5'- and 3'-termini is phosphorothioate-modified. |
| | | strand c | SEQ ID No. 491:<br><br>ACGGGCCACATCAACTCATTG ATAGACAATGCGTCCACTGCC CGTAAGGCGGCTGGCGGCCAT AGCCGTGGGCGCCGCT | The first phosphodiester bond from each of the 5'- and 3'-termini is phosphorothioate-modified. |
| 67 | Apt AS1411-TAP siRNA-Apt CD16a-Apt CTLA-4-DN A carrier | strand a | SEQ ID No. 492:<br><br>GGTGGTGGTGGTTGTGGTGGT GGTGGAAAAUUCUGAACCGU GUGCAGCUU | The first phosphodiester bond from the 5'-end is phosphorothioate-modified; in the RNA sequence downstream of "AAAA", the first and second phosphodiester bonds from each of the 5'- and 3'-termini are phosphorothioate-modified; in this RNA sequence, the 2nd, 6th, 8th, 9th, 12th, 14th and 16th nucleotides counted from the 5'-end are 2'-fluoro (2'-F) modified and the remaining nucleotides are 2'-O-methyl (2'-OMe) modified. |
| | | strand b | SEO ID No. 493:<br><br>GCGGCGCCCACGAGCGTTCCG GGAGAGGCCAAAACCACTGC GGGGGTCTATACGTGAGGAA GAAGTGG | The first phosphodiester bond from each of the 5'- and 3'-termini and the phosphodiester bond at the 35th position counted from the 5'-end are phosphorothioate-modified. |
| | | strand c | SEQ ID No. 494:<br><br>GGCCTCTCCCGGTTCGCCGCC AGCCGCCAAAAGGUGGAAGA GGGAUGGGCCGACGUGCCGC AU | In the RNA sequence downstream of "AAAA", C/U are 2'-fluoro (2'-F) modified and A/G are 2'-O-methyl (2'-OMe) modified; the first phosphodiester bond from each of the 5'- and 3'-termini is phosphorothioate-modified. |
| | | strand d | SEO ID No. 495:<br><br>GGCGGCTGGCGGCCATAGCC GTGGGCGCCGCAAAAGCUGC ACACGGUUCAGAAU | The first phosphodiester bond from the 5'-end is phosphorothioate-modified; in the RNA sequence downstream of "AAAA", the first and second phosphodiester bonds from each of the 5'- and 3'-termini are phosphorothioate-modified; in this RNA sequence, the 7th, 9th, 10th and 11th nucleotides counted from the 5'-end are 2'-fluoro (2'-F) modified and the remaining RNA nucleotides are 2'-O-methyl (2'-OMe) modified. |

|  | Molecule | Single strand | Sequence | Modification or remarks |
|---|---|---|---|---|
| 68 | Apt AS1411-TAP siRNA-Apt CD16a-Apt Act-12c-DNA carrier | strand a | SEQ ID No. 492:<br><br>GGTGGTGGTGGTTGTGGTGGT GGTGGAAAAUUCUGAACCGU GUGCAGCUU | The first phosphodiester bond from the 5'-end is phosphorothioate-modified; in the RNA sequence downstream of "AAAA", the first and second phosphodiester bonds from each of the 5'- and 3'-termini are phosphorothioate-modified; in this RNA sequence, the 2nd, 6th, 8th, 9th, 12th, 14th and 16th nucleotides counted from the 5'-end are 2'-fluoro (2'-F) modified and the remaining nucleotides are 2'-O-methyl (2'-OMe) modified. |
|  |  | strand b | SEQ ID No. 493:<br>GCGGCGCCCACGAGCGTTCCG GGAGAGGCCAAAACCACTGC GGGGGTCTATACGTGAGGAA GAAGTGG | The first phosphodiester bond from each of the 5'- and 3'-termini is phosphorothioate-modified. |
|  |  | strand c | SEQ ID No. 496:<br>GGCCTCTCCCGGTTCGCCGCC AGCCGCCAAAACGGGGAAAA CGGGGAAAGTCACGGGGGGT TTCAGATGTTCTGATCGGTGT GGAG | The first phosphodiester bond from each of the 5'- and 3'-termini is phosphorothioate-modified. |
|  |  | strand d | SEQ ID No. 495:<br><br>GGCGGCTGGCGGCCATAGCC GTGGGCGCCGCAAAAGCUGC ACACGGUUCAGAAU | The first phosphodiester bond from the 5'-end is phosphorothioate-modified; in the RNA sequence downstream of "AAAA", the first and second phosphodiester bonds from each of the 5'- and 3'-termini are phosphorothioate-modified; in this RNA sequence, the 7th, 9th, 10th and 11th nucleotides counted from the 5'-end are 2'-fluoro (2'-F) modified and the remaining RNA nucleotides are 2'-O-methyl (2'-OMe) modified. |

EP 4 745 242 A1

| | Molecule | Single strand | Sequence | Modification or remarks |
|---|---|---|---|---|
| 69 | Apt AS1411-TAP siRNA-Apt OX40-Apt Act-12c-DNA carrier | strand a | SEQ ID No. 497:<br><br>CCACTGCGGGGGTCTATACGT GAGGAAGAAGTGGAAGCGGC GCCCACGAGCGTTCCGGGAG AGGCCAAGCUGCACACGGUU CAGAAU | In the RNA sequence "GCUGCACACGGUUCAGAAU", the first and second phosphodiester bonds from each of the 5'- and 3'-termini are phosphorothioate-modified; in this RNA sequence, the 7th, 9th, 10th and 11th nucleotides counted from the 5'-end are 2'-fluoro (2'-F) modified and the remaining RNA nucleotides are 2'-O-methyl (2'-OMe) modified. |
| | | strand b | SEO ID No. 492:<br><br>GGTGGTGGTGGTTGTGGTGGT GGTGGAAAAUUCUGAACCGU GUGCAGCUU | The first phosphodiester bond from the 5'-end is phosphorothioate-modified; in the RNA sequence downstream of "AAAA", the first and second phosphodiester bonds from each of the 5'- and 3'-termini are phosphorothioate-modified; in this RNA sequence, the 2nd, 6th, 8th, 9th, 12th, 14th and 16th nucleotides counted from the 5'-end are 2'-fluoro (2'-F) modified and the remaining nucleotides are 2'-O-methyl (2'-OMe) modified. |
| | | strand c | SEQ ID No. 498:<br><br>GGCCTCTCCCGGTTCGCCGCC AGCCGCCAAGGGAGGACGAU GCGGCAGUCUGCAUCGUAGG AAUCGCCACCGUAUACUUUC CCACCAGACGACUCGCUG | The first phosphodiester bond from each of the 5'- and 3'-termini is phosphorothioate-modified; in the RNA region, C/U are 2'-fluoro (2'-F) modified and A/G are 2'-O-methyl (2'-OMe) modified. |
| | | strand d | SEQ ID No. 499:<br><br>CGGGGAAAGTCACGGGGGGT TTCAGATGTTCTGATCGGTGT GGAGAAAAGGCGGCTGGCGG CCATAGCCGTGGGCGCCGC | The first phosphodiester bond from each of the 5'- and 3'-termini is phosphorothioate-modified. |

(continued)

|  | Molecule | Single strand | Sequence | Modification or remarks |
|---|---|---|---|---|
| 70 | Apt CTLA-4-Apt CD40-Apt FAP-DNA carrier | strand a | SEQ ID No. 500:<br><br>GGUGGAAGAGGGAUGGGCCG ACGUGCCGCAUTTTTTGCGGC GCCCACGAGCGTTCCGGGAG AGGCCT | The first phosphodiester bond from each of the 5'- and 3'-termini is phosphorothioate-modified; in the RNA sequence upstream of "TTTTT", C/U are 2'-fluoro (2'-F) modified and A/G are 2'-O-methyl (2'-OMe) modified. |
|  |  | strand b | SEQ ID No. 501:<br><br>CCAACGAGTAGGCGATAGCG CGTGGTTTTTGGCCTCTCCCG GTTCGCCGCCAGCCGCCT | The first phosphodiester bond from each of the 5'- and 3'-termini is phosphorothioate-modified. |
|  |  | strand c | SEO ID No. 416:<br><br>CCGCTCGAGCTAGTCTGACAA AGAGAAACACTTTTTGGCGGC | The first phosphodiester bond from each of the 5'- and 3'-termini is phosphorothioate-modified. |
|  |  |  | TGGCGGCCATAGCCGTGGGCG CCGCT |  |

| | Molecule | Single strand | Sequence | Modification or remarks |
|---|---|---|---|---|
| 71 | Apt VEGF165-AS AP1 siRNA-Apt CD24-Apt SARS-CoV-2 -N48-DNA carrier | strand a | SEQ ID No. 502:<br><br>CGGAAUCAGUGAAUGCUUAU ACAUCCGAAUGAUAUUAUGG AAGCAAAUUU | The first phosphodiester bond from the 5'-end is phosphorothioate-modified; for the first 29 nucleotides counted from the 5'-end, C/U are 2'-fluoro (2'-F) modified and A/G are 2'-O-methyl (2'-OMe) modified; from the 30th nucleotide counted from the 5'-end, the 2nd, 6th, 8th, 9th, 12th, 14th and 16th nucleotides are 2'-fluoro (2'-F) modified and the remaining nucleotides are 2'-O-methyl (2'-OMe) modified; furthermore, in the 3'-end RNA sequence "UGAUAUUAUGGAAGCAAAUUU", the first and second phosphodiester bonds from each of the 5'- and 3'-termini are phosphorothioate-modified. |
| | | strand b | SEQ ID No. 503:<br>GCGGCGCCCACGAGCGTTCC GGAGAGGCCAAGCTGGATGT CGCTTACGACAATATTCCTTA GGGGCACCGCTACATTGACA ATCCAGC C | The first phosphodiester bond from the 3'-end is phosphorothioate-modified. |
| | | strand c | SEQ ID No. 504:<br>GGCCTCTCCCGGTTCGCCGCC AGCCGCCAAATCCAGAGTGA CGCAGCATATGTGGGTGGGTG GGCGGTTATGCTGAGTCAGCC TTGCTTGGACACGGTGGCTTA GT | The first and the 32nd phosphodiester bonds counted from the 5'-end are phosphorothioate-modified. |
| | | strand d | SEQ ID No. 505:<br><br>GGCGGCTGGCGGCCATAGCC GTGGGCGCCGCAAAUUUGCU UCCAUAAUAUCAUU | The first and second phosphodiester bonds from the 5'-end are phosphorothioate-modified; in the 3'-end RNA sequence "AUUUGCUUCCAUAAUAUCAUU", the first and second phosphodiester bonds from each of the 5'- and 3'-termini are phosphorothioate-modified; in this RNA sequence, the 7th, 9th, 10th and 11th nucleotides counted from the 5'-end are 2'-fluoro (2'-F) modified and the remaining RNA nucleotides are 2'-O-methyl (2'-OMe) modified. |

| | Molecule | Single strand | Sequence | Modification or remarks |
|---|---|---|---|---|
| 72 | 3*Apt FGF5-DNA carrier | strand a | SEQ ID No. 506:<br><br>GGGCGACCUCUCCGUACUGA CCUACAGAGCGACAUACUAG UGUAUCCAGAUCGCCCUGCG GCGCCCACGAGCGUUCCGGGA GAGGCCT | RNA with C/U 2'-fluoro (2'-F) and A/G 2'-O-methyl (2'-OMe); the first phosphodiester bond from each of the 5'- and 3'-termini is phosphorothioate-modified. |
| | | strand b | SEQ ID No. 507:<br><br>GGCGACCUCUCCGUACUGAC CUACAGAGCGACAUACUAGU GUAUCCAGAUCGCCCUGGCC TCTCCCGGTTCGCCGCCAGCC GCCT | RNA with C/U 2'-fluoro (2'-F) and A/G 2'-O-methyl (2'-OMe); the first phosphodiester bond from each of the 5'- and 3'-termini is phosphorothioate-modified. |
| | | strand c | SEQ ID No. 508:<br><br>GGGCGACCUCUCCGUACUGA CCUACAGAGCGACAUACUAG UGUAUCCAGAUCGCCCUGGC GGCTGGCGGCCATAGCCGTGG GCGCCGCT | RNA with C/U 2'-fluoro (2'-F) and A/G 2'-O-methyl (2'-OMe); the first phosphodiester bond from each of the 5'- and 3'-termini is phosphorothioate-modified. |
| 73 | Apt AS1411-ASAP | strand a | SEQ ID No. 509:<br><br>GGTGGTGGTGGTTGTGGTGGT | The first phosphodiester bond from the 5'-end is phosphorothioate-modified; in |

145

EP 4 745 242 A1

| | Molecule | Single strand | Sequence | Modification or remarks |
|---|---|---|---|---|
| | 1 siRNA-Apt VEGF165-Apt SARS-CoV-2-N 48-RNA carrier | | GGTGGAAUGAUAUUAUGGAA GCAAAUUU | the 3'-end RNA sequence "UGAUAUUAUGGAAGCAAAUUU", the first and second phosphodiester bonds from each of the 5'- and 3'-termini are phosphorothioate-modified; in this RNA sequence, the 2nd, 6th, 8th, 9th, 12th, 14th and 16th nucleotides counted from the 5'-end are 2'-fluoro (2'-F) modified and the remaining nucleotides are 2'-O-methyl (2'-OMe) modified. |
| | | strand b | SEQ ID No. 510: GCGGCGCCCACGAGCGTTCCG GGAGAGGCCAACGGAAUCAG UGAAUGCUUAUACAUCCG | The first and second phosphodiester bonds from each of the 5'- and 3'-termini are phosphorothioate-modified; in the RNA sequence, C/U are 2'-fluoro (2'-F) modified and A/G are 2'-O-methyl (2'-OMe) modified. |
| | | strand c | SEQ ID No. 511: GGCCTCTCCCGGTTCGCCGCC AGCCGCCAAGCTGGATGTCGC TTACGACAATATTCCTTAGGG GCACCGCTACATTGACACATC CAGC | The first phosphodiester bond from each of the 5'- and 3'-termini is phosphorothioate-modified. |
| | | strand d | SEQ ID No. 512: GGCGGCUGGCGGCCAUAGCC GUGGGCGCCGCAAAUUUGCU UCCAUAAUAUCAUU | The first phosphodiester bond from the 5'-end and, in the RNA sequence "AUUUGCUUCCAUAAUAUCAUU", the first and second phosphodiester bonds from each of the 5'- and 3'-termini are phosphorothioate-modified; in the first 33 nucleotides, A/G are 2'-O-methyl (2'-OMe) modified and C/U are 2'-fluoro (2'-F) modified; in the RNA sequence "AUUUGCUUCCAUAAUAUCAUU", the 7th, 9th, 10th and 11th nucleotides counted from the 5'-end are 2'-fluoro (2'-F) modified and the remaining RNA nucleotides are 2'-O-methyl (2'-OMe) modified. |

(continued)

| | Molecule | Single strand | Sequence | Modification or remarks |
|---|---|---|---|---|
| 74- | CpG2006-Apt CD40-Apt PD-1-DNA carrier | strand a | SEQ ID No. 309:<br><br>TCGTCGTTTTGTCGTTTTGTCG TTTTTTTGCGACGCCCACGAG CGTTCCGGGAGAGGAGC | In the 5'-end CpG2006 region, all phosphodiester bonds between the 1st to 23rd nucleotides counted from the 5'-end are phosphorothioate-modified. |
| | | strand b | SEQ ID No. 330:<br><br>CCAACGAGTAGGCGATAGCG CGTGGTTTTTGCTCCTCTCCCG GTTCGCCGCGAGCCGCG | The first four phosphodiester bonds from the 5'-end are phosphorothioate-modified, and the first four nucleotides from the 5'-end are 2'-O-methoxyethyl (2'-O-MOE) modified. |
| | | strand c | SEQ ID No. 513:<br><br>ACCGACAGTGAAGGACTCAG CGAACTCTCAGACTCGGTTCT TTTTCGCGGCTCGCGGCCATA GCCGTGGGCGTCGC | The first four phosphodiester bonds from he 5'-end are phosphorothioate-modified, and the first four nucleotides from the 5'-end are 2'-O-methoxyethyl (2'-O-MOE) modified. |
| 75- | Apt CD24-CpG20 06 variant-Apt CD40-Apt PD-L1-DNA carrier (SCLC) | strand a | SEO ID No. 514:<br><br>TATGTGGGTGGGTGGGCGGTT ATGCTGAGTCAGCCTTGCTGT CGTTGCGACGCCCACGAGCGT TCCGGGAGAGGAGC | The first to third phosphodiester bonds from the 5'-end are phosphorothioate-modified. |
| | | strand b | SEQ ID No. 330:<br><br>CCAACGAGTAGGCGATAGCG CGTGGTTTTTGCTCCTCTCCCG GTTCGCCGCGAGCCGCG | The first four phosphodiester bonds from the 5'-end are phosphorothioate-modified. |
| | | strand c | SEQ ID No. 311:<br><br>AACAACGTATAACAATGCCCA CGTCACCAGAGTACTATGGTT TTTCGCGGCTCGCGGCCATAG | The first six nucleotides from the 5'-end are 2'-O-methoxyethyl (2'-O-MOE) modified. |
| | | | CCGTGGGCGTCGC | |

147

EP 4 745 242 A1

| | Molecule | Single strand | Sequence | Modification or remarks |
|---|---|---|---|---|
| 76- | Apt HBsAg-HBV siRNA-miR-3 4-Apt PD-L1-miR-5 42-Apt AS1411-DNA carrier (for treating hepatitis B) | strand a | SEQ ID No. 515:<br>CGCCCACGAGCGTTCCGGGAG AUGGACUUCUCUCAAUUUUC UUU | In the 3'-end RNA sequence, C/U are 2'-fluoro (2'-F) modified; the first two phosphodiester bonds from the 3'-end are phosphorothioate-modified. |
| | | strand b | SEQ ID No. 516:<br>CACAGCGAACAGCGGCGGAC ATAATAGTGCTTACTACGACU AGAAAAUUGAGAGAAGUCCU U | The first three phosphodiester bonds from the 5'-end and the first two phosphodiester bonds from the 3'-end are phosphorothioate-modified; in the RNA region, C/U are 2'-fluoro (2'-F) modified. |
| | | strand c | SEQ ID No. 517:<br>TCTCCCGGTTCGCCGCGAGTG TGACAGAACAACGTATAACA ATGCCCACGTCACCAGAGTAC TATGG | The first three phosphodiester bonds from the 3'-end are phosphorothioate-modified. |
| | | strand d | SEQ ID No. 518:<br>CTCGCGGCCATAGCCGTGGGC GTGGCAGTGTGGTGGTGGTGG TTGTGGTGGTGGTGG | The first three phosphodiester bonds from the 3'-end are phosphorothioate-modified. |
| 77- | Apt IL-4Ra-Apt TIMC-d-4*mi R-126-3p-RN A carrier | strand a | SEQ ID No. 519:<br>AAAAAGCAACAGGGUGCUCC AUGCGCAUGGAACCUGCGCG UCGCCCACGAGCGUUCCGGG AGA | The first three nucleotides from the 5'-end are 2'-O-methoxyethyl (2'-O-MOE) modified, and the first three phosphodiester bonds from the 5'-end are phosphorothioate-modified. |
| | | strand b | SEQ ID No. 520:<br>AAGCAACACUUAGUCGCGAU UGAUACGUGCGCAGUCAUUC UCCCGGUUCGCCGCGAG | The first three nucleotides from the 5'-end are 2'-O-methoxyethyl (2'-O-MOE) modified, and the first three phosphodiester bonds from the 5'-end are phosphorothioate-modified. |
| | | strand c | SEQ ID No. 521:<br>UCGUACCUCGUACCUCGUAC CUCGUACCUCUCGCGGCCAU AGCCGUGGGCG | The phosphodiester bonds within the UCGUACC sequences of miR-126-3p at positions 1-7, 8-14, 15-21 and 21-27 from the 5'-end are phosphorothioate-modified, and the nucleotides at positions 1-7, 8-14, 15-21 and 21-27 are 2'-O-methoxyethyl (2'-O-MOE) modified. |

| | Molecule | Single strand | Sequence | Modification or remarks |
|---|---|---|---|---|
| 78- | Apt CD3-4-Apt PD-1-Apt BCMA-DNA carrier | strand a | SEQ ID No. 15:<br>GACGCCCACGAGCGTTCCGGG AGAGGTGTAGCACGGTGGC | |
| | | strand b | SEQ ID No. 306:<br>TCTCGGACGCGTGTGGTCGGC CGAGTGGCCCACGGTAGAAG GGTTAGAACTGCTGGTTGGTG AATCTCGCTGCCTGGCCCTAG AGTGTTTTTTGCCACCGTGCT ACA | The first two phosphodiester bonds from the 5'-end are phosphorothioate-modified. |
| | | strand c | SEQ ID No. 16:<br>CCTCTCCCGGTTCGCCGCGAG CCTCGGCGCGGCCGTG | |
| | | strand d | SEQ ID No. 307:<br>ACCGACAGTGAAGGACTCAG CGAACTCTCAGACTCGGTTCT TTTTCACGGCCGCGCCGA | The first four phosphodiester bonds from the 5'-end are phosphorothioate-modified. |
| | | strand e | SEQ ID No. 17:<br>GGCTCGCGGCCATAGCCGTGG GCGTCTGCTGCTGCTGCTG | |
| | | strand f | SEQ ID No. 308:<br>AGUGCAAGACGUUCGCAGAU UAGCGAAAAGAGGGUCUCAU UGACUAGUACUCAGCAGCAG CAGCA | In the RNA, the first two phosphodiester bonds from the 3'-end are phosphorothioate-modified and the corresponding nucleotides are 2'-O-methoxyethyl (2'-O-MOE) modified, and the remaining C/U nucleotides in the |
| | | | | RNA are 2-fluoro (2-1) modified. |

149

EP 4 745 242 A1

|  | Molecule | Single strand | Sequence | Modification or remarks |
|---|---|---|---|---|
| 79- | CpG2006-Apt CD38-Apt PD-L1-DNA carrier | strand a | SEQ ID No. 309:<br>TCGTCGTTTTGTCGTTTTGTCG TTTTTTTGCGACGCCCACGAG CGTTCCGGGAGAGGAGC | All phosphodiester bonds between the 1st to 24th nucleotides counted from the 5'-end are phosphorothioate-modified. |
|  |  | strand b | SEQ ID No 310:<br>TACGTGAATCTCGTACGATAC TCTGTAAGCGTTTTTTGCTCCT CTCCCGGTTCGCCGCGAGCCG CG | The first three nucleotides from the 5'-end are 2'-O-methoxyethyl (2'-O-MOE) modified. |
|  |  | strand c | SEQ ID No. 311:<br>AACAACGTATAACAATGCCCA CGTCACCAGAGTACTATGGTT TTTCGCGGCTCGCGGCCATAG CCGTGGGCGTCGC | The first six nucleotides from the 5'-end are 2'-O-methoxyethyl (2'-O-MOE) modified. |
| 80- | Apt CD20-CpG-B YZD Apt CD38-miR-3 4a-Apt CD3-DNA carrier | strand a | ID No. 312:<br>TGCGTGTGTAGTGTGTCTGTT TTTTATCTTCTTTTATCTACTC TTAGGGATTTGGGCGGAGCGA AGCGACGCCCACGAGCGTTCC GGGAGAGGAGC | The first three phosphodiester bonds from the 5'-end are phosphorothioate-modified. |
|  |  | strand b | SEQ ID No. 313:<br>TACGTGAATCTCGTACGATAC TCTGTAAGCGTTGTGACAGGC TCCTCTCCCGGTTCGCCGCGA GCCGCG | The first three nucleotides from the 5'-end are 2'-O-methoxyethyl (2'-O-MOE) modified. |
|  |  | strand c | SEQ ID No. 314:<br>GCCGCGGGGTGGGTCTAGTGT GGATGTTTAGGGGGCGGCTTT TTCGCGGCTCGCGGCCATAGC CGTGGGCGTCGC | The first four nucleotides from the 5'-end are 2'-O-methoxyethyl (2'-O-MOE) modified. |

(continued)

|  | Molecule | Single strand | Sequence | Modification or remarks |
|---|---|---|---|---|
| 81- | CpG2006-Ap t CD38-miR-1 26-Apt PD-L1-DNA carrier | strand a | SEQ ID No. 309:<br>TCGTCGTTTTGTCGTTTTGTCG TTTTTTTGCGACGCCCACGAG CGTTCCGGGAGAGGAG*C | The phosphodiester bonds between the 1st to 23rd nucleotides counted from the 5'-end and the first phosphodiester bond from the 3'-end are phosphorothioate-modified. |
|  |  | strand b | SEQ ID No. 315:<br>TACGTGAATCTCGTACGATAC TCTGTAAGCGTUCGUACCGGC TCCTCTCCCGGTTCGCCGCGA GCCGCG | The nucleotides at positions 1 to 3 and 33 to 39 counted from the 5'-end are 2'-O-methoxyethyl (2'-O-MOE) modified. |
|  |  | strand c | SEQ ID No. 311:<br>AACAACGTATAACAATGCCCA CGTCACCAGAGTACTATGGTT TTTCGCGGCTCGCGGCCATAG CCGTGGGCGTCGC | The first six nucleotides from the 5'-end are 2'-O-methoxyethyl (2'-O-MOE) modified. |
| 82- | Apt BCMA-miR-126-Apt CD38-miR-1 22-Apt CD3-miR-34-DNA carrier | strand a | SEQ ID No. 316:<br>AGUGCAAGACGUUCGCAGAU UAGCGAAAAGAGGGUCUCAU UGACUAGUACUCGUACCGGC GACGCCCACGAGCGTTCCGGG AGAGGAGC | The first four nucleotides from the 5'-end are 2'-O-methoxyethyl (2'-O-MOE) modified; in the remaining RNA region, C/U nucleotides are 2'-fluoro (2'-F) modified. |
|  |  | strand b | SEQ ID No. 317:<br>TACGTGAATCTCGTACGATAC TCTGTAAGCGTGGAAGTGTGC TCCTCTCCCGGTTCGCCGCGA GCCGCG | The first three nucleotides from the 5'-end are 2'-O-methoxyethyl (2'-O-MOE) modified. |
|  |  | strand c | SEQ ID No. 318:<br>AGCCGCGGGGTGGGTCTAGTG TGGATGTTTAGGGGGCGGCTT GTGACAGCGCGGCTCGCGGCC ATAGCCGTGGGCGTCGC | The first four nucleotides from the 5'-end are 2'-O-methoxyethyl (2'-O-MOE) modified. |
| 83- | Apt | strand a | SEQ ID No. 319: | The phosphodiester bonds between the |

| | Molecule | Single strand | Sequence | Modification or remarks |
|---|---|---|---|---|
| | GPC3-AmiR-21-Apt CD38-miR-12 2-Apt PD-L1-miR-3 4-DNA carrier (HCC) | | TAACGCTGACCTTAGCTGCAT GGCTTTACATGTTCCAGATAA GCTGCGACGCCCACGAGCGTT CCGGGAGAGGAGC | 1st to 3rd nucleotides counted from the 5'-end are phosphorothioate-modified. |
| | | strand b | SEQ ID No. 317: TACGTGAATCTCGTACGATAC TCTGTAAGCGTGGAAGTGTGC TCCTCTCCCGGTTCGCCGCGA GCCGCG | The phosphodiester bonds between the 1st to 3rd nucleotides counted from the 5'-end are phosphorothioate-modified. |
| | | strand c | SEQ ID No. 320: AACAACGTATAACAATGCCCA CGTCACCAGAGTACTATGGTG TGACAGCGCGGCTCGCGGCCA TAGCCGTGGGCGTCGC | The 1st to 6th nucleotides counted from the 5'-end are 2'-O-methoxyethyl (2'-O-MOE) modified. |
| 84- | CpG2006-Apt VEGF 165-mi R-122-Apt PD-L1-DNA carrier (HBV-HCC) | strand a | SEQ ID No. 309: TCGTCGTTTTGTCGTTTTGTCG TTTTTTTGCGACGCCCACGAG CGTTCCGGGAGAGGAGC | In the 5'-end CpG2006 region, all phosphodiester bonds between the 1st to 23rd nucleotides counted from the 5'-end are phosphorothioate-modified. |
| | | strand b | SEQ ID No. 321: CGGAAUCAGUGAAUGCUUAU ACAUCCGGGAAGTGTGCTCCT CTCCCGGTTCGCCGCGAGCCG CG | The 1st to 4th nucleotides counted from the 5'-end are 2'-O-methoxyethyl (2'-O-MOE) modified, and the remaining RNA nucleotides have C/U 2'-fluoro (2'-F) modifications. |
| | | strand c | SEQ ID No. 311: AACAACGTATAACAATGCCCA CGTCACCAGAGTACTATGGTT TTTCGCGGCTCGCGGCCATAG CCGTGGGCGTCGC | The 1st to 6th nucleotides counted from the 5'-end are 2'-O-methoxyethyl (2'-O-MOE) modified. |

| | Molecule | Single strand | Sequence | Modification or remarks |
|---|---|---|---|---|
| 85- | CpG2006-Apt HBsAg-miR-122-Apt PD-L1-DNA carrier (HBV) | strand a | SEQ ID No. 309:<br>TCGTCGTTTTGTCGTTTTGTCG TTTTTTTGCGACGCCCACGAG CGTTCCGGGAGAGGAGC | In the 5'-end CpG2006 region, all phosphodiester bonds between the 1st to 23rd nucleotides counted from the 5'-end are phosphorothioate-modified. |
| | | strand b | SEQ ID No. 322:<br>CACAGCGAACAGCGGCGGAC ATAATAGTGCTTACTACGACG GAAGTGTGCTCCTCTCCCGGT TCGCCGCGAGCCGCG | The phosphodiester bonds between the 1st to 3rd nucleotides counted from the 5'-end are phosphorothioate-modified. |
| | | strand c | SEQ ID No. 311:<br>AACAACGTATAACAATGCCCA CGTCACCAGAGTACTATGGTT TTTCGCGGCTCGCGGCCATAG CCGTGGGCGTCGC | The 1st to 6th nucleotides counted from the 5'-end are 2'-O-methoxyethyl (2'-O-MOE) modified. |
| 86- | Apt GSK836-Apt HBsAg-miR-122-Apt PD-L1-DNA carrier (HBV) | strand a | SEQ ID No. 323:<br>GCAGAGGTGAAGCGAAGTCG TTTTTGCGACGCCCACGAGCG TTCCGGGAGAGGAGC | The phosphodiester bonds between the 1st to 5th nucleotides counted from the 5'-end are phosphorothioate-modified. |
| | | strand b | SEQ ID No. 322:<br>CACAGCGAACAGCGGCGGAC ATAATAGTGCTTACTACGACG GAAGTGTGCTCCTCTCCCGGT TCGCCGCGAGCCGCG | The phosphodiester bonds between the 1st to 3rd nucleotides counted from the 5'-end are phosphorothioate-modified. |
| | | strand c | SEQ ID No. 311:<br>AACAACGTATAACAATGCCCA CGTCACCAGAGTACTATGGTT TTTCGCGGCTCGCGGCCATAG CCGTGGGCGTCGC | The 1st to 6th nucleotides counted from the 5'-end are 2'-O-methoxyethyl (2'-O-MOE) modified. |

| | Molecule | Single strand | Sequence | Modification or remarks |
|---|---|---|---|---|
| 87- | Apt BCMA-Apt CD38-miR-12 6-Apt PD-L1-miR-3 4-DNA carrier (MM) | strand a | SEQ ID No. 522:<br>AGUGCAAGACGUUCGCAGAU UAGCGAAAAGAGGGUCUCAU UGACUAGUACAAGCGACGCC CACGAGCGTTCCGGGAGAGG AGC | In the RNA region, the first 52 nucleotides counted from the 5'-end are 2'-fluoro (2'-F) modified. |
| | | strand b | SEQ ID No. 325:<br>TACGTGAATCTCGTACGATAC | The phosphodiester bonds between the 1st to 3rd nucleotides counted from the |
| | | | TCTGTAAGCGTGTCGTTGCTC CTCTCCCGGTTCGCCGCGAGC CGCG | 5'-end are phosphorothioate-modified. |
| | | strand c | SEQ ID No. 320:<br>AACAACGTATAACAATGCCCA CGTCACCAGAGTACTATGGTG TGACAGCGCGGCTCGCGGCCA TAGCCGTGGGCGTCGC | The 1st to 6th nucleotides counted from the 5'-end are 2'-O-methoxyethyl (2'-O-MOE) modified. |
| 88- | Apt AS1411-Apt FGF2-3*Ami R-21-DNA carrier | strand a | SEQ ID No. 326:<br>GGTGGTGGTGGTTGTGGTGGT GGTGGTTTTTGCGCCCACGAG CGTTCCGGGAGAGC | N/A |
| | | strand b | SEQ ID No. 327:<br>GGGAUACUAGGGCAUUAAUG UUACCAGUGUAGUCCCUCCC UGCTCTCCCGGTTCGCCGCCA GCCGCC | In the RNA sequence, C/U are 2'-fluoro (2'-F) modified. |
| | | strand c | SEQ ID No. 328:<br>GATAAGCTGATAAGCTGATAA GCTGGCGGCAGGCGGCCATA GCCGTGGGCGC | The phosphodiester bonds between the 1st to 7th, 9th to 16th, and 18th to 25th nucleotides counted from the 5'-end are phosphorothioate-modified. |

154

| | Molecule | Single strand | Sequence | Modification or remarks |
|---|---|---|---|---|
| 89- | Apt HBsAg-Apt CD40-Apt PD-L1-DNA carrier (HBV) | strand a | SEQ ID No. 329:<br>CACAGCGAACAGCGGCGGAC ATAATAGTGCTTACTACGACT TTTTGCGACGCCCACGAGCGT TCCGGGAGAGGAGC | The phosphodiester bonds between the 1st to 3rd nucleotides counted from the 5'-end are phosphorothioate-modified. |
| | | strand b | SEQ ID No. 330:<br>CCAACGAGTAGGCGATAGCG CGTGGTTTTTGCTCCTCTCCCG GTTCGCCGCGAGCCGCG | The first and second phosphodiester bonds from each of the 5'- and 3'-termini are phosphorothioate-modified. |
| | | strand c | SEQ ID No. 331:<br>ACGGGCCACATCAACTCATTG ATAGACAATGCGTCCACTGCC CGTTTTTTCGCGGCTCGCGGC CATAGCCGTGGGCGTCGC | The first and second phosphodiester bonds from each of the 5'- and 3'-termini are phosphorothioate-modified. |
| 90- | Apt HBsAg-Apt CD40-Apt PD-L1 (HD)-DNA carrier (HBV) | strand a | SEQ ID No. 329:<br>CACAGCGAACAGCGGCGGAC ATAATAGTGCTTACTACGACT TTTTGCGACGCCCACGAGCGT TCCGGGAGAGGAGC | The phosphodiester bonds between the 1st to 3rd nucleotides counted from the 5'-end are phosphorothioate-modified. |
| | | strand b | SEQ ID No. 330:<br>CCAACGAGTAGGCGATAGCG CGTGGTTTTTGCTCCTCTCCCG GTTCGCCGCGAGCCGCG | The phosphodiester bonds between the 1st to 22nd nucleotides counted from each of the 5'- and 3'-termini are phosphorothioate-modified. |
| | | strand c | SEQ ID No. 311:<br>AACAACGTATAACAATGCCCA CGTCACCAGAGTACTATGGTT TTTCGCGGCTCGCGGCCATAG CCGTGGGCGTCGC | The 1st to 5th nucleotides counted from the 5'-end are 2'-O-methoxyethyl (2'-O-MOE) modified. |
| | Apt BCMA - Apt CD38-Apt CPG - Apt CD19-AmiR-21-DNA3-TY (MM) | strand a | SEQ ID No. 15:<br>GACGCCCACGAGCGTTCCGGG AGAGGTGTAGCACGGTGGC | |

155

EP 4 745 242 A1

| | Molecule | Single strand | Sequence | Modification or remarks |
|---|---|---|---|---|
| 91- | Apt BCMA - Apt CD38-Apt CPG - Apt CD19-AmiR-21-DNA3-TY (MM) | strand b | SEQ ID No. 16:<br>CCTCTCCCGGTTCGCCGCGAG CCTCGGCGCGGCCGTG | |
| | | strand c | SEQ ID No. 17:<br>GGCTCGCGGCCATAGCCGTGG GCGTCTGCTGCTGCTGCTG | |
| | | strand d | SEQ ID No. 332:<br>TACGTGAATCTCGTACGATAC TCTGTAAGCGTTTAGCGAAGC CACCGTGCTACA | The phosphodiester bonds between the 1st to 3rd nucleotides counted from the 5'-end are phosphorothioate-modified. |
| | | strand e | SEQ ID No. 333:<br>UGAGCCCUGUUCGACAGGAG | RNA with C/U 2'-fluoro (2'-F) modifications. |
| | | | GCUCAGAUAAGCUCACGGCC GCGCCGA | |
| | | strand f | SEQ ID No. 334:<br>AGUGCAAGACGUUCGCAGAU UAGCGAAAAGAGGGUCUCAU UGACUAGUACAACAGCAGCA GCAGCA | RNA with C/U 2'-fluoro (2'-F) modifications. |
| 92- | Apt BCMA-Apt CD38-Apt CD19-AmiR-21-DNA3-TY (MM) | strand a | SEQ ID No. 15:<br>GACGCCCACGAGCGTTCCGGG AGAGGTGTAGCACGGTGGC | |
| | | strand b | SEQ ID No. 16:<br>CCTCTCCCGGTTCGCCGCGAG CCTCGGCGCGGCCGTG | |
| | | strand c | SEQ ID No. 17:<br>GGCTCGCGGCCATAGCCGTGG GCGTCTGCTGCTGCTGCTG | |

156

| | Molecule | Single strand | Sequence | Modification or remarks |
|---|---|---|---|---|
| | | strand d | SEQ ID No. 335:<br>TACGTGAATCTCGTACGATAC TCTGTAAGCGTTTTTTGCCAC CGTGCTACA | The phosphodiester bonds between the 1st to 3rd nucleotides counted from the 5'-end are phosphorothioate-modified. |
| | | strand e | SEQ ID No. 333:<br>UGAGCCCUGUUCGACAGGAG GCUCAGAUAAGCUCACGGCC GCGCCGA | RNA with C/U 2'-fluoro (2'-F) modifications. |
| | | strand f | SEQ ID No. 334:<br>AGUGCAAGACGUUCGCAGAU UAGCGAAAAGAGGGUCUCAU UGACUAGUACAACAGCAGCA GCAGCA | RNA with C/U 2'-fluoro (2'-F) modifications. |
| 93- | CpG2006-Ap t CD38-Apt PD-L1-DNA3 -TY (tumor) | strand a | SEQ ID No. 15:<br>GACGCCCACGAGCGTTCCGGG AGAGGTGTAGCACGGTGGC | |
| | | strand b | SEQ ID No. 16:<br>CCTCTCCCGGTTCGCCGCGAG CCTCGGCGCGGCCGTG | |
| | | strand c | SEQ ID No. 17:<br>GGCTCGCGGCCATAGCCGTGG GCGTCTGCTGCTGCTGCTG | |
| | | strand d | SEQ ID No. 335:<br>TACGTGAATCTCGTACGATAC TCTGTAAGCGTTTTTTGCCAC CGTGCTACA | The phosphodiester bonds between the 1st to 3rd nucleotides counted from the 5'-end are phosphorothioate-modified. |
| | | strand e | SEQ ID No. 336:<br>ACGGGCCACATCAACTCATTG ATAGACAATGCGTCCACTGCC CGTTTTTTCACGGCCGCGCCG A | The phosphodiester bonds between the 1st to 3rd nucleotides counted from the 5'-end are phosphorothioate-modified. |

| | Molecule | Single strand | Sequence | Modification or remarks |
|---|---|---|---|---|
| | | strand f | SEQ ID No. 337:<br>TCGTCGTTTTGTCGTTTTGTCG TTTTTTTCAGCAGCAGCAGCA | The phosphodiester bonds between the 1st to 23rd nucleotides counted from the 5'-end are phosphorothioate-modified. |
| 94- | Apt BCMA-Apt CD38-Apt CPG- Apt PD-L1-DNA3 -TY(MM) | strand a | SEQ ID No. 15:<br>GACGCCCACGAGCGTTCCGGG AGAGGTGTAGCACGGTGGC | |
| | | strand b | SEQ ID No. 16:<br>CCTCTCCCGGTTCGCCGCGAG CCTCGGCGCGGCCGTG | |
| | | strand c | SEQ ID No. 17:<br>GGCTCGCGGCCATAGCCGTGG GCGTCTGCTGCTGCTGCTG | |
| | | strand d | SEQ ID No. 332:<br>TACGTGAATCTCGTACGATAC TCTGTAAGCGTTTAGCGAAGC CACCGTGCTACA | In the DNStrand a, the first three phosphodiester bonds from the 5'-end are phosphorothioate-modified. |
| | | strand e | SEQ ID No. 336:<br>ACGGGCCACATCAACTCATTG ATAGACAATGCGTCCACTGCC CGTTTTTTCACGGCCGCGCCG A | The first three phosphodiester bonds from the 5'-end are phosphorothioate-modified. |
| | | strand f | SEQ ID No. 334:<br>AGUGCAAGACGUUCGCAGAU UAGCGAAAAGAGGGUCUCAU UGACUAGUACAACAGCAGCA GCAGCA | RNStrand a in which C/U nucleotides are 2'-fluoro (2'-F) modified. |
| | | strand a | SEQ ID No. 15:<br>GACGCCCACGAGCGTTCCGGG AGAGGTGTAGCACGGTGGC | |

|  | Molecule | Single strand | Sequence | Modification or remarks |
|---|---|---|---|---|
| 95- | Apt BCMA-Apt CD40-Apt PD-L1-DNA3 -TY(MM) | strand b | SEQ ID No. 16:<br>CCTCTCCCGGTTCGCCGCGAG CCTCGGCGCGGCCGTG |  |
|  |  | strand c | SEQ ID No. 17:<br>GGCTCGCGGCCATAGCCGTGG GCGTCTGCTGCTGCTGCTG |  |
|  |  | strand d | SEQ ID No. 338:<br>GCCAACGAGTAGGCGATAGC GCGTGGCTTTTTGCCACCGTG CTACA | The first three phosphodiester bonds from the 5'-end are phosphorothioate-modified. |
|  |  | strand e | SEQ ID No. 336:<br>ACGGGCCACATCAACTCATTG ATAGACAATGCGTCCACTGCC CGTTTTTTCACGGCCGCGCCG A | The first three phosphodiester bonds from the 5'-end are phosphorothioate-modified. |
|  |  | strand f | SEQ ID No. 334:<br>AGUGCAAGACGUUCGCAGAU UAGCGAAAAGAGGGUCUCAU UGACUAGUACAACAGCAGCA GCAGCA | RNStrand a in which C/U nucleotides are 2'-fluoro (2'-F) modified. |
| 96 | AmiR-21-Apt AS1411-Apt CD40-2*bioti n-DNA carrier | strand a | SEQ ID No. 393:<br>GATAAGCTGCGACGCCCACG AGCGTTCCGGGAGAGGAG | The nucleotides at positions 1 to 8 counted from the 5'-end are fully locked nucleic acid (LNA) modified; the 3'-end is biotin-modified. |
|  |  | strand b | SEQ ID No. 394:<br>GGTGGTGGTGGTTGTGGTGGT GGTGGTTTTTCTCCTCTCCCGG TTCGCCGCGAGCCGCG |  |
|  |  | strand c | SEQ ID No. 437:<br>CCAACGAGTAGGCGATAGCG CGTGGTTTTTCGCGGCACGCG GCCATAGCCGTGGGCGTCGC | The 3'-end is biotin-modified. |

EP 4 745 242 A1

159

(continued)

| | Molecule | Single strand | Sequence | Modification or remarks |
|---|---|---|---|---|
| 97 | 2*AmiR-21-4 *biotin-DNA carrier | strand a | SEQ ID No. 523:<br>GATAAGCTGCGACGCCCACG AGCGTTCCGGGAGAGGAGC | The nucleotides at positions 1 to 8 counted from the 5'-end are fully locked nucleic acid (LNA) modified, and the 3'-end is biotin-modified. |
| | | strand b | SEQ ID No. 25:<br>GCTCCTCTCCCGGTTCGCCGC GAGCCGCGT | Biotin is attached to each of the 5'- and 3'-termini. |
| | | strand c | SEQ ID No. 524:<br>GATAAGCTCGCGGCTCGCGGC CATAGCCGTGGGCGTCGCT | The nucleotides at positions 1 to 8 counted from the 5'-end are fully locked nucleic acid (LNA) modified, and the 3'-end is biotin-modified. |
| 98 | Apt TTA1-doxoru bicin-4*biotin -DNA carrier | strand a | SEQ ID No. 33:<br>GCGGCGCCCACGAGCGTTCCG GGAGC | Biotin is attached to each of the 5'- and 3'-termini. |
| | | strand b | SEQ ID No. 525:<br>CCTGCACTTGGCTTGGATTTC AGAAGGGAGACCCTTTTTGCT CCCGGTTCGCCGCCAGCCGCC ; | |
| | | strand c | SEQ ID No. 35:<br>GGCGGCAGGCGGCCATAGCC GTGGGCGCCGC | Biotin is attached to each of the 5'- and 3'-termini. |

| | Molecule | Single strand | Sequence | Modification or remarks |
|---|---|---|---|---|
| 99 | Apt TTA1-2*Surviv in siRNA-DNA carrier | strand a | SEQ ID No. 526:<br>GCGGCGCCCACGAGCGTTCCG GGAGCGCAGGUUCCUUAUCU GUCACAUU | siRNA sense strand: the first and second phosphodiester bonds from each of the 5'- and 3'-termini are phosphorothioate-modified. |
| | | strand b | SEQ ID No. 525:<br>CCTGCACTTGGCTTGGATTTC AGAAGGGAGACCCTTTTTGCT CCCGGTTCGCCGCCAGCCGCC | |
| | | strand c | SEQ ID No. 527:<br>GGCGGCAGGCGGCCATAGCC GTGGGCGCCGCGCAGGUUCC UUAUCUGUCACAUU | siRNA sense strand: the first and second phosphodiester bonds from each of the 5'- and 3'-termini are phosphorothioate-modified. |
| | | strand d | SEQ ID No. 300:<br>UGUGACAGAUAAGGAACCUG CAG | siRNA antisense strand: the first and second phosphodiester bonds from each of the 5'- and 3'-termini are phosphorothioate-modified. |
| 100 | Apt CD40 CpG2006-DNA carrier | strand a | SEQ ID No. 528:<br>CCACTGCGGGGGTCTATACGT GAGGAAGAAGTGGTTTTTGCG ACGCCCACGAGCGTTCCGGGA GAGGAGC; | The phosphodiester bonds between the 1st to 3rd nucleotides counted from the 5'-end are phosphorothioate-modified. |
| | | strand b | SEQ ID No. 330:<br>CCAACGAGTAGGCGATAGCG CGTGGTTTTTGCTCCTCTCCCG GTTCGCCGCGAGCCGCG; | The phosphodiester bonds between the 1st to 3rd nucleotides counted from the 5'-end are phosphorothioate-modified. |
| | | strand c | SEQ ID No. 529:<br>TCGTCGTTTTGTCGTTTTGTCG TTTTTTTCGCGGCTCGCGGCC ATAGCCGTGGGCGTCGC. | The phosphodiester bonds between the 1st to 23rd nucleotides counted from the 5'-end are phosphorothioate-modified. |

EP 4 745 242 A1

161

It should be noted that, for the siRNA-containing drugs listed in the foregoing table, depending on the precise position within the strand and the surrounding sequence context, in order to avoid undesired complementary base pairing between the resulting double-stranded siRNA and an adjoining transition sequence (including, for example, A, AA, AAA, or U, UU, UUU, etc.)-which could impair subsequent siRNA biological activity-one may, while preserving normal siRNA function, correspondingly add to or delete from the 5' and/or 3' end of the sense strand or the antisense strand by 1-2 nucleotides. That is, the target sequence intended to be silenced by the sense and antisense strands of the siRNA is not changed. Such adaptive end extension or trimming by 1-2 nucleotides can readily be implemented by those skilled in the art.

Additionally, the "strand a," "strand b," "strand c," "strand d," "strand e," and "strand f" in the foregoing table are merely listings of the individual single-stranded oligonucleotides used in preparing the nucleic-acid drug and are not in one-to-one correspondence with the sequence a, sequence b, and sequence c of the nucleic-acid carrier. In certain drugs, the strand a, strand b, or strand c may consist solely of the sequence a (e.g., Drug 98), the sequence b (e.g., Drugs 2, 19, 20, and 97), or the sequence c (e.g., Drugs 91-95). In other drugs, the strand a, strand b, or strand c may be the overall length of sequence a formed by sequence a, b, or c together with the sequence of an oligonucleotide effector molecule, optionally including an intervening transition sequence (e.g., Drugs 32, 26, etc.). In still other drugs, the strand a, strand b, or strand c may be the overall length of sequence a formed by joining the nucleic-acid sequence of a given aptamer, via a transition sequence, to the antisense strand of an siRNA (e.g., Drugs 31, 33, 35, etc.). The remaining strand d, strand e, and strand f may likewise take such forms, and may also be sequences formed by linking the sense strand of an siRNA to sequence a, b, or c. Details are not repeated herein. In the foregoing table, aptamers are denoted by "Apt"; for example, the PD-1 aptamer is denoted "Apt PD-1." An asterisk "*" denotes a phosphorothioate (PS) modification, and a plus sign "+" denotes a locked-nucleic-acid (LNA) modification; in the event of any omission or ambiguity in the explanations, the nucleotide sequences shall govern.

The carrier "DNA3-TY" used in the table above is a universal carrier that employs a single-stranded bridging to load different oligonucleotide effector molecules; its specific sequence is as follows:

Table 6:

| Universal carrier DNA3-TY | strand a | SEQ ID No. 15: GACGCCCACGAGCGTTCCGGGAGAGGTGTAGCACGGT GGC |
|---|---|---|
| | strand b | SEQ ID No. 16: CCTCTCCCGGTTCGCCGCGAGCCTCGGCGCGGCCGTG |
| | strand c | SEQ ID No. 17: GGCTCGCGGCCATAGCCGTGGGCGTCTGCTGCTGCTGC TG |

In one specific nucleic-acid drug, CpG oligodeoxynucleotides (CpG ODNs) and oligonucleotides such as a CD40 aptamer, anti-miR-21 (AmiR-21), and the AS1411 aptamer are appended in a sequence-extension manner to the 5' ends of the three single strands of the nucleic-acid carrier using an oligonucleotide synthesizer; in an assembly buffer, the strands self-assemble according to Watson-Crick base-pairing principles to yield, by way of example, a 2*CpG ODN (1826/2006)-CD40 aptamer-DNA carrier.

Mediated by the CD40 aptamer, the CpG ODNs can actively and efficiently target and bind to B cells and plasmacytoid dendritic cells (pDCs) in the tumor microenvironment that overexpress CD40. CpG ODNs can directly activate B cells to produce antibodies and, by stimulating and activating pDCs, induce secretion of Th1-type and pro-inflammatory cytokines, thereby promoting T-cell activation and eliciting an immune response.

When CD40 mediates binding to CD40 on the surfaces of B cells and dendritic cells, CD40-CD40L (CD154) signaling interactions further activate antigen-presenting cells (APCs). In particular, activated dendritic cells function as a bridge between tumor cells and tumor-specific cytotoxic T lymphocytes (CTLs), being capable of presenting tumor antigens to T cells and activating T cells.

In the foregoing drug, the OX40/OX40L costimulatory axis may also be involved. OX40 (CD134) is a costimulatory receptor of the TNFR superfamily expressed on activated effector T (Teff) cells and regulatory T cells (Tregs). Under conditions of a weak immune response, an OX40 aptamer engaging the OX40 receptor on activated CD4$^+$ and CD8$^+$ T cells triggers bidirectional costimulatory signaling, promoting T-cell activation, proliferation, and trafficking, and generating a robust, durable, non-antigen-specific immune response. Costimulation through OX40/0X40L is particularly important for T-cell survival and persistence.

The mannose receptor (MR) is predominantly expressed on macrophages and dendritic cells (DCs). A mannose moiety directs nucleic-acid nanoparticles to specifically bind MR and to be internalized via MR-mediated endocytosis. This pathway is characterized by high efficiency, selectivity, and saturability, and the presentation efficiency of mannose-conjugated antigens and peptides can be increased by about 200-10,000-fold.

Mucin 1 (MUC1) is overexpressed on the surface of virtually all epithelial adenocarcinoma cells, at levels exceeding those of normal tissues by ten-fold or more. Its transmembrane and cytoplasmic domains are highly conserved across species and display a non-polar distribution. A MUC1 aptamer can be co-synthesized with the scaffold sequence of the nucleic-acid carrier in a sequence-extension manner, which is convenient and efficient; "TTTTT" serves as a linker/bridge and spacer between the MUC1 aptamer and the nucleic-acid carrier, maintaining the aptamer's conformation and stability and thereby enhancing MUC1 aptamer/receptor-mediated targeting. MUC1 is thus an advantageous target for active, tumor-specific immunotherapy.

AmiR-21 (anti-miR-21) specifically binds to and knocks down miR-21 overexpressed in tumor cells and, via the miR-21/PTEN/PI3K/AKT pathway, suppresses the activation and expansion of myeloid-derived suppressor cells (MDSCs) to mitigate an immunosuppressive tumor microenvironment; it also blocks or reduces the phagocytic uptake of miR-21 by immune cells in the vicinity of dorsal root ganglion (DRG) neurons, thereby alleviating cancer-related and neuropathic pain caused by local inflammation.

The folate receptor (FR) exhibits strong tissue and tumor specificity and is overexpressed in multiple tumor cell types-particularly in non-small cell lung cancer (NSCLC), where each cell can display up to several hundred thousand FR molecules-whereas FR is virtually absent on healthy human blood cells. Folate-FR-mediated folate-functionalized nanocarriers show excellent targeting specificity: folate binds FR with high affinity, and a folate-targeted nanocarrier, when loaded with a drug, can, via FR-mediated mechanisms and receptor-mediated endocytosis, deliver the drug precisely to cancer cells and into the cytoplasm to exert its effect, thereby improving drug safety and bioavailability.

The AS1411 aptamer binds to nucleolin overexpressed on the surface of tumor cells, directing the internalization of AmiR-21 (anti-miR-21) into target cells and, acting as a chaperone, mediating transit across endosomes and lysosomes so as to enable AmiR-21 to escape enzymatic degradation and reach the cytoplasm for specific knockdown of miR-21; the AS 1411 aptamer itself also inhibits tumor growth and progression.

Mucin 1 (MUC1) is overexpressed on the surface of nearly all epithelial adenocarcinoma cells at levels at least an order of magnitude higher than in normal tissues. Its transmembrane and cytoplasmic domains are highly conserved across species and display a non-polar distribution. A MUC1 aptamer can be co-synthesized with the scaffold sequence of the nucleic-acid nanocarrier in a sequence-extension manner for convenience and efficiency; a polythymidine linker "TTTTT" serves as the bridge and spacer between the MUC1 aptamer and the nucleic-acid carrier, maintaining the aptamer's conformation and stability and thereby enhancing MUC1 aptamer/receptor-mediated targeting. MUC1 is therefore an advantageous target for active, tumor-specific immunotherapy.

Biotin H (biotin): Studies indicate that certain tumor cells that overexpress the folate receptor (FR) and the vitamin B12 receptor also highly express a biotin receptor (BR), often at even higher levels. Drug-delivery investigations using different vitamins as guiding ligands for the same tumor cell type show that biotin exhibits superior targeting relative to folate, vitamin B12 (cobalamin), riboflavin, and the like. Accordingly, we adopt biotin as a baseline targeting configuration for drug delivery; using polyethylene glycol (PEG) as a linker, biotin can be conveniently installed at any 5' or 3' end of the nucleic-acid nanocarrier. Through multivalent and/or multiplex targeting mediated by multiple composite guiding ligands, binding opportunities are increased and targeting efficiency is improved. At present, the reported antitumor spectrum of biotin-guided targeted therapy encompasses multiple malignancies including ovarian, colon, lung, renal, hepatic, and breast cancers.

A15 aptamer: CD133$^+$ tumor cells possess enhanced self-renewal, differentiation, and tumorigenic capacity. CD133 is a commonly used marker of tumor stem-like cells and is highly expressed in numerous solid tumors (e.g., glioblastoma, breast, gastric, hepatic, pulmonary, pancreatic, prostate, and colorectal cancers). The A15 aptamer-SEQ ID No. 129: CCCTCCTACATAGGG / SEQ ID No. 130: CCCUCCUACAUAGGG-is an aptamer to CD133 and, in vivo, exhibits a targeting effect for tracking and specifically binding CD133$^+$ tumor cells. We append the A15 aptamer in a sequence-extension manner to one arm of the three-armed nucleic-acid nanocarrier; A15 thereby mediates further tracking of CD133$^+$ tumor stem-like "seed" cells and suppresses their expansion, to achieve control or regression of tumor progression.

TTA1 aptamer: Tenascin-C is an extracellular-matrix glycoprotein that is highly expressed in most solid tumors. The TTA1 aptamer specifically binds the tenascin-C receptor and is used as a targeting moiety ("warhead") by appending it, in a sequence-extension manner, to the 5' end of the nanocarrier scaffold sequence, thereby mediating rapid uptake of the nucleic-acid nanocarrier by tumor cells. When the TTA1 aptamer is linked to the carrier via a polythymidine "TTTTT" linker/spacer, its serum stability and spatial conformation are markedly enhanced, thereby maintaining tenascin-C-mediated targeted drug-delivery efficacy.

In designing certain combined immunotherapeutic compositions, the following considerations are taken into account: tumor cells often exhibit low expression of major histocompatibility complex (MHC) molecules, lack costimulatory molecules, display defects in antigen processing, and show altered expression of T-cell signaling molecules, resulting in diminished antitumor immunity-a principal cause of tumorigenesis and progression. In some embodiments of the present application, CpG oligodeoxynucleotides (CpG ODNs), a CD40 aptamer, AmiR-21 (anti-miR-21), and an AS1411 aptamer oligonucleotide are appended, in a sequence-extension manner, to the 5' ends of the three single strands of the DNA nucleic-acid carrier provided herein using an oligonucleotide synthesizer; in assembly buffer, the strands self-assemble according to Watson-Crick base-pairing principles to yield 2×CpG ODN (1826/2006)-CD40 aptamer-DNA carrier and 2×AmiR-21-AS1411 aptamer-biotin-DNA carrier nanoparticulates, together with a single-stranded OX40L aptamer. When this three-component combination immunomodulator is administered by intravenous (tail-vein) injection, the CpG ODNs, under mediation by the CD40 aptamer, can actively and efficiently target and bind B cells and plasmacytoid dendritic cells (pDCs) in the tumor microenvironment that overexpress CD40. CpG ODNs can directly activate B cells to produce antibodies and, by stimulating and activating pDCs, induce secretion of Th1-type and pro-inflammatory cytokines, thereby promoting T-cell activation and eliciting immune responses. When the CD40 aptamer mediates engagement with CD40 on the surfaces of B cells and dendritic cells, CD40-CD40L signaling further activates antigen-presenting cells (APCs). In particular, activated dendritic cells function as a bridge between tumor cells and tumor-specific cytotoxic T lymphocytes (CTLs), presenting tumor antigens to T cells and activating them. Low doses of CpG induce (secondarily and transiently) the expression of OX40 on CD4$^+$ T cells in the tumor microenvironment, after which an OX40L aptamer can trigger T-cell immune responses. Anti-AmiR-21 specifically binds to and knocks down miR-21 overexpressed in tumor cells and, via the miR-21/PTEN/PI3K/AKT pathway, suppresses the activation and expansion of myeloid-derived suppressor cells (MDSCs) to mitigate the immunosuppressive tumor microenvironment; it also blocks or reduces the phagocytic uptake of miR-21 by immune cells surrounding dorsal root ganglion (DRG) neurons, thereby alleviating cancer-related and neuropathic pain caused by local inflammation. The AS1411 aptamer binds nucleolin overexpressed on the surface of tumor cells, directs the internalization of AmiR-21 into target cells, and, acting as a chaperone, mediates transit across endosomes and lysosomes to enable AmiR-21 to escape enzymatic degradation, reach the cytoplasm, and specifically knock down miR-21; the AS1411 aptamer itself also exhibits inhibitory effects on tumor growth and progression. In view of the pervasive heterogeneity of tumors-particularly the greater complexity of solid tumors-in certain embodiments the CpG ODN, CD40 aptamer, OX40/0X40L, and anti-miRNA (AmiRNA) oligonucleotide aptamers are combined, as dictated by the indication, into differently configured combination immunomodulators to mobilize endogenous T-cell immune responses, achieving significant antitumor immunotherapeutic efficacy across multiple murine models.

Set forth below, with reference to the foregoing specific compositions of certain nucleic-acid drugs, is a further description of their mechanisms of action.

Drug 1) 3* CpG 2006-DNA carrier

This agent is an actively immunostimulatory nucleic-acid nanotherapeutic designed to increase uptake by antigen-presenting cells (APCs) within tumor tissue, thereby enhancing the immunostimulatory activity of CpG 2006, further activating immune responses, and inhibiting tumor growth. The key design logic is as follows:

1. Sequence-extension modification: Three CpG 2006 segments are respectively appended to the 5' ends of the three arms of the nucleic-acid nanocarrier, forming a 3*CpG 2006-DNA nucleic-acid immunostimulant. This architecture improves the stability and effectiveness of CpG 2006 within tumor tissue.

2. Tumor-tissue "funnel" effect: Tumor vasculature typically exhibits enlarged interendothelial gaps, enabling nanoparticles to enter tumor tissue more readily via a "funnel" (leaky-vasculature) effect. Accordingly, the nucleic-acid immunostimulant more readily accesses tumor tissue, improving therapeutic outcome.

3. APC phagocytic uptake: APCs phagocytose and process antigens and present antigenic peptides to T cells to initiate immune responses. The nucleic-acid immunostimulant increases APC uptake of CpG 2006, thereby augmenting the ensuing immune response.

4. Structural stabilization by the nucleic-acid carrier: The nanocarrier provides structural stability and protects CpG 2006 from enzymatic degradation, thereby enhancing its stability and bioavailability within tumor tissue.

In summary, this actively immunostimulatory nucleic-acid nanotherapeutic leverages the tumor "funnel" effect, APC phagocytic uptake, and the structural-stabilizing function of the nucleic-acid carrier to increase intratumoral uptake of CpG 2006 and its stimulatory activity, thereby activating immune responses and inhibiting tumor growth.

Drug 2) 2*CpG 1826-2*mannose-DNA carrier (for targeted stimulation of immune responses)

This composition is a specifically targeted, actively immunostimulatory nucleic-acid nanotherapeutic. By appending two mannose moieties at the 3' end of the nucleic-acid carrier, an active-targeting function is conferred. While retaining the nanoparticles' passive intratumoral accumulation ("funnel") effect, the construct additionally gains the capability to actively target tumor tissue. The mechanism of action is summarized as follows:

1. Mannose modification: Two mannose moieties are installed at the 3' end of the nucleic-acid carrier to provide an active-targeting function. Mannose exhibits specific affinity for mannose receptors on tumor-associated cells, thereby enhancing targeting of the nanoparticles to tumor tissue.

2. Active-targeting function: Through the specific affinity between mannose and the mannose receptor, the 2*CpG 1826-2*mannose-DNA nucleic-acid nanoparticles actively target murine tumor tissue. This targeting increases intratumoral concentrations of CpG 1826 and thereby improves therapeutic efficacy.

3. TLR9 engagement: CpG 1826 is a CpG oligodeoxynucleotide that binds Toll-like receptor 9 (TLR9) on immune cells. TLR9 recognizes CpG oligonucleotides and activates immune cells such as dendritic cells and B cells.

4. Immune activation: By engaging TLR9, CpG 1826 activates dendritic cells and B cells, further promoting immune responses. Dendritic cells present tumor antigens and activate T cells, while B cells secrete antibodies. These immune responses contribute to inhibition of tumor growth.

5. Enhanced antitumor effect: The active-targeting function increases nanoparticle concentration within tumor tissue. Binding of the nanoparticles to mannose receptors on tumor-associated cells facilitates cellular uptake, enabling CpG 1826 to more readily enter cells and exert its immunostimulatory activity.

6. Inhibition of tumor growth: By activating immune cells and augmenting immune responses, the 2*CpG 1826-2*mannose-DNA carrier assists in eliminating tumor cells and inhibiting tumor growth, thereby achieving superior efficacy in tumor therapy.

7. Reduced adverse effects: Owing to the active-targeting function, the 2*CpG 1826-2*mannose-DNA nucleic-acid nanoparticles act predominantly within tumor tissue, thereby reducing impact on normal tissues and decreasing adverse effects during treatment.

In summary, this specifically targeted, actively immunostimulatory nucleic-acid nanotherapeutic integrates (i) the structural-stabilization function of the nucleic-acid carrier, (ii) the active-targeting conferred by mannose modification, and (iii) the TLR9-mediated immunostimulatory activity of CpG 1826. These cooperating mechanisms afford improved efficacy, precision, and safety in tumor therapy.

Drug 3) 2*CpG 1826-CD40 aptamer-DNA carrier

This composition is a specifically targeted, actively immunostimulatory dual-combination nucleic-acid nanotherapeutic. By introducing, on the nucleic-acid nanocarrier, a CD40 aptamer (CD40L) that confers both targeting mediation and costimulatory function, tumor-induced immunosuppression is relieved and the immune system is activated to eliminate tumor cells. The mechanism of action and key design logic are as follows:

1. Passive-targeting effect: The 2*CpG 1826-CD40 aptamer-DNAh nucleic-acid immunostimulant retains the intrinsic passive targeting of nanoparticles to tumor tissue, i.e., utilizing the leaky-vasculature ("funnel") phenomenon of tumor blood vessels to facilitate nanoparticle entry into tumor tissue.

2. Incorporation of a CD40 aptamer (CD40L): Incorporation of a CD40 aptamer on the nanocarrier imparts stronger immunostimulatory capacity. The CD40 aptamer functions as a costimulatory moiety that binds the CD40 receptor on immune cells, thereby activating immune cells such as dendritic cells.

3. Reversal of tumor immunosuppression: Tumor cells commonly exploit immunosuppressive mechanisms to evade immune attack. Binding of the CD40 aptamer to CD40 activates dendritic cells and relieves tumor-imposed immunosuppression, restoring the immune system's capacity to attack tumor cells.

4. Immune-system activation: Activation of the CD40L-CD40 signaling pathway drives dendritic-cell maturation and antigen presentation to T cells. In addition, the CD40 aptamer can promote B-cell proliferation and antibody secretion. These responses contribute to tumor-cell clearance.

5. Tumor-cell elimination: By relieving immunosuppression and activating the immune system, the 2*CpG 1826-CD40L-DNAh nucleic-acid immunostimulant enables more effective recognition and attack of tumor cells, thereby inhibiting tumor growth and dissemination; this combined action affords higher therapeutic efficacy.

6. Enhanced therapeutic specificity: The targeting-mediation function of the CD40 aptamer improves therapeutic specificity. Because CD40 is primarily expressed on immune cells, CD40L-CD40 signaling acts mainly on immune cells, thereby reducing adverse effects on normal tissues.

7. Reduced adverse effects: As the 2*CpG 1826-CD40 aptamer-DNAh immunostimulant acts predominantly within tumor tissue and the CD40 aptamer provides targeting mediation, treatment-related adverse effects are reduced and safety is improved.

In summary, this specifically targeted, actively immunostimulatory dual-combination nucleic-acid nanotherapeutic integrates the nanocarrier's passive-targeting effect with a CD40 aptamer that confers both targeting mediation and costimulatory function. These cooperative mechanisms yield improved efficacy, precision, and safety in tumor therapy by relieving tumor immunosuppression, activating the immune system, and promoting tumor-cell clearance.

2*CpG 1826-CD40 aptamer-2*mannose-DNAh. Building upon the foregoing 2*CpG 1826-CD40 aptamer-DNAh construct, this nucleic-acid immunotherapeutic further introduces two mannose moieties at two 3' ends of the core scaffold sequence of the nanocarrier, thereby enhancing composite targeting-mediation to the tumor microenvironment, increasing intratumoral immune responsiveness, and enabling dose reduction with a concomitant decrease in potential adverse effects. With reference to the foregoing description of CpG 1826 and related agonists, the mechanism of action is accordingly analyzed and described in detail.

Drug 4) 2*CpG 2006-CD40 aptamer-DNA carrier (an actively immunostimulatory nucleic-acid nanotherapeutic)

This composition is a specifically targeted, actively immunostimulatory dual-combination nucleic-acid nanotherapeutic that incorporates CpG 2006 (a humanized TLR9 agonist) and a CD40 aptamer. CpG 2006, as a humanized TLR9 agonist, exhibits greater biocompatibility for human use than the murine CpG 1826 while still retaining immunostimulatory activity in mice. The mechanism of action for active antitumor immunotherapy is as follows:

1. Passive-targeting effect: Exploiting the leaky-vasculature ("funnel") phenomenon of tumor tissue, the 2*CpG 2006-CD40 aptamer-DNA nucleic-acid nanotherapeutic more readily enters tumor tissue.

2. TLR9 stimulation: CpG 2006 stimulates TLR9 receptors on immune cells, thereby activating immune cells and eliciting antitumor responses.

3. CD40-aptamer-mediated costimulation: Incorporation of a CD40 aptamer (CD40L) confers enhanced immune-activating capacity. The CD40 aptamer binds CD40 receptors on immune cells and activates immune cells such as dendritic cells, B cells, and macrophages.

4. Relief of tumor immunosuppression: Engagement of the CD40 aptamer with CD40 activates dendritic cells, thereby counteracting tumor-imposed immunosuppression and restoring the immune system's ability to attack tumor cells.

5. Immune-system activation: Activation of the CD40-CD40L signaling pathway promotes dendritic-cell maturation and presentation of antigen to T cells. In addition, CD40L promotes B-cell proliferation and antibody secretion. These immune responses facilitate clearance of tumor cells.

6. Tumor-cell clearance: By relieving immunosuppression and activating the immune system, the 2*CpG 2006-CD40 aptamer-DNA actively immunostimulatory nanotherapeutic enables more effective recognition and elimination of tumor cells, thereby inhibiting tumor growth and dissemination.

7. Enhanced therapeutic specificity: The targeting-mediation function of CD40L enhances therapeutic specificity. Because CD40 is primarily expressed on immune cells, CD40-CD40L signaling acts mainly on immune cells, reducing adverse effects on normal tissues.

8. Reduced adverse effects: As the 2*CpG 2006-CD40 aptamer-DNA nanotherapeutic acts predominantly within tumor tissue and CD40L provides targeting mediation, treatment-related adverse effects are reduced and safety is improved.

In summary, this specifically targeted, actively immunostimulatory dual-combination nucleic-acid nanotherapeutic integrates the carrier's passive-targeting effect, the humanized TLR9 agonist CpG 2006, and a CD40 aptamer possessing both targeting-mediation and costimulatory functions. These cooperative mechanisms confer improved efficacy, precision, and safety in tumor therapy by relieving tumor immunosuppression, activating the immune system, and promoting tumor-cell clearance.

Drug 5) 4*CpG 2006-CD40 aptamer-DNA carrier

This composition is a specifically targeted, actively immunostimulatory, dual-combination nucleic-acid nanotherapeutic. Building upon the foregoing Drug 4) 2*CpG 2006-CD40 aptamer-DNA nanotherapeutic, two CpG 2006 segments are further arranged in tandem and, in a sequence-extension manner, appended to two 5' ends of the nanocarrier's core scaffolstrand ds; the CD40 aptamer is appended to the 5' end of the remaining core scaffolstrand d. Thus, while preserving CpG 2006 functionality, the CpG 2006 loading is doubled, which enhances loading and delivery efficiency. The mechanism and key logic are as follows:

1. Twofold CpG 2006 loading: By tandemly appending two CpG 2006 segments to two 5' ends of the nanocarrier, the payload of CpG 2006 per nanoparticle is doubled, thereby increasing the efficiency of immune-cell stimulation.

2. Preservation of CD40-aptamer function: While increasing CpG 2006 loading, the CD40 aptamer is appended to the 5' end of the remaining core scaffolstrand d so that its targeting and costimulatory functions are maintained, thereby enhancing immunostimulatory activity without sacrificing CD40-mediated targeting.

3. Improved loading and delivery efficiency: The twofold CpG 2006 payload increases the amount of CpG 2006 deliverable to tumor tissue and immune cells at a given dose, thereby improving therapeutic performance.

4. Augmented immune activation: With doubled CpG 2006 loading, activation of TLR9 and stimulation of immune cells are strengthened, further relieving tumor-induced immunosuppression, activating the immune system, and promoting tumor-cell clearance.

5. Retention of nanocarrier attributes: The design retains the nanocarrier's passive-targeting effect-facilitated entry into tumor tissue via leaky ("funnel") tumor vasculature-while the scaffold confers structural stability that protects CpG 2006 and the CD40 aptamer, preserving in vivo activity.

6. Enhanced therapeutic specificity: Targeting mediation by the CD40 aptamer improves specificity. Because CD40 is primarily expressed on immune cells, CD40-CD40L pathway engagement acts mainly on immune cells, reducing off-target effects on normal tissues.

7. Reduced adverse effects: As the 4*CpG 2006-CD40 aptamer-DNA nanotherapeutic acts predominantly within tumor tissue and includes CD40-mediated targeting, treatment-related adverse effects are reduced and safety is improved.

In summary, relative to the 2*CpG 2006-CD40 aptamer-DNA nanotherapeutic, this specifically targeted, actively immunostimulatory dual-combination construct doubles the CpG 2006 loading to enhance loading and delivery efficiency. The cooperative mechanisms confer improved therapeutic efficacy, precision, and safety, more effectively relieving tumor immunosuppression, activating the immune system, and clearing tumor cells.

Drug 6) 2*CpG 1826-CD40 aptamer-2*mannose-DNA carrier

This composition is a specifically targeted, actively immunostimulatory dual-combination nucleic-acid nanotherapeutic. On the basis of the 2*CpG 1826-CD40 aptamer-DNA construct, two mannose moieties are appended, in a sequence-extension manner, to two 3' ends of the core scaffolstrand ds of the nanocarrier, thereby enhancing composite targeting mediation to the tumor microenvironment, increasing intratumoral immune responsiveness, and enabling dose reduction with a concomitant decrease in potential adverse effects. The mechanism of action is as follows:

1. Enhanced targeting capability: By installing two mannose moieties at two 3' ends of the nanocarrier's core scaffolstrand ds, the nanotherapeutic is endowed with stronger targeting capability. Mannose exhibits specific affinity for mannose-receptor proteins on tumor-associated cells, thereby achieving active targeting to tumor tissue.

2. Increased immune-response effect: Mannose-modified nucleic-acid nanoparticles display higher uptake in tumor tissue, thereby increasing the local concentration of CpG 1826. This facilitates activation of a greater number of immune cells and enhances the immune response within tumor tissue.

3. Dose reduction: Owing to the strengthened targeting capability, a lower dose can achieve comparable therapeutic effect, reducing accumulation in normal tissues.

4. Fewer adverse effects: Improved targeting and lower dosing decrease off-target accumulation in normal tissues, thereby reducing potential adverse effects.

5. Retention of CD40-aptamer function: The nanotherapeutic retains the CD40 aptamer, which further augments immune-cell activation and immune responses.

6. Activation of the TLR9 pathway: CpG 1826 is a TLR9 agonist that binds TLR9 receptors and activates downstream signaling in immune cells. This induces production of inflammatory cytokines and activates antitumor immune cells such as dendritic cells (DCs) and macrophages.

7. Costimulatory activity of the CD40 aptamer: By binding CD40 receptors on immune cells, the CD40 aptamer provides costimulatory signaling, further activating antitumor immune cells and enhancing immune responses.

8. Augmented immunologic clearance of tumor cells: Through the foregoing mechanisms, the 2*CpG 1826-CD40 aptamer-2*mannose-DNA nanotherapeutic activates immune cells to more effectively recognize and clear tumor cells.

9. Inhibition of tumor growth: Cytokines and immune cells activated by the treatment cooperatively suppress tumor-cell growth and dissemination, thereby achieving a therapeutic antitumor effect.

Accordingly, this specifically targeted, actively immunostimulatory dual-combination nucleic-acid nanotherapeutic achieves effective tumor immunotherapy through multiple mechanisms-composite targeting mediation, activation of the TLR9 pathway, and CD40-aptamer costimulation-thereby improving efficacy and safety while enabling dose reduction and mitigating potential adverse effects.

Drug 7) CpG 1826-CD40 aptamer-mannose-MUC1 aptamer-DNA carrier (a nucleic-acid immunotherapeutic)

This composition is a specifically targeted, actively immunostimulatory triple-combination nucleic-acid nanotherapeutic for tumor immunotherapy. It constitutes an improvement over the foregoing CpG 1826-CD40 aptamer-2*mannose-DNA construct by additionally incorporating a MUC1 aptamer (preferably by sequence extension), thereby enhancing targeting mediation to tumors with high MUC1-receptor expression.

1. CpG 1826: CpG 1826 is a CpG oligodeoxynucleotide analog that activates the Toll-like receptor 9 (TLR9) pathway. TLR9 is an essential pattern-recognition receptor of the immune system that recognizes CpG DNA motifs in pathogens. Upon TLR9 activation, inflammatory cytokines are produced, thereby eliciting an immune response.

2. CD40 aptamer: CD40 ligand (CD40L) is a surface molecule that interacts with the CD40 receptor. The CD40L-CD40 signaling pathway plays a key role in regulating the activity of B cells, T cells, and other immune cells. Activation of the CD40 pathway enhances tumor-specific T-cell activity and exerts antitumor effects; in this composition, a CD40 aptamer is employed to engage the CD40 pathway.

3. Mannose: Mannose is a carbohydrate that can be used in drug-delivery systems. Here, mannose functionalization of the nanocarrier helps improve stability and bioavailability.

4. MUC1 aptamer: MUC1 is a protein highly expressed on the surface of many tumor cells. Incorporation of a MUC1 aptamer enables specific targeting of MUC1-expressing tumor cells, permitting the drug to act more precisely on tumor cells and thereby reducing effects on normal cells.

5. DNA: A synthetic DNA sequence self-assembles via complementary base-pairing to form the nucleic-acid nanocarrier, which interconnects the foregoing components and provides structural system stability, maintaining aptamer three-dimensional conformation, recognition/binding activity, and appropriate inter-element spacing, so as to constitute a complete nucleic-acid immunotherapeutic.

In summary, the specifically targeted, actively immunostimulatory triple-combination nucleic-acid nanotherapeutic is a targeted immunotherapy for tumors that highly express the MUC1 receptor. By combining CpG 1826, a CD40 aptamer, mannose, a MUC1 aptamer, and DNA, the composition can function as follows:

1. Elicit an immune response: CpG 1826 activates the TLR9 pathway, and CD40L activates the CD40 signaling pathway, thereby promoting the production of inflammatory cytokines and the activation of immune cells to mount an anti-tumor immune response.

2. Provide specific targeting: The incorporated MUC1 aptamer enables specific recognition and targeting of MUC1-expressing tumor cells, enhancing therapeutic effect while reducing impact on normal cells.

3. Serve as a drug-delivery vehicle: Mannose functionalization of the nanocarrier helps improve drug stability and bioavailability, enabling the composition to remain effective in vivo and to reach the intended site of action.

4. DNA interconnection of components: The synthetic DNA sequence self-assembles to form the nucleic-acid nanocarrier that links the foregoing components into an integrated structure.

Accordingly, this specifically targeted, actively immunostimulatory triple-combination nucleic-acid nanotherapeutic is expected to provide an effective modality for tumor immunotherapy, particularly for tumor cells expressing MUC1. However, further experimental and clinical studies are needed to evaluate safety, efficacy, and potential adverse effects so as to ensure that the intended therapeutic outcomes can be achieved in practical use.

Drug 8) 2*CpG 2006-CD40 aptamer-AmiR-21-3*mannose-DNA carrier (a nucleic-acid-based combined gene-immunotherapy agent)

This composition is a specifically targeted, actively immunostimulatory and gene-regulatory triple-combination nucleic-acid nanotherapeutic. On the basis of the 2*CpG 2006-CD40 aptamer-mannose-DNA carrier, introduction of anti-miR-21 (AmiR-21) adds a gene-silencing function to the pre-existing targeted immunostimulation. The integrated, antitumor composite mechanism of action is as follows:

1. 2*CpG 2006: CpG 2006 is a CpG oligodeoxynucleotide analog that activates the Toll-like receptor 9 (TLR9) pathway. TLR9 is a key pattern-recognition receptor that detects CpG DNA motifs. Upon activation, it induces production of inflammatory cytokines and elicits an immune response.

2. CD40 aptamer: A CD40-targeting aptamer engages the CD40 signaling pathway, which is critical for regulating B cells, T cells, and other immune cells. Activation of CD40 signaling enhances tumor-specific T-cell activity and thereby exerts antitumor effects.

3. AmiR-21 (anti-miR-21): Anti-miR-21 is an inhibitor directed against microRNA-21 (miR-21), which is overexpressed in many tumor types and associated with tumor growth, invasion, and metastasis. Inhibiting miR-21 activity suppresses cancer-cell proliferation, invasion, and metastatic potential, thereby restraining tumor progression.

4. 3*mannose: Three mannose moieties provide composite targeting guidance by specifically mediating enhanced binding to mannose receptors that are overexpressed in tumor tissue, thereby augmenting targeting to the tumor microenvironment.

5. DNA carrier: A synthetic DNA sequence self-assembles via complementary base-pairing to form a highly biocompatible nucleic-acid nanocarrier that interconnects the foregoing components into an integrated nucleic-acid immunotherapeutic.

In summary, the composite antitumor mechanism of this specifically targeted, actively immunostimulatory and gene-regulatory triple-combination nucleic-acid nanotherapeutic is as follows:

1. Immune activation: CpG 2006 activates the TLR9 pathway, and the CD40 aptamer activates the CD40 signaling pathway, thereby enhancing the activity of tumor-specific T cells. The activated immune cells recognize and attack tumor cells, thereby inhibiting tumor progression.

2. Suppression of oncogenic gene expression: AmiR-21 suppresses the activity of miR-21, reducing the expression of genes associated with tumor growth, invasion, and metastasis. This prevents cancer-cell proliferation, invasion, and metastasis, further restraining tumor development.

3. Drug delivery: The DNA nanocarrier increases drug stability and bioavailability, allowing the composition to remain effective in vivo and reach the site of action.

4. Integrated action: The synthetic DNA sequence interconnects the components so that the nanonucleic-acid immunotherapeutic forms an integrated structure, thereby achieving the combined effects of immune activation and suppression of oncogenic gene expression.

Accordingly, this specifically targeted, actively immunostimulatory and gene-regulatory triple-combination nucleic-acid nanotherapeutic acts through both immune activation and suppression of oncogenic gene expression to achieve composite inhibition of tumors. This integrated approach is expected to improve therapeutic outcomes and reduce the risk of tumor progression and metastasis. Further experimental and clinical studies to evaluate safety, efficacy, and potential adverse effects will help ensure that the intended therapeutic effects can be achieved in practical application.

Drug 9) 2*CpG 2006-CD40 aptamer-CD16a aptamer-CTLA-4 aptamer-DNA carrier

This composition is a specifically targeted, actively immunostimulatory quadruple-combination nucleic-acid nanotherapeutic. Building on the foregoing 2*CpG 2006-CD40 aptamer-DNA immunostimulant, it further introduces CD16a and CTLA-4 aptamers to address tumors with a more severe immunosuppressive milieu or relapsed/refractory disease. The mechanism of action is summarized as follows:

1. CpG 2006: A CpG oligodeoxynucleotide analog that activates the Toll-like receptor 9 (TLR9) pathway. TLR9 is a key pattern-recognition receptor that detects CpG DNA; its activation induces inflammatory cytokine production and elicits immune responses.

2. CD40 aptamer: CD40 is a receptor that interacts with CD40L (CD154). The CD40L-CD40 signaling axis plays a pivotal role in regulating B cells, T cells, and other immune cells. Engagement of this pathway enhances tumor-specific T-cell activity and exerts antitumor effects.

3. CD16a aptamer: CD16a is an Fc receptor principally expressed on natural killer (NK) cells and on subsets of macrophages. CD16a recognizes antibody-tumor-cell complexes and triggers NK-cell and macrophage antitumor effector functions. Introduction of a CD16a aptamer potentiates immune-cell-mediated tumor suppression.

4. CTLA-4 aptamer: CTLA-4 (cytotoxic T-lymphocyte-associated antigen 4) is an immune checkpoint protein expressed on activated T cells. By binding B7 molecules (CD80/CD86), CTLA-4 suppresses T-cell activity and dampens immune responses. In tumor immunotherapy, blockade of CTLA-4 restores T-cell function and enhances antitumor immunity. Incorporation of a CTLA-4 aptamer inhibits CTLA-4/B7 interactions, thereby releasing checkpoint inhibition and augmenting T-cell responses.

5. DNA carrier: A synthetic DNA sequence that interconnects the foregoing components to form an integrated nucleic-acid immunotherapeutic.

In summary, building upon the original immunostimulatory composition, this specifically targeted, actively immunostimulatory quadruple-combination nucleic-acid nanotherapeutic introduces CD16a and CTLA-4 aptamers to achieve the following:

1. Enhancement of NK-cell and macrophage antitumor activity: Incorporation of a CD16a aptamer augments the ability of natural killer (NK) cells and macrophages to recognize and attack tumor cells.

2. Release of inhibition on T-cell activity: Incorporation of a CTLA-4 aptamer blocks CTLA-4 binding to B7 molecules (CD80/CD86), thereby relieving suppression of T-cell activity and enhancing antitumor immune responses.

3. Synergistic effect via integrated combination immunotherapy: CpG 2006 and the CD40 aptamer, assembled on a DNAh nucleic-acid nanocarrier, constitute an integrated, actively combined immunotherapy in which agonist-mediated activation is coupled with checkpoint "brake" release, while also conferring targeting and cooperative effects. This strategy is advantageous for restoring patient immune responsiveness while helping to avoid excessive immune activation.

Accordingly, this integrated, specifically targeted, actively immunostimulatory quadruple-combination nanotherapeutic exerts synergy in immune activation and in overcoming an immunosuppressive milieu, and is expected to improve therapeutic outcomes for tumors with severe immunosuppression or relapsed/refractory disease. However, further experimental and clinical studies are required to evaluate safety, efficacy, and potential adverse effects to ensure that the intended therapeutic performance can be achieved in practical use.

Drug 10) 2*CpG 2006-CTLA-4 aptamer-PD-L1 aptamer-DNA carrier (an actively combined immunotherapeutic)

This composition is a specifically targeted, actively immunostimulatory triple-combination nucleic-acid nanotherapeutic in which immune checkpoint inhibitors (CTLA-4 and PD-L1 aptamers) are combined with an immunostimulant (CpG 2006) to inhibit tumor growth. The mechanism of action is analyzed as follows:

1. Immunostimulation by CpG 2006: CpG 2006 is a CpG oligodeoxynucleotide analog that activates the Toll-like receptor 9 (TLR9) pathway. TLR9 is a pattern-recognition receptor that detects CpG DNA in pathogens. Upon TLR9 activation, inflammatory cytokines are produced, thereby eliciting immune responses. Dendritic cells (DCs) and other antigen-presenting cells (APCs) are activated to capture, process, and present tumor antigens, thereby initiating activation of tumor-specific T cells.

2. Immunomodulation by a CTLA-4 aptamer: CTLA-4 (cytotoxic T-lymphocyte-associated antigen-4) is an immune-checkpoint receptor expressed on activated T cells. CTLA-4 competes with CD28 for binding to B7 molecules (CD80/CD86), suppressing T-cell activity and dampening immune responses. In tumor immunotherapy, incorporation of a CTLA-4 aptamer blocks CTLA-4/B7 interactions, inhibits CTLA-4-mediated suppressive signaling, restores or enhances T-cell activity, augments antitumor immunity, and improves antitumor efficacy.

3. Immune checkpoint release by a PD-L1 aptamer: PD-L1 (programmed death-ligand 1) is an immune-checkpoint protein expressed on the surfaces of tumor cells and immune cells. By binding its receptor PD-1 (programmed death-1), PD-L1 suppresses T-cell activity and establishes an immunosuppressive environment. In tumor immunotherapy, blocking the interaction between PD-L1 and PD-1 can restore T-cell activity and enhance antitumor immune responses.
In this drug, a PD-L1 aptamer binds PD-L1 within the PD-1/PD-L1 axis and prevents engagement of PD-1 with PD-L1, thereby releasing PD-1/PD-L1-mediated suppression of T cells. As a result, T-cell antitumor activity is restored, enabling the cells to attack and kill tumor cells.

4. DNA carrier: A synthetic DNA sequence interconnects the foregoing components to form an integrated nucleic-acid immunotherapeutic.

These three components act synergistically to activate and amplify immune responses for effective tumor inhibition. Specifically, CpG 2006 activates immune cells to initiate the response, while the CTLA-4 and PD-L1 aptamers relieve checkpoint-mediated suppression to enhance T-cell antitumor function. Additional effects include:

4. Antiproliferative activity against tumor cells: Under treatment, activated tumor-specific T cells recognize and attack tumor cells, abrogating their capacity for growth and dissemination. Activated T cells also release cytokines such as tumor necrosis factor (TNF) and interferon-$\gamma$ (IFN-$\gamma$), which suppress tumor-cell proliferation and growth.

5. Anti-angiogenic activity: In addition to killing tumor cells, activated immune cells inhibit tumor angiogenesis. Cytokines produced by immune cells (e.g., IFN-$\gamma$) suppress proliferation of tumor-associated endothelial cells, weakening tumor blood supply and causing tumor-cell death due to deprivation of oxygen and nutrients.

6. Anti-metastatic activity: By activating the immune system and enhancing immune surveillance, the agent helps prevent tumor-cell dissemination and metastasis; immune pressure diminishes tumor-cell migratory capacity, thereby reducing metastatic risk.

7. Induction of antitumor immune memory: After tumor clearance, a portion of activated immune cells differentiates into memory cells, establishing long-term immune memory. Upon tumor recurrence, memory cells rapidly recognize and eliminate tumor cells, improving long-term therapeutic outcomes.

8. Personalized immunotherapy: The specifically targeted, actively combined triple-combination nanotherapeutic supports individualized treatment. Because tumors in different patients may exhibit distinct immunosuppressive mechanisms, targeted modulation of the CTLA-4 and PD-L1 pathways enables more precise and effective immunotherapy.

9. Reduction of immunotherapy-related adverse effects: High-affinity, PD-L1-aptamer-dominated precise targeting of tumor and immune cells, together with secondary adjacent recognition by CTLA-4, reduces damage to normal tissues and mitigates adverse effects. Synergy among components can also permit dose reduction, further decreasing undesirable reactions.

10. Combination with other modalities: This integrated, specifically targeted, actively combined triple-combination nanotherapeutic can be used in combination with other treatments (e.g., surgery, radiotherapy, chemotherapy) to achieve greater efficacy. Immunotherapy can potentiate these modalities while lowering risks of recurrence and metastasis.

In summary, through multi-pathway synergy, this integrated, specifically targeted, actively immunostimulatory triple-combination nucleic-acid nanotherapeutic activates and augments immune responses to achieve effective tumor suppression. It offers advantages including personalization, reduced adverse effects, and enhanced compatibility with other therapies, thereby providing a new option for tumor treatment. Further experimental and clinical evaluation of safety, efficacy, and potential adverse effects is warranted to ensure attainment of the intended therapeutic outcomes in practical use.

Drug 11) CpG 2006-CD40 aptamer-PD-L1 aptamer-DNA carrier (hereinafter "CCPD3")

This composition is a specifically targeted, actively immunostimulatory triple-combination nucleic-acid nanotherapeutic. In animal treatment studies of relapsed small-cell lung cancer (SCLC) and glioma or brain metastases, it achieved significant inhibition of tumor growth, prolongation of survival, and tumor clearance.

1. Immunostimulatory activity: CpG 2006 is a CpG oligodeoxynucleotide analog that activates the Toll-like receptor 9 (TLR9) pathway, inducing production of inflammatory cytokines. TLR9 activation enhances the activity of antigen-presenting cells (APCs), such as dendritic cells, thereby increasing T-cell activation and tumor-specific immune responses. This immunostimulatory effect helps initiate immune attack on tumors.

2. Immunologic synergy: Upon binding of the CD40 aptamer to the CD40 receptor, APC activity is further enhanced, which in turn augments T-cell activation and tumor-specific immune responses. Synergy between the CD40 aptamer and CpG 2006 further strengthens immune attack on tumors.

3. Immune-checkpoint blockade: PD-L1 (programmed death-ligand 1) is an immune-checkpoint protein that, upon binding to its receptor PD-1 (programmed death-1), suppresses T-cell activity and creates an immunosuppressive milieu. In this combined immunotherapeutic, blockade of PD-L1/PD-1 engagement restores T-cell activity and enhances antitumor immune responses.

4. Lymphocyte infiltration: The combined immunotherapeutic increases infiltration of CD8$^+$ T cells within tumor tissue, thereby improving tumor susceptibility to immune attack. Such infiltration is critical to immune-mediated tumor clearance because it enables direct contact with, and attack upon, tumor cells.

5. Antitumor cytotoxicity: By eliciting tumor-specific immune responses, the agent enhances cytotoxic T-lymphocyte (CTL) killing of tumor cells, thereby achieving tumor clearance.

6. Suppression of immunosuppressive cells: The agent suppresses the function of immunosuppressive cells in the tumor microenvironment-such as regulatory T (Treg) cells and myeloid-derived suppressor cells (MDSCs)-thereby strengthening antitumor immunity.

7. Inhibition of tumor growth-promoting factors: By modulating the expression and signaling of tumor growth-promoting factors (e.g., VEGF, TGF-β), the agent inhibits tumor angiogenesis, invasion, and metastasis, further limiting tumor growth and spread.

8. Systemic immune response: During inhibition of tumor growth, the agent induces systemic immune responses, enabling recognition and attack of tumor cells throughout the body and effectively preventing recurrence and metastasis.

9. Durable immune memory: The agent induces formation of immunological memory cells such that, after tumor clearance, the immune system remains vigilant against tumor cells. This supports long-term therapeutic benefit and reduces the risk of relapse.

In summary, the CpG 2006-CD40 aptamer-PD-L1 aptamer-DNA carrier, a specifically targeted, actively immunostimulatory triple-combination nucleic-acid nanotherapeutic, achieves potent immune-mediated clearance of relapsed small-cell lung cancer and glioma or brain metastases through coordinated functions across multiple axes and effector molecules. These coordinated functions include activation of immune responses, immunologic synergy, immune-checkpoint blockade, lymphocyte infiltration, antitumor cytotoxicity, suppression of immunosuppressive cells, inhibition of tumor growth factors, induction of systemic immune responses, and establishment of durable immune memory. Owing to this multi-effect profile, the combined agent acts concomitantly at multiple levels to effect efficient tumor clearance.

In animal studies, this specifically targeted, actively immunostimulatory triple-combination nanotherapeutic produced significant inhibition of tumor growth, prolonged survival, and tumor clearance. For clinical application, additional research and clinical trials are preferably conducted to evaluate safety, efficacy, and potential adverse effects. In practice, depending on tumor type and individual patient characteristics, optimization may include aptamer selection, drug design and refinement, carrier selection and optimization, combination-immunotherapy regimens, and individualized treatment plans, so as to improve therapeutic efficacy, reduce the risk of adverse effects, and enhance patient quality of life.

Additionally, within this triple-combination agent the PD-L1 aptamer exhibits high affinity and serves as the primary targeting and checkpoint-blocking moiety, binding PD-L1 in the PD-1/PD-L1 pathway to prevent PD-1/PD-L1 engagement and thereby release and restore the tumor-killing function of clonally expanding T cells. By contrast, the CD40 aptamer has relatively lower affinity and provides secondary targeting mediation together with costimulatory signaling. This affinity-tiered targeting design in oncology affords the following advantages:

1. Optimization of immune responses: By employing a high-affinity PD-L1 aptamer to dominate the therapeutic effect, the PD-1/PD-L1 checkpoint pathway can be more effectively blocked, thereby activating systemic immune responses within the tumor microenvironment. Meanwhile, although the CD40 aptamer exhibits comparatively lower affinity, its adjacent recognition and costimulatory activities can, in a targeted manner, enhance immune-cell activation and expansion, further augmenting antitumor immunity.

2. Balancing immune activation and adverse effects: Owing to the lower affinity of the CD40 aptamer, its secondary targeting-mediation and costimulatory function can temper excessive immune activation. This helps reduce off-target immunotoxic effects potentially associated with agonists, such as autoimmune disorders and inflammatory reactions, while sustaining antitumor immune activation.

3. Improved therapeutic selectivity: An affinity-tiered design utilizing a high-affinity PD-L1 aptamer together with a lower-affinity CD40 aptamer enhances selectivity within tumor tissue. Consequently, the therapeutic agent is more likely to act on tumor cells rather than normal cells, thereby reducing injury to normal tissues.

4. Personalized therapy: The affinity-tiered targeting design can be adjusted according to tumor type, disease course, and the patient's immune status. This enables individualized treatment regimens that achieve higher efficacy with a lower risk of adverse effects.

In summary, the affinity-tiered targeting design within this specifically targeted, actively immunostimulatory triple-combination nucleic-acid nanotherapeutic offers multiple advantages in oncology, including optimization of immune responses in the tumor microenvironment, balanced activation versus toxicity, improved therapeutic selectivity, and personalization.

Additionally, this therapeutic agent can be combined with other antitumor treatment modalities-such as radiotherapy, chemotherapy, targeted therapy, and other immunotherapies-to achieve greater efficacy with fewer adverse effects. Within such combination regimens, the affinity-tiered targeting design may be tuned to the patient's specific condition and therapeutic needs in order to obtain optimal therapeutic outcomes.

CCPD3 Variant 1. The principal difference from CCPD3 is that this agent employs an alternative PD-L1 aptamer (HQ PD-L1). Its mechanism of action is identical to that of CCPD3. Owing to the consecutive arrangement of guanosine (G) residues within the HQ PD-L1 aptamer, its stability and affinity are further improved. In addition, the HQ PD-L1 aptamer has a sequence length of only 33 nucleotides, which confers better cost-effectiveness, more robust quality control, and higher synthetic efficiency. The advantages of using the HQ PD-L1 aptamer are as follows:

1. Stability and affinity: The consecutive guanosine residues enhance the stability and affinity of the HQ PD-L1 aptamer, thereby facilitating more effective blockade of the PD-1/PD-L1 immune-checkpoint pathway and improving immunotherapeutic efficacy.

2. Economics: With a length of only 33 nt, the HQ PD-L1 aptamer is less costly to synthesize, reducing overall treatment cost and improving accessibility.

3. Quality control and synthetic efficiency: The shorter sequence improves quality-control robustness and manufacturing (synthetic) efficiency, helping ensure product quality and performance while reducing inter-batch variability.

4. Scope of use: As with CCPD3, this agent is applicable to multiple tumor types, particularly PD-L1-expressing tumors such as small-cell lung cancer, glioma, and renal cell carcinoma.

Accordingly, the CpG 2006-CD40 aptamer-HQ PD-L1 aptamer-DNA carrier is an advantageous, specifically targeted, actively immunostimulatory triple-combination nucleic-acid nanotherapeutic. However, prior to clinical application, further studies and clinical trials are required to evaluate safety, efficacy, and potential adverse effects. In practice, optimization may consider aptamer selection, drug design and optimization, carrier selection and optimization, combination-immunotherapy strategies, and individualized treatment regimens, so as to improve therapeutic effect, reduce adverse-effect risk, and enhance patient quality of life.

CCPD3 Variants 2 and 3. These variants differ from CCPD3 in that the sequences a, b, and c are shortened, while the mechanism of action remains the same. Shortening the lengths of the carrier-backbone a/b/c sequences is intended-while maintaining sufficient structural stability-to reduce sequence-synthesis complexity, improve quality-control capability, increase the payload fraction of effector molecules and the specific surface area, lower overall cost, and improve efficiency.

CCPD3 Variant 4. The distinction from CCPD3 likewise lies in shortening the sequences a, b, and c, and in shortening XDPD-L1; the mechanism of action is identical to that of CCPD3. This variant affords increased configurational optionality.

CCPD3 Variants 5 and 6. These differ from CCPD3 in that four strands- a, b, c, and d -are employed. This assembly scheme is adopted primarily to address the excessive length of the PD-L1 aptamer sequence, which reduces synthetic efficiency, makes it difficult to achieve high purity, and consequently lowers nanoparticle assembly yield. The approach constitutes a comprehensive comparative strategy, recognizing that assembly efficiency is determined chiefly by the purity of each single strand.

CCPD3 Variant 7. This variant differs from CCPD3 by employing a universal carrier sequence (the sequences a, b, and c) furnished with a single-stranded bridging. The three desired oligonucleotide effector molecules (the sequences d, e, and f) are joined to the ends of the carrier's three sequences via complementarity to the adhesive-bridge sequences. In other words, the construct is split into six strands, using a six-strand assembly strategy in which the core carrier backbone introduces a universal "adhesive-bridge" to allow different aptamers or other effector molecules to be arbitrarily configured into various integrated combinations, thereby facilitating new-drug development. This may, to some extent, result in increased molecular weight and a potential increase in overall cost.

CCPD3 Variant 8. This variant differs from CCPD3 in that the sequences a, b, and are shortened and the CD40 aptamer and PD-L1 aptamer appended to the 5' ends of the sequences b and c, respectively, are modified at the first four nucleotides with 2'-O-methoxyethyl (2'-O-MOE); moreover, the cytidine (Cm) residues bear a 5-methyl substituent on the base (canonical cytidine lacks a 5-methyl group). The resulting sequence architecture affords a more stable drug construct.

CCPD3 Variant 9. This variant differs from Variant 8 in that the CD40 aptamer and PD-L1 aptamer linked to the 5' ends of the sequences b and c are modified at the first five nucleotides with 2'-O-MOE, with the Cm residues bearing a 5-methyl substituent on the base; additionally, the CD40 aptamer sequence at the 5' end of the sequence b lacks the first nucleotide. The resulting sequence architecture affords a more stable drug construct.

CCPD3 Variant 10. This variant differs from Variant 9 only in that the PD-L1 aptamer sequence linked to the 5' end of the sequence c is different; likewise, the first five nucleotides are 2'-O-MOE-modified, with the Cm bases bearing a 5-methyl substituent (canonical cytidine lacks a 5-methyl group). The resulting sequence architecture affords a more stable drug construct.

CCPD3 Variant 11. This variant differs from CCPD3 Variant 10 in that the sequence c is divided into two segments: one segment is the sequence c bearing, at its 5' end, a 14-nt single-stranded adhesive-bridge (linker) sequence (i.e., SEQ ID No. 299: CGCGGCTCGCGGCTCGCGGCTCGCGGCCATAGCCGTGGGCGTCGC ); the other segment is a PD-L1 aptamer sequence (SEQ ID No. 241: ACGGGCCACATCAACTCATTGATAGACAATGCGTCCACTGCCCGT ) that is joined via the sequence complementary to the bridging. In addition, the PD-L1 aptamer sequence is modified at the 3'-end nucleotides at positions 1-5 with 2'-O-MOE. The foregoing is synthesized in the form of four strands, affording high synthetic efficiency and low cost. The adhesive-bridge pairing includes 14 base pairs, for a total of 40 hydrogen bonds, conferring high connection stability.

CCPD3 Variant 12. This variant differs from Variant 11 only in that the PD-L1 aptamer sequence joined via the sequence complementary to the bridging is PD-L1-ZD (i.e., SEQ ID No. 249: CTACGAGACGAACTTATGCGTAATAA TGACTGTCGTAG ), which likewise are 2'-O-MOE modified at the 3'-end nucleotides at positions 1-5. As above, the construct is synthesized as four strands with high synthetic efficiency and low cost, and the bridging includes 14 base pairs totaling 40 hydrogen bonds, providing high connection stability.

Drug 12) CD16a aptamer-CTLA-4 aptamer-CpG 2006-DNA carrier

This composition is a specifically targeted, actively immunostimulatory triple-combination nucleic-acid nanotherapeutic. As indicated above, the functions of the respective effector molecules are as follows:

1. CD16a aptamer: CD16a is a low-affinity Fc receptor (FcγRIIIa) typically expressed on natural killer (NK) cells and macrophages. By recognizing antigen-antibody complexes on tumor cells, CD16a mediates antitumor activity. A CD16a aptamer can enhance NK- and macrophage-mediated killing of tumor cells.

2. CD40 aptamer: CD40 is a costimulatory molecule in the immune system; upon engagement with CD40L, it activates macrophages and dendritic cells (DCs), thereby promoting presentation of tumor antigens and activation of T-cell immune responses. A CD40 aptamer augments antitumor immune responses and further improves therapeutic efficacy.

3. CpG 2006: CpG 2006 is an immunostimulatory oligonucleotide that activates dendritic cells and B cells, enhancing their antigen-presentation functions. By stimulating immune cells, CpG 2006 improves recognition and presentation of tumor antigens and thereby strengthens immune responses.

4. DNA carrier: A nucleic-acid nanocarrier that integrates the foregoing three components within a single molecular construct to achieve multi-pronged attack on tumor cells. Consolidation of these moieties within one nanonucleic-acid entity improves targeting, bioavailability, and therapeutic performance.

Summary. The CD16a aptamer-CD40 aptamer-CpG 2006-DNA carrier specifically targeted, actively immunostimulatory triple-combination nanotherapeutic mounts multi-pathway attack on tumor cells by combining NK- and macrophage-mediated antitumor mechanisms, CD40-mediated activation of T-cell responses, and CpG 2006-mediated enhancement of antigen presentation. This multi-pathway synergy improves therapeutic efficacy and helps overcome tumor immune evasion and drug resistance.

1. Tumor microenvironment modulation: This specifically targeted, actively immunostimulatory triple-combination nucleic-acid nanotherapeutic is configured to remodel the tumor microenvironment from an immunosuppressive state to an immune-activated state. By activating dendritic cells, macrophages, and NK cells and enhancing their antigen-presentation functions, it improves recognition and clearance of tumor antigens.

2. Reinforcement of immune memory: By activating T-cell immune responses, the agent promotes the generation of immunological memory cells, which can be rapidly reactivated upon tumor recurrence, thereby improving the long-term efficacy of immunotherapy.

3. Reduction of adverse effects: Integration of multiple aptamers within a single nucleic-acid nanoparticle increases targeting specificity and bioavailability, reducing the risk of adverse effects. In addition, synergy among the three aptamers can permit dose reductions for individual aptamers, thereby lowering the patient's drug burden and toxicity.

4. Personalized therapy: The specifically targeted, actively immunostimulatory triple-combination nucleic-acid nanotherapeutic can be optimized and adjusted according to a patient's specific circumstances. For example, aptamers may be designed to target particular tumor antigens, or the ratios and dosages of the aptamers may be adjusted based on the patient's immune status, thereby achieving individualized treatment.

Drug 13) 2* CpG 2006-CD40 aptamer-PD-L1 aptamer-C12 aptamer-DNA carrier

This composition is a specifically targeted, actively immunostimulatory quadruple-combination nucleic-acid nanotherapeutic. Based on the 2*CpG 2006-CD40 aptamer-PD-L1 aptamer-DNA construct, it introduces a C12 aptamer directed to a protein highly expressed on small-cell lung cancer (SCLC) cells, thereby enhancing targeting to the tumor microenvironment-particularly in patients with absent or very low PD-L1 expression. The mechanism of action is as follows:

1. Targeting: The C12 aptamer (SEQ ID No. 164: GTGGATTGTTGTGTTCTGTTGGTTTTTGTGTTGTC ) is a ligand that specifically binds HDLBP (high-density lipoprotein-binding protein) on the surface of SCLC tumor cells. In PD-L1-negative or low-PD-L1-expression patients, the C12 aptamer increases tumor-cell targeting and thereby enhances therapeutic efficacy.

2. Immunostimulation: The CD40 aptamer activates dendritic cells and enhances antigen-presentation functions, thereby activating T-cell immune responses; CpG 2006 stimulates immune cells to secrete antitumor cytokines and further augments the immune response.

3. Immune-checkpoint blockade: The PD-L1 aptamer blocks the PD-1/PD-L1 pathway, restoring T-cell function suppressed by tumor cells and strengthening antitumor immunity.

4. Synergy: Cooperative action among the C12, CD40, and PD-L1 aptamers increases targeting to the tumor microenvironment and immunostimulatory activity, potentially improving efficacy, reducing adverse effects, and allowing dose reduction.

In summary, by introducing the C12 aptamer, this specifically targeted, actively immunostimulatory quadruple-combination nucleic-acid nanotherapeutic enhances targeting for PD-L1-negative or low-PD-L1 SCLC while preserving immunostimulation and checkpoint-blockade functions. This design is expected to improve therapeutic outcomes, reduce adverse effects, increase survival, and provide a more effective treatment option for SCLC patients.

5. Broader indications: In certain small-cell lung cancer patients in whom PD-L1 is not expressed or is expressed at low levels, conventional immune-checkpoint inhibitors may fail to achieve adequate efficacy. Introduction of the C12 aptamer into the multi-aptamer, actively combined nucleic-acid immunotherapeutic addresses this problem, enabling more patients to benefit from immunotherapy.

6. Tolerability and safety: The multi-aptamer design facilitates dose reduction of the therapeutic agent, thereby lowering toxic reactions and adverse effects. By optimizing aptamer affinity and selectivity, impact on normal tissues can be reduced, improving treatment tolerability and safety.

7. Drug synergy: The multi-aptamer, actively combined nucleic-acid immunotherapeutic may also be used in combination with other drugs or treatment modalities to achieve superior antitumor effects. For example, combination with traditional therapies such as radiotherapy or chemotherapy, or with other targeted agents or immunotherapeutics, can further enhance therapeutic efficacy.

In summary, by introducing the C12 aptamer, this specifically targeted, actively immunostimulatory quadruple-combination nucleic-acid nanotherapeutic improves targeting for PD-L1-negative or low-PD-L1 small-cell lung cancer patients while fully retaining the original immunostimulatory and immune-checkpoint-blockade functions. This design is expected to improve therapeutic outcomes, reduce adverse effects, and provide more effective treatment options for patients.

Drug 14) CpG 2006-C12 aptamer-CD47 siRNA-PD-L1 aptamer-PD-L1 siRNA-DNA carrier

This is an innovative, multifunctional therapy that combines specifically targeted active immunostimulation with gene regulation, forming an integrated, synergistically potentiated quintuple-combination nucleic-acid nanotherapeutic. By uniting multiple effector functions, the composition achieves effective antitumor treatment. The mechanisms of action and cooperative effects are analyzed as follows:

1. CpG 2006 DNA: As an immunostimulant, CpG 2006 DNA activates Toll-like receptor 9 (TLR9), thereby inducing release of inflammatory cytokines such as IFN-$\alpha$, TNF-$\alpha$, and IL-6. These cytokines help elicit antitumor immune responses, including activation of macrophages, natural killer (NK) cells, and T lymphocytes.

2. C12 aptamer: The C12 aptamer is a nucleic-acid ligand that specifically binds the HDLBP protein on the surface of small-cell lung cancer cells. Via the C12 aptamer, the immunotherapeutic achieves highly selective delivery to tumor cells, improving therapeutic efficacy while reducing impact on normal tissues.

3. CD47 siRNA: CD47 is an immune-checkpoint protein that inhibits macrophage phagocytosis of tumor cells. CD47 siRNA targets CD47 mRNA in tumor cells, lowering CD47 protein expression so that macrophages can more effectively engulf tumor cells.

4. PD-L1 aptamer: The PD-L1 aptamer is a nucleic-acid ligand that specifically binds the PD-L1 protein. Through PD-L1 aptamer-mediated targeting, the immunotherapeutic achieves highly selective delivery to tumor cells, thereby improving efficacy and reducing effects on normal tissues.

5. PD-L1 siRNA: PD-L1 is an immune-checkpoint protein that suppresses T-cell activation and proliferation via binding to the PD-1 receptor. PD-L1 siRNA targets PD-L1 mRNA in tumor cells, decreasing PD-L1 protein expression and thereby enhancing T-cell cytotoxicity against tumor cells.

6. DNA nucleic-acid nanocarrier: The nanocarrier interconnects all of the foregoing functional moieties into a stable nanoscale structure, improving the stability, targeting specificity, and bioavailability of the immunotherapeutic.

Synergistic effects:

This specifically targeted, actively immunostimulatory and gene-regulatory quintuple-combination nucleic-acid nanotherapeutic exerts synergy via multiple pathways and effector molecules to achieve enhanced antitumor efficacy:

1. Targeted entry: Through the targeting actions of the C12 and PD-L1 aptamers, the nanonucleic-acid therapeutic can enter tumor cells with precision, thereby reducing impact on normal tissues.

2. Augmented immune activation: CpG 2006 DNA activates immune cells and induces antitumor immune responses. This response is further enhanced by CD47 siRNA and PD-L1 siRNA, which reduce expression of the immunosuppressive proteins CD47 and PD-L1, facilitating immune-cell attack on tumor cells.

3. Multimodal immunotherapy: By combining immune activation, immune-checkpoint blockade, and RNA interference (RNAi), a higher level of antitumor immune response is achieved, thereby improving therapeutic efficacy.

4. Overcoming immune evasion: This multifunctional, synergistically potentiated agent addresses features of different tumor subtypes and counters mechanisms such as immune evasion, thereby strengthening immune clearance of tumors.

5. Reduced toxicity: The synergistic treatment strategy can lower the adverse effects and toxicity associated with monotherapy, improving patient quality of life and survival.

In summary, this specifically targeted, actively immunostimulatory and gene-regulatory quintuple-combination nucleic-acid nanotherapeutic achieves synergistic therapy via multiple pathways and effector molecules, demonstrating potent immune- and gene-regulatory tumor-clearing effects. The therapy provides a novel and effective option for the treatment of refractory tumors such as small-cell lung cancer.

Drug 15) 2*CpG 2006-CD40 aptamer-CD16a aptamer-DNA carrier

This composition is a specifically targeted, actively immunostimulatory triple-combination nucleic-acid nanotherapeutic. Its mechanism of action is the same as that of Drug 12 CD16a aptamer-CD40 aptamer-CpG 2006-DNA carrier, except that the dose ratio has been optimized: the proportion of CpG 2006 within the integrated combination is increased so as to improve immune efficiency and reduce potential immuno-toxic adverse effects.

Drug 16) 2*CpG 2006-CD40 aptamer-CD16a aptamer-FAP aptamer-DNA carrier

This composition is a specifically targeted, actively immunostimulatory quadruple-combination nucleic-acid nanotherapeutic. Its mechanism of action is as follows:

1. CpG 2006 (immunostimulant): CpG 2006 activates the Toll-like receptor 9 (TLR9) pathway to stimulate immune cells (e.g., dendritic cells and B cells), thereby promoting antitumor immune responses.

2. CD40 aptamer (costimulation): Functionally analogous to CD40L, the CD40 aptamer binds CD40 and activates dendritic cells, which in turn activate T cells and drive antitumor immune responses, enhancing the immune system's capacity to attack tumors.

3. CD16a aptamer (NK-cell recruitment/activation): CD16a is expressed on NK cells, monocytes, and macrophages, and at lower levels on dendritic cells; it is an activating receptor. An aptamer directed to CD16a can activate and recruit NK cells. As innate cytotoxic effectors, NK cells directly kill pathogens and tumor cells and, relative to T cells, exhibit strong tumor recognition and cytolytic capacity. This agent thereby augments immune attack on tumors.

4. FAP aptamer (tumor stroma targeting): Tumor stroma-comprising cancer-associated fibroblasts (CAFs) and extracellular matrix (ECM)-is increasingly recognized as a key participant in tumorigenesis, therapy resistance, angiogenesis, invasion, and metastasis. FAP (fibroblast activation protein) is a CAF marker that regulates ECM and exhibits multiple pro-tumor functions. FAP is a serine oligopeptidase predominantly expressed during development and rarely in healthy adult tissues; however, it is highly up-regulated in sites of pathological tissue remodeling (including cancer, fibrosis, arthritis, wounds, or inflamed tissue), particularly on fibroblasts. An aptamer to FAP can directly inhibit FAP expression, thereby inhibiting tumor progression.

The design of this specifically targeted, actively immunostimulatory quadruple-combination nucleic-acid nanotherapeutic affords synergistic efficacy in tumor therapy. Such synergy arises from the functional complementarity of the respective aptamers and is achieved via multiple pathways-including combined activation of the immune system, inhibition of tumor angiogenesis, and recruitment of natural killer (NK) cells-thereby collectively effecting potent suppression of tumors. This integrated therapeutic strategy is expected to provide cancer patients with a more effective and safer treatment option, improving survival and quality of life.

Drug 17) 2*CpG 2006-VEGF aptamer-CD40 aptamer-PD-L1 aptamer-CD16a aptamer-DNA carrier

This composition is a specifically targeted, actively immunostimulatory quintuple-combination nucleic-acid nanotherapeutic formed by the combination of multiple aptamers, each conferring a defined function that cooperatively inhibits tumor growth and dissemination. The functions of the individual components and their antitumor mechanisms are as follows:

1. CpG 2006 (two copies): A CpG oligodeoxynucleotide analog that activates the Toll-like receptor 9 (TLR9) pathway, thereby stimulating immune cells (e.g., dendritic cells and B cells) and promoting antitumor immune responses.

2. VEGF aptamer: By targeting vascular endothelial growth factor (VEGF), the aptamer inhibits tumor angiogenesis. Because angiogenesis is critical for tumor growth and metastasis, blockade of VEGF activity slows tumor progression and reduces metastatic potential.

3. CD40 aptamer: Functionally analogous to CD40L; upon binding to CD40, it activates dendritic cells and subsequently T cells, thereby driving antitumor immune responses and enhancing the immune system's capacity to attack tumors.

4. PD-L1 aptamer: By binding PD-L1, the aptamer blocks the PD-1/PD-L1 immune-checkpoint pathway, restoring T-cell activity so that T cells can more effectively attack tumor cells and inhibit tumor growth.

5. CD16a aptamer: A CD16a aptamer is capable of activating and recruiting natural killer (NK) cells. NK cells are key effector cells of the immune system and can directly kill tumor cells. By activating NK cells, the nucleic-acid nanotherapeutic enhances the immune system's antitumor attack capability.

6. DNA carrier: As a nucleic-acid nanocarrier, it interconnects all of the foregoing functional moieties into a stable nanoscale architecture, thereby improving the stability, targeting specificity, and bioavailability of the immunotherapeutic.

Through the cooperative action of these aptamers, this specifically targeted, actively immunostimulatory quintuple-combination nucleic-acid nanotherapeutic can inhibit tumor growth and progression at multiple levels:

1. Stimulation of immune responses: The CpG2006 and CD40L aptamers jointly activate immune cells, such as dendritic cells and T cells, thereby increasing the immune system's antitumor attack capability.

2. Blockade of tumor angiogenesis: A VEGF aptamer can inhibit VEGF in tumor cells, thereby preventing formation of tumor microvasculature and slowing tumor growth.

3. Restoration of T-cell activity: A PD-L1 aptamer blocks the PD-1/PD-L1 immune-checkpoint pathway, restoring T-cell activity so that T cells can more effectively attack tumor cells.

4. Activation of NK cells: A CD16a aptamer activates and recruits NK cells, enhancing the immune system's antitumor attack capability.

This specifically targeted, actively immunostimulatory quintuple-combination nanonucleic-acid therapeutic is therefore designed to deliver synergistic efficacy in cancer treatment. The synergy derives from functional complementarity among the aptamers and is achieved via multiple pathways-including combined immune activation, inhibition of tumor angiogenesis, and recruitment of NK cells-to collectively effect robust tumor suppression. This integrated therapeutic strategy is expected to provide cancer patients with a more effective and safer treatment option, improving survival and quality of life.

Drug 18) 2*AmiR-21-TTA1 aptamer-DNA carrier

This is a specifically gene-regulatory nucleic-acid nanotherapeutic. Its mechanism of action includes the following:

1. Broad suppression via anti-miR-21: miR-21 is a microRNA overexpressed in many tumor cell types and plays key roles in promoting tumor growth, invasion, migration, and resistance to apoptosis. By incorporating anti-miR-21 functionality, the agent broadly suppresses the biological activity of miR-21, thereby counteracting tumor progression.

2. Passive "funnel" targeting of nanoparticles: The nanoparticle exploits the distinctive vasculature of tumor tissue to enter and accumulate by a passive "funnel" effect; enlarged vascular pores and poor lymphatic drainage enable enhanced intratumoral entry and retention, increasing local drug concentration and therapeutic efficacy.

3. Active targeting mediated by the TTA1 aptamer: The TTA1 aptamer is a ligand that specifically targets receptors on the surfaces of tumor cells (including glioma). Through TTA1-mediated active targeting, the 2*AmiR-21-TTA1 aptamer-DNA nanocarrier precisely engages tumor cells, thereby improving antitumor performance.

4. Modulation of distal miR-21: During miR-21 inhibition, the agent regulates not only intratumoral miR-21 but also miR-21 at sites distal to the tumor. Such systemic modulation helps suppress tumor-cell growth and development and slows disease progression.

5. Inhibition of glioma and other tumors: Acting through the foregoing multi-level mechanisms, the agent selectively inhibits the growth and development of glioma and other tumors. Anti-miR-21 reduces tumor-cell proliferative capacity, invasiveness, and anti-apoptotic traits; the passive targeting effect of the nanoparticle and the active targeting of TTA1 act synergistically to raise intratumoral drug concentration and enhance antitumor efficacy; systemic modulation of distal miR-21 further contributes to global suppression of tumor growth and spread.

In summary, the 2*AmiR-21-TTA1 aptamer-DNA carrier specifically gene-regulatory nucleic-acid nanotherapeutic employs multiple cooperating mechanisms-anti-miR-21 activity, passive nanoparticle targeting, TTA1-mediated active targeting, and distal miR-21 modulation-to effectively inhibit the growth and development of glioma and other tumors. This integrated, specifically gene-regulatory nanotherapeutic offers a new approach and modality for cancer treatment and has substantial potential for clinical application.

Drug 19) 2*AmiR-21-3*biotin-DNA carrier

This agent is a specifically gene-regulatory nucleic-acid nanotherapeutic in which three biotin (Bio) moieties are appended to the 3' ends of the three component strands of the nanocarrier, so as to specifically target tumor cells that overexpress biotin-receptor proteins (tumor cells up-regulate biotin receptors to recruit biotin as a nutrient for proliferative expansion). By increasing the intracellular uptake of anti-miR-21 in tumor cells, the 2*AmiR-21-3*Bio-DNAh3.2 agent enhances gene-regulatory antitumor activity.

This specifically gene-regulatory nanotherapeutic utilizes both a "funnel" passive-targeting effect and biotin-mediated active targeting to achieve tumor-cell specificity.

First, the "funnel" passive-targeting effect refers to passive accumulation of nanoparticles within tumor tissue during circulation, due to intravascular pressure gradients, angiogenic vasculature with enlarged fenestrations, and poor lymphatic drainage. This physicochemical, passive targeting increases intratumoral drug concentration and thereby augments antitumor efficacy.

Secondly, the biotin-mediated active targeting refers to modification of the nanonucleic-acid, specifically gene-inhibitory, construct with three biotin (Bio) moieties at the 3' end, so as to achieve specific targeting by binding to tumor cells that highly express biotin-receptor proteins. To sustain proliferative expansion, tumor cells commonly overexpress biotin receptors on their surfaces to recruit biotin as a nutrient source. Accordingly, this actively mediated targeting increases the cellular uptake of the nanonucleic-acid specific gene inhibitor by tumor cells.

By virtue of the synergistic action between the "funnel" passive-targeting effect and biotin-mediated active targeting, the agent achieves high accumulation within tumor tissue and acts specifically on tumor cells. The anti-miR-21 component suppresses intracellular expression of miR-21 in tumor cells, thereby reducing their proliferative capacity, invasiveness, and anti-apoptotic properties, and further exerting an antitumor effect.

Moreover, antigen-presenting cells (APCs), particularly macrophages, undergo polarization from an M2 phenotype to an M1 phenotype under modulation by anti-miR-21, thereby exerting antitumor phagocytic activity; anti-miR-21 thus plays an important role in regulating antitumor immune responses. Macrophages are a key class of APCs and, in response to different signaling stimuli, can assume distinct functional phenotypes: M1 macrophages display pro-inflammatory and antitumor characteristics, whereas M2 macrophages exhibit anti-inflammatory and pro-tumorigenic characteristics.

Anti-miR-21, by modulating signaling pathways within the tumor microenvironment, induces polarization of macrophages from an M2 phenotype to an M1 phenotype. M1 macrophages secrete various cytokines, including interleukin-12 (IL-12) and tumor necrosis factor-a (TNF-$\alpha$), as well as nitric oxide (NO), all of which exhibit antitumor activity. These mediators suppress tumor-cell proliferation, invasiveness, and resistance to apoptosis, and enhance immune surveillance and clearance of tumor cells.

Meanwhile, M1-polarized macrophages possess strong phagocytic capacity and can efficiently engulf tumor cells and other deleterious substances, thereby further exerting antitumor effects. In addition, M1 macrophages activate T cells via antigen presentation, enhancing T-cell cytotoxicity against tumor cells and thus suppressing tumor growth and progression on multiple levels.

Accordingly, under modulation by anti-miR-21, macrophage polarization from an M2 to an M1 phenotype not only enables intrinsic antitumor phagocytic activity but also activates other immune cells, cooperatively producing a more potent antitumor effect.

In summary, the specifically gene-regulatory nucleic-acid nanotherapeutic achieves efficient, specific targeting of tumor cells through the synergy between the "funnel" passive-targeting effect and biotin-mediated active targeting, and, by virtue of anti-miR-21 functionality, inhibits tumor growth and progression.

Drug 20): 2*AmiR-21 - 4*Biotin - DNA Carrier

This is a nano-nucleic-acid therapeutic agent for specific gene regulation. It is the same as drug (19), except that, among the three constituent oligonucleotide sequences forming the carrier, the four ends that are not modified with anti-miR-21 are each biotinylated, so as to enhance targeted binding efficiency and increase the bioavailability of the anti-miR-21.

Drug 21): 2*AmiR-21 - AS1411 Aptamer - DNA Carrier

This is a nano-nucleic-acid therapeutic agent for specific gene regulation in which the AS1411 aptamer is introduced to achieve efficient and specific targeting of tumor tissues and cells. AS1411 is a nucleic acid aptamer with high affinity for nucleolin (NCL) and binds to nucleolin, which is commonly overexpressed on tumor cells. Because nucleolin is expressed at relatively low levels in normal cells, the AS1411 aptamer enables selective targeting of tumor cells.

First, the high affinity of the AS1411 aptamer ensures that the nano-nucleic-acid gene-regulating agent can efficiently bind to nucleolin present on the surface of tumor cells. This targeting effect can increase the concentration of the nano-nucleic-acid gene-regulating agent within tumor tissue, thereby enhancing the action of anti-miR-21 against tumor cells.

Second, upon binding of the AS1411 aptamer to nucleolin, the aptamer can act as a chaperone to promote entry of the nano-nucleic-acid gene-regulating agent into tumor cells. Without intending to be bound by theory, nucleolin may participate in transport via endoplasmic-reticulum/Golgi pathways, thereby facilitating passage across the cell membrane and entry into the cytoplasm to effect inhibition of intracellular miR-21.

Accordingly, the 2*AmiR-21-AS1411 aptamer-DNA-carrier nano-nucleic-acid therapeutic agent for specific gene regulation achieves selective targeting of tumor cells through the AS1411 aptamer, thereby improving the antitumor activity of the specific gene-regulation nano-nucleic-acid therapeutic agent. In addition, after the AS1411 aptamer binds to nucleolin, the efficiency with which the nanoparticles enter the cytoplasm of target cells can be increased, further enhancing the effect of anti-miR-21 on tumor cells. This multi-mechanism specific gene-regulation nano-nucleic-acid therapeutic agent provides a new therapeutic strategy for antitumor treatment.

Drug 22): 2*AmiR-21-AS1411 Aptamer-DNA Carrier (Enhanced Version)

This is a nano-nucleic-acid therapeutic agent for specific gene regulation. On the basis of the foregoing Drug 21, the enhanced version, through optimized design, achieves greater resistance to nuclease-mediated degradation, higher serum stability, and a longer in-vivo circulation time. These improvements are conducive to enhancing the in-vivo efficacy of the nano-nucleic-acid gene-regulating agent and also provide conditions for subsequent combination with classical antitumor chemotherapeutic agents.

Extension of the three double-stranded core scaffold sequences is the key factor in achieving these advantages. This design enhances the carrier's resistance to nuclease cleavage, rendering it more stable in serum and thereby reducing the risk of enzymatic degradation in vivo. In addition, this improvement can prolong the residence time of the nano-nucleic-acid carrier in the human bloodstream, affording more opportunities to reach tumor tissue and exert its activity.

The enhanced design not only improves antitumor efficacy but also prepares the agent for further combination with classical antitumor chemotherapeutics. Such a combination strategy is expected to realize a stronger antitumor effect-both by regulating intracellular signaling pathways in tumor cells via anti-miR-21 and by directly killing tumor cells via the chemotherapeutic drug. This synergistic action can improve therapeutic outcomes and reduce adverse effects, providing a new therapeutic strategy for cancer treatment.

Drug 23): 2*AmiR-21-AS1411 Aptamer-Biotin-DNA Carrier

This is a nano-nucleic-acid therapeutic agent for specific gene regulation. On the basis of the foregoing 2*AmiR-21-AS1411 aptamer-DNA carrier, a biotin (Bio) moiety is additionally introduced, thereby further enhancing the efficiency of targeted binding to tumor tissues and cells and, consequently, increasing the bioavailability of the anti-miR-21 moiety and its tumor-inhibitory effect.

Biotin, as a high-affinity ligand for cell-surface receptors, can bind to biotin receptors that are highly expressed on the surface of tumor cells, thereby further strengthening the selective targeting of this therapeutic agent to tumor cells. Introduction of the biotin modification not only increases accumulation of the therapeutic agent within tumor tissue but also helps raise the uptake rate of the anti-miR-21 moiety into target cells, thereby enhancing inhibition of tumor cells.

With the combined AS1411 aptamer and biotin modification, this specific gene-regulation nano-nucleic-acid therapeutic agent exhibits higher specificity and efficiency in antitumor activity. The AS1411 aptamer can bind to nucleolin that is highly expressed in tumor cells, thereby achieving specific targeting of tumor tissue. The biotin modification further strengthens binding of the nano-nucleic-acid gene-regulating agent to the tumor-cell surface and increases the bioavailability of the anti-miR-21 moiety.

In summary, this specific gene-regulation nano-nucleic-acid therapeutic agent employs dual targeting and anchoring via the AS1411 aptamer and biotin modification, achieving higher tumor-targeting efficiency and antitumor efficacy. This strategy is expected to provide a new therapeutic approach for cancer treatment, improving efficacy and reducing adverse effects.

Drug 24): 2×AmiR-21-CD40 Aptamer-DNA Carrier

This is a nano-nucleic-acid therapeutic agent that combines specific gene regulation with immunomodulation. Through the synergistic action of anti-miR-21 and a CD40 aptamer, inhibition is achieved against multiple tumors, including melanoma, colorectal cancer, breast cancer, and B-cell lymphoma. The mechanism of action is analyzed as follows:

1. Action of the anti-miR-21 moieties: miR-21 is a microRNA highly expressed in many tumor types and is related to tumor initiation, progression, invasion, and metastasis. The two anti-miR-21 oligonucleotide moieties specifically hybridize to miR-21 and inhibit its biological function, thereby reducing the proliferative, migratory, and invasive capabilities of tumor cells and slowing tumor development.

2. Action of the CD40 aptamer: CD40 is an immune-cell surface molecule that plays a key role in eliciting antitumor immune responses. The CD40 aptamer can bind to CD40 proteins on tumor cells and antigen-presenting cells (APCs), and possesses targeting-mediated and co-stimulatory immune-activating functions. It activates antitumor immune cells such as dendritic cells (DCs) and B cells, promoting their transition to more mature and activated states. In addition, by activating DCs and B cells, the CD40 aptamer facilitates the activation of antitumor T cells, thereby enhancing the immune system's capacity to attack tumors.

By virtue of the synergy between the anti-miR-21 moieties and the CD40 aptamer, the 2* AmiR-21-CD40 aptamer-DNA carrier dual-function nano-nucleic-acid therapeutic both directly suppresses tumor-cell growth and invasiveness and activates systemic immune attack against tumors. This combined effect enables the therapeutic to exhibit favorable inhibitory activity across tumor types such as melanoma, colorectal cancer, breast cancer, and B-cell lymphoma. Moreover, owing to the high biocompatibility and stability of the DNA carrier, the in-vivo bioavailability and antitumor efficacy of this combined specific gene-regulation and immunomodulation nano-nucleic-acid therapeutic are expected to be improved.

Drug 25): 2*AmiR-21-MUC1 Aptamer-3*Biotin-DNA Carrier

This is a nano-nucleic-acid therapeutic agent for specific gene regulation. Through the combined action of anti-miR-21, a MUC1 aptamer, and biotin (Bio) moieties, effective inhibition of tumors is achieved. The antitumor mechanism is analyzed in detail below:

1. Action of the anti-miR-21 moieties: miR-21 is a microRNA highly expressed in many tumor types, and its overexpression is associated with tumor initiation, progression, invasion, and metastasis. The two AmiR-21 moieties specifically hybridize to miR-21 and inhibit its biological function, thereby reducing the proliferative, migratory, and invasive capabilities of tumor cells and slowing tumor development.

2. Action of the MUC1 aptamer: MUC1 is a glycoprotein that is highly expressed on the surface of many cancer cells, such as those of breast, colorectal, and ovarian cancers. The MUC1 aptamer can specifically bind to MUC1 molecules on the surface of tumor cells, thereby achieving targeting of tumor cells. Such targeting helps increase the concentration of the nano-nucleic-acid therapeutic within tumor tissue and enhances its antitumor effect.

3. Action of the biotin (Bio) moieties: Biotin is a vitamin widely present in living organisms, and certain tumor cells exhibit high-level expression of biotin receptors on their surfaces. In the 3*Bio-DNA carrier, the biotin moieties can bind to biotin receptors on the surface of tumor cells, further strengthening the targeting of the nano-nucleic-acid therapeutic to tumor cells. This targeting effect helps raise the concentration of the nano-nucleic acid in tumor tissue, thereby enhancing the antitumor effect.

By virtue of the synergy among anti-miR-21, the MUC1 aptamer, and the biotin moieties, the 2*AmiR-21-MUC1 aptamer-3*Bio-DNA carrier specific gene-regulation nano-nucleic-acid therapeutic can both directly suppress tumor-cell growth and invasiveness and achieve efficient targeting of tumor cells. This combined action enables the therapeutic to exhibit favorable inhibitory effects across multiple tumor types. In addition, because specific gene-regulation nano-nucleic-acid therapeutics possess high biocompatibility and targeting specificity, they impose relatively low toxic side effects on normal tissues during treatment, thereby improving patient tolerability and safety.

4. Activation of antitumor immune responses: By inhibiting intracellular miR-21 in tumor cells, the anti-miR-21 moieties can affect the immune-evasion capability of tumor cells. In addition, after the MUC1 aptamer binds to MUC1 on the surface of tumor cells, it may alert the immune system and thereby activate antitumor immune responses. This process helps enhance the immune system's tumor recognition and attack capabilities, improving therapeutic efficacy.

5. Inhibition of tumor angiogenesis: The anti-miR-21 moieties can influence various pro-angiogenic factors produced by tumor cells, such as VEGF and bFGF. By suppressing the expression of these factors, tumor angiogenesis can be indirectly inhibited, thereby affecting tumor growth and metastasis.

In summary, the 2*AmiR-21-MUC1 aptamer-3*Bio-DNA carrier specific gene-regulation nano-nucleic-acid therapeutic achieves effective tumor inhibition through multiple synergistic pathways. The mechanisms of action include suppression of the biological function of miR-21, enhanced targeting to tumor cells, activation of antitumor immune responses, and inhibition of tumor angiogenesis. This multi-effector molecular combination design enables the therapeutic to exhibit favorable therapeutic effects across multiple tumor types, including melanoma, colorectal cancer, breast cancer, and B-cell lymphoma, thereby providing a new strategy for cancer therapy.

Drug 26): AmiR-21-AS1411 Aptamer-A15 Aptamer-2*Biotin-DNA Carrier

This is a nano-nucleic-acid therapeutic agent for specific gene regulation, featuring a multi-effector molecular combination design. Its principal mechanisms include the following:

1. Suppression of the biological function of miR-21: The AmiR-21 (anti-miR-21) moiety binds to miR-21 within tumor cells and inhibits its biological function, thereby affecting multiple cellular behaviors, including tumor-cell growth, metastasis, and apoptosis.

2. Dual-targeting synergy: The AS1411 aptamer has high affinity for and binds nucleolin, enabling selective recognition of and targeting to tumor cells. The A15 aptamer is capable of specifically binding the CD133 protein, thereby enabling targeting to tumor stem cells. Through these two-tier targeting-guidance effects, the cellular uptake of the anti-miR-21 moiety by tumor cells and cancer stem cells is enhanced, thereby improving therapeutic efficacy.

3. Antitumor activity of the AS1411 aptamer: Following binding of the AS1411 aptamer to nucleolin, not only is the efficiency with which the nano-nucleic-acid particles enter the cytoplasm of target cells increased, but the aptamer also directly inhibits tumor-cell growth. This dual action allows the AS1411 aptamer to play an important antitumor role during treatment.

4. Biotin-modification-enhanced targeting: Introduction of two biotin (Bio) moieties further strengthens binding of the therapeutic to tumor cells that highly express biotin-receptor proteins, increases the bioavailability of the anti-miR-21 moiety, and enhances the antitumor effect.

5. Modulation of the tumor microenvironment: The anti-miR-21 moiety exerts regulatory effects on the tumor microenvironment, including inhibition of tumor angiogenesis and modulation of tumor-associated immune cells, thereby helping to suppress tumor growth and metastasis.
In summary, the AmiR-21-AS1411 aptamer-A15 aptamer-2*Biotin-DNA carrier nano-nucleic-acid therapeutic agent achieves effective inhibition of tumors and tumor stem cells through multiple synergistic pathways. The mechanisms include suppression of the biological function of miR-21, dual-targeting synergy, antitumor activity of the AS1411 aptamer, biotin-modification-enhanced targeting, and modulation of the tumor microenvironment. This multi-mechanism design provides a new strategy for cancer therapy.

6. Improving specificity of antitumor therapy and reducing adverse effects: Through the dual-targeting action of the AS1411 aptamer and the A15 aptamer, the AmiR-21-AS1411 aptamer-A15 aptamer-2*Bio-DNA carrier specific gene-regulation nano-nucleic-acid therapeutic can more precisely recognize tumor cells and tumor stem cells, thereby avoiding impact on normal cells, improving therapeutic specificity, and reducing adverse effects.

7. Enhancing stability and bioavailability of the therapeutic: By introducing double-stranded core scaffold sequences into the DNA carrier, the nuclease-resistance of the nano-nucleic-acid carrier is strengthened, its stability in serum is increased, and its residence time in the human bloodstream is prolonged. This helps to improve the bioavailability of the therapeutic and further enhance its antitumor effect.

8. Potential combination therapy: When used in combination with other antitumor treatments (e.g., chemotherapy, radiotherapy, immunotherapy), synergistic effects can be achieved, thereby improving overall therapeutic efficacy.

Through these combined actions, the AmiR-21-AS1411 aptamer-A15 aptamer-2*Bio-DNA carrier specific gene-regulation nano-nucleic-acid therapeutic exhibits significant effects in suppressing the growth and progression of multiple tumors, including melanoma, colorectal cancer, breast cancer, and B-cell lymphoma. Its multi-pathway synergistic mechanism is expected to provide a more precise and effective new strategy for cancer therapy.

Drug 27): 2*Mannose-2*AmiR-21-DNA Carrier

This is a nano-nucleic-acid therapeutic agent for specific gene regulation. By combining anti-miR-21 moieties with mannose modification, it achieves antitumor therapy and suppression of tumor-associated pain. The mechanisms of action are detailed as follows:

1. Antitumor mechanism: (a) targeted inhibition of miR-21: miR-21 is a microRNA highly expressed in many tumor cell types and closely associated with tumor growth, invasion, and metastasis; two AmiR-21 (anti-miR-21) moieties specifically hybridize to and inhibit miR-21, thereby blocking its tumor-promoting activity; (b) effects on apoptosis and proliferation: inhibition of miR-21 restores expression of its target genes PTEN and PDCD4, thereby promoting apoptosis of tumor cells and suppressing cellular proliferation; and (c) inhibition of tumor angiogenesis: inhibition of miR-21 down-regulates expression of vascular endothelial growth factor (VEGF), thereby reducing tumor angiogenesis and diminishing the tumor's growth capacity.

2. Mechanism of alleviating tumor-associated pain: (a) reduction of inflammatory responses-anti-miR-21 decreases inflammation by down-regulating the expression of pro-inflammatory cytokines and chemokines, such as IL-6 and TNF-$\alpha$, and mitigation of inflammation reduces pressure in peritumoral tissues, thereby lessening pain; (b) modulation of neural transmission-anti-miR-21 regulates pain-related genes, including TRPV1 and CGRP, which play key roles in nociception and signal transduction, and by modulating the expression of these genes anti-miR-21 helps relieve tumor pain; and (c) improvement of the tumor microenvironment-by inhibiting tumor growth and invasiveness, anti-miR-21 improves the tumor microenvironment and reduces intercellular mechanical stress, thereby diminishing pain.

In summary, the 2*mannose-2*AmiR-21-DNA carrier achieves antitumor activity and alleviation of tumor-associated pain by employing the anti-miR-21 moieties to inhibit tumor growth, invasion, and metastasis, while concurrently reducing inflammatory responses, modulating neural transmission, and improving the tumor microenvironment; moreover, the 2*mannose modification enhances the in-vivo stability and targeting of the nano-nucleic-acid therapeutic agent.

3. Role of mannose modification: (a) enhanced stability-mannose modification increases the in-vivo stability of the nano-nucleic-acid therapeutic agent, conferring greater resistance to enzymatic (e.g., nuclease) degradation and thereby prolonging its residence time in the bloodstream; (b) enhanced targeting-mannose modification facilitates binding of the nano-nucleic-acid therapeutic agent to specific receptors on the surface of tumor cells, thereby increasing tumor-cell selectivity and reducing impact on normal cells and associated adverse effects.

In summary, the 2*mannose-2*AmiR-21-DNA carrier specific gene-regulation nano-nucleic-acid therapeutic agent, by virtue of the antitumor activity of its anti-miR-21 moieties and its capacity to alleviate tumor-associated pain, exhibits broad prospects for application in cancer therapy; moreover, mannose modification confers improved in-vivo stability and targeting specificity, thereby offering enhanced potential for clinical use in the treatment of cancer.

Drug 28): 2*Mannose-A15 Aptamer-2*AmiR-21-DNA Carrier

This is a nano-nucleic-acid therapeutic agent for specific gene regulation, which combines mannose modification, an A15 aptamer (specifically binding the CD133 protein), and anti-miR-21 moieties, and exhibits antitumor activity including activity directed to tumor stem cells. The mechanisms of action are as follows:

1. Antitumor action of the anti-miR-21 moieties: The anti-miR-21 moieties selectively reduce miR-21 levels, thereby inhibiting tumor-cell growth, invasion, and metastasis, while concurrently attenuating inflammatory responses and improving the tumor microenvironment to achieve an antitumor effect.

2. Role of the A15 aptamer (specific binder of the CD133 protein): The A15 aptamer specifically binds to the CD133 protein, which is predominantly expressed on the surface of tumor stem cells. Guided by the A15 aptamer, the nano-nucleic-acid therapeutic agent can more efficiently target tumor stem cells, thereby inhibiting the growth of tumor stem cells and their capacity to maintain tumor tissue.

3. Role of mannose modification: (a) Enhancing stability-mannose modification increases the in-vivo stability of the nano-nucleic-acid therapeutic agent, conferring greater resistance to enzymatic (e.g., nuclease) degradation and thereby prolonging its residence time in the bloodstream; (b) Enhancing targeting-mannose modification facilitates binding of the nano-nucleic-acid therapeutic agent to specific receptors on the surface of tumor cells, thereby increasing tumor-cell selectivity and reducing impact on normal cells and associated adverse effects.

In summary, the 2*mannose-A15 aptamer-2*AmiR-21-DNA carrier specific gene-regulation nano-nucleic-acid therapeutic agent exhibits broad prospects for antitumor application through the synergistic actions of: inhibition of tumor-cell growth by the anti-miR-21 moieties, selective targeting of tumor stem cells by the A15 aptamer, and enhanced in-vivo stability and targeting specificity conferred by mannose modification.

Drug 29): 2*Mannose-survivin siRNA-DNA Carrier

This is a specific gene-regulation nano-nucleic-acid therapeutic agent that combines mannose modification, an siRNA directed against survivin, and a DNA carrier scaffold. The therapeutic exhibits antitumor activity primarily by lowering survivin expression. In addition to the inherent passive accumulation of nanoparticles in tumor tissue, the mechanisms of action are as follows:

1. Antitumor action of survivin-targeting siRNA: Survivin is an anti-apoptotic protein typically overexpressed in tumor cells and contributes to tumor growth. The survivin-targeting siRNA specifically binds to and induces degradation of survivin mRNA, thereby reducing survivin protein levels and inducing apoptosis, and inhibiting tumor-cell growth, invasion, and metastasis.

2. Role of mannose modification: (a) Enhanced stability: Mannose modification increases the in-vivo stability of the nano-nucleic-acid therapeutic agent, conferring greater resistance to enzymatic degradation and thereby prolonging its survival time in the bloodstream; (b) Enhanced targeting: Mannose modification facilitates binding of the nano-nucleic-acid therapeutic agent to specific receptors on the surface of tumor cells, thereby improving tumor-cell targeting, reducing impact on normal cells, and lowering adverse effects.

3. Role of the DNA carrier scaffold: The DNA carrier scaffold protects the siRNA from enzymatic degradation, enhancing its stability and bioavailability. In addition, the DNA scaffold improves delivery efficiency, enabling the siRNA to more readily enter target cells.

Accordingly, the 2*mannose-survivin siRNA-DNA carrier specific gene-regulation nano-nucleic-acid therapeutic agent suppresses tumor-cell growth, invasion, and metastasis by lowering survivin expression via siRNA, while mannose modification improves the therapeutic's stability and targeting, and the DNA carrier scaffold provides protective and delivery functions, collectively producing an antitumor effect.

Drug 30): 2*Mannose-A15 Aptamer-survivin siRNA-DNA Carrier

This is a specific gene-regulation nano-nucleic-acid therapeutic agent, comprising mannose modification, an A15 aptamer, an inhibitory agent directed against survivin (e.g., survivin-targeting siRNA), anti-miR-21 moieties, and a DNA carrier scaffold. The therapeutic exhibits multiple antitumor effects. In addition to the nanoparticle-mediated "funnel" targeting/accumulation effect in tumor tissue and pain-alleviating effects, the mechanisms of action include:

1. Targeting action of the A15 aptamer: The A15 aptamer specifically binds the CD133 protein, which is highly expressed on tumor stem cells. Via A15-aptamer mediation, the nano-nucleic-acid therapeutic can target tumor stem cells, thereby more effectively inhibiting tumor growth and progression.

2. Inhibition of survivin: As noted above, survivin is an anti-apoptotic protein typically overexpressed in tumor cells. The inhibitory agent within the nano-nucleic-acid therapeutic (e.g., survivin siRNA) specifically reduces survivin expression, thereby inducing apoptosis of tumor cells and inhibiting tumor-cell growth, invasion, and metastasis.

3. Antitumor action of anti-miR-21: The anti-miR-21 moieties reduce the expression of miR-21, which is commonly overexpressed in many tumors, thereby suppressing tumor-cell proliferation, invasion, and metastasis and promoting apoptosis. In addition, anti-miR-21 can drive macrophage polarization from the M2 phenotype toward the M1 phenotype, thereby enhancing antitumor phagocytic activity.

4. Roles of mannose modification and the DNA scaffold: Mannose modification and the DNA carrier scaffold enhance the stability, bioavailability, and delivery efficiency of the nano-nucleic-acid therapeutic, thereby prolonging its residence time in the bloodstream and improving the efficiency of entry into target cells.

Accordingly, the 2*mannose-A15 aptamer-survivin siRNA-DNA carrier specific gene-regulation nano-nucleic-acid therapeutic exerts antitumor activity through multiple complementary mechanisms. In addition to the foregoing, the nanoparticles' "funnel" tumor-tissue accumulation effect constitutes an important factor underpinning the therapeutic's potent antitumor performance.

"Funnel" tumor-tissue targeted-accumulation effect refers to the characteristic accumulation of nanoparticles within tumor tissue. Without intending to be bound by theory, this phenomenon is primarily attributable to abnormal tumor vasculature and increased vascular permeability, together with enlarged intercellular gaps among tumor cells. These features permit nanoparticles to traverse vessel walls more readily, enter tumor tissue, and accumulate therein. In addition, the lymphatic system within tumor tissue is incomplete, resulting in reduced clearance of nanoparticles and further enhancing their accumulation.

The "funnel" targeted-accumulation effect of the present specific gene-regulation nano-nucleic-acid therapeutic agent within tumor tissue can potentiate its antitumor activity because:

1. Increasing therapeutic-agent concentration: Accumulation of nanoparticles within tumor tissue increases the effective intratumoral concentration of the therapeutic agent, thereby improving therapeutic efficacy.

2. Reducing side effects: Because nanoparticles preferentially accumulate in tumor tissue, distribution of the therapeutic agent to normal tissues is reduced, thereby lowering adverse effects and toxicity.

3. Synergistic action: The nanoparticles' "funnel" tumor-tissue targeted-accumulation effect acts in concert with the foregoing A15-aptamer-mediated targeting, survivin inhibition, and anti-miR-21 activity to collectively produce an antitumor effect.

In summary, the 2*mannose-A15 aptamer-survivin siRNA-DNA carrier specific gene-regulation nano-nucleic-acid therapeutic agent exerts potent antitumor activity through multiple mechanisms, including the nanoparticles' "funnel" tumor-tissue targeted-accumulation effect; accordingly, the agent exhibits broad prospects for application and may constitute an effective modality for future antitumor therapy.

Drug 31): AS1411 Aptamer-survivin siRNA-PSMA Aptamer-3*AmiR-21-RNA Carrier

This is a multifunctional, dual-combination specific gene-regulation nano-nucleic-acid therapeutic agent intended for comprehensive treatment of castration-resistant prostate cancer (CRPC). Its CRPC-inhibitory mechanisms are as follows:

1. AS1411 aptamer: AS1411 is an aptamer that specifically binds nucleolin, which is highly expressed on tumor cells. Through AS 1411 binding, the nano-nucleic-acid gene therapeutic can selectively target prostate-cancer cells and increase the intratumoral concentration of the therapeutic agent, thereby improving therapeutic efficacy.

2. Survivin inhibition: The therapeutic includes an siRNA directed against survivin, an anti-apoptotic protein overexpressed in many tumor cells. Suppressing survivin expression induces apoptosis of prostate-cancer cells and thereby inhibits tumor growth.

3. PSMA aptamer: Prostate-specific membrane antigen (PSMA) is highly expressed on the surface of prostate-cancer cells. The PSMA aptamer further improves selective targeting of the nano-nucleic-acid therapeutic agent to prostate-cancer cells, enhancing therapeutic efficacy.

4. Anti-miR-21: miR-21 is a microRNA overexpressed in many tumors and associated with tumor-cell proliferation, invasion, and therapeutic resistance. The anti-miR-21 moieties in the therapeutic suppress miR-21 activity, thereby slowing the growth and invasion of prostate-cancer cells.

5. RNA carrier: The nano-nucleic-acid RNA carrier protects the active components, increasing their stability and bioavailability, which enhances antitumor activity and reduces adverse effects.
Accordingly, through the synergistic action of the foregoing mechanisms, the AS1411 aptamer-survivin siRNA-PSMA aptamer-3*AmiR-21-RNA carrier gene therapeutic can effectively inhibit the growth and progression of castration-resistant prostate cancer. This integrated therapeutic strategy has broad prospects for application and may constitute an effective modality for future prostate-cancer therapy.

6. "Funnel" effect: Without intending to be bound by theory, nanoparticles exhibit a passive "funnel" tumor-tissue targeted-accumulation effect attributable to abnormal tumor vasculature and altered hemodynamics. Via this effect, nanoparticles preferentially accumulate within tumor tissue, thereby increasing the effective intratumoral concentration of the therapeutic agent and enhancing therapeutic efficacy.

7. Pain alleviation: In addition to inhibiting tumor growth, anti-miR-21 exerts pain-alleviating effects. miR-21 plays a key role in nociceptive signaling pathways; suppression of miR-21 reduces transmission of pain signals, thereby mitigating pain symptoms in patients with prostate cancer.

Accordingly, through the synergistic action of the foregoing mechanisms, the AS1411 aptamer-survivin siRNA-PSMA aptamer-3*AmiR-21-RNA carrier specific gene-regulation dual-combination nano-nucleic-acid therapeutic effectively inhibits the growth, progression, and invasion of castration-resistant prostate cancer while alleviating patient pain symptoms. This integrated therapeutic strategy possesses substantial potential value and may constitute an important modality for future treatment of prostate cancer.

Drug 32): 3*AmiR-21-ATP Aptamer-FGF2 Aptamer-DNA Carrier

This is a specific gene-regulation nano-nucleic-acid therapeutic agent that combines anti-miR-21, an ATP aptamer, an FGF2 aptamer, and a DNA carrier, and exhibits multiple antitumor activities as well as mechanisms for suppressing cancer-induced bone pain, as follows:

1. Anti-miR-21 action: miR-21 is a microRNA highly expressed in many tumors and closely associated with tumor growth, invasion, and therapeutic resistance. By inhibiting miR-21, proliferation and invasion of tumor cells can be suppressed and tumor drug resistance reduced.

2. ATP action: ATP serves as an energy supplier in cells and participates in numerous biological processes. In tumor therapy, ATP can be exploited to disrupt tumor-cell energy metabolism, thereby inhibiting tumor-cell growth.

3. FGF2 action: Fibroblast growth factor 2 (FGF2) is a growth factor that promotes cell growth, proliferation, and differentiation. In tumor therapy, FGF2 can be used to stimulate repair and regeneration of normal tissues, thereby mitigating side effects caused by antitumor treatment.

4. DNA nano-carrier: As a nanocarrier, DNA can effectively deliver the $3 \times$ AmiR-21, ATP, and FGF2 to tumor tissue, increasing the intratumoral concentration of the therapeutic agent and thereby improving therapeutic efficacy.

5. Passive "funnel" targeting effect: Without intending to be bound by theory, nanoparticles exhibit a passive "funnel" effect toward tumor tissue due to abnormal tumor vasculature and altered hemodynamics. Via this effect, nanoparticles accumulate in tumor tissue, thereby increasing the intratumoral concentration of the therapeutic agent and enhancing therapeutic efficacy.

Accordingly, the 3*AmiR-21-ATP aptamer-FGF2 aptamer-DNA carrier specific gene-regulation nano-nucleic-acid therapeutic exerts potent antitumor activity through multiple synergistic mechanisms, while the FGF2-mediated promotion of normal-tissue repair and regeneration can alleviate side effects caused by antitumor therapy. This integrated therapeutic strategy possesses substantial potential utility.

Analgesic mechanisms for cancer-induced bone pain attributable to the combination of anti-miR-21 and an FGF2 aptamer-without intending to be bound by theory-are as follows:

1. Anti-miR-21-mediated pain relief: Studies indicate that miR-21 plays a key role in the genesis of cancer pain by regulating the expression of pain-related genes. Inhibiting miR-21 function reduces transmission of nociceptive signals, thereby alleviating cancer pain.

2. Role of the FGF2 aptamer in relieving bone pain: Fibroblast growth factor 2 (FGF2) promotes cell growth, proliferation, and differentiation. In the context of bone-pain relief, FGF2 can stimulate repair and regeneration of bone tissue and mitigate tissue injury and inflammatory responses, thereby reducing cancer-induced bone pain.

3. Targeting of tumor and bone tissues: Nanoparticles bearing the combined anti-miR-21 and FGF2 aptamer exhibit a passive "funnel" tumor-tissue targeted-accumulation effect, enabling efficient intratumoral accumulation. In addition, the FGF2 aptamer exhibits strong affinity for bone tissue, thereby improving targeting to bone and increasing local concentrations of anti-miR-21 and FGF2 in bone pain-related regions.

4. Synergistic action: The combination of anti-miR-21 and the FGF2 aptamer affords multi-effector synergy-simultaneously relieving cancer pain and promoting repair and regeneration of bone tissue-thereby improving therapeutic effectiveness and enhancing the quality of life of patients with cancer-induced bone pain.

Accordingly, the combination of anti-miR-21 and an FGF2 aptamer provides significant relief of cancer-induced bone pain via multiple mechanisms acting in concert, improving therapeutic outcomes and reducing patient suffering.

Drug 33): AS1411 Aptamer-Survivin siRNA-PSMA Aptamer-3*AmiR-21-RNA Carrier

Drug 33 has the same mechanism of action as Drug 31; it is likewise a targeted, multifunctional, gene-regulatory dual-combination nanonucleic-acid therapeutic. The only difference from Drug 31 is that, in Drug 33, the "a" strand of the RNA carrier is replaced with a DNA sequence; that is, in the RNA carrier of Drug 33), the sequence a is DNA, while the sequences b and c are RNA.

Drug 34): 3*AmiR-21-AS1411 Aptamer-FAP Aptamer-DNA Carrier

This is a targeted, multifunctional nanonucleic-acid therapeutic employing a triple-combination of immune- and gene-regulatory modalities. Its mechanisms of action are as follows:

1. AmiR-21: MicroRNA-21 (miR-21) is overexpressed in many cancers and is associated with tumor-cell proliferation, apoptosis, invasion, and metastasis; it typically promotes growth and inhibits apoptosis. An anti-miR-21 (AmiR-21) oligonucleotide binds to miR-21 and prevents its interaction with target genes, thereby reducing miR-21 activity and inhibiting tumor-cell growth.

2. AS1411 aptamer: A nucleic-acid aptamer that targets nucleolin on tumor cells and exerts antitumor activity.

3. FAP aptamer: Fibroblast activation protein (FAP) is a serine peptidase expressed predominantly during development and rarely in healthy adult tissues; however, it is highly up-regulated in remodeling pathological tissues (including cancer, fibrosis, arthritis, wounds, and inflamed sites), particularly on fibroblasts. Accordingly, a FAP aptamer can directly inhibit FAP expression and thereby suppress tumor progression.

This integrated, tumor-targeted triple-combination design provides synergistic efficacy for cancer therapy and is expected to afford more effective and safer treatment options for patients, improving survival and quality of life.

Drug 35): AS1411 Aptamer-Survivin siRNA-EpCAM Aptamer-3*AmiR-21-DNA Carrier

This is a targeted, multifunctional immuno- and gene-regulatory triple-combination nanonucleic-acid therapeutic. AS1411, Survivin siRNA, the EpCAM aptamer, and a DNA carrier bearing three copies of anti-miR-21 (3*AmiR-21) are each technologies or agents used for gene therapy. By employing specific gene-targeting mechanisms to inhibit or promote gene expression, therapeutic effects are achieved. The gene-targeting mechanisms of the respective effector moieties are as follows:

1. AS1411: A G-rich oligonucleotide aptamer configured to bind to and functionally inhibit nucleolin (NCL) and heat-shock protein 70 (HSP70) in cancer cells, thereby suppressing tumor-cell proliferation and growth. AS1411 is further capable of binding to, and inhibiting, tissue factor (TF), thereby suppressing tumor angiogenesis and metastasis. NCL is overexpressed in multiple cancers; upon binding to NCL, AS1411 can inhibit tumor-cell proliferation by inducing apoptosis. AS1411 can also facilitate cellular uptake of the integrated nanonucleic-acid multi-gene regulator.

2. Survivin siRNA: Survivin is an anti-apoptotic protein highly expressed in many malignancies. Survivin siRNA is a small interfering RNA that specifically suppresses expression of the Survivin gene, thereby promoting apoptosis and death of cancer cells. Incorporation of Survivin siRNA into the integrated nanonucleic-acid multi-gene regulator enhances inhibition of tumor cells.

3. EpCAM aptamer: Epithelial cell adhesion molecule (EpCAM) is a membrane protein expressed on many tumor cells and commonly overexpressed in epithelial tumors; it participates in cell proliferation, differentiation, adhesion, and migration, and its overexpression is associated with cancer initiation and progression. An aptamer targeting EpCAM suppresses its expression and induces host immune cells to attack and destroy cancer cells. The EpCAM aptamer also serves as a targeting ligand, enabling the multi-gene regulator to enter tumor cells with higher specificity and thereby enhancing tumor-cell selectivity.

4. 3*AmiR-21-DNA Carrier

The 3*AmiR-21-DNA carrier is a gene-therapy agent comprising a complex of three anti-microRNA-21 oligonucleotides (AmiR-21; antisense to microRNA-21, miR-21) associated with a DNA carrier. MicroRNA-21 (miR-21) is overexpressed in many cancers and is implicated in tumor-cell proliferation, apoptosis, invasion, and metastasis; it typically promotes growth and suppresses apoptosis. The AmiR-21 oligonucleotide is configured to bind miR-21 and prevent its interaction with target gene transcripts, thereby reducing miR-21 activity. In this way, AmiR-21 inhibits tumor-cell growth and enhances the antitumor effect of the integrated nanonucleic-acid multi-gene regulator.

In summary, the integrated nanonucleic-acid multi-gene regulator achieves tumor-targeted therapy by combining AS1411/EpCAM aptamers, Survivin siRNA, and anti-miR-21 effector molecules. Synergistic interactions among these moieties increase targeting specificity and thereby reduce adverse effects on normal cells.

During treatment, the AS1411 and EpCAM aptamers first direct the integrated nanonucleic-acid multi-gene regulator to the tumor-cell surface. AS1411 binds nucleolin (NCL), which is highly expressed in tumor cells, and the EpCAM aptamer binds the overexpressed epithelial cell adhesion molecule (EpCAM). Such specific binding facilitates cellular internalization of the nanonucleic-acid multi-gene regulator.

Once internalized, the Survivin siRNA and anti-miR-21 effector molecules exert their functions. The Survivin siRNA down-regulates expression of Survivin, an anti-apoptotic protein, thereby inhibiting tumor-cell growth, while the anti-miR-21 binds miR-21, preventing its association with its targets and reducing miR-21 activity. Accordingly, anti-miR-21 suppresses tumor-cell growth and augments the antitumor efficacy of the integrated nanonucleic-acid multi-gene regulator.

Thus, through synergistic action, the integrated nanonucleic-acid multi-gene regulator provides tumor-targeted therapy that improves therapeutic efficacy while reducing effects on normal cells. This multi-target, multifunctional nanodrug delivery system exhibits substantial clinical potential and may serve as an effective modality for cancer treatment.

Drug 36): IL-4Rα Aptamer-TGF-β Aptamer-PD-L1 Aptamer-2*AmiR-21-DNA Carrier

This is a targeted, multifunctional, immuno- and gene-regulatory quadruple-combination nanonucleic-acid therapeutic. The immunotherapeutic agent integrates three aptamers and two copies of anti-miR-21 on a DNA carrier via sequence extension, forming a single, integrated therapeutic construct configured to inhibit the growth and progression of multiple tumor types. The functions of each moiety and their cooperative effects are as follows:

1. IL-4Rα Aptamer: Interleukin-4 receptor alpha chain (IL-4Rα) is a cell-surface receptor expressed on immune cells and may also be overexpressed on tumor cells. By specifically targeting IL-4Rα, the therapeutic agent is configured to promote activation of tumor-specific immune effector cells, including T cells and natural killer (NK) cells, thereby enhancing antitumor immune responses.

2. TGF-β Aptamer: Transforming growth factor-beta (TGF-β) is a pleiotropic cytokine that plays a key role in tumor progression. TGF-β suppresses immune-cell activity, thereby reducing the likelihood that tumor cells will be attacked by the immune system. By specifically targeting TGF-β, the therapeutic agent is configured to inhibit its immunosuppressive activity and thereby enhance immune-mediated antitumor responses.

3. PD-L1 Aptamer: Programmed death-ligand 1 (PD-L1) is a protein expressed on the surface of tumor cells and binds to the programmed cell death 1 (PD-1) receptor on immune cells, thereby suppressing immune-cell function. By specifically targeting PD-L1, the therapeutic agent is configured to block the PD-1/PD-L1 interaction, thereby at least partially restoring immune-cell function and enhancing immune-mediated antitumor responses.

4. Anti-miR-21: MicroRNA-21 (miR-21) is a small non-coding RNA overexpressed in many cancers and typically promotes tumor-cell proliferation while inhibiting apoptosis. An anti-miR-21 oligonucleotide is configured to bind miR-21 and prevent its association with its target gene transcripts (mRNAs), thereby reducing miR-21 activity. In this manner, anti-miR-21 inhibits tumor-cell growth and enhances the antitumor efficacy of the integrated nanonucleic-acid combined immunotherapeutic agent.

5. DNA Carrier: A DNA nanocarrier configured to deliver the foregoing therapeutic moieties. By sequence extension, the DNA carrier incorporates the IL-4Rα aptamer, TGF-β aptamer, PD-L1 aptamer, and one or more anti-miR-21 oligonucleotides into a single, integrated therapeutic construct. This configuration ensures coordinated and synergistic activity among the components, thereby enhancing therapeutic efficacy.

Synergistic effects of the respective moieties:

1. Activation of the immune system: By concurrently targeting IL-4Rα, TGF-β, and PD-L1, the therapeutic agent is configured to enhance antitumor immunity through multiple mechanisms. The IL-4Rα aptamer promotes activation of immune effector cells (e.g., T cells and NK cells), the TGF-β aptamer alleviates immunosuppression, and the PD-L1 aptamer restores immune-cell function by blocking PD-1/PD-L1 signaling. Acting in concert, the three aptamers potentiate immune responses in oncology treatment.

2. Suppression of tumor-cell growth: By reducing miR-21 activity, the anti-miR-21 oligonucleotide directly inhibits tumor-cell proliferation and induces apoptosis. Concurrent immune activation further restrains tumor growth. This two-pronged inhibitory effect endows the agent with enhanced antitumor efficacy.

3. Targeting specificity and safety: Utilizing a DNA nanonucleic-acid carrier, the therapeutic moieties are delivered as an integrated construct into tumor cells. This configuration increases targeting specificity, reduces adverse effects on normal cells, and enables improved intracellular synergy among the components, thereby enhancing overall therapeutic performance.

In summary: The targeted immuno- and gene-regulatory quadruple-combination nanonucleic-acid therapeutic achieves tumor-directed treatment by integrating three aptamers with anti-miR-21 molecules on a DNA carrier. Synergistic interactions among these moieties enhance immune-mediated attack on tumors, suppress tumor-cell growth, and improve targeting specificity and safety. This multi-target, multifunctional therapeutic exhibits substantial clinical application potential and may become an effective modality for cancer treatment. Illustrative application scenarios include:

1. Treatment of multiple cancer types: The therapeutic agent may be used to treat various cancers, including, without limitation, lung cancer, liver cancer, and breast cancer. It may be administered as monotherapy or in combination with other modalities (e.g., chemotherapy, radiotherapy, or molecularly targeted agents) to improve efficacy.

2. Personalized therapy: Owing to its high targeting specificity and safety profile, the composition may be adjusted according to patient-specific factors to implement personalized treatment, potentially reducing adverse effects, improving therapeutic outcomes, and prolonging survival.

3. Optimization of immunotherapy: The therapeutic agent may be combined with other immunotherapeutic approaches-such as immune checkpoint inhibitors or CAR-T cell therapies-to jointly enhance antitumor immunity. Such combinations may be more effective than monotherapy while reducing immune-related adverse events.

4. Development of drug-delivery platforms: Drawing on the design principles of the nanonucleic-acid combined immunotherapeutic, additional targeted and synergistic drug-delivery platforms may be developed to increase therapeutic efficacy, reduce side effects, and improve patient quality of life.

In summary, through synergistic interaction among its constituent moieties, the targeted immuno- and gene-regulatory quadruple-combination nanonucleic-acid immunotherapeutic is configured to improve treatment targeting specificity and safety, thereby offering a new approach to cancer therapy. In future research and clinical practice, such an innovative therapeutic agent may play an increasingly important role.

Drug 37): AS1411 Aptamer-ATAD2 siRNA-GPC3 Aptamer-5*AmiR-21-DNA Carrier

This is a targeted immuno- and gene-regulatory quadruple-combination nanonucleic-acid therapeutic that achieves tumor-directed treatment by combining AS 1411, ATAD2 siRNA, a GPC3 aptamer, and anti-miR-21 effector molecules on a DNA carrier. The functions and antitumor mechanisms of the respective components are as follows:

1. AS1411: A nucleic-acid aptamer exhibiting high binding specificity, configured to bind nucleolin (NCL) on the surface of tumor cells, which is overexpressed in multiple cancers. Upon binding NCL, AS1411 can inhibit tumor-cell proliferation by inducing apoptosis. AS1411 can also facilitate cellular uptake of the integrated nanonucleic-acid multi-gene regulator.

2. ATAD2 siRNA: ATPase family AAA-domain containing protein 2 (ATAD2) is a cancer-associated gene whose encoded protein is overexpressed in many tumor types. An ATAD2 small interfering RNA (siRNA) down-regulates ATAD2 expression, thereby suppressing tumor-cell growth. Incorporation of ATAD2 siRNA into the integrated nanonucleic-acid multi-gene regulator enhances inhibition of tumor cells.

3. GPC3 aptamer: Glypican-3 (GPC3) is a cell-surface heparan sulfate proteoglycan commonly overexpressed on epithelial tumor cells such as hepatocellular carcinoma. The GPC3-targeting aptamer functions as a targeting ligand, enabling the multi-gene regulator to enter GPC3-expressing cancer cells with increased specificity and thereby enhancing tumor-cell selectivity.

4. Anti-miR-21: MicroRNA-21 (miR-21) is a small non-coding RNA overexpressed in many cancers that typically promotes tumor-cell proliferation and suppresses apoptosis. An anti-miR-21 oligonucleotide is configured to bind miR-21 and prevent its association with its target gene transcripts (mRNAs), thereby reducing miR-21 activity. In this manner, anti-miR-21 inhibits tumor-cell growth and enhances the antitumor efficacy of the integrated nanonucleic-acid multi-gene regulator.

By assembling the foregoing moieties onto a DNA carrier, their synergistic action is achieved, thereby enhancing antitumor efficacy. The synergistic mechanisms by which these moieties exert antitumor effects are as follows:

1. Targeting specificity: The AS1411 aptamer and the GPC3 aptamer, by binding to nucleolin (NCL) and the GPC3 receptor, respectively, confer high tumor-cell targeting specificity, thereby facilitating precise delivery of the therapeutic composition to cancer cells, improving therapeutic efficacy, and reducing adverse effects on normal cells.

2. Anticancer gene regulation: ATAD2 siRNA and anti-miR-21 oligonucleotides, by targeting the ATAD2 gene and miR-21, respectively, are configured to suppress tumor-cell growth and modulate apoptotic signaling pathways. This dual gene-regulation strategy enhances antitumor activity and improves therapeutic efficacy.

3. Apoptosis induction: AS1411 is configured to inhibit tumor growth by inducing apoptosis in tumor cells. This activity synergizes with the anticancer actions of ATAD2 siRNA and anti-miR-21, thereby augmenting overall antitumor efficacy.

4. Enhanced drug delivery: The presence of the AS1411 and GPC3 aptamers is configured to enhance tumor-cell uptake of the DNA carrier, thereby increasing the bioavailability and therapeutic efficacy of the therapeutic composition.

Accordingly, the antitumor mechanism of the integrated, targeted immuno- and gene-regulatory quadruple-combination nanonucleic-acid therapeutic principally includes targeting specificity, anticancer gene regulation, apoptosis induction, and enhanced drug delivery. Through cooperative action of the constituent moieties, the composition is configured to achieve efficient treatment across multiple tumor types and may offer substantial potential for future research and clinical application in oncology.

Drug 38): Vap7 Aptamer-TGF-β Aptamer-Act-12c Aptamer-3* AmiR-21-DNA Carrier

This is a targeted immuno- and gene-regulatory quadruple-combination nanonucleic-acid therapeutic that achieves tumor-cell-directed treatment by combining a Vap7 aptamer, a TGF-β aptamer, an Act-12c aptamer, and anti-miR-21 effector molecules. The respective functions and antitumor mechanisms are as follows:

1. Vap7: An aptamer directed to tumor-cell-surface-specific molecules, configured to recognize and target tumor cells. By virtue of Vap7-mediated targeting, therapeutic moieties can be delivered more precisely to cancer cells, improving therapeutic efficacy while reducing adverse effects on normal cells.

2. TGF-β aptamer: Transforming growth factor-β (TGF-β) is overexpressed in many tumors and typically promotes tumor-cell growth, invasion, and metastasis. The TGF-β aptamer is configured to bind TGF-β and prevent its interaction with its receptor, thereby inhibiting activity of the TGF-β signaling pathway. In this manner, the TGF-β aptamer suppresses tumor-cell growth, invasion, and metastasis to exert an antitumor effect.

3. Act-12c aptamer: An aptamer targeting specific molecules within the immunosuppressive tumor microenvironment. Inclusion of the Act-12c aptamer enhances modulation of the tumor immune microenvironment by the therapeutic composition, thereby strengthening antitumor immune responses.

4. Anti-miR-21: MicroRNA-21 (miR-21) is a small non-coding RNA overexpressed in many cancers that typically promotes tumor-cell growth and inhibits apoptosis. An anti-miR-21 molecule is configured to bind miR-21 and prevent its association with target gene transcripts, thereby reducing miR-21 activity. Accordingly, anti-miR-21 suppresses tumor-cell growth and augments the antitumor efficacy of the integrated nanonucleic-acid multi-gene regulator.

By assembling the foregoing moieties onto a DNA carrier, their synergistic effects are realized, thereby enhancing antitumor efficacy. The synergistic mechanisms by which these moieties act against tumors are as follows:

1. Targeting specificity: The Vap7 aptamer, the TGF-β aptamer, and the Act-12c aptamer are configured to achieve highly specific targeting of tumor cells. This facilitates accurate delivery of the therapeutic composition to cancer cells, improves therapeutic efficacy, and reduces adverse effects on normal cells.

2. Regulation of tumor-associated signaling: Anti-miR-21 oligonucleotides, by targeting miR-21, are configured to suppress tumor-cell growth and modulate survival/apoptosis signaling pathways. In parallel, the TGF-β aptamer inhibits activity of the TGF-β signaling pathway by preventing ligand-receptor interaction, thereby suppressing tumor-cell growth, invasion, and metastasis. This multi-pathway regulation strategy enhances antitumor activity and improves therapeutic outcomes.

3. Immune modulation: The Act-12c aptamer modulates the tumor immune microenvironment and enhances antitumor immune responses. This activity acts in concert with the antitumor actions of the other moieties to augment overall efficacy.

4. Enhanced drug delivery: The presence of the Vap7, TGF-β, and Act-12c aptamers promotes tumor-cell uptake of the DNA carrier, thereby increasing the bioavailability and therapeutic effectiveness of the therapeutic composition.

Accordingly, the antitumor mechanisms of the composition principally include targeting specificity, regulation of tumor-associated signaling, immune modulation, and enhanced drug delivery. Through cooperative action of its constituent moieties, the integrated, targeted immuno- and gene-regulatory quadruple-combination nanonucleic-acid therapeutic is configured to achieve efficient treatment across multiple tumor types and may offer substantial potential for future research and clinical application in oncology.

Drug 39): AS1411 Aptamer-3* AmiR-21-IL-4Rα Aptamer-VCAM-1 Aptamer-DNA Carrier

This is a targeted immuno- and gene-regulatory quadruple-combination nanonucleic-acid therapeutic configured to achieve tumor-cell-directed treatment by combining the AS 1411 aptamer, anti-miR-21 effector molecules, an IL-4Rα aptamer, and a VCAM-1 aptamer. The respective functions and antitumor mechanisms are as follows:

1. AS1411 aptamer: AS1411 is a nucleic-acid aptamer exhibiting high binding specificity. It primarily binds nucleolin (NCL) on the surface of tumor cells, a protein overexpressed in multiple cancers. By binding NCL, AS1411 induces apoptosis and suppresses tumor-cell proliferation. AS1411 can also facilitate cellular uptake of the integrated nanonucleic-acid multi-gene regulator.

2. Anti-miR-21: MicroRNA-21 (miR-21) is a small non-coding RNA overexpressed in many cancers that typically promotes tumor-cell proliferation and suppresses apoptosis. An anti-miR-21 molecule (e.g., an antisense oligonucleotide) is configured to bind miR-21 and prevent its association with its target gene transcripts (mRNAs), thereby reducing miR-21 activity. In this manner, anti-miR-21 inhibits tumor-cell growth and enhances the antitumor efficacy of the integrated nanonucleic-acid multi-gene regulator.

3. IL-4Rα Aptamer: The interleukin-4 receptor alpha subunit (IL-4Rα) is a cell-surface antigen typically expressed on cancer-associated immunosuppressive cells. An IL-4Rα-binding aptamer is configured to target such immunosuppressive cells, thereby improving the tumor immune microenvironment and enhancing antitumor immune responses.

4. VCAM-1 Aptamer: Vascular cell adhesion molecule-1 (VCAM-1) is a cell-surface protein that, in cancer, is typically involved in intercellular adhesion and signal transduction. A VCAM-1-binding aptamer is configured to target tumor vasculature and tumor cells, thereby inhibiting tumor-cell invasion and metastasis.

The synergistic mechanisms underlying the anti-tumor activity of these molecules include:

1. Targeting. The AS1411, IL-4RA and VCAM-1 aptamers exhibit a high degree of tumor-cell targeting, which facilitates accurate delivery of the therapeutic agent to cancer cells, thereby improving therapeutic efficacy while reducing adverse effects on normal cells.

2. Tumor-signaling regulation. An anti-miR-21 oligonucleotide specifically hybridizes to miR-21 and thereby modulates signaling pathways governing tumor-cell proliferation and apoptosis. In parallel, the AS1411, IL-4RA and VCAM-1 aptamers respectively regulate distinct signaling pathways so as to jointly inhibit tumor-cell growth, invasion and metastasis. Such multi-pathway regulation enhances anti-tumor activity and improves treatment outcomes.

3. Immunomodulation. The IL-4RA aptamer modulates the tumor immune microenvironment and enhances anti-tumor immune responses. This action operates synergistically with the anti-tumor effects of the other molecules to augment overall efficacy.

4. Drug delivery. The presence of the AS1411, IL-4RA and VCAM-1 aptamers promotes uptake of the DNA carrier (vector) by tumor cells, thereby increasing the bioavailability and efficacy of the therapeutic agent.

In summary, the anti-tumor mechanism of the pharmaceutical composition includes targeting, regulation of tumor-associated signaling pathways, immunomodulation, and drug delivery. This integrated, specificity-directed, four-in-one nano-nucleic-acid therapeutic composition, which combines immune-modulatory and gene-regulatory functions, achieves high therapeutic efficacy across multiple tumor types through the synergistic action of its constituent molecules. The inventive therapeutic composition may possess substantial application potential in future research and clinical practice and offers a new option for cancer treatment.

Drug 40) : GPC1 Aptamer-AS1411 Aptamer-EpCAM Aptamer-2* Anti-miR-21-DNA Carrier

This is an integrated, specificity-directed, four-component nano-nucleic-acid therapeutic that, by incorporating a GPC1 aptamer, an AS1411 aptamer, an EpCAM aptamer, and an anti-miR-21 effector (two copies) on a DNA carrier, is configured to achieve targeted treatment of cancer cells. The respective functions and anti-tumor mechanisms are as follows:

1. GPC1 aptamer. Glypican-1 (GPC1) is a cell-surface protein whose expression is elevated in many tumor types. A GPC1-binding aptamer specifically binds GPC1 on cancer cells, thereby enhancing tumor targeting of the therapeutic agent and reducing off-target effects on normal cells.

2. AS1411 aptamer. AS1411 is a nucleic-acid aptamer with high binding specificity that primarily binds cell-surface nucleolin (NCL), a protein overexpressed in multiple cancers. Through NCL binding, AS1411 induces apoptosis and inhibits proliferation of tumor cells, and further facilitates cellular uptake/internalization of the integrated nano-nucleic-acid multi-gene regulator.

3. EpCAM aptamer. Epithelial cell adhesion molecule (EpCAM) is a cell-surface antigen commonly overexpressed on epithelial tumor cells. The EpCAM-binding aptamer serves as a targeting ligand to direct the multi-gene regulator preferentially into cancer cells, thereby increasing tumor-cell specificity of the integrated nano-nucleic-acid therapeutic.

4. Anti-miR-21 (AmiR-21). MicroRNA-21 (miR-21) is a small RNA overexpressed in many cancers that promotes growth and suppresses apoptosis in tumor cells. The anti-miR-21 effector hybridizes to and sequesters miR-21, preventing its interaction with target transcripts and reducing miR-21 activity. Consequently, anti-miR-21 inhibits tumor-cell growth and augments the anti-tumor efficacy of the integrated nano-nucleic-acid multi-gene regulator.

The synergistic mechanisms underlying the anti-tumor activity of these molecules include:

1. Targeting. The GPC1, AS1411 and EpCAM aptamers collectively enhance targeting of the therapeutic agent, enabling preferential entry into tumor cells and thereby improving therapeutic efficacy while reducing adverse effects on normal cells.

2. Inhibition of tumor growth. The anti-miR-21 molecule suppresses the activity of miR-21, thereby reducing the proliferative capacity of tumor cells. In parallel, the AS1411 aptamer induces apoptosis, further inhibiting tumor-cell proliferation. Acting in concert, these molecules achieve multi-pathway inhibition of tumor growth.

3. Tumor-microenvironment modulation. By targeting epithelial tumor cells, the EpCAM aptamer can affect intercellular adhesion and signal transduction, thereby altering the tumor microenvironment, which facilitates enhancement of the anti-tumor effect of the integrated nano-nucleic-acid multi-gene regulator.

4. Synergy. In combination, the foregoing molecules effect multi-pathway inhibition of tumor growth, metastasis and invasion. The GPC1, AS1411 and EpCAM aptamers increase the targeting of the therapeutic moieties, enabling anti-miR-21 to more selectively suppress cancer-cell growth. Such synergistic action confers enhanced anti-tumor efficacy on the integrated nano-nucleic-acid multi-gene regulator.

In summary, by combining multiple targeting ligands with an anti-miR-21 effector, the drug achieves multi-pathway suppression of tumors. The integrated, specificity-directed, four-component nano-nucleic-acid therapeutic can efficiently inhibit the growth and progression of multiple tumor types and has substantial potential for clinical application.

Drug 41) : 3*AmiR-21-AS1411 Aptamer-CD40 Aptamer-2*Biotin-DNA Carrier

This is a specificity-directed immunomodulatory and gene-regulatory dual-modality nano-nucleic-acid therapeutic, comprising three copies of an anti-miR-21 oligonucleotide (AmiR-21), an AS1411 aptamer, a CD40-binding (agonist) aptamer, and a biocompatible DNA carrier bearing two biotin groups (2*biotin). The components act in concert to effect multi-pathway inhibition of tumors. The respective functions and anti-tumor mechanisms are as follows:

1. Anti-miR-21 (AmiR-21). microRNA-21 (miR-21) is a small RNA that is overexpressed in many cancers and typically promotes growth while suppressing apoptosis in tumor cells. The anti-miR-21 oligonucleotide is complementary to miR-21 and hybridizes thereto, thereby preventing miR-21 from associating with its target transcripts and reducing miR-21 activity. In this manner, anti-miR-21 inhibits tumor-cell growth.

2. AS1411 aptamer. AS1411 is a nucleic-acid aptamer with high binding specificity that primarily binds cell-surface nucleolin (NCL), a protein overexpressed in multiple cancers. Upon NCL binding, AS1411 induces apoptosis and inhibits proliferation of tumor cells, and further facilitates cellular uptake/internalization of the nano-nucleic-acid therapeutic.

3. CD40 aptamer. The CD40-binding aptamer agonizes CD40, thereby activating immune cells in the peritumoral milieu (e.g., dendritic cells) and promoting T-cell activation, which enhances anti-tumor immune responses.

4. 2*Biotin-DNA carrier. A biocompatible DNA carrier operably links the anti-miR-21, AS1411 aptamer and CD40 aptamer components with two biotin moieties. The carrier improves stability and bioavailability of the therapeutic assembly while reducing potential immunogenicity.

Antitumor Mechanism of Action:

1. Inhibition of tumor growth: An anti-miR-21 agent reduces the activity of microRNA-21 (miR-21), thereby suppressing tumor-cell growth and invasion. In addition, the AS1411 aptamer induces apoptosis of tumor cells and inhibits their proliferation.

2. Augmentation of antitumor immunity: Activation of the CD40 pathway stimulates immune cells, including dendritic cells and T cells, thereby enhancing immune attack against tumors and activating immune responses within the tumor microenvironment.

3. Improved targeting selectivity: The AS 1411 aptamer binds to nucleolin (NCL) on the surface of tumor cells, thereby increasing the tumor specificity of the nucleic-acid nanotherapeutic and reducing adverse effects on normal cells.

4. Role of biotin: Biotin, as a molecular tag, contributes to improved stability and in-vivo bioavailability of the nucleic-acid nanotherapeutic, while reducing potential immunogenicity. In the disclosed nanotherapeutic, biotin can further enhance antitumor activity in the following ways:

1. Enhanced targeting: Biotin specifically binds to biotin receptors highly expressed on the surface of tumor cells, thereby directing the nanotherapeutic more precisely to tumor cells and reducing side effects on normal cells.

2. Improved stability and bioavailability: As a molecular tag, biotin helps maintain the stability of the nanotherapeutic and increases its in-vivo bioavailability.

3. Synergistic effect: Biotin acts in concert with other components (e.g., anti-miR-21, the AS1411 aptamer, and CD40 ligand, CD40L) to achieve multi-pathway suppression of tumor growth, thereby improving overall therapeutic efficacy.

Accordingly, through the foregoing actions, biotin plays an important synergistic role in the nucleic-acid nanotherapeutic that integrates specific immunomodulation and gene regulation, contributing to enhanced overall antitumor effects.

Drug 42): IL-4Rα aptamer-VCAM-1 aptamer-PD-L1 aptamer-3*anti-miR-21-DNA carrier

This agent is a quadruple-combination nucleic-acid nanotherapeutic integrating specific immunomodulation and gene regulation. The components are assembled on a DNA carrier in a sequence-extension manner to form a unitary therapeutic molecule that is capable of efficiently inhibiting the growth and progression of multiple tumor types. The functions of each moiety and their cooperative effects are as follows:

1. IL-4RA (IL-4Rα) aptamer. Interleukin-4 receptor alpha (IL-4Rα) is a cell-surface receptor whose expression is elevated in certain cancers and is associated with tumor development and aggravation. An IL-4Rα-binding nucleic-acid aptamer targets IL-4Rα on tumor cells, thereby enhancing the tumor specificity of the nanotherapeutic.

2. VCAM-1 aptamer. Vascular cell adhesion molecule-1 (VCAM-1) is a cell-adhesion molecule highly expressed on tumor vascular endothelial cells and plays a key role in tumor infiltration and metastasis. A VCAM-1-binding aptamer enables preferential entry of the nanotherapeutic into tumor tissue, thereby improving therapeutic efficacy.

3. PD-L1 aptamer. Programmed death-ligand 1 (PD-L1) is an immune-checkpoint molecule overexpressed in many tumors. Upon binding to PD-1 on immune cells, PD-L1 suppresses immune activity and facilitates immune evasion. A PD-L1-binding aptamer antagonizes the PD-1/PD-L1 interaction, thereby enhancing immune-cell attack against tumor cells.

4. Anti-miR-21: MicroRNA-21 (miR-21) is a microRNA overexpressed in multiple tumor types and plays an important role in tumor-cell proliferation, invasion, and suppression of apoptosis. An anti-miR-21 oligonucleotide, by sequence-specific hybridization to miR-21, prevents miR-21 from binding to its target transcripts (mRNAs), thereby reducing the functional activity of miR-21 and, consequently, inhibiting tumor-cell growth and invasion.

5. DNA carrier (scaffold). A nucleic-acid DNA carrier serves as a nanostructured scaffold that co-assembles the foregoing aptamers and the anti-miR-21 moieties into a single integrated therapeutic molecule via sequence extension, providing structural stability and facilitating efficient entry into tumor cells and subsequent release of the respective active components.

Synergistic Effects:

1. Targeting specificity. Binding by the IL-4Rα, VCAM-1, and PD-L1 aptamers confers enhanced tumor selectivity on the nucleic-acid nanotherapeutic. By concurrently targeting these three tumor-associated targets (receptors), the construct more effectively identifies and engages tumor cells while reducing damage to normal cells.

2. Immune activation. By blocking the interaction between PD-L1 and the PD-1 receptor, immune-cell activity is potentiated, facilitating recognition and clearance of tumor cells and thereby improving the efficacy of immunotherapy.

3. Antitumor activity. Anti-miR-21 inhibits tumor-cell growth and invasion. In the presence of the three aptamer modules, intracellular delivery of anti-miR-21 into tumor cells is facilitated, thereby augmenting the antitumor effect.

4. Combination modality. The disclosed quadruple-combination nucleic-acid nanotherapeutic integrating specific immune modulation and gene regulation combines tumor-cell targeting, immune-cell activation, and suppression of tumor growth within a single, unitary construct. This combination strategy enables multi-pathway intervention against tumors and enhances therapeutic outcomes.

In summary, the quadruple-combination nanotherapeutic is an integrated therapeutic molecule that couples tumor targeting, immune activation, and growth inhibition; it efficiently recognizes and engages tumor cells, enhances immune-mediated clearance, and suppresses tumor-cell growth and invasion. Such synergistic effects indicate substantial potential utility in antitumor therapy.

Drug 43): TAP siRNA - 3*anti-miR-21 - 2*AS1411 aptamer - DNA carrier

This agent is a specific gene-regulation dual-combination nucleic-acid nanotherapeutic that combines TAP siRNA, anti-miR-21, and AS1411 aptamer moieties on a DNA carrier to form a single, integrated therapeutic construct for efficient antitumor intervention. The functional roles of the respective components and their cooperative mechanisms are as follows:

1. TAP siRNA. TAP (transporter associated with antigen processing, "TAP") is present in tumor cells and is involved in transporting antigenic peptides for presentation at the cell surface. A TAP-targeting small interfering RNA (TAP siRNA) transiently reduces TAP expression, thereby decreasing the display of tumor antigens at the cell surface and rendering tumor cells more readily recognized and attacked by the immune system.

2. Anti-miR-21: MicroRNA-21 (miR-21) is a microRNA overexpressed in multiple cancers that, in tumor cells, promotes proliferation and suppresses apoptosis. An anti-miR-21 oligonucleotide, by sequence-specific hybridization to miR-21, prevents miR-21 from binding to its target gene transcripts (mRNAs), thereby reducing the functional activity of miR-21. Consequently, anti-miR-21 inhibits tumor-cell growth and enhances the antitumor efficacy of the integrated multi-gene-regulation nucleic-acid nanotherapeutic.

3. AS1411: AS1411 is a nucleic-acid aptamer characterized by high binding specificity. It binds predominantly to cell-surface nucleolin (NCL) on tumor cells, a protein overexpressed in multiple cancers. Upon binding to NCL, AS1411 inhibits tumor-cell proliferation by inducing apoptosis. In addition, AS1411 promotes cellular uptake (e.g., receptor-mediated internalization) of the integrated multi-gene-regulation nucleic-acid nanotherapeutic.

4. DNA carrier (scaffold): A DNA-based carrier configured to co-assemble TAP siRNA, anti-miR-21 oligonucleotide(s), and AS 1411 aptamer moieties into a single, integrated (unitary) therapeutic molecule. The carrier provides a structurally stable framework for the aptamer and anti-miR-21 components, thereby facilitating efficient uptake by tumor cells and subsequent intracellular release of the respective active agents.

Synergistic Effects:

1. Targeting specificity. Binding by the AS 1411 aptamer confers tumor-cell targeting on the nucleic-acid nanotherapeutic, thereby reducing adverse effects on normal cells and improving therapeutic efficacy.

2. Immune recognition. TAP siRNA transiently reduces and/or modulates expression of TAP, thereby decreasing display of tumor antigens at the cell surface and, as a consequence, enhancing the susceptibility of tumor cells to immune recognition and attack. This heightened immune recognition improves the effectiveness of immunotherapy.

3. Antitumor activity. By lowering the functional activity of miR-21, anti-miR-21 inhibits tumor-cell growth. In combination with the antitumor actions of TAP siRNA and AS 1411, the construct synergistically suppresses tumor-cell growth and progression.

4. Apoptosis. Upon binding to nucleolin (NCL), the AS1411 aptamer induces apoptosis of tumor cells. This pro-apoptotic effect promotes elimination of tumor cells and thereby suppresses tumor growth and dissemination.

5. Drug delivery. A DNA carrier serves as a stable scaffold that enables efficient cellular uptake and intracellular release of the TAP siRNA, anti-miR-21, and AS1411 aptamer components, thereby increasing bioavailability and enhancing antitumor efficacy.

In summary, the disclosed specific gene-regulation dual-combination nucleic-acid nanotherapeutic integrates multiple functional moieties that act cooperatively to suppress tumor-cell growth and progression. The unitary construct leverages the complementary functions of its components to improve therapeutic outcomes and provides a promising direction for cancer therapy.

Drug 44): PD-1 aptamer-AS1411 aptamer-PD-L1 aptamer-3*anti-miR-21 - DNA carrier

This agent is a specific immunomodulation and gene-regulation triple-combination nucleic-acid nanotherapeutic in which the foregoing components are co-assembled on a DNA carrier via sequence-extension to form a single, unitary therapeutic molecule. The construct is configured to effectively inhibit the growth and progression of multiple tumor types. The functional roles of each component are as follows:

1. PD-1 aptamer. Programmed cell death protein 1 (PD-1) is an immune-checkpoint receptor primarily expressed on immune cells. Tumor cells evade immune attack by expressing programmed death-ligand 1 (PD-L1), which binds PD-1 and suppresses immune-cell activity. A PD-1-binding aptamer blocks the PD-1/PD-L1 interaction, thereby restoring antitumor immune activity against tumor cells and suppressing tumor growth.

2. AS1411 aptamer. AS1411 is a nucleic-acid aptamer with high binding specificity that binds predominantly to cell-surface nucleolin (NCL) on tumor cells, a protein overexpressed in many cancers. Upon binding to NCL, AS1411 induces apoptosis and thereby inhibits tumor-cell proliferation. AS1411 also promotes cellular uptake (e.g., receptor-mediated internalization) of the integrated multi-gene-regulation nucleic-acid nanotherapeutic.

3. PD-L1 aptamer. A PD-L1-binding aptamer targets PD-L1 on tumor-cell surfaces and prevents its interaction with PD-1, thereby activating immune-cell attack on tumor cells and enhancing the effectiveness of immunotherapy.

4. Anti-miR-21: MicroRNA-21 (miR-21) is a microRNA overexpressed in multiple cancers that, in tumor cells, promotes proliferation and suppresses apoptosis. An anti-miR-21 oligonucleotide hybridizes sequence-specifically to miR-21, thereby preventing miR-21 from binding to its target gene transcripts (mRNAs) and reducing the functional activity of miR-21. Consequently, anti-miR-21 inhibits tumor-cell growth and enhances the antitumor efficacy of the integrated multi-gene-regulation nucleic-acid nanotherapeutic.

Through synergistic action, the molecules described herein exert anti-tumor effects in at least the following aspects:

a) Restoration of immune-cell activity: Binding by the PD-1 aptamer and the PD-L1 aptamer relieves tumor-mediated suppression of immune cells and restores their capacity to attack tumor cells. The PD-1 aptamer exhibits a substantially lower binding affinity than the PD-L1 aptamer and therefore functions in a secondary, subordinate adjacent-recognition role. This affinity-differentiated design ensures anchoring of the nanoparticle within the tumor microenvironment while enabling adjacent recruitment and activation of T-cell immune responses. Such affinity-tiered dual blockade is conducive to re-establishing systemic anti-tumor immunity in patients with relapsed, resistant, and refractory tumors, while reducing potential immune-related toxicities.

b) Tumor-cell targeting: The AS1411 aptamer binds nucleolin (NCL) on the surface of tumor cells, increasing the specificity of the therapeutic agent for tumor cells and reducing effects on normal cells.

c) Induction of tumor-cell apoptosis: The AS 1411 aptamer induces apoptosis of tumor cells, thereby inhibiting tumor growth.

d) Inhibition of miR-21 activity: An anti-miR-21 agent prevents miR-21 from binding to its target genes, thereby decreasing its activity and suppressing tumor-cell growth.

In summary, the integrated triple-combination nano-nucleic acid therapeutic that couples targeted immunity with gene regulation-through the synergy of multiple aptamers and the anti-miR-21 agent-achieves targeted treatment of tumor cells, restores immune-cell attack capability, and inhibits tumor-cell proliferation. Such an integrated therapeutic has the potential to provide new breakthroughs in cancer treatment.

Drug 45) : CpG1826-CD40 Aptamer-anti-miR-21-3*Biotin-DNA carrier

This disclosure relates to a nucleic-acid nanotherapeutic that integrates specific active immunization with gene regulation as a triple-combination therapy and is intended to inhibit tumor growth and progression. The functional components and their cooperative effects are set forth below:

1. CpG1826. CpG1826 is a CpG oligodeoxynucleotide (ODN) that mimics the immunostimulatory activity of bacterial DNA. By activating Toll-like receptor 9 (TLR9), CpG1826 promotes activation and proliferation of immune cells (such as dendritic cells and B cells), thereby enhancing antitumor immune responses.

2. CD40. Engagement of CD40 with its ligand CD40L activates immune cells and augments antitumor immunity; for example, it induces dendritic-cell maturation and increases tumor-antigen presentation, thereby stimulating T-cell activation. In the present construct, a CD40 aptamer serves as the CD40-agonist module.

3. AmiR-21. AmiR-21 is an anti-microRNA agent directed against microRNA-21 (miR-21). miR-21 is overexpressed in multiple tumors and promotes tumor-cell growth and resistance to apoptosis. By binding miR-21 and preventing its interaction with target genes, AmiR-21 inhibits miR-21 activity and thereby slows tumor growth.

4. 3*Bio. Denotes three biotin moieties. Biotin can specifically bind to biotin-binding receptor proteins on the surface of tumor cells. Conjugation of biotin to the therapeutic construct improves targeting to tumor cells and reduces effects on normal cells.

5. DNA nucleic-acid carrier (scaffold). The foregoing functional moieties are combined via sequence extension on a DNA scaffold to form an integrated therapeutic construct, thereby achieving synergistic action.

In summary, the integrated nucleic-acid nanotherapeutic combining specific active immunization and gene regulation achieves tumor-cell targeting, enhances antitumor immune responses, and inhibits tumor-cell growth through the concerted action of multiple functional modules, and is expected to provide a new solution for cancer therapy.

Drug 46) : ATP Aptamer-anti-miR-21-FGF2 Aptamer-anti-miR-1306-DNA carrier

Pain has become the third major health problem after cardiovascular/cerebrovascular diseases and cancer, seriously affecting human health and quality of life. Pain is a warning signal of tissue injury or disease invasion and is a common clinical symptom, whereas chronic pain is an independent disease entity.

An ATP-aptamer-AmiR-21-FGF2-aptamer-AmiR-1306-DNA carrier is a novel, specific gene-regulation, dual-combination nucleic-acid nanotherapeutic and functions as an integrated analgesic. Its mechanism of action primarily involves the following:

1. ATP aptamer: ATP (adenosine triphosphate) is the most important intracellular molecule for energy storage and transfer. In living organisms, ATP transfers phosphate groups from one molecule to another to effect energy transduction. An ATP aptamer is an oligonucleotide capable of binding ATP; by binding ATP, it can modulate the P2X3 receptor, thereby reducing pain perception.

2. AmiR-21: formally AntagomiR-21, an inhibitory oligonucleotide specifically targeting miR-21. By binding to miR-21, AmiR-21 regulates the level of miR-21 and thus alleviates pain. miR-21 is a non-coding RNA whose primary function is to bind the mRNA of target genes and thereby regulate target-gene expression.

3. FGF2 aptamer: an oligonucleotide capable of binding FGF2. By binding FGF2, it can inhibit the interaction of FGF2 with its cellular receptor, thereby mitigating bone disorders and pain. FGF2 (fibroblast growth factor-2) is a bioactive polypeptide that mainly participates in cell proliferation, differentiation, and migration. FGF2 plays an important role in tissue repair, neuronal growth, and in the initiation and maintenance of pain.

4. AmiR-1306: formally AntagomiR-1306-3p, an inhibitory oligonucleotide specifically targeting miR-1306-3p. By binding to miR-1306-3p, AmiR-1306-3p regulates the level of miR-1306-3p, thereby reducing pain perception. miR-1306-3p is a microRNA, i.e., a non-coding RNA molecule, that primarily binds to target-gene mRNA to affect its expression and thereby regulate biological processes.

5. DNA nucleic-acid nanocarrier (DNAh): configured to achieve stable encapsulation, protection, and targeted delivery of therapeutic molecules.

In summary, the integrated analgesic composition exerts effective inhibition of pain through multiple pathways, including targeting the ATP-dependent P2X3 receptor, regulating the levels of miR-21-5p and miR-1306-3p, and targeting FGF2. The specific mechanisms are as follows:

1. Targeting the ATP-dependent P2X3 receptor: the ATP aptamer in the analgesic has specificity for ATP and is capable of binding to and sequestering/blocking ATP, thereby inhibiting activation of the P2X3 receptor. As the P2X3 receptor plays a key role in nociceptive transmission, inhibiting its activation helps effectively reduce pain perception in a subject.

2. Regulating miR-21-5p levels: AmiR-21, as a component of the analgesic, regulates the level of miR-21-5p. Lowering miR-21-5p can attenuate neurogenic hypersensitivity and the recruitment of inflammatory macrophages in the dorsal root ganglion (DRG), thereby alleviating pain.

3. Targeting fibroblast growth factor-2 (FGF2): the FGF2 aptamer in the analgesic can block the binding of FGF2 to its four cellular receptors. By inhibiting FGF2-induced downstream signaling and cell proliferation, and by restoring osteoblast differentiation suppressed by FGF2, the agent helps reduce bone disorders and pain.

4. Regulating miR-1306-3p levels: studies have shown that miR-1306-3p has key interaction sites within the extracellular domain of P2X3R. Through AmiR-1306-3p, the analgesic-under targeting guidance of the DNAh carrier and the aptamers-can enter exosomes, down-regulate miR-1306-3p levels, modulate P2X3 receptor activity, and thereby reduce pain perception.

5. DNA Nucleic-Acid Nanocarrier. The DNAh nucleic-acid nanocarrier assembles the foregoing therapeutic moieties into a highly integrated nanoscale complex. Such an architecture facilitates multi-pathway synergistic actions to enhance analgesic efficacy. In addition, the DNAh nucleic-acid nanocarrier protects the encapsulated therapeutic cargo, increases cargo stability, optimizes particle size, enables penetration across biological barriers, and coordinates the release rate and duration of action of multiple therapeutic moieties.

This integrated, targeted gene-regulation dual-combination nucleic-acid nanotherapeutic, as compared with opioid-class agents, offers advantages of fewer adverse effects, stronger target specificity, multi-pathway synergy, broad potential indications, and a reduced propensity for drug tolerance. Accordingly, the targeted gene-regulation dual-combination nucleic-acid nanotherapeutic is expected to serve as an effective non-opioid alternative analgesic, with substantial research value and promising clinical application prospects.

Drug 47) : CD16a Aptamer-CTLA-4 Aptamer-PD-L1 Aptamer-DNA Carrier

This agent is a targeted immunomodulatory triple-combination nucleic-acid nanotherapeutic. The therapeutic construct is assembled on a DNA carrier by sequential extension to form a composite therapeutic molecule. The molecule exhibits potent inhibition of the growth and progression of multiple tumors. The mechanistic basis is analyzed below from the standpoint of the functions of the individual components and their synergistic effects:

1. CD16a: CD16a (FcγRIIIa) is a receptor expressed on the surface of immune cells, predominantly on natural killer (NK) cells and macrophages. By binding to antibodies, CD16a mediates activation of immune effector cells and thereby produces antitumor effects. Accordingly, CD16a can serve as a target for immunotherapy; incorporation of a CD16a-targeting aptamer enhances the tumor-cell specificity of the integrated nucleic-acid nanotherapeutic for combination immunotherapy.

2. CTLA-4: CTLA-4 (cytotoxic T-lymphocyte-associated antigen 4) is an inhibitory receptor expressed on immune cells. Through interaction with CD80/CD86, it suppresses T-cell activation and proliferation. Therefore, a CTLA-4 aptamer can block the interaction between CTLA-4 and its ligands, thereby releasing T-cell inhibition and increasing the capacity of immune cells to attack tumor cells.

3. PD-L1: PD-L1 (programmed death-ligand 1) is a protein expressed on the surface of tumor cells which, by binding to the PD-1 (programmed death 1) receptor, inhibits immune-cell activity and enables tumor cells to evade immune surveillance. Via high-affinity binding to PD-L1 (by the PD-L1 aptamer), the integrated nucleic-acid nanotherapeutic for combination immunotherapy can block the PD-L1/PD-1 interaction, thereby activating immune cells to attack tumor cells.

Synergistic effects:

CD16a, CTLA-4 and PD-L1 aptamers act synergistically during treatment, thereby achieving more efficient and effective antitumor activity.

1. CD16a × CTLA-4 synergy: Activation of immune effector cells is induced via CD16a, and the interaction between CTLA-4 and its ligands is concurrently blocked, thereby simultaneously activating immune cells and releasing inhibitory signals. Such synergy enhances the ability of immune cells to attack tumor cells, improving therapeutic efficacy.

2. CD16a × PD-L1 synergy: By binding PD-L1, the integrated nucleic-acid nanotherapeutic for active combination immunotherapy blocks the PD-L1/PD-1 interaction and thereby activates immune-cell attack on tumor cells. In parallel, CD16a induces immune-cell activation, further strengthening the cytotoxic activity of immune cells against tumor cells. This synergy contributes to more effective antitumor outcomes.

3. CTLA-4 × PD-L1 synergy: Blocking the interaction between CTLA-4 and its ligands releases T-cell inhibition and increases the ability of immune cells to attack tumor cells. At the same time, binding PD-L1 activates immune-cell attack on tumor cells, thereby further enhancing antitumor efficacy. This synergy helps achieve a stronger immunotherapeutic effect.

In summary, the integrated, targeted, immunoregulatory triple-combination nucleic-acid nanotherapeutic achieves potent inhibition of the growth and progression of multiple tumors by exploiting the synergistic actions of the three aptamer components. The integrated molecule simultaneously modulates multiple immunotherapy targets: the PD-L1 aptamer, owing to its high affinity, has priority to direct targeting and checkpoint blockade; the CD16a and CTLA-4 aptamers, having comparatively secondary affinity, operate via a proximity-recognition mechanism to recruit NK cells and to further close/block the CTLA-4 checkpoint on T cells. As a result, the antitumor capacity of immune cells is fully mobilized, providing tumor patients with a new and effective therapeutic approach.

Drug 48) : CD16a-aptamer-Act-12c-aptamer-PD-Ll-aptamer-DNA carrier

This agent is a targeted immunoregulatory triple-combination nucleic-acid nanotherapeutic. It includes a DNA carrier on which are disposed three aptamers that specifically bind CD16a, Act-12c, and PD-L1, respectively, so as to achieve potent inhibition of tumors. By concurrently modulating multiple immunotherapy targets, the integrated therapeutic mobilizes the antitumor activity of immune effector cells and provides a new and effective treatment option for cancer patients. The functional roles of the respective components and their cooperative effects are as follows:

1. CD16a aptamer: CD16a is an activating receptor on immune-cell surfaces, mainly on natural killer (NK) cells and macrophages. The CD16a aptamer binds CD16a to activate immune cells and enhance their cytotoxic attack on tumor cells. Binding to CD16a can further induce antibody-dependent cellular cytotoxicity (ADCC)-the CD16a aptamer acting functionally analogous to an anti-CD16a antibody-thereby promoting clearance of tumor cells.

2. Act-12c aptamer: Act-12c is an immune-stimulatory agonist capable of eliciting antitumor responses by immune cells. Upon binding Act-12c, the integrated nucleic-acid nanotherapeutic for active combination immunotherapy stimulates immune cells to produce large amounts of antitumor cytokines, thereby augmenting the immune attack on tumor cells.

3. PD-L1 aptamer: PD-L1 is an immunosuppressive protein commonly overexpressed on tumor-cell surfaces. By binding the PD-1 receptor on immune cells, PD-L1 suppresses immune-cell activity and enables tumor immune evasion. Through specific binding to PD-L1, the integrated nucleic-acid nanotherapeutic blocks the PD-L1/PD-1 interaction, thereby releasing immune inhibition and enhancing immune-cell cytotoxicity against tumor cells.

Synergistic Effects: By incorporating CD16a, Act-12c, and PD-L1 as three functional modules, the integrated nucleic-acid nanotherapeutic for active combination immunotherapy achieves potent inhibition of tumors. Under the synergistic action of CD16a, Act-12c and PD-L1, the antitumor activity of the integrated agent is realized as follows:

1. Activation of multiple immune-cell types: Upon binding CD16a, the agent is configured to activate various immune effector cells, including natural killer (NK) cells and macrophages, thereby enhancing their cytotoxic attack on tumor cells. Concurrently, stimulation by Act-12c further increases immune-cell activity, eliciting a stronger antitumor response.

2. Enhanced recognition of tumor cells by immune cells: PD-L1 is commonly overexpressed on tumor-cell surfaces. Through specific binding to PD-L1, the agent blocks the interaction between PD-L1 and the PD-1 receptor on immune cells, thereby improving immune-cell recognition of tumor cells and enabling more effective killing of the tumor cells.

3. Release of antitumor cytokines: Via Act-12c, the agent specifically recruits and activates CD4$^+$ T cells, thereby stimulating immune cells to produce large amounts of antitumor cytokines. These cytokines potentiate immune-cell cytolytic activity and promote recruitment of additional immune cells to tumor tissue, resulting in a coordinated, synergistic antitumor effect.

4. Immune-memory effect: As immune cells attack tumor cells, the immune system establishes durable antitumor immunological memory. When tumor cells re-emerge, the immune system can rapidly recognize and attack them, thereby reducing the risk of tumor recurrence.

In summary, the integrated nucleic-acid nanotherapeutic for active combination immunotherapy is a targeted triple-combination construct that, by synergistically acting on multiple immunotherapy targets including CD16a, Act-12c, and PD-L1, is configured to activate immune cells, enhance tumor-cell recognition, induce the release of antitumor cytokines, and establish an immunological memory response, thereby effectively inhibiting the growth and progression of various tumors. This synergistic mechanism provides an antitumor strategy with broad prospects. Representative application scenarios and advantages include:

1. Broad applicability across tumor types: Because the targeted molecules are widely present on the surfaces of many tumor cells, the integrated agent has wide antitumor application potential and can serve as a generally applicable therapeutic approach for multiple tumor types.

2. Improved efficacy of immunotherapy: By acting concurrently on multiple immunotherapy targets, the antitumor effect of the agent is greater than that of single-target regimens, thereby improving the success rate of immunotherapy and providing more effective options for clinical use.

3. Reduced adverse effects and toxicity: By operating on specific immunotherapy targets, the integrated agent can avoid damage to normal cells, thereby reducing treatment-related side effects and toxicity and improving patient quality of life.

4. Overcoming immune tolerance: By releasing inhibitory signals on immune cells, the agent can overcome patient immune tolerance and thus improve the long-term efficacy of immunotherapy.

5. Personalized treatment strategy: The integrated agent can be optimized and adjusted according to a patient's tumor type and immune status, thereby enabling personalized therapy to meet individual clinical needs.

Accordingly, this integrated, targeted, immunoregulatory triple-combination nucleic-acid nanotherapeutic, through synergistic modulation of multiple immunotherapy targets, offers substantial advantages and wide application prospects. With continued research and clinical evaluation, the agent is expected to provide more effective and safer therapeutic options for cancer patients.

Drug 49) : CTLA-4-aptamer-PD-L1-aptamer-PD-1-aptamer-DNA carrier

This disclosure relates to a targeted immunoregulatory triple-combination nucleic-acid nanotherapeutic. The three aptamer moieties are assembled on a DNA carrier by a sequential-extension mode to form an integrated therapeutic molecule that is configured to potently inhibit the growth and progression of multiple recurrent, drug-resistant, and refractory tumors. The mechanism of action and synergistic effects are set out below:

1. PD-L1 aptamer. PD-L1 (programmed death ligand-1) is an immunosuppressive protein on the surface of tumor cells that, upon binding to its receptor PD-1, suppresses immune-cell activity and enables tumor immune evasion. A PD-L1-binding aptamer having high affinity for PD-L1 can guide the nanoparticle to the tumor microenvironment and functionally occlude PD-L1, thereby blocking its interaction with PD-1 and restoring immune-cell activity.

2. CTLA-4 aptamer. CTLA-4 (cytotoxic T-lymphocyte-associated antigen 4) is an immune checkpoint molecule expressed on T cells which binds members of the B7 family and inhibits T-cell activation and proliferation. A CTLA-4 aptamer binds CTLA-4 and blocks its interaction with B7 ligands, thereby enhancing T-cell immune responses.

3. PD-1 aptamer: PD-1 (programmed death-1) is an immune-checkpoint receptor primarily expressed on the surface of T cells; upon binding PD-L1 it suppresses T-cell activity. A PD-1-binding aptamer is configured to bind PD-1 protein and block its interaction with PD-L1, thereby restoring T-cell immune function.

4. Synergy: By simultaneously targeting PD-L1, CTLA-4, and PD-1, the triple-aptamer nucleic-acid nanotherapeutic is configured to effectively relieve tumor-mediated immunosuppression. This synergistic action enhances immune-cell attack against tumor cells, enabling efficient treatment of recurrent, treatment-resistant, and refractory tumors.

5. CTLA-4 × PD-1 aptamer synergy: CTLA-4 and PD-1 are distinct immune checkpoints that act at different stages of the T-cell response-CTLA-4 primarily during initial T-cell activation, and PD-1 during the effector phase. Concurrent blockade of both checkpoints more effectively activates T cells and augments their cytotoxic activity toward tumor cells.

6. CTLA-4/PD-1 aptamers with PD-L1 aptamer synergy: Co-targeting CTLA-4, PD-1, and PD-L1 removes tumor-cell immunosuppression at multiple levels. On one hand, blocking CTLA-4 and PD-1 restores T-cell activity; on the other hand, occluding PD-L1 prevents tumor cells from exploiting the PD-1/PD-L1 pathway to evade immune attack. This multi-pronged mechanism facilitates effective therapy for recurrent, resistant, and refractory tumors.

7. Tumor microenvironment (TME). The TME contains numerous immunosuppressive factors, such as regulatory T cells (Tregs) and tumor-associated macrophages (TAMs). These factors suppress T-cell activity through immune-checkpoint pathways, including CTLA-4 and PD-1. Accordingly, simultaneous targeting of these checkpoints can more effectively overcome immunosuppression within the TME.

8. Safety. Conventional single-target immunotherapies may cause severe immune-related adverse events. By contrast, use of the triple-aptamer nucleic-acid nanotherapeutic for active combination immunotherapy enables precise tumor targeting and reduces effects on normal cells, thereby improving treatment safety.

9. Stronger immune activation. By concurrently engaging CTLA-4, PD-1, and PD-L1 checkpoints, the combination agent is configured to activate immune cells at multiple levels and enhance their cytotoxic attack against tumor cells. This multi-pronged activation helps overcome tumor resistance to immune surveillance and achieves effective therapy for difficult-to-treat tumors.

10. Improved therapeutic outcomes. Relative to a single immune-checkpoint inhibitor, the triple-aptamer agent provides superior efficacy. Studies indicate that such combination-checkpoint strategies can significantly prolong progression-free survival (PFS) and overall survival (OS) while reducing recurrence rates.

11. Applicability to multiple tumor types. The strategy is suitable for a broad spectrum of tumors, including solid tumors and hematologic malignancies. Moreover, because the approach does not depend on a specific tumor antigen, it may be applied across a wide patient population.

12. Lower adverse effects. Because the agent precisely targets tumor cells and reduces effects on normal cells, its adverse-event profile is lower than that of conventional single-target immunotherapies, thereby improving patient quality of life and decreasing the likelihood of treatment interruption.

13. Broader applicability. This treatment strategy is not only suitable for first-line therapy, but may also be used as second-line or later-line therapy for recurrent and treatment-resistant tumors, thereby providing additional therapeutic options for difficult-to-treat patients.

14. Personalized therapy. Because of inter-patient biological heterogeneity, single-agent immunotherapy may be ineffective for certain subjects. By concurrently targeting multiple immune checkpoints, the disclosed triple-aptamer nucleic-acid nanotherapeutic for active combination immunotherapy can increase response rates and deliver tangible clinical benefit to a larger proportion of patients.

15. Potential combination regimens. In addition to use as a monotherapy, the triple-aptamer nucleic-acid nanotherapeutic may be combined with other modalities (e.g., chemotherapy, radiotherapy, targeted therapy) to achieve improved outcomes. Such combination strategies help overcome resistance to single-modality therapy and enhance efficacy.

16. Continual research and optimization. With deeper understanding of immunotherapy mechanisms, the design and application of the triple-aptamer nucleic-acid nanotherapeutic can be further optimized to realize more effective and safer treatment. For example, modifying aptamer structures and/or combinations can improve targeting and bioactivity to suit different tumor types and disease stages.

17. Tumor-prevention strategy. In future applications, the targeted immunoregulatory triple-combination nucleic-acid therapeutic may be employed for early prevention of tumors-for example, by screening high-risk populations and administering immunotherapy to eligible subjects to prevent tumor initiation and progression.

Accordingly, the disclosed targeted immunoregulatory triple-combination nucleic-acid nanotherapeutic possesses substantial clinical potential and is configured to efficiently inhibit the growth and progression of multiple recurrent, resistant, and refractory tumors. It is expected to improve the current landscape of cancer therapy and to provide patients with better quality of life and survival prospects.

Drug 50) : PD-1 Aptamer-IL-4Rα Aptamer-OX40 Aptamer-DNA Carrier

This disclosure relates to a targeted immunoregulatory triple-combination nucleic-acid nanotherapeutic. The integrated construct is assembled on a DNA carrier by sequential extension, incorporating therapeutic moieties directed to PD-1, IL-4Rα, and OX40, and is configured to potently inhibit the growth and progression of multiple recurrent, treatment-resistant, and refractory tumors. The functional roles of the respective components and their cooperative effects are analyzed below:

1. PD-1 aptamer. PD-1 (Programmed Death-1) is an immune-checkpoint receptor predominantly expressed on T cells. The PD-1-binding aptamer binds PD-1 and blocks its interaction with PD-L1 (Programmed Death-Ligand 1), thereby releasing suppressed T cells and enhancing their cytotoxic activity against tumor cells.

2. IL-4Rα aptamer. IL-4Rα (interleukin-4 receptor alpha) is the receptor for cytokine IL-4. In the tumor microenvironment, IL-4/IL-4Rα signaling promotes M2 macrophage polarization, thereby dampening antitumor immunity. The IL-4Rα-binding aptamer binds IL-4Rα and blocks its interaction with IL-4, inhibiting M2-type macrophage formation and augmenting immune-cell attack on tumors.

3. OX40 aptamer. OX40 (also termed CD134) is a co-stimulatory receptor expressed on activated T cells; engagement with its ligand OX40L promotes T-cell activation, expansion, and survival. An agonistic OX40 aptamer that binds OX40 further enhances T-cell antitumor activity.

4. Synergy of the PD-1 aptamer and the IL-4Rα aptamer. By concurrently blocking PD-1 and IL-4Rα, immune effector cells are activated along two axes: in one aspect, the PD-1-binding aptamer reactivates suppressed T cells, enhancing their cytotoxicity toward tumor cells; in another aspect, the IL-4Rα-binding aptamer inhibits IL-4-driven M2 macrophage polarization, thereby strengthening immune attack on tumors. This synergy helps overcome tumor resistance to immune surveillance and enables effective treatment of refractory malignancies.

5. Synergy of the PD-1 aptamer and the OX40 aptamer. The PD-1-binding aptamer reactivates inhibited T cells, and the OX40-binding (agonistic) aptamer engages OX40 to further augment T-cell antitumor activity. Such synergy maintains T cells in an activated state with sustained proliferation and survival, thereby supporting continuous attacks on tumor cells.

6. Synergy of the IL-4Rα aptamer and the OX40 aptamer. The IL-4Rα-binding aptamer suppresses M2 macrophage formation and increases the antitumor capacity of immune cells, while the OX40-binding (agonistic) aptamer further strengthens T-cell antitumor responses. This synergy helps maintain immune-cell activity within the tumor microenvironment and sustain antitumor efficacy.

7. Overall cooperative effect of the triple-aptamer nucleic-acid nanotherapeutic. The combination agent produces an integrated synergistic effect by simultaneously targeting the key immunoregulatory molecules PD-1, IL-4Rα, and OX40, thereby achieving multi-layer activation of immune cells and generating a stronger antitumor effect. This multi-axis activation is conducive to overcoming resistance to single-agent immunotherapy and to improving therapeutic outcomes.

8. Broader applicability. This combination strategy is suitable not only for first-line therapy but also for second- or later-line treatment of recurrent or treatment-tolerant tumors, thereby providing additional therapeutic options for patients with difficult-to-treat cancers.

9. Lower adverse effects. Because the combination agent precisely targets tumor cells and reduces impact on normal cells, its adverse-event profile is lower than that of conventional single-target immunotherapy. This helps improve patient quality of life and lessen treatment-related discomfort.

10. Personalized therapy. Biological heterogeneity among tumor patients can render single-agent immunotherapy ineffective in certain subjects. By concurrently targeting multiple immunoregulatory molecules, the disclosed triple-aptamer nucleic-acid nanotherapeutic for active combination immunotherapy can increase response rates and deliver tangible clinical benefit to more patients.

11. Potential combination regimens. The combination agent may be used as a monotherapy or in combination with other modalities (e.g., chemotherapy, radiotherapy, targeted therapy) to achieve improved outcomes. Such combination strategies help overcome resistance to single-modality treatment and enhance efficacy.

12. Ongoing research and optimization. With deeper understanding of immunotherapy mechanisms, the design and application of the triple-aptamer nucleic-acid nanotherapeutic can be continually improved to realize more effective and safer treatment. For example, modifying aptamer structures and/or combinations can optimize targeting and bioactivity to accommodate different tumor types and disease stages.

13. Tumor-prevention strategy. In future applications, the triple-aptamer nucleic-acid nanotherapeutic may be employed for early tumor prevention-for example, by screening high-risk populations and administering immunotherapy to eligible subjects to prevent tumor onset and progression.

Accordingly, the disclosed targeted immunoregulatory triple-combination nucleic-acid nanotherapeutic possesses substantial clinical potential, is configured to efficiently inhibit the growth and progression of multiple recurrent, treatment-tolerant, and refractory tumors, and is expected to improve the current landscape of cancer therapy and patients' quality of life and survival prospects. However, achieving this goal will require extensive basic and clinical research to fully elucidate its mechanism of action, safety, and efficacy, and to enable continual refinement and optimization of the therapeutic strategy.

Drug 51): CD16a Aptamer-OX40 Aptamer-PD-L1 Aptamer-DNA Carrier

A targeted immunomodulatory tri-aptamer nucleic-acid nanotherapeutic is provided. The three aptamer modules are integrated on a DNA scaffold through a sequence-extension assembly, thereby forming a unitary therapeutic construct capable of effectively inhibiting the growth and progression of multiple tumor types. The mechanisms of action and synergistic effects are set forth below:

1. CD16a Aptamer. CD16a is a low-affinity Fc$\gamma$ receptor predominantly expressed on natural killer (NK) cells and macrophages. Binding of a CD16a-targeting aptamer activates NK cells and macrophages, eliciting antitumor activity. NK cells mediate direct cytotoxicity against tumor cells, while macrophages contribute via phagocytosis of tumor cells and secretion of antitumor cytokines.

2. OX40 Aptamer. OX40 is a costimulatory receptor expressed on activated T cells. An OX40-agonist aptamer engages OX40 to enhance T-cell activation, proliferation, and effector function, thereby augmenting the immune response. OX40 signaling is important for maintaining an effective antitumor immune response.

3. PD-L1 Aptamer. PD-L1 is an immunosuppressive ligand broadly expressed on tumor cells and immune cells. By sequestering PD-L1 and thereby blocking PD-1/PD-L1 signaling, a PD-L1-antagonist aptamer restores T-cell antitumor activity.

4. Targeting of Immune Effector Cells. Through concurrent targeting of NK cells, macrophages, and T cells, the tri-aptamer construct enhances immune-cell-mediated tumor attack. The CD16a aptamer activates NK cells and macrophages; the OX40 aptamer activates T cells; and the PD-L1 aptamer relieves tumor-induced suppression of immune cells.

5. Activation of Multiple Immune Effector Pathways. By acting on distinct immune-cell populations via the CD16a, OX40, and PD-L1 aptamers, multiple antitumor effector pathways are engaged. NK cells and macrophages provide direct tumor-cell killing, while T cells eliminate tumor cells through antigen-specific recognition. Such multi-pathway activation yields a more effective antitumor response.

6. Enhancement of Immune Activity within the Tumor Microenvironment (TME). Neutralization of PD-L1 restores T-cell effector function in the TME. In addition, OX40 costimulation promotes T-cell infiltration into the TME, increasing the magnitude of the antitumor immune response.

7. Prolongation of Immune-Cell Persistence. OX40-mediated activation supports extended survival/persistence of activated T cells, enabling sustained antitumor activity. Following blockade of PD-1/PD-L1 signaling by the PD-L1 aptamer, T-cell effector function is maintained, further extending their persistence.

8. Induction of Tumor-Specific Immunological Memory. Engagement of OX40 signaling promotes the generation of tumor-specific immunological memory, facilitating rapid recall responses upon tumor recurrence and thereby reducing the risk of relapse.

In summary, by acting on different immune-cell subsets, activating multiple antitumor effector pathways, enhancing immune activity in the TME, prolonging immune-cell persistence, and inducing tumor-specific immunological memory, this targeted, sequence-extended, tri-aptamer DNA-scaffold therapeutic exhibits synergistic mechanisms that enable effective inhibition of tumor growth and progression.

Drug 52): CD16a Aptamer-VEGF165 Aptamer-PD-L1 Aptamer-DNA Carrier

A targeted immunomodulatory tri-aptamer nucleic-acid nanotherapeutic is provided. The three aptamer modules are integrated on a DNA scaffold having sequence-extension architecture, thereby forming a unitary therapeutic construct configured to achieve potent tumor suppression. The functional roles of each aptamer and their cooperative mechanisms are set forth below:

1. CD16a Aptamer (FcγRIIIa, CD16a). CD16a is a low-affinity Fc receptor predominantly expressed on natural killer (NK) cells, monocytes, and macrophages. CD16a recognizes and binds the Fc portion of antibodies, triggering antibody-dependent cellular cytotoxicity (ADCC). In the present construct, a CD16a-binding aptamer engages CD16a to potentiate immune-effector attack on tumor cells, including ADCC-mediated cytotoxicity.

2. VEGF165 Aptamer. VEGF165 is a key pro-angiogenic factor essential for tumor growth and metastasis; by binding receptors on endothelial cells, it promotes neovascularization that supplies nutrients and oxygen to tumors. The VEGF165-targeting aptamer inhibits the activity of VEGF165, thereby blocking tumor angiogenesis and consequently attenuating tumor growth.

3. PD-L1 Aptamer. PD-L1 is an immunosuppressive ligand that, upon binding to PD-1 on T cells, dampens T-cell activity and suppresses immune responses; tumor cells frequently overexpress PD-L1 to evade immune surveillance. The PD-L1-targeting aptamer interrupts the PD-1/PD-L1 interaction, restoring T-cell function and enhancing immune-mediated tumor clearance.

Synergistic Effects:

1. Enhancement of Tumor-Cell Immunologic Recognition. A CD16a (FcγRIIIa)-binding aptamer enables immune effector cells to recognize and attack tumor cells more effectively, while a PD-L1-antagonist aptamer relieves immunosuppression and restores T-cell function. In combination, these two modalities markedly increase immune-mediated clearance of tumor cells.

2. Inhibition of Tumor Angiogenesis. A VEGF 165-targeting aptamer suppresses tumor neovascularization, thereby limiting tumor growth. Concurrent restoration of T-cell function by the PD-L1 aptamer further facilitates immune-cell attack on tumor vasculature, collectively weakening the tumor's growth capacity.

3. Amplification of Antitumor Immune Responses. Through coordinated action, the CD16a, VEGF165, and PD-L1 aptamers enhance immune-system attack on tumors. The CD16a aptamer promotes antibody-dependent cellular cytotoxicity (ADCC) and increases immune-cell cytotoxicity toward tumor cells; the PD-L1 aptamer abrogates T-cell suppression, enabling more effective T-cell-mediated killing; and the VEGF165 aptamer blocks angiogenesis required for tumor expansion. Together, these mechanisms synergize to achieve potent inhibition of tumor growth and progression.

In summary, the disclosed targeted immunomodulatory tri-aptamer nucleic-acid nanotherapeutic-comprising CD16a, VEGF165, and PD-L1 aptamers integrated on a sequence-extended DNA scaffold-achieves robust suppression of recurrent, resistant, and refractory tumors by restoring immune attack on tumor cells, inhibiting tumor angiogenesis, and reversing immunosuppression. This unitary, DNA-scaffolded tri-aptamer construct provides a new strategy for cancer immunotherapy and is effective to deliver improved therapeutic outcomes in subjects in need thereof.

Drug 53): CD16a Aptamer-CTLA-4 Aptamer-TIM-3 Aptamer-DNA Carrier

A targeted immunomodulatory tri-aptamer nucleic-acid nanotherapeutic is provided. The three aptamer modules are integrated on a DNA scaffold via sequence-extension assembly, thereby forming a unitary therapeutic construct configured to potently inhibit growth and progression of recurrent, resistant, and refractory tumors. The functional roles and cooperative mechanisms are set forth below:

1. CD16a Aptamer. CD16a is an activating receptor on natural killer (NK) cells that recognizes antibody-opsonized tumor cells through Fc engagement and mediates antibody-dependent cellular cytotoxicity (ADCC). A CD16a-binding aptamer engages CD16a on NK cells (and, in certain embodiments, on other FcγRIIIa-expressing effector cells), thereby potentiating ADCC and enabling direct cytolysis of tumor cells.

2. CTLA-4 Aptamer. CTLA-4 is an inhibitory receptor on T cells that binds B7 family ligands and functionally antagonizes CD28 costimulation, thereby suppressing T-cell activation. A CTLA-4-antagonist aptamer blocks the interaction of CTLA-4 with B7 ligands (e.g., B7-1/CD80 and B7-2/CD86), relieving inhibitory signaling, promoting T-cell activation and expansion, and enhancing T-cell-mediated antitumor activity.

3. TIM-3 Aptamer. TIM-3 is an inhibitory receptor expressed on immune cells, including T cells and NK cells; engagement by its ligand galectin-9 attenuates immune-cell activation. A TIM-3-antagonist aptamer disrupts TIM-3/galectin-9 binding, restores immune-cell function, and augments antitumor immune responses.

Synergistic effects:

1. Restoration and activation of immune-cell function: The CTLA-4 aptamer and the TIM-3 aptamer respectively block inhibitory receptors on T cells and NK cells, thereby restoring and activating these immune cells. As a result, the immune system exhibits increased cytotoxic capacity within the tumor microenvironment (TME).

2. Direct tumor-cell killing: Engagement of NK cells via the CD16a aptamer enables direct killing of antibody-opsonized (antibody-coated) tumor cells through antibody-dependent cellular cytotoxicity (ADCC), thereby effecting efficient tumor clearance.

3. Augmentation of immune activity in the TME: Use of the CTLA-4 and TIM-3 aptamers not only enhances immune-cell activity within the TME but, by relieving inhibitory signaling, also strengthens interactions between immune cells and tumor cells. This heightened immune activity helps remodel the TME and further suppress tumor growth.

4. Enhanced persistence of immune cells: By blocking CTLA-4 and TIM-3 inhibitory signaling, immune cells display increased persistence within the TME, permitting sustained antitumor pressure, which facilitates tumor eradication and reduces the risk of recurrence.

5. Reinforcement of immunological memory: Effective activation of T cells promotes the formation of immunological memory. Upon tumor re-emergence, the immune system can rapidly recognize and eliminate tumor cells, thereby preventing recurrence and metastasis.

In summary, the specific immunomodulatory tri-aptamer nucleic-acid nanotherapeutic agent, configured as a single integrated therapeutic molecule by sequence-extension assembly on a DNA carrier, can achieve efficient suppression of recurrent, resistant, and refractory tumors. Through synergistic action, it restores and activates immune cells, directly kills tumor cells, improves the tumor microenvironment, and enhances immune-cell persistence and immunological memory, thereby providing a new and effective strategy for tumor therapy.

Drug 54: PD-1 Aptamer-CTLA-4 Aptamer-LAG-3 Aptamer-DNA Carrier

The present disclosure provides a specific immunomodulatory triple-combination nucleic-acid nanotherapeutic agent; the mechanism of action thereof is set forth as follows:

1. PD-1 aptamer: Programmed cell death protein 1 (PD-1) is an immune checkpoint receptor predominantly expressed on immune cells. Tumor cells evade immune attack by expressing programmed death-ligand 1 (PD-L1), which engages PD-1 and suppresses immune-cell activity. The PD-1 aptamer is configured to block the interaction between PD-1 and PD-L1, thereby restoring immune-cell cytotoxic activity against tumor cells and inhibiting tumor growth.

2. CTLA-4 aptamer: Cytotoxic T-lymphocyte-associated antigen 4 (CTLA-4) is an inhibitory receptor on immune cells that, through binding to CD80/CD86, suppresses T-cell activation and proliferation. The CTLA-4 aptamer is configured to inhibit the interaction of CTLA-4 with CD80/CD86, thereby releasing T-cell inhibition and enhancing immune-cell attack on tumor cells.

3. LAG-3 aptamer: Lymphocyte-activation gene-3 (LAG-3) is a prominent target of next-generation immune checkpoint inhibition and suppresses T-cell immune activity primarily by down-modulating signal transduction in the major histocompatibility complex-T-cell receptor (MHC-TCR) pathway. The LAG-3 aptamer is configured to inhibit LAG-3 activity, thereby relieving and re-activating T-cell immunity and increasing killing of tumor cells.

In the foregoing tri-aptamer immunomodulatory nanotherapeutic, the combined action of the three aptamers blocks or attenuates tumor cell-mediated inhibition of immune cells, thereby enhancing immune-cell cytotoxicity toward tumor cells. The agent specifically alleviates or reduces tumor-associated immunosuppression, improving treatment precision and efficacy.

Drug 55) : CD16a Aptamer-Act-12c Aptamer-MUC1 (5TR1) Aptamer-DNA Carrier

The present disclosure relates to a specific immunomodulatory triple-combination nucleic-acid nanotherapeutic, in which the three aptamers are combined as a single, integrated therapeutic molecule on a DNAh carrier by sequence-extension assembly, the construct comprising CD16a, Act-12c, and MUC1 (5TR1) aptamers. The architecture is designed to activate immune effector cells and to achieve tumor-cell-specific targeting. The mechanism of action is described below from the standpoint of individual molecular functions and synergistic effects.

1. CD16a aptamer. CD16a is a low-affinity Fc receptor primarily expressed on natural killer (NK) cells and a subset of macrophages. Upon binding to CD 16a, the CD16a aptamer is configured to activate NK cells and macrophages, increasing their cytotoxic and phagocytic activities and thereby enhancing clearance of tumor cells.

2. Act-12c aptamer. The Act-12c aptamer targets and activates CD4$^+$ T cells. CD4$^+$ T cells play a pivotal role in antitumor immunity, including promoting the generation and activation of cytotoxic T lymphocytes (CTLs), activating B cells to produce antibodies, and secreting cytokines to modulate immune responses. By binding a specific receptor on CD4$^+$ T cells, the Act-12c aptamer activates CD4$^+$ T cells, thereby enhancing antitumor immune responses.

3. MUC1 (5TR1) aptamer. MUC1 is a glycoprotein highly expressed on the surface of multiple tumor cells; compared with normal cells, tumor-cell MUC1 displays aberrant glycosylation and serves as a tumor-associated antigen. The MUC1 (5TR1) aptamer binds MUC1 with high affinity, thereby directing the nanotherapeutic to tumor cells with specificity.

In the specific immunomodulatory triple-combination nucleic-acid nanotherapeutic described herein, synergistic cooperation among the three aptamers enhances interactions between immune cells and tumor cells. The CD16a aptamer is configured to activate NK cells and macrophages, and the Act-12c aptamer is configured to activate CD4$^+$ T cells; these immune populations act cooperatively to augment the antitumor immune response. Concurrently, the MUC1 (5TR1) aptamer directs the nucleic-acid nanotherapeutic specifically to tumor cells, thereby improving treatment precision and efficacy.

During targeting of tumor cells by the immunotherapeutic, activation of CD4$^+$ T cells promotes the generation and activation of cytotoxic T lymphocytes (CTLs), induces B-cell antibody production, and mediates cytokine secretion to modulate immune responses. Activation of NK cells and macrophages enhances clearance of tumor cells. Through such synergy, the tri-aptamer integrated nucleic-acid immunotherapeutic is effective to suppress the growth and progression of multiple recurrent, treatment-resistant, and refractory tumors.

In addition, the DNA carrier, as a biocompatible and biodegradable nucleic-acid carrier, ensures stable in-vivo delivery of the aptamers and increases the bioavailability of the therapeutic. During treatment, the DNA carrier can be gradually degraded by endogenous enzymes, thereby reducing potential toxicity and adverse effects.

In summary, the mechanism of action of this specific immunomodulatory triple-combination nucleic-acid nanotherapeutic includes synergistic activation of immune cells together with specific targeting of tumor cells, thereby achieving inhibition of tumor growth and progression. This distinctive design and synergistic effect confer substantial clinical application potential and may provide a new therapeutic option for recurrent, treatment-resistant, and refractory tumors.

Drug 56) : CD16a Aptamer-TGF-β Aptamer-PD-L1 Aptamer-DNA Carrier

Provided herein is a specific immunomodulatory triple-combination nucleic-acid nanotherapeutic, the agent being assembled as a single integrated therapeutic molecule on a DNA carrier by sequence-extension. The design is intended to suppress tumor growth and progression through synergistic action. The mechanism of action is described in detail below:

1. CD16a aptamer. CD16a is an immune receptor expressed on natural killer (NK) cells and macrophages and is associated with antitumor activity. By binding the CD16a receptor, the CD16a aptamer is configured to activate NK cells and macrophages, thereby inducing cytotoxic responses and effecting clearance of tumor cells.

2. TGF-β aptamer. Transforming growth factor-β (TGF-β) is an immunosuppressive cytokine typically upregulated in the tumor microenvironment. Elevated TGF-β suppresses immune-cell activity and promotes tumor immune evasion. By binding TGF-β, the TGF-β aptamer is configured to inhibit the biological activity of TGF-β, thereby relieving immunosuppression of immune cells and enhancing antitumor immune responses.

3. PD-L1 aptamer. Programmed death-ligand 1 (PD-L1) is an immune-checkpoint protein that interacts with the PD-1 receptor to inhibit T-cell activity, resulting in tumor immune evasion. By binding PD-L1, the PD-L1 aptamer is configured to block the interaction between PD-L1 and PD-1, thereby removing inhibition of T cells and augmenting tumor immune clearance.

4. DNA carrier. As a biocompatible and biodegradable nucleic-acid carrier, the DNA carrier ensures stable in-vivo delivery of the aptamers and increases the bioavailability of the therapeutic. The DNA nucleic-acid carrier can be gradually degraded by endogenous nucleases, thereby reducing potential toxicity and adverse effects.

Through the synergistic action of these four molecular components, the tri-aptamer integrated nucleic-acid immunotherapeutic agent is capable of efficiently inhibiting the growth and progression of multiple tumors. This unique design and synergy confer substantial potential for clinical application. The potential clinical effects include, without limitation, the following:

1. Enhanced cytotoxicity: NK cells and macrophages activated by the CD16a aptamer can more effectively recognize and eliminate tumor cells. Concurrently, the PD-L1 aptamer relieves inhibition of T cells, thereby further strengthening the immune system's attack on tumors.

2. Inhibition of tumor growth and metastasis: By blocking the biological activity of TGF-β, the TGF-β aptamer suppresses tumor-cell proliferation, invasion, and migration. This helps to limit tumor spread and metastasis and reduces the risk of tumor recurrence.

3. Improvement of the tumor microenvironment: The anti-TGF-β and PD-L1 aptamers improve the tumor microenvironment by lowering levels of immunosuppressive factors and enhancing immune-cell activity, thereby enabling more effective immune-mediated tumor clearance.

4. Potential for personalized therapy: Because the tri-aptamer integrated nucleic-acid immunotherapeutic acts in combination against multiple targets, it offers high flexibility. The aptamer composition can be adjusted and optimized according to a patient's specific condition to achieve improved individualized therapeutic outcomes.

5. Use in combination with other treatments: The tri-aptamer integrated nucleic-acid immunotherapeutic can be administered in combination with other treatment modalities (e.g., surgery, radiotherapy, chemotherapy) to realize improved therapeutic efficacy.

6. Reduced adverse effects: Owing to its high degree of tumor targeting, the therapeutic can reduce impact on normal tissues and thereby decrease potential adverse effects during treatment.

In summary, the specific immunomodulatory triple-combination nucleic-acid nanotherapeutic achieves effective inhibition of tumor growth and progression through synergistic action. Its distinctive design and synergistic effects provide new opportunities for clinical application and are expected to deliver improved outcomes for patients with various recurrent, treatment-resistant, and refractory tumors.

Drug 57) : CD16a Aptamer-CD40 Aptamer-CTLA-4 Aptamer-DNA Carrier

Provided herein is a specific immunomodulatory triple-combination nucleic-acid nanotherapeutic, in which a CD16a aptamer, a CD40/CD40L-pathway aptamer, and a CTLA-4 aptamer are assembled together on a DNA carrier to achieve synergistic activity and effectively inhibit tumor growth and progression. A detailed analysis of the functional roles of each molecular component and their cooperative effects is set forth below:

1. CD16a aptamer. CD16a (Fc$\gamma$RIIIa) is an immune receptor expressed on natural killer (NK) cells and on a subset of macrophages. Upon binding CD 16a, the CD16a aptamer is configured to activate NK cells and macrophages and enhance their cytotoxic functions, thereby facilitating tumor-cell clearance and inhibiting tumor growth.

2. CD40L aptamer. CD40 ligand (CD40L; CD154) is a costimulatory molecule predominantly present on activated T-cell surfaces. By binding CD40, the CD40L aptamer is configured to activate macrophages, dendritic cells, and B cells, thereby increasing the immune system's antitumor effector capacity.

3. CTLA-4 aptamer. Cytotoxic T-lymphocyte-associated antigen 4 (CTLA-4) is an inhibitory receptor on T cells. Engagement of CTLA-4 with B7-family ligands suppresses T-cell activity and weakens immune responses. The CTLA-4 aptamer is configured to competitively block the interaction between CTLA-4 and B7-family ligands, thereby restoring T-cell activity and enhancing immune responses.

Synergy analysis:

1. Immune-cell activation synergy. The CD16a aptamer and the CD40L aptamer act, respectively, on NK cells, macrophages, and other antigen-presenting cells (e.g., dendritic cells), jointly promoting immune-cell activation and proliferation, thereby increasing the immune system's antitumor attack.

2. Immunosuppression-release synergy. The CTLA-4 aptamer relieves inhibition of T cells, enabling them to regain antitumor effector function. In parallel, the CD40L aptamer further enhances T-cell activity, enabling stronger function by T cells within the tumor microenvironment during the immune response. The combined effect of these actions serves to fully activate T cells and enhance tumor-cell clearance.

3. Multi-cell-type synergy. The CD16a, CD40L, and CTLA-4 aptamers, respectively, target NK cells, macrophages, dendritic cells, and T cells, thereby comprehensively mobilizing the immune system and forming a robust antitumor immune response.

4. Immunoregulatory balance. By activating immune cells and releasing T-cell inhibition, the tri-aptamer integrated nucleic-acid immunotherapeutic mobilizes immune potency while helping maintain immune system homeostasis, which helps prevent immune-related adverse effects and improves treatment safety.

5. Targeting of the tumor microenvironment. Owing to high affinity for their respective receptors, the three aptamers enable the tri-aptamer integrated nucleic-acid immunotherapeutic to precisely target the tumor microenvironment. Within the tumor microenvironment, their synergistic actions can more effectively inhibit tumor growth and progression.

In summary, the specific immunomodulatory triple-combination nucleic-acid nanotherapeutic achieves effective inhibition of the growth and progression of multiple tumors through multi-pathway synergy. The synergy encompasses immune-cell activation, relief of immunosuppression, multi-cell-type cooperation, and maintenance of immunoregulatory balance, thereby providing a powerful therapeutic strategy for tumor immunotherapy.

Drug 58) : HER2 Aptamer-HER3 Aptamer-CD40 Aptamer-CD16a Aptamer-DNA Carrier

Provided herein is a specific immunomodulatory quadruple-combination nucleic-acid nanotherapeutic, assembled as a single integrated therapeutic molecule on a DNA carrier by sequence-extension. The agent is capable of efficiently inhibiting the growth and progression of multiple tumors. Its mechanism of action involves the following:

1. Dual blockade of oncogenic signaling pathways. The HER2 and HER3 aptamers respectively target two key tumor growth-factor receptors, HER2 and HER3. These receptors are overexpressed in many tumors and play pivotal roles in signal-transduction pathways that promote tumor-cell proliferation and invasion. Concurrent inhibition of HER2 and HER3 interrupts oncogenic signaling, thereby inhibiting tumor growth.

2. Activation of tumor-specific immune responses. The CD40 aptamer targets CD40 and activates immune cells such as dendritic cells (DCs) and macrophages. CD40 is a critical immunoregulatory molecule required for activation of immune cells and promotion of antitumor immune responses. Activation of CD40 enhances tumor-specific immunity.

3. Enhancement of natural killer (NK) cell antitumor activity. The CD16a aptamer engages the CD16a receptor on NK cells. NK cells can directly kill tumor cells. By engaging CD16a, NK-cell antitumor activity is enhanced, thereby further inhibiting tumor growth.

4. Synergistic effects. The foregoing molecular components act cooperatively-i.e., blocking tumor signaling pathways, activating tumor-specific immune responses, and enhancing NK-cell antitumor activity-thereby enabling more effective suppression of tumor growth and progression.

5. Targeting of the tumor microenvironment. Owing to their high-affinity binding to respective receptors, these aptamers enable the integrated nucleic-acid immunotherapeutic to precisely target the tumor microenvironment (TME). Within the TME, the aptamers permit precise regulation of tumor cells and immune cells, thereby improving therapeutic efficacy.

6. Reduced adverse effects: Because this combination immunotherapeutic can precisely target tumor cells and the tumor microenvironment (TME), it reduces impact on normal cells and thereby decreases adverse effects. This improves treatment safety and enhances patient tolerability.

7. Personalized therapy: The combination immunotherapeutic can be optimized according to a patient's tumor type and immune status. By adjusting the composition and concentrations of the aptamers, a personalized (individualized) treatment regimen can be provided, thereby improving therapeutic outcomes.

8. Overcoming tumor resistance: Because the combination immunotherapeutic simultaneously targets multiple signaling pathways and immunoregulatory checkpoints, it has potential to overcome tumor resistance. During therapy, tumor cells may develop resistance to a particular pathway or checkpoint; however, by addressing multiple targets concurrently, the therapeutic increases the likelihood of clinical success and reduces the risk of resistance.

In summary, the specific immunomodulatory quadruple-combination nucleic-acid nanotherapeutic achieves precise modulation of tumor cells and immune cells by acting on multiple signaling pathways and immunoregulatory checkpoints. Such synergistic action enables efficient inhibition of the growth and progression of diverse tumors and confers high therapeutic potential.

Drug 59) : CD16a Aptamer-Act-12c Aptamer-CTLA-4 Aptamer-GPC1 Aptamer-DNA Carrier

Provided herein is a specific immunomodulatory quadruple-combination nucleic-acid nanotherapeutic, comprising multiple functional aptamers assembled with a DNA carrier. The agent is effective for inhibiting tumors with high GPC1 expression (e.g., hepatocellular carcinoma and gastric cancer). A detailed analysis of the functions of the aptamers and their cooperative effects is set forth below:

1. CD16a aptamer. CD16a (FcγRIIIa) is an innate-immune receptor primarily present on natural killer (NK) cells and macrophages. The CD16a aptamer is configured to engage CD16a and, via an antibody-dependent cellular cytotoxicity (ADCC) mechanism, activate NK cells and macrophages, thereby effecting efficient attack and clearance of tumor cells.

2. Act-12c aptamer. The Act-12c aptamer is an activating aptamer configured to activate CD4$^+$ T cells. CD4$^+$ T cells play a key role in antitumor immunity and induce activation and proliferation of other immune cells (e.g., CD8$^+$ T cells and NK cells), thereby enhancing clearance of tumor cells.

3. CTLA-4 aptamer. Cytotoxic T-lymphocyte-associated antigen 4 (CTLA-4) is an immune-checkpoint receptor predominantly expressed on activated T cells. Inhibition of CTLA-4 signaling relieves T-cell suppression and enhances antitumor activity. The CTLA-4 aptamer is configured to block the binding of CTLA-4 to its ligands (e.g., B7-family ligands), thereby activating the antitumor function of T cells.

4. GPC1 aptamer. Glypican-1 (GPC1) is a tumor-associated antigen highly expressed on the surface of tumor cells such as those of liver and gastric cancers. The GPC1 aptamer specifically recognizes and binds GPC1, thereby achieving targeted action on tumor cells. Such targeting increases the intratumoral concentration and effect of the combination immunotherapeutic within the tumor microenvironment, thereby improving therapeutic efficacy.

5. Synergistic effects: The aptamers act synergistically within the combination immunotherapeutic. The CD16a and Act-12c aptamers are configured to activate, respectively, NK cells and macrophages, and CD4$^+$ T cells, thereby enhancing their cytotoxic activity against tumor cells. In parallel, the CTLA-4 aptamer blocks inhibitory immune signaling, thereby further increasing the antitumor activity of T cells. These immune cells cooperate within the tumor microenvironment (TME) to augment tumor-cell clearance. In addition, the GPC1 aptamer specifically targets GPC1-high-expressing tumor cells, thereby increasing the concentration and effect of the therapeutic agent in the TME.

In GPC1-high tumors such as hepatocellular carcinoma and gastric cancer, the combination immunotherapeutic exerts inhibitory effects through the following mechanisms:

1. Targeting specificity: The GPC1 aptamer confers high targeting specificity, increasing the intratumoral/TME concentration of the therapeutic agent and thereby improving antitumor efficacy.

2. Activation of immune cells: The CD16a and Act-12c aptamers are configured to activate NK cells, macrophages, and CD4$^+$ T cells, enabling these cells to exert stronger antitumor functions in the TME.

3. Release of immunosuppression: The CTLA-4 aptamer blocks immunosuppressive signaling and activates T cells, thereby enhancing their antitumor activity.

4. Cooperative action: The aptamers act in concert within the therapeutic agent to enhance clearance of tumor cells, thereby achieving effective suppression of GPC1-high tumors such as liver and gastric cancers.

In summary, this specific immunomodulatory quadruple-combination nucleic-acid nanotherapeutic achieves effective inhibition of GPC1-high tumors (e.g., hepatocellular and gastric cancers) through multi-pathway synergistic action. This therapeutic strategy provides a new direction for overcoming tumor drug resistance and improving therapeutic outcomes.

Drug 60) : CD16a Aptamer-HER3 Aptamer-VEGF 165 Aptamer-DNA Carrier

Provided herein is a specific immunomodulatory triple-combination nucleic-acid nanotherapeutic, comprising a CD16a aptamer, a HER3 aptamer, and a VEGF165 aptamer on a DNA carrier. The combination immunotherapeutic is designed to inhibit tumors with high expression of HER3 and VEGF165 via the synergistic mechanisms set forth below:

1. Targeting specificity. The HER3 aptamer has high specificity and is configured to recognize and bind tumor cells with high HER3 expression. This targeted interaction increases the concentration of the therapeutic within the tumor microenvironment (TME), thereby improving antitumor efficacy. Likewise, the VEGF165 aptamer is configured to specifically bind tumor cells that express VEGF165, thereby further enhancing targeting.

2. Inhibition of tumor-cell signaling. HER3 is a member of the epidermal growth factor receptor family and plays a key role in the growth and differentiation of many tumor cells. Upon binding to HER3, the HER3 aptamer inhibits HER3 signal transduction, thereby impeding tumor-cell growth and differentiation.

3. Inhibition of tumor angiogenesis. VEGF165 is a pro-angiogenic factor crucial for tumor growth, metastasis, and invasion. By binding VEGF165, the VEGF165 aptamer inhibits tumor angiogenesis and thereby blocks the blood supply to tumors, resulting in suppression of tumor growth.

4. Activation of immune cells. The CD16a aptamer is configured to activate immune cells such as NK cells and macrophages. These immune cells cooperate within the TME to enhance clearance of tumor cells, thereby inhibiting tumor growth.

5. Synergistic action. The HER3 and VEGF165 aptamers, when used in combination, exert cooperative effects by targeting/inhibiting tumor-cell signaling pathways and suppressing angiogenesis; in parallel, the CD16a aptamer activates immune effector cells and increases the capacity to clear tumor cells. These cooperative actions collectively produce an antitumor effect.

6. Overcoming tumor drug resistance. Certain tumors may develop drug resistance during treatment, thereby diminishing efficacy. By simultaneously addressing multiple targets, the combination immunotherapeutic reduces the likelihood of resistance and improves therapeutic success.

7. Improvement of quality of life: The combination immunotherapeutic can reduce tumor burden and alleviate patient symptoms (e.g., pain, fatigue), thereby improving patient quality of life.

8. Improved treatment safety: Owing to the high specificity of the aptamers, the combination immunotherapeutic can inhibit tumor growth while reducing toxicity and adverse effects on normal tissues, thereby enhancing treatment safety.

9. Personalized therapy: Based on the characteristics of a patient's tumor, aptamers directed to different targets may be selected for combination administration to implement a personalized treatment strategy, thereby improving therapeutic outcomes.

In summary, the specific immunomodulatory triple-combination nucleic-acid nanotherapeutic achieves effective inhibition of tumors with high expression of HER3 and VEGF 165 through multi-pathway synergistic action. This approach provides a new strategy and methodology for tumor immunotherapy and is expected to deliver improved clinical outcomes and quality of life for patients. Nevertheless, clinical trials are required in practical application to verify and ensure its safety and efficacy.

Drug 61) : VEGF Aptamer-GPC1 Aptamer-PD-L1 Aptamer-CD16a Aptamer-DNA Carrier

This disclosure relates to a targeted, immunomodulatory, four-component nucleic-acid nanotherapeutic. A DNA carrier (scaffold) operably links multiple aptamers into a unitary therapeutic construct. The combination immunotherapeutic agent exhibits inhibitory activity against tumors characterized by high expression of VEGF165, GPC1 and/or PD-L1. The mechanism of action is described below:

1. Targeting VEGF165. Vascular endothelial growth factor 165 (VEGF165) is a key pro-angiogenic factor that promotes endothelial-cell proliferation, migration and differentiation, thereby supporting tumor neovascularization. In the present combination agent, the VEGF aptamer binds VEGF165 with high specificity and blocks its interaction with its receptor(s), thereby inhibiting tumor angiogenesis and restraining tumor growth and metastasis.

2. Targeting GPC1. Glypican-1 (GPC1) is a glycoprotein widely expressed on the surface of various tumor cells, including but not limited to hepatocellular carcinoma and gastric cancer. The GPC1 aptamer binds GPC1 and suppresses GPC1-associated signaling, thereby slowing tumor growth. Because GPC1 is expressed at relatively low levels in normal tissues, such targeted action reduces undesired toxicity to normal tissues.

3. Targeting PD-L1. Programmed death-ligand 1 (PD-L1) is an immune-checkpoint protein expressed on tumor cells. Engagement of PD-L1 with PD-1 suppresses immune-cell activity and enables immune evasion. The PD-L1 aptamer binds PD-L1 with high specificity and prevents its interaction with PD-1, thereby restoring immune-cell activity and enhancing antitumor immune responses.

4. CD16a Aptamer. CD16a (FcγRIIIa) is a receptor on immune effector cells that, upon engagement, activates such cells, including natural killer (NK) cells. In this combination agent, the CD16a aptamer enhances immune-cell activation and augments immune-mediated tumor clearance.

5. Synergistic effects. By simultaneously targeting multiple pathways, the agent achieves synergistic antitumor effects, including:

a. Integrated anti-angiogenesis. Targeting VEGF165 inhibits tumor angiogenesis, thereby slowing tumor growth;

concomitant targeting of GPC1 further modulates tumor-associated signaling pathways, synergistically suppressing angiogenesis.

b. Enhanced immune clearance. The PD-L1 aptamer blocks PD-1/PD-L1 interactions to restore immune activity, while the CD16a aptamer activates NK and other immune effector cells; together these actions markedly potentiate antitumor immunity.

c. Reduced toxicity. Given the relatively low expression of GPC1 in normal tissues and the high specificity of the VEGF165 and PD-L1 aptamers, the combination agent exhibits decreased off-target toxicity to normal tissues, thereby improving safety.

d. Addressing tumor resistance. Because the disclosed targeted, immunomodulatory, four-aptamer nucleic-acid nanotherapeutic suppresses tumors via multiple targeting pathways, it mitigates resistance associated with monotherapy. The multipathway synergism decreases the likelihood that a tumor will develop drug resistance and increases the probability of achieving therapeutic success.

Summary. The disclosed targeted, immunomodulatory, four-aptamer nucleic-acid nanotherapeutic inhibits tumors through multipathway targeting, including anti-angiogenesis, enhancement of immune-mediated clearance, reduction of adverse effects/toxicity to normal tissues, and mitigation of tumor resistance. These functional modules act cooperatively to effect potent inhibition of tumors exhibiting high expression of VEGF165, GPC1, and/or PD-L1.

Drug 62) : PD-1 Aptamer-PD-L1 Aptamer-2*AmiR-21-Act-12c Aptamer-DNA Carrier

This disclosure provides a tumor-specific immuno-gene-regulatory quadruple-combination nucleic-acid nanotherapeutic, in which a DNA carrier assembles, by sequential extension, the constituent modules into a single integrated therapeutic molecule. The unique combination can effectively inhibit the growth and progression of multiple tumors. The mechanism of action is as follows:

1. PD-1 aptamer. Programmed cell death protein 1 (PD-1) is an immune-checkpoint receptor primarily expressed on immune cells. Tumor cells evade immune attack by expressing programmed cell death ligand 1 (PD-L1), which binds PD-1 and suppresses immune-cell activity. The PD-1 aptamer blocks the interaction between PD-1 and PD-L1, thereby restoring the ability of immune cells to attack tumor cells and inhibiting tumor growth.

2. PD-L1 aptamer. The PD-L1 aptamer binds PD-L1 on the surface of tumor cells and prevents its interaction with PD-1, thereby relieving immunosuppression and activating immune cells to attack tumor cells. This action helps enhance the efficacy of immunotherapy.

3. AmiR-21: miR-21 is a microRNA (miRNA) that is overexpressed in multiple tumor types and typically exerts pro-proliferative and anti-apoptotic effects in tumor cells. An anti-miR-21 agent (e.g., an antisense oligonucleotide) specifically hybridizes to miR-21 and prevents its association with its target gene transcripts, thereby reducing the functional activity of miR-21. Accordingly, anti-miR-21 inhibits the growth of tumor cells.

4. Act-12c Aptamer: Act-12c is an immunostimulatory agonist capable of eliciting an antitumor immune response. An aptamer that binds Act-12c ("Act-12c aptamer") specifically associates with Act-12c and, upon such binding, stimulates immune cells to produce large amounts of antitumor cytokines, thereby enhancing immune-cell-mediated cytotoxic activity against tumor cells.

In summary, the tumor-specific, immunomodulatory and gene-regulatory, quadruple-combination nucleic-acid nanotherapeutic described herein operates through synergy between the gene-regulatory agent AmiR-21 and multiple immunotherapy targets, including Act-12c, PD-1 and PD-L1, thereby activating immune cells, enhancing recognition of tumor cells, inducing the secretion of antitumor cytokines, and establishing immunological memory, which in turn efficiently inhibits the growth and progression of multiple tumor types. Such synergism provides an antitumor therapeutic strategy with broad applicability.

Drug 63) : HER3 Aptamer-EGFR siRNA-AS 1411 Aptamer-Survivin siRNA-HER2 Aptamer-DNAh Carrier

Provided herein is a tumor-specific, immunomodulatory and gene-regulatory triple-combination nucleic-acid nanotherapeutic that constitutes an innovative therapeutic strategy for the treatment of tumors overexpressing HER2 and/or HER3. Within the construct, the HER2 and HER3 aptamers confer dual functionality-tumor-cell targeting and inhibitory activity-and act cooperatively to achieve effective suppression of HER2/HER3-overexpressing tumors. The mechanisms of action are described in detail below:

1. HER2 aptamer. The HER2 aptamer specifically binds the HER2 receptor on the surface of HER2-overexpressing tumor cells, thereby enabling precise recognition and targeting of tumor cells.

2. HER3 aptamer. The HER3 aptamer binds to HER3 expressed on tumor-cell surfaces, further enhancing the construct's tumor-cell targeting capability.

3. EGFR (siRNA). An siRNA directed against epidermal growth factor receptor (EGFR) reduces EGFR expression via RNA interference (RNAi), thereby inhibiting EGFR-mediated signaling and slowing tumor growth.

4. AS1411 aptamer. AS1411 is a nucleic-acid aptamer that specifically binds nucleolin (NCL) displayed on the surface of tumor cells and is effective to induce apoptosis of tumor cells.

5. Survivin (siRNA). An siRNA targeting survivin reduces survivin expression via RNAi, thereby inhibiting tumor-cell proliferation and promoting apoptosis; survivin (BIRC5) is an anti-apoptotic protein that supports tumor growth.

6. DNAh carrier. The DNAh nanocarrier assembles the foregoing aptamers and siRNAs into a single, unitary therapeutic molecule by sequence extension, thereby improving the stability and delivery efficiency of the agent.

Synergistic effects:

a. Targeting activity. The HER2 and HER3 aptamers specifically recognize tumor cells with high HER2/HER3 expression, thereby increasing the targeting specificity of the therapeutic agent.

b. Pathway inhibition. EGFR (siRNA) and survivin (siRNA) reduce the expression levels of their respective proteins, thereby inhibiting signaling pathways associated with tumor growth and slowing tumor progression.

c. Apoptosis induction. AS1411 induces apoptosis of tumor cells by binding to nucleolin (also referred to as nucleolar protein A).

d. Cooperative apoptosis. Apoptosis induced via AS 1411-nucleolin engagement acts synergistically with the effect of survivin (siRNA), thereby further promoting tumor-cell apoptosis.

e. Combined targeting. The HER2 and HER3 aptamers act in combination to mount a stronger targeted attack against tumor cells that co-overexpress HER2/HER3.

f. Molecular cooperativity. Through assembly by sequence extension on a DNA carrier, the various aptamers (and siRNAs) operate within a single, unitary therapeutic construct to exert cooperative effects, thereby improving therapeutic efficacy.

In view of the foregoing molecular functions and synergistic interactions, the disclosed tumor-specific, immunomodulatory and gene-regulatory triple-combination nucleic-acid nanotherapeutic is configured to inhibit, at multiple levels, the growth and progression of HER2/HER3-overexpressing tumors. First, aptamer-mediated targeting enables the therapeutic agent to act precisely on tumor cells. Second, by suppressing the EGFR- and survivin-associated signaling pathways, the agent interferes with key processes required for tumor-cell growth. Finally, the combined action of AS 1411 and survivin-targeting siRNA induces apoptosis of tumor cells, thereby further restraining tumor growth. Accordingly, the agent exhibits strong antitumor activity and provides an effective therapeutic strategy for the treatment of tumors with high HER2/HER3 expression.

Drug 64) : Survivin siRNA-TFRA3 Aptamer-AS1411 Aptamer-3*AmiR-21-DNA Carrier

Disclosed is a quadruple-combination nucleic-acid nanotherapeutic for tumor-specific immunomodulation and gene regulation, which achieves synergistic antitumor activity by incorporating distinct molecular aptamers/oligonucleotides. Specifically, the integrated therapeutic includes the following components:

1. Survivin (siRNA): Survivin is an anti-apoptotic protein that promotes tumor growth. A small interfering RNA (siRNA) directed to Survivin reduces intracellular Survivin expression via RNA interference (RNAi), thereby inhibiting tumor-cell proliferation and inducing apoptosis.

2. AS1411: AS1411 is a guanine-rich nucleic-acid aptamer that targets the nucleolin (NCL) receptor on the surface of tumor cells. Nucleolin is highly expressed in many tumor cells and is closely associated with tumor growth and invasiveness. By binding to nucleolin, AS1411 suppresses nucleolin activity and thereby inhibits tumor-cell proliferation and invasion.

3. TFRA3 aptamer: Transferrin receptor (TfR)-targeting aptamers with different sequences are known in the art. TFRA3 is one such aptamer, having the sequence TfA3: SEQ ID No. 262: GCGTGGTCACACGC, which exhibits high specificity for binding to the transferrin receptor and can specifically block the binding of transferrin to the transferrin receptor.

4. AmiR-21 (anti-miR-21): miR-21 is a microRNA overexpressed in multiple tumor types that generally promotes cell growth and suppresses apoptosis. An anti-miR-21 oligonucleotide binds to miR-21 and prevents its interaction with target genes, thereby reducing miR-21 activity and inhibiting tumor-cell growth.

In summary, by combining multiple molecular aptamers/oligonucleotides, the therapeutic specifically targets tumor cells and enhances tumor suppression. On this basis, the structures and combinations of the molecular aptamers can be further optimized to improve therapeutic specificity and safety. The therapeutic may also be administered in combination with other treatment modalities (e.g., chemotherapy, radiotherapy, or targeted therapy) to achieve comprehensive treatment and to improve patient quality of life and prognosis.

Drug 65): AS1411 Aptamer - EGFR siRNA - VEGF165 Aptamer - PD-L1 Aptamer - RNA Carrier

This disclosure relates to a triple-combination nano-nucleic-acid therapeutic agent for specific immunity and gene regulation. The agent achieves synergistic anti-tumor therapy by combining different molecular aptamers. Specifically, the integrated therapeutic includes the following molecular aptamers on an RNA carrier:

1. AS1411: AS1411 is a guanine-rich nucleic-acid aptamer capable of targeting the nucleolin receptor on the surface of tumor cells. Nucleolin is highly expressed in many tumor cells and is closely associated with tumor growth and invasiveness. By binding to nucleolin, AS1411 inhibits its activity, thereby preventing proliferation and invasion of tumor cells.

2. EGFR siRNA: This is a small interfering RNA directed against epidermal growth factor receptor (EGFR). EGFR is overexpressed in many tumors. By specifically suppressing EGFR expression, EGFR siRNA interferes with tumor-cell proliferation and survival signaling pathways, thereby inhibiting tumor growth.

3. VEGF165 Aptamer: This aptamer binds with high affinity to vascular endothelial growth factor 165 (VEGF165), an angiogenic factor highly expressed in tumors. By binding to VEGF165, the aptamer prevents VEGF165 from engaging its receptor, thereby inhibiting tumor angiogenesis and weakening the tumor blood supply.

4. PD-L1 Aptamer: This aptamer targets programmed death-ligand 1 (PD-L1), which is highly expressed in the tumor microenvironment and is implicated in immune evasion. By blocking PD-L1, the aptamer prevents PD-L1 from binding to the PD-1 receptor, thereby restoring the immune system's ability to clear tumor cells.

The four molecular aptamers act cooperatively to achieve multi-pronged tumor inhibition:

1. Block tumor-cell proliferation and invasion: AS1411 and EGFR siRNA inhibit the nucleolin and EGFR signaling pathways, directly disrupting tumor-cell growth and invasiveness.

2. Inhibit tumor angiogenesis: The VEGF165 aptamer blocks the VEGF165 signaling pathway, reducing the tumor's blood supply and restricting tumor growth.

3. Activate immune-mediated clearance: The PD-L1 aptamer blocks the PD-L1/PD-1 interaction, reactivating immune attack on tumor cells and thereby achieving immunotherapy of the tumor.

In summary, this triple-combination nano-nucleic-acid therapeutic agent combines multiple molecular aptamers to implement multi-pathway, multi-target synergistic therapy directed at tumor cells and their microenvironment. Such synergy enhances therapeutic efficacy and improves tumor suppression, particularly for tumors with high expression of nucleolin, VEGF165, and PD-L1. Future studies may further investigate the application potential of this triple-combination therapeutic across different tumor types, optimize the structures and combinations of molecular aptamers to improve specificity and safety, and explore combination regimens with other therapies (e.g., chemotherapy, radiotherapy, and targeted therapy) to achieve comprehensive treatment and improve patient quality of life and prognosis.

Drug 66): CEA Aptamer - CD16a Aptamer - Act-12c Aptamer - PD-L1 Aptamer - DNA Carrier

This disclosure provides a quadruple-combination nano-nucleic-acid therapeutic agent for specific immunity. Its mechanisms of action are as follows:

1. CEA: Carcinoembryonic antigen (CEA) is a glycoprotein widely used as a tumor marker and is notably overexpressed in colorectal cancer. Elevated expression also occurs in other cancers, such as lung and breast cancer. CEA belongs to the cell-adhesion molecule family, participates in intercellular interactions and signal transduction, and may contribute to cancer-cell survival and metastasis in the tumor microenvironment. The CEA aptamer is an oligonucleotide with high affinity and specificity that recognizes and binds the CEA protein. Because aptamer binding to a target protein can affect its function, the CEA aptamer, by binding to CEA, may exert an inhibitory effect on CEA function and thereby block its role in tumor growth and metastasis.

2. CD16a Aptamer: CD16a is a low-affinity Fc receptor typically expressed on natural killer (NK) cells and macrophages. By recognizing antigen-antibody complexes on tumor cells, CD16a mediates anti-tumor activity. A CD16a aptamer can enhance NK-cell and macrophage cytotoxicity against tumor cells.

3. Act-12c Aptamer: The Act-12c aptamer targets a specific molecule present in the immunosuppressive tumor microenvironment. Through binding of the Act-12c aptamer, the therapeutic's modulation of the tumor immune microenvironment is enhanced, thereby augmenting anti-tumor immune responses.

4. PD-L1 Aptamer: Programmed death-ligand 1 (PD-L1) is an immune checkpoint molecule overexpressed in many tumors. Upon binding the PD-1 receptor, PD-L1 suppresses immune-cell activity and enables tumor immune escape. A PD-L1 aptamer blocks the PD-L1/PD-1 interaction, thereby enhancing immune-cell attack against tumor cells.

This quadruple-combination nano-nucleic-acid therapeutic agent engages multiple mechanisms to target the tumor microenvironment, exert inhibitory and activating effects, and specifically activate a patient's own immune response, thereby suppressing and clearing tumor cells. The therapeutic features strong targetability, multi-modal action, synergistic effects, and personalization potential, and represents a promising anti-tumor agent.

Drug 67): AS1411 Aptamer - TAP siRNA - CD16a Aptamer - CTLA-4 Aptamer - DNA Carrier

This disclosure concerns a triple-combination nano-nucleic-acid therapeutic agent for specific immunity and gene regulation, whose mechanisms involve multiple aspects, as detailed below.

First, the therapeutic achieves tumor-microenvironment targeting mediated by AS 1411. AS1411 is a molecule with DNA-binding capability that can specifically bind to nucleic acids on the surface of tumor cells and thereby mediate targeted delivery to the tumor microenvironment. In addition, the three aptamers included in the therapeutic are able to target specific receptors, such as CD16a, further improving the agent's directional delivery to the tumor microenvironment and achieving better therapeutic effect.

Second, the therapeutic is capable of exerting both inhibitory and activating activities. CD16a is a receptor expressed on natural killer cells that mediates cytotoxic activation and also participates in regulation of immune responses. The TAP siRNA in the therapeutic is designed to knock down gene expression of the TAP transporter mRNA and to transiently increase tumor-cell antigen expression, thereby reducing immune-escape capability of tumor cells and enhancing antigen recognition and sensitization of immune cells. Meanwhile, CTLA-4-DNAh3.2 is capable of binding to the CTLA-4 protein, thereby inhibiting negative regulatory signaling in T cells, activating T-cell function, and increasing the immune system's recognition and sensitivity toward tumors.

Finally, the therapeutic specifically activates the patient's own immune response and suppresses and clears tumor cells. The TAP siRNA modulates antigen expression by tumor cells, thereby improving immune recognition and sensitivity to tumor antigens. Concurrently, CD16a mediates NK-cell recognition and clearance of tumor cells, while CTLA-4-DNAh3.2 inhibits tumor-cell growth and proliferation, ultimately achieving suppression and clearance of tumor cells.

In summary, this novel triple-combination nano-nucleic-acid therapeutic agent for specific immunity and gene regulation operates through multiple mechanisms and exhibits the following characteristics:

1. Strong targetability: AS1411 has DNA-binding capability and can specifically bind nucleic acids on the surface of tumor cells to realize targeted delivery to the tumor microenvironment; at the same time, the three aptamers can target specific receptors (e.g., CD16a), further improving directional delivery to the tumor microenvironment and thereby achieving better therapeutic effect.

2. Multiple modes of action: The therapeutic not only enables targeted delivery of anti-tumor agents but also exerts inhibitory and activating activities, and specifically activates a patient's own immune response, thereby suppressing and clearing tumor cells.

3. Synergistic effects: The multiple components within the therapeutic act synergistically and mutually reinforce one another to improve therapeutic efficacy.

4. Personalized therapy: By targeting regulation of tumor-cell gene expression, the therapeutic enables personalized treatment and thereby achieves improved outcomes.

In summary, the novel triple-combination nano-nucleic-acid therapeutic agent for specific immunity and gene regulation exhibits multiple mechanisms of action, is capable of targeting the tumor microenvironment, exerts both inhibitory and stimulatory activities, and specifically activates the patient's endogenous immune response, thereby inhibiting and clearing tumor cells. The therapeutic is characterized by high target specificity, multi-modal action, synergistic effects, and suitability for personalized therapy, and is therefore a promising anti-tumor therapeutic agent.

Drug 68): AS1411 Aptamer - TAP siRNA - CD16a Aptamer - Act-12c Aptamer - DNA Carrier

This disclosure provides a novel triple-combination nano-nucleic-acid therapeutic agent for specific immunity and gene regulation.

First, the therapeutic achieves AS1411-mediated targeting of the tumor microenvironment. AS1411 is a molecule having DNA-binding capability that can specifically bind nucleic acids on the surface of tumor cells and mediate targeted delivery to the tumor microenvironment. The three aptamers in the therapeutic can target specific receptors, such as CD16a and Act-12c, thereby further improving directional delivery to the tumor microenvironment and achieving better therapeutic effect.

Second, the therapeutic is capable of exerting both inhibitory and activating activities. The TAP siRNA can specifically suppress expression of target genes, thereby reducing the immune-escape capability of tumor cells and enhancing their susceptibility. At the same time, Act-12c in the therapeutic can bind to CD4+ T cells, thereby activating their immune function and enhancing immune recognition and cytotoxicity against tumor cells.

Finally, the therapeutic can specifically activate the patient's endogenous immune response and suppress and clear tumor cells. The TAP siRNA can regulate antigens expressed by tumor cells, thereby enhancing the immune system's recognition and sensitivity to tumor antigens. Concurrently, CD16a can mediate natural killer (NK) cell recognition and clearance of tumor cells, whereas Act-12c can activate CD4+ T-cell function to strengthen the immune attack against tumors, ultimately achieving suppression and clearance of tumor cells.

Within the therapeutic, different components exhibit synergistic effects that mutually reinforce one another and improve efficacy. Specifically, the following synergies are present:

1. Targeting synergy: AS1411 together with the three aptamers jointly targets receptors on the surface of tumor cells, thereby enhancing directional delivery to the tumor microenvironment. Meanwhile, the three aptamers and Act-12c can jointly target CD4+ T cells and NK cells to intensify the immune attack.

2. Inhibitory-effect synergy: The TAP siRNA and a CTLA-4-DNA carrier both exert inhibitory effects by specifically suppressing gene expression in tumor cells, reducing immune escape. In addition, the CTLA-4-DNA carrier can suppress negative regulatory signaling in T cells to enhance overall immune activity.

3. Activating-effect synergy: Act-12c and CD16a both confer activating effects, enabling activation of CD4+ T cells and NK cells and enhancing their cytotoxic activity against tumor cells.

4. Personalized-therapy synergy: The TAP siRNA exhibits specificity and can selectively regulate gene expression in tumor cells to realize personalized treatment. Act-12c also exhibits specificity, selectively activating CD4+ T-cell immune function while avoiding over-activation and immune suppression.

In summary, the different components of this triple-combination nano-nucleic-acid therapeutic agent for specific immunity and gene regulation act synergistically to mutually enhance and improve therapeutic efficacy. Such synergy is reflected in targeting, inhibitory, and activating effects as well as in personalized treatment, thereby affording improved therapeutic outcomes and higher safety. Accordingly, the therapeutic is a promising anti-tumor agent with broad application prospects.

Drug 69): CD16a Aptamer - AS1411 Aptamer - TAP siRNA - OX40 Aptamer - Act-12c Aptamer - DNA Carrier

This disclosure relates to a novel quadruple-combination nano-nucleic-acid therapeutic agent for specific immunity and gene regulation. Through the combined action of multiple components, the agent targets the tumor microenvironment, exerts inhibitory and activating activities, enhances tumor-cell susceptibility, specifically activates the patient's endogenous immune response, and suppresses and clears tumor cells. The mechanisms of action are analyzed as follows:

1. AS1411 targeting: AS1411 is a molecule with DNA-binding capability that can specifically bind nucleic acids on the surface of tumor cells and mediate targeted delivery to the tumor microenvironment.

2. Activation by CD16a and Act-12c: Both CD16a and an Act-12c-DNA carrier confer activating effects, enabling activation of NK-cell and CD4+ T-cell immune functions and enhancing their cytotoxicity against tumor cells. The Act-12c-DNA carrier can also suppress negative regulatory signaling in T cells to enhance immune activity.

3. OX40 co-stimulation: OX40 is an activating co-stimulatory molecule that can activate CD4+ T-cell immune function and augment cytotoxicity against tumor cells.

4. Regulatory effect of TAP siRNA: TAP siRNA can specifically suppress gene expression in tumor cells, reducing immune escape and enhancing tumor-cell susceptibility. TAP siRNA can also modulate antigen expression by tumor cells to improve immune recognition and sensitivity to tumor antigens.

5. Act-12c aptamer activation: The Act-12c aptamer is an activating molecule that binds specific receptors on CD4+ T cells and activates their immune function, thereby enhancing cytotoxicity toward tumor cells. By engaging activating receptors such as CD28, ICOS, and OX40 on CD4+ T cells, the Act-12c aptamer activates T-cell immune function. Unlike CTLA-4, these receptors activate, rather than inhibit, T-cell immunity and thereby enhance anti-tumor cytotoxicity.

6. Synergy among AS 1411/TAP siRNA/CD16a/OX40/Act-12c-DNAh3.2: Different components act synergistically to mutually enhance therapeutic effect. For example, AS1411 and CD16a can jointly target receptors on the surface of tumor cells to strengthen directional delivery to the tumor microenvironment; CD16a and Act-12c-DNAh3.2 can jointly activate NK cells and CD4+ T cells to enhance their cytotoxicity; and TAP siRNA together with OX40 can cooperatively regulate tumor-cell gene expression and antigen expression to increase tumor-cell susceptibility and immune recognition.

In summary, the different components of this quadruple-combination nano-nucleic-acid therapeutic agent for specific immunity and gene regulation act synergistically to mutually enhance and improve therapeutic efficacy. Such synergy is reflected in targeting, inhibitory, and activating effects as well as in regulatory effects and personalized treatment, thereby affording improved therapeutic outcomes and higher safety. Accordingly, this quadruple-combination therapeutic is a promising anti-tumor agent with broad application prospects.

Drug 70): CTLA-4 Aptamer - CD40 Aptamer - FAP Aptamer - DNA Carrier

This disclosure provides a specific-immunity triple-combination nano-nucleic-acid therapeutic agent. The agent increases cytotoxic activity against tumor cells through the synergistic action of three aptamer molecules. The mechanisms of action are as follows:

1. CTLA-4 Aptamer: Cytotoxic T-lymphocyte-associated antigen-4 (CTLA-4) is an inhibitory receptor expressed on immune cells. By interacting with CD80/CD86, CTLA-4 suppresses T-cell activation and proliferation. Accordingly, a CTLA-4 aptamer can block the interaction between CTLA-4 and its ligands, thereby releasing T-cell inhibition and enhancing the ability of immune cells to attack tumor cells.

2. CD40 Aptamer: The CD40 aptamer targets CD40 and activates immune cells such as dendritic cells (DCs) and macrophages. CD40 is a key immunoregulatory molecule that plays a critical role in activating immune cells and promoting anti-tumor immune responses. Activation of CD40 can thus enhance tumor-specific immune responses.

3. FAP Aptamer: The tumor stroma-comprising cancer-associated fibroblasts (CAFs) and extracellular matrix (ECM)-is increasingly recognized as an important participant in tumorigenesis, therapeutic resistance, angiogenesis, invasion, and metastasis. Fibroblast activation protein (FAP) serves as a CAF marker, participates in ECM regulation, and exhibits multiple pro-tumor activities. FAP is a serine oligopeptidase, expressed mainly during development and rarely in healthy adult tissues. However, at sites of pathological tissue remodeling (including cancer, fibrosis, arthritis, wounds, or inflammation), especially on fibroblasts, FAP is highly upregulated. Therefore, a FAP aptamer can directly inhibit FAP expression and thereby suppress tumor progression.

In summary, this integrated specific immunomodulatory triple-combination nano-nucleic-acid therapeutic agent utilizes the synergistic action of three aptamer molecules to efficiently inhibit the growth and progression of various tumors, thereby fully harnessing the anti-tumor capacity of immune cells and providing a novel and effective therapeutic approach for cancer patients.

Drug 71): VEGF165 Aptamer - ASAP1 siRNA - CD24A-2 Aptamer - SARS-CoV-2-N48 Aptamer - DNA Carrier

This disclosure relates to a specific-immunity and gene-regulation quadruple-combination nano-nucleic-acid therapeutic agent, which is specifically directed to treatment of SARS-CoV-2 infection. The therapeutic employs multiple aptamers and siRNA molecules to achieve targeted suppression of the transcription/replication milieu of SARS-CoV-2, thereby treating coronavirus infection. The mechanisms of action are analyzed as follows:

1. Targeting by SARS-CoV-2-N48 Aptamer: The SARS-CoV-2-N48 aptamer is a molecule exhibiting high affinity for host cells of SARS-CoV-2 and can specifically bind receptors on the surface of host cells for SARS-CoV-2, thereby realizing targeted delivery against the virus.

2. Targeting and Inhibitory Effects of VEGF165 Aptamer: The VEGF165 aptamer is a targeting molecule capable of specifically binding receptors on the surface of tumor cells. In addition, the VEGF165 aptamer can inhibit expression of VEGF165 within the viral transcription/replication environment, reducing viral susceptibility and infectivity.

3. Regulatory Effect of ASAP1 siRNA: ASAP1 siRNA is a regulatory molecule configured to target and suppress gene expression of guanylate kinase 1 (GK1) ASAP1 in host cells, so as to shut down this enzyme-guanylate kinase 1 (GK1)-thereby halting replication of SARS-CoV-2 and suppressing viral susceptibility and infectivity.

4. Activating effect of CD24: CD24 is an activating co-stimulatory molecule configured to activate the antiviral immune functions of the immune system and to enhance macrophage phagocytic clearance of cells infected with SARS-CoV-2.

5. Synergistic effects: Synergy is present among the different components, which mutually reinforce one another to improve therapeutic efficacy. For example, the SARS-CoV-2-N48 aptamer can target receptors on the surface of host cells for SARS-CoV-2 to realize targeted delivery; the VEGF165 aptamer and ASAP1 siRNA can jointly target and suppress expression of VEGF165 and ASAP1 within the viral transcription/replication milieu, thereby reducing the susceptibility and infectivity of SARS-CoV-2; CD24 can activate antiviral immune functions of the immune system and enhance resistance to SARS-CoV-2; and the DNA carrier can assist the foregoing molecules in achieving targeted delivery and enhance their biological stability and pharmacological activity.

In summary, synergy among the different components of this specific-immunity and gene-regulation quadruple-combination nano-nucleic-acid therapeutic agent enables mutual enhancement and improved therapeutic outcomes. Such synergy is reflected in targeting, inhibitory, and activating effects, as well as in regulatory effects and personalized treatment, thereby offering improved therapeutic efficacy and higher safety.

It should be noted that SARS-CoV-2 exhibits strong transmissibility and mutational capacity; treatment of infection is therefore highly complex. Although the present specific-immunity and gene-regulation quadruple-combination nano-nucleic-acid therapeutic agent may afford therapeutic benefit, its efficacy requires verification and confirmation in larger-scale experiments and clinical trials. In addition, strict adherence to instructions for use and associated precautions is required so as to avoid unnecessary risks and adverse reactions.

Drug 72): 3* FGF5 Aptamer - DNA Carrier

This disclosure provides a targeted aptamer therapeutic agent. Fibroblast growth factor 5 (FGF5) is a key molecule associated with alopecia; inhibition of FGF5 can effectively promote hair regrowth. The drug employs three FGF5 aptamer segments modified via sequence extension, and is configured to specifically bind to and inhibit the FGF5 protein. FGF5 is a fibroblast growth factor closely related to hair growth and shedding. Accordingly, inhibition of FGF5 can significantly suppress alopecia and even promote hair regrowth. The mechanisms for inhibiting hair loss or promoting hair regrowth are analyzed below.

1. Targeting and inhibitory actions of the FGF5 aptamer: The FGF5 aptamer is a targeting molecule capable of specifically binding to the FGF5 protein and inhibiting its activity. In hair-follicle cells, FGF5 suppresses hair growth and promotes hair shedding. Therefore, the targeting and inhibitory actions of the FGF5 aptamer can significantly suppress alopecia and may promote hair regrowth.

2. Assisting function of the DNA carrier: The DNA structural carrier assists the FGF5 aptamer in achieving targeted delivery and enhances its biological stability and pharmacological activity. In the present therapeutic, the carrier facilitates targeted delivery of the FGF5 aptamer and augments its inhibitory effect.

3. Inhibition of the negative impact of FGF5 on hair growth: High expression of FGF5 in hair-follicle cells inhibits hair growth and promotes hair shedding. Therefore, suppressing FGF5 expression can reduce its negative impact on hair growth and thereby promote hair regrowth.

4. Possible mechanisms for promoting hair regrowth: In addition to suppressing FGF5 expression, the FGF5 aptamer may promote hair regrowth through other mechanisms. For example, binding of the FGF5 aptamer may alter cellular signaling pathways and thereby promote hair growth and regeneration.

In summary, this targeted aptamer therapeutic agent specifically binds to and inhibits FGF5, thereby suppressing alopecia and potentially promoting hair regrowth. The therapeutic exhibits favorable target specificity and safety and has potential application value. It should be noted that hair growth and shedding constitute a complex physiological process influenced by multiple factors; therefore, the therapeutic effect requires verification and confirmation in larger-scale experiments and clinical trials. In addition, strict adherence to instructions for use and precautions is required to avoid unnecessary risks and adverse reactions.

Drug 73): AS1411 Aptamer - ASAP1 siRNA - VEGF Aptamer - SARS-CoV-2-N48 Aptamer - RNA Carrier

This disclosure relates to a nano-nucleic-acid therapeutic agent directed against SARS-CoV-2. The therapeutic employs ASAP1 siRNA to target and knock down expression of the ASAP1 gene in host cells, thereby inhibiting the activity of guanylate kinase 1 (GK1). Meanwhile, the VEGF and SARS-CoV-2-N48 aptamers can target receptors on the surface of host cells for SARS-CoV-2 to realize targeted, nano-nucleic-acid-mediated blockade, thereby preventing replication and infection by SARS-CoV-2. The mechanisms of action are analyzed as follows.

1. Targeting by the AS1411 aptamer: The AS1411 aptamer is a targeting molecule capable of specifically binding receptors on the surface of tumor cells. In the present therapeutic, the AS1411 aptamer can, by specifically binding to ASAP1 mRNA on the surface of host cells, realize targeted delivery of ASAP1 siRNA and knockdown of ASAP1 gene expression.

2. Inhibitory effect of ASAP1 siRNA: ASAP1 siRNA is an inhibitory molecule configured to specifically suppress expression of the ASAP1 gene in host cells. ASAP1 is a key enzyme that regulates the activity of guanylate kinase 1 (GK1). Knockdown by ASAP1 siRNA can inhibit GK1 activity, thereby preventing replication and infection by SARS-CoV-2.

3. Targeting and inhibitory effects of the VEGF165 aptamer: The VEGF165 aptamer is a targeting molecule capable of specifically binding receptors on the surface of tumor cells. In addition, the VEGF165 aptamer can inhibit expression of VEGF165 within the viral transcription/replication milieu, reducing viral susceptibility and infectivity.

4. Targeting by the SARS-CoV-2-N48 aptamer: The SARS-CoV-2-N48 aptamer is a molecule exhibiting high affinity for host cells of SARS-CoV-2 and can specifically bind receptors on the surface of such host cells, thereby realizing targeted delivery against SARS-CoV-2.

5. Assisting function of the RNA carrier: The RNA carrier assists the foregoing molecules in achieving targeted delivery and enhances their biological stability and pharmacological activity. In the present therapeutic, the RNA carrier assists AS1411, ASAP1 siRNA, VEGF165, and SARS-CoV-2-N48 aptamers in targeted delivery and blockade, thereby improving therapeutic effect.

6. Synergistic effects: Synergy is present among the different components, which mutually reinforce one another to improve therapeutic efficacy. For example, ASAP1 siRNA can specifically suppress ASAP1 gene expression and thereby inhibit GK1 activity; the VEGF 165 aptamer can specifically suppress VEGF165 expression within the viral transcription/replication milieu to reduce SARS-CoV-2 susceptibility and infectivity; the SARS-CoV-2-N48 aptamer can target receptors on the surface of host cells of SARS-CoV-2 to realize targeted delivery; and the RNA carrier can assist the foregoing molecules in achieving targeted delivery and enhance their biological stability and pharmacological activity.

In summary, through the synergistic action of multiple targeting molecules, this therapeutic intervenes in multiple stages pertinent to SARS-CoV-2 and thereby prevents viral replication and infection. The therapeutic exhibits favorable specificity, targetability, and safety, and has high clinical application value.

It should be noted that SARS-CoV-2 exhibits strong transmissibility and mutational capacity; treatment of SARS-CoV-2 infection is therefore highly complex. Although the present therapeutic may provide certain therapeutic benefits, its efficacy requires verification and confirmation in larger-scale experiments and clinical trials. In addition, strict adherence to instructions for use and precautions is required to avoid unnecessary risks and adverse reactions.

Drug 74): CpG2006 - CD40 Aptamer - PD-1 Aptamer - DNA Carrier

This disclosure relates to a specific active-immunity triple-combination nano-nucleic-acid therapeutic agent. The mechanism of action can be analyzed as follows:

1. CpG2006: CpG2006 is a CpG oligonucleotide analog that activates the Toll-like receptor 9 (TLR9) signaling pathway upon binding, thereby stimulating antiviral and anti-tumor immune responses. TLR9 is predominantly expressed on plasmacytoid dendritic cells (pDCs) and B lymphocytes. Activation of TLR9 induces immune cells to release large amounts of interferon-$\alpha$ (IFN-$\alpha$) and other cytokines, thereby enhancing immune surveillance and clearance of tumor cells.

2. CD40 (aptamer): The CD40 aptamer targets CD40 to modulate immune responses, including promoting maturation of dendritic cells (DCs) and enhancing antigen-presenting capacity. In this therapeutic, CD40 acts in concert with CpG2006 to further augment anti-tumor immune responses.

3. PD-1: Programmed death-1 (PD-1) is an inhibitory receptor primarily expressed on activated T cells. Tumor cells evade immune clearance by expressing PD-L1, which binds PD-1 and suppresses T-cell activation and proliferation. In the present disclosure, incorporation of a PD-1 antibody blocks the PD-1/PD-L1 interaction, releasing T cells from inhibition and restoring cytotoxic activity against tumor cells.

4. DNA nano-nucleic-acid delivery system: The delivery system functions as an efficient carrier configured to improve the in vivo stability and bioavailability of CpG2006, CD40L, and the PD-1 antibody. The DNAh3.3 nano-nucleic acid exhibits favorable biocompatibility and low toxicity, and can effectively reduce non-specific distribution and metabolism of immunotherapeutics in vivo to improve therapeutic effect. By integrating CpG2006, CD40L, and the PD-1 antibody, the nano-nucleic-acid delivery system unifies the three immunotherapeutics to achieve synergy within the tumor microenvironment.

In summary, the mechanism of this specific active-immunity triple-combination nano-nucleic-acid therapeutic agent can be summarized as follows:

1. CpG2006 activates TLR9 and induces production of antiviral and anti-tumor cytokines, thereby enhancing immune surveillance and clearance of tumor cells;

2. CD40L promotes DC maturation and antigen presentation, enhancing activation and proliferation of CD8$^+$ T cells and increasing tumor-specific cytotoxicity;

3. the PD-1 antibody blocks the PD-1/PD-L1 interaction, releasing T-cell inhibition and restoring cytotoxicity against tumor cells;

4. the DNA nano-nucleic-acid delivery system integrates CpG2006, CD40L, and the PD-1 antibody to realize synergistic action of the immunotherapeutics within the tumor microenvironment and improve therapeutic efficacy.

Through multi-pathway, multi-target synergy, this specific active-immunity triple-combination nano-nucleic-acid therapeutic agent has achieved significant efficacy across multiple tumor models in mice. In the future, such immunotherapeutics are expected to provide more effective and safer novel immunotherapies for clinical oncology; nevertheless, these findings require further validation in large-sample, multi-center clinical trials to ensure efficacy and safety in humans.

Drug 75): CD24 Aptamer - CD40 Aptamer - PD-L1 Aptamer - DNA Carrier

This disclosure provides a specific-immunity triple-combination nano-nucleic-acid therapeutic agent having potential application in the treatment of small-cell lung cancer (SCLC). The functions of each effector molecule and the synergistic mechanism after integration are analyzed below:

1. CD24: CD24 is a cell-surface glycoprotein closely associated with tumor invasiveness, metastasis, and immune evasion. CD24 expression is markedly elevated in SCLC and significantly impacts its onset, progression, and prognosis. In this therapeutic, a CD24-targeting aptamer recognizes and specifically binds CD24 on SCLC cells, thereby inhibiting invasiveness and metastatic potential while increasing tumor sensitivity to immunotherapy.

2. CpG2006: CpG2006 is a CpG oligonucleotide analog that activates the TLR9 pathway, stimulating antiviral and anti-tumor immune responses. TLR9 is mainly expressed on pDCs and B lymphocytes. Its activation induces robust secretion of IFN-$\alpha$ and other cytokines, thereby enhancing immune surveillance and clearance of tumor cells.

3. CD40 (aptamer): CD40 is a co-stimulatory molecule of the immune system which, by binding to the CD40L receptor, modulates immune responses. A CD40 aptamer can promote maturation of DCs and enhance antigen presentation. In addition, the CD40 aptamer can increase proliferation and activation of CD8$^+$ T cells, thereby improving tumor-specific cytotoxicity. In the present disclosure, the CD40 aptamer acts synergistically with CpG2006 to further strengthen anti-tumor immune responses.

4. PD-L1: PD-L1 is an immunosuppressive molecule primarily expressed on tumor cells. By binding to PD-1, PD-L1 suppresses T-cell activation and proliferation, enabling immune escape. Incorporation of a PD-L1 aptamer blocks the PD-LI/PD-1 interaction, releasing T-cell inhibition and restoring cytotoxic activity.

5. DNA nano-nucleic-acid delivery system: As an efficient delivery carrier, this system improves in vivo stability and bioavailability of the CD24 aptamer, CpG2006, CD40 aptamer, and PD-L1 aptamer. DNA nano-nucleic acids exhibit favorable biocompatibility and low toxicity and can effectively reduce non-specific distribution and metabolism in vivo to improve therapeutic outcomes. By combining the CD24 aptamer, CpG2006, CD40 aptamer, and PD-L1 aptamer, the nano-nucleic-acid delivery system integrates four immunotherapeutics to achieve synergy in the tumor microenvironment.

In summary, the mechanism of this specific-immunity triple-combination nano-nucleic-acid therapeutic agent can be summarized as follows:

1. the CD24 aptamer specifically binds CD24 on SCLC cells, inhibiting invasiveness and metastasis and enhancing sensitivity to immunotherapy;

2. CpG2006 activates TLR9 and induces robust antiviral and anti-tumor cytokine responses, enhancing immune surveillance and clearance of tumor cells;

3. the CD40 aptamer promotes DC maturation and antigen presentation, enhancing activation and proliferation of CD8$^+$ T cells and increasing tumor-specific cytotoxicity;

4. PD-L1 aptamer blocks the interaction between PD-L1 and PD-1, releasing T-cell inhibition and restoring cytotoxic activity against tumor cells.

5. DNA nano-nucleic-acid delivery system integrates the CD24 aptamer, CpG2006, CD40 aptamer, and PD-L1 aptamer into a unitary composition, thereby realizing synergistic action of the immunotherapeutics within the tumor microenvironment and improving therapeutic efficacy.

Through such multi-pathway, multi-target synergy, this specific-immunity triple-combination nano-nucleic-acid therapeutic agent exhibits potential application prospects in the treatment of small-cell lung cancer (SCLC). In the future, such immunotherapeutics are expected to provide more effective and safer novel immunotherapies for clinical oncology.

In subsequent studies, the respective proportions and dosages of the different components of the present specific-immunity triple-combination nano-nucleic-acid therapeutic agent may be further optimized to improve efficacy and reduce adverse effects. In addition, similar investigations may be conducted with respect to other tumor-associated molecules so as to expand the application scope of novel immunotherapeutics in oncology.

In summary, this nano-nucleic-acid triple-aptamer integrated active-combination immunotherapeutic provides a novel strategy for SCLC treatment. By virtue of the synergy among multiple effector molecules, an effective attack on tumor cells is achieved. The present research provides new ideas and methods for the field of immunotherapy and contributes to further development of clinical cancer treatment.

Drug 76): HBsAg Aptamer - HBV siRNA - miR-34 - PD-L1 Aptamer - miR-542 -AS1411 Aptamer - DNA Carrier

This disclosure relates to a specific-immunity and gene-regulation quintuple-combination nano-nucleic-acid therapeutic agent, which exhibits significant efficacy against hepatitis B virus (HBV) infection and HBV-related hepatocellular carcinoma (HCC). The functions of each effector molecule and the synergistic effects/mechanisms after integration are as follows:

1. HBsAg: Hepatitis B surface antigen (HBsAg) is the surface protein of HBV and a key factor during viral infection and replication. In the present disclosure, an HBsAg-targeting aptamer is configured to recognize and specifically bind HBsAg on the surface of HBV, thereby preventing viral entry and infection and inhibiting HBV replication.

2. HBV siRNA: HBV siRNA is a small interfering RNA targeting HBV genes. Through RNA interference (RNAi), it specifically targets and degrades viral genes, thereby inhibiting HBV replication and expression of viral proteins.

3. miR-34: miR-34 is a microRNA with anti-tumor activity that regulates expression of multiple tumor-associated genes and suppresses cell proliferation, invasion, and metastasis. In HBV-related HCC, miR-34 expression is typically downregulated; restoration of its expression suppresses growth and invasiveness of tumor cells.

4. PD-L1: PD-L1 is an immunosuppressive molecule primarily expressed on tumor cells. By binding the PD-1 receptor, PD-L1 suppresses T-cell activation and proliferation, enabling immune evasion. In the present disclosure, inclusion of a PD-L1 aptamer blocks the PD-L1/PD-1 interaction, releases T-cell inhibition, and restores cytotoxic activity against tumor cells.

5. miR-542: miR-542 is a microRNA that regulates biological processes including apoptosis, cell cycle, and autophagy. In HBV-related HCC, miR-542 expression is downregulated; increasing its expression can suppress tumor-cell growth and invasiveness.

6. AS1411: AS1411 is a guanine-rich nucleic-acid aptamer that specifically binds nucleolin (NCL; nucleolar protein A) on the surface of tumor cells, thereby inhibiting proliferation, migration, and invasion of tumor cells. AS1411 exhibits anti-tumor activity in HBV-related HCC.

7. DNA nano-nucleic-acid delivery system: As an efficient delivery vector, this system is configured to improve the in vivo stability and bioavailability of the HBsAg aptamer, HBV siRNA, miR-34, PD-L1 aptamer, miR-542, and AS 1411. DNA nano-nucleic acids exhibit favorable biocompatibility and low toxicity, and can effectively reduce non-specific distribution and metabolism of gene and immunotherapeutic agents in vivo to improve therapeutic outcomes. By combining the HBsAg aptamer, HBV siRNA, miR-34, PD-L1 aptamer, miR-542, and AS 1411, the nano-nucleic-acid delivery system integrates six therapeutic molecules to achieve synergistic action within the hepatic-carcinoma microenvironment.

In summary, the mechanisms of action of the specific-immunity and gene-regulation quintuple-combination nano-nucleic-acid therapeutic agent may be summarized as follows:

1. An aptamer targeting HBsAg prevents viral entry and infection and inhibits replication of hepatitis B virus (HBV).

2. HBV siRNA specifically degrades viral genes via an RNA interference (RNAi) mechanism, thereby inhibiting HBV replication and expression of viral proteins.

3. miR-34 and miR-542 regulate expression of tumor-associated genes to suppress proliferation, invasion, and metastasis of hepatocellular carcinoma cells.

4. A PD-L1 aptamer blocks the interaction between PD-L1 and PD-1, releasing T-cell inhibition and restoring cytotoxic activity against tumor cells.

5. AS1411 binds nucleolin (NCL) on the surface of tumor cells and inhibits tumor-cell proliferation, migration, and invasion.

6. A DNA nano-nucleic-acid delivery system integrates the six therapeutic molecules to realize synergistic action within the hepatocellular-carcinoma microenvironment and improve therapeutic efficacy.

Through such multi-pathway, multi-target synergy, the therapeutic exhibits potential application prospects in the treatment of HBV infection and HBV-associated hepatocellular carcinoma. These agents are expected to provide more effective and safer novel immunotherapies for clinical hepatology; however, confirmation in large-sample, multi-center clinical trials is required to ensure efficacy and safety in humans.

In future studies, the respective proportions and dosages of the different components may be further optimized to improve efficacy and reduce adverse effects. In addition, similar investigations may be conducted for other viral infections and virus-associated tumors so as to broaden the application scope of novel immunotherapeutics in disease treatment.

In summary, this specific-immunity and gene-regulation quintuple-combination nano-nucleic-acid therapeutic agent provides a novel strategy for treating HBV infection and HBV-associated hepatocellular carcinoma. By virtue of synergy among multiple effector molecules, effective attack on viral infection and tumor cells is achieved, thereby offering new ideas and approaches for immunotherapy and contributing to further development of clinical hepatology.

Drug 77): IL-4Rα Aptamer - TIMC-d Aptamer - 4*miR-126 - RNA Carrier

This disclosure relates to a specific-immunity and gene-regulation triple-combination nano-nucleic-acid therapeutic agent. The mechanisms for suppressing autoimmune diseases are as follows:

1. IL-4Rα: IL-4Rα is the α-subunit of the interleukin-4 receptor (IL-4R). IL-4R participates primarily in regulation of Th2 immune responses. Under certain pathological conditions (e.g., inflammation, autoimmune disease, tumors), IL-4Rα expression is upregulated, resulting in over-activation of Th2 responses. In the present disclosure, an IL-4Rα-targeting aptamer specifically binds and inhibits IL-4Rα function, thereby reducing over-activation of Th2 responses and alleviating inflammation and immunopathological injury.

2. TIMC-d: TIMC-d is an aptamer directed to the T-cell immunoglobulin and mucin domain (TIM) family. The TIM family is a group of membrane proteins that play key roles in immune regulation. Here, a TIM-family-targeting aptamer suppresses pathological over-activation of TIM-family members, modulates T-cell immune responses, and reduces inflammation and immunopathological injury.

3. miR-126: Full name miR-126-3p; miR-126-3p is a microRNA that regulates expression of multiple genes. In immune-related diseases and inflammation, miR-126-3p expression is typically downregulated. Increasing miR-126-3p expression suppresses activation of inflammatory cells and production of inflammatory mediators, thereby alleviating inflammation and immunopathological injury.

4. RNA nano-nucleic-acid delivery system: As an efficient delivery vector, the system improves in-vivo stability and bioavailability of the IL-4Rα aptamer, TIMC-d aptamer, and miR-126-3p. RNA nano-nucleic acids exhibit favorable biocompatibility and low toxicity and can effectively reduce non-specific distribution and metabolism of aptamers and miRNAs in vivo to improve therapeutic outcomes.

By combining the IL-4Rα aptamer, TIMC-d aptamer, and miR-126-3p, the nano-nucleic-acid delivery system realizes synergistic action of these three molecules in the treatment of inflammatory and immune-related diseases. The synergistic mechanisms may be summarized as follows:

1. Specifically inhibiting IL-4Rα function to reduce over-activation of Th2 responses and thereby alleviate inflammation and immunopathological injury.

2. Suppressing pathological over-activation of TIM-family members to modulate T-cell immune responses and thereby alleviate inflammation and immunopathological injury.

3. Increasing miR-126-3p expression to suppress activation of inflammatory cells and production of inflammatory mediators and thereby alleviate inflammation and immunopathological injury.

4. Integrating the three molecules via an RNA nano-nucleic-acid delivery system to realize synergy during treatment and improve therapeutic efficacy.

In summary, the specific-immunity and gene-regulation triple-combination nano-nucleic-acid therapeutic agent achieves effective treatment of inflammatory and immune-related diseases through multi-pathway, multi-target synergy. This work provides new ideas and approaches for immunotherapy and facilitates further development of clinical disease treatment.

Moreover, the respective proportions and dosages of the different components may be further optimized to improve efficacy and reduce adverse effects. Future studies may extend similar research to other inflammatory and immune-related diseases to broaden the application scope of novel immunotherapeutics in disease treatment.

Drug 78): CD3-4 Aptamer - PD-1 Aptamer - BCMA Aptamer - DNA Carrier

This disclosure relates to a specific-immunity modulatory triple-combination nano-nucleic-acid therapeutic agent. The mechanisms for inhibiting multiple myeloma (MM) are analyzed as follows:

1. CD3-4 aptamer: An aptamer directed to the CD3 protein on T-cell surfaces. By binding CD3, the aptamer recruits and activates T cells. In MM, immune tolerance to tumor cells is frequently observed. Activation of T cells by the CD3-4 aptamer enhances T-cell attack and clearance of myeloma cells, thereby suppressing tumor growth and dissemination.

2. PD-1 aptamer: Programmed cell death protein-1 (PD-1) is an inhibitory receptor typically expressed on activated T cells. Upon engagement with its ligand PD-L1 on tumor cells, PD-1 suppresses T-cell activation, enabling immune escape. A PD-1 aptamer blocks the PD-1/PD-L1 interaction, releases T-cell inhibition, and restores immune attack against myeloma cells.

3. BCMA aptamer: BCMA (B-cell maturation antigen; TNFRSF17) is highly expressed on myeloma cells; binding to its ligands promotes invasion, growth, and survival of myeloma cells. A BCMA aptamer, by binding BCMA, blocks ligand interactions and inhibits invasion, growth, and survival of myeloma cells, thereby achieving therapeutic effect against MM.

4. DNA nano-nucleic-acid delivery system: An efficient delivery carrier configured to improve in-vivo stability and bioavailability of the aptamers. Via the DNA nano-nucleic-acid delivery system, the three aptamers act more effectively on myeloma cells to realize synergistic anti-tumor effects.

In summary, this specific-immunity modulatory triple-combination nano-nucleic-acid therapeutic agent suppresses the progression of MM through the following synergies:

1. Activation of T cells: A CD3-4 aptamer activates T cells and enhances their ability to attack and clear myeloma cells.

2. Restoration of immune attack function: A PD-1 aptamer blocks the interaction between PD-1 and PD-L1, releases T-cell inhibition, and restores immune attack against myeloma cells.

3. Inhibition of myeloma-cell growth: A BCMA aptamer, by binding BCMA, blocks its interactions with ligands and inhibits invasion, growth, and survival of myeloma cells.

In addition, a DNA nano-nucleic-acid delivery system integrates the three aptamers into a unitary composition, thereby realizing synergistic action during treatment and improving therapeutic efficacy.

Such synergy provides new ideas and approaches for the treatment of multiple myeloma and facilitates further clinical development. It should be noted that validation in large-sample, multi-center clinical trials remains necessary to ensure efficacy and safety in humans. Moreover, the respective proportions and dosages of the different components of the specific-immunity modulatory triple-combination nano-nucleic-acid therapeutic agent may be further optimized to improve efficacy and reduce adverse effects. Future studies may conduct similar investigations in other tumor types so as to broaden the application scope of novel immunotherapeutics in disease treatment.

Drug 79): CpG2006 - CD38 Aptamer - PD-L1 Aptamer - DNA Carrier

This disclosure provides a specific active-immunity modulatory triple-combination nano-nucleic-acid therapeutic agent. The mechanisms for inhibiting multiple myeloma (MM) are analyzed as follows:

1. CpG2006 (aptamer): CpG2006 is a CpG oligodeoxynucleotide (CpG ODN) configured to activate the Toll-like receptor 9 (TLR9) signaling pathway. This pathway plays a key role in immune cells (such as dendritic cells and B cells) and can stimulate host immune responses. In MM, the CpG2006 aptamer can enhance the immune system's attack and clearance of myeloma cells.

2. CD38 aptamer: CD38 is a protein highly expressed on the surface of myeloma cells and promotes their growth and survival. A CD38-targeting aptamer, by binding CD38, can directly impact growth and survival of myeloma cells and thereby inhibit disease progression.

3. PD-L1 aptamer: PD-L1 is an immunosuppressive molecule commonly expressed on tumor-cell surfaces. Upon binding PD-1 (a receptor on T-cell surfaces), PD-L1 suppresses T-cell activation, enabling tumor immune escape. A PD-L1 aptamer blocks the PD-L1/PD-1 interaction, releases T-cell inhibition, and restores immune attack against myeloma cells.

Under the integrated synergy of these three aptamers, the nano-nucleic-acid triple-aptamer active-combination immunotherapeutic achieves the following functions:

1. Enhancement of immune responses: the CpG2006 aptamer activates the TLR9 pathway and strengthens host immune attack and clearance of myeloma cells.

2. Direct suppression of myeloma cells: the CD38 aptamer binds CD38 and affects myeloma-cell growth and survival.

3. Restoration of T-cell immune attack: the PD-L1 aptamer blocks PD-L1/PD-1 to release T-cell inhibition and re-enable immune attack on myeloma cells.

Accordingly, this specific active-immunity modulatory triple-combination nano-nucleic-acid therapeutic agent counters MM through synergy among the three aptamers, thereby improving therapeutic effect and helping reduce adverse effects.

It is noted that validation in large-sample, multi-center clinical trials remains necessary to ensure efficacy and safety in humans. Moreover, the respective proportions and dosages of the different components of the nano-nucleic-acid triple-aptamer composition may be further optimized to improve efficacy and reduce adverse effects. Future studies may conduct similar investigations in other tumor types so as to broaden the application scope of novel immunotherapeutics in disease treatment.

Drug 80): CD20 Aptamer-CpG-BYZD Aptamer-CD38 Aptamer-miR-34-CD3 Aptamer - DNA Carrier

This disclosure relates to a specific active-immunity and gene-regulation quintuple-combination nano-nucleic-acid therapeutic agent. The mechanisms for inhibiting multiple myeloma (MM) are analyzed as follows:

1. CD20 aptamer: CD20 is a molecule primarily expressed on B-cell surfaces. By binding CD20, a CD20 aptamer can directly affect the growth and survival of B cells (including myeloma cells), thereby suppressing progression of myeloma.

2. CpG-BYZD aptamer (PCIF1): PCIF1 is an enzyme involved in mRNA modification and plays an important role in growth and survival of tumor cells. An aptamer directed to PCIF1 (referred to herein as a CpG-BYZD aptamer) interferes with PCIF1 function and may inhibit growth and survival of myeloma cells.

3. CD38 aptamer: CD38 is highly expressed on myeloma-cell surfaces and promotes myeloma-cell growth and survival. A CD38 aptamer, by binding CD38, can directly impact growth and survival of myeloma cells and thereby inhibit disease progression.

4. miR-34 (miR-34a): miR-34a is a microRNA that specifically targets a conserved sequence of PD-L1 mRNA, thereby reducing PD-L1 protein expression, functionally blocking the PD-1/PD-L1 pathway, activating T-cell immune responses, and suppressing growth and invasion of multiple tumor cells. Delivery of miR-34a may thus exert inhibitory effects on myeloma cells.

5. CD3 aptamer: CD3 is an important molecule on T-cell surfaces. By binding CD3, a CD3 aptamer activates T cells and enhances their ability to attack and clear myeloma cells.

Under the integrated synergy of these aptamers/miRNA, the multi-aptamer active-combination nano-nucleic-acid immunotherapeutic achieves the following functions:

1. Direct suppression of myeloma cells: CD20, PCIF1 (CpG-BYZD), and CD38 aptamers bind their respective targets and affect growth and survival of myeloma cells.

2. Regulation of gene expression: miR-34a regulates expression of target genes to suppress myeloma-cell growth and invasion.

3. Activation of T-cell immune attack: the CD3 aptamer activates T cells, enhancing host immune attack and clearance of myeloma cells.

In view of the foregoing synergies, this specific-immunity and gene-regulation quintuple-combination nano-nucleic-acid therapeutic agent counteracts MM on multiple fronts. Such synergy improves therapeutic efficacy and helps reduce adverse effects.

It is noted that validation in large-sample, multi-center clinical trials remains necessary to ensure efficacy and safety in humans. Moreover, the respective proportions and dosages of the different components of the multi-aptamer nano-nucleic-acid composition may be further optimized to improve efficacy and reduce adverse effects. Future studies may conduct similar investigations for other tumor types so as to broaden the application scope of novel immunotherapeutics in disease treatment.

Drug 81): CpG2006 - CD38 Aptamer - miR-126 - PD-L1 Aptamer - DNA Carrier;

This disclosure relates to a specific-immunity and gene-regulation quadruple-combination nano-nucleic-acid therapeutic agent. The mechanisms of action are as follows:

1. CpG2006: CpG2006 is an immunostimulatory agent configured to activate the Toll-like receptor 9 (TLR9) pathway and thereby stimulate immune cells (e.g., dendritic cells and B cells), promoting anti-tumor immune responses.

2. miR-126: miR-126 is a microRNA that suppresses growth and invasion of various tumor cells. By delivering miR-126, inhibitory effects may be exerted on myeloma cells.

3. CD38 aptamer: CD38 is a protein highly expressed on myeloma-cell surfaces and promotes myeloma-cell growth and survival. A CD38-targeting aptamer, by binding CD38, can directly impact growth and survival of myeloma cells and thereby inhibit myeloma progression.

4. PD-L1 aptamer: By binding PD-L1, a PD-L1 aptamer blocks the PD-1/PD-L1 immune-checkpoint pathway and thereby restores T-cell activity. This enables T cells to attack tumor cells more effectively and thus suppress tumor growth.

The design of this specific-immunity and gene-regulation quadruple-combination nano-nucleic-acid therapeutic agent affords synergistic enhancement in oncology applications. This integrated therapeutic strategy is expected to provide cancer patients with more effective and safer treatment options, improving survival and quality of life.

Drug 82): BCMA Aptamer - miR-126 - CD38 Aptamer - miR-122 - CD3 Aptamer - miR-34 - DNA Carrier

This disclosure relates to a specific-immunity and gene-regulation sextuple-combination nano-nucleic-acid therapeutic agent. The mechanisms by which this multi-aptamer active gene-combination immunotherapeutic inhibits multiple myeloma (MM) are analyzed as follows:

1. BCMA aptamer: B-cell maturation antigen (BCMA) is predominantly expressed on plasma cells (including myeloma cells). By binding BCMA, a BCMA aptamer can directly affect growth and survival of plasma cells (including myeloma cells) and thereby suppress myeloma progression.

2. miR-126: miR-126 is a microRNA that suppresses growth and invasion of various tumor cells. By delivering miR-126, inhibitory effects may be exerted on myeloma cells.

3. CD38 aptamer: CD38 is highly expressed on myeloma-cell surfaces and promotes their growth and survival. A CD38 aptamer binds CD38 to directly affect myeloma-cell growth and survival and thus inhibit disease progression.

4. miR-122: miR-122 is a microRNA that also suppresses growth and invasion of various tumor cells. By delivering miR-122, inhibitory effects may be exerted on myeloma cells.

5. CD3 aptamer: CD3 is an important molecule on T-cell surfaces. By binding CD3, a CD3 aptamer activates T cells and enhances their ability to attack and clear myeloma cells.

6. miR-34: miR-34 is another microRNA that suppresses growth and invasion of various tumor cells. By delivering miR-34, inhibitory effects may be exerted on myeloma cells.

Under the integrated synergy of these components, the multi-aptamer active gene-combination nano-nucleic-acid immunotherapeutic achieves the following functions:

1. Direct suppression of myeloma cells: BCMA and CD38 aptamers, by binding their respective targets, affect myeloma-cell growth and survival.

2. Regulation of gene expression: miR-126, miR-122, and miR-34 regulate expression of target genes-especially by blocking PD-L mRNA to restore T-cell immune function-thereby suppressing myeloma-cell growth and invasion.

3. Activation of T-cell immune attack: the CD3 aptamer activates T cells to enhance host immune attack and clearance of myeloma cells.

In view of these synergies, the specific-immunity and gene-regulation sextuple-combination nano-nucleic-acid therapeutic agent counteracts multiple myeloma on multiple fronts, improving therapeutic effect and helping reduce adverse effects.
It is noted that validation in large-sample, multi-center clinical trials remains necessary to ensure efficacy and safety in humans. Moreover, the respective proportions and dosages of the different components of the multi-aptamer nano-nucleic-acid composition may be further optimized to improve efficacy and reduce adverse effects. Future studies may conduct similar investigations for other tumor types so as to broaden the application scope of novel immunotherapeutics in disease treatment.

Drug 83): GPC3 Aptamer - AmiR-21 - CD38 Aptamer - miR-122 - PD-L1 Aptamer - miR-34 - DNA Carrier

This disclosure relates to a specific-immunity and gene-regulation sextuple-combination nano-nucleic-acid therapeutic agent-i.e., a nano-nucleic-acid multi-aptamer active gene-combination immunotherapeutic-directed to treatment of hepatocellular carcinoma (HCC). Multiple effector molecules are integrated to achieve broader and more efficient anti-tumor activity. The functions of the respective effector molecules and the integrated synergistic effects are as follows:

1. GPC3: A hepatocellular-carcinoma-specific antigen that facilitates recognition and clearance of tumor cells.

2. AmiR-21: An anti-miR-21 agent configured to inhibit miR-21 activity and thereby slow tumor-cell growth and invasion.

3. CD38: A surface molecule on immune cells that promotes immune attack on tumor cells.

4. miR-122: Suppresses HBV cccDNA expression and regulates hepatic lipid metabolism, favoring inhibition of viral replication and tumor progression.

5. PD-L1: Relieves immune suppression and enhances host immune attack and clearance of tumor cells.

6. miR-34: Enhances immune attack and clearance of tumor cells by suppressing PD-L1 expression and inhibits tumor progression.

Integrated synergistic effects: Within this nano-nucleic-acid multi-aptamer composition, the effector molecules act in concert to realize synergistic anti-tumor effects. GPC3 and CD38 assist the immune system in recognizing and attacking tumor cells; AmiR-21, miR-122, and miR-34 suppress tumor growth and invasion by regulating gene expression; and the PD-L1 aptamer relieves immune suppression and enhances immune attack on tumor cells.

Such integrated synergy helps improve therapeutic efficacy, reduce adverse effects, and strengthen anti-tumor activity. Nevertheless, validation in large-sample, multi-center clinical trials remains necessary to ensure efficacy and safety in humans. In addition, the respective proportions and dosages of the different components of this nano-nucleic-acid multi-aptamer composition may be further optimized to improve efficacy and reduce adverse effects. In future research, the anti-tumor effect of this specific-immunity and gene-regulation sextuple-combination nano-nucleic-acid therapeutic agent may be further enhanced via the following approaches:

1. Optimization of the delivery system: Develop novel nano-carriers to improve bioavailability and targeting of aptamers, thereby enhancing efficacy and reducing adverse effects.

2. Combination therapy: Use this nano-nucleic-acid multi-aptamer together with other anti-tumor drugs or modalities (e.g., chemotherapy, radiotherapy, targeted therapy) to realize synergistic therapeutic effects and reduce side effects of monotherapies.

3. Personalized treatment: Customize multi-aptamer regimens based on a patient's tumor gene-expression profile, mutational status, and individual immune status to improve efficacy and lower the risk of adverse effects.

4. Dynamic monitoring: Monitor tumor biomarkers, immune-function indices, and related parameters to assess therapeutic effect in real time and adjust the regimen promptly, thereby improving efficacy and reducing adverse-effect risk.

5. Prophylactic application: For high-risk populations, implement early intervention to improve prophylactic application of the multi-aptamer nano-nucleic-acid therapeutic and reduce HCC incidence.

In summary, this specific-immunity and gene-regulation sextuple-combination nano-nucleic-acid therapeutic agent has substantial potential in oncology but still requires confirmation of efficacy and safety through clinical trials and further research.

Drug 84): CpG2006 - VEGF165 Aptamer - miR-122 - PD-L1 Aptamer - DNA Carrier

This disclosure relates to a specific active-immunity and gene-regulation quadruple-combination nano-nucleic-acid therapeutic agent-i.e., a nano-nucleic-acid multi-aptamer active gene-combination immunotherapeutic-directed to treatment of hepatocellular carcinoma (HCC) arising from hepatitis B virus (HBV) infection. The combination includes multiple effector molecules intended to enhance anti-tumor activity and immune modulation. The functions of the respective effectors and the integrated synergistic effects are as follows:

1. CpG2006: A CpG oligonucleotide that activates the immune system, inducing antiviral and anti-tumor responses and enhancing host attack on tumor cells.

2. VEGF165 aptamer: Inhibits tumor angiogenesis and thereby slows tumor growth and dissemination.

3. miR-122: Suppresses HBV cccDNA expression and regulates hepatic lipid metabolism, favoring inhibition of viral replication and tumor progression.

4. PD-L1 aptamer: Relieves immune suppression and enhances host immune attack and clearance of tumor cells.

Integrated synergistic effects: In this nano-nucleic-acid multi-aptamer composition, the effectors cooperate to realize synergistic anti-tumor effects. CpG2006 enhances immune attack on tumor cells by activating the immune system; the VEGF165 aptamer inhibits tumor angiogenesis and thus slows tumor growth; miR-122 suppresses viral replication and reduces virus-induced hepatic injury; and the PD-L1 aptamer relieves immune suppression and enhances immune attack on tumor cells.

Such integrated synergy helps improve therapeutic efficacy, reduce adverse effects, and strengthen anti-tumor activity. However, these results still require validation in large-sample, multi-center clinical trials to ensure efficacy and safety in humans. In addition, the respective proportions and dosages of the different components of this nano-nucleic-acid multi-aptamer composition may be further optimized to improve efficacy and reduce adverse effects. Future research may also develop novel nano-carriers to increase aptamer bioavailability and targeting, thereby enhancing efficacy and reducing adverse effects.

Combination therapy is likewise a promising direction. The nano-nucleic-acid multi-aptamer may be used in combination with other anti-tumor drugs or modalities (e.g., chemotherapy, radiotherapy, targeted therapy) to realize synergistic effects and lessen the side effects of single-agent therapy.

Personalized treatment is also a promising strategy. Based on tumor gene-expression profiles, mutational status, and a patient's immune status, personalized multi-aptamer regimens may be designed to improve efficacy and reduce adverse-effect risk.

Dynamic monitoring of therapeutic effect is another important measure. By monitoring tumor biomarkers, immune-function indices, and related parameters, treatment efficacy can be assessed in real time and regimens adjusted promptly, thereby improving efficacy and reducing adverse-effect risk.

For high-risk populations, prophylactic application may be attempted. Through early intervention, the prophylactic application of this nano-nucleic-acid multi-aptamer may be improved to reduce the incidence of liver cancer.

In summary, this specific active-immunity and gene-regulation quadruple-combination nano-nucleic-acid therapeutic agent holds substantial promise in oncology but still requires clinical trials and further research to verify efficacy and safety. Future studies may improve anti-tumor effects by optimizing the delivery system, employing combination therapy, personalizing treatment, implementing dynamic monitoring, and exploring prophylactic applications.

Drug 85): CpG2006 - HBsAg Aptamer - miR-122 - PD-L1 Aptamer - DNA Carrier

This disclosure relates to a specific active-immunity and gene-regulation quadruple-combination nano-nucleic-acid therapeutic agent. The mechanisms of action are as follows:

1. CpG2006: CpG2006 is an immunostimulatory agent configured to activate the Toll-like receptor 9 (TLR9) pathway and thereby stimulate immune cells (e.g., dendritic cells and B cells), promoting anti-tumor immune responses.

2. HBsAg aptamer: This component targets delivery of nanoparticles to HBV host-cell tissues and suppresses HBsAg expression.

3. miR-122: This component specifically binds to and inhibits HBV covalently closed circular DNA (cccDNA), thereby reducing viral load in host cells.

4. PD-L1 aptamer: This component blocks the PD-1/PD-L1 immune-checkpoint pathway and restores release of T-cell immune function.

By virtue of cooperative action among multiple effector molecules, the therapeutic facilitates achievement of a functional cure of HBV. CpG2006 oligonucleotide activates B-cell TLR9, enhancing immune responses; the HBsAg aptamer directs nanoparticles to HBV host cells to more effectively suppress HBsAg expression; miR-122 suppresses HBV cccDNA and reduces viral load; and the PD-L1 aptamer blocks the PD-1/PD-L1 checkpoint and restores T-cell function, thereby enabling a stronger immune response against HBV

Drug 86): ASO-GSK836 - HBsAg Aptamer - miR-122 - PD-L1 Aptamer - DNA Carrier

This disclosure relates to a specific active-immunity and gene-regulation quadruple-combination nano-nucleic-acid therapeutic agent in which multiple components act in concert to achieve a functional cure of hepatitis B virus (HBV).
ASO-GSK836 oligonucleotide: This component primarily activates B-cell TLR9, enhancing the immune response to HBV

HBsAg aptamer: This component targets delivery of nanoparticles to HBV host-cell tissues and suppresses HBsAg expression.

miR-122: This component specifically binds to and inhibits HBV cccDNA, thereby reducing viral load in host cells.

PD-L1 aptamer: This component blocks the PD-1/PD-L1 immune-checkpoint pathway and restores release of T-cell immune function.

The combination of these components provides a multi-pronged approach to functional cure of HBV The ASO-GSK836 oligonucleotide activates B-cell TLR9 and enhances immune responses; the HBsAg aptamer directs nanoparticles to HBV host cells to more effectively suppress HBsAg expression; miR-122 suppresses HBV cccDNA and reduces viral load; and the PD-L1 aptamer blocks the PD-1/PD-L1 checkpoint and restores T-cell function, thereby enabling a stronger immune response against HBV.

Drug 87): BCMA Aptamer - CD38 Aptamer - miR-126 - PD-L1 Aptamer - miR-34 - DNA Carrier

This disclosure relates to a specific-immunity and gene-regulation quintuple-combination nano-nucleic-acid therapeutic agent for treatment of multiple myeloma (MM). The combination includes multiple effector molecules intended to enhance anti-tumor activity and immune modulation. The functions of the respective effectors and the integrated synergistic effects are as follows:

1. BCMA (B-cell maturation antigen): BCMA is a plasma-cell surface molecule that binds growth factors APRIL and BAFF to promote plasma-cell survival and proliferation. Targeting BCMA selectively kills myeloma cells and reduces tumor burden.

2. CD38: CD38 is a glycohydrolase on the surface of myeloma cells with multiple biological functions. Targeting CD38 eliminates myeloma cells and enhances immune attack on tumors.

3. miR-126: miR-126 specifically binds and suppresses VCAM-1 mRNA, thereby affecting migration and invasion of myeloma cells. miR-126 also participates in angiogenesis, cell proliferation, and migration; in myeloma, it may inhibit cell growth by suppressing tumor angiogenesis and regulating the cell cycle.

4. PD-L1 (programmed death-ligand 1): PD-L1 is an immunosuppressive molecule that, by binding the PD-1 receptor, suppresses immune-cell function. Inhibiting PD-L1 activates immune cells and enhances attack on myeloma cells.

5. miR-34-5p: miR-34-5p is an anti-tumor microRNA shown to suppress PD-L1 expression by binding the 3'-UTR of PD-L1 (CD274) mRNA. By lowering PD-L1 expression, miR-34-5p enhances immune attack and clearance of tumor cells and thereby inhibits tumor progression.

Integrated synergistic effects: Within this nano-nucleic-acid multi-aptamer composition, the effector molecules act cooperatively to realize synergistic anti-tumor effects. BCMA and CD38 target myeloma cells directly to reduce tumor burden; miR-126 suppresses migration and invasion by inhibiting VCAM-1 mRNA, and may also inhibit myeloma-cell growth by suppressing angiogenesis and regulating the cell cycle; PD-L1 inhibition helps activate immune cells and enhance attack on myeloma cells; and miR-34-5p further enhances immune attack and clearance by suppressing PD-L1 expression.

This integrated synergy significantly improves therapeutic effect. By attacking myeloma cells via multiple pathways and modulating the immune system to enhance clearance of tumor cells, the combined therapeutic strategy may provide more effective treatment options for patients with multiple myeloma. However, further research and validation are required prior to clinical application to ensure safety and efficacy.

Drug 88): AS1411 Aptamer - FGF2 Aptamer - 3× AmiR-21 - DNA Carrier (for alleviation of cancer pain)

This disclosure relates to a specific targeted-knockdown and gene-regulation dual-combination nano-nucleic-acid therapeutic agent. Following peripheral axonal injury, expression of miR-21-5p in dorsal root ganglion (DRG) sensory neurons is upregulated, which may be associated with increased pain hypersensitivity and recruitment of inflammatory macrophages. The therapeutic includes two aptamers (AS1411 and FGF2) together with an anti-miR-21 sequence; by targeting and suppressing FGF2 and miR-21, cancer pain-particularly bone pain-can be significantly suppressed and alleviated. The synergistic mechanisms for inhibiting cancer pain are as follows:

1. Blocking neuron-macrophage communication: By suppressing expression of miR-21-5p, the amount of miR-21-5p in exosomes released by sensory neurons is reduced, thereby lowering recruitment of inflammatory macrophages.

This helps mitigate inflammatory responses and reduce pain hypersensitivity.

2. FGF2 inhibition: FGF2 promotes nerve growth and inflammatory responses; suppressing FGF2 expression reduces pain hypersensitivity and alleviates pain.

3. Anti-inflammatory activity: Suppressing expression of miR-21-5p and FGF2 mitigates inflammatory responses, further reducing the occurrence and exacerbation of cancer pain.

4. Regulation of apoptosis: miR-21 suppresses expression of apoptosis-related genes, leading to cancer-cell proliferation. By inhibiting miR-21, the anti-miR-21 sequence restores apoptosis, suppresses tumor growth, and reduces the occurrence of pain.

5. Impact on bone remodeling: Bone pain is often associated with bone metastasis and dysregulated bone remodeling. FGF2 and miR-21 influence bone metabolism; suppressing them helps restore bone-remodeling balance and thereby alleviates bone pain.

6. Inhibition of neural remodeling: Nerve injury and inflammation can induce neural remodeling and increase pain hypersensitivity. By suppressing FGF2 and miR-21, neural remodeling is reduced, further alleviating pain.

7. Reduction of neuronal excitability: Increased inflammatory responses and neuronal excitability are key drivers of pain. Suppressing expression of FGF2 and miR-21 reduces neuronal excitability and thereby lowers pain sensitivity.

8. Modulation of pain-signal transmission: Suppressing expression of FGF2 and miR-21 affects transmission of pain signals between the central and peripheral nervous systems, helping to diminish amplification of pain signaling and reduce pain sensitivity.

9. Improvement of the local tumor microenvironment: Anti-inflammatory effects and suppression of tumor growth improve the local tumor microenvironment and reduce pain sensitivity, thereby decreasing the occurrence and exacerbation of cancer pain and bone pain.

In summary, by targeting and suppressing FGF2 and miR-21-and their roles in tumor growth, metastasis, inflammatory responses, neuron-macrophage communication, and bone remodeling-this specific targeted-knockdown and gene-regulation dual-combination nano-nucleic-acid therapeutic agent can significantly suppress and alleviate cancer pain, particularly bone pain. The respective effector molecules act synergistically on multiple levels to markedly suppress and alleviate cancer pain and bone pain, providing a new and effective strategy for pain therapy.

Drug 89): HBsAg Aptamer - CD40 Aptamer - PD-L1 Aptamer - DNA Carrier

This disclosure provides a specific-immunity triple-combination nano-nucleic-acid therapeutic agent directed to hepatitis B virus (HBV) infection, featuring integrated synergy among multiple effector molecules. The agent includes three aptamers: HBsAg (hepatitis B surface antigen), CD40, and PD-L1. The functions and synergistic effects, as well as mechanisms of action, are as follows:

1. HBsAg (hepatitis B surface antigen): HBsAg is an important antigen on the surface of HBV and induces immune responses against HBV By targeting HBsAg, HBV-specific immune responses are enhanced, aiding viral clearance.

2. CD40: CD40 is an immune-cell surface molecule involved in regulation of immune-cell activation and immune responses. Activation of CD40 promotes activation of B cells and T cells and enhances immune attack against HBV

3. PD-L1: PD-L1 is an immunosuppressive molecule commonly expressed on tumor-cell surfaces. PD-L1 binds its receptor PD-1 and suppresses immune-cell activity, thereby allowing HBV to evade immune clearance. By inhibiting PD-L1, immune suppression is relieved and immune attack against HBV is enhanced.

Integrated synergistic effects within the nano-nucleic-acid therapeutic:

a. Enhanced immune response: The synergy of HBsAg, CD40, and PD-L1 enhances HBV-directed immune responses and facilitates viral clearance.

b. Relief of immune suppression: Inhibition of PD-L1 relieves immune suppression and enables immune cells to attack and clear HBV more effectively.

c. Promotion of immune-cell activation: Activation of CD40 enhances activation of B cells and T cells, thereby improving immune attack against HBV

In summary, through integrated synergy among multiple effector molecules, the specific-immunity triple-combination nano-nucleic-acid therapeutic agent can effectively treat hepatitis B virus (HBV) infection. The mechanisms of action are as follows:

1. Induction of HBV-specific immune responses: By targeting HBsAg, the immune system is induced to mount HBV-specific immune responses, facilitating viral clearance.

2. Promotion of immune-cell activation and enhancement of immune responses: Activation of CD40 promotes activation of B cells and T cells and enhances the immune system's attack against HBV.

3. Relief of immune suppression and enhancement of immune-cell viral clearance: Inhibition of PD-L1 relieves immune suppression and enables immune cells to attack and clear HBV more effectively.

Through the foregoing integrated synergy among multiple effector molecules, the therapeutic is expected to achieve functional cure of HBV infection. Of course, additional experimental and clinical research is required to verify the effectiveness and safety of this therapeutic strategy.

Drug 90): HBsAg Aptamer - CD40 Aptamer - PD-L1 Aptamer - DNA Carrier

This disclosure provides a specific-immunity triple-combination nano-nucleic-acid therapeutic agent; the mechanism is the same as above, except that a PD-L1 aptamer is employed (i.e., a PD-L1 aptamer is substituted).

Drug 91): BCMA Aptamer - CD38 Aptamer - CpG-BYZD - CD19 Aptamer - AmiR-21 - DNA Carrier

This disclosure relates to a specific active-immunity and gene-regulation quintuple-combination nano-nucleic-acid therapeutic agent. By means of a designer universal carrier-TY, multiple effector molecules are assembled via a single-stranded linker-bridge sequence, enabling modular assembly like Lego® bricks. For treatment of multiple myeloma (MM), anti-tumor effects are achieved through integrated synergy of multiple effector molecules. The functions of the respective effectors and the synergistic effects are as follows:

1. BCMA (B-cell maturation antigen): A tumor-associated antigen on multiple myeloma cell surfaces; targeting BCMA can effectively kill tumor cells.

2. CD38: Another important antigen on multiple myeloma cell surfaces. In combination with BCMA targeting, recognition and attack of myeloma cells by immune cells are enhanced.

3. CpG-BYZD: A CpG immunostimulant that can activate immune cells, such as dendritic cells (DCs), thereby enhancing immune responses against tumor cells.

4. CD19: An antigen on B-cell surfaces; by targeting CD19, myeloma cells as well as other malignant B cells can be killed.

5. AmiR-21: An anti-miR-21 inhibitor; by suppressing miR-21, growth and invasion of multiple myeloma cells can be reduced.

Through the above integrated synergy among multiple effector molecules, the therapeutic is expected to achieve significant anti-tumor effects in the treatment of multiple myeloma. Of course, the effectiveness and safety of this therapeutic strategy still require verification through additional experimental and clinical research.

Drug 92): BCMA Aptamer - CD38 Aptamer - CD19 Aptamer - AmiR-21 - DNA Carrier

This disclosure relates to a specific-immunity and gene-regulation quadruple-combination nano-nucleic-acid therapeutic agent for multiple myeloma (MM), in which anti-tumor effects are achieved via integrated synergy of multiple effector molecules. The functions of the respective effectors and the mechanisms by which the synergistic effects produce a targeted gene-combination immune response are as follows:

1. BCMA (B-cell maturation antigen): By targeting BCMA, tumor cells expressing this antigen can be effectively identified and killed. Such targeting enables the immune system to act specifically against myeloma cells and reduces damage to normal cells.

2. CD38: CD38 is another important antigen on MM cell surfaces. Through synergy with BCMA targeting, recognition and attack of myeloma cells by immune cells are enhanced, improving therapeutic effect.

3. CD19: CD19 is an antigen on B-cell surfaces. By targeting CD19, myeloma cells and other malignant B cells can be killed. In combination with BCMA and CD38 targeting, immune-therapy efficacy is further improved.

4. AmiR-21: Anti-miR-21 is an inhibitor directed to miR-21. By suppressing miR-21, growth and invasiveness of MM cells are reduced. It can also suppress metastasis and alleviate cancer pain in synergy with other targeted molecules, thereby helping to lower tumor burden and prevent recurrence.

Through the foregoing integrated synergy among multiple effector molecules, the therapeutic is expected to achieve significant anti-tumor effects in the treatment of MM. These synergistic effects help improve the efficacy of immune responses and thereby more effectively clear tumor cells. Of course, effectiveness and safety of this therapeutic strategy still require verification through additional experimental and clinical studies.

Drug 93): CpG2006 - CD38 Aptamer - PD-L1 Aptamer - DNA Carrier

This disclosure provides a specific active-immunity triple-combination nano-nucleic-acid therapeutic agent, with mechanisms as previously described; the nanoparticle is assembled using our shortened universal carrier TY.

Drug 94): BCMA Aptamer - CD38 Aptamer - CpG-BYZD - PD-L1 Aptamer - DNA Carrier

This disclosure provides a specific active-immunity quadruple-combination nano-nucleic-acid therapeutic agent, with mechanisms as previously described; the nanoparticle is assembled using our shortened universal carrier TY

Drug 95): BCMA Aptamer - CD40 Aptamer - PD-L1 Aptamer - DNA Carrier

This disclosure provides a specific-immunity triple-combination nano-nucleic-acid therapeutic agent, with mechanisms as previously described; the nanoparticle is assembled using our shortened universal carrier TY.

Drug 96): AmiR-21 - AS1411 Aptamer - CD40 Aptamer - 2 *Biotin - DNA Carrier

This disclosure relates to a specific gene-regulation and immunity dual-combination nano-nucleic-acid therapeutic agent. The mechanisms are as follows:

AmiR-21 (anti-miR-21): Anti-miR-21 suppresses expression of miR-21 within tumor cells, thereby reducing proliferative capacity, invasiveness, and anti-apoptotic properties, and exerting anti-tumor effects.

AS1411 aptamer: AS1411 is a nucleic-acid aptamer with high affinity for nucleolin; it binds nucleolin, which is broadly overexpressed on tumor cells. Because nucleolin expression is relatively low in normal cells, the AS1411 aptamer enables tumor-cell-specific targeting.

CD40 aptamer: CD40 is an immune-cell surface molecule that plays a key role in activation of anti-tumor immune responses. A CD40 aptamer can bind CD40 protein on tumor cells and on antigen-presenting cells (APCs) to provide targeted mediation and co-stimulatory immune activation, activating anti-tumor immune cells such as dendritic cells (DCs) and B cells and promoting their transition to more mature and activated states. In addition, by activating DCs and B cells, the CD40 aptamer promotes activation of anti-tumor T cells and thereby enhances immune attack on tumors.

Biotin: The active-mediation effect is achieved by modifying the 3' end of the nucleic-acid therapeutic with two biotin (Bio) molecules, which bind tumor cells that highly express biotin-receptor proteins to realize specific targeting. To support proliferative expansion, tumor cells highly express biotin receptors on their surfaces to recruit biotin as a nutrient. This active-mediation effect increases cellular uptake of the nano-nucleic-acid therapeutic in tumor cells.

In summary, by combining dual targeting/anchoring via the AS 1411 aptamer and biotin modification, higher tumor-targeting efficiency and anti-tumor activity are achieved. This strategy is expected to provide new treatment options in oncology, improving efficacy and reducing adverse effects. Through the synergy of anti-miR-21 and the CD40 aptamer, the therapeutic both directly suppresses tumor-cell growth and invasiveness and activates immune attack on tumors. Such combined action enables favorable inhibitory effects across multiple tumor types, including melanoma, colon cancer, breast cancer, and B-cell lymphoma. In addition, because the nano-nucleic-acid carrier exhibits high biocompatibility and stability, in-vivo bioavailability and anti-tumor activity of this specific gene-regulation plus immune-modulation dual-combination nano-nucleic-acid therapeutic are expected to be improved.

Drug 97): 2*AmiR-21 - 4*Biotin - DNA Carrier

This disclosure provides a specific gene-regulation nano-nucleic-acid therapeutic agent that achieves effective tumor inhibition through synergy between anti-miR-21 and biotin (Bio) molecules. The anti-tumor mechanisms are as follows:

1. Action of anti-miR-21 molecules: miR-21 is a microRNA highly expressed in many tumor types, with overexpression associated with tumorigenesis, progression, invasion, and metastasis. Two AmiR-21 molecules specifically bind miR-21 and suppress its biological function, thereby reducing tumor-cell proliferation, migration, and invasion and slowing tumor progression.

2. Action of biotin (Bio) molecules: Biotin is a vitamin widely present in biological systems; certain tumor cells overexpress biotin receptors on their surfaces. Four biotin moieties on the DNA carrier (4× Bio-DNA carrier) bind biotin receptors on tumor cells, further enhancing targeting of the nano-nucleic-acid therapeutic to tumor cells. Such targeting increases the intratumoral concentration of the therapeutic and augments anti-tumor efficacy.

In summary, this specific gene-regulation nano-nucleic-acid therapeutic agent achieves effective tumor suppression via dual-pathway synergy. For example, it exhibits favorable therapeutic effects in melanoma, colorectal cancer, breast cancer, and B-cell lymphoma, thereby providing a new strategy for cancer therapy.

Drug 98): TTA1 Aptamer - Epirubicin - 4× Biotin - DNA Carrier

This disclosure provides a specific gene-regulation nano-nucleic-acid and small-molecule chemotherapeutic combination therapeutic agent whose mechanisms include:
TTA1 aptamer (tenascin-C targeting): Tenascin-C is an extracellular-matrix glycoprotein highly expressed in most solid tumors. The TTA1 aptamer specifically binds tenascin-C, serving as a targeting "warhead" that mediates rapid uptake of the nano-nucleic-acid drug by tumor cells. Through TTA1-mediated active targeting, the drug acts more precisely on tumor cells, improving anti-tumor efficacy.
Epirubicin: The tetracyclic anthracycline framework of epirubicin intercalates specifically at GC/CG sites of the nano-nucleic-acid carrier. The amino group of the daunosamine moiety forms, under paraformaldehyde catalysis, a Schiff-base covalent linkage with the exocyclic amino group of guanine at a GC/CG site, thereby establishing a "physical intercalation + covalent linkage" stable-loading mode. After the TTA1 aptamer binds highly expressed tenascin-C on tumor-cell surfaces, the construct is internalized (e.g., via endocytosis) into the cytoplasm. Under intracellular conditions of low pH and high concentrations of the reducing agent glutathione, epirubicin dissociates from the nano-carrier; the released epirubicin enters the nucleus and intercalates specifically at GC/CG sites of nuclear double-stranded DNA, blocking transcription and thereby inhibiting tumor-cell differentiation and expansion to produce anti-tumor effects that suppress tumor development and progression.
Function of biotin (Bio) molecules: Biotin is widely present in vivo, and certain tumor cells overexpress biotin receptors. Four biotin moieties on the DNA carrier (4× Bio-DNA carrier) bind biotin receptors on tumor cells, further enhancing targeting of the nano-nucleic-acid therapeutic to tumor cells. Such targeting increases intratumoral drug concentration and augments anti-tumor efficacy.
In summary, this nano-nucleic-acid + chemotherapeutic combined therapeutic achieves higher and more specific anti-tumor effects through multi-molecule, multi-layer coordination.

Drug 99): TTA1 Aptamer - 2*Survivin siRNA - DNA Carrier

This disclosure provides a specific gene-regulation dual-combination nano-nucleic-acid therapeutic agent whose mechanisms include:

TTA1 aptamer (tenascin-C targeting): Tenascin-C is an extracellular-matrix glycoprotein highly expressed in most solid tumors. The TTA1 aptamer specifically binds tenascin-C, serving as a targeting "warhead" that mediates rapid uptake of the nano-nucleic-acid drug by tumor cells. Through active targeting, the agent acts more precisely on tumor cells and improves anti-tumor efficacy.

Survivin siRNA: Survivin is an inhibitor-of-apoptosis protein typically overexpressed in tumor cells and promotes tumor growth. Survivin-targeting siRNA ($2\times$) specifically binds and degrades survivin mRNA, thereby lowering survivin protein levels, inducing tumor-cell apoptosis, and inhibiting tumor-cell growth, invasion, and metastasis.

By integrating the TTA1 aptamer with survivin siRNA, the therapeutic specifically targets tumor cells while suppressing anti-apoptotic survivin within those cells, thereby inducing and promoting tumor-cell apoptosis and inhibiting growth, invasion, and metastasis to afford enhanced anti-tumor activity.

Drug 100): CD16a Aptamer - CD40 Aptamer - CpG2006 - DNA Carrier

This disclosure provides a specific active-immunity triple-combination nano-nucleic-acid therapeutic agent. The principal mechanisms of action include:

CD16a aptamer: CD16a (FcγRIIIa) is a receptor on immune-cell surfaces, predominantly on natural killer (NK) cells and macrophages. Engagement of CD 16a via a CD 16a-targeting aptamer induces activation of immune cells and thereby exerts anti-tumor effects. Accordingly, CD16a serves as an immunotherapy target that enhances tumor-cell specificity of the integrated nano-nucleic-acid active-combination immunotherapeutic.

CD40 aptamer: CD40 is an immune-cell surface molecule that plays a key role in activating anti-tumor immune responses. A CD40 aptamer binds CD40 protein on tumor cells and on antigen-presenting cells (APCs) to provide targeted mediation and co-stimulatory immune activation, activating anti-tumor immune cells such as dendritic cells (DCs) and B cells and promoting their transition to more mature and activated states. In addition, by activating DCs and B cells, the CD40 aptamer promotes activation of anti-tumor T cells and thereby enhances immune attack against tumors.

CpG2006: CpG2006 is a CpG oligonucleotide analog that activates the Toll-like receptor 9 (TLR9) signaling pathway upon binding, stimulating antiviral and anti-tumor immune responses. TLR9 is mainly expressed on plasmacytoid dendritic cells (pDCs) and B lymphocytes. Activation of TLR9 triggers immune cells to release large amounts of interferon-a (IFN-$\alpha$) and other cytokines, thereby enhancing immune surveillance and clearance of tumor cells.

Integration of the foregoing three effectors on a DNA carrier affords synergistic enhancement in oncology applications. The synergy arises from functional complementarity among the aptamers/agonist and, via joint activation of the immune system together with recruitment of NK cells and other pathways, collectively achieves effective tumor suppression. This integrated therapeutic strategy is expected to provide patients with more effective and safer treatment options and to improve survival and quality of life.

From the above examples of drug compositions and mechanisms of action, it can be seen that the nucleic-acid drugs of the present application-using nucleic-acid nanoparticles as an integrated drug-delivery carrier platform-can adopt a multi-layer, multi-agent "integrated" treatment strategy, combining systemic immunotherapy with local targeting within a single drug. Therefore, in terms of design complexity, developability, manufacturing difficulty, and pharmacodynamic performance, they offer advantages unmatched by other modalities (e.g., antibody-drug conjugates (ADCs), liposome-delivered nucleic-acid drugs, or polypeptide-nanoparticle delivery).

Moreover, from the compositions and mechanisms of multiple specific oligonucleotide drugs described above, it is apparent that most drugs of the present application are formed by loading different oligonucleotide effector molecules onto a carrier, and regardless of how such loading and drug-design combinations are implemented, the manufacturing process is essentially consistent. This integrated advantage extends beyond the action scope and applicable tumor types of single-oligonucleotide drugs and enables combination of oligonucleotide effectors with different therapeutic mechanisms, making the drugs highly suitable for pan-cancer advantages (see the Examples section). Many of these drugs encompass active stimulation of the immune system (e.g., CpG stimulators) and/or immune-cell activation (e.g., CD40-targeting aptamers) and/or relief of immunosuppression in the tumor microenvironment (e.g., PD-1 aptamers and/or PD-L1 aptamers). Specifically, as with CCPD3 and its variant drugs, the approach focuses on APCs and modulates three key switches-TLR9, CD40, and PD-L1-while simultaneously activating T cells to actively engage endogenous immunity.

According to a third aspect of the present application, there is provided a pharmaceutical composition comprising any of the above nucleic-acid drugs and, optionally, a pharmaceutically acceptable carrier and/or excipient.

The above pharmaceutically acceptable excipients may vary depending on the desired dosage form and/or route of administration, and may be reasonably selected from existing pharmaceutical excipients, including but not limited to pharmaceutically acceptable carriers, excipients, or adjuvants. The drug may be formulated in different dosage forms to accommodate various routes of administration, including oral, injectable, or transdermal administration. Dosage forms include, without limitation, capsules, tablets, oral solutions, injectables, or transdermal preparations.

Specifically, according to methods known in the pharmaceutical industry, the drugs of the present application may be formulated as capsules, tablets, oral solutions, injectables, or transdermal preparations. Preferably, the injectable is an intravenous injection. In preparing capsules, tablets, or oral solutions suitable for oral administration, sucrose, lactose, galactose, corn starch, gelatin, microcrystalline cellulose, and carboxymethyl cellulose may be used as carriers or excipients. In addition, using methods and auxiliary components known in the pharmaceutical industry, the drugs of the present application may be formulated as solutions and suspensions suitable for oral administration. When preparing solutions and suspensions suitable for parenteral (extragastrointestinal) administration, distilled water, water for injection, isotonic sodium chloride solution, glucose solution, or low-concentration phosphate-buffered saline (PBS; e.g., 1-100 mM) may be used as carriers or diluents. One or more additional auxiliary components or additives may be incorporated into such parenteral formulations; for example, ascorbic acid may be used as an antioxidant and sodium benzoate as a preservative. These dosage forms may further contain other solubilizers, disintegrants, colorants, dispersants, or surfactants.

In some preferred embodiments, the above pharmaceutical composition further includes any one or more of the foregoing nucleic-acid drugs. In other preferred embodiments, the pharmaceutical composition includes at least one of the foregoing nucleic-acid drugs. Certain pharmaceutical compositions are selected from any one or more of the following groups:

Combination 1): 2*CpG1826 - mannose - DNA carrier + 2*AmiR-21 - AS1411 aptamer - DNA carrier; i.e., the aforementioned Drug 2 + Drug 21;

Combination 2): 2*CpG1826 - CD40 aptamer - 2*mannose - DNA carrier + AmiR-21 - AS1411 aptamer - A15 aptamer - 2*biotin - DNA carrier; i.e., the aforementioned Drug 6 + Drug 26;

Combination 3): 2*CpG1826 - CD40 aptamer - 2*mannose - DNA carrier + OX40 (RNA/OMe); i.e., the aforementioned Drug 6 + the OX40 aptamer in RNA/OMe form;

Combination 4): 2*CpG1826 - CD40 aptamer - 2*mannose - DNA carrier + AmiR-21 - AS1411 aptamer - A15 aptamer - 2*biotin - DNA; i.e., the drugs listed above as Drug 3 + Drug 26;

Combination 5): 2*CpG1826 - CD40 aptamer - DNA carrier + 2*AmiR-21 - AS1411 aptamer - DNA carrier + OX40; i.e., the drugs listed above as Drug 3 + Drug 21 + the OX40 aptamer in DNA form;

Combination 6): 2*CpG2006 - CD40 aptamer - DNA carrier + 2*AmiR-21 - AS1411 aptamer - DNA carrier + OX40; i.e., the drugs listed above as Drug 4 + Drug 21 + the OX40 aptamer in DNA form;

Combination 7): 2*CpG2006 - CD40 aptamer - DNA carrier + 2*AmiR-21 - AS1411 aptamer - DNA carrier; i.e., the drugs listed above as Drug 4 + Drug 21;

Combination 8): 2*CpG1826 - CD40 aptamer - DNA carrier + 2*AmiR-21 - AS1411 aptamer - biotin - DNA carrier; i.e., the drugs listed above as Drug 3 + Drug 23;

Combination 9): 2*CpG2006 - CD40 aptamer - DNA carrier + 2*AmiR-21 - AS1411 aptamer - biotin - DNA carrier; i.e., the drugs listed above as Drug 4 + Drug 23;

Combination 10): 2*CpG1826 - CD40 aptamer - DNA carrier + 2*AmiR-21 - CD40 aptamer - DNA carrier + OX40; i.e., the aforementioned Drug 3 + Drug 24 + the OX40 aptamer in DNA form;

Combination 11): 2× CpG1826 - CD40 aptamer - DNA carrier + 3× AmiR-21 - AS1411 aptamer - CD40 aptamer - 2× biotin - DNA carrier; i.e., the aforementioned Drug 3 + Drug 41;

Combination 12): 4*CpG2006 - CD40 aptamer - DNA carrier + 3*AmiR-21 - AS1411 aptamer - CD40 aptamer - 2*biotin - DNA carrier; i.e., the drugs listed above as Drug 5 + Drug 41;

Combination 13): 4*CpG2006 - CD40 aptamer - DNA carrier + AmiR-21 - AS1411 aptamer - CD40 aptamer - 2*biotin - DNA carrier; i.e., the aforementioned Drug 5 + Drug 96;

Combination 14): Rac1 aptamer - Survivin siRNA - 2*biotin - 5TR1-R aptamer - DNA carrier + AmiR-21 - AS1411 aptamer - CD40 aptamer - 2*biotin - DNA carrier; i.e., the aforementioned Drug 96 + Drug 41;

Combination 15): 2*CpG1826 - CD40 aptamer - DNA carrier + 2*AmiR-21 - 4*biotin - DNA carrier + OX40; i.e., the aforementioned Drug 3 + Drug 97 + the OX40 aptamer in DNA form;

Combination 16): 2*CpG2006 - CD40 aptamer - DNA carrier + 2*AmiR-21 - 4*biotin - DNA carrier + OX40; i.e., the aforementioned Drug 4 + Drug 20 + the OX40 aptamer in DNA form;

Combination 17): 2*CpG2006 - CD40 aptamer - DNA carrier + 2*AmiR-21 - 4*biotin - DNA carrier; i.e., the aforementioned Drug 4 + Drug 20;

Combination 18): CpG1826 - CD40 aptamer - mannose - MUC1 aptamer - DNA carrier + 2*AmiR-21 - MUC1 aptamer - 3*biotin - DNA carrier + OX40; i.e., the aforementioned Drug 7 + Drug 25 + the OX40 aptamer in DNA form;

Combination 19): 3*CpG2006 - DNA carrier + 2*AmiR-21 - TTA1 aptamer - DNA carrier + TTA1 aptamer - epirubicin - 4*biotin - DNA carrier; i.e., the aforementioned Drug 1 + Drug 18 + Drug 98;

Combination 20): 3*CpG2006 - DNA carrier + 2*AmiR-21 - TTA1 aptamer - DNA carrier + OX40 (DNA form) + TTA1 aptamer - 2*Survivin siRNA - DNA carrier; i.e., the aforementioned Drug 1 + Drug 18 + the OX40 aptamer in DNA form + Drug 99;

Combination 21): 2*CpG1826 - CD40 aptamer - DNA carrier + 2*AmiR-21 - TTA1 aptamer - DNA carrier + OX40 (DNA); i.e., the aforementioned Drug 3 + Drug 18 + the OX40 aptamer in DNA form;

Combination 22): 2*CpG1826 - CD40 aptamer - DNA carrier + 2*AmiR-21 - TTA1 aptamer - DNA carrier; i.e., the aforementioned Drug 3 + Drug 18;

Combination 23): 2*CpG1826 - CD40 aptamer - DNA carrier + OX40 (DNA); i.e., the aforementioned Drug 3 + the OX40 aptamer in DNA form;

Combination 24): 2*CpG1826 - CD40 aptamer - DNA carrier + 2*AmiR-21 - TTA1 aptamer - DNA carrier + OX40 (locked nucleic acid); i.e., the aforementioned Drug 3 + Drug 18 + the OX40 aptamer in locked-nucleic-acid (LNA) form;

Combination 25): 2*CpG1826 - CD40 aptamer - DNA carrier + 2*AmiR-21 - TTA1 aptamer - DNA carrier + OX40 (RNA/OMe); i.e., the aforementioned Drug 3 + Drug 18 + the OX40 aptamer in RNA/OMe form;

Combination 26): 2*CpG1826 - CD40 aptamer - DNA carrier + 2*AmiR-21 - TTA1 aptamer - DNA carrier + 3*CD40; i.e., the aforementioned Drug 3 + Drug 18 + a CD40 aptamer;

Combination 27): 2*CpG1826 - CD40 aptamer - DNA carrier + 2*AmiR-21 - 4*biotin - DNA carrier + OX40 (DNA); i.e., the aforementioned Drug 3 + Drug 20 + a CD40 aptamer in DNA form;

Combination 28): 2*CpG2006 - CD40 aptamer - DNA carrier + 2*AmiR-21 - 3*biotin - DNA carrier + OX40 (DNA); i.e., the aforementioned Drug 4 + Drug 19 + a CD40 aptamer in DNA form;

The sequence of the above OX40 aptamer in DNA form: SEQ ID No. 227: CAGTCTGCATCGTAGGATTAGCCACCGUATCTTTCCCAC;

The sequence of the above OX40 aptamer in RNA form:

SEQ ID No. 226:
GGGAGGACGAUGCGGCAGUCUGCAUCGUAGGAAUCGCCACCGUAUACUUUCCCA
CCAGACGACUCGCUGAGGAUCCGAGA;

The OX40 aptamer in locked-nucleic-acid (LNA) form is defined such that, within the sequence of the OX40 aptamer in DNA form, the first five nucleotides at the 5' end and the first four nucleotides at the 3' end are LNA modified.

The above pharmaceutical compositions respectively employ a nucleic-acid carrier to carry different effector molecules and to exert corresponding pharmacological effects in combination form. In the present application, through various cell experiments and/or animal experiments, it has been confirmed that nucleic-acid drugs carried by the nucleic-acid carrier exhibit excellent efficacy in inhibiting tumor proliferation, in treating hepatitis B and hepatitis C, and in inhibiting or treating diseases caused by SARS-CoV-2 (see the Examples section for details).

In a fourth typical embodiment of the present application, there is provided the use of any of the above nucleic-acid carriers, nucleic-acid drugs, or pharmaceutical compositions in the preparation of a medicament for treating tumors, diseases caused by coronavirus infection, or liver diseases other than liver cancer. Under such use, the drug exhibits targeting, high delivery stability of the nucleic-acid drug, high efficacy, and low toxicity/adverse effects.

In the foregoing use, different oligonucleotide drugs may be designed depending on the tumor type or disease type; accordingly, the scope of application is very broad. The liver diseases other than liver cancer include hepatitis B or hepatitis C; the diseases caused by coronavirus infection include COVID-19; the tumors include, but are not limited to, any one or more of the following: male tumors, gynecologic tumors, respiratory-system tumors, digestive-system tumors, hematologic tumors, urinary-system tumors, bone tumors, neurologic tumors, dermatologic tumors, general-surgery tumors, or otorhinolaryngology/ophthalmology tumors. Preferably, male tumors are prostate cancer, penile cancer, testicular tumor, or male urethral cancer; gynecologic tumors are ovarian cancer, cervical cancer, endometrial carcinoma, uterine fibroid, vulvar cancer, or malignant hydatidiform mole; respiratory-system tumors are lung cancer, non-small-cell lung cancer, small-cell lung cancer, nasopharyngeal carcinoma, tracheal tumor, lung-cancer metastasis, pulmonary inflammatory pseudotumor, or radiation-induced lung cancer; digestive-system tumors are liver cancer, gastric cancer, large-intestine cancer, gallbladder cancer, esophageal cancer, rectal cancer, pancreatic cancer, or colon cancer; hematologic tumors are leukemia, lymphoma, lymphosarcoma, or multiple myeloma; urinary-system tumors are renal cancer, bladder cancer, or urological cancer; bone tumors are giant-cell tumor of bone, osteochondroma, or osteosarcoma; neurologic tumors are brain tumor, meningioma, intracranial tuberculoma, pituitary tumor, neuroblastoma, glioblastoma, or glioma; dermatologic tumors are skin cancer or melanoma; general-surgery tumors are breast cancer, lipoma, thyroid cancer, or thyroid tumor; and otorhinolaryngology/ophthalmology tumors are oral cancer, tongue cancer, laryngeal cancer, middle-ear cancer, gingival cancer, or intraorbital tumor.

For drug research and development or treatment of the foregoing tumors, the routes of administration during use include intratumoral administration, intravenous administration, or intraperitoneal administration. Each route has its respective advantages; an appropriate route may be selected comprehensively in view of the specific dosage form, mechanism of action, pharmacokinetics, and other factors of the drug.

For different tumors, the daily dose of the foregoing drugs also varies. As used herein, "dose" includes a therapeutically effective dose or a prophylactically effective dose. A "therapeutically effective dose" refers to an amount effective to achieve the desired therapeutic result at the necessary dose and for a specified duration. The therapeutically effective amount of an agent may vary depending on various factors, such as the subject's condition, age, sex, and body weight, as well as the agent's ability to elicit the desired response in the subject. A therapeutically effective amount is also an amount at which the therapeutic benefit exceeds any toxic or harmful effects of the agent. A "prophylactically effective dose" refers to an amount effective to achieve the desired prophylactic result at the necessary dose and for a specified time period, such as preventing or inhibiting elevation of urinary protein. The prophylactically effective amount may be determined in accordance with the foregoing description of the therapeutically effective amount. For any particular subject, the specific dose may be adjusted over time according to individual needs and the practitioner's professional judgment. Excipients may be selected from existing excipients appropriate to the dosage form or route of administration.

In one preferred embodiment of the invention, on a body-weight basis in experimental animals, a daily dose ranging from 0.1 μg/kg up to 100 mg/kg is safe; in specific cases, an actual effective dose range for a subject (e.g., a cancer patient) can be derived according to the conversion relationship (e.g., allometric conversion) between animal and human body weight and dosing.

In a fifth typical embodiment of the present application, there is further provided a method for preparing a nucleic-acid drug, comprising the steps of: obtaining the nucleic-acid drug by self-assembly of at least one strand from a nucleic-acid carrier containing sequences a, b, c and at least one oligonucleotide effector molecule loaded on the nucleic-acid carrier; wherein the oligonucleotide effector molecule is selected from at least one of: a nucleic-acid immunostimulant, a nucleic-acid aptamer, siRNA, miRNA, and ASO.

In the nucleic-acid drugs of the present application, both the carrier and the substances loaded thereon are nucleic-acid molecules, and therefore the drug can be obtained by self-assembly through synthesis of one or multiple strands according to the sequence composition. In specific synthesis, in view of formation and stability of the nucleic-acid drug structure, one or more spacer sequences or linker sequences (linkers) may be added between the sequences of the respective oligonucleotide effector molecules and the nucleic-acid carrier as needed. Preparation of the nucleic-acid drug by synthetic means is relatively simple and efficient and greatly reduces manufacturing cost.

To further improve synthetic convenience and efficiency and reduce synthesis cost, in preferred embodiments, the foregoing nucleic-acid drug is obtained by self-assembly of 2-8 strands, preferably 3-6 strands. As the length of a synthesized fragment increases, synthetic difficulty tends to increase and synthetic efficiency tends to decrease; separate synthesis of multiple segments corresponding to the sequences involved in each nucleic-acid drug helps improve synthetic efficiency and reduce synthesis cost.

When self-assembly involves more than three strands, the preparation method includes: providing, for self-assembly to form the nucleic-acid carrier, modified or unmodified sequences including the sequence a, the sequence b, and the sequence c (where "modified" refers to modification with a targeting small chemical molecule); and, by sequence complementarity, loading a related sequence of at least one oligonucleotide effector molecule onto the 5' end and/or 3' end of at least one of the sequence a, sequence b, and sequence c of the nucleic-acid carrier; wherein the oligonucleotide effector molecule is selected from at least one of: a nucleic-acid immunostimulant, a nucleic-acid aptamer, siRNA, miRNA, and ASO.

In the preparation method for the nucleic-acid drugs of the present application, a nucleic-acid carrier incorporating targeting molecules (small-molecule compounds and/or nucleic-acid aptamers) is employed to load oligonucleotide molecules, such that the resulting nucleic-acid drug concurrently exhibits targeting capability, high delivery stability, and high delivery efficiency.

In the foregoing method, when the types of oligonucleotide effector molecules loaded on the nucleic-acid carrier differ, the specific steps are correspondingly varied. For example, in certain embodiments where the loaded oligonucleotide effector molecule includes siRNA, the sense strand of the siRNA is synthesized at the 5' end and/or 3' end of at least one of the modified or unmodified sequences a, b, and c, and the antisense strand of the siRNA is co-assembled together with the modified or unmodified sequences a, b, and c, whereby the siRNA is connected to the nucleic-acid carrier through complementary pairing between the siRNA antisense and sense strands; preferably, the siRNA sense strand is directly synthesized via a spacer sequence at the 5' end and/or 3' end of at least one of the sequences a, b, and c; preferably, the spacer sequence is selected from 2-8, preferably 3-6, consecutive T or A nucleotides.

In certain preferred embodiments in which the oligonucleotide effector molecules include miRNA and/or a nucleic-acid immunostimulant, the miRNA is directly synthesized and/or the nucleic-acid immunostimulant is synthesized via a spacer sequence at the 5' end and/or 3' end of at least one of the modified or unmodified sequences a, b, and c, and the miRNA and/or the nucleic-acid immunostimulant is loaded onto the nucleic-acid carrier by self-assembly of the modified or unmodified sequences a, b, and c; preferably, the spacer sequence is selected from 2-8, preferably 3-6, consecutive T or A nucleotides.

In certain preferred embodiments in which the oligonucleotide effector molecules include a nucleic-acid aptamer, the nucleic-acid aptamer is synthesized at the 5' end and/or 3' end of at least one of the modified or unmodified sequences a, b, and c, and-by self-assembly of the modified or unmodified sequences a, b, and c-miRNA is loaded onto the nucleic-acid carrier; preferably, the nucleic-acid aptamer is directly synthesized via a spacer sequence at the 5' end and/or 3' end of at least one of the sequences a, b, and c; preferably, the spacer sequence is selected from 2-8, preferably 3-6, consecutive T or A nucleotides.

In certain preferred embodiments where the oligonucleotide effector molecules further include a nucleic-acid immunostimulant and/or a nucleic-acid aptamer, the nucleic-acid immunostimulant is synthesized via a spacer sequence at the 5' end and/or 3' end of at least one of the modified or unmodified sequences a, b, and c, at an end different from the end used for the siRNA sense strand; and/or the nucleic-acid aptamer is synthesized via a spacer sequence at the 5' end of the siRNA antisense strand to obtain an aptamer-siRNA fragment; the aptamer-siRNA fragment is co-incubated with the modified or unmodified sequences a, b, and c bearing the synthesized nucleic-acid immunostimulant for self-assembly, thereby loading the nucleic-acid aptamer onto the nucleic-acid carrier through complementary pairing of the siRNA antisense and sense strands.

In certain preferred embodiments in which the oligonucleotide effector molecules include miRNA and a nucleic-acid aptamer, the nucleic-acid aptamer is synthesized at the 3' end of the miRNA to obtain an aptamer-miRNA fusion single-stranded sequence; the aptamer-miRNA fusion single-stranded sequence is then subjected to self-assembly, thereby loading the nucleic-acid aptamer and the miRNA onto the nucleic-acid carrier.

In certain preferred embodiments in which the oligonucleotide effector molecules include a nucleic-acid immunostimulant and a nucleic-acid aptamer, the nucleic-acid immunostimulant and the nucleic-acid aptamer are independently synthesized at either end of at least one of the modified or unmodified sequences a, b, and c to obtain fusion sequences of the nucleic-acid immunostimulant and/or the nucleic-acid aptamer; the fusion sequences of the nucleic-acid immunostimulant and/or the nucleic-acid aptamer are then subjected to self-assembly, thereby loading the nucleic-acid aptamer and the nucleic-acid immunostimulant onto the nucleic-acid carrier.

In the preferred embodiments for the different situations described above, the specific loading modalities for different oligonucleotide effector molecules are illustrated. Briefly, depending on its particular biological function, a nucleic-acid aptamer may serve solely as a targeting moiety, or may possess both targeting function and drug-like activity (e.g.,

antibody-like activity). A nucleic-acid aptamer may be directly synthesized at the end of a single-stranded sequence that forms the nucleic-acid carrier (e.g., the sequences a, b, or c) via a spacer sequence, such as TTTTT or AAA; it may alternatively be synthesized via an miRNA serving as a spacer sequence (i.e., covalently linked by a chemical bond) at the end of the sequences a, b, or c; in addition, it may be "adsorbed" (physically associated) at the end of the sequences a, b, or c through complementary pairing between a single-stranded linker-bridge sequence and its complementary sequence, or through complementary pairing of the siRNA sense and antisense strands (i.e., by synthesizing, at the 5' end of the complementary sequence of the single-stranded linker-bridge or at the 5' end of the siRNA antisense strand, so as to realize "adsorption" on the nucleic-acid carrier).

In the foregoing preparation method, a targeting small chemical molecule may be present or absent depending on the type of oligonucleotide effector molecule to be loaded; because it is a small molecule, it can be introduced directly as a modifying group during synthesis of the sequences a, b, or c.

To further facilitate synthesis and reduce the cost of synthesizing long oligonucleotides, in certain preferred embodiments the nucleic-acid carrier adopts the aforementioned universal carrier DNA3-TY. This carrier has a single-stranded linker-bridge sequence at the 3' end of the sequences a, b, or c; other oligonucleotide drugs to be loaded (excluding siRNA) may, during separate synthesis, be provided with a complementary sequence to the single-stranded linker-bridge at their 3' end, so that, upon mixing with the sequences a, b, or c, self-assembly occurs and loading of the oligonucleotide drug is realized concurrently with carrier self-assembly. In the universal carrier DNA3-TY, each of the sequences a, b, and c include a carrier backbone sequence and a single-stranded linker-bridge sequence; the oligonucleotide effector molecules are respectively self-assembled with the sequences a, b, and c via the complementary sequence to the single-stranded linker-bridge sequence to form the nucleic-acid drug, wherein the oligonucleotide effector molecule is selected from at least one of: a nucleic-acid immunostimulant, a nucleic-acid aptamer, miRNA, and ASO; wherein the sequence a, sequence b, and sequence c are respectively as follows:

sequence a: SEQ ID No. 15: GACGCCCACGAGCGTTCCGGGAGAGG<u>TGTAGCACGGTGGC,</u> in which the underlined portion corresponds to single-stranded linker-bridge sequence A;

sequence b: SEQ ID No. 16: CCTCTCCCGGTTCGCCGCGAGCC<u>TCGGCGCGGCCGTG,</u> in which the underlined portion corresponds to single-stranded linker-bridge sequence B;

sequence c: SEQ ID No. 17: GGCTCGCGGCCATAGCCGTGGGCGTC<u>TGCTGCTGCTGCTG,</u> in which the underlined portion corresponds to single-stranded linker-bridge sequence C.

The following uses the nucleic-acid drug CpG2006 - CD40 aptamer - PD-L1 aptamer - DNA carrier as an example to explain, in detail, multiple possible self-assembly modes of the preparation method.

1) Three-strand self-assembly, wherein the three strands:

CpG2006-sequence a:
SEQ ID No. 309: TCGTCGTTTTGTCGTTTTGTCGTT**TTTTT**GCGACGCCCACGAGCGTTCCGGGAGAGGAG C, wherein phosphorothioate linkages are present between adjacent nucleotides at positions 1-24 from the 5' end; TTTTT serves as a spacer sequence; the region preceding TTTTT is the CpG2006 sequence, and the region following TTTTT is the sequence a;

CD40 aptamer-sequence b:
SEQ ID No. 330: CCAACGAGTAGGCGATAGCGCGTGG**TTTTT**GCTCCTCTCCCGGTTCGCCGCGAGCCG CG, wherein phosphorothioate linkages are present between adjacent nucleotides at positions 1-5 from the 5' end; TTTTT serves as a spacer sequence; the region preceding TTTTT is the CD40 aptamer sequence, and the region following TTTTT is the sequence b;

PD-L1 aptamer-sequence c:
SEQ ID No. 331: ACGGGCCACATCAACTCATTGATAGACAATGCGTCCACTGCCCGT**TTTTT**CGCGGCTC GC GGCCATAGCCGTGGGCGTCGC, wherein phosphorothioate linkages are present between adjacent nucleotides at positions 1-5 from the 5' end; TTTTT serves as a spacer sequence; the region preceding TTTTT is the PD-L1 aptamer sequence, and the region following TTTTT is the sequence c;

2) Four-strand self-assembly, wherein the four strands :

CpG2006-sequence a:

SEQ ID No. 309: TCGTCGTTTTGTCGTTTTGTCGTT**TTTTT**GCGACGCCCACGAGCGTTCCGGGAGAGGAG C, wherein phosphorothioate linkages are present between adjacent nucleotides at positions 1-24 from the 5' end; TTTTT serves as a spacer sequence; the region preceding TTTTT is the CpG2006 sequence, and the region following TTTTT is the sequence a;

CD40 aptamer-sequence b:
SEQ ID No. 339: CCAACGAGTAGGCGATAGCGCGTGG**TTT**GCTCCTCTCCCGGTTCGCCGCGAGCCTCG GC GCGGCCGTG, wherein phosphorothioate linkages are present between adjacent nucleotides at positions 1-4 from the 5' end; TTT serves as a spacer sequence; the region preceding TTT is the CD40 aptamer sequence, and the region following TTT is the sequence b;

PD-L1 aptamer - spacer sequence - complementary sequence B' of the single-stranded linker-bridge sequence B:
SEQ ID No. 336: ACGGGCCACATCAACTCATTGATAGACAATGCGTCCACTGCCCGT**TTTTT**CACGGCCG CG CCGA, wherein phosphorothioate linkages are present between adjacent nucleotides at positions 1-4 from the 5' end; TTTTT is a spacer sequence; the region preceding TTTTT is the PD-L1 aptamer sequence, and the region following TTTTT is the complementary sequence B';

sequence c:
SEQ ID No. 340: GGCTCGCGGCCATAGCCGTGGGCGTCGC;
Or,

The four strands are, respectively:

CpG2006 - spacer sequence - complementary sequence C' of the single-stranded linker-bridge sequence C:
SEQ ID No. 341: TCGTCGTTTTGTCGTTTTGTCGTTT**TTTTT**CAGCAGCAGCAGCA, wherein phosphorothioate linkages are present between adjacent nucleotides at positions 1-24 from the 5' end; TTTTT is a spacer sequence; the region preceding TTTTT is the CpG2006 sequence, and the region following TTTTT is the complementary sequence C';

sequence a: SEQ ID No. 18: GCGACGCCCACGAGCGTTCCGGGAGAGGAGC;

CD40 aptamer - sequence b:
SEQ ID No. 339: C*C*A*ACGAGTAGGCGATAGCGCGTGGTTTGCTCCTCTCCCGGTTCGCCGC GAGCCTCG GCGCGGCCGTG, wherein phosphorothioate linkages are present between adjacent nucleotides at positions 1-4 from the 5' end; TTT is a spacer sequence; the region preceding TTT is the CD40 aptamer sequence, and the region following TTT is the sequence b;

PD-L1 aptamer - sequence c:
SEQ ID No. 342: ACGGGCCACATCAACTCATTGATAGACAATGCGTCCACTGCCCGT**TTTTT**GGCTCG CGGC CATAGCCGTGGGCGTCGC, wherein phosphorothioate linkages are present between adjacent nucleotides at positions 1-4 from the 5' end; TTTTT is a spacer sequence; the region preceding TTTTT is the PD-L1 aptamer sequence, and the region following TTTTT is the sequence c;

3) Five-strand self-assembly, wherein the five strands:

sequence a: SEQ ID No. 15: GACGCCCACGAGCGTTCCGGGAGAGGTGTAGCACGGTGGC;

CD40 aptamer - spacer sequence - sequence b:
SEQ ID No. 339: C*C*A*ACGAGTAGGCGATAGCGCGTGG**TTT**GCTCCTCTCCCGGTTCGCCGC GAGCCTCG GCGCGGCCGTG, wherein phosphorothioate linkages are present between adjacent nucleotides at positions 1-4 from the 5' end; TTT is a spacer sequence; the region preceding TTT is the CD40 aptamer sequence, and the region following TTT is the sequence b;

PD-L1 aptamer - spacer sequence - complementary sequence B' of the single-stranded linker-bridge sequence B:
SEQ ID No. 336: ACGGGCCACATCAACTCATTGATAGACAATGCGTCCACTGCCCGT**TTTTT**CACGGCCG CG CCGA, wherein phosphorothioate linkages are present between adjacent nucleotides at positions 1-4 from the 5' end; TTTTT is a spacer sequence; the region preceding TTTTT is the PD-L1 aptamer sequence, and the region following TTTTT is the complementary sequence B';

CpG2006 sequence - spacer sequence - complementary sequence C' of the single-stranded linker-bridge sequence C:
SEQ ID No. 337: TCGTCGTTTTGTCGTTTTGTCGTT**TTTTT**CAGCAGCAGCAGCA, wherein phosphorothioate linkages are present between adjacent nucleotides at positions 1-24 from the 5' end; TTTTT is a spacer sequence; the region preceding TTTTT is the CpG2006 sequence, and the region following TTTTT is the complementary sequence C';

sequence c: SEQ ID No. 17:
GGCTCGCGGCCATAGCCGTGGGCGTCTGCTGCTGCTGCTG;

or the five strands are:

CpG2006 - spacer sequence - sequence a:
SEQ ID No. 343: TCGTCGTTTTGTCGTTTTGTCGTT**TTTTT**GACGCCCACGAGCGTTCCGGGAGAGG TGTAG CACGGTGGC, wherein phosphorothioate linkages are present between adjacent nucleotides at positions 1-24 from the 5' end; TTTTT is a spacer sequence; the region preceding TTTTT is the CpG2006 sequence, and the region following TTTTT is the sequence a;

CD40 aptamer - spacer sequence - complementary sequence A' of the single-stranded linker-bridge sequence A:
SEQ ID No. 338: GCCAACGAGTAGGCGATAGCGCGTGGC**TTTTT**GCCACCGTGCTACA, wherein phosphorothioate linkages are present in the phosphate backbone between adjacent nucleotides at positions 1-4 from the 5' end; TTTTT is a spacer sequence; the region preceding TTTTT is the CD40 aptamer sequence, and the region following TTTTT is the complementary sequence A';

sequence b: SEQ ID No. 16: CCTCTCCCGGTTCGCCGCGAGCCTCGGCGCGGCCGTG;

PD-L1 aptamer - spacer sequence - complementary sequence B' of the single-stranded linker-bridge sequence B:
SEQ ID No. 336: ACGGGCCACATCAACTCATTGATAGACAATGCGTCCACTGCCCGT**TTTTT**CACGGC CGCG CCGA, wherein phosphorothioate linkages are present in the phosphate backbone between adjacent nucleotides at positions 1-4 from the 5' end; TTTTT is a spacer sequence; the region preceding TTTTT is the PD-L1 aptamer sequence, and the region following TTTTT is the complementary sequence B';

sequence c: SEQ ID No. 340: GGCTCGCGGCCATAGCCGTGGGCGTCGC;

4) Six-strand self-assembly, wherein the six strands are, respectively:

sequence a: SEQ ID No. 15: GACGCCCACGAGCGTTCCGGGAGAGGTGTAGCACGGTGGC;

sequence b: SEQ ID No. 16: CCTCTCCCGGTTCGCCGCGAGCCTCGGCGCGGCCGTG;

sequence c: SEQ ID No. 17: GGCTCGCGGCCATAGCCGTGGGCGTCTGCTGCTGCTGCTG;

CpG2006 - spacer sequence - complementary sequence C' of the single-stranded linker-bridge sequence C:
SEQ ID No. 337: TCGTCGTTTTGTCGTTTTGTCGTT**TTTTT**CAGCAGCAGCAGCA, wherein phosphorothioate linkages are present in the phosphate backbone between adjacent nucleotides at positions 1-24 from the 5' end; TTTTT is a spacer sequence; the region preceding TTTTT is the CpG2006 sequence, and the region following TTTTT is the complementary sequence C';

CD40 aptamer - spacer sequence - complementary sequence A' of the single-stranded linker-bridge sequence A:
SEQ ID No. 338: GCCAACGAGTAGGCGATAGCGCGTGGC**TTTTT**GCCACCGTGCTACA, wherein phosphorothioate linkages are present in the phosphate backbone between adjacent nucleotides at positions 1-4 from the 5' end; TTTTT is a spacer sequence; the region preceding TTTTT is the CD40 aptamer sequence, and the region following TTTTT is the complementary sequence A';

PD-L1 aptamer - spacer sequence - complementary sequence B' of the single-stranded linker-bridge sequence B:

SEQ ID No. 336: ACGGGCCACATCAACTCATTGATAGACAATGCGTCCACTGCCCGT**TTTTT**CACGGCCG CG CCGA, wherein phosphorothioate linkages are present in the phosphate backbone between adjacent nucleotides at positions 1-4 from the 5' end; TTTTT is a spacer sequence; the region preceding TTTTT is the PD-L1 aptamer sequence, and the region following TTTTT is the complementary sequence B'.

In the above preparation method, the self-assembly step is similar to the self-assembly process of the nucleic-acid carrier sequences a, b, or c, except that-when loading via complementary pairing is required-the oligonucleotide effector molecule to be complementarily paired is placed as a separate sequence together with the sequences a, b, or c in the self-assembly system for self-assembly. In one preferred embodiment, the self-assembly step includes: dissolving the modified or unmodified sequences a, b, and c bearing at least one oligonucleotide effector molecule, and optionally a separately synthesized sequence of an oligonucleotide effector molecule, in an assembly solution; sequentially subjecting the mixture to a denaturation reaction and a renaturation reaction to form a crude self-assembly product; and subjecting the crude self-assembly product sequentially to purification, elution, and evaporation to dryness to obtain a targeted nucleic-acid carrier bearing at least one oligonucleotide effector molecule.

The molar ratio among the sequences a, b, and c is preferably 0.90-1.10 : 0.90-1.10 : 0.90-1.10, more preferably 1 : 1 : 1. Mixing the three sequences in approximately equal proportions facilitates more accurate self-assembly.

In the above preferred embodiments, the assembly solution is not particularly limited; depending on the sequences to be organized, a TMS aqueous solution, sodium chloride aqueous solution, magnesium chloride aqueous solution, or purified water may be reasonably selected.

In the above preferred embodiments, the specific suitable temperatures and pH values for the denaturation and renaturation reactions may be determined by repeated preliminary experiments. In the present application, the denaturation temperature is preferably 80-99 °C, more preferably 85-99 °C, and further preferably 90-99 °C; preferably, upon completion of the denaturation reaction, the reaction system is cooled to the renaturation temperature for the renaturation reaction, and finally cooled to obtain the crude self-assembly product; the renaturation temperature is 70-50 °C, more preferably 65-55 °C, and further preferably 63-57 °C; the renaturation time is 3-15 min, more preferably 3-10 min, and further preferably 3-5 min; preferably, during cooling of the reaction system to the holding temperature, the cooling rate is 2-10 °C/min, more preferably 2-6 °C/min, and further preferably 2-3 °C/min; preferably, the final cooling-end temperature is 0-25 °C, more preferably 0-15 °C, and further preferably 0-4 °C; and preferably, the pH of the denaturation reaction is 5.4-8.8.

During the above self-assembly process, although the synthesized sequences are single-stranded, high-temperature denaturation is first performed to avoid interference from secondary structures of the single-stranded sequences with the self-assembly process. The specific denaturation temperature may be reasonably selected within the preferred temperature ranges described above according to the composition, length, and number of the sequences to be self-assembled. Upon completion of the denaturation reaction, a renaturation (annealing) reaction is carried out so that intra-sequence or inter-sequence interactions promote self-assembly to form complementary pairing and specific spatial structures. The renaturation temperature is likewise reasonably selected between 70-50 °C depending on differences in sequence composition, length, and number. The final cooling step serves to consolidate and maintain the spatial structure formed by self-assembly. During cooling from the renaturation temperature to ambient temperature, room temperature, or refrigerated temperature, the mixture is gradually cooled at a cooling rate of 2-10 °C/min, which helps stabilize the three-dimensional structure formed by self-assembly.

An exemplary preparation procedure is described below in connection with the self-assembly of a DNA carrier: (1) Simultaneously mix the single-stranded sequence a, sequence b, and sequence c (which are modified or synthesized with other oligonucleotide molecules) in DEPC water or TMS buffer; (2) heat the mixed solution to 95 °C, maintain for 5 min, and then slowly cool to room temperature at a rate of 2 °C/min; (3) load the product onto an 8% (m/v) native PAGE gel and electrophorese at 100 V in TBM buffer at 4 °C to purify the complex; (4) excise the target band and elute at 37 °C in DNA elution buffer, then precipitate with ethanol overnight, and evaporate to dryness under reduced pressure at low temperature to obtain the self-assembled product, i.e., the DNA carrier.

The present application is further described in detail below with specific embodiments, which shall not be construed as limiting the scope of protection sought.

**Carrier Assembly Experiment**

Embodiment 1

This embodiment verifies the assembly rate of the DNA carrier.

Assembly Group I: C1 = 2*CpG2006 - CD40 aptamer - DNA carrier (the specific carrier sequence is shown in Table 5, Nucleic-Acid Drug 4); C2 = 2*CpG2006 - CD40 aptamer - DNA carrier (comparative example, the specific carrier sequence is shown in Table 7);

Assembly Group II: D1 = 2*AmiR-21 -AS1411 - DNA carrier (the specific carrier sequence is shown in Table 5, Nucleic-Acid Drug 21); D1 = 2*AmiR-21 -AS1411 - DNA carrier (comparative example, the specific carrier sequence is shown in Table 8);

Table 7 - C2 Sequences

| Name | Sequence (5'→3') | Remarks |
|---|---|---|
| Sequence a | TCGTCGTTTTGTCGTTTTGTCGTT**TTTTT**GCGACGCCC**GCAGCGUUCG**GGAGAGGAG (SEQ ID No. 530) | The first 1-23 phosphodiester linkages at the 5' end of CpG2006 are all replaced with phosphorothioate linkages. |
| Sequence b | CCAACGAGTAGGCGATAGCGCGTGG**TTTTT**CTCCTCTCC**CGUUCGCCG** CGAGCCGCG (SEQ ID No. 531) | / |
| Sequence c | TCGTCGTTTTGTCGTTTTGTCGTT**TTTTT**CGCGGCACG**CGGCCAUAGCGC**GGGCGTC GC (SEQ ID No. 532) | The first 1-23 phosphodiester linkages at the 5' end of CpG2006 are all replaced with phosphorothioate linkages. |

246

Table 8 - D2 Sequences

| Name | Sequence (5'→3') | Remarks |
|---|---|---|
| Sequence a | GATAAGCTGCGACGCCCGCAGCGUUCGGGAGAGGAG (SEQ ID No. 533) | Locked-nucleic-acid (LNA) modification at the 5'-end GATAAGCT motif. |
| Sequence b | GGTGGTGGTGGTTGTGGTGGTGGTGG**TTTTT**CTCCTCTCCCGUUCGGCCCGAGC CGCG (SEQ ID No. 534) | / |
| Sequence c | GATAAGCTCGCGGCACGGGCCCAUAGCGCGGGCGTCGC (SEQ ID No. 535) | Locked-nucleic-acid (LNA) modification at the 5'-end GATAAGCT motif. |

1. Experimental Materials and Reagents:

(1) Sequence a;

(2) Sequence b;

(3) Sequence c;

(4) TMS aqueous solution;

(5) TBM (Tris-borate-magnesium) buffer.

2. Experimental Method:

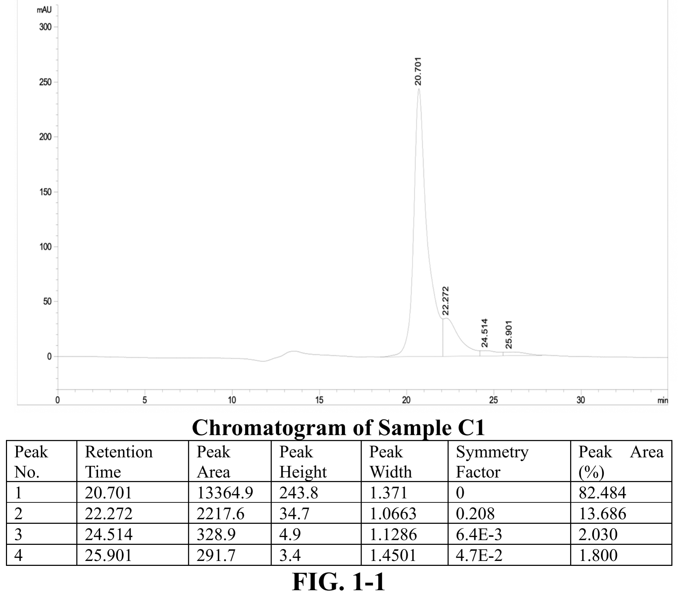

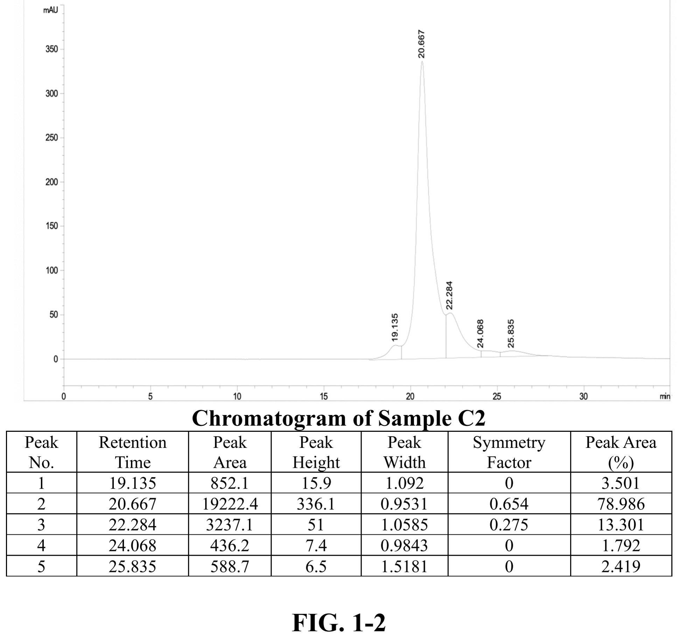

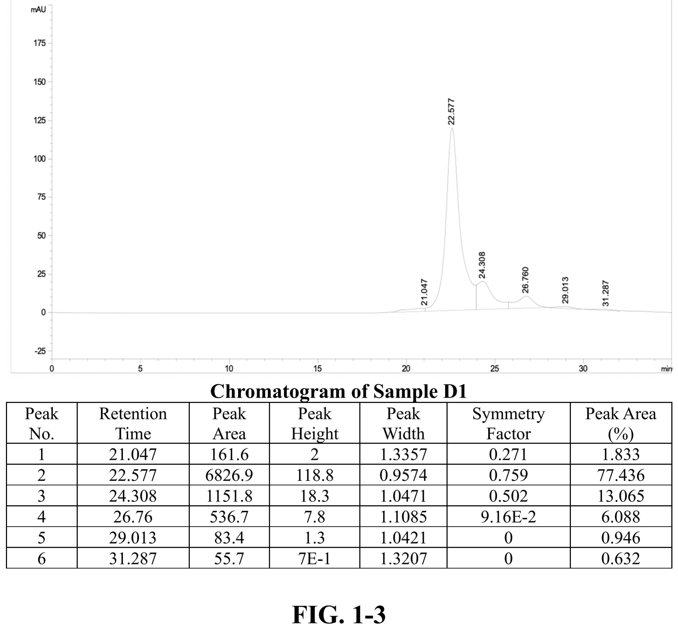

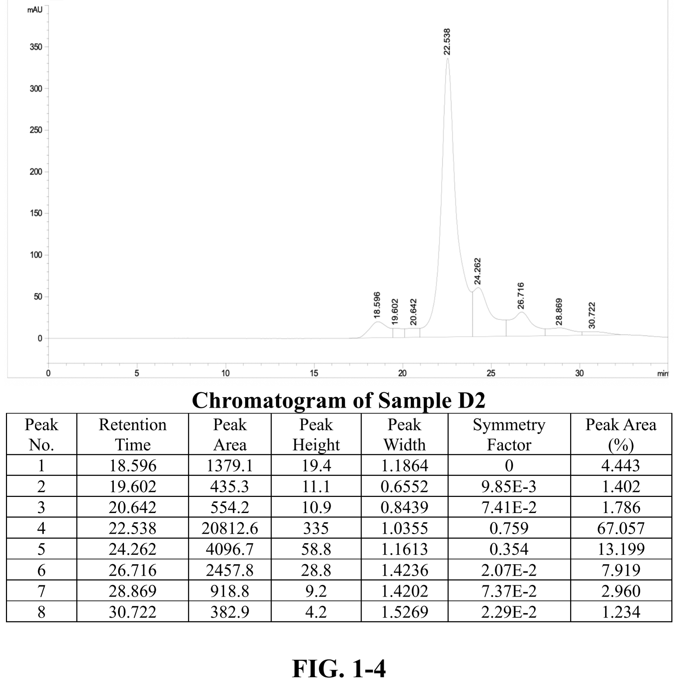

The sequences a, b and c for each group are dissolved in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the sequence-assembly product. The HPLC chromatographic results of the sequence-assembly products are shown in Figures 1-1 to 1-4.

Detection Conditions

Laboratory temperature: 24 °C

Laboratory humidity: 32 %

Sample preparation: Weigh 1.5 mg each of samples C1, C2, D1, and D2; add 1000 μL of the mobile phase to dissolve; place on a shaker at 37 °C, 100 rpm, for 30 min;

Mobile phase preparation (for C1, C2, D1, D2): Measure 500 mL of distilled water (Watsons), add 3.03 g Tris(hydroxymethyl)aminomethane (Tris) and 2.92 g sodium chloride; adjust to pH 8 with hydrochloric acid.

Instruments: Agilent 1200 (BN083); Thermo Shaker MB100-4A; Sartorius (BN446).

Detection wavelength: 260 nm

Flow rate: 0.6 mL/min

Conclusion: In Assembly Group I, the difference between C1 and C2 lies in the DNA carrier; in Assembly Group II, the difference between D1 and D2 likewise lies in the DNA carrier. As shown by the results of Figures 1-1 and 1-2 and Figures 1-3 and 1-4, the DNA carrier of the embodiments of the present invention, compared with the comparative examples, exhibits-under identical assembly conditions with the same effector molecules loaded-a higher area-normalized percentage of the principal peak, indicating better loading efficiency and higher assembly purity.

Drug Assembly Experiment

Embodiment 2

Assembly and synthesis of 3*CpG2006-DNA carrier (the specific carrier sequence is shown in Table 5, Nucleic-Acid Drug 1).

1. Experimental Materials and Reagents:

(1) Sequence a;

(2) Sequence b;

(3) Sequence c;

(4) TMS aqueous solution;

(5) TBM (Tris-borate-magnesium) buffer.

2. Experimental Method:

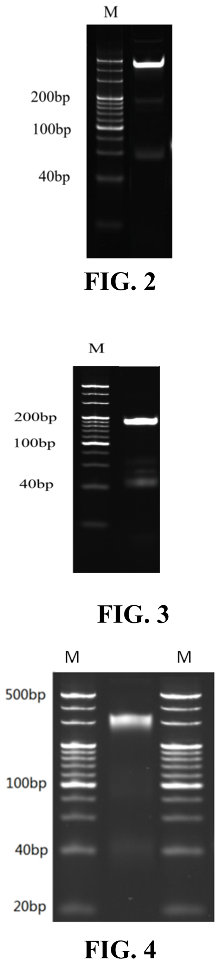

Dissolve the sequences a, b, and c separately in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the self-assembled product. The agarose gel electrophoresis results of the self-assembled product are shown in Figure 2.

Embodiment 3

Assembly and synthesis of 2*CpG1 826 - 2*mannose - DNA carrier (the specific carrier sequence is shown in Table 5, Nucleic-Acid Drug 2).

1. Experimental Materials and Reagents:

(1) Sequence a;

(2) Sequence b;

(3) Sequence c;

(4) TMS aqueous solution;

(5) TBM (Tris-borate-magnesium) buffer.

2. Experimental Method:

Dissolve the sequences a, b, and c separately in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the self-assembled product. The agarose gel electrophoresis results of the self-assembled product are shown in Figure 3.

Embodiment 4

Assembly and synthesis of 2*CpG1826 - CD40 aptamer - DNA carrier (the specific carrier sequence is shown in Table 5, Nucleic-Acid Drug 3).

1. Experimental Materials and Reagents:

(1) Sequence a;

(2) Sequence b;

(3) Sequence c;

(4) TMS aqueous solution;

(5) TBM (Tris-borate-magnesium) buffer.

2. Experimental Method:

Dissolve the sequences a, b, and c separately in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the self-assembled product. The agarose gel electrophoresis results of the self-assembled product are shown in Figure 4.

Embodiment 5

(I)
Assembly and synthesis of 2*CpG2006 - CD40 aptamer - DNA carrier(the specific carrier sequence is shown in Table 5, Nucleic-Acid Drug 4).

1. Experimental Materials and Reagents:

(1) Sequence a;

(2) Sequence b;

(3) Sequence c;

(4) TMS aqueous solution;

(5) TBM (Tris-borate-magnesium) buffer.

2. Experimental Method:

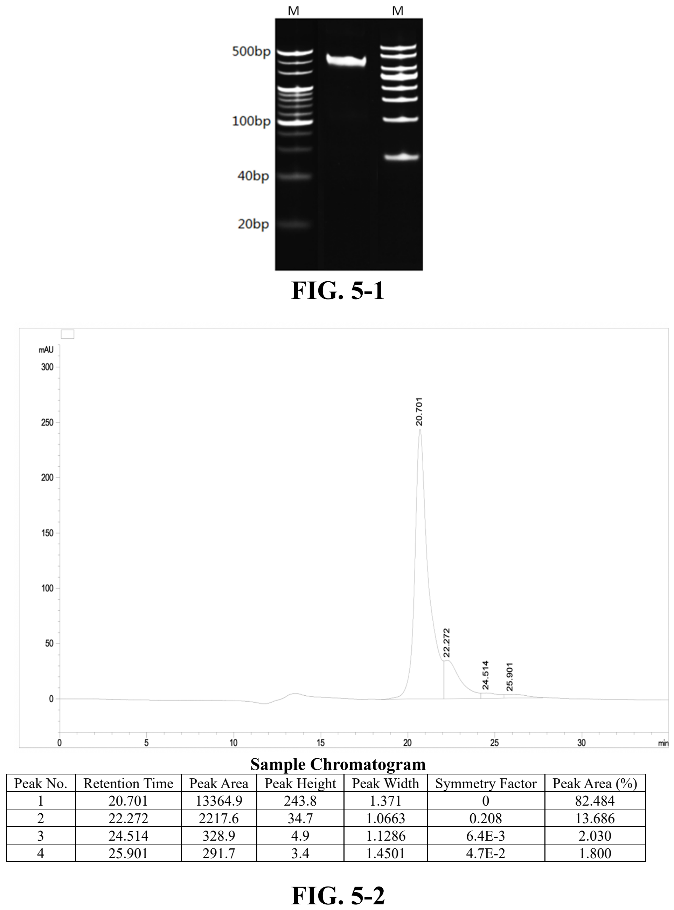

Dissolve the sequences a, b, and c separately in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the self-assembled product. The agarose gel electrophoresis results of the self-assembled product are shown in Figure 5-1. The HPLC chromatographic results are shown in Figure 5-2.

(II) Tm Determination

The melting-curve method is employed to determine the Tm of the 2*CpG2006 - CD40 aptamer - DNA carrier.

Reagents and instruments:

Table 9.

| Reagent Name | Cat. No. | Supplier |
|---|---|---|
| AE buffer | / | Takara |
| SYBR Green I | / | Self-prepared |

Table 10.

| Name | Model | Manufacturer |
|---|---|---|
| Real-Time System | CFX Connect | Bio-rad |
| Clean bench | HDL | Beijing HDL Apparatus Co.,LTd |

Steps:

① After diluting the sample with ultrapure water, mix 5 μg of the diluted sample with 2 μL SYBR Green I (1:200 dilution) to a final volume of 20 μL;

② Incubate at room temperature, protected from light, for 30 min;

③ Instrument measurement: set the program to start at 20 °C, increase the temperature at 0.1 °C/s to 95 °C, and record once every 5s.

Detection results:

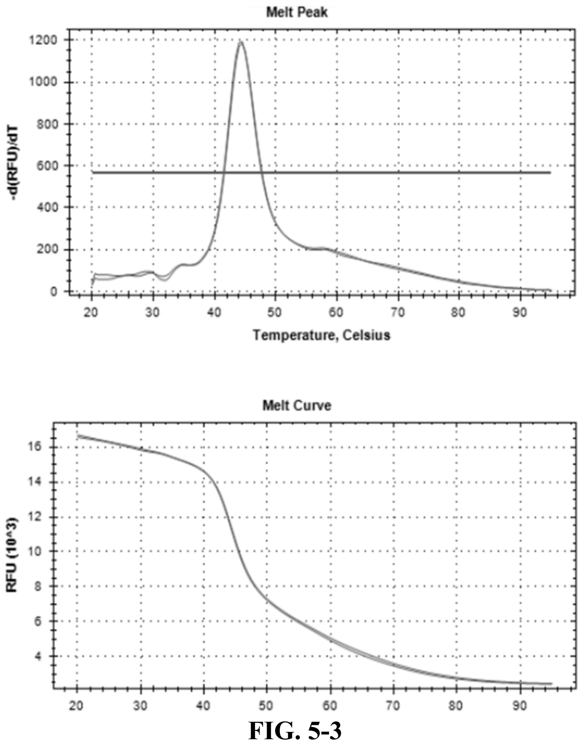

The melt peak and melting-curve plots are shown in the figure. From Figure 5-3 (upper panel Melt Peak: melting peak; x-axis: temperature, °C; lower panel Melt Curve: melting curve; x-axis: temperature, °C), it is evident that the melting temperature (Tm) of the 2*CpG2006 - CD40 aptamer - DNA carrier melting curve is relatively high, indicating a stable self-assembled structure.
(III)
The serum stability of the 2*CpG2006 - CD40 aptamer - DNA carrier is characterized by native PAGE.

Principal reagents and instruments: as follows:

Table 11

| Reagent name | Catalog No. | Manufacturer |
|---|---|---|
| 6×DNA Loading buffer | TSJ010 | Tsingke Bio |
| 20bp DNA Ladder | 3420A | TAKARA |
| *1163165 SolarGelRed Nucleic-Acid Stain** | E1020 | solarbio |
| 8% Native PAGE Gel | / | Self-prepared |
| 1× TBE Buffer (RNase-free) | / | Self-prepared |
| Serum (FBS) | / | Excel |
| RPMI 1640 | / | GBICO |

Table 12

| Name | Model | Manufacturer |
|---|---|---|
| Power supply (electrophoresis) | PowerPac Basic | Bio-rad |
| Vertical electrophoresis tank | Mini PROTEAN Tetra Cell | Bio-rad |
| Destaining shaker | TS-3D | orbital shaker |
| Gel imaging system | GenoSens1880 | Shanghai Clinx Co., Ltd |

Steps:

① Prepare the 2*CpG2006-CD40 aptamer-DNA carrier to the concentrations shown below, then dilute the prepared samples according to the table; prepare five dilution tubes and incubate the diluted samples in a 37 °C water bath for different times (0, 1 h, 2 h, 4 h, 7 h);

Table 13

| Sample name | Sample concentration ($\mu$g/mL) | Sample volume | 50% FBS-1640 / $\mu$L | 1640/ $\mu$L | Concentration after dilution($\mu$g/mL) |
|---|---|---|---|---|---|
| 2*CpG20 06-CD40 aptamer-DNA | | | 8 | 30.6 | 100 |

② Mix 5 $\mu$L of the processed sample with 1 $\mu$L of 6× DNA Loading Buffer; operate on ice and label;
③ Prepare an 8% native PAGE gel; load, on a single gel, the samples corresponding to the different incubation times; load the entire 6 $\mu$L of each processed sample; set the program to 100 V and run for 40 min;
④ After electrophoresis, stain, place on a horizontal shaker and gently shake for 30 min, then image.

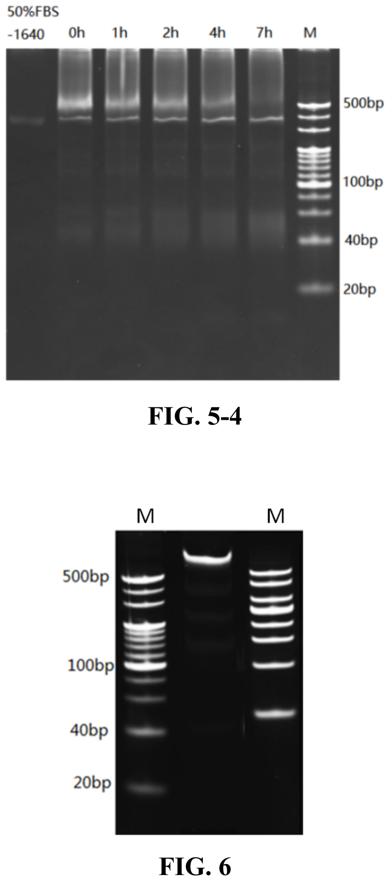

Electrophoresis results are shown in Figure 5-4; from left to right the lanes correspond to 0, 1 h, 2 h, 4 h, 7 h, and M: marker. From the serum-stability test results it can be seen that the native-gel bands at 0, 1 h, 2 h, 4 h, and 7 h show no obvious differences among the 2*CpG2006-CD40 aptamer-DNA carrier samples at different time points, indicating that the 2*CpG2006-CD40 aptamer-DNA carrier is relatively stable in 50% FBS in 1640 medium, with no obvious degradation.

Embodiment 6

Assembly and synthesis of 4*CpG2006 - CD40 aptamer - DNA carrier (the specific carrier sequence is shown in Table 5, Nucleic-Acid Drug 5).

1. Experimental Materials and Reagents:

    (1) Sequence a;

    (2) Sequence b;

    (3) Sequence c;

    (4) TMS aqueous solution;

    (5) TBM (Tris-borate-magnesium) buffer.

2. Experimental Method:

Dissolve the sequences a, b, and c separately in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the self-assembled product. The agarose gel electrophoresis results of the self-assembled product are shown in Figure 6.

Embodiment 7

Assembly and synthesis of 2*CpG1 826 - CD40 aptamer - 2*mannose - DNA carrier (the specific carrier sequence is shown in Table 5, Nucleic-Acid Drug 6).

1. Experimental Materials and Reagents:

(1) Sequence a;

(2) Sequence b;

(3) Sequence c;

(4) TMS aqueous solution;

(5) TBM (Tris-borate-magnesium) buffer.

2. Experimental Method:

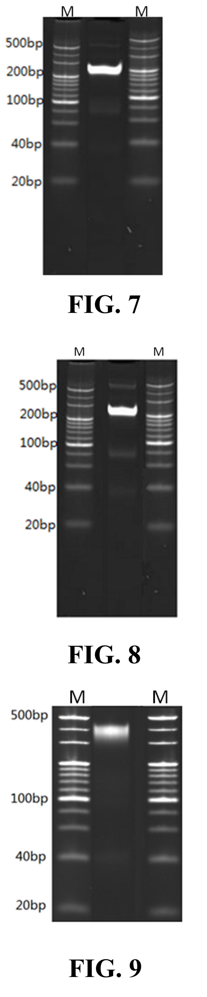

Dissolve the sequences a, b, and c separately in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the self-assembled product. The agarose gel electrophoresis results of the self-assembled product are shown in Figure 7.

Embodiment 8

Assembly and synthesis of CPG1826 - CD40 aptamer - mannose - MUC1 aptamer - DNA carrier (the specific carrier sequence is shown in Table 5, Nucleic-Acid Drug 7).

1. Experimental Materials and Reagents:

(1) Sequence a;

(2) Sequence b;

(3) Sequence c;

(4) TMS aqueous solution;

(5) TBM (Tris-borate-magnesium) buffer.

2. Experimental Method:

Dissolve the sequences a, b, and c separately in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the self-assembled product. The agarose gel electrophoresis results of the self-assembled product are shown in Figure 8.

Embodiment 9

Assembly and synthesis of 2*CpG2006 - CD40 aptamer - AmiR-21 - 3*mannose - DNA carrier (the specific carrier sequence is shown in Table 5, Nucleic-Acid Drug 8).

1. Experimental Materials and Reagents:

(1) Sequence a;

(2) Sequence b;

(3) Sequence c;

(4) TMS aqueous solution;

(5) TBM (Tris-borate-magnesium) buffer.

2. Experimental Method:

Dissolve the sequences a, b, and c separately in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the self-assembled product. The agarose gel electrophoresis results of the self-assembled product are shown in Figure 9.

Embodiment 10

Assembly and synthesis of 2*CpG2006 - CD40 aptamer - CD16a aptamer - CTLA-4 aptamer - DNA carrier (the specific carrier sequence is shown in Table 5, Nucleic-Acid Drug 9).

1. Experimental Materials and Reagents:

(1) Sequence a;

(2) Sequence b;

(3) Sequence c;

(4) TMS aqueous solution;

(5) TBM (Tris-borate-magnesium) buffer.

2. Experimental Method:

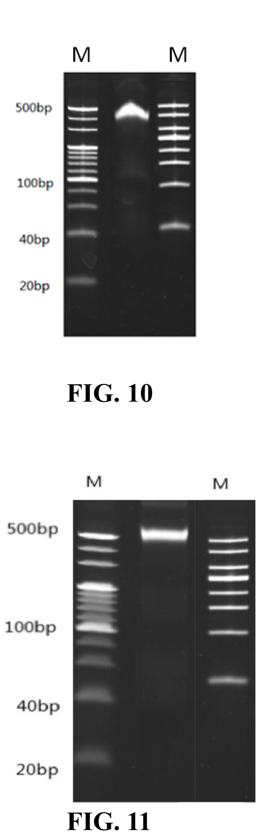

Dissolve the sequences a, b, and c separately in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the self-assembled product. The agarose gel electrophoresis results of the self-assembled product are shown in Figure 10.

Embodiment 11

Assembly and synthesis of 2*CpG2006 - CTLA-4 aptamer - PD-L1 aptamer - DNA carrier (the specific carrier sequence is shown in Table 5, Nucleic-Acid Drug 10).

1. Experimental Materials and Reagents:

(1) Sequence a;

(2) Sequence b;

(3) Sequence c;

(4) TMS aqueous solution;

(5) TBM (Tris-borate-magnesium) buffer.

2. Experimental Method:

Dissolve the sequences a, b, and c separately in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the self-assembled product. The agarose gel electrophoresis results of the self-assembled product are shown in Figure 11.

Embodiment 12

(I)
Assembly and synthesis of CpG2006 - CD40 aptamer - PD-L1 aptamer - DNA carrier (abbreviated as "CCPD3"; the specific carrier sequence is shown in Table 5).

1. Experimental Materials and Reagents:

(1) Sequence a;

(2) Sequence b;

(3) Sequence c;

(4) TMS aqueous solution;

(5) TBM (Tris-borate-magnesium) buffer.

2. Experimental Method:

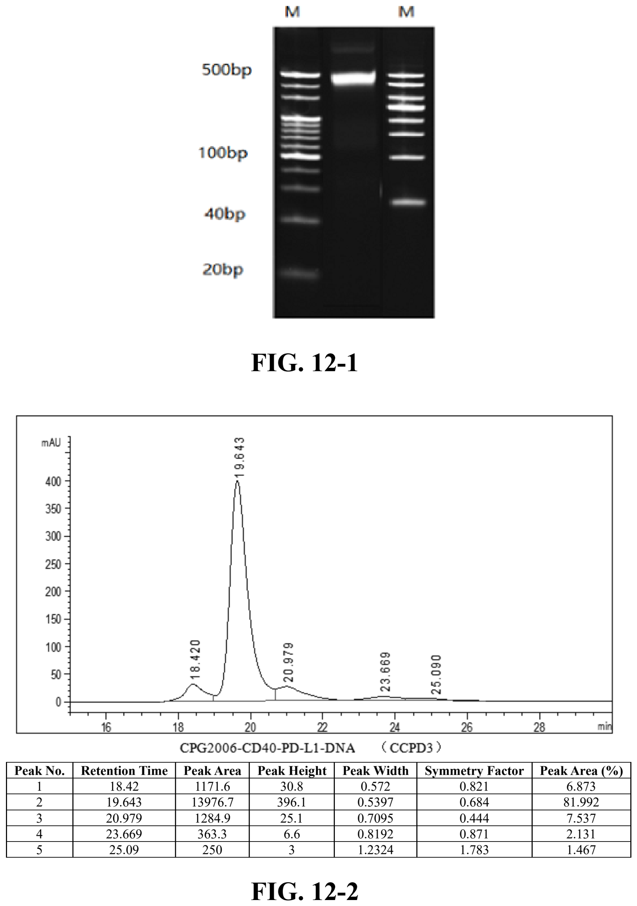

Dissolve the sequences a, b, and c separately in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the self-assembled product. The agarose gel electrophoresis results of the self-assembled product are shown in Figure 12-1. The HPLC chromatographic results of the self-assembled product are shown in Figure 12-2.
(II)
The melting-curve determination method is the same as in Embodiment 5.
The melt peak and melting-curve plots are shown in the figure. From Figure 12-3, the melting temperature (Tm) of the CCPD3 melting curve is relatively high, indicating that the self-assembled structure of the sample is stable.

Embodiment 13

(I)
In this embodiment, CCPD3 Variant 1 = CpG2006 - CD40 aptamer - PD-L1 aptamer - DNA (the same as CCPD3 in Embodiment 12, with the strand c replaced by HQ PD-L1).
For assembly and synthesis of CCPD3 Variant 1, the specific carrier sequences are shown in Table 5.

1. Experimental Materials and Reagents:

(1) Sequence a;

(2) Sequence b;

(3) Sequence c;

(4) TMS aqueous solution;

(5) TBM (Tris-borate-magnesium) buffer.

2. Experimental Method:

Dissolve the sequences a, b, and c separately in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the self-assembled product. The agarose gel electrophoresis results of the self-assembled product are shown in Figure 13-1. The HPLC chromatographic results of the sequence-assembly product are shown in Figure 13-2.
(II)
In this embodiment, CCPD3 Variant 2 = CpG2006 - CD40 aptamer - PD-L1 aptamer - DNA (compared with CCPD3 of Embodiment 12, the a, b, anstrand d cs are all shortened).
For assembly and synthesis of CCPD3 Variant 2, the specific carrier sequences are shown in Table 5.

1. Experimental Materials and Reagents:

(1) Sequence a;

(2) Sequence b;

(3) Sequence c;

(4) TMS aqueous solution;

(5) TBM (Tris-borate-magnesium) buffer.

2. Experimental Method:

Dissolve the sequences a, b, and c separately in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the self-assembled product. The agarose gel electrophoresis results of the self-assembled product are shown in Figure 13-3.
(III)
In this embodiment, CCPD3 Variant 3 = CpG2006 - CD40 aptamer - PD-L1 aptamer - DNA (compared with CCPD3 of Embodiment 12, the a, b, anstrand d cs are all shortened).
Step 1: Assembly and synthesis of CCPD3 Variant 3 (the specific carrier sequences are shown in Table 5).

1. Experimental Materials and Reagents:

(1) Sequence a;

(2) Sequence b;

(3) Sequence c;

(4) TMS aqueous solution;

(5) TBM (Tris-borate-magnesium) buffer.

2. Experimental Method:

Dissolve the sequences a, b, and c separately in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the self-assembled product. The agarose gel electrophoresis results of the self-assembled product are shown in Figure 13-4.
((IV)

In this embodiment, CCPD3 Variant 4 = CpG2006 - CD40 aptamer - PD-L1 aptamer - DNA (compared with CCPD3 of Embodiment 12, the a, b, anstrand d cs are shortened, and XDPD-L1 is used).

Step 1: Assembly and synthesis of CCPD3 Variant 4 (the specific carrier sequences are shown in Table 5).

1. Experimental Materials and Reagents:

(1) Sequence a;

(2) Sequence b;

(3) Sequence c;

(4) TMS aqueous solution;

(5) TBM (Tris-borate-magnesium) buffer.

2. Experimental Method:

Dissolve the sequences a, b, and c separately in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the self-assembled product. The agarose gel electrophoresis results of the self-assembled product are shown in Figure 13-5.
(V)

In this embodiment, CCPD3 Variant 5 = CpG2006 - CD40 aptamer - PD-L1 aptamer - DNA (compared with CCPD3 of Embodiment 12, it is split into four strands).

Step 1: Assembly and synthesis of CCPD3 Variant 5 (the specific carrier sequences are shown in Table 5).

1. Experimental Materials and Reagents:

(1) Sequence a;

(2) Sequence b;

(3) Sequence c;

(4) TMS aqueous solution;

(5) TBM (Tris-borate-magnesium) buffer.

2. Experimental Method:

Dissolve the sequences a, b, and c separately in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the self-assembled product. The agarose gel electrophoresis results of the self-assembled product are shown in Figure 13-6.

(VI)

In this embodiment, CCPD3 Variant 6 is assembled and synthesized.

Step 1: Assembly and synthesis of CCPD3 Variant 6 (the specific carrier sequences are shown in Table 5).

1. Experimental Materials and Reagents:

(1) Sequence a;

(2) Sequence b;

(3) Sequence c;

(4) TMS aqueous solution;

(5) TBM (Tris-borate-magnesium) buffer.

2. Experimental Method:

Dissolve the sequences a, b, and c separately in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the self-assembled product. The agarose gel electrophoresis results of the self-assembled product are shown in Figure 13-7.

(VII)

In this embodiment, CCPD3 Variant 7 is assembled and synthesized.

Step 1: Assembly and synthesis of CCPD3 Variant 7 (the specific carrier sequences are shown in Table 5).

1. Experimental Materials and Reagents:

(1) Sequence a;

(2) Sequence b;

(3) Sequence c;

(4) TMS aqueous solution;

(5) TBM (Tris-borate-magnesium) buffer.

2. Experimental Method:

Dissolve the sequences a, b, and c separately in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the self-assembled product. The agarose gel electrophoresis results of the self-assembled product are shown in Figure 13-8.

(VIII)

In this embodiment, CCPD3 Variant 8 is assembled and synthesized.

Step 1: Assembly and synthesis of CCPD3 Variant 8 (the specific carrier sequences are shown in Table 5).

1. Experimental Materials and Reagents:

(1) Sequence a;

(2) Sequence b;

(3) Sequence c;

(4) TMS aqueous solution;

(5) TBM (Tris-borate-magnesium) buffer.

2. Experimental Method:

Dissolve the sequences a, b, and c separately in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the self-assembled product. The agarose gel electrophoresis results of the self-assembled product are shown in Figure 13-9.

Conclusion: All variants of CCPD3 can be successfully assembled.

(IX)

In this embodiment, CCPD3 Variant 9 is assembled and synthesized.

Step 1: Assembly and synthesis of CCPD3 Variant 9 (the specific carrier sequences are shown in Table 5).

1. Experimental Materials and Reagents:

(1) Sequence a;;

(2) Sequence b;

(3) Sequence c;

(4) TMS aqueous solution;

(5) TBM (Tris-borate-magnesium) buffer.

2. Experimental Method:

Dissolve the sequences a, b, and c separately in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the self-assembled product. The agarose gel electrophoresis results of the self-assembled product are shown in Figure 13-10.

Conclusion: All variants of CCPD3 can be successfully assembled.

(X)

In this embodiment, CCPD3 Variant 10 is assembled and synthesized.

Step 1: Assembly and synthesis of CCPD3 Variant 10 (the specific carrier sequences are shown in Table 5).

1. Experimental Materials and Reagents:

(1) Sequence a;

(2) Sequence b;

(3) Sequence c;

(4) TMS aqueous solution;

(5) TBM (Tris-borate-magnesium) buffer.

2. Experimental Method:

Dissolve the sequences a, b, and c separately in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the self-assembled product. The agarose gel electrophoresis results of the self-assembled product are shown in Figure 13-11.
(XI)
In this embodiment, CCPD3 Variant 11 is assembled and synthesized.
Step 1: Assembly and synthesis of CCPD3 Variant 11 (the specific carrier sequences are shown in Table 5).

1. Experimental Materials and Reagents:

    (1) Sequence a;

    (2) Sequence b;

    (3) Sequence c;

    (4) TMS aqueous solution;

    (5) TBM (Tris-borate-magnesium) buffer.

2. Experimental Method:

Dissolve the sequences a, b, and c separately in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the self-assembled product. The agarose gel electrophoresis results of the self-assembled product are shown in Figure 13-12.
(XII)
In this embodiment, CCPD3 Variant 12 is assembled and synthesized.
Step 1: Assembly and synthesis of CCPD3 Variant 12 (the specific carrier sequences are shown in Table 5).

1. Experimental Materials and Reagents:

    (1) Sequence a;

    (2) Sequence b;

    (3) Sequence c;

    (4) TMS aqueous solution;

    (5) TBM (Tris-borate-magnesium) buffer.

2. Experimental Method:

Dissolve the sequences a, b, and c separately in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the self-assembled product. The agarose gel electrophoresis results of the self-assembled product are shown in Figure 13-13. Conclusion: All variants of CCPD3 can be successfully assembled.

Embodiment 14

According to the specific carrier sequences in Table 5, assemble and synthesize the following nano-nucleic-acid drugs: 2*CpG2006 - CD40 aptamer - PD-L1 aptamer - C12 aptamer - DNA carrier (Drug 13); 2*AmiR-21 - TTA1 aptamer - DNA carrier (Drug 18); 2*AmiR-21 - 3*Bio - DNA carrier (Drug 19); 2*AmiR-21 - 4*Bio - DNA carrier (Drug 20); 2*AmiR-21 -AS1411 aptamer - DNA carrier (Drug 21); 2*AmiR-21 - AS1411 aptamer - DNA carrier (enhanced version) (Drug 22); 2*AmiR-21 - AS1411 aptamer - Bio - DNA carrier (Drug 23); 2*AmiR-21 - CD40 aptamer - DNA carrier (Drug 24); 2*AmiR-21 - MUC1 aptamer - 3*Bio - DNA (Drug 25); 2*mannose - 2*AmiR-21 - DNA (Drug 27); 2*mannose - A15 aptamer - 2*AmiR-21 - DNA (Drug 28); 2*mannose - survivin siRNA - DNA (Drug 29); 2*mannose - A15 aptamer - survivin siRNA - AmiR-21 - DNA (Drug 30); AS1411 aptamer - survivin siRNA - PSMA aptamer - 3*AmiR-21 - RNA carrier (Drug 33); 3AmiR-21 / AS1411 aptamer / FAP aptamer - DNA carrier (Drug 34); 3*AmiR-21 -AS1411 aptamer - CD40 aptamer - 2*Bio - DNA carrier (Drug 41); TAP siRNA - 3*AmiR-21 - 2*AS1411 aptamer - 1 - DNA carrier (Drug 43); CpG1826 - CD40 aptamer - AmiR-21 - 3*Bio - DNA carrier (Drug 45); CD16a aptamer - CTLA-4 aptamer - PD-L1 aptamer - DNA carrier (Drug 47); CD16a aptamer - Act-12c aptamer - PD-L1 aptamer - DNA carrier (Drug 48); CTLA-4 aptamer - PD-L1 aptamer - PD-1 aptamer - DNA carrier (Drug 49); CD16a aptamer - Act-12c aptamer - MUC1 aptamer (5TR1) - DNA carrier (Drug 55); Her3 aptamer - EGFR siRNA - AS 1411 aptamer - survivin siRNA - Her2 aptamer - DNA carrier (Drug 63); AS1411 aptamer - TAP siRNA - CD16a aptamer - Act-12c aptamer - DNA carrier (Drug 68); CD16a aptamer - AS1411 aptamer - TAP siRNA - OX40 aptamer - Act-12c aptamer - DNA carrier (Drug 69); AS1411 aptamer - ASAP1 siRNA - VEGF aptamer - SARS-CoV-2-N48 aptamer - RNA (Drug 73); CD3-4 aptamer - PD-1 aptamer - BCMA aptamer - DNA carrier (Drug 78); CpG2006 - CD38 aptamer - miR-126 - PD-L1 aptamer - DNA carrier (Drug 81); BCMA aptamer - CD38 aptamer - miR-126 - PD-L1 aptamer - miR-34 - DNA carrier (MM) (Drug 87); AS1411 aptamer - FGF2 aptamer - 3*AmiR-21 - DNA carrier (Drug 88); HBsAg aptamer - CD40 aptamer - PD-L1 aptamer (CCPD3 - C-strand) - DNA carrier (HBV) (Drug 89); HBsAg aptamer - CD40 aptamer - PD-L1 aptamer (HD) - DNA carrier (HBV) (Drug 90); BCMA aptamer - CD38 aptamer - CpG aptamer - CD19 aptamer - AmiR-21 - DNA3-TY (MM) (Drug 91); BCMA aptamer - CD38 aptamer - CD19 aptamer - AmiR-21 - DNA3-TY carrier (MM) (Drug 92); CpG2006 - CD38 aptamer - PD-L1 aptamer - DNA3-TY (tumor) (Drug 93); BCMA aptamer - CD38 aptamer - CpG aptamer - PD-L1 aptamer - DNA3-TY (MM) (Drug 94); BCMA aptamer - CD40 aptamer - PD-L1 aptamer - DNA3-TY (MM) (Drug 95); AmiR-21 -AS1411 aptamer - CD40 aptamer - 2*biotin - DNA carrier (Drug 96); 2*AmiR-21 - 4*biotin - DNA carrier (Drug 97); TTA1 aptamer - epirubicin - 4*biotin - DNA carrier (Drug 98); and the universal carrier DNA3-TY (Table 6).

Assembly Steps:

1. Experimental Materials and Reagents:

(1) Sequence a;

(2) Sequence b;

(3) Sequence c;

(4) TMS aqueous solution;

(5) TBM (Tris-borate-magnesium) buffer.

2. Experimental Method:

Dissolve the sequences a, b, and c separately in TMS aqueous solution at a 1:1:1 molar ratio; after mixing, adjust the reaction mixture to pH 8.8. Heat the mixture to 99 °C, maintain for 5 min, then cool at 5 °C/min to 65 °C and hold for 3 min; subsequently cool at 5 °C/min to 2 °C to obtain the self-assembled product.

In addition, the specific procedures for the melting-curve and serum-stability experiments refer to the foregoing embodiments.

Wherein, the agarose gel electrophoresis results for the self-assembled product of 2*CpG2006-CD40 aptamer-PD-L1 aptamer-C12 aptamer-DNA carrier are shown in FIG. 14-1; the agarose gel electrophoresis results for the self-assembled product of 2*AmiR-21-TTA1 aptamer-DNA carrier are shown in FIG. 14-2; the agarose gel electrophoresis results for the self-assembled product of 2*AmiR-21-3*biotin-DNA carrier are shown in FIG. 14-3; the agarose gel electrophoresis results for the self-assembled product of 2*AmiR-21-4*biotin-DNA carrier are shown in FIG. 14-4; the agarose gel electrophoresis results for the self-assembled product of 2* AmiR-21-AS1411 aptamer-DNA carrier are shown in FIG. 14-5; the agarose gel electrophoresis results for the self-assembled product of the enhanced version of 2*AmiR-21-AS1411 aptamer-DNA carrier are shown in FIG. 14-6; the agarose gel electrophoresis results for the self-assembled product of 2*AmiR-21-AS1411 aptamer-biotin-DNA carrier are shown in FIG. 14-7; the agarose gel electrophoresis results for the self-assembled product of 2*AmiR-21-CD40 aptamer-DNA carrier are shown in FIG. 14-8; the agarose gel electrophoresis results for the self-assembled product of 2*AmiR-21-MUC1 aptamer-3*biotin-DNA carrier are shown in FIG. 14-9; the agarose gel electrophoresis results for the self-assembled product of 2*mannose-2*AmiR-21-DNA carrier are shown in FIG. 14-10; the agarose gel electrophoresis results for the self-assembled product of 2*mannose-A15 aptamer-2*AmiR-21-DNA carrier are shown in FIG. 14-11; the agarose gel electrophoresis results for the self-assembled product of 2*mannose-survivin siRNA-DNA carrier are shown in FIG. 14-12; the agarose gel electrophoresis results for the self-assembled product of 2*mannose-A15 aptamer-survivin siRNA-AmiR-21-DNA carrier are shown in FIG. 14-13; the HPLC chromatographic analysis results for the self-assembled product of AS1411 aptamer-survivin siRNA-PSMA aptamer-3 *AmiR-21-RNA carrier are shown in FIG. 14-14; the agarose gel electrophoresis results for the self-assembled product of 3*AmiR-21-AS1411 aptamer-FAP aptamer-DNA carrier are shown in FIG. 14-15A, and the HPLC chromatographic analysis results are shown in FIG. 14-15B; the agarose gel electrophoresis results for the self-assembled product of 3*AmiR-21-AS1411 aptamer-CD40 aptamer-2*biotin-DNA carrier are shown in FIG. 14-16; the agarose gel electrophoresis results for the self-assembled product of TAP siRNA-3*AmiR-21-2*AS1411 aptamer-1-DNA carrier are shown in FIG. 14-17; the agarose gel electrophoresis results for the self-assembled product of CpG1826-CD40 aptamer-AmiR-21-3×biotin-DNA carrier are shown in FIG. 14-18; the agarose gel electrophoresis results for the self-assembled product of CD16a aptamer-CTLA-4 aptamer-PD-L1 aptamer-DNA carrier are shown in FIG. 14-19; the agarose gel electrophoresis results for the self-assembled product of CD16a aptamer-Act-12c aptamer-PD-L1 aptamer-DNA are shown in FIG. 14-20; the agarose gel electrophoresis results for the self-assembled product of CTLA-4 aptamer-PD-L1 aptamer-PD-1 aptamer-DNA carrier are shown in FIG. 14-21A, the HPLC chromatographic analysis results are shown in FIG. 14-21B, the dissolution curve is shown in FIG. 14-21C, and the agarose gel electrophoresis results of the serum stability assay are shown in FIG. 14-21D; the HPLC chromatographic analysis results for the self-assembled product of CD16a aptamer-Act-12c aptamer-MUC1 aptamer (5TRI)-DNA carrier are shown in FIG. 14-22; the agarose gel electrophoresis results for the self-assembled product of HER3 aptamer-EGFR siRNA-AS 1411 aptamer-survivin siRNA-HER2 aptamer-DNA carrier are shown in FIG. 14-23; the agarose gel electrophoresis results for the self-assembled product of AS1411 aptamer- TAP siRNA-CD16a aptamer-Act-12c aptamer-DNA carrier are shown in FIG. 14-24; the agarose gel electrophoresis results for the self-assembled product of CD16a aptamer-AS1411 aptamer-TAP siRNA-OX40 aptamer-Act-12c aptamer-DNA carrier are shown in FIG. 14-25; the agarose gel electrophoresis results for the self-assembled product of AS 1411 aptamer-ASAP1 siRNA-VEGF aptamer-SARS-CoV-2-N48 aptamer-RNA are shown in FIG. 14-26A, and the HPLC chromatographic analysis results are shown in FIG. 14-26B; the agarose gel electrophoresis results for the self-assembled product of CD3-4 aptamer-PD-1 aptamer-BCMA aptamer-DNA carrier are shown in FIG. 14-27; the agarose gel electrophoresis results for the self-assembled product of CpG2006-CD38 aptamer-miR-126-PD-L1 aptamer-DNA carrier are shown in FIG. 14-28; the agarose gel electrophoresis results for the self-assembled product of BCMA aptamer-CD38 aptamer-miR-126-PD-L1 aptamer-miR-34-DNA carrier (MM) are shown in FIG. 14-29; the agarose gel electrophoresis results for the self-assembled product of AS 1411 aptamer-FGF2 aptamer-3×AmiR-21-DNA carrier are shown in FIG. 14-30; the agarose gel electrophoresis results for the self-assembled product of HBsAg aptamer-CD40 aptamer-PD-L1 aptamer (CCPD3-C chain)-DNA carrier (HBV) are shown in FIG. 14-31; the agarose gel electrophoresis results for the self-assembled product of HBsAg aptamer-CD40 aptamer-PD-L1 aptamer (HD)-DNA carrier (HBV) are shown in FIG. 14-32; the agarose gel electrophoresis results for the self-assembled product of BCMA aptamer-CD38 aptamer-CpG aptamer-CD19 aptamer-AmiR-21-DNA3-TY (MM) are shown in FIG. 14-33; the agarose gel electrophoresis results for the self-assembled product of BCMA aptamer-CD38 aptamer-CD19 aptamer-AmiR-21-DNA3-TY (MM) are shown in FIG. 14-34; the agarose gel electrophoresis results for the self-assembled product of CpG2006-CD38 aptamer-PD-L1 aptamer-DNA3-TY (tumor) are shown in FIG. 14-35; the agarose gel electrophoresis results for the self-assembled product of BCMA aptamer-CD38 aptamer-CpG aptamer-PD-L1 aptamer-DNA3-TY (MM) are shown in FIG. 14-36; the agarose gel electrophoresis results for the self-assembled product of BCMA aptamer-CD40 aptamer-PD-L1 aptamer-DNA3-TY (MM) are shown in FIG. 14-37; the agarose gel electrophoresis results for the self-assembled product of 1411 aptamer-CD40 aptamer-2*biotin-DNA carrier are shown in FIG. 14-38; the agarose gel electrophoresis results for the self-assembled product of 2*AmiR-21-4*biotin-DNA carrier are shown in FIG. 14-39; the agarose gel electrophoresis results for the self-assembled product of TTA1 aptamer-epirubicin-4*biotin-DNA carrier are shown in FIG. 14-40; and the agarose gel electrophoresis results for the self-assembled product of the universal carrier DNA3-TY are shown in FIG. 14-41.

Example 15

The drug CCPD3; the specific sequences are set forth in Table 5.

1. Materials and Reagents:

(1) Sequence a;

(2) Sequence b;

(3) Sequence c;

(4) TMS aqueous solution;

(5) TBM (Tris-borate-magnesium) buffer.

2. Experimental Method:

1) Sequence a, Sequence b, and Sequence c were separately dissolved in TMS aqueous solution and then combined at a molar ratio of 1:1:1. After mixing, the pH of the reaction mixture was adjusted to 8.8. The mixture was heated to 99°C and maintained for 5 minutes, then cooled at a rate of 5°C/min to 65°C and held for 3 minutes, followed by further cooling at 5°C/min to 2°C to obtain Self-Assembled Product 1. The HPLC chromatographic analysis results for Self-Assembled Product 1 are shown in FIG. 15-1.
2) The conditions in 1) were maintained unchanged, except that the feed amounts of sequence a, sequence b, and sequence c were adjusted to 95%, 100%, and 90%, respectively, of those used in (1). Self-assembly afforded Product 2. The HPLC chromatographic analysis results are shown in FIG. 15-2.
3) The conditions in 1) were maintained unchanged, except that the TMS aqueous solution was replaced with an aqueous sodium chloride solution. Self-assembly afforded Product 3. The HPLC chromatographic analysis results are shown in FIG. 15-3.
The preparation of the aqueous sodium chloride solution is set forth in Table 14.

Table 14

| Name | Concentration | Molecular weight (g/mol) | Amount (g) | Ultrapure water |
|---|---|---|---|---|
| Tris-HCl | 50 mmol/L | 157.6 | 7.88 | q.s. to 1 L |
| NaCl | 100 mmol/L | 58.44 | 5.84 | |
| $MgCl_2$ | 10 mmol/L | 95.21 | 0.95 | |

4) Under the same experimental conditions as in item 1), except that the TMS aqueous solution was replaced with purified water, self-assembly afforded Product 4. The HPLC chromatogram is shown in FIG. 15-4.
5) Using the melting-curve method (see the steps of Example 5), Self-Assembled Product 1 obtained in item 1) was tested. The results-melting peak and melting curve-are shown in FIG. *15-5.*
6) Under the same experimental conditions as in item 1), except that the denaturation temperature was changed from 99 °C to 85 °C, self-assembly afforded Product 5. The melting peak and melting curve are shown in FIG. 15-6.
7) Under the same experimental conditions as in item 1), except that the incubation (holding) condition was set to 67 °C for 5 min, self-assembly afforded Product 6. The melting peak and melting curve are shown in FIG. 15-7.
8) Under the same experimental conditions as in item 1), except that the incubation (holding) step was omitted (i.e., no holding at temperature), self-assembly afforded Product 7. The melting peak and melting curve are shown in FIG. 15-8.
9) Under the same experimental conditions as in item 1), except that during cooling of the reaction system to the incubation temperature the cooling rate was set to 10 °C/min, self-assembly afforded Product 8. The melting peak and melting curve are shown in FIG. 15-9.
10) Under the same experimental conditions as in item 1), except that during cooling of the reaction system to the incubation temperature the cooling rate was set to 2 °C/min, self-assembly afforded Product 9. The melting peak and melting curve are shown in FIG. 15-10.
11) Under the same experimental conditions as in item 1), except that the final cooling temperature was set to 25 °C, self-assembly afforded Product 10. The melting peak and melting curve are shown in FIG. 15-11.
12) Under the same experimental conditions as in item 1), except that the final cooling temperature was set to 25 °C, self-assembly afforded Product 11. The melting peak and melting curve are shown in FIG. 15-12.

13) Under the same experimental conditions as in item 1), except that the pH of the reaction mixture was set to 5.4, self-assembly afforded Product 12. The melting peak and melting curve are shown in FIG. 15-13.

14) Under the same experimental conditions as in item 1), except that the pH of the reaction mixture was set to 8.8, self-assembly afforded Product 13. The melting peak and melting curve are shown in FIG. 15-14.

**Pharmacodynamic Evaluation of Nucleic Acid Nanocarrier Combinations Carrying Immunomodulatory and Gene-Regulatory Drugs**

The specific structures or compositions corresponding to the drug names used in the Examples of this section are as described in the Detailed Description (Specific Embodiments) of the present invention.

Example 16

I. Definitions of Abbreviations

B16F10: murine B16F10 melanoma

T/C (%): relative tumor growth (treated/control)

$IR_{Tw}$%: tumor-weight inhibition rate

RTV: relative tumor volume

II. Objective of the Test:

To verify the anticancer effect of nucleic acid nanocarrier combinations carrying immunomodulatory and gene-regulatory drugs, and to compare differences in efficacy in inhibiting the growth of B16F 10 murine melanoma at different doses and via different routes of administration.

III. Principal Instruments:

Table 15

| Name | Model | Manufacturer |
|---|---|---|
| Thermostatic incubator | Thermo Forma 300118-280 | Thermo Fisher Scientific |
| Eletronic Digital Caliper | SANTO: 160950 | Shanghai Saituo Hardware Tools Co.,Ltd. |
| Electronic Balance | 270-9210 | Swiss Parg Oerlikon Co., Ltd. |
| Electronic Scale | Weiheng | Guangdong Weiheng Electronics Co., Ltd |
| Venous visual mouse tail fixator | GEGD-Q9G | Globalebio (Beijing) Technology Co., Ltd |
| Disposable Syringes | 1.0mL | BD Biosciences |
| Glass syringes | 0.25mL | Flying Pigeon Brand |

IV Experimental Animal Information

Species: C57BL/6J

Age: 6-7 weeks

Sex: male

Body weight: 15-17 g

Supplier: Beijing Huazhuokang Biotechnology Co., Ltd.

V. Experimental Scheme:

The animal inoculation and dosing regimens are set forth in Tables 16 and 17.

Table 16 - Animal Inoculation and Dosing Regimen

| Cell Line | No. of Mice | Mouse ID code | Inoculation site | No. of cells inoculated |
|---|---|---|---|---|
| B16F10 | 32 | See grouping table | Left axillodorsal s.c. | $1 \times 10^6$/mouse |
| B16F10 | 26 | See grouping table | bilateral axillodorsal s.c. | $1 \times 10^6$/unilateral /mouse |
| Note: The day of inoculation was designated as Day 0 (D0). | | | | |

Table 17 Animal grouping and drug administration

| Model | Groups | No. of animals | Treatment | Route of Administration | Dosing Volume | No. of administration | Dosing interval |
|---|---|---|---|---|---|---|---|
| B16F10 | Control (Negative control 3) | 10 (left tumor) | PBS | Tail vein injection | 200 μL | 5 | The grouping day (Day 1), Day 3, Day 5, Day 6, Day 7 |
| B16F10 | A | 10 (left tumor) | A | Tail vein injection | 200 μL | 5 | |
| B16F10 | B | 10 (left tumor) | B | Tail vein injection | 200 μL | 5 | |
| B16F10 | Blank (Negative control 1) | 6 (bilateral tumor) | -- | -- | -- | -- | |
| B16F10 | Control (Negative control 2) | 10 (bilateral tumor) | PBS | Left intratumoral injection | 20 μL for the first and second, 50 μL for the third to fifth administration | 5 | The grouping day (Day 1), Day 3, Day 5, Day 6, Day 7 |
| B16F10 | C | 10 (bilateral tumor) | C | Left intratumoral injection | 20 μL for the first and second, 50 μL for the third to fifth administration | 5 | |

Drug A under the "Treatment" column in Table 17 is 2*CpG 1826-2*Mannose-DNA carrier (50 μg) + 2*AmiR-21-AS1411 aptamer-DNA carrier (6 μg); Drug B is 2*CpG 1826-2*Mannose-DNA carrier (16 μg) + 2*AmiR-21-AS1411 aptamer-DNA carrier (6 μg); Drug C is 2*CpG 1826-2*Mannose-DNA carrier (50 μg) + 2*AmiR-21-AS1411 aptamer-DNA carrier (6 μg). This indicates that Drug A, Drug B, and Drug C are all Combination 1 (Drug 2 + Drug 21).

VI. Experimental Methods

1. Inoculation and Grouping

A murine B16F10 melanoma model was established using C57BL/6J male mice weighing 15.0-17.0 g. At the time of the experiment, well-growing tumor tissue was harvested, minced, and homogenized; a tumor cell suspension was prepared by dilution with sterile normal saline. B16F10 melanoma cells at $1 \times 10^6$ cells/0.2 mL were inoculated subcutaneously in the left axillary dorsal region of each mouse for the tail-vein dosing cohort, and subcutaneously in the left and right axillary dorsal regions of each mouse for the intratumoral-injection cohort. The day of inoculation was designated Day 0. On Day 5, the intravenous (tail-vein) dosing cohort was randomized according to left-side tumor volume into three groups: Negative Control-3 (PBS), Drug A, and Drug B, with 10 animals per group. The intratumoral-injection cohort was randomized according to right-side tumor volume into three groups: Negative Control-1 (no PBS and no drug), Negative Control-2 (PBS), and Drug C; Negative Control-1 included 6 animals, and each of the remaining groups included 10 animals. On the day of grouping, all animals commenced dosing by tail-vein injection and intratumoral injection, for a total of five administrations. Details of animal inoculation, grouping, and dosing are set forth in Tables 16 and 17.

2. Endpoints/Measurements

a) General clinical observations: The clinical condition of mice was observed three times per week during the study, including assessment of tumor nodule ulceration, general appearance/behavior, coat condition, and food intake.

b) Body weight: Mouse body weight was recorded three times per week.

c) Tumor volume: Tumor volume was measured three times per week using vernier calipers. The longest diameter (length) and the perpendicular short diameter (width) were measured, and tumor volume was calculated as: Volume = $0.5 \times$ length $\times$ (short diameter)$^2$.

$$\text{Tumor Growth Inhibition (TGI, \%)} = (1 - \text{T (tumor volume)} / \text{C (tumor volume)}) \times 100\%.$$

T/C (%) = RTV of treatment group / RTV of control group.
If TGI $\geq$ 60% and, upon statistical analysis, the tumor volume of the treatment group is significantly lower than that of the vehicle control group (P < 0.05), the treatment is deemed effective, i.e., it exhibits a significant inhibitory effect on tumor growth.

d) Tumor weight and photography: At study termination (D12), the remaining animals were euthanized by cervical dislocation, tumors were excised and weighed, and tumor photographs were taken for documentation. The tumor-weight inhibition rate (IRTW, %) was calculated, taking IRTW% > 60% as a reference indicator of efficacy. The calculation is as follows:

$$\text{IRTW (\%)} = (W_{\text{vehicle control}} - W_{\text{treatment}}) / W_{\text{vehicle control}} \times 100,$$

where W denotes tumor weight.

3. Statistical Analysis

Results were expressed as mean $\pm$ standard deviation (Mean $\pm$ SD). Graphs were generated and statistical analyses were performed in Excel using Student's t-test. Differences were considered statistically significant at P < 0.05.

VII. Experimental Results:

1. Clinical observations of test animals

Throughout the dosing period, animals exhibited good general condition with respect to activity and food intake. In the intravenous-dosing Group A, a death occurred on Day 9 following tail-vein injection; necropsy revealed no abnormalities, and ocular coloration was normal.

2. Changes in body weight of test animals

During the dosing period, body weight in all animals increased to a certain extent; however, compared with Negative Control Group 2, animals in the Drug C group showed less weight gain, and the difference was statistically significant. Body-weight changes for all animals are shown in Table 18 and FIGS. 16-1 and 16-2 ("Weight": body weight;

"Days after transplantation": days post-inoculation).

Table 18 Summary of Animal Body Weight

| Groups | No. of animals (n) | | Body weight (g) | | Body weight change (g) |
|---|---|---|---|---|---|
| | Before administration | D12 | Before administration | D12 | |
| Negative control group 3 | 10 | 10 | 16.1±0.7 | 19.2±1.6 | +3.1 |
| A | 10 | 9 | 16.0±0.5 | 18.9±1.2 | +2.9 |
| B | 10 | 10 | 16.2±0.8 | 18.8±1.6 | +2.7 |
| Negative control group 1 | 6 | 6 | 16.2±1.0 | 18.5±1.9 | +2.4 |
| Negative control group 2 | 10 | 10 | 16.2±1.0 | 19.6±1.5 | +3.4 |
| C | 10 | 10 | 16.1±0.8 | 18.3±1.1* | +2.2 |
| * P < 0.05, compared with Negative Control Group 2. Note: D12 denotes 12 days after inoculation of the tumor cell suspension. | | | | | |

3. Results of Tumor-Volume Inhibition

All animals were closely monitored for tumor growth throughout the study. Tumors were measured three times per week, results were recorded, and tumor growth inhibition rates were calculated.

Tail-vein administration cohort (tumor): Tumors in Negative Control Group 3 exhibited good growth. Compared with Negative Control Group 3, Group A and Group B showed tumor growth inhibition rates on D12 (calculated from tumor volume) of 54.14% and 51.67%, respectively-both < 60%; the T/C values (calculated from RTV) were 46.52% and 48.03%, respectively. In Group B, the tumor growth inhibition rate on D11 was 60.92% (> 60%), with T/C = 39.08%. The results are compiled in Tables 19-20, and the tumor growth curves are shown in FIGS. 16-3 and 16-4 ("Tumor volume": tumor volume; "Days after transplantation": days post-inoculation; "RTV": relative tumor volume).

Table 19 Summary of Tumor Growth Data (tail-vein dosing, left-side tumor

| Days | Control (Negative control 3) | | Group A | | | Group B | | |
|---|---|---|---|---|---|---|---|---|
| | TV(mm$^3$) | N | TV (mm$^3$) | N | TGI (%) | TV (mm$^3$) | N | TGI (%) |
| 5 | 172.4±21.8 | 10 | 174.2±21.1 | 10 | / | 173.2±16.6 | 10 | / |
| 7 | 475.2±117.5 | 10 | 380.7±95.9 | 10 | 19.88 | 297.8±62.8*** | 10 | 37.32 |
| 9 | 804.8±299.4 | 10 | 410.1±144.0** | 10 | 49.01 | 378.7±168.3*** | 10 | 52.95 |
| 10 | 1105.0±415.8 | 10 | 534.9±221.4** | 9 | 50.78 | 471.1±167.2*** | 10 | 57.36 |
| 11 | 1508.3±418.2 | 10 | 659.6±311.7*** | 9 | 56.27 | 589.4±153.7*** | 10 | 60.92 |
| 12 | 1693.9±603.8 | 10 | 776.9±361.0** | 9 | 51.14 | 818.6±319.3*** | 10 | 51.76 |
| **: P < 0.01; ***: P < 0.001; compared with Negative Control Group 3. Note: Days: number of days after inoculation of the tumor cell suspension; N: number of animals in the group included in the statistical analysis. | | | | | | | | |

Table 20 Summary of Tumor Growth Data (tail-vein administration, left-side tumor).

| Days | Control (Negative control 3) | | Group A | | | Group B | | |
|---|---|---|---|---|---|---|---|---|
| | RTV | N | RTV | N | T/C(%) | RTV | N | T/C (%) |
| 5 | 1±0 | 10 | 1±0 | 10 | / | 1±0 | 10 | / |
| 7 | 2.74±0.56 | 10 | 2.19±0.51* | 10 | 79.92 | 1.73±0.40*** | 10 | 63.03 |
| 9 | 4.69±1.69 | 10 | 2.37±0.87** | 10 | 50.60 | 2.21±1.01*** | 10 | 47.03 |

(continued)

| Days | Control (Negative control 3) | | Group A | | | Group B | | |
|---|---|---|---|---|---|---|---|---|
| | RTV | N | RTV | N | T/C(%) | RTV | N | T/C (%) |
| 10 | 6.36±2.26 | 10 | 3.24±1.60** | 9 | 50.91 | 2.74±1.00*** | 10 | 43.10 |
| 11 | 8.72±2.09 | 10 | 3.89±2.12*** | 9 | 44.63 | 3.41±0.84*** | 10 | 39.08 |
| 12 | 9.82±3.36 | 10 | 4.57±2.35** | 9 | 46.52 | 4.72±1.76*** | 10 | 48.03 |
| *P < 0.05, **P < 0.01, ***P < 0.001; compared with Negative Control Group 3. Note: Days: number of days after inoculation of the tumor cell suspension; N: number of animals in the group included in the statistical analysis. | | | | | | | | |

Intratumoral injection administration (tumor):

Left-side tumor: Tumors in Negative Control Groups -1 and 2 grew well, and there was no significant difference in tumor growth between Negative Control Groups -1 and 2. Compared with Negative Control Group 2, Group C showed a tumor growth inhibition rate on D12 (calculated from tumor volume) of 52.69%, which was < 60%; the T/C value (calculated from RTV) was 46.00%.

Right-side tumor: Tumors in Negative Control Groups -1 and 2 grew well, and there was no significant difference between the two negative controls. Compared with Negative Control Group 2, Group C showed a tumor growth inhibition rate on D12 (calculated from tumor volume) of 57.73%, which was < 60%; the T/C value (calculated from RTV) was 42.27%.

The results are compiled in Tables 21-24, and the tumor growth curves are shown in FIGS. 16-5, 16-6, 16-7, and 16-8.

Table 21 - Summary of Tumor Growth Data (intratumoral injection, left-side tumor).

| Days | Blank (Negative control 1) | | Control (Negative control 2) | | Group C | | |
|---|---|---|---|---|---|---|---|
| | TV(mm³) | N | TV (mm³) | N | TV (mm³) | N | TGI (%) |
| 5 | 191.2±20.6 | 6 | 191.6±32.0 | 10 | 200.9±29.9 | 10 | / |
| 7 | 555.2±134.1 | 6 | 510.1±127.6 | 10 | 372.6±135.0* | 10 | 26.95 |
| 9 | 789.0±139.2 | 6 | 633.9±129.0 | 10 | 470.4±198.8* | 10 | 25.80 |
| 10 | 926.5±193.8 | 6 | 784.1±180.0 | 10 | 575.0±227.6* | 10 | 26.67 |
| 11 | 1253.9±344.4 | 6 | 1136.0±248.6 | 10 | 579.7±307.4*** | 10 | 48.97 |
| 12 | 1274.9±444.0 | 6 | 1127.3±418.3 | 10 | 533.4±387.3** | 10 | 52.69 |
| *P < 0.05, **P < 0.01, ***P < 0.001; compared with Negative Control Group 2. Note: Days: number of days after inoculation of the tumor cell suspension; N: number of animals in the group included in the statistical analysis. | | | | | | | |

Table 22 Summary of Tumor Growth Data (intratumoral administration, left-side tumor)

| Days | Blank (Negative Control 1) | | Control (Negative Control 2) | | Group C | | |
|---|---|---|---|---|---|---|---|
| | RTV | N | RTV | N | RTV | N | T/C (%) |
| 5 | 1±0 | 6 | 1±0 | 10 | 1±0 | 10 | / |
| 7 | 2.94±0.79 | 6 | 2.72±0.8 | 10 | 1.86±0.64* | 10 | 68.63 |
| 9 | 4.19±1.04 | 6 | 3.40±0.91 | 10 | 2.34±0.91 * | 10 | 68.89 |
| 10 | 4.94±1.46 | 6 | 4.16±1.04 | 10 | 2.91±1.23* | 10 | 69.98 |
| 11 | 6.69±2.25 | 6 | 6.06±1.54 | 10 | 2.91±1.63*** | 10 | 48.01 |

(continued)

| Days | Blank (Negative Control 1) | | Control (Negative Control 2) | | Group C | | |
|---|---|---|---|---|---|---|---|
| | RTV | N | RTV | N | RTV | N | T/C (%) |
| 12 | 6.83±2.75 | 6 | 5.99±2.12 | 10 | 2.75±2.32** | 10 | 46.00 |

*P < 0.05, **P < 0.01, ***P < 0.001; compared with Negative Control Group 2.
Note: Days: number of days after inoculation of the tumor cell suspension; N: number of animals in the group included in the statistical analysis.

Table 23 - Summary of Tumor Growth Data (intratumoral administration, right-side tumor).

| Days | Blank (Negative control 1) | | Control (Negative control 2) | | Group C | | |
|---|---|---|---|---|---|---|---|
| | TV(mm³) | N | TV (mm³) | N | TV (mm³) | N | TGI (%) |
| 5 | 168.9±27.2 | 6 | 172.1±18.5 | 10 | 175.3±22.5 | 10 | / |
| 7 | 424.5±83.0 | 6 | 482.2±86.7 | 10 | 306.6±77.3*** | 10 | 36.41 |
| 9 | 545.2±62.3 | 6 | 680.0±190.7 | 10 | 430.7±155.4** | 10 | 36.66 |
| 10 | 771.6±213.6 | 6 | 758.7±270.3 | 10 | 405.9±186.9** | 10 | 46.50 |
| 11 | 1103.8±171.6 | 6 | 1225.0±232.1 | 10 | 531.0±258.1*** | 10 | 56.65 |
| 12 | 1091.4±293.7 | 6 | 1191.2±364.3 | 10 | 503.5±224.2*** | 10 | 57.73 |

**: P < 0.01; ***: P < 0.001; compared with Negative Control Group 2.
Note: Days: number of days after inoculation of the tumor cell suspension; N: number of animals in the group included in the statistical analysis.

Table 24 Summary of Tumor Growth Data (intratumoral administration, right-side tumor)

| Days | Blank (negative control-1) | | Control (negative control 2) | | C | | |
|---|---|---|---|---|---|---|---|
| | RTV | N | RTV | N | RTV | N | T/ C (%) |
| 5 | 1±0 | 6 | 1±0 | 10 | 1±0 | 10 | / |
| 7 | 2.52±0.36 | 6 | 2.86±0.74 | 10 | 1.78±0.52** | 10 | 62.12 |
| 9 | 3.26±0.33 | 6 | 4.01±1.30 | 10 | 2.48±0.90** | 10 | 61.90 |
| 10 | 4.50±0.63 | 6 | 4.41±1.57 | 10 | 2.39±1.25** | 10 | 54.31 |
| 11 | 6.55±0.46 | 6 | 7.19±1.59 | 10 | 3.10±1.68*** | 10 | 43.16 |
| 12 | 6.40±0.80 | 6 | 7.00±2.27 | 10 | 2.96±1.49*** | 10 | 42.27 |

**: P < 0.01; ***: P < 0.001; compared with Negative Control Group 2.
Note: Days: number of days after inoculation of the tumor cell suspension; N: number of animals in the group included in the statistical analysis.

4. Tumor-Weight Results

At 12 days after B16F10 inoculation, all animals were euthanized by cervical dislocation; tumors were excised, weighed, and photographed. The results are summarized in Tables 25-26 and FIGS. 16-9 and 16-10 ("Tumor weight": tumor weight; "left tumor": left-side tumor; "right tumor": right-side tumor).

For the tail-vein administration cohort, the tumor-weight inhibition rates in Group A and Group B were 41.93% and 50.58%, respectively.

For the intratumoral-injection cohort, the tumor-weight inhibition rate for the left-side tumor was 55.41%, and for the right-side tumor was 53.00%.

Table 25 - Summary of Tumor Weight (tail-vein administration).

| Group | Number of animals | Left tumor weight (g) | Left IR_tw % |
|---|---|---|---|
| Control (negative control 3) | 10 | 1.55±0.68 | / |
| A | 9 | 0.90±0.46* | 41.93 |
| B | 10 | 0.77±0.28** | 50.58 |
| *: P < 0.05; **: P < 0.01; compared with Negative Control Group 3. | | | |

Table 26 Summary of Tumor Weight (intratumoral administration)

| Group | Number of animals | Left tumor weight (g) | Left IR_tw % | Right tumor weight (g) | Right IR_tw % |
|---|---|---|---|---|---|
| Blank (negative control 1) | 6 | 1.63±0.63 | / | 1.32±0.39 | / |
| Control (negative control 2) | 10 | 1.45±0.49 | / | 1.48+0.64 | / |
| C | 10 | 0.65+0.38*** | 55.41 | 0.68±0.33** | 53.00 |
| Note: **: P<0.01, ***: P<0.001, compared with negative control group 2. | | | | | |

VIII. Experimental Conclusion:

Taking into comprehensive consideration the tumor growth inhibition rate and the tumor-weight inhibition rate, the conclusions are as follows:

In the tail-vein administration group, although the tumor inhibition rates at the end of the study (D12), calculated on the basis of tumor weight and tumor volume, did not exceed 60%, the results indicate that Drug A and Drug B each exerted a certain inhibitory effect on the growth of B16F10 tumors, with statistical significance; analysis of data obtained during the study further showed that the inhibitory effect on D11 was stronger than on D12. In the intratumoral injection group, Drug C exhibited a certain growth-inhibitory effect, with statistical significance, on both the injection-side tumor (left tumor) and the contralateral tumor (right tumor), but the tumor inhibition rate (calculated from tumor weight and tumor volume) did not exceed 60%; analysis of the study data indicated that, as the dosing period was extended, the tumor-growth inhibitory effect of Drug C progressively increased.

Example 17

I. Objective:

The primary objective of this study was to evaluate the inhibitory effects of five drugs on CT26 syngeneic allograft tumors.

II. Cell Information

CT26, murine colon carcinoma cell line; purchased from Aili Biotechnology (Shanghai) Co., Ltd.

III. Experimental Animal Information

Species: BALB/c mice (immunocompetent)

Age: 6-7 weeks

Sex: female

Body weight: 17-20 g

Supplier: Beijing Vital River Laboratory Animal Technology Co., Ltd.

IV Methods

1. Cell culture

Tumor cells were cultured in RPMI-1640 medium supplemented with heat-inactivated 10% fetal bovine serum, 100 U/mL penicillin, 100 μg/mL streptomycin, and 2 mM L-glutamine in a humidified incubator at 37 °C with 5% $CO_2$. When cells reached confluence (every 3-4 days), they were passaged; cells in the logarithmic growth phase were used for in vivo tumor inoculation.

2. Inoculation and grouping

CT26 cells at $1\times10^6$ cells/0.2 mL were inoculated subcutaneously into the left and right nuchal (cervical dorsal) regions of female BALB/c mice (with some animals inoculated on the right side only). The day of inoculation was designated D0. On D16, animals were randomized by right-side tumor volume into four groups: A (PBS), B, C, and D. On the day of randomization, all groups commenced intratumoral dosing, administered five times on an every-other-day schedule. In addition, beginning D18, animals in Group D received tail-vein injections of the drug, for a total of seven administrations, twice per week.

Details of animal inoculation, grouping, and dosing are provided in **Table 27** and **FIG. 17-1.**

Table 27 - Animal Inoculation and Dosing Plan

| (The day of inoculation is designated as D0.) | | | | |
|---|---|---|---|---|
| Cell line | Mice (n) | Mouse Nos. | Inoculation site | Inoculum |
| CT26 | 20 | 28~47 | Subcutaneous, left and right cervical-dorsal regions | $1\times10^6$ cells/mouse |
| CT26 | 6 | 48~53 | Subcutaneous, right cervical-dorsal region | $1\times10^6$ cells/mouse |

Table 28 - Animal Grouping and Dosing

| Model | Group | Animals (n) | Treatment | Route of administratio n | Dose volume per administration | No. of administrations | Dosing interval |
|---|---|---|---|---|---|---|---|
| CT26 | A | 2 (bilateral tumors) | PBS | Intratumoral, right-side tumor | 50ul | 5 | Day of grouping, and Days 3, 5, 7, 9 |
| CT26 | B | 2 (bilateral tumors) | CT26-B | Intratumoral, right-side tumor | 50ul | 5 | Day of grouping, and Days 3, 5, 7, 9 |
| CT26 | C | 2 (unilateral tumor) | CT26-C | Intratumoral | 50ul | 5 | Day of grouping, and Days 3, 5, 7, 9 |
| CT26 | D | 1 (bilateral tumors) | CT26-D | Intratumoral, right-side | 50ul | 5 | Day of grouping, and Days 3, 5, 7, 9 |
| | | | | tumor → and tail vein | 200ul | 8 | Day of grouping, and Days 3, 5, 7, 9 (intratumoral); twice weekly for four weeks (tail vein) |

Wherein: Group A received PBS; Group B received 3 ×CpG2006-DNA carrier (50 μg); Group C received 2×AmiR-21-TTA1 aptamer-DNA carrier (12 μg); Group D received 3*CpG2006-DNA carrier (50 μg) + 2*AmiR-21-TTA1 aptamer-DNA carrier (12 μg) + TTA1 aptamer-epirubicin-4*biotin-DNA carrier (0.12 mg). That is, Drug B corresponds to Drug 1 in Table 5; Drug C corresponds to Drug 18 in Table 5; and Drug D corresponds to Drug 1 + Drug 18 + Drug 88 in Table 5.

3. Endpoints/Measurements

Same as in Example 16.

4. Statistical Analysis

Same as in Example 16.

V. Experimental Results:

1. Clinical observations of test animals

During the dosing period, animals exhibited good general condition with respect to activity and food intake. Around D26, tumors began to ulcerate and form scabs. In Group D, desquamation and necrosis of the mid-tail were observed starting on D37, which was considered likely due to the relatively strong irritancy of the drug administered by tail-vein injection.

2. Changes in body weight of test animals

During the dosing period, body weight in all animals increased to some extent; however, the number of animals was too small for statistical analysis, and therefore only the body-weight data were summarized. Body-weight changes for all animals are shown in Table 29 and FIG. 17-2 ("weight": body weight; "Days after inoculation": days post-inoculation).

Table 29 Summary of animal body weight

| Group | Number of animals (n) | Before administration | D40 | Change in body weight (g) |
|---|---|---|---|---|
| A | 2 | 19.9±0 | 33.7±2.2 | +13.8 |
| | 2 | 19.6±1.8 | 31.3±1.8 | +11.8 |
| B C | 2 | 18.6±0.5 | 24.3±2.3 | +5.8 |
| D | 1 | 18.0 | 20.3 | +2.3 |

Note: D40, i.e., 40 days after tumor cell inoculation.

3. Results of Tumor-Volume Inhibition

Throughout the study, tumor growth was closely monitored; tumors were measured three times per week, results were recorded, and tumor growth inhibition rates were calculated. The results are summarized in Tables 30-31, and the tumor growth curves are shown in FIGS. 17-3 and 17-4.

Right-side tumor: Tumors in the control group grew well. Compared with the control group, Group B showed a tumor growth inhibition rate of 29.7% on D40 (<60%). Group C exhibited tumor growth inhibition rates of approximately 64%-65% on D26-D28 (>60%), but 42.1% on D40 (<60%). Group D exhibited tumor growth inhibition rates of approximately 66%-72% on D28-D40 (>60%).

Left-side tumor: Tumors in the control group grew well. Compared with the control group, the tumor growth inhibition rates on D40 were 5.6% and 40.6% in Groups B and D, respectively-both <60%.

Table 30 Summary of Tumor Growth Data (right side).

| Days | Group A | | Group B | | | Group C | | | Group D | | |
|---|---|---|---|---|---|---|---|---|---|---|---|
| | Tumor volume (mm$^3$) | N | Tumor volume (mm$^3$) | N | Tumor growth inhibition rate (%) | Tumor volume (mm$^3$) | N | Tumor growth inhibition rate (%) | Tumor volume (mm$^3$) | N | Tumor growth inhibition rate (%) |
| 16 | 296.8+119.3 | 2 | 272.8±65.1 | 2 | / | 384.5+233 | 2 | / | 264.1 | 1 | / |
| 19 | 837.7+235.3 | 2 | 531.5+131.1 | 2 | 31.0 | 660.5±284.3 | 2 | 39.1 | 354.9 | 1 | 52.4 |
| 21 | 1296.9+468.8 | 2 | 713.6+260.9 | 2 | 40.1 | 814.5±286.3 | 2 | 51.5 | 449.6 | 1 | 61.0 |
| 23 | 1657.9±300.6 | 2 | 959.3+365.7 | 2 | 37.0 | 1007.1+291.9 | 2 | 53.1 | 822.1 | 1 | 44.3 |
| 26 | 2815.1±629.4 | 2 | 1414.3±115.7 | 2 | 45.3 | 1306.7±372.3 | 2 | 64.2 | 1003.7 | 1 | 59.9 |
| 28 | 3461.5±297.5 | 2 | 1878.7±148.1 | 2 | 40.9 | 1553.6±503.5 | 2 | 65.4 | 964.7 | 1 | 68.7 |
| 30 | 4034.2±688.8 | 2 | 2383.1±369.4 | 2 | 35.7 | 2369±941.4 | 2 | 54.7 | 1205.2 | 1 | 66.4 |
| 33 | 5806.2±1592.9 | 2 | 3500.2±195.1 | 2 | 34.4 | 3265.7±1199.1 | 2 | 56.6 | 1489.6 | 1 | 71.2 |
| 35 | 6175.8±786 | 2 | 3918.7±90.9 | 2 | 31.0 | 4152.8±1637.2 | 2 | 48.1 | 1570.1 | 1 | 71.4 |
| 37 | 7607±1666 | 2 | 4532.6±205.2 | 2 | 35.2 | 5196±2541.9 | 2 | 47.3 | 1998.1 | 1 | 70.5 |
| 40 | 9228.5±3110.7 | 2 | 5961.9±653.7 | 2 | 29.7 | 6919±3376.1 | 2 | 42.1 | 2290.2 | 1 | 72.1 |
| Note: Days: number of days after inoculation of the tumor cell; N: number of animals in the group included in the statistical analysis. | | | | | | | | | | | |

Table 31 Summary of tumor growth data (left part)

| Days | Group A | | Group B | | | Group D | | | |
|---|---|---|---|---|---|---|---|---|---|
| | Tumor volume (mm$^3$) | N | Tumor volume (mm$^3$) | N | Tumor growth inhibition rate (%) | Tumor volume (mm$^3$) | N | Tumor growth inhibition rate (%) | Tumor volume (mm$^3$) |
| 16 | 331.4±138.2 | 2 | 351.1±121.9 | 2 | / | 248.3 | 1 | / | 507.5 |
| 19 | 745.6±103.6 | 2 | 698.2±205.8 | 2 | 11.6 | 432.3 | 1 | 22.6 | 1111.9 |
| 21 | 1077.4±32.8 | 2 | 1059.8±429.8 | 2 | 7.1 | 801.5 | 1 | 0.7 | 1459.9 |
| 23 | 1654.6±442.4 | 2 | 1325.1±442.7 | 2 | 24.4 | 1221.4 | 1 | 1.5 | 2305.8 |
| 26 | 1951.9±417.5 | 2 | 1893.3±686.9 | 2 | 8.4 | 1636.0 | 1 | -11.9 | 3568.5 |
| 28 | 2639.4±492.8 | 2 | 2365.5±625.6 | 2 | 15.4 | 1793.6 | 1 | 9.3 | 4358.5 |
| 30 | 3387.3±296 | 2 | 3110.6±617.2 | 2 | 13.3 | 2195.1 | 1 | 13.5 | 5484.9 |
| 33 | 6198.8±855.8 | 2 | 4642.5±517 | 2 | 29.3 | 2669.5 | 1 | 42.5 | 7172.3 |
| 35 | 6753.3±238.2 | 2 | 5364.2±991 | 2 | 25.0 | 2646.8 | 1 | 47.7 | 7706.3 |
| 37 | 8034±14.2 | 2 | 6777.5±603.7 | 2 | 20.4 | 3086.2 | 1 | 48.7 | 8653.8 |
| 40 | 8321.8±461.7 | 2 | 9303.7±917.8 | 2 | -5.6 | 3702.8 | 1 | 40.6 | 9734.2 |

4. Results of Tumor-Weight Inhibition

At 40 days after inoculation of CT26 cells, all animals were euthanized; tumors were excised, weighed, and photographed. The results are summarized in Table 32 and FIG. 17-5.

For Group D, the right-side tumor-weight inhibition rate (IRTW%) was 82.59%, and the left-side IRTW% was 66.42%, both > 60%. The IRTW% values for the remaining groups were all < 60%.

Table 32. Summary of Tumor Weight

| Group | Anima ls (n) | Right tumor weight (g) | Right IRTW (%) | Left tumor weight (g) | Left IRTW(%) |
|---|---|---|---|---|---|
| A(PBS) | 2 | 7.41±2.18 | / | 8.16±0.21 | / |
| B | 2 | 5.06±0.21 | 31.78 | 8.09±0.06 | 0.86 |
| C | 2 | 5.49±2.18 | 25.91 | / | / |
| D | 1 | 1.29 | 82.59 | 2.74 | 66.42 |

V. Experimental Results

1) Clinical observations and body weight: Drug administration did not cause abnormalities in body weight or clinical signs.

Right-side tumor (volume) results: Tumors in the control group grew well. Relative to the control group, the tumor-growth inhibition rate on D40 was 29.7% in Group B; in Group C it was approximately 64%-65% on D26-D28 (>60%) but 42.1% on D40 (<60%); in Group D it was approximately 66%-72% on D28-D40 (>60%).

Right-side tumor (weight) results: The IRTW% for Group D was 82.59%; the tumor-weight inhibition rates for the remaining groups were all < 60%.

Left-side tumor (volume) results: Tumors in the control group grew well. Relative to the control group, the tumor-growth inhibition rates on D40 were -5.6% and 40.6% for Groups B and D, respectively.

Left-side tumor (weight) results: The IRTW% for Group D was 66.42%; the tumor-weight inhibition rates for the remaining groups were all < 60%.

2) Conclusions:

Taking into comprehensive consideration the tumor-growth inhibition rate and the tumor-weight inhibition rate, the conclusions are as follows:

Right-side tumor: After five intratumoral injections administered concomitantly with seven tail-vein injections of a different drug, Group D showed a marked antitumor effect. Group C, after five intratumoral injections, exhibited an initially good inhibitory effect (>60%), which subsequently diminished. Group B, after five intratumoral injections, failed to effectively inhibit the transplanted tumor through the end of the study.

Left-side tumor: After five intratumoral injections administered concomitantly with seven tail-vein injections of a different drug, Group D showed a good antitumor effect. Groups B and C, after five intratumoral injections, failed to effectively inhibit the transplanted tumor through the end of the study.

The relatively large tumor volumes at the time of dosing may have had a certain impact on the antitumor efficacy of the test articles.

Example 18

I. Objective:

The primary objective of this study was to establish a CT26 tumor model and to verify the immunostimulatory effect and antitumor efficacy of the test articles; the specific objectives further included comparing differences in efficacy (pharmacodynamics) among different drug combinations, routes of administration, and dosing levels.

II. Cell Information

CT26, murine colon carcinoma cell line; purchased from Aili Biotechnology (Shanghai) Co., Ltd.

III. Experimental Animal Information

Species: BALB/c mice (immunocompetent)

Age: 6 weeks

Sex: female

Body weight: 17-23 g

Supplier: National Institutes for Food and Drug Control (NIFDC), China

IV. Methods

1. Cell culture

Tumor cells were cultured in RPMI-1640 medium supplemented with heat-inactivated 10% fetal bovine serum, 100 U/mL penicillin, 100 $\mu$g/mL streptomycin, and 2 mM L-glutamine in a humidified incubator at 37 °C with 5% $CO_2$. Cells were passaged every 3-4 days when confluent, and cells in the logarithmic growth phase were used for in vivo tumor inoculation.

2. Inoculation and grouping

CT26 cells at $0.84 \times 10^6$ cells/0.2 mL were inoculated subcutaneously into the left and right cervical-dorsal regions of female BALB/c mice (some animals were inoculated on the right side only). The day of inoculation was designated D0. On D7, animals were randomized by right-side tumor volume into groups Negative Control-2 (PBS), A, B, C, D, E, F, and G. On the day of randomization, all groups commenced intratumoral dosing or tail-vein dosing (administration sites are shown in Table 35), for a total of five administrations, every other day.

On D9, the remaining animals were further randomized by right-side tumor volume into Groups H and I. On the same day, both groups commenced intratumoral dosing (administration sites as shown in Table 35), for a total of five administrations, every other day.

Details of animal inoculation, grouping, and dosing are provided in Tables 33 and 34 and FIG. 18-1.

Table 33 - Animal Inoculation and Dosing Plan

| Animal inoculation (the day of inoculation is designated D0) | | | | |
|---|---|---|---|---|
| Cell line | Mice (n) | Mouse Nos. | Inoculation site | Inoculum |
| CT26 | 71 | 487-500, 101-159 | Subcutaneous, left and right cervical-dorsal regions | $0.84 \times 10^6$ cells/-mouse |
| CT26 | 9 | 478-486 | Subcutaneous, right cervical-dorsal region | $0.84 \times 10^6$ cells/-mouse |

Table 34. Animal Grouping and Dosing Details

| Group | Animals (n) | Test article | Route of administration | Dosing schedule and dose volume |
|---|---|---|---|---|
| Negative | 6 | PBS | Intratumoral | Day of grouping 20 $\mu$L; |
| Control-2 (PBS) | | | | Day 3 20 $\mu$L; Day 5 50 $\mu$L; Day 7 50 $\mu$L; Day 9 50 $\mu$L |
| A | 6 | A | Intratumoral | Day of grouping 20 $\mu$L; Day 3 20 $\mu$L; Day 5 50 $\mu$L; Day 7 50 $\mu$L; Day 9 50 $\mu$L |
| B | 6 | B | Intratumoral | Day of grouping 20 $\mu$L; Day 3 20 $\mu$L; Day 5 50 $\mu$L; Day 7 50 $\mu$L; Day 9 50 $\mu$L |
| C | 5 | C | Intratumoral | Day of grouping 20 $\mu$L; Day 3 20 $\mu$L; Day 5 50 $\mu$L; Day 7 50 $\mu$L; Day 9 50 $\mu$L |
| D | 6 | D | Intratumoral | Day of grouping 20 $\mu$L; Day 3 20 $\mu$L; Day 5 50 $\mu$L; Day 7 50 $\mu$L; Day 9 50 $\mu$L |
| E | 6 | E | Intratumoral | Day of grouping 20 $\mu$L; Day 3 20 $\mu$L; Day 5 50 $\mu$L; Day 7 50 $\mu$L; Day 9 50 $\mu$L |
| F | 6 | F | Intratumoral | Day of grouping 20 $\mu$L; Day 3 20 $\mu$L; Day 5 50 $\mu$L; Day 7 50 $\mu$L; Day 9 50 $\mu$L |
| G | 6 | G | Intratumoral | Day of grouping 20 $\mu$L; Day 3 20 $\mu$L; Day 5 50 $\mu$L; Day 7 50 $\mu$L; Day 9 50 $\mu$L |
| H | 4 | H | Intratumoral | Day of grouping 20 $\mu$L; Day 3 20 $\mu$L; Day 5 50 $\mu$L; Day 7 50 $\mu$L; Day 9 50 $\mu$L |
| I | 4 | I | Intratumoral | Day of grouping 20 $\mu$L; Day 3 20 $\mu$L; Day 5 50 $\mu$L; Day 7 50 $\mu$L; Day 9 50 $\mu$L |

Wherein, Group A includes 2*CpG1826-CD40 aptamer-DNA carrier (50 $\mu$g) + 2*AmiR-21-TTA1 aptamer-DNA carrier (6 $\mu$g) + OX40 aptamer (DNA form) (6 $\mu$g); Group B includes 2*CpG1826-CD40 aptamer-DNA carrier (10 $\mu$g) + 2*AmiR-21-TTA1 aptamer-DNA carrier (6 $\mu$g) + OX40 aptamer (DNA form) (6 $\mu$g); Group C includes 2*CpG1826-CD40 aptamer-DNA carrier (3 $\mu$g) + 2*AmiR-21-TTA1 aptamer-DNA carrier (3 $\mu$g) + OX40 aptamer (DNA form) (6 $\mu$g); Group D includes 2*CpG1826-CD40 aptamer-DNA carrier (50 $\mu$g) + 2*AmiR-21-TTA1 aptamer-DNA carrier (6 $\mu$g) + OX40 aptamer (DNA form) (6 $\mu$g); Group E includes 2*CpG1826-CD40 aptamer-DNA carrier (50 $\mu$g) + 2*AmiR-21-TTA1 aptamer-DNA carrier (6 $\mu$g); Group F includes 2*CpG1826-CD40 aptamer-DNA carrier (50 $\mu$g) + OX40 aptamer (DNA form) (6 $\mu$g); Group G includes 2*CpG1826-CD40 aptamer-DNA carrier (50 $\mu$g); Group H includes 2*AmiR-21-TTA1 aptamer-DNA carrier (6 $\mu$g); and Group I includes OX40 aptamer (DNA form) (6 $\mu$g). That is, Drugs A-D correspond to Combination 21) (Drug 3 + Drug 18 + OX40 aptamer in DNA form); Drug E corresponds to Combination 22) (Drug 3 + Drug 18); Drug F corresponds to Combination 23) (Drug 3 + OX40 aptamer in DNA form); Group G corresponds to Drug 3 in Table 5; and Group H corresponds to Drug 18.

Table 35 Routes and Sites of Administration in Animals

| Group | Animal No. | Route / Site | Group | Animal No. | Route / Site |
|---|---|---|---|---|---|
| Negative Control-2 (PBS) | 499 | Intratumoral administration to the **left** tumor | E | 103 | Intratumoral administration to the **left** tumor |
| Negative Control-2 (PBS) | 142 | Intratumoral administration to the **left** tumor | E | 109 | Intratumoral administration to the **left** tumor |
| Negative Control-2 (PBS) | 492 | Intratumoral administration to the **left** tumor | E | 102 | Intratumoral administration to the **right** tumor |
| Negative Control-2 (PBS) | 112 | Intratumoral administration to the **right** tumor | E | 134 | Intratumoral administration to the **right** tumor |
| Negative Control-2 (PBS) | 151 | Intratumoral administration to the **right** tumor | E | 129 | Intratumoral administration to the **right** tumor |
| Negative Control-2 (PBS) | 108 | Intratumoral administration to the **left** tumor | E | 122 | Intratumoral administration to the **right** tumor |
| A | 145 | Intratumoral administration to the **right** tumor | F | 491 | Intratumoral administration to the **right** tumor |
| A | 487 | Intratumoral administration to the **right** tumor | F | 150 | Intratumoral administration to the **left** tumor |
| A | 153 | Intratumoral administration to the **right** tumor | F | 128 | Intratumoral administration to the **left** tumor |
| A | 136 | Intratumoral administration to the **right** tumor | F | 494 | Intratumoral administration to the **left** tumor |
| A | 148 | Intratumoral administration to the **right** tumor | F | 127 | Intratumoral administration to the **right** tumor |
| A | 159 | Intratumoral administration to the **right** tumor | F | 107 | Intratumoral administration to the **right** tumor |
| B | 110 | Intratumoral administration to the **left** tumor | G | 140 | Intratumoral administration to the **left** tumor |
| B | 144 | Intratumoral administration to the **right** tumor | G | 124 | Intratumoral administration to the **right** tumor |
| B | 105 | Intratumoral administration to the **left** tumor | G | 121 | Intratumoral administration to the **right** tumor |
| B | 120 | Intratumoral administration to the **right** tumor | G | 126 | Intratumoral administration to the **right** tumor |
| B | 137 | Intratumoral administration to the **left** tumor | G | 133 | Intratumoral administration to the **right** tumor |
| B | 111 | Intratumoral administration to the **right** tumor | G | 113 | Intratumoral administration to the **right** tumor |
| C | 143 | Intratumoral administration to the **right** tumor | H | 157 | Intratumoral administration to the **right** tumor |
| C | 141 | Intratumoral administration to the **right** tumor | H | 495 | Intratumoral administration to the **right** tumor |
| C | 202(139) | Intratumoral administration to the **right** tumor | H | 104 | Intratumoral administration to the **left** tumor |
| C | 117 | Intratumoral administration to the **right** tumor | H | 130 | Intratumoral administration to the **right** tumor |

(continued)

| Group | Animal No. | Route / Site | Group | Animal No. | Route / Site |
|---|---|---|---|---|---|
| C | 493 | Intratumoral administration to the **right** tumor | I | 158 | Intratumoral administration to the **left** tumor |
| D | 478 | Intravenous (tail vein) | I | 106 | Intratumoral administration to the **left** tumor |
| D | 233(479) | Intravenous (tail vein) | I | 489 | Intratumoral administration to the **left** tumor |
| D | 480 | Intravenous (tail vein) | I | 116 | Intratumoral administration to the **right** tumor |
| D | 481 | Intravenous (tail vein) | | | |
| D | 485 | Intravenous (tail vein) | | | |
| D | 486 | Intravenous (tail vein) | | | |

3. Endpoints/Measurements

Same as in Example 16.

4. Statistical Analysis

Same as in Example 16.

V. Experimental Results:

1. Clinical observations of test animals

During the dosing period, animals exhibited good general condition with respect to activity and food intake.

2. Reactions after dosing and changes in body weight

During the dosing period, body weight in all animals increased to a certain extent. At the study endpoint, there were no significant differences in body weight between any dosing group and the PBS control group (P > 0.05). Body-weight changes for all animals are shown in Table 36 and FIG. 18-2.

Table 36. Summary of Animal Body Weight

| Group | Animals (n) | Pre-dose | D20 | Change in body weight(g) |
|---|---|---|---|---|
| Negative Control-2 (PBS) | 6 | $19.9\pm1.2$ | $23.2\pm1.9$ | +3.2 |
| A | 6 | $20.3\pm0.5$ | $23.4\pm1.2$ | +3.1 |
| B | 6 | $20.5\pm1.1$ | $22.9\pm1.3$ | +2.4 |
| C | 5 | $20.9\pm1.2$ | $23.3\pm0.8$ | +2.4 |
| D | 6 | $20.7\pm1.3$ | $21.7\pm1.1$ | +1.1 |
| E | 6 | $20.4\pm1.8$ | $22.9\pm1.6$ | +2.5 |
| F | 6 | $20.5\pm0.8$ | $22.4\pm1.2$ | +1.9 |
| G | 6 | $20\pm1$ | $22.6\pm1.2$ | +2.6 |
| H | 4 | $22.1\pm2.3$ | $24.5\pm1.9$ | +2.4 |
| I | 4 | $20.5\pm1.6$ | $22.7\pm2.2$ | +2.3 |

Note: D20 denotes 20 days after tumor cell inoculation.

3. Results of Tumor-Volume Inhibition

Throughout the study, tumor growth was closely monitored; tumors were measured three times per week, results were recorded, and the tumor growth inhibition rate (TGI) was calculated. The results are summarized in Tables 37-38, and the tumor growth curves are shown in FIGS. 18-3 to 18-4.

Treated-side tumor: Tumors in the control group grew well. Relative to the control group, the D20 TGIs for Groups A, B, C, D, E, F, and G were 89.0%, 65.4%, 73.3%, 80.8%, 95.4%, 92.8%, and 94.5%, respectively (all > 60%). At the study endpoint, Groups H and I showed TGIs of -19.8% and 27.7%, respectively (both < 60%).

Untreated-side tumor (contralateral): Tumors in the control group grew well. Relative to the control group, the D20 TGIs for Groups A, B, C, E, F, G, H, and I were 16.4%, 31.1%, 9.9%, 32.2%, 25.2%, 38.7%, -35.1%, and -67.1%, respectively (all < 60%).

Table 37

| Days | Negative control-2 (PBS) group | | Group A | | | Group B | | | Group C | | | Group D | | |
|---|---|---|---|---|---|---|---|---|---|---|---|---|---|---|
| | Tumor volume (mm$^3$) | N | Tumor volume (mm$^3$) | N | Tumor growth inhibition rate (%) | Tumor volume (mm$^3$) | N | Tumor growth inhibition rate (%) | Tumor volume (mm$^3$) | N | Tumor growth inhibition rate (%) | Tumor volume (mm$^3$) | N | Tumor growth inhibition rate (%) |
| 6 | 87.8±26.1 | 6 | 91.6±19.9 | 6 | 0.0 | 89.5±19.0 | 6 | 0.0 | 84.3±18.2 | 5 | 0.0 | 79.6±24.9 | 6 | 0.0 |
| 9 | 151.8±92.7 | 6 | 140.2±81.6 | 6 | 11.5 | 119.2±48.2 | 6 | 23.0 | 140.7±56.1 | 5 | 3.5 | 134.6+49.1 | 6 | 2.1 |
| 11 | 284.4+169. 8 | 6 | 205.8+100.6 | 6 | 30.6 | 198.6±92.7 | 6 | 31.5 | 171.5±80.0 | 5 | 37.2 | 209.3±104.4 | 6 | 18.8 |
| 13 | 212.9±140. 2 | 6 | 154.1±115.4 | 6 | 30.6 | 179.3±162. 8 | 6 | 17.4 | 178.2±90.9 | 5 | 12.8 | 204.6±116.1 | 6 | -6.1 |
| 15 | 673.8±335. 3 | 6 | 172.9±135.8 ** | 6 | 75.4 | 281.8±254. 1* | 6 | 59.0 | 281.0+287. 6 | 5 | 56.6 | 283.6±199.6* | 6 | 53.6 |
| 17 | 1057.4±502 .5 | 6 | 194.0±137.5 ** | 6 | 82.4 | 398.6±405. 3* | 6 | 63.0 | 279.2±275. 9* | 5 | 72.5 | 286.0±287.0* * | 6 | 70.2 |
| 20 | 1904.5±878 .4 | 6 | 217.7±209** | 6 | 89.0 | 670.6±744. 7* | 6 | 65.4 | 488.7±535. 8* | 5 | 73.3 | 332.1±455.5* * | 6 | 80.8 |

| Days | Group E | | | Group F | | | Group G | | | Group H | | | Group I | | |
|---|---|---|---|---|---|---|---|---|---|---|---|---|---|---|---|---|
| | Tumor volume (mm³) | N | Tumor growth inhibition rate (%) | Tumor volume (mm³) | N | Tumor growth inhibition rate (%) | Tumor volume (mm³) | N | Tumor growth inhibition rate (%) | Tumor volume (mm³) | N | Tumor growth inhibition rate (%) | Tumor volume (mm³) | N | Tumor growth inhibition rate (%) |
| 6 | 85.3±17.1 | 6 | 0.0 | 111.7±47.4 | 6 | 0.0 | 105.3±45.4 | 6 | 0.0 | 97.7±36.3 | 4 | 0.0 | 93.9±29.0 | | 0.0 |
| 9 | 139.9±87.1 | 6 | 5.1 | 145.5±60.4 | 6 | 24.7 | 92.0+30.5 | 6 | 49.5 | 159.8+70. 2 | 4 | 5.4 | 134.0±40. 7 | 4 | 17.5 |
| 11 | 141.8±72.6 | 6 | 48.7 | 216.4±105. 7 | 6 | 40.2 | 135.6±61.2 | 6 | 60.3 | 290.6±98. 5 | 4 | 8.1 | 136.2±57. 2 | 4 | 55.2 |
| 13 | 127.5±73.7 | 6 | 38.3 | 165.5±121. 1 | 6 | 38.9 | 112.4±35.9 | 6 | 56.0 | 545.9±307 .6* | 4 | -130.5 | 233.9±11 5.5 | 4 | -2.7 |
| 15 | 110.9±82.7 ** | 6 | 83.1 | 186.0±186. 0* | 6 | 78.3 | 141.5±45.8 ** | 6 | 82.5 | 814.2±473 .9 | 4 | -8.6 | 398.6±14 7.1 | 4 | 44.7 |
| 17 | 116.6±89.6 ** | 6 | 88.7 | 156.1±208. 5** | 6 | 88.4 | 92.6±50.1* * | 6 | 92.7 | 1408.8±96 4.9 | 4 | -19.8 | 817.9±28 7.4 | 4 | 27.7 |
| 20 | 85.3±48.8* * | 6 | 95.4 | 174.4±284. 1** | 6 | 92.8 | 125.8±99.2 ** | 6 | 94.5 | / | | / | / | | / |

**Note:** Compared with the negative control-2 (PBS) group, *P<0.05, **P<0.01; "Days" indicates the number of days after tumor cell inoculation; "N" indicates the number of animals in the group included in the statistics; groups H and I were subjected to time-regression processing.

280

Table 38

| Days | Negative control-2 (PBS) | | Group A | | | Group B | | | Group C | | | Group E | | |
|---|---|---|---|---|---|---|---|---|---|---|---|---|---|---|
| | Tumor volume (mm$^3$) | N | Tumor volume (mm$^3$) | N | Tumor growth inhibition rate (%) | Tumor volume (mm$^3$) | N | Tumor growth inhibition rate (%) | Tumor volume (mm$^3$) | N | Tumor growth inhibition rate (%) | Tumor volume (mm$^3$) | N | Tumor growth inhibition rate (%) |
| 6 | 115.4±52.1 | 6 | 144.2±64.3 | 6 | 0.0 | 129±65 | 6 | 0.0 | 166.6±70.9 | 5 | 0.0 | 115.1±59.8 | 6 | 0.0 |
| 9 | 198.6±104.8 | 6 | 264.8+148.5 | 6 | -6.7 | 163.5±138.3 | 6 | 26.3 | 278.6±158.7 | 5 | 2.8 | 156.4±94.1 | 6 | 21.0 |
| 11 | 366.4±233.3 | 6 | 459.8±240.7 | 6 | -0.5 | 365.5±323.3 | 6 | 10.7 | 512.5±325.7 | 5 | 3.1 | 263.5+152.8 | 6 | 27.9 |
| 13 | 334.6±247.3 | 6 | 604.3±154.6* | 6 | -44.5 | 406.7±354.3 | 6 | -8.7 | 743.2±381.6 | 5 | -53.8 | 333.2±201 | 6 | 0.2 |
| 15 | 908.6±560.7 | 6 | 1067.8±416.7 | 6 | 5.9 | 941.2±825.7 | 6 | 7.3 | 1273.9±413 | 5 | 2.9 | 566.1±352.2 | 6 | 37.5 |
| 17 | 1283.1±589.3 | 6 | 1298.3±567.2 | 6 | 19.0 | 1113.6+878. 2 | 6 | 22.3 | 1707.5±466.5 | 5 | 7.8 | 785.4±375.9 | 6 | 38.6 |
| 20 | 2061.2±831.8 | 6 | 2151.8±929.5 | 6 | 16.4 | 1588.2±1074 | 6 | 31.1 | 2682.8±507.6 | 5 | 9.9 | 1394.1±602.9 | 6 | 32.2 |

| Days | Group F | | | Group G | | | Group H | | | Group I | | |
|---|---|---|---|---|---|---|---|---|---|---|---|---|
| | Tumor volume (mm$^3$) | N | Tumor growth inhibition rate (%) | Tumor volume (mm$^3$) | N | Tumor growth inhibition rate (%) | Tumor volume (mm$^3$) | N | Tumor growth inhibition rate (%) | Tumor volume (mm$^3$) | N | Tumor growth inhibiti on rate (%) |
| 6 | 123.1±65.2 | 6 | 0.0 | 181.5±28.3* | 6 | 0.0 | 150±112.9 | 4 | 0.0 | 104.9±63.4 | 4 | 0.0 |
| 9 | 217.2±141.8 | 6 | -2.5 | 317.9±94.8 | 6 | -1.7 | 361.4±178.2 | 4 | -40.0 | 208±129.2 | 4 | -15.2 |
| 11 | 448.4±257.9 | 6 | -14.7 | 582.4±188.1 | 6 | -1.1 | 441.7±213.3 | 4 | 7.3 | 299.8±202.3 | 4 | 10.0 |
| 13 | 538.2±299.3 | 6 | -50.7 | 726.4±141.2** | 6 | -38.0 | 851.7±425.2 | 4 | -95.8 | 796.8±242.9 | 4 | -161.9 |
| 15 | 748.1±374.2 | 6 | 22.8 | 1055.9±218.4 | 6 | 26.1 | 1301.3±582.6** | 4 | -10.2 | 1230.1±313.3 | 4 | -48.9 |
| 17 | 994.6±480.5 | 6 | 27.3 | 1353.1±482.1 | 6 | 33.0 | 2252.8±1161.7 | 4 | -35.1 | 1949.7±501.9 | 4 | -67.1 |
| 20 | 1645.9±722.5 | 6 | 25.2 | 1989.6±747.9 | 6 | 38.7 | / | / | / | / | / | / |

Note: Compared with the negative control-2 (PBS) group, *P<0.05, **P<0.01; Days: days after inoculation of tumor cells; N: number of animals in the group included in the statistical analysis; Groups H and I were subjected to time-regression treatment.

4. Tumor-Weight Results

At 21 days after inoculation of CT26 cells, all animals were euthanized; tumors were excised, weighed, and photographed. The results are summarized in Table 39 and FIGS. 18-5 to 18-6.

Treated-side, before tumor resection: Compared with the control group, the tumor-weight inhibition rates for Groups A, B, C, D, E, F, and G on the treated side before resection were 94.7%, 74.5%, 81.3%, 85.6%, 97.8%, 95.3%, and 96.6%, respectively (all > 60%). For Groups H and I, the corresponding inhibition rates were 30.6% and 58.5%, respectively (both < 60%).

Treated-side, after tumor resection: Compared with the control group, the tumor-weight inhibition rates for Groups A, B, C, D, E, F, and G on the treated side after resection were 94.4%, 73.8%, 80.2%, 84.7%, 97.9%, 95.7%, and 96.4%, respectively (all > 60%). For Groups H and I, the corresponding inhibition rates were 27.2% and 57.4%, respectively (both < 60%).

Untreated-side, before tumor resection: Compared with the control group, the tumor-weight inhibition rates for Groups A, B, C, E, F, G, H, and I on the untreated side before resection were 6.1%, 27.3%, -4.5%, 41.2%, 30.5%, 12.0%, 6.4%, and -3.5%, respectively (all < 60%).

Untreated-side, after tumor resection: Compared with the control group, the tumor-weight inhibition rates for Groups A, B, C, E, F, G, H, and I on the untreated side after resection were 6.0%, 27.5%, -6.9%, 39.6%, 28.9%, 10.8%, 3.5%, and -6.5%, respectively (all < 60%).

Table 39. Summary of Tumor Weight.

| Group | No. of animals | Body weight before tumor removal during dosing period (g) | IR_TW% | Body weight after tumor removal during dosing period (g) | IR_TW% | Body weight before tumor removal during non-dosing period (g) | IR_TW% | Body weight after tumor removal during non-dosing period (g) | IR_TW% |
|---|---|---|---|---|---|---|---|---|---|
| Negative control-2 (PBS) | 6 | 1.60±0.65 | / | 1.52±0.59 | / | 1.73±0.75 | / | 1.65±0.68 | / |
| Group A | 6 | 0.09±0.12** | 94.7 | 0.09±0.12** | 94.4 | 1.63±0.68 | 6.1 | 1.55±0.64 | 6.0 |
| Group B | 6 | 0.41+0.44** | 74.5 | 0.40±0.42** | 73.8 | 1.26±0.83 | 27.3 | 1.20±0.79 | 27.5 |
| Group C | 5 | 0.30±0.32** | 81.3 | 0.30±0.32** | 80.2 | 1.81±0.34 | -4.5 | 1.76±0.29 | -6.9 |
| Group D | 6 | 0.23±0.37** | 85.6 | 0.23±0.37** | 84.7 | / | / | / | / |
| Group E | 6 | 0.04+0.03** | 97.8 | 0.03±0.02** | 97.9 | 1.02±0.41 | 41.2 | 1.00±0.40 | 39.6 |
| Group F | 6 | 0.08±0.14** | 95.3 | 0.07±0.12** | 95.7 | 1.20±0.47 | 30.5 | 1.17±0.46 | 28.9 |
| Group G | 6 | 0.06±0.07** | 96.6 | 0.06±0.07** | 96.4 | 1.52±0.68 | 12.0 | 1.47±0.66 | 10.8 |
| Group H | 4 | 1.11±0.72 | 30.6 | 1.11±0.71 | 27.2 | 1.62±0.67 | 6.4 | 1.59±0.66 | 3.5 |
| Group I | 4 | 0.67±0.27* | 58.5 | 0.65±0.25* | 57.4 | 1.79±0.52 | -3.5 | 1.76±0.52 | -6.5 |

**Note:** Compared with the negative control-2 (PBS) group. *P<0.05, **P<0.01.

Considering both the tumor growth-inhibition rate and the tumor weight-inhibition rate, the experimental conclusions are as follows:

Treated-side tumors (administration side): Groups A, B, C, D, E, F and G, after five administrations by intratumoral injection or by tail-vein intravenous injection of the respective test articles, exhibited a very marked tumor-inhibitory effect; Group I, after five intratumoral injections through to the end of the study, exhibited a comparatively good tumor-inhibitory effect; Group H, after five intratumoral injections through to the end of the study, showed a certain tumor-inhibitory trend.

Untreated-side tumors (contralateral side): Groups B, E and F showed a certain tumor-inhibitory trend through to the end of the study after five intratumoral administrations (on the treated side); Groups A, C, G, H and I, despite five intratumoral administrations through to the end of the study, consistently failed to effectively inhibit the contralateral implanted tumor.

Dose comparison: Groups A, B and C all received intratumoral administration at descending dose levels (high → medium → low). After five administrations, the treated side in all three groups exhibited very marked tumor-inhibitory effects with no substantial overall difference, among which Group A showed the relatively best inhibitory effect; on the untreated side, none of the groups showed an effective tumor-inhibitory effect overall, although some differences existed, with Group B showing a certain inhibitory trend. These results indicate that the dose-adjustment window is relatively wide.

Route-of-administration comparison: Groups A and D used the same test article; Group A received intratumoral administration, and Group D received intravenous administration. Both administration routes exhibited very marked tumor-inhibitory effects.

Drug-combination comparison: Groups A, E, F and G all exhibited very marked tumor-inhibitory effects; Group I exhibited a comparatively good tumor-inhibitory effect; Group H showed a certain tumor-inhibitory trend.

Example 19

I. Experimental objective:

The primary objective of this study is to establish a 4T1 tumor model and to verify the immunostimulatory activity and antitumor efficacy of the test article(s). The sub-objective is to compare differences in antitumor efficacy (pharmacodynamics) under different dose levels and routes of administration.

II. Cell information

4T1, murine mammary carcinoma cell line; source: Cell Resource Center, Institute of Basic Medical Sciences, Chinese Academy of Medical Sciences.

III. Experimental animal information

Species: BALB/c mouse (immunocompetent).

Age: 6-7 weeks.

Sex: female.

Body weight: 15-19 g.

Supplier: Beijing Vital River Laboratory Animal Technology Co., Ltd.

IV Experimental methods

1. Cell culture

Tumor cells were cultured in RPMI-1640 medium supplemented with 10% heat-inactivated fetal bovine serum, penicillin (100 U/mL), streptomycin (100 $\mu$g/mL), and L-glutamine (2 mM), in an incubator at 37 °C with 5% $CO_2$. Cells were passaged every 2-3 days upon reaching confluence, and logarithmic-phase tumor cells were used for in vivo tumor inoculation.

2. Tumor inoculation and group assignment

4T1 murine mammary carcinoma cells were inoculated subcutaneously at a dose of $2\times 10^6$ cells in 0.2 mL per site into female BALB/c mice at the left and right dorsal cervical (nuchal) regions (a subset of animals was inoculated on the right side only). The day of inoculation was designated D0. On D6, animals were randomized on the basis of the right-side tumor volume into six groups: Negative Control-1 (PBS), Negative Control-2 (PBS), Group A, Group B, Group C, and Group D. On the day of randomization, all groups commenced dosing either by intratumoral administration (into the right-side tumor) or by tail-vein intravenous administration, for a total of 8 administrations, given once every other day (QOD).
Detailed information on animal inoculation, grouping and dosing is shown in Table 40, Table 41, and Figure 19-1.

Table 40. Animal inoculation and dosing scheme

| Animal inoculation (the day of inoculation is defined as D0). | | | | |
|---|---|---|---|---|
| **Cell line** | **No. of mice** | **Mouse Nos.** | **Inoculation site** | **Inoculum per mouse** |
| 4T1 | 12 | 160-171 | Right dorsal cervical subcutis | $2\times10^6$ cells/mouse |
| 4T1 | 28 | 172-199 | Bilateral dorsal cervical subcutis (left and right) | $2\times10^6$ cells/mouse |

Table 41. Animal grouping and dosing

| Group | No. of animals (n) | Test article | Route of administration | Dosing schedule and dose volume |
|---|---|---|---|---|
| Negative Control-1 (PBS) | 5 | PBS | Intravenous | On the day of group assignment (200 $\mu$L), Day 3 (200 $\mu$L), Day 5 (200 $\mu$L), Day 7 (200 $\mu$L), Day 9 (200 $\mu$L) |
| Negative Control-2 (PBS) | 6 | PBS | Intratumoral (right-side tumor) | On the day of group assignment (20 $\mu$L), Day 3 (20 $\mu$L), Day 5 (50 $\mu$L), Day 7 (50 $\mu$L), Day 9 (50 $\mu$L) |
| A | 6 | A | Intratumoral (right-side tumor) | On the day of group assignment (20 $\mu$L), Day 3 (20 $\mu$L), Day 5 (50 $\mu$L), Day 7 (50 $\mu$L), Day 9 (50 $\mu$L) |
| B | 6 | B | Intratumoral (right-side tumor) | On the day of group assignment (20 $\mu$L), Day 3 (20 $\mu$L), Day 5 (50 $\mu$L), Day 7 (50 $\mu$L), Day 9 (50 $\mu$L) |
| C | 6 | C | Intratumoral (right-side tumor) | On the day of group assignment (20 $\mu$L), Day 3 (20 $\mu$L), Day 5 (50 $\mu$L), Day 7 (50 $\mu$L), Day 9 (50 $\mu$L) |
| D | 6 | D | Intravenous | On the day of group assignment (200 $\mu$L), Day 3 (200 $\mu$L), Day 5 (200 $\mu$L), Day 7 (200 $\mu$L), Day 9 (200 $\mu$L) |

Wherein A includes 2*CpG1826-CD40 aptamer-DNA carrier (50 $\mu$g) + 2*AmiR-21-4*Biotin (Bio)-DNA carrier (6 $\mu$g) + OX40 aptamer (DNA form) (6 $\mu$g); B includes 2*CpG1826-CD40 aptamer-DNA carrier (10 $\mu$g) + 2*AmiR-21-4*Biotin (Bio)-DNA carrier (6 $\mu$g) + OX40 aptamer (DNA form) (6 $\mu$g); C includes 2*CpG1826-CD40 aptamer-DNA carrier (6 $\mu$g) + 2*AmiR-21-4*Biotin (Bio)-DNA carrier (6 $\mu$g) + OX40 aptamer (DNA form) (6 $\mu$g); and D includes 2*CpG1826-CD40 aptamer-DNA carrier (50 $\mu$g) + 2*AmiR-21-4*Biotin (Bio)-DNA carrier (6 $\mu$g) + OX40 aptamer (DNA form) (6 $\mu$g); the A, B, C and D test articles are each Combination (27), namely Drug 3 + Drug 20 + the OX40 aptamer in DNA form.

3. Evaluation indices

Same as in Example 16.

4. Statistical analysis

Same as in Example 16.

V. Experimental results:

1. Clinical observations in experimental animals

During the dosing period, the animals remained active, fed normally, and were in good general condition.

2. Changes in body weight of experimental animals

During the dosing period, body weight in all groups increased to a certain extent. At the study endpoint, compared with Negative Control-2 (PBS), animals in Group A and Group B had significantly lower body weights ($P < 0.05$), whereas Group C showed no significant difference ($P > 0.05$). The differences in body weight observed for Groups A and B may be related to the larger tumor burden in the Negative Control-2 (PBS) group. Compared with Negative Control-1 (PBS), Group D showed no significant difference in body weight.

Table 42. Summary of Body Weight

| Group | n (animal s) | Before dosing (g) | D23 | P | Change(g) |
|---|---|---|---|---|---|
| Negative Control-1 (PBS, i.v.) | 5 | $18.1\pm0.2$ | $19.8\pm0.3$ | / | +1.7 |
| Negative Control-2 (PBS, i.t. to right tumor) | 6 | $19.1\pm0.6$ | $21.7\pm0.5$ | / | +2.7 |
| A | 6 | $18.4\pm0.5$ | $20.6\pm0.3$** | 0.0006 | +2.1 |
| B | 6 | $17.7\pm0.8$ | $20.1\pm0.4$** | 0.0001 | +2.3 |
| C | 6 | $18.7\pm0.3$ | $21.2\pm1$ | 0.2516 | +2.5 |
| D | 6 | $18.6\pm1.1$ | $19.5\pm0.9$ | 0.6220 | +0.9 |

*Notes:* Significance vs control: *$P < 0.05$, **$P < 0.01$. For Groups A-C the comparator is Negative Control-2 (PBS, i.t.); for Group D the comparator is Negative Control-1 (PBS, i.v.).

3. Results of tumor-volume inhibition

All animals were closely monitored for tumor growth throughout the study. Tumor volume was measured three times per week, the results were recorded, and the tumor growth-inhibition rate (TGI) was calculated. Numerical results are provided in Tables 43-44, and tumor growth curves are shown in Figures 19-3 to 19-4.

Right-side tumor (dosing side): Tumors in the control groups showed progressive growth. Relative to Negative Control-1 (PBS), Group D exhibited a TGI at D23 of 83.4%, i.e., >60%. Relative to Negative Control-2 (PBS), Groups A and B exhibited TGI at D23 of 79.7% and 69.5%, respectively (both >60%), whereas Group C exhibited a TGI of 39.0% (<60%).

Left-side tumor (contralateral side): Tumors in the control groups showed progressive growth. Relative to Negative Control-2 (PBS), Groups A, B, and C exhibited TGI at D23 of 35.7%, 44.3%, and 30.9%, respectively-each <60%.

Table 43

| Day s | Negative control-1 (PBS) group | | Negative control-2 (PBS) group | | Group A | | | Group B | | | Group C | | | Group D | | |
|---|---|---|---|---|---|---|---|---|---|---|---|---|---|---|---|---|
| | Tumor volume (mm$^3$) | N | Tumor volume (mm$^3$) | N | Tumor volume (mm$^3$) | N | Tumor growth inhibiti on rate(%) | Tumor volume (mm$^3$) | N | Tumor growth inhibiti on rate(%) | Tumor volume (mm$^3$) | N | Tumor growth inhibiti on rate | Tumor volume (mm$^3$) | N | Tumor growth inhibiti on rate(%) |
| 5 | 111.5 ± 39.2 | 5 | 88.4 ± 19.4 | 6 | 77.2 ± 19.6 | 6 | 0.0 | 90.4 ± 20.5 | 6 | 0.0 | 83.2 ± 33.2 | 6 | 0.0 | 79.6 ± 37.4 | 6 | 0.0 |
| 8 | 101.9 ± 54.2 | 5 | 207.3 ± 60.5 | 6 | 144.0 ± 45.2 | 6 | 20.5 | 138.6 ± 54.7 | 6 | 34.7 | 169.8 ± 60.3 | 6 | 13.0 | 134.5 ± 75.4 | 6 | -84.8 |
| 10 | 307.6 ± 94.0 | 5 | 293.7 ± 40.2 | 6 | 210.3 ± 45.8* | 6 | 15.2 | 177.4 ± 48.5** | 6 | 38.9 | 218.2 ± 91.9 | 6 | 18.3 | 160.3 ± 71.0* | 6 | 27.0 |
| 12 | 395.5 ± 134.5 | 5 | 376.4 ± 61.4 | 6 | 292.8 ± 112.9 | 6 | 11.0 | 218.8 ± 60.7** | 6 | 43.2 | 292.2 ± 99.5 | 6 | 17.5 | 149.2 ± 54.7** | 6 | 47.2 |
| 14 | 440.5 ± 150.4 | 5 | 5001 ± 103.5 | 6 | 214.0 ± 40.6** | 6 | 51.0 | 232.3 ± 107.7** | 6 | 54.6 | 307.7 ± 86.7** | 6 | 34.7 | 149.9 ± 87.8** | 6 | 52.3 |
| 16 | 595.2 ± 183.2 | 5 | 623.3 ± 62.9 | 6 | 237.8 ± 71.9** | 6 | 56.4 | 314.3 ± 167.4** | 6 | 50.7 | 367.4 ± 135.6** | 6 | 37.4 | 167.4 ± 125.0** | 6 | 60.6 |
| 19 | 809.6 ± 159.0 | 5 | 930.7 ± 106.6 | 6 | 310.0 ± 141.0** | 6 | 61.9 | 360.3 ± 194.6** | 6 | 62.2 | 517.9 ± 197.1** | 6 | 40.9 | 136.6 ± 131.9** | 6 | 76.4 |
| 21 | 977.8 ± 2642 | 5 | 1062.1 ± 69.7 | 6 | 213.7 ± 131.6** | 6 | 77.0 | 356.8 ± 217.9** | 6 | 67.2 | 630.2 ± 232.4** | 6 | 37.0 | 108.1 ± 121.7** | 6 | 84.5 |
| 23 | 1103.6 ± 192.9 | 5 | 1341.5 ± 205.2 | 6 | 237.7 ± 135.4** | 6 | 79.7 | 418.8 ± 306.1** | 6 | 69.5 | 770.3 ± 342.3** | 6 | 39.0 | 130.4 ± 160.2** | 6 | 83.4 |

Compared with the control group: *P<0.05, *P<0.01.*

Note: Days: number of days after inoculation with tumor cells; N: number of animals in the respective group included in the statistics.

Table 44

| Days | Negative control-2 (PBS) group | | Group A | | | Group B | | | Group C | | |
|---|---|---|---|---|---|---|---|---|---|---|---|
| | Tumor volume (mm$^3$), | N | Tumor volume (mm$^3$), | N | Tumor growth inhibition rate (%) | Tumor volume (mm$^3$) | N | Tumor growth inhibition rate (%) | Tumor volume (mm$^3$) | N | Tumor growth inhibition rate (%) |
| 5 | 81.2 ± 12.5 | 6 | 107.4 ± 37.5 | 6 | 0.0 | 124.3 ± 30.2** | 6 | 0.0 | 109.2 ± 46.6 | 6 | 0.0 |
| 8 | 143.4 ± 55.2 | 6 | 194.7 ± 47.2 | 6 | -2.5 | 244.9 ± 98.8 | 6 | -11.5 | 214.1 ± 54.5* | 6 | -10.9 |
| 10 | 247.5 ± 82.5 | 6 | 258.7 ± 56.8 | 6 | 21.0 | 277.0 ±30.6 | 6 | 27.0 | 262.8 ± 77.3 | 6 | 21.1 |
| 12 | 286.8 ± 64.2 | 6 | 311.5 ± 70.8 | 6 | 18.0 | 326.2 ± 44.5 | 6 | 25.8 | 415.9 ± 135.6 | 6 | -7.7 |
| 14 | 396.4 ± 109.5 | 6 | 421.5 ± 131.9 | 6 | 19.7 | 378.5 ± 49.1 | 6 | 37.7 | 400.8 ± 92.7 | 6 | 24.9 |
| 16 | 552.7 ± 118.1 | 6 | 543.6 ± 163.3 | 6 | 25.7 | 483.1 ± 91.0 | 6 | 42.9 | 570.0 ± 125.6 | 6 | 23.4 |
| 19 | 753.5 ± 133.2 | 6 | 814.0 ± 377.5 | 6 | 18.4 | 669.0 ± 127.4 | 6 | 42.0 | 768.6 ± 152.4 | 6 | 24.2 |
| 21 | 870.4 ± 133.6 | 6 | 773.6 ± 148.3 | 6 | 32.9 | 742.0 ± 87.2 | 6 | 44.4 | 914.5 ± 246.1 | 6 | 21.9 |
| 23 | 1112.4 ± 209.4 | 6 | 946.4 ± 143.8 | 6 | 35.7 | 950.0 ± 101.5 | 6 | 44.3 | 1034.1 ± 206.1 | 6 | 30.9 |

*Compared with the control group: *P<0.05, *P<0.01.
Note: Days: number of days after inoculation with tumor cells; N: number of animals in the respective group included in the statistics.

4. Tumor weight results

At 23 days after 4T1 cell inoculation (D23), all animals were euthanized; tumors were excised, weighed, and photographed. The results are summarized in Table 45 and Figures 19-5 to 19-6.

Right side-before eschar removal (before debridement): Relative to Negative Control-1 (PBS), Group D showed a tumor-weight inhibition rate (TWI) of 91.3%, i.e., >60%. Relative to Negative Control-2 (PBS), Groups A and B showed TWIs of 87.1% and 69.8%, respectively (>60%), whereas Group C showed a TWI of 51.0% (<60%).

Right side-after eschar removal (after debridement): Relative to Negative Control-1 (PBS), Group D showed a TWI of 92.2% (>60%). Relative to Negative Control-2 (PBS), Groups A and B showed TWIs of 88.1% and 70.5%, respectively (>60%), whereas Group C showed a TWI of 52.0% (<60%).

Left side-before eschar removal (before debridement): Relative to Negative Control-2 (PBS), Groups A, B, and C showed TWIs of 24.5%, 14.2%, and 3.4%, respectively-each <60%.

Left side-after eschar removal (after debridement): Relative to Negative Control-2 (PBS), Groups A, B, and C showed TWIs of 24.1%, 10.8%, and -2.2%, respectively-each <60%.

Table 45

| Group | Number of animals | Right-side tumor weight before tumor excision (g) | Right-side IRw% before tumor excision | Right-side tumor weight after tumor excision (g) | Right-sid e IRw% after tumor excision | Left-side tumor weight before tumor excision (g) | Left-side IRw% before tumor excision | Left-side tumor weight after tumor excision (g) | Left-side IRw% after tumor excision |
|---|---|---|---|---|---|---|---|---|---|
| Negative control-1 (PBS) group | 5 | 0.94 ± 0.20 | / | 0.86 ± 0.18 | / | / | / | / | / |
| Negative control-2 (PBS) group | 6 | 1.18 ± 0.19 | / | 1.13 ± 0.18 | / | 0.92 ± 0.19 | / | 0.82 ± 0.19 | / |
| Group A | 6 | 0.15 ± 0.13** | 87.1 | 0.13 ± 0.13** | 88.1 | 0.69 ± 0.16* | 24.5 | 0.62 ± 0.17 | 24.1 |
| Group B | 6 | 0.36 ± 0.27** | 69.8 | 0.33 ± 0.26** | 70.5 | 0.79 ± 0.13 | 14.2 | 0.73 + 0.13 | 10.8 |
| Group C | 6 | 0.58 + 0.24** | 51.0 | 0.54 ± 0.23** | 52.0 | 0.89 ± 0.17 | 3.4 | 0.84 ± 0.17 | -2.2 |
| Group D | 6 | 0.08 + 0.10** | 91.3 | 0.07 ± 0.08** | 92.2 | / | / | / | / |
| * P<0.05, ** P<0.01, compared with the control group. | | | | | | | | | |

VI. Experimental conclusion

Conclusion of the test:

Taking into account both the tumor growth-inhibition rate and the tumor weight-inhibition rate, the conclusions are as follows:

Right-side tumor (dosing side): After eight administrations by intratumoral injection or tail-vein intravenous injection of the respective test articles, Groups A, B, and D exhibited very marked antitumor effects. Group C, after eight intratumoral injections through to the end of the study, showed a certain tumor-inhibitory effect.

Left-side tumor (contralateral side): For Groups A, B, and C, despite eight intratumoral injections through to the end of the study, the contralateral implanted tumor could not be effectively inhibited.

Dose comparison: Groups A, B, and C all received intratumoral administration at descending dose levels (high → medium → low). After eight administrations, the tumor-inhibitory effect of Group A > Group B > Group C. On the untreated side, no effective tumor-inhibitory effect was observed overall, with Group A showing a certain inhibitory trend.

Route-of-administration comparison: Groups A and D received the same test article; Group A by intratumoral injection, Group D by intravenous injection. Both routes demonstrated that this test article produced a very marked inhibitory effect on the tumor at the dosing site..

Example 20

Study objective and principal instruments: identical to Example 16.

Experimental animal information-difference from Example 1: body weight: 16-18 g.

I. Experimental plan:

The animal inoculation and dosing regimens are shown in Tables 46 and 47.

Table 46 Animal inoculation and dosing regimen

| Cell line | Mice (n) | Mouse No. | Inoculation site | Number of inoculated cells |
|-----------|----------|-----------|------------------|----------------------------|
| B16F10 | 42 | See grouping table | Subcutaneously in the bilateral axillary dorsal region | $1 \times 10^5$ cells/mouse |
| Note: The day of inoculation was designated as D0. | | | | |

Table 47 Animal grouping and dosing regimen

| Model | Group | Number of animals | Treatment | Route of administration | Dose volume per administration | Number of administrations | Dosing schedule |
|-------|-------|-------------------|-----------|-------------------------|--------------------------------|---------------------------|-----------------|
| B16F10 | Negative control group (Control) | 10 | PBS | Intratumoral injection into the left-side tumor | 20 $\mu$L for the 1st and 2nd administrations; 50 $\mu$L for the 3rd to 5th administrations | 5 times | On the day of grouping (Day 1), and on Days 3, 5, 7 and 9 |
| B16F10 | A | 10 | A | | | | |
| B16F10 | B | 10 | B | | | | |
| B16F10 | C | 10 | C | Tail vein injection | 200 $\mu$L | 5 times | |

Wherein, in Table 47 under "Treatment," A includes 2*CpG1826-CD40 aptamer-2*mannose-DNA carrier (50 $\mu$g) together with AmiR-21-AS1411 aptamer-A15 aptamer-2*Biotin (Bio)-DNA carrier (12 $\mu$g); B includes 2*CpG1826-CD40 aptamer-2*mannose-DNA carrier (50 $\mu$g) together with the OX40 aptamer in RNA/2'-O-methyl (2'-OMe) form (6 $\mu$g); and C includes 2*CpG1826-CD40 aptamer-2*mannose-DNA carrier (16 $\mu$g) together with AmiR-21-AS1411 aptamer-A15 aptamer-2*Biotin (Bio)-DNA carrier (6 $\mu$g); that is, A and C correspond to Combination (2) (Drug 6 + Drug 26), whereas B corresponds to Combination (3) (Drug 6 + the OX40 aptamer in RNA form).

II. Experimental Methods

1. Tumor inoculation and group assignment

A murine melanoma B16F10 model was used. The test animals were C57BL/6J mice, male, with a body weight of 16.0-18.0 g. At the time of modeling, well-growing tumor tissue was excised, minced and homogenized, diluted with sterile normal saline, and prepared as a tumor-cell suspension. B16F10 melanoma cells ($1 \times 10^6$ cells/0.2 mL) were inoculated subcutaneously into the left and right axillary-dorsal regions of each mouse; the day of inoculation was designated D0. On D8, animals were randomized according to the right-side tumor volume into four groups: Negative Control (PBS), Drug A, Drug B, and Drug C, 10 animals per group. On the same day as randomization, dosing was initiated for all groups by intratumoral injection (Negative Control, A, B) or by tail-vein intravenous injection (C), for a total of five administrations.

During the study, tumor volume and body weight were measured and recorded three times per week. At study termination, animals were euthanized by cervical dislocation; tumors were excised, weighed and photographed. Based on tumor volume and tumor weight, the tumor growth-inhibition rate (TGI) [calculated as (1 - T/C), expressed as %] and the inhibition rate of tumor weight (IRTW, %) were determined. Details of inoculation, grouping and dosing are provided in Tables 46 and 47.

2. Evaluation indices

Spleen index and thymus index: At the end of the study (D17), the remaining animals were euthanized by cervical dislocation; the spleen and thymus were excised and weighed, and the spleen and thymus indices were calculated. Spleen index = spleen weight (mg) / body weight (g) $\times$10; Thymus index = thymus weight (mg) / body weight (g) $\times$10.

The remaining evaluation indices are the same as in Example 16.

3. Statistical analysis

Same as in Example 16.

III. Experimental Results:

1. Clinical observations in experimental animals

During the dosing period, the animals remained active, ate normally, and were in good general condition.

2. Changes in body weight of experimental animals

During the dosing period, body weight in all groups increased to a certain extent; however, the body weights of the dosing groups were significantly lower than those of the negative control group, with a statistically significant difference relative to the negative control. The body-weight changes for all animals are shown in Table 48 and Figure 20-1.

Table 48 Summary of Animal Body Weights

| Group | Number of animals (n) | | Body weight (g) | | Change in body weight (g) |
|---|---|---|---|---|---|
| | D0 | D18 | D0 | D18 | |
| Negative control | 10 | 10 | 16.4 ± 0.9 | 26.0 ± 1.3 | + 9.6 |
| A | 10 | 10 | 16.6 ± 0.6 | 22.9 ± 2.1*** | + 6.3 |
| B | 10 | 10 | 16.5 ± 1.2 | 23.1 ± 2.8** | + 6.6 |
| C | 10 | 10 | 17.0 ± 0.8 | 23.9 ± 2.5* | + 7.3 |

\*: P<0.05, \*\*: P<0.01, \*\*\*: P<0.001, compared with the negative control group.
Note: D0, the day of inoculation with the tumor cell suspension; D18, 18 days after inoculation with the tumor cell suspension.

3. Results of tumor-volume inhibition

All animals were closely monitored for tumor growth during the study. Tumor size was measured three times per week, the results were recorded, and the tumor growth inhibition (TGI) was calculated.
Intratumoral administration group: Left-side tumor: Tumors in the negative control group grew well. Compared with the negative control group, on D18 the tumor growth inhibition rates (calculated on the basis of tumor volume) for Groups A and B were 78.65% and 71.88%, respectively, i.e., >60%; the T/C values (calculated on the basis of RTV) were 22.56% and 27.59%, respectively, i.e., <40%. Right-side tumor: Tumors in the negative control group grew well. Compared with the negative control group, on D18 the tumor growth inhibition rates (calculated on the basis of tumor volume) for Groups A and B were 37.34% and 27.41%, respectively, i.e., <60%; the T/C values (calculated on the basis of RTV) were 62.45% and 74.57%, respectively, i.e., >40%.
Tail-vein intravenous administration group: Left-side tumor: Compared with the negative control group, on D18 the tumor growth inhibition rate (calculated on the basis of tumor volume) for Group C was 37.41% (<60%); the T/C value (calculated on the basis of RTV) was 63.90% (>40%). Right-side tumor: Compared with the negative control group, on D18 the tumor growth inhibition rate (calculated on the basis of tumor volume) for Group C was 46.79% (<60%); the T/C value (calculated on the basis of RTV) was 53.33% (>40%).
The results are entered in Tables 49-52, and the tumor growth curves are shown in Figures 20-2 to 20-5 (Tumor volume: tumor volume; Days after transplantation: days after inoculation; RTV: relative tumor volume).

Table 49 Summary of tumor growth data left-side tumor).

| Days | Negative control | | A | | | B | | | C | | |
|---|---|---|---|---|---|---|---|---|---|---|---|
| | Tumor volume (mm³), | N | Tumor volume (mm³) | N | Tumor growth inhibition rate(%) | Tumor volume (mm³) | N | Tumor growth inhibition rate(%) | Tumor volume (mm³) | N | Tumor growth inhibition rate (%) |
| 8 | 139.1 ± 8.8 | 10 | 132.2 ± 16.8 | 10 | / | 144.9 ± 27.0 | 10 | / | 133.9 ± 29.3 | 10 | / |
| 11 | 364.2 ± 79.8 | 10 | 405.5 ± 81.4 | 10 | -- | 365.8 ± 59.0 | 10 | -- | 318.8 ± 100.7 | 10 | 12.65 |

(continued)

| Days | Negative control | | A | | | B | | | C | | |
|---|---|---|---|---|---|---|---|---|---|---|---|
| | Tumor volume (mm$^3$), | N | Tumor volume (mm$^3$) | N | Tumor growth inhibition rate(%) | Tumor volume (mm$^3$) | N | Tumor growth inhibition rate(%) | Tumor volume (mm$^3$) | N | Tumor growth inhibition rate (%) |
| 13 | 689.2 ± 230.5 | 10 | 366.9 ± 92.7** | 10 | 46.77 | 703.6 ± 185.6 | 10 | 26.93 | 526.7 ± 205.0* | 10 | 23.57 |
| 15 | 1195.3 ± 370.5 | 10 | 304.6 ± 81.6*** | 10 | 74.51 | 487.1 ± 81.1*** | 10 | 59.25 | 759.7 ± 245.7** | 10 | 36.44 |
| 18 | 1787.8 ± 334.6 | 10 | 381.6 ± 202.1** | 10 | 78.65 | 502.7 ± 226.1*** | 10 | 71.88 | 1118.9 ± 427.9** | 10 | 37.41 |

* P<0.05; ** P<0.01; *** P<0.001, compared with the negative control group.
Note: Days: number of days after inoculation with the tumor cell suspension; N: number of animals in the respective group included in the statistics.

Table 50 Summary of tumor growth data (left-side tumor).

| Days | Negative control | | A | | | B | | | C | | |
|---|---|---|---|---|---|---|---|---|---|---|---|
| | RTV | N | RTV | N | T/C (%) | RTV | N | T/C (%) | RTV | N | T/C (%) |
| 8 | 1.00 ± 0 | 10 | 1.00 ± 0 | 10 | / | 1.00 ± 0 | 10 | / | 1.00 ± 0 | 10 | / |
| 11 | 2.67 ± 0.67 | 10 | 3.15 ± 1.57 | 10 | 118.05 | 2.58 ± 0.50 | 10 | 96.74 | 2.44 ± 0.72 | 10 | 91.67 |
| 13 | 5.06 ± 1.87 | 10 | 2.83 ± 0.90** | 10 | 56.05 | 3.56 ± 1.40 | 10 | 70.34 | 3.96 ± 1.29 | 10 | 78.38 |
| 15 | 8.75 ± 3.10 | 10 | 2.31 ± 0.56*** | 10 | 26.38 | 3.51 ± 1.59*** | 10 | 40.07 | 5.89 ± 2.26* | 10 | 67.27 |
| 18 | 13.15 ± 3.46 | 10 | 2.97 ± 1.76*** | 10 | 22.56 | 3.63 ± 1.98*** | 10 | 27.59 | 8.40 ± 2.94** | 10 | 63.90 |

*P<0.05, **P<0.01, ***P<0.001, compared with the negative control group.
Note: Days: number of days after inoculation with the tumor cell suspension; N: number of animals in the respective group included in the statistics.

Table 51 Summary of tumor growth data (left-side tumor).

| Days | Negative control | | A | | | B | | | C | | |
|---|---|---|---|---|---|---|---|---|---|---|---|
| | Tumor volume (mm$^3$), | N | Tumor volume (mm$^3$), | N | Tumor growth inhibition rate(%) | Tumor volume (mm$^3$) | N | Tumor growth inhibition rate(%) | Tumor volume (mm$^3$) | N | Tumor growth inhibition rate (%) |
| 8 | 152.4 ± 31.7 | 10 | 154.6 ± 33.8 | 10 | / | 154.0 ± 37.8 | 10 | / | 151.4 ± 31.1 | 10 | / |
| 11 | 583.7 ± 130.5 | 10 | 592.7 ± 164.0 | 10 | -- | 678.2 ± 297.4 | 10 | -- | 533.4 ± 141.9 | 10 | 8.62 |

(continued)

| Days | Negative control | | A | | | B | | | C | | |
|---|---|---|---|---|---|---|---|---|---|---|---|
| | Tumor volume $(mm^3)$, | N | Tumor volume $(mm^3)$, | N | Tumor growth inhibition rate(%) | Tumor volume $(mm^3)$ | N | Tumor growth inhibition rate(%) | Tumor volume $(mm^3)$ | N | Tumor growth inhibition rate (%) |
| 13 | 1065.8 ± 254.2 | 10 | 1071.2 ± 271.9 | 10 | -- | 992.8 ± 372.7 | 10 | 6.84 | 742.4 ± 213.3* | 10 | 30.34 |
| 15 | 1793.5 ± 572.6 | 10 | 1230.6 ± 366.8* | 10 | 31.39 | 1255.3 ± 316.3* | 10 | 30.01 | 1124.5 ± 490.3** | 10 | 37.30 |
| 18 | 2902.7 ± 802.0 | 10 | 1818.9 ± 566.5** | 10 | 37.34 | 2107.0 ± 889.5 | 10 | 27.41 | 1544.6 ± 479.9*** | 10 | 46.79 |

* P<0.05; ** P<0.01; *** P<0.001, compared with the negative control group.
Note: Days: number of days after inoculation with the tumor cell suspension; N: number of animals in the respective group included in the statistics.

Table 52 Summary of tumor growth data (left-side tumor).

| Days | Negative control | | A | | | B | | | C | | |
|---|---|---|---|---|---|---|---|---|---|---|---|
| | RTV | N | RTV | N | T/C (%) | RTV | N | T/C (%) | RTV | N | T/C (%) |
| 8 | 1.00 ± 0 | 10 | 1.00 ± 0 | 10 | / | 1.00±0 | 10 | / | 1.00 ± 0 | 10 | / |
| 11 | 3.88 ± 0.70 | 10 | 3.99 ± 1.48 | 10 | 102.86 | 4.87± 3.50 | 10 | 125.55 | 3.63 ± 1.08 | 10 | 93.56 |
| 13 | 7.10 ± 1.51 | 10 | 7.28 ± 2.57 | 10 | 102.52 | 6.98 ± 4.03 | 10 | 98.32 | 5.16 ± 2.02* | 10 | 72.71 |
| 15 | 12.01 ± 3.91 | 10 | 8.23 ± 2.86* | 10 | 68.54 | 8.49 ± 2.59* | 10 | 70.67 | 7.75 ± 3.75* | 10 | 64.53 |
| 18 | 19.83 ± 6.80 | 10 | 12.38 ± 4.88* | 10 | 62.45 | 14.79 ± 8.88 | 10 | 74.57 | 10.57 ± 4.18** | 10 | 53.33 |

*: P<0.05, **: P<0.01, compared with the negative control group.
Note: Days: number of days after inoculation with the tumor cell suspension; N: number of animals in the respective group included in the statistics.

4. Tumor weight results

At 18 days after B16F10 inoculation, all animals were euthanized by cervical dislocation; tumors were excised, weighed, and photographed. The results are summarized in Table 53 and Figure 20-6 (Tumor weight: tumor weight; left tumor: left-side tumor; right tumor: right-side tumor).

Intratumoral injection group: For the left-side tumor, the tumor weight inhibition rates for Drug A and Drug B were 78.44% and 69.44%, respectively, i.e., greater than 60%; for the right-side tumor, the tumor weight inhibition rates for Drug A and Drug B were 42.47% and 38.02%, respectively, i.e., less than 60%.

Tail-vein intravenous injection group: For Drug C, the tumor weight inhibition rate of the left-side tumor was 32.79% (<60%); the tumor weight inhibition rate of the right-side tumor was 38.89% (<60%).

Table 53. Summary of tumor weights (intratumoral administration).

| Group | No. of animals (n) | Left tumor weight (g) | Left IR_TW (%) | Right tumor weight (g) | Right IR_TW (%) |
|---|---|---|---|---|---|
| Negative control | 10 | 2.69 ± 0.65 | \ | 3.89 ± 0.89 | \ |
| A | 10 | 0.58 ± 0.24*** | 78.44 | 2.24 ± 0.68*** | 42.47 |
| B | 10 | 0.82 ± 0.41*** | 69.44 | 2.41 ± 1.04** | 38.02 |
| C | 10 | 1.81 ± 0.69* | 32.79 | 2.38 ± 0.89** | 38.89 |
| **: *P* < 0.01, ****P* < 0.001;* comparisons are vs. the negative-control group. | | | | | |

IV. Experimental Conclusions:

Taking into account both the tumor growth inhibition rate and the tumor-weight inhibition rate, the conclusions are as follows:

In the intratumoral administration groups, both Drug A and Drug B exhibited significant inhibitory effects on the growth of the injected-side tumor (left-side tumor). The inhibitory effect increased progressively with the extension of the dosing period and was statistically significant. However, the inhibitory effect on the contralateral tumor (right-side tumor) was not significant. Tail-vein intravenous administration of Drug C did not show an obvious inhibitory effect on tumor growth.

Example 21

I. Abbreviations

CT26: murine CT26 colon carcinoma.

II. Objective of the Study:

To verify the antitumor efficacy of nano-nucleic-acid carrier combinations delivering immunomodulatory and gene-regulatory drugs.

III. Principal Instruments:

As in Example 16.

IV. Cell Information

CT26, mouse colon carcinoma; supplied by the Institute of Materia Medica, Chinese Academy of Medical Sciences.

V. Experimental Animal Information

Species: BALB/c

Age: 6-7 weeks

Sex: male

Body weight: 16-18 g

Supplier: Beijing Huafukang Biotechnology Co., Ltd.

VI. Experimental Protocol:

The animal inoculation and dosing regimens are shown in Tables 54 and 55.

Table 54. Animal inoculation and dosing regimen.

| Cell line | No. of mice (n) | Mouse IDs | Inoculation site | Inoculum (cells per mouse) |
|---|---|---|---|---|
| CT26 | 20 | See grouping table | Subcutaneous, left axillary dorsal region | $1 \times 10^6$ |
| Note: The day of inoculation was designated as D0. | | | | |

Table 55 Animal grouping and dosing

| Model | Group | No. of animals (n) | Treatment | Route of administra-tion | Dose volume per administra-tion | No. of administ rations | Dosing days |
|---|---|---|---|---|---|---|---|
| CT26 | Negative control (Control) | 10 | PBS | Tail-vein injec-tion (i.v.) | 200 μL | 5 | Day of randomi zation (D1), D3, D5, D7, D9 |
| CT26 | A | 10 | A | Tail-vein injec-tion (i.v.) | 200 μL | 5 | |

Wherein, in the "Treatment" column of Table 55, Drug A is composed of 2 *CpG1826-CD40 aptamer-DNA carrier (50 μg) + 2 *AmiR-21-CD40 aptamer-DNA carrier (6 μg) + OX40 (RNA; 2'-O-Me) (6 μg); that is, Drug A is a combination of Drug 3 + Drug 24 + an OX40 aptamer in RNA form.

VII. Experimental methods

1. Inoculation and grouping

For the murine colon carcinoma CT26 model, male BALB/c mice weighing 16.0-18.0 g were used. At the time of the experiment, well-growing tumor tissue was harvested, minced, ground, and diluted with sterile normal saline to prepare a tumor-cell suspension. CT26 cells were inoculated subcutaneously into the left axillary-dorsal region of each mouse at 1 $\times 10^6$ cells/0.2 mL; the day of inoculation was designated D0. On D5, animals were randomly assigned by tumor volume into groups: negative control (PBS) and Drug A (two groups), with 10 animals per group. Beginning on the day of grouping, all animals in each group received tail-vein injections, for a total of five administrations. Details of inoculation, grouping, and dosing are shown in Tables 54 and 55.

2. Measurement Indices

Same as in Example 16.

3. Statistical Analysis

Same as in Example 16.

VIII. Experimental Results:

1. Clinical observations in experimental animals

During the dosing period, the experimental animals exhibited good general condition, including normal activity and food intake.

2. Changes in body weight of experimental animals

During the dosing period, the body weight of animals in all groups increased to a certain extent; however, compared with the negative control group, the increase in body weight in the Drug A group was smaller, and the difference was statistically significant. The results are recorded in Table 56, and the body-weight growth curves are shown in Figure 21-1.

Table 56. Summary of animal body weight.

| Group | Number of animals (n) | | Body weight (g) | | Change in body weight (g) |
|---|---|---|---|---|---|
| | D0 | D15 | D0 | D15 | |
| Negative control | 10 | 10 | $17.5 \pm 0.6$ | $22.8 \pm 1.4$ | + 5.2 |
| A | 10 | 10 | $17.6 \pm 0.7$ | $20.3 \pm 1.2^{***}$ | + 2.7 |

***: P<0.001, compared with the negative control group.
Note: D0, the day of inoculation with the tumor cell suspension; D15, 15 days after inoculation with the tumor cell suspension.

3. Tumor volume-inhibition results

All animals were closely monitored for tumor growth during the study. Tumor size was measured three times per week, the results were recorded, and the tumor growth inhibition (TGI) was calculated.

Tumors in the negative control group grew well. Compared with the negative control group, in Group A on D15 the tumor growth inhibition rate (calculated on the basis of tumor volume) was 57.18%, i.e., <60%; the T/C value (calculated on the basis of RTV) was 41.60%, i.e., >40%. The results are recorded in Tables 57-58, and the tumor growth curves are shown in Figures 21-2 and 21-3.

Table 57. Summary of tumor growth data.

| Days | Negative control | | Group A | | |
|---|---|---|---|---|---|
| | Tumor volume (mm$^3$) | N | Tumor volume (mm$^3$) | N | Tumor growth inhibition rate (%) |
| D5 | $98.5 \pm 23.0$ | 10 | $99.4 \pm 24.2$ | 10 | / |
| D7 | $384.5 \pm 106.3$ | 10 | $303.6 \pm 103.3$ | 10 | 21.03 |
| D9 | $698.0 \pm 223.5$ | 10 | $575.7 \pm 270.2$ | 10 | 17.53 |
| D12 | $1111.3 \pm 260.5$ | 10 | $648.6 \pm 309.9^{**}$ | 10 | 41.64 |
| D14 | $1900.4 \pm 428.3$ | 10 | $863.5 \pm 611.1^{***}$ | 10 | 54.56 |
| D15 | $2467.6 \pm 756.6$ | 10 | $1056.6 \pm 983.9^{***}$ | 10 | 57.18 |

**: P<0.01, ***: P<0.001, compared with the negative control group.
Note: Days: number of days after inoculation with the tumor cell suspension; N: number of animals in the respective group included in the statistics.

Table 58. Summary of tumor growth data.

| Days | Negative control | | Group A | | |
|---|---|---|---|---|---|
| | RTV | N | RTV | N | T/C (%) |
| D5 | $1.00 \pm 0$ | 10 | $1.00 \pm 0$ | 10 | / |
| D7 | $4.08 \pm 1.46$ | 10 | $3.29 \pm 1.65$ | 10 | 80.56 |
| D9 | $7.29 \pm 2.61$ | 10 | $5.96 \pm 3.30$ | 10 | 81.78 |
| D12 | $11.55 \pm 2.75$ | 10 | $6.66 \pm 3.51^{**}$ | 10 | 57.67 |
| D14 | $20.09 \pm 6.10$ | 10 | $8.80 \pm 6.68^{***}$ | 10 | 43.80 |
| D15 | $25.70 \pm 8.06$ | 10 | $10.69 \pm 10.99^{**}$ | 10 | 41.60 |

*P<0.05, **P<0.01, ***P<0.001, compared with the negative control group.
Note: Days: number of days after inoculation with tumor cells; N: number of animals in the respective group included in the statistics.

4. Tumor weight and gross observation results

At 15 days after CT26 inoculation, all animals were euthanized by cervical dislocation. Tumors were excised, weighed, and photographed. The results are summarized in Table 59 and Figure 21-4.

In the negative control group, tumors grew well. Upon gross examination after excision, necrotic tissue within the tumors was minimal (only 1/10 animals showed an obvious necrotic area), and the tumor interiors were rich in blood. In the group receiving tail-vein injections of Drug A, tumors in 6 animals were markedly reduced in size and the tumor tissue appeared gray-white; in 4 animals, obvious intratumoral necrotic areas were visible and there was relatively little blood. The tumor-weight inhibition rate of Drug A was 60.78%.

Table 59. Summary of tumor weights.

| Group | No. of animals (n) | Left-side tumor weight (g) | Left-side IR_TW (%) |
|---|---|---|---|
| Negative control | 10 | $3.32 \pm 0.83$ | \ |
| A | 10 | $1.30 \pm 1.17$*** | 60.78 |
| ***: $P < 0.001$, compared with the negative-control group. | | | |

IX. Experimental Conclusions:

Taking into comprehensive consideration the tumor growth-inhibition rate and the tumor-weight inhibition rate, the conclusions are as follows:
Upon tail-vein administration of Drug A, at the end of the study (D15) the tumor inhibition rate based on tumor weight exceeded 60% and was statistically significant, indicating that Drug A exerts a certain inhibitory effect on the growth of murine CT26 colon carcinoma, and that the inhibitory effect increased progressively with extension of the dosing period. The tumor inhibition rate calculated on the basis of tumor volume did not exceed 60%, which is presumed to be related to the density of the tumor tissue and the size of intratumoral necrotic areas. In the Drug A group, obvious necrotic regions were visible within the tumor tissue and there was relatively little blood; in 6 animals the tumors were markedly reduced in size and the tumor tissue appeared gray-white.

Example 22

I. Objective of the Experiment:

The primary objective of this study is to establish a CT26 tumor model to verify the immunostimulatory effect and antitumor efficacy of the test articles, and to compare differences in antitumor efficacy (potency) among different drugs.

II. Cell Information

CT26, murine colon carcinoma cell line; purchased from Aili Biotechnology (Shanghai) Co., Ltd.

III. Experimental Animal Information

Species: BALB/cCrSlcNifdc mice (immunocompetent)

Age: 6-7 weeks

Sex: female

Body weight: 18-22 g

Supplier of experimental animals: National Institutes for Food and Drug Control (NIFDC), China.

IV. Experimental Methods

1. Cell culture

Tumor cells were cultured in RPMI-1640 medium supplemented with 10% heat-inactivated fetal bovine serum (FBS), penicillin 100 U/mL, streptomycin 100 $\mu$g/mL, and L-glutamine 2 mM at 37 °C in a humidified incubator with 5% $CO_2$. Cells were subcultured every 2-3 days. Tumor cells in the logarithmic growth phase were used for in-vivo tumor inoculation.

2. Inoculation and grouping

CT26 murine colon carcinoma cells were inoculated at $2 \times 10^6$ cells/0.1 mL subcutaneously into the left and right dorsal cervical regions of female BALB/c mice; the day of inoculation was designated D0. On D7, 2 mice were enrolled into each of Groups A and B (grouping criterion: left-side tumor volume 80-140 mm$^3$). On D9, the remaining mice whose tumor volumes met the grouping requirements were assigned to groups and dosing was initiated. Details of animal inoculation, grouping, and dosing are provided in Tables 60 and 61 and Figure 22-1.

The detailed information on animal inoculation, grouping and administration is shown in Tables 60 and 61 and Figure 22-1.

Table 60. Animal inoculation and dosing scheme

| Animal inoculation details | | | | |
|---|---|---|---|---|
| Cell line | No. of mice | Mouse ID Nos. | Inoculation site | Cell inoculum |
| CT26 | 16 | 276-291 | Subcutaneous, bilateral dorsal cervical region | $2\times10^6$/animal |

Table 61. Animal grouping and dosing

| Group | No. of animals | Test article | Route / site of administration | Dose volume | Dosing schedule |
|---|---|---|---|---|---|
| A | 3 | A | Intratumoral (left-side tumor) | 30/50ul | On the day of grouping (30 $\mu$L/20 g), and on Days 3 (20 $\mu$L), 5 (50 $\mu$L), 7 (50 $\mu$L), and 9(50$\mu$L) |
| B | 3 | B | Intratumoral (left-side tumor) | 30/50ul | On the day of grouping (30 $\mu$L/20 g), and on Days 3 (20 $\mu$L), 5 (50 $\mu$L), 7 (50 $\mu$L), and 9 (50 $\mu$L) |
| C | 3 | C | Intratumoral (left-side tumor) | 40/50ul | On the day of grouping (30 $\mu$L/20 g), and on Days 3 (20 $\mu$L), 5 (50 $\mu$L), 7 (50 $\mu$L), and 9(50$\mu$L) |

Wherein, A includes 2 *CpG1826-CD40 aptamer-DNA carrier (50 $\mu$g) + 2*AmiR-21-TTA1 aptamer-DNA carrier (6 $\mu$g) + OX40 (locked nucleic acid, LNA) (6 $\mu$g); B includes 2 *CpG1826-CD40 aptamer-DNA carrier (50 $\mu$g) + 2 *AmiR-21-TTA1 aptamer-DNA carrier (6 $\mu$g) + OX40 (RNA/OME) (6 $\mu$g); and C includes 2 *CpG1826-CD40 aptamer-DNA carrier (50 $\mu$g) + 2 *AmiR-21-TTA1 aptamer-DNA carrier (6 $\mu$g) + 3*CD40 aptamer (50 $\mu$g). Accordingly, Drug A corresponds to Combination 24 (Drug 3 + Drug 18 + an OX40 aptamer in LNA form), Drug B corresponds to Combination 25 (Drug 3 + Drug 18 + an OX40 aptamer in RNA/OME form), and Drug C corresponds to Drug 3 + Drug 18 + 3*CD40 aptamer.

3. Measurement indices

Same as in Example 16.

4. Statistical analysis

Same as in Example 16.

V. Experimental Results:

# EP 4 745 242 A1

1. Clinical observations in experimental animals

During the dosing period, the animals exhibited good general condition, including normal activity and food intake.

2. Changes in body weight of experimental animals

During the dosing period, body weight in all groups increased to a certain extent. Body-weight changes for all animals are shown in Table 62 and Figure 22-2; individual body-weight data are provided in Table 63.

Table 62 Summary of animal body weight

| Group | No. of animals | Before dosing | D24 | Change in body weight(g) |
|---|---|---|---|---|
| Group A | 3 | 20.23±1.28 | 22.62±1.64 | +2.39 |
| Group B | 3 | 20.40±1.11 | 23.20±0.90 | +2.80 |
| Group C | 3 | 20.00±0.73 | 22.21±0.39 | +2.21 |

Note: The body-weight data of one mouse in Group A, one mouse in Group B, and all mice in Group C were adjusted by time-regression to D24. "D24" denotes 24 days after tumor-cell inoculation.

Table 63. Summary of individual animal body-weight data

| Group | DAY | 7 | 9 | 11 | 13 | 15 | 17 | 19 | 21 | | 24 | 26 |
|---|---|---|---|---|---|---|---|---|---|---|---|---|
| | Animal ID | / | / | / | / | / | / | / | / | | / | / |
| Group A | 277 | 19.53 | 18.96 | 19.20 | 19.74 | 19.33 | 19.74 | 19.75 | 20.64 | | 21.27 | 20.71 |
| Group A | 283 | 21.71 | 20.72 | 21.31 | 21.77 | 21.67 | 22.19 | 22.51 | 23.47 | | 24.44 | 25.01 |
| Group A | 282 | 19.45 | 19.17 | 20.06 | 19.93 | 20.62 | 20.51 | 20.89 | 21.70 | | 22.16 | / |
| Group B | 278 | 20.19 | 20.58 | 20.80 | 20.44 | 21.23 | 21.50 | 21.44 | 22.05 | | 22.54 | 23.76 |
| Group B | 284 | 19.42 | 18.95 | 19.37 | 19.70 | 20.66 | 21.61 | 21.53 | 21.97 | | 22.83 | 24.00 |
| Group B | 286 | 21.60 | 20.60 | 21.23 | 21.78 | 21.26 | 22.32 | 22.95 | 23.46 | | 24.23 | / |
| Group C | 290 | 19.66 | 18.80 | 18.83 | 19.55 | 19.81 | 20.66 | 21.26 | 21.84 | | 21.97 | / |
| Group C | 291 | 20.84 | 20.16 | 20.64 | 21.00 | 21.46 | 21.88 | 21.93 | 21.89 | | 22.01 | / |
| Group C | 276 | 19.51 | 18.52 | 19.16 | 19.78 | 20.48 | 21.02 | 21.39 | 21.92 | | 22.66 | / |

Note: Because mice Nos. 282, 286, 290, 291, and 276 were enrolled later (post-grouping), their data were subjected to time-regression adjustment.

3. Tumor volume-inhibition results

All animals were closely monitored for tumor growth during the study; tumor dimensions were measured three times per week and tumor volume was recorded. In the nine mice, tumors on the non-dosed side grew well. On the dosed side, one mouse in Group A and one mouse in Group B showed tumor regrowth after dosing was discontinued, whereas tumors in the remaining mice did not grow due to drug-mediated inhibition.

A summary of tumor volume on the dosed (left) side is provided in Table 64, and the left-side tumor growth curves are shown in Figure 22-3. A summary of tumor volume on the non-dosed (right) side is provided in Table 65, and the right-side tumor growth curves are shown in Figure 22-4. Individual tumor-volume values (left side) are presented in Table 66, and individual tumor-volume values (right side) are presented in Table 67.

Table 64. Summary of tumor volume on the dosed side (left)

| Days | Group A | | Group B | | Group C | | | |
|---|---|---|---|---|---|---|---|---|
| | Tumor volume(mm$^3$)[N] | | Tumor volume(mm$^3$)[N] | | Tumor Volume (mm$^3$) | N | | |
| 7 | 108.2±27.8 3 | | 108.1±30.9 3 | | 111.5±23.4 | 3 | | |
| 9 | 130.4±30 | | 3 | | 124.7±48 | 3 | 153.5±39 | 3 |
| 11 | 84.9±20.4 | | 3 | | 147±71.8 | 3 | 120.9±41.8 | 3 |

(continued)

| Days | Group A | | Group B | | Group C | | | |
|---|---|---|---|---|---|---|---|---|
| | Tumor volume(mm$^3$)N | | Tumor volume(mm$^3$)N | | Tumor Volume (mm$^3$) | N | | |
| 13 | 73.1±11.8 | 3 | 103.8±42.3 | 3 | 120.8±65.6 | 3 | | |
| 15 | 77.2±11.5 | 3 | 103.7±69 | 3 | 97.8±42.3 | 3 | | |
| 17 | 76.2±33.6 | 3 | 101.7±75.7 | 3 | 88.2±18.2 | 3 | | |
| 19 | 70.1±52.7 | 3 | 101.2±79.5 | 3 | 80.4±28.3 | 3 | | |
| 21 | 74.1±50.5 | 3 | 77.9±89.4 | 3 | 39.6±12.3 | 3 | | |
| 24 | 95.7±145 | 3 | 115.7±148.3 | 3 | 35.9±22.4 | 3 | | |
| 26 | 225.2±318.5 | 2 | 180.9±219.7 | 2 | / | | / | |

Note: The body-weight data of one mouse in Group A, one mouse in Group B, and all mice in Group C were subjected to time-regression adjustment. Days: number of days after tumor-cell inoculation.N: number of animals in the group included in the statistical analysis.

The main purpose was to establish a CT26 tumor model, verify the immunostimulatory response and antitumor efficacy of the test articles, and compare differences in antitumor efficacy (potency) among different drugs. During the dosing period, the experimental animals exhibited good general condition (activity, food intake, etc.), and body weight increased to a certain extent.

On the left (dosed) side, tumors in Groups A, B, and C were markedly inhibited, growth ceased and regressed; after dosing was discontinued, one mouse each in Groups A and B showed renewed growth, whereas the other tumors did not regrow. On the right (non-dosed) side, tumors in Groups A, B, and C were not inhibited and grew well.

Table 65. Summary of tumor volume on the non-dosed side (right)

| Days | A group | | B group | | C group | |
|---|---|---|---|---|---|---|
| | Tumor volume(mm$^3$) | N | Tumor volume(mm$^3$) | N | Tumor volume (mm$^3$) | N |
| 7 | 73.7±12.2 | 3 | 96.6±26.4 | 3 | 89.2±46.4 | 3 |
| 9 | 95.4±18.8 | 3 | 150.8±52.9 | 3 | 163.3±152.6 | 3 |
| 11 | 161.1±59.3 | 3 | 275.8±41.8 | 3 | 268.8±285.6 | 3 |
| 13 | 258.3±41.8 | 3 | 465.4±136 | 3 | 497.5±597.5 | 3 |
| 15 | 285.5±92.3 | 3 | 568.7±227 | 3 | 620.7±729.5 | 3 |
| 17 | 414.8±160.5 | 3 | 748.8±239.9 | 3 | 770±908.2 | 3 |
| 19 | 616±194.6 | 3 | 1055.5±324.7 | 3 | 965.7±1071.4 | 3 |
| 21 | 1062.4±410.8 | 3 | 1441.5±302.6 | 3 | 1305.5±1143 | 3 |
| 24 | 1511.8±314.8 | 3 | 2072.5±621.3 | 3 | 1405.7±983.9 | 3 |
| 26 | 1890.3±681.7 | 2 | 2639.5±642.1 | 2 | / | / |

Note: The body-weight data of one mouse in Group A, one mouse in Group B, and all mice in Group C were subjected to time-regression adjustment.Days: number of days after tumor-cell inoculation.N: number of animals in the group included in the statistical analysis.

Table 66. Individual mouse tumor-volume values (left side)

| Group | DAY | 7 | 9 | 11 | 13 | 15 | 17 | 19 | 21 | 24 | 26 |
|---|---|---|---|---|---|---|---|---|---|---|---|
| A | 277 | 137.7 | 163.5 | 106.7 | 86.0 | 74.5 | 45.3 | 29.1 | 36.6 | 0.0 | 0.0 |
| A | 283 | 104.2 | 122.7 | 66.2 | 70.5 | 89.8 | 112.0 | 129.6 | 131.5 | 262.6 | 450.4 |
| A | 282 | 82.7 | 105.0 | 81.8 | 62.8 | 67.3 | 71.4 | 51.7 | 54.2 | 24.6 | / |
| B | 278 | 136.4 | 155.4 | 158.8 | 125.0 | 80.8 | 72.6 | 56.1 | 34.3 | 32.8 | 25.6 |
| B | 284 | 112.8 | 149.1 | 212.1 | 131.2 | 181.2 | 187.7 | 192.9 | 180.7 | 286.9 | 336.3 |
| B | 286 | 75.0 | 69.4 | 70.0 | 55.0 | 49.1 | 44.9 | 54.4 | 18.7 | 27.4 | / |
| C | 290 | 138.4 | 196.4 | 143.6 | 173.4 | 141.3 | 90.6 | 113.1 | 52.4 | 60.1 | / |

(continued)

| Group | DAY | 7 | 9 | 11 | 13 | 15 | 17 | 19 | 21 | 24 | 26 |
|-------|-----|-----|-----|-----|-----|-----|-----|-----|-----|-----|-----|
| C | 291 | 100.7 | 144.2 | 146.5 | 141.6 | 95.3 | 105.0 | 63.8 | 27.7 | 31.6 | / |
| C | 276 | 95.5 | 120.1 | 72.7 | 47.3 | 56.8 | 68.9 | 64.2 | 38.6 | 16.0 | / |

Note: Because mice Nos. 282, 286, 290, 291, and 276 were enrolled later (post-grouping), their data were subjected to time-regression adjustment.

**Table 67. Individual mouse tumor-volume values (right side)**

| Group | DAY | 7 | 9 | 11 | 13 | 15 | 17 | 19 | 21 | 24 | 26 |
|-------|-----|-----|-----|-----|-----|-----|-----|-----|-----|-----|-----|
| A | 277 | 83.5 | 87.3 | 100.7 | 210.5 | 180.3 | 231.9 | 396.5 | 589.5 | 1153.1 | 1408.3 |
| A | 283 | 60.0 | 81.9 | 163.4 | 288.5 | 352.7 | 532.2 | 767.3 | 1266.0 | 1742.2 | 2372.3 |
| A | 282 | 77.6 | 116.9 | 219.3 | 275.9 | 323.5 | 480.4 | 684.1 | 1331.6 | 1640.0 | / |
| B | 278 | 123.0 | 206.5 | 322.9 | 599.6 | 750.2 | 924.0 | 1366.1 | 1779.5 | 2741.3 | 3093.5 |
| B | 284 | 70.3 | 101.3 | 261.3 | 468.8 | 641.9 | 847.0 | 1082.2 | 1349.0 | 1962.9 | 2185.5 |
| B | 286 | 96.7 | 144.7 | 243.3 | 327.8 | 314.1 | 475.4 | 718.3 | 1195.9 | 1513.2 | / |
| C | 290 | 140.1 | 335.6 | 598.1 | 1185.4 | 1458.2 | 1810.0 | 2194.9 | 2597.3 | 2501.0 | / |
| C | 291 | 49.4 | 45.2 | 88.8 | 107.6 | 123.6 | 133.5 | 229.4 | 425.3 | 596.7 | / |
| C | 276 | 78.1 | 109.1 | 119.5 | 199.5 | 280.4 | 366.4 | 472.8 | 893.8 | 1119.4 | / |

Note: Because mice Nos. 282, 286, 290, 291, and 276 were enrolled after group assignment, their data were subjected to time-regression adjustment.

VI. Experimental Conclusions

The primary objective of this study was to establish a CT26 tumor model, verify the immunostimulatory effects and antitumor efficacy of the test articles, and compare differences in antitumor efficacy (potency) among different drugs. During the dosing period, the experimental animals exhibited good general condition (activity, food intake, etc.), and body weight increased to some extent.

On the left (dosed) side, tumors in Groups A, B, and C were markedly inhibited, ceased growing, and regressed; after dosing was discontinued, one mouse each in Groups A and B showed renewed growth, whereas the remaining tumors did not regrow. On the right (non-dosed) side, tumors in Groups A, B, and C were not inhibited and grew well.

Example 23

I. Objective of the Study:

To verify the antitumor efficacy of nano-nucleic-acid carrier combinations carrying immunomodulatory and gene-regulatory drugs.

II. Cell Information

4T1, murine mammary carcinoma; supplied by the Institute of Materia Medica, Chinese Academy of Medical Sciences.

III. Experimental Animal Information

Species: BALB/c

Age: 8-10 weeks

Sex: female

Body weight: 16-18 g

Supplier: Beijing Huafukang Biotechnology Co., Ltd.

IV Experimental Methods

1. Inoculation and grouping

For the murine mammary carcinoma 4T1 model, female BALB/c mice weighing 16.0-18.0 g were used. At the time of the experiment, well-growing tumor tissue was harvested, minced and ground, and diluted with sterile normal saline to prepare a tumor-cell suspension. 4T1 mammary carcinoma cells were inoculated subcutaneously into the left axillary-dorsal region of each mouse at $1 \times 10^6$ cells/0.2 mL; the day of inoculation was designated D0. On D8, animals were randomized by tumor volume into groups: negative control (PBS) and Drug A (two groups), 10 animals per group. On the day of grouping, all animals began tail-vein intravenous dosing, for a total of eight administrations. Details of animal inoculation, grouping, and dosing are shown in Tables 68 and 69.

Table 68. Animal inoculation and dosing scheme.

| Cell line | No. of mice (n) | Mouse IDs | Inoculation site | Inoculum (cells per mouse) |
|---|---|---|---|---|
| 4T1 | 22 | See grouping table | Subcutaneous, left axillary dorsal region | $1 \times 10^6$ |
| Note: The day of inoculation was designated as D0. | | | | |

Table 69. Animal grouping and dosing

| Model | Group | No. of animals (n) | Treatment | Route of administration | Dose volume per administration | No. of administrations | Dosing days |
|---|---|---|---|---|---|---|---|
| 4T1 | Negative control (Control) | 10 | PBS | Tail-vein injection (i.v.) | 200 μL | 8 | Day of rando-miz ation (D1), D3, D5, D8, D11,D13 ,D15, D17 |
| 4T1 | A | 10 | A | Tail-vein injection (i.v.) | 200 μL | 8 | |

Wherein, A includes 2*CpG1826-CD40 aptamer-DNA carrier (50 μg) + 2 *AmiR-21-4*Bio-DNA carrier (6 μg) + OX40 (RNA/OME) (6 μg). Accordingly, Drug A corresponds to Drug 3 + Drug 20 + a CD40 aptamer in RNA form.

2. Measurement indices

Same as in Example 16.

3. Statistical analysis

Same as in Example 16.

V. Experimental results:

1. Clinical observations in experimental animals

In the negative control group, animals exhibited good general condition (activity, food intake, etc.) during the dosing period; however, one animal died accidentally during the third administration while being placed into the tail-vein injection restrainer. In the group receiving tail-vein injections of Drug A, some animals showed decreased activity, and one animal each died on D13, D17, and D20.

2. Changes in body weight of experimental animals

During the dosing period, body weight increased to a certain extent. The results are recorded in Table 70, and the body-weight growth curves are shown in Figure 23-1.

Table 70. Summary of animal body weight

| Group | Number of animals (n) | | Body weight (g) | | Change in body weight (g) |
|---|---|---|---|---|---|
| | D0 | D25 | D0 | D25 | |
| Negative control | 10 | 9 | $16.8 \pm 0.4$ | $19.6 \pm 1.6$ | + 2.8 |
| A | 10 | 7 | $17.3 \pm 0.5$ | $19.3 \pm 1.1$ | + 2.0 |
| Note: D0, the day of inoculation with the tumor cell suspension; D25, 25 days after inoculation with the tumor cell suspension. | | | | | |

3. Tumor volume-inhibition results

All animals were closely monitored for tumor growth during the study; tumor dimensions were measured three times per week, the results were recorded, and the tumor growth inhibition (TGI) was calculated.

Tumors in the negative control group grew well. Compared with the negative control group, in Group A on D25 the tumor growth inhibition rate (calculated on the basis of tumor volume) was 85.10% (>60%); the T/C value (calculated on the basis of RTV) was 14.98% (<40%). The results are recorded in Tables 71-72, and the tumor growth curves are shown in Figures 23-2 and 23-3.

**Table 71.** Summary of tumor growth data.

| Days | Negative control | | Group A | | |
|---|---|---|---|---|---|
| | Tumor volume (mm$^3$) | N | Tumor volume (mm$^3$) | N | Tumor growth inhibition rate (%) |
| D8 | $117.5 \pm 5.7$ | 10 | $117.5 \pm 4.3$ | 10 | / |
| D11 | $328.7 \pm 45.8$ | 10 | $288.6 \pm 88.0$ | 10 | 12.19 |
| D13 | $458.0 \pm 66.6$ | 9 | $154.7 \pm 51.9$*** | 9 | 66.22 |
| D15 | $619.1 \pm 62.2$ | 9 | $159.0 \pm 62.6$*** | 9 | 74.31 |
| D18 | $910.8 \pm 163.7$ | 9 | $205.8 \pm 70.2$*** | 8 | 77.40 |
| D20 | $1125.6 \pm 280.6$ | 9 | $245.3 \pm 86.3$*** | 8 | 78.23 |
| D22 | $1436.9 \pm 275.1$ | 9 | $222.4 \pm 136.0$*** | 7 | 84.52 |
| D25 | $1672.6 \pm 446.4$ | 9 | $249.2 \pm 149.1$*** | 7 | 85.10 |
| ***: P<0.001, compared with the negative control group. Note: Days: number of days after inoculation with the tumor cell suspension; N: number of animals in the respective group included in the statistics. | | | | | |

Table 72. Summary of tumor growth data

| Days | Negative control | | A group | | |
|---|---|---|---|---|---|
| | RTV | N | RTV | N | T/C (%) |
| D8 | $1.00 \pm 0$ | 10 | $1.00 \pm 0$ | 10 | / |
| D11 | $2.81 \pm 0.46$ | 10 | $2.46 \pm 0.75$ | 10 | 87.44 |
| D13 | $3.87 \pm 0.58$ | 9 | $1.31 \pm 0.41$*** | 9 | 33.91 |
| D15 | $5.26 \pm 1.46$ | 9 | $1.35 \pm 0.51$*** | 9 | 25.67 |
| D18 | $7.73 \pm 1.52$ | 9 | $1.76 \pm 0.60$*** | 8 | 22.83 |

(continued)

| Days | Negative control | | A group | | |
|---|---|---|---|---|---|
| | RTV | N | RTV | N | T/C (%) |
| D20 | 9.56 ± 2.53 | 9 | 2.09 ± 0.73*** | 8 | 21.88 |
| D22 | 12.20 ± 2.58 | 9 | 1.88 ± 1.10*** | 7 | 15.40 |
| D25 | 14.18 ± 3.94 | 9 | 2.12 ± 0.93*** | 7 | 14.98 |

***: P<0.001, compared with the negative control group.
Note: Days: number of days after inoculation with the tumor cell suspension; N: number of animals in the respective group included in the statistics.

4. Tumor weight and gross observation results

At 25 days after 4T1 inoculation, all animals were euthanized by cervical dislocation; tumors were excised, weighed, and photographed. The results are summarized in Table 73 and Figure 23-4.

In the negative control group, tumors grew well; upon gross examination after excision, necrotic tissue was minimal within the tumors. After tail-vein administration of Drug A, obvious intratumoral necrotic areas were observed and less blood was present. The tumor-weight inhibition rate of Drug A was 80.43%.

Table 73. Summary of tumor weights.

| Group | No. of animals (n) | Left-side tumor weight (g) | Left-side IR_TW (%) |
|---|---|---|---|
| Negative control | 9 | 2.77 ± 0.51 | \ |
| A | 7 | 0.54 ± 0.31*** | 80.43 |

***: P < 0.001, compared with the negative-control group.

VI. Experimental Conclusions:

Taking into comprehensive consideration the tumor growth-inhibition rate and the tumor-weight inhibition rate, the conclusions are as follows:

Following tail-vein administration of Drug A, at the end of the study (D25) the tumor inhibition rate-based on both tumor weight and tumor volume-exceeded 80% and was statistically significant, indicating that Drug A has a marked growth-inhibitory effect on murine 4T1 mammary carcinoma, and that the inhibitory effect increased progressively with extension of the dosing period. In Drug-A-treated animals the tumors were markedly reduced, with obvious intratumoral necrotic areas and relatively little blood observed. However, three animals in the dosing group died.

Example 24

Objective of the study, principal instruments, and experimental-animal information: same as in Example 20.

I. Experimental Protocol:

The animal inoculation and dosing regimens are shown in Tables 74 and 75.

Table 74. Animal inoculation and dosing scheme.

| Cell line | No. of mice (n) | Mouse IDs | Inoculation site | Inoculum (cells per mouse) |
|---|---|---|---|---|
| B16F10 | 22 | See grouping table | Subcutaneous, bilateral axillary dorsal regions (left and right) | $1 \times 10^6$ |

Note: The day of inoculation was designated as D0.

Table 75. Animal grouping and dosing

| Group | No. of mice (n) | Effector molecule | Route of administration | Dosing schedule |
|---|---|---|---|---|
| Negative control (Control) | 10 | PBS | Intratumoral injection | D9 and D11: 40 μL per mouse; D13 and D15: 50 μL per mouse |
| A | 10 | A | Intratumoral injection | |

Wherein, under "Treatment" in Table 75, Drug A includes 2*CPG1826-CD40 aptamer-2 *mannose-DNA carrier (50 μg) +AmiR-21-AS1411 aptamer-A15 aptamer-2*biotin-DNA carrier (12 μg). Accordingly, Drug A corresponds to Combination 2 (Drug 2 + Drug 26).

II. Experimental Methods

1. Inoculation and grouping

For the murine melanoma B16F10 model, C57BL/6J male mice weighing 16.0-18.0 g were used. At the time of the experiment, well-growing tumor tissue was harvested, minced and ground, and diluted with sterile normal saline to prepare a tumor-cell suspension. B16F10 melanoma cells were inoculated subcutaneously into the left and right axillary-dorsal regions of each mouse at $1 \times 10^6$ cells/0.2 mL; the day of inoculation was designated D0. On D9, animals were randomized by tumor volume into groups: negative control (PBS) and Drug A (two groups), 10 animals per group. On the day of grouping, all animals in each group began intratumoral injections, for a total of four administrations.

During the study, tumor volume and body weight were measured and recorded three times per week. At study end, animals were euthanized by cervical dislocation; tumors were excised, weighed, and photographed. Based on tumor volume and mass, the tumor growth inhibition (1-T/C, %) and the tumor-weight inhibition IRTW (%) were calculated. Details of inoculation, grouping and dosing are provided in Tables 74 and 75.

2. Measurement indices

Same as in Example 16.

3. Statistical analysis

Same as in Example 16.

III. Experimental Results:

1. Clinical observations in experimental animals

During the dosing period, the animals exhibited good general condition, including normal activity and food intake.

2. Changes in body weight of experimental animals

During the dosing period, body weight increased to some extent in all animals. The body-weight gain in the dosing groups was lower than that in the negative control group, but the difference was not statistically significant. Body-weight changes for all animals are shown in Table 76 and Figure 24-1.

Table 76. Summary of animal body weight

| Group | Number of animals | | Body weight (g) | | Change in body weight (g) |
|---|---|---|---|---|---|
| | D0 | D16 | D0 | D16 | |
| Negative control | 10 | 10 | 16.6 ± 0.4 | 20.9 ± 1.2 | +4.4 |
| A | 10 | 10 | 16.4 ± 0.5 | 19.7 ± 0.8 | + 3.3 |
| Note: D0, the day of inoculation with the tumor cell suspension; D16, 16 days after inoculation with the tumor cell suspension. | | | | | |

3. Tumor volume-inhibition results

All animals were closely monitored for tumor growth during the study; tumor size was measured three times per week, the results were recorded, and the tumor growth inhibition (TGI) was calculated.

Tumors in the negative control group grew well. Compared with the negative control group, in Group A on D16 the tumor growth inhibition rate (calculated on the basis of tumor volume) was 63.14% (>60%); the T/C value (calculated on the basis of RTV) was 36.70% (<40%); and the left-side tumor-weight inhibition rate was 69.17% (>60%).

The results are recorded in Tables 77-78, and the tumor growth curves are shown in Figures 24-2 and 24-3.

Table 77. Summary of tumor growth data.

| Days | Negative control | | Group A | | |
|---|---|---|---|---|---|
| | Tumor volume (mm$^3$) | N | Tumor volume (mm$^3$) | N | Tumor growth inhibition rate (%) |
| D9 | 118.3 $\pm$ 12.7 | 10 | 119.1 $\pm$ 12.0 | 10 | / |
| D12 | 787.5 $\pm$ 445.5 | 10 | 635.1 $\pm$ 212.7 | 10 | 19.35 |
| D14 | 1226.0 $\pm$ 574.1 | 10 | 534.4 $\pm$ 161.4** | 10 | 56.41 |
| D16 | 1623.4 $\pm$ 565.3 | 10 | 598.4 $\pm$ 227.1*** | 10 | 63.14 |

**: P<0.01, ***: P<0.001, compared with the negative control group.
Note: Days: number of days after inoculation with the tumor cell suspension; N: number of animals in the respective group included in the statistics.

Table 78. Summary of tumor growth data

| Days | Negative control | | Group A | | |
|---|---|---|---|---|---|
| | RTV | N | RTV | N | T/C (%) |
| D9 | 1.00 $\pm$ 0 | 10 | 1.00 $\pm$ 0 | 10 | / |
| D12 | 6.57 $\pm$ 3.23 | 10 | 5.37 $\pm$ 1.75 | 10 | 81.76 |
| D14 | 10.22 $\pm$ 4.12 | 10 | 4.49 $\pm$ 1.22*** | 10 | 43.93 |
| D16 | 13.67 $\pm$ 4.35 | 10 | 5.02 $\pm$ 1.74*** | 10 | 36.70 |

***: P<0.001, compared with the negative control group.
Note: Days: number of days after inoculation with the tumor cell suspension; N: number of animals in the respective group included in the statistics.

4. Tumor weight results

At 16 days after B16F10 inoculation, all animals were euthanized by cervical dislocation; tumors were excised, weighed, and photographed. The results are summarized in Table 79 and Figure 24-4.

Following intratumoral injection of Drug A, the tumor-weight inhibition rate was 69.17%, i.e., greater than 60%.

Table 79. Summary of tumor weights.

| Group | No. of animals (n) | Tumor weight (g) | IR_TW (%) |
|---|---|---|---|
| Negative control | 10 | 2.60 $\pm$ 1.00 | \ |
| A | 10 | 0.80 $\pm$ 0.43*** | 69.17 |

***: P < 0.001, compared with the negative-control group.

IV. Experimental Conclusions:

Taking into comprehensive consideration the tumor growth-inhibition rate and the tumor-weight inhibition rate, the conclusions are as follows:

Intratumoral injection of Drug A produced a marked inhibitory effect on the growth of the injected-side tumor, which was statistically significant.

Example 25

Objective of the study, principal instruments, cell information, and experimental-animal information: same as in Example 21.

I. Experimental Protocol:

The animal inoculation and dosing regimens are shown in Tables 80 and 81.

Table 80. Animal inoculation and dosing scheme.

| Cell line | No. of mice (n) | Mouse IDs | Inoculation site | Inoculum (cells per mouse) |
|---|---|---|---|---|
| CT26 | 20 | See grouping table | Subcutaneous, left axillary dorsal region | $1 \times 10^6$ |
| Note: The day of inoculation was designated as D0. | | | | |

Table 81. Animal grouping and dosing

| Model | Group | No. of animals (n) | Treatment | Route of administration | Dose volume per administration | No. of administrations | Dosing days |
|---|---|---|---|---|---|---|---|
| CT26 | Negative control (Control) | 10 | PBS | Tail-vein injection (i.v.) | 200 $\mu$L | 6 | D1,D3, D5,D7, D9,D12 |
| CT26 | A | 10 | A | Tail-vein injection (i.v.) | 200 $\mu$L | 6 | |

Wherein, in the "Treatment" column of Table 81, Drug A includes 2*CpG1826-CD40 aptamer-DNA carrier (50 $\mu$g) + 2*AmiR-21-CD40 aptamer-DNA carrier (10 $\mu$g) + OX40 (DNA) (6 $\mu$g). Accordingly, Drug A corresponds to Combination 10 (Drug 3 + Drug 24 + an OX40 aptamer in DNA form).

II. Experimental Methods

1. Inoculation and grouping

For the murine colon carcinoma CT26 model, male BALB/c mice weighing 16.0-18.0 g were used. At the time of the experiment, well-growing tumor tissue was harvested, minced and ground, and diluted with sterile normal saline to prepare a tumor-cell suspension. CT26 cells were inoculated subcutaneously into the left axillary-dorsal region of each mouse at $1 \times 10^6$ cells/0.2 mL; the day of inoculation was designated D0. On D1, animals were randomly assigned to groups: negative control (PBS) and Drug A (two groups), 10 animals per group. On the day of grouping, all groups began tail-vein intravenous dosing, for a total of six administrations. Details of inoculation, grouping, and dosing are provided in Tables 80 and 81.

2. Measurement indices

Same as in Example 16.

3. Statistical analysis

Same as in Example 16.

III. Experimental Results:

1. Clinical observations in experimental animals

During the dosing period, the animals exhibited good general condition, including normal activity and food intake. After tail-vein injection of Drug A, three animals died.

2. Changes in body weight of experimental animals

During the dosing period, body weight increased to a certain extent in all animals; however, compared with the negative control group, the increase in body weight in the Drug A group was smaller, and the difference was statistically significant. The results are recorded in Table 82, and the body-weight growth curves are shown in Figure 25-1.

Table 82. Summary of animal body weight

| Group | Number of animals (n) | | Body weight (g) | | Change in body weight (g) |
|---|---|---|---|---|---|
| | D0 | D15 | D0 | D15 | |
| Negative control | 10 | 10 | $16.6 \pm 0.6$ | $24.4 \pm 1.2$ | + 7.9 |
| A | 10 | 7 | $17.0 \pm 1.0$ | $19.8 \pm 1.2$*** | + 2.8 |
| ***: P<0.001, compared with the negative control group.<br>Note: D0, the day of inoculation with the tumor cell suspension; D15, 15 days after inoculation with the tumor cell suspension. | | | | | |

3. Tumor volume-inhibition results

All animals were closely monitored for tumor growth during the study; tumor dimensions were measured three times per week, the results were recorded, and the tumor growth inhibition (TGI) was calculated.
Tumors in the negative control group grew well. Compared with the negative control group, the tumor growth inhibition rate for Group A on D15 (calculated on the basis of tumor volume) was 89.67%, i.e., greater than 60%. The results are recorded in Table 83, and the tumor growth curves are shown in Figure 25-1.

Table 83. Summary of tumor growth data

| Days | Negative control | | Group A | | |
|---|---|---|---|---|---|
| | Tumor volume (mm$^3$) | N | Tumor volume (mm$^3$) | N | Tumor growth inhibition rate (%) |
| D8 | $168.8 \pm 33.0$ | 10 | $98.3 \pm 20.6$*** | 9 | 41.76 |
| D10 | $345.4 \pm 123.6$ | 10 | $97.2 \pm 33.1$*** | 9 | 71.87 |
| D13 | $1184.1 \pm 423.5$ | 10 | $170.6 \pm 74.0$*** | 7 | 85.59 |
| D15 | $1564.5 \pm 546.7$ | 10 | $161.7 \pm 98.8$*** | 7 | 89.67 |
| ***: P<0.001, compared with the negative control group.<br>Note: Days: number of days after inoculation with the tumor cell suspension; N: number of animals in the respective group included in the statistics. | | | | | |

4. Tumor weight and gross observation results

At 15 days after CT26 inoculation, all animals were euthanized by cervical dislocation; tumors were excised, weighed, and photographed. The results are summarized in Table 84 and Figure 25-3.
In the negative control group, tumors grew well; gross examination after excision showed minimal necrotic tissue within the tumors and abundant intratumoral blood. Following tail-vein administration of Drug A, tumors in the seven surviving animals were markedly reduced in size with less intratumoral blood. The tumor-weight inhibition rate of Drug A was 90.80%.

Table 84. Summary of tumor weights.

| Group | No. of animals (n) | Left tumor weight (g) | Left IR_TW (%) |
|---|---|---|---|
| Negative control | 10 | $2.59 \pm 0.81$ | \ |

(continued)

| Group | No. of animals (n) | Left tumor weight (g) | Left IR_TW (%) |
|-------|--------------------|-----------------------|----------------|
| A | 7 | 0.24 ± 0.19*** | 90.80 |

***: P < 0.001, compared with the negative-control group.

IV. Experimental Conclusions:

Taking into comprehensive consideration the tumor growth-inhibition rate and the tumor-weight inhibition rate, the conclusions are as follows:
Following tail-vein administration of Drug A, at the end of the study (D15) the tumor inhibition rate (calculated on the basis of tumor weight and tumor volume) exceeded 60% and was statistically significant, indicating that Drug A exerts a marked growth-inhibitory effect on murine CT26 colon carcinoma.

Example 26

I. Objective of the Study:

The primary objective was to establish a 4T1 tumor model and verify the antitumor efficacy of the drug.

II. Cell Information

4T1, murine mammary carcinoma; supplied by the Institute of Materia Medica, Chinese Academy of Medical Sciences.

III. Experimental Animal Information

Species: BALB/c

Age: 7-8 weeks

Sex: female

Body weight: 16-18 g

Supplier: Beijing Huafukang Biotechnology Co., Ltd.

IV Experimental Methods

1. Inoculation and grouping

For the murine mammary carcinoma 4T1 model, BALB/c female mice weighing 16.0-18.0 g were used. At the time of the experiment, well-growing tumor tissue was harvested, minced and ground, and diluted with sterile normal saline to prepare a tumor-cell suspension. 4T1 mammary carcinoma cells ($1 \times 10^6$ cells/0.2 mL) were inoculated subcutaneously into the left axillary-dorsal region of each mouse and the animals were grouped; the day of inoculation was designated D0. The animals were divided into three groups-negative control (PBS), Drug A, and Drug B-with 10 animals per group. On D3, the above three groups received tail-vein injections, for a total of eight administrations.
During the study, tumor volume and body weight were measured and recorded three times per week. At the end of the study, animals were euthanized by cervical dislocation; tumors were excised, weighed, and photographed. Based on tumor volume and tumor mass, the tumor growth inhibition rate (1-T/C, %) and the tumor-weight inhibition rate (IRTW, %) were calculated. Details of animal inoculation, grouping, and dosing are provided in Tables 85 and 86.

Table 85. Animal inoculation and dosing scheme

| Cell line | No. of mice | Mouse ID Nos. | Inoculation site | Cell inoculum |
|---|---|---|---|---|
| 4T1 | 32 | See grouping table | Subcutaneous, left axillary-dorsal region | $1\times10^6$ cells/mouse |
| *Note:* The day of inoculation was designated **D0.** | | | | |

Table 86. Animal grouping and dosing

| Model | Group | No. of animal s | Treatm ent | Route of administ ration | Dose volume per administra tion | No. of admi nistra tions | Dosing schedule |
|---|---|---|---|---|---|---|---|
| 4T1 | Negative control (Control) | 10 | PBS | Tail-vei n injection | 200μl | 8 | D3, D5, D7, D9, D11, D13, D15, D17 |
| 4T1 | A | 10 | A | | | | |
| 4T1 | B | 10 | B | | | | |

Wherein, A includes 2*CpG1826-CD40 aptamer-DNA carrier (50 μg) + 2 *AmiR-21-4*Bio-DNA carrier (12 μg); B includes 2*CpG1826-CD40 aptamer-DNA carrier (25 μg) + 2*AmiR-21-4*Bio-DNA carrier (12 μg). Accordingly, Drugs A and B correspond to Drug 3 + Drug 20.

2. Measurement indices

Same as in Example 16.

3. Statistical analysis

Same as in Example 16.

V. Experimental Results

1. Clinical observations in experimental animals

In Group A, one animal died after tail-vein injection dosing (death on D13). Animals in the other groups exhibited good general condition (activity, food intake, etc.) during the dosing period.

2. Changes in body weight of experimental animals

During the dosing period, body weight increased to some extent in all animals; however, in the group receiving tail-vein injections of Drug A, body weight was markedly lower than that of the negative control group, and the difference versus the negative control group was statistically significant. In the group receiving tail-vein injections of Drug B, body weight showed no statistically significant difference compared with the negative control group. Body-weight changes for all animals are shown in Table 87 and Figure 26-1.

Table 87. Summary of animal body weight

| Group | No. of animals | | Body weight(g) | | Change in bodyweight (g) |
|---|---|---|---|---|---|
| | D0 | D20 | D0 | D20 | |
| Negative control | 10 | 10 | 17.1±0.6 | 19.2±1.2 | +2.09 |
| A | 10 | 9 | 17.1±0.3 | 17.6±1.0** | +0.58 |

(continued)

| Group | No. of animals | | Body weight(g) | | Change in bodyweight (g) |
|---|---|---|---|---|---|
| | D0 | D20 | D0 | D20 | |
| B | 10 | 10 | $17.1\pm0.4$ | $18.3\pm0.9$ | + 1.26 |

** P < 0.01 vs. negative control group.
D0: day of tumor-cell inoculation. D20: 20 days after tumor-cell inoculation.

## 3. Tumor volume-inhibition results

All animals were closely monitored for tumor growth during the study; tumor dimensions were measured three times per week, the results were recorded, and the tumor growth inhibition (TGI) was calculated.

Compared with the negative control group, on D20 the tumor growth inhibition rates in Groups A and B (calculated on the basis of tumor volume) were 61.88% and 59.09%, respectively; thus, the TGI for Group A was >60%, whereas that for Group B was <60%.

The results are recorded in Table 88, and the tumor growth curves are shown in Figure 25-2.

Table 88. Summary of tumor growth data

| Days | Negative control | | A | | | B | | |
|---|---|---|---|---|---|---|---|---|
| | Tumor volume (mm$^3$) | N | Tumor volume (mm$^3$) | N | Tumor growth inhibition rate(%) | Tumor volume (mm$^3$), | N | Tumor growth inhibition rate (%) |
| D9 | $153.8 \pm 31.3$ | 10 | $128.0 \pm 38.9$ | 10 | 16.79 | $200.9 \pm 43.0*$ | 10 | / |
| D11 | $435.4 \pm 78.1$ | 10 | $247.2 \pm 65.5***$ | 10 | 43.22 | $261.5 \pm 79.8***$ | 10 | 39.95 |
| D14 | $580.8 \pm 122.0$ | 10 | $317.4 \pm 82.0***$ | 9 | 45.36 | $367.8 \pm 97.4***$ | 10 | 36.68 |
| D16 | $777.3 = 140.9$ | 10 | $395.0 \pm 111.7***$ | 9 | 49.18 | $439.8 \pm 163.6***$ | 10 | 43.42 |
| D18 | $1104.4 \pm 253.1$ | 10 | $475.0 \pm 105.5***$ | 9 | 56.99 | $473.8 \pm 165.9***$ | 10 | 57.10 |
| D20 | $1625.5 \pm 558.7$ | 10 | $619.6 \pm 158.8***$ | 9 | 61.88 | $665.0 \pm 308.5***$ | 10 | 59.09 |

*: P<0.05; ***: P<0.001, compared with the negative control group.
Note: Days: days after inoculation with tumor cell suspension; N: number of animals in the respective group included in the statistics.

## 4. Tumor weight results

At 20 days after 4T1 inoculation, all animals were euthanized by cervical dislocation; tumors were excised, weighed, and photographed. The results are summarized in Table 89 and Figure 25-3.

Following tail-vein administration of Drugs A and B, the tumor-weight inhibition rates (IRTW%) were 60.62% and 59.86%, respectively-i.e., Group A > 60% and Group B < 60%.

Table 89. Summary of tumor weights

| Group | No. of animals | Tumor weight (g) | IR$_{TW}$% |
|---|---|---|---|
| Negative control | 10 | $1.83\pm0.44$ | / |
| A | 9 | $0.72\pm0.21***$ | 60.62 |
| B | 10 | $0.74\pm0.39***$ | 59.86 |

***: P < 0.001, compared with the negative control group.

## VI. Experimental Conclusions

Taking into comprehensive consideration the tumor growth-inhibition rate and the tumor-weight inhibition rate, the conclusions are as follows:

Tail-vein administration of Drugs A and B produced a certain inhibitory effect on tumor growth, and the effect was statistically significant.

Example 27

I. Objective of the Study:

The primary objective was to establish an A20 tumor model and verify the antitumor efficacy of the test articles.

II. Cell Information

A20, murine B-cell lymphoma; supplied by the Institute of Materia Medica, Chinese Academy of Medical Sciences.

III. Experimental Animal Information

Species: BALB/c

Age: 7-8 weeks

Sex: male

Body weight: 16-18 g

Supplier: Beijing Huafukang Biotechnology Co., Ltd.

IV Experimental Methods

1. Inoculation and grouping

For the murine B-cell lymphoma A20 model, male BALB/c mice weighing 16.0-18.0 g were used. At the time of the experiment, well-growing tumor tissue was harvested, minced and ground, and diluted with sterile normal saline to prepare a tumor-cell suspension. A20 B-cell lymphoma cells ($1 \times 10^6$ cells/0.2 mL) were inoculated subcutaneously into the left axillary-dorsal region of each mouse, and the animals were grouped; the day of inoculation was designated D0. The animals were divided into three groups-negative control (PBS), Drug A, and Drug B-with 10 animals per group. Animals in the Drug A group received tail-vein injections starting on D1, for a total of 7 administrations; animals in the negative control and Drug B groups received tail-vein injections starting on D9, for a total of 4 administrations.

During the study, tumor volume and body weight were measured and recorded three times per week. At the end of the study, animals were euthanized by cervical dislocation; tumors were excised, weighed, and photographed. Based on tumor volume and tumor mass, the tumor growth inhibition rate (1-T/C, %) and the tumor-weight inhibition rate (IRTW, %) were calculated. Details of animal inoculation, grouping, and dosing are provided in Tables 90 and 91.

Table 90. Animal inoculation and dosing scheme

| Cell line | No. of mice (n) | Mouse IDs | Inoculation site | Inoculum (cells per mouse) |
|---|---|---|---|---|
| A20 | 33 | See grouping table | Subcutaneous, left axillary dorsal region | $1 \times 10^6$ |
| **Note:** The day of inoculation was designated as **D0**. | | | | |

Table 91. Animal grouping and dosing

| Model | Group | Number of animals | Treatment | Route of administration | Dose volume per administration | Number of administrations | Dosing schedule |
|---|---|---|---|---|---|---|---|
| A20 | Negative control (Control) | 10 | PBS | Tail vein injection | 200 µL | 4 times | D9, D11, D13, D15 |
| A20 | B | 10 | B | | | | |
| A20 | A | 10 | A | Tail vein injection | 200 µL | 7 times | D1, D3, D5, D7, D11, D13, D15 |

Wherein, A includes CpG1826-CD40 aptamer-mannose-MUC1 aptamer-DNA carrier (50 µg) + 2*AmiR-21-MUC1-3*Bio-DNA carrier (6 µg) + OX40 (DNA) (6 µg); B includes CpG1826-CD40 aptamer-DNA carrier (26 µg) + AmiR-21-3*Bio-DNA carrier (12 µg). Accordingly, Drug A corresponds to Combination 18 (Drug 7 + Drug 25 + an OX40 aptamer in DNA form), and Drug B corresponds to Drug 45 in Table 5.

2. Measurement indices

Same as in Example 16.

3. Statistical analysis

Same as in Example 16.

V. Experimental Results:

1. Clinical observations in experimental animals

In Group A, animals died successively after tail-vein injection dosing (2 deaths on D8, 3 deaths on D12, and 1 death on D13). Animals in the other groups exhibited good general condition (activity, food intake, etc.) during the dosing period.

2. Changes in body weight of experimental animals

During the dosing period, body weight increased to some extent in all animals; however, in the Drug A group the mice had markedly lower body weight than the negative control group, and the difference versus the negative control group was statistically significant. In the Drug B group, mouse body weight showed no significant difference compared with the negative control group. Body-weight changes for all animals are shown in Table 92 and Figure 27-1.

Table 92. Summary of animal body weight

| Group | Number of animals (n) | | Body weight (g) | | Change in body weight (g) |
|---|---|---|---|---|---|
| | D0 | D17 | D0 | (g) D17 | |
| Negative control | 10 | 10 | 16.7±0.5 | 23.2 ± 2.3 | + 6.5 |
| A | 10 | 4 | 16.7±0.5 | 17.7 ± 0.6*** | +1.1 |
| B | 10 | 10 | 16.3 ± 0.4 | 21.8 ± 1.0 | + 5.5 |

***: $P < 0.001$, compared with the negative control group.
Note: D0, the day of inoculation with tumor cell suspension; D17, 17 days after inoculation with tumor cell suspension.

3. Tumor volume-inhibition results

All animals were closely monitored for tumor growth during the study; tumor dimensions were measured three times per week, the results were recorded, and the tumor growth inhibition (TGI) was calculated.

Compared with the negative control group, on D17 the tumor growth inhibition rates (calculated on the basis of tumor volume) were 90.91% for Group A and 21.19% for Group B; thus, Group A > 60% and Group B < 60%. The T/C value for Group B (calculated on the basis of RTV) was 77.67%, i.e., >40%.

The results are recorded in Tables 93-95, and the tumor growth curves are shown in Figures 27-2 and 27-3.

Table 93. Summary of tumor growth data

| Days | Negative control | | A | | |
|---|---|---|---|---|---|
| | Tumor volume (mm$^3$) | N | Tumor volume (mm$^3$) | N | Tumor growth inhibition rate (%) |
| D9 | 117.7 $\pm$ 11.5 | 10 | 79.7 $\pm$ 10.0 | 8 | 32.31 |
| D11 | 494.4 $\pm$ 127.9 | 10 | 140.9 $\pm$ 43.5*** | 8 | 71.51 |
| D13 | 921.4 $\pm$ 146.4 | 10 | 173.3 $\pm$ 61.4*** | 4 | 81.19 |
| D15 | 1250.5 $\pm$ 241.2 | 10 | 152.9 $\pm$ 36.2*** | 4 | 87.78 |
| D17 | 1942.8 $\pm$ 591.3 | 10 | 176.6 $\pm$ 38.0*** | 4 | 90.91 |

***: P<0.001, compared with the negative control group.
Note: "Days" denotes the number of days after inoculation with the tumor cell suspension; "N" denotes the number of animals in the group included in the statistics.

Table 94. Summary of tumor growth data

| Days | B | | |
|---|---|---|---|
| | Tumor volume (mm$^3$) | N | Tumor growth inhibition rate (%) |
| D9 | 118.2 $\pm$ 14.7 | 10 | / |
| D11 | 580.9 $\pm$ 157.4 | 10 | -- |
| D13 | 833.0 $\pm$ 159.8 | 10 | 9.60 |
| D15 | 1194.3 $\pm$ 341.2 | 10 | 4.49 |
| D17 | 1531.2 $\pm$ 476.1 | 10 | 21.19 |

Table 95. Summary of tumor growth data

| Days | Negative control | | B | | |
|---|---|---|---|---|---|
| | RTV | N | RTV | N | T/C (%) |
| D9 | 1.00 $\pm$ 0 | 10 | 1.00 $\pm$ 0 | 10 | / |
| D11 | 4.21 $\pm$ 1.08 | 10 | 4.96 $\pm$ 1.51 | 10 | 117.78 |
| D13 | 7.92 $\pm$ 1.67 | 10 | 7.14 $\pm$ 1.71 | 10 | 90.24 |
| D15 | 10.69 $\pm$ 2.22 | 10 | 10.04 $\pm$ 2.20 | 10 | 93.94 |
| D17 | 16.64 $\pm$ 5.39 | 10 | 12.93 $\pm$ 3.64 | 10 | 77.67 |

Note: "Days" denotes the number of days after inoculation with the tumor cell suspension; "N" denotes the number of animals in the group included in the statistics.

4. Tumor weight results

At 17 days after A20 inoculation, all animals were euthanized by cervical dislocation; tumors were excised, weighed, and photographed. The results are summarized in Table 96 and Figure 27-4.

Following tail-vein administration of Drugs A and B, the tumor-weight inhibition rates (IRTW%) were 95.61 % and 25.83%, respectively-i.e., Group A > 60% and Group B < 60%.

Table 96. Summary of tumor weights.

| Group | No. of animals (n) | Tumor weight (g) | IR_TW (%) |
|---|---|---|---|
| Negative control | 10 | 2.99 ± 0.82 | \ |
| A | 4 | 0.13 ± 0.11*** | 95.61 |
| B | 10 | 2.21 ± 0.73* | 25.83 |
| *: P < 0.05, ***: P < 0.001 (**); comparisons are vs. the negative-control group. | | | |

VI. Experimental Conclusions

Taking into comprehensive consideration the tumor growth-inhibition rate and the tumor-weight inhibition rate, the conclusions are as follows:
Tail-vein administration of Drug A produced a significant inhibitory effect on tumor growth, with statistical significance; tail-vein administration of Drug B did not show an obvious inhibitory effect on tumor growth.

Example 28

I. Objective of the Study:

To verify the antitumor efficacy of nano-nucleic-acid carrier combinations delivering immunomodulatory and gene-regulatory drugs.

II. Cell Information

4T1, murine mammary carcinoma; supplied by the Institute of Materia Medica, Chinese Academy of Medical Sciences.

III. Experimental Animal Information

Species: BALB/c

Body weight: 13.0-16.0 g

Sex: female

Supplier: Beijing Huafukang Biotechnology Co., Ltd.

IV. Experimental Methods

1. Inoculation and grouping

For the murine mammary carcinoma 4T1 model, female BALB/c mice weighing 13.0-16.0 g were used. At the time of the experiment, well-growing tumor tissue was harvested, minced and ground, and diluted with sterile normal saline to prepare a tumor-cell suspension. 4T1 cells ($1 \times 10^6$ cells/0.2 mL) were inoculated subcutaneously into the left axillary-dorsal region of each mouse; the day of inoculation was designated D0. On D7, animals were randomized by tumor volume into five groups: A (vehicle control), B, C, D, and E, with 8 animals per group. On the day of grouping, all groups began tail-vein injections, for a total of seven administrations. Details of inoculation, grouping, and dosing are provided in Tables 97 and 98.

Table 97. Animal inoculation and dosing scheme

| Cell line | No. of mice (n) | Mouse IDs | Inoculation site | Inoculum (cells per mouse) |
|---|---|---|---|---|
| 4T1 | 42 | See grouping table | Subcutaneous, left axillary dorsal region | $1 \times 10^6$ |
| **Note:** The day of inoculation was designated as **D0**. | | | | |

Table 98. Animal grouping and dosing

| Group | No. of animals (n) | Treatment | Route of administration | Dose volume per administration | No. of administrations | Dosing days |
|---|---|---|---|---|---|---|
| A | 8 | A | Tail-vein injection (i.v.) | 200 μL | 7 | Day of ran- |
| B | 8 | B | Tail-vein injection (i.v.) | 200 μL | 7 | domizat ion (D1), |
| C | 8 | C | Tail-vein injection (i.v.) | 200 μL | 7 | D3,D5, D7, |
| D | 8 | D | Tail-vein injection (i.v.) | 200 μL | 7 | D9,D11, |
| E | 8 | E | Tail-vein injection (i.v.) | 200 μL | 7 | D13 |

Wherein, A is PBS; B includes 2*CpG2006-CD40 aptamer-DNA carrier (50 μg) + 2 *AmiR-21-4*Bio-DNA carrier (6 μg) + OX40 (DNA) (6 μg); C includes 2*CpG2006-CD40 aptamer-DNA carrier (16 μg) + 2*AmiR-21-4*Bio-DNA carrier (6 μg) + OX40 (DNA) (6 μg); D includes 2*CpG2006-CD40 aptamer-DNA carrier (50 μg) + 2*AmiR-21-4*Bio-DNA carrier (6 μg); and E includes 2*CpG2006-CD40 aptamer-DNA carrier (50 μg). Accordingly, Drugs B and C correspond to Combination 16 (Drug 4 + Drug 20 + an OX40 aptamer in DNA form); Drug D corresponds to Combination 17 (Drug 4 + Drug 20); and Drug E corresponds to Drug 4 in Table 6.

2. Measurement indices

Same as in Example 16.

3. Statistical analysis

Same as in Example 16.

V. Experimental Results:

1. Clinical observations in experimental animals

During the dosing period, all animals exhibited good general condition (activity, food intake, etc.); one animal in Group D died on D14.

2. Changes in body weight of experimental animals

During the dosing period, body weight increased to a certain extent in all animals. The results are recorded in Table 99, and the body-weight growth curves are shown in Figure 28-1.

Table 99. Summary of animal body weight

| Group | No. of animals | | | Body weight (g) | | | Change in body weight (g) |
|---|---|---|---|---|---|---|---|
| | D0 | D7 | D21 | D0 | D7 | D21 | |
| A | 8 | 8 | 8 | 13.9 ± 0.9 | 16.1 ± 1.2 | 16.7±1.2 | +2.8 |
| B | 8 | 8 | 8 | 14.3 ± 1.1 | 16.3 ± 0.9 | 17.4 ± 0.8 | +3.1 |
| C | 8 | 8 | 8 | 13.6 ± 0.3 | 15.3 ± 0.7 | 16.7 ± 1.3 | +3.1 |
| D | 8 | 8 | 7 | 14.2 ± 0.9 | 16.0 ± 0.7 | 17.1 ± 1.3 | +2.9 |

(continued)

| Group | No. of animals | | | Body weight (g) | | | Change in body weight (g) |
|---|---|---|---|---|---|---|---|
| | D0 | D7 | D21 | D0 | D7 | D21 | |
| E | 8 | 8 | 7 | 13.9 ± 0.9 | 15.9 ± 1.2 | 16.8 ± 1.1 | +2.9 |

Note: D0 indicates the day of inoculation with the tumor cell suspension; D7 indicates 7 days after inoculation with the tumor cell suspension (i.e. the day of grouping and dosing); D21 indicates 21 days after inoculation with the tumor cell suspension.

3. Tumor volume-inhibition results

All animals were closely monitored for tumor growth during the study; tumor dimensions were measured 1-2 times per week, the results were recorded, and the tumor growth inhibition (TGI) was calculated.

Compared with Group A (vehicle control), on D21 the tumor growth-inhibition rates (calculated on the basis of tumor volume) for Groups B, C, D, and E were 12.16%, 11.06%, 45.29%, and 4.79%, respectively, i.e., <60%; the T/C values (calculated on the basis of RTV) were 87.48%, 89.82%, 56.17%, and 97.19%, respectively, i.e., <40%.

Table 100. Summary of tumor growth data

| Days | A | | B | | |
|---|---|---|---|---|---|
| | Tumor volume ($mm^3$) | N | Tumor volume ($mm^3$) | N | Tumor growth inhibition rate (%) |
| D7 | 95.0±11.0 | 8 | 94.3±10.3 | 8 | / |
| D14 | 522.7±211.6 | 8 | 432.8±121.3 | 8 | 17.20 |
| D18 | 1079.2±229.6 | 8 | 789.2±230.7 | 8 | 26.87 |
| D21 | 1463.0±281.5 | 8 | 1285.1±359.6 | 8 | 12.16 |

| Days | C | | | D | | | E | | |
|---|---|---|---|---|---|---|---|---|---|
| | Tumor volume ($mm^3$) | N | Tumor growth inhibition rate (%) | Tumor volume ($mm^3$) | N | Tumor growth inhibition rate (%) | Tumor volume ($mm^3$) | N | Tumor growth inhibition rate (%) |
| D7 | 94.8±49.3 | 8 | / | 93.6±49.2 | 8 | / | 94.2±49.5 | 8 | / |
| D14 | 547.9±259.8 | 8 | -4.81 | 260.9±104.1* | 7 | 50.09 | 428.6±114.4 | 8 | 18.01 |
| D18 | 889.4±333.5 | 8 | 17.58 | 520.5±265.1*** | 8 | 51.77 | 845.0±223.8 | 8 | 21.70 |
| D21 | 1301.2±334.7 | 8 | 11.06 | 800.3±275.0*** | 8 | 45.29 | 1392.9±375.2 | 8 | 4.79 |

*: P<0.05, **: P<0.01, ***: P<0.001, compared with group A (solvent control).
Note: Days: number of days after inoculation of the tumor cell suspension; N: number of animals in the group included in the statistical analysis.

Table 101. Summary of tumor growth data

| Days | A | | B | | |
|---|---|---|---|---|---|
| | RTV | N | RTV | N | T/C (%) |
| D7 | 1.00±0 | 8 | 1.00+0 | 8 | / |
| D14 | 5.57±2.46 | 8 | 4.61±1.36 | 8 | 82.84 |
| D18 | 11.42±2.51 | 8 | 8.29±2.17* | 8 | 72.58 |
| D21 | 15.38±2.43 | 8 | 13.46±2.88 | 8 | 87.48 |

| Days | C | | | D | | | E | | |
|---|---|---|---|---|---|---|---|---|---|
| | RTV | N | T/C (%) | RTV | N | T/C (%) | RTV | N | T/C (%) |
| D7 | 1.00±0 | 8 | / | 1.00±0 | 8 | / | 1.00±0 | 8 | / |
| D14 | 5.84±2.94 | 8 | 104.92 | 2.81±1.07* | 7 | 50.50 | 4.60±1.31 | 8 | 82.51 |
| D18 | 9.42±3.51 | 8 | 82.48 | 5.65±2.94** | 8 | 49.54 | 9.04±2.50 | 8 | 79.16 |
| D21 | 13.82±3.90 | 8 | 89.82 | 8.64±2.75*** | 8 | 56.17 | 14.95±4.41 | 8 | 97.19 |
| *: P<0.05, **: P<0.01, ***: P<0.001, compared with group A (solvent control). Note: Days: number of days after inoculation of the tumor cells; N: number of animals in the group included in the statistical analysis. | | | | | | | | | |

4. Tumor weight and gross observation results

At 21 days after 4T1 inoculation, all animals were euthanized by cervical dislocation; tumors were excised, weighed, and photographed. The results are summarized in Table 102 and Figure 28-4.

In Group A (vehicle control), tumors grew well; gross examination after excision showed little necrotic tissue within the tumor mass.

Following tail-vein administration, the tumor-weight-based inhibition rates for Groups B, C, D, and E were 16.73%, 24.19%, 44.70%, and 17.69%, respectively-i.e., all <60%.

Table 102. Summary of tumor weights.

| Group | No. of animals (n) | Tumor weight (g) | IR_TW (%) |
|---|---|---|---|
| A | 8 | 2.21 ± 0.50 | \ |
| B | 8 | 1.84 ± 0.47 | 16.73 |
| C | 8 | 1.68 ± 0.38* | 24.19 |
| D | 7 | 1.22 ± 0.46** | 44.70 |
| E | 8 | 1.82 ± 0.21 | 17.69 |
| *: $P < 0.05$, **: $P < 0.01$; comparisons are vs. Group A (vehicle control). | | | |

VI. Experimental Conclusions

Taking into comprehensive consideration the tumor growth-inhibition rate and the tumor-weight inhibition rate, the conclusions are as follows:

Following tail-vein administration of Drugs B, C, D, and E, at the end of the study (D21) the tumor inhibition rates (calculated on the basis of tumor weight and tumor volume) did not exceed 60%, indicating that Drugs B, C, D, and E did not exhibit a significant growth-inhibitory effect on murine 4T1 mammary carcinoma.

Example 29

I. Objective of the Study:

The primary objective was to establish a 4T1 tumor model and verify the antitumor efficacy of the drug(s).

II. Cell Information

4T1, murine mammary carcinoma cell line.

III. Experimental Animal Information

Species: BALB/c mice

Age: 6 weeks

Sex: female

Supplier: SPF (Beijing) Biotechnology Co., Ltd.

IV. Experimental Methods

1. Cell culture

Cells were thawed and seeded into the corresponding culture medium (RPMI-1640 basal medium + 10% FBS + 1% P/S).

Same as in Example 21.

2. Inoculation and grouping

Inoculation site: orthotopic mammary fat pad.

Inoculum: $1 \times 10^6$ cells per mouse; 46 mice inoculated.

Inoculation volume: 0.1 Ml.

Inoculation method: cell inoculation.

4T1 cells were thawed and expanded to an appropriate number; the cell concentration was adjusted with sterile PBS to $1 \times 10^7$ cells/mL and kept on ice. Using a disposable sterile 1 mL syringe, 0.1 mL of the cell suspension was aspirated and inoculated beneath the mammary fat pad of each mouse; the day of inoculation was designated D0.

Grouping

On D7 after inoculation (tumor volume approximately 100 mm$^3$), animals were randomized by tumor volume into two groups: Group C (negative control) and Group D (treatment group). On the day of grouping, all groups (C-D) received tail-vein injections; thereafter, dosing was performed once every other day according to the dosing regimen in Table 104. The first dosing day was designated D7. Details of inoculation, grouping, and dosing are provided in Tables 103 and 104.

Table 103. Tumor cell inoculation scheme

| Cell line | Host animal (Species) | No. of animals (n) | Inoculation site | Mode of inoculation | Inoculum (cells/mouse) |
|---|---|---|---|---|---|
| 4T1 | BALB/c | 46 | Orthotopic mammary fat pad | Cell-suspension inoculation | $1\times10^6$ |
| **Note:** The day of inoculation was designated as **D0**. | | | | | |

Table 104. Animal selection, grouping and dosing regimen

| Group | Animal information | | | | Dosing information | | | | |
|---|---|---|---|---|---|---|---|---|---|
| | Species | Sex | Age | No. of animals | Subgroup | Test article | Route | Dose volume | Frequency |
| C | Balb/c | ♀ | 6 w | 8 | Negative control | C | i.v. | 200 μL | Qod |
| D | Balb/c | ♀ | 6 w | 8 | Treatment group | D | i.v. | 200 μL | Qod |
| i.v.: intravenous injection via the tail vein; Qod: once every other day. | | | | | | | | | |

Wherein, C is phosphate-buffered saline (PBS); D includes 2*CpG2006-CD40 aptamer-DNA carrier (50 $\mu$g) + 2*AmiR-21-3*Bio-DNA carrier (6 $\mu$g) + OX40 (DNA) (6 $\mu$g). Accordingly, Drug D corresponds to Combination 28 (Drug 4 + Drug 19 + a CD40 aptamer in DNA form).

3. Measurement indices

Same as in Example 16.

4. Statistical analysis

Same as in Example 16.

V. Experimental Results:

1. Clinical observations in experimental animals

During the dosing period, the animals exhibited emaciation, hypoactivity, and kyphosis; with continued dosing, mortality was observed.

2. Changes in body weight of experimental animals

In Group C (control), body weight showed a certain degree of decrease during D7-D24, followed by an upward trend during D24-D42. In Group D (treatment), body weight showed a certain degree of decrease during D7-D15, followed by an upward trend during D15-D42. Body-weight summary results are recorded in Table 105, and the body-weight growth curves are shown in Figures 29-1 and 29-2.

Table 105. Summary of animal body weight

| Group | No. of animals | | Body weight (g) | | | | Change in body weight (g) |
|---|---|---|---|---|---|---|---|
| | Start | End | D7 | D15 | D24 | D42 | |
| C | 8 | 0 | 19.4 $\pm$ 0.77 | 19.3 $\pm$ 0.77 | 18.2 $\pm$ 0.68 | 19.5 $\pm$ 0.36 | +0.1 |
| D | 8 | 0 | 19.0 $\pm$ 0.69 | 17.9 $\pm$ 1.27 | 19.4 $\pm$ 1.19 | 21.6 $\pm$ 1.06 | +2.6 |
| *: P<0.05, **: P<0.01, ***: P<0.001, compared with the negative control. Note: the day of dosing is recorded as D7. | | | | | | | |

3. Results for survival prolongation

Throughout the study, mouse deaths were recorded; animals were observed every other day, results were documented, and the survival prolongation rate (ILS%) was calculated.

Tumors in the negative control group grew well. Compared with Group C (negative control), Group D showed a survival prolongation rate of 132.18% ($\geq$125%), indicating a significant inhibitory effect on tumor growth. The results are recorded in Tables 106 and 107, and the survival days are shown in Figure 29-3.

Table 106. Summary of mouse survival days

| Group C | | | Group D | | |
|---|---|---|---|---|---|
| Animal ID | Date of death (YYYY/MM/DD) | Survival time (days) | Animal ID | Date of death (YYYY/MM/DD) | Survival time (days) |
| 31 | 2020/07/11 | 25 | 6 | 2020/08/04 | 49 |
| 39 | 2020/07/28 | 42 | 25 | 2020/07/19 | 33 |
| 14 | 2020/07/19 | 33 | 26 | 2020/07/29 | 43 |
| 46 | 2020/07/16 | 30 | 43 | 2020/07/31 | 45 |
| 2 | 2020/07/15 | 29 | 17 | 2020/07/29 | 43 |
| 40 | 2020/07/22 | 36 | 44 | 2020/07/24 | 38 |

(continued)

| Group C | | | Group D | | |
|---|---|---|---|---|---|
| Animal ID | Date of death (YYYY/MM/DD) | Survival time (days) | Animal ID | Date of death (YYYY/MM/DD) | Survival time (days) |
| 35 | 2020/07/24 | 38 | 3 | 2020/08/03 | 48 |

Table 107. Life-span extension

| Group | Median survival time (MST, days) | Life-span extension (T/C, %) |
|---|---|---|
| C | 32.875 | - |
| D | 43.125 | 131.18 |

VI. Experimental Conclusions

This study evaluated the inhibitory effect of Drug D on tumor growth in the 4T1 murine mammary carcinoma model. The results show that tail-vein administration of Drug D produced a marked inhibitory effect on the growth of 4T1 mammary carcinoma tumors in mice.

Example 30

Objective of the study, principal instruments, cell information, and experimental-animal information: same as in Example 21.

I. Experimental Protocol:

The animal inoculation and dosing regimens are shown in Tables 108 and 109.

Table 108. Animal inoculation and dosing scheme.

| Cell line | No. of mice (n) | Mouse IDs | Inoculation site | Inoculum (cells per mouse) |
|---|---|---|---|---|
| CT26 | 42 | See grouping table | Subcutaneous, left axillary dorsal region | $1 \times 10^6$ |
| **Note:** The day of inoculation was designated as **D0**. | | | | |

Table 109. Animal grouping and dosing

| Group | No. of animals (n) | Treatment | Route of administration | Dose volume per administration | No. of administrations | Dosing days |
|---|---|---|---|---|---|---|
| A | 8 | A | Tail-vein injection (i.v.) | 200 μL/25 g | 4 | D1 (day of randomization), D3, D5, D7 |
| B | 8 | B | Tail-vein injection (i.v.) | 200 μL/25 g | 4 | |
| C | 8 | C | Tail-vein injection (i.v.) | 200 μL/25 g | 4 | |
| D | 8 | D | Tail-vein injection (i.v.) | 200 μL/25 g | 4 | |
| E | 8 | E | Tail-vein injection (i.v.) | 200 μL/25 g | 4 | |

Wherein, in the "Treatment" column of Table 109: Drug A is PBS; Drug B includes 2 *CpG1826-CD40 aptamer-DNA carrier (40 μg) + 3*AmiR-21-AS1411 aptamer-CD40 aptamer-2*Bio-DNA carrier (12 μg); Drug C includes 4*CpG2006-CD40 aptamer-DNA carrier (25 μg) + 3*AmiR-21-AS1411 aptamer-CD40 aptamer-2*Bio-DNA carrier (12 μg); Drug D includes 4*CpG2006-CD40 aptamer-DNA carrier (50 μg) + 3*AmiR-21-AS1411 aptamer-CD40 aptamer-2*Bio-DNA carrier (12 μg); and Drug E includes 4 *CpG2006-CD40 aptamer-DNA carrier (100 μg) + 3*AmiR-21-AS1411 aptamer-CD40 aptamer-2*Bio-DNA carrier (12 μg). Accordingly, Drug B corresponds to Combination 11 (Drug 3 + Drug 41), and Drugs C, D, and E correspond to Combination 12 (Drug 5 + Drug 41).

II. Experimental Methods

1. Inoculation and grouping

For the murine colon carcinoma CT26 model, male BALB/c mice 5-7 weeks of age were used. At the time of the experiment, well-growing tumor tissue was harvested, minced and ground, and diluted with sterile normal saline to prepare a tumor-cell suspension. CT26 cells ($1 \times 10^6$ cells/0.2 mL) were inoculated subcutaneously into the left axillary-dorsal region of each mouse; the day of inoculation was designated D0. On D7, animals were randomized by tumor volume into five groups-A (vehicle control), B, C, D, and E-with 8 animals per group. On the day of grouping, all groups began tail-vein intravenous dosing, for a total of four administrations. Details of inoculation, grouping, and dosing are provided in Tables 108 and 109.

2. Measurement indices

Same as in Example 16.

3. Statistical analysis

Same as in Example 16.

III. Experimental Results:

1. Clinical observations in experimental animals

During the dosing period, all animals exhibited good general condition (activity, food intake, etc.). One animal in Group B and two animals in Group E died in the afternoon of D13.

2. Changes in body weight of experimental animals

During the dosing period, body weight increased to some extent in all animals; however, the body-weight gain in Groups B and E was lower than that in Group A (vehicle control). The results are recorded in Table 110, and the body-weight growth curves are shown in Figure 30-1.

Table 110. Summary of animal body weight

| Group | No. of animals | | | Body weight (g) | | | Change in body weight (g) |
|---|---|---|---|---|---|---|---|
| | D0 | D7 | D15 | D0 | D7 | D15 | |
| A | 8 | 8 | 8 | 23.3 ± 1.1 | 24.8 ± 0.8 | 26.9 ± 1.1 | +3.6 |
| B | 8 | 8 | 7 | 23.0 ± 0.8 | 25.1 ± 1.1 | 24.4 ± 1.3** | +1.4 |
| C | 8 | 8 | 8 | 23.2 ± 0.4 | 24.5 ± 0.9 | 26.3 ± 0.8 | +3.2 |
| D | 8 | 8 | 8 | 23.3 ± 0.7 | 24.7 ± 1.3 | 27.3 ± 2.2 | +3.9 |
| E | 8 | 8 | 6 | 23.2 ± 0.4 | 24.8 ± 1.0 | 24.3 ± 1.4** | +1.1 |

Note: D0 indicates the day of inoculation with the tumor cell suspension; D7 and D15 indicate 7 days and 15 days, respectively, after inoculation with the tumor cell suspension.
**: $P<0.01$, compared with Group A (vehicle control).
Note: D0 = day of inoculation with the tumor-cell suspension; D7 = 7 days after inoculation with the tumor-cell suspension (i.e., the day of grouping/dosing); D15 = 15 days after inoculation with the tumor-cell suspension.

3. Tumor volume-inhibition results

All animals were closely monitored for tumor growth during the study; tumors were measured twice per week, results were recorded, and the tumor growth inhibition (TGI) was calculated.
Compared with Group A (vehicle control), on D15 the tumor growth-inhibition rates (based on tumor volume) for Groups B, C, D, and E were 73.31%, 46.14%, 28.09%, and 37.26%, respectively (Group B > 60%; Groups C, D, E < 60%). The T/C values (based on RTV) were 27.98%, 53.35%, 73.55%, and 65.19%, respectively (Group B < 40%; Groups C, D, E > 40%).

Table 111. Summary of tumor growth data

| Days | A | | B | | |
|---|---|---|---|---|---|
| | Tumor volume (mm$^3$) | N | Tumor volume (mm$^3$) | N | Tumor growth inhibition rate (%) |
| D7 | 154.3±26.8 | 8 | 154.4±29.2 | 8 | / |
| D11 | 1283.5±195.4 | 8 | 475.3±178.4*** | 8 | 62.97 |
| D13 | 1833.0±335.5 | 8 | 556.7±257.0*** | 8 | 69.63 |
| D15 | 2465.8±489.4 | 8 | 658.1±429.5*** | 7 | 73.31 |

| Days | C | | Tumor growth inhibi-tion rate (%) | D | | Tumor growth inhibi-tion rate (%) | E | | Tumor growth inhibition rate (%) |
|---|---|---|---|---|---|---|---|---|---|
| | Tumor volume (mm$^3$) | N | | Tumor volume (mm$^3$) | N | | Tumor volume (mm$^3$) | N | |
| D7 | 156.6±27.7 | 8 | / | 154.3±37.1 | 8 | / | 154.2±40.1 | 8 | / |
| D11 | 797.3±243.6*** | 8 | 37.88 | 907.7±293.6** | 8 | 29.28 | 955.6±211.0** | 8 | 25.55 |
| D13 | 1030.7±448.9** | 8 | 43.77 | 1219.9 ±484.0* | 8 | 33.45 | 1061.0 ±387.8*** | 8 | 42.12 |
| D15 | 1328.1 ±353.0*** | 8 | 46.14 | 1773.3 ±708.2* | 8 | 28.09 | 1547.0±495.6** | 6 | 37.26 |

*: P<0.05, **: P<0.01, ***: P<0.001, compared with group A (solvent control).
Note: Days: number of days after inoculation of the tumor cell suspension; N: number of animals in the group included in the statistical analysis.

Table 112. Summary of tumor growth data

| Days | A | | B | | |
|---|---|---|---|---|---|
| | RTV | N | RTV | N | T/C (%) |
| D7 | 1.00+0 | 8 | 1.00+0 | 8 | / |
| D11 | 8.44±1.28 | 8 | 3.23±1.35*** | 8 | 38.22 |
| D13 | 12.44+4.40 | 8 | 3.72±1.63*** | 8 | 29.89 |
| D15 | 16.29±3.45 | 8 | 4.56+3.05*** | 7 | 27.98 |

| Days | C | | | D | | | E | | |
|---|---|---|---|---|---|---|---|---|---|
| | RTV | N | T/C (%) | RTV | N | T/C (%) | RTV | N | T/C (%) |
| D7 | 1.00+0 | 8 | / | 1.00+0 | 8 | / | 1.00+0 | 8 | / |
| D11 | 5.26±1.95** | 8 | 62.32 | 6.02±1.87** | 8 | 71.24 | 6.39±1.34** | 8 | 75.63 |
| D13 | 6.700±2.74** | 8 | 53.88 | 8.25±3.75 | 8 | 66.29 | 7.18±2.78* | 8 | 57.72 |
| D15 | 8.69±2.49*** | 8 | 53.35 | 11.98+5.60 | 8 | 73.55 | 10.62±4.49* | 6 | 65.19 |

*: P<0.05, **: P<0.01, ***: P<0.001, compared with group A (solvent control).
Note: Days: number of days after tumor cell inoculation; N: number of animals in the group included in the statistical analysis.

4. Tumor weight and gross observation results

At 15 days after CT26 inoculation, all animals were euthanized by cervical dislocation; tumors were excised, weighed, and photographed. The results are summarized in Table 113 and Figure 30-4.

In Group A (vehicle control), tumors grew well; gross examination after excision showed little necrotic tissue and relatively abundant blood within the tumor. In Group B, obvious intratumoral necrosis was observed with relatively little blood. Based on tumor weight, the growth-inhibition rates for Drugs B, C, D, and E against CT26 were 69.34%, 35.12%, 34.30%, and 36.56%, respectively; i.e., Group B > 60%, while Groups C, D, and E < 60%.

Table 113. Summary of tumor weights

| Group | No. of animals (n) | Tumor weight (g) | IR_TW (%) |
|-------|--------------------|--------------------|-----------|
| A | 8 | 4.13 $\pm$ 0.60 | \ |
| B | 7 | 1.27 $\pm$ 1.01*** | 69.34 |
| C | 8 | 2.68 $\pm$ 0.62*** | 35.12 |
| D | 8 | 2.72 $\pm$ 0.88** | 34.30 |
| E | 6 | 2.62 $\pm$ 0.74** | 36.56 |
| **: $P < 0.01$, ***: $P < 0.001$, compared with Group A (vehicle control). | | | |

IV. Experimental Conclusions:

Taking into comprehensive consideration the tumor growth-inhibition rate and the tumor-weight inhibition rate, the conclusions are as follows:

Following tail-vein administration of Drugs A (vehicle control), B, C, D, and E, at the end of the study (D15) the tumor-inhibition rate for Drug B (based on both tumor weight and tumor volume) exceeded 60%, suggesting that Drug B exerts a marked inhibitory effect on the growth of CT26 murine colon carcinoma. The tumor-inhibition rates for Drugs C, D, and E (based on tumor weight and tumor volume) did not exceed 60%, suggesting that Drugs C, D, and E do not exhibit a marked growth-inhibitory effect on CT26 murine colon carcinoma.

Example 31

Objective of the study, principal instruments, cell information, and experimental-animal information: same as Example 21.

I. Experimental protocol:

The animal inoculation and dosing regimens are shown in Tables 114 and 115.

Table 114. Animal inoculation and dosing scheme

| Cell line | No. of mice | Mouse ID Nos. | Inoculation site | Cell inoculum |
|-----------|-------------|---------------|------------------|---------------|
| CT26 | 26 | See grouping table | Subcutaneous, left axillary-dorsal region | $1 \times 10^6$ cells/mouse |
| *Note:* The day of inoculation was designated D0. | | | | |

Table 115. Animal grouping and dosing

| Group | No. of animals | Treatment | Route of administration | Dose volume per administration | No. of administrations | Dosing schedule |
|-------|----------------|-----------|-------------------------|--------------------------------|------------------------|-----------------|
| A | 8 | A | Intratumoral injection | 50 $\mu$l/20 g | 4 times | On the day of grouping (Day 1) and on Days 3, 5 and 7 |
| B | 8 | B | Intratumoral injection | 50 $\mu$l/20 g | 4 times | |

Wherein, in the "Treatment" column of Table 115, Drug A is PBS; Drug B includes 4 *CpG2006-CD40 aptamer-DNA carrier (50 µg) +AmiR-21-AS1411 aptamer-CD40 aptamer-2*Bio-DNA carrier (12 µg).

II. Experimental Methods

1. Inoculation and grouping

For the murine colon carcinoma CT26 model, male BALB/c mice 5-7 weeks of age were used. At the time of the experiment, well-growing tumor tissue was harvested, minced and ground, and diluted with sterile normal saline to prepare a tumor-cell suspension. CT26 cells ($1 \times 10^6$ cells/0.2 mL) were inoculated subcutaneously into the left axillary-dorsal region of each mouse; the day of inoculation was designated D0. On D7, animals were randomized by tumor volume into two groups: A (vehicle control) and B, with 8 animals per group. On the day of grouping, all groups began intratumoral injections, for a total of four administrations. Details of inoculation, grouping, and dosing are provided in Tables 114 and 115.

2. Measurement indices

Same as in Example 16.

3. Statistical analysis

Same as in Example 16.

III. Experimental Results:

1. Clinical observations in experimental animals

Same as in Example 16.

3. Statistical analysis

Same as in Example 16.

IV. Experimental Results:

1. Clinical observations in experimental animals

During the dosing period, all animals exhibited good general condition, including normal activity and food intake.

2. Changes in body weight of experimental animals

During the dosing period, body weight increased to a certain extent in all animals. The results are recorded in **Table 116,** and the body-weight growth curves are shown in **Figure 31-1.**

Table 116. Summary of animal body weight

| Group | No. of animals | | | Body weight (g) | | | Change in body weight (g) |
|---|---|---|---|---|---|---|---|
| | D0 | D7 | D15 | D0 | D7 | D15 | |
| A | 8 | 8 | 8 | 22.8±0.8 | 23.5±0.9 | 26.8±1.5 | +4.1 |
| B | 8 | 8 | 8 | 23.0±0.6 | 24.0±1.1 | 26.9±1.3 | +3.9 |

Note: D0, day of tumor-cell inoculation; D7, 7 days after tumor-cell inoculation (i.e., the day of grouping/dosing); D15, 15 days after tumor-cell inoculation.

3. Tumor volume-inhibition results

All animals were closely monitored for tumor growth during the study; tumors were measured twice per week, the results were recorded, and the tumor growth inhibition (TGI) was calculated. The results are summarized in Tables 117 and 118 and Figures 31-2 and 31-3.

Compared with Group A (vehicle control), the Group B tumor growth inhibition rate on D15 (based on tumor volume) was 20.44% (i.e., < 60%); the T/C value (based on RTV) was 80.82% (i.e., > 40%).

Table 117. Summary of tumor growth data

| Days | A | | B | | |
|---|---|---|---|---|---|
| | Tumor volume ($mm^3$) | N | Tumor volume ($mm^3$) | N | Tumor growth inhibition rate (%) |
| D7 | 155.8 $\pm$ 38.5 | 8 | 158.0 $\pm$ 35.8 | 8 | / |
| D11 | 930.2 $\pm$ 268.4 | 8 | 830.4 $\pm$ 240.0 | 8 | 10.73 |
| D13 | 1471.2 $\pm$ 407.3 | 8 | 1156.5 $\pm$ 343.4 | 8 | 21.39 |
| D15 | 2150.8 $\pm$ 656.5 | 8 | 1711.3 $\pm$ 584.8 | 8 | 20.44 |

Note: "Days" indicates the number of days after inoculation with the tumor cell suspension; "N" indicates the number of animals in the group included in the statistics.

Table 118. Summary of tumor growth data

| Days | A | | B | | |
|---|---|---|---|---|---|
| | RTV | N | RTV | N | T/C(%) |
| D7 | 1.00$\pm$0 | 8 | 1.00$\pm$0 | 8 | / |
| D11 | 6.14$\pm$1.63 | 8 | 5.62$\pm$2.38 | 8 | 91.52 |
| D13 | 9.79$\pm$2.88 | 8 | 7.98$\pm$3.99 | 8 | 81.54 |
| D15 | 14.59$\pm$5.35 | 8 | 11.79$\pm$6.38 | 8 | 80.82 |

Note: Days: number of days after tumor-cell inoculation; N: number of animals in the group included in the statistical analysis.

4. Tumor weight and gross observation results

At 15 days after CT26 inoculation, all animals were euthanized by cervical dislocation; tumors were excised, weighed, and photographed. The results are summarized in Table 119 and Figure 31-4.

In Group A (vehicle control), tumors grew well; upon gross examination after excision, necrotic tissue was minimal and intratumoral blood was abundant. Based on tumor weight, the growth-inhibition rate of Drug B against CT26 was 20.04%, i.e., <60%.

Table 119. Summary of tumor weights

| Group | No. of animals | Tumor weight (g) | $IR_{TW}$% |
|---|---|---|---|
| A | 8 | 3.51$\pm$0.82 | / |
| B | 8 | 2.80$\pm$0.63 | 20.04 |

V. Experimental Conclusions:

Taking into account both the tumor growth-inhibition rate and the tumor-weight inhibition rate, the conclusions are as follows:

After intratumoral injection of A (vehicle control) and Drug B, at the end of the study (D15) the tumor-inhibition rate of Drug B (calculated on the basis of tumor weight and tumor volume) did not exceed 60%, indicating that Drug B shows no marked growth-inhibitory effect on CT26 murine colon carcinoma.

Example 32

Objective of the study and principal instruments: same as in Example 16.

I. Experimental animal information

Species: C57BL/6JNifdc

Body weight: 16-19 g

Sex: male

Supplier: National Institutes for Food and Drug Control (NIFDC), China

II. Experimental protocol:

Animal inoculation and dosing scheme:

Table 120. Animal inoculation and dosing scheme.

| Cell line | No. of mice (n) | Mouse IDs | Inoculation site | Inoculum (cells per mouse) |
|---|---|---|---|---|
| B16F10 | 34 | See grouping table | Subcutaneous, left axillary dorsal region | $1 \times 10^6$ |
| Note: The day of inoculation was designated as D0. | | | | |

Table 121. Animal grouping and dosing

| Group | No. of animals (n) | Treatment | Route of administration | Dose volume per administration | No. of administrations | Dosing days |
|---|---|---|---|---|---|---|
| A | 8 | A | Tail-vein injection (i.v.) | 200 μL/20 g | 8 | D1,D3, D5,D7, D9,D11, D13,D15 |
| B | 8 | B | Tail-vein injection (i.v.) | 200 μL/20 g | 8 | |
| C | 8 | C | Tail-vein injection (i.v.) | 200 μL/20 g | 8 | |
| D | 8 | D | Tail-vein injection (i.v.) | 200 μL/20 g | 8 | |

Wherein, in the "Treatment" column of Table 121, Drug A is PBS; Drug B includes 2 *CpG1826-CD40 aptamer-DNA carrier (25 μg) + 2*AmiR-21-AS1411 aptamer-DNA carrier (6 μg) + OX40 (DNA) (6 μg); Drug C includes 2*CpG2006-CD40 aptamer-DNA carrier (25 μg) + 2*AmiR-21-AS 1411 aptamer-DNA carrier (6 μg) + OX40 (DNA) (6 μg); and Drug D includes 2*CpG2006-CD40 aptamer-DNA carrier (25 μg) + 2*AmiR-21-AS1411 aptamer-DNA carrier (6 μg). Accordingly, Drug B corresponds to Combination 5 (Drug 3 + Drug 21 + an OX40 aptamer in DNA form), Drug C corresponds to Combination 6 (Drug 4 + Drug 21 + an OX40 aptamer in DNA form), and Drug D corresponds to Combination 7 (Drug 4 + Drug 21).

III. Experimental Methods

1. Inoculation and grouping

For the murine melanoma B16F10 model, male C57BL/6JNifdc mice weighing 16-19 g were used. At the time of the experiment, well-growing tumor tissue was harvested, minced and ground, and diluted with sterile normal saline to prepare a tumor-cell suspension. B16F10 cells ($1 \times 10^6$ cells/0.2 mL) were inoculated subcutaneously into the left axillary-dorsal region of each mouse; the day of inoculation was designated D0. On D0, animals were randomized by tumor volume into four groups: A (vehicle control), B, C, and D, with 8 animals per group. On D1, all groups began tail-vein intravenous dosing, for a total of 8 administrations. Details of inoculation, grouping, and dosing are shown in Tables 120 and 121.

2. Measurement indices

Same as in Example 16.

3. Statistical analysis

Same as in Example 16.

III. Experimental Results:

1. Clinical observations in experimental animals

During the dosing period, all animals exhibited good general condition, including normal activity and food intake.

2. Changes in body weight of experimental animals

During the dosing period, body weight increased to a certain extent in all animals. The results are recorded in Table 122, and the body-weight growth curves are shown in Figure 32-1.

Table 122. Summary of animal body weight

| Group | No. of animals | | Body weight (g) | | Change in body weight (g) |
|---|---|---|---|---|---|
| | D0 | D17 | D0 | D17 | |
| A | 8 | 8 | 17.8 ± 0.9 | 24.7 ± 1.7 | +6.9 |
| B | 8 | 8 | 17.7 ± 1.1 | 21.2 ± 1.4** | +3.5 |
| C | 8 | 8 | 17.5 ± 1.1 | 22.5 ± 1.1* | +5.0 |
| D | 8 | 8 | 17.7 ± 1.0 | 21.9 ± 1.6* | +4.3 |

Note: D0 indicates the day of inoculation with the tumor cell suspension; D17 indicates 17 days after inoculation with the tumor cell suspension.
*: P<0.05, **: P<0.01, ***: P<0.001, compared with Group A (vehicle control).

3. Tumor volume-inhibition results

All animals were closely monitored for tumor growth during the study; tumors were measured twice per week, the results were recorded, and the tumor growth inhibition (TGI) was calculated.

Compared with Group A (vehicle control), on D17 the tumor growth-inhibition rates (based on tumor volume) for Groups B, C, and D were 74.62%, 65.36%, and 67.92%, respectively-i.e., greater than 60%.

Table 123. Summary of tumor growth data

| Days | A | | B | | |
|---|---|---|---|---|---|
| | Tumor volume (mm$^3$) | N | Tumor volume (mm$^3$) | N | Tumor growth inhibition rate (%) |
| D7 | 241.7±43.5 | 8 | 179.2±52.3* | 8 | 25.9 |
| D10 | 353.1±88.9 | 8 | 255.3±106.0 | 8 | 27.68 |
| D14 | 1167.4±401.7 | 8 | 297.9±124.6*** | 8 | 74.48 |
| D17 | 2190.7±608.5 | 8 | 556.1±213.7*** | 8 | 74.62 |

| Days | C | | D | | |
|---|---|---|---|---|---|
| | Tumor volume (mm$^3$) | N | Tumor volume (mm$^3$) | N | Tumor growth inhibition rate (%) |
| D7 | 194.5±48.7 | 8 | 143.5±36.4 | 8 | 40.6 |
| D10 | 156.3±40.1*** | 8 | 121.6±47.8*** | 8 | 65.55 |

(continued)

| Days | C | | D | | |
|---|---|---|---|---|---|
| | Tumor volume (mm$^3$) | N | Tumor volume (mm$^3$) | N | Tumor growth inhibition rate (%) |
| D14 | 333.9±128.2*** | 8 | 323.7±139.0*** | 8 | 72.27 |
| D17 | 758.9±468.3*** | 8 | 702.8±372.1*** | 8 | 67.92 |
| Note: Days: days after inoculation of the tumor cell suspension; N: number of animals in the group included in the statistical analysis. *: P<0.05, ***: P<0.001, compared with group A (vehicle control). | | | | | |

4. Tumor weight and gross observation results

At 17 days after B16F10 inoculation, all animals were euthanized by cervical dislocation; tumors were excised, weighed, and photographed. The results are summarized in Table 124 and Figure 32-3.
Based on tumor weight, the growth-inhibition rates of Drugs B, C, and D against B16F10 were 70.98%, 64.67%, and 66.95%, respectively-i.e., all >60%.

Table 124. Summary of tumor weights

| Group | No. of animals (n) | Tumor weight (g) | IR_TW (%) |
|---|---|---|---|
| A | 8 | 3.13 ± 1.15 | \ |
| B | 8 | 0.91 ± 0.39*** | 70.98 |
| C | 8 | 1.11 ± 0.68*** | 64.67 |
| D | 8 | 1.03 ± 0.53*** | 66.95 |
| ***: P < 0.001, compared with Group A (vehicle control). | | | |

5. Results for splenic index and thymic index

At 17 days after B16F10 inoculation, after orbital blood collection, all animals were euthanized by cervical dislocation; the spleen and thymus were excised and weighed. The results are summarized in Table 125.
In Group A (vehicle control), the splenic index of tumor-bearing mice was 91.13. In Groups B, C, and D, the splenic indices were 124.48, 115.96, and 110.07, respectively-slightly higher than in Group A; however, the differences were not statistically significant. The thymic indices of tumor-bearing mice in Groups B, C, and D were 15.43, 19.21, and 21.29, respectively, which were higher than the vehicle control (13.68); the increases in Groups C and D were statistically significant.

Table 125. Summary of splenic and thymic indices

| Group | No. of animals | Spleen index | Thymus index |
|---|---|---|---|
| A | 8 | 91.13 ± 27.65 | 13.68 ± 3.79 |
| B | 8 | 124.48 ± 28.42 | 15.43 ± 2.35 |
| C | 8 | 115.96 ± 12.71 | 19.21 ± 2.96** |
| D | 8 | 110.07 ± 16.72 | 21.29 ± 5.08** |
| **: P < 0.01, compared with Group A (vehicle control). | | | |

IV. Experimental Conclusions:

Taking into comprehensive consideration the tumor growth-inhibition rate and the tumor-weight inhibition rate, the conclusions are as follows:
Following tail-vein administration of Drugs B, C, and D, at the end of the study (D17) the tumor inhibition rates of Drugs B, C, and D (calculated on the basis of tumor weight and tumor volume) each exceeded 60%, indicating that Drugs B, C, and D exert a marked growth-inhibitory effect on B16F10 murine melanoma. Following tail-vein administration of Drugs C and D, at the end of the study (D17) the thymic index of tumor-bearing mice was significantly increased; however, Drugs B, C, and D had no significant effect on the splenic index of tumor-bearing mice.

Example 33

Objective of the study and principal instruments: same as in Example 16.

I. Experimental animal information

Species: C57BL/6JNifdc

Body weight: 16-19 g

Sex: male

Supplier: National Institutes for Food and Drug Control (Daxing), China

II. Experimental protocol:

Animal inoculation and dosing scheme:

Table 126. Animal inoculation and dosing scheme.

| Cell line | No. of mice (n) | Mouse IDs | Inoculation site |
|---|---|---|---|
| B16F10 | 32 | See grouping table | Subcutaneous, left axillary dorsal region |
| Note: The day of inoculation was designated as D0. | | | |

Table 127. Animal grouping and dosing

| Group | No. of animals (n) | Treatment | Route of administration | Dose volume per administration | No. of administrations | Dosing schedule (days) |
|---|---|---|---|---|---|---|
| A | 8 | B16F10 (5) A20200923 | Tail-vein injection (i.v.) | 200 μL/20 g | 7 | |
| B | 8 | B16F10 (5) B20200923 | Tail-vein injection (i.v.) | 200 μL/20 g | 7 | |
| C | 8 | B16F10 (5) C20200923 | Tail-vein injection (i.v.) | 200 μL/20 g | 7 | D1, D3, D5, D7, D9, D11, D13 |
| D | 8 | B16F10 (5) D20200923 | Tail-vein injection (i.v.) | 200 μL/20 g | 7 | |
| E | 8 | B16F10 (5) E20200923 | Tail-vein injection (i.v.) | 200 μL/20 g | 7 | |
| F | 8 | B16F10 (5) F20200923 | Tail-vein injection (i.v.) | 200 μL/20 g | 7 | |
| G | 8 | B16F10 (5) G20200923 | Tail-vein injection (i.v.) | 200 μL/20 g | 7 | |

Wherein, in the "Treatment" column of Table 127, Drug A is PBS; Drug B includes 2 *CpG1826-CD40 aptamer-DNA carrier (25 μg) + 2*AmiR-21-AS1411 aptamer-Bio-DNA carrier (6 μg); Drug C includes 2*CpG2006-CD40 aptamer-DNA carrier (25 μg) + 2 *AmiR-21-AS1411 aptamer-Bio-DNA carrier (6 μg); Drug D includes 2*CpG2006-CD40 aptamer-DNA carrier (25 µg) + 2*AmiR-21-AS1411 aptamer-Bio-DNA carrier (10 µg); Drug E includes 2*CpG2006-CD40 aptamer-DNA carrier (20 µg) + 2*AmiR-21-AS 1411 aptamer-Bio-DNA carrier (15 µg); Drug F includes 2*CpG2006-CD40 aptamer-DNA carrier (25 µg); and Drug G includes 2*AmiR-21-AS1411 aptamer-Bio-DNA carrier (8 µg). Accordingly, Drug B corresponds to Combination 8 (Drug 3 + Drug 23), and Drugs C, D, E, and F each correspond to Combination 9 (Drug 4 + Drug 23).

### III. Experimental Methods

#### 1. Inoculation and grouping

For the murine melanoma B16F10 model, male C57BL/6JNifdc mice weighing 16-19 g were used. At the time of the experiment, well-growing tumor tissue was harvested, minced and ground, and diluted with sterile normal saline to prepare a tumor-cell suspension. The B16F10 melanoma cell suspension was inoculated subcutaneously into the left axillary-dorsal region of each mouse (0.2 mL/mouse); the day of inoculation was designated D0. On D0, animals were randomized into seven groups: A (vehicle control), B, C, D, E, F, and G, with 8 animals per group. On D1, all groups began tail-vein injections, for a total of seven administrations. Details of inoculation, grouping, and dosing are provided in Tables 126 and 127.

#### 2. Measurement indices

Same as in Example 4.

#### 3. Statistical analysis

Same as in Example 16.

### IV. Experimental Results:

#### 1. Clinical observations in experimental animals

During the dosing period, all animals exhibited good general condition, including normal activity and food intake.

#### 2. Changes in body weight of experimental animals

During the dosing period, body weight increased to some extent in all animals. The body-weight gain in Groups B, D, E, and F was lower than that in Group A (vehicle control), but the differences were not statistically significant.
The results are recorded in Table 128, and the body-weight growth curves are shown in Figure 33-1.

**Table 128. Summary of animal body weight**

| Group | No. of animals | | Body weight (g) | | Change in body weight (g) |
|---|---|---|---|---|---|
| | D0 | D15 | D0 | D15 | |
| A | 8 | 8 | 17.8 ± 0.9 | 23.8 ± 2.3 | +6.0 |
| B | 8 | 8 | 17.7 ± 1.0 | 22.4 ± 1.2 | +4.7 |
| C | 8 | 8 | 17.6 ± 1.1 | 23.6 ± 2.6 | +6.0 |
| D | 8 | 8 | 18.5 ± 0.8 | 23.4 ± 1.1 | +5.0 |
| E | 8 | 8 | 18.0 ± 0.5 | 22.8 ± 1.2 | +4.8 |
| F | 8 | 8 | 18.2 ± 0.9 | 24.0 ± 1.4 | +5.8 |
| G | 8 | 8 | 17.4 ± 0.8 | 24.2 ± 2.7 | +6.8 |
| Note: D0 indicates the day of inoculation with the tumor cell suspension; D15 indicates 15 days after inoculation with the tumor cell suspension. | | | | | |

3. Tumor volume-inhibition results

All animals were closely monitored for tumor growth during the study; tumors were measured twice per week, the results were recorded, and the tumor growth inhibition (TGI) was calculated. The results are summarized in Table 129 and Figure 33-2.

In Group A (vehicle control) tumors grew well. Compared with Group A, the D15 TGI values (calculated on the basis of tumor volume) for Groups B, C, D, E, F, and G were 52.69%, 54.60%, 76.41%, 67.07%, 67.76%, and -1.73%, respectively; thus, Groups D, E, and F > 60%, while Groups B, C, and G < 60%.

Table 129. Summary of tumor growth data

| Days | A | | B | | | C | | |
|---|---|---|---|---|---|---|---|---|
| | Tumor volume (mm$^3$) | N | Tumor volume (mm$^3$) | N | Tumor growth inhibition rate (%) | Tumor volume (mm$^3$) | N | Tumor growth inhibition rate (%) |
| D7 | 160.3±35.4 | 8 | 163.9±39.6 | 8 | -2.30 | 121.0±31.9* | 8 | 24.50 |
| D10 | 801.4±165.5 | 8 | 382.9±194.8*** | 8 | 52.22 | 327.4±89.1*** | 8 | 59.14 |
| D14 | 1564.4±492.8 | 8 | 797.9±466.6** | 8 | 49.00 | 740.9:1:263.1*** | 8 | 52.64 |
| D15 | 2077.0±548.3 | 8 | 982.6±674.0** | 8 | 52.69 | 943.0±419.0*** | 8 | 54.60 |

| Days | D | | Tumor growth inhibition rate (%) | E | | Tumor growth inhibition rate (%) |
|---|---|---|---|---|---|---|
| | Tumor volume (mm$^3$) | N | | Tumor volume (mm$^3$) | N | |
| D7 | 148.0±32.6 | 8 | 7.70 | 113.5±16.7** | 8 | 29.20 |
| D10 | 308.4±50.3*** | 8 | 61.52 | 278.1±107.6*** | 8 | 65.29 |
| D14 | 484.6±97.4*** | 8 | 69.02 | 448.8±210.2*** | 8 | 71.31 |
| D15 | 489.9±157.5*** | 8 | 76.41 | 684.0±427.2*** | 8 | 67.07 |

| Days | F | | | G | | |
|---|---|---|---|---|---|---|
| | Tumor volume (mm$^3$) | N | Tumor growth inhibition rate (%) | Tumor volume (mm$^3$) | N | Tumor growth inhibition rate (%) |
| D7 | 116.6±47.2 | 8 | 27.30 | 113.5±21.9 | 8 | 29.20 |
| D10 | 268.1±76.3*** | 8 | 66.54 | 751.7±170.3 | 8 | 6.21 |
| D14 | 643.0±238.5*** | 8 | 58.90 | 1392.0±349.2 | 8 | 11.02 |
| D15 | 669.6±337.4*** | 8 | 67.76 | 2113.0±736.5 | 8 | -1.73 |

*Note:* Days: days after inoculation of the tumor cell suspension; N: number of animals in the group included in the statistical analysis.
*: P<0.05, **: P<0.01, ***: P<0.001, compared with group A (vehicle control).

4. Tumor weight

At 15 days after B16F10 inoculation, all animals were euthanized by cervical dislocation; tumors were excised, weighed, and photographed. The results are summarized in Table 130 and Figure 33-3.

Based on tumor weight, the growth-inhibition rates of Drugs B, C, D, E, F, and G against B16F10 were 49.71%, 48.05%, 65.84%, 58.09%, 51.85%, and -7.36%, respectively; i.e., Drug D > 60%, while Drugs B, C, E, F, and G < 60%.

Table 130. Summary of tumor weights

| Group | No. of animals (n) | Tumor weight (g) | IR_TW (%) |
|---|---|---|---|
| A | 8 | 2.57 ± 0.67 | \ |
| B | 8 | 1.29 ± 0.67** | 49.71 |
| C | 8 | 1.33 ± 0.29*** | 48.05 |
| D | 8 | 0.88 ± 0.18*** | 65.84 |
| E | 8 | 1.08 ± 0.36*** | 58.09 |
| F | 8 | 1.24 ± 0.46** | 51.85 |
| G | 8 | 2.75 ± 0.99 | -7.36 |
| **: P < 0.01, ***: P < 0.001, comparisons vs Group A (vehicle control). | | | |

5. Results for splenic index and thymic index

At 15 days after B16F10 inoculation, after orbital blood collection, all animals were euthanized by cervical dislocation; the spleen and thymus were excised and weighed. The results are summarized in Table 131.

The mean splenic index of tumor-bearing mice in the A (vehicle control) group was 69.93. The mean splenic indices in the B, C, **D,** E, F, and G groups were 109.70, 115.90, 129.21, 112.26, 93.10, and 74.44, respectively-higher than that of the A group; except for Group G, all differences were statistically significant. The mean thymic indices in the B, C, D, E, F, and G groups were 19.49, 21.41, 21.48, 24.22, 21.61, and 23.45, respectively. Except for Group E, which was slightly higher than the A (vehicle control) group (24.10), the other groups were lower than the A group; none of the differences were statistically significant.

Table 131. Summary of splenic and thymic indices

| Group | No. of animals | Spleen index | Thymus index |
|---|---|---|---|
| A | 8 | 69.93 ± 5.07 | 24.10 ± 4.58 |
| B | 8 | 109.70 ± 19.15*** | 19.49 ± 7.97 |
| C | 8 | 115.90 ± 17.39*** | 21.41 ± 5.28 |
| D | 8 | 129.21 ± 22.15*** | 21.48 ± 4.97 |
| E | 8 | 112.26 ± 19.45*** | 24.22 ± 6.03 |
| F | 8 | 93.10 ± 16.20** | 21.61 ± 6.14 |
| G | 8 | 74.44 ± 10.12 | 23.45 ± 5.36 |
| **: P < 0.01,***: P < 0.001; comparisons are vs. Group A (vehicle control). | | | |

IV. Experimental Conclusions:

Taking into comprehensive consideration the tumor growth-inhibition rate and the tumor-weight inhibition rate, the conclusions are as follows:
Following tail-vein administration of Drugs B, C, D, E, F, and G, at the end of the study (D15) the tumor-inhibition rate of Drug D (calculated on the basis of tumor weight and tumor volume) exceeded 60%, indicating that Drug D exerts a marked growth-inhibitory effect on B16F10 murine melanoma. Following tail-vein administration of Drugs B, C, D, E, and F, at D15 the splenic index of tumor-bearing mice was significantly increased; however, Drugs B, C, D, E, F, and G had no significant effect on the thymic index of tumor-bearing mice.

Example 34

I. Objective of the Study:

To verify the antitumor efficacy of nano-nucleic-acid carrier combinations delivering immunomodulatory and gene-regulatory drugs.

II. Cell Information

4T1, murine mammary carcinoma; supplied by the Institute of Materia Medica, Chinese Academy of Medical Sciences.

III. Experimental Animal Information

Species: BALB/cCrSlcNifde

Body weight: 17-19 g

Sex: female

Supplier: National Institutes for Food and Drug Control (Daxing), China

IV. Experimental Methods

1. Inoculation and grouping

For the murine mammary carcinoma 4T1 model, female BALB/cCrSlcNifdc mice weighing 17-19 g were used. At the time of the experiment, well-growing tumor tissue was harvested, minced and ground, and diluted with sterile normal saline to prepare a tumor-cell suspension. The 4T1 mammary carcinoma cell suspension was inoculated subcutaneously into the left axillary-dorsal region of each mouse (0.2 mL/mouse); the day of inoculation was designated D0. On D0, animals were randomized into four groups: A (vehicle control), B, C, and D, with 8 animals per group. On D1, all groups began tail-vein intravenous dosing, administered every other day for a total of 10 administrations. Details of inoculation, grouping, and dosing are provided in Tables 132A and 132B.

Table 132A. Animal inoculation and dosing scheme

| Cell line | No. of mice (n) | Mouse IDs | Inoculation site |
|---|---|---|---|
| 4T1 | 32 | See grouping table | Subcutaneous, left axillary dorsal region |
| Note: The day of inoculation was designated as D0. | | | |

Table 132B. Animal grouping and dosing regimen

| Group | No. of animals (n) | Route of administration | Dose volume per administration | No. of administrations | Dosing days |
|---|---|---|---|---|---|
| A | 8 | Tail-vein injection (i.v.) | 200 $\mu$L/20 g | 10 | D1,D3, D5,D7, D9, D11, D13,D15, D17, D19 |
| B | 8 | Tail-vein injection (i.v.) | 200 $\mu$L/20 g | 10 | |
| C | 8 | Tail-vein injection (i.v.) | 200 $\mu$L/20 g | 10 | |
| D | 8 | Tail-vein injection (i.v.) | 200 $\mu$L/20 g | 10 | |

Wherein, A is PBS; B includes 2*CpG2006-CD40 aptamer-DNA carrier (25 $\mu$g) + 2 *AmiR-21-AS1411 aptamer-Bio-DNA carrier (6 $\mu$g); C includes 2*CpG2006-CD40 aptamer-DNA carrier (25 $\mu$g) + 2*AmiR-21-AS1411 aptamer-Bio-DNA carrier (10 $\mu$g); and D includes 2*CpG2006-CD40 aptamer-DNA carrier (20 $\mu$g) + 2*AmiR-21-AS1411 aptamer-Bio-DNA carrier (15 $\mu$g). Accordingly, Drugs B, C, and D correspond to Combination 9 (Drug 4 + Drug 23).

3. Measurement indices

Same as in Example 16.

4. Statistical analysis

Same as in Example 16.

V Experimental Results:

1. Clinical observations in experimental animals

During the dosing period, all animals exhibited good general condition, including normal activity and food intake.

2. Changes in body weight of experimental animals

During the dosing period, body weight increased to some extent in all animals. The body-weight gain in Groups B, C, and D was higher than that in Group A (vehicle control), but the differences were not statistically significant. The results are recorded in Table 133, and the body-weight growth curves are shown in Figure 34-1.

Table 133

| Group | No. of animals | | Body weight (g) | | Change in body weight (g) |
|---|---|---|---|---|---|
| | D0 | D21 | D0 | D21 | |
| A | 8 | 8 | 17.4 ± 0.3 | 19.4 ± 1.2 | +2.0 |
| B | 8 | 8 | 17.3 ± 0.4 | 20.5 ± 1.1 | +3.1 |
| C | 8 | 8 | 17.1 ± 0.5 | 19.8 ± 1.7 | +2.7 |
| D | 8 | 8 | 17.5 ± 0.5 | 20.4 ± 0.8 | +3.0 |
| Note: D0 indicates the day of inoculation with the tumor cell suspension; D21 indicates 21 days after inoculation with the tumor cell suspension. | | | | | |

3. Tumor volume-inhibition results

All animals were closely monitored for tumor growth during the study; tumors were measured twice per week, results were recorded, and tumor growth inhibition (TGI) was calculated. The results are summarized in Table 134 and Figure 2.

Tumors in Group A (vehicle control) grew well. Compared with Group A, the D21 tumor growth-inhibition rates (based on tumor volume) for Groups B, C, and D were 67.80%, 76.83%, and 74.84%, respectively-all >60%.

Table 134. Summary of tumor growth data

| Days | A | | B | | |
|---|---|---|---|---|---|
| | Tumor volume (mm$^3$) | N | Tumor volume (mm$^3$) | N | Tumor growth inhibition rate (%) |
| D10 | 169.8±14.5 | 8 | 141.9±34.4 | 8 | 16.45 |
| D14 | 382.3±101.0 | 8 | 185.0±78.6*** | 8 | 51.62 |
| D17 | 537.4±141.8 | 8 | 251.6±159.3** | 8 | 53.18 |
| D21 | 1225.9±328.0 | 8 | 394.8±210.1*** | 8 | 67.80 |

| Days | C | | | D | | |
|---|---|---|---|---|---|---|
| | Tumor volume (mm$^3$) | N | Tumor growth inhibition rate (%) | Tumor volume (mm$^3$) | N | Tumor growth inhibition rate (%) |
| D10 | 104.9±20.2 | 8 | 38.23 | 104.3±13.3*** | 8 | 38.58 |
| D14 | 135.2±84.3*** | 8 | 64.65 | 210.6±75.0** | 8 | 44.91 |
| D17 | 220.2±73.7*** | 8 | 59.03 | 237.8±97.0*** | 8 | 55.74 |

(continued)

| Days | C | | | D | | |
|---|---|---|---|---|---|---|
| | Tumor volume (mm$^3$) | N | Tumor growth inhibition rate (%) | Tumor volume (mm$^3$) | N | Tumor growth inhibition rate (%) |
| D21 | 284.1±97.6*** | 8 | 76.83 | 308.4±173.6*** | 8 | 74.84 |
| **Note:** Days: days after inoculation of the tumor cell suspension; N: number of animals in the group included in the statistical analysis. **: P<0.01, ***: P<0.001, compared with group A (solvent control). | | | | | | |

4. Tumor weight results

At 21 days after 4T1 inoculation, all animals were euthanized by cervical dislocation; tumors were excised, weighed, and photographed. The results are summarized in Table 135 and Figure 34-3.

Based on tumor weight, the growth-inhibition rates of Drugs B, C, and D against 4T1 were 64.65%, 60.62%, and 61.01%, respectively-all >60%.

Table 135. Summary of tumor weights

| Group | No. of animals (n) | Tumor weight (g) | IR_TW (%) |
|---|---|---|---|
| A | 8 | 1.61 ± 0.51 | \ |
| B | 8 | 0.57 ± 0.33*** | 64.65 |
| C | 8 | 0.64 ± 0.28*** | 60.62 |
| D | 8 | 0.63 ± 0.33*** | 61.01 |
| ***: P < 0.001, compared with Group A (vehicle control). | | | |

5. Results for splenic index and thymic index

21 days after 4T1 inoculation, collect orbital blood, all animals were euthanized by cervical dislocation; the spleen and thymus were excised and weighed. The results are summarized in Table 136.

In the group receiving tail-vein administration of A (vehicle control), the mean splenic index of tumor-bearing mice was 266.38. In the groups receiving tail-vein administration of Drugs B, C, and D, the mean splenic indices were 279.45, 239.08, and 230.62, respectively; no statistically significant differences were observed compared with Group A (vehicle control). The mean thymic indices in Groups B, C, and D were 23.07, 21.96, and 21.37, respectively-all higher than Group A (14.21), and the increases were statistically significant.

Table 136. Summary of splenic and thymic indices

| Group | No. of animals | Spleen index | Thymus index |
|---|---|---|---|
| A | 8 | 266.38 ± 45.32 | 14.21 ± 5.90 |
| B | 8 | 279.45 ± 78.92 | 23.07 ± 5.62** |
| C | 8 | 239.08 ± 44.82 | 21.96 ± 2.70** |
| D | 8 | 230.62 ± 53.33 | 21.37 ± 5.76* |
| *: *P* < 0.05, **:P < 0.01; comparisons are vs. Group A (vehicle control). | | | |

VI. Experimental Conclusions

Taking into comprehensive consideration the tumor growth-inhibition rate and the tumor-weight inhibition rate, the conclusions are as follows:

Following tail-vein administration of Drugs B, C, and D, at the end of the study (D21) the tumor inhibition rates of B, C, and D (calculated on the basis of tumor weight and tumor volume) each exceeded 60%, indicating that Drugs B, C, and D exert a marked growth-inhibitory effect on 4T1 murine mammary carcinoma. At D21, tail-vein administration of Drugs B, C, and D significantly increased the thymic index of tumor-bearing mice, whereas no significant effect on the splenic index was observed.

Example 35

I. Objective of the Study:

To verify the antitumor efficacy of nano-nucleic-acid carrier combinations delivering immunomodulatory and gene-regulatory agents, and to assess differences arising from different dosing start times and treatment cycles (pharmacodynamic evaluation of the nano-nucleic-acid carrier combination delivering immunomodulatory and gene-regulatory agents against CT26 murine colon carcinoma).

II. Cell Information

CT26, murine colon carcinoma; supplied by the Institute of Materia Medica, Chinese Academy of Medical Sciences.

III. Experimental Animal Information

Species: BALB/c

Body weight: 16-18 g

Sex: male

Supplier: Beijing Huafukang Biotechnology Co., Ltd.

IV. Experimental Methods

1. Inoculation and grouping

For the murine colon carcinoma CT26 model, male BALB/c mice weighing 16-18 g were used. At the time of the experiment, well-growing tumor tissue was harvested, minced and ground, and diluted with sterile normal saline to prepare a tumor-cell suspension. The CT26 tumor-cell suspension was inoculated subcutaneously into the left axillary-dorsal region of each mouse (0.2 mL/mouse); the day of inoculation was designated D0. On D1, animals were randomized into three groups-A (vehicle control), B, and C-with 8 animals per group. On D1, animals in Groups A and B began tail-vein intravenous dosing, administered every other day for a total of 7 administrations; animals in Group C began tail-vein dosing on D7, administered every other day for a total of 4 administrations. Groups B and C were designed to examine differences in efficacy arising from different dosing start times and treatment cycles. Details of inoculation, grouping, and dosing are shown in Tables 137 and 138.

Table 137. Animal inoculation and dosing scheme

| Cell line | No. of mice (n) | Mouse IDs | Inoculation site |
|---|---|---|---|
| CT26 | 24 | See grouping table | Subcutaneous, left axillary dorsal region |
| Note: The day of inoculation was designated as D0. | | | |

Table 138. Animal grouping and dosing regimen

| Group | No. of animals (n) | Route of administration | Dose volume per administration | No. of administrations | Dosing days |
|---|---|---|---|---|---|
| A | 8 | Tail-vein injection (i.v.) | 200 μL/20 g | 7 | D1,D3,D5, D7,D9,D11, D13 |
| B | 8 | Tail-vein injection (i.v.) | 200 μL/20 g | 7 | |
| C | 8 | Tail-vein injection (i.v.) | 200 μL/20 g | 4 | D7,D9, D11, D13 |

Wherein, in the "Treatment" column of Table 138, Drug A is PBS; Drug B includes 2 *CpG2006-CD40 aptamer-DNA carrier (25µg) + 2*AmiR-21-AS1411 aptamer-Bio-DNA carrier (12µg); and Drug C includes 2*CpG2006-CD40 aptamer-DNA carrier (25µg) + 2 *AmiR-21-AS1411 aptamer-Bio-DNA carrier (12µg). Accordingly, Drugs B and C correspond to Combination 9 (Drug 4 + Drug 23).

2. Measurement indices

Same as in Example 16.

3. Statistical analysis

Same as in Example 16.

V. Experimental Results:

1. Clinical observations in experimental animals

During the dosing period, all animals exhibited good general condition, including normal activity and food intake.

2. Changes in body weight of experimental animals

During the dosing period, body weight increased to a certain extent in all animals. The body-weight gain in Group C was higher than that in Group A (vehicle control), and the difference was statistically significant. The results are recorded in Table 139, and the body-weight growth curves are shown in Figure 35-1.

Table 139. Summary of animal body weight

| Group | No. of animals | | Body weight (g) | | Change in body weight (g) |
|---|---|---|---|---|---|
| | D1 | D15 | D1 | D15 | |
| A | 8 | 8 | 19.1 ± 0.8 | 22.6 ± 0.8 | +3.6 |
| B | 8 | 8 | 18.9 ± 0.5 | 22.8 ± 1.0 | +3.9 |
| C | 8 | 8 | 19.3 ± 0.8 | 23.8 ± 0.5** | +4.5 |

Note: D1 indicates 1 day after inoculation with the tumor cell suspension; D15 indicates 15 days after inoculation with the tumor cell suspension.
**: $P<0.01$, compared with Group A (vehicle control).

3. Tumor volume-inhibition results

All animals were closely monitored for tumor growth during the study; tumors were measured twice per week, the results were recorded, and tumor growth inhibition (TGI) was calculated. The results are summarized in Table 140 and Figure 35-2.

Tumors in Group A (vehicle control) grew well. Compared with Group A, the D15 tumor growth-inhibition rates (based on tumor volume) for Groups B and C were 47.88% and 23.85%, respectively, i.e., both <60%.

Table 140. Summary of tumor growth data

| Days | A | | B | | | C | | |
|---|---|---|---|---|---|---|---|---|
| | Tumor volume (mm$^3$) | N | Tumor volume (mm$^3$) | N | Tumor growth inhibition rate (%) | Tumor volume (mm$^3$) | N | Tumor growth inhibition rate (%) |
| D7 | 173.6 ± 43.3 | 8 | 186.4 ± 51.3 | 8 | -7.40 | 173.9 ± 53.7 | 8 | -0.20 |
| D9 | 548.1 ± 155.4 | 8 | 422.5 ± 115.6 | 8 | 22.92 | 501.1 ± 189.6 | 8 | 8.57 |
| D12 | 1115.3 ± 262.2 | 8 | 622.8 ± 242.6** | 8 | 44.16 | 852.6 ± 354.6 | 8 | 23.55 |

(continued)

| Days | A | | | B | | | | C | | |
|---|---|---|---|---|---|---|---|---|---|---|
| | Tumor volume (mm$^3$) | N | | Tumor volume (mm$^3$) | N | Tumor growth inhibition rate (%) | | Tumor volume (mm$^3$) | N | Tumor growth inhibition rate (%) |
| D15 | 2382.6 ± 484.6 | 8 | | 1241.8 ± 752.7** | 8 | 47.88 | | 1814.2 ± 634.1 | 8 | 23.85 |

Note: "Days" denotes the number of days after inoculation with the tumor cell suspension; "N" denotes the number of animals in the group included in the statistics.
**: P<0.01, compared with Group A (vehicle control) group.

4. Tumor weight

At 15 days after CT26 inoculation, all animals were euthanized by cervical dislocation; tumors were excised, weighed, and photographed. The results are summarized in Table 141 and Figure 35-3.
Based on tumor weight, the growth-inhibition rates of Drugs B and C against CT26 were 41.58% and 19.06%, respectively-both <60%.

Table 141. Summary of tumor weight

| Group | No. of animals (n) | Tumor weight (g) | IR_TW (%) |
|---|---|---|---|
| A | 8 | 2.93 ± 0.47 | \ |
| B | 8 | 1.71 ± 0.89** | 41.58 |
| C | 8 | 2.37 ± 0.52* | 19.06 |

*: *P* < 0.05, **: P < 0.01, compared with Group A (vehicle control).

5. Results for splenic index and thymic index

At 15 days after CT26 inoculation, after orbital blood collection, all animals were euthanized by cervical dislocation; the spleen and thymus were excised and weighed. The results are summarized in Table 142.
In the A (vehicle control) group, the mean splenic index of tumor-bearing mice was 110.84. In the groups receiving tail-vein administration of Drugs B and C, the mean splenic indices were 241.02 and 196.48, respectively, showing statistically significant differences compared with Group A (vehicle control). The mean thymic indices in the B and C groups were 16.98 and 15.07, respectively-both higher than Group A (12.34), but the differences were not statistically significant.

Table 142. Summary of spleen and thymus indices

| Group | No. of animals | Spleen index | Thymus index |
|---|---|---|---|
| A | 8 | 110.84 ± 28.06 | 12.34 ± 3.76 |
| B | 8 | 241.02 ± 22.45*** | 16.98 ± 6.76 |
| C | 8 | 196.48 ± 27.06*** | 15.07 ± 4.18 |

***: P < 0.01, compared with Group A (vehicle control).

VI. Experimental Conclusions:

Taking into comprehensive consideration the tumor growth-inhibition rate and the tumor-weight inhibition rate, the conclusions are as follows:
Following tail-vein administration of Drugs B and C, at the end of the study (D15) the tumor-inhibition rates of Drugs B and C (calculated on the basis of tumor weight and tumor volume) did not exceed 60%, indicating that Drugs B and C do not exhibit a marked growth-inhibitory effect on CT26 murine colon carcinoma. At D15, tail-vein administration of Drugs B and C significantly increased the splenic index of tumor-bearing mice.

Example 36

I. Objective of the Study:

To verify the antitumor efficacy of nano-nucleic-acid carrier combinations delivering immunomodulatory and gene-regulatory drugs.

II. Cell Information

GL261, murine glioma; purchased from Guangzhou Jineo Biotechnology Co., Ltd.

III. Experimental Animal Information

Species: C57BL/6J

Body weight: 16-18 g

Sex: male

Supplier: Beijing Huafukang Biotechnology Co., Ltd.

IV. Experimental Methods

1. Inoculation and grouping

For the murine glioma GL261 model, male C57BL/6J mice weighing 16-18 g were used. At the time of the experiment, well-growing tumor tissue was harvested, minced and ground, and diluted with sterile normal saline to prepare a tumor-cell suspension. The GL261 tumor-cell suspension was inoculated subcutaneously into the left axillary-dorsal region of each mouse (0.2 mL/mouse); the day of inoculation was designated D0. On D1, animals were randomized into two groups-A (vehicle control) and B-with 8 animals per group. On D3, animals in Groups A and B began tail-vein intravenous dosing, administered every other day for a total of seven administrations. Details of inoculation, grouping, and dosing are provided in Tables 143 and 144.

Table 143. Animal inoculation and dosing scheme

| Tumor cell line | No. of mice (n) | Mouse IDs | Inoculation site |
|---|---|---|---|
| GL261 | 16 | See grouping table | Subcutaneous, left axillary dorsal region |
| Note: The day of inoculation was designated as **D0.** | | | |

Table 144. Animal grouping and dosing

| Group | No. of animals (n) | Route of administration | Dose volume per administration | No. of administrations | Dosing days |
|---|---|---|---|---|---|
| A | 8 | Tail-vein injection (i.v.) | 200 μL/20 g | 7 | D3,D5, D7, D9,D11, D13, D15 |
| B | 8 | Tail-vein injection (i.v.) | 200 μL/20 g | 7 | |

Wherein, A is PBS; B includes 2*CpG2006-CD40 aptamer-DNA carrier (25μg) + 2 *AmiR-21-AS1411 aptamer-Bio-DNA carrier (12μg). Accordingly, Drug B corresponds to Combination 9 (said Drug 4 + said Drug 23).

2. Measurement indices

Same as in Example 16.

3. Statistical analysis

Same as in Example 16.

V. Experimental Results:

1. Clinical observations in experimental animals

During the dosing period, all animals exhibited good general condition, including normal activity and food intake.

2. Changes in body weight of experimental animals

During the dosing period, body weight increased to a certain extent in all animals. The results are recorded in Table 145, and the body-weight growth curves of tumor-bearing animals are shown in Figure 36-1.

Table 145. Summary of animal body weight

| Group | No. of animals | | Body weight (g) | | Change in body weight (g) |
|---|---|---|---|---|---|
| | D1 | D17 | D1 | D17 | |
| A | 8 | 8 | 17.3 $\pm$ 0.4 | 21.1 $\pm$ 1.6 | +3.8 |
| B | 8 | 8 | 16.7 $\pm$ 0.5 | 20.8 $\pm$ 1.2 | +4.1 |
| Note: D1 indicates 1 day after inoculation with the tumor cell suspension; D17 indicates 17 days after inoculation with the tumor cell suspension. | | | | | |

3. Tumor volume-inhibition results

Throughout the study, all animals were closely monitored for tumor growth; tumors were measured twice per week, results were recorded, and tumor growth inhibition (TGI) was calculated. The results are summarized in Table 146 and Figure 36-2.

Tumors in Group A (vehicle control) grew well. Compared with Group A, the Group B tumor growth-inhibition rate on D17 (based on tumor volume) was 61.86%, i.e., greater than 60%.

Table 146. Summary of tumor growth data

| Days | A | | B | | |
|---|---|---|---|---|---|
| | Tumor volume (mm$^3$) | N | Tumor volume (mm$^3$) | N | Tumor growth inhibition rate (%) |
| D7 | 224.6 $\pm$ 53.5 | 8 | 146.7 $\pm$ 42.6** | 8 | 32.86 |
| D10 | 469.6 $\pm$ 104.5 | 8 | 258.3 $\pm$ 107.9** | 8 | 45.00 |
| D14 | 1141.6 $\pm$ 612.4 | 8 | 441.7 $\pm$ 157.9** | 8 | 61.30 |
| D17 | 1827.6 $\pm$ 902.2 | 8 | 697.1 $\pm$ 355.2** | 8 | 61.86 |
| Note: "Days" indicates the number of days after inoculation with the tumor cell suspension; "N" indicates the number of animals in the group included in the statistics.<br>**: P<0.01, compared with Group A (vehicle control). | | | | | |

4. Tumor weight

At 17 days after GL261 inoculation, all animals were euthanized by cervical dislocation; tumors were excised, weighed, and photographed. The results are summarized in Table 147 and Figure 36-3.

Based on tumor weight, the growth-inhibition rate of Drug B against GL261 was 64.18%, i.e., greater than 60%.

Table 147. Summary of tumor weights

| Group | No. of animals (n) | Tumor weight (g) | IR_TW (%) |
|---|---|---|---|
| A | 8 | 1.93 $\pm$ 1.05 | \ |

(continued)

| Group | No. of animals (n) | Tumor weight (g) | IR_TW (%) |
|-------|--------------------|------------------|-----------|
| B | 8 | $0.69 \pm 0.42$** | 64.18 |

**: $P < 0.01$ (**), compared with Group A (vehicle control).

5. Results for splenic index and thymic index

At 17 days after GL261 inoculation, all animals were euthanized by cervical dislocation; the spleen and thymus were excised and weighed. The results are summarized in Table 148.

In the group receiving tail-vein administration of A (vehicle control), the mean splenic index of tumor-bearing mice was 115.30; in the group receiving tail-vein administration of Drug B, the mean splenic index was 130.47, which did not differ significantly from Group A (vehicle control). The mean thymic index in the Drug B group was 20.20, which was higher than that of Group A (17.60), but the difference was not statistically significant.

Table 148. Summary of splenic and thymic indices

| Group | No. of animals | Spleen index | Thymus index |
|-------|----------------|--------------|--------------|
| A | 8 | $115.30 \pm 34.01$ | $17.60 \pm 5.00$ |
| B | 8 | $130.47 \pm 22.21$ | $20.20 \pm 4.89$ |

VI. Experimental Conclusions

Taking into comprehensive consideration the tumor growth-inhibition rate and the tumor-weight inhibition rate, the conclusions are as follows:

Following tail-vein administration of Drug B, at the end of the study (D17) the tumor-inhibition rate of Drug B (calculated on the basis of tumor weight and tumor volume) exceeded 60%, indicating that Drug B exerts a marked growth-inhibitory effect on murine GL261 glioma.

Example 37

I. Objective of the Study:

To verify the antitumor efficacy of nano-nucleic-acid carrier combinations delivering immunomodulatory and gene-regulatory agents.

II. Cell Information

Lewis, murine lung carcinoma; purchased from the Cell Resource Center, Institute of Basic Medical Sciences, Chinese Academy of Medical Sciences.

III. Experimental Animal Information

Species: C57BL/6J

Body weight: 16-18 g

Sex: male

Supplier: Beijing Huafukang Biotechnology Co., Ltd.

IV. Experimental Methods

1. Inoculation and grouping

For the murine lung carcinoma Lewis model, male C57BL/6J mice weighing 16-18 g were used. At the time of the experiment, well-growing tumor tissue was harvested, minced and ground, and diluted with sterile normal saline to prepare a tumor-cell suspension. The Lewis tumor-cell suspension was inoculated subcutaneously into the left axillary-dorsal region of each mouse (0.2 mL/mouse); the day of inoculation was designated D0. On D1, animals were randomized into two groups-A (vehicle control) and B-with 8 animals per group. On D3, animals in both groups began tail-vein injections, administered every other day for a total of 5 administrations. Details of inoculation, grouping, and dosing are shown in Tables 149 and 150.

Table 149. Animal inoculation and dosing scheme

| Tumor cell line | No. of mice (n) | Mouse IDs | Inoculation site |
|---|---|---|---|
| Lewis | 16 | See grouping table | Subcutaneous, left axillary dorsal region |
| **Note:** The day of inoculation was designated as **D0.** | | | |

Table 150. Animal grouping and dosing

| Group | No. of animals (n) | Route of administration | Dose volume per administration | No. of administrations | Dosing days |
|---|---|---|---|---|---|
| A | 8 | Tail-vein injection (i.v.) | 200 μL/20 g | 5 | D3,D5,D7, D9, D11 |
| B | 8 | Tail-vein injection (i.v.) | 200 μL/20 g | 5 | |

Wherein, A is PBS; B includes 2*CpG2006-CD40 aptamer-DNA carrier (25μg) + 2 *AmiR-21-AS1411 aptamer-Bio-DNA carrier (12μg). Accordingly, Drug B corresponds to Combination 9 (said Drug 4 + said Drug 23).

2. Measurement indices

Same as in Example 16.

3. Statistical analysis

Same as in Example 16.

V. Experimental Results:

1. Clinical observations in experimental animals

During the dosing period, all animals exhibited good general condition (activity, food intake, etc.); one animal in Group A died spontaneously on D11.

2. Changes in body weight of experimental animals

During the dosing period, body weight increased to a certain extent in all animals. The results are recorded in Table 151, and the body-weight growth curves of tumor-bearing animals are shown in Figure 37-1.

Table 151. Summary of animal body weight

| Group | No. of animals | | Body weight (g) | | Change in body weight (g) |
|---|---|---|---|---|---|
| | D1 | D13 | D1 | D13 | |
| A | 8 | 7 | 16.6 ± 0.5 | 21.2 ± 1.4 | +4.5 |
| B | 8 | 8 | 16.3 ± 0.7 | 21.1 ± 2.3 | +4.8 |
| Note: D1 indicates 1 day after inoculation with the tumor cell suspension; D13 indicates 13 days after inoculation with the tumor cell suspension. | | | | | |

3. Tumor volume-inhibition results

Throughout the study, all animals were closely monitored for tumor growth; tumors were measured twice per week, results were recorded, and tumor growth inhibition (TGI) was calculated. The results are summarized in Table 152 and Figure 37-2.

Tumors in Group A (vehicle control) grew well. Compared with Group A, the Group B tumor growth-inhibition rate on D13 (based on tumor volume) was 39.63%, i.e., <60%.

Table 152. Summary of tumor growth data

| Days | A | | B | | |
|---|---|---|---|---|---|
| | Tumor volume ($mm^3$) | N | Tumor volume ($mm^3$) | N | Tumor growth inhibition rate (%) |
| D6 | 648.6 ± 454.3 | 8 | 278.6 ± 91.9* | 8 | 57.05 |
| D9 | 1479.9 ± 722.0 | 8 | 791.2 ± 456.1* | 8 | 46.54 |
| D13 | 2649.1 ± 1427.9 | 7 | 1599.3 ± 1051.3* | 8 | 39.63 |
| Note: "Days" indicates the number of days after inoculation with the tumor cell suspension; "N" indicates the number of animals in the group included in the statistics. *: P<0.05, compared with Group A (vehicle control). | | | | | |

4. Tumor weight

At 13 days after Lewis inoculation, all animals were euthanized by cervical dislocation; tumors were excised, weighed, and photographed. The results are summarized in Table 153 and Figure 37-3.

Based on tumor weight, the growth-inhibition rate of Drug B against Lewis was 35.99%, i.e., <60%.

Table 153. Summary of tumor weights

| Group | No. of animals (n) | Tumor weight (g) | IR_TW (%) |
|---|---|---|---|
| A | 7 | 3.10 ± 1.48 | \ |
| B | 8 | 1.98 ± 0.73 | 35.99 |

5. Results for splenic index and thymic index

At 13 days after Lewis inoculation, all animals were euthanized by cervical dislocation; the spleen and thymus were excised and weighed. The results are summarized in Table 154.

In the group receiving tail-vein administration of A (vehicle control), the mean splenic index of tumor-bearing mice was 178.61; in the group receiving tail-vein administration of Drug B, the mean splenic index was 179.82, which did not differ significantly from Group A (vehicle control). The mean thymic index in the Drug B group was 15.27, which was higher than that of Group A (11.55), but the difference was not statistically significant.

Table 154. Summary of splenic and thymic indices

| Group | No. of animals | Spleen index | Thymus index |
|---|---|---|---|
| A | 7 | 178.61 ± 17.18 | 11.55 ± 7.18 |
| B | 8 | 179.82 ± 23.18 | 15.27 ± 8.81 |

VI. Experimental Conclusions:

Taking into comprehensive consideration the tumor growth-inhibition rate and the tumor-weight inhibition rate, the conclusions are as follows:

Following tail-vein administration of Drug B, at the end of the study (D13) the tumor-inhibition rate of Drug B (calculated on the basis of tumor weight and tumor volume) did not exceed 60%, indicating that Drug B does not exhibit a marked growth-inhibitory effect on murine Lewis carcinoma.

Example 38

I. Objective of the Study:

To verify the antitumor efficacy of nano-nucleic-acid carrier combinations delivering immunomodulatory and gene-regulatory agents.

II. Cell Information

MC38, murine colon carcinoma; purchased from the Cell Resource Center, Institute of Basic Medical Sciences, Chinese Academy of Medical Sciences.

III. Experimental Animal Information

Species: C57BL/6J

Body weight: 16-18 g

Sex: male

Supplier: Beijing Huafukang Biotechnology Co., Ltd.

IV. Experimental Methods

1. Inoculation and grouping

For the murine colon carcinoma MC38 model, male C57BL/6J mice weighing 16-18 g were used. At the time of the experiment, well-growing tumor tissue was harvested, minced and ground, and diluted with sterile normal saline to prepare a tumor-cell suspension. The MC38 tumor-cell suspension (0.2 mL/mouse) was inoculated subcutaneously into the left axillary-dorsal region; the day of inoculation was designated D0. On D1, animals were randomized into two groups: A (vehicle control) and B, 8 animals per group. On D3, animals in Groups A and B began tail-vein intravenous dosing, administered every other day for a total of seven administrations. Details of inoculation, grouping, and dosing are provided in Tables 155 and 156.

Table 155. Animal inoculation and dosing scheme

| Tumor cell line | No. of mice (n) | Mouse IDs | Inoculation site |
|---|---|---|---|
| MC38 | 16 | See grouping table | Subcutaneous, left axillary dorsal region |
| **Note:** The day of inoculation was designated as **D0.** | | | |

Table 156. Animal grouping and dosing

| Group | No. of animals (n) | Route of administration | Dose volume per administration | No. of administrations | Dosing days |
|---|---|---|---|---|---|
| A | 8 | Tail-vein injection (i.v.) | 200 μL/20 g | 7 | D3, D5, D7, D9,D11,D13 , D15 |
| B | 8 | Tail-vein injection (i.v.) | 200 μL/20 g | 7 | |

Wherein, A is PBS; B includes 2*CpG2006-CD40 aptamer-DNA carrier (25μg) + 2 *AmiR-21-AS1411 aptamer-Bio-DNA carrier (12μg)-i.e., Drug B corresponds to Combination 9 (Drug 4 + Drug 23).

2. Measurement indices

Same as in Example 16.

3. Statistical analysis

Same as in Example 16.

V. Experimental Results:

1. Clinical observations in experimental animals

During the dosing period, all animals exhibited good general condition (activity, food intake, etc.).

2. Changes in body weight of experimental animals

During the dosing period, body weight increased to a certain extent in all animals. At D17, body weight in Group B was higher than that in Group A (vehicle control), and the difference was statistically significant. The results are recorded in Table 157, and the body-weight growth curves of tumor-bearing animals are shown in Figure 38-1.

Table 157. Summary of animal body weight

| Group | No. of animals | | Body weight (g) | | Change in body weight (g) |
|---|---|---|---|---|---|
| | D1 | D17 | D1 | D17 | |
| A | 8 | 8 | $15.4 \pm 0.4$ | $19.2 \pm 2.4$ | +3.7 |
| B | 8 | 8 | $15.7 \pm 0.4$ | $21.3 \pm 0.9^*$ | +5.6 |
| Note: D1 indicates 1 day after inoculation with the tumor cell suspension; D17 indicates 17 days after inoculation with the tumor cell suspension.<br>*: $P<0.05$, compared with Group A (vehicle control). | | | | | |

3. Tumor volume-inhibition results

Throughout the study, all animals were closely monitored for tumor growth; tumors were measured twice per week, results were recorded, and tumor growth inhibition (TGI) was calculated. The results are summarized in Table 158 and Figure 38-2.

Tumors in Group A (vehicle control) grew well. Compared with Group A, the Group B tumor growth-inhibition rate on D17 (based on tumor volume) was 66.43%, i.e., greater than 60%.

Table 158. Summary of tumor growth data

| Days | A | | B | | |
|---|---|---|---|---|---|
| | Tumor volume ($mm^3$) | N | Tumor volume ($mm^3$) | N | Tumor growth inhibition rate (%) |
| D6 | $253.8 \pm 31.5$ | 8 | $132.0 \pm 19.1^{***}$ | 8 | 47.98 |
| D10 | $600.0 \pm 208.2$ | 8 | $260.6 \pm 108.4^{**}$ | 8 | 56.56 |
| D13 | $1349.8 \pm 528.5$ | 8 | $620.4 \pm 317.1^{**}$ | 8 | 54.04 |
| D17 | $1783.8 \pm 729.9$ | 8 | $598.8 \pm 235.6^{***}$ | 8 | 66.43 |
| Note: "Days" indicates the number of days after inoculation with the tumor cell suspension; "N" indicates the number of animals in the group included in the statistics.<br>**: $P<0.01$, ***: $P<0.001$, compared with Group A (vehicle control). | | | | | |

4. Tumor weight

At 17 days after MC38 inoculation, all animals were euthanized by cervical dislocation; tumors were excised, weighed, and photographed. The results are summarized in Table 159 and Figure 38-3.

Based on tumor weight, the growth-inhibition rate of Drug B against MC38 was 61.93%, i.e., greater than 60%.

Table 159. Summary of tumor weights

| Group | No. of animals (n) | Tumor weight (g) | IR_TW (%) |
|-------|--------------------|--------------------|-----------|
| A | 8 | $2.28 \pm 0.71$ | \ |
| B | 8 | $0.87 \pm 0.28$*** | 61.93 |

***: $P < 0.001$, compared with Group A (vehicle control).

5. Results for splenic index and thymic index

At 17 days after MC38 inoculation, all animals were euthanized by cervical dislocation; the spleen and thymus were excised and weighed. The results are summarized in Table 160.

In the A (vehicle control) group, the mean splenic index of tumor-bearing mice was 84.29; in the Drug B group, the mean splenic index was 126.33, which showed a statistically significant difference compared with the A (vehicle control) group. The mean thymic index in the Drug B group was 21.52, which was higher than that of Group A (16.60), but the difference was not statistically significant.

Table 160. Summary of splenic and thymic indices

| Group | No. of animals | Spleen index | Thymus index |
|-------|----------------|--------------|--------------|
| A | 8 | $84.29 \pm 15.24$ | $16.60 \pm 6.82$ |
| B | 8 | $126.33 \pm 29.75$** | $21.52 \pm 2.58$ |

**: $P < 0.01$ (**), compared with Group A (vehicle control).

VI. Experimental Conclusions

This study evaluated the growth-inhibitory effect of Drug B on murine MC38 colon carcinoma. Taking into comprehensive consideration the tumor growth-inhibition rate and the tumor-weight inhibition rate, the conclusions are as follows:

After tail-vein administration of Drug B, at the end of the study (D17) the tumor-inhibition rate of Drug B (calculated on the basis of tumor weight and tumor volume) exceeded 60%, indicating that Drug B exerts a marked growth-inhibitory effect on MC38 colon carcinoma in mice. Moreover, tail-vein administration of Drug B noticeably increased the splenic index of tumor-bearing mice at D17.

Example 39

I. Objective of the Study:

To establish a PANC02 tumor model and verify the drug's immunostimulatory and gene-regulatory effects as well as its antitumor efficacy.

II. Cell Information

PANC02 cell line, supplied by the Institute of Materia Medica, Chinese Academy of Medical Sciences.

III. Experimental Animal Information

Species: BALB/c mice (immunocompetent)

Age: 6-8 weeks

Sex: male

Supplier: Beijing Huazhuokang Biotechnology Co., Ltd.

IV. Experimental Methods

1. Inoculation and grouping

PANC02 murine pancreatic carcinoma model: male C57BL/6J mice, 16-18 g. At the time of the experiment, well-growing tumor tissue was harvested, minced and ground, and diluted with sterile normal saline to prepare a tumor-cell suspension. The PANC02 tumor-cell suspension was inoculated subcutaneously into the left axillary-dorsal region of each mouse (0.2 mL/mouse); the day of inoculation was designated D0. On D1, animals were randomized into two groups: A (vehicle control) and B, 8 animals per group. On D3, animals in Groups A and B began tail-vein injections, administered every other day for a total of 4 administrations. Details of inoculation, grouping, and dosing are shown in Tables 161 and 162.

Table 161. Animal inoculation and dosing scheme

| Tumor cell line | No. of mice (n) | Mouse IDs | Inoculation site |
|---|---|---|---|
| Pan02 | 16 | See grouping table | Subcutaneous, left axillary dorsal region |
| Note: The day of inoculation was designated as **D0.** | | | |

Table 162. Animal grouping and dosing regimen

| Group | No. of animals (n) | Route of administration | Dose volume per administration | No. of administrations | Dosing days |
|---|---|---|---|---|---|
| A | 8 | Tail-vein injection (i.v.) | 200 μL/20 g | 4 | D3, D5, D7, D9 |
| B | 8 | Tail-vein injection (i.v.) | 200 μL/20 g | 4 | |

Wherein, A is PBS; B includes 2*CpG2006-CD40 aptamer-DNA carrier (25 μg) + 2 *AmiR-21-AS1411 aptamer-Bio-DNA carrier (12 μg)-i.e., Drug B corresponds to Combination 9 (Drug 4 + Drug 23).

2. Measurement indices

Same as in Example 16.

3. Statistical analysis

Same as in Example 16.

V. Experimental Results:

1. Clinical observations in experimental animals

During the dosing period, all animals exhibited good general condition, including normal activity and food intake.

2. Changes in body weight of experimental animals

During the dosing period, body weight increased to a certain extent in all animals. At D11, the body weight of tumor-bearing mice in Group B was lower than that in Group A (vehicle control), but the difference was not statistically significant. The results are recorded in Table 163, and the body-weight growth curves of tumor-bearing animals are shown in Figure 39-1.

Table 163. Summary of animal body weight

| Group | No. of animals | | Body weight (g) | | Change in body weight (g) |
|---|---|---|---|---|---|
| | D1 | D11 | D1 | D11 | |
| A | 8 | 7 | 16.4 ± 0.6 | 21.3 ± 1.7 | +5.0 |

(continued)

| Group | No. of animals | | Body weight (g) | | Change in body weight (g) |
|---|---|---|---|---|---|
| | D1 | D11 | D1 | D11 | |
| B | 8 | 8 | 16.1 ± 0.4 | 19.9 ± 1.3 | +3.8 |

Note: D1 indicates 1 day after inoculation with the tumor cell suspension; D11 indicates 11 days after inoculation with the tumor cell suspension.

3. Tumor volume-inhibition results

Throughout the study, all animals were closely monitored for tumor growth; tumors were measured twice per week, results were recorded, and tumor growth inhibition (TGI) was calculated. The results are summarized in Table 164 and Figure 39-2. Compared with Group A (vehicle control), the Group B tumor growth-inhibition rate on D11 (based on tumor volume) was 43.07%, i.e., <60%.

Table 164. Summary of tumor growth data

| Days | A | | B | | |
|---|---|---|---|---|---|
| | Tumor volume (mm$^3$) | N | Tumor volume (mm$^3$) | N | Tumor growth inhibition rate (%) |
| D4 | 248.1 ± 61 | 8 | 140.2 ± 31.3*** | 8 | 43.50 |
| D7 | 1224.2 ± 250.8 | 8 | 916.8 ± 445.7 | 8 | 25.11 |
| D11 | 3922.0 ± 1156.3 | 7 | 2232.7 ± 1038.8* | 8 | 43.07 |

Note: "Days" indicates the number of days after inoculation with the tumor cell suspension; "N" indicates the number of animals in the group included in the statistics.
*: P<0.05, ***: P<0.001, compared with Group A (vehicle control).

4. Tumor weight

At 11 days after PANC02 inoculation, all animals were euthanized by cervical dislocation; tumors were excised, weighed, and photographed. The results are summarized in Table 165 and Figure 39-3.
Based on tumor weight, the growth-inhibition rate of Drug B against PANC02 was 43.11%, i.e., <60%.

Table 165. Summary of tumor weight

| Group | No. of animals (n) | Tumor weight (g) | IR_TW (%) |
|---|---|---|---|
| A | 7 | 3.85 ± 0.85 | \ |
| B | 8 | 2.19 ± 0.77** | 43.11 |

**: P < 0.01 (**), compared with Group A (vehicle control).

5. Results for splenic index and thymic index

At 11 days after PANC02 inoculation, all animals were euthanized by cervical dislocation; the spleen and thymus were excised and weighed. The results are summarized in Table 166.
In the group receiving tail-vein administration of A (vehicle control), the mean splenic index of tumor-bearing mice was 111.71. In the group receiving tail-vein administration of Drug B, the mean splenic index was 144.18, which showed a statistically significant difference compared with Group A (vehicle control). The mean thymic index in the Drug B group was 11.79, higher than Group A (10.08), but the difference was not statistically significant.

Table 166. Summary of spleen and thymus indices

| Group | No. of animals | Spleen index | Thymus index |
|---|---|---|---|
| A | 7 | 111.71 ± 15.47 | 10.08 ± 3.98 |

(continued)

| Group | No. of animals | Spleen index | Thymus index |
|---|---|---|---|
| B | 8 | 144.18 $\pm$ 21.56** | 11.79 $\pm$ 5.13 |

**: P < 0.01 (**), compared with Group A (vehicle control).

VI. Experimental Conclusions

Taking into comprehensive consideration the tumor growth-inhibition rate and the tumor-weight inhibition rate, the conclusions are as follows:

After tail-vein administration of Drug B, at the end of the study (D11) the tumor-inhibition rate of Drug B (calculated on the basis of tumor weight and tumor volume) did not exceed 60%, indicating that Drug B does not exhibit a marked growth-inhibitory effect on murine PANC02 pancreatic carcinoma. At D11, tail-vein administration of Drug B noticeably increased the splenic index of tumor-bearing mice.

Example 40

I. Objective of the Study:

To verify the antitumor efficacy of nano-nucleic-acid carrier combinations delivering immunomodulatory and gene-regulatory agents (pharmacodynamic evaluation of the nano-nucleic-acid carrier combination delivering immunomodulatory and gene-regulatory drugs against CT26 murine colon carcinoma).

II. Cell Information

CT26, murine colon carcinoma; supplied by Baiyao Zhida (Beijing) Nanobiotechnology Co., Ltd.

III. Experimental Animal Information

Species: BALB/c

Body weight: 16-18 g

Sex: male

Supplier: Beijing Huazhuokang Biotechnology Co., Ltd.

IV. Experimental Methods

1. Inoculation and grouping

For the murine CT26 colon carcinoma model, male BALB/c mice weighing 16-18 g were used. At the time of the experiment, well-growing tumor tissue was harvested, minced and ground, and diluted with sterile normal saline to prepare a tumor-cell suspension. The CT26 tumor-cell suspension (0.2 mL/mouse) was inoculated subcutaneously into the left axillary-dorsal region; the day of inoculation was designated D0. On D1, animals were randomized into three groups-A (vehicle control), B, and C-with 8 animals per group. On D1, animals in Groups A and B began tail-vein injections, administered every other day for a total of 7 doses; animals in Group C began tail-vein injections on D7, administered every other day for a total of 4 doses. Details of inoculation, grouping, and dosing are provided in Tables 167 and 168.

Table 167. Animal inoculation and administration protocol

| Tumor cell line | No. of mice | Mouse No. | Inoculation site |
|---|---|---|---|
| CT26 | 24 | see grouping table | subcutaneously in the left axillary dorsal region |
| Note: the day of inoculation is designated as D0. | | | |

Table 168. Animal grouping and dosing regimen

| Group | No. of animals (n) | Route of administration | Dose volume per administration | No. of administrations | Dosing days |
|---|---|---|---|---|---|
| A | 8 | Tail-vein injection (i.v.) | 200 μL/20 g | 7 | D3, D5, D7, D9, D11, D13, D15 |
| B | 8 | Tail-vein injection (i.v.) | 200 μL/20 g | 7 | |
| C | 8 | Tail-vein injection (i.v.) | 200 μL/20 g | 7 | |

Wherein, in the "Treatment" column of Table 168, Drug A is PBS; Drug B includes 2 *CpG2006-CD40 aptamer-DNA carrier (35μg) + 2*AmiR-21-AS 1411 aptamer-Bio-DNA carrier (12μg); and Drug C includes 2*CpG2006-CD40 aptamer-DNA carrier (35μg) + 2 *AmiR-21-AS1411 aptamer-Bio-DNA carrier (12μg) + OX40 (DNA) (12μg). Accordingly, Drug B corresponds to Combination 9 (said Drug 4 + said Drug 23), and Drug C corresponds to said Drug 4 + said Drug 23 + an OX40 aptamer in DNA form.

2. Measurement indices

Same as in Example 16.

3. Statistical analysis

Same as in Example 16.

V. Experimental Results:

1. Clinical observations in experimental animals

During the dosing period, all animals exhibited good general condition (activity, food intake, etc.).

2. Changes in body weight of experimental animals

During the dosing period, body weight increased to a certain extent in all animals. The results are recorded in Table 169, and the body-weight growth curves of tumor-bearing animals are shown in Figure 40-1.

Table 169. Summary of animal body weight

| Group | No. of animals | | Body weight (g) | | Change in body weight (g) |
|---|---|---|---|---|---|
| | D1 | D17 | D1 | D17 | |
| A | 8 | 8 | 16.3 ± 0.6 | 23.6 ± 1.6 | +7.3 |
| B | 8 | 8 | 16.6 ± 0.7 | 22.5 ± 1.4 | +6.0 |
| C | 8 | 8 | 16.3 ± 0.8 | 22.8 ± 1.4 | +6.5 |
| Note: D1 indicates 1 day after inoculation with the tumor cell suspension; D17 indicates 17 days after inoculation with the tumor cell suspension. | | | | | |

3. Tumor volume-inhibition results

All animals were closely monitored for tumor growth during the study; tumors were measured twice per week, results were recorded, and tumor growth inhibition (TGI) was calculated. The results are summarized in Table 170 and Figure 40-2.

Tumors in Group A (vehicle control) grew well. Compared with Group A, the D17 tumor growth-inhibition rates (based on tumor volume) for Groups B and C were 57.76% and 46.63%, respectively-both <60%.

Table 170. Summary of tumor growth data

| Days | A | | B | | | C | | |
|------|---|---|---|---|---|---|---|---|
| | Tumor volume (mm$^3$) | N | Tumor volume (mm$^3$) | N | Tumor growth inhibition rate (%) | Tumor volume (mm$^3$) | N | Tumor growth inhibition rate (%) |
| D6 | 228.1 ± 44.3 | 8 | 246.1 ± 56.1 | 8 | -7.87 | 190.0 ± 38.7 | 8 | 16.70 |
| D10 | 765.3 ± 108.9 | 8 | 337.1 ± 113.8*** | 8 | 55.95 | 409.0 ± 123.0*** | 8 | 46.55 |
| D13 | 1505.5 ± 288.5 | 8 | 850.7 ± 435.8** | 8 | 43.49 | 1201.0 ± 487.2 | 8 | 20.22 |
| D17 | 2832.8 ± 485.1 | 8 | 1196.5 ± 976.6*** | 8 | 57.76 | 1511.9 ± 770.3** | 8 | 46.63 |

Note: "Days" denotes the number of days after inoculation with the tumor cell suspension; "N" denotes the number of animals in the group included in the statistics.
**: P<0.01, ***: P<0.001, compared with Group A (vehicle control).

4. Tumor weight

At 17 days after CT26 inoculation, all animals were euthanized by cervical dislocation; tumors were excised, weighed, and photographed. The results are summarized in Table 171 and Figure 40-3.
Based on tumor weight, the growth-inhibition rates of Drugs B and C against CT26 were 57.00% and 39.22%, respectively-both <60%.

Table 171. Summary of tumor weight

| Group | No. of animals (n) | Tumor weight (g) | IR_TW (%) |
|-------|--------------------|------------------|-----------|
| A | 8 | 3.93 ± 0.51 | \ |
| B | 8 | 1.69 ± 1.35*** | 57.00 |
| C | 8 | 2.39 ± 1.06** | 39.22 |

**: P < 0.01 (**), P < 0.001 (***), compared with Group A (vehicle control).

5. Results for splenic index and thymic index

At 17 days after CT26 inoculation, after orbital blood collection, all animals were euthanized by cervical dislocation; the spleen and thymus were excised and weighed. The results are summarized in Table 172.
In the A (vehicle control) group, the mean splenic index of tumor-bearing mice was 174.79. In the groups receiving tail-vein administration of Drugs B and C, the mean splenic indices were 234.65 and 239.89, respectively, showing statistically significant differences compared with Group A (vehicle control). The mean thymic indices in the B and C groups were 18.88 and 17.98, respectively-both higher than Group A (15.46), but the differences were not statistically significant.

Table 172. Summary of spleen and thymus indices

| Group | No. of animals | Spleen index | Thymus index |
|-------|----------------|--------------|--------------|
| A | 8 | 174.79 ± 24.09 | 15.46 ± 5.51 |
| B | 8 | 234.65 ± 19.12*** | 18.88 ± 2.49 |
| C | 8 | 239.89 ± 19.86*** | 17.98 ± 4.11 |

***P < 0.001, compared with Group A (vehicle control).

VI. Experimental Conclusions:

Taking into comprehensive consideration the tumor growth-inhibition rate and the tumor-weight inhibition rate, the conclusions are as follows:
After tail-vein administration of Drugs B and C, at the end of the study (D17) the tumor-inhibition rates of B and C (calculated on the basis of tumor weight and tumor volume) both exceeded 60%, indicating that Drugs B and C do not exhibit a marked growth-inhibitory effect on CT26 murine colon carcinoma. Moreover, at D17, tail-vein administration of B and C markedly increased the splenic index of tumor-bearing mice.

Example 41

I. Objective of the Study:

To establish a CT26 tumor model and investigate, under the condition of an identical cumulative administered dose, the differences in antitumor efficacy (pharmacodynamics) between near-maximum high and low concentrations given at different dosing frequencies, as well as the animals' tolerance to cumulative toxicity.

II. Cell Information

CT26, murine colon carcinoma cell line; purchased from Aili Biotechnology (Shanghai) Co., Ltd.

III. Experimental Animal Information

Species: BALB/cCrSlcNifdc mice (immunocompetent)

Age: 6-7 weeks

Sex: female

Body weight: 18-22 g

Supplier: National Institutes for Food and Drug Control (NIFDC), China.

IV. Experimental Methods

1. Cell culture

Tumor cells were cultured in RPMI-1640 medium supplemented with 10% heat-inactivated fetal bovine serum, penicillin 100 U/mL, streptomycin 100 $\mu$g/mL, and L-glutamine 2 mM, at 37 °C in a 5% $CO_2$ incubator. Cells were subcultured every 2-3 days, and cells in the logarithmic growth phase were used for in-vivo tumor inoculation.

2. Inoculation and grouping

CT26 murine colon carcinoma cells ($2 \times 10^6$ cells/0.1 mL) were inoculated subcutaneously into the left and right dorsal cervical regions of female BALB/c mice; the day of inoculation was designated D0. On D7 and D9, nine animals were transferred to project SQ019134-BYZD18-TN. On D12, the remaining seven animals were enrolled in the present study and dosing was initiated.

Details of inoculation, grouping, and dosing are provided in Tables 173 and 174 and Figure 41-1.

Table 173. Animal inoculation and dosing scheme

| Animal inoculation details | | | | |
|---|---|---|---|---|
| Cell line | No. of mice | Mouse ID Nos. | Inoculation site | Cell inoculum |
| CT26 | 16 | 276-291 | Subcutaneous, bilateral dorsal cervical region | $2 \times 10^6$ cells/mouse |

**Table 174**

| Animal grouping and dosing | | | | | | |
|---|---|---|---|---|---|---|
| Group | No. of animals | Mouse ID | Test article | Route of administr ation | Dosing frequency & period | Dose volume |
| A | 1 | 289 | A | Tail-vein injection | Once weekly, total 2 administrations | 200μl/20g |
| B | 1 | 280 | B | Tail-vein injection | Once weekly, total 2 administrations | 200μl/20g |
| C | 1 | 281 | C | Tail-vein injection | Once weekly, total 2 administrations | 300μl/20g |
| F | 1 | 279 | F | Tail-vein injection | Once weekly, total 2 administrations | 200μl/20g |

Wherein, A is 3CpG2006-DNA carrier (50 μg) + 2AmiR21-TTA1 aptamer-DNA carrier (12 μg) + OX40 (DNA) (12 μg) + TTA1 aptamer-2Survivin siRNA-DNA carrier (12 μg);B is 3CpG2006-DNA carrier (10 μg) + 2AmiR21-TTA1 aptamer-DNA carrier (12 μg) + OX40 (DNA) (12 μg) + TTA1 aptamer-2Survivin siRNA-DNA carrier (12 μg);C is 3CpG2006-DNA carrier (100 μg) + 2AmiR21-TTA1 aptamer-DNA carrier (12 μg) + OX40 (DNA) (12 μg) + TTA1 aptamer-2*Survivin siRNA-DNA carrier (12 μg);F is epirubicin active pharmaceutical ingredient (0.12 mg/mouse).Namely, drug A is combination 19) (the above-mentioned drug 1) + the above-mentioned drug 18) + the above-mentioned drug 99)); drugs B and C are the above-mentioned drug 1) + the above-mentioned drug 18) + the above-mentioned OX40 aptamer in DNA form + the above-mentioned drug 99.

3. Measurement indices

Same as in Example 16.

4. Statistical analysis

Same as in Example 16.

V. Experimental Results:

**1. Clinical Observations of Animals**

All the experimental animals showed good activity and eating behavior during the treatment period.

**2. Changes in Animal Body Weight**

The animals showed a gradual increase in body weight during the treatment period. Group B showed the least body weight gain. The changes in animal body weight are summarized in Table 175 and Figure 41-2, with detailed body weight data shown in Table 176.

Table 175: Animal Body Weight Summary

| Groups | Animal No. | No. of animals | Before administration | D26 | Change in body weight (g) |
|---|---|---|---|---|---|
| Group A | 289 | 1 | 21.17 | 22.77 | +1.60 |
| Group B | 280 | 1 | 21.05 | 22.08 | +1.03 |
| Group C | 281 | 1 | 20.07 | 26.97 | +6.90 |
| Group F | 279 | 1 | 20.42 | 26.81 | +6.39 |

Note: D26 refers to 26 days after tumor cell injection.

Table 176: Individual Animal Body Weight Data Summary

| Group | DAY | 12 | 14 | 17 | 19 | 21 | 24 | 26 |
|---|---|---|---|---|---|---|---|---|
| A | 289 | 21.17 | 20.91 | 21.65 | 21.77 | 21.23 | 22.09 | 22.77 |
| B | 280 | 21.05 | 20.91 | 22.56 | 23.24 | 22.88 | 20.96 | 22.08 |
| C | 281 | 20.07 | 21.09 | 22.62 | 23.40 | 23.63 | 25.24 | 26.97 |

(continued)

| Group | DAY | 12 | 14 | 17 | 19 | 21 | 24 | 26 |
|---|---|---|---|---|---|---|---|---|
| F | 279 | 20.42 | 22.15 | 23.68 | 25.41 | 26.51 | 29.19 | 26.81 |

### 3. Tumor Volume Inhibition Results

All animals were closely monitored for tumor growth throughout the experiment, with tumor volume measured three times per week. Seven mice were used for each group. Tumor volume data is summarized in Table 177. Tumor growth curves are shown in Figure 41-3. The individual tumor volumes in mice are shown in Table 178 (right side) and Table 179 (left side).

Table 177: Tumor Volume Summary for Mice

| Group | DAY | 12 | 14 | 17 | 19 | 21 | 24 | 26 |
|---|---|---|---|---|---|---|---|---|
| A | 289 | 148.0 | 177.5 | 361.4 | 675.5 | 816.7 | 1337.1 | 1693.6 |
| B | 280 | 185.4 | 275.2 | 530.3 | 698.8 | 974.8 | 1224.3 | 1508.7 |
| C | 281 | 405.8 | 560.0 | 919.8 | 1271.2 | 1528.5 | 2150.8 | 2892.4 |
| F | 279 | 432.4 | 676.7 | 1271.2 | 1584.5 | 2133.5 | 3587.3 | 4177.7 |
| Note: Tables D-285 and G-288 represent the left tumor volumes of the mice, while the other animals show average right tumor volumes. | | | | | | | | |

Table 178: Individual Tumor Volume Data for Mice (Right Side)

| Group | DAY | 12 | 14 | 17 | 19 | 21 | 24 | 26 |
|---|---|---|---|---|---|---|---|---|
| A | 289 | 148.0 | 177.5 | 361.4 | 675.5 | 816.7 | 1337.1 | 1693.6 |
| B | 280 | 185.4 | 275.2 | 530.3 | 698.8 | 974.8 | 1224.3 | 1508.7 |
| C | 281 | 405.8 | 560.0 | 919.8 | 1271.2 | 1528.5 | 2150.8 | 2892.4 |
| F | 279 | 432.4 | 676.7 | 1271.2 | 1584.5 | 2133.5 | 3587.3 | 4177.7 |

Table 179: Individual Tumor Volume Data for Mice (Left Side)

| Group | DAY | 12 | 14 | 17 | 19 | 21 | 24 | 26 |
|---|---|---|---|---|---|---|---|---|
| A | 289 | 365.9 | 446.0 | 719.4 | 905.4 | 1039.6 | 1243.3 | 1785.3 |
| B | 280 | 370.4 | 450.0 | 704.7 | 822.5 | 1313.0 | 1537.0 | 1751.2 |
| C | 281 | 644.6 | 1016.5 | 1765.7 | 2079.9 | 2831.2 | 3819.1 | 4408.9 |
| F | 279 | 568.4 | 1089.2 | 1810.9 | 2531.6 | 3425.1 | 4999.6 | 5113.6 |

6. Experimental Conclusion

The primary aim of this experiment was to establish the CT26 tumor model, exploring the differences in tumor inhibition effects (drug efficacy) under different drug doses and frequencies, as well as the animals' cumulative tolerance to the drug.

During the experiment, all animals showed good activity, eating habits, and no visible skin rash, back arching, abdominal bloating, or loss of appetite. Animals exhibited a general increase in body weight during the treatment period, with the group treated with drug B showing the greatest body weight gain. Notably, the left tumor volume of mice in the B group was larger than the right side.

In comparison, group 7's tumor volume was significantly larger, with group A and B showing notably slower tumor growth than the other groups.

Experiment 42

I. Objective of the Experiment:

To verify the inhibitory effects of nanomolecular RNA carriers combined with immune modulation and base modification drugs on tumor suppression.

II . Cell Information:

CT26, mouse colon cancer cell line, purchased from Baidi Zhida (Beijing) Nanomaterials Technology Co., Ltd.

III. Experimental Animal Information:

Species: BALB/c

Body weight: 18-22 g

Sex: Male

Provided by: China National Food and Drug Testing Institute (Daxing)

IV. Experimental Methods

1. Inoculation and Grouping

The CT26 colorectal cancer model was established. The animals used were BALB/c mice, male, weighing 16-18 g. During the experiment, well-grown tumor tissues were collected, minced, and ground, and then the tumor cell suspension was prepared using sterile saline. The cell suspension was inoculated subcutaneously into the left flank of the mice (0.2 ml per mouse), and the inoculation was recorded as D0. At D1, the mice were randomly divided into seven groups: PBS, A, B, C, D, E, and F, with each group containing 8 mice. Starting from D3, the animals in each group began receiving tail vein injections, with injections administered every other day for a total of 6 doses. Details of the animal inoculation, grouping, and drug administration are shown in Tables 180 and 181.

**Table 180** Animal Inoculation and Drug Administration Plan

| Tumor cell line | No. of animals (n) | Animal IDs | Inoculation site |
|---|---|---|---|
| CT26 | 56 | See grouping table | Subcutaneous, medial aspect of the left proximal thigh (inguinal region) |
| Note: The day of inoculation was designated as D0. | | | |

Table 181. Animal Grouping and Drug Administration

| Group | No. of animals (n) | Route of administration | Dose volume per administration | No. of administrations | Dosing days |
|---|---|---|---|---|---|
| PBS | 8 | Tail-vein injection (i.v.) | 200 μL/20 g | 6 | D3, D5, D7, D9, D11, D13 |
| A | 8 | Tail-vein injection (i.v.) | 200 μL/20 g | 6 | |
| B | 8 | Tail-vein injection (i.v.) | 200 μL/20 g | 6 | |
| C | 8 | Tail-vein injection (i.v.) | 200 μL/20 g | 6 | |
| D | 8 | Tail-vein injection (i.v.) | 200 μL/20 g | 6 | |
| E | 8 | Tail-vein injection (i.v.) | 200 μL/20 g | 6 | |
| F | 8 | Tail-vein injection (i.v.) | 200 μL/20 g | 6 | |

In Table 181, under the "Treatment" section, the details for each drug are as follows:
Drug A consists of 2*CpG2006-CD40 aptamer-DNA carrier (25μg) + 2*AmiR-21-AS 1411 aptamer-DNA carrier (12μg);

Drug B consists of 2*CpG2006-CD40 aptamer-DNA carrier (25μg) + CD16a aptamer-CTLA-4 aptamer-PD-L1 aptamer-DNA carrier (24μg); Drug C consists of 2*CpG2006-CD40 aptamer-CD16a aptamer-CTLA-4 aptamer-DNA carrier (25μg) + 2*AmiR-21-AS1411 aptamer-DNA carrier (12μg); Drug D consists of CD16a aptamer-Act-12c aptamer-PD-L1-DNA carrier (24μg); Drug E consists of CD16a aptamer-CTLA-4 aptamer-PD-L1 aptamer-DNA carrier (24μg); and Drug F consists of 2*CpG2006-CD40 aptamer-CD16a aptamer-CTLA-4 aptamer-DNA carrier (25μg). Therefore, Drug A is a combination of Drug 4 and Drug 21, Drug B is a combination of Drug 4 and Drug 47, Drug C is a combination of Drug 9 and Drug 21, Drug D is Drug 48, Drug E is Drug 47, and Drug F is Drug 9.

2. Measurement indices

Same as in Example 16.

3. Statistical analysis

Same as in Example 16.

V. Experimental Results:

1. Clinical observations in experimental animals

During the dosing period, all animals exhibited good general condition (activity, food intake, etc.).

2. Changes in body weight of experimental animals

The body weight of all experimental animals increased to some degree during the treatment period. Compared to the PBS (solvent control) group, the body weight increase in Group B (tumor-bearing mice) was less, and this difference was statistically significant. The results are summarized in Table 182, and the tumor-bearing animal body weight change curve is shown in Figure 42-1.

Table 182: Animal Body Weight Summary

| Group | No. of animals | | Body weight (g) | | Change in body weight (g) |
|---|---|---|---|---|---|
| | D1 | D15 | D1 | D15 | |
| PBS | 8 | 8 | 18.0 ± 0.8 | 25.3 ± 1.4 | +7.3 |
| A | 8 | 8 | 17.9 ± 0.9 | 24.2 ± 1.3 | +6.3 |
| B | 8 | 8 | 17.8 ± 0.8 | 23.7 ± 1.2* | +5.9 |
| C | 8 | 8 | 18.3 ± 1.0 | 24.6 ± 1.3 | +6.3 |
| D | 8 | 8 | 17.9 ± 0.9 | 25.6 ± 1.2 | +7.7 |
| E | 8 | 8 | 17.9 ± 1.0 | 25.5 ± 1.2 | +7.5 |
| F | 8 | 8 | 17.6 ± 0.5 | 23.6 ± 2.1 | +6.1 |

Note: D1 indicates 1 day after inoculation with the tumor cell suspension; D15 indicates 15 days after inoculation with the tumor cell suspension.
*: $P < 0.05$, compared with the PBS (vehicle control) group.

3. Tumor Volume Inhibition Results

During the experiment, tumor growth in all animals was closely monitored with measurements taken twice per week, and tumor growth inhibition was calculated. The results are summarized in Table 183 and Figure 42-2.
The PBS (solvent control) group showed good tumor growth. Compared to the PBS (solvent control) group, A, B, C, and F groups showed tumor growth inhibition rates (based on tumor volume calculation) of 66.10%, 84.82%, 80.21%, 14.16%, 1.66%, and 74.23%, respectively. The A, B, C, and F groups all showed an inhibition rate greater than 60%.

Table 183: Tumor Growth Data Summary

| Days | PBS | | A | | | B | | | C | | |
|---|---|---|---|---|---|---|---|---|---|---|---|
| | Tumor volume (mm³) | N | Tumor volume (mm³) | N | Tumor growth inhibition rate (%) | Tumor volume (mm³) | N | Tumor growth inhibition rate (%) | Tumor volume (mm³) | N | Tumor growth inhibition rate (%) |
| D5 | 103.1±18.0 | 8 | 101.0±13.5 | 8 | 2.02 | 82.8±16.5* | 8 | 19.68 | 59.6±20.5*** | 8 | 42.22 |
| D8 | 269.3±64.2 | 8 | 177.2±41.5** | 8 | 34.19 | 147.8±54.4** | 8 | 45.12 | 122.6±33.5*** | 8 | 54.45 |
| D12 | 1319.4±312.3 | 8 | 485.9±176.2*** | 8 | 63.17 | 253.1±130.3*** | 8 | 80.82 | 301.5±115.2*** | 8 | 77.15 |
| D15 | 1910.7±313.1 | 8 | 647.7±315.1*** | 8 | 66.10 | 290.0±160.6*** | 8 | 84.82 | 378.2±231.6*** | 8 | 80.21 |

| Days | D | | | E | | | F | | |
|---|---|---|---|---|---|---|---|---|---|
| | Tumor volume ($mm^3$) | N | Tumor growth inhibition rate (%) | Tumor volume ($mm^3$) | N | Tumor growth inhibition rate (%) | Tumor volume ($mm^3$) | N | Tumor growth inhibition rate (%) |
| D5 | 98.6±19.2 | 8 | 4.38 | 102.4±18.5 | 8 | 0.65 | 100.4±12.5 | 8 | 2.59 |
| D8 | 229.6±35.2 | 8 | 14.75 | 227.7±98.2 | 8 | 15.43 | 196.6±70.4* | 8 | 26.99 |
| D12 | 1086.3±211.4 | 8 | 17.67 | 1064.4±327.0 | 8 | 19.32 | 361.7±169.7*** | 8 | 72.59 |
| D15 | 1640.2±504.1 | 8 | 14.16 | 1879.0±496.3 | 8 | 1.66 | 492.4±263.8*** | 8 | 74.23 |
| *Note:* Days: days after inoculation of the tumor cell suspension; N: number of animals in the group included in the statistical analysis. <br> *: $P<0.05$, **: $P<0.01$, ***: $P<0.001$, compared with the PBS (vehicle control) group. | | | | | | | | | |

4. Tumor Weight

Fifteen days after CT26 inoculation, all animals were euthanized by cervical dislocation, and the tumors were excised and weighed. The results are summarized in Table 184 and Figure 42-3.

Based on tumor weight calculations, compared to the PBS (solvent control) group, A-F groups showed tumor growth inhibition rates of 65.33%, 83.13%, 79.03%, 15.01%, 7.72%, and 75.74%, respectively. Groups A, B, C, and F all showed an inhibition rate greater than 60%.

Table 184: Tumor Weight Summary

| Group | No. of animals (n) | Tumor weight (g) | IR_TW (%) |
|---|---|---|---|
| PBS | 8 | 2.28 ± 0.52 | \ |
| A | 8 | 0.79 ± 0.42*** | 65.33 |
| B | 8 | 0.39 ± 0.26*** | 83.13 |
| C | 8 | 0.48 ± 0.25*** | 79.03 |
| D | 8 | 1.94 ± 0.47 | 15.01 |
| E | 8 | 2.11 ± 0.63 | 7.72 |
| F | 8 | 0.55 ± 0.32** | 75.74 |
| ***: $P < 0.001$, compared with the PBS (vehicle control) group. | | | |

5. Tumor and Lymph Node Size Results

Fifteen days after CT26 inoculation, all animals were euthanized by cervical dislocation, and the tumors were excised and weighed. The results are summarized in Table 185.

The average lymph node size of the PBS (solvent control) group was 123.88. The average lymph node sizes for A-F groups (tail vein injection) were 206.72, 214.90, 203.72, 129.11, 130.82, and 192.49, respectively. Compared to the PBS (solvent control) group, A, B, C, and F groups showed significantly higher average lymph node sizes, with statistically significant differences. The average lymph node sizes for A-F groups (tail vein injection) were 22.84, 27.12, 21.70, 23.75, 17.76, 20.50, and B group was significantly higher than the PBS (solvent control) group at 18.48, with statistically significant differences.

Table 185: Lymph Node Size and Lymph Node Index Summary

| Group | No. of animals | Spleen index | Thymus index |
|---|---|---|---|
| PBS | 8 | 123.88 ± 12.06 | 18.48 ± 7.13 |
| A | 8 | 206.72 ± 11.34*** | 22.84 ± 5.45 |
| B | 8 | 214.90 ± 17.12*** | 27.12 ± 5.14* |

(continued)

| Group | No. of animals | Spleen index | Thymus index |
|-------|----------------|--------------|--------------|
| C | 8 | 203.72 ± 10.49*** | 21.70 ± 5.34 |
| D | 8 | 129.11 ± 17.39 | 23.75 ± 6.11 |
| E | 8 | 130.82 ± 24.22 | 17.76 ± 4.83 |
| F | 8 | 192.49 ± 22.75*** | 20.50 ± 5.75 |

*: $P < 0.05$; $P < 0.001$; comparisons vs. PBS (vehicle control) group.

VI, Experimental Conclusion

This experiment investigates the inhibitory effects of A-F drugs on tumor growth and tumor weight in CT26 mouse colon cancer model. The experimental conclusion is as follows:
Tail vein injection of A-F drugs, at the conclusion of the experiment (D15), showed that the tumor growth inhibition rate (based on tumor weight and tumor volume calculations) exceeded 60%. This indicates that A, B, C, and F drugs had a significant effect on inhibiting the growth of CT26 mouse colon tumors. At the conclusion of the experiment (D15), tail vein injection of B drug clearly led to an increase in the lymph node index of the tumor-bearing mice, while tail vein injection of A, B, C, and F drugs demonstrated significantly higher lymph node index values in comparison to the PBS (solvent control) group.

Experiment Example 43

I , Objective of the Experiment

To verify the antitumor effects of nucleic acid-loaded carriers in combination with immune modulation and targeted gene therapy.

II, Cell Information

CT26 (mouse colon cancer) cell line, purchased from Baidu Zhida (Beijing) Nucleic Acid Technology Co., Ltd.

III, Animal Information

Species: BALB/c

Body weight: 16 - 18 g

Sex: female

Supplier: China National Food and Drug Inspection Research Institute (Daxing)

IV. Experimental Methods

1. Inoculation and Grouping

The tumor model used is the CT26 model, with male BALB/c mice, aged 16-18 g. The mice were obtained from a reputable supplier of laboratory animals and housed in suitable conditions. The CT26 tumor cell suspension was prepared by grinding and grinding tumor tissue following a saline immersion step to isolate the tumor cells, which were then injected subcutaneously into the left thigh of the mouse (0.2 ml/mouse). The inoculation was recorded as Day 0 (D0). At D1, the animals were randomly divided into groups: PBS, A, B, C, D, and E groups, with 8 animals per group. Starting on D3, each group received intravenous injections of drugs, for a total of 5 doses. Details of the inoculation, grouping, and drug administration are provided in Tables 186 and 187.

Table 186: Animal Inoculation and Drug Administration Scheme

| Tumor cell line | No. of mice (n) | Mouse IDs | Inoculation site |
|---|---|---|---|
| CT26 | 48 | See grouping table | Subcutaneous, medial aspect of the left proximal thigh (inguinal region) |
| **Note:** The day of inoculation was designated as **D0.** | | | |

Table 187: Animal Grouping and Drug Administration Details

| Group | No. of animals | Route of administration | Dose volume per administration | No. of administrations | Dosing days |
|---|---|---|---|---|---|
| PBS | 8 | Tail-vein injection (i.v.) | 0.2 mL/20 g | 5 | D3, D5, D7, D9, D11 |
| A | 8 | Tail-vein injection (i.v.) | 0.2 mL/20 g | 5 | |
| B | 8 | Tail-vein injection (i.v.) | 0.2 mL/20 g | 5 | |
| C | 8 | Tail-vein injection (i.v.) | 0.2 mL/20 g | 5 | |
| D | 8 | Tail-vein injection (i.v.) | 0.2 mL/20 g | 5 | |
| E | 8 | Tail-vein injection (i.v.) | 0.2 mL/20 g | 5 | |

In Table 187, Drug A is 2*CpG2006-CD40 aptamer-DNA carrier (30 µg); Drug B is 2*CpG2006-CD40 aptamer-DNA carrier (25 µg) + CD16a aptamer-CTLA-4 aptamer-PD-L1 aptamer-DNA carrier (24 µg); Drug C is 2*CpG2006-CD40 aptamer-CD16a aptamer-CTLA-4 aptamer-DNA carrier (25 µg); Drug D is CpG2006-CD40 aptamer-PD-L1 aptamer-DNA carrier (25 µg); Drug E is CD16a aptamer-CD40 aptamer-CpG2006-DNA carrier (25 µg). Drug A corresponds to Drug 4, Drug B corresponds to Drug 4 + Drug 47, Drug C corresponds to Drug 9, and Drug D corresponds to Drug 11.

2. Testing Indicators

Same as in Example 16.

3. Statistical Analysis

Same as in Example 16.

V, Experimental Results:

1. Experimental Animal Clinical Observation Results

All experimental animals showed normal activity and feeding during the drug administration period, and their general condition was generally good.

2. Experimental Animal Weight Changes

During the drug administration period, all experimental animals showed a certain degree of weight gain. Compared to the PBS (solvent control) group, the D group of tumor-bearing mice showed smaller weight increases, and the difference was statistically significant. The results are recorded in Table 188, and the tumor-bearing animal weight growth curve is shown in Figure 43-1.

Table 188: Summary of Animal Body Weight

| Group | No. of animals | | Body weight (g) | | Change in body weight (g) |
|---|---|---|---|---|---|
| | D1 | D13 | D1 | D13 | |
| PBS | 8 | 8 | 17.0 ± 0.7 | 21.2 ± 1.3 | +4.2 |
| A | 8 | 8 | 17.2 ± 0.5 | 21.5 ± 1.1 | +4.2 |

(continued)

| Group | No. of animals | | Body weight (g) | | Change in body weight (g) |
|---|---|---|---|---|---|
| | D1 | D13 | D1 | D13 | |
| B | 8 | 8 | 17.3 ± 0.4 | 21.0 ± 0.5 | +3.7 |
| C | 8 | 8 | 17.3 ± 0.6 | 20.5 ± 0.8 | +3.2 |
| D | 8 | 8 | 17.1 ± 0.3 | 19.1 ± 0.7** | +1.9 |
| E | 8 | 8 | 17.2 ± 0.4 | 20.2 ± 0.7 | +3.1 |

Note: D1 indicates 1 day after inoculation with the tumor cell suspension; D13 indicates 13 days after inoculation with the tumor cell suspension.
**: P<0.01, compared with the PBS (vehicle control) group.

3. Tumor Growth Inhibition Results

During the experiment, the tumor growth status of all animals was closely monitored, with measurements taken twice a week to record and calculate the tumor growth inhibition rate. The results are summarized in Table 189 and Figure 43-2.

Compared to the PBS (solvent control) group, the PBS group showed good tumor growth. In comparison to the PBS (solvent control) group, the tumor growth inhibition rates for Groups A-E on D13 were 44.47%, 55.94%, 44.36%, 80.13%, 51.13%, and the D group was greater than 60%, based on tumor volume calculations.

Table 189: Tumor Growth Data Summary

| Days | PBS | | A | | | B | | |
|---|---|---|---|---|---|---|---|---|
| | Tumor volume (mm$^3$) | N | Tumor volume (mm$^3$) | N | Tumor growth inhibition rate (%) | Tumor volume (mm$^3$) | N | Tumor growth inhibition rate (%) |
| D4 | 119.4±19.1 | 8 | 106.8±29.7 | 8 | 10.57 | 106.9±29.1 | 8 | 10.50 |
| D7 | 300.1±71.9 | 8 | 277.2±85.8 | 8 | 7.66 | 271.8±56.1 | 8 | 9.44 |
| D11 | 1060.6±245.0 | 8 | 691.8±201.9** | 8 | 34.77 | 503.3±128.3*** | 8 | 52.54 |
| D13 | 1671.1±362.2 | 8 | 927.9±199.6*** | 8 | 44.47 | 736.2±205.3*** | 8 | 55.94 |

| Days | C | | | D | | | E | | |
|---|---|---|---|---|---|---|---|---|---|
| | Tumor volume (mm$^3$) | N | Tumor growth inhibition rate (%) | Tumor volume (mm$^3$) | N | Tumor growth inhibition rate (%) | Tumor volume (mm$^3$) | N | Tumor growth inhibition rate (%) |
| D4 | 129.5±21.8 | 8 | -8.40 | 123.9±23.9 | 8 | -3.73 | 109.0±16.1 | 8 | 8.75 |
| D7 | 234.4±73.2 | 8 | 21.91 | 253.2±67.1 | 8 | 15.63 | 288.1±75.9 | 8 | 4.00 |
| D11 | 563.2±214.1*** | 8 | 46.90 | 302.3±56.5*** | 8 | 71.50 | 532.4±223.2*** | 8 | 49.80 |
| D13 | 929.7±441.3** | 8 | 44.36 | 332.0±90.7*** | 8 | 80.13 | 816.7±553.0** | 8 | 51.13 |

**Note:** Days: days after inoculation of the tumor cell suspension; N: number of animals in the group included in the statistical analysis.
**: P<0.01, ***: P<0.001, compared with the PBS (vehicle control) group.

4. Tumor Weight

After 13 days of CT26 inoculation, the animals were euthanized following blood collection and cervical dislocation. The tumors were then excised and weighed. The results are summarized in Table 190 and Figure 43-3.

Based on tumor weight calculations, compared to the PBS (solvent control) group, Groups A-E showed tumor growth inhibition rates of 45.44%, 48.94%, 44.13%, 79.40%, 49.06%, and the D group showed a tumor inhibition rate greater than 60%.

Table 190: Tumor Weight Summary

| Group | No. of animals | Tumor weight (g) | IR_TW (%) |
|---|---|---|---|
| PBS | 8 | 2.00 ± 0.38 | \ |
| A | 8 | 1.09 ± 0.27*** | 45.44 |
| B | 8 | 1.02 ± 0.25*** | 48.94 |
| C | 8 | 1.12 ± 0.43** | 44.13 |
| D | 8 | 0.41 ± 0.17*** | 79.40 |
| E | 8 | 1.02 ± 0.53** | 49.06 |

*: $P < 0.05$; $P < 0.01$; $P < 0.001$; all comparisons are vs. PBS (vehicle control) group.

5. Tumor Size and Spleen Index Results

After 13 days of CT26 inoculation, blood was collected and all animals were euthanized via cervical dislocation, followed by the removal of tumors and spleens. The results are summarized in Table 191 and Table 192.

For the PBS (solvent control) group, the average tumor size in tumor-bearing mice was 104.83. For the PBS (solvent control) group, the average spleen index was 222.23, 196.76, 215.67, 174.08, and 195.27 for groups A-E, with statistical significance compared to the PBS (solvent control) group. The average spleen index of the A-E drug-treated groups was significantly higher than the PBS (solvent control) group, with statistical significance. The average spleen index for group A was 21.41, group B was 17.98, group C was 15.40, group D was 8.13, and group E was 15.87, while group D showed the highest spleen index compared to the PBS (solvent control) group, and the difference was statistically significant.

Table 191: Tumor Size and Spleen Index Summary

| Group | No. of animals | Spleen index (mg/10 g body weight) | Thymus index (mg/10 g body weight) |
|---|---|---|---|
| PBS | 8 | 104.83 ± 14.98 | 16.43 ± 5.05 |
| A | 8 | 222.23 ± 13.91*** | 21.41 ± 4.11* |
| B | 8 | 196.76 ± 10.98*** | 17.98 ± 3.85 |
| C | 8 | 215.67 ± 12.78*** | 15.40 ± 4.57 |
| D | 8 | 174.08 ± 5.89*** | 8.13 ± 3.33** |
| E | 8 | 195.27 ± 17.31*** | 15.87 ± 5.04 |

*: $P < 0.05$; $P < 0.01$; $P < 0.001$; all comparisons are vs. PBS (vehicle control) group.

Table 192. Summary of organ indices

| Group | No. of animals | Liver index (g/100 g body weight) | Kidney index (g/100 g body weight) | Heart index (g/100 g body weight) | Lung index (g/100 g body weight) |
|---|---|---|---|---|---|
| PBS | 8 | 6.13 ± 0.45 | 1.55 ± 0.11 | 0.48 ± 0.06 | 0.61 ± 0.05 |
| D | 8 | 7.18 ± 0.41*** | 1.65 ± 0.13 | 0.44 ± 0.04 | 0.66 ± 0.02* |
| E | 8 | 7.40 ± 0.49*** | 1.65 ± 0.18 | 0.47 ± 0.04 | 0.66 ± 0.06 |

*: $P < 0.05$; $P < 0.01$; $P < 0.001$; comparisons are vs PBS (vehicle control) group.

6. After 13 days of CT26 inoculation, blood was collected from the orbital sinus, and routine blood tests and biochemical assays were conducted. The results are as follows:

Table 193

| Group | No. of animals | Peripheral blood cell counts | | | |
|---|---|---|---|---|---|
| | | WBC (×10⁹/L) | LYM% (%) | MON% (%) | NEUT% (%) |
| PBS | 8 | 11.84 ± 3.12 | 15.13 ± 14.92 | 1.10 ± 1.48 | 83.78 ± 16.39 |
| A | 8 | 8.71 ± 1.95* | 24.03 ± 9.6 | 2.11 ± 1.48 | 73.86 ± 10.64 |
| B | 8 | 7.88 ± 1.81** | 16.85 ± 5.94 | 1.76 ± 0.68 | 81.39 ± 6.46 |
| C | 8 | 9.21 ± 1.40* | 17.58 ± 8.41 | 1.69 ± 1.96 | 80.74 ± 10.24 |
| D | 8 | 11.18 ± 2.19 | 14.21 ± 5.20 | 0.91 ± 1.18 | 84.88 ± 6.29 |
| E | 8 | 10.08 ± 2.17 | 18.95 ± 5.89 | 1.75 ± 1.30 | 79.30 ± 6.75 |

*: P<0.05, **: P<0.01, compared with the PBS (vehicle control) group.
WBC: white blood cell count
LYM%: percentage of lymphocytes
MON%: percentage of monocytes
NEUT%: percentage of neutrophils

Table 194

| Group | No. of animals | Peripheral blood erythrocyte indices | | | | | | |
|---|---|---|---|---|---|---|---|---|
| | | RBC (×10¹²/L) | HGB (g/L) | HCT (L/L) | MCV (fL) | MCH (pg) | MCHC (g/L) | RDW (fL) |
| PBS | 8 | 8.22 ± 0.44 | 134.13 ± 12.47 | 39.46 ± 1.68 | 48.06 ± 0.69 | 16.35 ± 1.39 | 340.13 ± 29.93 | 17.88 ± 0.70 |
| A | 8 | 7.79 ± 0.31* | 115.50 ± 5.15** | 36.84 ± 1.8** | 47.24 ± 0.64* | 14.83 ± 0.43* | 313.75 ± 10.15* | 16.96 ± 1.09 |
| B | 8 | 7.00 ± 0.45*** | 108.88 ± 7.06*** | 32.90 ± 2.17*** | 46.99 ± 0.29** | 15.56 ± 0.39 | 330.88 ± 8.56 | 17.50 ± 0.68 |
| C | 8 | 7.33 ± 0.39*** | 110.63 ± 5.10*** | 34.81 ± 2.50*** | 47.48 ± 1.01 | 15.11 ± 0.26* | 318.25 ± 9.94 | 17.09 ± 0.95 |
| D | 8 | 7.30 ± 0.37*** | 111.13 ± 4.7*** | 34.90 ± 1.71*** | 47.83 ± 0.85 | 15.23 ± 0.45* | 319.00 + 11.76 | 18.13 ± 0.10 |
| E | 8 | 7.31 ± 0.36*** | 110.25 ± 4.74*** | 35.63 ± 2.75** | 48.70 ± 2.05 | 15.08 ± 0.32* | 310.38 ± 17.12* | 17.11 ± 0.49* |

*: P<0.05, **: P<0.01, ***: P<0.001, compared with the PBS (vehicle control) group.
RBC: red blood cell count
HGB: hemoglobin concentration
HCT: hematocrit
MCV: mean corpuscular volume
MCH: mean corpuscular hemoglobin
MCHC: mean corpuscular hemoglobin concentration
RDW: red cell distribution width

Table 195

| Group | No. of animals | Peripheral blood platelet parameters | | | |
|---|---|---|---|---|---|
| | | PLT (×10⁹/L) | PCT (L/L) | MPV (fl) | PDW (fl) |
| PBS | 8 | 503.00 ± 23.89 | 0.26 ± 0.01 | 5.13 ± 0.21 | 12.55 ± 0.82 |
| A | 8 | 398.00 ± 47.84*** | 0.21 ± 0.03*** | 5.16 ± 0.14 | 12.23 ± 0.51 |
| B | 8 | 468.38 ± 79.33 | 0.25 ± 0.04 | 5.31 ± 0.15 | 12.49 ± 0.71 |
| C | 8 | 436.88 ± 50.9** | 0.23 ± 0.03* | 5.26 ± 0.09 | 12.40 ± 0.31 |

(continued)

| Group | No. of animals | Peripheral blood platelet parameters | | | |
|---|---|---|---|---|---|
| | | PLT ($\times 10^9$/L) | PCT (L/L) | MPV (fl) | PDW (fl) |
| D | 8 | 411.50 ± 67.15** | 0.22 ± 0.03** | 5.29 ± 0.22 | 12.44 ± 0.56 |
| E | 8 | 453.63 ± 43.83* | 0.25 ± 0.02 | 5.53 ± 0.29** | 13.03 ± 0.65 |

*: P<0.05, **: P<0.01, ***: P<0.001, compared with the PBS (vehicle control) group.
PLT: platelet count
PCT: plateletcrit
MPV: mean platelet volume
PDW: platelet distribution width

Table 196

| Group | No.of animals | ALT (U/L) | AST (U/L) | TP (g/L) | ALB (g/L) | UREA (mmol/L) | CREA ($\mu$mol/L) |
|---|---|---|---|---|---|---|---|
| PBS | 8 | 18.00 ± 3.55 | 158.63 ± 53.69 | 68.25 ± 1.28 | 16.00 ± 0.76 | 4.49 ± 1.79 | 89.75 ± 24.66 |
| D | 8 | 24.38 ± 3.70* | 92.50 ± 6.59** | 66.63 ± 2.26 | 14.88 ± 0.64* | 6.18 ± 0.51* | 58.00 ± 10.23* |
| E | 8 | 25.88 ± 3.27** | 122.38 ± 55.05 | 66.50 ± 2.33 | 14.88 ± 0.83* | 5.29 ± 0.60 | 49.38 ± 11.44** |

*: $P < 0.05$; $P < 0.01$; *$P < 0.001$; all comparisons are vs. PBS (vehicle control).
ALT: alanine aminotransferase; AST: aspartate aminotransferase; TP: total protein; ALB: albumin; UREA: urea nitrogen; CREA: creatinine.

VI, Experimental Conclusion

Considering the tumor growth inhibition rate and the tumor weight inhibition rate, the experimental conclusions are as follows:

For the tail vein injection of Drugs A-E, at the end of the experiment (D13), the tumor growth inhibition rate (calculated based on tumor weight and tumor volume) exceeded 60%, indicating that Drug D had the most significant growth inhibitory effect on CT26 tumor-bearing mice. At the conclusion of the experiment (D13), the tail vein injection of Drugs A-E showed a marked increase in the spleen index in tumor-bearing mice. The tail vein injection of Drug A showed a significant increase in the spleen index of tumor-bearing mice, while the tail vein injection of Drug D resulted in a significant decrease in the spleen index of tumor-bearing mice. Tail vein injection of Drugs D and E showed varying degrees of effect on the main blood and major organ indices in tumor-bearing mice.

Example 44

( I ) I , Objective of the Experiment:

Evaluate the efficacy of CCPD3 and its combinations with different molecular components in the tumor model of MC38 subcutaneous transplantation in mice.

II , Cell Information

MC38 cells were purchased from Zhejiang Mingfu Biotechnology Co., Ltd.

III, Experimental Animal Information:

Species: C57BL/6

Grade: SPF (Specific Pathogen Free)

Sex: Male

Quantity: 170 mice

Age/Months: 6-8 weeks

Weight: 18-22g

Source: StemBiotech (Beijing) Biotechnology Co., Ltd.

IV, Experimental Methods

1. Inoculation and Grouping

MC38 cells were cultured in DMEM with 10% fetal bovine serum (including 1% NEAA, 1% sodium pyruvate, 10mM HEPES), 37°C, 5% $CO_2$ for cell culture. The cells were passaged every 3 to 4 days after reaching confluence, and the tumor cells were prepared for the subcutaneous inoculation of the tumor in mice.

Tumor-bearing MC38 cells were resuspended in PBS at a concentration of $4 \times 10^7$ cells/mL, and inoculated subcutaneously into the right flank of experimental animals at a volume of 50 μL. When the tumor size reached approximately 50-60 mm$^3$, the experimental drugs were administered. A total of 18 groups were established, with 8 animals per group, as per the experimental design.

**Table 197: Experimental Groups**

| Group | Treatment | Number of Animals (n) | Dose (mg/kg) | Administration Route | Dosing Frequency |
|---|---|---|---|---|---|
| G1 | Vehicle(PBS) | 8 | -- | i.v. | god×8-10 |
| G2 | CCPD3 High Dose | 8 | 22.56 | i.v. | god×8-10 |
| G3 | CCPD3 Medium Dose | 8 | 11.28 | i.v. | god×8-10 |
| G4 | CCPD3 Low Dose | 8 | 5.64 | i.v. | god×8-10 |
| G5 | CPG2006 | 8 | 2.80 | i.v. | god×8-10 |
| G6 | CD40 Aptamer | 8 | 2.83 | i.v. | god×8-10 |
| G7 | PD-L1Aptamer | 8 | 5.01 | i.v. | god×8-10 |
| G8 | CPG2006-DNA Carrier | 8 | 13.54 | i.v. | god×8-10 |
| G9 | CD40 Aptamer-DNA Carrier | 8 | 13.57 | i.v. | god×8-10 |
| G10 | PD-L1 Aptamer - DNA Carrier | 8 | 15.75 | i.v. | god×8-10 |
| G11 | CPG2006-CD40 Aptamer - DNA Carrier | 8 | 16.96 | i.v. | god×8-10 |
| G12 | CPG2006-PD-L1 Aptamer - DNA Carrier | 8 | 19.13 | i.v. | god×8-10 |
| G13 | CD40 Aptamer -PD-L1 Aptamer - DNA Carrier | 8 | 19.17 | i.v. | god×8-10 |
| G14 | DNA Carrier | 8 | 10.15 | i.v. | god×8-10 |

(continued)

| Group | Treatment | Number of Animals (n) | Dose (mg/kg) | Administration Route | Dosing Frequency |
|---|---|---|---|---|---|
| G15 | mPD-L1 | 8 | 5.00 | i.p. | god×8-10 |
| G16 | CCPD3(P2) | 8 | 11.29 | i.v. | god×8-10 |
| G17 | CCPD3(P3) | 8 | 11.01 | i.v. | god×8-10 |
| G18 | CCPD3(P4) | 8 | 10.66 | i.v. | god×8-10 |

Note: The dosing amount is calculated based on the anhydrous free base, and the administration volume is calculated according to the animal's body weight at 10 $\mu$L/g. If there is a weight loss of 15-20%, the dosing amount can be adjusted. Dosing will resume once the animal's weight recovers to within 95% of its baseline. i.v.: (Tail) intravenous injection; i.p.: intraperitoneal injection; qod×8: administration once every other day, for a total of 8 doses.

2. Endpoints

Same as in Example 16.

3. Statistical analysis

Same as in Example 16.

V, Experimental results

1. Clinical observations in animals

All animals exhibited good general condition (activity, food intake, etc.) during the dosing period.

2. Tumor-volume inhibition

Throughout the study, tumor growth was closely monitored; tumor volumes were measured twice weekly, the results were recorded, and the tumor growth inhibition (TGI) rate was calculated. The results are summarized in Figure 44-1 (tumor growth curves of the MC38 subcutaneous xenograft model after initiation of treatment; "Group" corresponds to the group abbreviation G used in Table 197).

3. Changes in body weight

Body-weight change curves of animals in the MC38 subcutaneous xenograft model after initiation of treatment are shown in Figure 44-2.

VI, Study conclusions

1. At the time of the last dosing (D22) and on the day dosing was discontinued (D23), the test article CCPD3 produced a significant antitumor effect in the high- (22.56 mg/kg), medium- (11.28 mg/kg), and low-dose (5.64 mg/kg) groups, with no significant differences among the three groups. However, on Day 7 after dosing cessation (D29), the tumor volume in the high-dose (22.56 mg/kg) group was significantly lower than that in the low-dose (5.64 mg/kg) group, indicating a dose-response relationship.

2. The antitumor effect of the high-dose CCPD3 (22.56 mg/kg) group was superior to that of the positive-control mPD-L1 (5 mg/kg) group.

3. The antitumor effect of the high-dose CCPD3 (22.56 mg/kg) group was superior to that of the groups containing molar-equivalent amounts of the single or dual components CD40 aptamer and/or PD-L1 aptamer-namely, the CD40 aptamer (2.83 mg/kg) group, PD-L1 aptamer (5.01 mg/kg) group, CD40 aptamer-DNA3 (13.57 mg/kg) group, PD-L1 aptamer-DNA3 (15.75 mg/kg) group, and CD40 aptamer/PD-L1 aptamer-DNA3 (19.17 mg/kg) group-and was also superior to the DNA3 (10.15 mg/kg) group.

4. At the last dosing (D22), both the high-dose CCPD3 (22.56 mg/kg) group and the groups containing molar-equivalent amounts of CPG2006 components (CPG2006 (2.8 mg/kg), CPG2006-DNA3 (13.54 mg/kg), CPG2006/CD40 aptamer-DNA3 (16.96 mg/kg), CPG2006/PD-L1 aptamer-DNA3 (19.13 mg/kg)) showed significant antitumor activity. However, on Day 13 after dosing cessation (D35), the sustained antitumor effect of the high-dose CCPD3 (22.56 mg/kg) group was superior to that of all groups containing molar-equivalent CPG2006 component(s) listed above.

5. At the last dosing (D22) and on the day dosing was discontinued (D23), the high-dose CCPD3 (22.56 mg/kg) group, CCPD3(ct) (11.29 mg/kg) group, CCPD3(HD) (11.01 mg/kg) group, and CCPD3(XD) (10.66 mg/kg) group each exhibited significant antitumor activity, with no significant differences among the four groups. On Day 7 after dosing cessation (D29), the antitumor effect of the high-dose CCPD3 (22.56 mg/kg) group was superior to that of the CCPD3(XD) (10.66 mg/kg) group; on Day 13 (D35), it was superior to those of the CCPD3(ct) (11.29 mg/kg) and CCPD3(HD) (11.01 mg/kg) groups.

6. In summary, the test article CCPD3 demonstrated a dose-response relationship; the high-dose (22.56 mg/kg) group showed a significant antitumor effect and the best durability of tumor suppression.

7. Tumor-bearing mice exhibited good tolerability to all test articles at the doses used in this study.

(11) Determination by flow cytometry of the binding capacity of CCPD3-Bio to PBMCs from different species.

1. **Experimental grouping**

CCPD3-Cy5 at different concentrations was co-incubated with PBMCs (peripheral blood mononuclear cells) from various species. Flow cytometry was used to determine the binding capacity of the test article to PBMCs. Details are shown below:

Table198

| Cell type | Test article name | Test-article concentration (nM) |
|---|---|---|
| Human PBMCs | BYZD-CCPD3-Cy5 | |
| Mouse PBMCs | BYZD-CCPD3-Cy5 | 500, 200, 100, 0 |
| Rat PBMCs | BYZD-CCPD3-Cy5 | |
| Canine PBMCs | BYZD-CCPD3-Cy5 | |
| Monkey PBMCs | BYZD-CCPD3-Cy5 | |

**2. Principal reagents**

Table 199

| Name | Brand | Catalog No. |
|---|---|---|
| Human PBMC | Aoneng Biology | PB003F-C |
| Mouse PBMC | Aoneng Biology | MOSPB-002F-C |
| Rat PBMC | Aoneng Biology | RPB-001F |
| Canine PBMC | Aoneng Biology | DPB-002F |
| Cynomolgus PBMC | Aoneng Biology | NHP-PB002F |
| RPMI medium 1640 basic | Gibco | C11875500BT |
| FBS | Gibco | 10091-148 |

(continued)

| Name | Brand | Catalog No. |
|------|-------|-------------|
| 7-AAD | Biolegend | 420404 |

### 3. Test methods

1) Thaw (resuscitate) PBMCs and determine the cell count.

2) Dispense PBMCs into six tubes ($1 \times 10^6$ cells per tube). Centrifuge at $200 \times$ g for 15 min, discard the supernatant, and resuspend the cells in 100 $\mu$L PBS. Add 5 $\mu$L, 2 $\mu$L, 1 $\mu$L, or 0 $\mu$L of 10 $\mu$M CCPD3-Cy5 solution to the respective tubes and mix thoroughly to obtain final CCPD3-Cy5 concentrations of 500 nM, 200 nM, 100 nM, and 0 nM; 0 nM served as the control group.

3) Incubate 30 min at room temperature protected from light.

4) After incubation, add 1 mL PBS to wash the cells, centrifuge at $800 \times$ g for 5 min, and discard the supernatant. Wash once more with 1 mL PBS and centrifuge again at $800 \times$ g for 5 min.

5) Discard the supernatant and resuspend the cells in 100 $\mu$L PBS. Five minutes before flow-cytometric acquisition, add 5 $\mu$L 7-AAD and mix. Acquire the CCPD3-Cy5 signal in the APC channel and the 7-AAD signal in the PerCP channel.

### 4. Data processing and analysis

Flow-cytometry data were analyzed and plotted using FlowJo software. After excluding non-viable cells based on 7-AAD, the percentage of CCPD3-Cy5-positive PBMCs was determined.

### 5. Experimental results

Among monocytes from different species, CCPD3-Cy5 showed the highest binding percentage to human monocytes, with the highest mean fluorescence intensity (MFI). The binding percentage and MFI for monkey monocytes were second only to human (see Figure 44-3A, 44-3B). Among lymphocytes from different species, human lymphocytes exhibited the lowest binding percentage and the lowest MFI for CCPD3-Cy5. The binding percentage and MFI for monkey lymphocytes were slightly higher than those of human (see Figure 44-3C, 44-3D).

Figure 44-3. (A) Binding percentage of CCPD3-Cy5 at 0, 100 nM, 200 nM, and 500 nM to monocytes from different species. (B) Mean fluorescence intensity of CCPD3-Cy5 binding to monocytes from different species at 0, 100 nM, 200 nM, and 500 nM. (C) Binding percentage of CCPD3-Cy5 at 0, 100 nM, 200 nM, and 500 nM to lymphocytes from different species. (D) Mean fluorescence intensity of CCPD3-Cy5 binding to lymphocytes from different species at 0, 100 nM, 200 nM, and 500 nM.

### 6. Experimental conclusions

(1) Cy5-labeled CCPD3 exhibited binding capability to PBMCs from human, monkey, dog, mouse, and rat from this lot.

(2) The binding capability of Cy5-labeled CCPD3 to monkey PBMCs in this lot was most similar to that observed for human PBMCs in this lot.

(III)
In-vivo efficacy study of CCPD3 in the mouse glioma GL261-Luc orthotopic tumor model:

Cell culture

GL261-Luc cells were purchased from Ningbo Mingzhou Biotechnology Co., Ltd. Species authentication confirmed a murine cell line, with no contamination by rat or human cells. Cells were cultured in DMEM supplemented with 10% fetal bovine serum (FBS), 1% L-glutamine, and 1% HEPES at 37 °C in a 5% $CO_2$ humidified incubator. Cells were subcultured every 3-4 days upon reaching confluence, and log-phase tumor cells were used for in-vivo orthotopic tumor inoculation.

Experimental animals

Species: C57BL/6

Health status/grade: SPF

Age: 6-8 weeks

Sex: female

Body weight: 18-22 g

Animal housing site: Animals were housed at Youji (Tianjin) Pharmaceutical Technology Co., Ltd. in an IVC barrier system. Upon receipt, animals were quarantined/acclimated for at least 3 days; only animals that passed the health inspection were enrolled in the formal study. Throughout the experiment, the animal room was maintained at 20.5-24.5 °C, 40-70% relative humidity, with a 12-h light/12-h dark cycle. Three to five animals per cage were housed, with ad libitum access to water and feed.

Experimental methods

1, Inoculation and randomization: GL261-Luc cells were resuspended in PBS at $1 \times 10^9$ cells/mL and inoculated by orthotopic intracranial injection, 5 $\mu$L per mouse. Seven (7) days after tumor-cell inoculation, animals were randomized to treatment, 2 groups, n = 6 per group. The dosing scheme was as follows:

2, Dosing scheme:

Table 200

| Group | No. of animals | Test article | Dose per administration | Route | Dosing schedule |
|---|---|---|---|---|---|
| 1 | 6 | Physiological saline (vehicle) | -- | Tail-vein i.v. | Once every other day (QOD) |
| 2 | 6 | CCPD3 (also BYZD-CCPD3) | 200 $\mu$g/mouse | Tail-vein i.v. | Once every other day (QOD) |

Assessment endpoints

(1) After randomization, each group of mice was examined with a small-animal in-vivo imaging system and BLI (bioluminescence imaging) values were measured. Body weight was recorded twice weekly to assess its relationship to dosing time. Survival and general health (e.g., activity, food intake) were monitored throughout dosing.

(2) Mice were checked daily. Animals that became moribund were euthanized, and survival time was recorded; observation continued for up to 138 days post tumor-cell inoculation. At study end, the median survival time (MST) for each group was calculated. The increase in life span (ILS%) for the treatment group was calculated as:

$$ILS\% = (MST\_treatment\ /\ MST\_vehicle - 1) \times 100\%.$$

(3) For each group, MST and ILS% were calculated and analyzed statistically; p < 0.05 was considered significant. BLI value curves were plotted for each group and analyzed statistically; p < 0.05 was considered significant.

(4) Upon death, brain tissue was fixed in paraformaldehyde, paraffin-embedded, sectioned, stained with H&E, and examined/photographed.

(5) At the end of observation, surviving mice were anesthetized for retro-orbital blood collection, then euthanized. Brain tissue and the left and right femurs were collected; bone marrow cells were obtained by flushing the marrow cavity with PBS. Brain tissue was fixed in paraformaldehyde, paraffin-embedded, sectioned, stained with H&E, and examined/photographed. Flow cytometry of peripheral blood and bone-marrow cells was performed to determine the proportions of CD4+ T cells, CD8+ T cells, and regulatory T (Treg) cells.

Data processing and statistical analysis

IBM SPSS Statistics 21.0 was used. One-way ANOVA or the Kruskal-Wallis test was applied for between-group analyses of BLI (bioluminescence imaging) values. Kaplan-Meier survival analysis was used for between-group comparisons of survival time. p < 0.05 was considered statistically significant.

Results

(1) Responses after dosing and changes in body weight of test animals

After dosing commenced, body-weight change curves for each group are shown in Fig. 44-4 (*Body weight; Days after inoculation*), and percentage body-weight change is shown in Fig. 44-5.
From Figs. 44-4 and 44-5, the vehicle group exhibited >15% body-weight loss by Day 39; mice in the CCPD3-treated group showed good general condition, no marked body-weight loss, no treatment interruption, and no other abnormal signs or unexpected deaths.

(2) In-vivo imaging (BLI) results

On D5 after tumor inoculation, mice were imaged with a small-animal in-vivo imaging system for bioluminescence imaging (BLI) and then randomized to treatment; thereafter imaging was performed twice per week, BLI curves were plotted, and statistical analyses were conducted.
Bioluminescence imaging (BLI) images of the mice in each group after tumor inoculation are shown in Figures 44-6 to 44-9 (Figure 44-6 shows the dorsal in vivo imaging of the mice, Figure 44-7 shows the left-side in vivo imaging of the mice, Figure 44-8 shows the right-side in vivo imaging of the mice, and Figure 44-9 shows the ventral in vivo imaging of the mice; "negtive control": negative control; "radiance": radiance; "color scale": color scale). The curves of changes in BLI values for the mice in each group are shown in Figure 44-10 (in Figure 44-10, A represents the curve of changes in BLI values for dorsal in vivo imaging of the mice, B represents the curve of changes in BLI values for left-side in vivo imaging of the mice, C represents the curve of changes in BLI values for right-side in vivo imaging of the mice, and D represents the curve of changes in BLI values for ventral in vivo imaging of the mice; "Total flux": total flux; "Days after tumor incubation": days after tumor inoculation). The statistical analysis of BLI data for the GL261-Luc orthotopic xenograft tumor model is shown in Table 201.

Table 201

| BLI imaging acquisition view | Time point (day) | $P$ |
|---|---|---|
| Dorsal | D8 | >0.05 |
| | D11 | >0.05 |
| | D15 | 0.008 |
| | D18 | 0.060 |
| | D21 | 0.043 |
| | D25 | 0.032 |
| | D28 | 0.051 |
| Left lateral | D18 | 0.060 |
| | D21 | 0.059 |
| | D25 | 0.017 |
| | D28 | 0.020 |
| Right lateral | D18 | 0.281 |
| | D21 | 0.124 |
| | D25 | 0.064 |
| | D28 | 0.044 |
| Ventral | D18 | 0.223 |
| | D21 | 0.093 |
| | D25 | 0.047 |
| | D28 | 0.032 |

From the above data, there was a significant difference between the CCPD3-treated group and the control group. After Day 28, the number of surviving mice in the control group was insufficient for statistical analysis; the mean BLI of the treated group showed a rebound. By Day 102, the BLI of the treated group was at an extremely low level.

(3) Survival observation

The survival curves are shown in Fig. 44-11 (*Total flux; Days after tumor inoculation*). Statistical results for survival in the GL261-Luc orthotopic xenograft model are summarized in Table 202.

Table 202

| Group | No. of animals | MST (days) | ILS/% | P* |
|---|---|---|---|---|
| Physiological saline | 6 | 32 | - | - |
| CCPD3 | 6 | 101 | 215.63% | 0.015 |

Survival was monitored up to 138 days after tumor-cell inoculation. As shown above, the median survival time (MST) of the control group was 32 days, whereas the MST of the CCPD3 group was 101 days. The increase in life span (ILS%) of the treated group was 215.63%, representing a significant difference versus control (p = 0.015; p = 0.001).

(4) Histopathology of brain sections-Hematoxylin-Eosin (H&E) staining: results

Control group (G1). In mice at D32, D39, and D41, low-power views showed that brain tissue near the rostral margin was largely invaded by tumor tissue; in the region near the caudal margin, tumor tissue compressed and infiltrated the cerebral parenchyma, with hemorrhage observed around the tumor and within the ventricles. High-power views showed densely packed tumor cells with marked variation in nuclear size, pronounced pleomorphism, numerous mitotic figures, and foci of tumor-cell necrosis. See Fig. 44-12.

In the CCPD3-treated group (G2), for mouse D34, low-power magnification revealed tumor tissue and hemorrhage in the ventromedial and lateral cerebral parenchyma near the rostral margin; no tumor tissue was seen near the caudal margin, though parenchymal hemorrhage was present. For mouse D37, low-power magnification showed the brain tissue near the rostral margin to be almost completely invaded by tumor, whereas the architecture near the caudal margin was normal. At high-power magnification, the brain contained densely packed tumor cells with marked variation in nuclear size (anisokaryosis), pronounced atypia, numerous mitotic figures, and foci of tumor-cell necrosis. The H&E-stained histopathological findings of the mouse brain sections are shown in Fig. 44-12.

In the three surviving mice of the CCPD3-treated group, the brain architecture at D138 was intact, with no obvious abnormalities. Representative hematoxylin-eosin (H&E)-stained histopathology of mouse brain sections is shown in Fig. 44-12.

(5) Results of CD4$^+$ T cells, CD8$^+$ T cells, and Treg cells in mouse peripheral blood and bone marrow

At Day 138 (D138), peripheral blood and bone-marrow cells from CCPD3-treated surviving mice were collected for flow-cytometric analysis, with healthy 6-8-week-old C57BL/6 mice used as normal controls for comparison. The percentages of CD4$^+$ T cells, CD8$^+$ T cells, and regulatory T (Treg) cells in peripheral blood and bone marrow are shown in Table 203, and the statistical analysis of these percentages is presented in Table 204:

Table 203. Proportions of CD4$^+$ T cells, CD8$^+$ T cells, and Treg cells in peripheral blood and bone marrow

| Tissue | Group | CD4$^+$ T cells(%) | CD8$^+$ T cells(%) | Treg cells(%) |
|---|---|---|---|---|
| Peripheral blood | Normal control | 58.46 $\pm$ 1.71 | 35.63 $\pm$ 1.31 | 0.34 $\pm$ 0.06 |
| | CCPD3-treated | 54.03 $\pm$ 1.81 | 35.94 $\pm$ 1.27 | 4.13 $\pm$ 0.92 |
| Bone marrow | Normal control | 8.87 $\pm$ 1.27 | 24.08 $\pm$ 3.41 | 2.57 $\pm$ 0.30 |
| | CCPD3-treated | 21.18 $\pm$ 2.06 | 48.64 $\pm$ 2.36 | 11.94 $\pm$ 0.47 |

*Note:* Data are presented as mean $\pm$ standard error.

Table 204. Statistical analysis of the proportions of CD4$^+$ T cells, CD8$^+$ T cells, and Treg cells in peripheral blood and bone marrow (P values)

| Tissue | Group | $P^a$ | $P^c$ | $P^c$ | $P^d$ | $P^e$ | $P^f$ |
|---|---|---|---|---|---|---|---|
| Peripheral blood | CCPD3-treated | >0.05 | >0.05 | >0.05 | - | - | - |
| Bone marrow | CCPD3-treated | - | - | - | 0.016 | 0.001 | 1.82E-4 |

Note: a: peripheral-blood CD4$^+$ T cells, comparison between the treated group and the normal control group; b: peripheral-blood CD8$^+$ T cells, comparison between the treated group and the normal control group; c: peripheral-blood Treg cells, comparison between the treated group and the normal control group; d: bone marrow CD4$^+$ T cells, comparison between the treated group and the normal control group; e: bone marrow CD8$^+$ T cells, comparison between the treated group and the normal control group; f: bone marrow Treg cells, comparison between the treated group and the normal control group.

Based on the foregoing data, the percentages of CD4$^+$ T cells, CD8$^+$ T cells, and regulatory T (Treg) cells in the peripheral blood of the treated group showed no significant differences compared with the normal control group, whereas the percentages of CD4$^+$ T cells, CD8$^+$ T cells, and Treg cells in the bone marrow of the treated group were significantly higher than those of the normal controls.

Conclusions:

1. The test article CCPD3 exhibited a significant tumor-growth inhibitory effect in the GL261-Luc orthotopic xenograft model.

2. CCPD3 significantly improved survival of mice in the GL261-Luc orthotopic xenograft model.

3. Considering both in-vivo imaging and histopathology, at D138 the three surviving mice in the CCPD3-treated group showed complete regression of brain tumors.

4. In the CCPD3-treated group, the percentages of CD4$^+$ T cells, CD8$^+$ T cells, and regulatory T (Treg) cells in peripheral blood did not differ significantly from those of the normal controls, whereas the percentages of CD4$^+$ T cells, CD8$^+$ T cells, and Treg cells in bone marrow were significantly higher than in the normal controls.

5. Tumor-bearing mice displayed good tolerability to CCPD3 at the doses used in this study.

(IV)

Plasma stability study

Species: Human, rat, mouse, monkey

Observation times: 0h, 0.5h, 6h, 12h, 24h, 48h

Number of test articles: 11

Analytical method: Each test article was prepared in 100% plasma at a relatively high concentration (3 mg/mL), incubated in a 37 °C incubator for the indicated periods, withdrawn, 100-fold diluted, and subjected to electrophoretic analysis. Results are shown in Fig. 44-13 (left to right: 0 h, 0.5 h, 6 h, 12 h, 24 h, 48 h).

Additionally, in human, rat, and monkey plasma, all 11 test articles-CCPD3, CPG2006, CD40 aptamer, PD-L1 aptamer, CPG2006-CCPD3-strand a, CD40 aptamer-CCPD3-strand b, PD-L1 aptamer-CCPD3-strand c, CPG2006-DNA3, CD40 aptamer-DNA3, PD-L1 aptamer-DNA3, and DNA3-remained relatively stable within 48 h.

Mouse plasma results are shown in Fig. 44-14 (left to right: 0 h, 0.5 h, 6 h, 12 h, 24 h, 48 h): test articles containing the b- and/or c-strand-namely CCPD3, CD40 aptamer-CCPD3-strand b, PD-L1 aptamer-CCPD3-strand c, CPG2006-DNA3, CD40 aptamer-DNA3, PD-L1 aptamer-DNA3, and DNA3-underwent severe degradation by 6 h, decreasing to ~20% of the 0 h level; by 12 h they were essentially undetectable;

Conclusion: The b- and c-strands may be insufficiently stable in mouse plasma, leading to degradation of test articles that contain the b- and/or c-strands.

The foregoing description merely illustrates preferred embodiments of the present invention and is not intended to limit the invention. Various changes and modifications may be made by those skilled in the art. Any modifications, equivalent substitutions, or improvements made within the spirit and scope of the invention are intended to be encompassed within the scope of protection of the present invention.

**Claims**

1. A nucleic acid carrier, **characterized in that** the nucleic acid carrier comprises a sequence a, a sequence b, and a sequence c, the nucleic acid carrier being selected from the group consisting of a DNA carrier, an RNA carrier, and a DNA-RNA hybrid carrier;

   wherein the sequence a, the sequence b, and the sequence c self-assemble to form the nucleic acid carrier;
   wherein the sequence a, the sequence b, and the sequence c comprise carrier backbone sequences, the carrier backbone sequences comprising the following core sequences: an sequence a1, sequence a b1, and sequence a c1, or any variant thereof comprising an insertion, deletion, or substitution of at least one nucleotide in at least one of the sequence a1, the sequence b1, and the sequence c1;
   wherein, in the DNA carrier, the sequence a1 is SEQ ID No. 1, the sequence b1 is SEQ ID No. 2, and the sequence c1 is SEQ ID No. 3;
   wherein, in the RNA carrier, the sequence a1 is SEQ ID No. 4, the sequence b1 is SEQ ID No. 5, and the sequence c1 is SEQ ID No. 6; and
   wherein the DNA-RNA hybrid carrier is a carrier formed by self-assembly from a combination of the sequences of the DNA carrier and the sequences of the RNA carrier;
   SEQ ID No. 1: ACGAGCGTTCCG;
   SEQ ID No. 2: CGGTTCGCCG;
   SEQ ID No. 3: CGGCCATAGCCGT;
   SEQ ID No. 4: ACGAGCGUUCCG;

SEQ ID No. 5: CGGUUCGCCG;
SEQ ID No. 6: CGGCCAUAGCCGU.

**2.** The nucleic acid carrier according to claim 1, wherein the carrier backbone sequence further comprises a first extension segment and an optional second extension segment, the first extension segment and the second extension segment being located at the 5' end and/or the 3' end of any one of the sequences a1, b1, and c1,

preferably, each of the first extension segment and the second extension segment is independently 1-14 nt in length;
preferably, the first extension segment is selected from any one or more of the following DNA extension segments or RNA extension segments corresponding thereto:

1) 5' end of the sequence a1: GACGCCC, 3' end of the sequence c1: GGGCGTC;
2) 3' end of the sequence a1: GGAGAGG, 5' end of the sequence b1: CCTCTCC;
3) 3' end of the sequence b1: CGAGCC, 5' end of the sequence c1: GGCTCG or GGCACG;
4) 5' end of the sequence a1: GGCGCCCor GACGCCC, 3' end of the sequence c1: GGGCGCC;
5) 3' end of the sequence a1: GGAG, 5' end of the sequence b1: CTCC;
6) 3' end of the sequence b1: CCAGCC, 5' end of the sequence c1: GGCACGor GGCTCG;

preferably, the second extension segment is selected from any one or more of the following DNA extension segments or RNA extension segments corresponding thereto:

1) at the 5' end of the sequence a1: C or GC; and at the 3' end of the sequence c1: GC or GCT;
2) at the 3' end of the sequence a1: AG, AGC, or AGCT; and at the 5' end of the sequence b1: GCT or CT;
3) at the 3' end of the sequence b1: GC, GCG, or GCGT; and at the 5' end of the sequence c1: GC or CGC;
4) at the 3' end of the sequence a1: C, AGGCC, AGGCCT, or AGGAG; and at the 5' end of the sequence b1: G, GGCCT, or CTCCT;
5) at the 3' end of the sequence b1: GCC or GCCT; and at the 5' end of the sequence c1: GGC.

**3.** The nucleic acid carrier according to claim 1 or 2, wherein the nucleic acid carrier further comprises a single-stranded adhesive-bridge sequence and/or a transition sequence,

wherein the single-stranded adhesive-bridge sequence is selected from any one or more of the following:

1) located at the 3' end of the scaffold sequence of the sequence a: SEQ ID No. 7: TGTAGCACGGTGGC or the complementary sequence thereof, SEQ ID No. 8: GCCACCGTGCTACA;
2) located at the 3' end of the scaffold sequence of the sequence b: SEQ ID No. 9: TCGGCGCGGCCGTG or the complementary sequence thereof, SEQ ID No. 10: CACGGCCGCGCCGA;
3) located at the 3' end of the scaffold sequence of the sequence c:

i) SEQ ID No. 11: TGCTGCTGCTGCTG or the complementary sequence thereof, SEQ ID No. 12: CAGCAGCAGCAGCA;
ii) SEQ ID No. 13: CGCGGCTCGCGGCT or the complementary sequence thereof, SEQ ID No. 14: AGCCGCGAGCCGCG;

wherein the transition sequence is sequence a formed by n consecutive U, T, or A nucleotides, wherein n is 2-8, preferably an integer from 3 to 6, or sequence a formed by a random combination of A, U, T, C, and/or G; the transition sequence being located at the 5' end or the 3' end of the core sequence;
preferably, in the nucleic acid carrier, the sequence a, the sequence b, and the sequence c are, respectively, as follows:

the sequence a is SEQ ID No. 15:
GACGCCCACGAGCGTTCCGGGAGAGG<u>TGTAGCACGGTGGC</u>, wherein the underlined portion denotes the single-stranded adhesive-bridge sequence;
the sequence b is SEQ ID No. 16:
CCTCTCCCGGTTCGCCGCGAGCC<u>TCGGCGCGGCCGTG</u>, wherein the underlined portion denotes the single-stranded adhesive-bridge sequence;
the sequence c is SEQ ID No. 17:

GGCTCGCGGCCATAGCCGTGGGCGTC<u>TGCTGCTGCTGCTG</u>, wherein the underlined portion denotes the single-stranded adhesive-bridge sequence;or
in the nucleic acid carrier, the sequence a, the sequence b, and the sequence c are, respectively, as follows: the sequence a is SEQ ID No. 18:

GCGACGCCCACGAGCGTTCCGGGAGAGGAGC;
the sequence b is SEQ ID No. 19: GCTCCTCTCCCGGTTCGCCGCGAGCCGCG;
the sequence c is SEQ ID No. 20:
<u>CAGCAGCAGCAGCAC</u>GCGGCTCGCGGCCATAGCCGTGGGCGTCGC, wherein the underlined portion denotes the single-stranded adhesive-bridge sequence.

4. The nucleic acid carrier according to claim 1 or 2, wherein the scaffold sequences that self-assemble to form the nucleic acid carrier are respectively selected from any one of the following sets:

1) sequence a: SEQ ID No. 21: GCGACGCCCACGAGCGTTCCGGGAGAGGAG,

sequence b: SEQ ID No. 22: CTCCTCTCCCGGTTCGCCGCGAGCCGCG,
sequence c: SEQ ID No. 23: CGCGGCACGCGGCCATAGCCGTGGGCGTCGC;

2) sequence a:SEQ ID No. 24: GCGACGCCCACGAGCGTTCCGGGAGAGGAGCT,

sequence b: SEQ ID No. 19: GCTCCTCTCCCGGTTCGCCGCGAGCCGCG,
sequence c: SEQ ID No. 23: CGCGGCACGCGGCCATAGCCGTGGGCGTCGC;

3) sequence a:SEQ ID No. 24: GCGACGCCCACGAGCGTTCCGGGAGAGGAGCT,

sequence b: SEQ ID No. 25: GCTCCTCTCCCGGTTCGCCGCGAGCCGCGT,
sequence c: SEQ ID No. 26: CGCGGCTCGCGGCCATAGCCGTGGGCGTCGCT;

4) sequence a:SEQ ID No. 24: GCGACGCCCACGAGCGTTCCGGGAGAGGAGCT,

sequence b: SEQ ID No. 25: GCTCCTCTCCCGGTTCGCCGCGAGCCGCGT,
sequence c: SEQ ID No. 27: CGCGGCACGCGGCCATAGCCGTGGGCGTCGCT;

5) sequence a: SEQ ID No. 28: GACGCCCACGAGCGTTCCGGGAGAGG,

sequence b: SEQ ID No. 29: CCTCTCCCGGTTCGCCGCGAGCC,
sequence c: SEQ ID No. 30: GGCTCGCGGCCATAGCCGTGGGCGTC;

6) sequence a: SEQ ID No. 18: GCGACGCCCACGAGCGTTCCGGGAGAGGAGC,

sequence b: SEQ ID No. 19: GCTCCTCTCCCGGTTCGCCGCGAGCCGCG,
sequence c: SEQ ID No. 31: CGCGGCTCGCGGCCATAGCCGTGGGCGTCGC;

7) sequence a:SEQ ID No. 24: GCGACGCCCACGAGCGTTCCGGGAGAGGAGCT,

sequence b: SEQ ID No. 19: GCTCCTCTCCCGGTTCGCCGCGAGCCGCG,
sequence c: SEQ ID No. 26: CGCGGCTCGCGGCCATAGCCGTGGGCGTCGCT;

8) sequence a:SEQ ID No. 24: GCGACGCCCACGAGCGTTCCGGGAGAGGAGCT,

sequence b: SEQ ID No. 19: GCTCCTCTCCCGGTTCGCCGCGAGCCGCG,
sequence c: SEQ ID No. 31: CGCGGCTCGCGGCCATAGCCGTGGGCGTCGC;

9) sequence a:SEQ ID No. 24: GCGACGCCCACGAGCGTTCCGGGAGAGGAGCT,

sequence b: SEQ ID No. 25: GCTCCTCTCCCGGTTCGCCGCGAGCCGCGT,
sequence c: SEQ ID No. 26: CGCGGCTCGCGGCCATAGCCGTGGGCGTCGCT;

10) sequence a: SEQ ID No. 24:GCGACGCCCACGAGCGTTCCGGGAGAGGAGCT,

sequence b: SEQ ID No. 25: GCTCCTCTCCCGGTTCGCCGCGAGCCGCGT,
sequence c: SEQ ID No. 31: CGCGGCTCGCGGCCATAGCCGTGGGCGTCGC;

11) sequence a: SEQ ID No. 18:GCGACGCCCACGAGCGTTCCGGGAGAGGAGC;

sequence b: SEQ ID No. 25: GCTCCTCTCCCGGTTCGCCGCGAGCCGCGT;
sequence c: SEQ ID No. 26: CGCGGCTCGCGGCCATAGCCGTGGGCGTCGCT;

12) sequence a: SEQ ID No. 32:CGACGCCCACGAGCGTTCCGGGAGAGGAGC;

sequence b: SEQ ID No. 19: GCTCCTCTCCCGGTTCGCCGCGAGCCGCG;
sequence c: SEQ ID No. 31: CGCGGCTCGCGGCCATAGCCGTGGGCGTCGC;

13) sequence a: SEQ ID No. 33: GCGGCGCCCACGAGCGTTCCGGGAGC;

sequence b: SEQ ID No. 34: GCTCCCGGTTCGCCGCCAGCCGCC;
sequence c: SEQ ID No. 35: GGCGGCAGGCGGCCATAGCCGTGGGCGCCGC;

14) sequence a: SEQ ID No. 36: GCGGCGCCCACGAGCGTTCCGGGAGAGGCC;

sequence b: SEQ ID No. 37: GGCCTCTCCCGGTTCGCCGCCAGCCGCC;
sequence c: SEQ ID No. 38: GGCGGCTGGCGGCCATAGCCGTGGGCGCCGC;

15) sequence a: SEQ ID No. 39:GCGGCGCCCACGAGCGTTCCGGGAGAGGCCT;

sequence b: SEQ ID No. 40: GGCCTCTCCCGGTTCGCCGCCAGCCGCCT;
sequence c: SEQ ID No. 41: GGCGGCTGGCGGCCATAGCCGTGGGCGCCGCT;

16) sequence a: SEQ ID No. 42: GCGGCGCCCACGAGCGUUCCGGGAGAGGCC;

sequence b: SEQ ID No. 43: GGCCUCUCCCGGUUCGCCGCCAGCCGCC;
sequence c: SEQ ID No. 44: GGCGGCUGGCGGCCAUAGCCGUGGGCGCCGC;

17) sequence a: SEQ ID No. 45: CGACGCCCACGAGCGTTCCGGGAGAGGAG;

sequence b: SEQ ID No. 46: CTCCTCTCCCGGTTCGCCGCCAGCCGCC;
sequence c: SEQ ID No. 35: GGCGGCAGGCGGCCATAGCCGTGGGCGCCGC;

18) sequence a: SEQ ID No. 36: GCGGCGCCCACGAGCGTTCCGGGAGAGGCC;

sequence b: SEQ ID No. 43: GGCCUCUCCCGGUUCGCCGCCAGCCGCC;
sequence c: SEQ ID No. 44: GGCGGCUGGCGGCCAUAGCCGUGGGCGCCGC;

19) sequence a: SEQ ID No. 36: GCGGCGCCCACGAGCGTTCCGGGAGAGGCC;

sequence b: SEQ ID No. 40: GGCCTCTCCCGGTTCGCCGCCAGCCGCCT;
sequence c: SEQ ID No. 38: GGCGGCTGGCGGCCATAGCCGTGGGCGCCGC;

20) sequence a: SEQ ID No. 36: GCGGCGCCCACGAGCGTTCCGGGAGAGGCC;

sequence b: SEQ ID No. 40: GGCCTCTCCCGGTTCGCCGCCAGCCGCCT;
sequence c: SEQ ID No. 41: GGCGGCTGGCGGCCATAGCCGTGGGCGCCGCT;

21) sequence a: SEQ ID No. 36: GCGGCGCCCACGAGCGTTCCGGGAGAGGCC;

sequence b: SEQ ID No. 37: GGCCTCTCCCGGTTCGCCGCCAGCCGCC;

sequence c: SEQ ID No. 44: GGCGGCUGGCGGCCAUAGCCGUGGGCGCCGC;

22) sequence a: SEQ ID No. 33: GCGGCGCCCACGAGCGTTCCGGGAGC;

sequence b: SEQ ID No. 46: CTCCTCTCCCGGTTCGCCGCCAGCCGCC;
sequence c: SEQ ID No. 35: GGCGGCAGGCGGCCATAGCCGTGGGCGCCGC;

23) sequence a: SEQ ID No. 47: GCGGCGCCCACGAGCGTTCCGGGAGAGGAG;

sequence b: SEQ ID No. 46: CTCCTCTCCCGGTTCGCCGCCAGCCGCC;
sequence c: SEQ ID No. 35: GGCGGCAGGCGGCCATAGCCGTGGGCGCCGC;

24) sequence a: SEQ ID No. 48: CGCCCACGAGCGTTCCGGGAGA;

sequence b: SEQ ID No. 49: TCTCCCGGTTCGCCGCGAG;
sequence c: SEQ ID No. 50: CTCGCGGCCATAGCCGTGGGCG;

25) sequence a: SEQ ID No. 51: UCGCCCACGAGCGUUCCGGGAGA;

sequence b: SEQ ID No. 52: UCUCCCGGUUCGCCGCGAG;
sequence c: SEQ ID No. 53: UCUCGCGGCCAUAGCCGUGGGCG;

26) sequence a: SEQ ID No. 54: GCGCCCACGAGCGTTCCGGGAGAGC;

sequence b: SEQ ID No. 55: CCCUGCTCTCCCGGTTCGCCGCCAGCCGCC;
sequence c: SEQ ID No. 56: GGCGGCAGGCGGCCATAGCCGTGGGCGC.

5. The nucleic acid carrier according to any one of claims 1 to 4, wherein at least one of the base, the ribose, and the phosphate in the sequence a, the sequence b, and the sequence c comprises at least one modifiable site, and any of the modifiable sites with at least one modification selected from: -F (fluoro), methyl, amino, disulfide, mercapto (sulfhydryl), carboxyl, thio-, and thioate;

preferably, portions of the phosphate backbone of the sequence a, the sequence b, and the sequence c comprise phosphorothioate modification;
preferably, when the nucleic acid carrier is an RNA carrier or a DNA-RNA hybrid carrier, C and U residues in the sequences in RNA form in the nucleic acid carrier are 2'-F-modified and A and G residues are 2'-OMe-modified.

6. A nucleic acid drug, **characterized in that** the nucleic acid drug comprises the nucleic acid carrier according to any one of claims 1 to 5 and a bioactive substance borne on the nucleic acid carrier, the bioactive substance comprising an oligonucleotide effector molecule and a targeting molecule for targeted delivery of the oligonucleotide molecule, wherein the oligonucleotide effector molecule is selected from at least one of: an immunostimulatory nucleic acid, a nucleic acid aptamer, siRNA, miRNA, and an antisense oligonucleotide (ASO); and

when the oligonucleotide effector molecule comprises the nucleic acid aptamer, the targeting molecule comprises at least the nucleic acid aptamer;
when the oligonucleotide effector molecule does not comprise the nucleic acid aptamer, the targeting molecule comprises at least a small-molecule compound having targeting activity.

7. The nucleic acid drug according to claim 6, wherein the nucleic acid immunostimulant comprises one or more selected from the group consisting of CpG2006, a CpG2006 variant, CpG1826, CpG2216, CpG2395, CpG-ODNT7, and CpG-BYZD;

preferably, the sequence of CpG2006 is:

SEQ ID No. 57: TCGTCGTTTTGTCGTTTTGTCGTT, or
SEQ ID No. 58: UCGUCGUUUUGUCGUUUUGUCGUU;

preferably, the sequence of the CpG2006 variant is GTCGTT;

preferably, the sequence of CpG1826 is SEQ ID No. 59: TCCATGACGTTCCTGACG;

preferably, the sequence of CpG2216 is SEQ ID No. 60: GGGGGACGATCGTCGGGGGG;

preferably, the sequence of CpG2395 is SEQ ID No. 61: TCGTCGTTTTCGGCGCGCGCCG;

preferably, the sequence of CpG-ODNT7 is SEQ ID No. 62: TCGTCGTCGTCGTCGTCGTCGor SEQ ID No. 63: TCGTCGTCGTCGTCGTCGTCGTCG;

preferably, the sequence of CpG-BYZD is AGCGAA .

8. The nucleic acid drug according to claim 6, wherein the siRNA comprises one or more siRNAs targeting any of the following molecules: ASAP1, ATAD2, CD24, CD47, EGFR, HBV, HSP, HS70, PD-L1, PARP-1, Survivin, TAP, TIM-3, TGF-β1, and VEGF-C;

preferably, the siRNA for ASAP1 is selected from any of the following: i) sense strand: SEQ ID No. 64: AUUUGCUUCCAUAAUAUCAUU, antisense strand: SEQ ID No. 65: UGAUAUUAUGGAAGCAAAUUU; ii) sense strand: SEQ ID No. 66: UUAGGUUUGGGGUUGGAUCUU, antisense strand: SEQ ID No. 67: GAUCCAACCCCAAACCUAAUU;

preferably, the siRNA for ATAD2 comprises an antisense strand SEQ ID No. 68: GCGUCGAAGLTUGUAGGALTCTCTU, and a sense strand SEQ ID No. 69: AAUCCUACAACUUCGACGCUU;

preferably, the siRNA for CD24 comprises a sense strand SEQ ID No. 70: UGUUUACAUUGUUGAGGUAUU, and an antisense strand SEQ ID No. 71: UACCUCAACAAUGUAAACUUU;

preferably, the siRNA for CD47 is selected from any of the following: i) sense strand: SEQ ID No. 72: GGUGAUUACCCAGAGAUAUTT, antisense strand: SEQ ID No. 73: AUAUCUCUGGGUAAUCACCTT; ii) sense strand: SEQ ID No. 74: UGGUGAAAGAGGUCAUUCCUU, antisense strand: SEQ ID No. 75: GGAAUGACCUCUUUCACCAUU; iii) sense strand: SEQ ID No. 76: GGAAUGACCUCUUUCACCATT, antisense strand: SEQ ID No. 77: UGGUGAAAGAGGUCAUUCCTT; iv) sense strand: SEQ ID No. 78: GGUGAUUACCCAGAGAUAUUU, antisense strand: SEQ ID No. 79: AUAUCUCUGGGUAAUCACCUU;

preferably, the siRNA for EGFR is selected from any of the following: i) sense strand: SEQ ID No. 80: GGCUGGUUAUGUCCUCAUUUU, antisense strand: SEQ ID No. 81: AAUGAGGACAUAACCAGCCUU; ii) sense strand: SEQ ID No. 82: CCUUAGCAGUCUUAUCUAAUU, antisense strand: SEQ ID No. 83: UUAGAUAAGACUGCUAAGGUU; iii) sense strand: SEQ ID No. 84: UGCCUUAGCAGUCUUAUCUAAUU, antisense strand: SEQ ID No. 85: UUAGAUAAGACUGCUAAGGCAUU; iv) sense strand: SEQ ID No. 86: UGCCUUAGCAGUCUUAUCUAAUUUU, antisense strand: SEQ ID No. 87: AAUUAGAUAAGACUGCUAAGGCAUU;

preferably, the siRNA for HBV comprises a sense strand SEQ ID No. 88: GGACUUCUCUCAAUUUUCUUU, and an antisense strand SEQ ID No. 89: AGAAAAUUGAGAGAAGUCCUU;

preferably, the siRNA for HSP comprises a sense strand SEQ ID No. 90: CGCAGAACACCGUGUUCGAUU, and an antisense strand SEQ ID No. 91: UCGAACACGGUGUUCUGCGUU;

preferably, the siRNA for HS70 comprises a sense strand SEQ ID No. 92: GGCCAACAAGAUCACCAUCUU, and an antisense strand SEQ ID No. 93: GAUGGUGAUCUUGUUGGCCUU;

preferably, the siRNA for PD-L1 is selected from any of the following: i) sense strand: SEQ ID No. 94: CCAGCACACUGAGAAUCAAUU, antisense strand: SEQ ID No. 95: UUGAUUCUCAGUGUGCUGGUU; ii) sense strand: SEQ ID No. 96: AGACGUAAGCAGUGUUGAATT, antisense strand: SEQ ID No. 97: UUCAACACUGCUUACGUCUTT;

preferably, the siRNA for PARP-1 comprises a sense strand: SEQ ID No. 98: GAGGAAGGUAUCAACAAAUTT, and an antisense strand SEQ ID No. 99: AUUUGUUGAUACCUUCCUCTT;

preferably, the siRNA for Survivin is selected from any of the following: i) sense strand: SEQ ID No. 100: GCAGGUUCCUUAUCUGUCACAUU, antisense strand: SEQ ID No. 101: UGUGACAGAUAAGGAACCUGCUU; ii) sense strand: SEQ ID No. 102: GGAAUUGGAAGGCUGGGAACCUU, antisense strand: SEQ ID No. 103: GGUUCCCAGCCUUCCAAUUCCUU; iii) sense strand: SEQ ID No. 104: UGCAGGUUCCUUAUCUGUCATT, antisense strand: SEQ ID No. 105: UGACAGAUAAGGAACCUGCTT; iv) sense strand: SEQ ID No. 106: CUGCAGGUUCCUUAUCUGUCACAUU, antisense strand: SEQ ID No. 107: UGUGACAGAUAAGGAACCUGCAGUU;

preferably, the siRNA for TAP is selected from any of the following: i) sense strand: SEQ ID No. 108: GCUGCACACGGUUCAGAAUUU, antisense strand: SEQ ID No. 109: AUUCUGAACCGUGUGCAGCUU; ii) sense strand: SEQ ID No. 110: CAGGAUGAGUUACUUGAAAUU, antisense strand: SEQ ID No. 111: UUUCAAGUAACUCAUCCUGUU

preferably, the siRNA for TIM-3 comprises a sense strand SEQ ID No. 112: GUGCUCAGGACUGAUGAAATT, and an antisense strand SEQ ID No. 113: UUUCAUCAGUCCUGAGCACTT;

preferably, the siRNA for TGF-β1 is selected from any of the following: i) sense strand: SEQ ID No. 114:

GUCAACUGUGGAGCAACACUU, antisense strand: SEQ ID No. 115: GUGUUGCUCCACAGUUGACUU; ii) sense strand: SEQ ID No. 116: GCAACAACGCCAUCUAUGATT, antisense strand: SEQ ID No. 117: UCAUAGAUGGCGUUGUUGCTT;

preferably, the siRNA for VEGF-C is selected from any of the following: i) sense strand: SEQ ID No. 118: GCAAGACGUUGUUUGAAAUUAUU, antisense strand: SEQ ID No. 119: UAAUUUCAAACAACGUCUUGCUU; ii) sense strand: SEQ ID No. 120: CAGCAAGACGUUGUUUGAAAUUAUU, antisense strand: SEQ ID No. 121: UAAUUUCAAACAACGUCUUGCUGUU; iii) sense strand: SEQ ID No. 122: CAGGAUGGUAAAGACUACAUU, antisense strand: SEQ ID No. 123: UGUAGUCUUUACCAUCCUGUU;

preferably, the miRNA comprises one or more selected from miR-34, miR-542, miR-126, and miR-122;

preferably, the ASO comprises one or more selected from A-miR21, A-miR-10a, A-miR-30c, and A-miR-1306;

preferably, the A-miR21 is selected from any of the following in DNA or RNA form:

i) GATAAGCT;
ii) SEQ ID No. 124: GTCAACATCAGTCTGATAAGCTA;
iii) SEQ ID No. 125: TCAACATCAGTCTGATAAGCTA;
iv) the sequence of (i) or (ii) in which T is replaced with U;

preferably, A-miR-10a is ACAGGGTA;

preferably, A-miR-30c is SEQ ID No. 126: GCTGAGAGTGTAGGATGTTTACA;

preferably, the sequence of miR-34 is TGTGACAG;

preferably, the sequence of miR-542 is TGGCAGTGT;

preferably, the sequence of miR-126 is UCGUACC ; or UCGUACCG; or CGTACCGor GTCGTT;

preferably, the sequence of miR-122 is GGAAGTGT;

preferably, the sequence of AmiR1306 is SEQ ID No. 127: CATCACCACCAGAGCCAACGTC.

9. The nucleic acid drug according to claim 6, wherein the nucleic acid aptamer comprises a nucleic acid aptamer in DNA form or in RNA form;

preferably, the nucleic acid aptamer in DNA form comprises at least one aptamer selected from the group consisting of: A1, A15, AS1411, AFP, ATP, Act-12c, A18, BAF7-1, C-MetSL-1, CH6, CA2, CRAC Orail target, CEA, CEA-18, CEA-T84, CSC1, CSC13, CD40, CD16a, CD19, CD3-4, CD44, CD12, CD20, CD24, CD24A-2, CD33, CD38, CD105, CD117, CD63, CD123, EGFR, EpCAM, EcR, FAP, GPC1, GPC3, GSK836, HBsAg, Her2, Her3, HMGA2, H2, IFN-γ, IL-4Ra, IL-17, LZH8, MUC1, M5, M7, M1, N5, N-G-Dua, NKG2D_#-20-N-15, NSE, SARS-CoV-2-N15, SARS-CoV-2-N48, SARS-CoV-2-N58, SARS-CoV-2-N61, OX40, PSMA, PDGFRβ, PDGF, PD-L1, PD1, PTK-7, ProGRP-48, SF, TBA15, TBA29, TIMC-d, TRRA4, TFRA3, TTA1, TLS9a, TGF-βII, TNF-α, TNF, T1, Vap7, VEGF121, VEGF-V7t1, VCAM-1, VCAM-12d, PL-45, EP166, AGC, Karpas299, SW620, MDA-MB-231, MCF-7 and PC-3;

the nucleic acid aptamer in RNA form comprises at least one aptamer selected from the group consisting of: A15, AS1411, BCMA, CTLA-4, CCL1, CEA, CD4-3, CD28, CD44, IL-4RA, EGFR, EpCAM, FGF2, FGF5, Her2, Her3, LAG-3, MUC1, MRP1, OX40, PSMA, PDGFRβ, TFRA4, TFRA3, TTA1, TIM3, TIMC-11, VEGF, VEGF165 and 4-1BB;

preferably, the nucleic acid aptamer is selected from at least one aptamer having sequence a selected from the following:

A1 aptamer: SEQ ID No. 128:
GGTTGCATGCCGTGGGGAGGGGGGTGGGTTTTATAGCGTACTCAG;
A15 aptamer: SEQ ID No. 129: CCCTCCTACATAGGG; or SEQ ID No. 130: CCCUCCUACAUAGGG;
AS1411 aptamer:

1)SEQ ID No. 131: GGTGGTGGTGGTTGTGGTGGTGGTGG; or
2)SEQ ID No. 132: GGUGGUGGUGGUUGUGGUGGUGGUGG; or
3)SEQ ID No. 133: AUUCUGAACCGUGUGCAGCACCACGCUGCACACGGUUCAGAAUACACA;

AFP aptamer: SEQ ID No. 134:

GGCAGGAAGACAAACAAGCTTGGCGGCGGGAAGGTGTTTAAATTCCCGGGTCT GCGTGGTCTGTGGTGCTGT;

ATP aptamer: SEQ ID No. 135: ACCTGGGGAGTATTGGGGAGGAAGG, or SEQ ID No. 136: GGGAGGACGATGCGGAGGAAGGGTAGG;

Act-12c aptamer: SEQ ID No. 137: CGGGGAAAGTCACGGGGGGGTTTCAGATGTTCTGATCGGTGTGGAG;

A18 aptamer: SEQ ID No. 138: CCAGATAGTCCCTGG;

BAF aptamer: SEQ ID No. 139: GATAACGGGCACGAATTCGGAGTG;

BCMA aptamer: SEQ ID No. 140: AGUGCAAGACGUUCGCAGAUUAGCGAAAAGAGGGUCUCAUUGA CUAGUAC;

CTLA-4 aptamer: 1) SEQ ID No. 141: GGUGGAAGAGGGAUGGGCCGACGUGCCGCAU; or 2) SEQ ID No. 142: TCCCTACGGCGCTAACGATGGTGAAAATGGGCCTAGGGTGGACGGTGCCACCGTGC TACAAC;

C-Met-SL1 aptamer: SEQ ID No. 143: ATCAGGCTGGATGGTAGCTCGGTCGGGGTGGGTGGGTTGG CAAGTCTGAT;

CH6 aptamer:
SEQ ID No. 144: AGTCTGTTGGACCGAATCCCGTGGACGCACCCTTTGGACG;

CA2 aptamer: SEQ ID No. 145: CCCACGTCTGCGCTTAGCTCCTGGGCCTGGATGGGC;

CCL1 aptamer:
SEQ ID No. 146: UGACUCCUCUGACAGCCUAAUUUCUCCCGAUUACCCUG;

CRAC Orail target aptamer:
SEQ ID No. 147: CCAGTAGCCATACCGGTTTGTGGATGGGGTGTATGCGAGT;

CEA aptamer:

1) SEQ ID No. 148: CTAGGATCCCCACTCACCATCTCTCAGCTTGCTTCCTAGC; or
2) SEQ ID No. 149: CUAGGAUCCCCACUCACCAUCUCUCAGCUUGCUUCCUAGC; or
3) SEQ ID No. 150: TTAACTTATTCGACCATA; or
4) SEQ ID No. 151:

TCGCGCGAGTCGTCTGGGGAACCATCGAGTTACACCGACCTTCTATGTGCGGCC CCCCGCATCGTCCTCCC;

CSC1 aptamer: SEQ ID No. 152:

ACCTTGGCTGTCGTGTTGTAGGTGGTTTGCTGCGGTGGGCTCAAGAAGAAAGC GCAAAGAGGTCAGTGGTCAGAGCGT;

CSC13 aptamer: SEQ ID No. 153:

ACCTTGGCTGTCGTGTTGTGGGGTGTCGTATCTTTCGTGTCTTATTATTTTCTAGG TGGAGGTCAGTGGTCAGAGCGT;

CD40 aptamer:

1) SEQ ID No. 154: CCAACGAGTAGGCGATAGCGCGTGG;
2) SEQ ID No. 155: AGAGACGATGCGGCCAACGAGTAGGCGATAGCGCGTGGCAGAGCGTCGCT;
3) SEQ ID No. 156: GCCAACGAGTAGGCGATAGCGCGTGGC;

CD16a aptamer:
SEQ ID No. 157: CCACTGCGGGGGTCTATACGTGAGGAAGAAGTGG;

CD19 aptamer:

1) SEQ ID No. 158:

TGCGTGTGTAGTGTGTGTCGTTTCTCCTTTTTTTGGTTGCTGCTCTTAGGGATTTG GGCGG;

or
2)SEQ ID No. 159:

TGCGTGTGTAGTGTGTCTGTTCTCCTTTTTTTGGTTGCTGCTCTTAGGGATTTGG GCGG,

3)SEQ ID No. 160: UGAGCCCUGUUCGACAGGAGGCUCA;

CD4-3 aptamer:
SEQ ID No. 161: GGGAGGACGAUGCGGUUUGGGGUUUUCCCGUGCCCCAGACGACUCGCCCGA;
CD3-4 aptamer:
SEQ ID No. 162:

TCTCGGACGCGTGTGGTCGGCCGAGTGGCCCACGGTAGAAGGGTTAGAACTGC TGGTTGGTGAATCTCGCTGCCTGGCCCTAGAGTG;

CD44 aptamer:
SEQ ID No. 163: CCAAGGCCTGCAAGGGAACCAAGGACACAG;
CD12 aptamer:
SEQ ID No. 164: GTGGATTGTTGTGTTCTGTTGGTTTTTGTGTTGTC;
CD20 aptamer: SEQ ID No. 165:

TGCGTGTGTAGTGTGTCTGTTTTTTATCTTCTTTTATCTACTCTTAGGGATTTGGG CGG;

CD24 aptamer:
SEQ ID No. 166: TATGTGGGTGGGTGGGCGGTTATGCTGAGTCAGCCTTGCT;
CD24A-2 aptamer: SEQ ID No. 167:

ATCCAGAGTGACGCAGCATATGTGGGTGGGTGGGCGGTTATGCTGAGTCAGCCT TGCTTGGACACGGTGGCTTAGT;

CD28 aptamer: SEQ ID No. 168:

GGGAGAGAGGAAGAGGGAUGGGGAUUAGACCAUAGGCUCCCAACCCCCCCC GGGAGAGAGGAAGAGGGAUGGGGAUUAGACCAUAGGCUCCCAACCCCCGGG;

CD33 aptamer: SEQ ID No. 169:

TACCAGTGCGATGCTCAGCACGCTTATAGGGGCTGGACAAAATTCTACCCAGCC TTT;

CD38 aptamer: SEQ ID No. 170:
TACGTGAATCTCGTACGATACTCTGTAAGCGT;
CD44 aptamer: SEQ ID No. 171:

GGGAUGGAUCCAAGCUUACUGGCAUCUGGAUUUGCGCGUGCCAGAAUAAAG AGUAUAACGUGUGAAUGGGAAGCUUCGAUAGGAAUUCGG;

CD105 aptamer: SEQ ID No. 172: GATCAGTTTTCCATGCCAGTTGGTATTCCGCGACAGTTTGATCTC;
CD117 aptamer: SEQ ID No. 173:
GGGGCCGGGGCAAGGGGGGGGTACCGTGGTAGGAC;
CD63 aptamer: SEQ ID No. 174:
CACCCCACCTCGCTCCCGTGACACTAATGCTA;

CD123 aptamer: SEQ ID No. 175:

TGCGTGTGTAGTGTGTCTGGGCTACATCGATGAGCTGCCTAGGGTCCCTCTTAGG GATTTGGGCGG;

EGFR aptamer:

1)SEQ ID No. 176: GCCTTAGTAACGTGCTTTGATGTCGATTCGACAGGAGGC; or
2)SEQ ID No. 177: GCCUUAGUAACGUGCUUUGAUGUCGAUUCGACAGGAGGC; or
3)SEQ ID No. 178:

UGCCGCUAUAAUGCACGGAUUUAAUCGCCGUAGAAAAGCAUGUCAAAGCCG UU;

EpCAM aptamer:

1)SEQ ID No. 179: GCGACUGGUUACCCGGUCG; or
2)SEQ ID No. 180: CACTACAGAGGTTGCGTCTGTCCCACGTTGTCATGGGGGGTTGGCCTG; or
3)SEQ ID No. 181: GACAAACGGGGGAAGATTTGACGTCGACGAC;

EcR aptamer: SEQ ID No. 182: GCAGGTCCACTGCGGGGGGTCTATACGTGAGGAAGAAGTGGG-CAGGTC;
FGF2 aptamer:

1)SEQ ID No. 183: GGGAUACUAGGGCAUUAAUGUUACCAGUGUAGUCCC;
2)SEQ ID No. 184: GGGAAACUAGGGCGUUAACGUGACCAGUGUUUCUCGA;
3)SEQ ID No. 185: GGGAAACUAGGGCGUUAACGUGACCAGUGUUUCCC;

FGF5 aptamer: SEQ ID No. 186:

GGGCGACCUCUCCGUACUGACCUACAGAGCGACAUACUAGUGUAUCCAGAU CGCCC;

FAP aptamer: 1)SEQ ID No. 187: TGGGGGTTGAGGCTAAGCCGA; or
2)SEQ ID No. 188: CCGCTCGAGCTAGTCTGACAAAGAGAAACAC;
GPC1 aptamer: SEQ ID No. 189: AACGGAGTGTGGCTAACTCGA;
GPC3 aptamer: SEQ ID No. 190:
TAACGCTGACCTTAGCTGCATGGCTTTACATGTTCCA;
GSK836 aptamer: SEQ ID No. 191: GCAGAGGTGAAGCGAAGTCG;
HBsAg aptamer:
SEQ ID No. 192: CACAGCGAACAGCGGCGGACATAATAGTGCTTACTACGAC;
Her2 aptamer:

1)SEQ ID No. 193: AGCCGCGAGGGGAGGGATAGGGTAGGGCGCGGCT; or
2)SEQ ID No. 194: AGCCGCGAGGGGAGGGAUAGGGUAGGGCGCGGCU;

Her3 aptamer:

1)SEQ ID No. 195:

GGGAGCTCAGAATAAACGCTCAAAGGGTCAAGCTGATTACACTTTGTCCACTAT TGGGTCCTTCGACATGAGGCCCGGATC;

or
2)SEQ ID No. 196:

GGGAGCTCAGAATAAACGCTCAAGGCTAACAGCACGCAACGGGGGGGAGTAAT CGTGTCTGTTCGACATGAGGCCCGGATC;

or
3)SEQ ID No. 197:
CAGCGAAAGUUGCGUAUGGGUCACAUCGCAGGCACAUGUCAUCUGGGCG; or
4)SEQ ID No. 198:

GAAUUCCGCGUGUGCCAGCGAAAGUUGCGUAUGGGCCACAUCGCAGGCACA UGUCAUCUGGGCGGUCCGUUCGGGAUCC;

HMGA2 aptamer: SEQ ID No. 199: GGAAAAAATTTTTTAAAAAACCC;
H2 aptamer: SEQ ID No. 200:

GGGCCGTCGAACACGAGCATGGTGCGTGGACCTAGGATGACCTGAGTACTGTC C;

;
IFN-γ aptamer: SEQ ID No. 201:

CCGCCCAAATCCCTAAGAGAAGACTGTAATGACATCAAACCAGACACACACAC TACACACGCA;

IL-4Ra aptamer: 1)SEQ ID No. 202:

GGAGGACGAUGCGGAAAAAGCAACAGGGUGCUCCAUGCGCAUGGAACCUGC GCGCAGACGACUCGCUGAGGAUCCGAGA; or 2)SEQ ID No. 203:
AAAAAGCAACAGGGUGCUCCAUGCGCAUGGAACCUGCGCG;

IL-17 aptamer:

1)SEQ ID No. 204: CTTGGATCACCATAGTCGCTAGTCGAGGCT; or
2)SEQ ID No. 205: GCGGCATCCTATCACGCATTGACC

LAG-3 aptamer: SEQ ID No. 206:

GGGAGAGAGAUAUAAGGGCCUCCUGAUACCCGCUGCUAUCUGGACCGAUCC CAUUACCAAAUUCUCUCCC;

LZH8 aptamer: SEQ ID No. 207:

ATCCAGAGTGACGCAGCATATTAGTACGGCTTAACCCCATGGTGGACACGGTGG CTTAGT;

MUC1 aptamer:

1)SEQ ID No. 208: GCAGTTGATCCTTTGGATACCCTGG; or
2)SEQ ID No. 209: GAAGTGAAAATGACAGAACACAACA; or
3)SEQ ID No. 210:

AACCGCCCAAATCTCTAAGAGTCGGACTGCAACCTATGCTATCGTTGATGTCTGT CCAAGCAACACAGACACACTACACACACGCACA;

or
4)SEQ ID No. 211: AATGACAGAACACAACATT; or
5)SEQ ID No. 212: GCAGUUGAUCCUUUGGAUACCCUGG;

M5 aptamer: SEQ ID No. 213:

AGCAGCACAGAGGTCAGATGCTTGGTTCCACCGTACTGACTGTAGTAAAATCTG
ATCACTCCTATGCGTGCTACCGTGAA;

M7 aptamer: SEQ ID No. 214:

AGCAGCACAGAGGTCAGATGTAGTCGGTCTTCTTGTTTGAAACTGCTAATTTTG
AAAAAACCTATGCGTGCTACCGTGAA;

M1 aptamer: SEQ ID No. 215:

AGCAGCACAGAGGTCAGATGATATAACCTTAATAAATAAAATATAAATTATTTAAT
CTTACCTATGCGTGCTACCGTGAA;

MRP1 aptamer: SEQ ID No. 216:
GGGAGAAUAGUCAACAAAUCGUUUGGGGCGACUUCUCCUUCCUUUCUCCC;
N5 aptamer: SEQ ID No. 217:
GATTGAGTAGATAGTGGTTCTGTACGTAGTGAAAGAGTGG;
N-G-Dua aptamer: SEQ ID No. 218:

CAAGTTGCTCGTCGCGATACGTTTGGTTGGTGTGGTTGGCAGTATCGCAGGTCC
AAGTTGCTCGTCGCGATACAACGGAGTGTGGCTAACTCGA;

NKG2D_#-20-N-15 aptamer: SEQ ID No. 219:
CAAGTTGCTCGTCGCGATACGTTTGGTTGGTGTGGTTGGCAGTATC;
NSE aptamer: SEQ ID No. 220:
TCACACGGACCTCTCTCTACATTAATTGCGCATTTCGTT;
SARS-CoV-2-N15 aptamer: SEQ ID No. 221:

GCTGGATGTTCATGCTGGCAAAATTCCTTAGGGGCACCGTTACTTTGACACATCC
AGC;

SARS-CoV-2-N48 aptamer: SEQ ID No. 222:

GCTGGATGTCGCTTACGACAATATTCCTTAGGGGCACCGCTACATTGACACATCC
AGC;

SARS-CoV-2-N58 aptamer: SEQ ID No. 223:

GCTGGATGTCACCGGATTGTCGGACATCGGATTGTCTGAGTCATATGACACATCC
AGC;

SARS-CoV-2-N61 aptamer: SEQ ID No. 224:

GCTGGATGTTGACCTTTACAGATCGGATTCTGTGGGGCGTTAAACTGACACATC
CAGC;

OX40 aptamer:

1)SEQ ID No. 225:

GGGAGGACGATGCGGCAGTCTGCATCGTAGGAATCGCCACCGTATACTTTCCCACCAGACGACTCGCTGAGGATCCGAGA;

2)SEQ ID No. 226:

GGGAGGACGAUGCGGCAGUCUGCAUCGUAGGAAUCGCCACCGUAUACUUUCCCACCAGACGACUCGCUGAGGAUCCGAGA;

3)SEQ ID No. 227: CAGTCTGCATCGTAGGATTAGCCACCGUATCTTTCCCAC;
4)SEQ ID No. 228:

GGGAGGACGAUGCGGCAGUCUGCAUCGUAGGAAUCGCCACCGUAUACUUUCCCACCAGACGACUCGCUG;

5)SEQ ID No. 229:
CAGUCUGCAUCGUAGGAAUCGCCACCGUAUACUUUCCCAC;
6)SEQ ID No. 230:

GGGAUGCGGAAAAAAGAACACUUCCGAUUAGGGCCCACCCUAACGGCCGCAGAC;

PSMA aptamer:

1)SEQ ID No. 231: GGGAGGACGATGCGGATCAGCCATGTTTACGTCACTCCT; or
2)SEQ ID No. 232:
GCGTTTTCGCTTTTGCGTTTTGGGTCATCTGCTTACGATAGCAATGCT; or
3)SEQ ID No. 233:
GGGAGGACGAUGCGGAUCAGCCAUGUUUACGUCACUCCU; or
4)SEQ ID No. 234:
GGGACCGAAAAAGACCUGACUUCUAUACUAAGUCUACGUUCCC;

PDGFRβ aptamer:

1)SEQ ID No. 235: TGTCGTGGGGCATCGAGTAAATGCAATTCGACA; or
2)SEQ ID No. 236: UGUCGUGGGGCAUCGAGUAAAUGCAAUUCGACA;

PDGF aptamer:
SEQ ID No. 237: CAGGCTACGGCACGTAGAGCATCACCATGATCCTG;
PD-L1 aptamer: selected from any one or more of the following:

1)SEQ ID No. 238:
AACAACGTATAACAATGCCCACGTCACCAGAGTACTATGG;
2)SEQ ID No. 239: GCCCCAGTTATGCTTTCCCCCTCTGTCTCTTTG;
3)SEQ ID No. 240: ATCGCCCGCAGCACCCATTTGTTTTTTTTTG;
4)SEQ ID No. 241:
ACGGGCCACATCAACTCATTGATAGACAATGCGTCCACTGCCCGT;
5)SEQ ID No. 242:
TGCCCGCACATCAACTCATTGATAGACAATGCGTCCACTCGGGCA;
6)SEQ ID No. 243:
TGCCCGCACATCAACTCATTGATAGACAATGCGTCCACTACGGGC;
7)SEQ ID No. 244:
CGGGCACACATCAACTCATTGATAGACAATGCGTCCACTGCCCGT;
8)SEQ ID No. 245:
GTTGGTCACATCAACTCATTGATAGACAATGCGTCCACTACCAAC;

9)SEQ ID No. 246:
GGGCCACATCAACTCATTGATAGACAATGCGTCCACTGCCC,
10)SEQ ID No. 247:
TGGTTGCACATCAACTCATTGATAGACAATGCGTCCACTCAACCA;
11)SEQ ID No. 248: TACAGGTTCTGGGGGGTGGGTGGGGAACCTGTT;
12)SEQ ID No. 249: CTACGAGACGAACTTATGCGTAATAATGACTGTCGTAG;

PD1 aptamer: selected from any one or more of the following:

1)SEQ ID No. 250:

GACGATAGCGGTGACGGCACAGACGGTACAGTTCCCGTCCCTGCACTACACGTA
TGCCGCTTCCGTCCGTCGCTC;

2)SEQ ID No. 251:

GAGCGACGGACGGAAGCGGCATACGTGTAGTGCAGGGACGGGAACTGTACCGT
CTGTGCCGTCACCGCTATCGTC;

3)SEQ ID No. 252:

GGATCCTATGACGCATTGACCCGCTGCCTCTACTGAGGCTGTGTCAGTGTGCGG
CTCGGACTGTTGAATTC;

4)SEQ ID No. 253:

AGCGGTGACGGCACAGACGGTACAGTTCCCGTCCCTGCACTACACGTATGCCGC
T;

5)SEQ ID No. 254:
ACCGACAGTGAAGGACTCAGCGAACTCTCAGACTCGGTTC;

PTK-7 aptamer:
SEQ ID No. 255: ATCTAACTGCTGCGCCGCCGGGAAAATACTGTACGGTTAGA;
ProGRP-48 aptamer: SEQ ID No. 256:
CATGCGGAGTAGAGCGAGCCCAGATAGTCCCTGGTTATTTCCTTAGG;
SF aptamer:
SEQ ID No. 257: GATCTCTCTCTGCCCTAAGTCCGCACCCGTGCTTCCCTGT;
TBA15 aptamer: SEQ ID No. 258: GGTTGGTGTGGTTGG;
TBA29 aptamer: SEQ ID No. 259:
AGTCCGTGGTAGGGCAGGTTGGGGTGACT;
TFRA4 aptamer: SEQ ID No. 260: GCGTGGTACCACGC; or SEQ ID No. 261:
GCGUGGUACCACGC;
TFRA3 aptamer:

1)SEQ ID No. 262: GCGTGGTCACACGC; or
2)SEQ ID No. 263: GCGUGGUCACACGC; or
3)SEQ ID No. 264:

GCGGCGCCCACGAGCGTTCGCGTGGTCACACGCGTTCCGCCCTCCTACATAGGG
CGCATAGCCGTGGGCGCCGC;

TTA1 aptamer:

SEQ ID No. 265: CCTGCACTTGGCTTGGATTTCAGAAGGGAGACCC; or
SEQ ID No. 266: CTGCACTTGGCTTGGATTTCAGAAGGGAGACCC; or

SEQ ID No. 267: CCUGCACUUGGCTTGGAUUUCAGAAGGGAGACCC;

TLS9a aptamer:
SEQ ID No. 268: AGTCCATTTTATTCCTGAATATTTGTTAACCTCATGGAC;
TGF-β aptamer: SEQ ID No. 269:

ACATTGCTGCGTGATCGCCTCACATGGGTTTGTCTGGTCGATTTGGAGGTGGTG GGTGGC;

TIM3 aptamer: SEQ ID No. 270:

GGGAGAGGACCAGUAGCCACUAUGGUGUUGGAGCUAGCGGCAGAGCGUCGC GGUCCCUCCC; or SEQ ID No. 271:

GGGAGAGGACCAGUACUGGUAGUUCUCUGUGCGACUCCUACAGAGCGUCGC GGUCCCUCCC;

TIMC-d aptamer:
SEQ ID No. 272: AAGCAACACUUAGUCGCGAUUGAUACGUGCGCAGUCAU;
TIMC-11 aptamer: SEQ ID No. 273:

GGAGGACGAUGCGGGGAAGCAACACUUAGUCGCGAUUGAUACGUGCGCAGU CAUCAGACGACUCGCUGAGGAUCCGAGA;

TNF-α aptamer: SEQ ID No. 274:
GCGGCCGATAAGGTCTTTCCAAGCGAACGAAAA;
TNF aptamer: SEQ ID No. 275:
GCGCCACTACAGGGGAGCTGCCATTCGAATAGGTGGGCCGC;
T1 aptamer: SEQ ID No. 276:

CGCTCGATAGATCGAGCTTCGCTCGATGTGGTGTTGTGGGGGCTTGTATTGGTCG ATCACGCTCTAGAGCACTG;

Vap7 aptamer: SEQ ID No. 277: TGGTGGGGGTGGACGGGCCGGGTAGA;
VEGF aptamer:
SEQ ID No. 278: AUGCAGUUUGAGAAGUCGCGCAU; or SEQ ID No. 279: GGTGGGGGTGGACGGGCCGGGTAGA;
VEGF165 aptamer: SEQ ID No. 280: CGGAAUCAGUGAAUGCUUAUACAUCCG;
VEGF121 aptamer: SEQ ID No. 281: TGTGGGGGTGGACTGGGTGGGTACC;
VEGF-V7t1 aptamer: SEQ ID No. 282: TGTGGGGGTGGACGGGCCGGGTAGA;
VCAM-1 aptamer: SEQ ID No. 283:

ATACCAGCTTATTCAATTGGACACGGCAAAGGGGTATAGCCTACCGGACCGTGA ACATGGAATGGTGTGCTGCGTGGAGATAGTAAGTGCAATCT; or

SEQ ID No. 284:

GGACACGGCAAAGGGGTATAGCCTACCGGACCGTGAACATGGAATGGTGTGCT GCGTGG;

VCAM-12d aptamer:
SEQ ID No. 285: AGGGAATCTTGCCTAGGGAGGGAGTAGCGAAAGGGCTCA;
4-1BB aptamer: SEQ ID No. 286:

GGGAGAGAGGAAGAGGGAUGGGCGACCGAACGUGCCCUUCAAAGCCGUUCACUAACCAGUGGCAUAACCCAGAGGUCGAUAGUACUGGAUCCCCC;

PL-45 aptamer: SEQ ID No. 287:

ACTCATAGGGTTAGGGGCTGCTGGCCAGATACTCAGATGGTAGGGTTACTATGAGC;

EP166 aptamer:
SEQ ID No. 288: AACAGAGGGACAAACGGGGGAAGATTTGACGTCGACGACA;
AGC aptamer: SEQ ID No. 289:
CGACCCGGCACAAACCCAGAACCATATACACGATCATTAGTCTCCTGGGCCG;
Karpas299 aptamer: SEQ ID No. 290:
ATCCAGAGTGACGCAGCACCACCACCGTACAATTTTTTCATTACCTACTCGGC;
SW620 aptamer: SEQ ID No. 291:
CCCATCAATGTTACGACCCGCTAGGGCTGCTGTGCCATCGGGTAA;
MDA-MB-231 aptamer: SEQ ID No. 292:

AGAATTCAGTCGGACAGCGAAGTAGTTTTCCTTCTAACCTAAGAACCCGCGGCAGTTTAATGTAGATGGACGAA;

MCF-7 aptamer: SEQ ID No. 293:
GCATGGGGTTTCGGCGTTTCGTCTATCTTGTTTCTGTTAGCGTCT;
PC-3 aptamer: SEQ ID No. 294:

TGCCACTACAGCTGGTTCGGTTTGGTGACTTCGTTCTTCGTTGTGGTGCTTAGTGGC.

**10.** The nucleic acid drug according to claim 6, wherein the small-molecule compound having a targeting function is selected from the group consisting of folic acid, biotin, vitamin B12, and mannose, and is located at the 5' or 3' end of at least one of the sequence a, the sequence b, and the sequence c.

**11.** The nucleic acid drug according to any one of claims 6-10, wherein the respective different oligonucleotide effector molecules are each independently loaded onto the nucleic acid carrier by any one or more of the following:(1) a single-stranded adhesive bridge sequence;(2) sequence a complementary to the single-stranded adhesive bridge sequence;(3) a transition (spacer) sequence; wherein the single-stranded adhesive bridge sequence and the transition (spacer) sequence are those of the nucleic acid carrier recited in claim 3;

preferably, at least one of the oligonucleotide effector molecules comprises one or more nucleotide residues bearing a modification selected from at least one of: fluoro substitution (F), methyl, amino, disulfide, carbonyl, carboxyl, thiol (mercapto), aldehyde, phosphorothioate, inverted dT (3'-3' thymidine), and locked nucleic acid (LNA);
preferably, in the siRNA, the phosphate backbones at the 5' ends of the antisense and sense strands comprise phosphorothioate modification(s), and the 3' end comprises either a dTdT modification or UU overhang, or, the 3' end of the antisense strand is linked to AA, UU, or any dinucleotide combination;
preferably, the miRNA comprises phosphorothioate or locked nucleic acid modification; preferably, the nucleic acid aptamer comprises one or more modifications selected from phosphorothioate, 2'-F, and 2'-O-MOE;
preferably, the immunostimulatory nucleic acid comprises phosphorothioate modification.

**12.** The nucleic acid drug according to claim 11, wherein the nucleic acid drug is selected from any one of the following:

Drug 1): 3*CpG2006-DNA carrier, wherein the DNA carrier is group 13 of the nucleic acid carrier recited in claim 4, and the three CpG2006 molecules are respectively linked via the transition sequence to the 5' ends of the sequence a, the sequence b, and the sequence c;
preferably, the CpG2006 has the sequence SEQ ID No. 57: TCGTCGTTTTGTCGTTTTGTCGTT, with phosphorothioate modification present between adjacent nucleotides in the phosphate backbone;

preferably, the transition sequence is TTTTT;

Drug 2): 2*CpG1826-2*mannose-DNA carrier, wherein the DNA carrier is group 1 of the nucleic acid carrier recited in claim 4, the two CpG1826 molecules are respectively linked via the transition sequence to the 5' ends of the sequence a and the sequence c, and the two mannose moieties are located respectively at the 5' end and the 3' end of the sequence b;

preferably, the transition sequence is TTTTT;

preferably, the CpG1826 has the sequence: SEQ ID No. 59: TCCATGACGTTCCTGACG, with phosphorothioate modification present between adjacent nucleotides in the phosphate backbone;

Drug 3): 2*CpG1826-CD40 aptamer-DNA carrier, wherein the DNA carrier is group 1 of the nucleic acid carrier recited in claim 4, the two CpG1826 molecules are respectively linked via the transition sequence to the 5' ends of the sequence a and the sequence c, and the CD40 aptamer is linked via the transition sequence to the 5' end of the sequence b;

preferably, the CpG1826 has the sequence: SEQ ID No. 59: TCCATGACGTTCCTGACG, with phosphorothioate modification present between adjacent nucleotides in the phosphate backbone;

preferably, the transition sequence is TTTTT;

preferably, the CD40 aptamer has the sequence SEQ ID No. 154: CCAACGAGTAGGCGATAGCGCGTGG;

Drug 4): 2*CpG2006-CD40 aptamer-DNA carrier, wherein the DNA carrier is group 1 of the nucleic acid carrier recited in claim 4, the two CpG2006 molecules are respectively linked via the transition sequence to the 5' ends of the sequence a and the sequence c, and the CD40 aptamer is linked via the transition sequence to the 5' end of the sequence b;

preferably, the CpG2006 has the sequence SEQ ID No. 57: TCGTCGTTTTGTCGTTTTGTCGTT, with phosphorothioate modification present between adjacent nucleotides in the phosphate backbone;

preferably, the transition sequence is TTTTT;

preferably, the CD40 aptamer has the sequence SEQ ID No. 154: CCAACGAGTAGGCGATAGCGCGTGG;

Drug 5): 4*CpG2006-CD40 aptamer-DNA carrier, wherein the DNA carrier is group 1 of the nucleic acid carrier recited in claim 4, two CpG2006 molecules are arranged in tandem to form a CpG2006 dimer, and two such CpG2006 dimers are respectively linked to the 5' ends of the sequence a and the sequence c, and the CD40 aptamer is linked via the transition sequence to the 5' end of the sequence b;

preferably, the CpG2006 has the sequence SEQ ID No. 57: TCGTCGTTTTGTCGTTTTGTCGTT, with phosphorothioate modification present between adjacent nucleotides in the phosphate backbone;

preferably, the transition sequence is TTTTT;

preferably, the CD40 aptamer has the sequence SEQ ID No. 154: CCAACGAGTAGGCGATAGCGCGTGG;

Drug 6): 2*CpG1826-CD40 aptamer-2*mannose-DNA carrier, wherein the DNA carrier is group 1 of the nucleic acid carrier recited in claim 4, the two CpG1826 molecules are respectively linked via the transition sequence to the 5' ends of the sequence a and the sequence c, the CD40 aptamer is linked via the transition sequence to the 5' end of the sequence b, and the two mannose moieties are respectively located at the 3' ends of the sequence b and the sequence c;

preferably, the CpG1826 has the sequence SEQ ID No. 59: TCCATGACGTTCCTGACG, with phosphorothioate modification present between adjacent nucleotides in the phosphate backbone;

preferably, the transition sequence is TTTTT;

preferably, the CD40 aptamer has the sequence SEQ ID No. 154: CCAACGAGTAGGCGATAGCGCGTGG, with phosphorothioate modification present in the phosphate backbone between the first three nucleotides at the 5' end;

Drug 7): CpG1826-CD40 aptamer-mannose-MUC1 aptamer-DNA carrier, wherein the DNA carrier is group 1 of the nucleic acid carrier recited in claim 4, the CpG1826 is linked via the transition sequence to the 5' end of the sequence a, the CD40 aptamer is linked via the transition sequence to the 5' end of the sequence b, the MUC1 aptamer is linked via the transition sequence to the 5' end of the sequence c, and the mannose is located at the 3' end of the sequence c;

preferably, the CpG1826 has the sequence SEQ ID No. 59: TCCATGACGTTCCTGACG, with phosphorothioate modification present between adjacent nucleotides in the phosphate backbone;

preferably, the transition sequence is;

preferably, the CD40 aptamer has the sequence SEQ ID No. 154: CCAACGAGTAGGCGATAGCGCGTGG;

preferably, the MUC1 aptamer has the sequence SEQ ID No. 208: GCAGTTGATCCTTTGGATACCCTGG;

Drug 8): 2*CpG2006-CD40 aptamer-AmiR-21-3*mannose-DNA carrier, wherein the DNA carrier is group 1 of the nucleic acid carrier recited in claim 4, the two CpG2006 molecules are respectively linked via the transition sequence to the 5' ends of the sequence a and the sequence c, the CD40 aptamer is linked via the transition sequence to the 5' end of the sequence b, the AmiR-21 is directly linked to the 3' end of the sequence b, and the three mannose moieties are respectively linked to the 3' ends of the sequence a, the AmiR-21, and the sequence c;

preferably, the CpG2006 has the sequence SEQ ID No. 57: TCGTCGTTTTGTCGTTTTGTCGTT, with phosphorothioate modification present between adjacent nucleotides in the phosphate backbone;

preferably, the transition sequence is TTTTT;

preferably, the CD40 aptamer has the sequence SEQ ID No. 154: CCAACGAGTAGGCGATAGCGCGTGG;

preferably, the AmiR-21 has the sequence GATAAGCT, each nucleotide being LNA-modified;

Drug 9): 2*CpG2006-CD40 aptamer-CD16a aptamer-CTLA-4 aptamer-DNA carrier, wherein the DNA carrier is group 2 of the nucleic acid carrier recited in claim 4, the two CpG2006 molecules are respectively linked via the transition sequence to the 5' ends of the sequence a and the sequence c, the CD40 aptamer is linked via the transition sequence to the 3' end of the sequence b, the CD16a aptamer is linked via the transition sequence to the 5' end of the sequence b, and the CTLA-4 aptamer is linked via the transition sequence to the 3' end of the sequence c;

preferably, the CpG2006 has the sequence SEQ ID No. 57: TCGTCGTTTTGTCGTTTTGTCGTT, with phosphorothioate modification present between adjacent nucleotides in the phosphate backbone;

preferably, the transition sequence is TTTTT;

preferably, the CD16a aptamer has the sequence: SEQ ID No. 157: CCACTGCGGGGGTCTATACGTGAGG AAGAAGTGG, with a phosphorothioate modification between the first two nucleotides at the 5' end;

preferably, the CD40 aptamer has the sequence:

SEQ ID No. 154: CCAACGAGTAGGCGATAGCGCGTGG, with a phosphorothioate modification in the phosphate backbone between the last two nucleotides at the 3' end;

preferably, the CTLA-4 aptamer has the sequence:

SEQ ID No. 141: GGUGGAAGAGGGAUGGGCCGACGUGCCGCAU, with a phosphorothioate modification in the phosphate backbone between the second and third nucleotides from the 3' end;

Drug 10): 2*CpG2006-CTLA-4 aptamer-PD-L1 aptamer-DNA carrier, wherein the DNA carrier is group 3 of the nucleic acid carrier recited in claim 4; the two CpG2006 molecules are arranged in tandem to form a CpG2006 dimer, the CpG2006 dimer being linked via the transition sequence to the 5' end of the sequence a; the PD-L1 aptamer being linked via the transition sequence to the 5' end of the sequence c; and the CTLA-4 aptamer being linked via the transition sequence to the 5' end of the sequence b;

preferably, the CpG2006 has the sequence SEQ ID No. 57: TCGTCGTTTTGTCGTTTTGTCGTT, with phosphorothioate modification present between adjacent nucleotides in the phosphate backbone;

preferably, the PD-L1 aptamer has the sequence SEQ ID No. 241: ACGGGCCACATCAACTCATTGATAGAC AATGCGTCCACTGCCCGT, with a phosphorothioate modification in the phosphate backbone between the first two nucleotides at the 5' end;

preferably, the CTLA-4 aptamer has the sequence SEQ ID No. 141: GGUGGAAGAGGGAUGGGCCGACGUGCCGCAU, with a phosphorothioate modification in the phosphate backbone between the first two nucleotides at the 5' end;

preferably, the CpG2006 has the sequence SEQ ID No. 57: TCGTCGTTTTGTCGTTTTGTCGTT, with phosphorothioate modification present between adjacent nucleotides in the phosphate backbone;

preferably, the transition sequence is TTTTT;

Drug 11): CpG2006-CD40 aptamer-PD-L1 aptamer-DNA carrier, selected from any one of the following structures:

i) CCPD3: the DNA carrier is group 12 of the nucleic acid carrier recited in claim 4; the CpG2006 is linked via the transition sequence to the 5' end of the sequence a; the PD-L1 aptamer is linked via the transition sequence to the 5' end of the sequence c; and the CD40 aptamer is linked via the transition sequence to the 5' end of the sequence b; wherein, in the DNA carrier, the phosphate backbone at the 3' ends of the sequence a, the sequence b, and the sequence c comprises phosphorothioate modification at the first three internucleotide linkages from the 3' end;

preferably, the CpG2006 has the sequence SEQ ID No. 57: TCGTCGTTTTGTCGTTTTGTCGTT, with phosphorothioate modification present between adjacent nucleotides in the phosphate backbone;

preferably, the PD-L1 aptamer has the sequence SEQ ID No. 241: ACGGGCCACATCAACTCATTG ATAGACAATGCGTCCACTGCCCGT, with phosphorothioate modification at the first three internucleotide linkages at the 5' end;

preferably, the CD40 aptamer has the sequence SEQ ID No. 154: CCAACGAGTAGGCGATAGCGC GTGG, with phosphorothioate modification at the first three internucleotide linkages at the 5' end;

preferably, the transition sequence is TTTTT;

Or,

ii) CCPD3 Variant 1: the DNA carrier is group 12 of the nucleic acid carrier recited in claim 4; the CpG2006 is linked via the transition sequence TTTTT to the 5' end of the sequence a; the CD40 aptamer is linked via the transition sequence TTTTT to the 5' end of the sequence b; and the PD-L1 aptamer is linked via the transition sequence ATTT to the 5' end of the sequence c; wherein, in the DNA carrier, the phosphate backbone at the 3' ends of the sequence a, the sequence b, and the sequence c comprises phosphorothioate modification at the first two internucleotide linkages from the 3' end;

preferably, the CpG2006 has the sequence SEQ ID No. 57: TCGTCGTTTTGTCGTTTTGTCGTT, with phosphorothioate modification present between adjacent nucleotides in the phosphate backbone;
preferably, the CD40 aptamer has the sequence SEQ ID No. 154: CCAACGAGTAGGCGATAGCGC GTGG, with phosphorothioate modification at the first three internucleotide linkages at the 5' end;
preferably, the PD-L1 aptamer has the sequence SEQ ID No. 238: AACAACGTATAACAATGCCCAC GTCACCAGAGTACTATGG, with phosphorothioate modification at the first four internucleotide linkages at the 5' end;
Or,

iii) CCPD3 Variant 2: the sequences of the DNA carrier are:

sequence a: SEQ ID No. 295: GCCCACGAGCGTTCCGGGAGA;
sequence b: SEQ ID No. 49: TCTCCCGGTTCGCCGCGAG;
sequence c: SEQ ID No. 296: CTCGCGGCCATAGCCGTGGGC;
the CpG2006 is linked via the transition sequence TTTT to the 5' end of the sequence a; the CD40 aptamer is linked via the transition sequence TTTT to the 5' end of the sequence b; and the PD-L1 aptamer is linked via the transition sequence TTTT to the 5' end of the sequence c;
preferably, the CpG2006 has the sequence SEQ ID No. 57: TCGTCGTTTTGTCGTTTTGTCGTT, with phosphorothioate modification present between adjacent nucleotides in the phosphate backbone;
preferably, the CD40 aptamer has the sequence SEQ ID No. 156: GCCAACGAGTAGGCGATAGCG CGTGGC, with phosphorothioate modification at the first four internucleotide linkages at the 5' end;
preferably, the PD-L1 aptamer has the sequence SEQ ID No. 241: ACGGGCCACATCAACTCATTG ATAGACAATGCGTCCACTGCCCGT, with phosphorothioate modification at the first five internucleotide linkages at the 5' end;
Or,

iv) CCPD3 Variant 3: the sequences of the DNA carrier are:

sequence a: SEQ ID No. 295: GCCCACGAGCGTTCCGGGAGA;
sequence b: SEQ ID No. 49: TCTCCCGGTTCGCCGCGAG;
sequence c: SEQ ID No. 296: CTCGCGGCCATAGCCGTGGGC;
The CpG2006 is linked via the transition sequence TTTT to the 5' end of the sequence a; the CD40 aptamer is linked via the transition sequence TTTT to the 5' end of the sequence b; and the PD-L1 aptamer is linked via the transition sequence TTTT to the 5' end of the sequence c;
preferably, the CpG2006 has the sequence SEQ ID No. 57: TCGTCGTTTTGTCGTTTTGTCGTT, with phosphorothioate modification present between adjacent nucleotides in the phosphate backbone;
preferably, the CD40 aptamer has the sequence SEQ ID No. 156: GCCAACGAGTAGGCGATAGCG CGTGGC, with phosphorothioate modification at the first four internucleotide linkages from the 5' end;
preferably, the PD-L1 aptamer has the sequence SEQ ID No. 246: GGGCCACATCAACTCATTGAT AGACAATGCGTCCACTGCCC, with phosphorothioate modification at the first four internucleotide linkages from the 5' end;

v) CCPD3 Variant 4:
The sequences of the DNA carrier are:

sequence a: SEQ ID No. 295: GCCCACGAGCGTTCCGGGAGA;
sequence b: SEQ ID No. 49: TCTCCCGGTTCGCCGCGAG;
sequence c: SEQ ID No. 296: CTCGCGGCCATAGCCGTGGGC ;
The CpG2006 is linked via the transition sequence TTTT to the 5' end of the sequence a; the CD40 aptamer is linked via the transition sequence TTTT to the 5' end of the sequence b; and the PD-L1 aptamer is linked via the transition sequence TTTT to the 5' end of the sequence c;

preferably, the CpG2006 has the sequence SEQ ID No. 57: TCGTCGTTTTGTCGTTTTGTCGTT, with phosphorothioate modification present between adjacent nucleotides in the phosphate backbone;
preferably, the CD40 aptamer has the sequence SEQ ID No. 156: GCCAACGAGTAGGCGATAGCG CGTGGC, with phosphorothioate modification at the first four internucleotide linkages from the 5' end;
preferably, the PD-L1 aptamer has the sequence SEQ ID No. 248: TACAGGTTCTGGGGGGTGGG TGGGGAACCTGTT, with phosphorothioate modification at the first four internucleotide linkages from the 5' end;

vi) CCPD3 Variant 5:
The sequences of the DNA carrier are:

sequence a: SEQ ID No. 18: GCGACGCCCACGAGCGTTCCGGGAGAGGAGC;
sequence b: SEQ ID No. 19: GCTCCTCTCCCGGTTCGCCGCGAGCCGCG;
sequence c: SEQ ID No. 20: CAGCAGCAGCAGCACGCGGCTCGCGGCCATAGCCGTGGGCGTC GC, wherein, SEQ ID No. 12: CAGCAGCAGCAGCA is a single-stranded linker sequence;
The CpG2006 is linked via the transition sequence TTTT to the 5' end of the sequence a; the CD40 aptamer is linked via the transition sequence TTTTT to the 5' end of the sequence b; and the PD-L1 aptamer is linked via the transition sequence TTTTT to the 3' end of the complementary sequence of the single-stranded linker sequence, and is connected to the 5' end of the sequence c by complementary pairing between the single-stranded linker sequence and its complementary sequence;
preferably, the CpG2006 has the sequence SEQ ID No. 57: TCGTCGTTTTGTCGTTTTGTCGTT, with phosphorothioate modification present between adjacent nucleotides in the phosphate backbone;
preferably, the CD40 aptamer has the sequence SEQ ID No. 154: CCAACGAGTAGGCGATAGCGC GTGG, with phosphorothioate modification at the first five internucleotide linkages from the 5' end;
preferably, the complementary-sequence-of-the-single-stranded-linker-PD-L1-aptamer has the sequence SEQ ID No. 297: TGCTGCTGCTGCTGTTTTTACGGGCCACATCAACTCATTGATAGACAA TGCGTCCACT GCCCGT, with phosphorothioate modification at the first four internucleotide linkages from the 3' end; wherein, SEQ ID No. 11: TGCTGCTGCTGCTG is the complementary sequence of the single-stranded linker sequence;

vii) CCPD3 Variant 6:
The sequences of the DNA carrier are:

sequence a: SEQ ID No. 18: GCGACGCCCACGAGCGTTCCGGGAGAGGAGC;
sequence b: SEQ ID No. 19: GCTCCTCTCCCGGTTCGCCGCGAGCCGCG;
sequence c: SEQ ID No. 20:
CAGCAGCAGCAGCACGCGGCTCGCGGCCATAGCCGTGGGCGTCGC, wherein, SEQ ID No. 12: CAGCAGCAGCAGCA is a single-stranded linker sequence;
The CpG2006 is linked via the transition sequence TTTT to the 5' end of the sequence a; the CD40 aptamer is linked via the transition sequence TTTTT to the 5' end of the sequence b; and the PD-L1 aptamer is linked via the transition sequence TTTTT to the 3' end of the complementary sequence of the single-stranded linker sequence, and is connected to the 5' end of the sequence c by complementary pairing between the single-stranded linker sequence and its complementary sequence;
preferably, the CpG2006 has the sequence SEQ ID No. 57: TCGTCGTTTTGTCGTTTTGTCGTT, with phosphorothioate modification present between adjacent nucleotides in the phosphate backbone;
preferably, the CD40 aptamer has the sequence SEQ ID No. 154: CCAACGAGTAGGCGATAGCGC GTGG, with phosphorothioate modification at the first five internucleotide linkages from the 5' end;
preferably, the
complementary-sequence-of-the-single-stranded-linker-TTTTT-PD-L1-aptamer has the sequence SEQ ID No. 298:
TGCTGCTGCTGCTGTTTTTTACAGGTTCTGGGGGGTGGGTGGGGAACCTGTT, with phosphorothioate modification at the first four internucleotide linkages from the 3' end; wherein, SEQ ID No. 11: TGCTGCTGCTGCTG is the complementary sequence of the single-stranded linker sequence;

viii) CCPD3 Variant 7:

The sequences of the DNA carrier are: sequence a: SEQ ID No. 15: GACGCCCACGAGCGTTCCG GGAGAGGTGTAGCACGGTGGC, wherein, SEQ ID No. 7: TGTAGCACGGTGGC is single-stranded linker sequence A;

sequence b: SEQ ID No. 16: CCTCTCCCGGTTCGCCGCGAGCCTCGGCGCGGCCGTG, wherein, SEQ ID No. 9: TCGGCGCGGCCGTG is single-stranded linker sequence B;

sequence c:: SEQ ID No. 17: GGCTCGCGGCCATAGCCGTGGGCGTCTGCTGCTGCTGCTG, wherein, SEQ ID No. 11: TGCTGCTGCTGCTG is single-stranded linker sequence C;

The CpG2006 is linked, via a transition sequence, to the 5' end of the complementary sequence C' of the single-stranded linker sequence C, and is connected to the 3' end of the sequence c by complementary pairing between the complementary sequence C' and the single-stranded linker sequence C;

The PD-L1 aptamer is successively linked to a transition sequence and to the complementary sequence B' of the single-stranded linker sequence B, and is connected to the 3' end of the sequence b by complementary pairing between the complementary sequence B' and the single-stranded linker sequence B;

The CD40 aptamer is successively linked to a transition sequence and to the complementary sequence A' of the single-stranded linker sequence A, and is connected to the 3' end of the sequence a by complementary pairing between the complementary sequence A' and the single-stranded linker sequence A;

preferably, the CpG2006 has the sequence SEQ ID No. 57: TCGTCGTTTTGTCGTTTTGTCGTT, with phosphorothioate modification present between adjacent nucleotides in the phosphate backbone;

preferably, the CD40 aptamer has the sequence SEQ ID No. 156: GCCAACGAGTAGGCGATAGCG CGTGGC, with phosphorothioate modification at the first four internucleotide linkages from the 5' end;

preferably, the PD-L1 aptamer has the sequence SEQ ID No. 241: ACGGGCCACATCAACTCATTG ATAGACAATGCGTCCACTGCCCGT, with phosphorothioate modification at the first four internucleotide linkages from the 5' end;

preferably, the transition sequence is TTTTT;

iv) CCPD3 Variant 8:

sequence a: SEQ ID No. 18: GCGACGCCCACGAGCGTTCCGGGAGAGGAGC;

sequence b: SEQ ID No. 19: GCTCCTCTCCCGGTTCGCCGCGAGCCGCG;

sequence c: SEQ ID No. 31: CGCGGCTCGCGGCCATAGCCGTGGGCGTCGC;

The CpG2006 is linked via the transition sequence to the 5' end of the sequence a; the CD40 aptamer is linked via the transition sequence to the 5' end of the sequence b; and the PD-L1 aptamer is linked via the transition sequence to the 5' end of the sequence c;

preferably, the CpG2006 has the sequence SEQ ID No. 57: TCGTCGTTTTGTCGTTTTGTCGTT, with phosphorothioate modification present between adjacent nucleotides in the phosphate backbone;

preferably, the CD40 aptamer has the sequence SEQ ID No. 156: GCCAACGAGTAGGCGATAGCG CGTGGC, with 2'-O-MOE modification on the ribose of the first four nucleotides at the 5' end, and 5-methyl modification on the nucleobase(s) corresponding to Cm;

preferably, the PD-L1 aptamer has the sequence SEQ ID No. 241: ACGGGCCACATCAACTCATTG ATAGACAATGCGTCCACTGCCCGT, with 2'-O-MOE modification on the ribose of the first four nucleotides at the 5' end, and 5-methyl modification on the nucleobase(s) corresponding to Cm;

preferably, the transition sequence is TTTTT;

v) CCPD3 Variant 9:

sequence a: SEQ ID No. 18: GCGACGCCCACGAGCGTTCCGGGAGAGGAGC;

sequence b: SEQ ID No. 19: GCTCCTCTCCCGGTTCGCCGCGAGCCGCG;

sequence c: SEQ ID No. 31: CGCGGCTCGCGGCCATAGCCGTGGGCGTCGC;

The CpG2006 is linked via the transition sequence to the 5' end of the sequence a; the CD40 aptamer is linked via the transition sequence to the 5' end of the sequence b; and the PD-L1 aptamer is linked via the transition sequence to the 5' end of the sequence c;

preferably, the CpG2006 has the sequence SEQ ID No. 57: TCGTCGTTTTGTCGTTTTGTCGTT, with phosphorothioate modification present between adjacent nucleotides in the phosphate backbone;

preferably, the CD40 aptamer has the sequence SEQ ID No. 154: CCAACGAGTAGGCGATAGCGC GTGG, with 2'-O-MOE modification on the ribose of the first five nucleotides at the 5' end, and 5-methyl modification on the nucleobases of three nucleotides corresponding to Cm;

preferably, the PD-L1 aptamer has the sequence SEQ ID No. 241: ACGGGCCACATCAACTCATTG ATAGACAATGCGTCCACTGCCCGT, with 2'-O-MOE modification on the ribose of the first five nucleotides at the 5' end, and 5-methyl modification on the nucleobase of one nucleotide corresponding to Cm; preferably, the transition sequence is TTTTT;

vi) CCPD3 Variant 10:

sequence a: SEQ ID No. 18: GCGACGCCCACGAGCGTTCCGGGAGAGGAGC;
sequence b: SEQ ID No. 19: GCTCCTCTCCCGGTTCGCCGCGAGCCGCG;
sequence c: SEQ ID No. 31: CGCGGCTCGCGGCCATAGCCGTGGGCGTCGC;
The CpG2006 is linked via the transition sequence to the 5' end of the sequence a; the CD40 aptamer is linked via the transition sequence to the 5' end of the sequence b; and the PD-L1 aptamer is linked via the transition sequence to the 5' end of the sequence c;
preferably, the CpG2006 has the sequence SEQ ID No. 57: TCGTCGTTTTGTCGTTTTGTCGTT, with phosphorothioate modification present between adjacent nucleotides in the phosphate backbone;
preferably, the CD40 aptamer has the sequence SEQ ID No. 154: CCAACGAGTAGGCGATAGCGCGTGG, with 2'-O-MOE modification on the ribose of the first five nucleotides at the 5' end, and 5-methyl modification on the nucleobases of three nucleotides corresponding to Cm;
preferably, the PD-L1 aptamer has the sequence SEQ ID No. 249: CTACGAGACGAACTTATGCGTAATAATGACTGTCGTAG, with 2'-O-MOE modification on the ribose of the first five nucleotides at the 5' end, and 5-methyl modification on the nucleobase of one nucleotide corresponding to Cm;
preferably, the transition sequence is TTTTT;

vii) CCPD3 Variant 11:

sequence a: SEQ ID No. 18: GCGACGCCCACGAGCGTTCCGGGAGAGGAGC;
sequence b: SEQ ID No. 19: GCTCCTCTCCCGGTTCGCCGCGAGCCGCG;
sequence c: SEQ ID No. 299: <u>CGCGGCTCGCGGCT</u>CGCGGCTCGCGGCCATAGCCGTGGGCGTCGC, the underlined portion being a linker sequence;
The CpG2006 is linked via the transition sequence to the 5' end of the sequence a; the CD40 aptamer is linked via the transition sequence to the 5' end of the sequence b; and the PD-L1 aptamer is linked to the 3' end of the complementary sequence of the linker sequence, SEQ ID No. 14: AGCCGCGAGCCGCG, and is connected to the 5' end of the sequence c by complementary pairing between the complementary sequence of the linker sequence and the linker sequence;
preferably, the CpG2006 has the sequence SEQ ID No. 57: TCGTCGTTTTGTCGTTTTGTCGTT, with phosphorothioate modification present between adjacent nucleotides in the phosphate backbone;
preferably, the CD40 aptamer has the sequenceSEQ ID No. 154: CCAACGAGTAGGCGATAGCGCGTGG, with 2'-O-MOE modification on the ribose of the first five nucleotides at the 5' end;
preferably, the PD-L1 aptamer has the sequence SEQ ID No. 241: ACGGGCCACATCAACTCATTGATAGACAATGCGTCCACTGCCCGT, with 2'-O-MOE modification on the ribose of the first 1-5 nucleotides at the 3' end;
preferably, the transition sequence is TTTTT.

viii) CCPD3 Variant 12:

sequence a: SEQ ID No. 18: GCGACGCCCACGAGCGTTCCGGGAGAGGAGC;
sequence b: SEQ ID No. 19: GCTCCTCTCCCGGTTCGCCGCGAGCCGCG;
sequence c: SEQ ID No. 299: <u>CGCGGCTCGCGGCT</u>CGCGGCTCGCGGCCATAGCCGTGGGCGTCGC, the underlined portion being a linker sequence;
The CpG2006 is linked via a transition sequence to the 5' end of the sequence a; the CD40 aptamer is linked via a transition sequence to the 5' end of the sequence b; and the PD-L1 aptamer is linked via a transition sequence to the 3' end of the complementary sequence of the linker sequence,SEQ ID No. 14: AGCCGCGAGCCGCG, and is connected to the 5' end of the sequence c by complementary pairing between the complementary sequence of the linker sequence and the linker sequence;
preferably, the CpG2006 has the sequence SEQ ID No. 57: TCGTCGTTTTGTCGTTTTGTCGTT, with phosphorothioate modification present between adjacent nucleotides in the phosphate backbone;
preferably, the CD40 aptamer has the sequence SEQ ID No. 154: CCAACGAGTAGGCGATAGCGCGTGG, with 2'-O-MOE modification on the ribose of the first five nucleotides at the 5' end;
preferably, the PD-L1 aptamer has the sequence SEQ ID No. 249: CTACGAGACGAACTTATGCGTAATAATGACTGTCGTAG, with 2'-O-MOE modification on the ribose of the first 1-5 nucleotides at the 3' end;

preferably, the transition sequence is TTTTT;

Drug 12): CD16a aptamer-CTLA-4 aptamer-CpG2006-DNA carrier, wherein the DNA carrier is group 4 of the nucleic acid carrier recited in claim 4; the CD16a aptamer is linked via a transition sequence to the 5' end of the sequence a; the CTLA-4 aptamer is linked via a transition sequence to the 5' end of the sequence b; and the CpG2006 is linked via a transition sequence to the 5' end of the sequence c; wherein, in the DNA carrier, the phosphate backbone at the 3' ends of the sequence a, the sequence b, and the sequence c comprises phosphorothioate modification at the first two internucleotide linkages from the 3' end;

preferably, the CD16a aptamer has the sequence:

SEQ ID No. 157: CCACTGCGGGGGTCTATACGTGAGGAAGAAGTGG, with a phosphorothioate modification between the first two nucleotides at the 5' end;

preferably, the CTLA-4 aptamer has the sequence:

SEQ ID No. 141: GGUGGAAGAGGGAUGGGCCGACGUGCCGCAU, with a phosphorothioate modification between the first two nucleotides at the 5' end, C and U being 2'-F-modified, and A and G being 2'-OMe (2'-O-methyl)-modified;

preferably, the CpG2006 has the sequence SEQ ID No. 57: TCGTCGTTTTGTCGTTTTGTCGTT, with phosphorothioate modification present between adjacent nucleotides in the phosphate backbone;

preferably, the transition sequence is TTTTT;

Drug 13): 2*CpG2006-CD40 aptamer-PD-L1 aptamer-C12 aptamer-DNA carrier, wherein the DNA carrier is group 5 of the nucleic acid carrier recited in claim 4; the two CpG2006 molecules are respectively linked via a transition sequence to the 5' ends of the sequence a and the sequence c; the CD40 aptamer is located at the 5' end of the sequence b; wherein, in the DNA carrier, the phosphate backbone at the 3' ends of the sequence a, the sequence b, and the sequence c comprises phosphorothioate modification at the first two internucleotide linkages from the 3' end;

The PD-L1 aptamer is successively linked to the transition sequence and to the complementary sequence B' of the single-stranded linker sequence B, the single-stranded linker sequence B being located at the 3' end of the sequence b; the PD-L1 aptamer is connected at the 3' end of the sequence b by complementary base pairing between the complementary sequence B' and the single-stranded linker sequence B,

The C12 aptamer is successively linked to the transition sequence and to the complementary sequence C' of the single-stranded linker sequence C, the single-stranded linker sequence C being located at the 3' end of the sequence c; the C12 aptamer is connected at the 3' end of the sequence c by complementary base pairing between the complementary sequence C' and the single-stranded linker sequence C;

preferably, the CpG2006 has the sequence SEQ ID No. 57: TCGTCGTTTTGTCGTTTTGTCGTT, with phosphorothioate modification present between adjacent nucleotides in the phosphate backbone;

preferably, the transition sequence is TTTTT;

preferably, the CD40 aptamer has the sequence:

SEQ ID No. 154: CCAACGAGTAGGCGATAGCGCGTGG, with phosphorothioate modification at the first three internucleotide linkages at the 5' end;

preferably, the PD-L1 aptamer has the sequence:

SEQ ID No. 241: ACGGGCCACATCAACTCATTGATAGACAATGCGTCCACTGCCCGT, with phosphorothioate modification at the first three internucleotide linkages at the 5' end;

preferably, the C12 aptamer has the sequence:

SEQ ID No. 164: GTGGATTGTTGTGTTCTGTTGGTTTTTGTGTTGTC, with phosphorothioate modification at the first three internucleotide linkages at the 5' end;

preferably, the single-stranded linker sequence B is SEQ ID No. 7: TGTAGCACGGTGGC, , and the complementary sequence B' is SEQ ID No. 8: GCCACCGTGCTACA;

preferably, the single-stranded linker sequence C is SEQ ID No. 11: TGCTGCTGCTGCTG, , and the complementary sequence C' is SEQ ID No. 12: CAGCAGCAGCAGCA;

Drug 14): CpG2006-C12 aptamer-CD47 siRNA-PD-L1 aptamer-PD-L1 siRNA-DNA carrier, wherein the DNA carrier is group 6 of the nucleic acid carrier recited in claim 4; the CpG2006 is linked via a transition sequence to the 5' end of the sequence a; the C12 aptamer is linked via a transition sequence to the antisense strand of the CD47 siRNA; the sense strand of the CD47 siRNA is located at the 5' end of the sequence b; the antisense strand of the CD47 siRNA hybridizes complementarily to the sense strand to connect the C12 aptamer and the CD47 siRNA at the 5' end of the sequence b; the sense strand of the PD-L1 siRNA is linked to the 5' end of the sequence c; and the PD-L1 aptamer is linked via a transition sequence to the antisense strand of the PD-L1 siRNA; wherein, in the DNA carrier, the phosphate backbone at the 3' ends of the sequence a, the sequence b, and the sequence c comprises phosphorothioate modification at the first three internucleotide linkages from the 3' end;

preferably, the C12 aptamer has the sequence:

SEQ ID No. 164: GTGGATTGTTGTGTTCTGTTGGTTTTTGTGTTGTC, with phosphorothioate modification at the first three internucleotide linkages at the 5' end;

preferably, the CD47 siRNA comprises an antisense strand SEQ ID No. 79: AUAUCUCUGGGUAAUCACCUU and a sense strand SEQ ID No. 78: GGUGAUUACCCAGAGAUAUUU; the antisense strand has phosphorothioate modification at the first three internucleotide linkages at the 5' end; and the sense strand has phosphorothioate modification at the first three internucleotide linkages at the 5' end and at the first three internucleotide linkages at the 3' end;

preferably, the PD-L1 aptamer has the sequence:

SEQ ID No. 239: GCCCCAGTTATGCTTTCCCCCTCTGTCTCTTTG, with phosphorothioate modification at the first three internucleotide linkages at the 5' end;

preferably, the PD-L1 siRNA comprises a sense strand SEQ ID No. 94: CCAGCACACUGAGAAUCAAUU and an antisense strand SEQ ID No. 95: UUGAUUCUCAGUGUGCUGGUU; the sense strand has phosphorothioate modification at the first three internucleotide linkages at the 5' end and at the first three internucleotide linkages at the 3' end; and the antisense strand has phosphorothioate modification at the first three internucleotide linkages at the 5' end;

preferably, the CpG2006 has the sequence SEQ ID No. 57: TCGTCGTTTTGTCGTTTTGTCGTT, with phosphorothioate modification present between adjacent nucleotides in the phosphate backbone;

preferably, the transition sequence is TTTTT;

Drug 15): 2*CpG2006-CD40 aptamer-CD16a aptamer-DNA carrier, wherein the DNA carrier is group 7 of the nucleic acid carrier recited in claim 4; the two CpG2006 molecules are respectively linked via the transition sequence to the 5' ends of the sequence a and the sequence c; the CD40 aptamer is linked via the transition sequence to the 5' end of the sequence b; and the CD16a aptamer is linked via the transition sequence to the 3' end of the sequence b; wherein, in the DNA carrier, the phosphate backbone at the 3' ends of the sequence a and the sequence c comprises phosphorothioate modification at the first two internucleotide linkages from the 3' end;

preferably, the CpG2006 has the sequence SEQ ID No. 57: TCGTCGTTTTGTCGTTTTGTCGTT, with phosphorothioate modification present between adjacent nucleotides in the phosphate backbone;

preferably, the transition sequence is TTTTT;

preferably, the CD16a aptamer has the sequence:

SEQ ID No. 157: CCACTGCGGGGGTCTATACGTGAGGAAGAAGTGG, with phosphorothioate modification at the first two internucleotide linkages from the 3' end;

preferably, the CD40 aptamer has the sequence:

SEQ ID No. 154: CCAACGAGTAGGCGATAGCGCGTGG, with phosphorothioate modification in the phosphate backbone between the terminal two nucleotides at the 5' end;

Drug 16): 2*CpG2006-CD40 aptamer-CD16a aptamer-FAP aptamer-DNA carrier, wherein the DNA carrier is group 8 of the nucleic acid carrier recited in claim 4; the two CpG2006 molecules are respectively linked via the transition sequence to the 5' ends of the sequence a and the sequence c; the CD40 aptamer is linked via the transition sequence to the 5' end of the sequence b; the CD16a aptamer is linked via the transition sequence to the 3' end of the sequence b; and the FAP aptamer is linked via the transition sequence to the 3' end of the sequence c; wherein, in the DNA carrier, the phosphate backbone at the 3' end of the sequence a comprises phosphorothioate modification at the first two internucleotide linkages from the 3' end;

preferably, the CpG2006 has the sequence SEQ ID No. 57: TCGTCGTTTTGTCGTTTTGTCGTT, with phosphorothioate modification present between adjacent nucleotides in the phosphate backbone;

preferably, the transition sequence is TTTTT;

preferably, the CD16a aptamer has the sequence:

SEQ ID No. 157: CCACTGCGGGGGTCTATACGTGAGGAAGAAGTGG, with phosphorothioate modification at the first two internucleotide linkages from the 3' end;

preferably, the CD40 aptamer has the sequence:

SEQ ID No. 154: CCAACGAGTAGGCGATAGCGCGTGG, with phosphorothioate modification in the phosphate backbone between the terminal two nucleotides at the 5' end;

preferably, the FAP aptamer has the sequence:

SEQ ID No. 188: CCGCTCGAGCTAGTCTGACAAAGAGAAACAC, with phosphorothioate modification in the phosphate backbone between the terminal two nucleotides at the 3' end;

Drug 17): 2*CpG2006-VEGF aptamer-CD40 aptamer-PD-L1 aptamer-CD16a aptamer-DNA carrier, wherein the DNA carrier is group 6 of the nucleic acid carrier recited in claim 4; the two CpG2006 molecules are respectively linked via the transition sequence to the 5' ends of the sequence a and the sequence c; the CD40 aptamer is linked via the transition sequence to the 5' end of the sequence b; the PD-L1 aptamer is linked via the transition sequence to the 3' end of the sequence b; the CD16a aptamer is linked via the transition sequence to the 3' end of the sequence c; and the VEGF aptamer is linked via the transition sequence to the 3' end of the sequence a;

preferably, the CpG2006 has the sequence SEQ ID No. 57: TCGTCGTTTTGTCGTTTTGTCGTT, with phosphorothioate modification present between adjacent nucleotides in the phosphate backbone;

preferably, the VEGF aptamer has the sequence:

SEQ ID No. 279: GGTGGGGGTGGACGGGCCGGGTAGA, with a phosphorothioate modification in the phosphate backbone between the terminal two nucleotides at the 3' end;

preferably, the CD40 aptamer has the sequence:

SEQ ID No. 154: CCAACGAGTAGGCGATAGCGCGTGG, with a phosphorothioate modification in the phosphate backbone between the first two nucleotides at the 5' end;

preferably, the PD-L1 aptamer has the sequence SEQ ID No. 241: ACGGGCCACATCAACTCATTG ATAGACAATGCGTCCACTGCCCGT, with a phosphorothioate modification in the phosphate backbone between the terminal two nucleotides at the 3' end;

preferably, the CD16a aptamer has the sequence SEQ ID No. 157: CCACTGCGGGGGTCTATACG TGAGGAAGAAGTGG, with a phosphorothioate modification in the phosphate backbone between the terminal two nucleotides at the 3' end;

Drug 18): 2*AmiR-21-TTA1 aptamer-DNA carrier, wherein the DNA carrier is group 13 of the nucleic acid carrier recited in claim 4; two AmiR-21 moieties are directly linked to the 5' ends of the sequence a and the sequence c, respectively; and the TTA1 aptamer is linked via a transition sequence to the 5' end of the sequence b;

preferably, the transition sequence is TTTTT;

preferably, the AmiR-21 has the sequence GATAAGCT, each nucleotide being LNA-modified;

preferably, the TTA1 aptamer has the sequence:

SEQ ID No. 266: CTGCACTTGGCTTGGATTTCAGAAGGGAGACCC;

Drug 19): 2*AmiR-21-3×biotin-DNA carrier, wherein the DNA carrier is group 13 of the nucleic acid carrier recited in claim 4; two AmiR-21 moieties are respectively directly linked to the 5' ends of the sequence a and the sequence c; and three biotin moieties are respectively linked to the 5' end and the 3' end of the sequence b and to the 3' end of the sequence c;

preferably, the AmiR-21 has the sequence GATAAGCT, each nucleotide being LNA-modified;

Drug 20): 2*AmiR-21-4*biotin-DNA carrier, wherein the DNA carrier is group 13 of the nucleic acid carrier recited in claim 4; two AmiR-21 moieties are respectively directly linked to the 5' ends of the sequence a and the sequence c; and four biotin moieties are respectively linked to the 3' end of the sequence a, the 5' end and the 3' end of the sequence b, and the 3' end of the sequence c;

preferably, the AmiR-21 has the sequence GATAAGCT, each nucleotide being LNA-modified;

Drug 21): 2*AmiR-21-AS1411 aptamer-DNA carrier, wherein the DNA carrier is group 13 of the nucleic acid carrier recited in claim 4; two AmiR-21 moieties are respectively directly linked to the 5' ends of the sequence a and the sequence c; and the AS1411 aptamer is linked via a transition sequence to the 5' end of the sequence b;

preferably, the transition sequence is TTTTT;

preferably, the AmiR-21 has the sequence GATAAGCT, each nucleotide being LNA-modified;

preferably, the AS 1411 aptamer has the sequence:

SEQ ID No. 131: GGTGGTGGTGGTTGTGGTGGTGGTGG;

Drug 22): 2*AmiR-21-AS1411 aptamer-DNA carrier, wherein the DNA carrier is group 23 of the nucleic acid carrier recited in claim 4; two AmiR-21 moieties are respectively directly linked to the 5' ends of the sequence a and the sequence c; and the AS1411 aptamer is linked via a transition sequence to the 5' end of the sequence b;

preferably, the transition sequence is TTTTT;

preferably, the AmiR-21 has the sequence GATAAGCT, with 2'-OMe (2'-O-methyl) modification at positions 1 and 3-5 and 2'-F modification at positions 2 and 6-8;

preferably, the AS 1411 aptamer has the sequence:

SEQ ID No. 131: GGTGGTGGTGGTTGTGGTGGTGGTGG;

Drug 23): 2*AmiR-21-AS1411 aptamer-biotin-DNA carrier, wherein the DNA carrier is group 13 of the nucleic acid carrier recited in claim 4; two AmiR-21 moieties are respectively directly linked to the 5' ends of the sequence a and the sequence c; the AS 1411 aptamer is linked via a transition sequence to the 5' end of the sequence b; and biotin is linked to the 3' end of the sequence c;

preferably, the transition sequence is TTTTT;

preferably, the AmiR-21 has the sequence GATAAGCT, each nucleotide being LNA-modified;

preferably, the AS 1411 aptamer has the sequence:

SEQ ID No. 131: GGTGGTGGTGGTTGTGGTGGTGGTGG;

Drug 24): 2*AmiR-21-CD40 aptamer-DNA carrier, wherein the DNA carrier is group 13 of the nucleic acid carrier recited in claim 4; two AmiR-21 moieties are respectively directly linked to the 5' ends of the sequence a and the sequence c; and the CD40 aptamer is linked via a transition sequence to the 5' end of the sequence b;

preferably, the transition sequence is TTTTT;

preferably, the AmiR-21 has the sequence GATAAGCT, each nucleotide being LNA-modified;

preferably, the CD40 aptamer has the sequence SEQ ID No. 154: CCAACGAGTAGGCGATAGCGCGTGG;

Drug 25): 2*AmiR-21-MUC1 aptamer-3×biotin-DNA carrier, wherein the DNA carrier is group 13 of the nucleic acid carrier recited in claim 4; two AmiR-21 moieties are respectively directly linked to the 5' ends of the sequence a and the sequence c; and the MUC1 aptamer is linked via a transition sequence to the 5' end of the sequence b;

preferably, the transition sequence is TTTTT;

preferably, the AmiR-21 has the sequence GATAAGCT, each nucleotide being LNA-modified;

preferably, the MUC1 aptamer has the sequence SEQ ID No. 208: GCAGTTGATCCTTTGGATACCCTGG;

Drug 26): AmiR-21-AS1411 aptamer-A15 aptamer-2*biotin-DNA carrier, wherein the DNA carrier is group 13 of the nucleic acid carrier recited in claim 4; the AmiR-21 is directly linked to the 5' end of the sequence a; the AS1411 aptamer and the A15 aptamer are respectively linked via a transition sequence to the 5' ends of the sequence b and the sequence c; and the two biotin moieties are respectively linked to the 3' ends of the sequence b and the sequence c;

preferably, the transition sequence is TTTTT;

preferably, the AmiR-21 has the sequence GATAAGCT, each nucleotide being LNA-modified;

preferably, the AS 1411 aptamer has the sequence SEQ ID No. 131: GGTGGTGGTGGTTGTGGTGGTGGTGG;

preferably, the A15 aptamer has the sequence SEQ ID No. 129: CCCTCCTACATAGGG;

Drug 27): 2*mannose-2*AmiR-21-DNA carrier, wherein the DNA carrier is group 13 of the nucleic acid carrier recited in claim 4; the two mannose moieties are respectively linked to the 5' end and the 3' end of the sequence b; and the two AmiR-21 moieties are respectively directly linked to the 5' ends of the sequence a and the sequence c;

preferably, the AmiR-21 has the sequence GATAAGCT, each nucleotide being LNA-modified;

Drug 28): 2*mannose-A15 aptamer-2*AmiR-21-DNA carrier, wherein the DNA carrier is group 13 of the nucleic acid carrier recited in claim 4; the A15 aptamer is linked via a transition sequence to the 5' end of the sequence b; the two mannose moieties are respectively linked to the 5' end of the A15 aptamer and to the 3' end of the sequence b; and the two AmiR-21 moieties are respectively directly linked to the 5' ends of the sequence a and the sequence c;

preferably, the AmiR-21 has the sequence GATAAGCT, each nucleotide being LNA-modified;

preferably, the A15 aptamer has the sequence SEQ ID No. 129: CCCTCCTACATAGGG;

Drug 29): 2*mannose-survivin siRNA-DNA carrier, wherein the DNA carrier is group 13 of the nucleic acid carrier recited in claim 4; the two mannose moieties are respectively linked to the 5' end and the 3' end of the sequence b; the sense strand of the survivin siRNA is linked to the 3' end of the sequence c; and the antisense strand of the survivin siRNA is connected at the 3' end of the sequence c by complementary pairing to the sense strand;

preferably, the survivin siRNA comprises a sense strand SEQ ID No. 104: UGCAGGUUCCUUAUCUGUCATT, and an antisense strand SEQ ID No. 105: UGACAGAUAAGGAACCUGCTT, the TT at the 3' ends of both the sense and antisense strands being dTdT-modified;

Drug 30): 2*mannose-A15 aptamer-survivin siRNA-DNA carrier, wherein the DNA carrier is group 22 of the nucleic acid carrier recited in claim 4; the A15 aptamer is linked via the transition sequence TTTTT to the 5' end of the sequence b; the two mannose moieties are respectively linked to the 5' end of the A15 aptamer and to the 3' end of the sequence b; the sense strand of the survivin siRNA is linked to the 3' end of the sequence c; and the antisense strand of the survivin siRNA is connected at the 3' end of the sequence c by complementary pairing to the sense strand;

preferably, the sense strand of the survivin siRNA is SEQ ID No. 104: UGCAGGUUCCUUAUCUGUCATT;

The antisense strand is SEQ ID No. 105: UGACAGAUAAGGAACCUGCTT; and the TT at the 3' ends of both the sense and antisense strands are dTdT-modified;

preferably, the A15 aptamer has the sequence SEQ ID No. 129: CCCTCCTACATAGGG;

Drug 31): AS1411 aptamer-survivin siRNA-PSMA aptamer-3*AmiR-21-RNA carrier, wherein the DNA carrier is group 16 of the nucleic acid carrier recited in claim 4; the PSMA aptamer is linked via the transition sequence AA to the 3' end of the sequence a; three AmiR-21 moieties are linked in tandem via the transition sequence AA to the 3' end of the sequence b; the sense strand of the survivin siRNA is linked via the transition sequence AAA to the 3' end of the sequence c; and the AS 1411 aptamer is linked via the transition sequence TT to the antisense strand of the survivin siRNA, and is connected to the 3' end of the sequence c by complementary pairing between the antisense and sense strands of the survivin siRNA; wherein, in the DNA carrier, the sequences a, b, and c each independently comprise a phosphorothioate modification between the first two nucleotides at the 5' end;

preferably, the AS 1411 aptamer has the sequence SEQ ID No. 131: GGTGGTGGTGGTTGTGGTGGTGGTGG;

preferably, the survivin siRNA comprises a sense strand SEQ ID No. 100: GCAGGUUCCUUAUCUGUCACAUU and an antisense strand SEQ ID No. 300: UGUGACAGAUAAGGAACCUGCAG, the sense and antisense strands each having phosphorothioate modification at the first three internucleotide linkages at the 5' end and at the terminal three internucleotide linkages at the 3' end;

preferably, the PSMA aptamer has the sequence SEQ ID No. 234: GGGACCGAAAAAGACCUGACUUCUAUACUAAGUCUACGUUCCC, with a phosphorothioate modification in the phosphate backbone between the terminal two nucleotides at the 3' end;

preferably, the AmiR-21 has the sequence GAUAAGCU, with phosphorothioate modification present between adjacent nucleotides in the phosphate backbone;

Drug 32): 3*AmiR-21-ATP aptamer-FGF2 aptamer-DNA carrier, wherein the DNA carrier is group 15 of the nucleic acid carrier recited in claim 4; three AmiR-21 moieties are directly linked in tandem to the 5' end of the sequence a; the ATP aptamer is linked via the transition sequence TTTTT to the 5' end of the sequence b; and the FGF2 aptamer is linked via U to the 5' end of the sequence c; wherein, in the DNA carrier, the sequences a, b, and c each independently comprise a phosphorothioate modification between the first two nucleotides at the 3' end;

preferably, the ATP aptamer has the sequence SEQ ID No. 135: ACCTGGGGAGTATTGGGGAGGA AGG, with a phosphorothioate modification in the phosphate backbone between the first two nucleotides at the 5' end;

preferably, the FGF2 aptamer has the sequence SEQ ID No. 183: GGGAUACUAGGGCAUUAAUGUUACCAGUGUAGUCCC, with a phosphorothioate modification in the phosphate backbone between the first two nucleotides at the 5' end;

preferably, the AmiR-21 has the sequence GATAAGCT, with phosphorothioate modification present between adjacent nucleotides in the phosphate backbone;

Drug 33): AS1411 aptamer-survivin siRNA-PSMA aptamer-3 *AmiR-21-DNA-RNA hybrid carrier, wherein the DNA-RNA hybrid carrier is group 18 of the nucleic acid carrier recited in claim 4; the PSMA aptamer is linked via the transition sequence AA to the 3' end of the sequence a; three AmiR-21 moieties are linked in tandem via the transition sequence AA to the 3' end of the sequence b; the sense strand of the survivin siRNA is linked via the transition sequence AAAA to the 3' end of the sequence c; the AS1411 aptamer is linked via the transition sequence AA to the 5' end of the antisense strand of the survivin siRNA; and the antisense strand is connected by complementary pairing to the sense strand of the survivin siRNA; wherein, in the carrier, the sequences b and c each independently comprise a phosphorothioate modification between the first two nucleotides at the 5' end;

preferably, the AS 1411 aptamer has the sequence SEQ ID No. 131: GGTGGTGGTGGTTGTGGTG GTGGTGG, with a phosphorothioate modification in the phosphate backbone between the first two nucleotides at the 5' end;

preferably, the survivin siRNA comprises an antisense strand SEQ ID No. 300: UGUGACAGAUAAGGAACCUGCAG and a sense strand SEQ ID No. 100: GCAGGUUCCUUAUCUGUCACAUU, each strand having phosphorothioate modification at the first three internucleotide linkages at the 5' end and at the terminal three internucleotide linkages at the 3' end;

preferably, the PSMA aptamer has the sequence SEQ ID No. 234: GGGACCGAAAAAGACCUGACUUCUAUACUAAGUCUACGUUCCC, with 2'-F modification at C and U, 2'-OMe (2'-O-methyl) modification at A and G, and a phosphorothioate modification between the first two nucleotides at the 3' end;

preferably, the AmiR-21 has the sequence GAUAAGCU, with phosphorothioate modification present between adjacent nucleotides in the phosphate backbone;

Drug 34): 3*AmiR-21-AS1411 aptamer-FAP aptamer-DNA carrier, wherein the RNA carrier is group 15 of the nucleic acid carrier recited in claim 4; three AmiR-21 moieties are linked in tandem via the transition sequence TTTTTT to the 5' end of the sequence a; the AS 1411 aptamer is linked via the transition sequence TTTTT to the 5' end of the sequence b; and the FAP aptamer is linked via the transition sequence TTTTT to the 5' end of the sequence c;

preferably, the AmiR-21 has the sequence GATAAGCT, with phosphorothioate modification present between adjacent nucleotides in the phosphate backbone;

preferably, the AS 1411 aptamer has the sequence SEQ ID No. 131: GGTGGTGGTGGTTGTGGTG GTGGTGG, with a phosphorothioate modification between the first two nucleotides at the 5' end;

preferably, the FAP aptamer has the sequence SEQ ID No. 188: CCGCTCGAGCTAGTCTGACAAA GAGAAACAC, with a phosphorothioate modification between the first two nucleotides at the 5' end;

Drug 35): AS1411 aptamer-survivin siRNA-EpCAM aptamer-3 *AmiR-21-DNA carrier, wherein the DNA carrier is group 14 of the nucleic acid carrier recited in claim 4; the AS 1411 aptamer is linked via the transition sequence AA to the 5' end of the antisense strand of the survivin siRNA; the sense strand of the survivin siRNA is linked via the transition sequence AAAA to the 3' end of the sequence c; the antisense and sense strands of the survivin siRNA are connected by complementary pairing; three AmiR-21 moieties are linked in tandem via the transition sequence AA to the 3' end of the sequence b; and the EpCAM aptamer is linked via the transition sequence AA to the 3' end of the sequence a; wherein, in the DNA carrier, the phosphate backbone between the first two nucleotides at the 5' end of the sequence b comprises a phosphorothioate modification;

preferably, the AS 1411 aptamer has the sequence SEQ ID No. 131: GGTGGTGGTGGTTGTGGTG GTGGTGG, with a phosphorothioate modification between the first two nucleotides at the 5' end;

preferably, the survivin siRNA comprises an antisense strand SEQ ID No. 300: UGUGACAGAUAAGGAACCUGCAG and a sense strand SEQ ID No. 100: GCAGGUUCCUUAUCUGUCACAUU; both the sense and antisense strands comprise phosphorothioate modifications at the first three internucleotide linkages at the 5' end and at the terminal three internucleotide linkages at the 3' end; for the antisense strand, positions 2, 6, 8, 9, 12, 14, and 16 are 2'-F-modified and the remaining positions are 2'-OMe (2'-O-methyl)-modified; for the sense strand, positions 7, 9, 10, and 11 are 2'-F-modified and the remaining positions are 2'-OMe-modified;

preferably, the AmiR-21 has the sequence GATAAGCT, with phosphorothioate modifications present between adjacent nucleotides in the phosphate backbone;

preferably, the EpCAM aptamer has the sequence SEQ ID No. 179: GCGACUGGUUACCCGGUCG, with 2'-F modification at C and U and 2'-OMe modification at A and G, and with phosphorothioate modifications at the first three internucleotide linkages at the 5' end and at the terminal three internucleotide linkages at the 3' end;

Drug 36): IL-4RA aptamer-TGF-β aptamer-PD-L1 aptamer-2*AmiR-21-DNA carrier, wherein the DNA carrier is group 15 of the nucleic acid carrier recited in claim 4; the IL-4RA aptamer is linked via one AmiR-21 to the 5' end of the sequence a; the TGF-β aptamer is linked via the transition sequence TTTTT to the 5' end of the sequence b; and the PD-L1 aptamer is linked via one AmiR-21 to the 5' end of the sequence c; wherein, in the DNA carrier, the sequences a, b, and c each independently comprise a phosphorothioate (PT) modification between the first two nucleotides at the 3' end;

preferably, the IL-4RA aptamer has the sequence SEQ ID No. 203: AAAAAGCAACAGGGUGCUCCAUGCGCAUGGAACCUGCGCG, with PT modification at the first four internucleotide linkages from the 5' end;

preferably, the TGF-β aptamer has the sequence SEQ ID No. 269: ACATTGCTGCGTGATCGCCTC ACATGGGTTTGTCTGGTCGATTTGGAGGTGGTGGGT GGC, with PT modification at the first four internucleotide linkages from the 5' end;

preferably, the PD-L1 aptamer has the sequence SEQ ID No. 241: ACGGGCCACATCAACTCATTG ATAGACAATGCGTCCACTGCCCGT, with PT modification at the first four internucleotide linkages from the 5' end;

preferably, the AmiR-21 has the sequence GATAAGCT, with PT modification present between adjacent nucleotides in the phosphate backbone;

Drug 37): AS1411 aptamer-ATAD2 siRNA-GPC3 aptamer-5*AmiR-21-DNA carrier, wherein the DNA carrier is group 19 of the nucleic acid carrier recited in claim 4; the AS 1411 aptamer is linked via one AmiR-21 to the 5' end of the antisense strand of the ATAD2 siRNA; the sense strand of the ATAD2 siRNA is linked via one AmiR-21 to the 3' end of the sequence c; the antisense and sense strands of the ATAD2 siRNA are connected by complementary pairing; one AmiR-21 is linked to the 3' end of the sequence a;

the GPC3 aptamer is linked via one AmiR-21 to the 5' end of the sequence b; and one AmiR-21 is linked to the 5' end of the sequence c; wherein, in the DNA carrier, the sequence b comprises a PT modification between the first two nucleotides at the 5' end;

preferably, the AS 1411 aptamer has the sequence SEQ ID No. 131: GGTGGTGGTGGTTGTGGTG GTGGTGG, with PT modification between the first two nucleotides at the 5' end;

preferably, the ATAD2 siRNA comprises an antisense strand SEQ ID No. 68: GCGUCGAAGUUGUAGGAUUUU and a sense strand SEQ ID No. 69: AAUCCUACAACUUCGACGCUU, the sense and antisense strands each having PT modification at the first three internucleotide linkages at the 5' end and at the terminal three internucleotide linkages at the 3' end, and having 2'-F modification at positions 2, 6, 8, 9, 12, 14, and 16 from the 5' end, with the remaining positions being 2'-OMe-modified;

preferably, the GPC3 aptamer has the sequence SEQ ID No. 190: TAACGCTGACCTTAGCTGCAT GGCTTTACATGTTCCA, with PT modification between the first three internucleotide linkages at the 5' end;

preferably, the AmiR-21 has the sequence GATAAGCT, with PT modification present between adjacent nucleotides in the phosphate backbone;

Drug 38): Vap7 aptamer-TGF-β aptamer-Act-12c aptamer-3*AmiR-21-DNA carrier, wherein the DNA carrier is group 15 of the nucleic acid carrier recited in claim 4; the Vap7 aptamer is linked via two AmiR-21 moieties arranged in tandem to the 5' end of the sequence a; the TGF-β aptamer is linked via the transition sequence TTTTT to the 5' end of the sequence b; and the Act-12c aptamer is linked via one AmiR-21 to the 5' end of the sequence c; wherein, in the DNA carrier, the sequences a, b, and c each independently comprise a phosphorothioate modification between the first two nucleotides at the 3' end;

preferably, the Vap7 aptamer has the sequence SEQ ID No. 277: TGGTGGGGGTGGACGGGCCG GGTAGA, with phosphorothioate modification at the first four internucleotide linkages from the 5' end;

preferably, the AmiR-21 has the sequence GATAAGCT, with phosphorothioate modification present between adjacent nucleotides in the phosphate backbone;

preferably, the TGF-β aptamer has the sequence SEQ ID No. 269: ACATTGCTGCGTGATCGCCTC ACATGGGTTTGTCTGGTCGATTTGGAGGTGGTGGGT GGC, with phosphorothioate modification at the first four internucleotide linkages from the 5' end;

preferably, the Act-12c aptamer has the sequence SEQ ID No. 137: CGGGGAAAGTCACGGGGGG TTTCAGATGTTCTGATCGGTGTGGAG, with phosphorothioate modification at the first four internucleotide linkages from the 5' end;

Drug 39): AS1411 aptamer-3*AmiR-21-IL-4RA aptamer-VCAM-1 aptamer-DNA carrier, wherein the DNA carrier is group 15 of the nucleic acid carrier recited in claim 4; the AS 1411 aptamer is linked in sequence via the transition sequence TTTTT and three AmiR-21 moieties arranged in tandem to the 5' end of the sequence a; the IL-4RA aptamer and the VCAM-1 aptamer are respectively linked via the transition sequence TTTTT to the 5' ends of the sequence b and the sequence c; wherein, in the DNA carrier, the sequences a, b, and c each independently comprise a phosphorothioate modification between the first two nucleotides at the 3' end;

preferably, the AS1411 aptamer has the sequence SEQ ID No. 131: G*GTGGTGGTGGTTGTGGTGGTGGTGG, with phosphorothioate modification between the first two nucleotides at the 5' end;

preferably, the AmiR-21 has the sequence GATAAGCT, with phosphorothioate modification present between adjacent nucleotides in the phosphate backbone;

preferably, the IL-4RA aptamer has the sequence SEQ ID No. 203: AAAAAGCAACAGGGUGCUCCAUGCGCAUGGAACCUGCGCG, with phosphorothioate modification between the first two nucleotides at the 5' end, C and U being 2'-F-modified, and A and G being 2'-OMe-modified;

preferably, the VCAM-1 aptamer has the sequence SEQ ID No. 284: GGACACGGCAAAGGGGTAT AGCCTACCGGACCGTGAACATGGAATGGTGTGCTGCG TGG, with phosphorothioate modification between the first two nucleotides at the 5' end;

Drug 40): GPC1 aptamer-AS1411 aptamer-EpCAM aptamer-2*AmiR-21-DNA carrier, wherein the DNA carrier is group 14 of the nucleic acid carrier recited in claim 4; the GPC1 aptamer is linked via one AmiR-21 to the 5' end of the sequence a; the AS 1411 aptamer is linked via the transition sequence TTTTT to the 5' end of the sequence b; and the EpCAM aptamer is linked via one AmiR-21 to the 5' end of the sequence c; wherein, in the DNA carrier, the sequences a, b, and c each independently comprise (i) phosphorothioate modification between the first three internucleotide linkages from the 3' end, and (ii) 2'-O-MOE modification on the ribose of the first two nucleotides from the 3' end;

preferably, the GPC1 aptamer has the sequence SEQ ID No. 189: AACGGAGTGTGGCTAACTCGA, with phosphorothioate modification at the first four internucleotide linkages from the 5' end;

preferably, the AmiR-21 has the sequence GATAAGCT, with phosphorothioate modification present between adjacent nucleotides in the phosphate backbone;

preferably, the AS 1411 aptamer has the sequence SEQ ID No. 131: GGTGGTGGTGGTTGTGGTG GTGGTGG, with phosphorothioate modification between nucleotide positions 1-2, 3-4, 13-14, 16-17, and 24-25 from the 5' end;

preferably, the EpCAM aptamer has the sequence SEQ ID No. 179: GCGACUGGUUACCCGGUCG, with phosphorothioate modification at the first four internucleotide linkages from the 5' end, C and U being 2'-F-modified and A and G being 2'-OMe-modified;

Drug 41): 3* AmiR-21-AS1411 aptamer-CD40 aptamer-2*biotin-DNA carrier, wherein the DNA carrier is group 1 of the nucleic acid carrier recited in claim 4; three AmiR-21 moieties are respectively linked to the 5' end of the sequence a, the 3' end of the sequence a, and the 3' end of the sequence b; the AS 1411 aptamer is linked via the transition sequence TTTTT to the 5' end of the sequence b; the CD40 aptamer is linked via the transition sequence TTTTT to the 5' end of the sequence c; one biotin is attached to the 3' end of the AmiR-21 located at the 3' end of the sequence a, and the other biotin is attached to the 3' end of the sequence c;

preferably, the AmiR-21 has the sequence GATAAGCT, each nucleotide being LNA-modified;

preferably, the AS 1411 aptamer has the sequence SEQ ID No. 131: GGTGGTGGTGGTTGTGGTGGTGGTGG;

preferably, the CD40 aptamer has the sequence SEQ ID No. 154: CCAACGAGTAGGCGATAGCGCGTGG;

Drug 42): IL-4RA aptamer-VCAM-12d aptamer-PD-L1 aptamer-3*AmiR-21-DNA carrier, wherein the DNA carrier is group 3 of the nucleic acid carrier recited in claim 4; the IL-4RA aptamer is linked via a first AmiR-21 to the 5' end of the sequence a; the VCAM-12d aptamer is linked via a second AmiR-21 to the 5' end of the sequence b; and the PD-L1 aptamer is linked via a third AmiR-21 to the 5' end of the sequence c; wherein, in the DNA carrier, the sequences a, b, and ceach independently comprise a phosphorothioate modification between the first two nucleotides at the 3' end;

preferably, the IL-4RA aptamer has the sequence SEQ ID No. 203: AAAAAGCAACAGGGUGCUCCAUGCGCAUGGAACCUGCGCG, with phosphorothioate modification at the first four internucleotide linkages from the 5' end, and with C and U being 2'-F-modified and A and G being 2'-OMe (2'-O-methyl)-modified;

preferably, the VCAM-12d aptamer has the sequence:

SEQ ID No. 285: AGGGAATCTTGCCTAGGGAGGGAGTAGCGAAAGGGCTCA, with phosphorothioate modification at the first four internucleotide linkages from the 5' end;

preferably, the PD-L1 aptamer has the sequence SEQ ID No. 241: ACGGGCCACATCAACTCATTG ATAGACAATGCGTCCACTGCCCGT, with phosphorothioate modification at the first four internucleotide linkages from the 5' end;

preferably, the AmiR-21 has the sequence GATAAGCT, with phosphorothioate modification present between adjacent nucleotides in the phosphate backbone;

Drug 43): TAP siRNA-3*AmiR-21-2*AS1411 aptamer-DNA carrier, wherein the DNA carrier is group 6 of the nucleic acid carrier recited in claim 4; the sense strand of the TAP siRNA is linked via the transition sequence AAAA to the 3' end of the sequence c; one AS1411 aptamer is linked via the transition sequence AAAA to the antisense strand of the TAP siRNA and, through complementary pairing of the antisense strand with the sense strand, connects the AS 1411 aptamer to the 3' end of the sequence c; another AS 1411 aptamer is linked via the transition sequence AAAA to the 3' end of the sequence a; and three AmiR-21 moieties are linked in tandem via the transition sequence AA to the 3' end of the sequence b; wherein, in the DNA carrier, the sequences a, b, and c each independently comprise a phosphorothioate modification between the first two nucleotides at the 5' end;

preferably, the TAP siRNA comprises a sense strand SEQ ID No. 301: GCUGCACACGGUUCAGAAU and an antisense strand SEQ ID No. 109: AUUCUGAACCGUGUGCAGCUU, the sense and antisense strands each having phosphorothioate modifications at the first three internucleotide linkages at the 5' end and at the terminal three internucleotide linkages at the 3' end; the antisense strand has 2'-F modifications at positions 2, 6, 8, 9, 12, 14, and 16 from the 5' end, with the remaining positions being 2'-OMe-modified, and the sense strand has 2'-F modifications at positions 7, 9, 10, and 11 from the 5' end, with the remaining positions being 2'-OMe-modified;

preferably, the AmiR-21 has the sequence GATAAGCT, with phosphorothioate modification present between adjacent nucleotides in the phosphate backbone;

preferably, the AS1411-1 aptamer has the sequenceSEQ ID No. 131: GGTGGTGGTGGTTGTGGT GGTGGTGG, with phosphorothioate modification between the first two nucleotides at the 5' end;

Drug 44): PD-1 aptamer-AS1411 aptamer-PD-L1 aptamer-3*AmiR-21-DNA carrier, wherein the DNA carrier is selected from group 3 of the nucleic acid carriers recited in claim 4; the PD-1 aptamer is linked via one AmiR-21 to the 5' end of the sequence a; the AS1411 aptamer is linked via one AmiR-21 to the 5' end of the sequence b; the PD-L1 aptamer is linked via one AmiR-21 to the 5' end of the sequence c; and, for each of the sequences a, b, and c, there is independently a phosphorothioate modification between the first two nucleotides at the 3' end;

preferably, the PD-1 aptamer has the sequence SEQ ID No. 253: AGCGGTGACGGCACAGACGGT ACAGTTCCCGTCCCTGCACTACACGTATGCCGCT, with phosphorothioate modifications in the backbone between adjacent nucleotides at the first four positions from the 5' end;

preferably, the AS 1411 aptamer has the sequence SEQ ID No. 131: GGTGGTGGTGGTTGTGGTG GTGGTGG, with a phosphorothioate backbone modification between adjacent nucleotides at the first two positions from the 5' end;

preferably, the PD-L1 aptamer has the sequence SEQ ID No. 241: ACGGGCCACATCAACTCATTG ATAGACAATGCGTCCACTGCCCGT, with phosphorothioate backbone modifications between adjacent nucleotides at the first four positions from the 5' end;

preferably, AmiR-21 has the sequence GATAAGCT, with phosphorothioate modifications between adjacent nucleotides;

Drug 45): CpG1826-CD40 aptamer-AmiR-21-3*biotin-DNA carrier, wherein the DNA carrier is group 13 of the nucleic acid carriers of claim 4; CpG1826 is connected to the 5' end of the sequence a via the transition sequence; the CD40 aptamer is connected to the 5' end of the sequence b via the transition sequence; the AmiR-21 is connected to the 5' end of the sequence c via the transition sequence; and three biotins are respectively attached to the 3' ends of the sequences a, b, and c;

preferably, the CpG1826 sequence is SEQ ID No. 59: TCCATGACGTTCCTGACG, with phosphorothioate modifications between adjacent nucleotides;

preferably, the transition sequence is TTTTT;

preferably, the CD40 aptamer has the sequence SEQ ID No. 154: CCAACGAGTAGGCGATAGCGCGTGG;

preferably, AmiR-21 has the sequence GATAAGCT, with each nucleotide being a locked nucleic acid (LNA);

Drug 46): ATP aptamer-AmiR-2l-FGF2 aptamer-AmiR-1306-DNA carrier, wherein in the DNA carrier the sequence a is SEQ ID No. 18: GCGACGCCCACGAGCGTTCCGGGAGAGGAGC; the sequence b is SEQ ID No. 19: GCTCCTCTCCCGGTTCGCCGCGAGCCGCG; and the sequence c is SEQ ID No. 31: CGCGGCTCGCGGCCATAGCCGTGGGCGTCGC; the ATP aptamer is connected to the 5' end of the sequence a via the transition sequence TTTTT; the FGF2 aptamer is connected to the 5' end of the sequence b via the transition sequence U; and AmiR-1306 and AmiR-21 are connected in sequence to the 5' end of the sequence c;

The ATP aptamer has the sequence SEQ ID No. 136: GGGAGGACGATGCGGAGGAAGGGTAGG, with phosphorothioate modifications in the backbone between adjacent nucleotides at the first four positions from the 5' end;

The FGF2 aptamer has the sequence SEQ ID No. 183: GGGAUACUAGGGCAUUAAUGUUACCAGUGUAGUCCC, wherein cytidine (C) and uridine (U) residues bear 2'-F modifications;

AmiR-1306 has the sequence SEQ ID No. 127: CATCACCACCAGAGCCAACGTC, with phosphorothioate modifications in the backbone between adjacent nucleotides at the first five positions from the 5' end;

AmiR-21 has the sequence GATAAGCT, with each nucleotide being a locked nucleic acid (LNA);

Drug 47): A CD16a aptamer-CTLA-4 aptamer-PD-L1 aptamer-DNA carrier, wherein the DNA carrier is selected from group 9 of the nucleic acid carriers recited in claim 4; the CD16a aptamer is connected to the 5' end of the sequence a via the transition sequence; the CTLA-4 aptamer is connected to the 5' end of the sequence b via the transition sequence; the PD-L1 aptamer is connected to the 5' end of the sequence c; and, for each of the sequences a, b, and c of the DNA carrier, there is independently a phosphorothioate modification between the first two nucleotides at the 3' end;

preferably, the CD16a aptamer has the sequence:

SEQ ID No. 157: CCACTGCGGGGGGTCTATACGTGAGGAAGAAGTGG, with phosphorothioate back-bone modifications between adjacent nucleotides at the first two positions from the 5' end;

preferably, the CTLA-4 aptamer has the sequence:

SEQ ID No. 141: GGUGGAAGAGGGAUGGGCCGACGUGCCGCAU, with phosphorothioate backbone modifications between adjacent nucleotides at the first two positions from the 5' end, and with C and/or U residues bearing 2'-F substitutions and A and/or G residues bearing 2'-O-methyl (2'-OMe) substitutions;

preferably, the PD-L1 aptamer has the sequence SEQ ID No. 241: ACGGGCCACATCAACTCATTG ATAGACAATGCGTCCACTGCCCGT, with phosphorothioate backbone modifications between adjacent nucleotides at the first two positions from the 5' end;

Drug 48): A CD16a aptamer-Act-12c aptamer-PD-L1 aptamer-DNA carrier, wherein the DNA carrier is selected from group 9 of the nucleic acid carriers recited in claim 4; the CD16a aptamer is connected to the 5' end of the sequence a via the transition sequence; the Act-12c aptamer is connected to the 5' end of the sequence b via the transition sequence; the PD-L1 aptamer is connected to the 5' end of the sequence c; and, for each of the sequences a, b, and c of the DNA carrier, there is independently a phosphorothioate modification between the first two nucleotides at the 3' end;

preferably, the CD16a aptamer has the sequence SEQ ID No. 157: CCACTGCGGGGGTCTATACG TGAGGAAGAAGTGG, with phosphorothioate backbone modifications between adjacent nucleotides at the first two positions from the 5' end;

preferably, the Act-12c aptamer has the sequence SEQ ID No. 137: CGGGGAAAGTCACGGGGGG TTTCAGATGTTCTGATCGGTGTGGAG, with phosphorothioate backbone modifications between adjacent nucleotides at the first two positions from the 5' end;

preferably, the PD-L1 aptamer has the sequence SEQ ID No. 241: ACGGGCCACATCAACTCATTG ATAGACAATGCGTCCACTGCCCGT, with phosphorothioate backbone modifications between adjacent nucleotides at the first two positions from the 5' end;

Drug 49): A CTLA-4 aptamer-PD-L1 aptamer-PD-1 aptamer-DNA carrier, wherein the DNA carrier is selected from group 5 of the nucleic acid carriers recited in claim 4;

The PD-L1 aptamer is successively linked to the transition sequence and to the complementary sequence A' of single-stranded bridging sequence A, the single-stranded bridging sequence A being located at the 3' end of the sequence a, and the PD-L1 aptamer being connected to the 3' end of the sequence a by complementary pairing between the complementary sequence A' and the single-stranded bridging sequence A;

The PD-1 aptamer is successively linked to the transition sequence and to the complementary sequence B' of single-stranded bridging sequence B, the single-stranded bridging sequence B being located at the 3' end of the sequence b, and the PD-1 aptamer being connected to the 3' end of the sequence b by complementary pairing between the complementary sequence B' and the single-stranded bridging sequence B;

The CTLA-4 aptamer is linked to the complementary sequence C' of single-stranded bridging sequence C, the single-stranded bridging sequence C being located at the 3' end of the sequence c, and the CTLA-4 aptamer being connected to the 3' end of the sequence c by complementary pairing between the complementary sequence C' and the single-stranded bridging sequence C;

preferably, the PD-L1 aptamer has the sequence SEQ ID No. 241: ACGGGCCACATCAACTCATTG ATAGACAATGCGTCCACTGCCCGT, with phosphorothioate backbone modifications between adjacent nucleotides at the first three positions from the 5' end;

preferably, the PD-1 aptamer has the sequence SEQ ID No. 254: ACCGACAGTGAAGGACTCAGC GAACTCTCAGACTCGGTTC, with phosphorothioate backbone modifications between adjacent nucleotides at the first three positions from the 5' end;

preferably, the CTLA-4 aptamer has the sequence SEQ ID No. 141: GGUGGAAGAGGGAUGGGCCGACGUGCCGCAU, with phosphorothioate backbone modifications between adjacent nucleotides at the first three positions from the 5' end, the ribose of the first two 5'-end nucleotides bearing 2'-O-MOE modifications, the remaining C or U residues bearing 2'-F modifications, and A or G residues bearing 2'-OMe modifications;

preferably, the single-stranded bridging sequence A is SEQ ID No. 7: TGTAGCACGGTGGC, and the complementary sequence A' is SEQ ID No. 8: GCCACCGTGCTACA;

preferably, the single-stranded bridging sequence B is SEQ ID No. 7: TGTAGCACGGTGGC, and the complementary sequence B' is SEQ ID No. 8: GCCACCGTGCTACA;

preferably, the single-stranded bridging sequence C is SEQ ID No. 11: TGCTGCTGCTGCTG, and the complementary sequence C' is SEQ ID No. 12: CAGCAGCAGCAGCA;

Drug 50): A PD-1 aptamer-IL-4Rα aptamer-OX40 aptamer-DNA carrier, wherein the DNA carrier is selected from group 6 of the nucleic acid carriers recited in claim 4; the PD-1 aptamer is connected to the 5' end of the sequence a via the transition sequence AAA; the IL-4Rα aptamer is connected to the 5' end of the sequence b via the transition sequence AAA; the OX40 aptamer is connected to the 5' end of the sequence c via the transition sequence AAA; and, for each of the sequences a, b, and c of the DNA carrier, there are independently phosphorothioate modifications between the first three nucleotides at the 3' end;

preferably, the PD-1 aptamer has the sequence SEQ ID No. 253: AGCGGTGACGGCACAGACGGT ACAGTTCCCGTCCCTGCACTACACGTATGCCGCT, with phosphorothioate backbone modifications between adjacent nucleotides at the first four positions from the 5' end;

preferably, the IL-4Rα aptamer has the sequence SEQ ID No. 203: AAAAAGCAACAGGGUGCUCCAUGCGCAUGGAACCUGCGCG, with phosphorothioate backbone modifications between adjacent nucleotides at the first four positions from the 5' end;

preferably, the OX40 aptamer has the sequence SEQ ID No. 229: CAGUCUGCAUCGUAGGAAUCGCCACCGUAUACUUUCCCAC, with phosphorothioate backbone modifications between adjacent nucleotides at the first four positions from the 5' end, the ribose of the first three 5'-end nucleotides bearing 2'-O-MOE modifications, the remaining C or U residues bearing 2'-F modifications, and A or G residues bearing 2'-OMe modifications;

Drug 51): A CD16a aptamer-OX40 aptamer-PD-L1 aptamer-DNA carrier, wherein the DNA carrier is selected from group 3 of the nucleic acid carriers recited in claim 4; the CD16a aptamer is connected to the 5' end of the sequence a via a transition sequence; the OX40 aptamer is connected to the 5' end of the sequence b via a transition sequence; the PD-L1 aptamer is connected to the 5' end of the sequence c via a transition sequence; and, for each of the sequences a, b, and c of the DNA carrier, there is independently a phosphorothioate modification between the first two nucleotides at the 3' end;

preferably, the CD16a aptamer has the sequence SEQ ID No. 157: CCACTGCGGGGGTCTATACG TGAGGAAGAAGTGG, with phosphorothioate backbone modifications between adjacent nucleotides at the first two positions from the 5' end;

preferably, the OX40 aptamer has the sequence SEQ ID No. 230: GGGAUGCGGAAAAAAGAACAC UUCCGAUUAGGGCCCACCCUAACGGCCGCAGAC, wherein C or U residues bear 2'-fluoro (2'-F) modifications and A or G residues bear 2'-O-methyl (2'-OMe) modifications, and with phosphorothioate backbone modifications between adjacent nucleotides at the first two positions from the 5' end;

preferably, the PD-L1 aptamer has the sequence SEQ ID No. 241: ACGGGCCACATCAACTCATTG ATAGACAATGCGTCCACTGCCCGT, with phosphorothioate backbone modifications between adjacent nucleotides at the first two positions from the 5' end;

Drug 52): A CD16a aptamer-VEGF 165 aptamer-PD-L1 aptamer-DNA carrier, wherein the DNA carrier is selected from group 3 of the nucleic acid carriers recited in claim 4; the CD16a aptamer is connected to the 5' end of the sequence a via a transition sequence; the VEGF165 aptamer is connected to the 5' end of the sequence b via a transition sequence; the PD-L1 aptamer is connected to the 5' end of the sequence c via a transition sequence; and, for each of the sequences a, b, and c of the DNA carrier, there is independently a phosphorothioate modification between the first two nucleotides at the 3' end;

preferably, the CD16a aptamer has the sequence SEQ ID No. 157: CCACTGCGGGGGTCTATACG TGAGGAAGAAGTGG, with phosphorothioate backbone modifications between adjacent nucleotides at the first two positions from the 5' end;

preferably, the VEGF165 aptamer has the sequence SEQ ID No. 280: CGGAAUCAGUGAAUGCUUAUACAUCCG, wherein C or U residues bear 2'-F modifications and A or G residues bear 2'-OMe modifications, and with phosphorothioate backbone modifications between adjacent nucleotides at the first two positions from the 5' end;

preferably, the PD-L1 aptamer has the sequence SEQ ID No. 241: ACGGGCCACATCAACTCATTG ATAGACAATGCGTCCACTGCCCGT, with phosphorothioate backbone modifications between adjacent nucleotides at the first two positions from the 5' end;

Drug 53): A CD16a aptamer-CTLA-4 aptamer-TIM3 aptamer-DNA carrier, wherein the DNA carrier is selected from group 3 of the nucleic acid carriers recited in claim 4; the CD16a aptamer is connected to the 5' end of the sequence a via a transition sequence; the CTLA-4 aptamer is connected to the 5' end of the sequence b via a transition sequence; the TIM3 aptamer is connected to the 5' end of the sequence c via a transition sequence; and, for each of the sequences a, b, and c of the DNA carrier, there is independently a phosphorothioate modification between the first two nucleotides at the 3' end;

preferably, the CD16a aptamer has the sequence SEQ ID No. 157: CCACTGCGGGGGTCTATACG TGAGGAAGAAGTGG, with phosphorothioate backbone modifications between adjacent nucleotides at the first two positions from the 5' end;

preferably, the CTLA-4 aptamer has the sequence SEQ ID No. 141: GGUGGAAGAGGGAUGGGCCGACGUGCCGCAU, with phosphorothioate backbone modifications between adjacent nucleotides at the first two positions from the 5' end, C or U residues bearing 2'-F modifications, and A or G residues bearing 2'-OMe modifications;

preferably, the TIM3 aptamer has the sequence SEQ ID No. 270: GGGAGAGGACCAGUAGCCACU

AUGGUGUUGGAGCUAGCGGCAGAGCGUCGCGGU CCCUCCC, with phosphorothioate backbone modifications between adjacent nucleotides at the first two positions from the 5' end, C or U residues bearing 2'-F modifications, and A or G residues bearing 2'-OMe modifications;

Drug 54): A PD-1 aptamer-CTLA-4 aptamer-LAG-3 aptamer-DNA carrier, wherein the DNA carrier is selected from group 3 of the nucleic acid carriers recited in claim 4; the PD-L1 aptamer is connected to the 5' end of the sequence a via a transition sequence; the CTLA-4 aptamer is connected to the 5' end of the sequence b via a transition sequence; the LAG-3 aptamer is connected to the 5' end of the sequence c via a transition sequence; and, for each of the sequences a, b, and c of the DNA carrier, there is independently a phosphorothioate modification between the first two nucleotides at the 3' end;

preferably, the PD-1 aptamer has the sequence: SEQ ID No. 253: AGCGGTGACGGCACAGACGG TACAGTTCCCGTCCCTGCACTACACGTATGCCGCT, with phosphorothioate backbone modifications between adjacent nucleotides at the first two positions from the 5' end;

preferably, the CTLA-4 aptamer has the sequence: SEQ ID No. 141: GGUGGAAGAGGGAUGGGCCGACGUGCCGCAU, with phosphorothioate backbone modifications between adjacent nucleotides at the first two positions from the 5' end, C or U residues bearing 2'-fluoro (2'-F) modifications, and A or G residues bearing 2'-O-methyl (2'-OMe) modifications;

preferably, the LAG-3 aptamer has the sequence: SEQ ID No. 206: GGGAGAGAGAUAUAAGGGC CUCCUGAUACCCGCUGCUAUCUGGACCGAUCCCAU UACCAAAUUCUCUCCC, with phosphorothioate backbone modifications between adjacent nucleotides at the first two positions from the 5' end, C or U residues bearing 2'-F modifications, and A or G residues bearing 2'-OMe modifications;

Drug 55): A CD16a aptamer-Act-12c aptamer-MUC1 aptamer-DNA carrier, wherein the DNA carrier is selected from group 3 of the nucleic acid carriers recited in claim 4; the CD16a aptamer is connected to the 5' end of the sequence a via a transition sequence; the Act-12c aptamer is connected to the 5' end of the sequence b via the transition sequence; the MUC1 aptamer is connected to the 5' end of the sequence c via the transition sequence; and, for each of the sequences a, b, and c of the DNA carrier, there is independently a phosphorothioate modification between the first two nucleotides at the 3' end;

preferably, the CD16a aptamer has the sequence: SEQ ID No. 157: CCACTGCGGGGGTCTATACG TGAGGAAGAAGTGG, with phosphorothioate backbone modifications between adjacent nucleotides at the first two positions from the 5' end;

preferably, the Act-12c aptamer has the sequence: SEQ ID No. 137: CGGGGAAAGTCACGGGGG GTTTCAGATGTTCTGATCGGTGTGGAG, with phosphorothioate backbone modifications between adjacent nucleotides at the first two positions from the 5' end;

preferably, the MUC1 aptamer has the sequence: SEQ ID No. 209: GAAGTGAAAATGACAGAACACAACA;

Drug 56): A CD16a aptamer-TGF-β aptamer-PD-L1 aptamer-DNA carrier, wherein the DNA carrier is selected from group 3 of the nucleic acid carriers recited in claim 4; the CD16a aptamer is connected to the 5' end of the sequence a via a transition sequence; the TGF-β aptamer is connected to the 5' end of the sequence b via the transition sequence; the PD-L1 aptamer is connected to the 5' end of the sequence a via a transition sequence; and, for each of the sequences a, b, and c of the DNA carrier, there is independently a phosphorothioate modification between the first two nucleotides at the 3' end;

preferably, the CD16a aptamer has the sequence SEQ ID No. 157: CCACTGCGGGGGTCTATACG TGAGGAAGAAGTGG, with phosphorothioate backbone modifications between adjacent nucleotides at the first two positions from the 5' end;

preferably, the TGF-β aptamer has the sequence SEQ ID No. 269: ACATTGCTGCGTGATCGCCTC ACATGGGTTTGTCTGGTCGATTTGGAGGTGGTGGGT GGC, with phosphorothioate backbone modifications between adjacent nucleotides at the first two positions from the 5' end;

preferably, the PD-L1 aptamer has the sequence SEQ ID No. 241: ACGGGCCACATCAACTCATTG ATAGACAATGCGTCCACTGCCCGT, with phosphorothioate backbone modifications between adjacent nucleotides at the first two positions from the 5' end;

Drug 57): A CD16a aptamer-CD40 aptamer-CTLA-4 aptamer-DNA carrier, wherein the DNA carrier is selected from group 3 of the nucleic acid carriers recited in claim 4; the CD16a aptamer is connected via a transition sequence to the 5' end of the sequence a; the CD40 aptamer is connected via the transition sequence to the 5' end of the sequence b; the CTLA-4 aptamer is connected via a transition sequence to the 5' end of the sequence c; and, for each of the sequences a, b, and c of the DNA carrier, there is independently a phosphorothioate modification between the first two nucleotides at the 3' end;

preferably, the CD16a aptamer has the sequence SEQ ID No. 157: CCACTGCGGGGGTCTATACG TGAGGAAGAAGTGG, with phosphorothioate backbone modifications between adjacent nucleotides at the first two positions from the 5' end;

preferably, the CD40 aptamer has the sequence SEQ ID No. 154: CCAACGAGTAGGCGATAGCGC GTGG, with phosphorothioate backbone modifications between adjacent nucleotides at the first two positions from the 5' end;

preferably, the CTLA-4 aptamer has the sequence SEQ ID No. 141: GGUGGAAGAGGGAUGGGCCGACGUGCCGCAU, with phosphorothioate backbone modifications between adjacent nucleotides at the first two positions from the 5' end, C or U residues bearing 2'-fluoro (2'-F) modifications, and A or G residues bearing 2'-O-methyl (2'-OMe) modifications;

Drug 58): A HER2 aptamer-HER3 aptamer-CD40 aptamer-CD16a aptamer-DNA carrier, wherein the DNA carrier is selected from group 7 of the nucleic acid carriers recited in claim 4; the HER3 aptamer is connected to the 5' end of the sequence a via the transition sequence AA; the HER2 aptamer is connected to the 5' end of the sequence c via the transition sequence AA; the CD40 aptamer and the CD16a aptamer are respectively connected to the 5' and 3' ends of the sequence b via the transition sequence AA; and, for each of the sequences a and c of the DNA carrier, there is independently a phosphorothioate modification between the first two nucleotides at the 3' end;

preferably, the HER2 aptamer has the sequence SEQ ID No. 194: AGCCGCGAGGGGAGGGAUAGGGUAGGGCGCGGCU, with phosphorothioate backbone modifications between adjacent nucleotides at the first two positions from the 5' end, C or U residues bearing 2'-fluoro (2'-F) modifications, and A or G residues bearing 2'-O-methyl (2'-OMe) modifications;

preferably, the HER3 aptamer has the sequence SEQ ID No. 197: CAGCGAAAGUUGCGUAUGGGUCACAUCGCAGGCACAUGUCAUCUGGGCG, with phosphorothioate backbone modifications between adjacent nucleotides at the first two positions from the 5' end, C or U residues bearing 2'-F modifications, and A or G residues bearing 2'-OMe modifications;

preferably, the CD40 aptamer has the sequence SEQ ID No. 154: CCAACGAGTAGGCGATAGCGC GTGG, with phosphorothioate backbone modifications between adjacent nucleotides at the first two positions from the 5' end;

preferably, the CD16a aptamer has the sequence SEQ ID No. 157: CCACTGCGGGGGTCTATACG TGAGGAAGAAGTGG, with phosphorothioate backbone modifications between adjacent nucleotides at the first two positions from the 5' end;

Drug 59): A CD16a aptamer-Act-12c aptamer-CTLA-4 aptamer-GPC1 aptamer-DNA carrier, wherein the DNA carrier is selected from group 10 of the nucleic acid carriers recited in claim 4; the CD16a aptamer is connected to the 5' end of the sequence a via the transition sequence TTTTT; the Act-12c aptamer is connected to the 5' end of the sequence b via the transition sequence TTTTT; the CTLA-4 aptamer and the GPC1 aptamer are respectively connected to the 5' and 3' ends of the sequence c via the transition sequence TTT; and, for each of the sequences a and b of the DNA carrier, there is independently a phosphorothioate modification between the first two nucleotides at the 3' end;

preferably, the CD16a aptamer has the sequence SEQ ID No. 157: CCACTGCGGGGGTCTATACG TGAGGAAGAAGTGG, with phosphorothioate backbone modifications between adjacent nucleotides at the first two positions from the 5' end;

preferably, the Act-12c aptamer has the sequence SEQ ID No. 137: CGGGGAAAGTCACGGGGGG TTTCAGATGTTCTGATCGGTGTGGAG, with phosphorothioate backbone modifications between adjacent nucleotides at the first two positions from the 5' end;

preferably, the CTLA-4 aptamer has the sequence SEQ ID No. 141: GGUGGAAGAGGGAUGGGCCGACGUGCCGCAU, with phosphorothioate backbone modifications between adjacent nucleotides at the first two positions from the 5' end, C or U residues bearing 2'-F modifications, and A or G residues bearing 2'-OMe modifications;

preferably, the GPC1 aptamer has the sequence SEQ ID No. 189: AACGGAGTGTGGCTAACTCGA, with a phosphorothioate backbone modification between the terminal two nucleotides at the 3' end;

Drug 60): A CD16a aptamer-HER3 aptamer-VEGF 165 aptamer-DNA carrier, wherein the DNA carrier is selected from group 3 of the nucleic acid carriers recited in claim 4; the CD16a aptamer is connected to the 5' end of the sequence a via the transition sequence TTTTT; the HER3 aptamer is connected to the 5' end of the sequence b via the transition sequence TTTTT; the VEGF165 aptamer is connected to the 5' end of the sequence c via the transition sequence TTTTT; and, for each of the sequences a, b, and c of the DNA carrier, there are independently phosphorothioate backbone modifications between the first two nucleotides from the 3' end;

preferably, the CD16a aptamer has the sequence SEQ ID No. 157: CCACTGCGGGGGTCTATACG TGAGGAAGAAGTGG, with phosphorothioate backbone modifications between adjacent nucleotides at the first two positions from the 5' end;

preferably, the HER3 aptamer has the sequence SEQ ID No. 197: CAGCGAAAGUUGCGUAUGGGUCACAUCGCAGGCACAUGUCAUCUGGGCG, with phosphorothioate backbone modifications between adjacent nucleotides at the first two positions from the 5' end, cytidine (C) or uridine (U) residues bearing 2'-fluoro (2'-F) substitutions, and adenosine (A) or guanosine (G) residues bearing 2'-O-methyl (2'-OMe) substitutions;

preferably, the VEGF165 aptamer has the sequence SEQ ID No. 280: CGGAAUCAGUGAAUGCUUAUACAUCCG, with phosphorothioate backbone modifications between adjacent nucleotides at the first two positions from the 5' end, C or U residues bearing 2'-F substitutions, and A or G residues bearing 2'-OMe substitutions;

Drug 61): A VEGF aptamer-GPC1 aptamer-PD-L1 aptamer-CD16a aptamer-DNA carrier, wherein the DNA carrier is selected from group 11 of the nucleic acid carriers recited in claim 4; the VEGF aptamer and the GPC1 aptamer are respectively connected to the 5' and 3' ends of the sequence a via the transition sequence TTTTT; the PD-L1 aptamer and the CD16a aptamer are respectively connected to the 5' ends of the sequence b and the sequence c via the transition sequence TTTTT; and, for each of the sequences b and c of the DNA carrier, there are independently phosphorothioate backbone modifications between the first two nucleotides from the 3' end;

preferably, the VEGF aptamer has the sequence SEQ ID No. 279: GGTGGGGGTGGACGGGCCG GGTAGA, with phosphorothioate backbone modifications between adjacent nucleotides at the first two positions from the 5' end;

preferably, the GPC1 aptamer has the sequence SEQ ID No. 189: AACGGAGTGTGGCTAACTCG*A, with a phosphorothioate backbone modification between the terminal two nucleotides at the 3' end;

preferably, the PD-L1 aptamer has the sequence SEQ ID No. 241: ACGGGCCACATCAACTCATTG ATAGACAATGCGTCCACTGCCCGT, with phosphorothioate backbone modifications between adjacent nucleotides at the first two positions from the 5' end;

preferably, the CD16a aptamer has the sequence SEQ ID No. 157: CCACTGCGGGGGTCTATACG TGAGGAAGAAGTGG, with phosphorothioate backbone modifications between adjacent nucleotides at the first two positions from the 5' end;

Drug 62): A PD-1 aptamer-PD-L1 aptamer-2×AmiR-21-Act-12c aptamer-DNA carrier, wherein the DNA carrier is selected from group 3 of the nucleic acid carriers recited in claim 4; the PD-1 aptamer is connected to the 5' end of the sequence a via one AmiR-21; the PD-L1 aptamer is connected to the 5' end of the sequence b via the other AmiR-21; the Act-12c aptamer is connected to the 5' end of the sequence c via the transition sequence TTTTT; and, for each of the sequences a, b, and c of the DNA carrier, there is independently a phosphorothioate modification between the first two nucleotides at the 3' end;

preferably, the PD-1 aptamer has the sequence SEQ ID No. 253: AGCGGTGACGGCACAGACGGT ACAGTTCCCGTCCCTGCACTACACGTATGCCGCT, with phosphorothioate backbone modifications between adjacent nucleotides at the first four positions from the 5' end;

preferably, the PD-L1 aptamer has the sequence SEQ ID No. 241: ACGGGCCACATCAACTCATTG ATAGACAATGCGTCCACTGCCCGT, with phosphorothioate backbone modifications between adjacent nucleotides at the first four positions from the 5' end;

preferably, the Act-12c aptamer has the sequence SEQ ID No. 137: CGGGGAAAGTCACGGGGGG TTTCAGATGTTCTGATCGGTGTGGAG, with phosphorothioate backbone modifications between adjacent nucleotides at the first four positions from the 5' end;

preferably, AmiR-21 has the sequence GATAAGCT, with phosphorothioate modifications between adjacent nucleotides;

Drug 63): A HER3 aptamer-EGFR siRNA-AS1411 aptamer-Survivin siRNA-HER2 aptamer-DNA carrier, wherein the DNA carrier is group 14 of the nucleic acid carriers of claim 4; the AS1411 aptamer is connected to the 3' end of the sequence a via the transition sequence AAAA; the HER2 aptamer is connected to the 3' end of the sequence b via the transition sequence AAAA; the sense strand of the EGFR siRNA is connected to the 3' end of the sequence c via the transition sequence AAAA; the sense strand of the Survivin siRNA is connected to the 3' end of the sequence b via the transition sequence AAAA; and, for each of the sequences a, b, and c of the DNA carrier, there is independently a phosphorothioate modification between the first two nucleotides at the 5' end;

The HER3 aptamer is connected via the transition sequence AA to the antisense strand of the EGFR siRNA and, through complementary pairing between the antisense strand and the sense strand of the EGFR siRNA, is thereby connected to the 3' end of the sequence c;

The HER2 aptamer is connected via the transition sequence **AA** to the antisense strand of the Survivin siRNA and, through complementary pairing between the antisense strand and the sense strand of the Survivin siRNA, is thereby connected to the 3' end of the sequence b;

preferably, the HER3 aptamer has the sequence SEQ ID No. 197: CAGCGAAAGUUGCGUAUGGGUCACAUCGCAGGCACAUGUCAUCUGGGCG, with phosphorothioate backbone modifications between adjacent nucleotides at the first three positions from the 5' end;

preferably, the EGFR siRNA has an antisense strand SEQ ID No. 302: AAUUAGAUAAGACUGCUAAGGCA and a sense strand SEQ ID No. 82: CCUUAGCAGUCUUAUCUAAUU, both strands bearing phosphorothioate backbone modifications between adjacent nucleotides at the first three positions from the 5' end and at the last three positions at the 3' end;

preferably, the AS 1411 aptamer has the sequence SEQ ID No. 131: GGTGGTGGTGGTTGTGGTG GTGGTGG, with a phosphorothioate backbone modification between the terminal two nucleotides at the 3' end;

preferably, the Survivin siRNA has a sense strand SEQ ID No. 303: GCAGGUUCCUUAUCUGUCACA, And an antisense strand SEQ ID No. 300: UGUGACAGAUAAGGAACCUGCAG, wherein the antisense strand with phosphorothioate backbone modifications between adjacent nucleotides at the first three positions from the 5' end and at the last three positions at the 3' end;

preferably, the HER2 aptamer has the sequence SEQ ID No. 194: AGCCGCGAGGGGAGGGAUAGGGUAGGGCGCGGCU, with phosphorothioate backbone modifications between adjacent nucleotides at the first two positions from the 5' end, cytidine (C) or uridine (U) residues bearing 2'-fluoro (2'-F) modifications, and adenosine (A) or guanosine (G) residues bearing 2'-O-methyl (2'-OMe) modifications;

Drug 64): A Survivin siRNA-TFRA3 aptamer-AS1411 aptamer-3×AmiR-21-DNA carrier, wherein the DNA carrier is group 17 of the nucleic acid carriers of claim 4; the TFRA3 aptamer is connected to the 5' end of the sequence a via one AmiR-21; FA is connected to the 3' end of the sequence a; the AS 1411 aptamer is connected to the 5' end of the sequence b via one AmiR-21; the sense strand of the Survivin siRNA is connected to the 5' end of the sequence c via one AmiR-21; and the antisense strand of the Survivin siRNA is connected to the sense strand by complementary base pairing; for each of the sequences a, b, and c of the DNA carrier, there are independently phosphorothioate modifications between the first two nucleotides at the 5' end;

preferably, the TFRA3 aptamer has the sequence SEQ ID No. 262: GCGTGGTCACACGC;

preferably, AmiR-21 has the sequence GATAAGCT;

preferably, the AS 1411 aptamer has the sequence SEQ ID No. 131: GGTGGTGGTGGTTGTGGTGGTGGTGG;

preferably, the sense strand of the Survivin siRNA has the sequence SEQ ID No. 304: GCAGGUUCCUUAUCUGUCA, with phosphorothioate backbone modifications between adjacent nucleotides at the first three positions from the 5' end and at the last three positions at the 3' end, and with 2'-fluoro (2'-F) substitutions at positions 7 and 9-11 counting from the 5' end and 2'-O-methyl (2'-OMe) substitutions at the remaining positions;

And the antisense strand of the Survivin siRNA has the sequence SEQ ID No. 305: UGACAGAUAAGGAACCUGCAGUU, with phosphorothioate backbone modifications between adjacent nucleotides at the first three positions from the 5' end and at the last three positions at the 3' end, with 2'-F substitutions at positions 2, 6, 8, 9, 12, 14, and 16 counting from the 5' end, and 2'-OMe substitutions at the remaining positions;

Drug 65): An AS 1411 aptamer-EGFR siRNA-VEGF 165 aptamer-PD-L1 aptamer-RNA carrier, wherein the RNA carrier is group 16 of the nucleic acid carriers recited in claim 4; the VEGF165 aptamer is connected to the 3' end of the sequence a via the transition sequence AA; the PD-L1 aptamer is connected to the 3' end of the sequence b via the transition sequence AA; the sense strand of the EGFR siRNA is connected to the 3' end of the sequence c via the transition sequence AA; the AS 1411 aptamer is connected to the 5' end of the antisense strand of the EGFR siRNA via the transition sequence AA; the antisense strand of the EGFR siRNA is connected to the sense strand by complementary base pairing; and the sequence a of the DNA carrier has a phosphorothioate backbone modification between the first two nucleotides at the 5' end;

preferably, the AS 1411 aptamer has the sequence SEQ ID No. 131: GGTGGTGGTGGTTGTGGTG GTGGTGG, with a phosphorothioate backbone modification between the first two nucleotides from the 5' end;

preferably, the EGFR siRNA has a sense strand SEQ ID No. 82: CCUUAGCAGUCUUAUCUAAUU, with phosphorothioate backbone modifications between adjacent nucleotides at the last three positions at the 3' end, and an antisense strand SEQ ID No. 302: AAUUAGAUAAGACUGCUAAGGCA, with phosphorothioate backbone modifications between adjacent nucleotides at the first three positions from the 5' end and at the last three positions at the 3' end;

preferably, the VEGF165 aptamer has the sequence SEQ ID No. 280: CGGAAUCAGUGAAUGCUUAUACAUCCG, wherein C and U residues bear 2'-fluoro (2'-F) substitutions, A and G residues bear 2'-O-methyl (2'-OMe) substitutions, and there is a phosphorothioate backbone modification between the terminal two nucleotides at the 3' end;

preferably, the PD-L1 aptamer has the sequence SEQ ID No. 241: ACGGGCCACATCAACTCATTG ATAGACAATGCGTCCACTGCCCGT, wherein C and U residues bear 2'-F substitutions, A and G residues bear 2'-OMe substitutions, and there is a phosphorothioate backbone modification between the terminal two nucleotides at the 3' end;

Drug 66): A CEA aptamer-CD16a aptamer-Act-12c aptamer-PD-L1 aptamer-DNA carrier, wherein the DNA carrier is group 20 of the nucleic acid carriers recited in claim 4; the CEA aptamer is connected to the 5' end of the sequence a via the transition sequence AA; the CD16a aptamer is connected to the 3' end of the sequence a via the transition sequence AA; the Act-12c aptamer and the PD-L1 aptamer are respectively connected to the 5' ends of the sequence b and the sequence c via the transition sequence AA; and, for each of the sequences b and c of the DNA carrier, there is independently a phosphorothioate backbone modification between the first two nucleotides at the 3' end;

preferably, the CEA aptamer has the sequence SEQ ID No. 150: TTAACTTATTCGACCATA, with a phosphorothioate backbone modification between nucleotides 1 and 2 from the 5' end;

preferably, the CD16a aptamer has the sequence SEQ ID No. 157: CCACTGCGGGGGTCTATACG TGAGGAAGAAGTGG, with a phosphorothioate backbone modification between the terminal two nucleotides at the 3' end;

preferably, the Act-12c aptamer has the sequence SEQ ID No. 137: CGGGGAAAGTCACGGGGGG TTTCAGATGTTCTGATCGGTGTGGAG, with a phosphorothioate backbone modification between nucleotides 1 and 2 from the 5' end;

preferably, the PD-L1 aptamer has the sequence SEQ ID No. 241: ACGGGCCACATCAACTCATTG ATAGACAATGCGTCCACTGCCCGT, with a phosphorothioate backbone modification between nucleotides 1 and 2 from the 5' end;

Drug 67): An AS 1411 aptamer-TAP siRNA-CD 16a aptamer-CTLA-4 aptamer-DNA carrier, wherein the DNA carrier is group 14 of the nucleic acid carriers recited in claim 4; the CD16a aptamer is connected to the 3' end of the sequence a via the transition sequence AAAA; the CTLA-4 aptamer is connected to the 3' end of the sequence b via the transition sequence AAAA; the sense strand of the TAP siRNA is connected to the 3' end of the sequence c via the transition sequence AAAA; the AS1411 aptamer is connected to the 5' end of the antisense strand of the TAP siRNA via the transition sequence AAA; the antisense strand of the TAP siRNA is connected to the sense strand by complementary base pairing; and, for each of the sequences a, b, and c of the DNA carrier, there is independently a phosphorothioate modification between the first two nucleotides at the 5' end;

preferably, the AS 1411 aptamer has the sequence SEQ ID No. 131: GGTGGTGGTGGTTGTGGTG GTGGTGG, with a phosphorothioate backbone modification between nucleotides 1 and 2 from the 5' end;

preferably, the TAP siRNA has a sense strand SEQ ID No. 301: GCUGCACACGGUUCAGAAU, with phosphorothioate backbone modifications between adjacent nucleotides at positions 1-3 from the 5' end and at the last three positions at the 3' end, and with 2'-fluoro (2'-F) substitutions at positions 7 and 9-11 from the 5' end and 2'-O-methyl (2'-OMe) substitutions at the remaining positions;

The antisense strand is SEQ ID No. 109: AUUCUGAACCGUGUGCAGCUU, with phosphorothioate backbone modifications between adjacent nucleotides at positions 1-3 from the 5' end and at the last three positions at the 3' end, with 2'-F substitutions at positions 2, 6, 8, 9, 12, 14, and 16 from the 5' end, and 2'-OMe substitutions at the remaining positions;

preferably, the CD16a aptamer has the sequence SEQ ID No. 157: CCACTGCGGGGGTCTATACG TGAGGAAGAAGTGG, with phosphorothioate backbone modifications between nucleotides 1 and 2 from the 5' end and between the terminal two nucleotides at the 3' end;

preferably, the CTLA-4 aptamer has the sequence SEQ ID No. 141: GGUGGAAGAGGGAUGGGCCGACGUGCCGCAU, with a phosphorothioate backbone modification between the terminal two nucleotides at the 3' end, C and U residues bearing 2'-F substitutions, and A and G residues bearing 2'-O-methyl (2'-OMe) substitutions;

Drug 68): An AS1411 aptamer-TAP siRNA-CD16a aptamer-Act-12c aptamer-DNA carrier, wherein the DNA carrier is group 14 of the nucleic acid carriers recited in claim 4; the CD16a aptamer is connected to the 3' end of the sequence a via the transition sequence AAAA; the Act-12c aptamer is connected to the 3' end of the sequence b via the transition sequence AAAA; the sense strand of the TAP siRNA is connected to the 3' end of the sequence c via the transition sequence AAAA; the AS1411 aptamer is connected to the 5' end of the antisense strand of the TAP siRNA via the transition sequence AAA; the antisense strand of the TAP siRNA is connected to the sense strand by complementary base pairing; and, for each of the sequences a, b, and c of the DNA carrier, there is independently a phosphorothioate modification between the first two nucleotides at the 5' end;

preferably, the AS 1411 aptamer has the sequence SEQ ID No. 131: GGTGGTGGTGGTTGTGGTG GTGGTGG, with a phosphorothioate backbone modification between nucleotides 1 and 2 from the 5' end;

preferably, the TAP siRNA has a sense strand SEQ ID No. 301: GCUGCACACGGUUCAGAAU, with phosphorothioate backbone modifications between adjacent nucleotides at positions 1-3 from the 5' end and at the last three positions at the 3' end, and with 2'-fluoro (2'-F) substitutions at positions 7 and 9-11 from the 5' end and 2'-O-methyl (2'-OMe) substitutions at the remaining positions;

The antisense strand is SEQ ID No. 109: AUUCUGAACCGUGUGCAGCUU, with phosphorothioate backbone modifications between adjacent nucleotides at positions 1-3 from the 5' end and at the last three positions at the 3' end, with 2'-F substitutions at positions 2, 6, 8, 9, 12, 14, and 16 from the 5' end, and 2'-OMe substitutions at the remaining positions;

preferably, the CD16a aptamer has the sequence SEQ ID No. 157: CCACTGCGGGGGTCTATACG TGAGGAAGAAGTG*G, with a phosphorothioate backbone modification between the terminal two nucleotides at the 3' end;

preferably, the Act-12c aptamer has the sequence SEQ ID No. 137: CGGGGAAAGTCACGGGGGG TTTCAGATGTTCTGATCGGTGTGGAG, with a phosphorothioate backbone modification between the terminal two nucleotides at the 3' end;

Drug 69): A CD16a aptamer-AS1411 aptamer-TAP siRNA-OX40 aptamer-Act-12c aptamer-DNA carrier, wherein the DNA carrier is group 14 of the nucleic acid carriers recited in claim 4; the CD16a aptamer is linked to the 5' end of the sequence a via the transition sequence AA; the sense strand of the TAP siRNA is linked to the 3' end of the sequence a via the transition sequence AA; the AS 1411 aptamer is linked to the 5' end of the antisense strand of the TAP siRNA via the transition sequence AAA; the antisense strand of the TAP siRNA is connected to the sense strand by complementary base pairing; the OX40 aptamer is linked to the 3' end of the sequence b via the transition sequence AA; the Act-12c aptamer is linked to the 5' end of the sequence c via the transition sequence AAAA; and, for each of the sequences b and c of the DNA carrier, there is independently a phosphorothioate backbone modification between the first two nucleotides at the 5' end;

preferably, the CD16a aptamer has the sequence SEQ ID No. 157: CCACTGCGGGGGTCTATACG TGAGGAAGAAGTGG, with a phosphorothioate backbone modification between nucleotides 1 and 2 from the 5' end;

preferably, the AS 1411 aptamer has the sequence SEQ ID No. 131: GGTGGTGGTGGTTGTGGTG GTGGTGG, with a phosphorothioate backbone modification between nucleotides 1 and 2 from the 5' end;

preferably, the TAP siRNA has a sense strand SEQ ID No. 301: GCUGCACACGGUUCAGAAU, with phosphorothioate backbone modifications between adjacent nucleotides at positions 1-3 from the 5' end and at the last three positions at the 3' end, and with 2'-fluoro (2'-F) substitutions at positions 7 and 9-11 from the 5' end and 2'-O-methyl (2'-OMe) substitutions at the remaining positions;

The antisense strand is SEQ ID No. 109: AUUCUGAACCGUGUGCAGCUU, with phosphorothioate backbone modifications between adjacent nucleotides at positions 1-3 from the 5' end and at the last three positions at the 3' end, with 2'-F substitutions at positions 2, 6, 8, 9, 12, 14, and 16 from the 5' end, and 2'-OMe substitutions at the remaining positions;

preferably, the OX40 aptamer has the sequence SEQ ID No. 228: GGGAGGACGAUGCGGCAGUC UGCAUCGUAGGAAUCGCCACCGUAUACUUUCCCA CCAGACGACUCGCUG, with a phosphorothioate backbone modification between the terminal two nucleotides at the 3' end, and with cytidine (C) and uridine (U) residues bearing 2'-F substitutions and adenosine (A) and guanosine (G) residues bearing 2'-OMe substitutions;

preferably, the Act-12c aptamer has the sequence SEQ ID No. 137: CGGGGAAAGTCACGGGGGG TTTCAGATGTTCTGATCGGTGTGGAG, with a phosphorothioate backbone modification between nucleotides 1 and 2 from the 5' end;

Drug 70): A CTLA-4 aptamer-CD40 aptamer-FAP aptamer-DNA carrier, wherein the DNA carrier is group 15 of the nucleic acid carriers recited in claim 4; the CTLA-4 aptamer is connected to the 5' end of the sequence a via the transition sequence TTTTT; the CD40 aptamer is connected to the 5' end of the sequence b via the transition sequence TTTTT; the FAP aptamer is connected to the 5' end of the sequence c via the transition sequence TTTTT; and, for each of the sequences a, b, and c of the DNA carrier, there is independently a phosphorothioate modification between the first two nucleotides from the 3' end;

preferably, the CTLA-4 aptamer has the sequence SEQ ID No. 141: GGUGGAAGAGGGAUGGGCCGACGUGCCGCAU, with a phosphorothioate backbone modification between nucleotides 1 and 2 from the 5' end, cytidine (C) and uridine (U) residues bearing 2'-fluoro (2'-F) substitutions, and adenosine (A) and guanosine (G) residues bearing 2'-O-methyl (2'-OMe) substitutions;

preferably, the CD40 aptamer has the sequence SEQ ID No. 154: CCAACGAGTAGGCGATAGCGC GTGG, with a phosphorothioate backbone modification between nucleotides 1 and 2 from the 5' end;

preferably, the FAP aptamer has the sequence SEQ ID No. 188: CCGCTCGAGCTAGTCTGACAAA GAGAAACAC, with a phosphorothioate backbone modification between nucleotides 1 and 2 from the 5' end;

Drug 71): A VEGF165 aptamer-ASAP1 siRNA-CD24A-2 aptamer-SARS-CoV-2-N48 aptamer-DNA carrier, wherein the DNA carrier is group 14 of the nucleic acid carriers recited in claim 4; the sense strand of the ASAP1 siRNA is connected to the 3' end of the sequence c via the transition sequence AA; the VEGF 165 aptamer is connected to the 5' end of the antisense strand of the ASAP1 siRNA via the transition sequence AA; the antisense strand of the ASAP1 siRNA is connected to the sense strand by complementary base pairing; the CD24A-2 aptamer is connected to the 3' end of the sequence b via the transition sequence AA; the SARS-CoV-2-N48 aptamer is connected to the 3' end of the sequence a via the transition sequence AA; and, for each of the sequences b and c of the DNA carrier, there is independently a phosphorothioate modification between the first two nucleotides at the 5' end;

preferably, the VEGF165 aptamer has the sequence SEQ ID No. 280: CGGAAUCAGUGAAUGCUUAUACAUCCG, with a phosphorothioate backbone modification between nucleotides 1 and 2 from the 5' end, cytidine (C) and uridine (U) residues bearing 2'-fluoro (2'-F) substitutions, and adenosine (A) and guanosine (G) residues bearing 2'-O-methyl (2'-OMe) substitutions;

preferably, the ASAP 1 siRNA has an antisense strand SEQ ID No. 65: UGAUAUUAUGGAAGCAAAUUU, with phosphorothioate backbone modifications between adjacent nucleotides at positions 1-3 from the 5' end and at the last three positions at the 3' end, with 2'-F substitutions at positions 2, 6, 8, 9, 12, 14, and 16 from the 5' end, and 2'-OMe substitutions at the remaining positions;

And a sense strand SEQ ID No. 64: AUUUGCUUCCAUAAUAUCAUU, with phosphorothioate backbone modifications between adjacent nucleotides at positions 1-3 from the 5' end and at the last three positions at the 3' end, with 2'-F substitutions at positions 7 and 9-11 from the 5' end, and 2'-OMe substitutions at the remaining positions;

preferably, the SARS-CoV-2-N48 aptamer has the sequence SEQ ID No. 222: GCTGGATGTCGCT TACGACAATATTCCTTAGGGGCACCGCTACATTGACACATCCA GC, with a phosphorothioate backbone modification between the terminal two nucleotides at the 3' end;

preferably, the CD24A-2 aptamer has the sequence SEQ ID No. 167: ATCCAGAGTGACGCAGCAT ATGTGGGTGGGTGGGCGGTTATGCTGAGTCAGCCTTG CTTGGACACGGTGGCTTAGT, with a phosphorothioate backbone modification between nucleotides 1 and 2 from the 5' end;

Drug 72): A 3*FGF5 aptamer-DNA carrier, wherein the DNA carrier is group 15 of the nucleic acid carriers recited in claim 4; the three FGF5 aptamers are respectively connected via U to the 5' ends of the sequences a, b, and c; and, for each of the sequences a, b, and c of the DNA carrier, there is independently a phosphorothioate modification between the first two nucleotides at the 3' end;

preferably, the FGF5 aptamer has the sequence SEQ ID No. 186: GGGCGACCUCUCCGUACUGA CCUACAGAGCGACAUACUAGUGUAUCCAGAUCGC CC, with a phosphorothioate backbone modification between nucleotides 1 and 2 from the 5' end, C and U residues bearing 2'-F substitutions, and A and G residues bearing 2'-OMe substitutions;

Drug 73): An AS1411 aptamer-ASAP1 siRNA-VEGF165 aptamer-SARS-CoV-2-N48 aptamer-RNA carrier, wherein the DNA carrier is group 21 of the nucleic acid carriers recited in claim 4; the VEGF165 aptamer is connected to the 3' end of the sequence a via the transition sequence AA; the SARS-CoV-2-N48 aptamer is connected to the 3' end of the sequence b via the transition sequence AA; the sense strand of the ASAP1 siRNA is connected to the 3' end of the sequence c; the AS 1411 aptamer is connected to the 5' end of the antisense strand of the ASAP1 siRNA via the transition sequence AA; the antisense strand of the ASAP1 siRNA is connected to the sense strand by complementary base pairing; for each of the sequences a, b, and c of the DNA carrier, there is independently a phosphorothioate modification between the first two nucleotides at the 5' end; and, in the sequence c, adenosine (A) and guanosine (G) residues bear 2'-O-methyl (2'-OMe) substitutions, and cytidine (C) and uridine (U) residues bear 2'-fluoro (2'-F) substitutions;

preferably, the AS 1411 aptamer has the sequence SEQ ID No. 131: GGTGGTGGTGGTTGTGGTG GTGGTGG, with a phosphorothioate backbone modification between nucleotides 1 and 2 from the 5' end;

preferably, the antisense strand of the ASAP1 siRNA is SEQ ID No. 65: UGAUAUUAUGGAAGCAAAUUU, with phosphorothioate backbone modifications between adjacent nucleotides at positions 1-3 from the 5' end and at the last three positions at the 3' end, with 2'-F substitutions at positions 2, 6, 8, 9, 12, 14, and 16 from the 5' end, and 2'-O-Me substitutions at the remaining positions;

The sense strand of the ASAP1 siRNA is SEQ ID No. 64: AUUUGCUUCCAUAAUAUCAUU, with phosphorothioate backbone modifications between adjacent nucleotides at positions 1-3 from the 5' end and at the last three positions at the 3' end;

preferably, the VEGF165 aptamer has the sequence SEQ ID No. 280: CGGAAUCAGUGAAUGCUUAUACAUCCG, with C and U residues bearing 2'-F substitutions and A and G residues bearing 2'-OMe substitutions;

preferably, the SARS-CoV-2-N48 aptamer has the sequence SEQ ID No. 222: GCTGGATGTCGCT TACGACAATATTCCTTAGGGGCACCGCTACATTGACACATCCA GC, with a phosphorothioate backbone modification between the terminal two nucleotides at the 3' end;

Drug 74): A CpG2006-CD40 aptamer-PD-1 aptamer-DNA carrier, wherein the DNA carrier is group 6 of the nucleic acid carriers recited in claim 4; CpG2006 is connected to the 5' end of the sequence a via a transition sequence; the CD40 aptamer is connected to the 5' end of the sequence b via the transition sequence; and the PD-1 aptamer is connected to the 5' end of the sequence c via the transition sequence;

preferably, CpG2006 has the sequence SEQ ID No. 57: TCGTCGTTTTGTCGTTTTGTCGTT, with phosphorothioate backbone modifications between adjacent nucleotides;

preferably, the CD40 aptamer has the sequence SEQ ID No. 154: CCAACGAGTAGGCGATAGCGC GTGG, with the ribose 2' positions of the first four 5'-end nucleotides bearing 2'-O-methoxyethyl (2'-MOE) modifications, and with phosphorothioate backbone modifications between adjacent nucleotides at the first five positions from the 5' end;

preferably, the PD-1 aptamer has the sequence SEQ ID No. 254: ACCGACAGTGAAGGACTCAGC GAACTCTCAGACTCGGTTC, with the ribose 2' positions of the first four 5'-end nucleotides bearing 2'-MOE modifications, and with phosphorothioate backbone modifications between adjacent nucleotides at the first five positions from the 5' end;

Drug 75): A CD24 aptamer-CpG2006 variant-CD40 aptamer-PD-L1 aptamer-DNA carrier, wherein the DNA carrier is group (6) of the nucleic acid carriers recited in claim 4; the CD24 aptamer is connected to the 5' end of the sequence a via the CpG2006 variant sequence GTCGTT; the CD40 aptamer is connected to the 5' end of the sequence b via the transition sequence TTTTT; the PD-L1 aptamer is connected to the 5' end of the sequence c via the transition sequence TTTTT;

preferably, the CD24 aptamer has the sequence SEQ ID No. 166: TATGTGGGTGGGTGGGCGGTT ATGCTGAGTCAGCCTTGCT, with phosphorothioate backbone modifications between adjacent nucleotides at the first four positions from the 5' end;

preferably, the CD40 aptamer has the sequence SEQ ID No. 154: CCAACGAGTAGGCGATAGCGC GTGG, with phosphorothioate backbone modifications between adjacent nucleotides at the first five positions from the 5' end;

preferably, the PD-L1 aptamer has the sequence SEQ ID No. 238: AACAACGTATAACAATGCCCAC GTCACCAGAGTACTATGG, with the ribose 2' positions of the first six 5'-end nucleotides bearing 2'-O-methoxyethyl (2'-MOE) modifications;

Wherein the transition sequence TTTTT is a PEG-modified TTTTT;

Drug 76): An HBsAg aptamer-HBV siRNA-miR-34-PD-1 aptamer-miR-542-AS1411 aptamer-DNA carrier, wherein the DNA carrier is group 24 of the nucleic acid carriers recited in claim 4; the sense strand of the HBV siRNA is connected to the 3' end of the sequence a; the HBsAg aptamer is linked to and located at the 5' end of the antisense strand of the HBV siRNA; the PD-L1 aptamer is connected to the 3' end of the sequence b via miR-34; and the AS1411 aptamer is connected to the 3' end of the sequence c via miR-542;

preferably, the HBV siRNA has a sense strand SEQ ID No. 88: GGACUUCUCUCAAUUUUCUUU and an antisense strand SEQ ID No. 89: AGAAAAUUGAGAGAAGUCCUU, wherein cytidine (C) and uridine (U) residues in both strands bear 2'-fluoro (2'-F) substitutions, and the phosphorothioate backbone modifications are present between the last three nucleotides at the 3' end;

preferably, the HBsAg aptamer has the sequence SEQ ID No. 192: CACAGCGAACAGCGGCGGAC ATAATAGTGCTTACTACGAC, with phosphorothioate backbone modifications between adjacent nucleotides at the first four positions from the 5' end;

preferably, miR-34 has the sequence TGTGACAG;

preferably, the PD-L1 aptamer has the sequence SEQ ID No. 238: AACAACGTATAACAATGCCCAC GTCACCAGAGTACTATGG, with the ribose moieties of the three nucleotides at the 3' end bearing 2'-O-methoxyethyl (2'-MOE) modifications;

preferably, miR-542 has the sequence TGGCAGTGT;

preferably, the AS1411 aptamer has the sequence SEQ ID No. 131: GGTGGTGGTGGTTGTGGTG GTGGTGG, with 2'-O-MOE phosphorothioate backbone modifications between the four nucleotides at the 3' end;

Drug 77): An IL-4Rα aptamer-TIMC-d aptamer-4×miR-126-RNA carrier, wherein the RNA carrier is group 25 of the nucleic acid carriers recited in claim 4; the IL-4Rα aptamer is directly connected to the 5' end of the sequence a; the TIMC-d aptamer is directly connected to the 5' end of the sequence b; and four copies of miR-126 are tandemly connected to the 5' end of the sequence c;

preferably, the IL-4Rα aptamer has the sequence SEQ ID No. 203: AAAAAGCAACAGGGUGCUCCAUGCGCAUGGAACCUGCGCG, with phosphorothioate backbone modifications between adjacent nucleotides at the first four positions from the 5' end;

preferably, the TIMC-d aptamer has the sequence SEQ ID No. 272: AAGCAACACUUAGUCGCGAUUGAUACGUGCGCAGUCAU, with phosphorothioate backbone modifications between adjacent nucleotides at the first four positions from the 5' end;

preferably, miR-126 has the sequence:

UCGUACC, with phosphorothioate backbone modifications between adjacent nucleotides;

Drug 78): A CD3-4 aptamer-PD-1 aptamer-BCMA aptamer-DNA carrier, wherein the DNA carrier is group 24 of the nucleic acid carriers recited in claim 4; the CD3-4 aptamer is linked, via the transition sequence TTTTTT, to the complementary sequence A' of the single-stranded bridging sequence A in the sequence a, and is connected to the 3' end of the sequence a by complementary pairing between the complementary sequence A' and the single-stranded bridging sequence A;

The PD-1 aptamer is linked, via the transition sequence TTTTT, to the complementary sequence B' of the single-stranded bridging sequence B in the sequence b, and is connected to the 3' end of the sequence b by complementary pairing between the complementary sequence B' and the single-stranded bridging sequence B;

The BCMA aptamer is linked to the complementary sequence C' of the single-stranded bridging sequence C in the sequence c, and is connected to the 3' end of the sequence c by complementary pairing between the complementary sequence C' and the single-stranded bridging sequence C;

The sequence a is SEQ ID No. 15: GACGCCCACGAGCGTTCCGGGAGAGGTGTAGCACGGTGGC, the underlined portion denoting the single-stranded bridging sequence A;

preferably, the sequence of CD3-4 aptamer-transition sequence-complementary sequence A' of single-stranded bridging sequence A :

SEQ ID No. 306:

TCTCGGACGCGTGTGGTCGGCCGAGTGGCCCACGGTAGAAGGGTTAGAACTGC TGGTTGGTGAATCTCGCTGCCTGGCCCTAGAGTGTTTTTT<u>GCCACCGTGCTACA;</u>

The sequence b of the DNA carrier:

SEQ ID No. 16: <u>CCTCTCCCGGTTCGCCGCGAGCCTCGGCGCGGCCGTG,</u> the underlined portion denoting the single-stranded bridging sequence B;

preferably, the sequence of PD-1 aptamer-transition sequence TTTTT-complementary sequence B' of single-stranded bridging sequence B is SEQ ID No. 307:

ACCGACAGTGAAGGACTCAGCGAACTCTCAGACTCGGTTCTTTTT<u>CACGGCCG CGCCGA,</u> with phosphorothioate backbone modifications between adjacent nucleotides at the first five positions from the 5' end;

The sequence c is SEQ ID No. 17:

<u>GGCTCGCGGCCATAGCCGTGGGCGTCTGCTGCTGCTGCTG,</u> the underlined portion denoting the single-stranded bridging sequence C;

preferably, the sequence of BCMA aptamer-transition sequence U-complementary sequence C' of single-stranded bridging sequence C is SEQ ID No. 308:

AGUGCAAGACGUUCGCAGAUUAGCGAAAAGAGGGUCUCAUUGACUAGUACU <u>CAGCAGCAG-CAGCA,</u> the underlined portion denoting the complementary sequence C' of the single-stranded bridging sequence C, with phosphorothioate backbone modifications between the last three nucleotides at the 3' end, with the ribose moieties of the first four 5'-end nucleotides bearing 2'-O-methoxyethyl (2'-MOE) modifications, and with 2'-fluoro (2'-F) substitutions on cytidine (C) and uridine (U) residues among the remaining nucleotides;

Drug 79): A CpG2006-CD38 aptamer-PD-L1 aptamer-DNA carrier; wherein the DNA carrier is group 6 of the nucleic acid carriers recited in claim 4; CpG2006 is connected to the 5' end of the sequence a via the transition sequence TTTTT; the CD38 aptamer is connected to the 5' end of the sequence b via the transition sequence TTTTT; and the PD-L1 aptamer is connected to the 5' end of the sequence c via the transition sequence TTTTT;

preferably, the CpG2006-transition sequence TTTTT-sequence a is SEQ ID No. 309: TCGTCGTTTTT GTCGTTTTGTCGTTTTTTTGCGACGCCCACGAGCGTTCCGGGAGAGG AGC,wherein the CpG2006 segment has phosphorothioate backbone modifications between adjacent nucleotides;

preferably, the CD38 aptamer-transition sequence TTTTT-sequence b is SEQ ID No. 310: T A C G T G A A T C T C G T A C G A T A C T C T G T A A G C G T T T T T T G C T C C T C T C C C G G T T C G CCGCGAGCCGCG,with the ribose of the first three 5'-end nucleotides bearing 2'-O-methoxyethyl (2'-MOE) modifications;

preferably the PD-L1 aptamer-transition sequence TTTTT-sequence c is SEQ ID No. 311: AACAACGTATAACAATGCCCACGTCACCAGAGTACTATGGTTTTTCGCGGCTC GCGGCCATAGC CGTGGGCGTCGC,with the ribose of the first six 5'-end nucleotides bearing 2'-O-methoxyethyl (2'-MOE) modifications;

Drug 80): A CD20 aptamer-CpG-BYZD-CD38 aptamer-miR-34-CD3 aptamer-DNA carrier, wherein the DNA carrier is group 6 of the nucleic acid carriers recited in claim 4; the CD20 aptamer is connected to the 5' end of the sequence a via the CpG-BYZD sequence AGCGAA; the CD38 aptamer is connected to the 5' end of the sequence b via miR-34; and the CD3 aptamer is connected to the 5' end of the sequence c via the transition sequence TTTTT;

preferably, the CD20 aptamer-CpG-BYZD-sequence a is SEQ ID No. 312: <u>TGCGTGTGTAGTGTGT CTGTTTTTTATCTTCTTTTATCTACTCTTAGGGATTTGGGCGG</u> AGCGAAGCGACGCCCACGAGC GTTCCGGGAGAGGAGC, wherein SEQ ID No. 165: TGCGTGTGTAGTGTGTCTGTTTTTTATCTTC TTTTATCTACTCTTAGGGATTTGGGCGG_ constitutes the CD20 aptamer and AGCGAA constitutes the CpG-BYZD, and the phosphorothioate backbone has modifications between adjacent nucleotides at the first four positions from the 5' end (of the CD20 aptamer segment);

preferably, the CD38 aptamer-miR-34-sequence b is SEQ ID No. 313: <u>TACGTGAATCTCGTACGAT ACTCTGTAAGCGTT</u>GTGACAGGCTCCTCTCCCGGTTCGC CGCGAGCCGCG, with 2'-O-methoxyethyl (2'-MOE) modifications on the ribose of the first three 5'-end nucleotides, TGTGACAG being the miR-34 segment, the sequence preceding TGTGACAG being the CD38 aptamer, and the sequence following TGTGACAG being the sequence b;

preferably, the CD3 aptamer-transition sequence TTTTT-sequence c is SEQ ID No. 314 GCCGCGG GGTGGGTCTAGTGTGGATGTTTAGGGGGCGGCTTTTTCGCGGCTCGCGG CCATAGCCGTGGG CGTCGC, with 2'-MOE modifications on the ribose of the first four 5'-end nucleotides;

Drug 81): A CpG2006-CD38 aptamer-miR-126-PD-L1 aptamer-DNA carrier; wherein the DNA carrier is group 6 of the nucleic acid carriers recited in claim 4; CpG2006 is connected to the 5' end of the sequence a via the transition sequence TTTTT; the CD38 aptamer is connected to the 5' end of the sequence b via miR-126; and the PD-L1 aptamer is connected to the 5' end of the sequence c via the transition sequence TTTTT;

preferably, the CpG2006-transition sequence TTTTT-sequence a is SEQ ID No. 309: TCGTCGTTTTT GTCGTTTTGTCGTTTTTTTGCGACGCCCACGAGCGTTCCGGGAGAGG AGC, wherein the CpG2006 segment has phosphorothioate backbone modifications between adjacent nucleotides, and the sequence a has a phosphorothioate backbone modification between the terminal two nucleotides at the 3' end;

preferably, the CD38 aptamer-miR-126-sequence b is SEQ ID No. 315: TACGTGAATCTCGTACGA TACTCTGTAAGCGT<u>UCGUACCGG</u>CTCCTCTCCCGGTTCG CCGCGAGCCGCG, wherein the CD38 aptamer has the sequence SEQ ID No. 170: TACGTGAATCTCGTACGATACTCTGTAAGCGT, with 2'-O-methoxyethyl (2'-MOE) modifications on the ribose of the first three 5'-end nucleotides; and UCGUACCG is the miR-126 sequence, each nucleotide thereof bearing a 2'-O-MOE modification on the ribose;

preferably, the PD-L1 aptamer-transition sequence TTTTT-sequence c is SEQ ID No. 311: AACAACGTATAACAATGCCCACGTCACCAGAGTACTATGGTTTTTCGCGGCTCG CGGCCATAGC CGTGGGCGTCGC, with 2'-O-MOE modifications on the ribose of the first six 5'-end nucleotides of the PD-L1 aptamer;

Drug 82): A BCMA aptamer-miR-126-CD38 aptamer-miR-122-CD3 aptamer-miR-34-DNA carrier,

wherein the DNA carrier is group 6 of the nucleic acid carriers recited in claim 4; the BCMA aptamer is connected to the 5' end of the sequence a via the transition sequence U and miR-126; the CD38 aptamer is connected to the 5' end of the sequence b via miR-122; and the CD3 aptamer is connected to the 5' end of the sequence c via miR-34;

preferably, the BCMA aptamer-transition sequence U-miR-126-sequence a is SEQ ID No. 316: AGUGCAAGACGUUCGCAGAUUAGCGAAAAGAGGGUCUCAUUGACUAGUACU CGTACCGGCG ACGCCCACGAGCGTTCCGGGAGAGGAGC, wherein CGTACCG is the miR-126 sequence, the portion preceding CGTACCG is the BCMA aptamer sequence, and the portion following CGTACCG is the sequence a; within the BCMA aptamer sequence, the ribose 2' positions of the first four 5'-end nucleotides with 2'-O-methoxyethyl (2'-MOE) modifications, and among the remaining positions the C and U residues with 2'-fluoro (2'-F) substitutions;

preferably, the CD38 aptamer-miR-122-sequence b is SEQ ID No. 317: TmAmCmGTGAATCTCGTA CGATACTCTGTAAGCGTGGAAGTGTGCTCCTCTCCCGGT TCGCCGCGAGCCGCG, with 2'-MOE modifications on the ribose of the first three 5'-end nucleotides; GGAAGTGT is the miR-122 sequence, the portion preceding GGAAGTGT is the CD38 aptamer sequence, and the portion following GGAAGTGT is the sequence b;

preferably, the CD3 aptamer-miR-34-sequence c is SEQ ID No. 318: AmGmCmCmGCGGGGTGGG TCTAGTGTGGATGTTTAGGGGGCGGCTTGTGACAGCG CGGCTCGCGGCCATAGCCGTGGGCG TCGC, with 2'-MOE modifications on the ribose of the first four 5'-end nucleotides; TGTGACA is the miR-34 sequence, the portion preceding TGTGACA is the CD3 aptamer sequence, and the portion following TGTGACA is the sequence c;

Drug 83): A GPC3 aptamer-AmiR-21-CD38 aptamer-miR-122-PD-L1 aptamer-miR-34-DNA carrier, wherein the DNA carrier is group 6 of the nucleic acid carriers recited in claim 4; the GPC3 aptamer is connected to the 5' end of the sequence a via AmiR-21; the CD38 aptamer is connected to the 5' end of the sequence b via miR-122; and the PD-L1 aptamer is connected to the 5' end of the sequence c via miR-34;

preferably, the GPC3 aptamer-AmiR-21-sequence a is SEQ ID No. 319: TAACGCTGACCTTAGCTG CATGGCTTTACATGTTCCAGATAAGCTGCGACGCCCAC GAGCGTTCCGGGAGAGGAGC, with phosphorothioate backbone modifications between adjacent nucleotides at the first four positions from the 5' end; GATAAGCT is the AmiR-21 sequence, the portion preceding GATAAGCT is the GPC3 aptamer sequence, and the portion following GATAAGCT is the sequence a;

preferably, the CD38 aptamer-miR-122-sequence b is SEQ ID No. 317: TACGTGAATCTCGTACGA TACTCTGTAAGCGTGGAAGTGTGCTCCTCTCCCGGTTCG CCGCGAGCCGCG, with phosphorothioate backbone modifications between adjacent nucleotides at the first four positions from the 5' end; GGAAGTGT is the miR-122 sequence, the portion preceding GGAAGTGT is the CD38 aptamer sequence, and the portion following GGAAGTGT is the sequence b;

preferably, the PD-L1 aptamer-miR-34-sequence c is SEQ ID No. 320: AACAACGTATAACAATGCC CACGTCACCAGAGTACTATGGTGTGACAGCGCGGCTC GCGGCCATAGCCGTGGGCGTCGC, with 2'-O-methoxyethyl (2'-MOE) modifications on the ribose of the first six 5'-end nucleotides; TGTGACAG is the miR-34 sequence, the portion preceding TGTGACAG is the PD-L1 aptamer sequence, and the portion following TGTGACAG is the sequence c;

Drug 84): A CpG2006-VEGF165 aptamer-miR-122-PD-L1 aptamer-DNA carrier, wherein the DNA carrier is group 6 of the nucleic acid carriers recited in claim 4; CpG2006 is connected to the 5' end of the sequence a via the transition sequence TTTTT; the VEGF165 aptamer is connected to the 5' end of the sequence b via miR-122; and the PD-L1 aptamer is connected to the 5' end of the sequence c via the transition sequence TTTTT;

preferably, the CpG2006-transition sequence TTTTT-sequence a is SEQ ID No. 309: TCGTCGTTTT GTCGTTTTGTCGTTTTTTTGCGACGCCCACGAGCGTTCCGGGAGAGG AGC, wherein the CpG2006 segment has phosphorothioate backbone modifications between adjacent nucleotides;

preferably, the VEGF165 aptamer-miR-122-sequence b is SEQ ID No. 321: CGGAAUCAGUGAAU GCUUAUACAUCCGGGAAGTGTGCTCCTCTCCCGGTTCGCCG CGAGCCGCG, with 2'-O-methoxyethyl (2'-MOE) modifications on the ribose of the first four 5'-end nucleotides, and with cytidine (C) and uridine (U) residues among the remaining RNA bearing 2'-fluoro (2'-F) substitutions; GGAAGTGT is the miR-122 sequence, the portion preceding GGAAGTGT is the VEGF165 aptamer sequence, and the portion following GGAAGTGT is the sequence b;

preferably, the PD-L1 aptamer-transition sequence TTTTT-sequence c is SEQ ID No. 311: AACAACGTATAACAATGCCCACGTCACCAGAGTACTATGGTTTTTCGCGGCTCG CGGCCATAGC CGTGGGCGTCGC, with 2'-MOE modifications on the ribose of the first six 5'-end nucleotides of the PD-L1 aptamer;

Drug 85): A CpG2006-HBsAg aptamer-miR-122-PD-L1 aptamer-DNA carrier, wherein the DNA carrier is group 6 of the nucleic acid carriers recited in claim 4; CpG2006 is connected to the 5' end of the sequence a via the transition sequence TTTTT; the HBsAg aptamer is connected to the 5' end of the sequence b via miR-122; and the PD-L1 aptamer is connected to the 5' end of the sequence c via the transition sequence TTTTT;

preferably, the CpG2006-transition sequence TTTTT-sequence a is SEQ ID No. 309: TCGTCGTTTT GTCGTTTTGTCGTTTTTTTGCGACGCCCACGAGCGTTCCGGGAGAGG AGC, wherein the CpG2006 segment has phosphorothioate backbone modifications between adjacent nucleotides;

preferably, the HBsAg aptamer-miR-122-sequence b is SEQ ID No. 322: CACAGCGAACAGCGGC GGACATAATAGTGCTTACTACGAC<u>GGAAGTGT</u>GCTCCTCTC CCGGTTCGCCGCGAGCCGCG, with phosphorothioate backbone modifications between adjacent nucleotides at the first four positions from the 5' end; GGAAGTGT is the miR-122 sequence, the portion preceding GGAAGTGT is the HBsAg aptamer sequence, and the portion following GGAAGTGT is the sequence b;

preferably, the PD-L1 aptamer-transition sequence TTTTT-sequence c is SEQ ID No. 311: AACAAC GTATAACAATGCCCACGTCACCAGAGTACTATGGTTTTTCGCGGCTCGCGG CCATAGCCGTGG GCGTCGC, with 2'-O-methoxyethyl (2'-MOE) modifications on the ribose of the first six 5'-end nucleotides of the PD-L1 aptamer;

Drug 86): An ASO-GSK836-HBsAg aptamer-miR-122-PD-L1 aptamer-DNA carrier, wherein the DNA carrier is group 6 of the nucleic acid carriers recited in claim 4; ASO-GSK836 is connected to the 5' end of the sequence a via the transition sequence TTTTT; the HBsAg aptamer is connected to the 5' end of the sequence b via miR-122; and the PD-L1 aptamer is connected to the 5' end of the sequence c via the transition sequence TTTTT;

preferably, the ASO-GSK836-transition sequence TTTTT-sequence a is SEQ ID No. 323: GCAGAG GTGAAGCGAAGTCGTTTTTGCGACGCCCACGAGCGTTCCGGGAGAG GAGC, with phosphorothioate backbone modifications between adjacent nucleotides at the first six positions from the 5' end;

preferably, the HBsAg aptamer-miR-122-sequence b is SEQ ID No. 322: CACAGCGAACAGCGGC GGACATAATAGTGCTTACTACGAC**GGAAGTGT**GCTCCTCT CCCGGTTCGCCGCGAGCCGCG, with phosphorothioate backbone modifications between adjacent nucleotides at the first four positions from the 5' end; **GGAAGTGT** is the miR-122 sequence, the portion preceding GGAAGTGT is the HBsAg aptamer sequence, and the portion following GGAAGTGT is the sequence b;

preferably, the PD-L1 aptamer-transition sequence TTTTT-sequence c is SEQ ID No. 311: AACAACGTATAACAATGCCCACGTCACCAGAGTACTATGGTTTTTCGCGGCTCG CGGCCATAGC CGTGGGCGTCGC, with 2'-O-methoxyethyl (2'-MOE) modifications on the ribose of the first six 5'-end nucleotides of the PD-L1 aptamer;

Drug 87): A BCMA aptamer-CD38 aptamer-miR-126-PD-L1 aptamer-miR-34-DNA carrier; wherein the DNA carrier is group 6 of the nucleic acid carriers recited in claim 4; the BCMA aptamer is connected to the 5' end of the sequence a via the transition sequence UU; the CD38 aptamer is connected to the 5' end of the sequence b via miR-126; and the PD-L1 aptamer is connected to the 5' end of the sequence c via miR-34;

preferably, the BCMA aptamer-transition sequence UU-sequence a is SEQ ID No. 324: AGUGCAA GACGUUCGCAGAUUAGCGAAAAGAGGGUCUCAUUGACUAGUACUUGC GACGCCCACGAGC GTTCCGGGAGAGGAGC, wherein SEQ ID No. 140: AGUGCAAGACGUUCGCAGAUUAGCGAAA AGAGGGUCUCAUUGACUAGUAC is the BCMA aptamer in RNA form, all nucleotides of which with 2'-fluoro (2'-F) substitutions, and the remaining portion constitutes the sequence a;

preferably, the CD38 aptamer-miR-126-sequence b is SEQ ID No. 325: TACGTGAATCTCGTACGA TACTCTGTAAGCGT<u>GTCGTT</u>GCTCCTCTCCCGGTTCGCCG CGAGCCGCG, with phosphorothioate backbone modifications between adjacent nucleotides at the first four positions from the 5' end; GTCGTT is the miR-126 sequence, the portion preceding GTCGTT is the CD38 aptamer sequence, and the portion following GTCGTT is the sequence b;

preferably, the PD-L1 aptamer-miR-34-sequence c is SEQ ID No. 320: AACAACGTATAACAATGCC CACGTCACCAGAGTACTATGG<u>TGTGACAG</u>CGCGGCTCG CGGCCATAGCCGTGGGCGTCGC, with 2'-O-methoxyethyl (2'-MOE) modifications on the ribose of the first six 5'-end nucleotides; TGTGACAG is the miR-34 sequence, the portion preceding TGTGACAG is the PD-L1 aptamer sequence, and the portion following TGTGACAG is the sequence c;

Drug 88): An AS1411 aptamer-FGF2 aptamer-3 ×AmiR-21-DNA carrier; the AS1411 aptamer is connected to the 5' end of the sequence a of the DNA carrier via the transition sequence TTTTT; the FGF2 aptamer is connected to the 5' end of the sequence b via the transition nucleotide U; and three AmiR-21 units are directly, tandemly connected to the 5' end of the sequence c;

preferably, the AS1411 aptamer-transition sequence TTTTT-sequence a is SEQ ID No. 326:

GGTGGTGGTGGTTGTGGTGGTGGTGGTTTTTGCGCCCACGAGCGTTCCGGGAG AGC;

preferably, the FGF2 aptamer-transition nucleotide U-sequence b is SEQ ID No. 327: GGGAUACUA GGGCAUUAAUGUUACCAGUGUAGUCCCUCCCUGCTCTCCCGGTTCG CCGCCAGCCGCC, wherein SEQ ID No. 183: GGGAUACUAGGGCAUUAAUGUUACCAGUGUAGUCCC is the FGF2 aptamer in RNA form, with cytidine (C) and uridine (U) residues bearing 2'-fluoro (2'-F) substitutions, and the remaining portion constituting the sequence b;

preferably, the 3*AmiR-21-sequence c is SEQ ID No. 328: GATAAGCTGATAAGCTGATAAGCTGGC GGCAGGCGGCCATAGCCGTGGGCGC, GATAAGCT, wherein GATAAGCT is the AmiR-21 sequence and there are phosphorothioate backbone modifications between adjacent nucleotides;

Drug 89): An HBsAg aptamer-CD40 aptamer-PD-L1 aptamer-DNA carrier, wherein the HBsAg aptamer is connected to the 5' end of the sequence a of the DNA carrier via the transition sequence TTTTT; the CD40 aptamer is connected to the 5' end of the sequence b via the transition sequence TTTTT; and the PD-L1 aptamer is connected to the 5' end of the sequence c via the transition sequence TTTTT;

preferably, the HBsAg aptamer-transition sequence TTTTT-sequence a is SEQ ID No. 329: CACAGCGAACAGCGGCGGACATAATAGTGCTTACTACGAC<u>TTTTT</u>GCGACGCCC ACGAGCGTT CCGGGAGAGGAGC, with phosphorothioate backbone modifications between adjacent nucleotides at the first four positions from the 5' end; the portion preceding TTTTT is the HBsAg aptamer sequence, and the portion following TTTTT is the sequence a;

preferably, the CD40 aptamer-transition sequence TTTTT-sequence b is SEQ ID No. 330: CCAACGAGTAGGCGATAGCGCGTGG<u>TTTTT</u>GCTCCTCTCCCGGTTCGCCGCGAG CCGCG, with phosphorothioate backbone modifications between adjacent nucleotides at the first three positions from the 5' end and at the last three positions at the 3' end; the portion preceding TTTTT is the CD40 aptamer sequence, and the portion following TTTTT is the sequence b;

preferably, the PD-L1 aptamer-transition sequence TTTTT-sequence c is SEQ ID No. 331: ACGGGCCACATCAACTCATTGATAGACAATGCGTCCACTGCCCG<u>TTTTT</u>CGCG GCTCGCGGC CATAGCCGTGGGCGTCGC, with phosphorothioate backbone modifications between adjacent nucleotides at the first three positions from the 5' end and at the last three positions at the 3' end; the portion preceding TTTTT is the PD-L1 aptamer sequence, and the portion following TTTTT is the sequence c;

Drug 90): An HBsAg aptamer-CD40 aptamer-PD-L1 aptamer-DNA carrier, which is identical to Drug 89 except that the sequence of the PD-L1 aptamer is different; wherein the PD-L1 aptamer-transition sequence TTTTT-sequence c is SEQ ID No. 311: AACAACGTATAACAATGCCCACGTCACCAGAG TACTATGGTTTTTCGCGGCTCGCG GCCATAGCCGTGGGCGTCGC, with 2'-O-methoxyethyl (2'-MOE) modifications on the ribose of the first six nucleotides from the 5' end; the portion preceding TTTTT is the PD-L1 aptamer sequence, and the portion following TTTTT is the sequence c;

Drug 91): A BCMA aptamer-CD38 aptamer-CpG-BYZD-CD19 aptamer-AmiR-21-DNA carrier, wherein the DNA carrier has the following sequences:

the sequence a is SEQ ID No. 15: GACGCCCACGAGCGTTCCGGGAGAGGTGTAGCACGGTGGC, the underlined portion denoting single-stranded bridging sequence A;

the sequence b is SEQ ID No. 16: <u>CCTCTCCCGGTTCGCCGCGAGCCTCGGCGCGGCCGTG,</u> the underlined portion denoting single-stranded bridging sequence B;

the sequence c is SEQ ID No. 17: <u>GGCTCGCGGCCATAGCCGTGGGCGTCTGCTGCTGCTGCTG,</u> the underlined portion denoting single-stranded bridging sequence C;

the CD38 aptamer is connected, via the transition sequence TT and CpG-BYZD, to the complementary sequence A' of single-stranded bridging sequence A, the complementary sequence A' being complementary to single-stranded bridging sequence A; wherein CD38 aptamer-CpG-BYZD-complementary sequence A' is SEQ ID No. 332: TACGTGAATCTCGTACGATACTCTGTAAGCG TTTAGCGAAGCCACCGTGCTACA, in which SEQ ID No. 170: TACGTGAATCTCGTACGATAC

TCTGTAAGCGT is the CD38 aptamer (with phosphorothioate backbone modifications between adjacent nucleotides at the first four positions from the 5' end); SEQ ID No. 8 GCCACCGTGCTACA is the complementary sequence A'; TT is the transition sequence; and AGCGAA is CpG-BYZD; the CD19 aptamer is connected via AmiR-21 to the 5' end of the complementary sequence B' of single-stranded bridging sequence B, the complementary sequence B' being complementary to single-stranded bridging sequence B; wherein CD19 aptamer-AmiR-21-complementary sequence B' is SEQ ID No. 333: UGAGCCCUGUUCGACAGGAGGCUCAGAUAAGCUCACGGCCGCGCCGA, in which SEQ ID No. 160: UGAGCCCUGUUCGACAGGAGGCUCA is the CD19 aptamer sequence (with 2'-fluoro (2'-F) substitutions on C and U residues); GAUAAGCU is the AmiR-21 sequence (with 2'-F substitutions on C and U residues); and SEQ ID No. 10 CACGGCCGCGCCGA is the complementary sequence B';

the BCMA aptamer is connected via the transition sequence AA to the 5' end of the complementary sequence C' of single-stranded bridging sequence C, the complementary sequence C' being complementary to single-stranded bridging sequence C; wherein BCMA aptamer-transition sequence AA-complementary sequence C' is SEQ ID No. 334: AGUGCAAGACGUUCGCAGAUU AGCGAAAAGAGGGUCUCAUUGACUAGUACAACA GCAGCAGCAGCA, in which SEQ ID No. 12: CAGCAGCAGCAGCA is the complementary sequence C' and SEQ ID No. 140: AGUGCAA GACGUUCGCAGAUUAGCGAAAAGAGGGUCUCAUUGACUAGUAC is the BCMA aptamer sequence, the RNA form of which is 2'-F substituted on C and U residues;

Drug 92): BCMA aptamer-CD38 aptamer-CD19 aptamer-AmiR-21-DNA carrier, wherein the DNA carrier comprises a sequence a, a sequence b and a sequence c as follows:

the sequence a is SEQ ID No. 15:

GACGCCCACGAGCGTTCCGGGAGAGG<u>TGTAGCACGGTGGC</u>, the underlined portion being the single-stranded bridging sequence A; the sequence b is SEQ ID No. 16: CCTCTCCCGGTTCGCCGCGAGCC<u>TCGGCGCGGCCGTG</u>, the underlined portion being the single-stranded bridging sequence B; the sequence c is SEQ ID No. 17: GGCTCGCGGCCATAGCCGTGGGCGTC<u>TGCTGCTGCTGCTG</u>, he underlined portion being the single-stranded bridging sequence C;

the CD38 aptamer is linked, via a transition sequence TTTTT, to the complementary sequence A' of the single-stranded bridging sequence A, the complementary sequence A' being complementary to the single-stranded bridging sequence A; wherein the sequence of "CD38 aptamer-transition sequence TTTTT-complementary sequence A'" is SEQ ID No. 335: TACGTGAATCTCGTACGATACTCTGTAAGCGT**TTTTT**GCCACCGTGCTACA, SEQ ID No. 170: TACGTGAATCTCGTACGATACTCTGTAAGCGT is the sequence of the CD38 aptamer and the internucleotide phosphate backbone between the first four nucleotides at the 5' end is phosphorothioate-modified; and SEQ ID No. 8: GCCACCGTGCTACA is the complementary sequence A' of the single-stranded bridging sequence A;

the CD19 aptamer is linked, via AmiR-21, to the complementary sequence B' of the single-stranded bridging sequence B, the complementary sequence B' being complementary to the single-stranded bridging sequence B; wherein the sequence of "CD19 aptamer-AmiR-21-complementary sequence B'" is SEQ ID No. 333: UGAGCCCUGUUCGACAG-GAGGCUCA<u>GAUAAGCU</u>CACGGCCGCGCCGA, wherein SEQ ID No. 160: UGAGCCCUGUUCGACAGGAGGCUCA is the sequence of the CD19 aptamer, with the C and U residues 2'-fluoro-modified; GAUAAGCU is the sequence of AmiR-21, with the C and U residues 2'-fluoro-modified; and SEQ ID No. 10: CACGGCCGCGCCGA is the complementary sequence B';

the BCMA aptamer is linked, via a transition sequence AA, to the 5' end of the complementary sequence C' of the single-stranded bridging sequence C, the complementary sequence C' being complementary to the single-stranded bridging sequence C; wherein the sequence of "BCMA aptamer-transition sequence AA-complementary sequence C'" is SEQ ID No. 334: GUGCAAGACGUUCGCAGAUUAGCGAAAAGAGGGUCUCAUUGA CUAGUAC<u>AA</u>CAG CAGCAGCAGCA, wherein SEQ ID No. 12: CAGCAGCAGCAGCA is the complementary sequence C'; and SEQ ID No. 140: AGUGCAAGACGUUCGCAG AUUAGCGAAAAGAGGGUCUCAUUGACUAGUAC is the sequence of the BCMA aptamer; the cytidine (C) and uridine (U) bases in the RNA sequence of the BCMA aptamer are 2'-fluoro-modified;

Drug 93): CpG2006-CD38 aptamer-PD-L1 aptamer-DNA carrier, wherein the DNA carrier comprises a sequence a, a sequence b, and a sequence c as follows:

the sequence a is SEQ ID No. 15: GACGCCCACGAGCGTTCCGGGAGAGG<u>TGTAGCACGGTGGC</u>, the underlined portion being the single-stranded bridging sequence A;

the sequence b is SEQ ID No. 16: CCTCTCCCGGTTCGCCGCGAGCC<u>TCGGCGCGGCCGTG</u>, the underlined portion being the single-stranded bridging sequence B;

the sequence c is SEQ ID No. 17: GGCTCGCGGCCATAGCCGTGGGCGTC<u>TGCTGCTGCTGCTG</u>, <u>the underlined portion being the single-stranded bridging sequence C;</u>

the CD38 aptamer is linked, via a transition sequence TTTTT, to the complementary sequence A' of the single-stranded bridging sequence A, the complementary sequence A' being complementary to the single-stranded bridging sequence A and being connected at the 3' end of the sequence a by complementary base pairing; wherein the sequence of "CD38 aptamer-transition sequence TTTTT-complementary sequence A'" is SEQ ID No. 335: TACGTGAATCTCGTACGATACTCTGTAAGCGT<u>TTTTT</u>GCCACCGTGCTACA; wherein SEQ ID No. 170: TACGTGAATCTCGTACGATACTCTGTAAGCGT is the sequence of the CD38 aptamer and the internucleotide phosphate linkages between the first four nucleotides at the 5' end are phosphorothioate-modified; and SEQ ID No. 8: GCCACCGTGCTACA is the complementary sequence A' of the single-stranded bridging sequence A;

Wherein the PD-L1 aptamer is linked, via a transition sequence TTTTT, to the 5' end of the complementary sequence B' of the single-stranded bridging sequence B, and the PD-L1 aptamer is connected at the 3' end of the sequence b through complementary base pairing between the complementary sequence B' and the single-stranded bridging sequence B;

Wherein CpG2006 is linked, via a transition sequence TTTTT, to the 5' end of the complementary sequence C' of the single-stranded bridging sequence C, and CpG2006 is connected at the 3' end of the sequence c through complementary base pairing between the complementary sequence C' and the single-stranded bridging sequence C;

Wherein the sequence of PD-L1 aptamer-transition sequence TTTTT-complementary sequence B' is SEQ ID No. 336: ACGGGCCACATCAACTCATTGATAGACAATGCGTCCACTGCCCGT<u>TTTTT</u>CACGGCCGCGCCGA, the phosphate backbone between the first four adjacent nucleotides at the 5' end being phosphorothioate-modified; wherein SEQ ID No. 241: ACGGGCCACATCAACTCATTGATAGACAATGCGTCCACTGCCCGT is the sequence of the PD-L1 aptamer, and SEQ ID No. 10: CACGGCCGCGCCGA is the complementary sequence B';

Wherein the sequence of CpG2006-transition sequence TTTTT-complementary sequence C' is SEQ ID No. 337: TCGTCGTTTTGTCGTTTTGTCGTT<u>TTTTT</u>CAGCAGCAGCAGCA, wherein SEQ ID No. 57: TCGTCGTTTTGTCGTTTTGTCGTT s the sequence of CpG2006, the phosphate backbone between adjacent nucleotides within the CpG2006 sequence being phosphorothioate-modified; and SEQ ID No. 12: CAGCAGCAGCAGCA is the complementary sequence C';

Drug 94): BCMA aptamer-CD38 aptamer-CpG-BYZD-PD-L1 aptamer-DNA carrier, wherein the DNA carrier comprises a sequence a, a sequence b, and a sequence c as follows:

the sequence a is SEQ ID No. 15: GACGCCCACGAGCGTTCCGGGAGAGG<u>TGTAGCACGGTGGC</u>, the underlined portion being the single-stranded bridging sequence A;

the sequence b is SEQ ID No. 16: CCTCTCCCGGTTCGCCGCGAGCC<u>TCGGCGCGGCCGTG</u>, the underlined

portion being the single-stranded bridging sequence B;

the sequence c is SEQ ID No. 17: GGCTCGCGGCCATAGCCGTGGGCGTC<u>TGCTGCTGCTGCTG</u>, the underlined portion being the single-stranded bridging sequence C;

Wherein the CD38 aptamer is linked, via CpG-BYZD, to the complementary sequence A' of the single-stranded bridging sequence A, the complementary sequence A' being complementary to the single-stranded bridging sequence A and being joined by complementary base pairing to the 3' end of the sequence a; wherein the sequence of "CD38 aptamer-CpG-BYZD-complementary sequence A'": SEQ ID No. 332: TACGTGAATCTCGTACGATACTCTGTAAGCGTTTAGC GAAGCCACCGTGCTACA; wherein SEQ ID No. 170: TACGTGAATCTCGTAC GATACTCTGTAAGCGT is the sequence of the CD38 aptamer and the internucleotide phosphate backbone between the first four nucleotides at the 5' end is phosphorothioate-modified; SEQ ID No. 8: GCCACCGTGCTACA is the complementary sequence A' of the single-stranded bridging sequence A; TT is the transition sequence; and AGCGAA is the sequence of CpG-BYZD;

Wherein the PD-L1 aptamer is linked, via a transition sequence TTTTT, to the 5' end of the complementary sequence B' of the single-stranded bridging sequence B, and the PD-L1 aptamer is connected at the 3' end of the sequence b through complementary base pairing between the complementary sequence B' and the single-stranded bridging sequence B;

Wherein the BCMA aptamer is directly linked to the 5' end of the complementary sequence C' of the single-stranded bridging sequence C, and the BCMA aptamer is connected at the 3' end of the sequence c through complementary base pairing between the complementary sequence C' and the single-stranded bridging sequence C;

Wherein the sequence of PD-L1 aptamer-transition sequence TTTTT-complementary sequence B' is SEQ ID No. 336: ACGGGCCACATCAACTCATTGATAGACAATGCGTCCACTGCCCGTTTTTTC ACG GCCGCGCCGA, the phosphate backbone between the first four adjacent nucleotides at the 5' end being phosphorothioate-modified; wherein SEQ ID No. 241: ACGGGCCACATCAACTCATTGATAGACAATGCGTCCACTGCCCGT is the sequence of the PD-L1 aptamer; and SEQ ID No. 10: CACGGCCGCGCCGA is the complementary sequence B';

Wherein the sequence of BCMA aptamer-transition sequence AA-complementary sequence C' is SEQ ID No. 334: AGUGCAAGACGUUCGCAGAUUAGCGAAAAGAGGGUCUCAUUGACUAGU ACA ACAGCAGCAGCAGCA; wherein SEQ ID No. 140: AGUGCAAGACGUU CGCAGAUUAGCGAAAAGAGGGUCUCAUUGACUAGUAC is the sequence of the BCMA aptamer, the cytidine (C) and uridine (U) bases in the RNA sequence of the BCMA aptamer being 2'-fluoro-modified; and SEQ ID No. 12: CAGCAGCAGCAGCA is the complementary sequence C';

Drug 95): BCMA aptamer-CD40 aptamer-PD-L1 aptamer-DNA carrier, wherein the DNA carrier comprises a sequence a, a sequence b and a sequence c as follows:

> the sequence a is SEQ ID No. 15: GACGCCCACGAGCGTTCCGGGAGAGG<u>TGTAGCACGGTGGC</u>, the underlined portion being the single-stranded bridging sequence A;
>
> the sequence b is SEQ ID No. 16: CCTCTCCCGGTTCGCCGCGAGCC<u>TCGGCGCGGCCGTG</u>, the underlined portion being the single-stranded bridging sequence B;
>
> the sequence c is SEQ ID No. 17: GGCTCGCGGCCATAGCCGTGGGCGTC<u>TGCTGCTGCTGCTG</u>, the underlined portion being the single-stranded bridging sequence C;
>
> Wherein the CD40 aptamer is linked, via a transition sequence TTTTT, to the complementary sequence A' of the single-stranded bridging sequence A, the complementary sequence A' being complementary to the single-stranded bridging sequence A and being joined by complementary base pairing to the 3' end of the sequence a; wherein the sequence of "CD40 aptamer-transition sequence TTTTT-complementary sequence A‴": SEQ ID No. 338: GCCAA CGAGTAGGCGATAGCGCGTGGC**TTTTT**GCCACCGTGCTACA, wherein SEQ ID No. 156: GCCAACGAGTAGGCGATAGCGCGTGGC is the sequence of the CD40 aptamer and the internucleotide phosphate backbone between the first four nucleotides at the 5' end is phosphorothioate-modified; and SEQ ID No. 8: GCCACCGTGCTACA is the complementary sequence A';

Wherein the PD-L1 aptamer is linked, via a transition sequence TTTTT, to the 5' end of the complementary sequence B' of the single-stranded bridging sequence B, and the PD-L1 aptamer is connected at the 3' end of the sequence b by complementary base pairing between the complementary sequence B' and the single-stranded bridging sequence B;

Wherein the BCMA aptamer is directly linked to the 5' end of the complementary sequence C' of the single-stranded bridging sequence C, and the BCMA aptamer is connected at the 3' end of the sequence c by complementary base pairing between the complementary sequence C' and the single-stranded bridging sequence C;

Wherein the sequence of "PD-L1 aptamer-transition sequence TTTTT-complementary sequence B‴" is SEQ ID No. 336: ACGGGCCACATCAACTCATTGATAGACAATGCGTCCACTGCCCGT<u>TTT TT</u>CACG GCCGCGCCGA, the phosphate backbone between the first four adjacent nucleotides at the 5' end being phosphorothioate-modified; wherein SEQ ID No. 241: ACGGGCCACATCAACTCATTGATAGACAATGCGTCCA CTGCCCGT is the sequence of the PD-L1 aptamer; and SEQ ID No. 10: CACGGCCGCGCCGA is the complementary sequence B';

Wherein the sequence of "BCMA aptamer-transition sequence AA-complementary sequence C‴": SEQ ID No. 334: AGUGCAAGACGUUCGCAGAUUAGCGAAAAGAGGGU CUCAUUGACUAGUACAACA GCAGCAGCAGCA; wherein SEQ ID No. 140: AGUGCAAGACGUUCGCAGAUUAGCGAAAAGAGGGUCUCAUUG ACUAGUAC is the sequence of the BCMA aptamer, the cytidine (C) and uridine (U) bases in the RNA sequence of the BCMA aptamer being 2'-fluoro-modified; and SEQ ID No. 12: CAGCAGCAGCAGCA is the complementary sequence C';

Drug 96): AmiR-21-AS1411 aptamer-CD40 aptamer-2*biotin-DNA carrier; wherein the DNA carrier is selected from group 1 of the nucleic acid carriers as defined in claim 4; the AmiR-21 is linked to the 5' end of the sequence a; the AS 1411 aptamer is linked to the 5' end of the sequence b via the linker sequence "TTTTT"; the CD40 aptamer is linked to the 5' end of the sequence c via the linker sequence "TTTTT"; and one of the biotin modifications is disposed at the 3' end of the sequence a and the other biotin modification is disposed at the 3' end of the sequence c;

the sequence of AmiR-21 is GATAAGCT, each nucleotide being LNA-modified;

the sequence of the AS1411 aptamer is SEQ ID No. 131: GGTGGTGGTGGTTGTGGTGGTGGTGG;

the sequence of the CD40 aptamer is SEQ ID No. 154: CCAACGAGTAGGCGATAGCGCGTGG;

Drug 97): 2*AmiR-21-4*biotin-DNA carrier; wherein the DNA carrier is the group 11 nucleic acid carrier as defined in claim 4; the two AmiR-21 moieties are directly linked respectively to the 5' ends of the sequence a and the sequence c; and the four biotin moieties are respectively linked at the 3' end of the sequence a, the 5' end of the sequence b, the 3' end of the sequence b, and the 3' end of the sequence c;

preferably, the sequence of AmiR-21 is GATAAGCT, each nucleotide being LNA-modified;

Drug 98): TTA1 aptamer-epirubicin-4*biotin-DNA carrier;

Wherein the DNA carrier is selected from group 13 of the nucleic acid carriers as recited in claim 4; four AmiR-21 moieties are respectively and directly linked to the 5' end of the sequence a, the 3' end of the sequence a, the 5' end of the sequence c, and the 3' end of the sequence c; the TTA1 aptamer is linked to the 5' end of the sequence b via a linker sequence; and the epirubicin is inserted, in a specific intercalative manner, between adjacent GC base pairs of the nucleic acid carrier;

preferably, the TTA1 aptamer has the sequence:

SEQ ID No. 265: CCTGCACTTGGCTTGGATTTCAGAAGGGAGACCC;

Drug 99): TTA1 aptamer-2*Survivin siRNA-DNA carrier;

Wherein the DNA carrier is selected from group 13 of the nucleic acid carriers as recited in claim 4; the TTA1 aptamer is linked to the 5' end of the sequence b via a linker sequence; the sense strands of the two Survivin siRNAs are linked to the 3' ends of the sequence b and the sequence c, respectively; and the antisense strands of the two Survivin siRNAs are linked to the 3' ends of the sequence b and the sequence c by complementary hybridization to the respective sense strands of the Survivin siRNA;

preferably, the TTA1 aptamer is SEQ ID No. 265: CCTGCACTTGGCTTGGATTTCAGAAGGGAGACCC;

preferably, the antisense strand of the Survivin siRNA is SEQ ID No. 300: UGUGACAGAUAAGGAACCUGCAG;

And the sense strand of the Survivin siRNA is SEQ ID No. 100: GCAGGUUCCUUAUCUGUCACAUU; and both the sense and antisense strands of the Survivin siRNA have phosphorothioate modifications in the phosphate backbone between adjacent nucleotides at the first three 5'-end positions and between adjacent nucleotides at the last three 3'-end positions;

Drug 100): A CD16a aptamer-CD40 aptamer-CpG2006-DNA carrier, wherein the DNA carrier is selected from group 6 of the nucleic acid carriers as recited in claim 4; the CD16a aptamer is linked to the 5' end of the sequence a via a linker sequence; the CD40 aptamer is linked to the 3' end of the sequence b via the linker sequence; and the CpG2006 is linked to the 5' end of the sequence c via the linker sequence;

preferably, the CD16a aptamer has the sequence SEQ ID No. 157: CCACTGC GGGGGGTCTATACGTGAGGAAGAAGTGG, and the first three internucleotide linkages from the 5' end are phosphorothioate linkages;

preferably, the CD40 aptamer has the sequence SEQ ID No. 154: CCAACGA GTAGGCGATAGCGCGTGG, and the first three internucleotide linkages from the 5' end are phosphorothioate linkages;

preferably, the CpG2006 has the sequence SEQ ID No. 57: TCGTCGTTTTGT CGTTTTGTCGTT, and all internucleotide linkages in the phosphate backbone are phosphorothioate linkages;

preferably, the linker sequence is TTTTT.

13. A pharmaceutical composition comprising any one or more of the nucleic acid drugs according to any one of claims 6-12, and optionally a pharmaceutically acceptable carrier and/or excipient.

14. The composition of claim 13, wherein at least one drug in the pharmaceutical composition is selected from the nucleic acid drugs according to claim 12, the pharmaceutical composition being selected from any one of the following combinations:

Combination 1): Drug 2) + Drug 21);
Combination 2): Drug 6) + Drug 26);
Combination 3): Drug 6) + the OX40 aptamer in RNA form;
Combination 4): Drug 3) + Drug 26);
Combination 5): Drug 3) + Drug 21) + the OX40 aptamer in DNA form;
Combination 6): Drug 4) + Drug 21) + the OX40 aptamer in DNA form;
Combination 7): Drug 4) + Drug 21);
Combination 8): Drug 3) + Drug 23);
Combination 9): Drug 4) + Drug 23);

Combination 10): Drug 3) + Drug 24) + the OX40 aptamer in DNA form;

Combination 11): Drug 3) + Drug 41);

Combination 12): Drug 5) + Drug 41);

Combination 13): Drug 5) + Drug 96);

Combination 14): Drug 96) + Drug 41);

Combination 15): Drug 3) + Drug 97) + the OX40 aptamer in DNA form;

Combination 16): Drug 4) + Drug 20) + the OX40 aptamer in DNA form;

Combination 17): Drug 4) + Drug 20);

Combination 18): Drug 7) + Drug 25) + the OX40 aptamer in DNA form;

Combination 19): Drug 1) + Drug 18) + Drug 98);

Combination 20): Drug 1) + Drug 18) + the OX40 aptamer in DNA form + Drug 99);

Combination 21): Drug 3) + Drug 18) + the OX40 aptamer in DNA form;

Combination 22): Drug 3) + Drug 18);

Combination 23: Drug 3) + the OX40 aptamer in DNA form;

Combination 24): Drug 3) + Drug 18) + the OX40 aptamer in locked nucleic acid (LNA) form;

Combination 25): Drug 3) + Drug 18) + the OX40 aptamer in RNA form;

Combination 26): Drug 3) + Drug 18) + the CD40 aptamer in DNA form;

Combination 27): Drug 3) + Drug 20) + the CD40 aptamer in DNA form;

Combination 28): Drug 4) + Drug 19) + the CD40 aptamer in DNA form;

wherein the OX40 aptamer in DNA form has the sequence:

SEQ ID No. 227: CAGTCTGCATCGTAGGATTAGCCACCGUATCTTTCCCAC;

the OX40 aptamer in RNA form has the sequence:

SEQ ID No. 226:

GGGAGGACGAUGCGGCAGUCUGCAUCGUAGGAAUCGCCACCGUAUACUUUC CCACCAGACGACUCGCUGAGGAUCCGAGA;

the OX40 aptamer in locked nucleic acid form is such that, in the sequence of the OX40 aptamer in DNA form, the first five 5'-end nucleotides and the first four 3'-end nucleotides are LNA-modified.

15. Use of any one of the nucleic acid carriers according to any one of claims 1-5, any one of the nucleic acid drugs according to any one of claims 6-12, or the pharmaceutical composition according to claim 13 or 14, in the preparation of a medicament for treating tumors, liver diseases other than liver cancer, or diseases caused by coronavirus infection.

16. The use according to claim 15, wherein the liver disease other than liver cancer is hepatitis B or hepatitis C; the diseases caused by coronavirus infection include COVID-19; and the tumor is selected from andrological tumors, gynecologic tumors, respiratory system tumors, digestive system tumors, hematologic tumors, urinary system tumors, bone tumors, neurologic tumors, dermatologic tumors, general-surgical tumors, or otorhinolaryngology tumors;

preferably, the andrological tumors are selected from prostate cancer, penile cancer, testicular tumor, or male urethral cancer; the gynecologic tumors are selected from ovarian cancer, cervical cancer, endometrial cancer, uterine myoma, vulvar cancer, or malignant hydatidiform mole; the respiratory system tumors are selected from lung cancer, non-small cell lung cancer, small cell lung cancer, nasopharyngeal carcinoma, tracheal tumor, metastatic lung cancer, pulmonary inflammatory pseudotumor, or radiation-induced lung cancer; the digestive system tumors are selected from liver cancer, gastric cancer, colorectal cancer, gallbladder cancer, esophageal cancer, rectal cancer, pancreatic cancer, or colon cancer; the hematologic tumors are selected from leukemia, lymphoma, lymphosarcoma, or multiple myeloma; the urinary system tumors are selected from renal cancer, bladder cancer, or urinary tract cancer; the bone tumors are selected from giant cell tumor of bone, osteochondroma, or osteosarcoma; the neurologic tumors are selected from brain tumor, meningioma, cerebral tuberculoma, pituitary tumor, neuroblastoma, glioblastoma, or cerebral glioma; the dermatologic tumors are selected from skin cancer or melanoma; the general-surgery tumors are selected from breast cancer, lipoma, thyroid cancer, or thyroid tumor; and the otorhinolaryngology tumors are selected from oral cancer, tongue cancer, laryngeal cancer, middle-ear carcinoma, gingival cancer, or intraorbital tumor;

preferably, in the course of the use, the route of administration of the drug is intratumoral administration, intravenous administration, or intraperitoneal administration;

preferably, in the course of the use, the daily administration dose of the drug is from 0.1 μg/kg to 100 mg/kg.

17. A method for preparing the nucleic acid drug according to any one of claims 6-16, **characterized in that** the method comprises obtaining the nucleic acid drug by self-assembly of at least one strand from a nucleic acid carrier containing the sequence a, the sequence b and the sequence c, and at least one oligonucleotide effector molecule borne on the nucleic acid carrier;

wherein the oligonucleotide effector molecule is selected from at least one of a nucleic acid immunostimulant, a nucleic acid aptamer, an siRNA, an miRNA, and an antisense oligonucleotide (ASO).

18. The method according to claim 17, wherein the nucleic acid drug is obtained by self-assembly of 2-8 strands, preferably 3-6 strands.

19. The method according to claim 18, wherein, when self-assembly involves more than three strands, the method comprises:

providing three sequences, namely the sequence a, the sequence b and the sequence c, which are modified or unmodified, for self-assembly to form the nucleic acid carrier, the modification being a modification with a targeting small molecule;

by sequence complementarity (complementary base pairing), attaching the sequence of at least one oligonucleotide effector molecule to the 5' end and/or the 3' end of at least one of the sequence a, the sequence b and the sequence c of the nucleic acid carrier;

wherein the oligonucleotide effector molecule is selected from at least one of: a nucleic acid immunostimulant, a nucleic acid aptamer, siRNA, miRNA, and an antisense oligonucleotide (ASO).

20. The method according to claim 19, wherein, when the oligonucleotide effector molecule comprises an siRNA;

the sense strand of the siRNA is synthesized at the 5' end and/or the 3' end of at least one of the sequence a, the sequence b and the sequence c, which may be modified or unmodified, and the antisense strand of the siRNA is co-assembled by self-assembly together with the modified or unmodified sequence a, sequence b and sequence c, thereby attaching the siRNA to the nucleic acid carrier through complementary hybridization between the antisense strand and the sense strand of the siRNA;

preferably, the sense strand of the siRNA is directly synthesized onto the 5' end and/or the 3' end of at least one of the sequence a, the sequence b and the sequence c via a linker sequence;

preferably, the linker sequence is selected from 2-8, preferably 3-6, consecutive T, A or U nucleotides.

21. The method according to claim 19, wherein, when the oligonucleotide effector molecule comprises an miRNA and/or the nucleic acid immunostimulant, the miRNA is directly synthesized and/or the nucleic acid immunostimulant is synthesized via a linker sequence at the 5' end and/or the 3' end of at least one of the modified or unmodified sequence a, sequence b and sequence c, and, by self-assembly of the modified or unmodified sequence a, sequence b and sequence c, the miRNA and/or the nucleic acid immunostimulant is/are loaded onto the nucleic acid carrier;

preferably, the linker sequence is selected from 2-8, preferably 3-6, consecutive T or A nucleotides.

22. The method according to claim 19, wherein the oligonucleotide effector molecule comprises a nucleic acid aptamer, the nucleic acid aptamer being synthesized at the 5' end and/or the 3' end of at least one of the sequence a, the sequence b and the sequence c, which may be modified or unmodified, and, by self-assembly of the modified or unmodified sequence a, sequence b and sequence c, the nucleic acid aptamer is loaded onto the nucleic acid carrier;

preferably, the nucleic acid aptamer is directly synthesized via a linker sequence at the 5' end and/or the 3' end of at least one of the sequence a, the sequence b and the sequence c;

preferably, the linker sequence is selected from 2-8, preferably 3-6, consecutive T or A nucleotides.

23. The method according to claim 20, wherein, when the oligonucleotide effector molecule further comprises the nucleic acid immunostimulant and/or the nucleic acid aptamer;

the nucleic acid immunostimulant is synthesized via a linker sequence at the 5' end and/or the 3' end of at least one of the modified or unmodified sequence a, sequence b and sequence c, at an end different from that of the siRNA sense strand; and/or

the nucleic acid aptamer is synthesized via the linker sequence at the 5' end of the siRNA antisense strand to obtain an aptamer-siRNA fragment;
the aptamer-siRNA fragment is co-incubated with the modified or unmodified sequence a, sequence b and sequence c on which the nucleic acid immunostimulant has been synthesized, to effect said self-assembly, thereby mounting the nucleic acid aptamer onto the nucleic acid carrier through complementary hybridization between the siRNA antisense strand and the siRNA sense strand.

24. The method according to claim 22, wherein, when the oligonucleotide effector molecule further comprises the miRNA and the nucleic acid aptamer;

the nucleic acid aptamer is synthesized at the 5' end or the 3' end of the miRNA to obtain an aptamer-miRNA fusion single-stranded sequence;
the aptamer-miRNA fusion single-stranded sequence is caused to participate in said self-assembly, thereby loading the nucleic acid aptamer and the miRNA onto the nucleic acid carrier.

25. The method according to claim 22, wherein, when the oligonucleotide effector molecule further comprises the nucleic acid immunostimulant and the nucleic acid aptamer;

the nucleic acid immunostimulant and the nucleic acid aptamer are each independently synthesized at either end of at least one of the modified or unmodified sequence a, sequence b and sequence c to obtain a nucleic acid immunostimulant and/or nucleic acid aptamer fusion sequence;
the nucleic acid immunostimulant and/or nucleic acid aptamer fusion sequence is caused to participate in said self-assembly, thereby loading the nucleic acid aptamer and the nucleic acid immunostimulant onto the nucleic acid carrier.

26. The method according to claim 19, wherein the sequence a, the sequence b, and the sequence c each comprise a carrier backbone sequence and a single-stranded cohesive-bridge sequence; one or more oligonucleotide effector molecules are self-assembled with the sequences a, b and/or c via sequence a complementary to said single-stranded cohesive-bridge sequence, thereby forming the nucleic acid drug;
preferably, the sequence a, the sequence b and the sequence c are respectively as follows:

the sequence a is SEQ ID No. 15: GACGCCCACGAGCGTTCCGGGAGAGGTGTAGCACGGTGGC, wherein the underlined portion is single-stranded cohesive-bridge sequence A;
the sequence b is SEQ ID No. 16: CCTCTCCCGGTTCGCCGCGAGCCTCGGCGCGGCCGTG, wherein the underlined portion is single-stranded cohesive-bridge sequence B;
the sequence c is SEQ ID No. 17: GGCTCGCGGCCATAGCCGTGGGCGTCTGCTGCTGCTG, where-in the underlined portion is single-stranded cohesive-bridge sequence C.

27. According to the method of claim 25, wherein the nucleic acid drug is a CpG2006-CD40 aptamer-PD-L1 aptamer-DNA carrier, the preparation method comprises any one of the following self-assembly modes:

1) Three-strand self-assembly, wherein the three strands are, respectively:

CpG2006-sequence a: SEQ ID No. 309:
TCGTCGTTTTGTCGTTTTGTCGTTTTTTTGCGACGCCCACGAGCGTTCCGGGAGAGG AGC, wherein the first twenty-four internucleotide linkages from the 5' end are phosphorothioate linkages; "TTTTT" is a linker sequence; the portion preceding "TTTTT" is the CpG2006 sequence, and the portion following "TTTTT" is the sequence a;
CD40 aptamer-sequence b: SEQ ID No. 330:
CCAACGAGTAGGCGATAGCGCGTGG**TTTTT**GCTCCTCTCCCGGTTCGCCGCGAG CCGCG, wherein the first five internucleotide linkages from the 5' end are phosphorothioate linkages; "TTTTT" is a linker sequence; the portion preceding "TTTTT" is the CD40 aptamer sequence, and the portion following "TTTTT" is the sequence b; and
PD-L1 aptamer-sequence c: SEQ ID No. 331:
ACGGGCCACATCAACTCATTGATAGACAATGCGTCCACTGCCCGT**TTTTT**CGCG GCTCGCGGC CATAGCCGTGGGCGTCGC, wherein the first five internucleotide linkages from the 5' end are phosphorothioate linkages; "TTTTT" is a linker sequence; the portion preceding "TTTTT" is the PD-L1 aptamer sequence, and the portion following "TTTTT" is the sequence c;

2) Four-strand self-assembly, wherein the four strands are, respectively:

CpG2006-sequence a:SEQ ID No. 309: TCGTCGTTTTGTCGTTTTGTCGTTTTTTTGCGACGCCCA CGAGCGTTCCGGGAGAGG AGC, wherein the first twenty-four internucleotide linkages from the 5' end are phosphorothioate linkages; TTTTT is a linker sequence; the portion preceding "TTTTT" is the CpG2006 sequence, and the portion following "TTTTT" is the sequence a;

CD40 aptamer-sequence b: SEQ ID No. 339: CCAACGAGTAGGCGATAGCGCGTGG**TTT**GCTCCT CTCCCGGTTCGCCGCGAGCCTC GGCGCGGCCGTG, wherein the first four internucleotide linkages from the 5' end are phosphorothioate linkages; TTT is a linker sequence; the portion preceding "TTT" is the CD40 aptamer sequence, and the portion following "TTT" is the sequence b;

PD-L1 aptamer-linker-complementary sequence B' of single-stranded cohesive-bridge sequence B: SEQ ID No. 336: ACGGGCCACATCAACTCATTGATAGACAATGCGTCCACTGCCCGT**TTTTT**CAC GGCC GCGCCGA, wherein the first four internucleotide linkages from the 5' end are phosphorothioate linkages; TTTTT is a linker sequence; the portion preceding "TTTTT" is the PD-L1 aptamer sequence, and the portion following "TTTTT" is the complementary sequence B'; and

sequence c: SEQ ID No. 340: GGCTCGCGGCCATAGCCGTGGGCGTCGC;

Or,

the four strands are, respectively:

CpG2006-linker-complementary sequence C' of single-stranded cohesive-bridge sequence C: SEQ ID No. 341: TCGTCGTTTTGTCGTTTTGTCGTTT**TTTTT**CAGCAGCAGCAGCA, wherein the first twenty-four internucleotide linkages from the 5' end are phosphorothioate linkages; TTTTT is a linker sequence; the portion preceding "TTTTT" is the CpG2006 sequence, and the portion following "TTTTT" is the complementary sequence C';

sequence a: SEQ ID No. 18: GCGACGCCCACGAGCGTTCCGGGAGAGGAGC;

CD40 aptamer-sequence b: SEQ ID No. 339: CCAACGAGTAGGCGATAGCGCGTGGTTTGCTCCT CTCCCGGTTCGCCGCGAGCCTCG GCGCGGCCGTG, wherein the first four internucleotide linkages from the 5' end are phosphorothioate linkages; TTT is a linker sequence; the portion preceding "TTT" is the CD40 aptamer sequence, and the portion following "TTT" is the sequence b; and

PD-L1 aptamer-sequence c: SEQ ID No. 342: ACGGGCCACATCAACTCATTGATAGACAATGCGT CCACTGCCCGT**TTTTT**GGCTCGC GGCCATAGCCGTGGGCGTCGC, wherein the first four internucleotide linkages from the 5' end are phosphorothioate linkages; TTTTT is a linker sequence; the portion preceding "TTTTT" is the PD-L1 aptamer sequence, and the portion following "TTTTT" is the sequence c;

3) Five-strand self-assembly, wherein the five strands are, respectively:

sequence a: SEQ ID No. 15: GACGCCCACGAGCGTTCCGGGAGAGGTGTAGCACGGTGGC;

CD40 aptamer-linker-sequence b: SEQ ID No. 339: C C A A C G A G T A G G C G A T A G C G C G T G G**TTT**G C T C C T C T C C C G G T T C G C C G C G A G C C TCGGCGCGGCCGTG, wherein the first four internucleotide linkages from the 5' end are phosphorothioate linkages; TTT is the linker sequence; the portion preceding "TTT" is the CD40 aptamer sequence, and the portion following "TTT" is the sequence b;

PD-L1 aptamer-linker-complementary sequence B' of single-stranded cohesive-bridge sequence B: SEQ ID No. 336: ACGGGCCACATCAACTCATTGATAGACAATGCGTCCACTGCCCGT**TTTTT**CAC GGCC GCGCCGA, wherein the first four internucleotide linkages from the 5' end are phosphorothioate linkages; TTTTT is the linker sequence; the portion preceding "TTTTT" is the PD-L1 aptamer sequence, and the portion following "TTTTT" is the complementary sequence B';

CpG2006 sequence-linker-complementary sequence C' of single-stranded cohesive-bridge sequence C: SEQ ID No. 337: TCGTCGTTTTGTCGTTTTGTCGTT**TTTTT**CAGCAGCAGCAGCA, wherein the first twenty-four internucleotide linkages from the 5' end are phosphorothioate linkages; TTTTT is the linker sequence; the portion preceding "TTTTT" is the CpG2006 sequence, and the portion following "TTTTT" is the complementary sequence C';

sequence c: SEQ ID No. 17: GGCTCGCGGCCATAGCCGTGGGCGTCTGCTGCTGCTGCTG;

alternatively, the five strands are, respectively:

CpG2006-linker-sequence a: SEQ ID No. 343: TCGTCGTTTTGTCGTTTTGTCGTT**TTTTT**GAC

GCCCACGAGCGTTCCGGGAGAGGTG TAGCACGGTGGC, wherein the first twenty-four internucleotide linkages from the 5' end are phosphorothioate linkages; TTTTT is the linker sequence; the portion preceding "TTTTT" is the CpG2006 sequence, and the portion following "TTTTT" is the sequence a;

CD40 aptamer-linker-complementary sequence A' of single-stranded cohesive-bridge sequence A: SEQ ID No. 338: GCCAACGAGTAGGCGATAGCGCGTGGC**TTTTT**GCCACCGTGCTACA, wherein the first four internucleotide linkages from the 5' end are phosphorothioate linkages; TTTTT is the linker sequence; the portion preceding "TTTTT" is the CD40 aptamer sequence, and the portion following "TTTTT" is the complementary sequence A';

sequence b: SEQ ID No. 16:
CCTCTCCCGGTTCGCCGCGAGCCTCGGCGCGGCCGTG;

PD-L1 aptamer-linker-complementary sequence B' of single-stranded cohesive-bridge sequence B: SEQ ID No. 336: ACGGGCCACATCAACTCATTGATAGACAATGCGTCCACTGCCCGT**TTTT T**CACGGCC GCGCCGA, wherein the first four internucleotide linkages from the 5' end are phosphorothioate linkages; TTTTT is the linker sequence; the portion preceding "TTTTT" is the PD-L1 aptamer sequence, and the portion following "TTTTT" is the complementary sequence B';

sequence c: SEQ ID No. 340: GGCTCGCGGCCATAGCCGTGGGCGTCGC;

4) Six-strand self-assembly, wherein the six strands are, respectively:

sequence a: SEQ ID No. 15:
GACGCCCACGAGCGTTCCGGGAGAGGTGTAGCACGGTGGC;

sequence b: SEQ ID No. 16:
CCTCTCCCGGTTCGCCGCGAGCCTCGGCGCGGCCGTG;

sequence c: SEQ ID No. 17:
GGCTCGCGGCCATAGCCGTGGGCGTCTGCTGCTGCTGCTG;

CpG2006-linker-complementary sequence C' of single-stranded cohesive-bridge sequence C: SEQ ID No. 337:
TCGTCGTTTTGTCGTTTTGTCGTT**TTTTT**CAGCAGCAGCAGCA, wherein the first twenty-four internucleotide linkages from the 5' end are phosphorothioate linkages; TTTTT is the linker sequence; the portion preceding "TTTTT" is the CpG2006 sequence, and the portion following "TTTTT" is the complementary sequence C';

CD40 aptamer-linker-complementary sequence A' of single-stranded cohesive-bridge sequence A: SEQ ID No. 338: GCCAACGAGTAGGCGATAGCGCGTGGC**TTTTT**GCCACCGTGCTACA, wherein the first four internucleotide linkages from the 5' end are phosphorothioate linkages; TTTTT is the linker sequence; the portion preceding "TTTTT" is the CD40 aptamer sequence, and the portion following "TTTTT" is the complementary sequence A';

PD-L1 aptamer-linker-complementary sequence B' of single-stranded cohesive-bridge sequence B: SEQ ID No. 336: ACGGGCCACATCAACTCATTGATAGACAATGCGTCCACTGCCCGT**TTTTT**CAC GGCC GCGCCGA, wherein the first four internucleotide linkages from the 5' end are phosphorothioate linkages; TTTTT is the linker sequence; the portion preceding "TTTTT" is the PD-L1 aptamer sequence, and the portion following "TTTTT" is the complementary sequence B'.

28. The method according to any one of claims 17-27, wherein the step of self-assembly comprises:

dissolving the modified or unmodified sequence a, sequence b and sequence c bearing at least one of the oligonucleotide effector molecule, and optionally the separately synthesized sequence(s) of the oligonucleotide effector molecule(s), in an assembly solution, followed by sequentially denaturation and annealing (renaturation) to obtain a crude self-assembly product;

subjecting the crude self-assembly product sequentially to purification, elution and evaporation to dryness to obtain a targeted nucleic acid carrier bearing at least one of the oligonucleotide effector molecule;

preferably, the molar ratio among the sequence a, the sequence b and the sequence c is 0.90-1.10:0.90-1.10:0.90-1.10, more preferably 1:1:1;

preferably, the assembly solution is a TMS aqueous solution, a sodium chloride aqueous solution, a magnesium chloride aqueous solution, or purified water;

preferably, the temperature of the denaturation reaction is 80-99 °C, more preferably 85-99 °C, and further preferably 90-99 °C;

preferably, upon completion of the denaturation reaction, the reaction system is cooled to the annealing temperature to carry out the annealing reaction and finally cooled to obtain the crude self-assembly product; wherein the holding temperature is 70-50 °C, more preferably 65-55 °C, and further preferably 63-57 °C; the annealing time is 3-15 min, more preferably 3-10 min, and further preferably 3-5 min;

preferably, during cooling of the reaction system to the holding temperature, the cooling rate is 2-10 °C/min, more preferably 2-6 °C/min, and further preferably 2-3 °C/min;

preferably, the final cooling end temperature is 0-25 °C, more preferably 0-15 °C, and further preferably 0-4 °C;

preferably, the pH of the denaturation reaction is 5.4-8.8.

## Chromatogram of Sample C1

| Peak No. | Retention Time | Peak Area | Peak Height | Peak Width | Symmetry Factor | Peak Area (%) |
|---|---|---|---|---|---|---|
| 1 | 20.701 | 13364.9 | 243.8 | 1.371 | 0 | 82.484 |
| 2 | 22.272 | 2217.6 | 34.7 | 1.0663 | 0.208 | 13.686 |
| 3 | 24.514 | 328.9 | 4.9 | 1.1286 | 6.4E-3 | 2.030 |
| 4 | 25.901 | 291.7 | 3.4 | 1.4501 | 4.7E-2 | 1.800 |

## FIG. 1-1

## Chromatogram of Sample C2

| Peak No. | Retention Time | Peak Area | Peak Height | Peak Width | Symmetry Factor | Peak Area (%) |
|---|---|---|---|---|---|---|
| 1 | 19.135 | 852.1 | 15.9 | 1.092 | 0 | 3.501 |
| 2 | 20.667 | 19222.4 | 336.1 | 0.9531 | 0.654 | 78.986 |
| 3 | 22.284 | 3237.1 | 51 | 1.0585 | 0.275 | 13.301 |
| 4 | 24.068 | 436.2 | 7.4 | 0.9843 | 0 | 1.792 |
| 5 | 25.835 | 588.7 | 6.5 | 1.5181 | 0 | 2.419 |

# FIG. 1-2

**Chromatogram of Sample D1**

| Peak No. | Retention Time | Peak Area | Peak Height | Peak Width | Symmetry Factor | Peak Area (%) |
|---|---|---|---|---|---|---|
| 1 | 21.047 | 161.6 | 2 | 1.3357 | 0.271 | 1.833 |
| 2 | 22.577 | 6826.9 | 118.8 | 0.9574 | 0.759 | 77.436 |
| 3 | 24.308 | 1151.8 | 18.3 | 1.0471 | 0.502 | 13.065 |
| 4 | 26.76 | 536.7 | 7.8 | 1.1085 | 9.16E-2 | 6.088 |
| 5 | 29.013 | 83.4 | 1.3 | 1.0421 | 0 | 0.946 |
| 6 | 31.287 | 55.7 | 7E-1 | 1.3207 | 0 | 0.632 |

# FIG. 1-3

## Chromatogram of Sample D2

| Peak No. | Retention Time | Peak Area | Peak Height | Peak Width | Symmetry Factor | Peak Area (%) |
|---|---|---|---|---|---|---|
| 1 | 18.596 | 1379.1 | 19.4 | 1.1864 | 0 | 4.443 |
| 2 | 19.602 | 435.3 | 11.1 | 0.6552 | 9.85E-3 | 1.402 |
| 3 | 20.642 | 554.2 | 10.9 | 0.8439 | 7.41E-2 | 1.786 |
| 4 | 22.538 | 20812.6 | 335 | 1.0355 | 0.759 | 67.057 |
| 5 | 24.262 | 4096.7 | 58.8 | 1.1613 | 0.354 | 13.199 |
| 6 | 26.716 | 2457.8 | 28.8 | 1.4236 | 2.07E-2 | 7.919 |
| 7 | 28.869 | 918.8 | 9.2 | 1.4202 | 7.37E-2 | 2.960 |
| 8 | 30.722 | 382.9 | 4.2 | 1.5269 | 2.29E-2 | 1.234 |

# FIG. 1-4

**FIG. 2**

**FIG. 3**

**FIG. 4**

FIG. 5-1

### Sample Chromatogram

| Peak No. | Retention Time | Peak Area | Peak Height | Peak Width | Symmetry Factor | Peak Area (%) |
|----------|----------------|-----------|-------------|------------|-----------------|---------------|
| 1 | 20.701 | 13364.9 | 243.8 | 1.371 | 0 | 82.484 |
| 2 | 22.272 | 2217.6 | 34.7 | 1.0663 | 0.208 | 13.686 |
| 3 | 24.514 | 328.9 | 4.9 | 1.1286 | 6.4E-3 | 2.030 |
| 4 | 25.901 | 291.7 | 3.4 | 1.4501 | 4.7E-2 | 1.800 |

FIG. 5-2

FIG. 5-3

**FIG. 5-4**

**FIG. 6**

**FIG. 7**

**FIG. 8**

**FIG. 9**

**FIG. 10**

**FIG. 11**

**FIG. 12-1**

CPG2006-CD40-PD-L1-DNA （CCPD3）

| Peak No. | Retention Time | Peak Area | Peak Height | Peak Width | Symmetry Factor | Peak Area (%) |
|---|---|---|---|---|---|---|
| 1 | 18.42 | 1171.6 | 30.8 | 0.572 | 0.821 | 6.873 |
| 2 | 19.643 | 13976.7 | 396.1 | 0.5397 | 0.684 | 81.992 |
| 3 | 20.979 | 1284.9 | 25.1 | 0.7095 | 0.444 | 7.537 |
| 4 | 23.669 | 363.3 | 6.6 | 0.8192 | 0.871 | 2.131 |
| 5 | 25.09 | 250 | 3 | 1.2324 | 1.783 | 1.467 |

**FIG. 12-2**

FIG. 12-3

**FIG. 13-1**

CPG2006-CD40-PD-L1-DNA　（CCPD3）

| Peak No. | Retention Time | Peak Area | Peak Height | Peak Width | Symmetry Factor | Peak Area (%) |
|---|---|---|---|---|---|---|
| 1 | 18.987 | 731 | 14.5 | 0.7196 | 1.044 | 8.676 |
| 2 | 20.431 | 7223.2 | 163.7 | 0.6569 | 0.691 | 85.772 |
| 3 | 21.971 | 212.5 | 7.6 | 0.3434 | 6.77E-2 | 2.522 |
| 4 | 22.614 | 188.2 | 4.7 | 0.4827 | 0.119 | 2.234 |
| 5 | 24.798 | 34 | 1.7 | 0.2593 | 8.019 | 0.403 |
| 6 | 24.868 | 37.4 | 1.7 | 0.2828 | 7.69E-2 | 0.444 |

**FIG. 13-2**

**FIG. 13-3**

**FIG. 13-4**

**FIG. 13-5**

**FIG. 13-6**

**FIG. 13-7**

**FIG. 13-8**

**FIG. 13-9**

| Peak No. | Retention Time | Peak Area | Peak Height | Peak Width | Symmetry Factor | Peak Area (%) |
|----------|----------------|-----------|-------------|------------|-----------------|---------------|
| 1 | 18.558 | 1388.2 | 31.8 | 0.6218 | 0.844 | 7.278 |
| 2 | 19.929 | 15883.7 | 397.5 | 0.5902 | 0.679 | 83.270 |
| 3 | 21.311 | 1355.9 | 23.7 | 0.7862 | 0.245 | 7.108 |
| 4 | 24.19 | 179.8 | 3.5 | 0.7661 | 1.017 | 0.942 |
| 5 | 25.147 | 267.4 | 4 | 0.9924 | 0.609 | 1.402 |

**FIG. 13-10**

| Peak No. | Retention Time | Peak Area | Peak Height | Peak Width | Symmetry Factor | Peak Area (%) |
|---|---|---|---|---|---|---|
| 1 | 18.607 | 1059.9 | 24.7 | 0.6216 | 0.706 | 6.762 |
| 2 | 19.92 | 12781.3 | 341.4 | 0.5608 | 0.677 | 81.538 |
| 3 | 21.315 | 1299.3 | 21.7 | 0.8151 | 0.383 | 8.289 |
| 4 | 24.12 | 298.2 | 5.2 | 0.8398 | 0.783 | 1.902 |
| 5 | 25.115 | 236.4 | 3.6 | 0.9589 | 0.361 | 1.508 |

## FIG. 13-11

Detection Chromatogram (Enlarged View)

| Peak No. | Retention Time | Peak Area | Peak Height | Peak Width | Symmetry Factor | Peak Area (%) |
|---|---|---|---|---|---|---|
| 1 | 18.94 | 549.4 | 13.1 | 0.6075 | 1.189 | 3.452 |
| 2 | 20.454 | 13022.1 | 307.4 | 0.6222 | 0.837 | 81.832 |
| 3 | 21.952 | 739.5 | 17.3 | 0.642 | 0.504 | 4.647 |
| 4 | 22.788 | 473.8 | 9.2 | 0.6904 | 0.543 | 2.978 |
| 5 | 24.89 | 470.9 | 6.1 | 1.0816 | 1.975 | 2.959 |
| 6 | 25.719 | 657.5 | 5.7 | 1.4888 | 0.187 | 4.132 |

## FIG. 13-12

Detection Chromatogram (Enlarged View)

| Peak No. | Retention Time | Peak Area | Peak Height | Peak Width | Symmetry Factor | Peak Area (%) |
|---|---|---|---|---|---|---|
| 1 | 18.951 | 710.2 | 16.3 | 0.653 | 1.318 | 3.941 |
| 2 | 20.474 | 14300.3 | 328.2 | 0.6524 | 0.902 | 79.346 |
| 3 | 22.053 | 1539.9 | 19.8 | 1.0667 | 0.627 | 8.544 |
| 4 | 24.826 | 1472.2 | 11.1 | 1.7536 | 0.439 | 8.169 |

**FIG. 13-13**

**FIG.14-1**

**FIG.14-2**

**FIG.14-3**

**FIG.14-4**

**FIG.14-5**

**FIG.14-6**

**FIG.14-7**

**FIG.14-8**

**FIG.14-9**

**FIG.14-10**

454

**FIG.14-11**

**FIG.14-12**

**FIG.14-13**

| Peak No. | Retention Time | Peak Area | Peak Height | Peak Width | Symmetry Factor | Peak Area (%) |
|---|---|---|---|---|---|---|
| 1 | 9.212 | 102.3 | 2.8 | 0.5734 | 0.749 | 0.576 |
| 2 | 18.253 | 876 | 22 | 0.5916 | 0.926 | 4.932 |
| 3 | 19.4 | 13488 | 372.9 | 0.5499 | 0.697 | 75.949 |
| 4 | 20.401 | 2655.7 | 53.3 | 0.6777 | 0.465 | 14.960 |
| 5 | 22.588 | 636.2 | 5.2 | 1.5639 | 0.589 | 3.582 |

# FIG.14-14

3AmiR-21/AS1411/FAP-DNA

| Peak No. | Retention Time | Peak Area | Peak Height | Peak Width | Symmetry Factor | Peak Area (%) |
|---|---|---|---|---|---|---|
| 1 | 19.983 | 5861.5 | 66.3 | 1.4726 | 0 | 95.665 |
| 2 | 24.676 | 184.2 | 1.7 | 1.8139 | 0 | 3.007 |
| 3 | 26.276 | 81.3 | 1.1 | 1.2134 | 4.98E-2 | 1.328 |

# FIG.14-15A

FIG.14-15B

FIG.14-16

**FIG.14-17**

**FIG.14-18**

**FIG.14-19**

**FIG.14-20**

**FIG.14-21A**

**FIG.14-21B**

FIG.14-21C

FIG.14-21D

| Peak No. | Retention Time | Peak Area | Peak Height | Peak Width | Symmetry Factor | Peak Area (%) |
|---|---|---|---|---|---|---|
| 1 | 18.933 | 126.3 | 2.8 | 0.633 | 0.549 | 2.283 |
| 2 | 21.204 | 4166.4 | 119.4 | 0.5191 | 0.642 | 75.309 |
| 3 | 22.096 | 614.5 | 19.7 | 0.46 | 0.428 | 11.106 |
| 4 | 23.235 | 290 | 6.7 | 0.6661 | 0.876 | 5.241 |
| 5 | 24.695 | 36.1 | 1.4 | 0.4305 | 0.955 | 0.653 |
| 6 | 26.372 | 63.8 | 1.4 | 0.6861 | 0.415 | 1.153 |
| 7 | 27.457 | 70.9 | 2 | 0.5072 | 0.817 | 1.282 |
| 8 | 28.287 | 164.5 | 3 | 0.7333 | 0.591 | 2.973 |

## FIG.14-22

## FIG.14-23

**FIG.14-24**

**FIG.14-25**

**FIG.14-26A**

AS1411-ASAPisiRNA/VEGF/SARS-COV-2-N48-RNA

| Peak No. | Retention Time | Peak Area | Peak Height | Peak Width | Symmetry Factor | Peak Area (%) |
|----------|----------------|-----------|-------------|------------|-----------------|---------------|
| 1 | 18.629 | 178.7 | 4.9 | 0.6114 | 0 | 3.999 |
| 2 | 19.998 | 3861.5 | 48.7 | 1.3214 | 0.931 | 86.417 |
| 3 | 23.496 | 210.1 | 3.1 | 1.1425 | 5.39E-2 | 4.703 |
| 4 | 24.062 | 218.1 | 2.4 | 1.0646 | 0 | 4.881 |

**FIG.14-26B**

**FIG.14-27**

**FIG.14-28**

**FIG.14-29**

**FIG.14-30**

**FIG.14-31**

**FIG.14-32**

**FIG.14-33**

**FIG.14-34**

**FIG.14-35**

**FIG.14-36**

**FIG.14-37**

**FIG.14-38**

**FIG.14-39**

**FIG.14-40**

**FIG.14-41**

| Peak No. | Retention Time | Peak Area | Peak Height | Peak Width | Symmetry Factor | Peak Area (%) |
|----------|----------------|-----------|-------------|------------|-----------------|---------------|
| 1 | 18.558 | 1388.2 | 31.8 | 0.6218 | 0.844 | 7.278 |
| 2 | 19.929 | 15883.7 | 397.5 | 0.5902 | 0.679 | 83.270 |
| 3 | 21.311 | 1355.9 | 23.7 | 0.7862 | 0.245 | 7.108 |
| 4 | 24.19 | 179.8 | 3.5 | 0.7661 | 1.017 | 0.942 |
| 5 | 25.147 | 267.4 | 4 | 0.9924 | 0.609 | 1.402 |

**FIG.15-1**

| Peak No. | Retention Time | Peak Area | Peak Height | Peak Width | Symmetry Factor | Peak Area (%) |
|---|---|---|---|---|---|---|
| 1 | 18.813 | 968.5 | 21.9 | 0.6392 | 0.872 | 5.326 |
| 2 | 20.274 | 14068.7 | 336.5 | 0.6158 | 0.656 | 80.278 |
| 3 | 21.657 | 1292.6 | 28.2 | 0.7783 | 0.206 | 7.375 |
| 4 | 24.519 | 1195.5 | 15.1 | 1.1101 | 0.428 | 6.821 |

**FIG.15-2**

| Peak No. | Retention Time | Peak Area | Peak Height | Peak Width | Symmetry Factor | Peak Area (%) |
|---|---|---|---|---|---|---|
| 1 | 18.582 | 655.8 | 15.7 | 0.6154 | 0.77 | 5.077 |
| 2 | 19.906 | 9961.5 | 269.4 | 0.5551 | 0.698 | 75.340 |
| 3 | 21.269 | 954 | 18.1 | 0.7418 | 0.461 | 7.385 |
| 4 | 23.989 | 1446.6 | 23.8 | 0.9004 | 0.555 | 11.198 |

**FIG.15-3**

| Peak No. | Retention Time | Peak Area | Peak Height | Peak Width | Symmetry Factor | Peak Area (%) |
|---|---|---|---|---|---|---|
| 1 | 18.697 | 776.6 | 19 | 0.5916 | 0.828 | 5.335 |
| 2 | 20.126 | 11081.5 | 284.2 | 0.5749 | 0.71 | 76.133 |
| 3 | 21.563 | 805.3 | 18.6 | 0.5924 | 0.613 | 5.533 |
| 4 | 22.158 | 566.6 | 13.7 | 0.5848 | 0.14 | 3.893 |
| 5 | 24.363 | 678 | 11 | 0.86 | 1.187 | 4.658 |
| 6 | 25.401 | 647.4 | 9.2 | 0.9382 | 0.469 | 4.448 |

# FIG.15-4

**FIG.15-5**

FIG.15-6

FIG.15-7

**FIG.15-8**

**FIG.15-9**

**FIG.15-10**

FIG.15-11

**FIG.15-12**

| Peak No. | Retention Time | Peak Area | Peak Height | Peak Width | Symmetry Factor | Peak Area (%) |
|---|---|---|---|---|---|---|
| 1 | 17.574 | 314.4 | 13.8 | 0.3456 | 0.66 | 2.345 |
| 2 | 18.114 | 1277.5 | 28.5 | 0.6562 | 0.559 | 9.530 |
| 3 | 19.61 | 9950.8 | 278.9 | 0.5283 | 0.707 | 74.220 |
| 4 | 20.983 | 1202.9 | 23.9 | 0.6982 | 0.391 | 8.972 |
| 5 | 23.706 | 92.4 | 2.9 | 0.5162 | 1.079 | 0.689 |
| 6 | 24.828 | 187.9 | 5.7 | 0.4816 | 0.738 | 1.401 |
| 7 | 25.332 | 222.2 | 7.3 | 0.4976 | 0.48 | 1.657 |
| 8 | 26.631 | 59.2 | 2.2 | 0.431 | 1.215 | 0.442 |
| 9 | 27.388 | 99.6 | 2.2 | 0.7311 | 2.512 | 0.743 |

# FIG.15-13

| Peak No. | Retention Time | Peak Area | Peak Height | Peak Width | Symmetry Factor | Peak Area (%) |
|---|---|---|---|---|---|---|
| 1 | 18.558 | 1388.2 | 31.8 | 0.6218 | 0.844 | 7.278 |
| 2 | 19.929 | 15883.7 | 397.5 | 0.5902 | 0.679 | 83.270 |
| 3 | 21.311 | 1355.9 | 23.7 | 0.7862 | 0.245 | 7.108 |
| 4 | 24.19 | 179.8 | 3.5 | 0.7661 | 1.017 | 0.942 |
| 5 | 25.147 | 267.4 | 4 | 0.9924 | 0.609 | 1.402 |

# FIG.15-14

Mean Body Weight of Mice (Intravenous Administration)

**FIG.16-1**

Mean Body Weight of Mice (Intratumoral Injection Administration)

**FIG.16-2**

Left-Side Tumor Growth Curve (Intravenous Administration)

**FIG.16-3**

Left-Side Tumor Growth Curve (Intravenous Administration)

**FIG.16-4**

Left-Side Tumor Growth Curve Following Intratumoral Injection Administration

**FIG.16-5**

Left-Side Tumor Growth Curve Following Intratumoral Injection Administration

**FIG.16-6**

Right-Side Tumor Growth Curve Following Intratumoral Injection Administration

**FIG.16-7**

Right-Side Tumor Growth Curve Following Intratumoral Injection Administration

**FIG.16-8**

## Tumor Weight (Tail Vein Injection Administration)

**FIG.16-9**

## Tumor Weight (Intratumoral Injection Administration)

**FIG.16-10**

**FIG.17-1**

**FIG.17-2**

FIG.17-3

FIG.17-4

## Tumor Weight

**FIG.17-5**

**FIG.18-1**

## Body Weight Gain Curve

**FIG.18-2**

Tumor Growth Curve of the Treated-Side Tumor

**FIG.18-3**

Tumor Growth Curve of the Untreated-Side Tumor

**FIG.18-4**

## Treated-Side Tumor Weight

**FIG.18-5**

## Untreated-Side Tumor Weight

**FIG.18-6**

Tumor Inoculation    Grouping

Sample Collection

D0    D6    D8    D10    D12    D14    D16    D18    D20    D23

Administration    √    √    √    √    √    √    √    √

# FIG.19-1

**Body Weight Growth Curve of Mice**

**Days Post Inoculation**

Negative Control-1 (PBS) Group — Negative Control-2 (PBS) Group — Group A
Group B — Group C — Group D

# FIG.19-2

Right-Side Tumor Growth Curve

FIG.19-3

Left-Side Tumor Growth Curve

FIG.19-4

**FIG.19-5**

**FIG.19-6**

## Mean Body Weight of Mice

**FIG.20-1**

Left-Side Tumor Growth Curve

**FIG.20-2**

Left-Side Tumor Growth Curve

FIG.20-3

Right-Side Tumor Growth Curve

FIG.20-4

Right-Side Tumor Growth Curve

**FIG.20-5**

Tumor Weight

**FIG.20-6**

**Mean Body Weight of Mice**

**Days after transplanation**

# FIG.21-1

**Tumor Growth Curve**

**Days after transplanation**

# FIG.21-2

**Tumor Growth Curve**

**FIG.21-3**

**Tumor Weight**

**FIG.21-4**

Tumor inoculation

Two animals were assigned to each of Groups A and B

The remaining animals were assigned to the groups

Sample collection

D0     D7     D9     D11     D13     D15     D17     D26

Administration to the first two animals in Groups A/B    √    √    √    √    √

Administration to the remaining animals    √    √    √    √    √

**FIG.22-1**

## Animal Body Weight Gain Curve

Body Weight (g) vs Days After Inoculation

Group A — Group B — Group C

**Days After Inoculation**

**FIG.22-2**

## Tumor Growth Curve on the Drug-Administration Side

**FIG.22-3**

## Tumor Growth Curve on the Non-Drug-Administration Side

**FIG.22-4**

Average Body Weight of Mice

**FIG.23-1**

Tumor Growth Curve

**FIG.23-2**

Tumor Growth Curve

**FIG.23-3**

Tumor Weight

**FIG.23-4**

**Average Body Weight of Mice**

FIG.24-1

**Tumor Growth Curve**

FIG.24-2

**Tumor Growth Curve**

**FIG.24-3**

**Tumor Weight**

**FIG.24-4**

**Average Body Weight of Mice**

**FIG.25-1**

**Tumor Growth Curve**

**FIG.25-2**

**Tumor Weight**

**FIG.25-3**

**Average Body Weight of Mice**

Days after transplanation

**FIG.26-1**

**Tumor Growth Curve**

## FIG.26-2

**Tumor Weight**

## FIG.26-3

**Average Body Weight of Mice**

FIG.27-1

**Tumor Growth Curve**

FIG.27-2

**Tumor Growth Curve**

**FIG.27-3**

**Tumor Weight**

**FIG.27-4**

**Average Body Weight of Mice**

**FIG.28-1**

**Tumor Growth Curve**

**FIG.28-2**

FIG.28-3

FIG.28-4

## Body Weight Curve(n=8)

**FIG.29-1**

## Relative Body Weight

**FIG.29-2**

**FIG.29-3**

**FIG.30-1**

**Tumor Growth Curve**

FIG.30-2

**Tumor Growth Curve**

FIG.30-3

**FIG.30-4**

**FIG.31-1**

**FIG.31-2**

**Tumor Growth Curve**

## FIG.31-3

## FIG.31-4

**Average Body Weight of Mice**

**FIG.32-1**

**Tumor Growth Curve**

**FIG.32-2**

**FIG.32-3**

Average Body Weight of Mice

Days after transplantation

**FIG.33-1**

**FIG.33-2**

**FIG.33-3**

**Average Body Weight of Mice**

**FIG.34-1**

**Tumor Growth Curve**

**FIG.34-2**

**FIG.34-3**

**Average Body Weight of Mice**

**Days after transplantation**

**FIG.35-1**

**Tumor Growth Curve**

FIG.35-2

FIG.35-3

**Average Body Weight of Mice**

FIG.36-1

**Tumor Growth Curve**

FIG.36-2

**FIG.36-3**

Average Body Weight of Mice

Days after transplantation

**FIG.37-1**

**FIG.37-2**

**FIG.37-3**

**Average Body Weight of Mice**

FIG.38-1

**Tumor Growth Curve**

FIG.38-2

**FIG.38-3**

Average Body Weight of Mice

**FIG.39-1**

**Tumor Growth Curve**

FIG.39-2

FIG.39-3

**Average Body Weight of Mice**

FIG.40-1

**Tumor Growth Curve**

FIG.40-2

**FIG.40-3**

**FIG.41-1**

**Animal Body Weight Gain Curve**

**FIG.41-2**

**Tumor Growth Curve**

**FIG.41-3**

**Average Body Weight of Mice**

FIG.42-1

**Tumor Growth Curve**

FIG.42-2

**FIG.42-3**

Average Body Weight of Mice

**FIG.43-1**

**FIG.43-2**

**FIG.43-3**

FIG.44-1

FIG.44-2

CCPD3-Cy5+ Monocytes

A

Monocytes

B

CCPD3-Cy5+ lymphocytes

C

lymphocytes

D

■ Human
■ Monkey—Cynomolgus monke
■ Dog—Beagle dog
■ Mouse—C57BL
■ Rat—Sprague-Dawley (SD)

**FIG.44-3**

**FIG.44-4**

**FIG.44-5**

FIG.44-6

**FIG.44-7**

**FIG.44-8**

**FIG.44-9**

A

B

C

D

FIG.44-10

FIG.44-11

G1(D32), HE 20×   G1(D32), HE 20×   G1(D32),HE 200×   G1(D39), HE 20×

G1(D39), HE 200×   G1(D41), HE 20×   G1(D41), HE 200×

G2(D34), HE 20×   G2(D34), HE 20×   G2(D34), HE 200×   G2(D37), HE 20×

G2(D37), HE 200×   G2(D138), HE 20×   G2(D138), HE 20×

**FIG.44-12**

CCPD3   CPG2006   CD40   PD-L1   CPG2006–CCPD3 a-strand   CD40–CCPD3 b-strand

PD-L1–CCPD3 c-strand   CPG2006–DNA3   CD40–DNA3   PD-L1–DNA3   DNA3

**FIG.44-13**

FIG.44-14

## INTERNATIONAL SEARCH REPORT

| | |
|---|---|
| | International application No. |
| | **PCT/CN2024/112170** |

| A. | CLASSIFICATION OF SUBJECT MATTER |
|---|---|

C12N15/87(2006.01)i

According to International Patent Classification (IPC) or to both national classification and IPC

| B. | FIELDS SEARCHED |
|---|---|

Minimum documentation searched (classification system followed by classification symbols)

IPC:C12N

Documentation searched other than minimum documentation to the extent that such documents are included in the fields searched

Electronic data base consulted during the international search (name of data base and, where practicable, search terms used)

CNABS, CNTXT, EPTXT, JPTXT, TWTXT, USTXT, VEN, WOTXT, cnki, 万方, WANFANG, ncbi, 百度, BAIDU, 百度学术, BAIDU SCHOLAR, 中国专利生物序列检索系统, China Patents Biological Sequence Search System, blast, EBI, STN, Uniprot: 百药智达（北京）纳米生物技术有限公司, 王力源, 魏鸿, 王萌, 核酸载体, DNA载体, RNA载体, 载体, SEQ ID NO: 1-56

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 117343965 A (BAIYAO ZHIDA (BEIJING) NANO BIOTECHNOLOGY CO., LTD.) 05 January 2024 (2024-01-05)<br>claims 1-28 | 1-28 |
| PX | CN 116785445 A (BAIYAO ZHIDA (BEIJING) NANO BIOTECHNOLOGY CO., LTD.) 22 September 2023 (2023-09-22)<br>claims 1-14, description, paragraphs [0004]-[0011] and [0053]-[0274] | 1-28 |
| X | CN 110711253 A (BAIYAO ZHIDA (BEIJING) NANO BIOTECHNOLOGY CO., LTD.) 21 January 2020 (2020-01-21)<br>claims 1-11, 14, and 17, and description, paragraphs [0005], [0042], [0094], [0097], [0113]-[0130], [0132]-[0133], [0135], [0137], [0147], and [0176]-[0181] | 1-28 |
| A | CN 114222616 A (LEMONEX INC.) 22 March 2022 (2022-03-22)<br>entire document | 1-28 |

| ✓ Further documents are listed in the continuation of Box C. | ✓ See patent family annex. |
|---|---|

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 November 2024** | **15 November 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/112170** |

## C. DOCUMENTS CONSIDERED TO BE RELEVANT

| Category* | Citation of document, with indication, where appropriate, of the relevant passages | Relevant to claim No. |
| --- | --- | --- |
| A | CN 105018529 A (SHANGHAI JIAO TONG UNIVERSITY) 04 November 2015 (2015-11-04) <br> entire document | 1-28 |
| A | CN 106362163 A (SHANGHAI JIAO TONG UNIVERSITY) 01 February 2017 (2017-02-01) <br> entire document | 1-28 |

Form PCT/ISA/210 (second sheet) (July 2022)

## INTERNATIONAL SEARCH REPORT

| International application No. |
|---|
| **PCT/CN2024/112170** |

**Box No. I**       **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

     a.    ☑   forming part of the international application as filed.

     b.    ☐   furnished subsequent to the international filing date for the purposes of international search (Rule 13*ter*.1(a)),

          ☐   accompanied by a statement to the effect that the sequence listing does not go beyond the disclosure in the international application as filed.

2.   ☐   With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/CN2024/112170**

| Patent document cited in search report | | | Publication date (day/month/year) | Patent family member(s) | | | Publication date (day/month/year) |
|---|---|---|---|---|---|---|---|
| CN | 117343965 | A | 05 January 2024 | None | | | |
| CN | 116785445 | A | 22 September 2023 | None | | | |
| CN | 110711253 | A | 21 January 2020 | None | | | |
| CN | 114222616 | A | 22 March 2022 | EP | 3991755 | A1 | 04 May 2022 |
| | | | | EP | 3991755 | A4 | 14 June 2023 |
| | | | | EP | 3991755 | B1 | 21 August 2024 |
| | | | | KR | 20220004588 | A | 11 January 2022 |
| | | | | KR | 102698700 | B1 | 27 August 2024 |
| CN | 105018529 | A | 04 November 2015 | None | | | |
| CN | 106362163 | A | 01 February 2017 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310871667